# EUROPEAN PATENT SPECIFICATION

(11) **EP 0 908 457 B1**
(45) Date of publication and mention of the grant of the patent: **13.11.2002**
(21) Application number: 98307495.6
(22) Date of filing: 16.09.1998
(51) Int. Cl.: C07D 413/04, A01N 43/80, C07F 9/6558, C07F 9/6541, C07D 417/04, C07D 417/14, C07D 413/14

(54) **3-(1,2-benzisothiazol- and isoxazol-5-Y1)-2,4(1H,3H)-pyrimidinedione or thione and 3-(1,2-benzisothiazol- and isoxazol-5-y1)-4(3H)-pyrimidinone or thione herbicidal agents**
3-(1,2-Benzisothiazol- und Isoxazol-5-yl)-2,4(1H,3H)-pyrimidindion oder -thion und 3-(1,2-Benzisothiazol-und Isoxazol-5-yl)-4(3H)pyrimidinon oder -thion als Herbizide
Dérivés de 3-(1,2-benzisothiazol- et isoxazol-5-yl)-2,4(1H,3H)-pyrimidinedione ou thione et de 3-(1,2-benzisothiazol- et isoxazol-5-yl)-4(3H)-pyrimidinone ou thione comme agents herbicides

(30) Priority: 17.09.1997 US 931451; 04.08.1998 US 128831
(43) Date of publication of application: 14.04.1999
(73) Proprietor: BASF AKTIENGESELLSCHAFT, 67056 Ludwigshafen (DE)
(72) Inventor: Wepplo, Peter John, Princeton, New Jersey 08540 (US); Manfred, Mark Christopher, Hamilton, New Jersey 08609 (US); Rampulla, Richard Anthony, Whitehouse Station, New Jersey 08889 (US); Cossette, Michael Vernie, Plainsboro, New Jersey 08536 (US); Guaciaro, Michael Anthony, East Windsor, New Jersey 08536 (US); Haley, Gregory Jay, Langhorne, Pennsylvania 19047 (US); Bullock, Billy Gene, Englishtown, New Jersey 07726 (US); Barnes, Keith Douglas, Newtown, Pennsylvania 18940 (US); Meier, Gary Allen, Lambertville, New Jersey 08530 (US); Hunt, David Allen, Newtown, Pennsylvania 18940 (US); Alvarado, Sergio Ivan, Trenton, New Jersey 08638 (US); Carlsen, Marianne, Yardley, Pennsylvania 19067-7410 (US); Heffernan, Gavin David, Bordentown, New Jersey 08505 (US)
(74) Representative: Köster, Reinhold, Dr.

(56) References cited:
- EP-A- 0 408 382
- EP-A- 0 442 655
- EP-A- 0 726 268
- WO-A-97/08170
- WO-A-97/12886

## Description

Weeds cause tremendous global economic losses by reducing crop yields and lowering crop quality. Worldwide, agronomic crops must compete with hundreds of weed species.

In spite of the commercial herbicides available today, damage to crops caused by weeds still occurs. Accordingly, there is ongoing research to create more effective and/or more selective herbicidal agents.

Certain benzisoxazole and benzisothiazole herbicidal agents are described in U.S. Patent Nos. 5,484,763 and 5,523,278. Those patents generically disclose benzisoxazole and benzisothiazole compounds that are substituted with a Q group which may be one of 22 specified groups. However, only one Q group (Q6) is exemplified in those patents. In addition, those patents do not disclose that their compounds are useful for the selective control of weeds in the presence of corn and wheat.

Surprisingly, it has now been found that certain of the compounds generically disclosed in those patents are more effective and/or more selective herbicidal agents than the corresponding compounds wherein Q is Q6.

It is therefore an object of the present invention to provide compounds which are highly effective for the control of undesirable plant species.

It is also an object of the present invention to provide methods for the control of undesirable plant species.

It is a further object of the present invention to provide methods for the selective control of undesirable plant species in the presence of crops.

Those and other objects and features of the present invention will become more apparent from the detailed description thereof set forth below.

### SUMMARY OF THE INVENTION

The present invention describes 3-(1,2-benzisothiazol- and isoxazol-5-yl)-2,4(1H,3H)-pyrimidinedione or thione and 3-(1,2-benzisothiazol- and isoxazol-5-yl)-4(3H)-pyrimidinone or thione compounds which are useful as herbicidal agents.

The compounds of the present invention are represented by structural formulas I and II 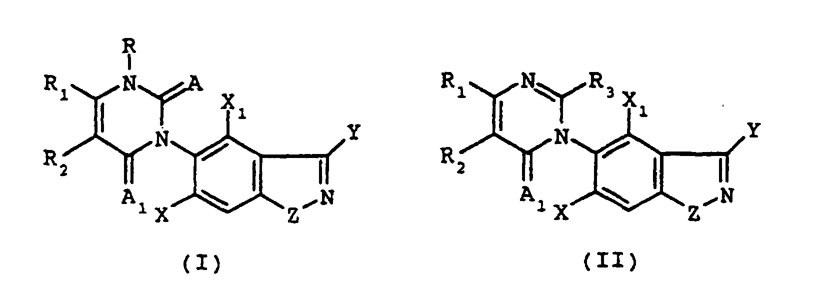 wherein
R is C₁-C₆alkyl, C₁-C₆haloalkyl, C₃-C₇cycloalkyl, C₃-C₆alkenyl, C₃-C₆alkynyl, benzyl, OR₄ or NR₅R₆;
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally interrupted by oxygen, sulfur or nitrogen, and optionally substituted with one to three methyl groups or one or more halogen atoms;
R₃ is halogen or A₂R₁₁;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is O, S(O)ₚ or NR₁₃;
X₃ is -C(=A₃)-, -C(=NOR₁₄)- or -C(=N-NR₁₃R₅₀);
R₄, R₅, R₆, R₇, R₈, R₉, R₁₀, R₁₁ and R₁₄ are each independently hydrogen, C₁-C₆alkyl, C₃-C₇cycloalkyl, C₂-C₈-alkoxyalkyl, C₂-C₆alkenyl, C₂-C₆alkynyl or benzyl;
R₁₂ is hydrogen,
   a C₁-C₈alkyl, C₃-C₇cycloalkyl, C₂-C₈alkenyl, C₅-C₆cycloalkenyl or C₂-C₈alkynyl group, wherein each group is optionally substituted with any combination of one to six halogen atoms, one to three C₁-C₂₀alkoxy groups optionally substituted with one C₁-C₆alkoxy, benzyloxy or C₁-C₆alkylthio group, one or two C₁-C₁₀haloalkoxy groups, one or two NR₁₅R₁₆ groups, one to three S(O)_{q}R₁₇ groups, one or two cyano groups, one or two C₃-C₇cycloalkyl groups, one thiocyano group, one O(SO₂)R₁₇ group, one O(NO₂) group, one or two C(O)R₁₈ groups, one or two CO₂R₁₉ groups, one or two C(O)SR₁₉ groups, one or two C(O)NR₂₀R₂₁ groups, one to three X₄R₂₂ groups, one or two P(O)(OR₂₃)₂ groups, one or two Si(R₂₄)₃ groups,
   one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one or two phenyl groups wherein each phenyl group is independently optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group,
   phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group, or
   indan optionally substituted with any combination of one or two halogen atoms, one or two C₁-C₄alkyl groups, one C₁-C₄alkoxy group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₁-C₈alkoxyalkyl, C₁-C₆alkyl, C₃-C₇cycloalkyl, C₃-C₆alkenyl, C₃-C₆alkynyl, benzyl, OR₂₃, cyano, SO₂R₅₁ or one 5- to 12-membered monocyclic or fused bicyclic heterocyclic ring optionally
   substituted with one or more groups independently selected from halogen, cyano, nitro, amino, hydroxyl, C₁-C₄alkyl, C₁-C₆haloalkyl, C₁-C₄alkoxy or C₁-C₄haloalkoxy, and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃,
   C(O)NR₃₅R₃₆, C(O)R₃₃, SO₂R₄₇,
   C₁-C₂₀alkyl substituted with one hydroxyl, benzyloxy, nitro, OC(O)R₃₃, C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one hydroxyl group, one
   C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one hydroxyl, C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈; C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one hydroxyl, C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three-C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₁₆ and R₂₁ are each independently hydrogen, C₁-C₆alkyl, C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently NR₄₆R₄₈, -N=CR₄₆R₄₈, C₁-C₈alkyl optionally substituted with one C₁-C₆alkoxy, thiophene or furan group, C₁-C₆haloalkyl, C₃-C₈cycloalkyl, CO₂R₂₃, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, menganese, copper, zinc, cobalt, silver, nickel, ammonium or organic ammonium cation;
R₃₂ is hydrogen,
   Si(R₄₇)₃,
   di(C₁-C₄alkyl)amino,
   C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)amino group,
   C₁-C₁₅haloalkyl,
   C₃-C₈cycloalkyl optionally substituted with one CO₂R₄₈ group,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   2-, 3- or 4-pyridyl,
   2- or 3-furyl,
   2- or 3-thienyl,
   2-tetrahydrofuranyl,
   C₁-C₄alkyl substituted with one pyridyl, furyl, thienyl, tetrahydrofuryl, C₃-C₈cycloalkyl, dioxane or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups, and
   when R₄₆ and R₄₈ are taken together with the atom to which they are attached, they form a 5- to 12-membered monocyclic or fused bicyclic, heterocyclic or cycloalkyl ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups; and
A, A₁, A₂, A₃, A₄, X₄ and X₅ are each independently O or S;
   and
the optical isomers, diastereomers and/or tautomers thereof.

This invention also relates to compositions containing those compounds and methods for using those compounds and compositions. Advantageously, it has been found that the compounds of the present invention, and compositions containing them, are useful for the control of undesirable plant species. The compounds of the present invention are especially useful for the selective control of undesirable plant species in the presence of crops.

### DETAILED DESCRIPTION OF THE INVENTION

The present invention provides a method for the control of undesirable plant species which comprises applying to the foliage of said plants or to the soil or water containing seeds or other propagating organs thereof, a herbicidally effective amount of a 3-(1,2-benzisothiazol- or isoxazol-5-yl)-2,4(1H,3H)-pyrimidinedione or thione compound of formula I or a 3-(1,2-benzisothiazol- or isoxazol-5-yl)-4(3H)-pyrimidinone or thione compound of formula II.

The herbicidal compounds of the present invention are represented by structural formulas I and II 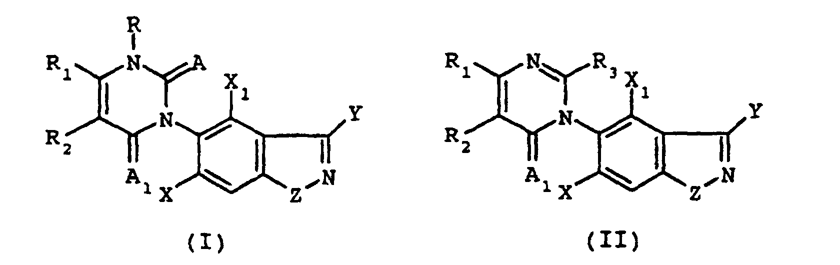 wherein R, R₁, R₂, R₃, A, A₁, X, X₁, Y and Z are as defined above for formulas I and II.

Surprisingly, it has been discovered that the formula I and II compounds of this invention are more effective and/or more selective herbicidal agents than the corresponding compounds exemplified in U.S. Patent Nos. 5,484,763 and 5,523,278 wherein Q is Q6. In addition, we have found that the formula I compounds of the present invention are particularly useful for the selective control of undesirable plant species in the presence of crops such as corn, wheat, rice and soybeans.

Preferred compounds of the present invention are those represented by structural formulas I and II 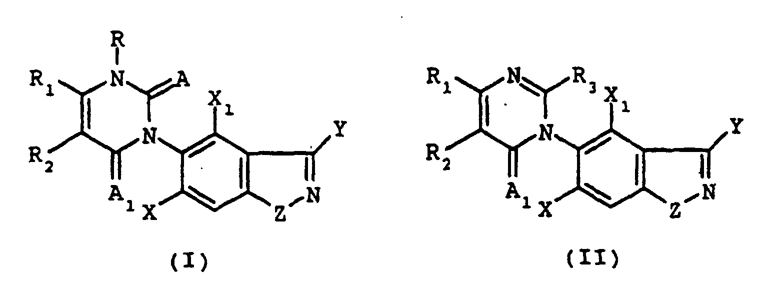 wherein
R is C₁-C₆alkyl, C₁-C₆haloalkyl, C₃-C₇cycloalkyl, C₃-C₆alkenyl, C₃-C₆alkynyl, benzyl, OR₄ or NR₅R₆;
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally interrupted by oxygen, sulfur or nitrogen, and optionally substituted with one to three methyl groups or one or more halogen atoms;
R₃ is halogen or A₂R₁₁;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is O, S(O)ₚ or NR₁₃;
X₃ is -C(=A₃)- or -C(=NOR₁₄)-;
R₄, R₅, R₆, R₇, R₈, R₉, R₁₀, R₁₁ and R₁₄ are each independently hydrogen, C₁-C₆alkyl, C₃-C₇cycloalkyl, C₂-C₆alkenyl, C₂-C₆alkynyl or benzyl;
R₁₂ is hydrogen,
   a C₁-C₆alkyl, C₃-C₇cycloalkyl, C₂-C₆alkenyl, C₅-C₆cycloalkenyl or C₂-C₆alkynyl group, wherein each group is optionally substituted with any combination of one to six halogen atoms, one to three C₁-C₂₀alkoxy groups, one or two C₁-C₁₀haloalkoxy groups, one or two NR₁₅R₁₆ groups, one to three S(O)_{q}R₁₇ groups, one or two cyano groups, one or two C(O)R₁₈ groups, one or two CO₂R₁₉ groups, one or two C(O)NR₂₀R₂₁ groups, one to three X₄R₂₂ groups, one or two P(O)(OR₂₃)₂ groups, one or two Si(R₂₄)₃ groups,
   one 4- to 10-membered monocyclic or fused bicyclic heterocyclic ring optionally subsituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one phenyl group optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group, or
   phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group,
   and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₃ is hydrogen, C₁-C₆alkyl, C₃-C₇cycloalkyl, C₃-C₆alkenyl, C₃-C₆alkynyl, benzyl, OR₂₃, cyano or SO₂R₅₁, and when R₁₃ and R₁₂ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃,
   C₁-C₂₀alkyl substituted with one C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NHOR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one C₁-C₆alkoxy, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one C₁-C₆alkoxy, C₁-C₄alkyl, C,-C,alkenyl, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one C₁-C₆alkoxy, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   C(O)R₄₃;
R₁₆ and R₂₁ are each independently hydrogen, C₁-C₆alkyl, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently C₁-C₄alkyl, C₁-C₄haloalkyl, CO₂R₂₃, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
   Si(R₄₇)₃,
   di(C₁-C₄alkyl)amino,
   C₁-C₂₀alkyl optionally substituted with one CO₂R₄₈, C₁-C₆alkoxy or di(C₁-C₄alkyl)amino group,
   C₁-C₁₅haloalkyl,
   C₃-C₈cycloalkyl,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   2-, 3- or 4-pyridyl,
   2- or 3-furyl,
   2- or 3-thienyl,
   2-tetrahydrofuranyl,
   C₁-C₄alkyl substituted with one pyridyl, furyl, thienyl, tetrahydrofuryl or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups; and
A, A₁, A₂, A₃, A₄, X₄ and X₅ are each independently O or S;
   and
the optical isomers, diastereomers and/or tautomers thereof.

Another group of preferred compounds of the present invention are those having the structural formula I 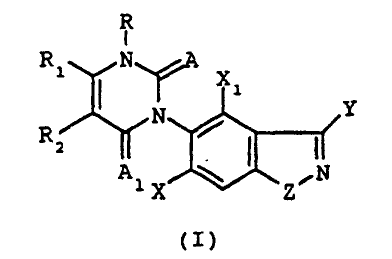 wherein
R is C₁-C₆alkyl, C₁-C₆haloalkyl, C₃-C₇cycloalkyl, C₃-C₆alkenyl, C₃-C₆alkynyl, benzyl, OR₄ or NR₅R₆;
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally interrupted by oxygen, sulfur or nitrogen, and optionally substituted with one to three methyl groups or one or more halogen atoms;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is O, S(O)ₚ or NR₁₃;
X₃ is -C(=A₃)-, -C(=NOR₁₄)- or -C(=N-NR₁₃R₅₀);
R₄, R₅, R₆, R₇, R₈, R₉, R₁₀ and R₁₄ are each independently hydrogen, C₁-C₆alkyl, C₃-C₇cycloalkyl, C₂-C₈-alkoxyalkyl, C₂-C₆alkenyl, C₂-C₆alkynyl or benzyl;
R₁₂ is hydrogen,
   a C₁-C₈alkyl, C₃-C₇cycloalkyl, C₂-C₈alkenyl, C₅-C₆cycloalkenyl or C₂-C₈alkynyl group, wherein each group is optionally substituted with any combination of one to six halogen atoms, one to three C₁-C₂₀alkoxy groups optionally substituted with one C₁-C₆alkoxy, benzyloxy or C₁-C₆alkylthio group, one or two C₁-C₁₀haloalkoxy groups, one or two NR₁₅R₁₆ groups, one to three S(O)_{q}R₁₇ groups, one or two cyano groups, one or two C₃-C₇cycloalkyl groups, one thiocyano group, one O(SO₂)R₁₇ group, one O(NO₂) group, one or two C(O)R₁₈ groups, one or two CO₂R₁₉ groups, one or two C(O)SR₁₉ groups, one or two C(O)NR₂₀R₂₁ groups, one to three X₄R₂₂ groups, one or two P(O)(OR₂₃)₂ groups, one or two Si(R₂₄)₃ groups,
   one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one or two phenyl groups wherein each phenyl group is independently optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group,
   phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group, or
   indan optionally substituted with any combination of one or two halogen atoms, one or two C₁-C₄alkyl groups, one C₁-C₄alkoxy group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group,
   and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₃ is hydrogen, C₁-C₈alkoxyalkyl, C₁-C₆alkyl, C₃-C₇cycloalkyl, C₃-C₆alkenyl, C₃-C₆alkynyl, benzyl, OR₂₃, cyano, SO₂R₅₁ or one 5- to 12-membered monocyclic or fused bicyclic heterocyclic ring optionally substituted with one or more groups independently selected from halogen, cyano, nitro, amino, hydroxyl, C₁-C₄alkyl, C₁-C₄haloalkyl, C₁-C₄alkoxy or C₁-C₄haloalkoxy, and when R₁₃ and R₁₂ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃, C(O)NR₃₅R₃₆, C(O)R₃₃, SO₂R₄₇,
   C₁-C₂₀alkyl substituted with one hydroxyl, benzyloxy, nitro, OC(O)R₃₃, C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one hydroxyl group, one C₁-C₆alkoxy, CO₂R₃₂, C(O)R₃₃, C(A₆R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one hydroxyl, C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one hydroxyl, C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₁₆ and R₂₁ are each independently hydrogen, C₁-C₆alkyl, C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently NR₄₆R₄₈, -N=CR₄₆R₄₈, C₁-C₈alkyl optionally substituted with one C₁-C₆alkoxy, thiophene or furan group, C₁-C₆haloalkyl, C₃-C₈cycloalkyl, CO₂R₂₃, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
   Si(R₄₇)₃,
   di(C₁-C₄alkyl)amino,
   C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)amino group,
   C₁-C₁₅haloalkyl,
   C₃-C₈cycloalkyl optionally substituted with one CO₂R₄₈ group,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   2-, 3- or 4-pyridyl,
   2- or 3-furyl,
   2- or 3-thienyl,
   2-tetrahydrofuranyl,
   C₁-C₄alkyl substituted with one pyridyl, furyl, thienyl, tetrahydrofuryl, C₃-C₈cycloalkyl, dioxane or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups, and
   when R₄₆ and R₄₈ are taken together with the atom to which they are attached, they form a 5- to 12-membered monocyclic or fused bicyclic, heterocyclic or cycloalkyl ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups; and
A, A₁, A₃, A₄, X₄ and X₅ are each independently O or S; and the optical isomers, diastereomers and/or tautomers thereof.

**More preferred formula** I herbicidal agents of the present invention are those wherein
R is C₁-C₄alkyl, C₁-C₄haloalkyl, C₃-C₄alkenyl, C₃-C₄alkynyl or benzyl;
R₁ is halogen or C,-C,haloalkyl;
R₂ is hydrogen, halogen or C₁-C₄haloalkyl;
X and X₁ are each independently hydrogen or halogen;
Z is O or S;
Y is cyano, R₁₂, X₂R₁₂ or X₃X₂R₁₂;
X₂ is O, S or NR₁₃;
X₃ is -C(=A,)-, -C(=NOR₁₄)- or -C(=N-NR₁₃R₅₀) ;
R₁₂ is hydrogen,
   a C₁-C₆alkyl, C₃-C₇cycloalkyl, C₂-C₆alkenyl, C₅-C₆cycloalkenyl or C₂-C₆alkynyl group, wherein each group is optionally substituted with any combination of one to three halogen atoms, one or two C₁-C₂₀alkoxy groups, one or two C₁-C₁₀haloalkoxy groups, one NR₁₅R₁₆ group, one S(O)_{q}R₁₇ group, one or two cyano groups, one or two C(O)R₁₈ groups, one or two CO₂R₁₉ groups, one or two C(O)SR₁₉ groups, one or two C(O)NR₂₀R₂₁ groups, one or two X₄R₂₂ groups, one P(O)(OR₂₃)₂ group, one Si(R₂₄)₃ group,
   one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one phenyl group optionally substituted with any combination of one to three halogen atoms, one C₁-C₆alkyl group, one C₁-C₆alkoxy group, one C₃-C₇cycloalkyl group, one cyano group, one nitro group, one C(O)R₂₅ group, one CO₂R₂₆ group or one X₅R₂₇ group, or
   phenyl optionally substituted with any combination of one or two halogen atoms, one to three C₁-C₄alkyl groups, one or two C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₂-C₆alkoxyalkyl, C₁-C₄alkyl, C₃-C₇cycloalkyl, C₃-C₆alkenyl, C₃-C₆alkynyl, benzyl, OR₂₃, cyano or SO₂R₅₁;
R₂₈ is halogen;
R₂₂ and R₂₇ are each independently hydrogen, Si(R₃₁)₃, C(O)NR₃₅R₃₆, C(O)R₃₃, SO₂R₄₇,
   C₁-C₂₀alkyl substituted with one hydroxyl, benzyloxy, nitro, OC(O)R₃₃, C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one hydroxyl group, one C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one hydroxyl, C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one hydroxyl, C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₁₄ is hydrogen, C₁-C₆alkyl, C₃-C₇cycloalkyl, C₂-C₈alkoxyalkyl, C₂-C₆alkenyl, C₂-C₆alkynyl or benzyl;
R₁₆ and R₂₁ are each independently hydrogen, C₁-C₆alkyl, C₁-C₄alkoxy, C,-C,cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₁₇, R₁₈, R₂₅, R₃₀, R₃₃, R₄₅ and R₅₁ are each independently NR₄₆R₄₈, -N=CR₄₆R₄₈, C₁-C₈alkyl optionally substituted with one C₁-C₆alkoxy, thiophene or furan group, C₁-C₆haloalkyl, C₃-C₈cycloalkyl, CO₂R₂₃, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
   Si(R₄₇)₃,
   C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)amino group,
   C₁-C₁₅haloalkyl,
   C₃-C₈cycloalkyl optionally substituted with one CO₂R₄₈ group,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   C₁-C₄alkyl substituted with one pyridyl, furyl, thienyl, tetrahydrofuryl, C₃-C₈cycloalkyl, dioxane or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently
   selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
q is an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups; and
A, A₁, A₃, A₄, X₄ and X₅ are each independently O or S; and
the optical isomers, diastereomers and/or tautomers thereof.

Another group of more preferred herbicidal agents of formula I are those wherein
R is C₁-C₄alkyl;
R₁ is C₁-C₄haloalkyl;
R₂ is hydrogen,
X and X₁ are each independently hydrogen or halogen;
Z is O or S;
Y is R₁₂, OR₁₂, C(O)NR₁₃R₁₂, C(O)R₁₂, CO₂R₁₂, C(O)SR₁₂ or C(=NOR₁₄);
R₁₂ is hydrogen,
   a C₁-C₆alkyl or C₂-C₆alkenyl group, wherein each group is optionally substituted with any combination of one to three halogen atoms, one or two C₁-C₄-alkoxy groups, one C₁-C₁₀haloalkoxy group, one NR₁₅R₁₆ group, one S(O)_{q}R₁₇ group, one or two cyano groups, one or two C(O)R₁₈ groups, one or two CO₂R₁₉ groups, one or two C(O)NR₂₀R₂₁ groups, one X₄R₂₂ group, one P(O)(OR₂₃)₂ group, one OC(O)R₃₃ group,
   one imidazole ring optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   one phthalimide ring optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   one quaternary organic ammonium group, or
   one phenyl group optionally substituted with any combination of one to three halogen atoms, one C₁-C₄alkyl group, one C₁-C₄alkoxy group or one X₅R₂₇ group, or
   phenyl optionally substituted with any combination of one or two halogen atoms, one to three C₁-C₄alkyl groups, one C₁-C₄alkoxy group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₁-C₆alkoxyalkyl, C₁-C₄alkyl, OR₂₃, cyano or SO₂R₅₁;
R₂₈ is halogen;
R₂₂ and R₂₇ are each independently hydrogen, C(O)R₃₃, SO₂R₄₇,
   C₁-C₄alkyl substituted with one C₁-C₆alkoxy, CO₂R₃₂, C(O)R₃₃, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₃-C₆alkenyl optionally substituted with one C₁-C₆alkoxy, CO₂R₃₂, C(O)R₃₃ or C(OR₃₄)₂ group,
   C₃-C₆alkynyl,
   phenyl optionally substituted with any combination of one to three halogen atoms, one or two C₁-C₄alkyl groups, one or two C₁-C₄alkoxy groups, one C₁-C₄haloalkyl group, one C₁-C₄haloalkoxy group, one cyano group, one nitro group, one NR₃₉R₄₀ group or one C(O)R₃₃ group, or
   benzyl optionally substituted on the phenyl ring with any combination of one to three halogen atoms, one or two C₁-C₄alkyl groups, one or two C₁-C₄alkoxy groups, one C₁-C₄haloalkyl group, one C₁-C₄haloalkoxy group, one cyano group, one nitro group, one NR₃₉R₄₀ group or one C(O)R₃₃ group;
R₂₀ is hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₁₄ is hydrogen, C₁-C₆alkyl, C₃-C₇cycloalkyl, C₂-C₈alkoxyalkyl, C₂-C₆alkenyl, C₂-C₆alkynyl or benzyl;
R₁₅ is hydrogen,
   C₁-C₄alkyl optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   C(O)R₄₃;
R₁₆ is hydrogen, C₁-C₄alkyl, P(O)(OR₄₄)₂, SO₂R₄₅, C(O)R₄₆, or
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₂₁ is hydrogen, C₁-C₆alkyl, C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₁₇, R₁₈, R₂₅, R₃₀, R₃₃, R₄₅ and R₅₁ are each independently NR₄₆R₄₈, C₁-C₄alkyl, C₁-C₄haloalkyl,
   C₂-C₆alkenyl optionally substituted with phenyl, benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group;
R₁₉ and R₂₆ are each independently hydrogen, C₁-C₄alkyl, C₃-C₆alkenyl, C₃-C₆alkynyl, Si(R₄₁)₃, benzyl, phenyl, furfuryl, pyridyl, thienyl, oximino or an alkali metal, alkaline earth metal; manganese, copper, zinc, cobalt, silver, nickel, ammonium or organic ammonium cation;
R₃₂ is hydrogen,
   Si(R₄₇)₃,
   C₁-C₄alkyl optionally substituted with one CO₂R₄₈, OC(O)R₃₃ or C₁-C₄alkoxy group,
   C₁-C₄haloalkyl,
   C₃-C₈cycloalkyl,
   C₃-C₈halocycloalkyl,
   C₃-C₆alkenyl optionally substituted with one phenyl group,
   C₃-C₆haloalkenyl,
   C₅-C₈cycloalkenyl,
   C₅-C₈halocycloalkenyl,
   C₃-C₆alkynyl optionally substituted with one phenyl group,
   C₃-C₆haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   C₁-C₄alkyl substituted with one pyridyl, furyl, thienyl, tetrahydrofuryl or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₄alkyl and R₃₆ and R₅₀ are each independently hydrogen, cyano, C₁-C₄alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
q is an integer of 0, 1 or 2;
R₂₉ is hydrogen, C₁-C₄alkyl or C₁-C₄haloalkyl;
R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₃₉, R₄₀, R₄₂, R₄₃, R₄₄, R₄₆ and R₄₈ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
A₁, X₄ and X₅ are each independently O or S;
R₂₃ is hydrogen or C₁-C₄ alkyl; and
A is O.

A third group of more preferred herbicidal agents of formula I are those wherein
R is methyl;
R₁ is trifluoromethyl;
R₂ and X₁ are hydrogen;
X is hydrogen or fluorine;
Z is O or S;
Y is R₁₂, CO₂R₁₂, C(O)R₁₂ or C(O)NR₁₃R₁₂;
R₁₂ is hydrogen,
   C₁-C₆alkyl optionally substituted with any combination of one to three halogen atoms, one hydroxyl group, one or two C₁-C₄alkoxy groups, one C₁-C₄haloalkoxy group, one SO₂R₁₇ group, one or two cyano groups, one C(O)R₁₈ group, one or two CO₂R₁₉ groups, one or two C(O)NR₂₀R₂₁ groups, one P(O)(OR₂₃)₂ group or one OC(O)R₃₃ group,
   C₂-C₆alkenyl optionally substituted with any combination of one C(O)R₁₈ group or one CO₂R₁₉ group, 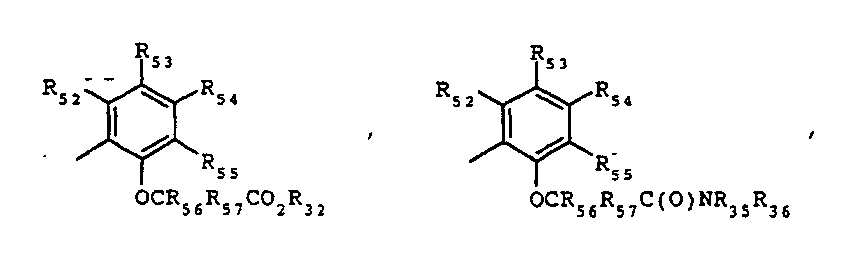 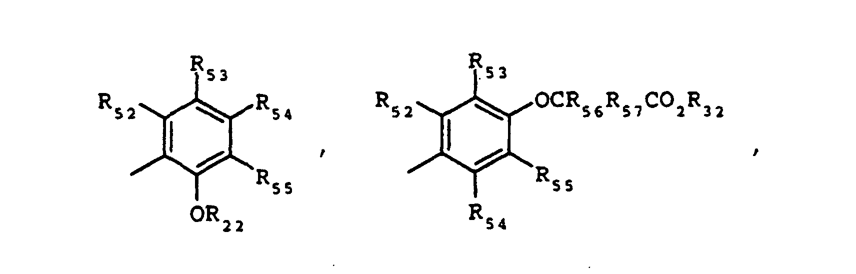 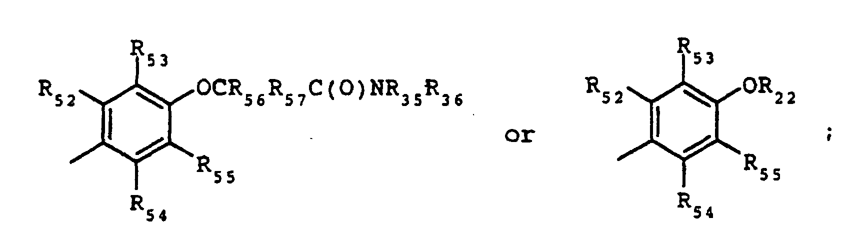
R₁₃ is hydrogen, C₁-C₄ alkyl or SO₂R₅₁;
R₁₄ is hydrogen, C₁-C₄alkyl or benzyl;
R₁₈, R₂₃, R₅₆ and R₅₇ are each independently hydrogen or C₁-C₃alkyl;
R₅₂, R₅₃, R₅₄ and R₅₅ are each independently hydrogen, C₁-C₃alkyl, C₂-C₃alkenyl, C₁-C₃alkoxy or halogen, provided that at least one of R₅₂, R₅₃, R₅₄ and R₅₅ is hydrogen;
R₁₉ is hydrogen, C₁-C₄alkyl, C₃-C₆alkenyl, C₃-C₆alkynyl, benzyl, pyridyl or an alkali metal, alkaline earth metal, ammonium or organic ammonium cation;
R₂₀ is hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₂₁ is hydrogen, C₁-C₆alkyl, C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups-or one to three C₁-C₄haloalkoxy groups, or
   phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₂₂ is hydrogen, C(O)R₃₃, SO₂R₄₇,
   C₁-C₄alkyl optionally substituted with one cyano group,
   C₃-C₆alkenyl, or
   C₃-C₆alkynyl;
R₃₂ is hydrogen,
   C₁-C₄alkyl optionally substituted with one CO₂R₄₈ or C₁-C₄alkoxy group,
   C₁-C₄haloalkyl,
   C₃-C₆alkenyl optionally substituted with one phenyl group,
   C₃-C₆alkynyl optionally substituted with one phenyl group,
   C₁-C₄alkyl substituted with one pyridyl, furyl, thienyl, tetrahydrofuryl or NR₄₉R₅₀ group, or
   an alkali metal, alkaline earth metal, ammonium or organic ammonium cation;
R₃₅ and R₄₉ are each independently hydrogen or C₁-C₄alkyl;
R₃₆ is SO₂R₅₁ or C₁-C₃alkoxy;
R₁₇, R₃₃ and R₅₁ are each independently C₁-C₄alkyl, C₁-C₄haloalkyl, NR₄₆R₄₈, C₃-C₆cycloalkyl or an isoxazole ring;
R₄₅ is NR₄₆R₄₈, C₁-C₄alkyl, C₁-C₄haloalkyl, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group;
R₂₆ is hydrogen, C₁-C₄alkyl, C₃-C₆alkenyl, C₃-C₆alkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₄₁ is C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₄₂, R₄₃ and R₄₄ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
X₄ is O or S;
R₄₆ and R₄₈ are each independently hydrogen, C₁-C₄alkyl or benzyl;
R₄₇ is C₁-C₄alkyl;
R₅₀ is C₁-C₄alkyl or
   phenyl optionally substituted with one or more groups independently selected from halogen, cyano, C₁-C₄alkyl, C₁-C₄haloalkyl, C₁-C₄alkoxy and C₁-C₄haloalkoxy groups; and
A and A₁ are O.

Most preferred formula I compounds of this invention are those wherein
R is methyl;
R₁ is trifluoromethyl;
R₂ and X₁⁻ are hydrogen;
X is hydrogen or fluorine;
Z is O or S;
Y is C₁-C₃alkyl, CO₂R₃₂, halomethyl, cyanomethyl, C(CH₃)₂CO₂R₃₂, C₁-C₃alkoxyethyl, C₁-C₃alkoxymethyl, hydroxymethyl, CHO, C(O)CH₃, CH(CH₃)(C₁-C₄alkoxy), C(CH₃)₂CN, CH[O(C₁-C₃alkyl)]₂, CH₂SO₂R₁₇, C(O)NHSO₂R₅₁, 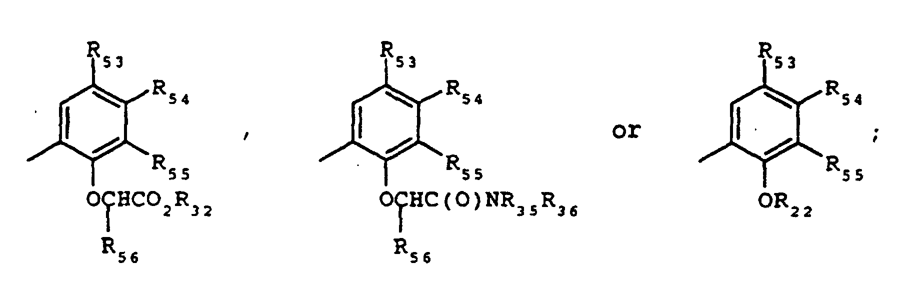
R₅₃, R₅₄, R₅₅ and R₅₆ are each independently hydrogen or methyl, provided that at least one of R₅₃, R₅₄ and R₅₅ is hydrogen;
R₂₂ is cyanomethyl, methyl, ethyl, allyl or propargyl;
R₃₂ is hydrogen,
   C₁-C₄alkyl optionally substituted with one CO₂R₄₈ group,
   C₁-C₄haloalkyl,
   C₃-C₆alkenyl, or
   C₃-C₆alkynyl;
R₃₅ is hydrogen;
R₃₆ is SO₂R₅₁ or C₁-C₃alkoxy;
R₁₇ and R₅₁ are each independently C₁-C₄alkyl, C₁-C₄haloalkyl, NR₄₆R₄₈, C₃-C₆cycloalkyl or an isoxazole ring;
R₄₆ and R₄₈ are each independently hydrogen, C₁-C₄alkyl or benzyl; and
A and A₁ are O.

3-(1,2-Benzisothiazol- and isoxazol-5-yl)-2,4(1H,3H)-pyrimidinedione or thiodione compounds of the present invention which are particularly-effective herbicidal agents include the group consisting of
isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}acetate;
2-(dimethylamino)ethyl {{2-(5-[3,6-dihydro-3-methyl-2,6-dioxo-4-(trifluoromethyl)-1(2H)-pyrimidinyl)-1,2-benzisothiazol-3-yl}-*p*-tolyl}oxy}acetate;
methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate;
methyl 2-{{6-{5-(3,6-dihydro-3-methyl-4-(trifluoromethyl)-2,6-dioxo-1(2H)-pyrimidinyl]-1,2-benzisothiazol-3-yl}-3,4-xylyl}oxy}propionate;
2-propynyl {{2-{5-[3,6-dihydro-3-methyl-2,6-dioxo-4-(trifluoromethyl)-1(2H)-pyrimidinyl]-1,2-benzisothiazol-3-yl}-p-tolyl}oxy}acetate;
3-[3-(6-methoxy*-m*-tolyl)-1,2-benzisothiazol-5-yl]-1-methyl-6-(trifluoromethyl)-2,4(1H,3H)-pyrimidinedione;
3-[3-(*p*-ethylphenyl)-1,2-benzisothiazol-5-yl]-1-methyl-6-(trifluoromethyl)-2,4(1H,3H)-pyrimidinedione;
1-methyl-3-(3-methyl-1,2-benzisothiazol-5-yl)-6-(trifluoromethyl)-2,4(1H,3H)-pyrimidinedione;
methyl 2-{{6-{5-[3,6-dihydro-3-methyl-4-(trifluoromethyl)-2,6-dioxo-1(2H)-pyrimidinyl]-1,2-benzisothiazol-3-yl}-*m*-tolyl}oxy}propionate;
methyl {{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}acetate;
3-[3-(dibromomethyl)-1,2-benzisothiazol-5-yl]-1-methyl-6-(trifluoromethyl)-2,4(1H,3H)-pyrimidinedione;
3-(6-fluoro-3-methyl-1,2-benzisoxazol-5-yl)-1-methyl-6-(trifluoromethyl)-2,4(1H,3H)-pyrimidinedione;
ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
1-methyl-3-{3-[6-(2-propynyloxy)-*m*-tolyl]-1,2-benzisothiazol-5-yl]-6-(trifluoromethyl)-2,4(1H,3H)-pyrimidinedione;
3-{3-{6-[(1-benzimidazolylcarbonyl)methoxy]-*m*-tolyl}-1,2-benzisothiazol-5-yl]-1-methyl-6-trifluoromethyl)-2,4(1H,3H)-pyrimidinedione;
3-{3-{6-{[(*m*-cyanophenyl)carbamoyl]methoxy}-*m*-tolyl}-1,2-benzisothiazol-5-yl}-1-methyl-6-(trifluoromethyl)-2,4(1H,3H)pyrimidinedione;
allyl {{2-{5-[3,6-dihydro-3-methyl-2,6-dioxo-4-(trifluoromethyl)-1(2H)-pyrimidinyl)-1,2-benzisothiazol-3-yl}-*p*-tolyl}oxy}acetate;
3-phenyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate; 1-methyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-chloroethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate;
2,2,2-trifluoroethyl {{2-{5-[3,6-dihydro-3-methyl-2,6-dioxo-4-(trifluoromethyl)-1(2H)-pyrimidinyl] -1,2-benzisothiazol-3-yl}-*p*-tolyl}oxy}acetate;
3-[3-(2-methoxy-*p*-tolyl)-1,2-benzisothiazol-5-yl]-1-methyl-6-(trifluoromethyl)-2,4(1H,3H)-pyrimidinedione;
2-[3-(6-methoxy-3,4-xylyl)-1,2-benzisothiazol-5-yl]-1-methyl-6-(trifluoromethyl)-2,4(1H,3H)-pyrimidinedione;
methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate;
methyl {{6-{5-[3,6-dihydro-3-methyl-2,6-dioxo-4-(trifluoromethyl)-1(2H)-pyrimidinyl]-1,2-benzisothiazol-3-yl}-2,4-xylyl}oxy)acetate;
3-{3-{6-{[(*m*-cyanophenyl)carbamoyl]methoxy}-*m*-tolyl}-1,2-benzisothiazol-5-yl}-1-methyl-6-(trifluoromethyl)-2,4(1H,3H)-pyrimidinedione;
methyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
1-methyl-3-(3-[(methylsulfonyl)methyl]-1,2-benzisothiazol-5-yl}-6-(trifluoromethyl)-2,4(1H,3H)-pyrimidinedione;
2-propynyl {{2-{5-(3,6-dihydro-3-methyl-2,6-dioxo-4-(trifluoromethyl)-1(2H)-pyrimidinyl]-1,2-benzisoxazol-3-yl}-*p*-tolyl}oxy}acetate;
3-[3-(*o*-methoxyphenyl)-1,2-benzisothiazol-5-yl]-1-methyl-6-(trifluoromethyl)-2,4(1H,3H)-pyrimidinedione;
N-{{{2-(5-[3,6-dihydro-3-methyl-2,6-dioxo-4-(trifluoromethyl)-1(2H)-pyrimidinyl]-1,2-benzisothiazol-3-yl}*-p*-tolyl}oxy}acetyl}methanesulfonamide;
2-propynyl 2-{{2-[5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate;
2-propynyl {*o*-{5-[3,6-dihydro-3-methyl-2,6-dioxo-4-(trifluoromethyl)-1(2H)-pyrimidinyl]-1,2-benzisothiazol-3-yl]phenoxy]acetate;
5-[3,6-dihydro-3-methyl-2,6-dioxo-4-(trifluoromethyl)-1(2H)-pyrimidinyl]-1,2-benzisothiazole-3-acetonitrile;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
methyl {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetate;
methyl {{6-{5-[3,6-dihydro-3-methyl-2,6-dioxo-4-(trifluoromethyl)-1(2H)-pyrimidinyl]-6-fluoro-1,2-benzisothiazol-3-yl}-*m*-tolyl}oxy}acetate;
{*o*-{5-[3,6-dihydro-3-methyl-2,6-dioxo-4-(trifluoromethyl)-1(2H)-pyrimidinyl]-1,2-benzisothiazol-3-yl}phenoxy}acetic acid;
2-{*o*-[5-[3,6-dihydro-3-methyl-2,6-dioxo-4-(trifluoromethyl)-1(2H)-pyrimidinyl]-1,2-benzisothiazol-3-yl}phenoxy}propionic acid;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methoxyacetamide;
methyl 2-{2-{5-[3,6-dihydro-3-methyl-2,6-dioxo-4-(trifluoromethyl)-1(2H)-pyrimidinyl]-1,2-benzisothiazol-3-yl}-4-methoxyphenoxy}propionate;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(methylsulfonyl)acetamide;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(methylsulfonyl)propionamide;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl-N-(methylsulfonyl)acetamide;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-methyl-N-(methylsulfonyl)acetamide;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(ethylsulfonyl)acetamide;
3-[3-(hydroxymethyl)-1,2-benzisothiazol-5-yl]-1-methyl-6-(trifluoromethyl)-2,4(1H,3H)-pyrimidinedione, 2-thiophenecarboxylate (ester);
3-[3-(hydroxymethyl)-1,2-benzisothiazol-5-yl]-1-methyl-6-(trifluoromethyl)-2,4(1H,3H)-pyrimidinedione, benzoate (ester);
3-[3-(hydroxymethyl)-1,2-benzisothiazol-5-yl]-1-methyl-6-(trifluoromethyl)-2,4(1H,3H)-pyrimidinedione, methoxyacetate (ester);
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-α,α-dimethyl-1,2-benzisothiazole-3-acetonitrile;
5-[3,6-dihydro-3-methyl-2,6-dioxo-4-(trifluoromethyl)-1(2H)-pyrimidinyl]-6-fluoro-1,2-benzisothiazole-3-acetonitrile;
5-[3,6-dihydro-3-methyl-2,6-dioxo-4-(trifluoromethyl)-1(2H)-pyrimidinyl]-α-ethyl-1,2-benzisothiazole-3-acetonitrile;
5-[3,6-dihydro-3-methyl-2,6-dioxo-4-(trifluoromethyl)-1(2H)-pyrimidinyl]-α,α-diethyl-1,2-benzisothiazole-3-acetonitrile;
3-[6-fluoro-3-(methoxymethyl)-1,2-benzisothiazol-5-yl]-1-methyl-6-(trifluoromethyl)-2,4(1H,3H)-pyrimidinedione;
3-[3-(hydroxymethyl)-1,2-benzisothiazol-5-yl]-1-methyl-6-(trifluoromethyl)-2,4(1H,3H)-pyrimidinedione, 5-isoxazolecarboxylate (ester);
3-[3-(hydroxymethyl)-1,2-benzisothiazol-5-yl]-1-methyl-6-(trifluoromethyl)-2,4(1H,3H)-pyrimidinedione, cyclopropanecarboxylate (ester);
3-(3-acetyl-1,2-benzisothiazol-5-yl)-1-methyl-6-(trifluoromethyl)-2,4(1H,3H)-pyrimidinedione;
3-[3-(1-methoxyethyl)-1,2-benzisothiazol-5-yl]-1-methyl-6-(trifluoromethyl)-2,4(1H,3H)-pyrimidinedione;
N-(benzylsulfonyl)-5-[3,6-dihydro-3-methyl-2,6-dioxo-4-(trifluoromethyl)-1(2H)-pyrimidinyl]-1,2-benzisothiazole-3-carboxamide;
methyl 5-[3,6-dihydro-3-methyl-2,6-dioxo-4-(trifluoromethyl)-1(2H)-pyrimidinyl]-1,2-benzisothiazole-3-carboxylate;
benzyl 5-[3,6-dihydro-3-methyl-2,6-dioxo-4-(trifluoromethyl)-1(2H)-pyrimidinyl]-α,α-dimethyl-1,2-benzisothiazole-3-acetate;
N-(benzylsulfonyl)-5-[3,6-dihydro-3-methyl-2,6-dioxo-4-(trifluoromethyl)-1(2H)-pyrimidinyl]-6-fluoro-1,2-benzisothiazole-3-carboxamide;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-N-[(*p*-methylbenzyl)sulfonyl]-1,2-benzisothiazole-3-carboxamide;
N-(benzylsulfonyl)-5-[3,6-dihydro-3-methyl-2,6-dioxo-4-(trifluoromethyl)-1(2H)-pyrimidinyl]-6-fluoro-1,2-benzisothiazole-3-carboxamide;
3-[3-(1-hydroxyethyl)-1,2-benzisothiazol-5-yl]-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-1,2-benzisothiazole-3-carboxaldehyde, 3-(dimethyl acetal);
3-(6-fluoro-3-methyl-1,2-benzisothiazol-5-yl)-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-6-fluoro-1,2-benzisothiazole-3-carboxaldehyde, 3-(dimethyl acetal);
5-[3,6-dihydro-3-methyl-2,6-dioxo-4-(trifluoromethyl)-1(2H)-pyrimidinyl]-6-fluoro-1,2-benzisothiazole-3-carboxylic acid;
5-[3,6-dihydro-3-methyl-2,6-dioxo-4-(trifluoromethyl)-1(2H)-pyrimidinyl]-α-methyl-1,2-benzisothiazole-3-acetonitrile;
N-(benzylsulfonyl)-5-[3,6-dihydro-3-methyl-2,6-dioxo-4-(trifluoromethyl)-1(2H)-pyrimidinyl]-N-(ethoxymethyl)-1,2-benzisothiazole-3-carboxamide;
5-[3,6-dihydro-3-methyl-2,6-dioxo-4-(trifluoromethyl)-1(2H)-pyrimidinyl)-N-(dimethylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide;
methyl 5-[3,6-dihydro-3-methyl-2,6-dioxo-4-(trifluoromethyl)-1(2H)-pyrimidinyl]-α,α-dimethyl-1,2-benzisothiazole-3-acetate;
3-[3-(hydroxymethyl)-1,2-benzisothiazol-5-yl]-1-methyl-6-(trifluoromethyl)-2,4(1H,3H)-pyrimidinedione, 2-furoate (ester);
N-[(*m*-chlorobenzyl)sulfonyl]-5-[3,6-dihydro-3-methyl-2,6-dioxo-4-(trifluoromethyl)-1(2H)-pyrimidinyl]-1,2-benzisothiazole-3-carboxamide;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide; and
3-[3-(hydroxymethyl)-1,2-benzisothiazol-5-yl]-1-methyl-6-(trifluoromethyl)-2,4(1H,3H)-pyrimidinedione, methylcarbamate (ester), among others.

Exemplary of halogen hereinabove are fluorine, chlorine, bromine and iodine. The terms haloalkyl, halocycloalkyl, haloalkoxy, haloalkenyl, halocycloalkenyl and haloalkynyl as used in the specification and claims designate an alkyl group, a cycloalkyl group, an alkoxy group, an alkenyl group, a cycloalkenyl group and an alkynyl group substituted with one or more halogen atoms, respectively. In formulas I and II above, alkali metals include: sodium, potassium and lithium. Alkaline earth metals of formulas I and II include magnesium and calcium. Further, the term organic ammonium is defined as a group consisting of a positively charged nitrogen atom joined to from one to four aliphatic groups, each containing from one to sixteen carbon atoms.

In formulas I and II above, 5- to 12-membered monocyclic or fused bicyclic, heterocyclic rings include, but are not limited to, benzimidazole, imidazole, imidazoline-2-thione, indole, isatoic anhydride, morpholine, piperazine, piperidine, purine, pyrazole, pyrrole, pyrrolidine and 1,2,4-triazole rings wherein each ring is optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups.

In formulas I and II above, 4- to 10-membered heterocyclic rings include, but are not limited to, imidazole and phthalimide rings wherein each ring is optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups.

The formula I and II compounds of the present invention are effective herbicidal agents useful for the control of a wide variety of undesirable plant species. Those compounds are effective for controlling weeds native to both dry land and wet land areas. The compounds are effective in controlling the above-said plants when applied to the foliage thereof or to the soil or water containing seeds or other propagating organs thereof such as stolons, tubers or rhizomes, at rates of from about 0.001 kg/ha to 4 kg/ha and preferably from about 0.001 kg/ha to 1 kg/ha.

Advantageously, it has been found that the formula I and II compounds of this invention are particularly useful for the selective control of undesirable plant species in the presence of crop plants, crop seeds or other crop propagating organs. In particular, some of the compounds of this invention are selective in soybeans and/or cereal crops such as corn, wheat and rice when applied as preemergence and/or postemergence treatments.

In addition, it has been found that the formula I and II compounds of this invention may be used for the selective control of undesirable plant species in transplanted rice culture by applying a herbicidally effective amount of a formula I compound to the soil or water containing seeds or other propagating organs of said undesirable plant species after the rice has been transplanted.

Formula I compounds of this invention which are especially useful for the selective control of undesirable plant species in the presence of corn include
isopropyl {{2-{5-[3,6-dihydro-3-methyl-2,6-dioxo-4-(trifluoromethyl)-1(2H)-pyrimidinyl]-1,2-benzisothiazol-3-yl}*-p*-tolyl}oxy}acetate;
methyl-{{2-{5-[3,6-dihydro-3-methyl-4-(trifluoromethyl)-2,6-dioxo-1(2H)-pyrimidinyl]-1,2-benzisothiazol-3-yl]-*p*-tolyl}oxy}acetate;
2-(dimethylamino)ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-propynyl {{2-{5-[3,6-dihydro-3-methyl-2,6-dioxo-4-(trifluoromethyl)-1(2H)-pyrimidinyl]-1,2-benzisothiazol-3-yl}-p-tolyl}oxy}acetate;
3-[3-(*p*-ethylphenyl)-1,2-benzisothiazol-5-yl]-1-methyl-6-(trifluoromethyl)-2,4(1H,3H)-pyrimidinedione;
methyl {{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy)acetate;
3-[3-(dibromomethyl)-1,2-benzisothiazol-5-yl]-1-methyl-6-(trifluoromethyl)-2,4(1H,3H)-pyrimidinedione;
1-methyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
1-methyl-3-{3-[(methylsulfonyl)methyl]-1,2-benzisothiazol-5-yl}-6-(trifluoromethyl)-2,4(1H,3H)-pyrimidinedione;
2-propynyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
3-[3-(o-methoxyphenyl)-1,2-benzisothiazol-5-yl]-1-methyl-6-(trifluoromethyl)-2,4(1H,3H)-pyrimidinedione;
N-{{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetyl}methanesulfonamide;
2-propynyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate;
5-[3,6-dihydro-3-methyl-2,6-dioxo-4-(trifluoromethyl)-1(2H)-pyrimidinyl]-1,2-benzisothiazole-3-acetonitrile;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
2-{{2-{5-[3,6-dihydro-3-methyl-2,6-dioxo-4-(trifluoromethyl)-1(2H)-pyrimidinyl]-1,2-benzisothiazol-3-yl}-p-tolyl}oxy}-N-methyl-N-(methylsulfonyl)acetamide;
3-[3-(hydroxymethyl)-1,2-benzisothiazol-5-yl]-1-methyl-6-(trifluoromethyl)-2,4(1H,3H)-pyrimidinedione, methylcarbamate (ester);
N-(benzylsulfonyl)-5-[3,6-dihydro-3-methyl-2,6-dioxo-4-(trifluoromethyl)-1(2H)-pyrimidinyl]-1,2-benzisothiazole-3-carboxamide;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide;
5-[3,6-dihydro-3-methyl-2,6-dioxo-4-(trifluoromethyl)-1(2H)-pyrimidinyl]-6-fluoro-1,2-benzisothiazole-3-carboxylic acid; and
N-(benzylsulfonyl)-5-[3,6-dihydro-3-methyl-2,6-dioxo-4-(trifluoromethyl)-1(2H)-pyrimidinyl]-6-fluoro-1,2-benzisothiazole-3-carboxamide, among others.

Formula I compounds of the present invention which are particularly useful for the selective control of undesirable plant species in the presence of wheat include
isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}acetate;
2-(dimethylamino)ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-propynyl {{2-{5-[3,6-dihydro-3-methyl-2,6-dioxo-4-(trifluoromethyl)-1(2H)-pyrimidinyl]-1,2-benzisothiazol-3-yl}-p-tolyl}oxy}acetate;
methyl 2-{{6-{5-[3,6-dihydro-3-methyl-4-(trifluoromethyl)-2,6-dioxo-1(2H)-pyrimidinyl]-1,2-benzisothiazol-3-yl}-*m*-tolyl}oxy}propionate;
3-(6-fluoro-3-methyl-1,2-benzisoxazol-5-yl)-1-methyl-6-(trifluoromethyl)-2,4(1H,3H)-pyrimidinedione;
3-{3-{6-[(1-benzimidazolylcarbonyl)methoxy]-*m*-tolyl}-1,2-benzisothiazol-5-yl)-1-methyl-6-trifluoromethyl)-2,4(1H,3H)-pyrimidinedione;
allyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
3-phenyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-chloroethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate;
2,2,2-trifluoroethyl {{2-{5-(3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{6-{5-[3,6-dihydro-3-methyl-2,6-dioxo-4-(trifluoromethyl)-1(2H)-pyrimidinyl]-1,2-benzisothiazol-3-yl}-2,4-xyly}oxy}acetate;
{*o*-{5-[3,6-dihydro-3-methyl-2,6-dioxo-4-(trifluoromethyl)-1(2H)-pyrimidinyl]-1,2-benzisothiazol-3-yl}phenoxy}acetic acid;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methoxyacetamide;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl-N-(methylsulfonyl)acetamide;
methyl {{6-(5-[3,6-dihydro-3-methyl-2,6-dioxo-4-(trifluoromethyl)-1(2H)-pyrimidinyl]-6-fluoro-1,2-benzisothiazol-3-yl}-*m*-tolyl}oxy}acetate;
3-(3-acetyl-1,2-benzisothiazol-5-yl)-1-methyl-6-(trifluoromethyl)-2,4(1H,3H)-pyrimidinedione;
methyl 5-[3,6-dihydro-3-methyl-2,6-dioxo-4-(trifluoromethyl)-1(2H)-pyrimidinyl]-1,2-benzisothiazole-3-carboxylate;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
N-[(*m*-chlorobenzyl)sulfonyl]-5-[3,6-dihydro-3-methyl-2,6-dioxo-4-(trifluoromethyl)-1(2H)-pyrimidinyl]-1,2-benzisothiazole-3-carboxamide;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-((isopropylsulfamoyl)-1,2-benzisothiazole-3-carboxamide; and
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide, among others.

3-(1,2-Benzisothiazol-5-yl)-2,4(1H,3H)-pyrimidinedione compounds of this invention which are especially useful for the selective control of undesirable plant species in the presence of rice include
isopropyl {{2-{5-[3,6-dihydro-3-methyl-2,6-dioxo-4-(trifluoromethyl)-1(2H)-pyrimidinyl]-1,2-benzisothiazol-3-yl]-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}acetate;
3-[3-(6-methoxy-*m*-tolyl)-1,2-benzisothiazol-5-yl]-1-methyl-6-(trifluoromethyl)-2,4(1H,3H)-pyrimidinedione;
ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
1-methyl-3-{3-[6-(2-propynyloxy)-*m*-tolyl]-1,2-benzisothiazol-5-yl}-6-(trifluoromethyl)-2,4(1H,3H)-pyrimidinedione;
3-{3-{6-{[(*m*-cyanophenyl)carbamoyl]methoxy}-*m*-tolyl}-1,2-benzisothiazol-5-yl}-1-methyl-6-(trifluoromethyl)-2,4(1H,3H)pyrimidinedione;
3-[3-(2-methoxy-*p*-tolyl)-1,2-benzisothiazol-5-yl]-1-methyl-6-(trifluoromethyl)-2,4(1H,3H)-pyrimidinedione;
2-[3-(6-methoxy-3,4-xylyl)-1,2-benzisothiazol-5-yl]-1-methyl-6-(trifluoromethyl)-2,4(1H,3H)-pyrimidinedione;
3-{3-{6-{[(*m*-cyanophenyl)carbamoyl]methoxy}-*m*-tolyl}-1,2-benzisothiazol-5-yl]-1-methyl-6-(trifluoromethyl)-2,4(1H,3H)-pyrimidinedione;
2-propynyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-α-ethyl-1,2-benzisothiazole-3-acetonitrile;
5-[3,6-dihydro-3-methyl-2,6-dioxo-4-(trifluoromethyl)-1(2H)-pyrimidinyl]-α,α-diethyl-1,2-benzisothiazole-3-acetonitrile;
3-[3-(hydroxymethyl)-1,2-benzisothiazol-5-yl]-1-methyl-6-(trifluoromethyl)-2,4(1H,3H)-pyrimidinedione, 5-isoxazolecarboxylate (ester);
3-[3-(hydroxymethyl)-1,2-benzisothiazol-5-yl]-1-methyl-6-(trifluoromethyl)-2,4(1H,3H)-pyrimidinedione, cyclopropanecarboxylate (ester);
methyl 5-[3,6-dihydro-3-methyl-2,6-dioxo-4-(trifluoromethyl)-1(2H)-pyrimidinyl]-1,2-benzisothiazole-3-carboxylate;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
benzyl 5-[3,6-dihydro-3-methyl-2,6-dioxo-4-(trifluoromethyl)-1(2H)-pyrimidinyl]-α,α-dimethyl-1,2-benzisothiazole-3-acetate;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide; and
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide, among others.

Formula I compounds of this invention which are particularly useful for the selective control of undesirable plant species in the presence of soybeans include
methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate;
methyl 2-{{6-{5-(3,6-dihydro-3-methyl-4-(trifluoromethyl)-2,6-dioxo-1(2H)-pyrimidinyl]-1,2-benzisothiazol-3-yl}-3,4-xylyl}oxy}propionate;
1-methyl-3-(3-methyl-1,2-benzisothiazol-5-yl)-6-(trifluoromethyl)-2,4(1H,3H)-pyrimidinedione;
methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate;
2-propynyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
3-[3-(*o*-methoxyphenyl)-1,2-benzisothiazol-5-yl]-1-methyl-6-(trifluoromethyl)-2,4(1H,3H)-pyrimidinedione;
2-propynyl 2-{{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate;
2-propynyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
methyl {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetate;
methyl {{6-{5-[3,6-dihydro-3-methyl-2,6-dioxo-4-(trifluoromethyl)-1(2H)-pyrimidinyl]-6-fluoro-1,2-benzisothiazol-3-yl}-*m*-tolyl}oxy}acetate;
3-[3-(hydroxymethyl)-1,2-benzisothiazol-5-yl]-1-methyl-6-(trifluoromethyl)-2,4(1H,3H)-pyrimidinedione, benzoate (ester);
3-[3-(hydroxymethyl)-1,2-benzisothiazol-5-yl]-1-methyl-6-(trifluoromethyl)-2,4(1H,3H)-pyrimidinedione, 2-thiophenecarboxylate (ester);
3-[3-(hydroxymethyl)-1,2-benzisothiazol-5-yl]-1-methyl-6-(trifluoromethyl)-2,4(1H,3H)-pyrimidinedione, methoxyacetate (ester);
3-[3-(hydroxymethyl)-1,2-benzisothiazol-5-yl]-1-methyl-6-(trifluoromethyl)-2,4(1H,3H)-pyrimidinedione, cyclopropanecarboxylate (ester);
3-[3-(hydroxymethyl)-1,2-benzisothiazol-5-yl]-1-methyl-6-(trifluoromethyl)-2,4(1H,3H)-pyrimidinedione, 2-furoate (ester);
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide; and
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide, among others.

While the compounds of this invention are effective for controlling undesirable plant species when employed alone, they may also be used in combination with other biological chemicals, including herbicides.

The compounds of this invention may be applied to the foliage of undesirable plant species or to the soil or water containing seeds or other propagating organs thereof in the form of a solid or liquid herbicidal composition, comprising a herbicidally effective amount of the desired compound dispersed or dissolved in an agronomically acceptable, inert solid or liquid carrier. The compositions may be applied as preemergence or postemergence treatments.

The formula I and II compounds of the present invention may be formulated as emulsifiable concentrates, wettable powders, granular formulations, suspension concentrates, flowable concentrates and the like.

Formula I compounds wherein Z is S may be prepared, as illustrated in Flow Diagram I, by cyclizing a ketone of formula III with sulfur and ammonium hydroxide or ammonia to form a nitrobenzisothiazole of formula IV, reducing the formula IV compounds using conventional reducing agents such as an iron in acetic acid to form an aminobenzisothiazole of formula V, reacting the formula V compound with phosgene or a phosgene equivalent to form an isocyanate of formula VI, reacting the isocyanate with a 3-aminoacrylate of formula VII in the presence of a base such as sodium hydride to form an intermediate compound of formula VIII, and reacting the formula VIII compound with an electrophile of formula IX in the presence of a base such as potassium carbonate.

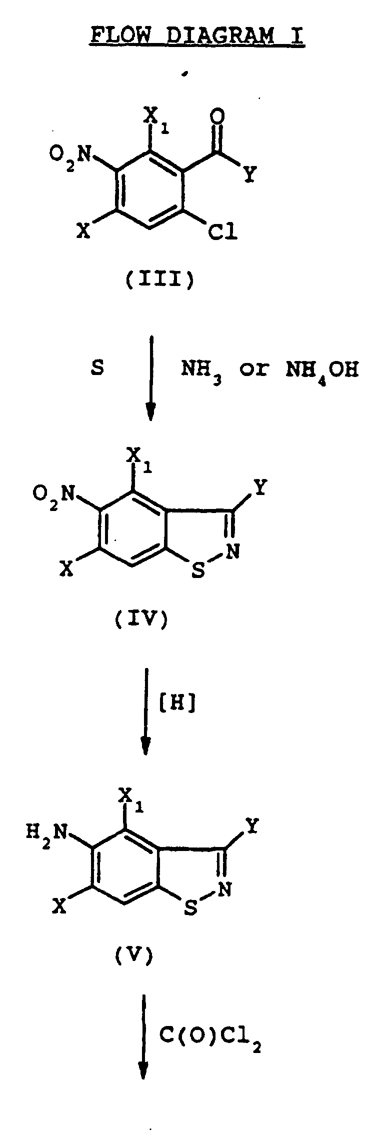

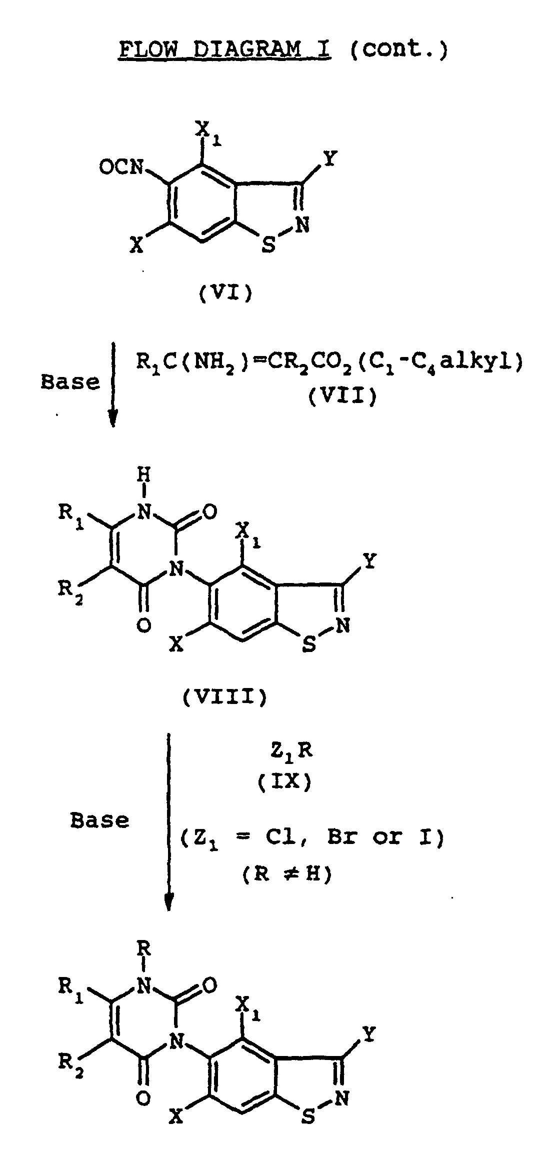

Formula I compounds wherein Z is S may also be prepared, as shown in Flow Diagram II, by reacting an aminobenzisothiazole of formula V with a 1,3-propanedione of formula X in the presence of a base such as triethylamine to form an intermediate compound of formula XI, and reacting the formula XI compound with phosgene or thiophosgene or an equivalent thereof followed by an amine of formula XII in the presence of a base such as triethylamine or pyridine.

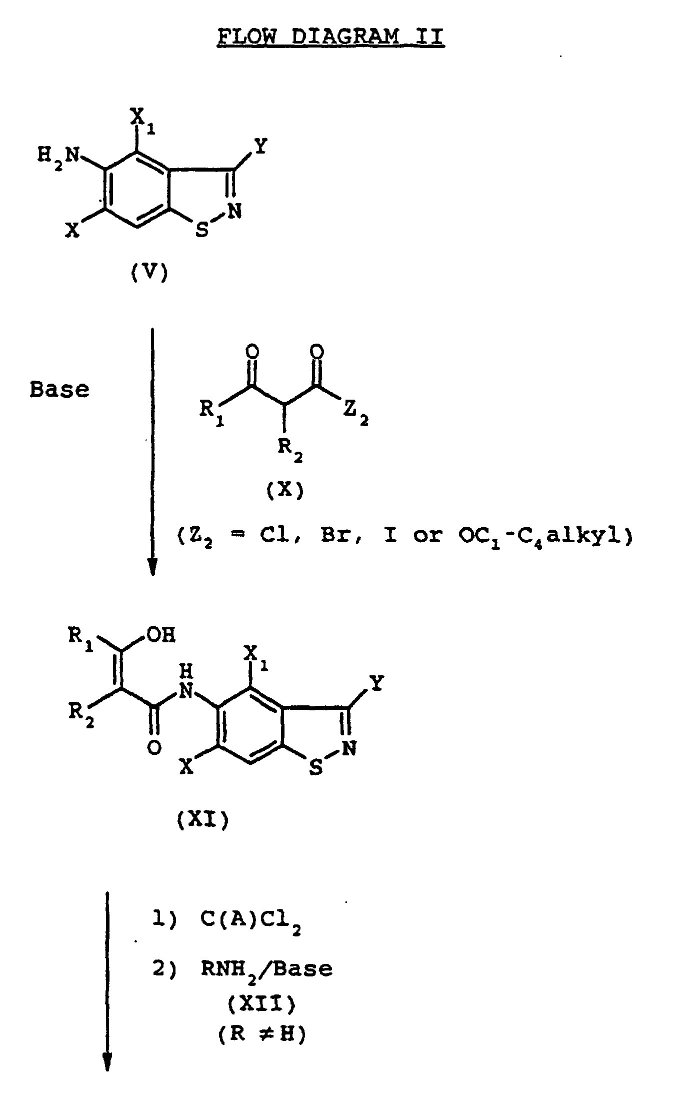

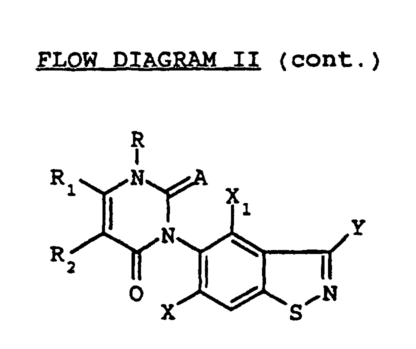

Alternatively, formula I compounds wherein Z is S may be prepared, as shown in Flow Diagram III, by reacting an aminobenzisothiazole of formula V with a 2-dialkylamino-6H-1,3-oxazin-6-one of formula XIII in the presence of an organic acid such as acetic acid to form an intermediate compound of formula VIII, and reacting the formula VIII compound with an electrophile of formula IX in the presence of a base such as potassium carbonate.

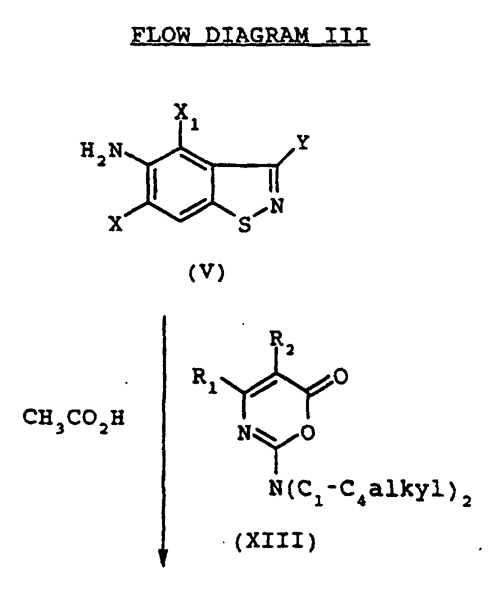

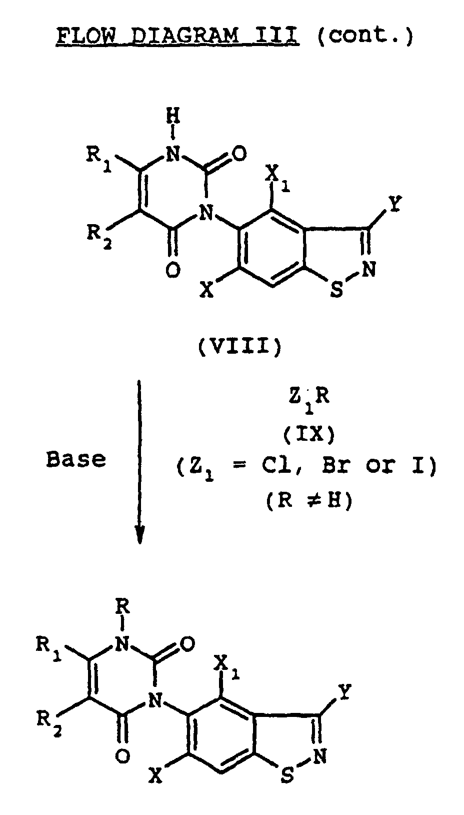

Formula I compounds wherein Y is phenyl substituted with one X₄R₂₂ group may be prepared by reacting a formula I compound wherein Y is phenyl substituted with one C₁-C₄alkoxy or C₁-C₄alkylthio group with boron tribromide to form the corresponding compound wherein Y is phenyl substituted with one hydroxy or thio group, and reacting the thus formed compound with an electrophile of formula Z₁R₂₂ (Z₁ = Cl, Br or I) in the presence of base such as potassium hydroxide.

Formula I compounds wherein Z is O may be prepared, as shown in Flow Diagram IV, by reacting a ketone of formula III with hydroxylamine hydrochloride optionally in the presence of sodium acetate to form an oxime of formula XIV, cyclizing the formula XIV compound with a base such as potassium hydroxide to form a nitrobenzisoxazole of formula XV, reducing the formula XV compound using conventional reducing agents such as tin(II) chloride in acetic acid to form an aminobenzisoxazole of formula XVI, reacting the formula XVI compound with a 2-dialkylamino-6H-1,3-oxazin-6-one of formula XIII in the presence of an organic acid such as acetic acid to form an intermediate compound of formula XVII, and reacting the formula XVII compound with an electrophile of formula IX in the presence of a base such as potassium carbonate.

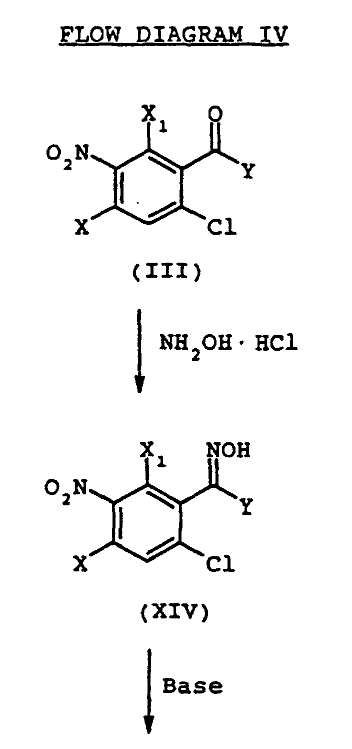

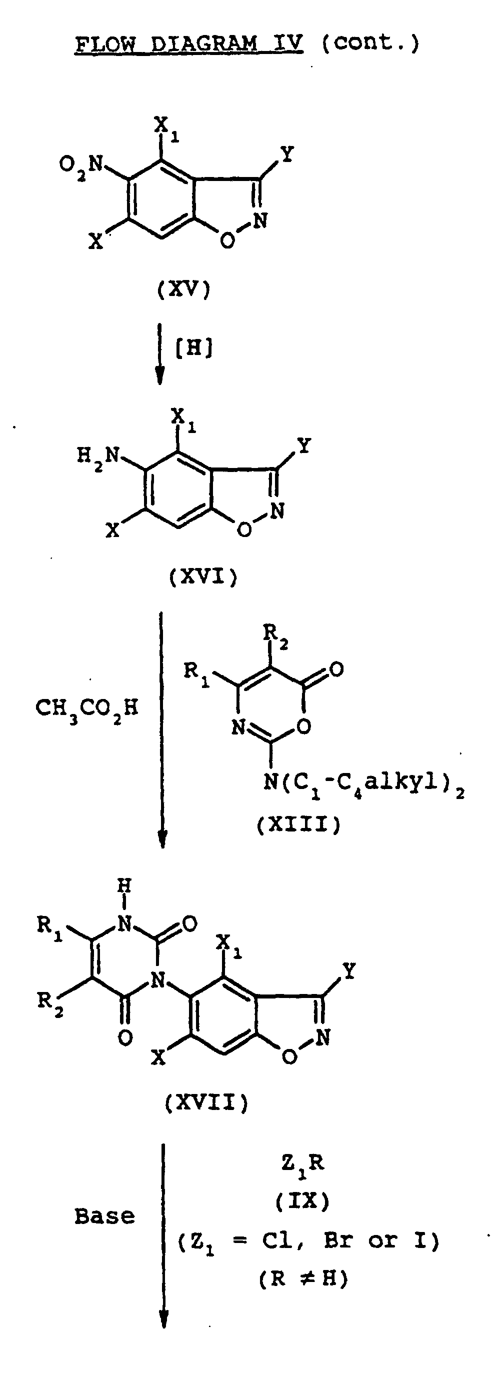

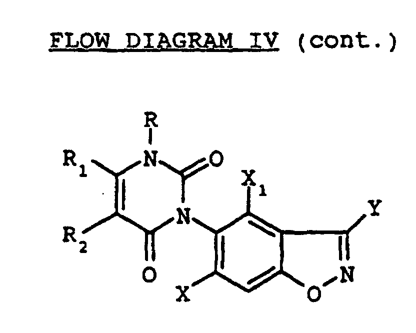

Alternatively, nitrobenzisoxazole compounds of formula XV may be prepared, as shown in Flow Diagram V, by reacting a ketone of formula XVIII with hydroxylamine hydrochloride optionally in the presence of a base such as sodium acetate to form an oxime of formula XIX, cyclizing the formula XIX compound with 1,1'-carbonyldiimidazole in the presence of a base such as triethylamine to form a benzisoxazole of formula XX, and nitrating the formula XX compound using conventional methods such as a nitric acid/sulfuric acid mixture.

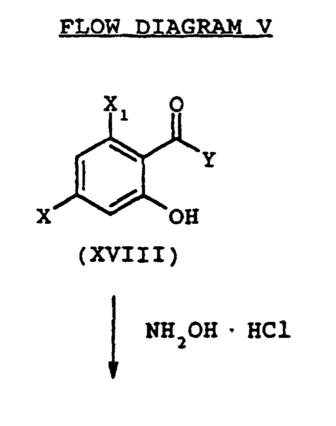

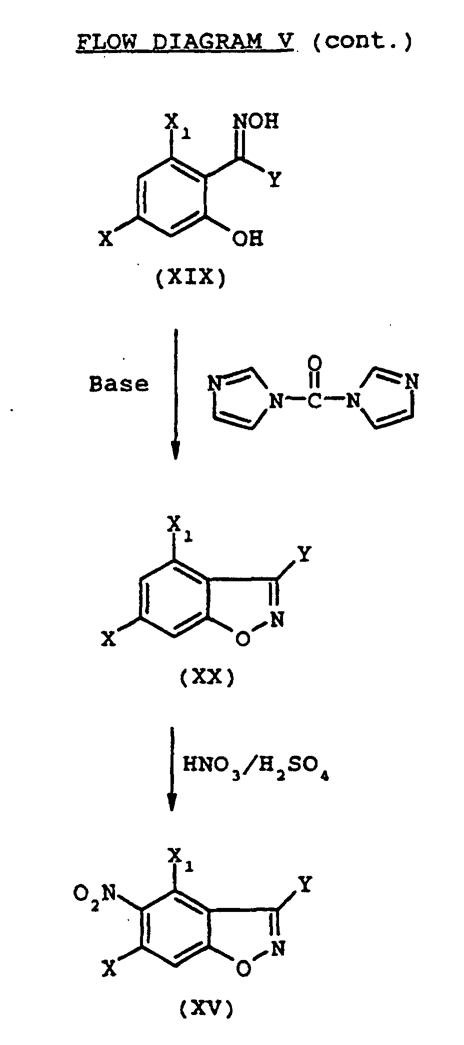

Formula I compounds wherein Y is halomethyl may be prepared, as shown in Flow Diagram VI, by halogenating a formula I compound wherein Y is methyl with a halogenating agent such as N-bromosuccinimide, N-chlorosuccinimide, N-iodosuccinimide, bromine, chlorine, sulfuryl chloride, sulfuryl bromide and the like.

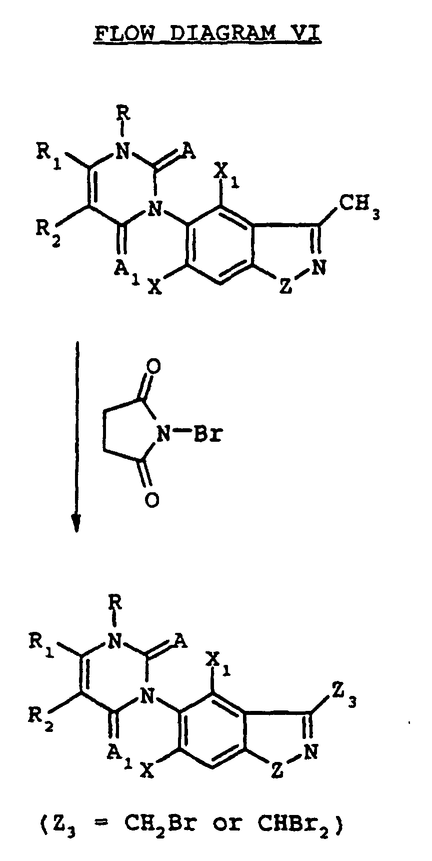

Formula I compounds wherein Y is a C₁-C₂₀alkoxymethyl group may be prepared, as shown in Flow Diagram VII, by reacting a formula I compound wherein Y is bromomethyl with a C₁-C₂₀alcohol in the presence of silver trifluoromethanesulfonate.

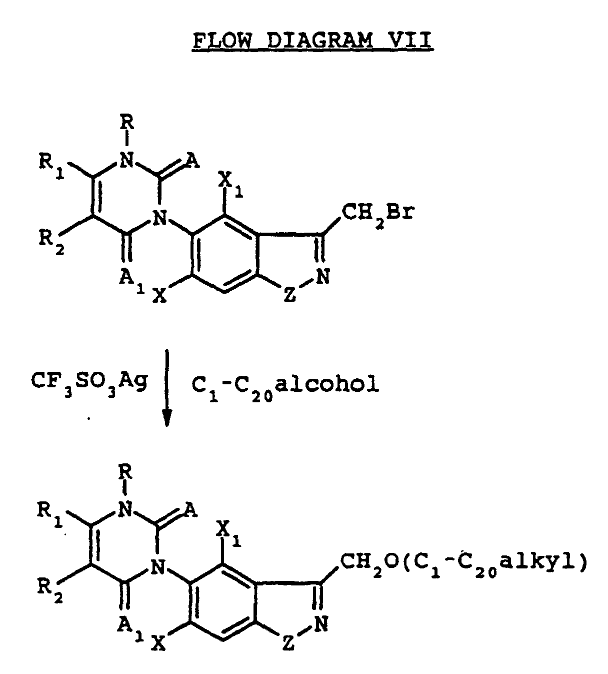

Formula I compounds wherein Y is methyl substituted with one X₄R₂₂ group may be prepared, as shown in Flow Diagram VIII, by reacting a formula I compound wherein Y is bromomethyl with an alcohol or thiol of formula XXI in the presence of a base such as sodium hydride.

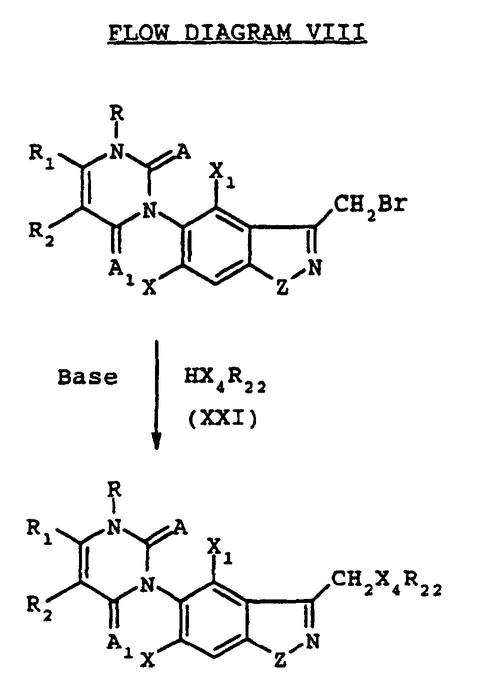

Formula I compounds wherein Y is C₁-C₄alkylthiomethyl may be prepared, as shown in Flow Diagram IX, by reacting a formula I compound wherein Y is bromomethyl with an alkali metal thioalkoxide.

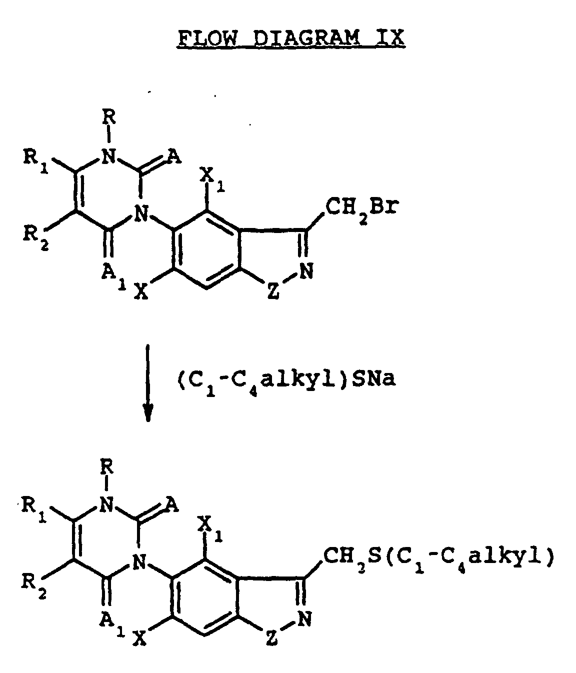

Formula V intermediate compounds wherein Y is OR₁₂ may be prepared, as shown in Flow Diagram X, by nitrating a benzisoxazol-3-ol or benzisothiazol-3-ol of formula XXII with a conventional nitrating agent such as a nitric acid/sulfuric acid mixture to form a 5-nitrobenzisoxazol-3-ol or 5-nitrobenzisothiazol-3-ol of formula XXIII, reacting the formula XXIII compound with an electrophile of formula XXIV in the presence of a base such'as potassium carbonate to form an intermediate compound of formula XXV, and reducing the formula XXV compound using conventional reducing agents.

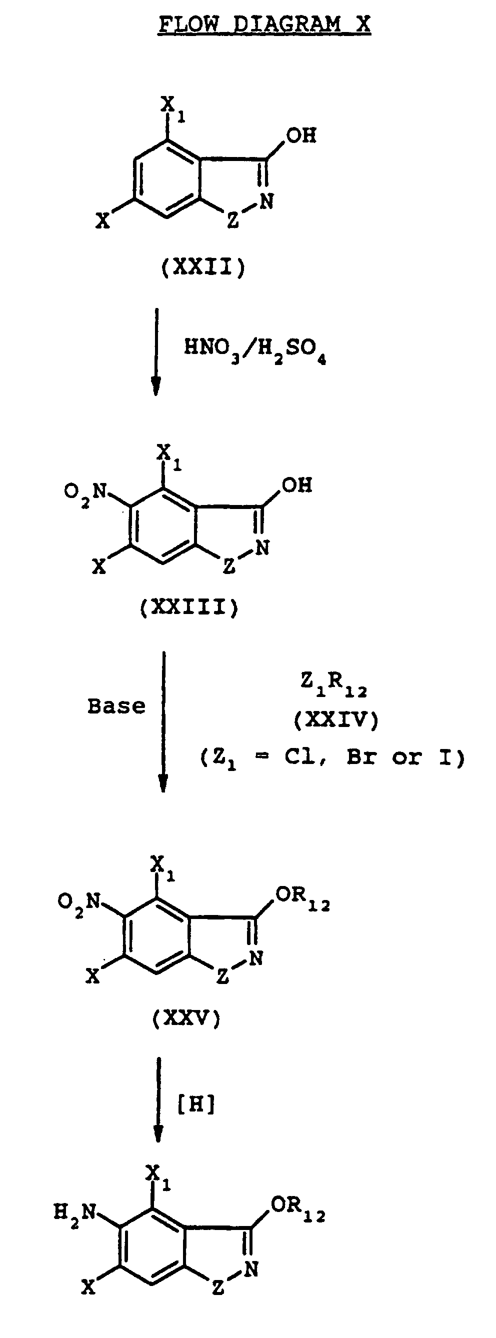

Formula V intermediate compounds wherein Y is Cl or Br may be prepared, as shown in Flow Diagram XI, by reacting a 5-nitrobenzisoxazol-3-ol or 5-nitrobenzisothiazol-3-ol of formula XXIII with phosphorous oxychloride, phosphorous oxybromide or phosphorous pentabromide to form a 3-halo-5-nitrobenzisoxazol or 3-halo-5-nitrobenzisothiazol of formula XXVI, and reducing the formula XXVI compound using conventional reducing agents.

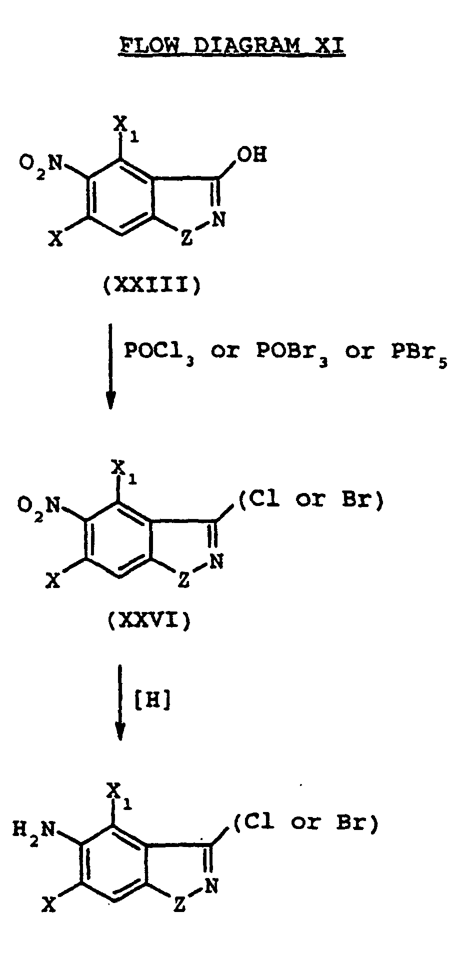

Formula I compounds wherein A is 0 and A₁ is S may be prepared, as shown in Flow Diagram XII, by reacting a formula I compound wherein A and A₁ are O with Lawesson's Reagent (2,4-bis(4-methoxyphenyl)-1,3-dithia-2,4-diphosphetane-2,4-disulfide) in the presence of a base such as sodium hydrogen carbonate.

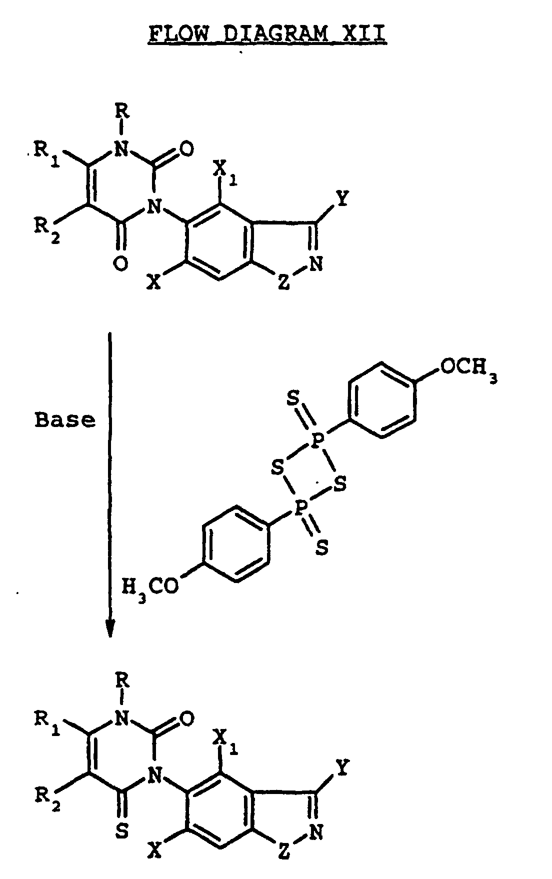

Formula I compounds wherein A and A₁ are S may be prepared, as shown in Flow Diagram XIII, by reacting a formula I compound wherein A is S and A₁ is O with Lawesson's Reagent in the presence of a base.

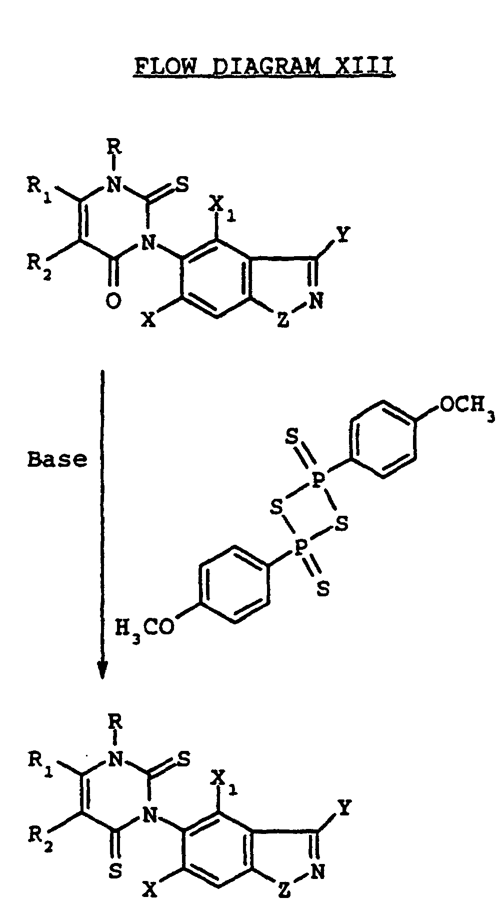

Formula I compounds wherein R₁₉, R₂₆ and R₃₂ are hydrogen may be prepared by hydrolyzing a corresponding formula I ester with an acid such as trifluoroacetic acid. The resultant formula I acid may then be reacted with thionyl chloride or oxalyl chloride to form an acid chloride which is then reacted with an R₁₉OH, R₂₆OH or R₃₂OH alcohol or an HNR₂₀R₂₁, HNR₃₅R₃₆ or HNR₄₉R₅₀ amine to form additional compounds of formula I.

Formula I compounds wherein R₁₉, R₂₆ and R₃₂ are an alkali metal, alkaline earth metal, manganese, copper, zinc, cobalt, silver, nickel, ammonium or organic ammonium cation may be prepared from formula I compounds wherein R₁₉, R₂₆ and R₃₂ are hydrogen using conventional methods.

Intermediate compounds of formula IV wherein Y is alkyl substituted with one CO₂R₁₉ group may be prepared, as shown in Flow Diagram XIV, by reacting an intermediate compound of formula XXVI with an oxylate of formula XXVII in the presence of a base to form an intermediate compound, reacting the intermediate compound with urea to form a compound of formula XXVIII wherein Y is methyl substituted with one CO₂R₁₉ group, and optionally reacting the formula XXVIII compound with a C₁-C₅alkyl halide in the presence of a base such as sodium hydride to form a compound of formula XXIX. In addition, the formula XXVIII and XXIX compounds may be saponified to form compounds wherein R₁₉ is hydrogen.

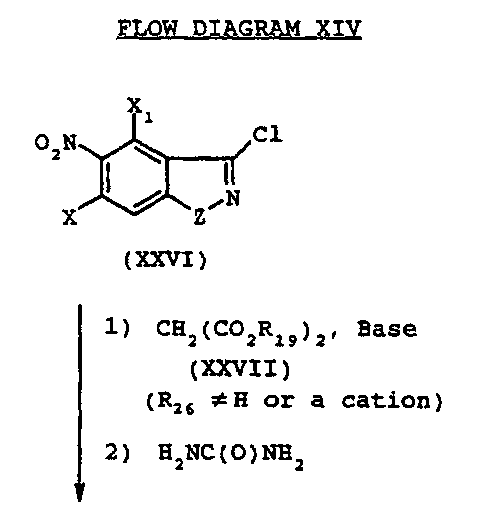

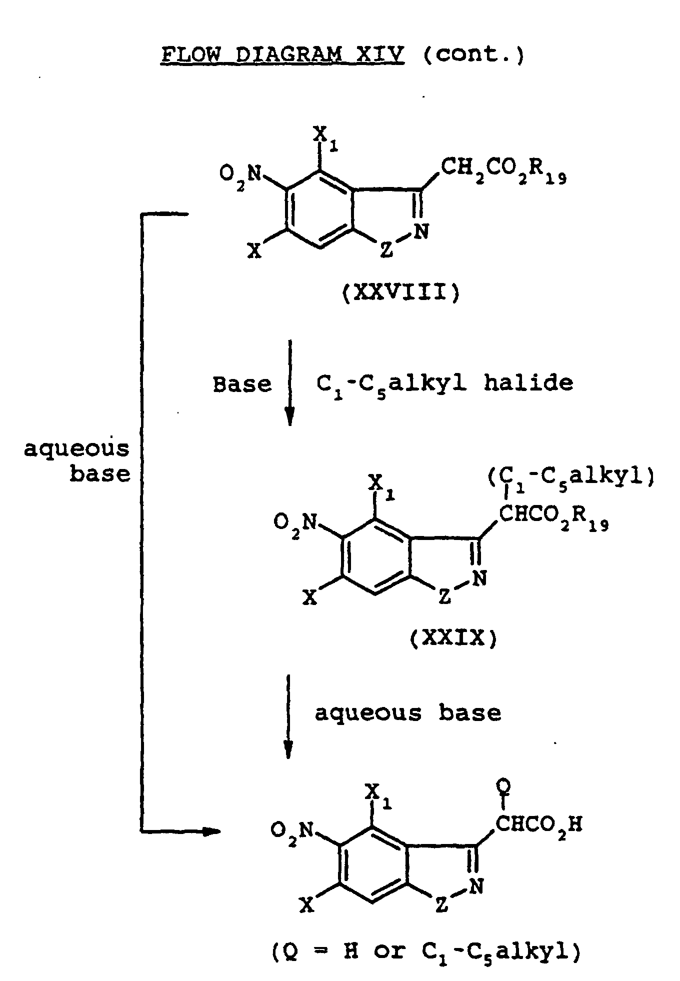

Alternatively, formula III compounds wherein Y is an α-methylacetate group may be prepared as shown in Flow Diagram XV.

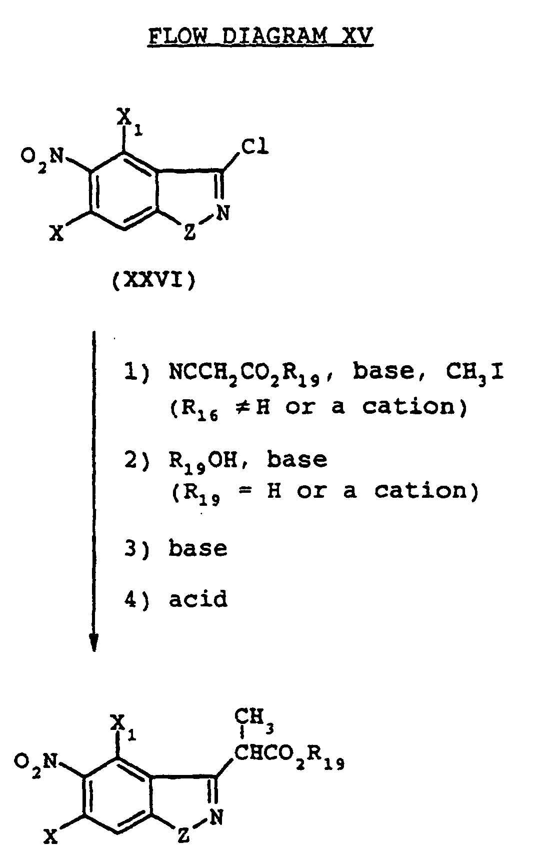

Intermediate compounds of formula III wherein Y is methyl substituted with one CO₂R₁₉ group may be prepared by reacting an acid chloride of formula XXX with an acetate of formula XXXI in the presence of a base such as sodium hydride, and optionally reacting the formula III compound wherein Y is methyl substituted with one CO₂R₁₉ group with a C₁-C₅alkyl halide in the presence of a base such as sodium hydride to form a formula III compound wherein Y is an α-alkylacetate group. The reactions are shown below in Flow Diagram XVI.

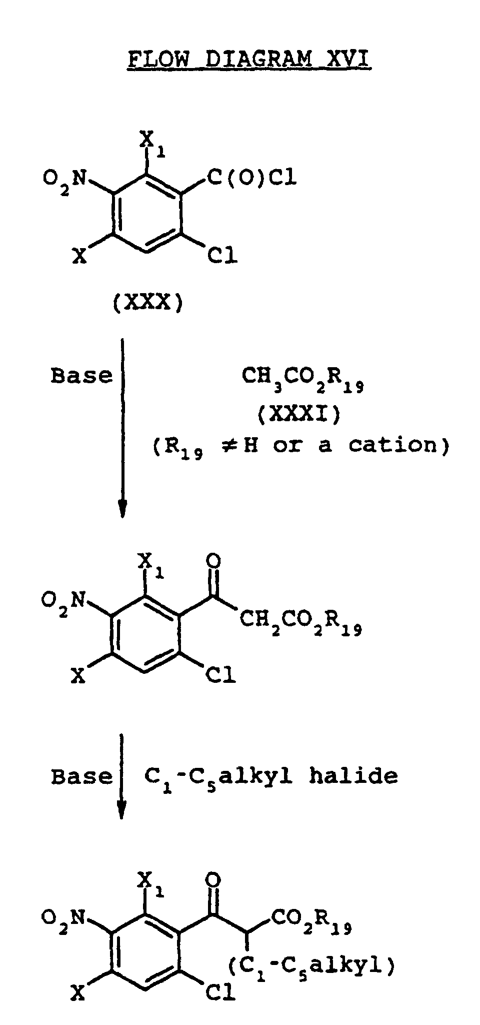

Formula IV and XV compounds wherein Y is an alkyl or phenyl group substituted with one C(O)R₁₈ or C(O)R₂₅ group may be prepared by reacting the corresponding acid chloride with an appropriately substituted magnesium bromide compound, copper bromide compound or lithium compound. The reaction scheme is exemplified below for the case where Y is phenyl substituted with one C(O)R₂₅ group.

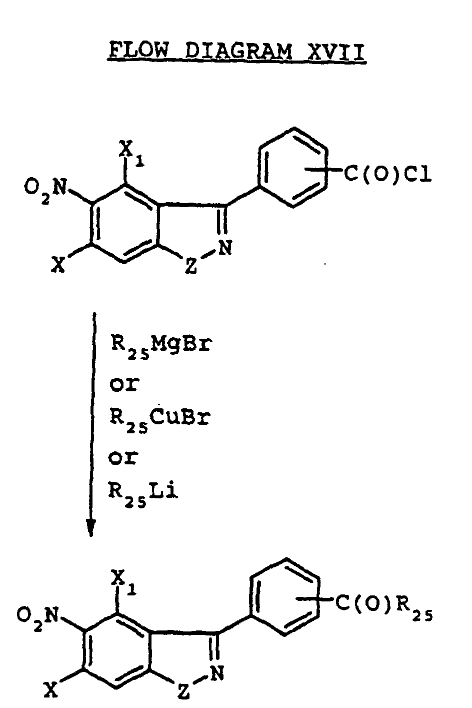

Formula I, IV and XV compounds wherein Y is alkyl, cycloalkyl, alkenyl, cycloalkenyl, alkynyl or phenyl substituted with a CHO group may be prepared by reducing a formula I, IV or XV compound wherein Y is alkyl, cycloalkyl, alkenyl, cycloalkenyl, alkynyl or phenyl substituted with a CO₂H group with a conventional reducing agent such as a borane-tetrahydrofuran complex to form a formula I, IV or XV compound wherein Y is alkyl, cycloalkyl, alkenyl, cycloalkenyl, alkynyl or phenyl substituted with a CH₂OH group, and oxidizing the resultant alcohol with a conventional oxidizing agent such as chromium(VI) oxide. The reaction scheme is exemplified in Flow Diagram XVIII for the case where Y is phenyl substituted with one CHO group.

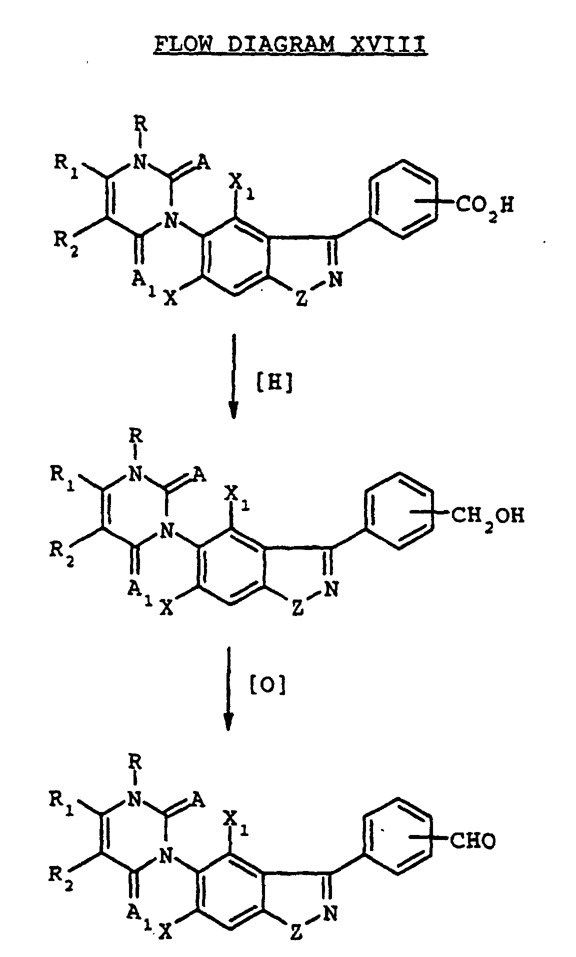

Formula IV an XV compounds wherein Y is alkyl or cycloalkyl substituted with a C(O)R₁₈ group may be prepared by reacting a formula IV or XV compound wherein Y is haloalkyl or halocycloalkyl with a lithium reagent of formula XXXII to form an intermediate compound, and reacting the intermediate compound with a Lewis Acid such as borontrifluoride in the presence of water. The reaction scheme is exemplified in Flow Diagram XIX for the case where Y is methyl substituted with one C(O)R₁₈ group.

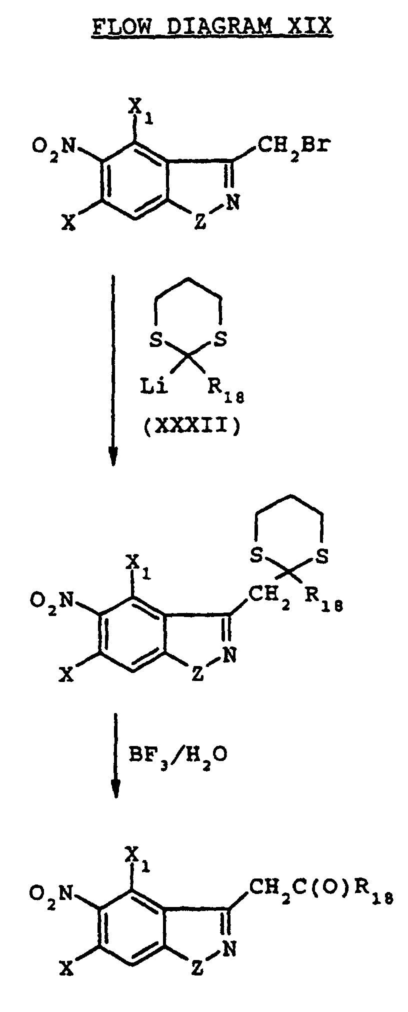

Formula I compounds wherein Y is formyl may also be prepared as shown in Flow Diagram XX.

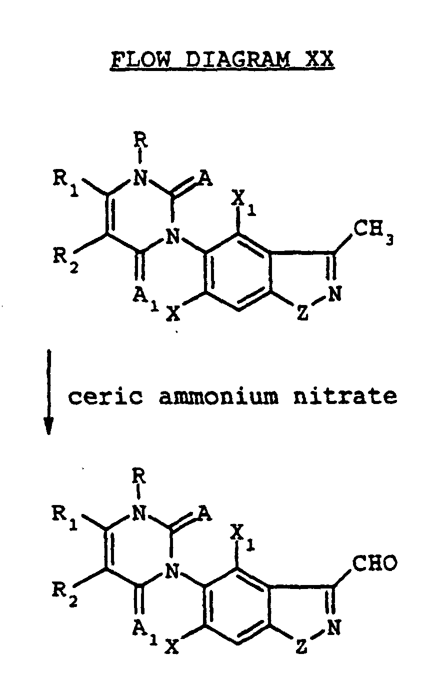

Formula IV an XV compounds wherein Y is X₂R₁₂ and R₁₂ is optionally substituted phenyl may be prepared, as shown in Flow Diagram XXI, by reacting a formula IV or XV compound wherein Y is Br with an R₁₂X₂H compound in the presence of a base such as potassium carbonate.

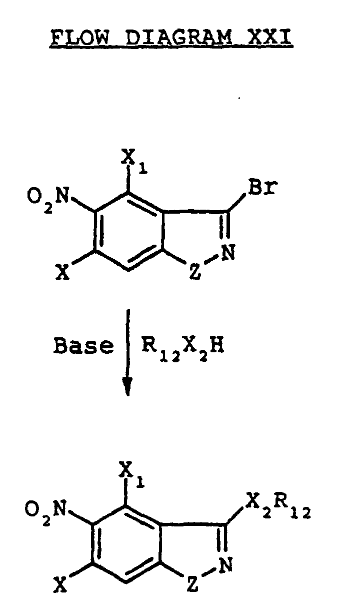

Formula IV and XV compounds wherein Y is trifluoromethyl may be prepared, as shown in Flow Diagram XXII, by reacting a formula IV or XV compound wherein Y is Br with a silyl compound of formula XXXIII in the presence of copper(I) iodide and potassium iodide.

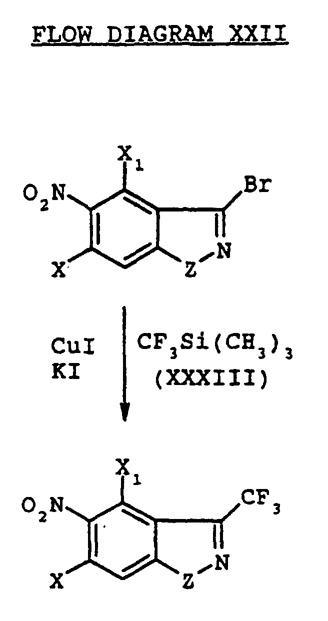

Alternatively, formula III and XIV compounds wherein Y is trifluoromethyl may be prepared by reacting a formula IV or XV compound wherein Y is Br with sodium trifluoroacetate in the presence of a catalyst such as a tetrasubstituted palladium catalyst. The reaction scheme is shown in Flow Diagram XXIII.

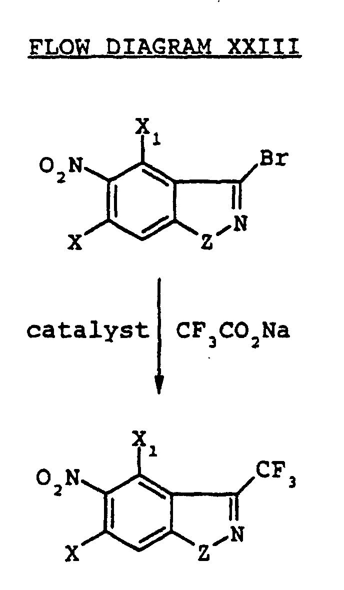

Formula I compounds wherein Y is CH₂CN may be prepared, as shown in Flow Diagram XXIV, by reacting a formula I compound wherein Y is CH₂Br with potassium cyanide.

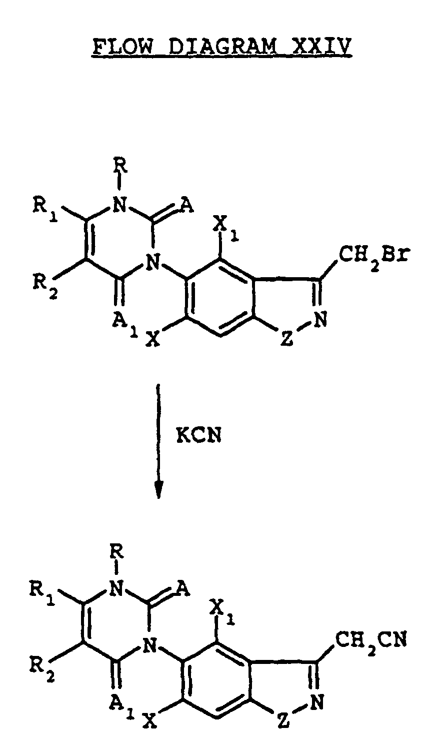

Formula I compounds wherein Y is R₁₂ or X₂R₁₂ and R₁₂ is chloroalkyl, fluoroalkyl or iodoalkyl may be prepared by reacting the corresponding formula I compound wherein R₁₂ is bromoalkyl with lithium chloride, sodium iodide, potassium fluoride or silver(I) fluoride. The reaction scheme is exemplified below in Flow Diagram XXV for the case where Y is fluoromethyl, iodomethyl and chloromethyl.

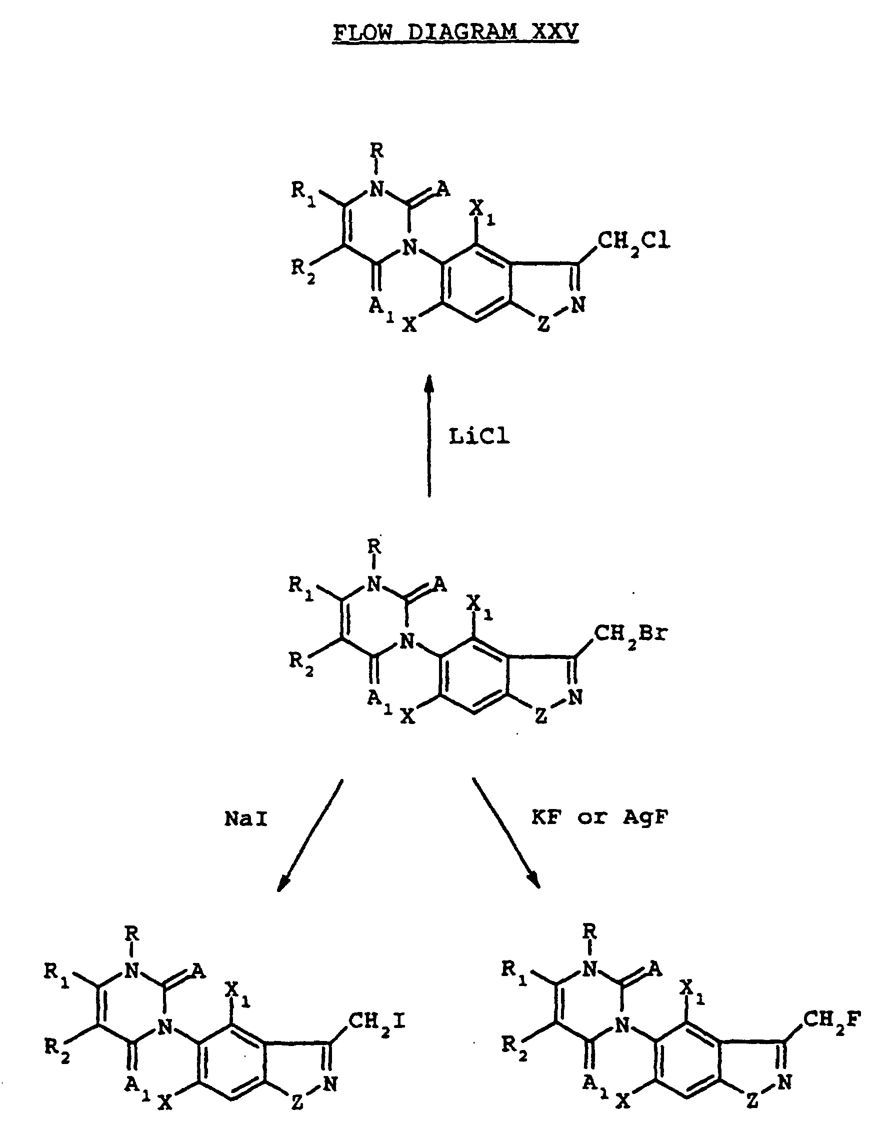

Formula I compounds wherein A is S and A₁ is O may be prepared, as shown in Flow Diagram XXVI, by reacting an amine of formula V or XVI with thiophosgene or a thiophosgene equivalent in the presence of a base such as triethylamine to form an isothiocyanate of formula XXXIV, and reacting the formula XXXIV compound with an alkene of formula XXXV in the presence of a base such as sodium hydride.

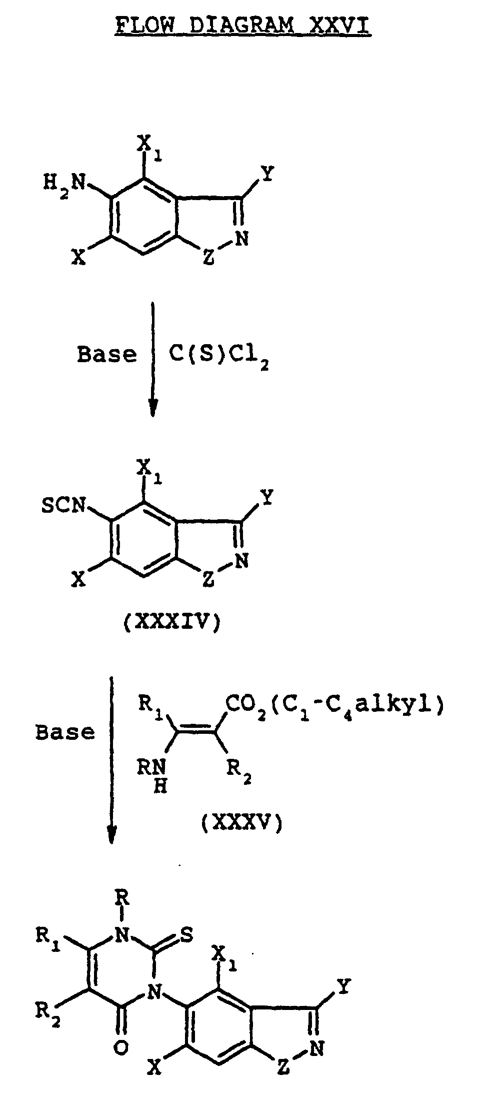

Formula I compounds wherein A and A₁ are O may be prepared, as shown in Flow Diagram XXVII, by reacting an isocyanate of formula XXXVI with an alkene of formula XXXVII in the presence of a base such as sodium hydride to form a urea of formula XXXVIII, cyclizing the formula XXXVIII compound with acid to form an intermediate compound of formula XXXIX, and reacting the formula XXXIX compound with an electrophile of formula XL in the presence of a base such as potassium carbonate.

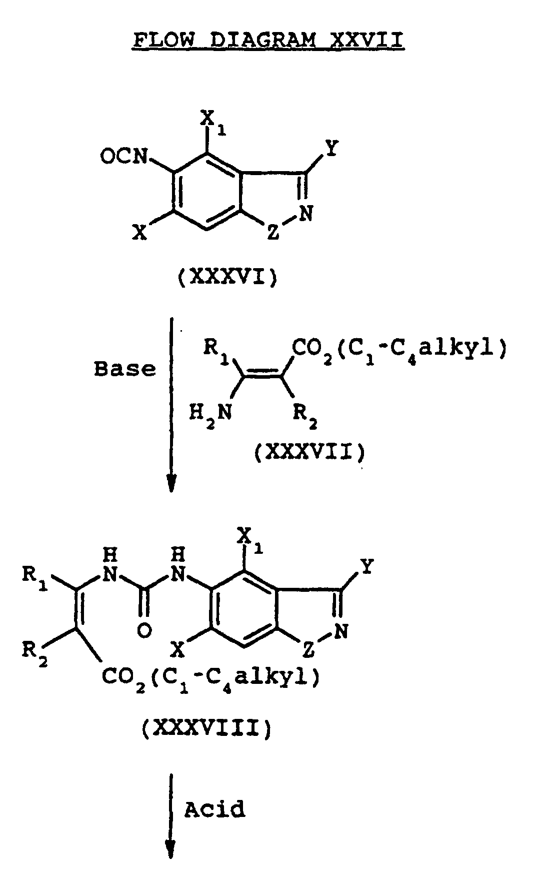

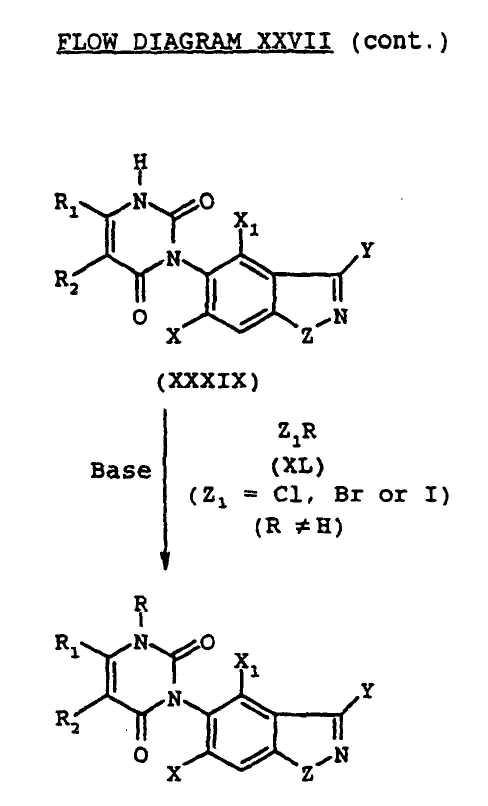

Intermediate compounds of formula III wherein Y is acetate substituted with α-(C₁-C₆alkyl) or α,α-bis(C₁-C₆-alkyl) may be prepared by treated an acid chloride of formula XLI with zinc and an α-halo ester of formula XLII to form an intermediate keto-ester of formula XLIII, which is nitrated to form an intermediate of formula III. The reaction scheme is shown in Flow Diagram XXVIII.

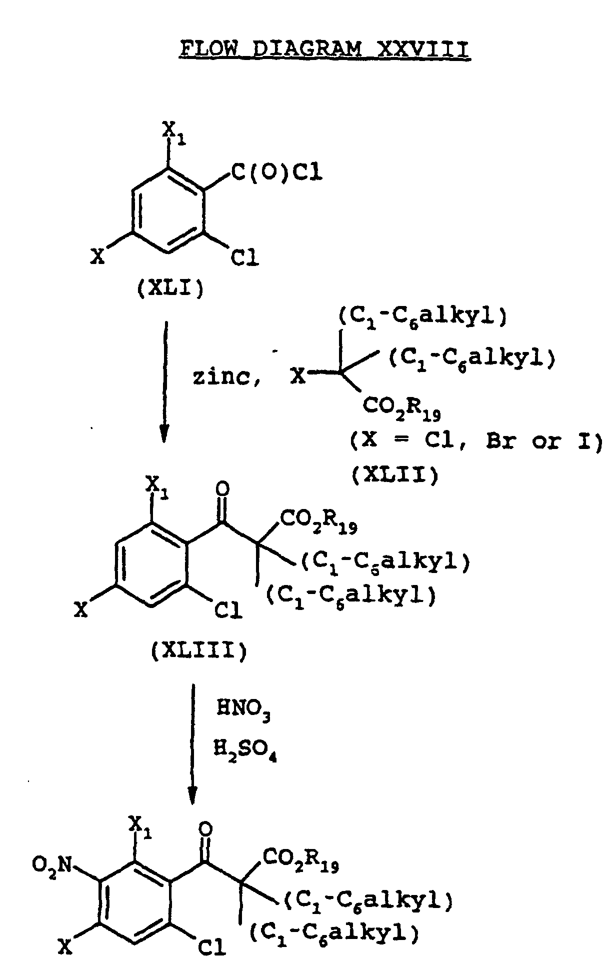

Formula V intermediate compounds wherein Y is Cl or Br may also be prepared, as shown in Flow Diagram XXIX, by reacting a 5-nitrobenzisothiazole or 5-nitrobenzisoxazole of formula XLIV with chlorine or bromine in an acid such as acetic acid. The reaction scheme is shown below in Flow Diagram XXIX.

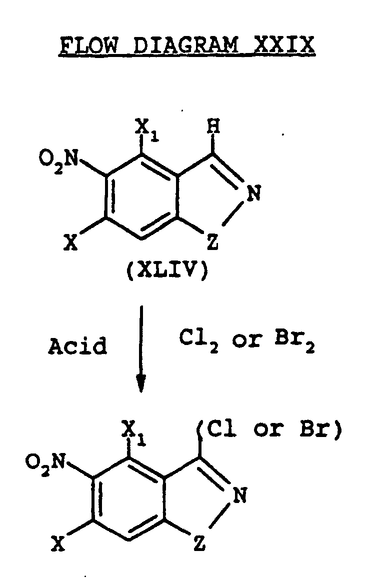

Formula I compounds wherein Y is C(O)X₂R₁₂ and X₂ is O or S may be prepared as shown in Flow Diagram XXX.

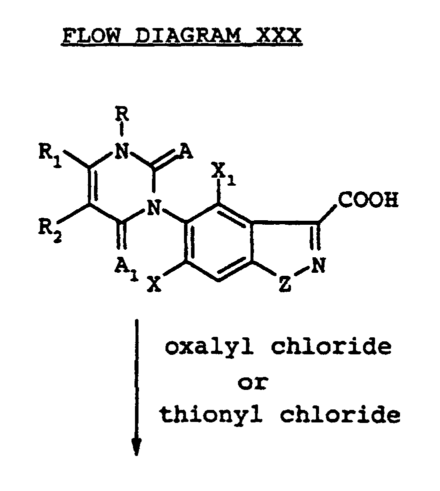

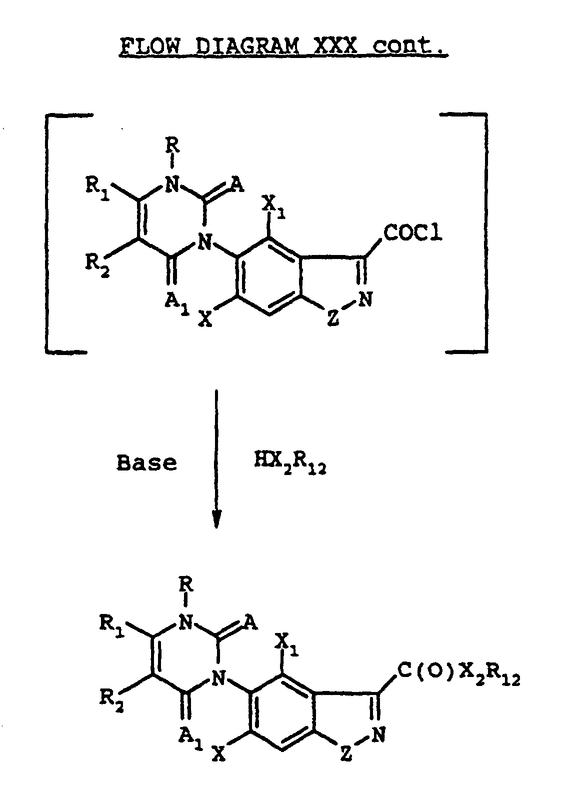

Formula I compounds wherein Y is C(O)NR₁₂R₁₃ may be prepared as shown below in Flow Diagram XXXI.

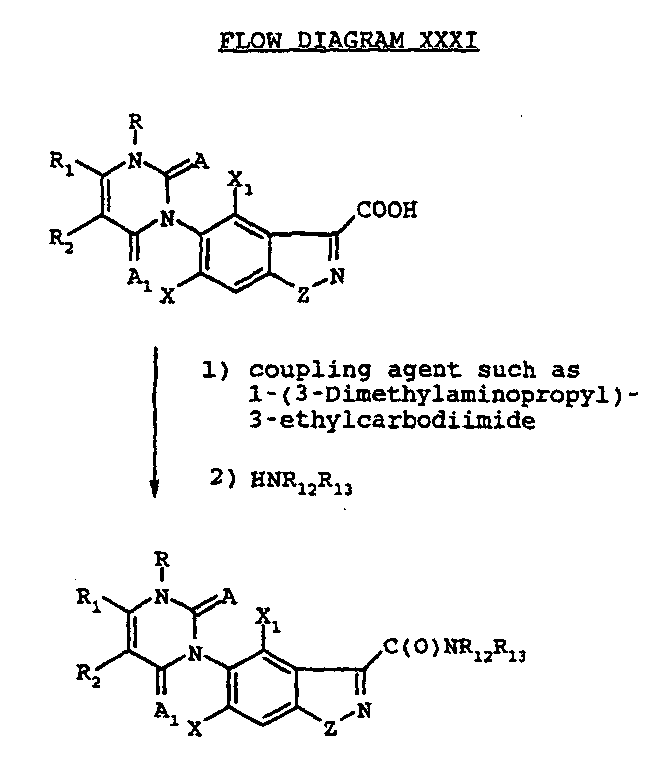

Formula I compounds wherein Y is C(O)NR₁₃SO₂NR₄₆R₄₈ may be prepared as shown below in Flow Diagram XXXII.

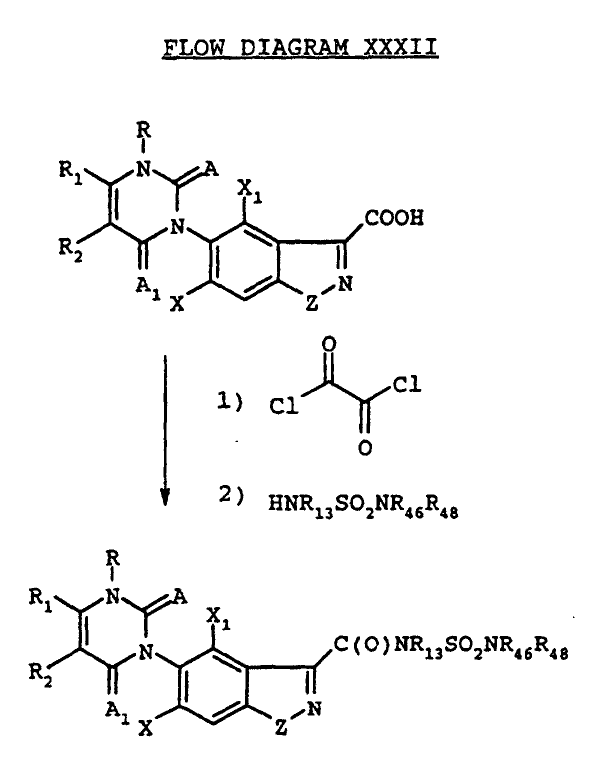

Formula I compounds wherein Y is C(O)N(R₁₂)OR₂₃ may be prepared as shown in Flow Diagram XXXIV.

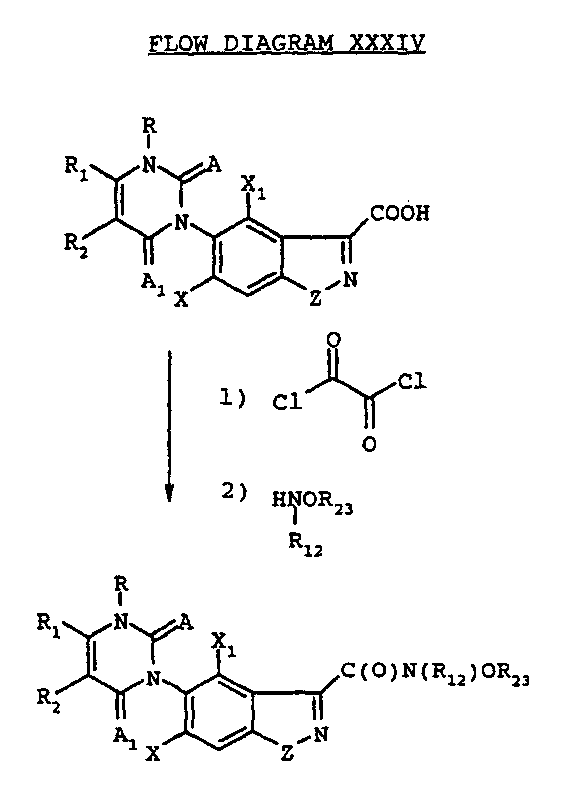

Formula I compounds wherein Y is CH(OH)R₂₃, C(O)R₂₃, CH(OR₂₂)R₂₃ and CH(R₂₃)OC(O)R₃₃ may be prepared as shown below in Flow Diagram XXXV.

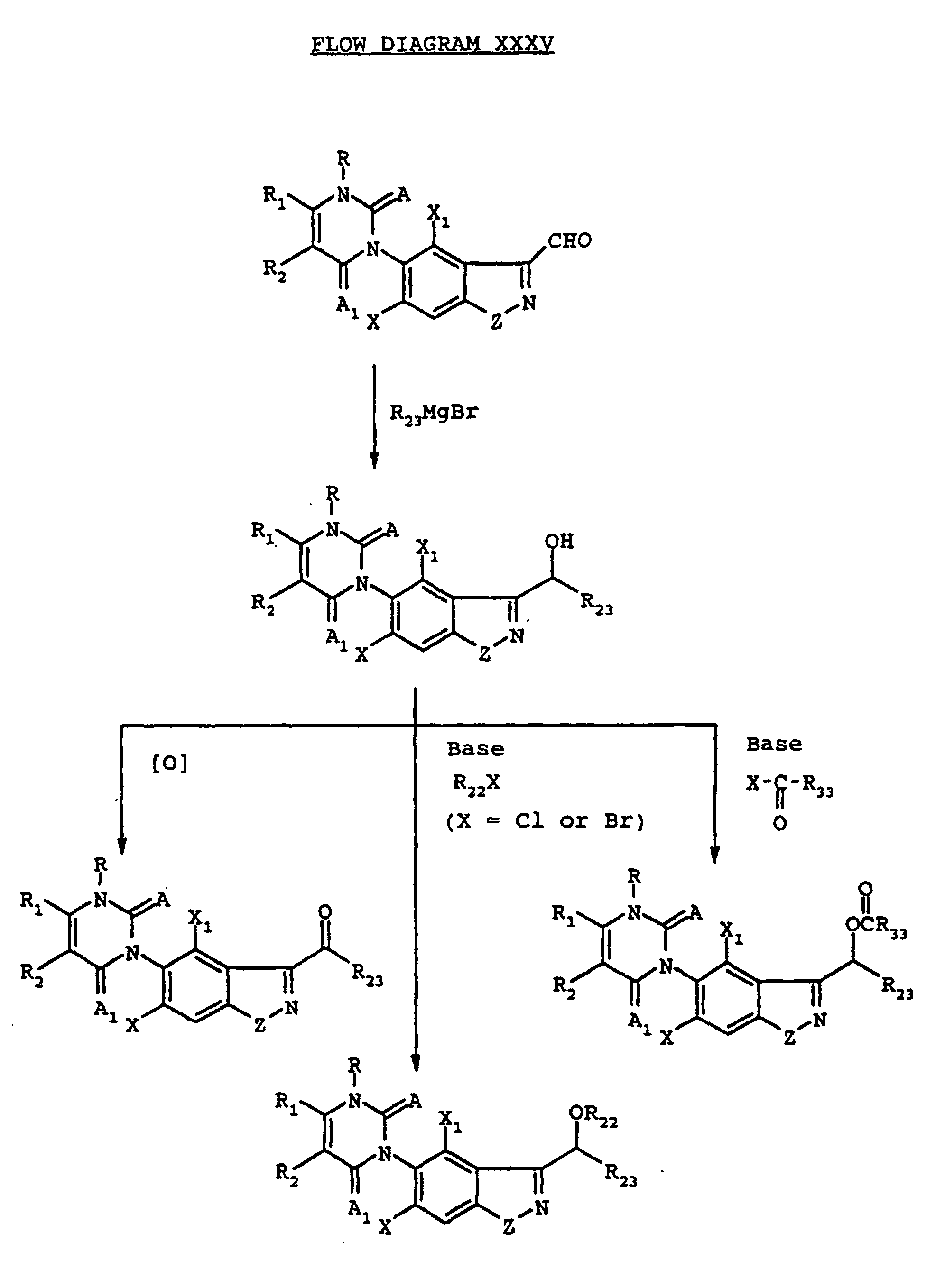

Other methods for the preparation of formula I compounds and methods for the preparation of formula II compounds will become apparent from the examples set forth below. In addition, certain compounds of formulas I and II may be converted into other compounds of formulas I and II by using conventional procedures known to those skilled in the art.

The present invention also relates to intermediate compounds having the structural formula XLV 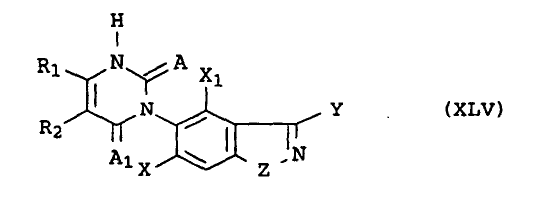 wherein R₁, R₂, A, A₁, X, X₁, Y and Z are as described hereinabove for formula I.

Preferred intermediate compounds of formula XLV are those wherein
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally inter-rupted by oxygen, sulfur or nitrogen, and optionally substituted with one to three methyl groups or one or more halogen atoms;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is O, S(O)ₚ or NR₁₃;
X₃ is -C(=A₃)- or -C(=NOR₁₄)-;
R₄, R₅, R₆, R₇, R₈, R₉, R₁₀ and R₁₄ are each independently hydrogen, C₁-C₆alkyl, C₃-C₇cycloalkyl, C₂-C₆alkenyl, C₂-C₆alkynyl or benzyl;
R₁₂ is hydrogen,
   a C₁-C₆alkyl, C₃-C₇cycloalkyl, C₂-C₆alkenyl, C₅-C₆cycloalkenyl or C₂-C₆alkynyl group, wherein each group is optionally substituted with any combination of one to six halogen atoms, one to three C₁-C₂₀alkoxy groups, one or two C₁-C₁₀haloalkoxy groups, one or two NR₁₅R₁₆ groups, one to three S(O)_{q}R₁₇ groups, one or two cyano groups, one or two C(O)R₁₈ groups, one or two CO₂R₁₉ groups, one or two C(O)NR₂₀R₂₁ groups, one to three X₄R₂₂ groups, one or two P(O)(OR₂₃)₂ groups, one or two Si(R₂₄)₃ groups,
   one 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one phenyl group optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group, or
   phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group,
   and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₃ is hydrogen, C₁-C₆alkyl, C₃-C₇cycloalkyl, C₃-C₆alkenyl, C₃-C₆alkynyl, benzyl, OR₂₃, cyano or SO₂R₅₁, and when R₁₃ and R₁₂ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃, C₁-C₂₀alkyl substituted with one C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NHOR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one C₁-C₆alkoxy, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one C₁-C₆alkoxy, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₂-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   C(O)R₄₃;
R₁₆ and R₂₁ are each independently hydrogen, C₁-C₆alkyl, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently C₁-C₄alkyl, C₁-C₄haloalkyl, CO₂R₂₃,
   C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
   Si(R₄₇)₃,
   di(C₁-C₄alkyl)imino,
   C₁-C₂₀alkyl optionally substituted with one CO₂R₄₈, C₁-C₆alkoxy or di(C₁-C₄alkyl)imino group,
   C₁-C₁₅haloalkyl,
   C₃-C₈cycloalkyl,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   2-, 3- or 4-pyridyl,
   2- or 3-furyl,
   2- or 3-thienyl,
   2-tetrahydrofuranyl,
   C₁-C₄alkyl substituted with one pyridyl, furyl, thienyl, tetrahydrofuryl or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups; and
A, A₁, A₃, A₄, X₄ and X₅ are each independently O or S; and
the optical isomers, diastereomers and/or tautomers thereof.

Another group of preferred intermediate compounds of formula XLV are those wherein
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally interrupted by oxygen, sulfur or nitrogen, and optionally substituted with one to three methyl groups or one or more halogen atoms;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is O, S(O)ₚ or NR₁₃;
X₃ is -C(=A₃)-, -C(=NOR₁₄)- or -C(=N-NR₁₃R₅₀) ;
R₄, R₅, R₆, R₇, R₈, R₉, R₁₀ and R₁₄ are each independently hydrogen, C₁-C₆alkyl, C₃-C₇cycloalkyl, C₂-C₈-alkoxyalkyl, C₂-C₆alkenyl, C₂-C₆alkynyl or benzyl;
R₁₂ is hydrogen,
   a C₁-C₈alkyl, C₃-C₇cycloalkyl, C₂-C₈alkenyl, C₅-C₆cycloalkenyl or C₂-C₈alkynyl group, wherein each group is optionally substituted with any combination of one to six halogen atoms, one to three C₁-C₂₀alkoxy groups optionally substituted with one C₁-C₆alkoxy, benzyloxy or C₁-C₆alkylthio group, one or two C₁-C₁₀haloalkoxy groups, one or two NR₁₅R₁₆ groups, one to three S(O)_{q}R₁₇ groups, one or two cyano groups, one or two C₃-C₇cycloalkyl groups, one thiocyano group, one O(SO₂)R₁₇ group, one O(NO₂) group, one or two C(O)R₁₈ groups, one or two CO₂R₁₉ groups, one or two C(O)SR₁₉ groups, one or two C(O)NR₂₀R₂₁ groups, one to three X₄R₂₂ groups, one or two P(O)(OR₂₃)₂ groups, one or two Si(R₂₄)₃ groups,
   one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one or two phenyl groups wherein each phenyl group is independently optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group,
   phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group, or
   indan optionally substituted with any combination of one or two halogen atoms, one or two C₁-C₄alkyl groups, one C₁-C₄alkoxy group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group,
   and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₃ is hydrogen, C₁-C₈alkoxyalkyl, C₁-C₆alkyl, C₃-C₇cycloalkyl, C₃-C₆alkenyl, C₃-C₆alkynyl, benzyl, OR₂₃, cyano, SO₂R₅₁ or one 5- to 12-membered monocyclic or fused bicyclic heterocyclic ring optionally substituted with one or more groups independently selected from halogen, cyano, nitro, amino, hydroxyl, C₁-C₄alkyl, C₁-C₄haloalkyl, C₁-C₄alkoxy or C₁-C₄haloalkoxy, and when R₁₃ and R₁₂ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃, C(O)NR₃₅R₃₆, C(O)R₃₃, SO₂R₄₇,
   C₁-C₂₀alkyl substituted with one hydroxyl, benzyloxy, nitro, OC(O)R₃₃, C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one hydroxyl group, one C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one hydroxyl, C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one hydroxyl, C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₆alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₁₆ and R₂₁ are each independently hydrogen, C₁-C₆alkyl, C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently NR₄₆R₄₈, -N=CR₄₆R₄₈, C₁-C₈alkyl optionally substituted with one C₁-C₆alkoxy, thiophene or furan group, C₁-C₆haloalkyl, C₃-C₈cycloalkyl, CO₂R₂₃, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
   Si(R₄₇)₃,
   di(C₁-C₄alkyl)imino,
   C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)imino group,
   C₁-C₁₅haloalkyl,
   C₃-C₈cycloalkyl optionally substituted with one CO₂R₄₈ group,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   2-, 3- or 4-pyridyl,
   2- or 3-furyl,
   2- or 3-thienyl,
   2-tetrahydrofuranyl,
   C₁-C₄alkyl substituted with one pyridyl, furyl, thienyl, tetrahydrofuryl, C₃-C₈cycloalkyl, dioxane or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups, and
   when R₄₆ and R₄₈ are taken together with the atom to which they are attached, they form a 5- to 12-membered monocyclic or fused bicyclic, heterocyclic or cycloalkyl ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups; and
A, A₁, A₃, A₄, X₄ and X₅ are each independently O or S; and
the optical isomers, diastereomers and/or tautomers thereof.

More preferred formula XLV intermediate compounds of this invention are those wherein
R₁ is halogen or C₁-C₄haloalkyl;
R₂ is hydrogen, halogen or C₁-C₄haloalkyl;
X and X₁ are each independently hydrogen or halogen;
Z is O or S;
Y is cyano, R₁₂, X₂R₁₂ or X₃X₂R₁₂;
X₂ is O, S or NR₁₃;
X₃ is -C(=A₃)-, -C(=NOR₁₄)- or -C(=N-NR₁₃R₅₀);
R₁₂ is hydrogen,
   a C₁-C₆alkyl, C₃-C₇cycloalkyl, C₂-C₆alkenyl, C₅-C₆cycloalkenyl or C₂-C₆alkynyl group, wherein each group is optionally substituted with any combination of one to three halogen atoms, one or two C₁-C₂₀alkoxy groups, one or two C₁-C₁₀haloalkoxy groups, one NR₁₅R₁₆ group, one S(O)_{q}R₁₇ group, one or two cyano groups, one or two C(O)R₁₈ groups, one or two CO₂R₁₉ groups, one or two C(O)SR₁₉ groups, one or two C(O)NR₂₀R₂₁ groups, one or two X₄R₂₂ groups, one P(O)(OR₂₃)₂ group, one Si(R₂₄)₃ group,
   one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
   one quaternary organic ammonium group, or
   one phenyl group optionally substituted with any combination of one to three halogen atoms, one C₁-C₆alkyl group, one C₁-C₆alkoxy group, one C₃-C₇cycloalkyl group, one cyano group, one nitro group, one C(O)R₂₅ group, one CO₂R₂₆ group or one X₅R₂₇ group, or
   phenyl optionally substituted with any combination of one or two halogen atoms, one to three C₁-C₄alkyl groups, one or two C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₂-C₆alkoxyalkyl, C₁-C₄alkyl, C₃-C₇cycloalkyl, C₃-C₆alkenyl, C₃-C₆alkynyl, benzyl, OR₂₃, cyano or SO₂R₅₁;
R₂₈ is halogen;
R₂₂ and R₂₇ are each independently hydrogen, Si(R₃₁)₃, C(O)NR₃₅R₃₆, C(O)R₃₃, SO₂R₄₇,
   C₁-C₂₀alkyl substituted with one hydroxyl, benzyloxy, nitro, OC(O)R₃₃, C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano or 2-dioxolanyl group,
   one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
   C₃-C₂₀alkenyl optionally substituted with one to three halogen atoms, one hydroxyl group, one C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   C₅-C₈cycloalkenyl optionally substituted with one hydroxyl, C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
   C₃-C₁₀alkynyl optionally substituted with one hydroxyl, C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
   benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
   a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
   a C₁-C₆alkyl, C₃-C₇cycloalkyl, C₂-C₆alkenyl or C₂-C₃alkynyl group wherein each group is optionally substituted with one CO₂R₄₂ group,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
   C(O)R₄₃, or
   CO₂R₄₂;
R₁₄ is hydrogen, C₁-C₆alkyl, C₃-C₇cycloalkyl, C₂-C₈alkoxyalkyl, C₂-C₆alkenyl, C₂-C₆alkynyl or benzyl;
R₁₆ and R₂₁ are each independently hydrogen, C₁-C₆alkyl, C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
   benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
   phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₁₇, R₁₈, R₂₅, R₃₀, R₃₃, R₄₅ and R₅₁ are each independently NR₄₆R₄₈, -N=CR₄₆R₄₈, C₁-C₈alkyl optionally substituted with one C₁-C₆alkoxy, thiophene or furan group, C₁-C₆haloalkyl, C₃-C₈cycloalkyl, CO₂R₂₃, C₂-C₆alkenyl optionally substituted with phenyl,
   benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
   phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
   a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
   Si(R₄₇)₃,
   C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)imino group,
   C₁-C₁₅haloalkyl,
   C₃-C₈cycloalkyl optionally substituted with one CO₂R₄₈ group,
   C₃-C₈halocycloalkyl,
   C₃-C₂₀alkenyl optionally substituted with one phenyl group,
   C₃-C₁₅haloalkenyl,
   C₅-C₈cycloalkenyl,
   C₅-C₈halocycloalkenyl,
   C₃-C₂₀alkynyl optionally substituted with one phenyl group,
   C₃-C₂₀haloalkynyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
   C₁-C₄alkyl substituted with one pyridyl, furyl, thienyl, tetrahydrofuryl, C₃-C₈cycloalkyl, dioxane or NR₄₉R₅₀ group, or
   an alkali metal, alkaline earth metal, manganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
q is an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₂-C₆alkenyl, C₃-C₈cycloalkyl,
   C₁-C₈alkyl optionally substituted with one or two C₁-C₄alkoxy groups,
   benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
   phenyl optionally substituted with one or more groups independently selected from halogen, cyano, nitro, C₁-C₄alkyl, C₁-C₄haloalkyl, C₁-C₄alkoxy and C₁-C₄haloalkoxy groups; and
A, A₁, A₃, A₄, X₄ and X₅ are each independently O or S; and
the optical isomers, diastereomers and/or tautomers thereof.

In order to facilitate a further understanding of the invention, the following examples are presented to illustrate more specific details thereof. The invention is not to be limited thereby except as defined in the claims.

### EXAMPLE 1

### Preparation of 2'-Chloro-2-methoxy-5-methyl-5'-nitrobenzophenone

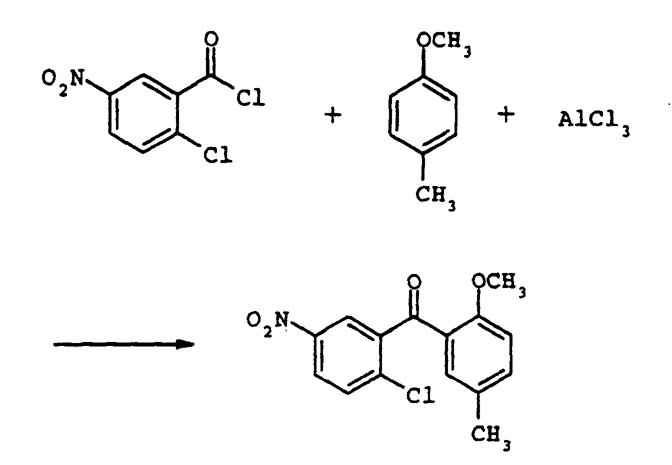

A mixture of aluminum chloride (33.3 g, 25.0 mmol) in methylene chloride is cooled to about 5 °C, treated over one hour with *p*-methylanisole (31.6 g, 25.0 mmol) while maintaining the reaction mixture temperature below 10 °c, treated over 20 minutes with a solution of 2-chloro-5-nitrobenzoyl chloride (50.0 g, 22.7 mmol) in methylene chloride while maintaining the reaction mixture temperature below 10 °C, warmed to and stirred at room temperature for 60 minutes, and poured onto ice. The resultant aqueous mixture is treated with concentrated hydrochloric acid (50 mL) and extracted with methylene chloride. The organic extract is dried over anhydrous magnesium sulfate and concentrated *in vacuo* to give a yellow solid. After placing the solid in a Kugelrohr apparatus at 40 °C to remove residual *p*-methylanisole, the title product is obtained as a beige solid (68.8 g, 99.1%) which is identified by NMR spectral analyses.

Using essentially the same procedure, the following compounds are obtained:

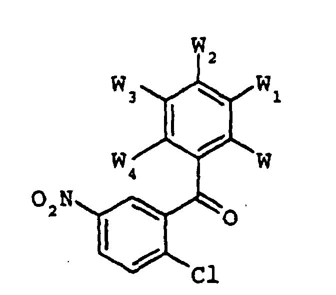

| **W** | **W**_{**1**} | **W**_{**2**} | **W**_{**3**} | **W**_{**4**} | **mp °C** |
|---|---|---|---|---|---|
| H | I | H | H | OCH₃ | 115-116.5 |
| H | H | CH₃ | H | OCH₃ | |
| H | H | C₂H₅ | H | H | |
| H | CH₃ | CH₃ | H | OCH₃ | |
| H | H | OCH₃ | H | H | 108-112 |
| H | C₂H₅ | H | H | OCH₃ | 98-99.5 |
| H | H | OCH₃ | H | CH₃ | 91-92 |
| H | H | CH₃ | H | H | 95.5-96.5 |
| H | H | SCH₃ | H | H | 127-128 |
| H | H | CH₃ | H | OCH₃ | 91-92.5 |
| H | H | C₂H₅ | H | H | |
| H | H | Cl | H | H | 88.5-90.5 |
| H | H | F | H | H | 68-69.5 |
| H | Cl | H | H | OCH₃ | 124-126 |
| H | OCH₃ | H | H | OCH₃ | 71-73 |
| H | H | OCH₃ | H | OCH₃ | 98-100 |
| H | CH₃ | CH₃ | H | OCH₃ | 127-129 |
| H | H | Cl | H | OCH₃ | 96-99 |
| CH₃ | H | CH₃ | H | OCH₃ | 108.5-110 |
| H | H | H | CH ₃ | OCH₃ | 71-74 |
| H | H | N(CH₃)SO₂CH₃ | H | H | |
| H | CH₃ | Cl | H | OCH₃ | 126-128 |
| H | CH₃ | H | CH₃ | OCH₃ | 110-112 |
| CH₃ | CH₃ | CH₃ | H | OCH₃ | 104-106 |
| H | CH(CH₃)₂ | H | H | OCH₃ | 69-71 |
| H | CH₃ | H | H | H | |
| H | H | H | H | CN | |
| H | H | H | H | OCH₃ | |
| H | OCH₃ | H | H | H | |
| H | F | H | H | OCH₃ | |
| H | H | F | H | OCH₃ | |
| H | H | H | H | SCH₃ | |
| H | H | H | H | CH₃ | |
| H | H | H | H | F | |
| H | SCH₃ | H | H | H | |
| H | H | OCH₃ | H | H | |
| H | -(CH₂)₃- | | H | OCH₃ | |

### EXAMPLE 2

### Preparation of 3-(6-Methoxy-m-tolyl)-5-nitro-1,2-benzisothiazole

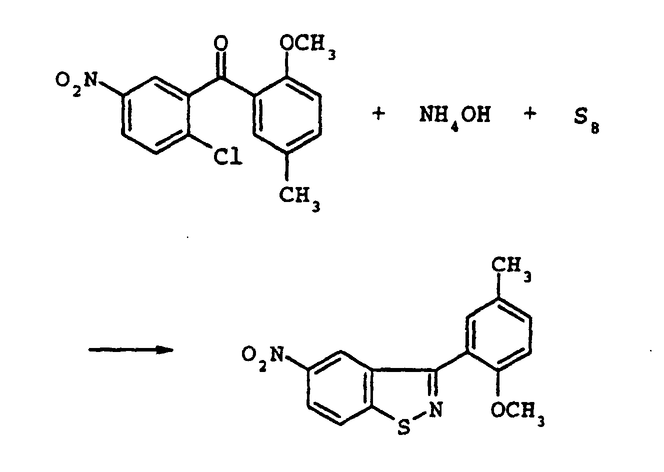

Ammonium hydroxide (350 mL of a 30% solution, 270 mmol) is added to a mixture of 2'-chloro-2-methoxy-5-methyl-5'-nitrobenzophenone (68.7 g, 22.5 mmol) and sulfur (7.57 g, 23.6 mmol) in N,N-dimethylformamide. The resultant reaction mixture is stirred at 80 °C for 19.5 hours, cooled to 40 °C, treated with additional ammonium hydroxide (50 mL of a 30% solution), stirred at 80 °C for 25 hours, cooled, and poured onto ice. The resultant aqueous mixture is filtered to obtain the title product as a yellow solid (63.5 g, 93.9%) which is identified by NMR spectral analyses.

Using essentially the same procedure, the following compounds are obtained:

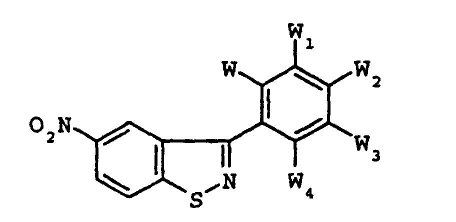

| **W** | **W**_{**1**} | **W**_{**2**} | **W**_{**3**} | **W**_{**4**} | **mp °C** |
|---|---|---|---|---|---|
| H | H | CH₃ | H | OCH₃ | 201-203 |
| H | CH₃ | CH₃ | H | OCH₃ | 199-200 |
| H | CH₃ | H | H | H | 116.5-117.5 |
| H | H | Cl | H | OCH₃ | 229-231 |
| H | H | H | CH₃ | OCH₃ | 134-136 |
| H | H | H | H | CN | 187.5-189 |
| H | H | H | H | OCH₃ | 193-198 |
| H | H | OCH₃ | H | H | 201-203 |
| H | OCH₃ | H | H | H | 174-175 |
| H | F | H | H | OCH₃ | 224-226 |
| H | C₂H₅ | H | H | OCH₃ | 153-154.5 |
| H | H | CH₃ | H | H | 188-189 |
| H | H | N(CH₃)SO₂CH₃ | H | H | |
| H | CH₃ | Cl | H | OCH₃ | 230-234 |
| H | I | H | H | OCH₃ | |
| H | H | SCH₃ | H | H | 177.5-178.5 |
| H | H | OCH₃ | H | CH₃ | 131-135 |
| H | H | F | H | H | 226-228 |
| H | H | Cl | H | H | 217.5-219 |
| H | H | F | H | OCH₃ | 224-225 |
| H | H | H | H | SCH₃ | 114.5-115.5 |
| H | H | CH₃ | H | OCH₃ | 201-203 |
| H | OCH₃ | H | H | OCH₃ | 195-196 |
| H | H | H | H | CH₃ | 145-146 |
| H | H | H | H | F | 181-182 |
| H | H | OCH₃ | H | OCH₃ | 171-172.5 |
| H | SCH₃ | H | H | H | 139-140.5 |
| H | CH₃ | H | CH₃ | OCH₃ | |
| CH₃ | CH₃ | CH₃ | H | OCH₃ | |
| H | CH(CH₃)₂ | H | H | OCH₃ | |
| H | H | CH₃ | CH₃ | OCH₃ | |
| H | -(CH₂)₃- | | H | OCH₃ | |

### EXAMPLE 3

### Preparation of 3-Methyl-5-nitro-1,2-benzisothiazole

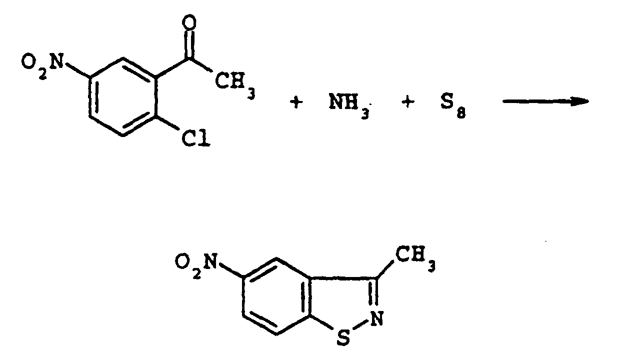

Ammonia (45 g, 2,642 mmol) is bubbled into methanol at -40 °C in a steel bomb. Sulfur (30.5 g, 95.0 mmol) and 2'-chloro-5'-nitroacetophenone (19 g, 95.0 mmol) are then added. The bomb is sealed and heated at about 90 °C overnight. After cooling, the reaction mixture is removed from the bomb and concentrated *in vacuo* to obtain a residue. The residue is diluted with methylene chloride, passed through a plug of silica gel and concentrated *in vacuo* to give the title product as an orange solid (12.0 g) which is identified by NMR spectral analyses.

Using essentially the same procedure, the following compounds are obtained:

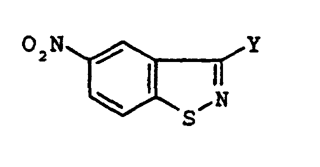

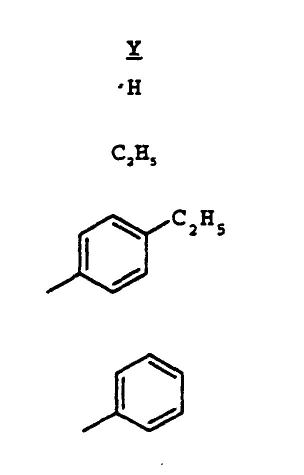

### EXAMPLE 4

### Preparation of 5-Amino-3-(6-methoxy-m-tolyl)-1,2-benzisothiazole

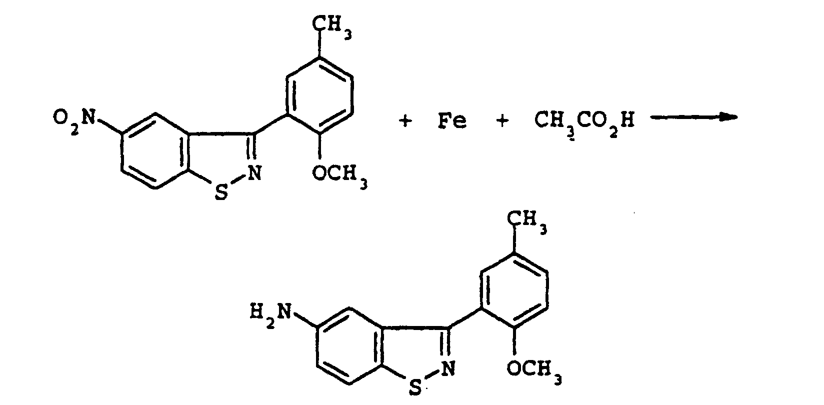

A mixture of 3-(6-methoxy-*m*-tolyl)-5-nitro-1,2-benzisothiazole (63.0 g, 0.210 mol), 5% acetic acid (1.52 L, 1.21 mol) and ethyl acetate (975 mL) is heated to 65 °C, treated portionwise with iron powder (58.6 g, 1.05 mol), stirred at 65 °C, and filtered through quartz filter paper. The filtrate phases are separated and the aqueous phase is extracted with ethyl acetate. The organic phase and extracts are combined, washed sequentially with water and brine, dried over anhydrous magnesium sulfate, and concentrated *in vacuo* to obtain the title product as an orange oil (55.7 g, 98.1%) which is identified by NMR spectral analyses.

Using essentially the same procedure, the following compounds are obtained:

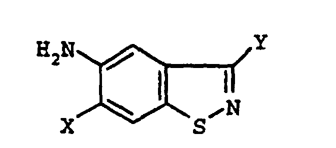

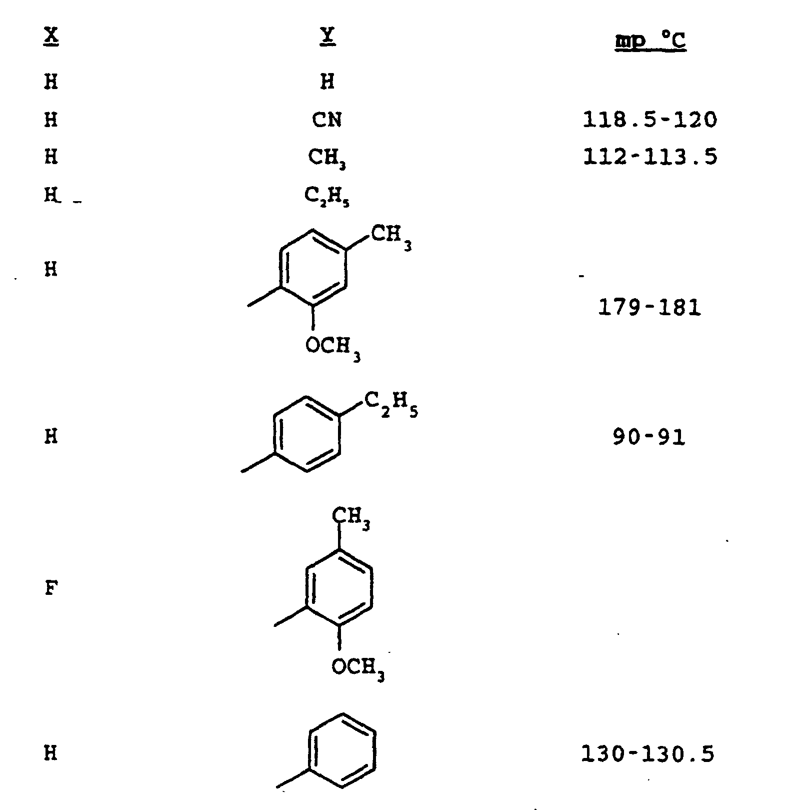

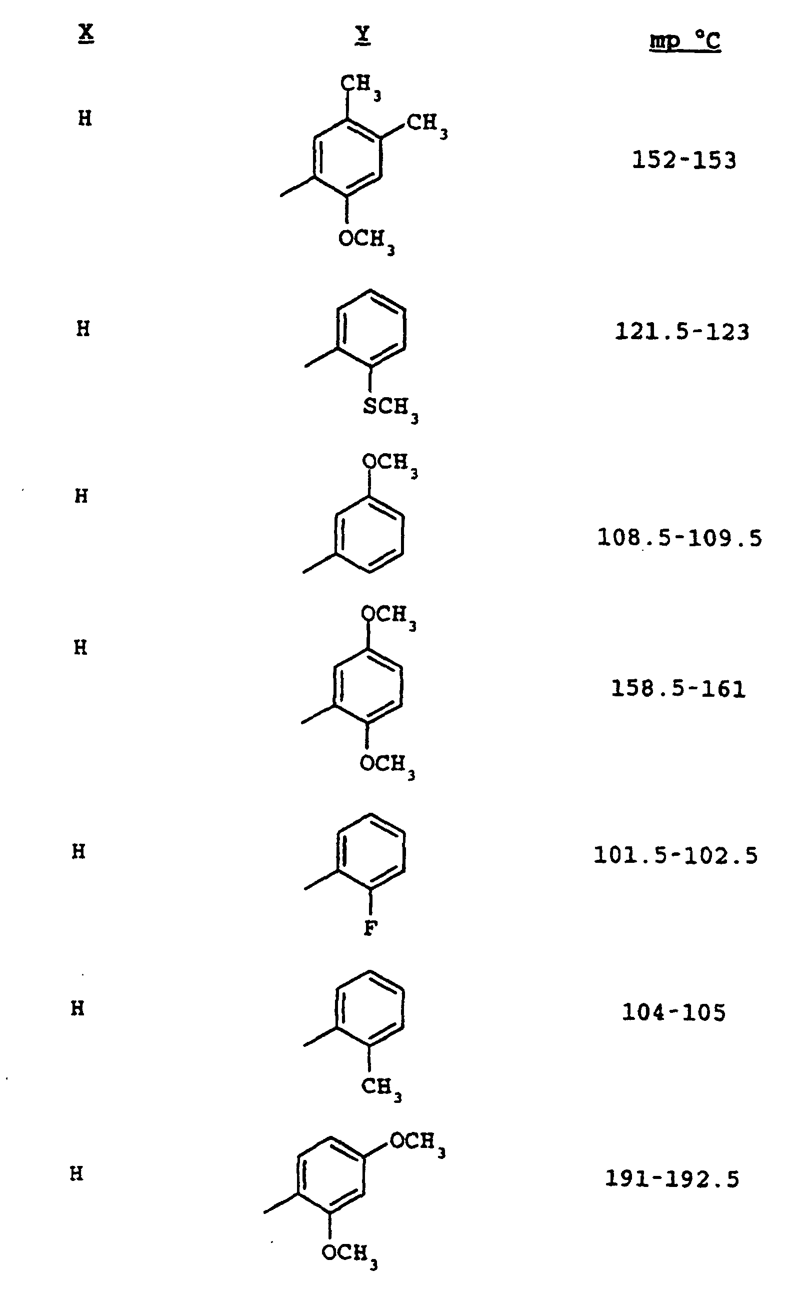

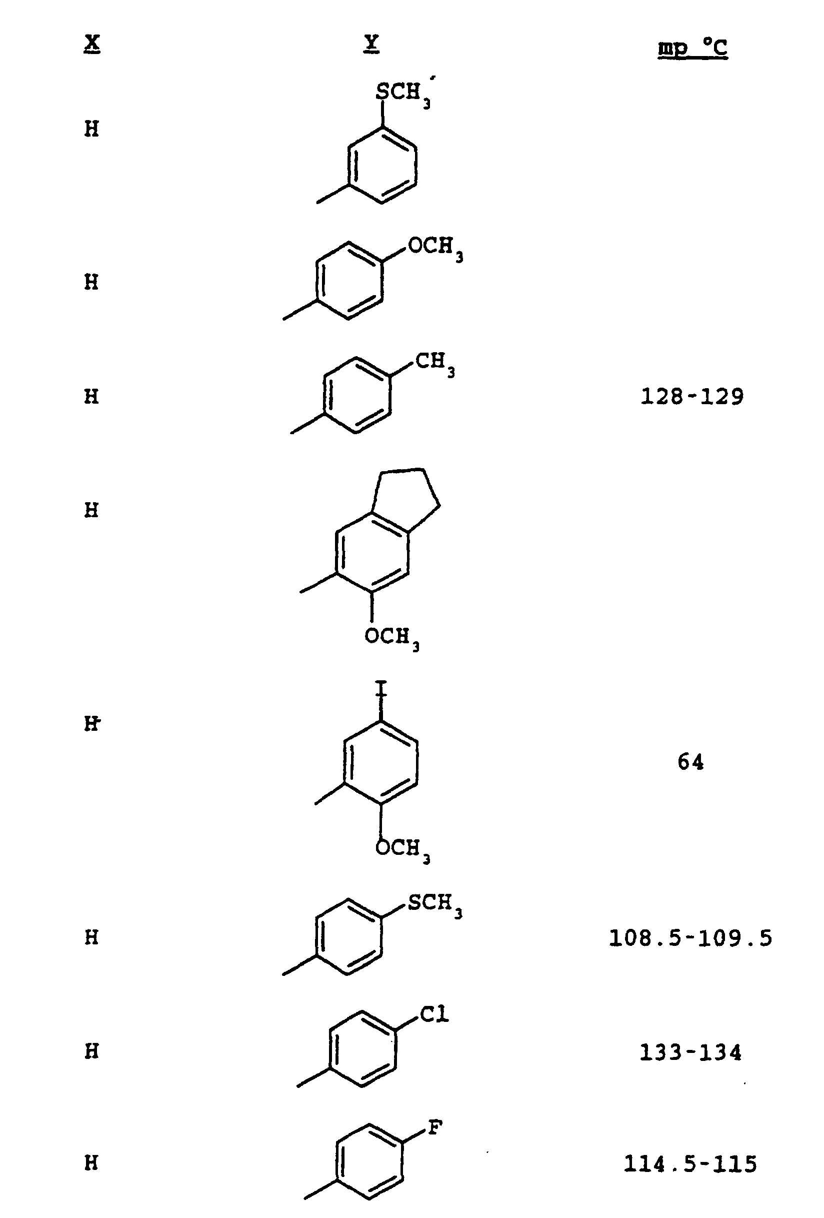

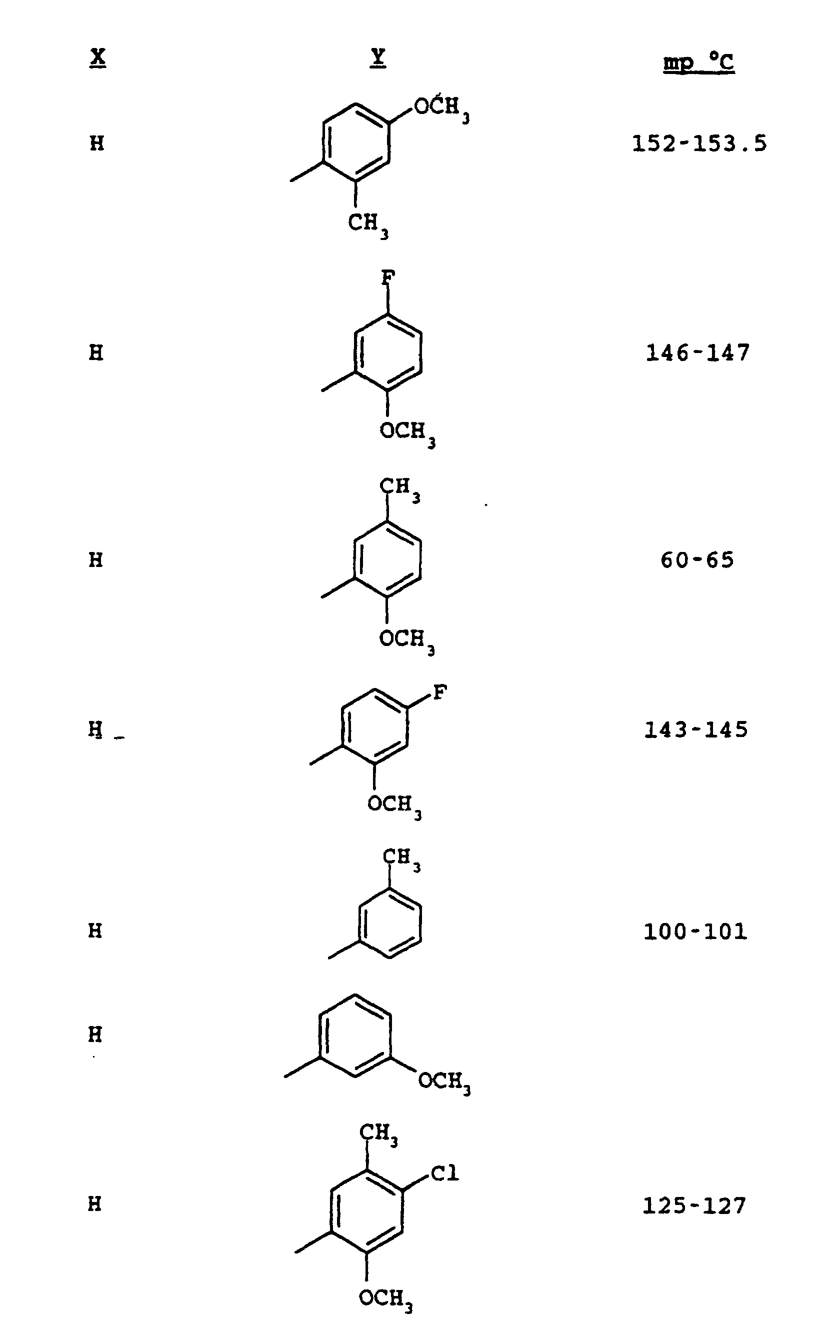

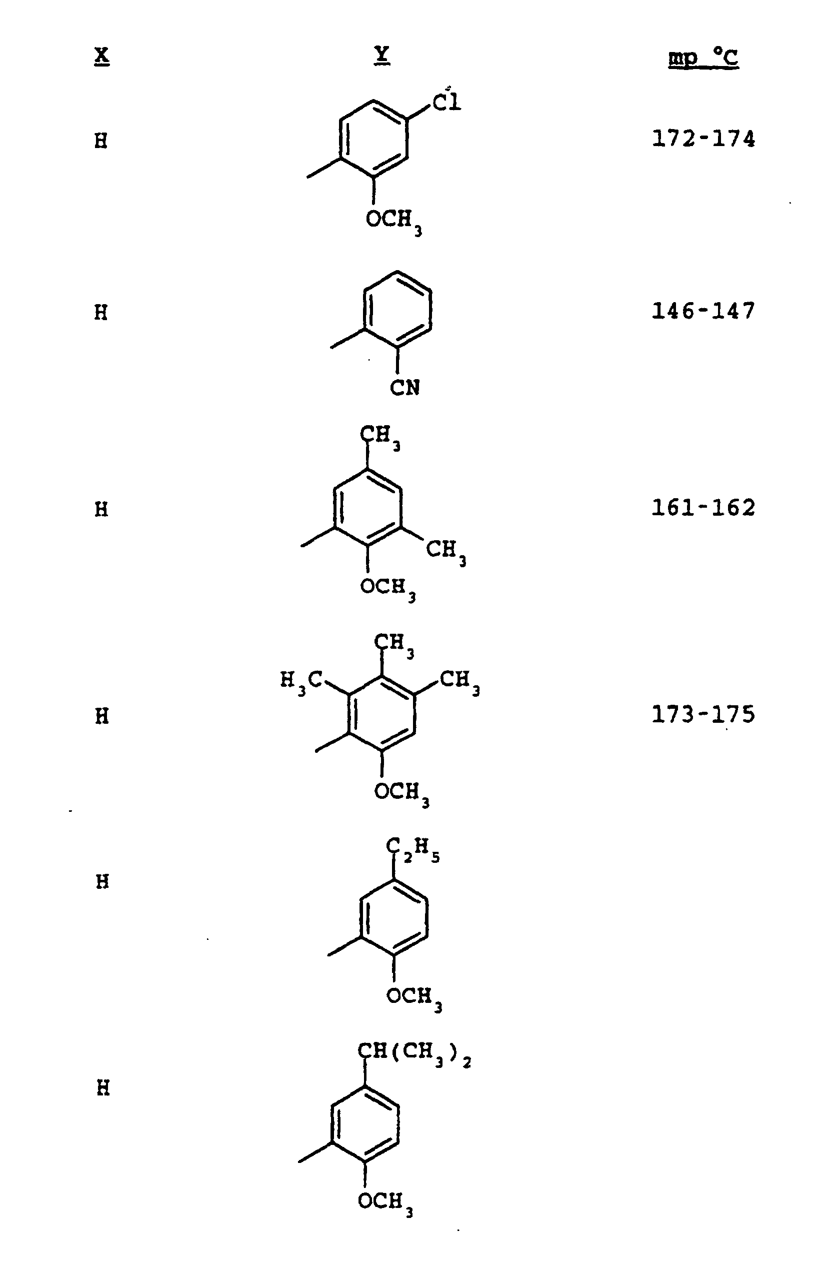

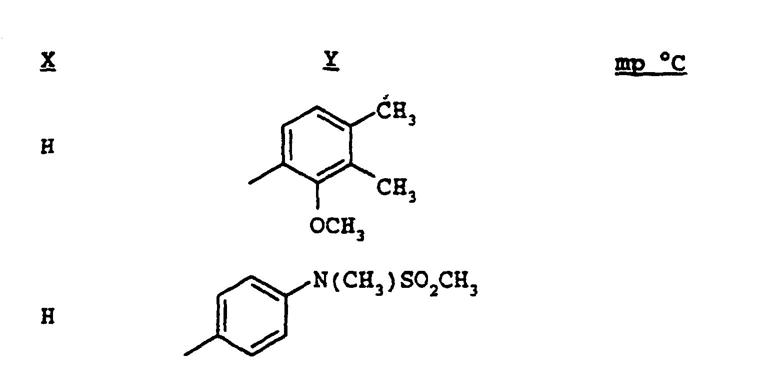

### EXAMPLE 5

### Preparation of 3-[3-(6-Methoxy-3,4-xylyl)-1,2-benzisothiazol-5-yl]-6-(trifluoromethyl)-2,4(1H,3H)-pyrimidinedione

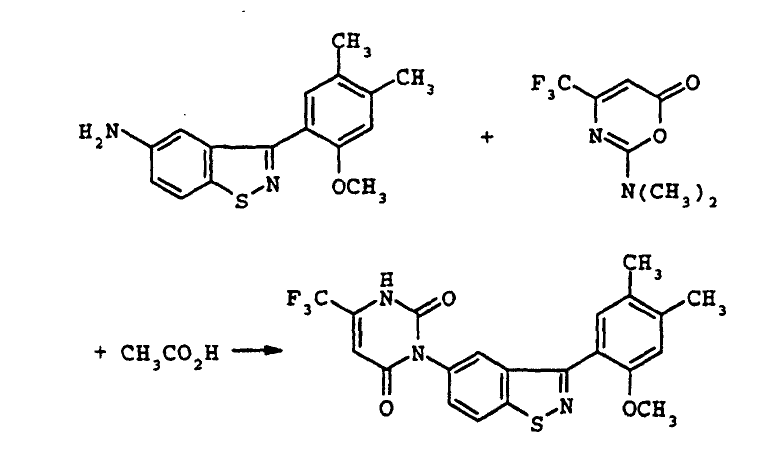

A mixture of 5-amino-3-(6-methoxy-3,4-xylyl)-1,2-benzisothiazole (8.53 g, 30.0 mmol), 2-dimethylamino-4-(trifluoromethyl)-6H-1,3-oxazin-6-one (6.87 g, 33.0 mmol) and acetic acid is refluxed for 2 hours, cooled, and poured into water. The resultant aqueous mixture is filtered to obtain a solid. A solution of the solid in methylene chloride is washed sequentially with water and brine, dried over anhydrous magnesium sulfate, and concentrated *in vacuo* to obtain an oil. Column chromatography of the oil using silica gel and a 5% diethyl ether in methylene chloride solution gives the title product as a yellow foam (8.37 g, 62.0%) which is identified by NMR spectral analyses.

Using essentially the same procedure, the following compounds are obtained:

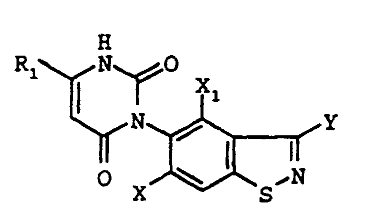

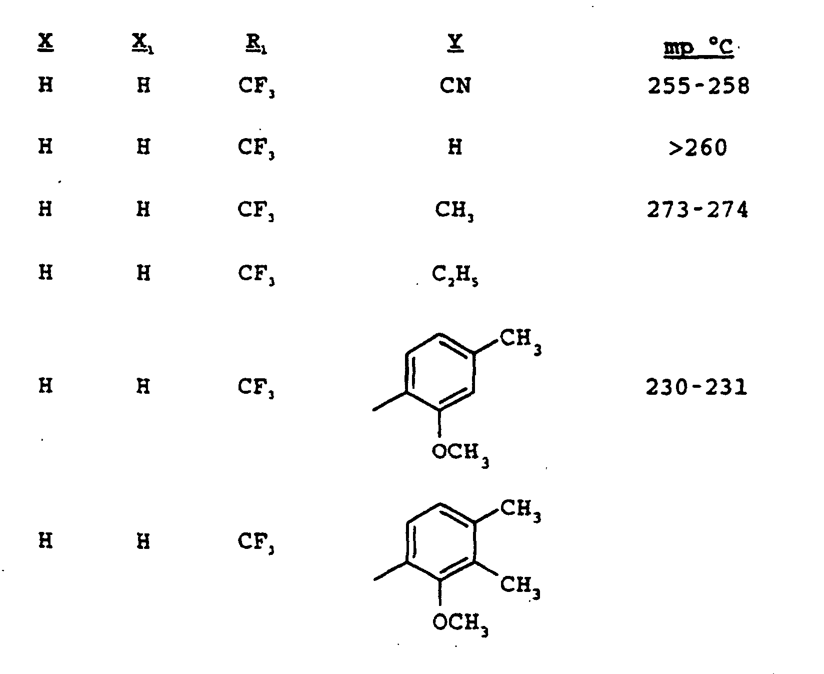

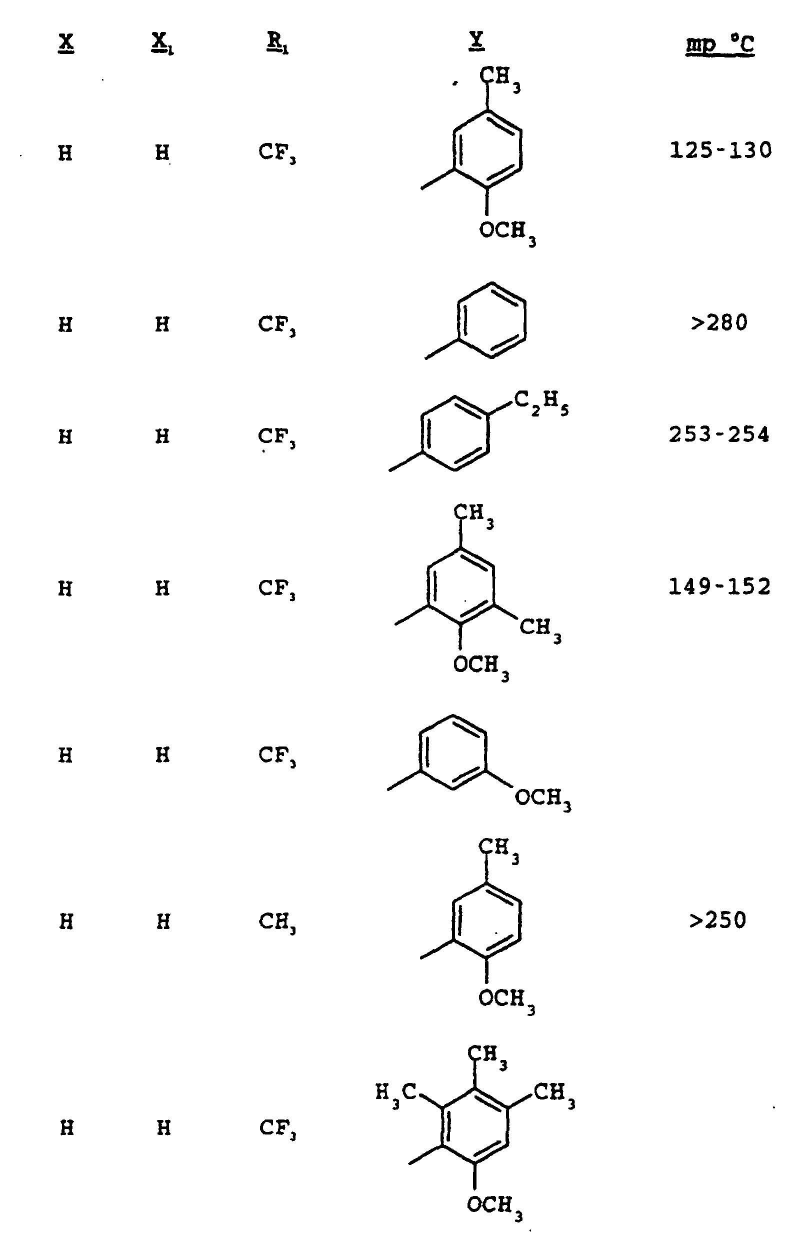

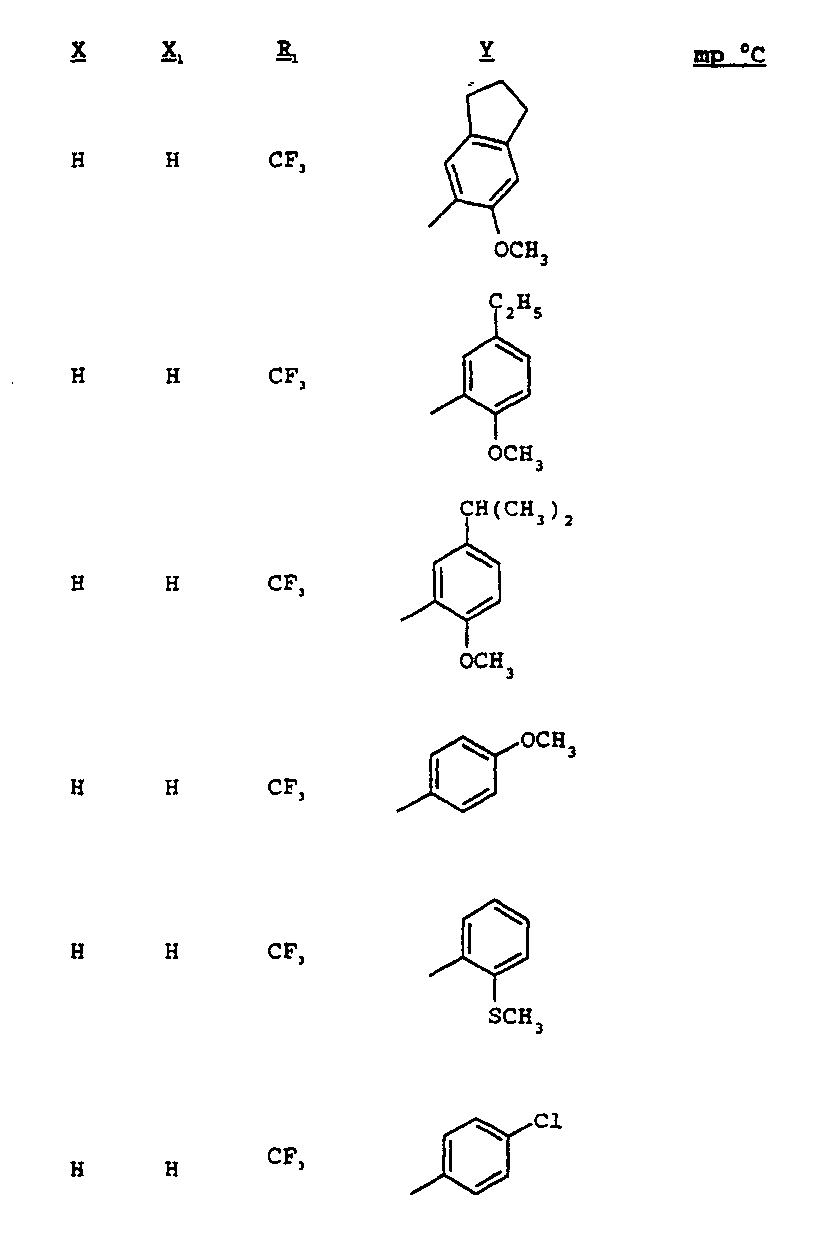

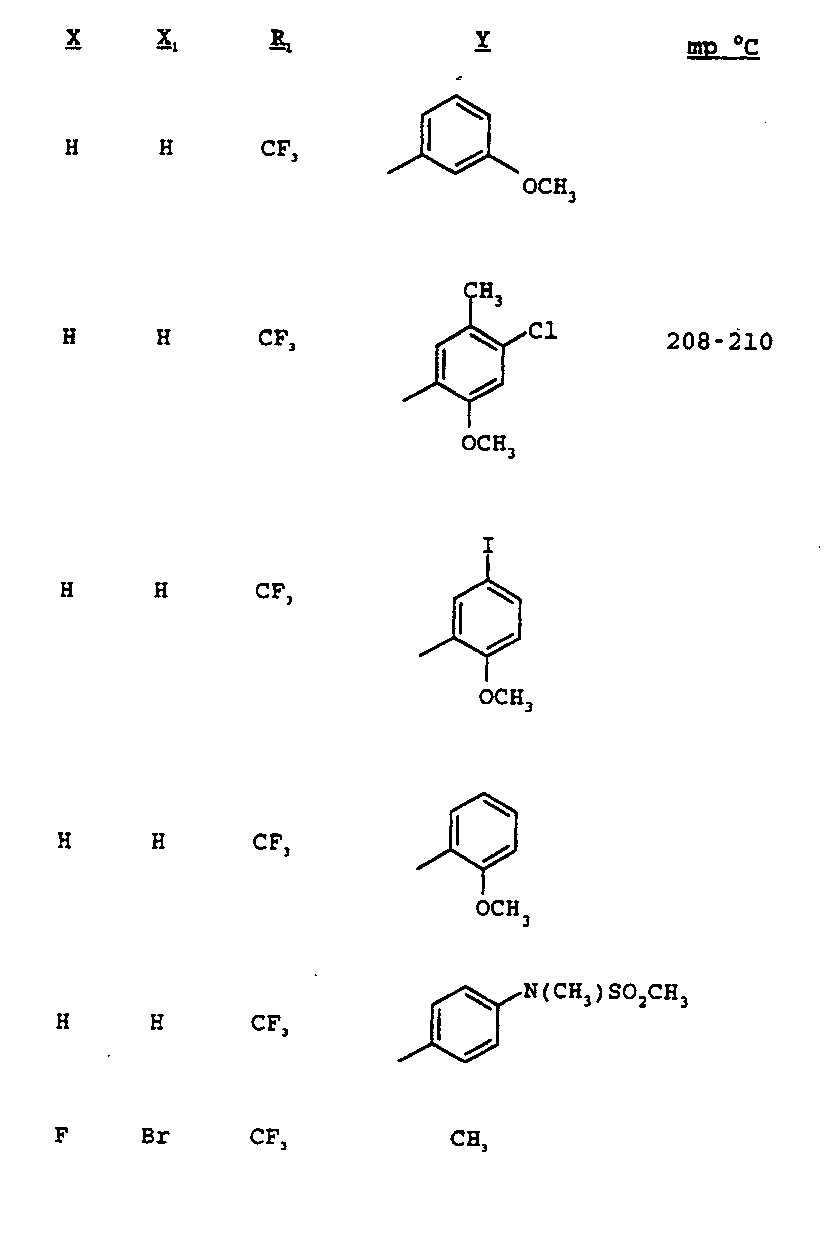

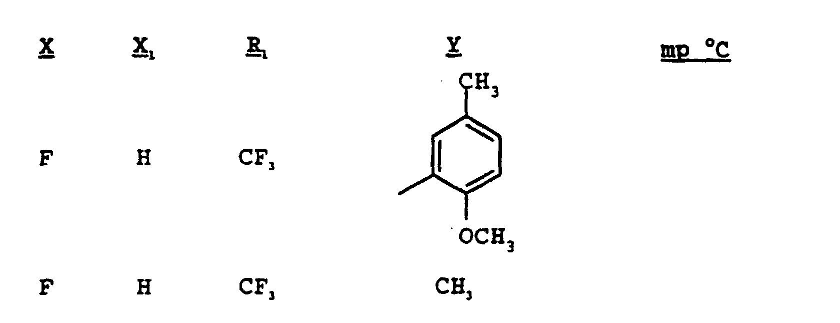

### EXAMPLE 6

### Preparation of 3-(6-Methoxy-m-tolyl)-1,2-benzisothiazol-5-yl isocyanate

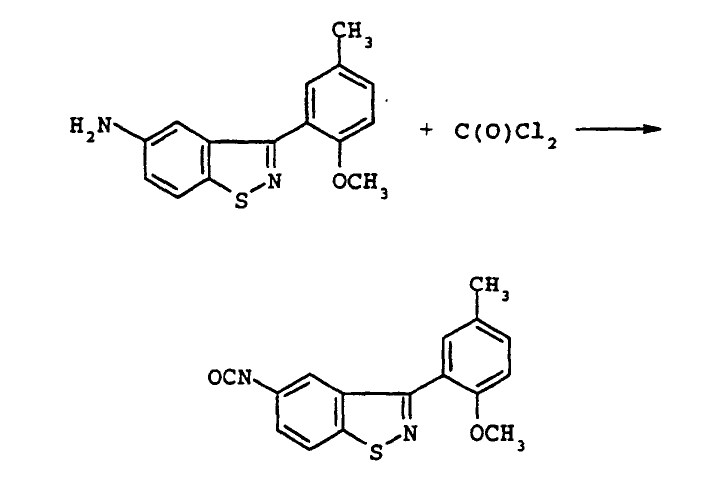

A solution of 5-amino-3-(6-methoxy-*m*-tolyl)-1,2-benzisothiazole (157 g, 0.581 mol) in toluene is treated with phosgene (1,074 mL of a 20% solution in toluene, 2.06 mol), heated to and stirred at reflux for 5 hours, treated with additional phosgene (100 mL of a 20% solution in toluene), refluxed overnight, and concentrated *in vacuo* to give the title product as a yellow-orange solid (169 g, 98.2%) which is identified by NMR spectral analyses.

Using essentially the same procedure, but using phosgene or thiophosgene, the following compounds are obtained:

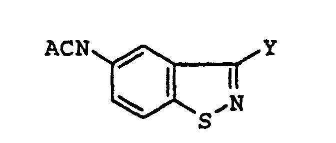

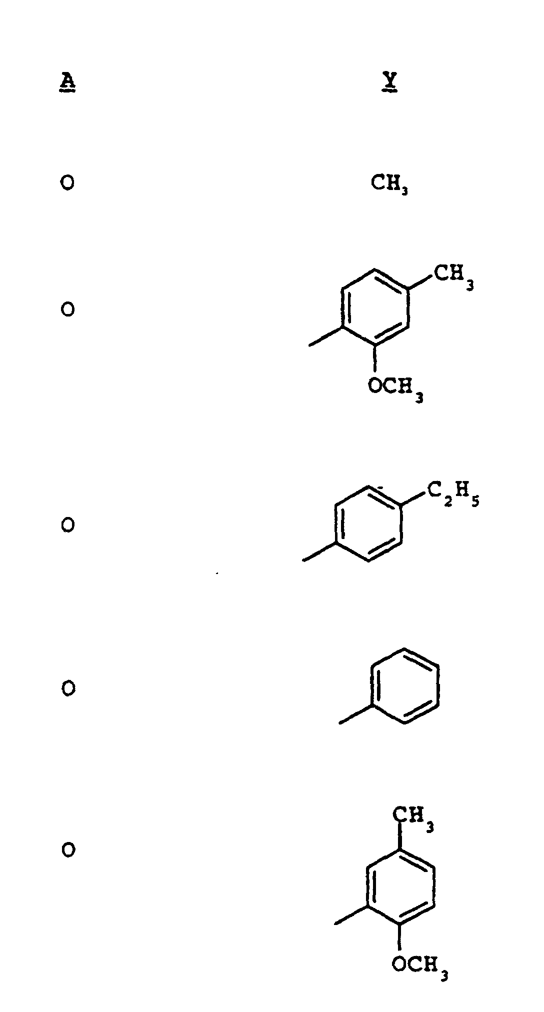

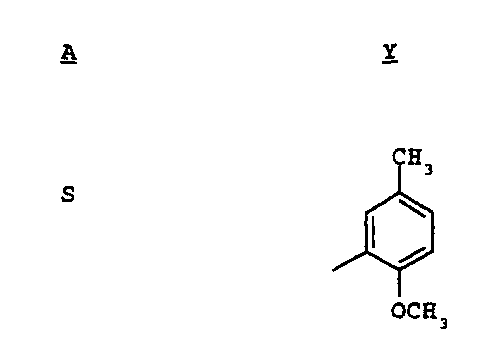

### EXAMPLE 7

### Preparation of 3-[3-(6-Methoxy-m-tolyl)-1,2-benzisothiazol-5-yl]-6-(trifluoromethyl)-2,4(1H,3H)-pyrimidinedione

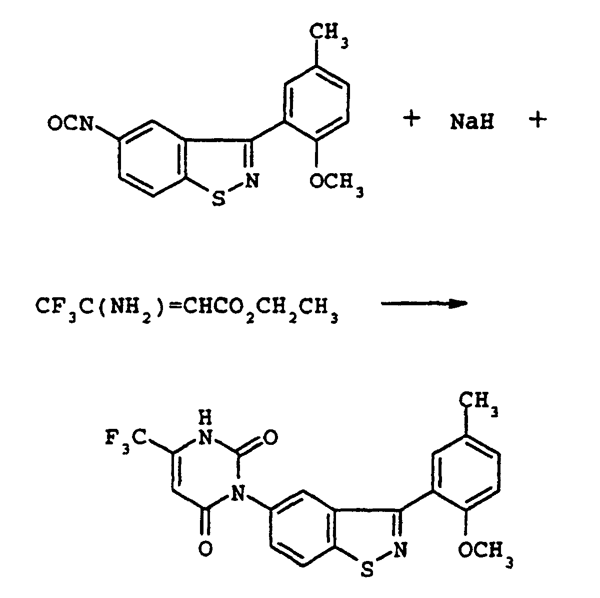

A mixture of 60% sodium hydride in mineral oil (15.9 g, 0.397 mol) and N,N-dimethylformamide is cooled to -10 °C, treated over one hour with a solution of ethyl 3-amino-4,4,4-trifluorocrotonate (72.7 g, 0.397 mol) in ether, warmed to and stirred at room temperature for 90 minutes, cooled to -70 °c, treated over 80 minutes with a solution of 3-(6-methoxy-*m*-tolyl)-1,2-benzisothiazol-5-yl isocyanate (107 g, 0.361 mol) in N,N-dimethylformamide while maintaining the temperature below about -65 °C, slowly warmed to and stirred at room temperature overnight, and poured onto ice. The resultant aqueous mixture is washed with ethyl acetate, acidified to pH 2 with concentrated hydrochloric acid, and extracted with diethyl ether. The organic extract is dried over anhydrous magnesium sulfate and concentrated *in vacuo* to give the title product as an orange oil (110 g, 70.5%) which is identified by NMR spectral analyses.

Using essentially the same procedure, the following compounds are obtained:

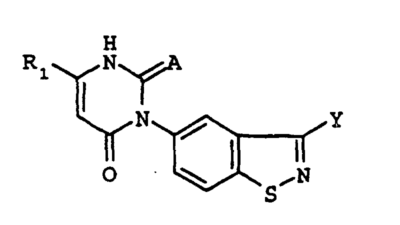

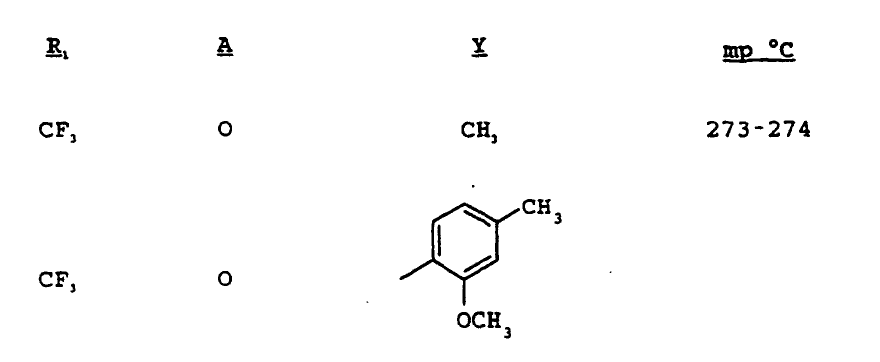

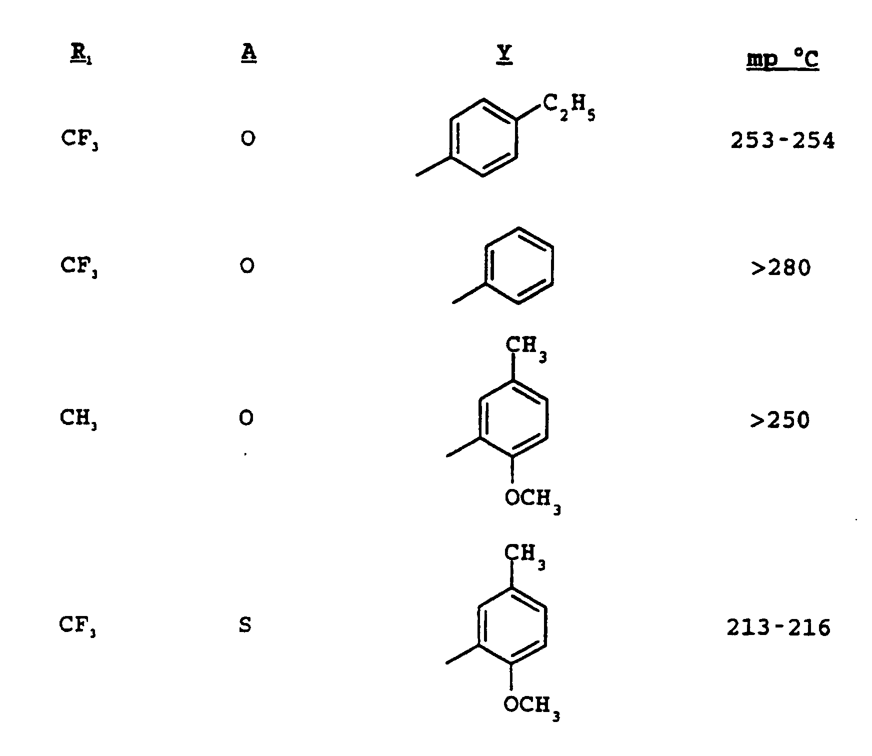

### EXAMPLE 8

### Preparation of 3-[3-(6-Methoxy-m-tolyl)-1,2-benzisothiazol-5-yl]-1-methyl-6-(trifluoromethyl)-2,4(1H,3H)-pyrimidinedione

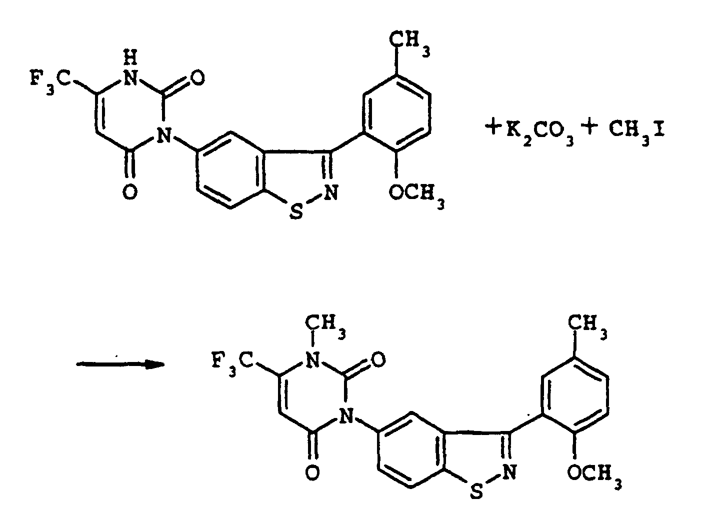

A mixture of 3-[3-(6-methoxy-*m*-tolyl)-1,2-benzisothiazol-5-yl]-6-(trifluoromethyl)-2,4(1H,3H)-pyrimidinedione (160 g, 0.369 mol), potassium carbonate (76.6 g, 0.554 mol) and iodomethane (34.5 mL, 0.554 mol) in N,N-dimethylformamide is stirred at room temperature for 4 hours, and poured onto ice. The resultant aqueous mixture is extracted with methylene chloride. The organic extract is diluted with hexanes, washed with water, dried over anhydrous magnesium sulfate, and concentrated *in vacuo* to obtain the title product as an orange foam (163 g, 98.8%) which is identified by NMR spectral analyses.

Using essentially the same procedure, the following compounds are obtained:

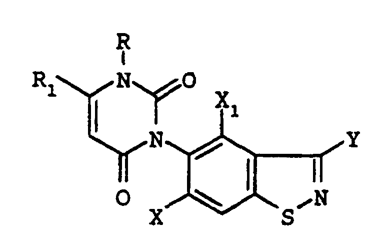

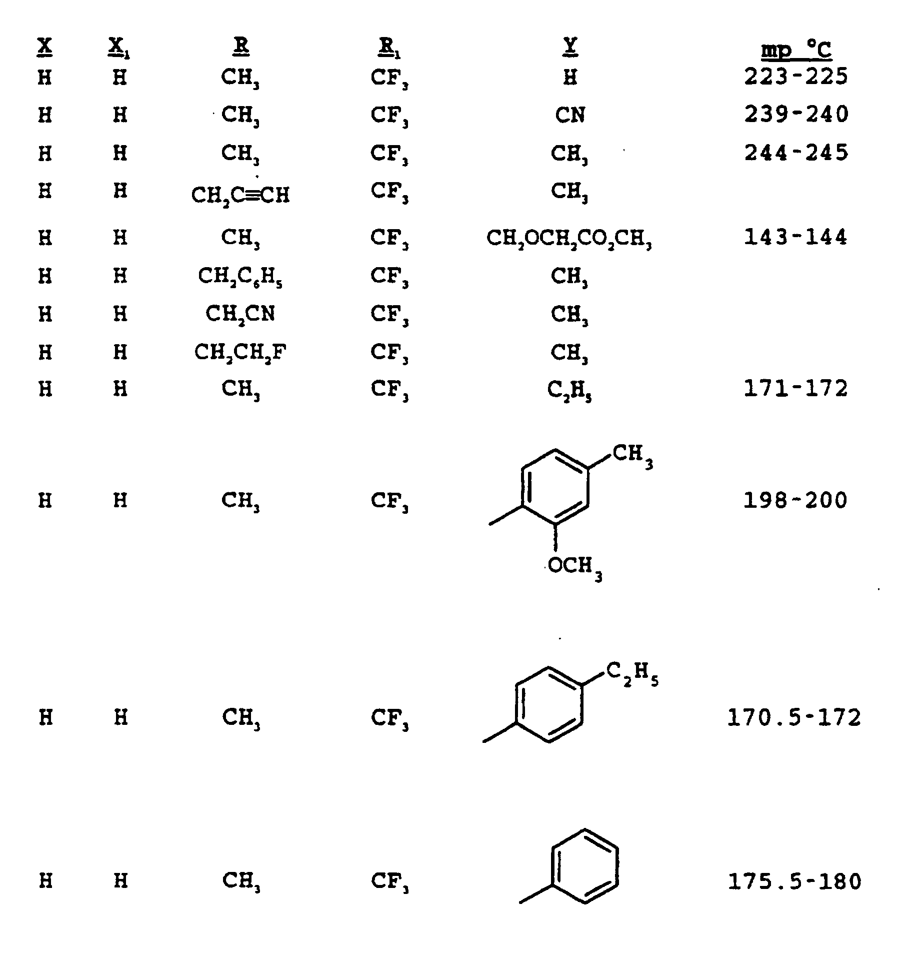

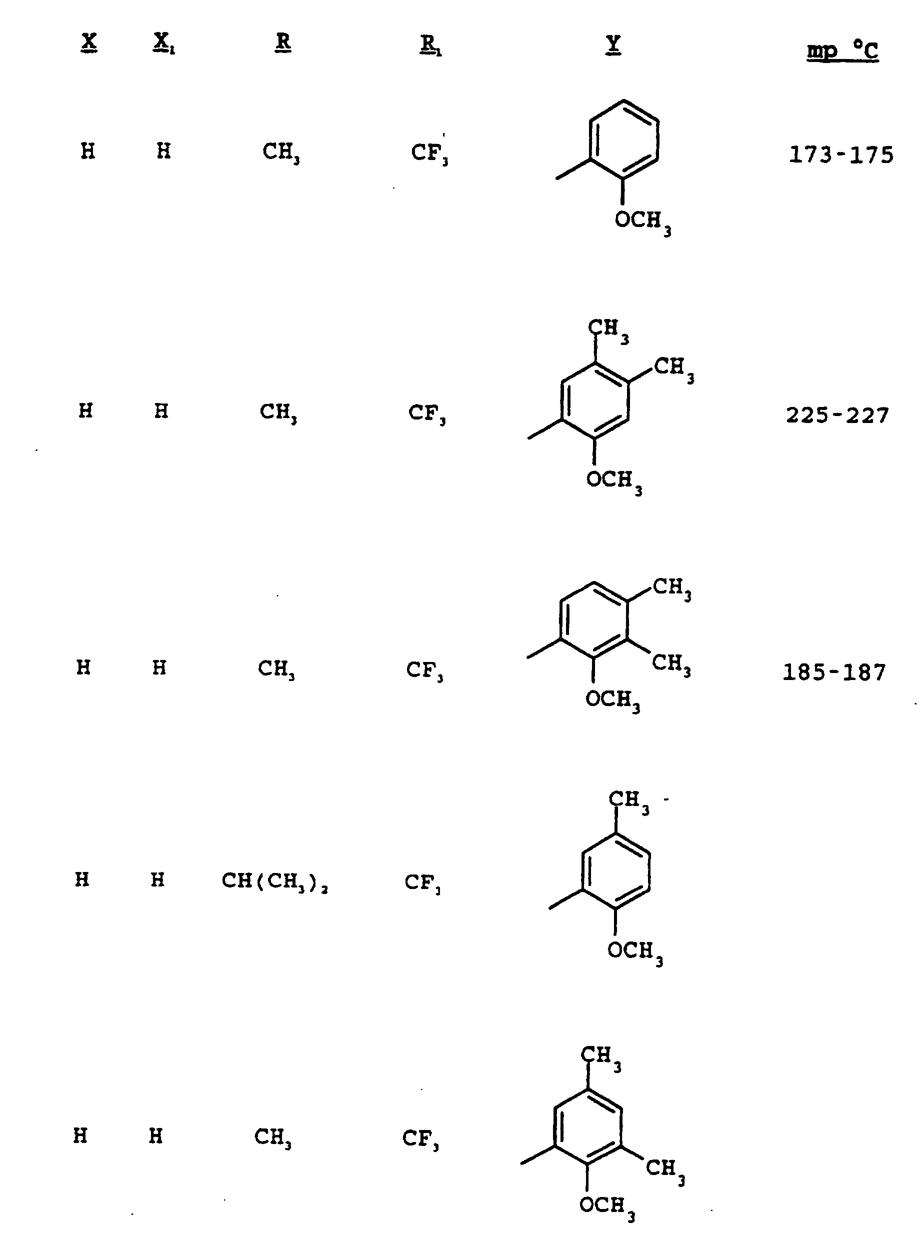

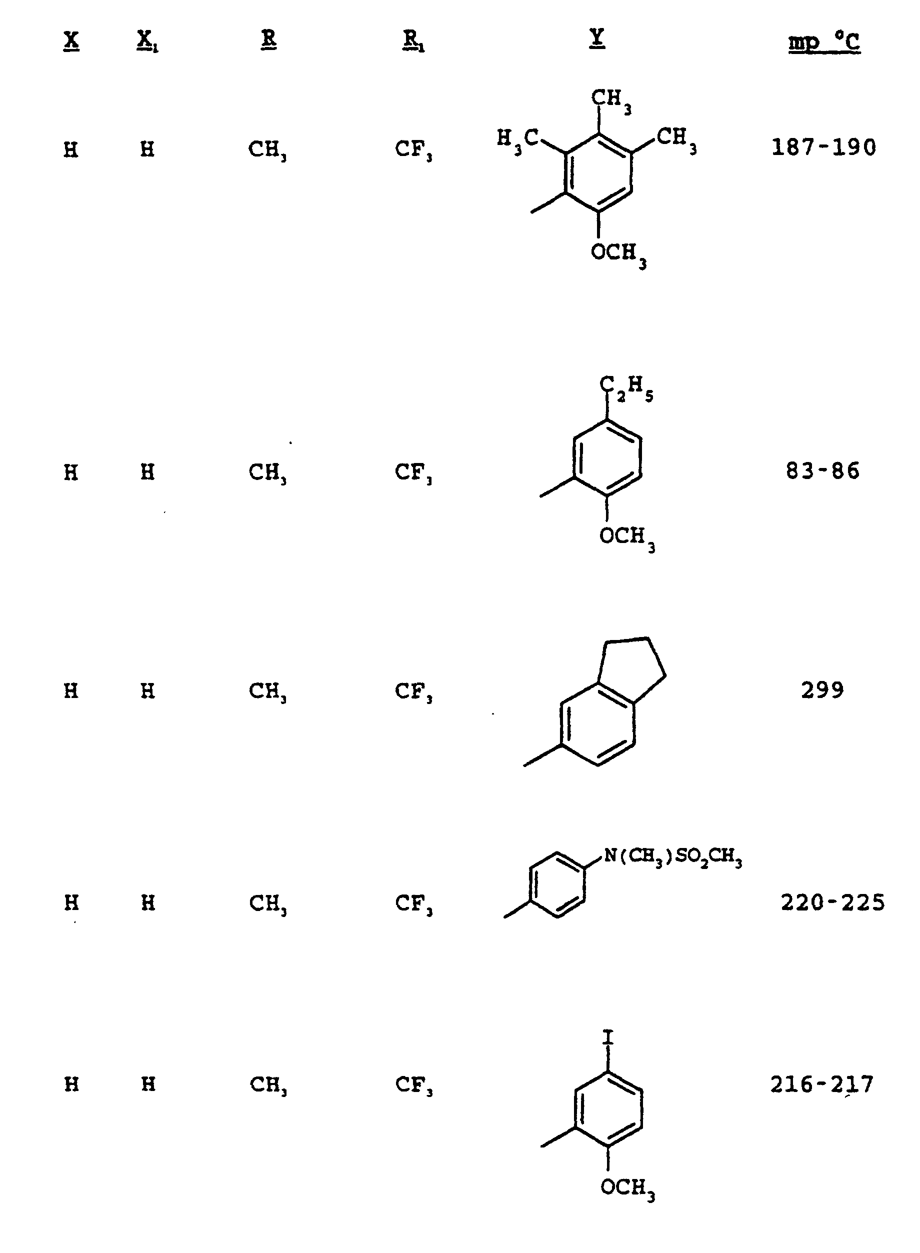

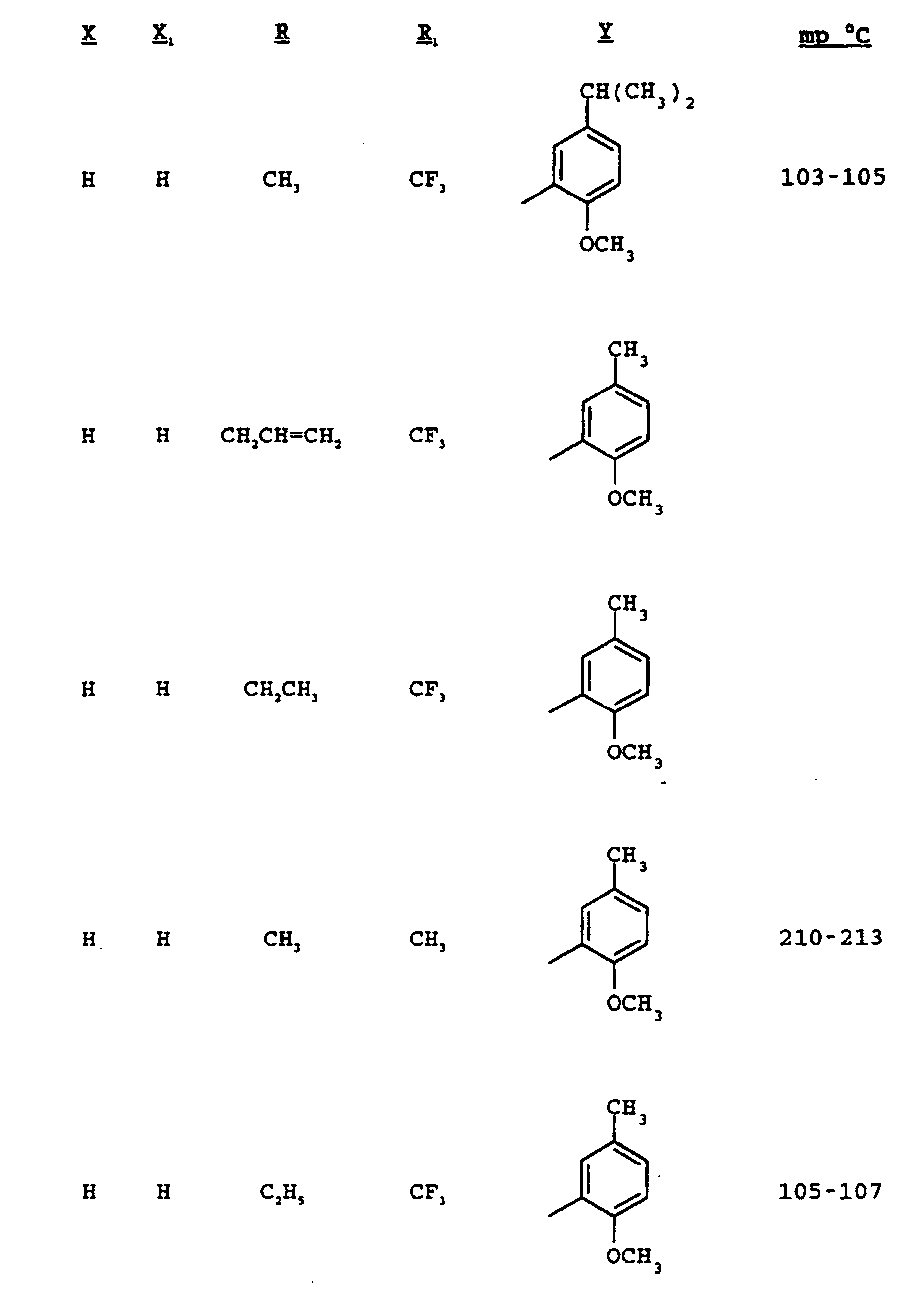

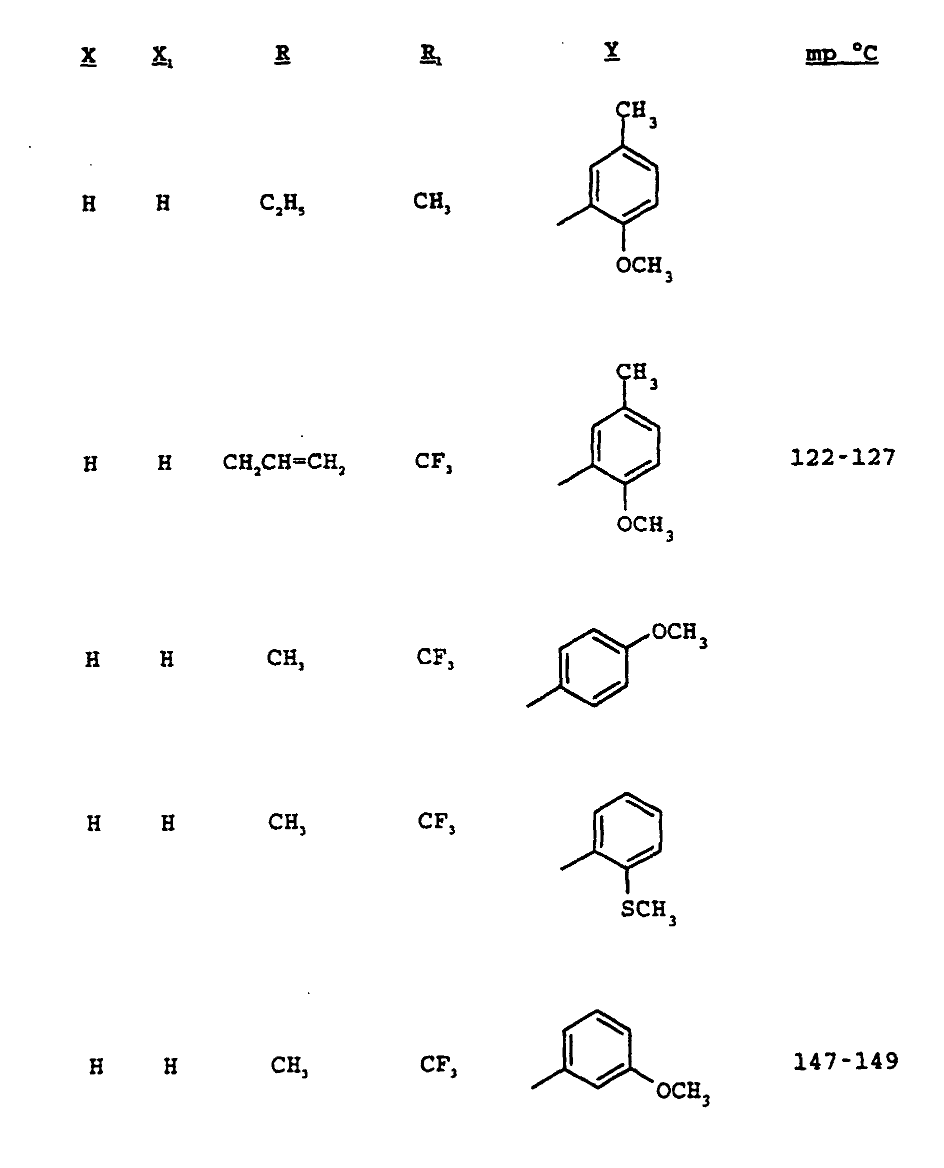

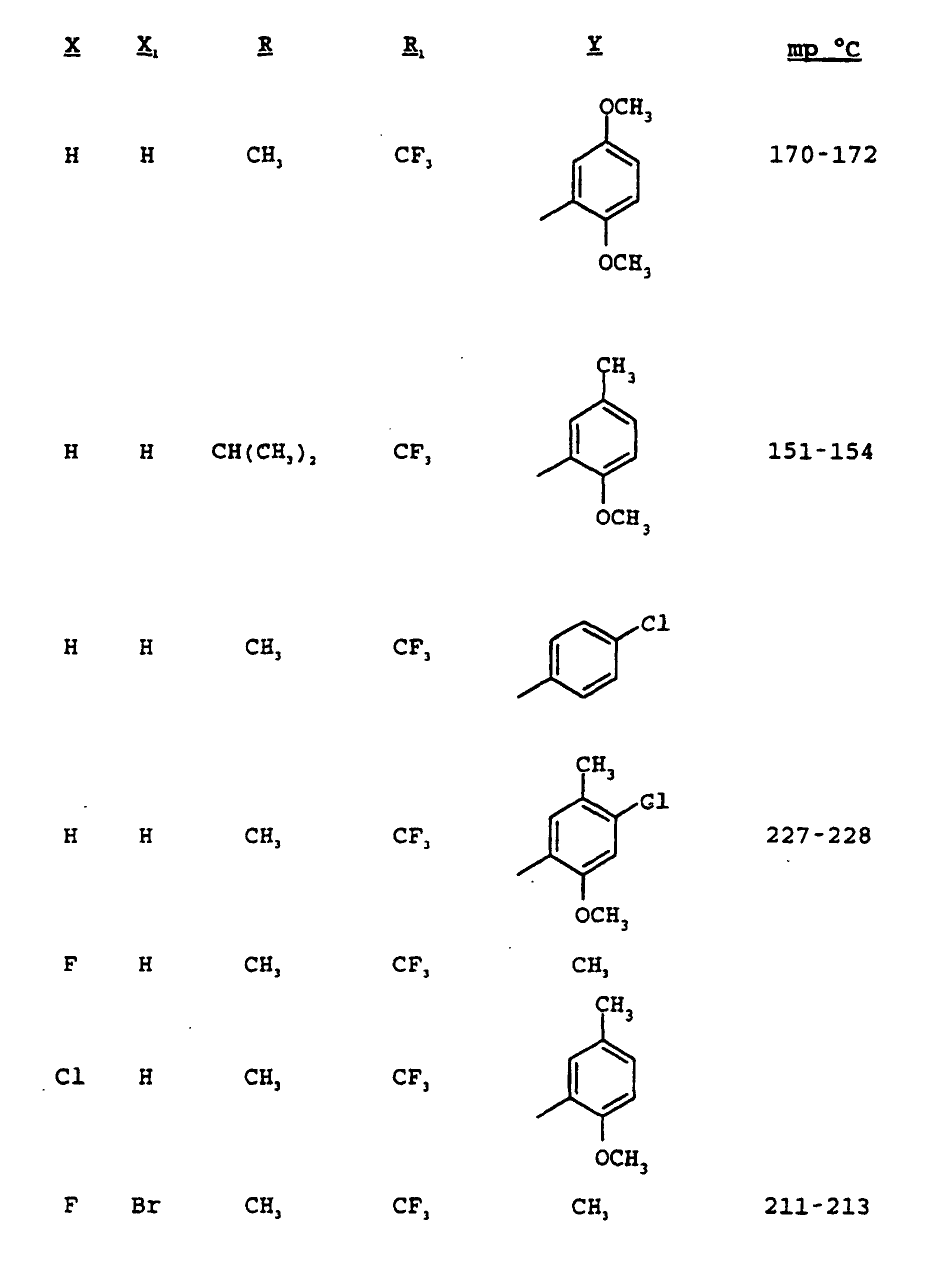

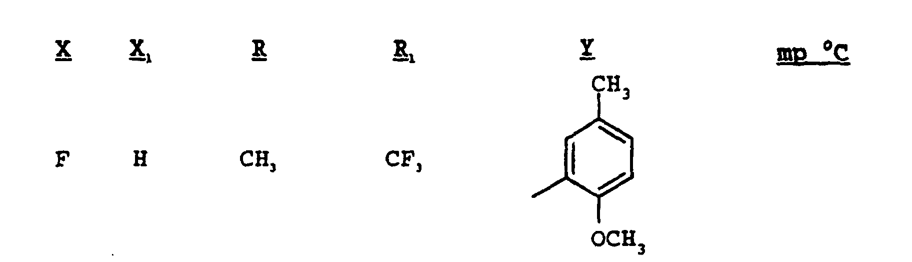

Additionally, in certain instances where R is CF₃ and Y is methyl, the alkylation occurs on both nitrogen and oxygen to afford the following mixtures:

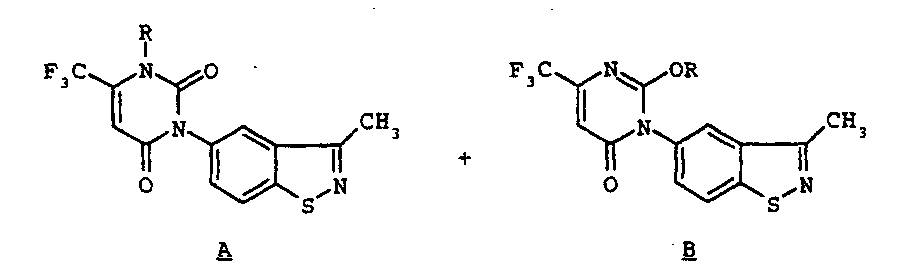

| **R** | **%A** | **%B** |
|---|---|---|
| CH₂CH₃ | 50 | 50 |
| CH₂CH(CH₃)₂ | 55 | 45 |
| CH₂CH=CH₂ | 85 | 15 |

Similarly, the following O-alkylated and N-alkylated compounds are isolated as mixtures and subsequently separated by column chromatography:

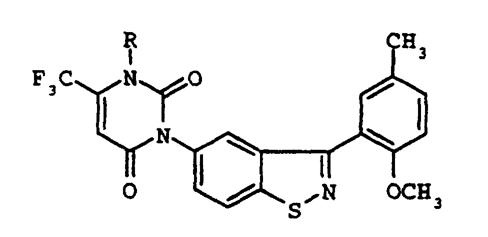

| **R** | **mp °C** |
|---|---|
| CH₂CH₃ | 105-107 |
| CH₂CH=CH₂ | 122-127 |

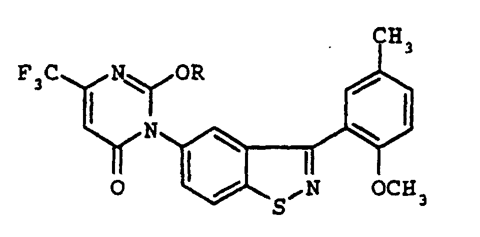

| **R** | **mp °C** |
|---|---|
| CH₂CH₃ | 143-146 |
| CH₂CH=CH₂ | 129-133 |

In addition, the following S-alkylated product is obtained when A is sulfur:

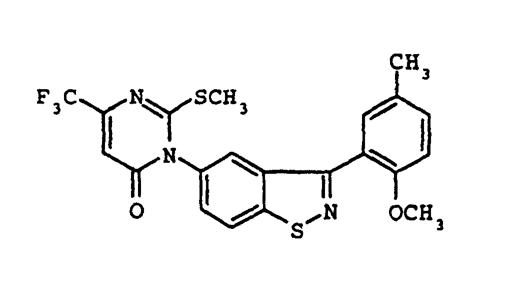

### EXAMPLE 9

### Preparation of 3-[3-(6-Hydroxy-m-tolyl)-1,2-benzisothiazol-5-yl]-1-methyl-6-(trifluoromethyl)-2,4(1H,3H)-pyrimidinedione

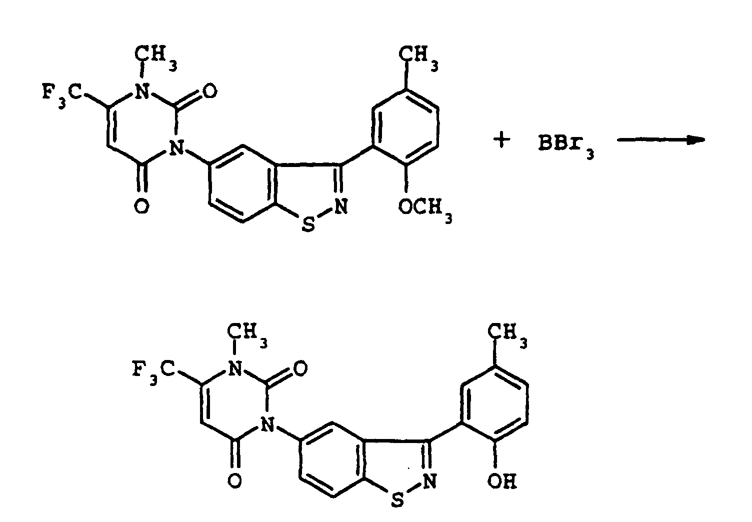

A solution of 3-[3-(6-methoxy-*m*-tolyl)-1,2-benzisothiazol-5-yl]-1-methyl-6-(trifluoromethyl)-2,4(1H,3H)-pyrimidinedione (111 g, 0.248 mol) in methylene chloride is cooled to -5 °C, treated over one hour with a 1 M solution of boron tribromide in methylene chloride (322 mL, 0.322 mol), stirred for one hour, washed sequentially with water and brine, dried over anhydrous magnesium sulfate, and concentrated *in vacuo* to give the title product as a yellow solid (105 g, 98.1%) which is identified by NMR spectral analyses.

Using essentially the same procedure, the following compounds are obtained:

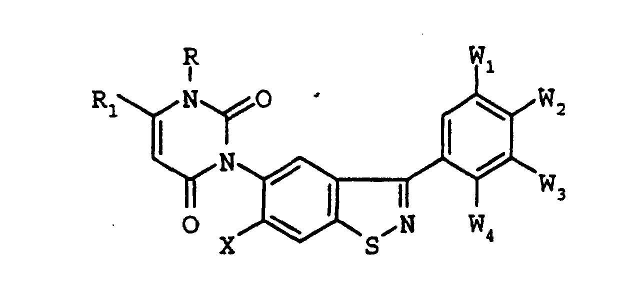

| **X** | **R** | **R**_{**1**} | **W**_{**1**} | **W**_{**2**} | **W**_{**3**} | **W**_{**4**} | **mp °C** |
|---|---|---|---|---|---|---|---|
| H | CH₃ | CF₃ | I | H | H | OH | |
| H | CH₃ | CF₃ | H | CH₃ | H | OH | |
| H | CH₃ | CF₃ | CH₃ | CH₃ | H | OH | 265-266 |
| H | CH₃ | CH₃ | CH₃ | H | H | OH | >250 |
| H | C₂H₅ | CF₃ | CH₃ | H | H | OH | |
| H | CH₂CH=CH₂ | CF₃ | CH₃ | H | H | OH | |
| H | CH₃ | CF₃ | CH₃ | H | CH₃ | OH | |
| H | CH₃ | CF₃ | C₂H₅ | H | H | OH | |
| H | CH₃ | CF₃ | CH(CH₃)₂ | H | H | OH | |
| H | C₂H₅ | CH₃ | CH₃ | H | H | OH | |
| H | CH₃ | CF₃ | H | H | H | OH | |
| H | CH₃ | CF₃ | H | OH | H | H | |
| H | CH₃ | CF₃ | CHO | H | H | OH | |
| H | CH₃ | CF₃ | H | CH₃ | CH₃ | OH | >260 |
| H | CH₃ | CF₃ | H | H | OH | H | 200-202 |
| H | CH₃ | CF₃ | I | CH₃ | H | OH | >250 |
| H | CH₃ | CF₃ | -(CH₂)₃- | | H | OH | 244-247 |
| H | CH₃ | CF₃ | OH | H | H | OH | 239-242 |
| H | CH₃ | CF₃ | OCH₃ | H | H | OH | 180-182 |
| H | CH₃ | CF₃ | CH₃ | H | Cl | OH | 232-235 |
| F | CH₃ | CF₃ | CH₃ | H | H | OH | |

### EXAMPLE 10

### Preparation of Methyl {{2-{5-[3,6-dihydro-3-methyl-4-(trifluoromethyl)-2,6-dioxo-1(2H)-pyrimidinyl]-1,2-benzisothiazol-3-yl}-p-tolyl}oxy}acetate

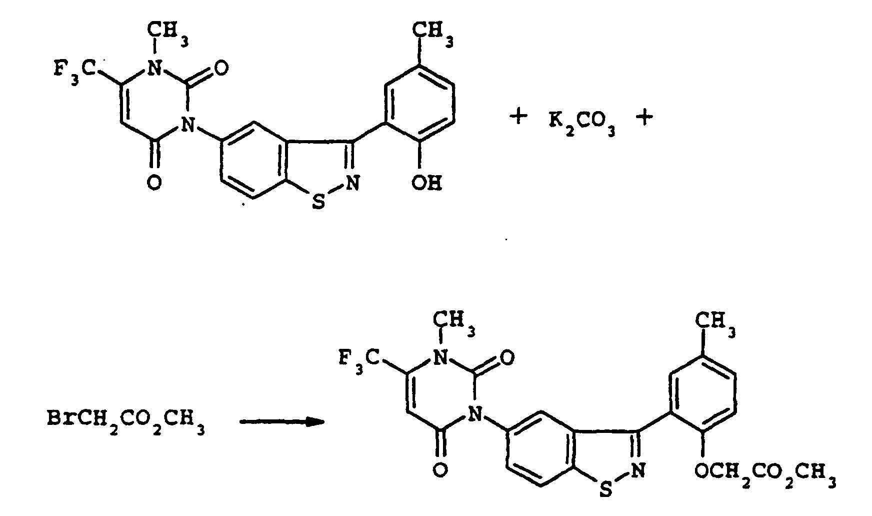

A mixture of 3-[3-(6-hydroxy-*m*-tolyl)-1,2-benzisothiazol-5-yl]-1-methyl-6-(trifluoromethyl)-2,4(1H,3H)-pyrimidinedione (70.6 g, 0.163 mol), potassium carbonate (27.1 g, 0.196 mol) and methyl bromoacetate in N,N-dimethylformamide is stirred at room temperature for 39 hours, diluted with ethyl acetate, washed sequentially with water and brine, dried over anhydrous magnesium sulfate, and concentrated *in vacuo* to obtain a yellow foam. Flash column chromatography of the yellow foam using silica gel and 39:1 to 9:1 methylene chloride in diethyl ether solutions gives the title product as a white foam (67.9 g, 82.4%) which is identified by NMR spectral analyses.

Using essentially the same procedure, the following compounds are obtained:

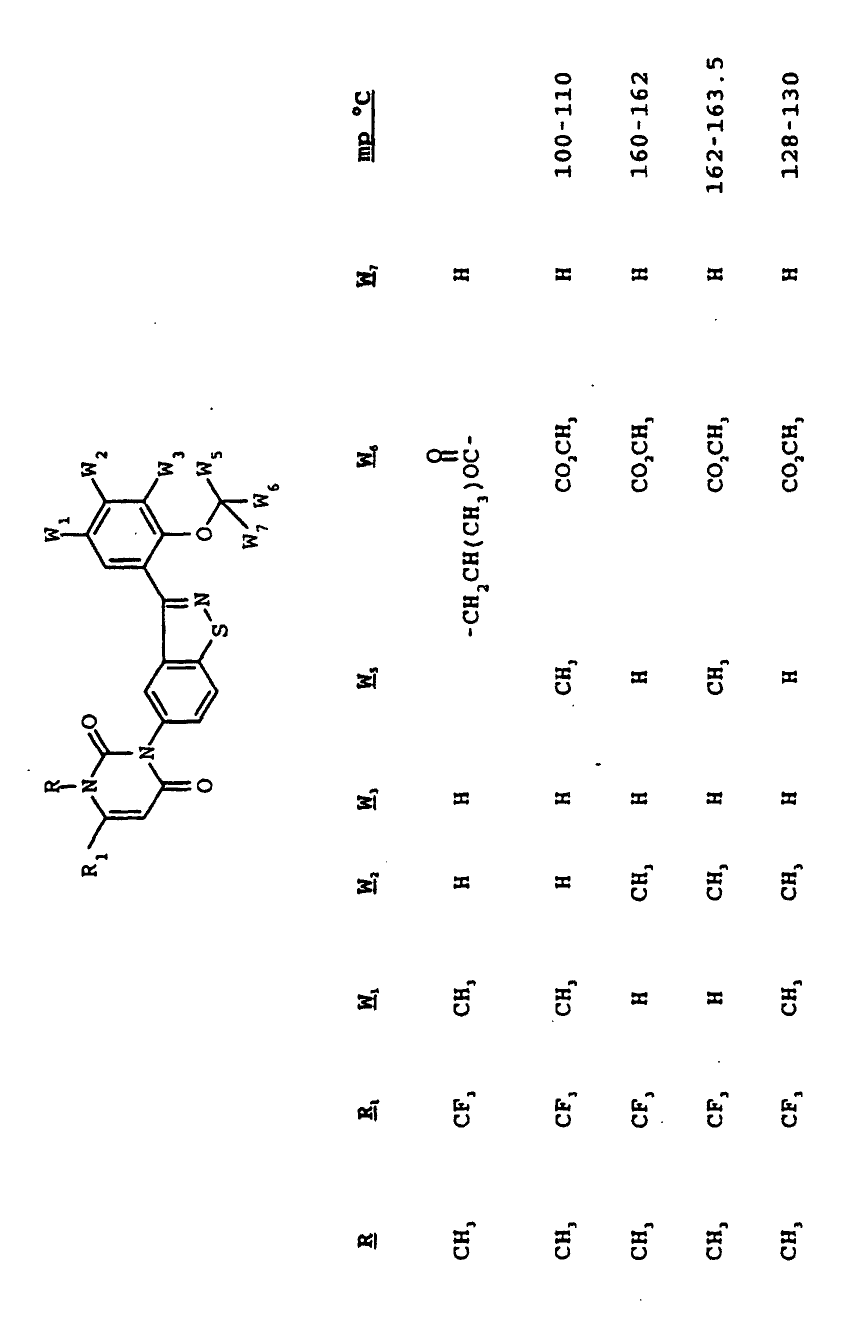

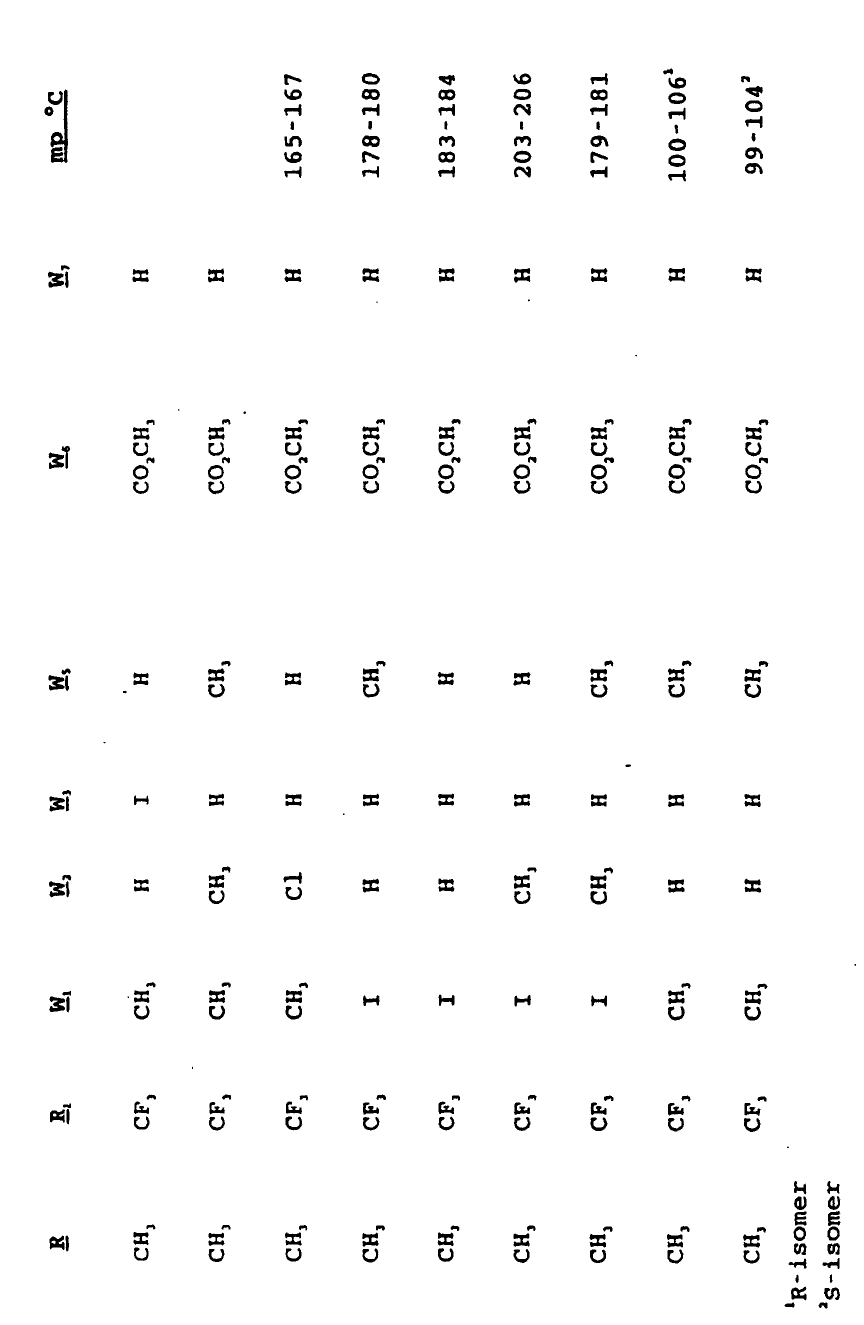

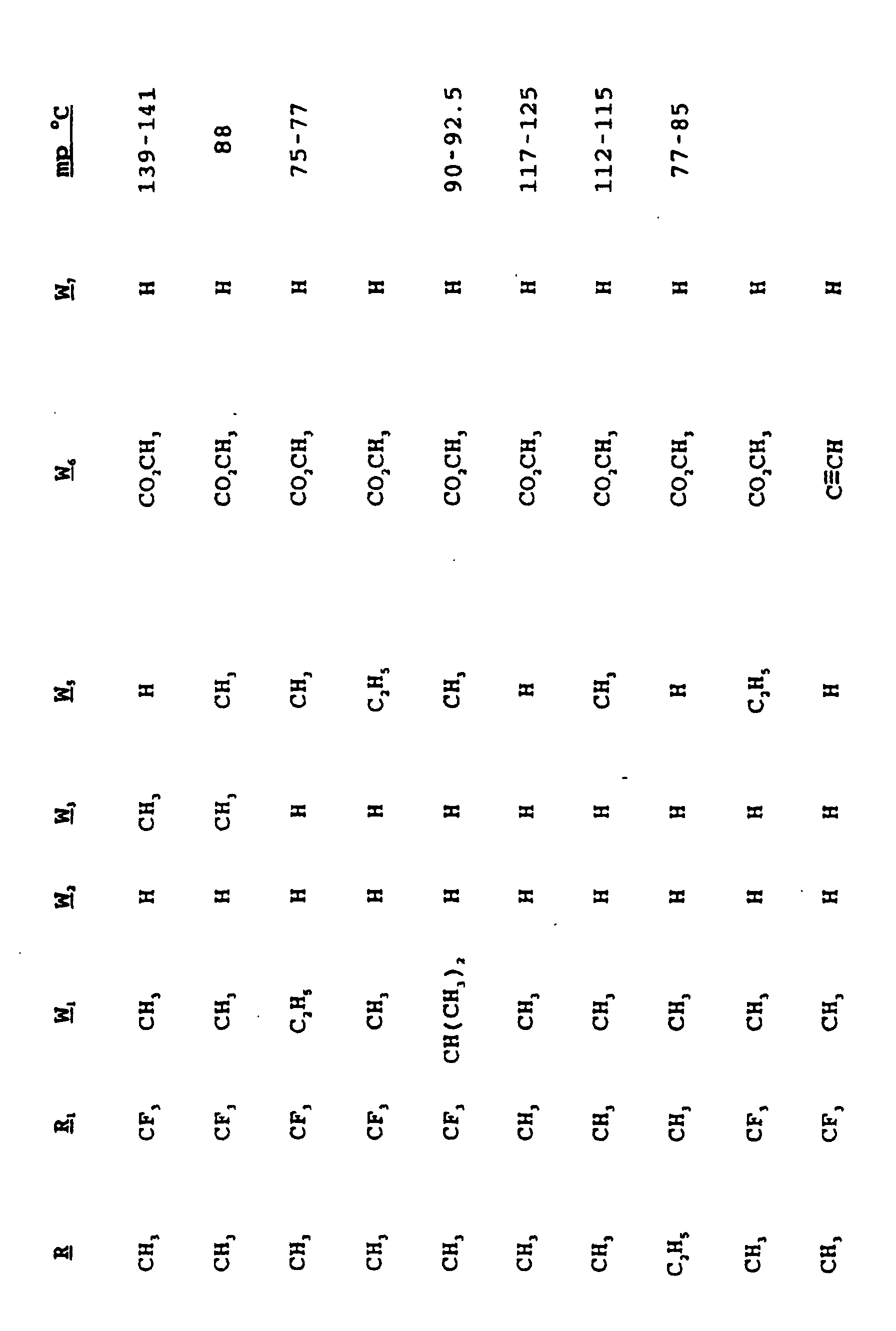

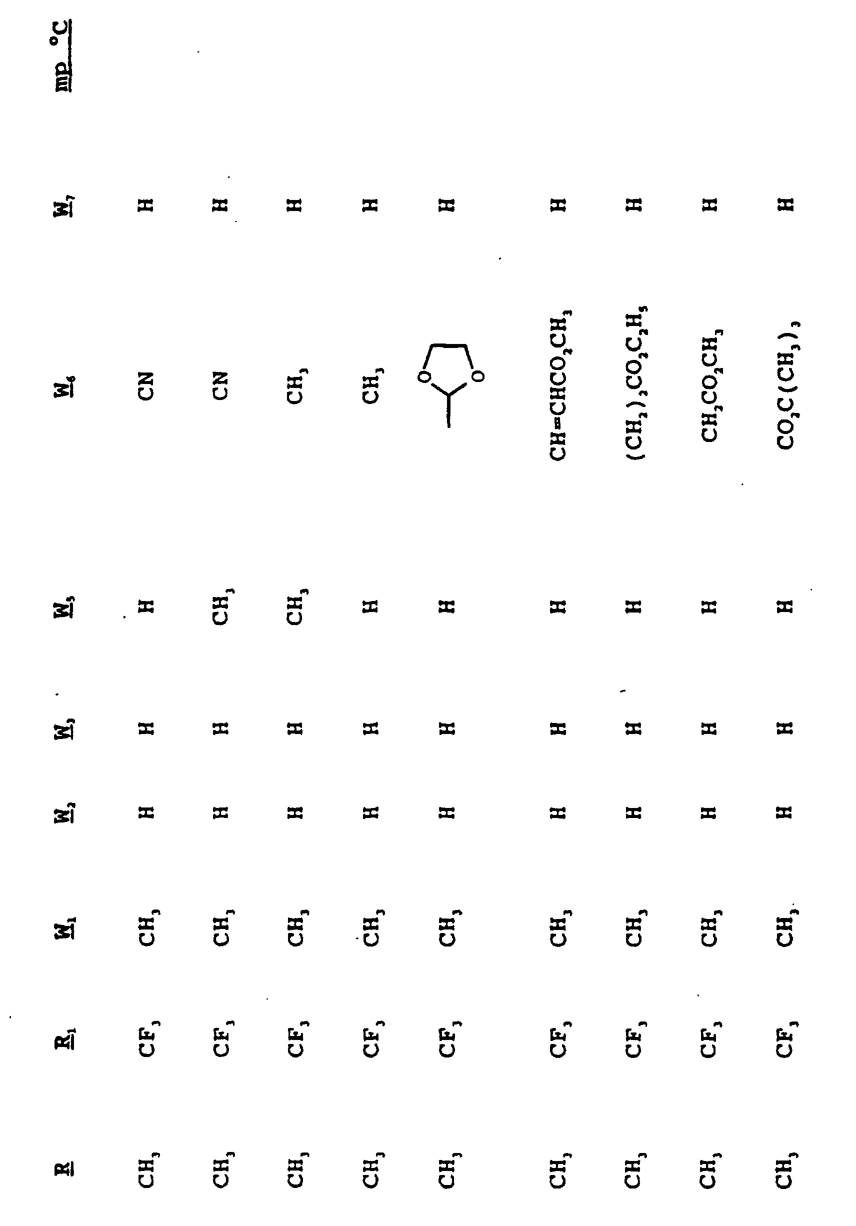

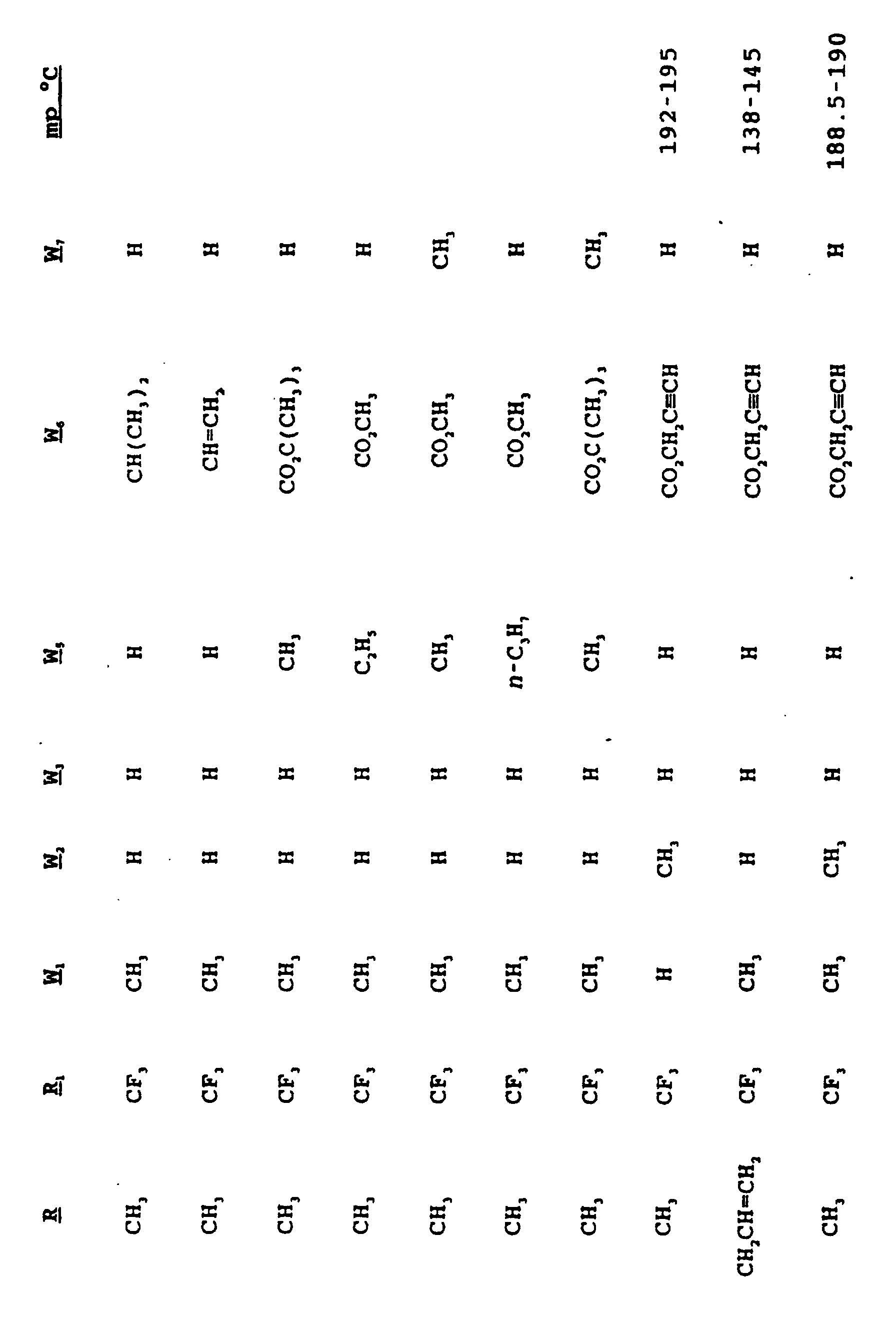

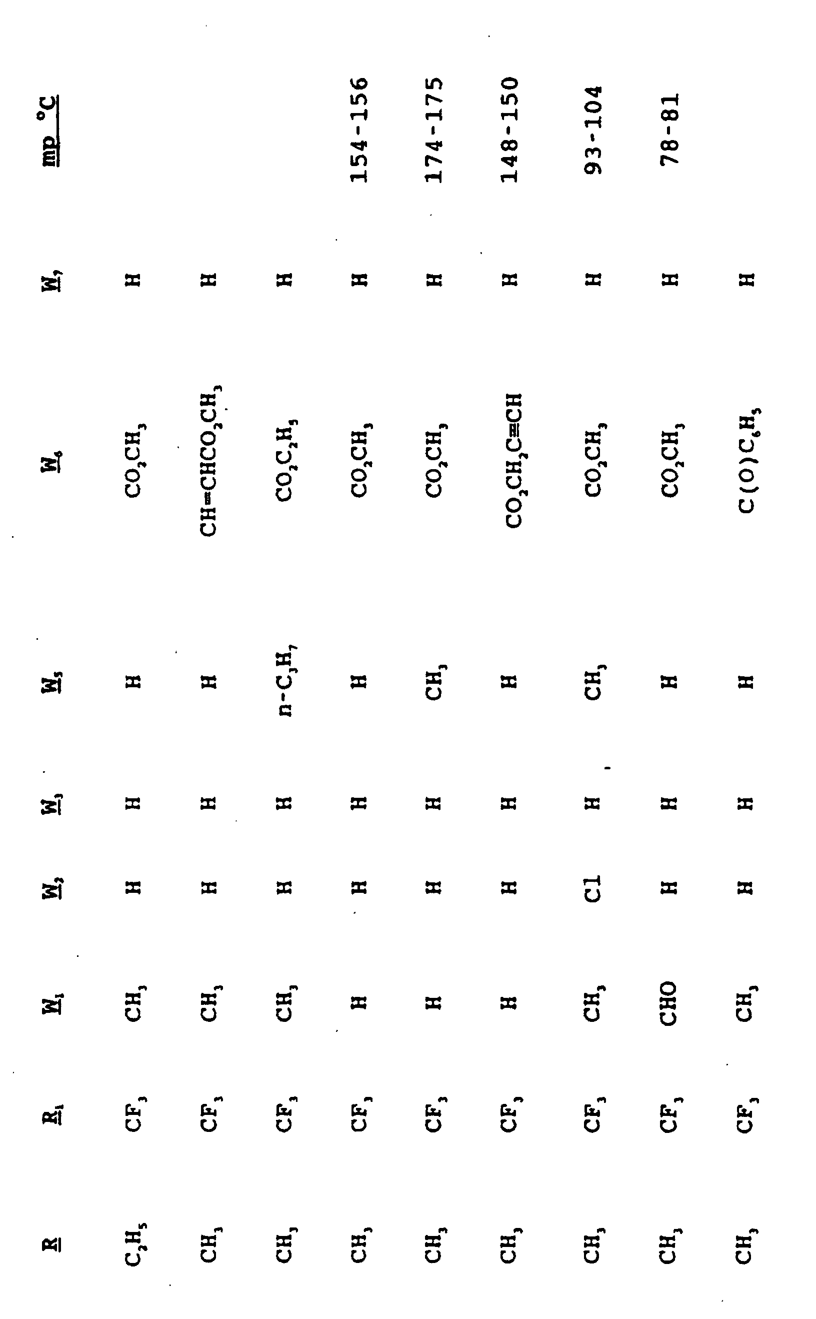

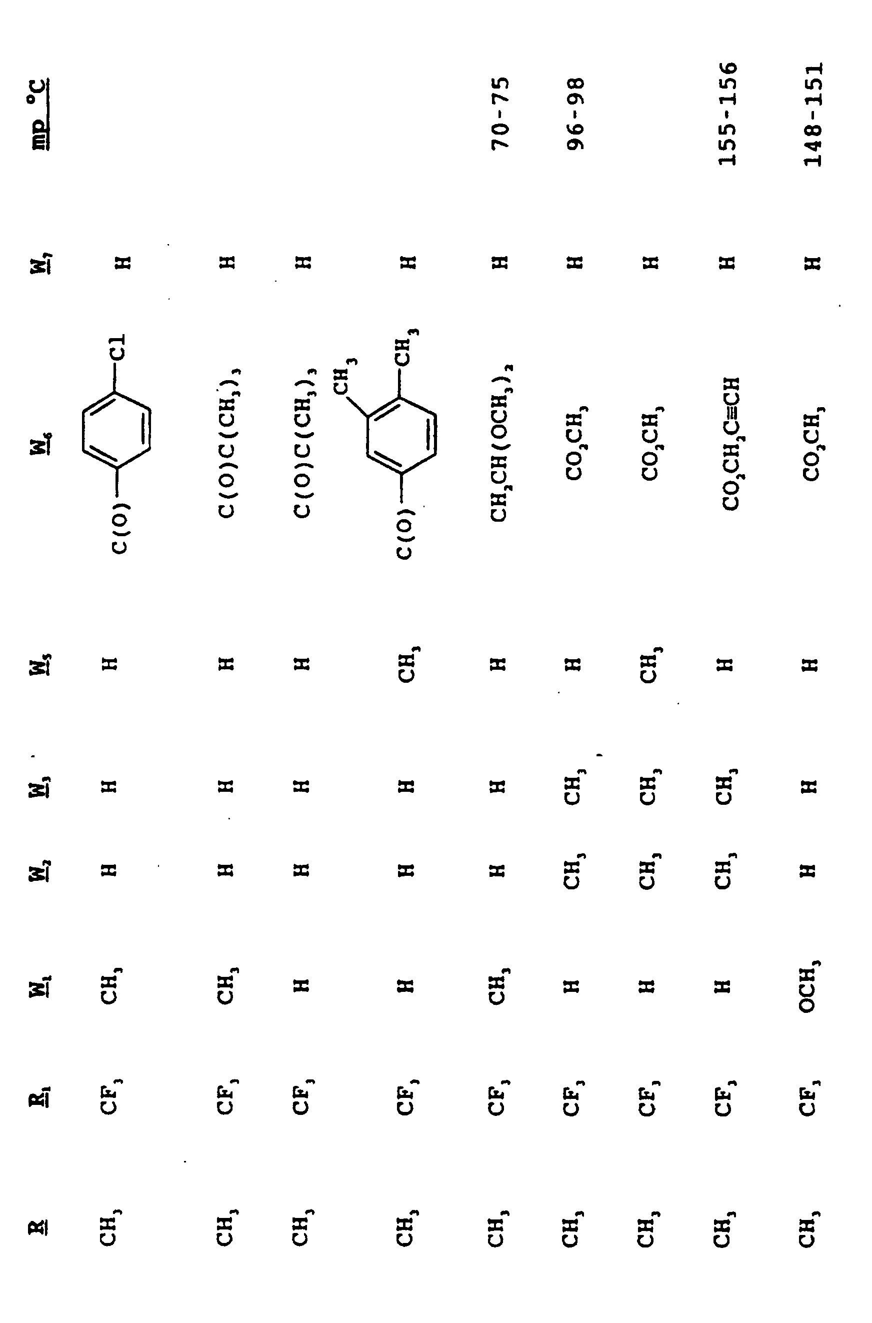

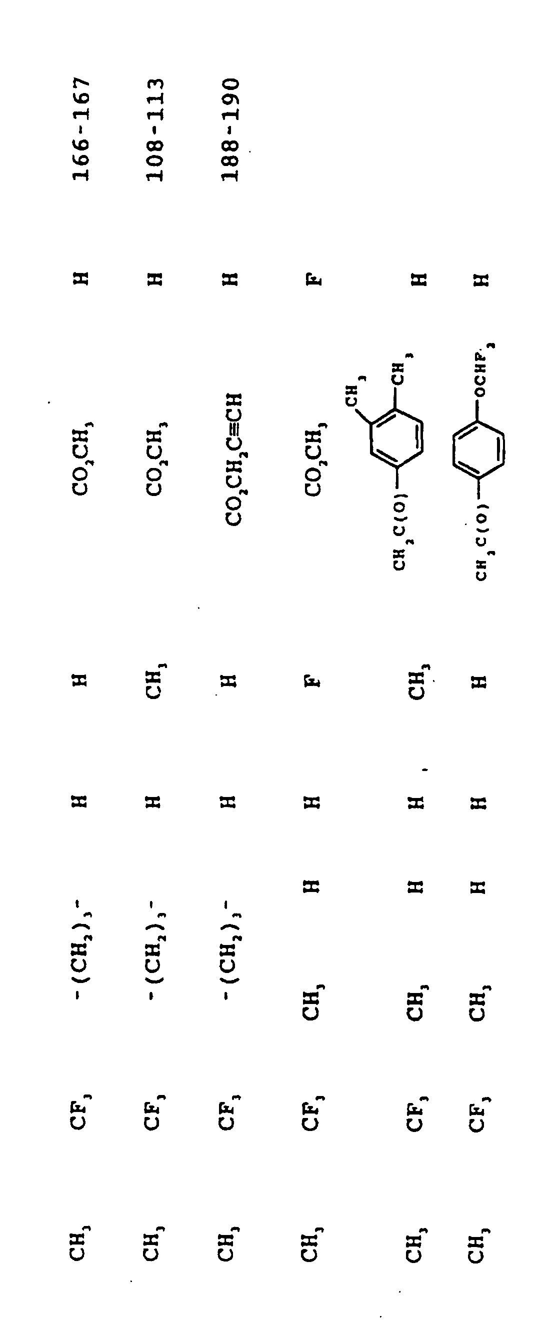

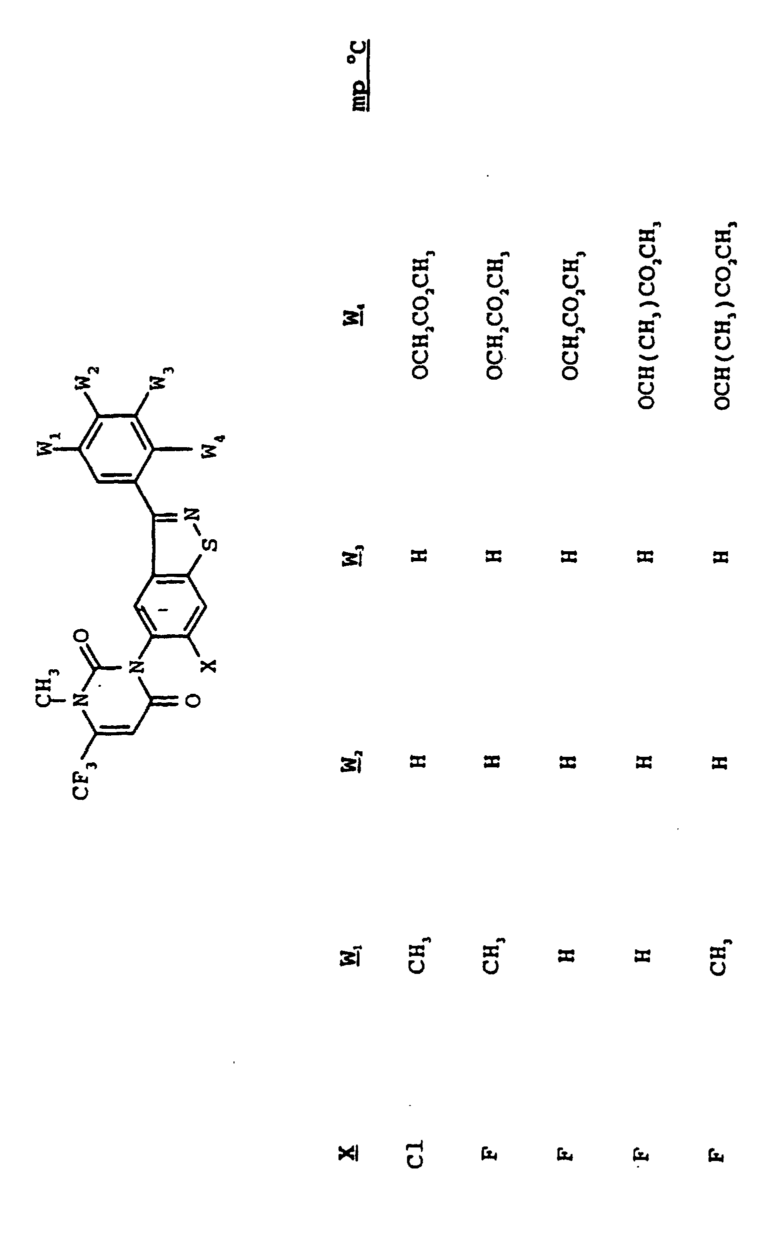

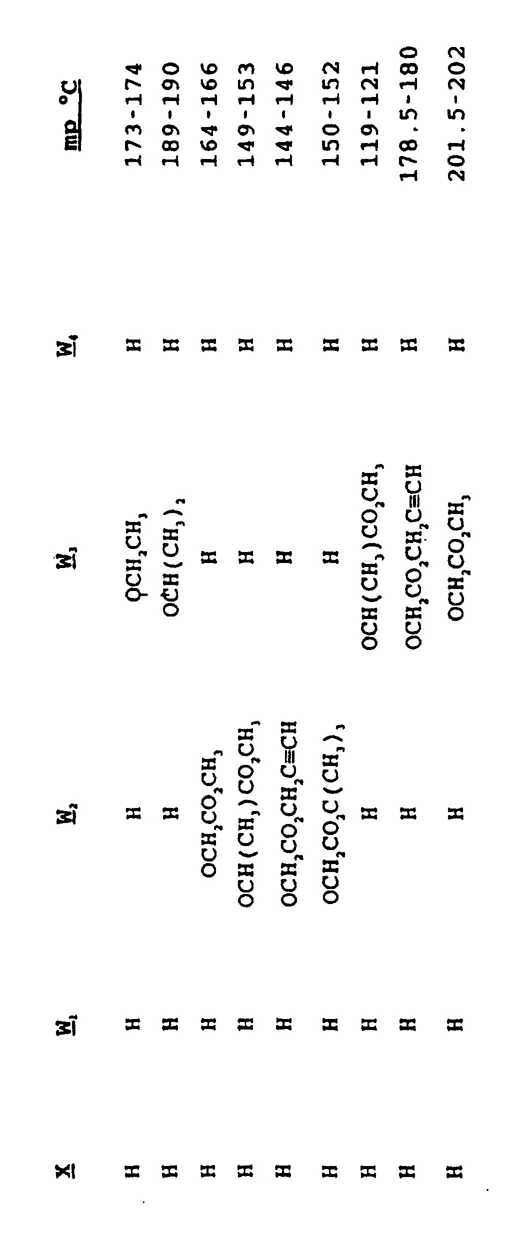

### EXAMPLE 11

### Preparation of 2-Chloro-2'-methoxy-5'-methyl-5-nitrobenzophenone, oxime

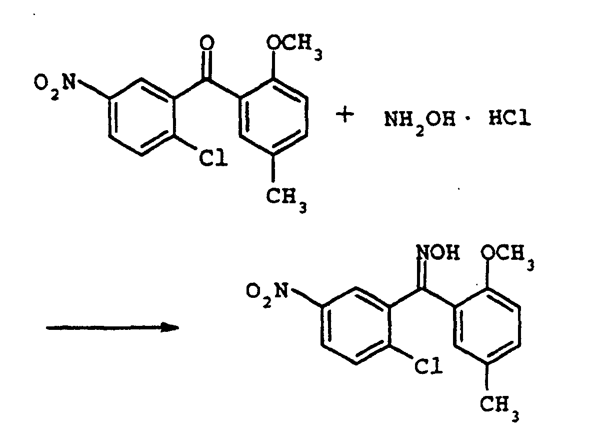

A mixture of 2-chloro-2'-methoxy-5'-methyl-5-nitrobenzophenone (90.0 g, 0.294 mol) in ethanol is treated with a solution of hydroxylamine hydrochloride (102.3 g, 1.47 mol) in water, refluxed overnight, and poured onto ice. The resultant aqueous mixture is filtered to obtain a solid. The solid is washed with water and dried in a hot vacuum oven overnight to give the title product as a white solid (84.2 g) which is identified by ¹H NMR spectral analysis.

Using essentially the same procedure, the following compounds are obtained:

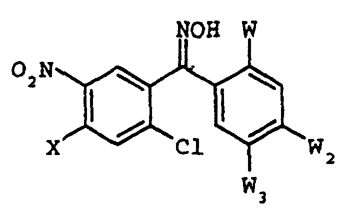

| **X** | **W** | **W**_{**2**} | **W**_{**3**} | **mp °C** |
|---|---|---|---|---|
| H | OCH₃ | H | H | 173-178 |
| H | H | H | H | 143-145 |
| H | H | OCH₃ | H | 191-192.5 |
| H | OCH₃ | H | F | |
| H | H | OCH₂CO₂CH₃ | H | 150-155 |
| H | OCH₃ | H | CH₃ | 185.5-186.5 |
| F | OCH₃ | H | CH₃ | |

### EXAMPLE 12

### Preparation of 3-(6-Methoxy-m-tolyl)-5-nitro-1,2-benzisoxazole

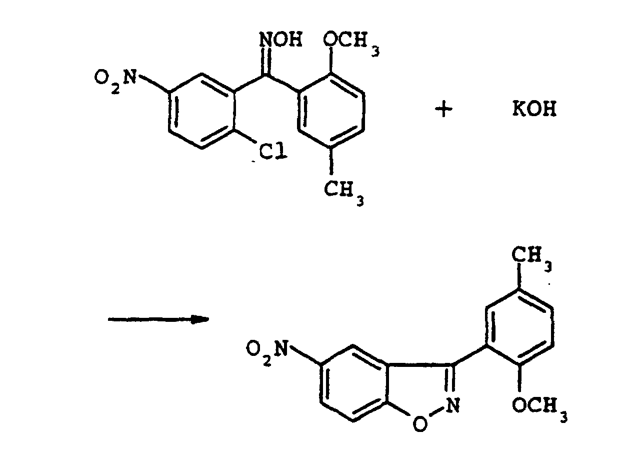

A mixture of 2-chloro-2'-methoxy-5'-methyl-5-nitrobenzophenone, oxime (84.0 g, 0.262 mol) in ethanol is warmed to 65 °C, treated with 150 mL of 10% potassium hydroxide solution over 25 minutes, heated to 78 °C over one hour, cooled, and poured onto ice. The resultant aqueous mixture is filtered to obtain a solid. The solid is washed with water, dried, recrystallized from N,N-dimethylformamide, washed sequentially with N,N-dimethylformamide and ethanol, and dried in a vacuum oven at 80 °C to give the title product as a solid (mp 225-226 °C) which is identified by ¹H NMR spectral analysis.

Using essentially the same procedure, the following compounds are obtained:

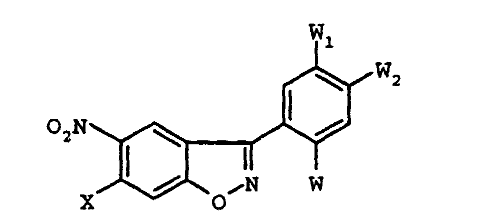

| **X** | **W** | **W**_{**1**} | **W**_{**2**} | **mp °C** |
|---|---|---|---|---|
| H | OCH₃ | H | H | 170-171 |
| H | H | H | H | 138-139 |
| H | H | H | OCH₃ | 205-207 |
| F | OCH₃ | CH₃ | H | |

### EXAMPLE 13

### Preparation of 5-Amino-3-(6-methoxy-m-tolyl)-1,2-benzisoxazole and 5-Amino-4-chloro-3-(6-methoxy-m-tolyl)-1,2-benzisoxazole

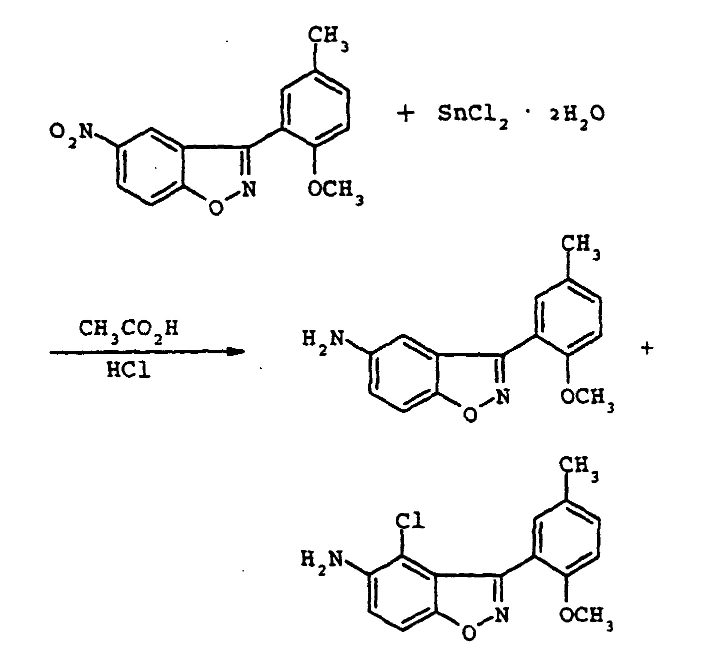

A mixture of 3-(6-methoxy-*m*-tolyl)-5-nitro-1,2-benzisoxazole (20.0 g, 0.0703 mol) in acetic acid (380 mL) is warmed, treated with a warm solution of tin(II) chloride dihydrate (47.4 g, 0.210 mol) in concentrated hydrochloric acid (110 mL), refluxed for one hour, cooled to 10 °C, and concentrated *in vacuo* to obtain a gum. The gum is added to water with stirring to obtain a slurry. The slurry is treated with 80 g of 50% sodium hydroxide solution, stirred at 60 °C to 80 °C over one hour, cooled, and decanted to obtain a residue. A mixture of the residue in ethanol is treated with potassium hydroxide (10 g), heated overnight, cooled to room temperature, neutralized with hydrochloric acid, and concentrated *in vacuo* to obtain a residue. The residue is diluted with ethyl acetate and filtered. The filtrate is concentrated *in vacuo* and chromatographed using silica gel and a 2% ethyl acetate in methylene chloride solution to give the title products as semi-solids which are identified by elemental and mass spectral analyses.

Using essentially the same procedure, but substituting 5-nitro-3-phenyl-1,2-benzisoxazole for 3-(6-methoxy-*m*-tolyl)-5-nitro-1,2-benzisoxazole, 5-amino-3-phenyl-1,2-benzisoxazole is obtained.

### EXAMPLE 14

### Preparation of 3-[3-(6-Methory-m-tolyl)-1,2-benzisoxazol-5-yl]-6-(trifluoromethyl)-2,4(1H,3H)-pyrimidinedione

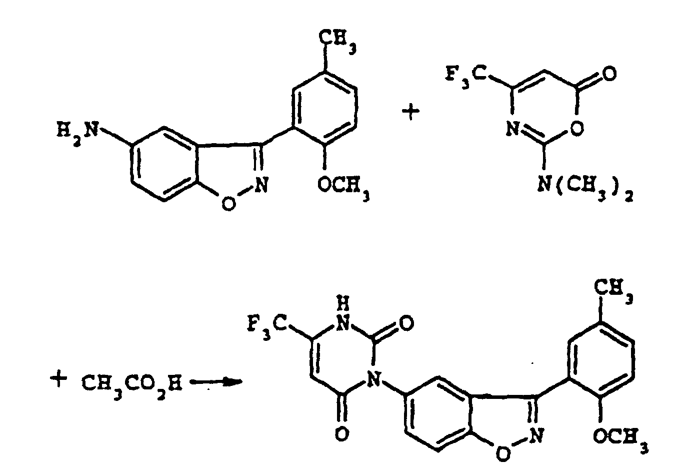

A mixture of 5-amino-3-(6-methoxy-*m*-tolyl)-1,2-benzisoxazole (8.40 g, 0.033 mol), 2-dimethylamino-4-(trifluoromethyl)-6H-1,3-oxazin-6-one (7.60 g, 0.036 mol), and acetic acid is refluxed for three hours, cooled, poured onto ice, and diluted with water. The resultant aqueous mixture is filtered to obtain a solid. The solid is washed with water and dried to give the title product as a pink solid which is identified by NMR spectral analyses.

Using essentially the same procedure, the following compounds are obtained:

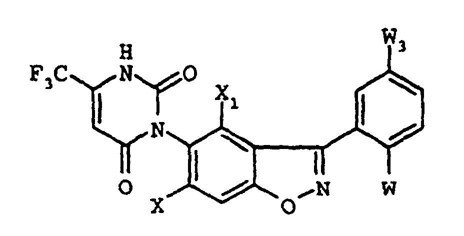

| **X** | **X**_{**1**} | **W** | **W**_{**3**} | **mp °C** |
|---|---|---|---|---|
| H | H | OCH₃ | H | 214-216 |
| H | H | H | H | |
| H | Cl | OCH₃ | CH₃ | |
| F | H | OCH₃ | CH₃ | |

### EXAMPLE 15

### Preparation of 3-[3-(6-Methoxy-m-tolyl)-1,2-benzisoxazol-5-yl]-1-methyl-6-(trifluoromethyl)-2,4(1H,3H)-pyrimidinedione

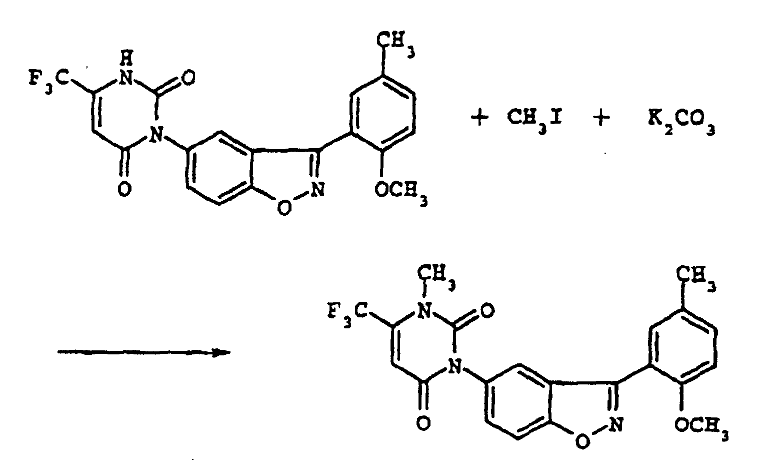

A mixture of 3-[3-(6-methoxy-*m*-tolyl)-1,2-benzisoxazol-5-yl)-6-(trifluoromethyl)-2,4(1H,3H)-pyrimidinedione (10.5 g, 0.0255 mol) and potassium carbonate (7.04 g, 0.051 mol) in N,N-dimethylformamide is stirred for 15 minutes, treated with methyl iodide (7.24 g, 0.051 mol), stirred overnight, and poured onto ice. The resultant aqueous mixture is extracted with methylene chloride. The organic extracts are combined, washed with water, dried over anhydrous sodium sulfate, and concentrated *in vacuo* to obtain a brown glass. Dry column chromatography of the glass using silica gel and a hexanes/ethyl acetate solution (3:1) gives the title product as an off-white solid which is identified by NMR spectral analyses.

Using essentially the same procedure, the following compounds are obtained:

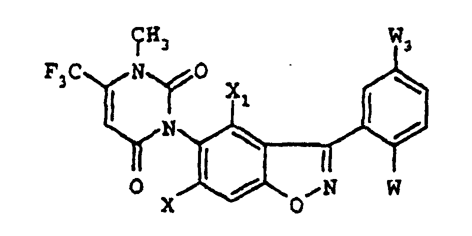

| **X** | **X**_{**1**} | **W** | **W**_{**3**} | **mp °C** |
|---|---|---|---|---|
| H | H | OCH₃ | H | |
| H | H | H | H | 225-226.5 |
| H | Cl | OCH₃ | CH₃ | |
| F | H | OCH₃ | CH₃ | |
| F | H | OCH₃ | H | |

### EXAMPLE 16

### Preparation of 3-[3-(6-Hydroxy-m-tolyl)-1,2-benzisoxazol-5-yl]-1-methyl-6-(trifluoromethyl)-2,4(1H,3H)-pyrimidinedione

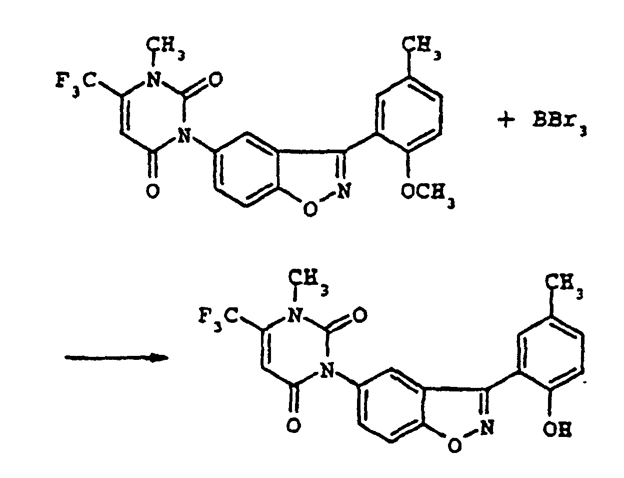

A mixture of 3-[3-(6-methoxy-*m*-tolyl)-1,2-benzisoxazol-5-yl]-1-methyl-6-(trifluoromethyl)-2,4(1H,3H)-pyrimidinedione (5.00 g, 0.0116 mol) in methylene chloride is cooled to -10 °C, treated dropwise with 15.1 mL of 1 M boron tribromide in methylene chloride while maintaining the reaction mixture temperature at -15 °C to 0 °C, warmed to room temperature, treated dropwise with 5 mL of the boron tribromide solution, stirred at room temperature for one hour, and poured into dilute hydrochloric acid. The resultant aqueous mixture is diluted with acetone, concentrated *in vacuo*, diluted with water, and filtered to obtain a solid. The solid is washed with water and dried in a vacuum oven at 60 °C to give the title product as a yellow solid which is identified by mass spectral analysis.

Using essentially the same procedure, the following compounds are obtained:

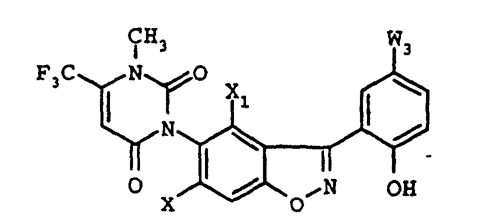

| **X** | **X**_{**1**} | **W**_{**3**} |
|---|---|---|
| H | H | H |
| H | Cl | CH₃ |
| F | H | CH₃ |
| F | H | H |

### EXAMPLE 17

### Preparation of Methyl {{2-{5-[3,6-dihydro-3-methyl-2,6-dioxo-4-(trifluoromethyl)-1(2H)-pyrimidinyl]-1,2-benzisoxazol-3-yl}-p-tolyl}oxy}acetate

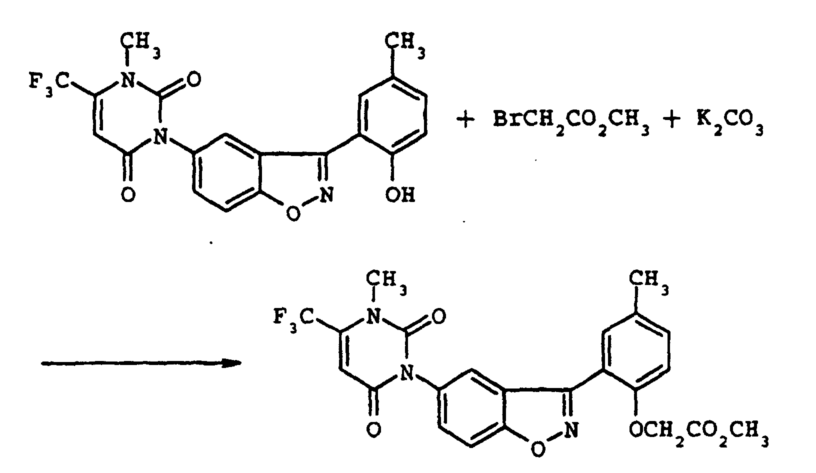

A mixture of 3-[3-(6-hydroxy-*m*-tolyl)-1,2-benzisoxazol-5-yl]-1-methyl-6-(trifluoromethyl)-2,4(1H,3H)-pyrimidinedione (2.25 g, 0.00540 mol), and potassium carbonate (1.50 g, 0.0108 mol) in N,N-dimethylformamide is stirred for several minutes, treated with methyl bromoacetate (0.903 g, 0.00590 mol), stirred at room temperature, and poured into an ice-water mixture. The resultant aqueous mixture is filtered to obtain a solid. Dry column chromatography of the solid using silica gel and a 4% ether in methylene chloride solution gives the title product as a glass which is identified by NMR spectral analyses.

Using essentially the same procedure, the following compounds are obtained:

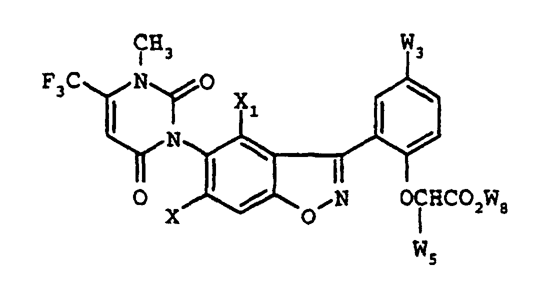

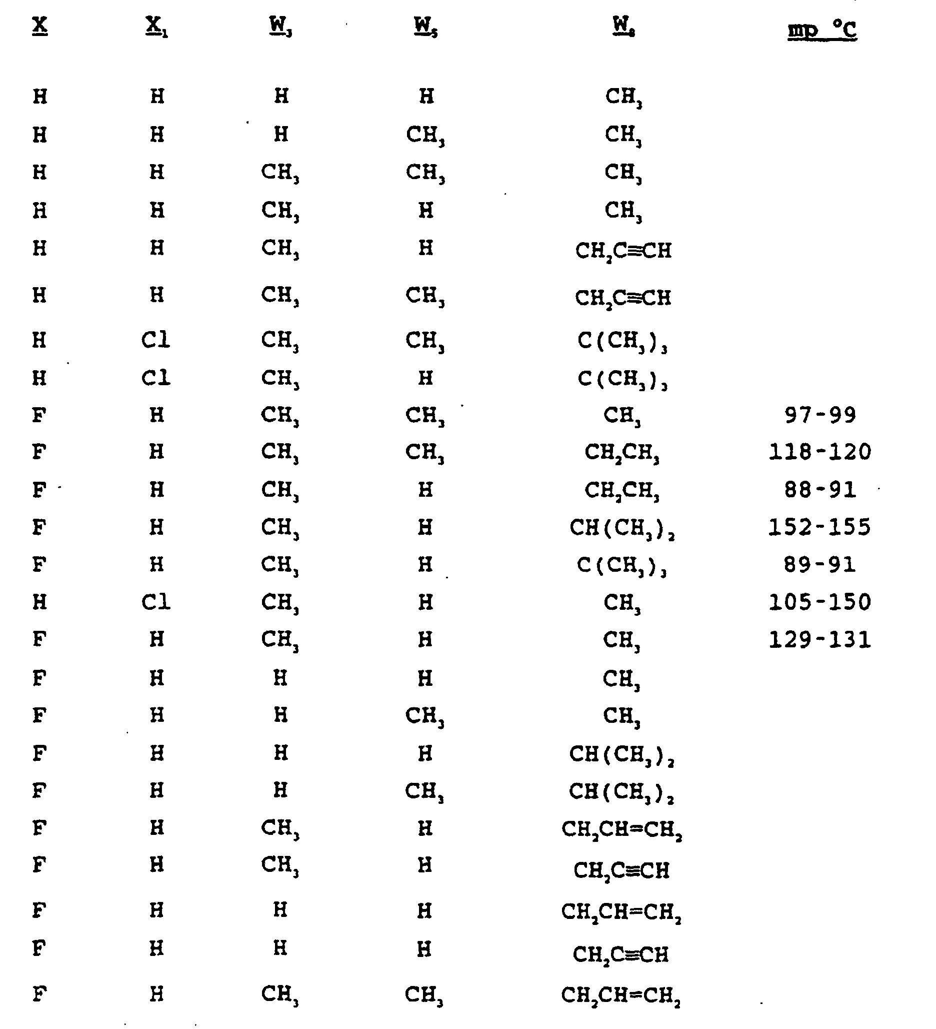

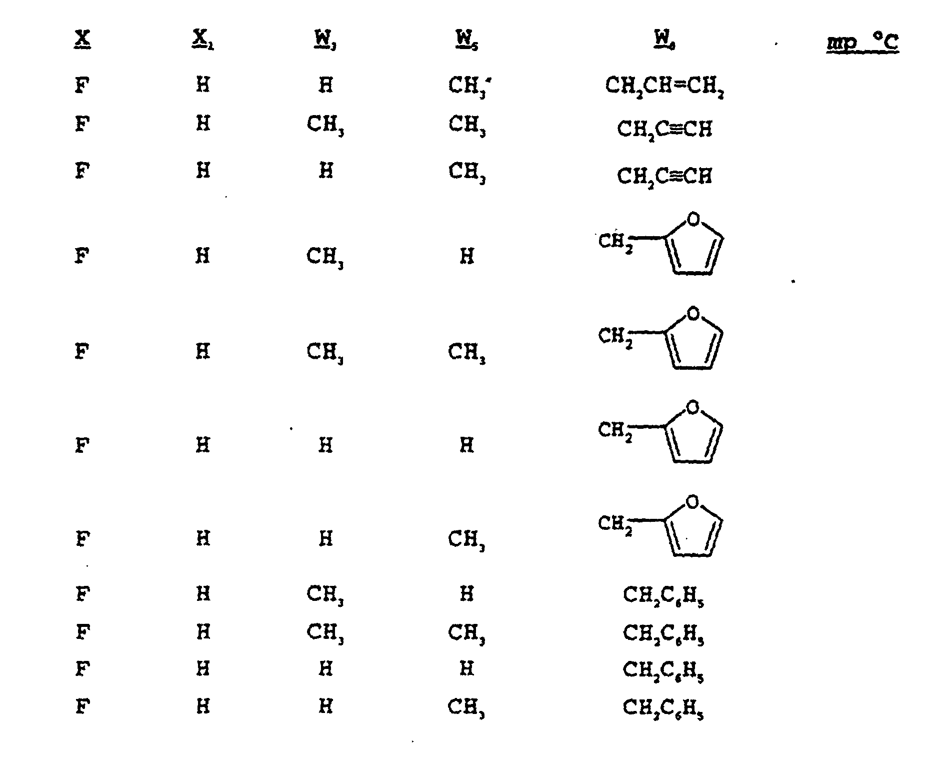

### EXAMPLE 18

### Preparation of 3-[3-Bromomethyl)-1,2-benzisothiazol-5-yl]-1-methyl-6-(trifluoromethyl)-2,4(1H,3H)-pyrimidinedione and 3-[3-(Dibromomethyl)-1,2-benzisothiazol-5-yl]-1-methyl-6-(trifluoromethyl)-2,4(1H,3H)-pyrimidinedione

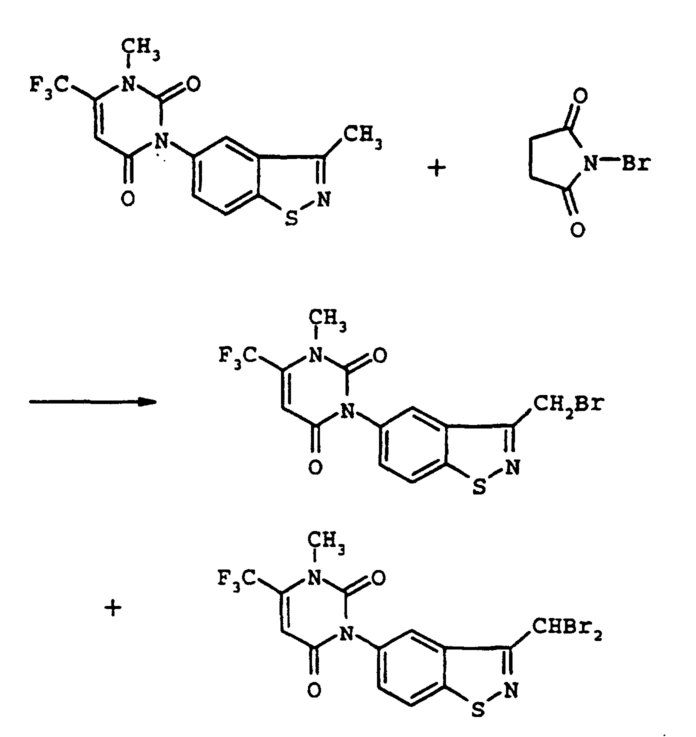

A mixture of 1-methyl-3-(3-methyl-1,2-benzisothiazol-5-yl)-6-(trifluoromethyl)-2,4(1H,3H)-pyrimidinedione (16.6 g, 48.8 mmol), N-bromosuccinimide (34.8 g, 195 mmol) and benzoyl peroxide (0.295 g, 1.22 mmol) in 1,2-dichloroethane is refluxed for one hour, treated with additional benzoyl peroxide (0.30 g), refluxed for 3.5 hours, and cooled to room temperature. Column chromatography of the cooled reaction mixture using silica gel and a methylene chloride/hexanes solution (1:1) gives a mixture of the title products-which is dissolved in methylene chloride. The resultant organic solution is washed sequentially with 15% sodium hydrogen sulfite solution and water, dried over anhydrous magnesium sulfate, and concentrated *in vacuo* to obtain a yellow solid. Column chromatography of the yellow solid using silica gel and a 1% tert-butyl methyl ether in methylene chloride solution gives 3-[3-(bromomethyl)-1,2-benzisothiazol-5-yl]-1-methyl-6-(trifluoromethyl)-2,4(1H,3H)-pyrimidinedione as an off-white solid, mp 203-204 °C, and 3-[3-(dibromomethyl)-1,2-benzisothiazol-5-yl]-1-methyl-6-(trifluoromethyl)-2,4(1H,3H)-pyrimidinedione as an off-white solid, mp 107-110 °C.

Using essentially the same procedure on 1-methyl-3-(3-methyl-6-fluoro-1,2-benzisothiazol-5-yl)-6-(trifluoromethyl)-2,4(1H,3H)-pyrimidinedione, the following compounds are obtained:

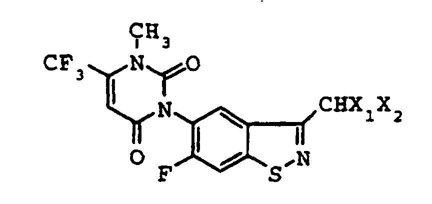

| **X**_{**1**} | **X**_{**2**} | **mp °C** |
|---|---|---|
| H | Br | 185-187 |
| Br | Br | 239-240 |

### EXAMPLE 19

### Preparation of 3-[3-(Methoxymethyl)-1,2-benzisothiazol-5-yl]-1-methyl-6-(trifluoromethyl)-2,4(1H,3H)-pyrimidinedione

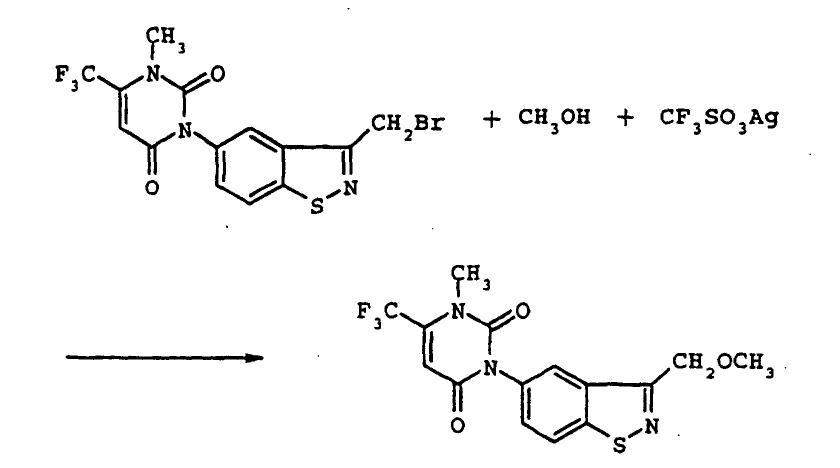

A solution of 3-[3-(bromomethyl)-1,2-benzisothiazol-5-yl]-1-methyl-6-(trifluoromethyl)-2,4(1H,3H)-pyrimidinedione (0.750 g, 1.79 mmol) in 1,2-dichloroethane is diluted with methanol, treated with silver trifluoromethanesulfonate (0.610 g, 2.37 mmol), stirred overnight at room temperature, and filtered through diatomaceous earth. The resultant filtrate is washed with saturated sodium hydrogen carbonate solution, dried over anhydrous magnesium sulfate, and concentrated *in vacuo* to obtain a solid. Column chromatography of the solid using silica gel, and 2% and 5% diethyl ether in methylene chloride solutions gives the title product as a white solid (0.600 g, mp 161-163 °C) which is identified by ¹H NMR spectral analysis.

Using essentially the same procedure, the following compounds are obtained:

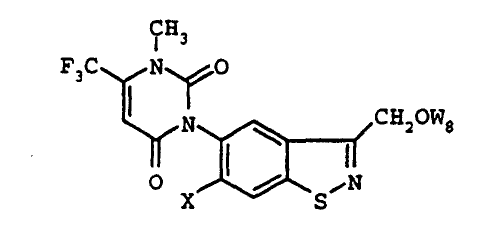

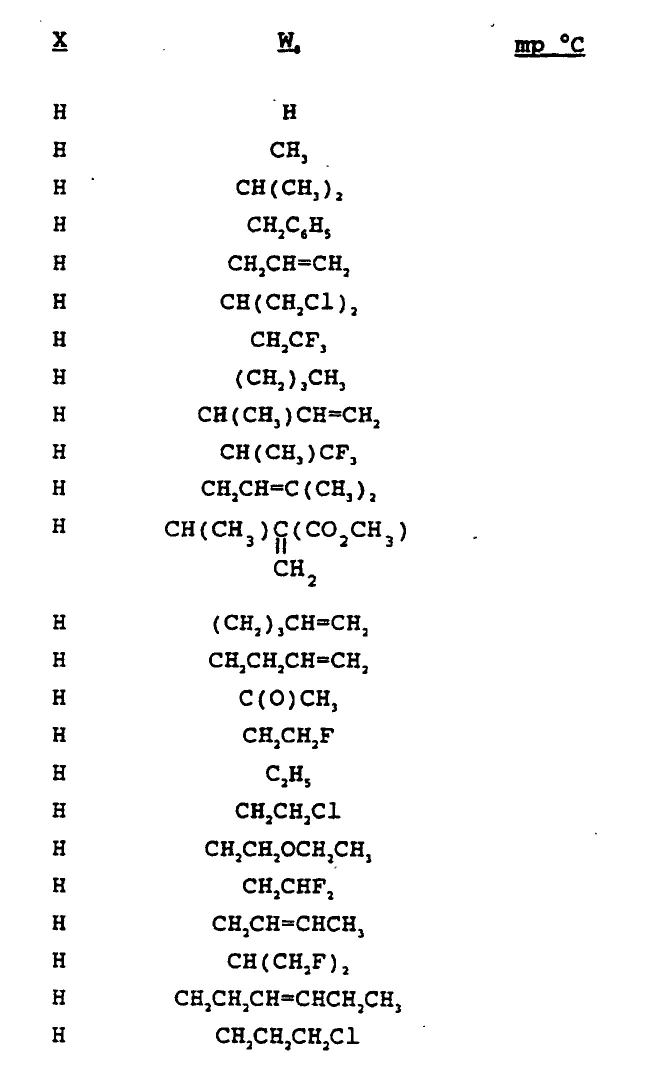

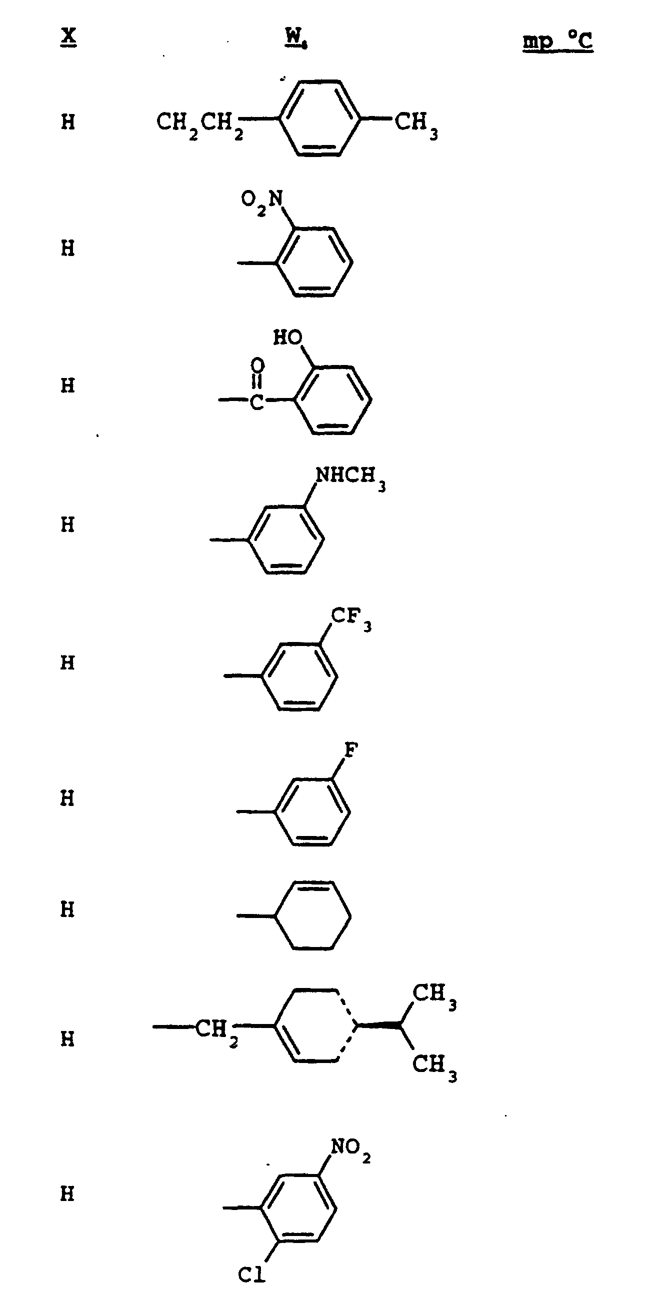

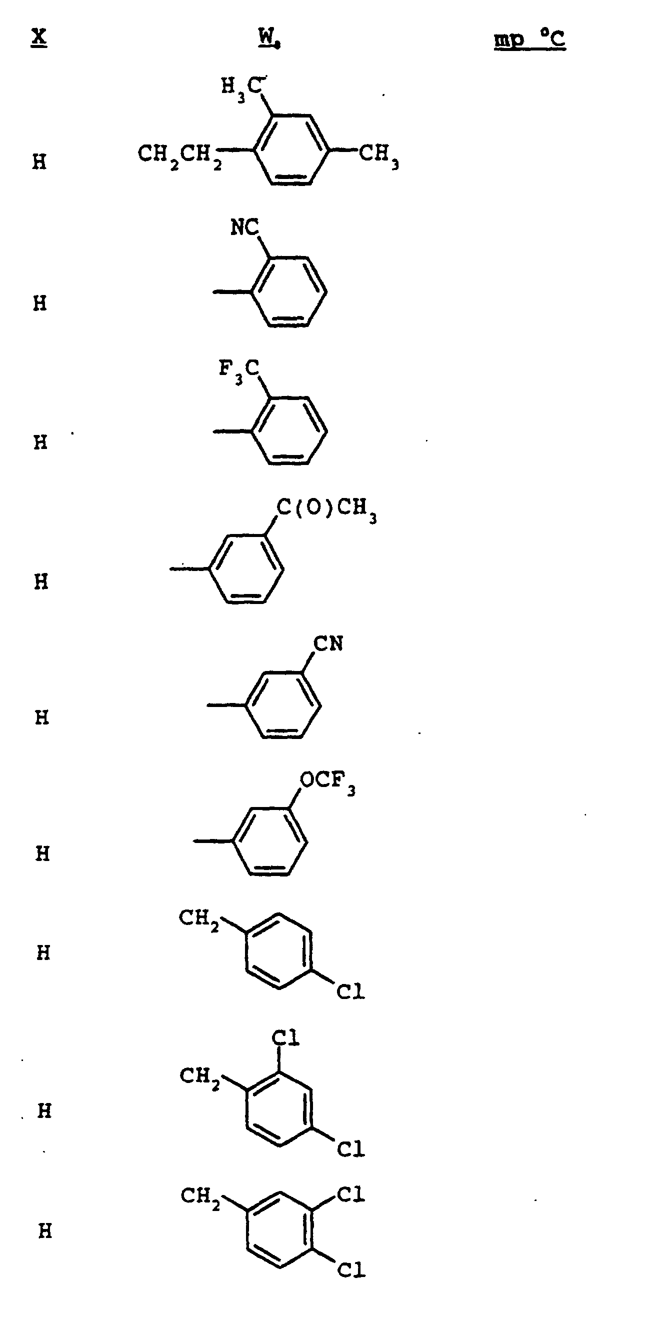

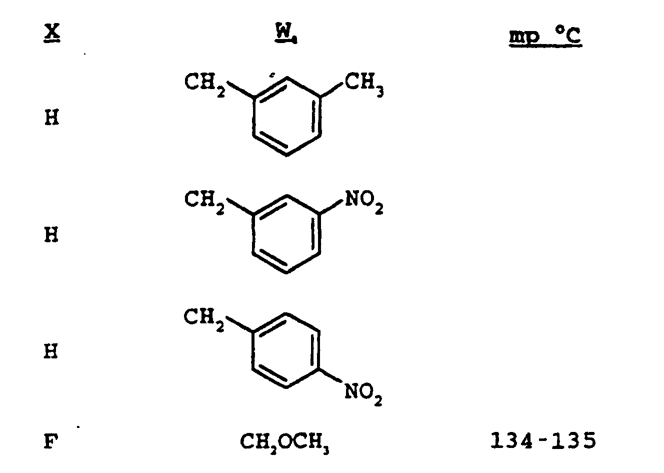

### EXAMPLE 20

### Preparation of Methyl {{{5-[3,6-dihydro-3-methyl-2,6-dioxo-4-(trifluoromethyl)-1(2H)-pyrimidinyl]-1,2-benzisothiazol-3-yl}methyl}thio}acetate

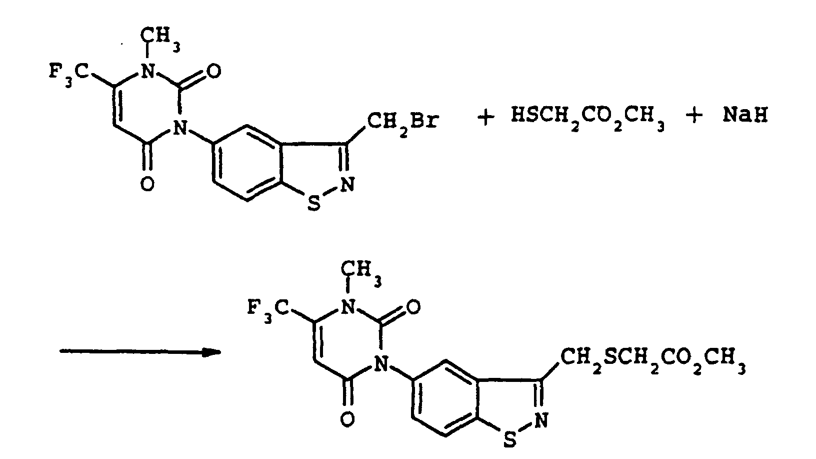

A mixture of hexanes washed sodium hydride (0.0360 g of a 60% dispersion in oil, 0.900 mmol) in tetrahydrofuran is treated with methyl thioglycolate (90.0 mL, 1.00 mmol), stirred until gas evolution stops, treated dropwise with a solution of 3-[3-(bromomethyl)-1,2-benzisothiazol-5-yl]-1-methyl-6-(trifluoromethyl)-2,4(1H,3H)-pyrimidinedione (0.300 g, 0.710 mmol) in tetrahydrofuran, stirred for 10 minutes, and concentrated *in vacuo* to obtain a residue. The residue is diluted with methylene chloride and water. The organic phase is separated, dried over anhydrous magnesium sulfate, and concentrated *in vacuo* to obtain an oil. Column chromatography of the oil using silica gel and a 5% diethyl ether in methylene chloride solution gives the title product as a clear oil which is identified by ¹H NMR spectral analysis.

Using essentially the same procedure, the following compounds are obtained:

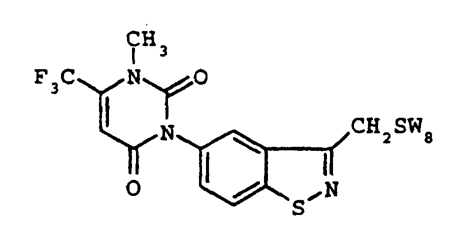

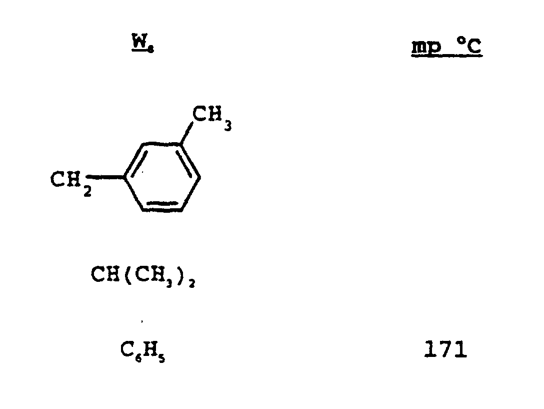

### EXAMPLE 21

### Preparation of 1-Methyl-3-{3-{(methylthio)methyl]-1,2-benzisothiazol-5-yl}-6-(trifluoromethyl)-2,4(1H,3H)-pyrimidinedione

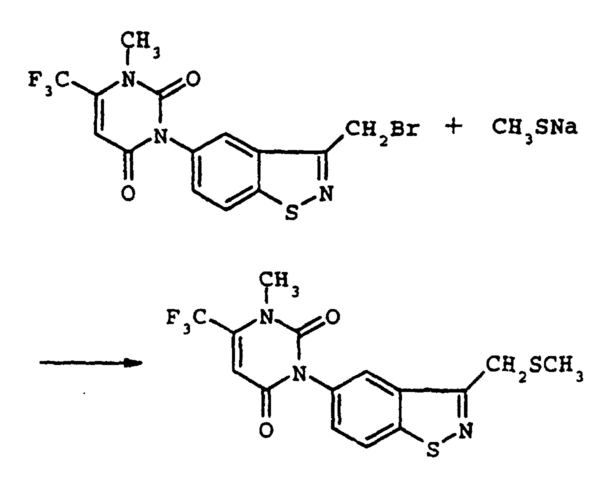

A solution of 3-[3-(bromomethyl)-1,2-benzisothiazol-5-yl]-1-methyl-6-(trifluoromethyl)-2,4(1H,3H)-pyrimidinedione (1.66 g, 3.95 mmol) in acetonitrile is treated with sodium thiomethoxide (0.332 g, 4.74 mmol), stirred overnight at room temperature, treated with additional sodium thiomethoxide (about 15 mg), stirred for one hour, and concentrated *in vacuo* to obtain a residue. The residue is diluted with methylene chloride and water. The organic phase is separated, washed with brine, dried over anhydrous magnesium sulfate, and concentrated *in vacuo* to obtain a solid. Column chromatography of the solid using silica gel and a 2% diethyl ether in methylene chloride solution gives the title product as a white solid which is identified by ¹H NMR spectral analysis.

### EXAMPLE 22

### Preparation of m-Fluorophenyl acetate

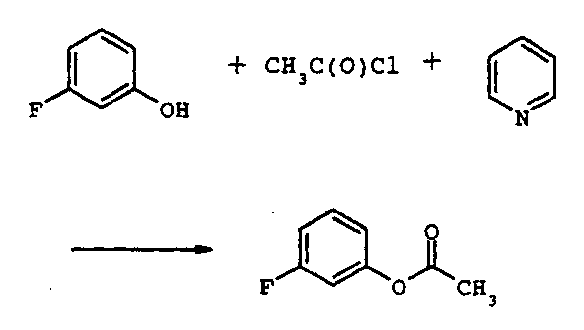

A solution of 3-fluorophenol (100 g, 0.890 mol) in methylene chloride is cooled to 0 °C to 5 °C, treated with pyridine (75.0 mL, 0.930 mol), stirred for several minutes, treated dropwise with acetyl chloride (66.0 mL, 0.930 mol) while maintaining the reaction mixture temperature below 17 °C, stirred at ice-bath temperature for two hours, warmed to room temperature, and poured into an ice-water mixture. The organic phase is separated, washed with brine, dried over anhydrous magnesium sulfate, and concentrated *in vacuo* to obtain the title product as a yellow oil which is identified by ¹H NMR spectral analysis.

### EXAMPLE 23

### Preparation of 4'-Fluoro-2'-hydroxyacetophenone

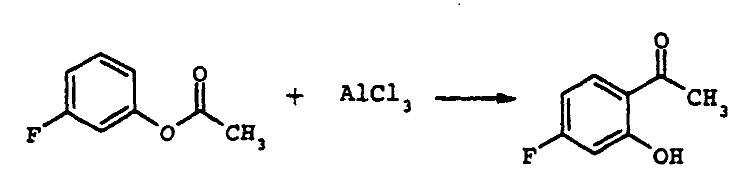

*m*-Fluorophenyl acetate (123 g, 0.798 mol) is cooled with an ice-bath, treated portionwise with aluminum chloride (150 g, 1.12 mol), stirred at 190 °C for one hour, and cooled to obtain a solid. A mixture of ice, water and hydrochloric acid, and methylene chloride are added to the solid. The resultant mixture is stirred for several minutes, and the phases are separated. The organic phase is washed sequentially with water, saturated sodium hydrogen carbonate solution and brine, dried over anhydrous magnesium sulfate, and concentrated *in vacuo* to obtain the title product (99.0 g) which is identified by ¹H NMR spectral analysis.

### EXAMPLE 24

### Preparation of 4'-Fluoro-2'-hydroxyacetophenone, oxime

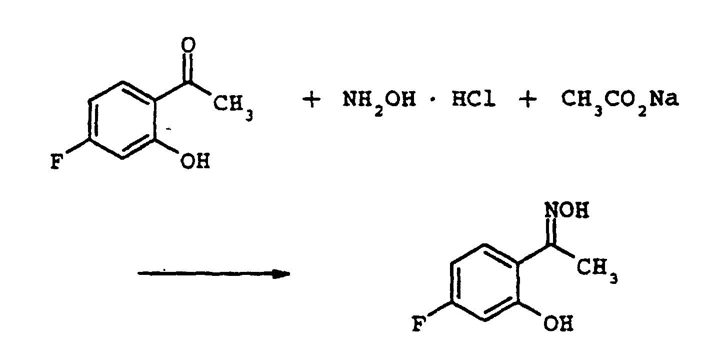

A mixture of 4'-fluoro-2'-hydroxyacetophenone (99.0 g, 0.640 mol), hydroxylamine hydrochloride (89.0 g, 1.28 mol), and sodium acetate (79.0 g, 0.960 mol) in methanol is refluxed for one hour and poured into an ice-water mixture. The resultant aqueous mixture is filtered to obtain a solid. The solid is dissolved in methylene chloride, and the resultant organic solution is dried over anhydrous magnesium sulfate, concentrated *in vacuo*, diluted with hexanes, and filtered to give the title product as a solid (55.0 g, mp 112-114 °C) which is identified by ¹H NMR spectral analysis.

### EXAMPLE 25

### Preparation of 6-Fluoro-3-methyl-1,2-benzisoxazole

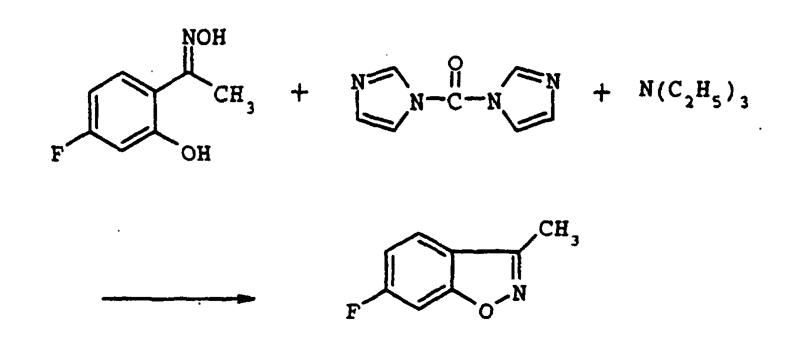

A mixture of 4'-fluoro-2'-hydroxyacetophenone, oxime (47.0 g, 0.278 mol) in tetrahydrofuran is heated to just under reflux, treated with a solution of 1,1'-carbonyldiimidazole (55.0 g, 0.340 mol) and triethylamine (39.0 g, 0.390 mol) in tetrahydrofuran, refluxed for one hour, cooled, concentrated *in vacuo*, and poured into an ice-water mixture. The resultant aqueous mixture is extracted with ether. The organic extracts are combined, washed sequentially with saturated ammonium chloride solution and brine, dried over anhydrous magnesium sulfate, and concentrated *in vacuo* to obtain an oil. Column chromatography of the oil using silica gel and a methylene chloride/hexanes solution (1:1) gives the title product as a yellow oil which is identified by ¹H NMR spectral analysis.

### EXAMPLE 26

### Preparation of 6-Fluoro-3-methyl-5-nitro-1,2-benzisoxazole

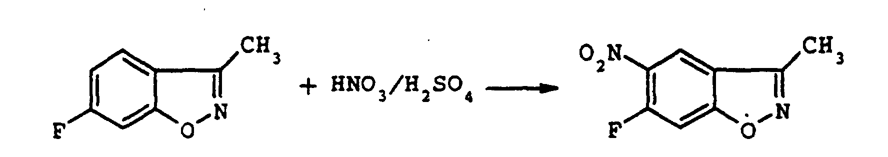

A mixture of 6-fluoro-3-methyl-1,2-benzisoxazole (23.5 g, 0.156 mol) in concentrated sulfuric acid is cooled with an ice-bath, treated dropwise with 90% nitric acid (8.50 mL) while maintaining the reaction mixture temperature below 15 °C, stirred for one hour at ice-bath temperature, treated with additional 90% nitric acid (5.80 mL), warmed to and stirred at room temperature overnight, and poured onto ice. The resultant aqueous mixture is filtered to obtain a solid. The solid is air-dried and dissolved in methylene chloride. The resultant organic solution is dried over anhydrous magnesium sulfate, diluted with hexanes, and filtered to give the title product as a purple solid which is identified by ¹H NMR spectral analysis.

### EXAMPLE 27

### Preparation of 5-Amino-6-fluoro-3-methyl-1,2-benzisoxazole and 5-Amino-4-chloro-6-fluoro-3-methyl-1,2-benzisoxazole

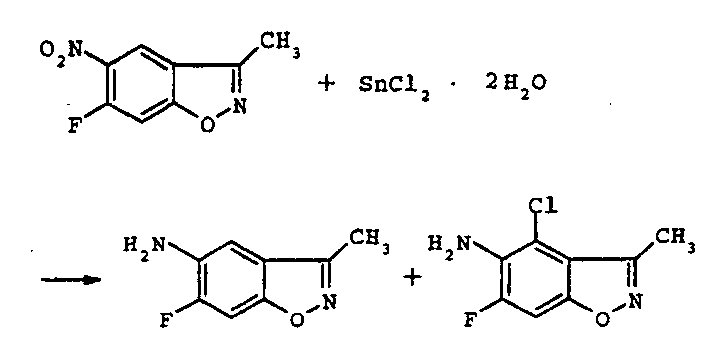

A mixture of 6-fluoro-3-methyl-5-nitro-1,2-benzisoxazole (3.00 g, 0.0153 mol) and acetic acid (85.0 mL) is heated to 40 °C, treated with a solution of tin(II) chloride dihydrate (9.70 g, 0.0430 mol) and concentrated hydrochloric acid (45.0 mL), refluxed for 90 minutes, concentrated *in vacuo*, neutralized with 2N sodium hydroxide solution and filtered to obtain a solid. Column chromatography of the solid using silica gel and methylene chloride gives the title products as solids which are identified by NMR spectral analyses.

Using essentially the same procedure, but using an ethyl acetate/ethanol mixture instead of acetic acid, the following compounds are obtained:

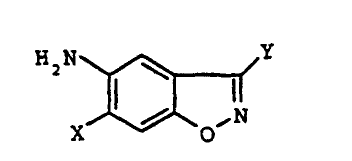

| **X** | **Y** |
|---|---|
| H | CH₃ |
| H | Cl |
| H | OCH₂CO₂CH₃ |
| H | OCH(CH₃)₂ |
| H | OCH(CH₃)CO₂CH₃ |
| F | OCH₂CO₂CH₃ |
| H | OCH₃ |

### EXAMPLE 28

### Preparation of 3-(6-Fluoro-3-methyl-1,2-benzisoxazol-5-yl)-6-(trifluoromethyl)-2,4(1H,3H)-pyrimidinedione

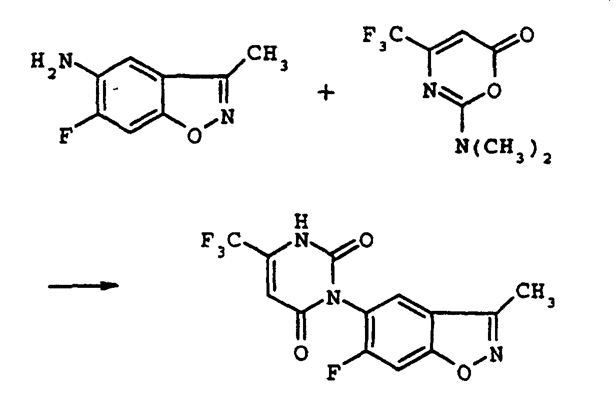

A mixture of 5-amino-6-fluoro-3-methyl-1,2-benzisoxazole (4.85 g, 0.029 mol) and 2-dimethylamino-4-(trifluoromethyl)-6H-1,3-oxazin-6-one (6.70 g, 0.0320 mol) in acetic acid is refluxed for 90 minutes, cooled to room temperature, and poured into an ice-water mixture. The resultant aqueous mixture is filtered to obtain a solid. The solid is air-dried and dissolved in ethyl acetate. The resultant organic solution is washed with brine, dried over anhydrous magnesium sulfate, and concentrated *in vacuo* to give the title product as a yellow solid (7.00 g, mp 235-237 °C) which is identified by NMR spectral analyses.

Using essentially the same procedure, the following compounds are obtained:

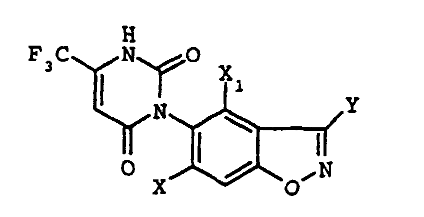

| **X** | **X**_{**1**} | **Y** | **mp °C** |
|---|---|---|---|
| H | H | CH₃ | 283-285 |
| H | H | Cl | |
| H | H | OCH₂CO₂CH₃ | 180-182 |
| H | H | OCH(CH₃)₂ | 213-215 |
| H | H | OCH₃ | 230-235 |
| F | Cl | CH₃ | 125-130 |

### EXAMPLE 29

### Preparation of 3-(6-Fluoro-3-methyl-1,2-benzisoxazol-5-yl)-1-methyl-6-(trifluoromethyl)-2,4(1H,3H)-pyrimidinedione

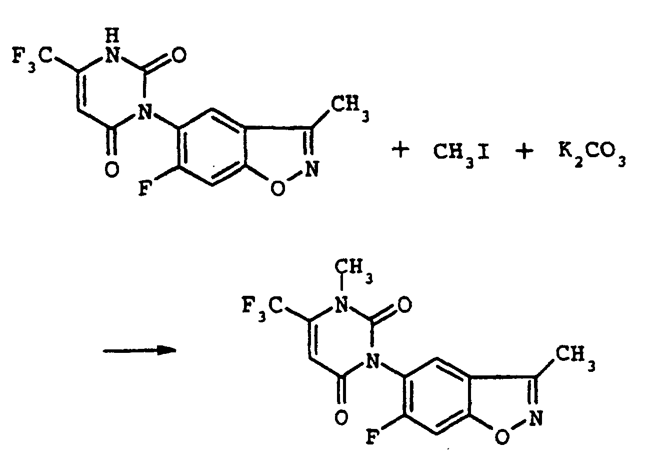

A mixture of 3-(6-fluoro-3-methyl-1,2-benzisoxazol-5-yl)-6-(trifluoromethyl)-2,4(1H,3H)-pyrimidinedione (3.00 g, 9.12 mmol) and potassium carbonate (2.52 g, 18.2 mmol) in N,N-dimethylformamide is stirred for 15 minutes, treated with methyl iodide (2.58 g, 18.2 mmol), stirred at room temperature overnight, and poured into an ice-water mixture. The resultant aqueous mixture is filtered to obtain a solid which is recrystallized from a methylene chloride/hexanes solution to give the title product as a white solid (mp 158-159 °C) which is identified by NMR spectral analyses.

Using essentially the same procedure, the following compounds are obtained:

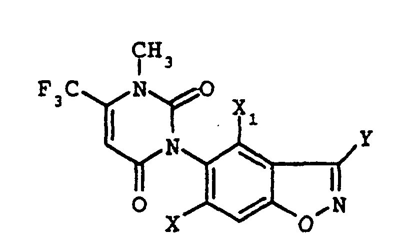

| **X** | **X**_{**1**} | **Y** | **mp °C** |
|---|---|---|---|
| H | H | CH₃ | 196-198 |
| H | H | Cl | 168.5-170 |
| H | H | OCH₂CO₂CH₃ | 156-157 |
| H | H | OCH(CH₃)₂ | |
| H | H | OCH₃ | 160-161 |
| F | Cl | CH₃ | 154-155 |

### EXAMPLE 30

### Preparation of 2'-Chloro-5'-nitroacetophenone, oxime

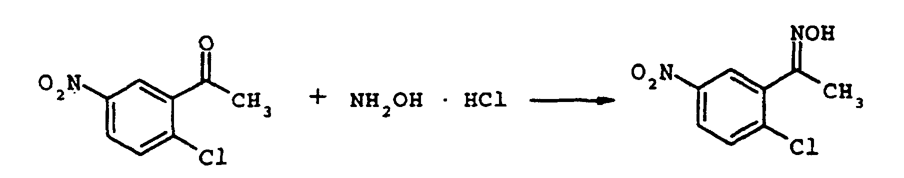

A mixture of 2'-chloro-5'-nitroacetophenone (50.0 g, 0.250 mol) in ethanol is treated with a solution of hydroxylamine hydrochloride (83.0 g, 1.19 mol) in water, refluxed overnight, cooled to room temperature, and filtered to give the title product as a solid (mp 165-167 °C) which is identified by NMR spectral analyses.

### EXAMPLE 31

### Preparation of 3-Methyl-5-nitro-1,2-benzisoxazole

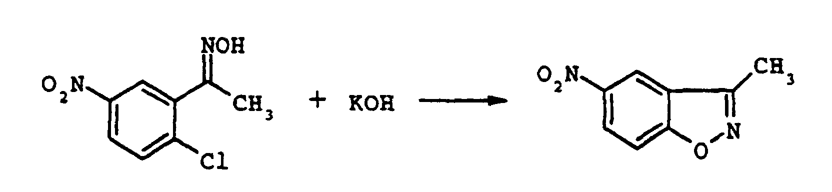

A mixture of 2'-chloro-5'-nitroacetophenone, oxime (30.0 g, 0.140 mol) in ethanol is treated dropwise with 10% potassium hydroxide solution (7.86 g KOH), stirred at room temperature for one hour, refluxed overnight, cooled, and poured into water. The resultant aqueous mixture is filtered to obtain a solid. Column chromatography of the solid using silica gel and methylene chloride gives the title product as a yellow solid (mp 84.5-86.5 °C) which is identified by NMR spectral analyses.

### EXAMPLE 32

### Preparation of 5-Nitro-1,2-benzisoxazol-3-ol

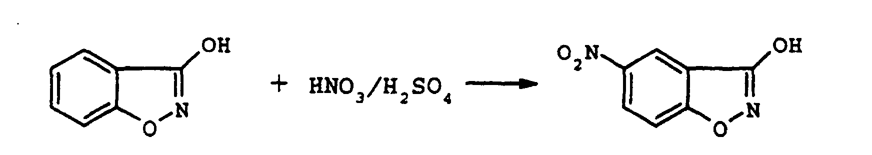

1,2-Benzisoxazol-3-ol (19.7 g, 0.146 mol) is added portionwise to concentrated sulfuric acid. The resultant reaction mixture is treated dropwise with 70% nitric acid (11.3 mL), stirred for 90 minutes, and poured onto ice. The resultant aqueous mixture is filtered to obtain a waxy paste. The paste is recrystallized from a methanol/water mixture to give the title product as a solid which is identified by ¹H NMR spectral analysis.

Using essentially the same procedure, the following compounds are obtained:

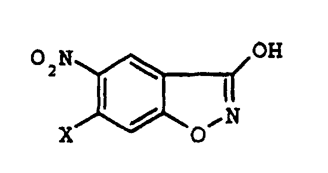

| |
|---|
| X |
| Cl |
| F |

### EXAMPLE 33

### Preparation of Methyl [(5-nitro-1,2-benzisoxazol-3-yl)oxy]acetate

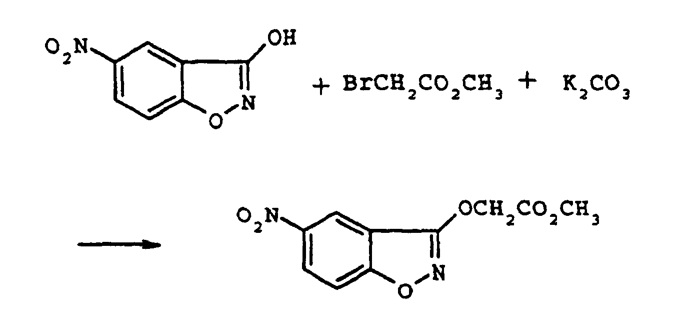

A mixture of 5-nitro-1,2-benzisoxazol-3-ol (3.90 g, 0.0220 mol) and potassium carbonate (4.17 g, 0.0300 mol) in N,N-dimethylformamide is stirred for 30 minutes, treated with methyl bromoacetate (3.96 g, 0.0260 mol), stirred overnight at room temperature, and poured into an acidic ice-water mixture. The resultant aqueous mixture is extracted with ethyl acetate. The organic extracts are combined, washed sequentially with water and brine, dried over anhydrous magnesium sulfate, and concentrated *in vacuo* to obtain a yellow oil. Column chromatography of the oil using silica gel and a (1:1) to (4:1) methylene chloride/hexanes gradient gives the title product as a white solid (2.80 g, mp 72-73.5 °C) which is identified by NMR spectral analyses.

Using essentially the same procedure, the following compounds are obtained:

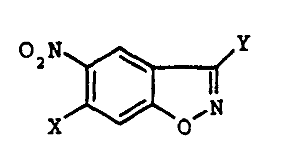

| **X** | **Y** | **mp °C** |
|---|---|---|
| H | OCH(CH₃)₂ | 81-83 |
| H | OCH₂CH=CH₂ | 70-72 |
| H | OCH₃ | 101.5-103 |
| Cl | OCH(CH₃)CO₂CH₃ | 98-100 |
| F | OCH₂CO₂CH₃ | 104-106 |

### EXAMPLE 34

### Preparation of 3-Chloro-5-nitro-1,2-benzisoxazole

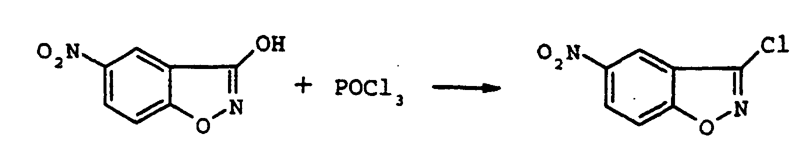

A mixture of 5-nitro-1,2-benzisoxazol-3-ol (4.00 g, 0.0220 mol) and phosphorus oxychloride (40.0 mL, 65.8 g, 0.429 mol) is placed in a glass bomb, heated at 150-155 °C for two hours, cooled overnight, concentrated *in vacuo,* diluted with methylene chloride, and brought to about pH 8 with sodium hydrogen carbonate solution. The phases are separated. The organic phase is washed sequentially with water and brine, dried over anhydrous magnesium sulfate, and concentrated *in vacuo* to obtain a residue. Column chromatography of the residue using silica gel and a methylene chloride/hexanes solution (1:1) gives the title product as an amber oil which is identified by NMR spectral analysis.

### EXAMPLE 35

### Preparation of Methyl 2-{{2-{5-[3,6-dihydro-3-methyl-2-oxo-6-thioxo-4-(trifluoromethyl)-1(2H)-pyrimidinyl]-1,2-benzisothiazol-3-yl}-p-tolyl}oxy}-propionate

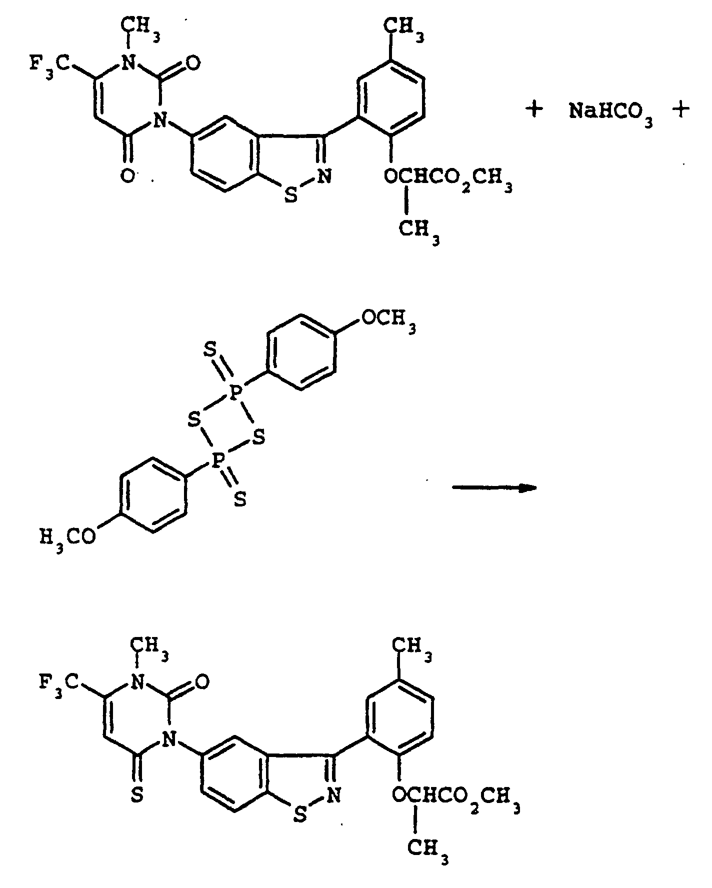

A mixture of methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate (2.00 g, 3.80 mmol), sodium hydrogen carbonate (1.24 g, 14.8 mmol) and Lawesson's Reagent (2,4-bis(4-methoxyphenyl)-1,3-dithia-2,4-diphosphetane-2,4-disulfide, 1.70 g, 4.20 mmol) in toluene is refluxed for two hours, filtered to remove solids, and concentrated *in vacuo* to obtain an orange foam. Column chromatography of the foam using silica gel, and 0% and 1% diethyl ether in methylene chloride solutions gives the title product as an orange foam (1.45 g, mp 110-112 °C) which is identified by NMR spectral analyses.

### EXAMPLE 36

### Preparation of 2-Chloro-2'-methoxy-5-nitrobenzophenone

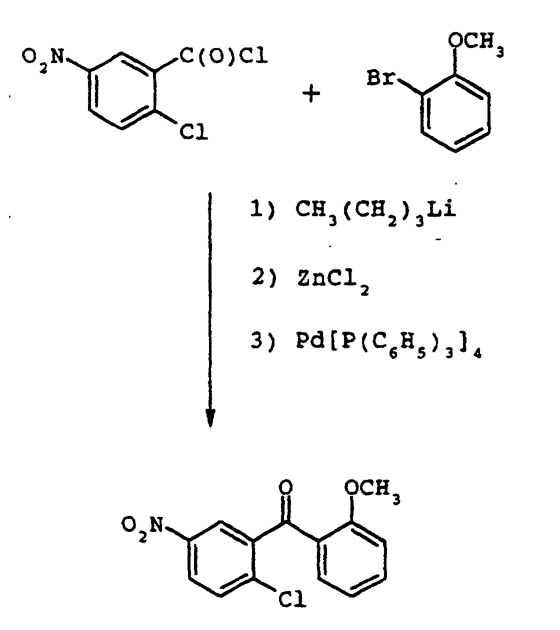

A solution of 2-bromoanisole (27.9 g, 145 mmol) in diethyl ether is cooled to -70 °C, treated with butyllithium (64.0 mL, 160 mmol), stirred at -70 °C for one hour, treated with 0.5 M zinc chloride in tetrahydrofuran solution (320 mL, 160 mmol), stirred for one hour at -70 °C, warmed to about 0 °C, and concentrated *in vacuo* to obtain a yellow-green oil. A solution of the oil in tetrahydrofuran is treated sequentially with tetrakis(triphenylphosphine)palladium(0) (5.00 g, 4.35 mmol) and a solution of 2-chloro-5-nitrobenzoyl chloride (35.0 g, 159 mmol) in tetrahydrofuran, stirred for three days, and poured into 10% hydrochloric acid. The resultant aqueous mixture is extracted with methylene chloride. The organic extracts are combined, washed sequentially with water and brine, dried over anhydrous magnesium sulfate, and concentrated *in vacuo* to obtain a semi-solid. The solid is triturated with diethyl ether to give the title product as a yellow solid which is identified by NMR spectral analyses.

Using essentially the same procedure, the following compounds are obtained:

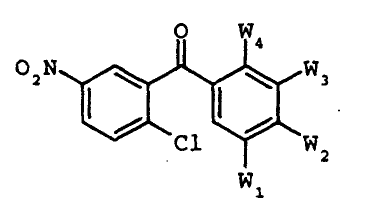

| **W**_{**1**} | **W**_{**2**} | **W**_{**3**} | **W**_{**4**} | **mp °C** |
|---|---|---|---|---|
| H | Cl | H | OCH₃ | 96-99 |
| H | H | CH₃ | OCH₃ | 71-74 |
| F | H | H | OCH₃ | |
| Cl | H | H | OCH₃ | 124-126 |
| OCH₃ | H | H | OCH₃ | 71-73 |
| H | OCH₃ | H | OCH₃ | 98-100 |
| H | F | H | OCH₃ | |
| H | H | CH₃ | H | 65-66.5 |
| H | H | SCH₃ | H | 87-88 |
| H | H | H | F | 118-120 |
| H | H | H | CH₃ | 78-79.5 |
| H | H | H | SCH₃ | 123-124.5 |
| H | F | H | H | |
| H | H | OCH₃ | H | |
| H | H | H | OCH₃ | |
| H | CH₃ | CH₃ | OCH₃ | |

### EXAMPLE 37

### Preparation of 2-Chloro-2'-methoxy-5-nitrobenzhydrol

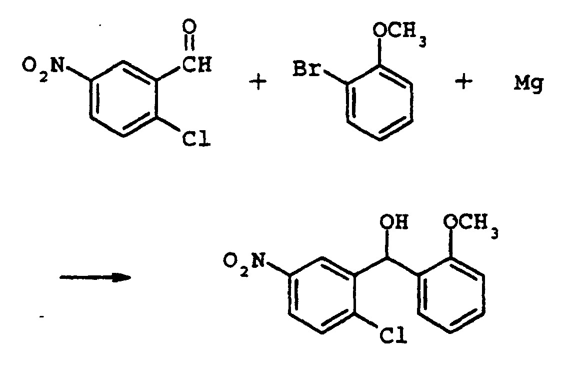

A solution of 2-bromoanisole (50.0 g, 0.267 mol) in ether is added portionwise to a mixture of magnesium (7.10 g, 0.293 mol) in ether. After the addition is complete, the reaction mixture is heated at reflux for one hour, diluted with ether, cooled to 0 °C, treated with a solution of 2-chloro-5-nitrobenzaldehyde (39.0 g, 0.210 mol) in tetrahydrofuran, warmed to room temperature, and diluted with an ice-water mixture. After acidifying the aqueous mixture with hydrochloric acid (pH 2 - pH 3), the organic phase is separated and the aqueous phase is extracted with ether. The organic extracts are combined, washed sequentially with 10% sodium hydrogen carbonate solution and brine, dried over anhydrous sodium sulfate, and concentrated *in vacuo* to give the title product as a brown gum.

Using essentially the same procedure, the following compounds are obtained:

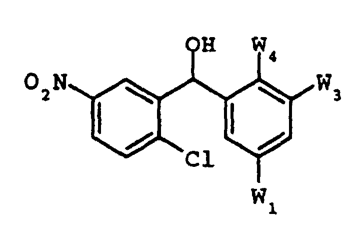

| **W** | **W**_{**3**} | **W**_{**4**} |
|---|---|---|
| OCH₃ | H | OCH₃ |
| CH₃ | H | OCH₃ |
| F | H | OCH₃ |
| H | OCH₃ | H |

### EXAMPLE 38

### Preparation of 2-Chloro-2'-methoxy-5-nitrobenzophenone

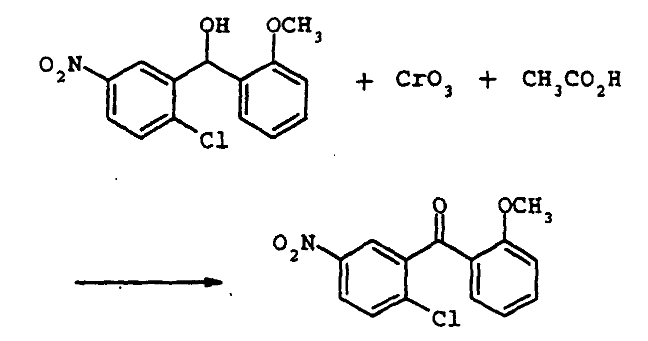

A solution of chromium(VI) oxide (91.0 g, 0.919 mol) in a water/acetic acid solution (1:4) is added portionwise to 2-chloro-2'-methoxy-5-nitrobenzhydrol (64.2 g, 0.219 mol) while maintaining the reaction mixture temperature at 25 °C to 35 °C. The reaction mixture is then stirred at 25 °C to 35 °C for one hour, cooled, diluted with water, and concentrated *in vacuo* to obtain a residue. The residue is diluted with water, and extracted with methylene chloride. The organic extracts are combined, dried over anhydrous sodium sulfate, mixed with silica gel (10 g), and filtered. The filtrate is concentrated *in vacuo* to obtain an oil. A solution of the oil in a methanol/water solution is decolorized with charcoal and concentrated *in vacuo* to yield a residue. Column chromatography of the residue using silica gel and methylene chloride/hexanes solutions gives the title product as a white solid.

Using essentially the same procedure, the following compounds are obtained:

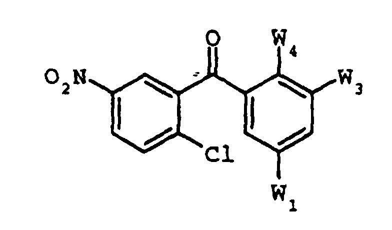

| **W**_{**1**} | **W**_{**3**} | **W**_{**4**} | **mp °C** |
|---|---|---|---|
| OCH₃ | H | OCH₃ | |
| CH₃ | H | OCH₃ | 109-111 |
| F | H | OCH₃ | 94-95 |
| H | OCH₃ | H | 79-81 |

### EXAMPLE 39

### Preparation of 2-{{2-{5-[3,6-dihydro-3-methyl-2,6-dioxo-4-(trifluoromethyl)-1(2H)-pyrimidinyl]-1,2-benzisothiazol-3-yl}-p-tolyl}oxy}propionic acid

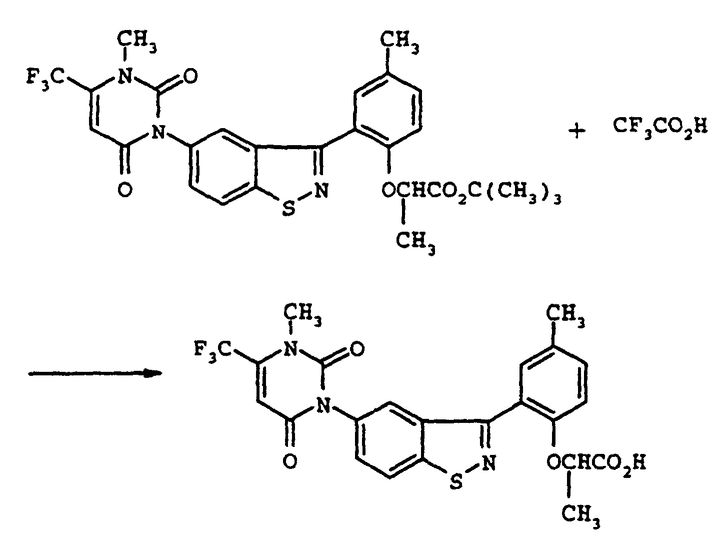

Trifluoroacetic acid (75 mL) is added to a solution of tert-butyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate (12.0 g, 21.4 mmol) in methylene chloride. The reaction mixture is stirred at room temperature for 24 hours and concentrated *in vacuo* to give the title product as a yellow foam which is identified by NMR spectral analyses.

Using essentially the same procedure, the following compounds are obtained:

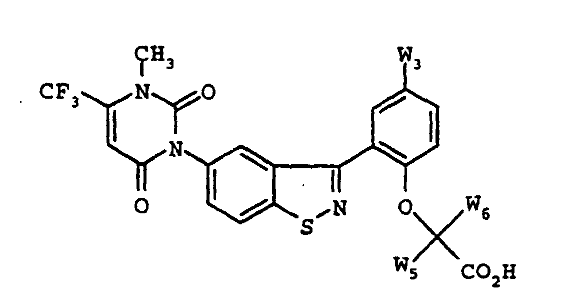

| **W**_{**3**} | **W**_{**5**} | **W**_{**6**} | **mp °C** |
|---|---|---|---|
| CH₃ | CH₃ | CH₃ | |
| H | H | H | 152-158 |
| H | CH₃ | H | 215-216 |
| CH₃ | H | H | |

Using essentially the same procedure, the following compound is also obtained:

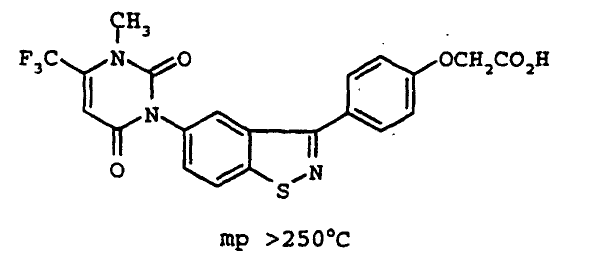

### EXAMPLE 40

### Preparation of 2-Propynyl 2-{{2-{5-[3,6-dihydro-3-methyl-2,6-dioxo-4-(trifluoromethyl)-1(2H)-pyrimidinyl]-1,2-benzisothiazol-3-yl}-p-tolyl}oxy}propionate

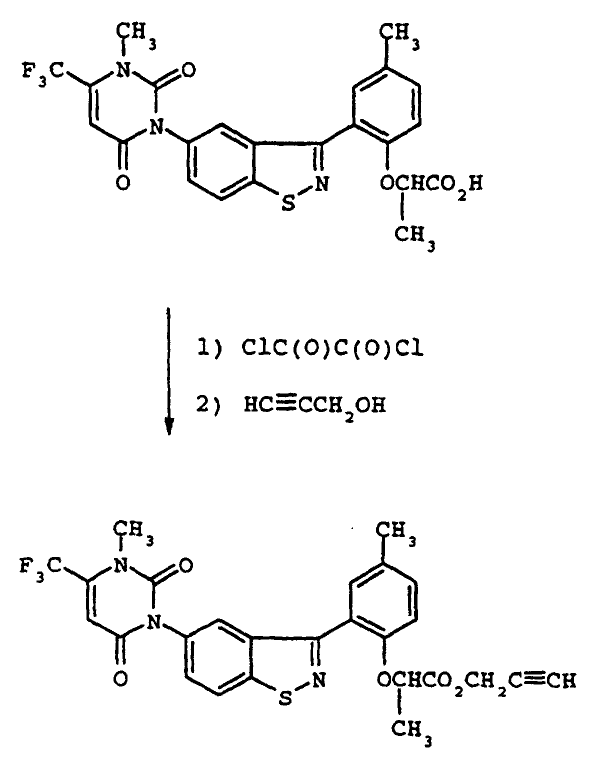

A mixture of 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionic acid (0.510 g, 1.00 mmol) and oxalyl chloride (0.380 g, 3.00 mmol) in methylene chloride is stirred at room temperature for 30 minutes and concentrated *in vacuo* to obtain a yellow foam. The foam is diluted with methylene chloride and the resultant solution is treated with a solution of propargyl alcohol (0.110 g, 2.00 mmol) in methylene chloride, stirred at room temperature for 24 hours, and concentrated *in vacuo* to obtain an oil. Flash column chromatography of the oil using silica gel, a hexanes/methylene chloride solution (1:1) and a 5% diethyl ether in methylene chloride solution gives the title product (0.400 g) which is identified by NMR spectral analyses.

Using essentially the same procedure, the following compounds are obtained:

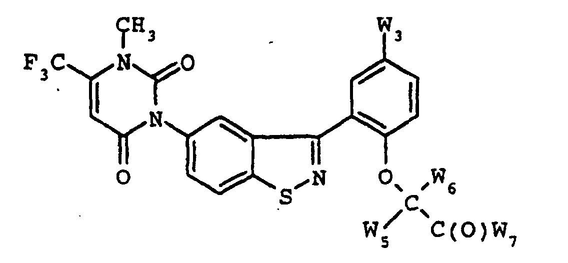

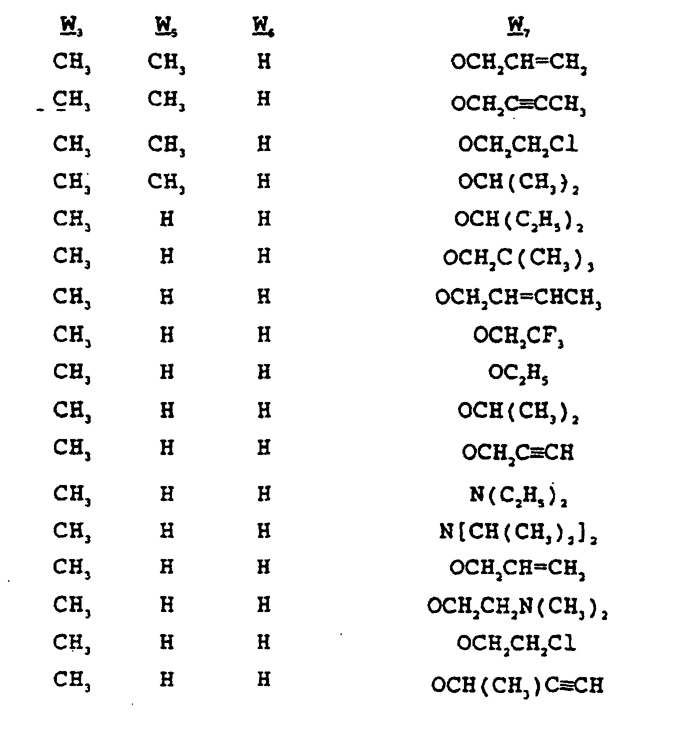

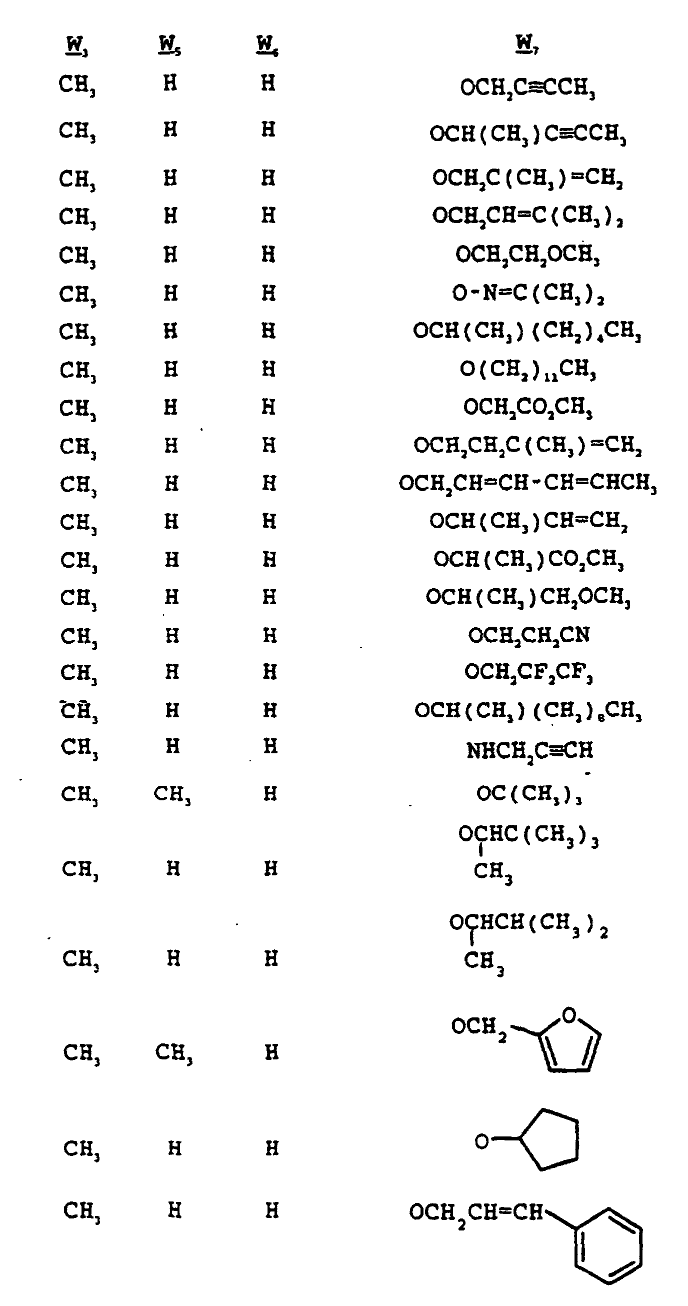

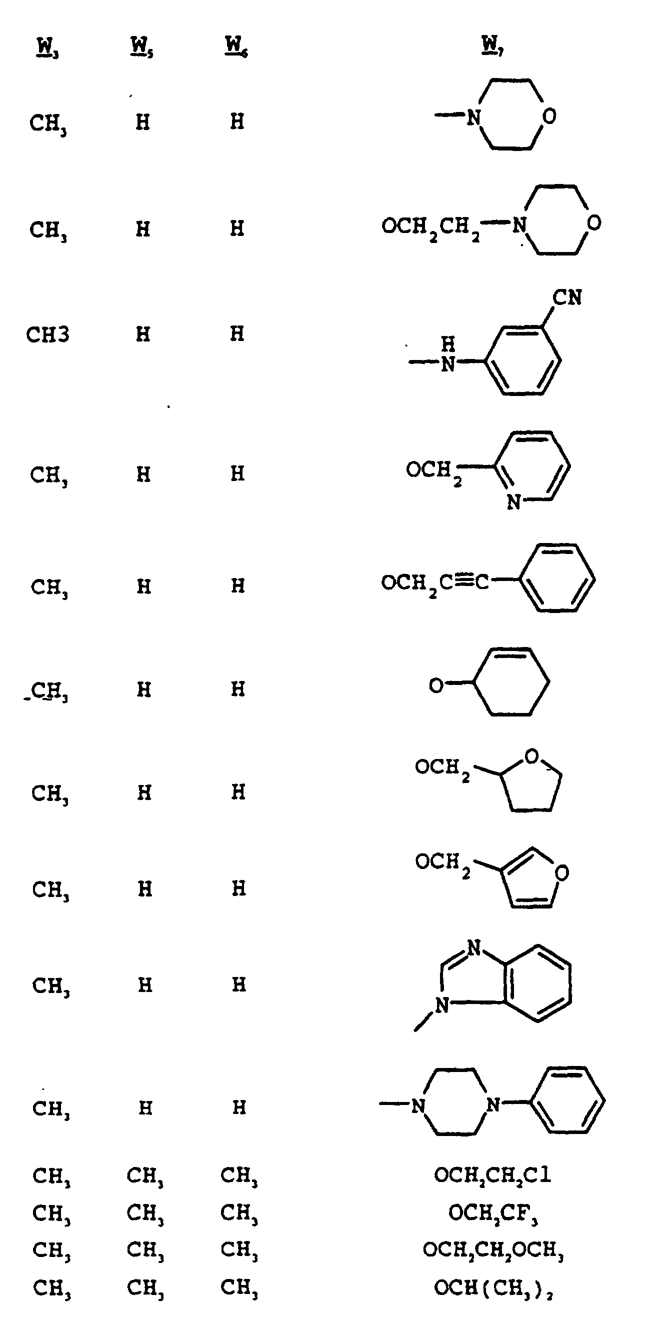

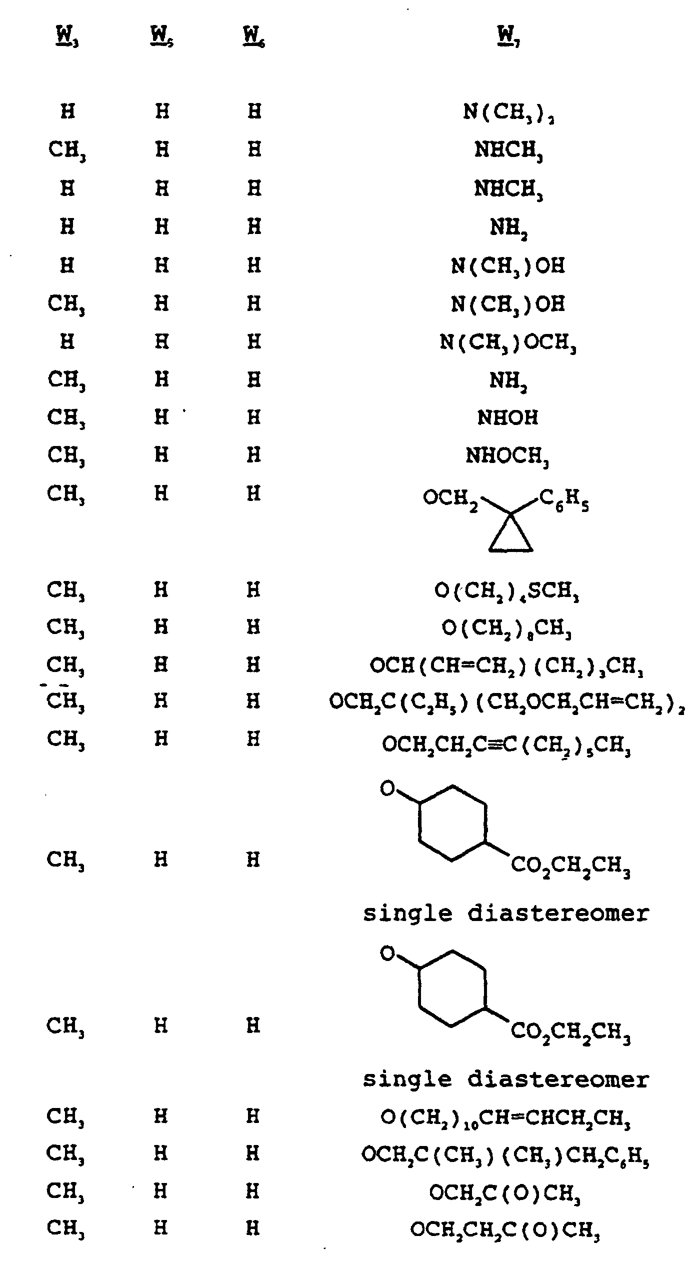

### EXAMPLE 41

### Preparation of 2-Chloro-4-fluoro-5-nitrobenzoyl chloride

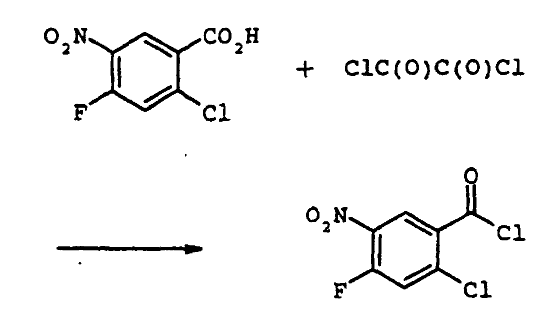

A mixture of 2-chloro-4-fluoro-5-nitrobenzoic acid (50.0 g, 0.228 mol) and N,N-dimethylformamide (5 drops) in 1,2-dichloroethane is treated dropwise with oxalyl chloride (30.8 mL, 0.353 mol), refluxed for 3 hours, cooled, and concentrated *in vacuo* to obtain the title product as an orange solid which is identified by NMR spectral analyses.

### EXAMPLE 42

### Preparation of 2'-Chloro-4'-fluoro-5'-nitroacetophenone

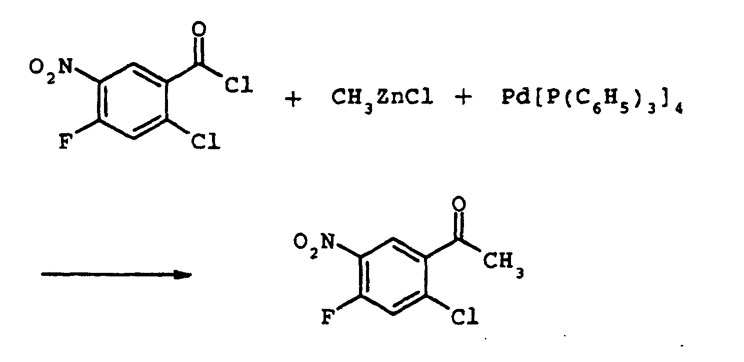

A 2 M solution of methylzinc chloride in tetrahydrofuran (5.00 mL, 10.1 mmol) is treated dropwise with a solution of 2-chloro-4-fluoro-5-nitrobenzoyl chloride (2.00 g, 8.40 mmol) in tetrahydrofuran, treated with tetrakis(triphenylphosphine)palladium(0) (0.400 g, 0.350 mmol), stirred at room temperature for one hour, and poured into 3 N hydrochloric acid. The resultant aqueous mixture is extracted with ethyl acetate. The organic extracts are combined, washed sequentially with water and saturated sodium hydrogen carbonate solution, dried over anhydrous magnesium sulfate, and concentrated *in vacuo* to obtain a dark liquid. Flash column chromatography of the liquid using silica gel and a methylene chloride in hexanes solution (6:4) gives the title product as an off-white solid (mp 66-68 °C) which is identified by NMR spectral analyses.

### EXAMPLE 43

### Preparation of 6-Amino-3-methyl-5-nitro-1,2-benzisothiazole

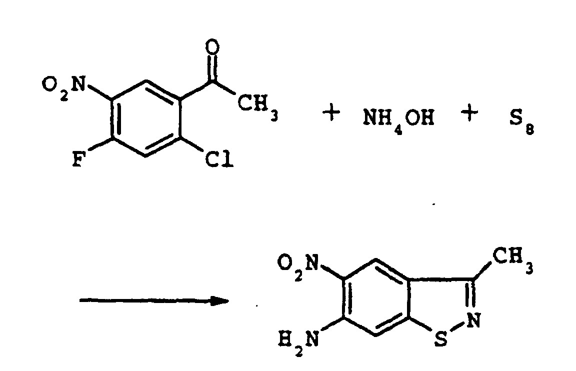

A mixture of 2'-chloro-4'-fluoro-5'-nitroacetophenone (12.0 g, 0.0552 mol), sulfur (1.77 g, 0.0552 mol), 30% ammonium hydroxide solution (100 mL, 0.856 mol), and methanol is placed in a steel bomb, heated at 85 °C overnight, cooled, treated with additional sulfur (0.270 g) and 30% ammonium hydroxide solution (50 mL), heated at 85 °C overnight, cooled, filtered to remove solids, and extracted with ethyl acetate. The organic extracts are combined, washed sequentially with water and brine, dried over anhydrous magnesium sulfate, and concentrated *in vacuo* to obtain a solid. Flash column chromatography of the solid using silica gel, and 0%, 1% and 2% diethyl ether in methylene chloride solutions gives the title product as an orange solid (4.19 g, mp 189-191 °C) which is identified by NMR spectral analyses.

Using essentially the same procedure, the following compound is obtained:

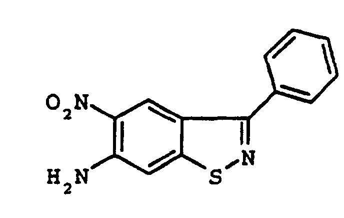

### EXAMPLE 44

### Preparation of 6-Chloro-3-methyl-5-nitro-1,2-benzisothiazole

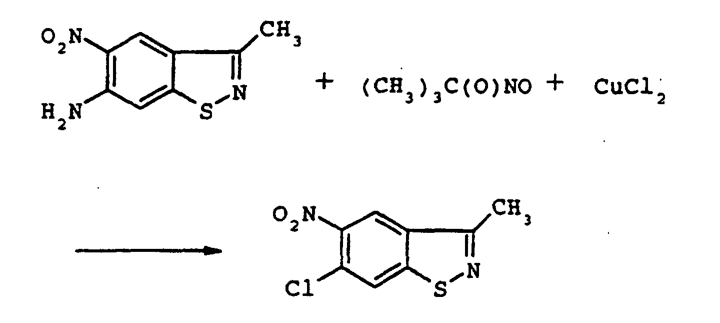

A mixture of tert-butyl nitrite (3.30 mL, 0.0278 mol) and copper(II) chloride (2.98 g, 0.0222 mol) in acetonitrile is heated to 65 °C, treated portionwise with 6-amino-3-methyl-5-nitro-1,2-benzisothiazole (3.88 g, 0.0185 mol), stirred at 65 °C, cooled to room temperature, and poured into 20% hydrochloric acid. The resultant aqueous mixture is extracted with ethyl acetate. The organic extracts are combined, washed with 20% hydrochloric acid, dried over anhydrous magnesium sulfate, and concentrated *in vacuo* to obtain a solid. Flash column chromatography of the solid using silica gel and methylene chloride/hexanes solutions (1:1 and 3:1) gives the title product as a pale, yellow solid (2.54 g, mp 156-158 °C) which is identified by NMR spectral analyses.

Using essentially the same procedure, the following compound is obtained:

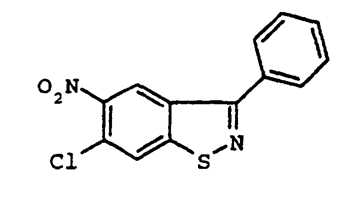

### EXAMPLE 45

### Preparation of 6-Fluoro-3-methyl-5-nitro-1,2-benzisothiazole

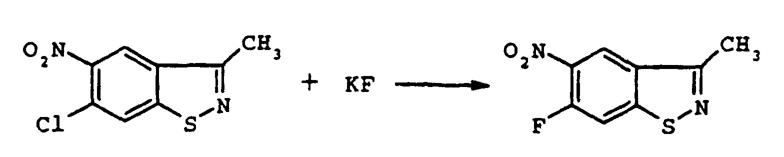

A mixture of 6-chloro-3-methyl-5-nitro-1,2-benzisothiazole (2.25 g, 9.80 mmol), potassium fluoride (2.85 g, 49.0 mmol), and 18-crown-6 (1.50 g, 5.70 mmol) in acetonitrile is heated in a sealed tube for 29 days, filtered to remove solids, and partially concentrated *in vacuo* to obtain a liquid. The liquid is diluted with ethyl acetate, washed sequentially with water and brine, dried over anhydrous magnesium sulfate, and concentrated *in vacuo* to obtain a dark, brown solid. Flash column chromatography of the solid using silica gel and a 10% to 50% ethyl acetate in hexanes gradient gives a yellow solid containing two components. Flash column chromatography of the yellow solid using silica gel and a 50% to 70% methylene chloride in hexanes gradient gives the title product as a pale, yellow solid (0.870 g, mp 118-119 °C) which is identified by NMR spectral analyses.

Using essentially the same procedure, the following compound is obtained:

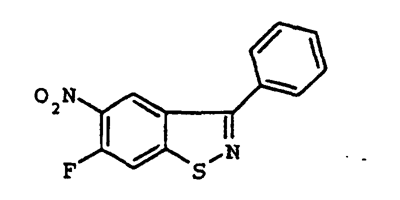

### EXAMPLE 46

### Preparation of 5-Amino-6-fluoro-3-methyl-1,2-benzisothiazole

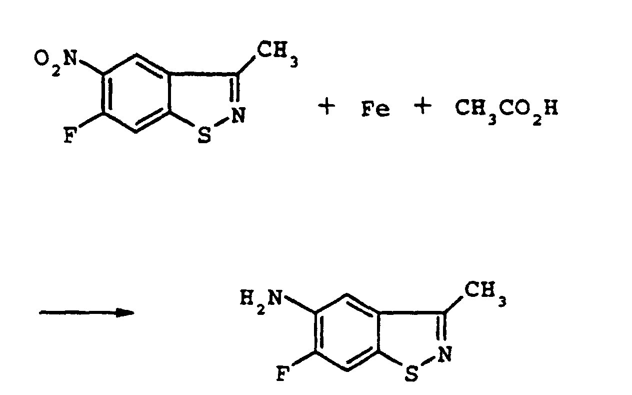

A solution of 6-fluoro-3-methyl-5-nitro-1,2-benzisothiazole (0.740 g, 3.50 mmol), 5% acetic acid (25.0 mL) and ethyl acetate is heated to 65 °C, treated with iron powder (0.980 g, 17.5 mmol), stirred at 65 °C for one hour, cooled to room temperature, and filtered to remove solids. The organic phase is separated, washed sequentially with water, saturated sodium hydrogen carbonate solution and brine, dried over anhydrous magnesium sulfate, and concentrated *in vacuo* to give the title product as an orange solid (0.610 g) which is identified by NMR spectral analyses.

Using essentially the same procedure, the following compound is obtained:

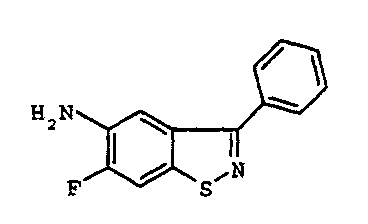

### EXAMPLE 47

### Preparation of 2-Chloro-4-fluoro-5-nitrobenzophenone

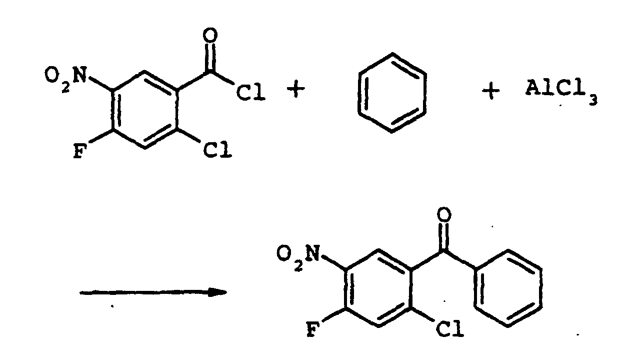

A solution of 2-chloro-4-fluoro-5-nitrobenzoyl chloride (26.7 g, 0.112 mol) and benzene (12.0 mL, 0.134 mol) in 1,1,2,2-tetrachloroethane is cooled to 0 °C to 5 °C, treated with aluminum chloride (18.1 g, 0.136 mol), stirred-for 15 minutes at about 8 °C, heated to and stirred at 50 °C for one hour, cooled to room temperature, and diluted sequentially with an ice-water mixture and concentrated hydrochloric acid. The organic phase is separated, washed sequentially with water and saturated sodium hydrogen carbonate solution, dried over anhydrous magnesium sulfate, and concentrated *in vacuo* to obtain a solid. Flash column chromatography of the solid using silica gel and methylene chloride gives the title product as an orange solid (30.8 g) which is identified by NMR spectral analyses.

### EXAMPLE 48

### Preparation of Methyl 2-{{2-{5-[3,6-dihydro -3-methyl-2,6-dioxo-4-(trifluoromethyl)-1(2H)-pyrimidinyl]-1,2-benzisothiazol-3-yl}-p-tolyl}oxy}propionate, S,S-dioxide

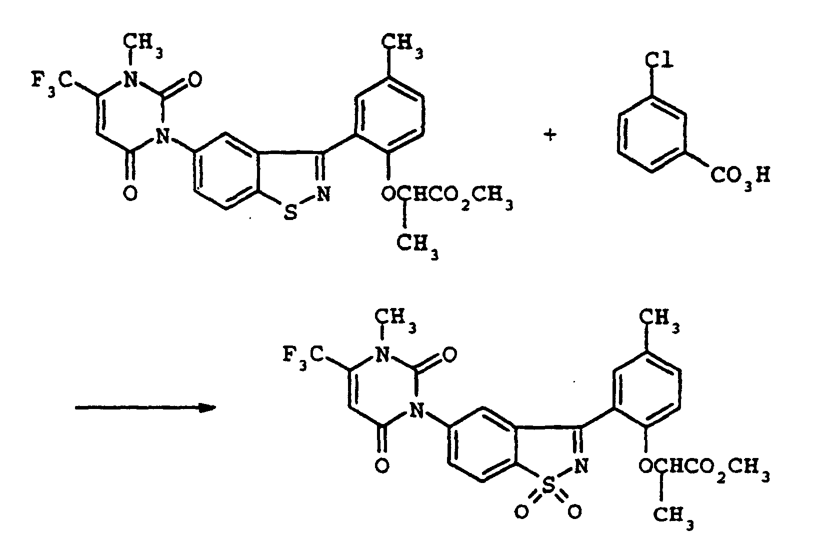

A mixture of methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate (2.00 g) and 3-chloroperoxybenzoic acid (2.00 g) in chloroform (50.0 mL) is stirred at room temperature overnight and diluted with saturated sodium hydrogen carbonate solution (100 mL). The organic phase is separated, dried over anhydrous sodium sulfate, concentrated *in vacuo,* diluted with methanol (15.0 mL), heated, cooled to room temperature and filtered to obtain a solid. The solid is washed with methanol and air-dried to give the title product as a yellow powder (1.50 g, mp 225-226 °C).

### EXAMPLE 49

### Preparation of 3-{3-[5-(Bromomethyl)-2-methoxyphenyl]-1,2-benzisothiazol-5-yl}-1-methyl-6-(trifluoromethyl)-2,4(1H,3H)pyrimidinedione and 3-{5-[3,6-Dihydro-3-methyl-2,6-dioxo-4-(trifluoromethyl)-1(2H)-pyrimidinyl]-1,2-benzisothiazol-3-yl}-4-methoxybenzaldehyde

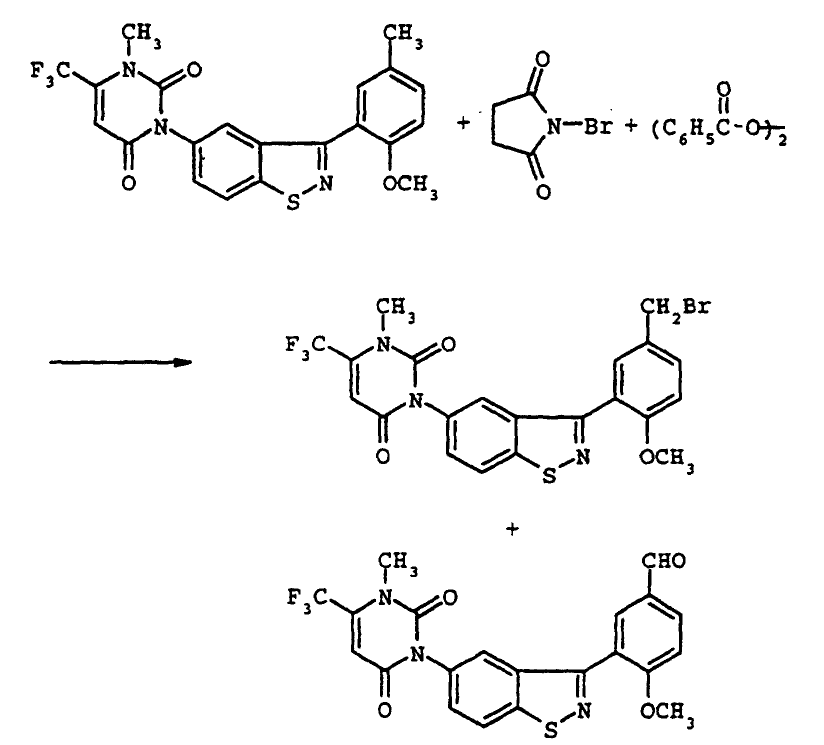

A mixture of 3-[3-(6-methoxy-*m*-tolyl)-1,2-benzisothiazol-5-yl]-1-methyl-6-(trifluoromethyl)-2,4(1H,3H)-pyrimidinedione (5.00 g, 11.2 mmol), N-bromosuccinimide (3.00 g, 16.9 mmol), and benzoyl peroxide (0.460 g, 1.90 mmol) in carbon tetrachloride is refluxed overnight, cooled to room temperature, diluted with methylene chloride, and filtered to remove solids. The filtrate is washed sequentially with 5% sodium *m*-bisulfate solution and 5% sodium bicarbonate solution, dried over anhydrous magnesium sulfate, and concentrated *in vacuo* to obtain an amber oil. Column chromatography of the oil using silica gel and a hexanes/ethyl acetate solution (9:1) gives 3-{3-[5-(bromomethyl)-2-methoxyphenyl]-1,2-benzisothiazol-5-yl]-1-methyl-6-(trifluoromethyl)-2,4(1H,3H)-pyrimidinedione as a light yellow solid (0.700 g, mp 158-160 °C) and 3-{5-[3,6-dihydro-3-methyl-2,6-dioxo-4-(trifluoromethyl)-1(2H)-pyrimidinyl]-1,2-benzisothiazol-3-yl}-4-methoxybenzaldehyde as a light yellow solid (1.50 g, mp 118-120 °C).

### EXAMPLE 50

### Preparation of {{5-[3,6-Dihydro-3-methyl-2,6-dioxo-4-(trifluoromethyl)-1(2H)-pyrimidinyl]-1,2-benzisothiazol-3-yl}methyl}trimethylammonium bromide

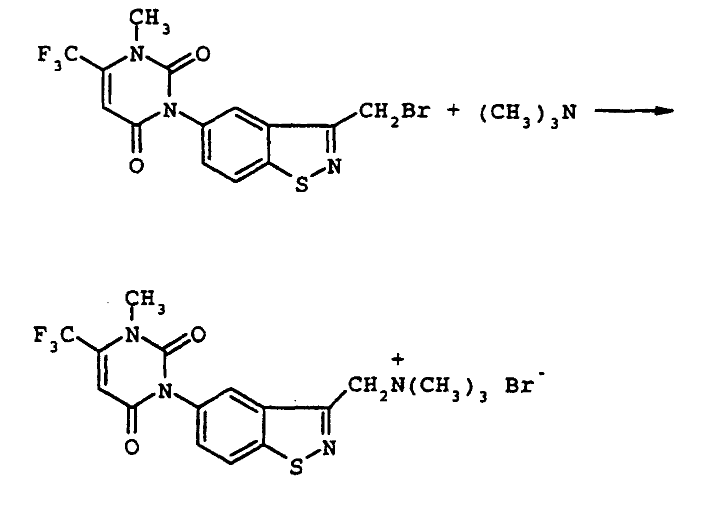

A mixture of 3-[3-(bromomethyl)-1,2-benzisothiazol-5-yl]-1-methyl-6-(trifluoromethyl)-2,4(1H,3H)-pyrimidinedione (20.0 g, 0.0480 mol) and toluene is heated to 90 °C, cooled to room temperature, treated with liquid trimethylamine (8.60 g, 0.145 mol), stirred at room temperature for 90 minutes, and filtered to obtain a solid. The solid is washed with toluene and dried in a vacuum oven at 45-50 °C to give the title product as a white solid which is identified by NMR spectral analysis.

Using essentially the same procedure, but using the appropriate secondary amine, the following compounds are obtained:

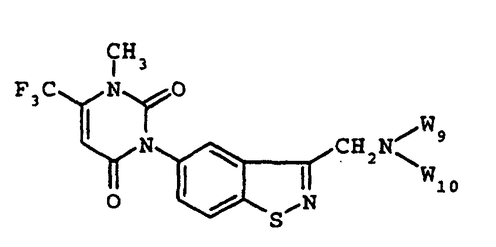

| **W**_{**9**} | **W**_{**10**} | **mp °C** |
|---|---|---|
| CH₃ | CH₂CO₂C₂H₅ | 119-121 |
| CH₃ | CH₃ | 200-201 |
| -CH=CH-N=CH- | | 206-207 |
| H | SO₂CH₃ | 119-121 |

### EXAMPLE 51

### Preparation of N-{{{2-{5-[3,6-Dihydro-3-methyl-2,6-dioxo-4-(trifluoromethyl)-1(2H)-pyrimidinyl]-1,2-benzisothiazol-3-yl}-p-tolyl}oxy}acetyl}-p-toluenesulfonamide

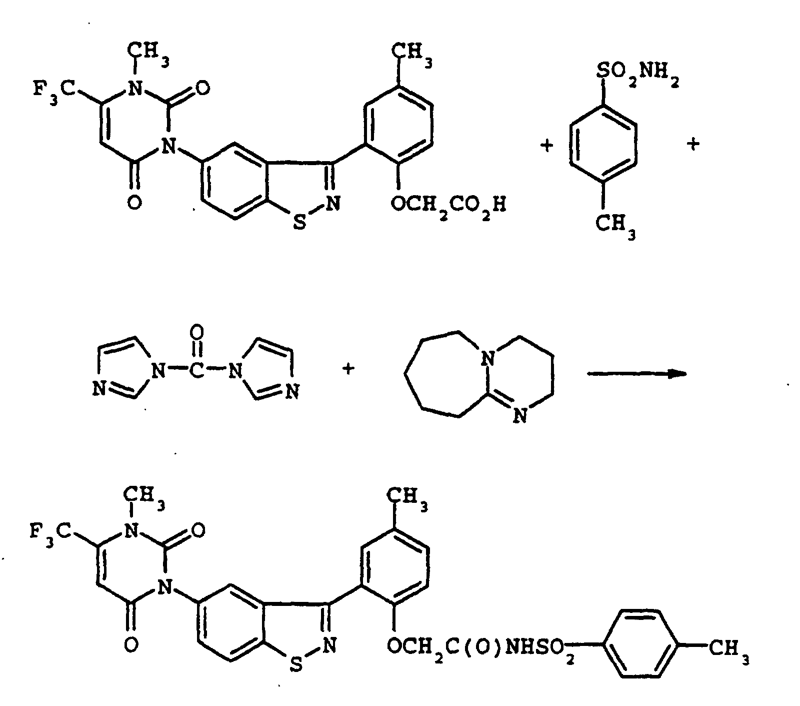

A mixture of 1,1'-carbonyldiimidazole (0.198 g, 1.22 mmol) and {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetic acid (0.300 g, 0.610 mmol) in tetrahydrofuran is stirred overnight at room temperature, refluxed for 30 minutes, cooled to room temperature, treated sequentially with a solution of *p*-toluenesulfonamide (0.209 g, 1.22 mmol) in tetrahydrofuran and a solution of 1,8-diazabicyclo[5.4.0]undec-7-ene (0.204 g, 1.34 mmol) in tetrahydrofuran, stirred at room temperature for three hours, and poured into 10% hydrochloric acid. The resulting aqueous mixture is filtered to obtain a solid. The solid is recrystallized from an acetonitrile/water solution to give the title product as an off-white solid (0.200 g, mp 140-144 °C).

Using essentially the same procedure, the following compounds are obtained:

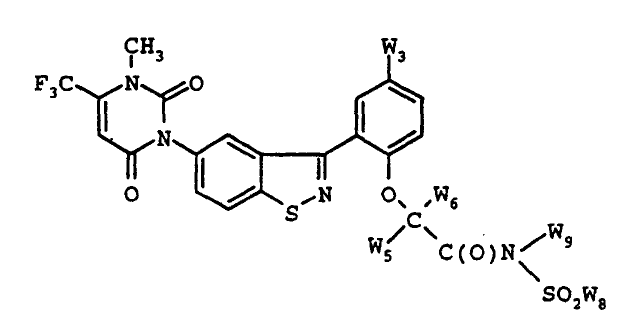

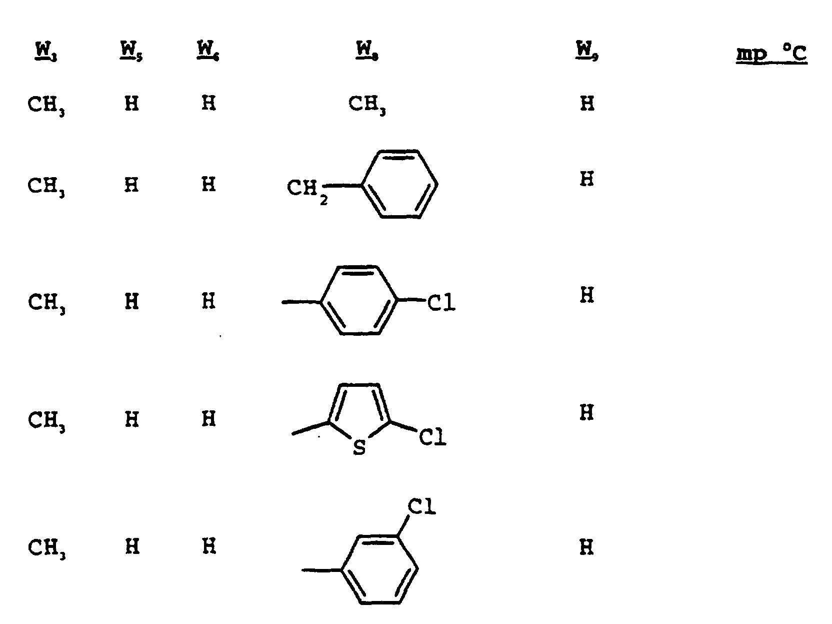

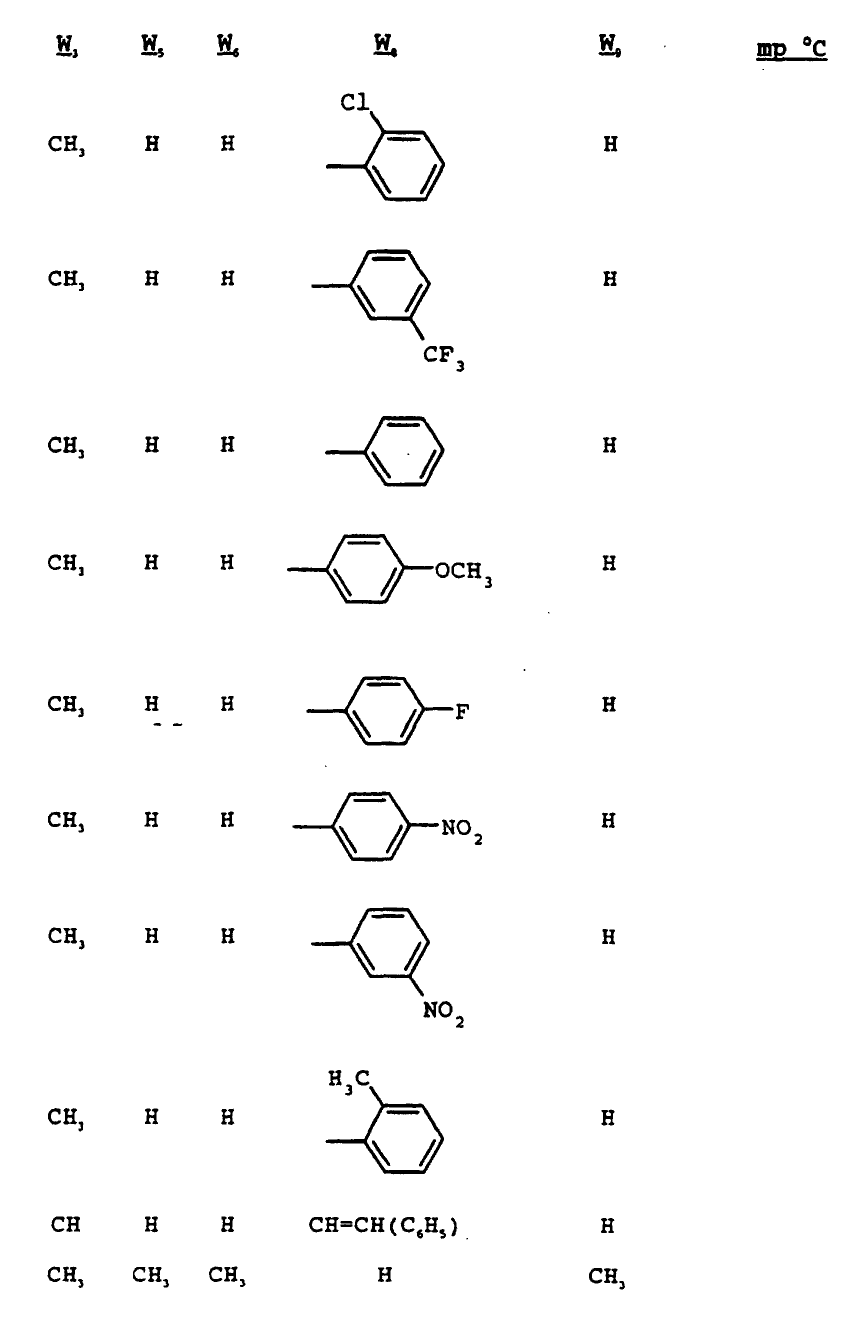

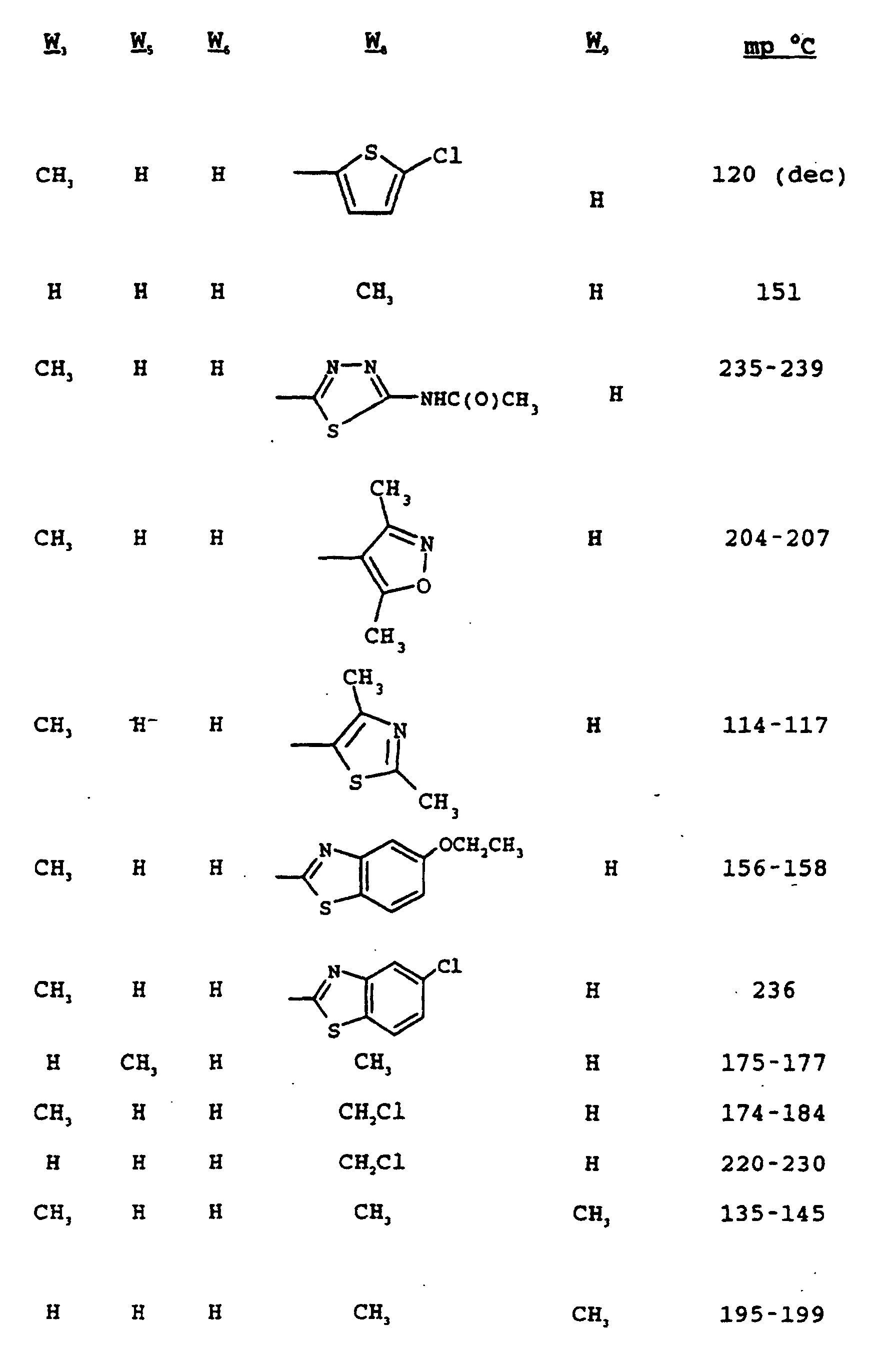

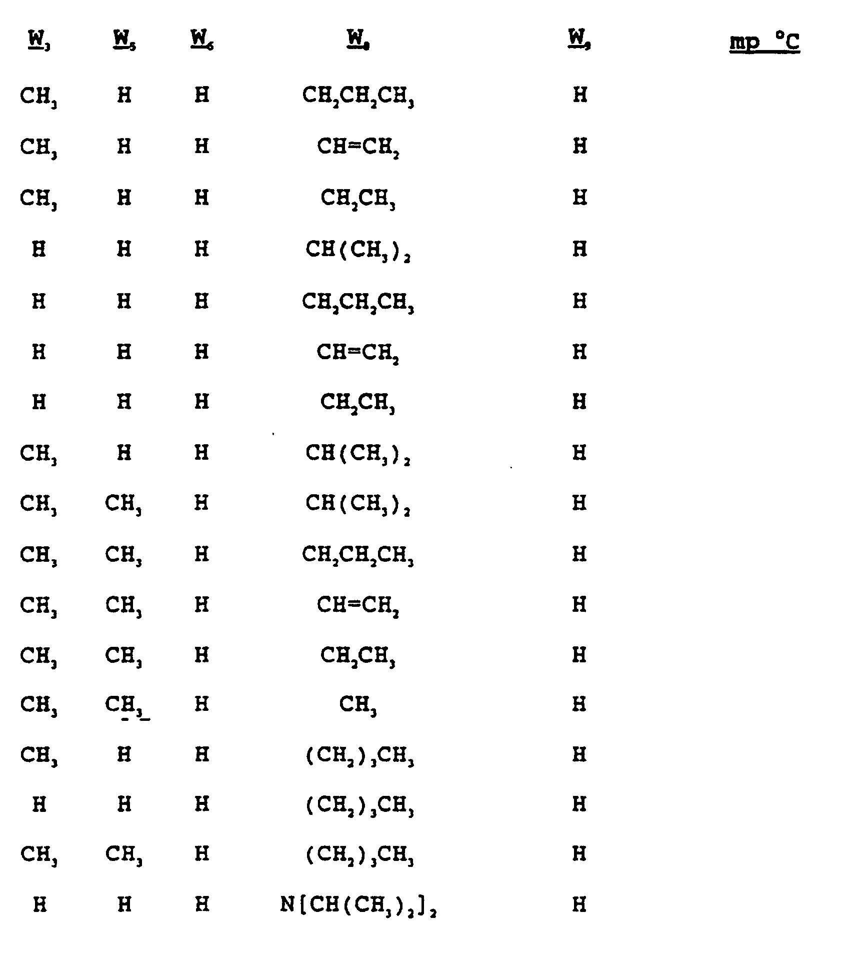

### EXAMPLE 52

### Preparation of 5-[3,6-Dihydro-3-methyl-2,6-dioxo-4-(trifluoromethyl)-1(2H)-pyrimidinyl]-1,2-benzisothiazole-3-carboxaldehyde

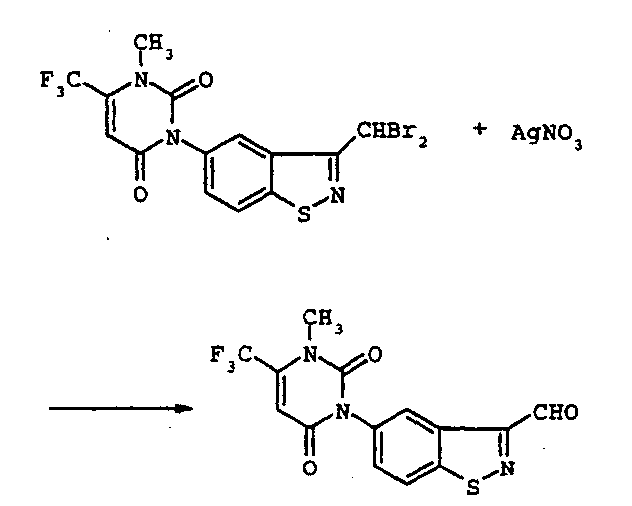

A solution of 3-[3-(dibromomethyl)-1,2-benzisothiazol-5-yl]-1-methyl-6-(trifluoromethyl)-2,4(1H,3H)-pyrimidinedione (0.500 g, 0.00100 mol) in a dioxane/water mixture (5:2) is treated with silver nitrate (0.340 g, 0.00200 mol), refluxed for 90 minutes, cooled to and stirred at room temperature overnight, refluxed for three hours, cooled, and filtered through a pad of diatomaceous earth. The resultant filtrate is concentrated *in vacuo* to obtain an aqueous mixture. The aqueous mixture is extracted with methylene chloride. The organic extract is washed sequentially with water and brine, dried over anhydrous magnesium sulfate, and concentrated *in vacuo* to obtain a solid. Column chromatography of the solid using silica gel and methylene chloride gives the title product as a solid (0.240 g, mp 193-194.5 °C).

Using essentially the same procedure on 3-[6-fluoro-3-(dibromomethyl)-1,2-benzisothiazol-5-yl]-1-methyl-6-(trifluoromethyl)-2,4(1H,3H)-pyrimidinedione, 5-[3,6-dihydro-3-methyl-2,6-dioxo-4-(trifluoromethyl)-1(2H)-pyrimidnyl-6-fluoro-1,2-benzisothiazole-3-carboxaldehyde is obtained.

### EXAMPLE 53

### Preparation of 1-Methyl-3-{3-[(methylsulfinyl)-methyl]-1,2-benzisothiazol-5-yl}-6-(trifluoromethyl)-2,4(1H,3H)-pyrimidinedione

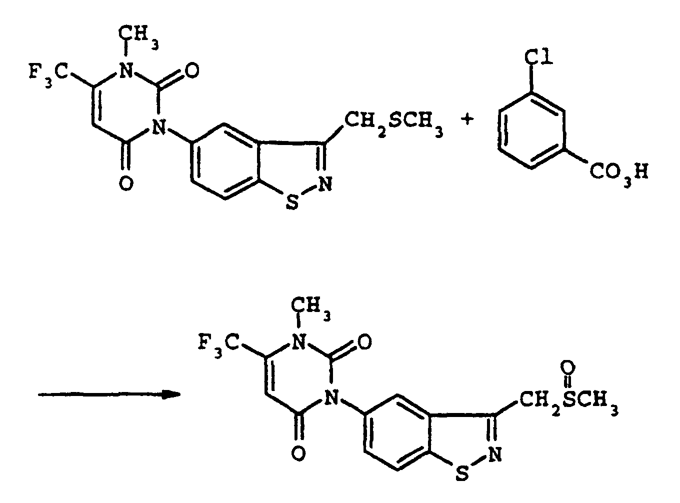

A solution of 1-methyl-3-{3-[(methylthio)methyl]-1,2-benzisothiazol-5-yl}-6-(trifluoromethyl)-2,4(1H,3H)-pyrimidinedione (0.740 g, 1.90 mmol) in methylene chloride is treated with 3-chloroperoxybenzoic acid (0.580 g, 60%, 2.00 mmol), stirred at room temperature for five minutes, diluted with additional methylene chloride, washed with 5% sodium carbonate solution, dried over anhydrous magnesium sulfate, and concentrated *in vacuo* to obtain a solid. Flash column chromatography of the solid using silica gel and a 10% ethyl acetate in methylene chloride solution gives the title product as a white solid which is identified by NMR spectral analysis.

Using essentially the same procedure, 1-methyl-3-{3-[(phenylsulfinyl)methyl]-1,2-benzisothiazol-5-yl}-6-(trifluoromethyl)-2,4(1H,3H)-pyrimidinedione is obtained from 1-methyl-3-{3-[(phenylthio)methyl]-1,2-benzisothiazol-5-yl]-6-(trifluoromethyl)-2,4(1H,3H)-pyrimidinedione.

### EXAMPLE 54

### Preparation of 1-Methyl-3-{3-[(methylsulfonyl)-methyl]-1,2-benzisothiazol-5-yl]-6-(trifluoromethyl)-2,4(1H,3H)-pyrimidinedione

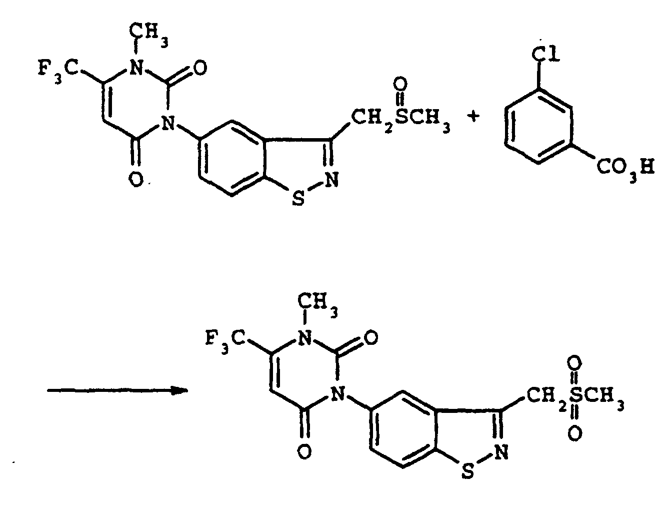

A mixture of 1-methyl-3-{3-[(methylsulfinyl)methyl]-1,2-benzisothiazol-5-yl}-6-(trifluoromethyl)-2,4(1H,3H)-pyrimidinedione (0.480 g, 1.24 mmol) and 3-chloroperoxybenzoic acid (0.600 g, 60%, 2.10 mmol) in methylene chloride is stirred at room temperature for four hours, washed with 5% sodium carbonate solution, dried over anhydrous magnesium sulfate, and concentrated *in vacuo* to obtain a solid. Flash column chromatography of the solid using silica gel and a 10% diethyl ether in methylene chloride solution gives the title product as a white solid which is identified by NMR spectral analyses.

Using essentially the same procedure, 1-methyl-3-{3-[(phenylsulfonyl)methyl]-1,2-benzisothiazol-5-yl}-6-(trifluoromethyl)-2,4(1H,3H)-pyrimidinedione is obtained from 1-methyl-3-{3-[(phenylsulfinyl)methyl]-1,2-benzisothiazol-5-yl}-6-(trifluoromethyl)-2,4(1H,3H)-pyrimidinedione.

### EXAMPLE 55

### Preparation of 2-Ethoxy-3-[3-(6-hydroxy-m-tolyl)-1,2-benzisothiazol-5-yl]-6-(trifluoromethyl)-4(3H)-pyrimidinone

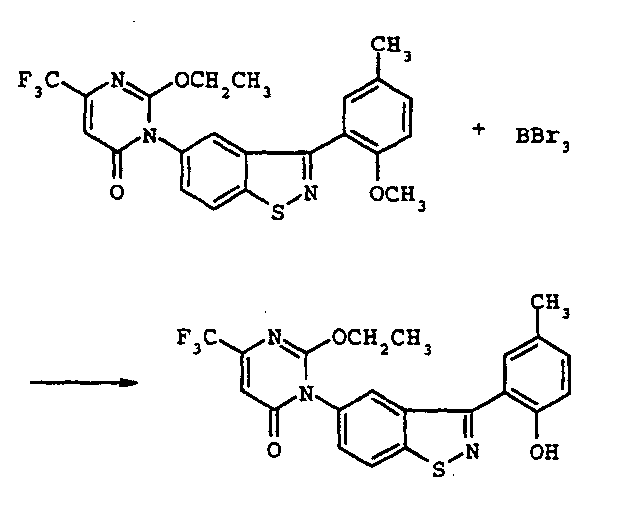

A solution of 2-ethoxy-[3-(6-methoxy-*m*-tolyl)-1,2-benzisothiazol-5-yl]-6-(trifluoromethyl)-4(3H)-pyrimidinone (0.490 g, 1.06 mmol) in methylene chloride is cooled to -5 °c, treated with a 1 M solution of boron tribromide in methylene chloride (1.38 mL, 1.38 mmol), stirred for six hours, washed sequentially with water and brine, dried over anhydrous magnesium sulfate, and concentrated *in vacuo* to give the title product as an off-white solid which is identified by NMR spectral analysis.

Using essentially the same procedure, the following compound is obtained:

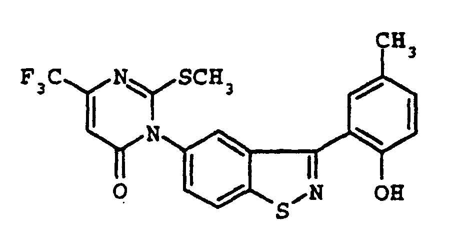

### EXAMPLE 56

### Preparation of Methyl {{2-{5-{2-ethoxy-6-oxo-4-(trifluoromethyl)-1(6H)-pyrimidinyl]-1,2-benzisothiazol-3-yl}-p-tolyl}oxy}acetate

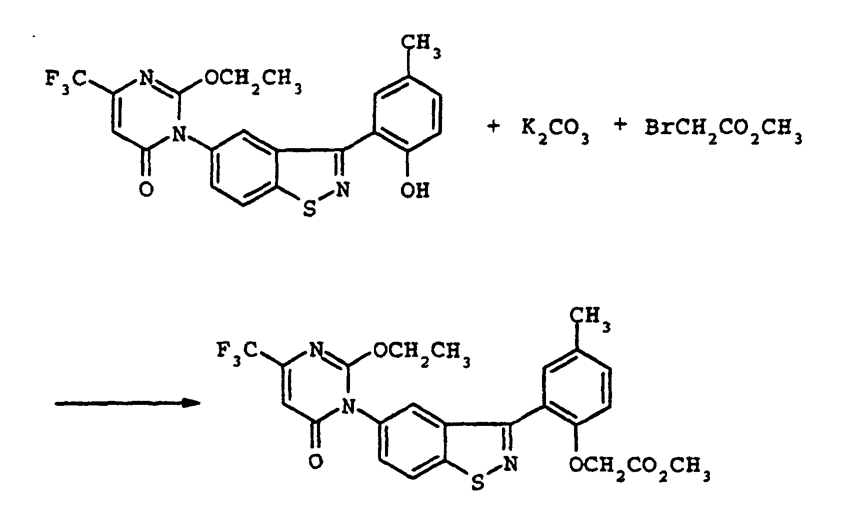

A mixture of 2-ethoxy-3-[3-(6-hydroxy-*m*-tolyl)-1,2-benzisothiazol-5-yl]-6-(trifluoromethyl)-4(3H)-pyrimidinone (0.470 g, 1.05 mmol), potassium carbonate (0.190 g, 1.37 mmol) and methyl bromoacetate (0.210 g, 1.37 mmol) in N,N-dimethylformamide is stirred overnight at room temperature, diluted with ethyl acetate, washed sequentially with water and brine, dried over anhydrous magnesium sulfate, and concentrated *in vacuo* to obtain a solid. Flash column chromatography of the solid using silica gel and a 10% diethyl ether in methylene chloride solution gives the title product as an off-white solid (0.450 g, mp 171-174 °C).

Using essentially the same procedure, the following compounds are obtained:

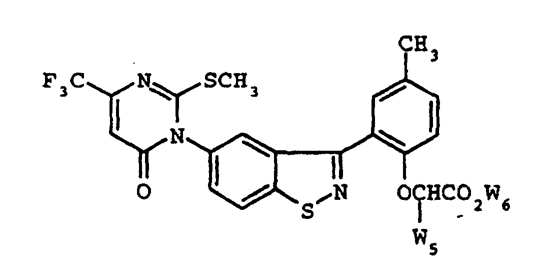

| **W**_{**5**} | **W**_{**6**} | **mp °C** |
|---|---|---|
| H | CH₃ | 95-99 |
| CH₃ | CH₃ | 93-100 |
| H | CH₂C≡CH | 187-188 |

### EXAMPLE 57

### Preparation of 2,2'-Dithiobis[5-nitrobenzoic acid]

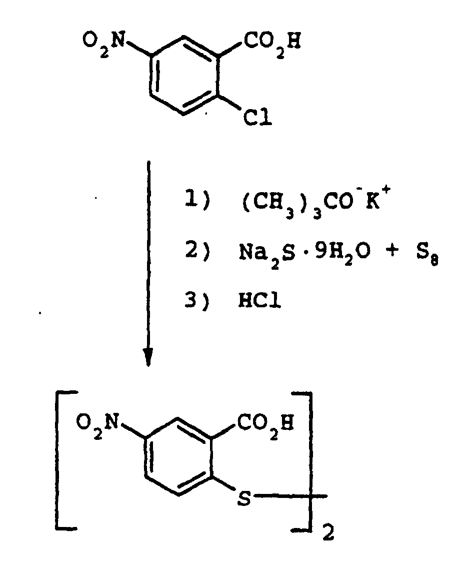

A mixture of 2-chloro-5-nitrobenzoic acid (100 g, 0.496 mol) in ethanol is treated portionwise with potassium tert-butoxide (55.5 g, 0.495 mol), diluted with additional ethanol, heated to reflux, treated portionwise with a solution prepared from sodium sulfide nonahydrate (60.0 g, 0.249 mol), sulfur (8.80 g, 0.274 mol) and water, refluxed for two hours, cooled to room temperature, and treated with concentrated hydrochloric acid. The resultant acidic mixture is stirred for one hour and filtered to obtain a solid. The solid is washed with water and air-dried to give the title product as a yellow powder which is identified by NMR spectral analysis.

### EXAMPLE 58

### Preparation of 5-Nitro-1,2-benzisothiazol-3(2H)-one

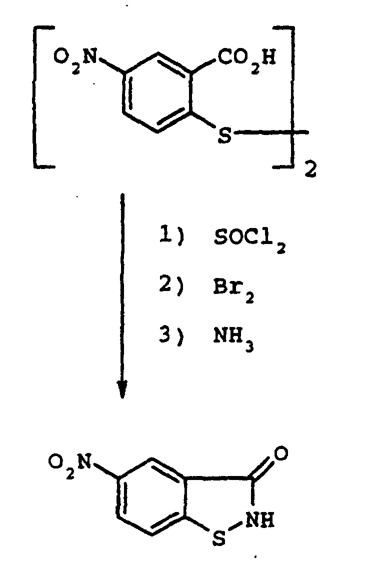

A mixture of 2,2'-dithiobis[5-nitrobenzoic acid] (44.6 g, 0.113 mol) and thionyl chloride (49.0 mL, 0.670 mol) in methylene chloride is treated with N,N-dimethylformamide (0.800 mL), refluxed overnight, concentrated *in vacuo*, and diluted with 1,2-dichloroethane. The resultant organic solution is treated with bromine (22.5 mL, 0.436 mol), stirred at room temperature for 20 minutes, refluxed for 3.5 hours, and concentrated *in vacuo* to obtain a residue. A solution of the residue in 1,2-dichloroethane is cooled with an ice-water bath, treated with concentrated ammonia (112 mL) over 15 minutes, stirred at room temperature for 16 hours, cooled with an ice-water bath, and treated with concentrated hydrochloric acid. The resultant aqueous mixture is stirred at room temperature for one hour and filtered to obtain a solid. The solid is washed with water and air-dried to give the title product as a yellow solid which is identified by NMR spectral analysis.

### EXAMPLE 59

### Preparation of 3-Chloro-5-nitro-1,2-benzisothiazole

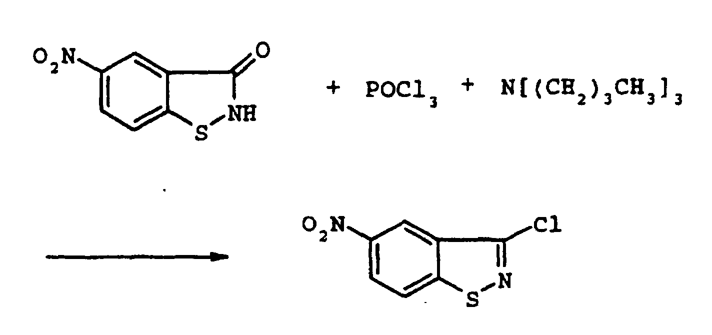

A mixture of 5-nitro-1,2-benzisothiazol-3(2H)-one (10.0 g, 0.0510 mol), phosphorus oxychloride (40.0 mL, 0.429 mol) and tributylamine (12.0 mL, 0.050 mol) is heated at 103-115 °C for six hours, stirred at room temperature overnight, and poured into an ice-water mixture. The resultant aqueous mixture is extracted with methylene chloride. The combined organic extracts are washed sequentially with water and saturated sodium hydrogen carbonate solution, dried over anhydrous sodium sulfate, and concentrated *in vacuo* to obtain a gum. Column chromatography of the gum using silica gel and methylene chloride gives the title product as an orange-yellow solid which is identified by NMR spectral analysis.

### EXAMPLE 60

### Preparation of Ethyl α-cyano-5-nitro-1,2-benzisothiazole-3-acetate

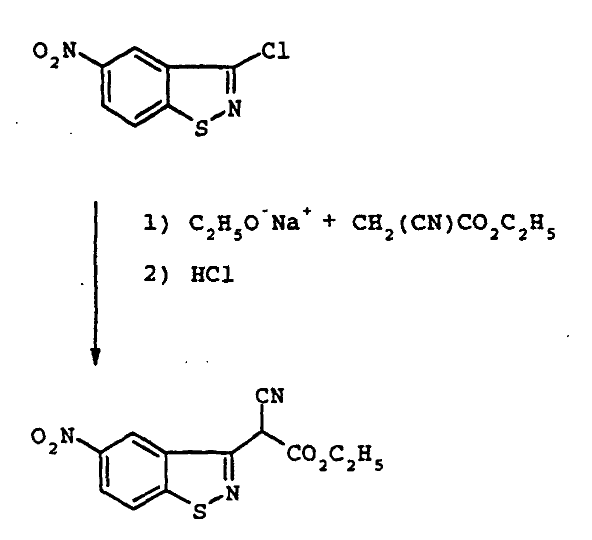

A sodium ethoxide solution (previously prepared from ethanol and sodium (1.00 g, 0.0430 mol)) is cooled with an ice-acetone bath, treated portionwise with ethyl cyanoacetate (4.51 g, 0.0398 mol), stirred at room temperature for 30 minutes, treated with 3-chloro-5-nitro-1,2-benzisothiazole (4.27 g, 0.0199 mol), stirred at room temperature overnight, cooled to 0 °C, and treated dropwise with 10% hydrochloric acid (15.0 mL). The resultant aqueous mixture is stirred at room temperature for one hour and filtered to obtain a solid. The solid is washed with ethanol and air-dried to give the title product as a yellow solid which is identified by NMR spectral analysis.

### EXAMPLE 61

### Preparation of Ethyl 5-nitro-1,2-benzisothiazole-3-acetate

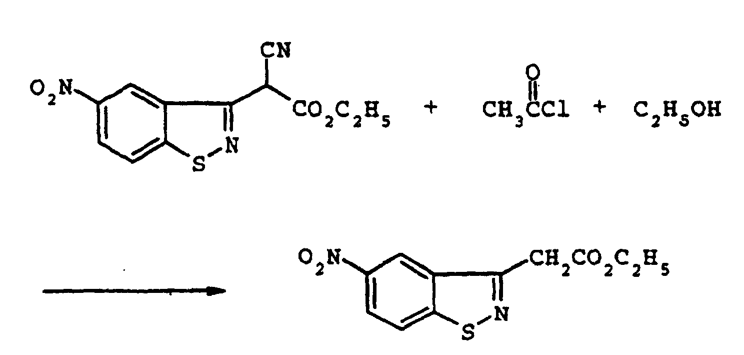

Ethyl α-cyano-5-nitro-1,2-benzisothiazole-3-acetate (6.67 g, 0.0229 mol) is added to a solution of acetyl chloride (67.0 mL) in ethanol. The reaction mixture is refluxed overnight, cooled, and filtered to remove solids. The resultant filtrate is concentrated *in vacuo* to obtain a brown semi-solid. A mixture of the semi-solid in diethyl ether is stirred for two hours and filtered to obtain a solid. The solid is washed with diethyl ether and air-dried to give the title product as yellow crystals (1.04 g, mp 91-92 °C).

### EXAMPLE 62

### Preparation of Ethyl 5-amino-1,2-benzisothiazole-3-acetate

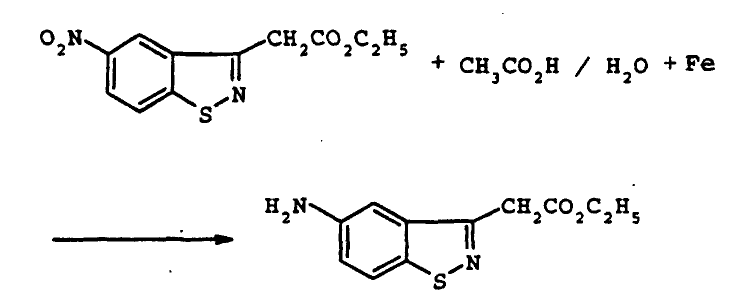

A 10% acetic acid solution (31.0 mL) is stirred at 50 °C, treated with iron powder (0.656 g), treated dropwise with a solution of ethyl 5-nitro-1,2-benzisothiazole-3-acetate (1.03 g, 3.88 mmol) in ethyl acetate, stirred at 50 °C for two hours, treated with additional iron powder (0.305 g), stirred at 50 °C for 15 minutes, and poured into saturated sodium hydrogen carbonate solution. The resultant aqueous mixture is extracted with ethyl acetate. The combined organic extracts are washed sequentially with water and brine, dried over anhydrous sodium sulfate, and concentrated *in vacuo* to obtain an oil. Column chromatography of the oil using silica gel and methylene chloride gives the title product as a yellow oil.

### EXAMPLE 63

### Preparation of Ethyl 5-[3,6-dihydro-2,6-dioxo-4-(trifluoromethyl)-1(2H)-pyrimidinyl]-1,2-benzisothiazole-3-acetate

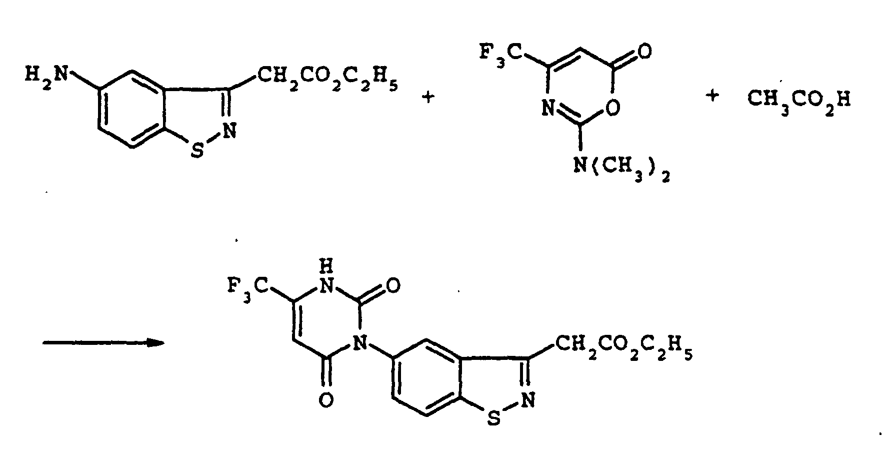

A mixture of ethyl 5-amino-1,2-benzisothiazole-3-acetate (0.748 g, 3.16 mmol) and 2-dimethylamino-4-(trifluoromethyl)-6H-1,3-oxazin-6-one (0.660 g, 3.17 mmol) in acetic acid is refluxed for three hours, concentrated *in vacuo,* and diluted with saturated sodium hydrogen carbonate solution. The resultant mixture is extracted with methylene chloride. The combined organic extracts are washed with water, dried over anhydrous sodium sulfate, and concentrated *in vacuo* to give the title product as a tan solid which is identified by NMR spectral analysis.

### EXAMPLE 64

### Preparation of Ethyl 5-[3,6-dihydro-3-methyl-2,6-dioxo-4-(trifluoromethyl)-1(2H)-pyrimidinyl]-1,2-benzisothiazole-3-acetate

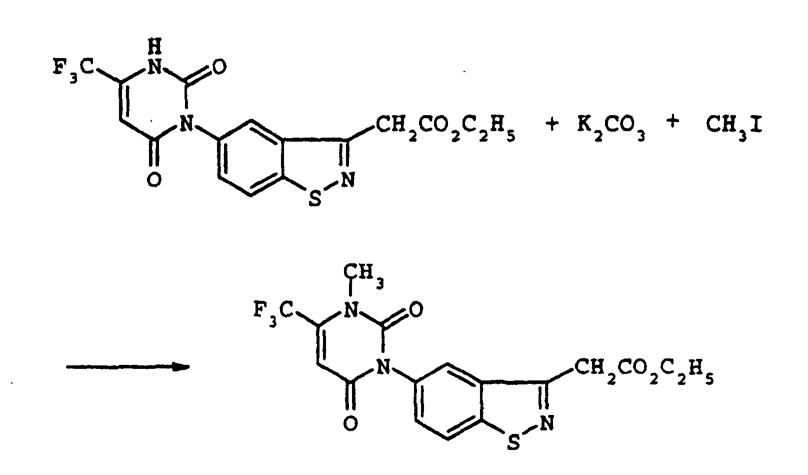

A mixture of ethyl 5-[3,6-dihydro-2,6-dioxo-4-(trifluoromethyl)-1(2H)-pyrimidinyl]-1,2-benzisothiazole-3-acetate (0.643 g, 0.00160 mol) and potassium carbonate (0.243 g, 0.00170 mol) in N,N-dimethylformamide is stirred at room temperature for 90 minutes, treated with iodomethane (0.320 mL, 0.00500 mol), stirred at room temperature overnight, and diluted with water. The resultant aqueous mixture is extracted with methylene chloride. The organic extract is washed with water, dried over anhydrous magnesium sulfate, and concentrated *in vacuo* to obtain a brown oil. Column chromatography of the oil using silica gel and a 10% ethyl acetate in hexanes solution gives the title product as a tan solid (0.362 g, mp 150-152 °C).

### EXAMPLE 65

### Preparation of 5-Amino-1,2-benzisothiazole-3-ethanol

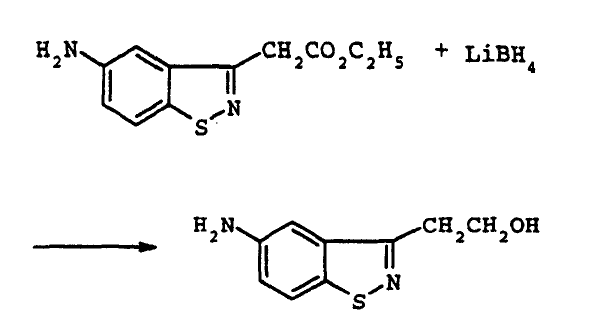

A mixture of ethyl 5-amino-1,2-benzisothiazole-3-acetate (3.14 g, 0.0133 mol) in tetrahydrofuran is cooled to -8 °C, treated dropwise with a 2 M solution of lithium borohydride in tetrahydrofuran (5.00 mL, 0.0100 mol), stirred at room temperature for 3.5 hours, cooled to -5 °C, treated with additional lithium borohydride solution (4.20 mL, 0.0084 mol), stirred at room temperature overnight, cooled to 0 °C, quenched sequentially with water (5.00 mL) and 10% hydrochloric acid (10.0 mL), stirred at room temperature for one hour, neutralized with saturated sodium hydrogen carbonate solution (10.0 mL), and concentrated *in vacuo* to obtain a residue. The residue is diluted with a 5% methanol in methylene chloride solution. The resultant mixture is diluted with water, brine and methylene chloride. The phases are separated and the organic phase is concentrated *in vacuo* to obtain an orange gum. The gum is crystallized from methanol to give the title product as a tan solid which is identified by NMR spectral analysis.

### EXAMPLE 66

### Preparation of 3-[3-(2-Hydroxyethyl)-1,2-benzisothiazol-5-yl-1-methyl-6-(trifluoromethyl)-2,4(1H,3H)-pyrimidinedione acetate

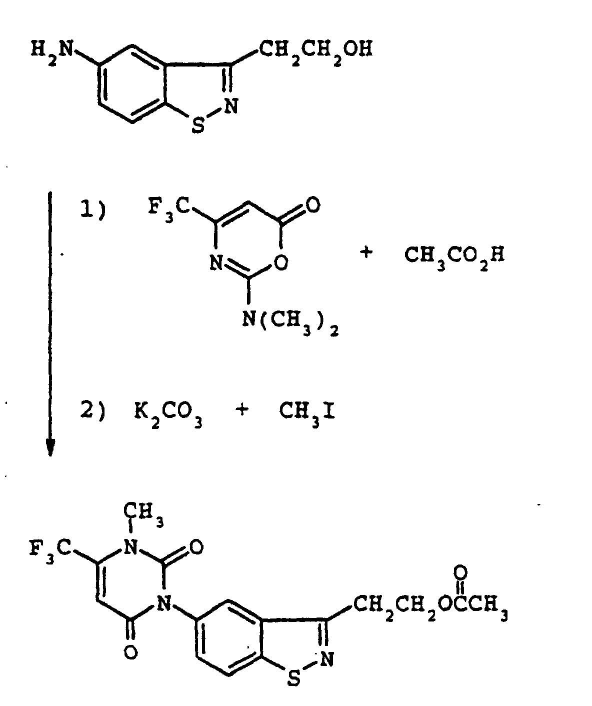

A mixture of 5-amino-1,2-benzisothiazole-3-ethanol (0.794 g, 4.09 mmol) and 2-dimethylamino-4-(trifluoromethyl)-6H-1,3-oxazin-6-one (0.850 g, 4.08 mmol) in acetic acid is refluxed for five hours and concentrated *in vacuo* to obtain a tan solid. A solution of the solid in N,N-dimethylformamide is treated with potassium carbonate (0.615 g, 4.45 mmol), stirred for one hour, treated with iodomethane (0.800 mL, 12.8 mmol), stirred overnight, and poured into an ice-water mixture. The resultant aqueous mixture is extracted with methylene chloride. The combined organic extracts are washed sequentially with water, 3% hydrochloric acid, water, saturated sodium hydrogen carbonate solution and water, dried over anhydrous sodium sulfate, and concentrated *in vacuo* to obtain a solid. Column chromatography of the solid using silica gel and methylene chloride gives the title product as a white solid which is identified by NMR spectral analysis.

### EXAMPLE 67

### Preparation of 3-[3-(2-Hydroxyethyl)-1,2-benzisothiazol-5-yl]-1-methyl-6-(trifluoromethyl)-2,4-(1H,3H)-pyrimidinedione

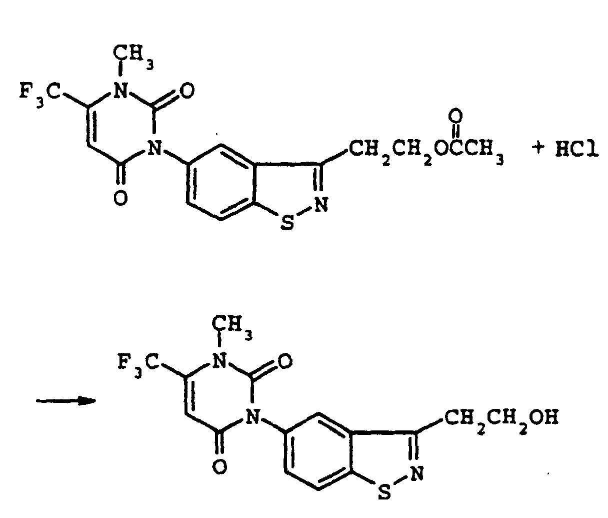

A mixture of 3-[3-(2-hydroxyethyl)-1,2-benzisothiazol-5-yl-1-methyl-6-(trifluoromethyl)-2,4(1H,3H)-pyrimidinedione acetate (1.00 g, 2.42 mmol) in 10% hydrochloric acid (250 mL) is refluxed for 35 minutes and filtered through glass wool to remove solids. The resultant filtrate is cooled and extracted with methylene chloride. The combined organic extracts are washed with water, dried over anhydrous sodium sulfate, and concentrated *in vacuo* to give the title product as a yellow solid which is identified by NMR spectral analysis.

### EXAMPLE 68

### Preparation of 5-Nitro-1,2-benzisothiazole-3-acetonitrile

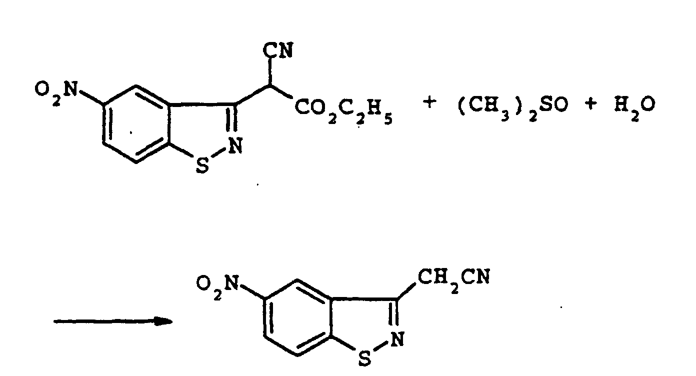

A mixture of ethyl 5-nitro-1,2-benzisothiazole-3-acetate (5.00 g, 17.2 mmol), water (1.00-mL), and methyl sulfoxide (35.0 mL) is stirred at 107 °C for 24 hours, stirred at room temperature for two days, and poured into an ice-water mixture. The resultant aqueous mixture is stirred for two hours and filtered to obtain a solid. The solid is washed with water and air-dried to give the title product as a tan solid.

### EXAMPLE 69

### Preparation of α,α-Dimethyl-5-nitro-1,2-benzisothiazole-3-acetonitrile

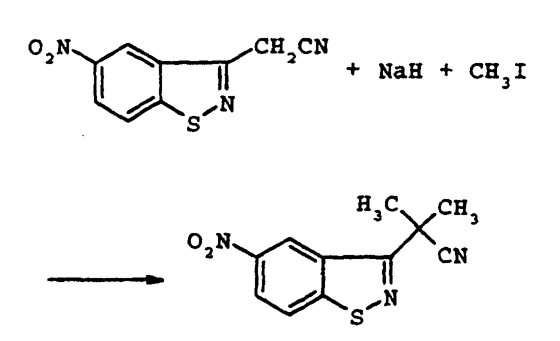

A mixture of 5-nitro-1,2-benzisothiazole-3-acetonitrile (1.29 g, 5.89 mmol) in N,N-dimethylformamide is cooled to -9 °C, treated with sodium hydride (1.00 g of a 60% dispersion in oil), stirred at -3 °C for 20 minutes, treated with iodomethane (5.00 mL), stirred at room temperature for four hours, and poured onto ice. The resultant aqueous mixture is treated with 10% hydrochloric acid and extracted with methylene chloride. The combined organic extracts are washed sequentially with water, saturated sodium hydrogen carbonate solution and water, dried over anhydrous sodium sulfate, and concentrated *in vacuo* to obtain a solid. column chromatography of the solid using silica gel and methylene chloride gives the title product as a yellow solid which is identified by NMR spectral analysis.

### EXAMPLE 70

### Preparation of Ethyl α,α-dimethyl-5-nitro-1,2-benzisothiazole-3-acetate

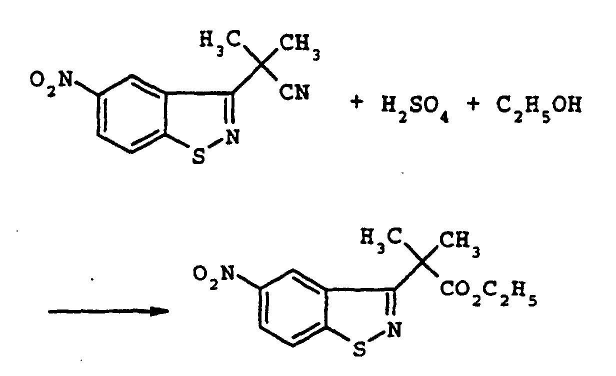

A mixture of α,α-dimethyl-5-nitro-1,2-benzisothiazole-3-acetonitrile (0.913 g, 3.69 mmol), water (0.450 mL), concentrated sulfuric acid (4.55 mL) and ethanol (9.10 mL) is refluxed for one hour, cooled, and poured onto ice. The resultant aqueous mixture is neutralized with saturated sodium bicarbonate solution and extracted with methylene chloride. The organic extract is washed with water, dried over anhydrous sodium sulfate, and concentrated *in vacuo* to obtain a solid. Column chromatography of the solid using silica gel and methylene chloride gives the title product as pale yellow crystals.

### EXAMPLE 71

### Preparation of Ethyl 5-amino-α,α-dimethyl-1,2-benzisothiazole-3-acetate

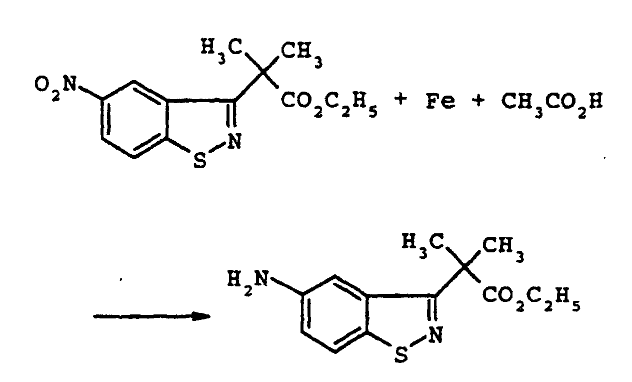

A mixture of ethyl α,α-dimethyl-5-nitro-1,2-benzisothiazole-3-acetate (0.714 g, 2.42 mmol), iron powder (0.500 g), 10% acetic acid (23.0 mL) and ethyl acetate (23.0 mL) is stirred at 54-58 °C for one hour, cooled, and poured into saturated sodium hydrogen carbonate solution. The resultant aqueous mixture is extracted with ethyl acetate. The combined organic extracts are washed sequentially with water and brine, dried over anhydrous sodium sulfate, and concentrated *in vacuo* to obtain a yellow oil. Column chromatography of the oil using silica gel and a 10% ethyl acetate in methylene chloride solution gives the title product as a light brown oil which is identified by NMR spectral analysis.

### EXAMPLE 72

### Preparation of Ethyl 5-[3,6-dihydro-3-methyl-2,6-dioxo-4-(trifluoromethyl)-1(2H)-pyrimidinyl]-α,α-dimethyl-1,2-benzisothiazole-3-acetate

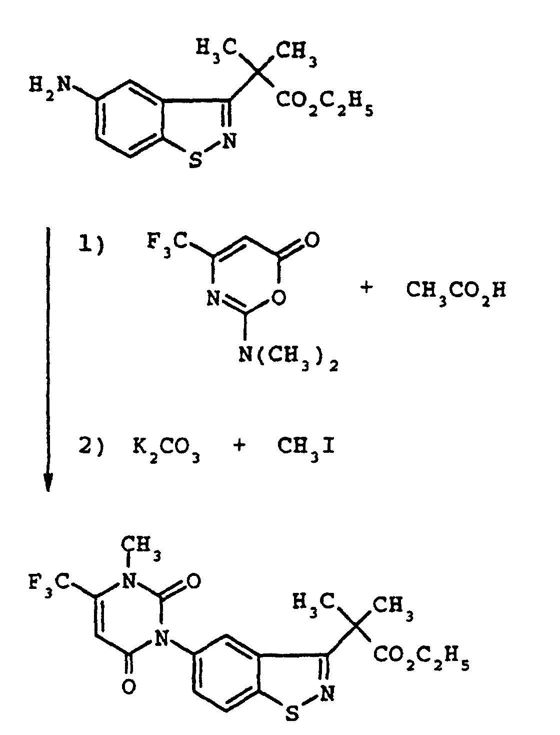

A mixture of ethyl 5-amino-α,α-dimethyl-1,2-benzisothiazole-3-acetate (0.546 g, 2.06 mmol) and 2-dimethylamino-4-(trifluoromethyl)-6H-1,3-oxazin-6-one (0.430 g, 2.06 mmol) in acetic acid is refluxed for 4.5 hours, concentrated *in vacuo*, and diluted with saturated sodium hydrogen carbonate solution. The resultant aqueous mixture is extracted with methylene chloride. The combined organic extracts are washed with water, dried over anhydrous sodium sulfate, and concentrated *in vacuo* to obtain a brown foam. A solution of the foam in N,N-dimethylformamide is treated with potassium carbonate (0.312 g, 2.25 mmol), stirred for one hour, treated with iodomethane (0.420 mL, 6.70 mmol), stirred overnight at room temperature, and poured into an ice-water mixture containing 20 mL of concentrated hydrochloric acid. The resultant aqueous mixture is extracted with methylene chloride. The combined organic extracts are washed sequentially with 10% hydrochloric acid, water, saturated sodium hydrogen carbonate solution and water, dried over anhydrous sodium sulfate and concentrated *in vacuo* to obtain a brown oil. Column chromatography of the oil using silica gel and a 33% ethyl acetate in methylene chloride solution affords a pink foam which is recrystallized from ethanol to give the title product as pink crystals, mp 164-167 °C.

### EXAMPLE 73

### Preparation of 5-Amino-3-chloro-1,2-benzisothiazole

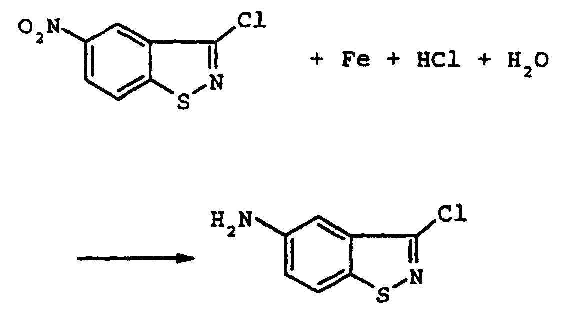

A solution of 3-chloro-5-nitro-1,2-benzisothiazole (2.00 g) in toluene is treated with iron powder (8.40 g, 325 mesh) and concentrated hydrochloric acid (8 drops), heated to reflux, treated dropwise with water (8.00 mL), refluxed for 35 minutes, cooled to room temperature, and filtered through diatomaceous earth. The resultant filtrate is concentrated *in vacuo* to obtain a residue. Flash column chromatography of the residue using silica gel and an ethyl acetate/hexanes solution (1:1) gives the title product.

### EXAMPLE 74

### Preparation of 3-(3-Chloro-1,2-benzisothiazol-5-yl)-6-(trifluoromethyl)-2,4(1H,3H)-pyrimidinedione

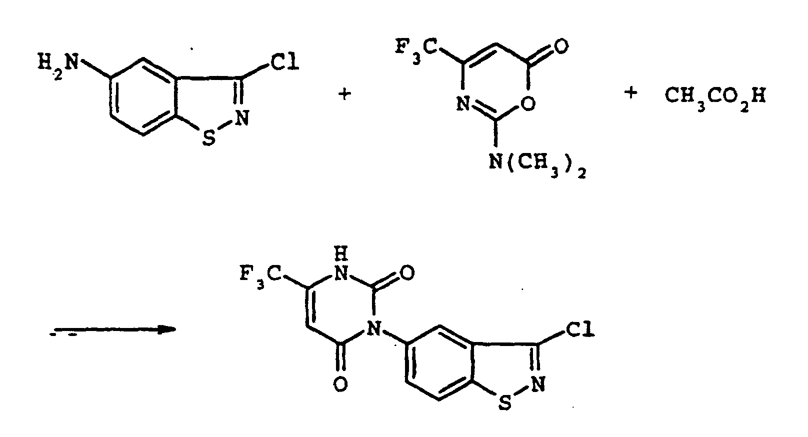

A mixture of 5-amino-3-chloro-1,2-benzisothiazole (1.10 g) and 2-dimethylamino-4-(trifluoromethyl)-6H-1,3-oxazin-6-one (1.38 g) in acetic acid (15.1 mL) is stirred at 90-105 °C for two hours, cooled to room temperature, and filtered to obtain 0.500 g of the title product as a solid. The resultant filtrate is diluted with water and filtered to obtain an additional 1.11 g of the title product.

### EXAMPLE 75

### Preparation of 3-(3-Chloro-1,2-benzisothiazol-5-yl)-1-methyl-6-(trifluoromethyl)-2,4(1H,3H)-pyrimidinedione

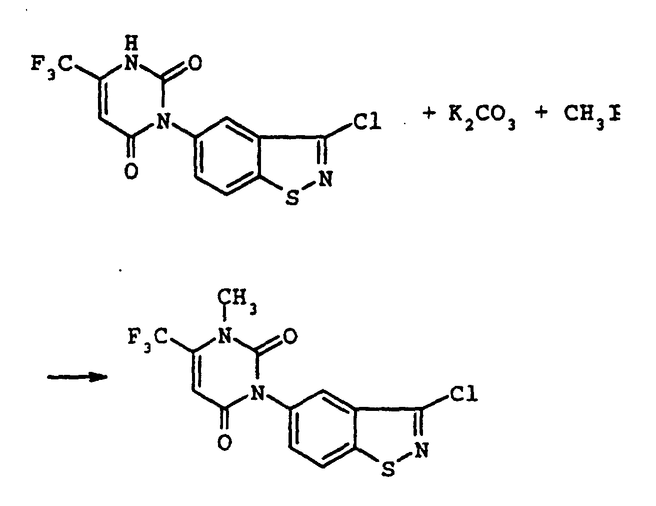

A mixture of 3-(3-chloro-1,2-benzisothiazol-5-yl)-6-(trifluoromethyl)-2,4(1H,3H)-pyrimidinedione (1.06 g), potassium carbonate (0.470 g) and iodomethane (0.500 mL) in N,N-dimethylformamide is stirred at room temperature for 90 minutes, treated with additional iodomethane (0.500 mL), stirred at room temperature for 15 minutes, and diluted with water. The resultant aqueous mixture is filtered to obtain a solid. The solid is washed with water and dried in a vacuum oven at room temperature to give the title product as a solid which is identified by NMR spectral analysis.

### EXAMPLE 76

### Preparation of 5-[3,6-Dihydro-3-methyl-2,6-dioxo -4-(trifluoromethyl)-1(2H)-pyrimidinyl]-1,2-benzisothiazole-3-malononitrile

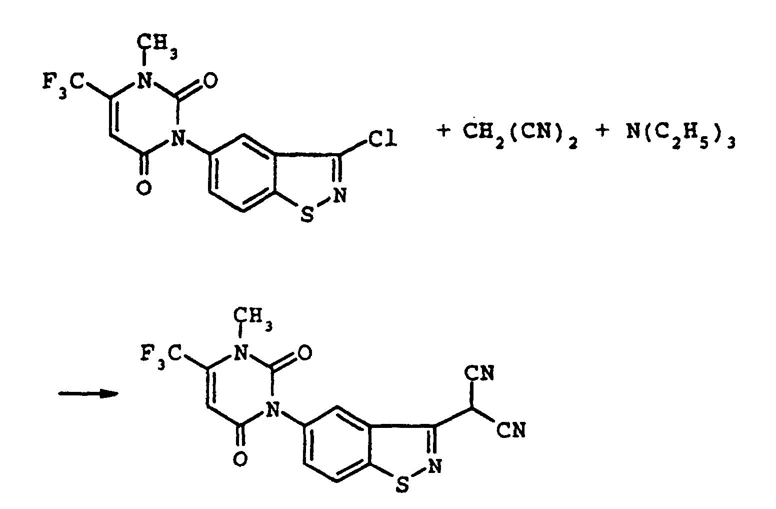

A mixture of 3-(3-chloro-1,2-benzisothiazol-5-yl)-1-methyl-6-(trifluoromethyl)-2,4(1H,3H)-pyrimidinedione (0.500 g), malononitrile (0.270 g) and triethylamine (1.50 g) in methyl sulfoxide is stirred at 60 °C for 15 minutes, cooled to room temperature, and diluted with water. The resultant aqueous mixture is extracted with ethyl acetate. The organic extract is washed with water, dried over anhydrous magnesium sulfate, and concentrated *in vacuo* to obtain a brown oil. The extracted aqueous phase is diluted with brine, acidified with concentrated hydrochloric acid, and extracted with ethyl acetate. The organic extract is dried over anhydrous magnesium sulfate and concentrated *in vacuo* to obtain a brown oil. Flash column chromatography of the combined oils using silica gel and an ethyl acetate/hexanes/methanol/acetic acid solution (10:10:4:1) gives the title product as an oil which is identified by NMR spectral analyses.

### EXAMPLE 77

### Preparation of 5-[3,6-Dihydro-3-methyl-2,6-dioxo-4-(trifluoromethyl)-1(2H)-pyrimidinyl]-1,2-benzisothiazole-3-acetonitrile

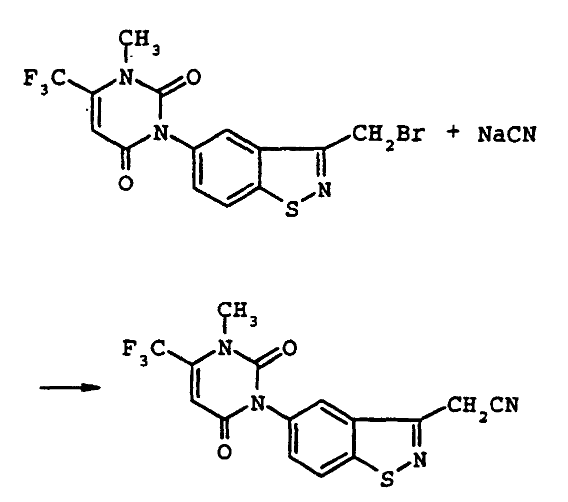

A mixture of 3-[3-(bromomethyl)-1,2-benzisothiazol-5-yl]-1-methyl-6-(trifluoromethyl)-2,4(1H,3H)-pyrimidinedione (0.420 g, 0.00100 mol) and sodium cyanide (0.0750 g, 0.00150 mol) in acetonitrile is stirred at room temperature overnight, and filtered to obtain a solid. The solid is dissolved in methylene chloride and the resultant solution is dried over anhydrous sodium sulfate, and concentrated *in vacuo* to obtain an off-white solid. Column chromatography of the solid using silica gel and a 10% ethyl acetate in methylene chloride solution gives the title product as a white solid which is identified by NMR spectral analysis.

Using essentially the same procedure 5-[3,6-dihydro-3-methyl-2,6-dioxo-4-(trifluoromethyl)-1(2H)-pyrimidinyl]-1,2-benzisothiazole-6-fluoro-3-acetonitrile is obtained from 3-[3-(bromomethyl)-6-fluoro-1,2-benzisothiazol-5-yl]-1-methyl-6-(trifluoromethyl)-2,4(1H,3H)-pyrimidinedione.

### EXAMPLE 78

### Preparation of Ethyl 5-[3,6-dihydro-3-methyl-2,6-dioxo-4-(trifluoromethyl)-1(2H)-pyrimidinyl]-1,2-benzisothiazole-3-acetate

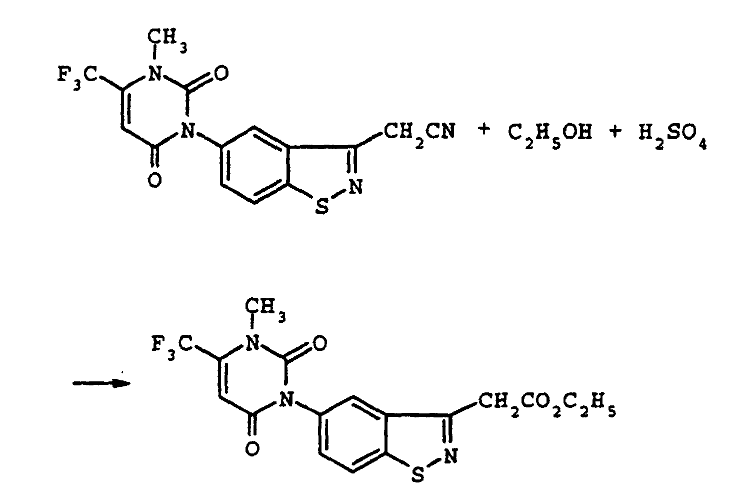

A mixture of 5-[3,6-dihydro-3-methyl-2,6-dioxo-4-(trifluoromethyl)-1(2H)-pyrimidinyl]-1,2-benzisothiazole-3-acetonitrile (1.00 g), ethanol (20.0 mL) and water (1.00 mL) is treated with concentrated sulfuric acid (10.0 mL), refluxed for 15 minutes, and poured into an ice-water mixture. The resultant aqueous mixture is extracted with methylene chloride. The combined organic extracts are washed sequentially with water, saturated sodium hydrogen carbonate solution and water, dried over anhydrous sodium sulfate, and concentrated *in vacuo* to obtain an orange foam. Column chromatography of the foam using silica gel and a 10% ethyl acetate in methylene chloride solution gives the title product as a solid which is identified by NMR spectral analysis.

### EXAMPLE 79

### Preparation of 5-[3,6-Dihydro-3-methyl-2,6-dioxo-4-(trifluoromethyl)-1(2H)-pyrimidinyl]-1,2-benzisothiazole-3-acetic acid

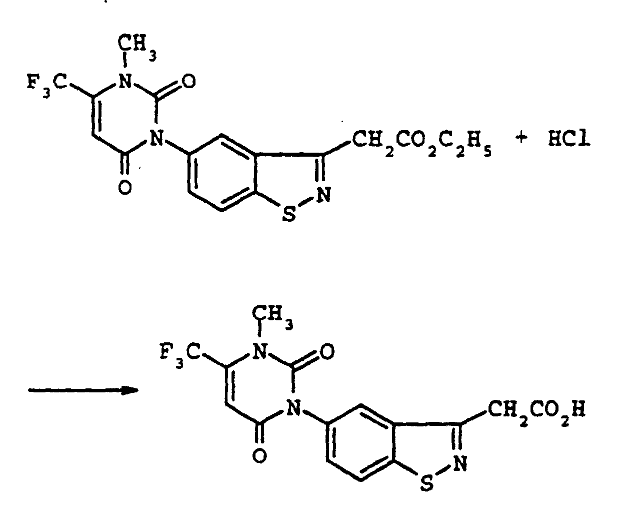

A mixture of ethyl 5-[3,6-dihydro-3-methyl-2,6-dioxo-4-(trifluoromethyl)-1(2H)-pyrimidinyl]-1,2-benzisothiazole-3-acetate (0.600 g), 10% hydrochloric acid (20.0 mL), and 1,2-dichloroethane (2.00 mL) is boiled for 30 minutes while allowing the 1,2-dichloroethane to evaporate, cooled, and poured onto ice. The resultant aqueous mixture is filtered to obtain a solid. The solid is washed with water and air-dried to give the title product as a white solid (0.550 g, mp 240-242 °C).

### EXAMPLE 80

### Preparation of Methyl 5-[3,6-dihydro-3-methyl-2,6-dioxo-4-(trifluoromethyl)-1(2H)-pyrimidinyl]-2-benzisothiazole-3-acetate

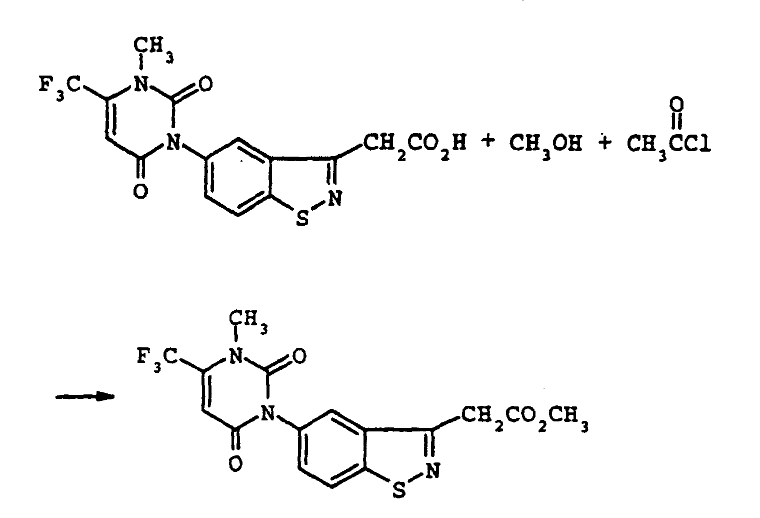

Acetyl chloride (11.0 mL) is added to methanol (110 mL) previously cooled with an ice-acetone bath. The resultant mixture is stirred at room temperature for 30 minutes, treated with 5-[3,6-dihydro-3-methyl-2,6-dioxo-4-(trifluoromethyl)-1(2H)-pyrimidinyl]-1,2-benzisothiazole-3-acetic acid (0.617 g, 1.60 mmol), refluxed for four hours, and poured into saturated sodium hydrogen carbonate solution. The resultant aqueous mixture is extracted with methylene chloride. The combined organic extracts are washed with water and concentrated *in vacuo* to obtain a residue. The residue is recrystallized from an acetone/hexanes solution to give the title product as a white solid (0.540 g, mp 175-176 °C).

### EXAMPLE 81

### Preparation of 5-[3,6-Dihydro-3-methyl-2,6-dioxo-4-(trifluoromethyl)-1(2H)-pyrimidinyl]-1,2-benzisothiazole-3-acetamide

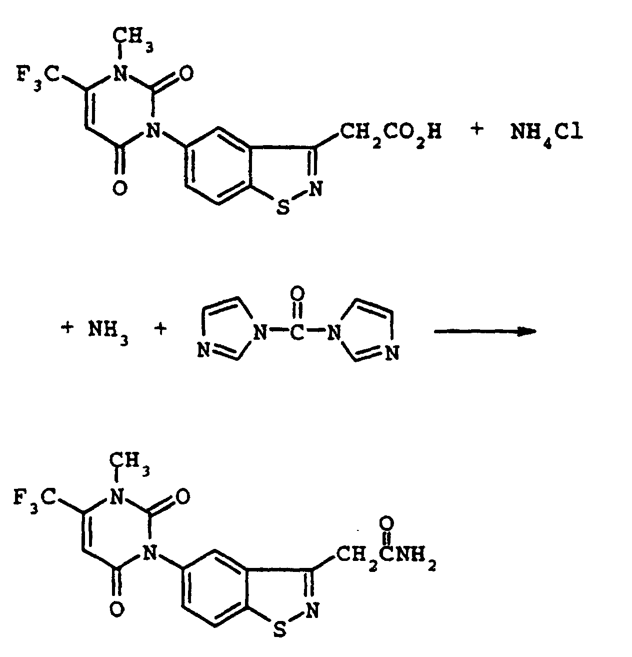

A mixture of 5-[3,6-dihydro-3-methyl-2,6-dioxo-4-(trifluoromethyl)-1(2H)-pyrimidinyl]-1,2-benzisothiazole-3-acetic acid (0.960 g, 0.00250 mol) and 1,1'-carbonyldiimidazole (0.420 g, 0.00259 mol) in tetrahydrofuran is stirred at room temperature for 4.5 hours, treated with ammonium chloride (0.230 g, 0.00430 mol), stirred overnight at room temperature, treated with concentrated ammonia (1.00 mL), stirred for 5 minutes, acidified with 10% hydrochloric acid, and concentrated *in vacuo* to obtain a residue. The residue is diluted with a 5% methanol in methylene chloride solution and the resultant mixture is stirred for three hours and filtered to obtain a solid. The solid is washed with a 5% methanol in methylene chloride solution, slurried in water, filtered, and dried to give the title product as a white solid which is identified by NMR spectral analysis.

### EXAMPLE 82

### Preparation of Sodium 2-{{2-{5-[3,6-dihydro-3-methyl-2,6-dioxo-4-(trifluoromethyl)-1(2H)-pyrimidinyl]-1,2-benzisothiazol-3-yl}-p-tolyl}oxy}propionate, DL-

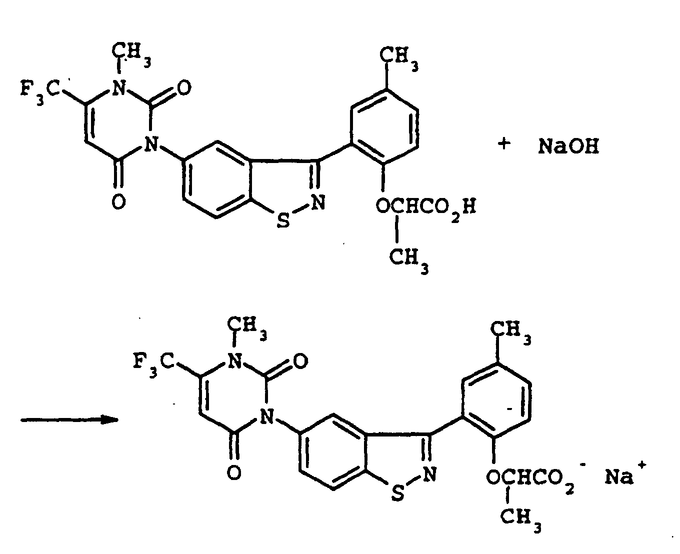

A mixture of 2-{{2-{5-[3,6-dihydro-3-methyl-2,6-dioxo-4-(trifluoromethyl)-1(2H)-pyrimidinyl]-1,2-benzisothiazol-3-yl}*-p*-tolyl}oxy}propionic acid (0.500 g) and sodium hydroxide (0.0360 g) in water is stirred at room temperature overnight, concentrated *in vacuo* to one-half volume, and filtered to give the title product as a solid which is identified by NMR spectral analysis.

Using essentially the same procedure, the following compounds are obtained:

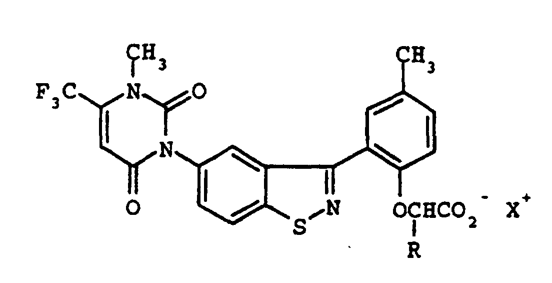

| **R** | **X**^{**+**} |
|---|---|
| CH₃ | HN⁺[CH(CH₃)₂]₂ |
| H | HN⁺[CH(CH₃)₂]₂ |
| CH₃ | NH₃ ⁺ |
| H | NH₃ ⁺ |
| H | Na⁺ |

### EXAMPLE 83

### Preparation of 2-Propynyl 2-{{2-{4-chloro-5-[3,6-dihydro-3-methyl-2,6-dioxo-4-(trifluoromethyl)-1(2H)-pyrimidinyl]-1,2-benzisoxazol-3-yl}-p-tolyl}oxy}-propionate

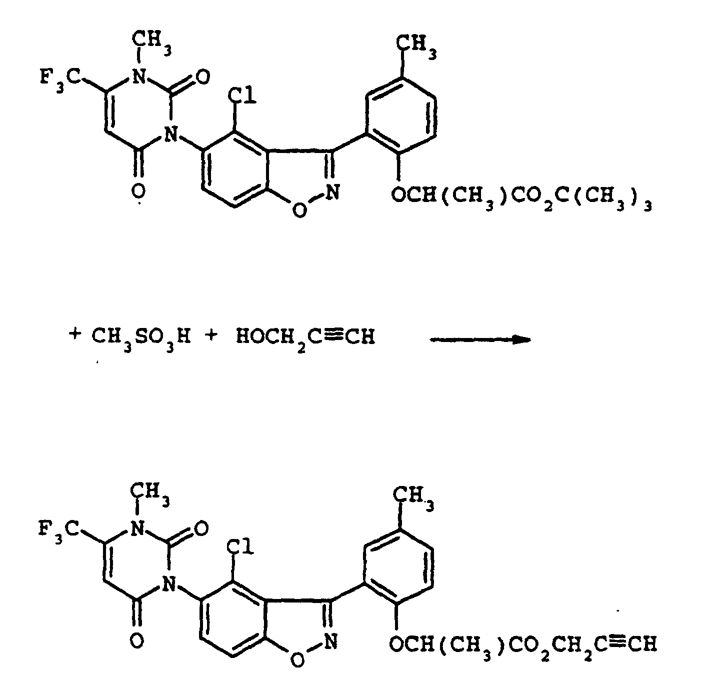

A mixture of *tert*-butyl 2-{{2-[4-chloro-5-[3,6-dihydro-3-methyl-2,6-dioxo-4-(trifluoromethyl)-1(2H)-pyrimidinyl]-1,2-benzisoxazol-3-yl}-*p*-tolyl]oxy]propionate (1.00 g, 0.00172 mol), propargyl alcohol (20.0 mL) and methanesulfonic acid (52.0 mg, 0.542 mmol) is stirred at 75-93 °C for 12 hours, stirred at room temperature for seven hours, and concentrated *in vacuo* to obtain a residue. The residue is diluted with methylene chloride and the resultant solution is passed through a silica gel plug and concentrated *in vacuo* to obtain a yellow gum. The gum is dissolved in diethyl ether and the resultant solution is washed with water, dried over anhydrous magnesium sulfate, and concentrated in *vacuo* to obtain a yellow gum. Column chromatography of the gum using silica gel and an ether/methylene chloride/hexanes solution (4:3:7) gives a foam. The foam is recrystallized from a methylene chloride/hexanes solution to provide the title product as a white solid, mp 137-147 °C.

Using essentially the same procedure, the following compounds are obtained:

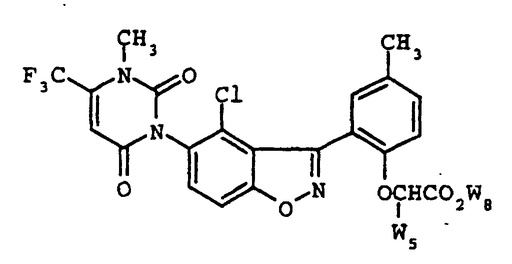

| **W**_{**5**} | **W**_{**8**} |
|---|---|
| CH₃ | CH₃ |
| H | CH₂C≡CH |

### EXAMPLE 84

### Preparation of [(5-Nitro-1,2-benzisothiazol-3-yl)-oxy]acetonitrile

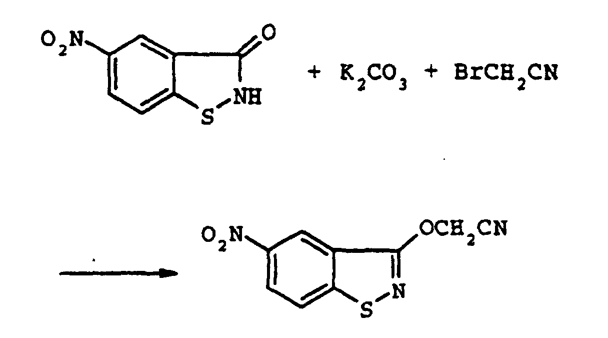

A mixture of 5-nitro-1,2-benzisothiazol-3(2H)-one (17.5 g, 89.2 mmol) in N,N-dimethylformamide is treated with potassium carbonate (18.5 g, 134 mmol), stirred at room temperature for 30 minutes, treated with bromoacetonitrile (16.0 g, 133 mmol), stirred at room temperature overnight, and poured onto ice. The resultant aqueous mixture is acidified to pH 3 with hydrochloric acid and extracted with ethyl acetate. The combined organic extracts are washed sequentially with water and brine, dried over anhydrous magnesium sulfate, and concentrated *in vacuo* to obtain a solid. Column chromatography of the solid using silica gel and methylene chloride gives the title product as a yellow solid (15.0 g, mp 123-124.5 °C).

Using essentially the same procedure, the following compounds are obtained:

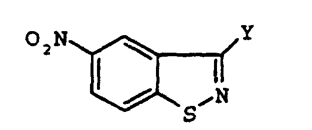

| **Y** | **mp °C** |
|---|---|
| OCH₃ | 108-109 |
| OCH(CH₃)₂ | |
| OCH₂CH=CH₂ | |
| OCH₂C≡CH | 115-117 |
| OCH₂CO₂CH₃ | |

### EXAMPLE 85

### Preparation of [(5-Amino-1,2-benzisothiazol-3-yl)-oxy]acetonitrile

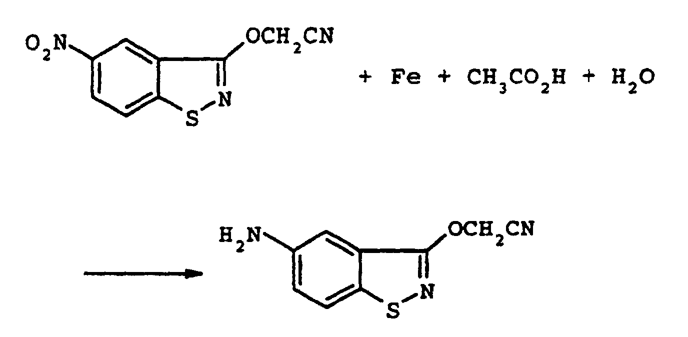

A mixture of iron powder (13.0 g, 0.233 mol) in a 5% acetic acid solution (65.0 mL) is heated to 50 °C, treated portionwise with a mixture of [(5-nitro-1,2-benzisothiazol-3-yl)oxy]acetonitrile (11.0 g, 0.047 mol), acetic acid (100 mL) and ethyl acetate (65.0 mL), refluxed for two hours, cooled to 40 °C, and filtered to remove solids. The phases are separated and the aqueous phase is extracted with ethyl acetate. The organic phase and the organic extracts are combined, washed sequentially with water and brine, dried over anhydrous magnesium sulfate, and concentrated *in vacuo* to give the title product as an oil which is identified by NMR spectral analysis.

Using essentially the same procedure, the following compounds are obtained:

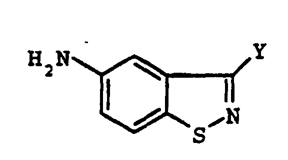

| **y** |
|---|
| OCH₃ |
| OCH(CH₃)₂ |
| OCH₂CH=CH₂ |
| OCH₂C≡CH |
| OCH₂CO₂CH₃ |

### EXAMPLE 86

### Preparation of {{5-[3,6-Dihydro-2,6-dioxo-4-(trifluoromethyl)-1(2H)-pyrimidinyl]-1,2-benzisothiazol-3-yl}oxy}acetonitrile

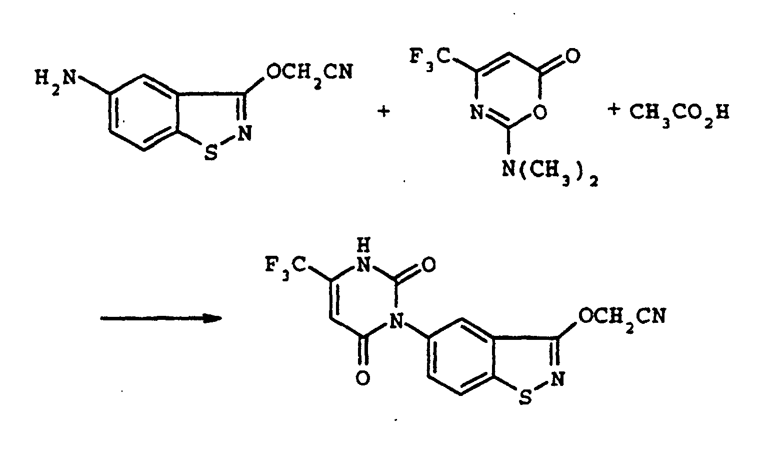

A mixture of [(5-amino-1,2-benzisothiazol-3-yl)oxy]-acetonitrile (4.30 g, 21.0 mmol) and 2-dimethylamino-4-(trifluoromethyl)-6H-1,3-oxazin-6-one (4.37 g, 21.0 mmol) in acetic acid is refluxed for three hours, stirred overnight at room temperature, and poured into an ice-water mixture. The resultant aqueous mixture is filtered to obtain a solid. The solid is washed with water and dried overnight in a vacuum oven at 55 °C to give the title product as a brown solid (2.63 g, mp 254-258 °C).

Using essentially the same procedure, the following compounds are obtained:

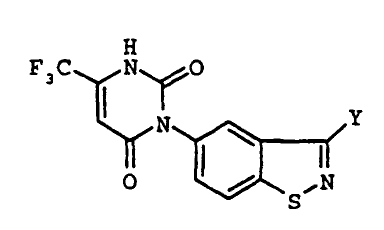

| **Y** | **mp °C** |
|---|---|
| OCH₃ | |
| OCH(CH₃)₂ | 180-185 |
| OCH₂CH=CH₂ | 210-212 |
| OCH₂C≡H | 212-215 |
| OCH₂CO₂CH₃ | |

### EXAMPLE 87

### Preparation of {{5-[3,6-Dihydro-3-methyl-2,6-dioxo-4-(trifluoromethyl)-1(2H)-pyrimidinyl]-1,2-benzisothiazol-3-yl}oxy}acetonitrile

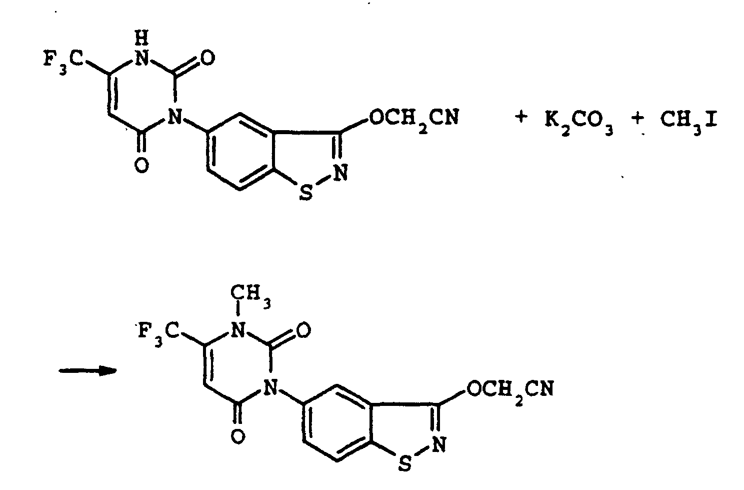

A mixture of {{5-[3,6-dihydro-2,6-dioxo-4-(trifluoromethyl)-1(2H)-pyrimidinyl]-1,2-benzisothiazol-3-yl}oxy}acetonitrile (2.63 g, 7.15 mmol) and potassium carbonate (1.97 g, 14.3 mmol) in N,N-dimethylformamide is stirred for 30 minutes, treated with iodomethane (2.03 g, 14.3 mmol), stirred overnight at room temperature, and poured into an ice-water mixture. The resultant aqueous mixture is filtered to obtain a solid. The solid is dissolved in ethyl acetate and the resultant solution is washed sequentially with water and brine, dried over anhydrous magnesium sulfate, and concentrated *in vacuo* to obtain a brown solid. Column chromatography of the solid using silica gel and methylene chloride affords a yellow solid. The yellow solid is recrystallized from a methylene chloride/hexanes solution to give the title product as an off-white solid, mp 265-266 °C.

Using essentially the same procedure, the following compounds are obtained:

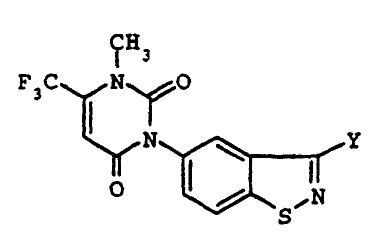

| **Y** | **mp °C** |
|---|---|
| OCH₃ | 198-199 |
| OCH(CH₃)₂ | 125-127 |
| OCH₂CH=CH₂ | 184-185 |
| OCH₂C≡CH | 201-202.5 |
| OCH₂CO₂CH₃ | 181-183 |

### EXAMPLE 88

### Preparation of 3-[3-(Dichloromethyl)-1,2-benzisothiazol-5-yl]-1-methyl-6-(trifluoromethyl)-2,4-(1H,3H)-pyrimidinedione

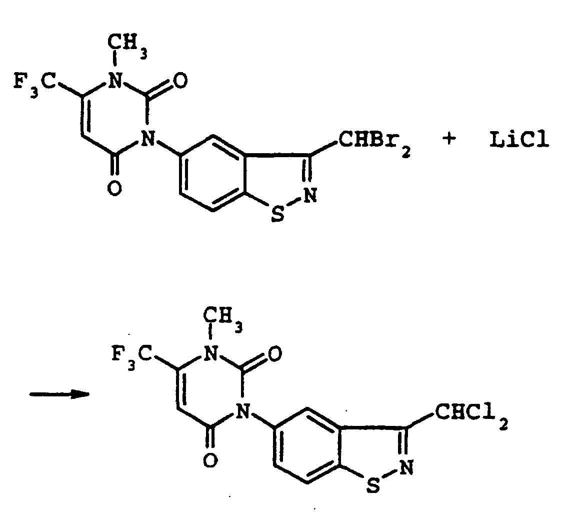

A solution of 3-[3-(dibromomethyl)-1,2-benzisothiazol-5-yl]-1-methyl-6-(trifluoromethyl)-2,4(1H,3H)-pyrimidinedione (0.750 g, 0.00150 mol) and lithium chloride (0.950 g, 0.0225 mol) in N,N-dimethylformamide is stirred at room temperature for 48 hours and diluted with ethyl acetate. The resultant mixture is washed sequentially with water and brine, dried over anhydrous magnesium sulfate, and concentrated *in vacuo* to obtain a foam. Column chromatography of the foam using silica gel, methylene chloride, and a 1% diethyl ether in methylene chloride solution gives the title product as a white foam (0.530 g, mp 148-149 °C).

Using essentially the same procedure, but substituting 3-[3-(bromomethyl)-1,2-benzisothiazol-5-yl]-1-methyl-6-(trifluoromethyl)-2,4(1H,3H)-pyrimidinedione for 3-[3-(dibromomethyl)-1,2-benzisothiazol-5-yl]-1-methyl-6-(trifluoromethyl)-2,4(1H,3H)-pyrimidinedione, 3-[3-(chloromethyl)-1,2-benzisothiazol-5-yl]-1-methyl-6-(trifluoromethyl)-2,4(1H,3H)-pyrimidinedione is obtained as a white solid, mp 201-203 °C.

### EXAMPLE 89

### Preparation of 3-[3-(Fluoromethyl)-1,2-benzisothiazol-5-yl]-1-methyl-6-(trifluoromethyl)-2,4(1H,3H)-pyrimidinedione

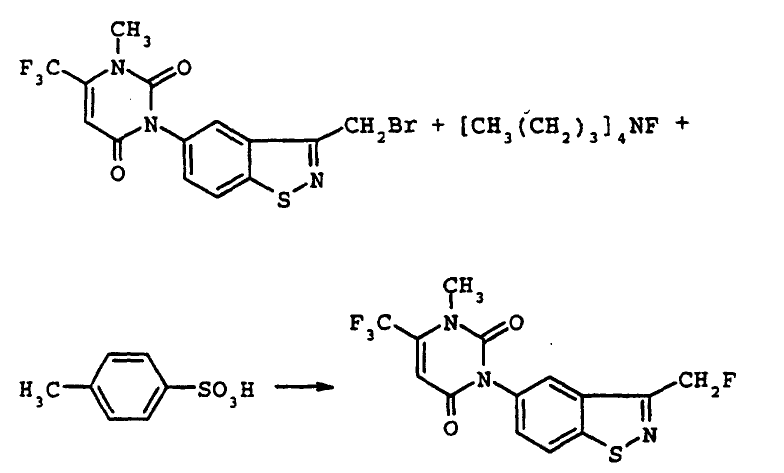

A solution of 3-[3-(bromomethyl)-1,2-benzisothiazol-5-yl]-1-methyl-6-(trifluoromethyl)-2,4(1H,3H)-pyrimidinedione (1.00 g, 0.00240 mol), 14.4 mL of a 1 M tetrabutylammonium fluoride in tetrahydrofuran solution (0.0144 mol) and anhydrous *p*-toluenesulfonic acid (1.36 g, 0.00790 mol) in tetrahydrofuran is stirred at room temperature for 30 minutes, refluxed for three hours, stirred at room temperature for three days, refluxed for 22 hours, partially concentrated *in vacuo*, and diluted with ethyl acetate. The resultant mixture is washed sequentially with water and brine, dried over anhydrous magnesium sulfate, and concentrated *in vacuo* to obtain an orange solid. Column chromatography of the solid using silica gel and a 2.5% diethyl ether in methylene chloride solution gives the title product as a white solid (0.42 g, mp 201-202 °C).

### EXAMPLE 90

### Preparation of 3-[3-(Difluoromethyl-1,2-benzisothiazol-5-yl]-1-methyl-6-(trifluoromethyl)-2,4(1H,3H)-pyrimidinedione

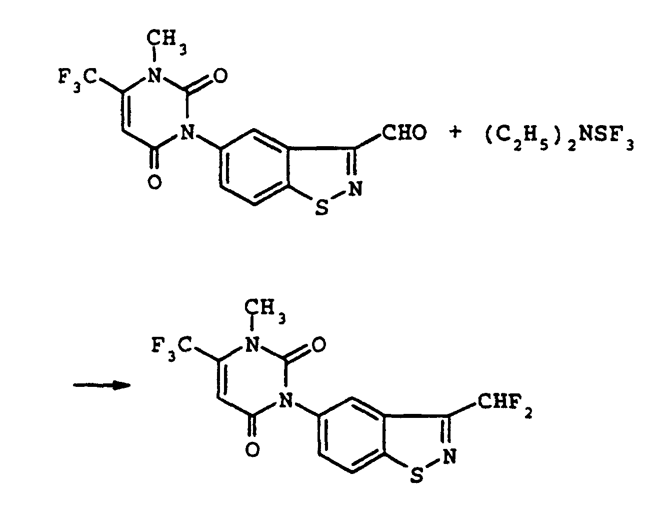

A solution of 5-[3,6-dihydro-3-methyl-2,6-dioxo-4-(trifluoromethyl)-1(2H)-pyrimidinyl]-1,2-benzisothiazole-3-carboxaldehyde (0.750 g, 0.00200 mol) and diethylaminosulfur trifluoride (0.260 mL, 0.00200 mol) in methylene chloride is stirred at room temperature for one hour, treated with additional diethylaminosulfur trifluoride (several drops), stirred at room temperature for 90 minutes, washed sequentially with water and brine, dried over anhydrous magnesium sulfate, and concentrated *in vacuo* to obtain a yellow solid. Flash column chromatography of the solid using silica gel and a 1% diethyl ether in methylene chloride solution gives the title product as a white solid (0.680 g, mp 183-184 °C).

### EXAMPLE 91

### Preparation of Diethyl {{5-[3,6-dihydro-3-methyl-2,6-dioxo-4-(trifluoromethyl)-1(2H)-pyrimidinyl]-1,2-benzisothiazol-3-yl}methyl}phosphonate

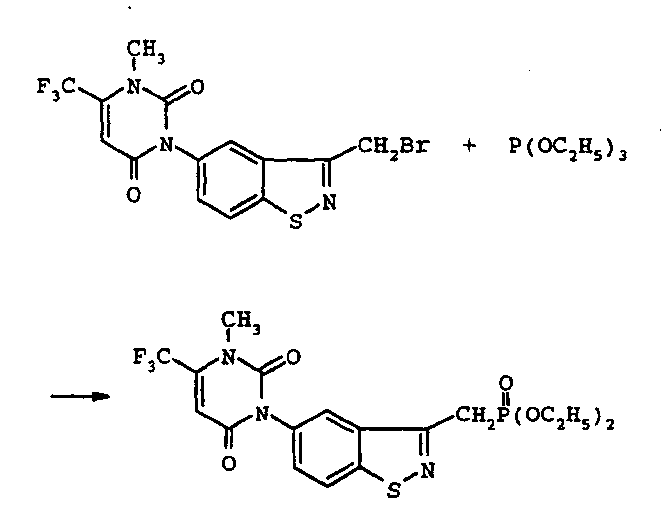

A mixture of 3-[3-(bromomethyl)-1,2-benzisothiazol-5-yl]-1-methyl-6-(trifluoromethyl)-2,4(1H,3H)-pyrimidinedione (1.00 g, 0.00240 mol) in diethyl ether is cooled to 10 °C, treated dropwise with triethyl phosphite (0.580 mL, 0.00340 mol), stirred at 10 °C for 90 minutes, diluted with 10 mL of tetrahydrofuran, stirred at room temperature overnight, refluxed for 1 day, cooled, partially concentrated *in vacuo* and diluted with methylene chloride. The resultant organic solution is washed with water, dried over anhydrous magnesium sulfate, and concentrated *in vacuo* to obtain a yellow solid. Flash column chromatography of the solid using silica gel and a diethyl ether/methylene chloride solution (1:1) gives the title product as a white solid (0.930 g, mp 142-144 °C).

Using essentially the same procedure, with the appropriate trialkyl phosphite, the following compounds are obtained:

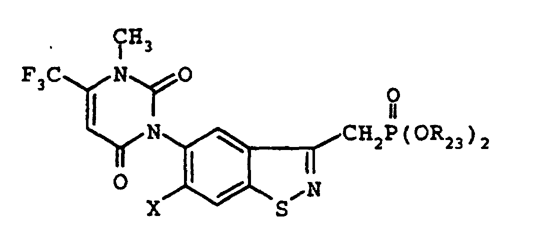

| **X** | **R**_{**23**} | **mp °C** |
|---|---|---|
| H | CH₃ | 124-127 |
| | | |
| H | CH(CH₃)₂ | 55-65 |
| | | |
| F | CH₂CH₃ | 65-75 |

### EXAMPLE 92

### Preparation of {{5-[3,6-Dihydro-3-methyl-2,6-dioxo-4-(trifluoromethyl)-1(2H)-pyrimidinyl]-1,2-benzisothiazol-3-yl}methyl}phosphonic acid

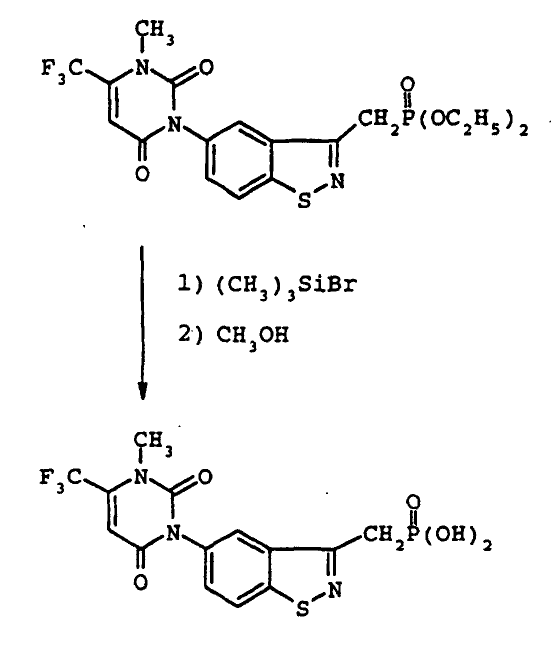

A solution of diethyl {{5-[3,6-dihydro-3-methyl-2,6-dioxo-4-(trifluoromethyl)-1(2H)-pyrimidinyl]-1,2-benzisothiazol-3-yl}methyl}phosphonate (1.00 g, 0.00210 mol) and bromotrimethylsilane (2.20 mL, 0.0168 mol) in methylene chloride (5.0 mL) is stirred at room temperature overnight and concentrated *in vacuo* to obtain a residue. The residue is diluted with methanol (10.0 mL), stirred at room temperature for 3.5 hours and concentrated *in vacuo* to obtain a tan solid. The solid is triturated in boiling methylene chloride, filtered and dried to give the title product as a beige solid (0.650 g, mp >230 °C).

### EXAMPLE 93

### Preparation of 1-Methyl-3-(3-propenyl-1,2-benzisothiazol-5-yl)-6-(trifluoromethyl)-2,4(1H,3H)-pyrimidinedione (Z)- and 1-Methyl-3-propenyl-1,2-benzisothiazol-5-yl)-6-(trifluoromethyl)-2,4(1H,3H)-pyrimidinedione, (E)- and (Z)-, (3:1)

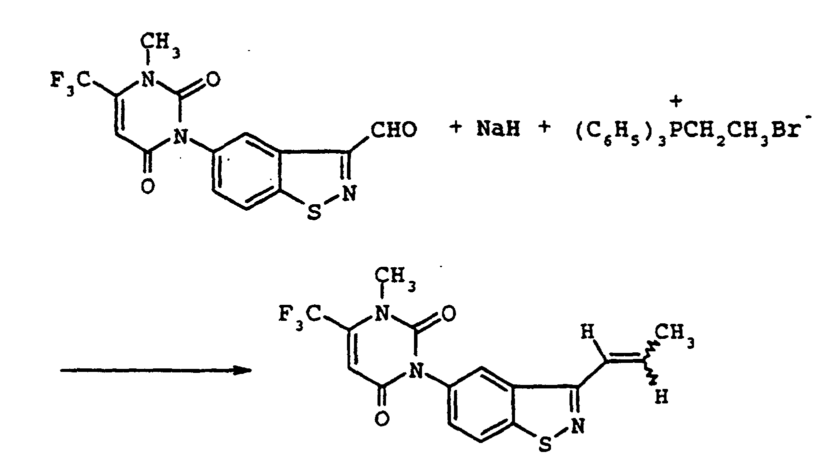

A mixture of (ethyl)triphenylphosphonium bromide (1.15 g, 0.00310 mol) in tetrahydrofuran is treated with sodium hydride (0.120 g, 0.00310 mol), stirred at room temperature for one hour, treated with a mixture of 5-[3,6-dihydro-3-methyl-2,6-dioxo-4-(trifluoromethyl)-1(2H)-pyrimidinyl]-1,2-benzisothiazole-3-carboxaldehyde (1.00 g, 0.00280 mol) in tetrahydrofuran, stirred at room temperature for one hour, and filtered. The resultant filtrate is partially concentrated *in vacuo*, diluted with methylene chloride, washed sequentially with water and brine, dried over anhydrous magnesium sulfate, and concentrated *in vacuo* to obtain an orange solid. Flash column chromatography of the solid using silica gel and a 1% diethyl ether in methylene chloride solution gives 1-methyl-3-(3-propenyl-1,2-benzisothiazol-5-yl)-6-(trifluoromethyl)-2,4(1H,3H)-pyrimidinedione, (Z)- as a white solid (0.350 g, mp 200-201 °C) and 1-methyl-3-(3-propenyl-1,2-benzisothiazol-5-yl)-6-(trifluoromethyl)-2,4(1H,3H)-pyrimidinedione, (E)- and (Z)-, (3:1) as a white solid (0.450 g, mp 228-230 °C).

Following essentially the same procedure and using the appropriately substituted phosphonium bromide, the following compounds are obtained:

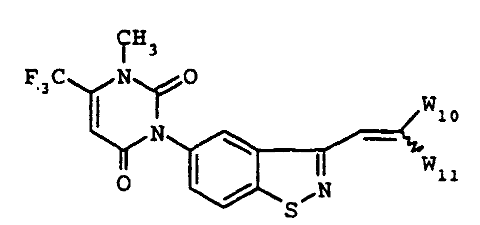

| **W**_{**10**} | **W**_{**11**} | **mp °C** |
|---|---|---|
| CO₂CH₃ | H | >230 |
| C(O)CH₃ | H | >230 |
| CHO | H | >230 |
| CH₂Br | H | 235 (dec) |
| C(O)C₆H₅ | H | |
| C(O)N(CH₃)OCH₃ | H | |
| CO₂CH₂=CH₂ | H | |
| CHO | CH₂CH₃ | |

### EXAMPLE 94

### Preparation of 1-Methyl-3-(3-morpholino-1,2-benzisothiazol-5-yl)-6-(trifluoromethyl)-2,4(1H,3H)-pyrimidinedione

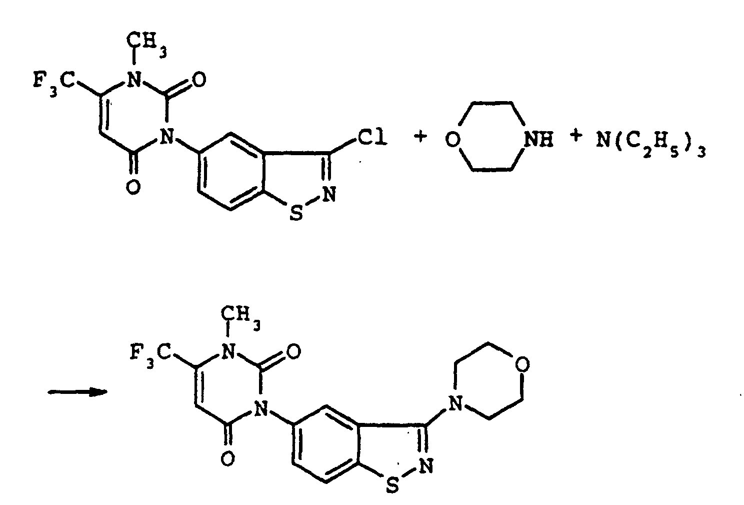

A mixture of 3-(3-chloro-1,2-benzisothiazol-5-yl)-1-methyl-6-(trifluoromethyl)-2,4(1H,3H)-pyrimidinedione (0.300 g), morpholine (2.00 g) and triethylamine (5.00 g) in tetrahydrofuran is refluxed for 18 hours and concentrated *in vacuo* to obtain a residue. Column chromatography of the residue using silica gel and 25% and 50% ethyl acetate in heptane solutions gives the title product as a yellow solid (0.210 g, mp 200-205 °C).

Using essentially the same procedure, but using the appropriate amine, the following compounds are obtained:

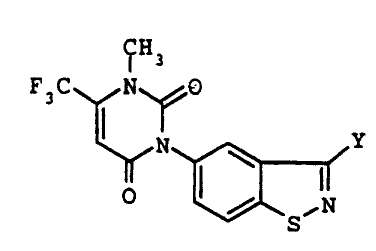

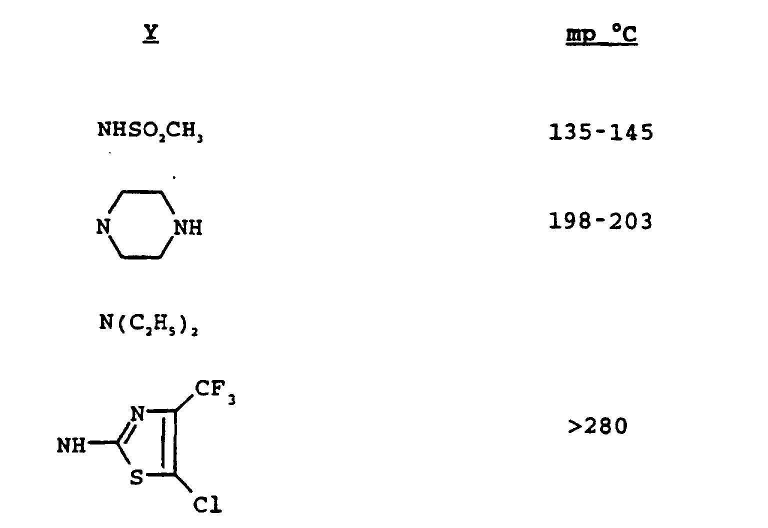

### EXAMPLE 95

### Preparation of 3-[3-(Azidomethyl)-1,2-benzisothiazol-5-yl]-1-methyl-6-(trifluoromethyl)-2,4(1H,3H)-pyrimidinedione

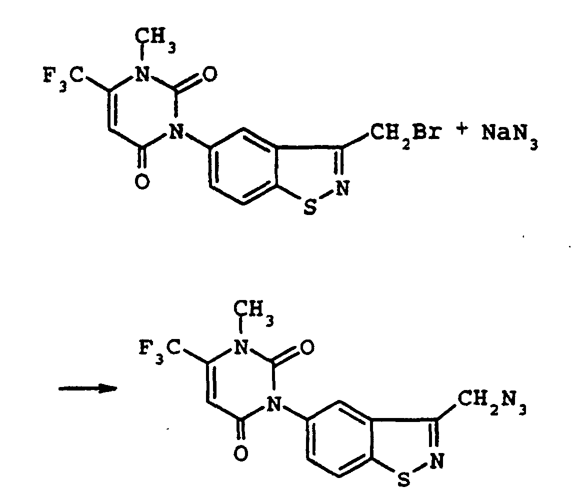

A solution of 3-[3-(bromomethyl)-1,2-benzisothiazol-5-yl]-1-methyl-6-(trifluoromethyl)-2,4(1H,3H)-pyrimidinedione (2.00 g, 4.76 mmol) in N,N-dimethylformamide is treated with sodium azide (0.325 g, 5.00 mmol), stirred overnight at room temperature, treated with 18-crown-6, stirred at room temperature for two hours, and poured into an ice-water mixture. The resultant aqueous mixture is filtered to obtain a solid. The solid is washed with water and air-dried to give the title product as a white solid which is identified by NMR spectral analysis.

### EXAMPLE 96

### Preparation of Diethyl {{5-[3,6-dihydro-3-methyl-2,6-dioxo-4-(trifluoromethyl)-1(2H)-pyrimidinyl]-1,2-benzisothiazol-3-yl}methyl}phosphoramidate

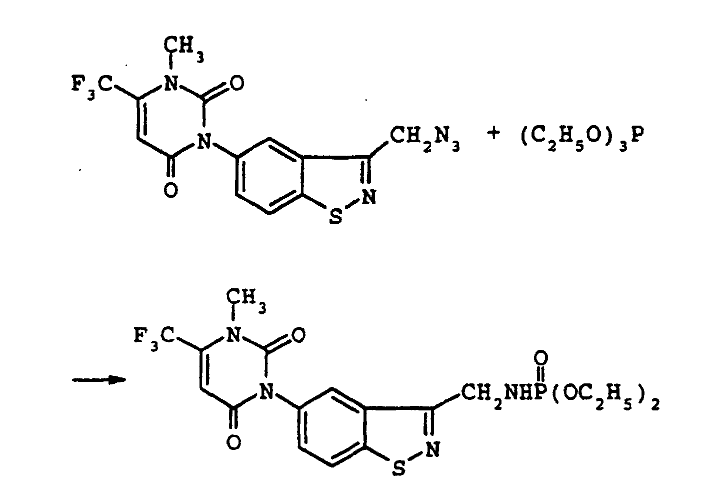

A mixture of 3-[3-(azidomethyl)-1,2-benzisothiazol-5-yl]-1-methyl-6-(trifluoromethyl)-2,4(1H,3H)-pyrimidinedione (1.61 g, 4.20 mmol) and triethyl phosphite (0.860 mL, 5.00 mmol) in dioxane is refluxed for 30 minutes, concentrated *in vacuo*, diluted with water and extracted with methylene chloride. The organic extracts are combined, dried over anhydrous magnesium sulfate, and concentrated *in vacuo* to obtain a yellow oil. Column chromatography of the oil using silica gel and 10% to 40% acetonitrile in methylene chloride solutions gives the title product as a yellow foam (1.00 g, mp 133-134 °C).

### EXAMPLE 97

### Preparation of tert-Butyl α-acetyl-5-[3,6-dihydro-3-methyl-2,6-dioxo-4-(trifluoromethyl)-1(2H)-pyrimidinyl]-1,2-benzisothiazole-3-propionate

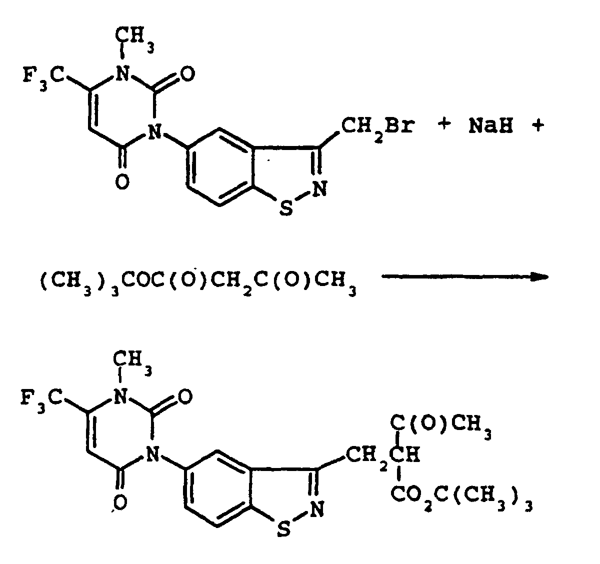

A mixture of sodium hydride (60% in oil, 0.293 g, 7.33 mmol) in tetrahydrofuran is cooled to 0 °C, treated dropwise with a solution of *tert*-butyl acetoacetate (1.00 mL, 6.03 mmol) in tetrahydrofuran, and stirred for several minutes. The resultant mixture is added dropwise to a solution of 3-[3-(bromomethyl)-1,2-benzisothiazol-5-yl]-1-methyl-6-(trifluoromethyl)-2,4(1H,3H)-pyrimidinedione (1.40 g, 3.33 mmol) in methyl sulfoxide. After the addition is complete, the reaction mixture is poured into an ice-water mixture containing a trace of 10% hydrochloric acid. The resultant aqueous mixture is extracted with methylene chloride. the organic extracts are combined, dried over anhydrous magnesium sulfate, and concentrated *in vacuo* to obtain an oil. Column chromatography of the oil using silica gel and 0%, 2%, 3%, and 15% diethyl ether in methylene chloride solutions gives the title product as a solid, mp 76-78 °C.

Using essentially the same procedure, the following compounds are obtained:

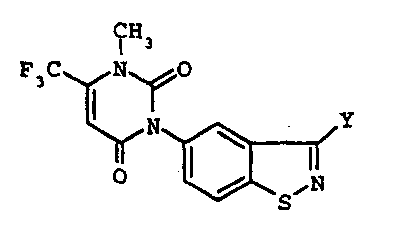

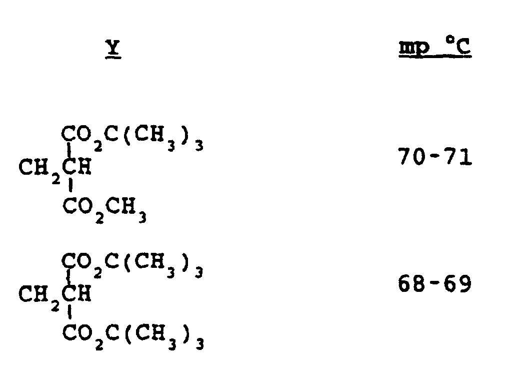

### EXAMPLE 98

### Preparation of 1-Methyl-3-[3-(3-oxobutyl)-1,2-benzisothiazol-5-yl]-6-(trifluoromethyl)-2,4(1H,3H)-pyrimidinedione and α-Acetyl-5-[3,6-dihydro-3-methyl-2,6-dioxo-4-(trifluoromethyl)-1(2H)-pyrimidinyl]-1,2-benzisothiazole-3-propionic acid

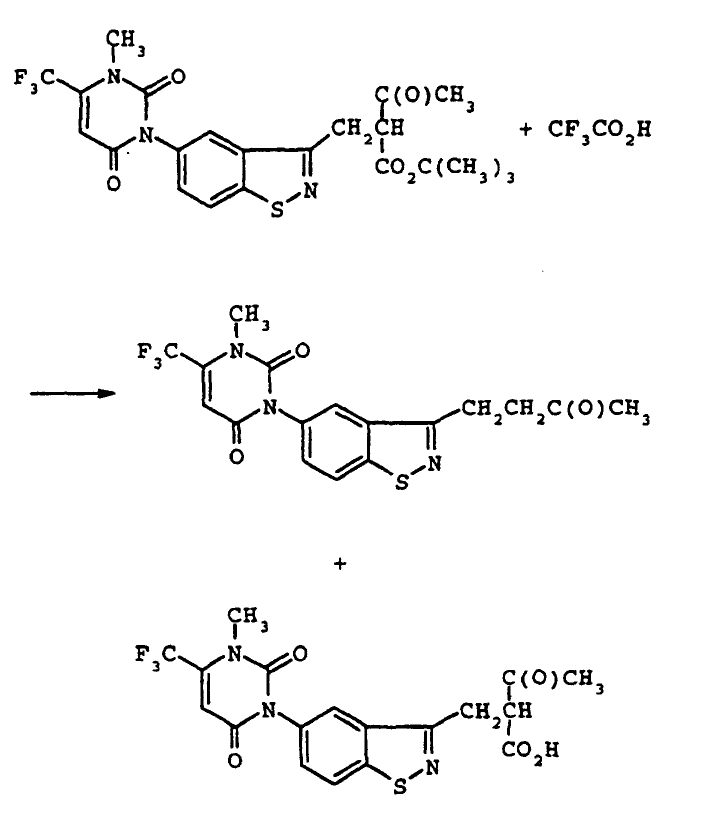

A mixture of *tert*-butyl α-acetyl-5-[3,6-dihydro-3-methyl-2,6-dioxo-4-(trifluoromethyl)-1(2H)-pyrimidinyl]-1,2-benzisothiazole-3-propionate (0.700 g) and trifluoroacetic acid (10.0 mL) in methylene chloride is stirred overnight at room temperature, concentrated *in vacuo*, and diluted with methylene chloride and saturated sodium hydrogen carbonate solution. The phases are separated. The organic phase is dried over anhydrous magnesium sulfate and concentrated *in vacuo* to give 1-methyl-3-[3-(3-oxobutyl)-1,2-benzisothiazol-5-yl]-6-(trifluoromethyl)-2,4(1H,3H)-pyrimidinedione as a white solid (0.310 g, mp 198-199 °C). The aqueous phase is acidified with concentrated hydrochloric acid, stirred for several minutes, and filtered to obtain a solid. The solid is washed with water and air-dried to give α-acetyl-5-[3,6-dihydro-3-methyl-2,6-dioxo-4-(trifluoromethyl)-1(2H)-pyrimidinyl]-1,2-benzisothiazole-3-propionic acid as a white solid, mp 191-193 °C.

Using essentially the same procedure, the following compound is obtained:

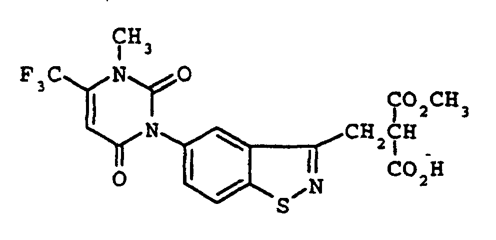

### EXAMPLE 99

### Preparation of Methyl 5'-[3,6-dihydro-3-methyl-2,6-dioxo-4-(trifluoromethyl)-1(2H)-pyrimidinyl]-1,2-benzisothiazole-3-propionate

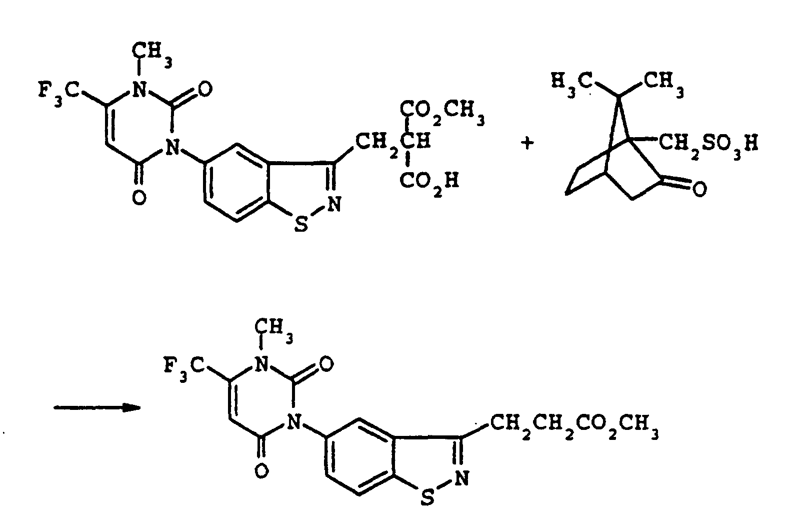

A mixture of monomethyl {5-{3,6-dihydro-3-methyl-2,6-dioxo-4-(trifluoromethyl)-1(2H)-pyrimidinyl]-1,2-benzisothiazol-3-yl}methyl}malonate (0.610 g) and camphor sulfonic acid (0.100 g) in toluene is refluxed for two hours, treated with additional camphor sulfonic acid (0.400 g), refluxed for three hours, concentrated *in vacuo*, diluted with 1,1,2,2-tetrachloroethane, refluxed for four hours, cooled to room temperature, concentrated *in vacuo,* and diluted with methylene chloride and saturated sodium hydrogen carbonate solution. The organic phase is separated, dried over anhydrous magnesium sulfate, and concentrated *in vacuo* to obtain a solid. The solid is stirred in hexanes for one hour, filtered, and dried to give the title product as a white solid (0.400 g, mp 176-178 °C).

### EXAMPLE 100

### Preparation of N-{{5-[3,6-Dihydro-3-methyl-2,6-dioxo-4-(trifluoromethyl)-1(2H)-pyrimidinyl]-1,2-benzisothiazole-3-yl}methyl}phthalimide

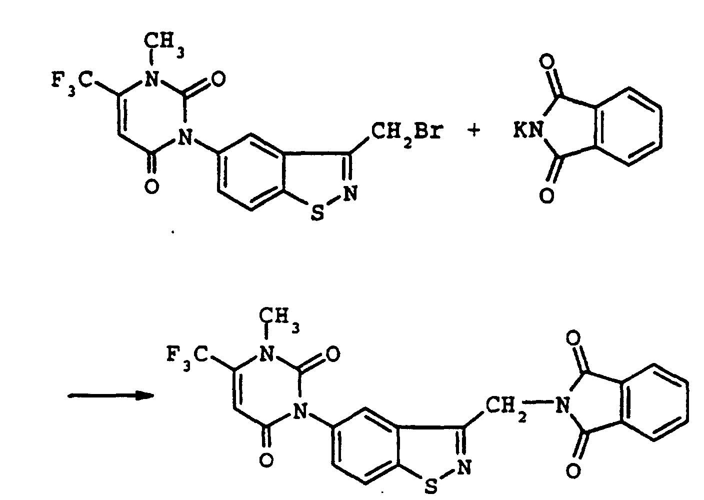

A mixture of 3-[3-(bromomethyl)-1,2-benzisothiazol-5-yl]-1-methyl-6-(trifluoromethyl)-2,4(1H,3H)-pyrimidinedione (0.750 g) and potassium phthalimide (0.350 g) in acetonitrile is refluxed for one hour, cooled to room temperature, and poured into an ice-water mixture. The resultant aqueous mixture is filtered to obtain a solid. The solid is washed with water and air-dried to give the title product (0.720 g, mp 204-208 °C).

### EXAMPLE 101

### Preparation of 3-[3-(o-Hydroxyphenyl)-1,2-benzisothiazol-5-yl]-1-methyl-6-(trifluoromethyl)-2,4(1H,3H)-pyrimidinedione trifluoromethylsulfonate (ester)

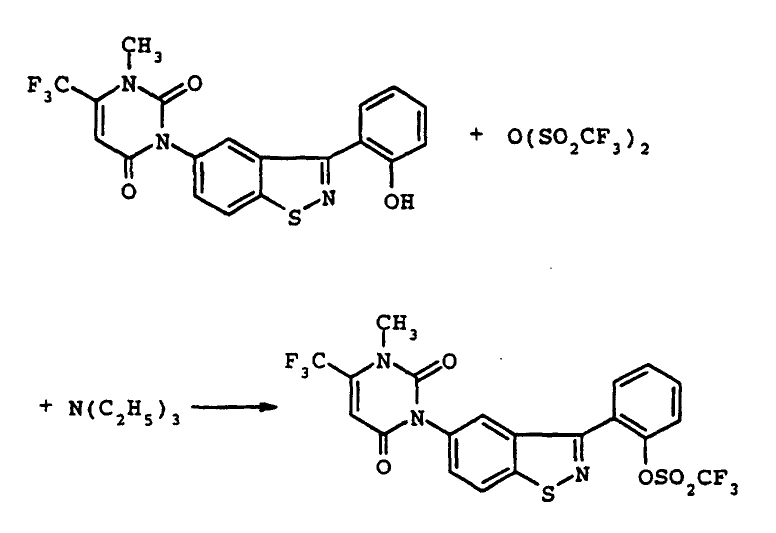

A mixture of 3-[3-(*o*-hydroxyphenyl)-1,2-benzisothiazol-5-yl]-1-methyl-6-(trifluoromethyl)-2,4(1H,3H)-pyrimidinedione (3.67 g, 8.75 mmol) and triethylamine (0.970 g, 9.63 mmol) in methylene chloride is cooled with an ice-water bath, treated with triflic anhydride (2.72 g, 9.63 mmol), stirred at room temperature overnight, and poured into water. The resultant aqueous mixture is extracted with methylene chloride. The organic extracts are combined, washed with brine, dried over anhydrous magnesium sulfate, and concentrated *in vacuo* to obtain an oil. Column chromatography of the oil using silica gel and a 3% diethyl ether in methylene chloride solution gives the title product as a white foam which is identified by NMR spectral analyses.

### EXAMPLE 102

### Preparation of Methyl o-{{5-[3,6-dihydro-3-methyl-2,6-dioxo-4-(trifluoromethyl)-1(2H)-pyrimidinyl]-1,2-benzisothiazol-3-yl}cinnamate

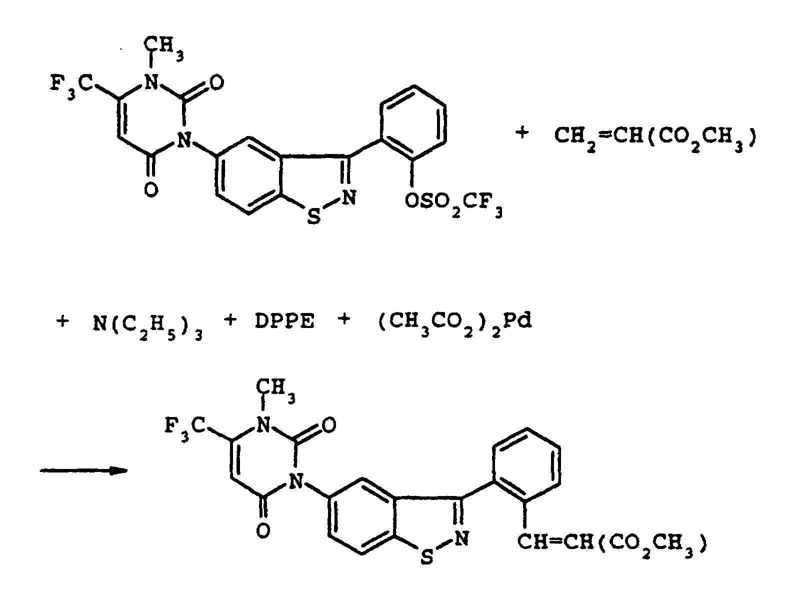

A solution of 3-[3-(*o*-hydroxyphenyl)-1,2-benzisothiazol-5-yl]-1-methyl-6-(trifluoromethyl)-2,4(1H,3H)-pyrimidinedione trifluoromethylsulfonate (ester) (1.00 g, 1.81 mmol) in N,N-dimethylformamide is treated sequentially with triethylamine (0.200 g, 1.99 mmol), methyl acrylate (0.310 g, 3.62 mmol), diphenylphosphinoethane (DPPE, 0.0220 g, 0.0500 mmol) and palladium(II) acetate (0.0100 g, 0.0450 mmol), stirred overnight at 100 °C, cooled to room temperature, and poured into water. The resultant aqueous mixture is filtered to obtain a solid. The solid is washed with water and dissolved in methylene chloride. The resultant organic solution is washed sequentially with water and brine, dried over anhydrous
magnesium sulfate, and concentrated *in vacuo* to obtain an oil. Column chromatography of the oil using silica gel and a 5% diethyl ether in methylene chloride solution affords an oil which is triturated with diethyl ether to give the title product as an off-white solid (0.460 g, mp 150-152 °C).

### EXAMPLE 103

### Preparation of a mixture of 3-[3-(Hydroxymethyl)-1,2-benzisothiazol-5-yl]-1-methyl-6-(trifluoromethyl)-2,4(1H,3H)-pyrimidinedione and 5-[3,6-Dihydro-3-methyl-2,6-dioxo-4-(trifluoromethyl)-1(2H)-pyrimidinyl]-1,2-benzisothiazole-3-carboxaldehyde, (2:3)

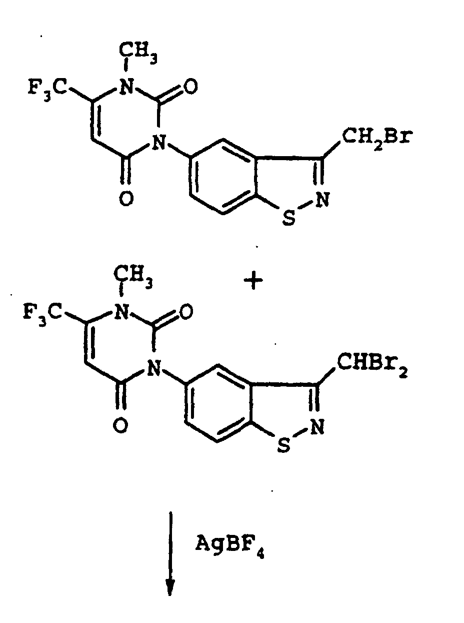

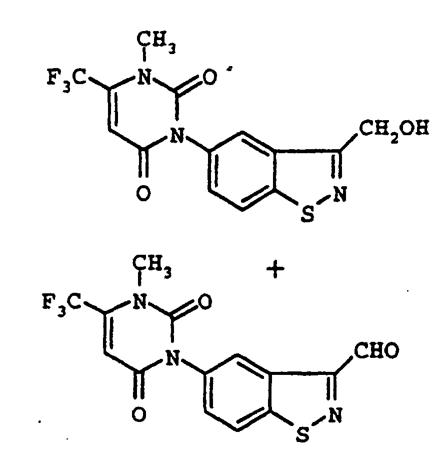

A solution of a 2:3 mixture of 3-[3-(bromomethyl)-1,2-benzisothiazol-5-yl]-1-methyl-6-(trifluoromethyl)-2,4(1H,3H)-pyrimidinedione and 3-[3-(dibromomethyl)-1,2-benzisothiazol-5-yl]-1-methyl-6-(trifluoromethyl)-2,4(1H,3H)-pyrimidinedione (5.00 g), silver tetrafluoroborate (3.50 g), 1,4-dioxane (30.0 mL) and water (10.0 mL) is refluxed for 2 hours, cooled, and filtered to remove solids. The resultant filtrate is poured into a methylene chloride and water mixture. The organic phase is separated, washed sequentially with water and brine, dried over anhydrous sodium sulfate, and concentrated *in vacuo* to give a 2:3 mixture of the title products.

### EXAMPLE 104

### Preparation of 5-[3,6-Dihydro-3-methyl-2,6-dioxo-4-(trifluoromethyl)-1(2H)-pyrimidinyl]-1,2-benzisothiazole-3-carboxylic acid

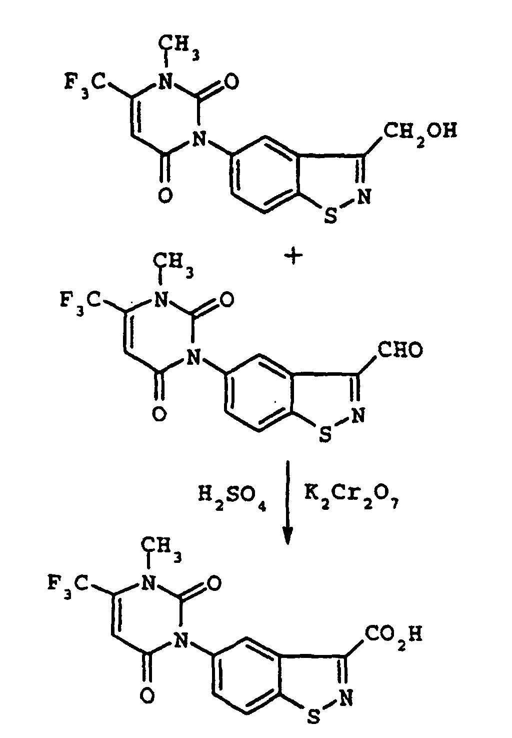

A solution of potassium dichromate (10.0 g) in 1.5 M sulfuric acid (200 mL) is cooled to 0°C, treated dropwise with a solution of a 2:3 mixture of 3-[3-(hydromethyl)-1,2-benzisothiazol-5-yl]-1-methyl-6-(trifluoromethyl)-2,4(1H,3H)-pyrimidinedione and 5-[3,6-dihydro-3-methyl-2,6-dioxo-4-(trifluoromethyl)-1(2H)-pyrimidinyl]-1,2-benzisothiazole-3-carboxaldehyde (12.0 g) in acetic acid, stirred at room temperature for 2 hours, and diluted with water. The resultant aqueous mixture is extracted with methylene chloride. The organic extracts are combined, washed sequentially with water and brine, dried over anhydrous sodium sulfate, and concentrated *in vacuo* to give the title product as a white solid (8.50 g, mp 149-152 °C).

### EXAMPLE 105

### Preparation of 5-[3,6-Dihydro-3-methyl-2,6-dioxo-4-(trifluoromethyl)-1(2H)-pyrimidinyl]-N-(methylsulfonyl)-1,2-benzisothiazole-3-carboxamide

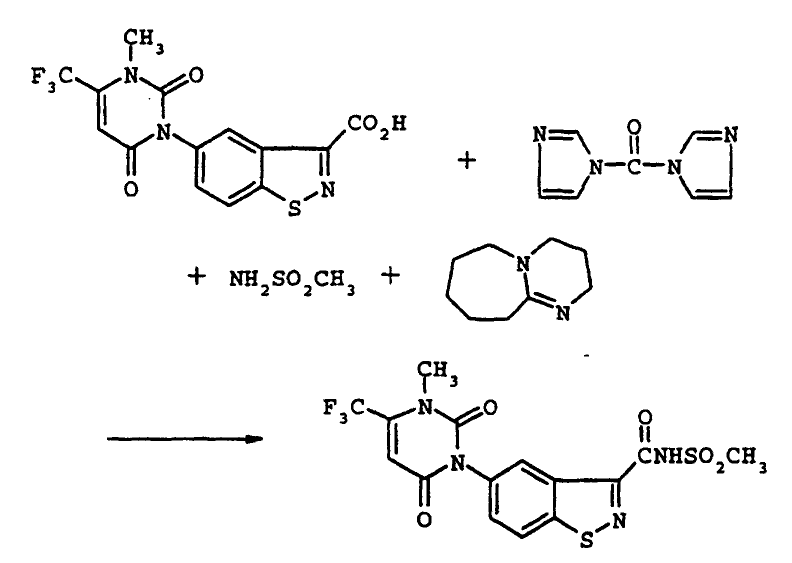

A solution of 5-[3,6-dihydro-3-methyl-2,6-dioxo-4-(trifluoromethyl)-1(2H)-pyrimidinyl]-1,2-benzisothiazole-3-carboxylic acid (0.400 g, 1.08 mmol) in tetrahydrofuran is treated with 1,1'-carbonyldiimidazole (0.260 g, 1.58 mmol), stirred at room temperature for 30 minutes, refluxed for 2 hours, cooled to room temperature, treated dropwise with a solution of methanesulfonamide (0.130 g, 1.37 mmol) and 1,8-diazabicyclo[5.4.0]undec-7-ene (0.230 g, 1.50 mmol), stirred at room temperature for 3 hours, and poured into 0.5 N hydrochloric acid. The resultant aqueous mixture is extracted with methylene chloride. The organic extracts are combined, washed with brine, dried over anhydrous sodium sulfate, and concentrated *in vacuo* to obtain a residue. Column chromatography of the residue using silica gel and a methylene chloride/acetic acid solution (10:1) gives the title product as a white solid (0.350 g, 72%, mp 241-243 °C).

Using essentially the same procedure, but using the appropriately substituted sulfonamide, the following compounds are obtained:

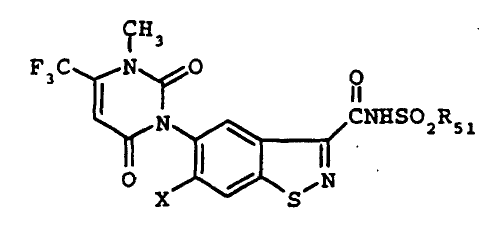

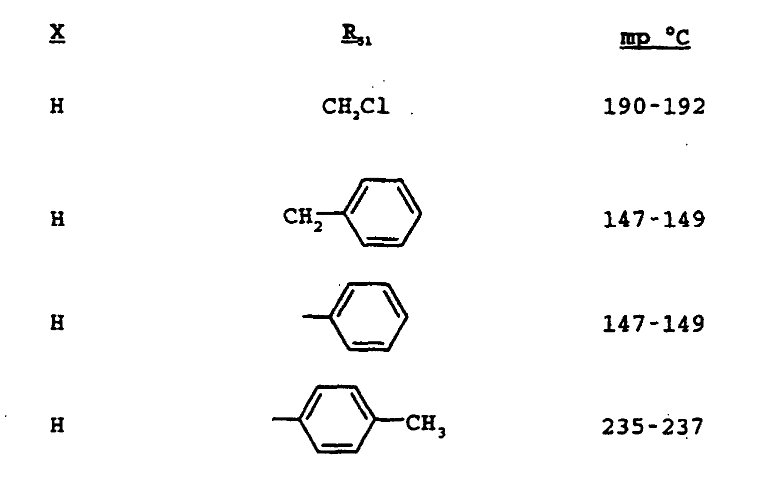

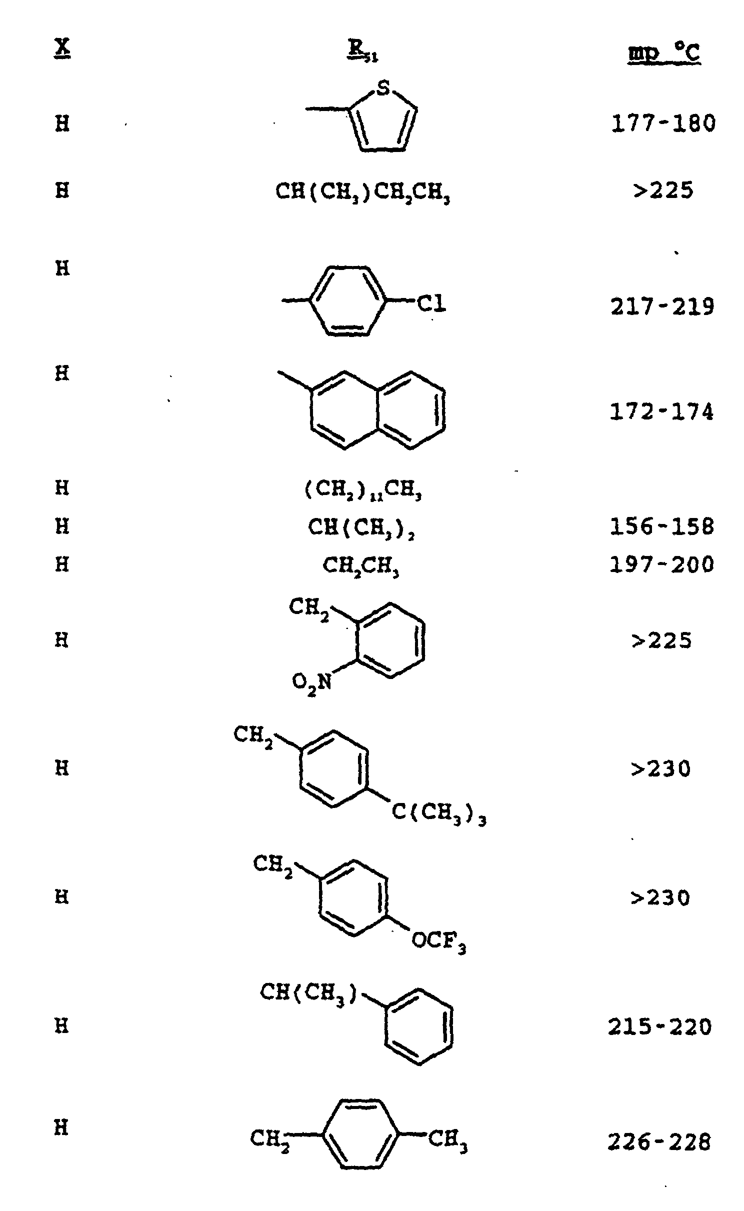

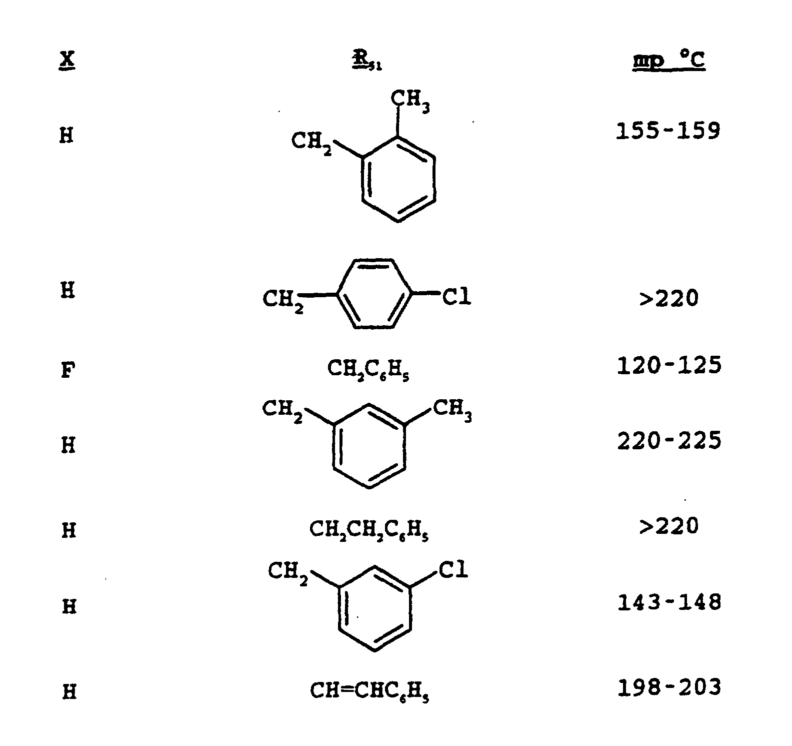

### EXAMPLE 106

### Preparation of 2-{{2-[5-[3,6-Dihydro-3-methyl-2,6-dioxo-4-(trifluoromethyl)-1(2H)-pyrimidinyl]-6-fluoro-1,2-benzisoxazol-3-yl}-p-tolyl}oxy-N-(methylsulfonyl)-acetamide

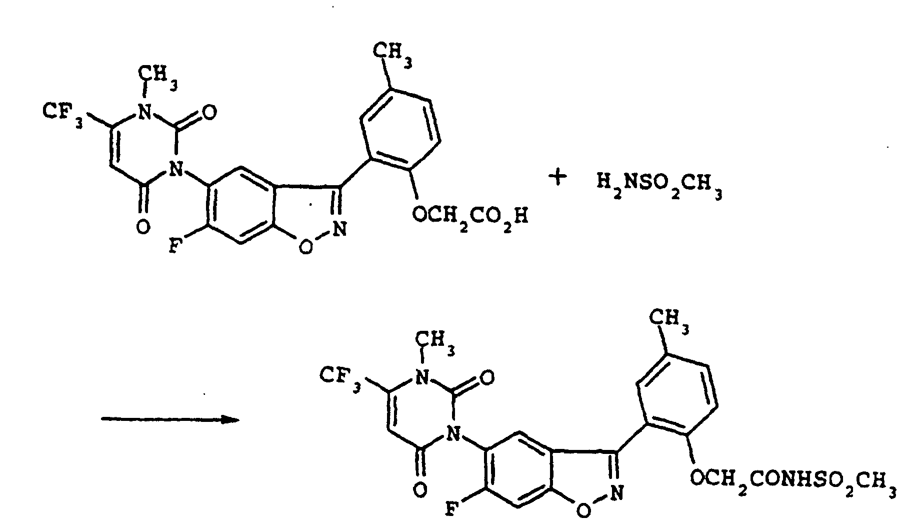

A solution of {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetic acid (1.54 g, 0.00312 mol) in tetrahydrofuran is added dropwise to a suspension of 1,1'-carbonyldiimidazole (1.06 g, 0.00655 mol) in tetrahydrofuran. The resultant mixture is stirred 1.5 hours and cooled to room temperature. A mixture of methanesulfonamide (0.594 g, 0.00624 mol), diazabicycloundecane (1.09 g, 0.00702 mol) and tetrahydrofuran is added dropwise. The resultant mixture is stirred overnight at room temperature and poured into 10% hydrochloric acid. The resultant white solid is filtered and dried to give the title compound (1.65 g, 91.6%, mp 123-125°C) which is identified by NMR spectral analysis.

### EXAMPLE 107

### Preparation of N-{{2-{{5-[3,6-Dihydro-3-methyl-2,6-dioxo-4-(trifluoromethyl)-1(2H)-pyrimidinyl]-1,2-benzisoxazol-3-yl}-p-tolyl}oxy}acetyl}glycine, methyl ester

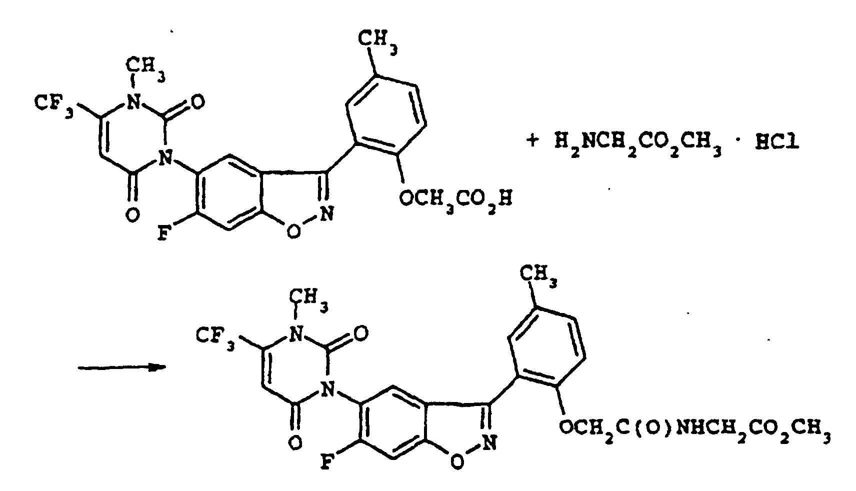

1,1'-Carbonyldiimidazole (0.340 g, 0.00203 mol) is added to a solution of {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetic acid (1.00 g, 0.00203 mol) in dimethylformamide at 0 °C. The mixture is stirred 30 minutes at 0°C and warmed to room temperature. Glycine methyl ester hydrochloride (0.260 g, 0.00207 mol) is added and the resultant mixture is stirred three days at room temperature. The mixture is diluted with water and filtered to afford a white solid, which is chromatographed on silica gel with ether:methylene chloride to afford the title compound as a white foam (0.830 g, 74.8%) which is identified by NMR spectral analysis.

### EXAMPLE 108

### Preparation of {{2-{5-[3,6-Dihydro-3-methyl-2,6-dioxo-4-(trifluoromethyl)-1(2H)-pyrimidinyl]-6-fluoro-1,2-benzisoxazol-3-yl}-p-tolyl}oxy}acetic acid

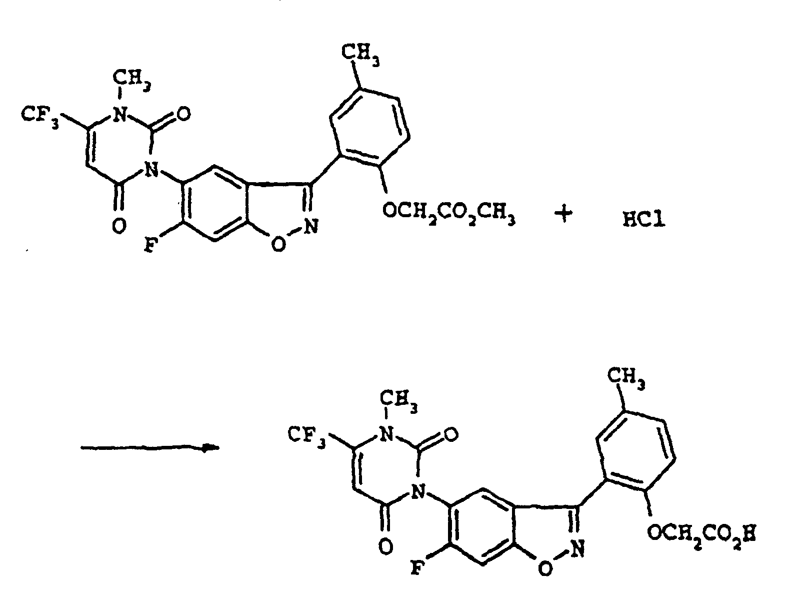

A mixture of {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetic acid, methyl ester (10.9 g, 0.0190 mol), 10% hydrochloric acid and acetic acid is stirred two hours at 90 °C, cooled, poured into water and extracted with ethyl acetate. The organic layers are combined, washed with water and brine, dried over anhydrous sodium sulfate, filtered and concentrated *in vacuo* to a foam. The foam is recrystallized from ethyl acetate to afford the title compound as a white solid (6.20 g, 66.2%, mp 123-126 °C).

### EXAMPLE 109

### Preparation of 2-{{2-{5-[3,6-Dihydro-3-methyl-2,6-dioxo-4-(trifluoromethyl)-1(2H)-pyrimidinyl]-1,2-benzisothiazol-3-yl}-p-tolyl}oxy}-2-methylpropionic acid, 2-propynyl ester

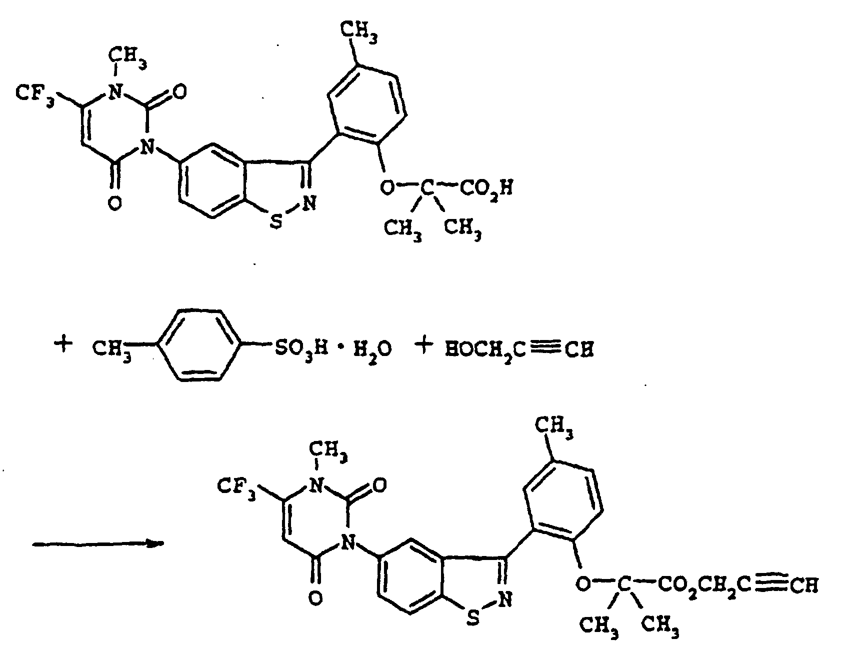

A mixture of 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methylpropionic acid (0.500 g, 0.000960 mol), p-toluenesulfonic acid (0.0100 g), propargyl alcohol (0.0538 g, 0.000960 mol) and toluene is stirred one day at 50°C, cooled to room temperature and concentrated *in vacuo* to obtain a residue. Chromatography of the residue on silica gel with ether:methylene chloride affords the title compound (0.360 g, 67.2%) which is identified by NMR spectral analysis.

Following essentially the same procedure, but using the appropriate carboxylic acids and alcohols, the following compounds are obtained:

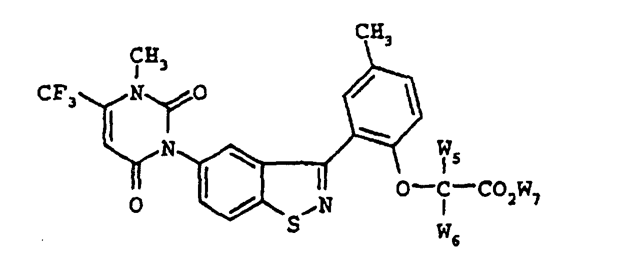

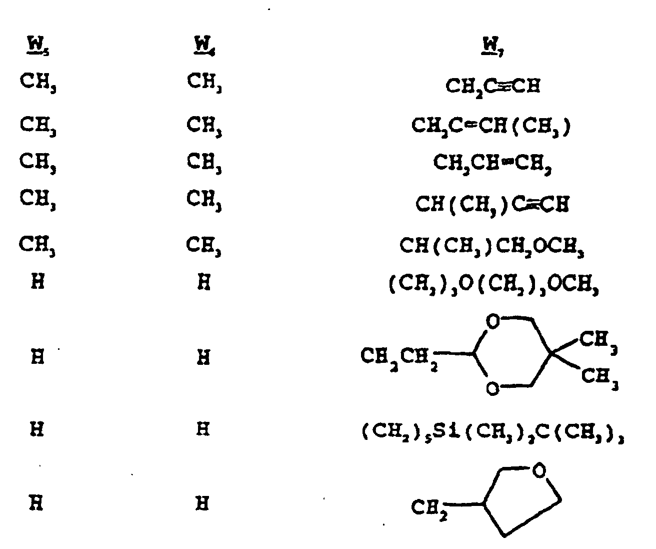

### EXAMPLE 110

### Preparation of 3-[3-(2-Hydroxy-3-iodo-m-tolyl)-1,2-benzisothiazol-5-yl]-1-methyl-6-(trifluoromethyl)-2,4(1H,3H)-pyrimidinedione

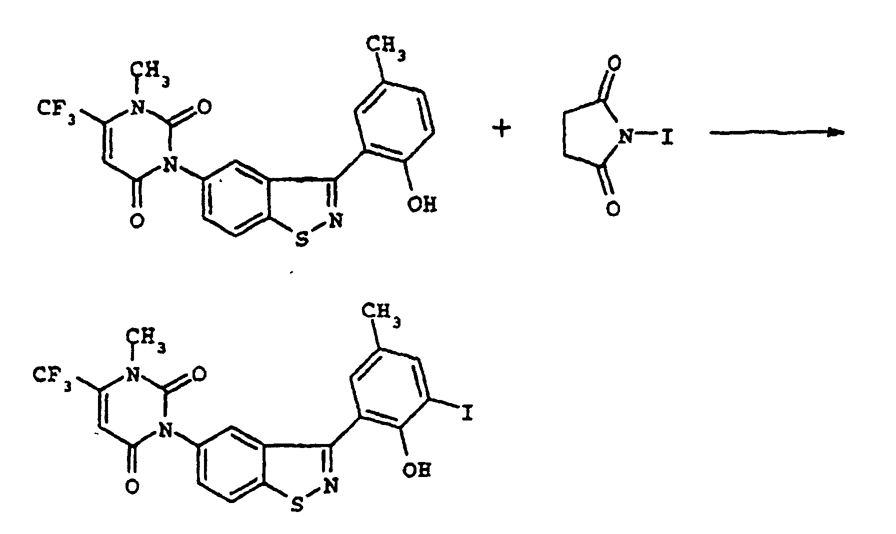

A solution of N-iodosuccinimide (0.240 g, 0.00107 mol) in acetone is added dropwise to a solution of 3-[3-(2-hydroxy-*m*-tolyl)-1,2-benzisothiazol-5-yl]-1-methyl-6-(trifluoromethyl)-2,4(1H,3H)-pyrimidinedione (0.430 g, 0.000992 mol) in acetone and the resultant mixture is stirred overnight at room temperature. Additional N-iodosuccinimide (0.120 g, 0.000635 mol) is added and the mixture is stirred three hours at room temperature. The mixture is concentrated *in vacuo* to afford a red gum which is chromatographed on silica gel with ether: methylene chloride. The fractions containing the title compound are washed with 1M sodium thiosulfate and brine, dried over anyhdrous magnesium sulfate and concentrated *in vacuo* to afford the title compound as a yellow solid (0.280 g, 46.8%, mp 241-242 °C).

### EXAMPLE 111

### Preparation of 3-[3-(5-Iodo-2-methoxy-p-tolyl)-1,2-benzisothiazol-5-yl]-1-methyl-6-(trifluoromethyl)-2,4(1H,3H)-pyrimidinedione

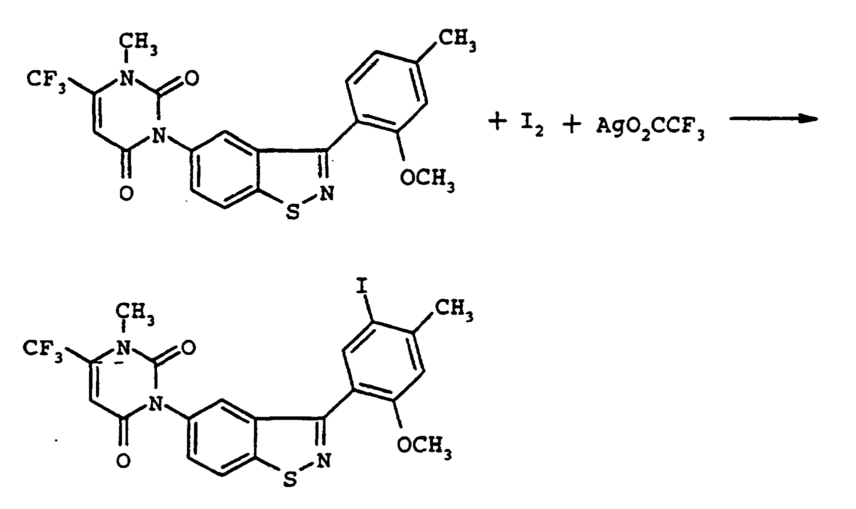

A mixture of 3-[3-(6-methoxy-*p*-tolyl)-1,2-benzisothiazol-5-yl]-1-methyl-6-(trifluoromethyl)-2,4(1H,3H)-pyrimidinedione (1.34 g, 0.00300 mol), silver trifluoroacetate and methylene chloride is treated with iodine (1.14 g, 0.00450 mol) in portions, stirred overnight at room temperature, diluted with methylene chloride, washed with 1M sodium thiosulfate and brine, dried over anhydrous magnesium sulfate, and concentrated *in vacuo* to a brown oil which solidifies on standing. Recrystallization from ether affords the title compound as a light yellow crystalline solid (1.50 g, 87.2%, mp 210-212 °C).

### EXAMPLE 112

### Preparation of 3-[3-(α,α-Dibromo-2-methoxy-p-tolyl)-1,2-benzisothiazol-5-yl]-1-methyl-6-(trifluoromethyl)-2,4(1H,3H)-pyrimidinedione

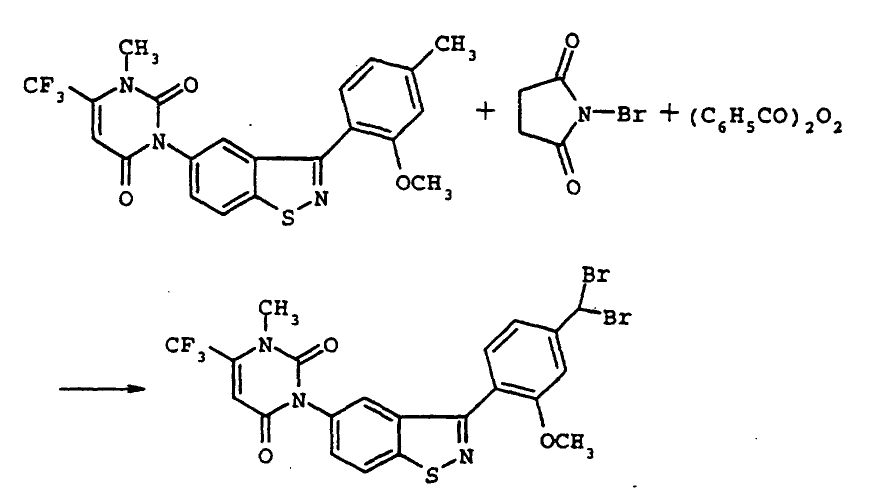

A mixture of 3-[3-(2-methoxy-*p*-tolyl)-1,2-benzisothiazol-5-yl]-1-methyl-6-(trifluoromethyl)-2,4-(1H,3H)-pyrimidinedione (1.34 g, 0.00300 mol), N-bromosuccinimide (1.39 g, 0.00780 mol), benzoyl peroxide (0.200 g) and carbon tetrachloride is stirred three hours at reflux and filtered hot. The filtrate is cooled to room temperature and concentrated *in vacuo.* The residue is chromatographed on silica gel with ether:methylene chloride to afford the title compound as a white foam (0.640 g, 35.3%) which is identified by NMR spectral analysis.

### EXAMPLE 113

### Preparation of 5-Nitro-1,2-benzisothiazole

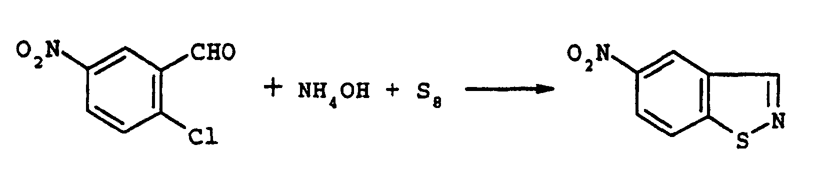

To a mixture of ammonium hydroxide (1000 ml) and N,N-dimethylformamide is added 2-chloro-5-nitrobenzaldehyde (300 g, 1.62 mol) and sulfur (54.4 g, 1.70 mol). The mixture is heated slowly to and stirred at 90 °C for one hour, cooled to room temperature, poured onto ice, and diluted with water. Filtration affords the title compound as a yellow solid (277.1 g, 94.9%).

### EXAMPLE 114

### Preparation of 3-Chloro-5-nitro-1,2-benzisothiazole

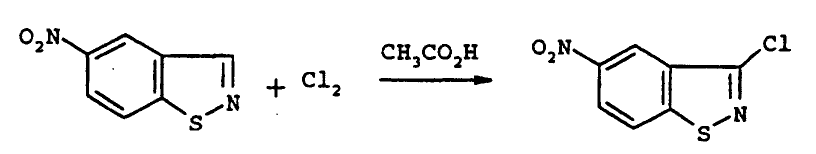

A suspension of 5-nitro-1,2-benzisothiazole (271 g, 1.50 mol) in acetic acid is heated to 80 °C to form a solution. The heating source is removed and chlorine gas is added continuously over six hours at 70-80 °C until saturation of the mixture occurs. The mixture is cooled to room temperature and stirred overnight. Filtration affords the title compound as a yellow crystalline solid (237 g, 73.6%) which is identified by NMR spectral analysis.

### EXAMPLE 115

### Preparation of 2'-Chloro-2-methyl-2-carboethoxy propiophenone

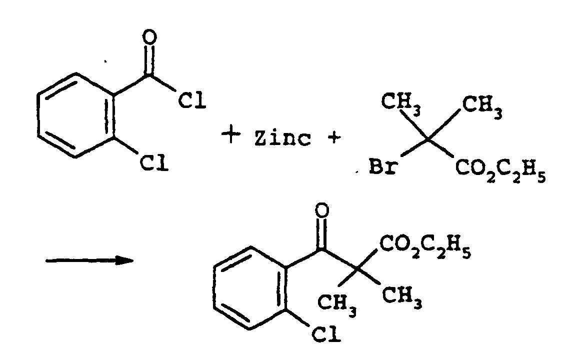

A mixture of 2-chlorobenzoyl chloride (52.2 g, 0.298 mol), ethyl 2-bromoisobutyrate (58.2 g, 0.298 mol) and ether is added in portions to zinc foil (19.5 g, 0.298 mol) and the resultant mixture stirred at reflux for three hours and overnight at room temperature. The mixture is poured into cold, dilute sulfuric acid and the organic layer is washed with saturated sodium bicarbonate and brine, dried over anhydrous magnesium sulfate and concentrated *in vacuo* to a yellow oil. The oil is chromatographed on silica gel with hexanes:ethyl acetate to afford the title compound as a colorless oil (41.8 g, 55.1%).

### EXAMPLE 116

### Preparation of 2'-Chloro-5'-nitro-2-methyl-2-carboethoxypropiophenone

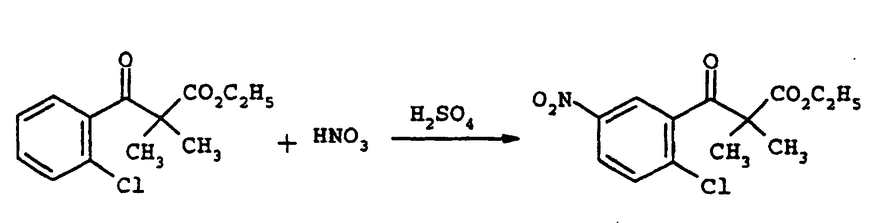

To concentrated sulfuric acid (15.0 ml) at 5 °C is added 2'-chloro-2-methyl-2-carboethoxypropiophenone (4.00 g, 0.01570 mol) followed by dropwise addition of concentrated nitric acid (90%, 0.740 ml, 0.0204 mol). After stirring 5 minutes, the mixture is poured onto ice and extracted with ethyl acetate. The organic layers are washed with saturated sodium bicarbonate and brine, dried over anhydrous magnesium sulfate, filtered and concentrated *in vacuo* to afford the title compound as a yellow oil (3.90 g, 83.0%) which is identified by NMR spectral analysis.

### EXAMPLE 117

### Preparation of Ethyl α,α-dimethyl-5-nitro-1,2-benzisothiazole-3-acetate

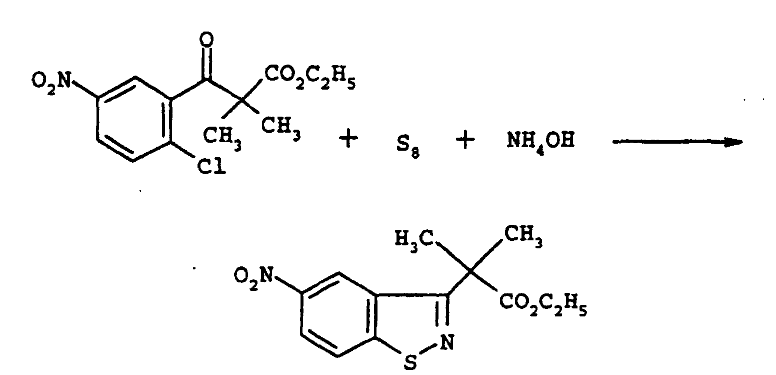

A mixture of 2'-chloro-5'-nitro-2-methyl-2-carboethoxypropiophenone (3.24 g, 0.00108 mol), N,N-dimethylformamide and sulfur (0.350 g, 0.00109 mol) is treated dropwise with ammonium hydroxide (9 ml), heated to and stirred at 70-80 °C for two hours, cooled to room temperature, and diluted with water. Filtration affords the title compound as a solid (2.49 g, 78.3%, mp 75-77 °C) which is identified by NMR spectral analysis.

### EXAMPLE 118

### Preparation of 1-Benzothiophen-2,3-dione

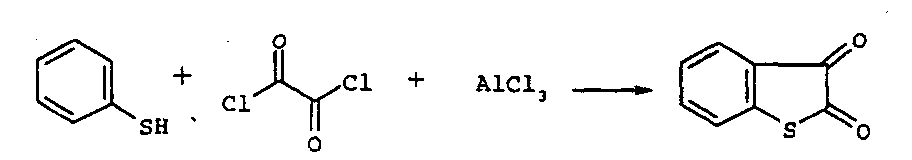

To a solution of thiophenol (100 g, 0.907 mol) in ether is added dropwise a solution of oxalyl chloride (175 g, 1.38 mol) in ether. The mixture is stirred two hours at reflux and concentrated *in vacuo.* The residue is taken up in methylene chloride and cooled to 0 °C. Aluminum chloride (145 g, 1.09 mol) is added in portions such that the temperature does not exceed 25 °C. The resultant mixture is stirred 30 minutes at reflux, cooled to room temperature and poured into ice water with stirring. The organic layer is washed with saturated sodium bicarbonate, water and brine, dried over anhydrous magnesium sulfate, filtered and concentrated *in vacuo* to an orange solid which is recrystallized from methylene chloride:hexanes to afford the title compound (102 g, 69.0%) which is identified by NMR spectral analysis.

### EXAMPLE 119

### Preparation of 1,2-Benzisothiazole-3-carboxamide

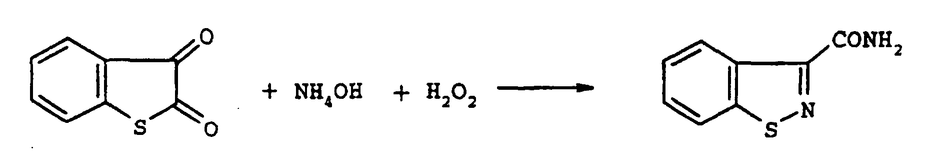

To ammonium hydroxide (1.78 l) is added 1-benzothiophen-2,3-dione (87.0 g, 0.530 mol) at 5-10 °C, followed by hydrogen peroxide (30% aqueous, 178 ml). The resultant mixture is filtered to obtain a yellow solid which is dried (77.0 g, 81.7%) and identified as the title compound by NMR and IR spectral analysis.

### EXAMPLE 120

### Preparation of 3-Cyano-5-nitro-1,2-benzisothiazole

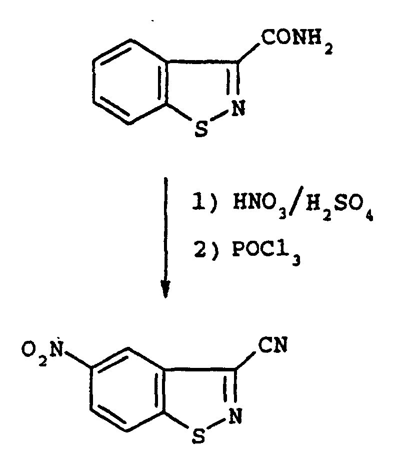

A solution of 1,2-benzisothiazole-3-carboxamide (12.0 g, 0.0674 mol) in concentrated sulfuric acid at 0-5 °C is treated dropwise with nitric acid (90%, 4.12 ml) such that the temperature does not exceed 10 °C, stirred one hour at 5 °C, and poured into ice water with vigorous stirring. The resultant suspension is filtered to obtain a solid. The solid is dried and recrystallized from acetonitrile to afford a white solid (10.0 g) which is treated with phosphorus oxychloride (60.0 ml). The resultant mixture is stirred at 90-100 °C for 90 minutes, cooled to room temperature, slowly poured into ice water with stirring, and filtered to obtain a solid. Recrystallization of the solid from methylene chloride:hexanes gives the title compound as an orange solid (8.00 g, 87.9%, mp 168-170 °C) which is identified by NMR and IR spectral analyses.

### EXAMPLE 121

### Preparation of 2,4-Difluoro-5-nitrobenzoyl chloride

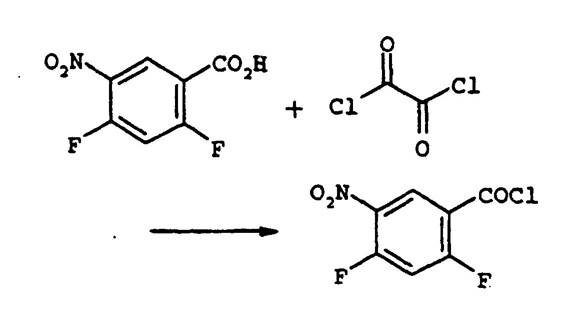

Oxalyl chloride (94.0 g, 0.739 mol) is added dropwise to a mixture of 2,4-difluoro-5-nitrobenzoic acid (100.0 g, 0.492 mol), methylene chloride and N,N-dimethylformamide (0.600 ml). The resultant mixture is stirred 3.25 hours at reflux, cooled to room temperature, and concentrated *in vacuo* to afford the title compound as a brown oil (111 g, 95.2%).

### EXAMPLE 122

### Preparation of 2',4'-Difluoro-2-methoxy-5-methyl-5'-nitrobenzophenone

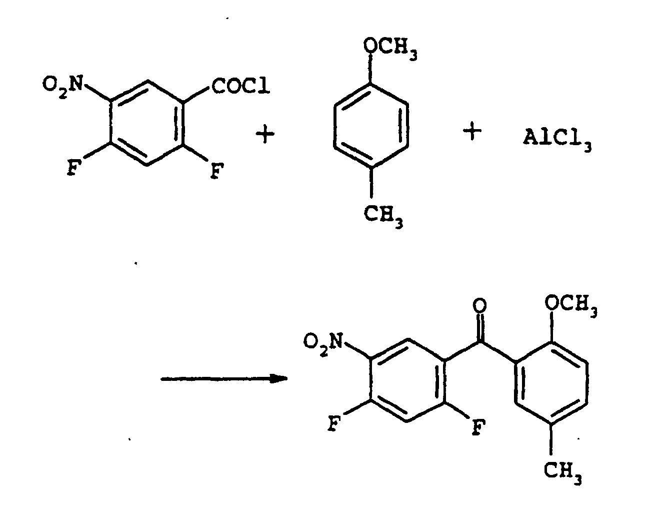

A mixture of aluminum chloride (62.3 g, 0.467 mol) and methylene chloride is cooled to -20 °C to -10 °C, treated with 4-methylanisole (60.1 g, 0.492 mol), treated dropwise with a mixture of 2,4-difluoro-5-nitrobenzoyl chloride (111 g, 0.468 mol) and methylene chloride over a 10 minute period, warmed to 0 °C, stirred overnight at ambient temperature, slowly poured onto ice with stirring, and diluted with methylene chloride. The organic layer is washed with water and brine, dried over anhydrous magnesium sulfate and concentrated *in vacuo* to a solid. The solid is recrystallized from acetonitrile to afford the title compound as a yellow solid (82.1 g, 54.0%) which is identified by NMR spectral analysis.

### EXAMPLE 123

### Preparation of 3-(6-Methoxy-m-tolyl)-6-amino-5-nitro-1,2-benzisothiazole

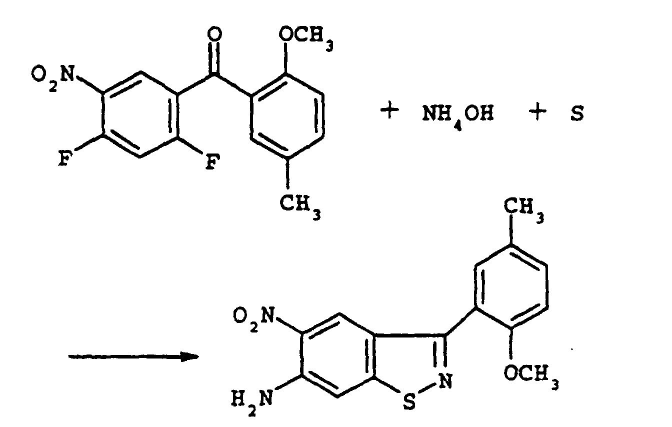

Ammonium hydroxide (330 ml) is added to a suspension of 2',4'-difluoro-2-methoxy-5-methyl-5'-nitrobenzophenone (60.0 g, 0.186 mol), sulfur (6.25 g, 0.195 mol) and N,N-dimethylformamide on an ice bath. The resultant mixture is allowed to warm to 35 °C, heated gradually to 81 °C over a two hour period, cooled to room temperature, and poured into water. The resultant solid is taken up in ethyl acetate and N,N-dimethylformamide, and washed with water. The organic layer is concentrated *in vacuo* to afford the title compound which is identified by NMR spectral analysis.

### EXAMPLE 124

### Preparation of 3-(6-Methoxy-m-tolyl)-6-chloro-5-nitro-1,2-benzisothiazole

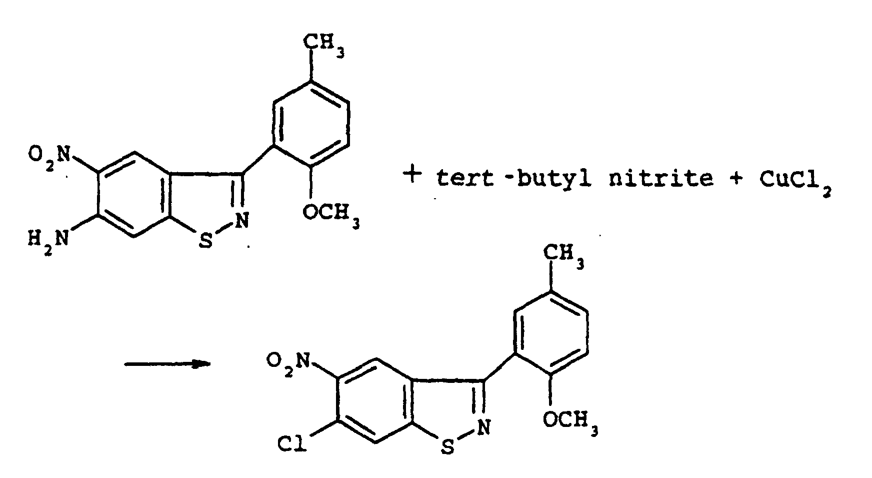

A mixture of *tert*-butyl nitrite (5.90 g, 0.0571 mol), copper chloride (6.20 g, 0.0457 mol) and acetonitrile is heated to 65-75 °C, treated with 3-(6-methoxy-*m*-tolyl)-6-amino-5-nitro-1,2-benzisothiazole (12.0 g, 0.0381 mol) over 10 minutes, stirred for two hours at 67-75 °C, treated with tert-butyl nitrite (1.50 ml) and copper chloride (1.00 g), stirred 40 minutes at 67-75°C, cooled to room temperature, and diluted with ethyl acetate. The organic layer is washed with 10% hydrochloric acid and filtered. The filtrate is washed with water and concentrated *in vacuo* to afford the title compound as a solid (10.6 g, 83.1%) which is identified by NMR and IR spectral analyses.

### EXAMPLE 125

### Preparation of 3-(6-Methoxy-m-tolyl)-6-fluoro-5-nitro-1,2-benzisothiazole

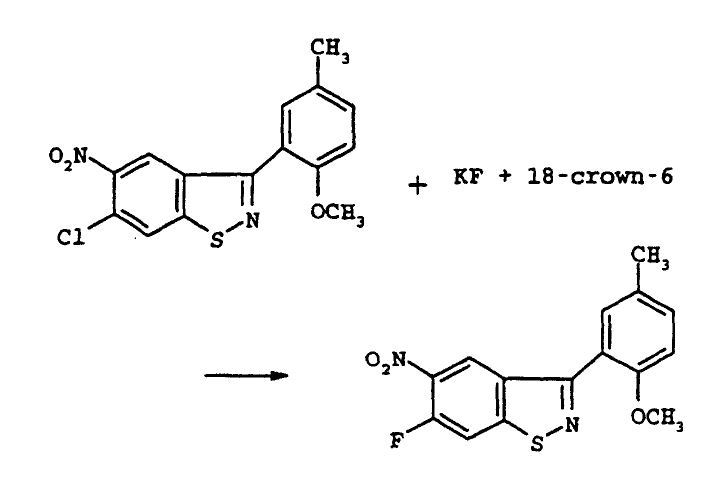

A mixture of 3-(6-methoxy-*m*-tolyl)-6-chloro-5-nitro-1,2-benzisothiazole (7.30 g, 0.0218 mol), potassium fluoride (6.33 g, 0.109 mol) 18-crown-6 (2.31 g, 0.0872 mol) and sulfolane is stirred 19 hours at 154°C, cooled to room temperature, and poured into ice water. The resultant solid is filtered and chromatographed on silica gel with methylene chloride to afford a solid which is recrystallized from acetonitrile to afford a tan powder. The powder is recrystallized from ethyl acetate to give the title compound as a tan solid (2.09 g, 29.9%) which is identified by NMR spectral analysis.

### EXAMPLE 126

### Preparation of 5-Amino-4-bromo-6-fluoro-3-methyl-1,2-benzisothiazole

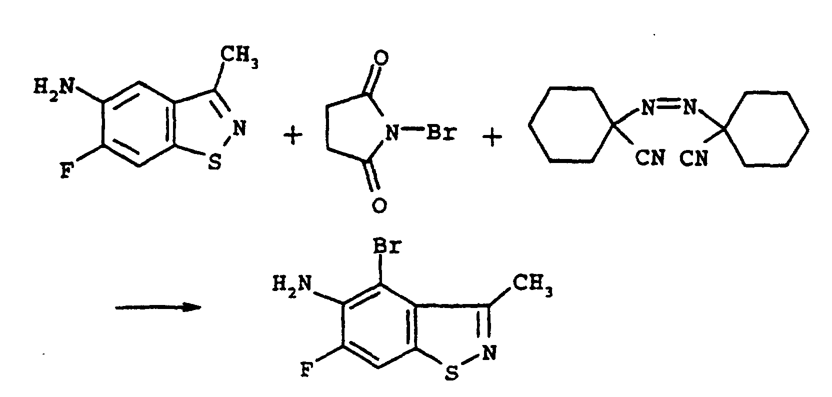

To a solution of 5-amino-6-fluoro-3-methyl-1,2-benzisothiazole (0.600 g, 0.00329 mol) in 1,2-dichloroethane is added N-bromosuccinimide (0.586 g, 0.00329 mol) followed by 1,1'-azobis(cyclohexanecarbonitrile) (0.0200 g). The mixture is stirred two hours at 70 °C, additional N-bromosuccinimide (0.240 g, 0.00135 mol) is added, and the mixture is stirred 40 minutes at 70°C. The mixture is then cooled to room temperature, filtered and concentrated *in vacuo* to obtain a residue. The residue is chromatographed on silica gel to give the title compound (0.870 g, 100%) which is identified by NMR spectral analysis.

### EXAMPLE 127

### Preparation of 5-Chloro-1-methyl-3-(3-methyl-1,2-benzisothiazol-5-yl)-6-(trifluoromethyl)-2,4(1H,3H)-pyrimidinedione

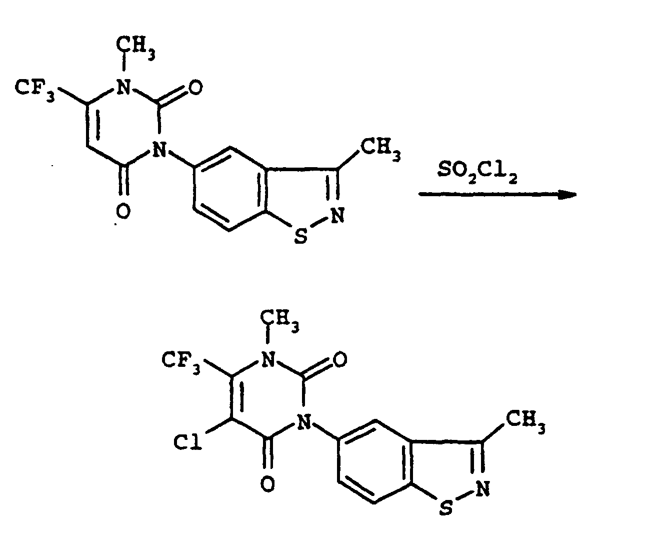

A mixture of 1-methyl-3-(3-methyl-1,2-benzisothiazol-5-yl)-6-(trifluoromethyl)-2,4(1H,3H)-pyrimidinedione (1.00 g, 0.00293 mol), sulfuryl chloride (2.00 g 0.0148 mol) and 1,2-dichloroethane is stirred for five hours at reflux, cooled to room temperature, and diluted with methylene chloride. The resultant mixture is washed with saturated sodium bicarbonate and brine, dried over anhydrous sodium sulfate, filtered and concentrated *in vacuo* to obtain a residue. The residue is chromatographed on silica gel with ethyl acetate/hexanes to afford the title product as a white solid (0.350 g, 31.8%, mp 252-254 °C) which is identified by NMR spectral analysis.

### EXAMPLE 128

### Preparation of 3-[3-(1-Bromoethyl)-1,2-benzisothiazol-5-yl]-1-methyl-6-(trifluoromethyl)-2,4(1H,3H)-pyrimidinedione and 3-[3-(1,1-dibromoethyl)-1,2-benzisothiazol-5-yl]-1-methyl-6-(trifluoromethyl)-2,4(1H,3H)-pyrimidinedione

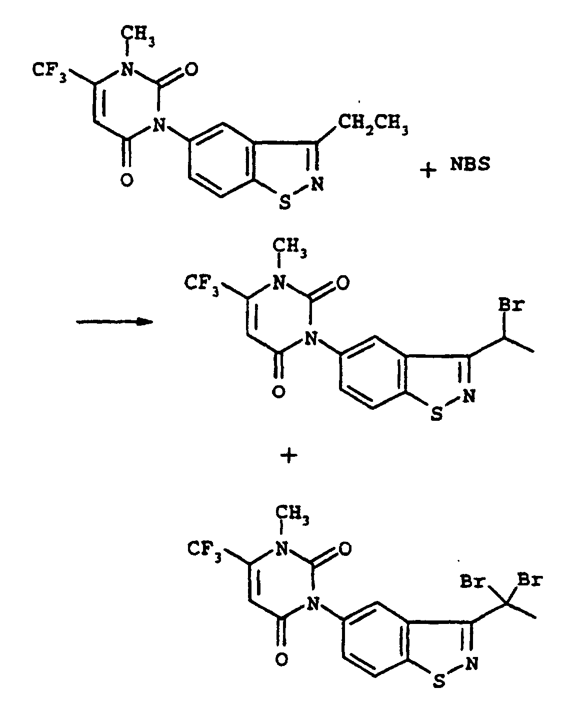

A mixture of 1-methyl-3-(3-methyl-1,2-benzisothiazol-5-yl)-6-(trifluoromethyl)-2,4(1H,3H)-pyrimidinedione (1.44 g, 0.00405 mol), N-bromosuccinimide (0.790 g, 0.00446 mol) and 1,2-dichloroethane is stirred under a 150 watt lamp for one hour at 60°C, cooled to room temperature, and concentrated *in vacuo* to a yellow solid. The yellow solid is flash chromatographed on silica gel with 80% to 70% hexanes:ethyl acetate to afford the 1,1-dibromoethyl benzisothiazole as a white foam (0.0600 g, 2.88%) and the 1-bromoethyl benzisothiazole as white crystals (1.73 g, 98.9%, mp 163 °C). Both products are identified by NMR spectral analyses.

In a similar manner, treatment of the above substrate with N-chlorosuccinimide affords 3-[3-(1-chloroethyl)-1,2-benzisothiazol-5-yl]-1-methyl-6-(trifluoromethyl)-2,4(1H,3H)-pyrimidinedione as a white solid, mp 169-171 °C.

### EXAMPLE 129

### Preparation of 3-[3-(Bromochloromethyl)-1,2-benzisothiazol-5-yl]-1-methyl-6-(trifluoromethyl)-2,4(1H,3H)-pyrimidinedione

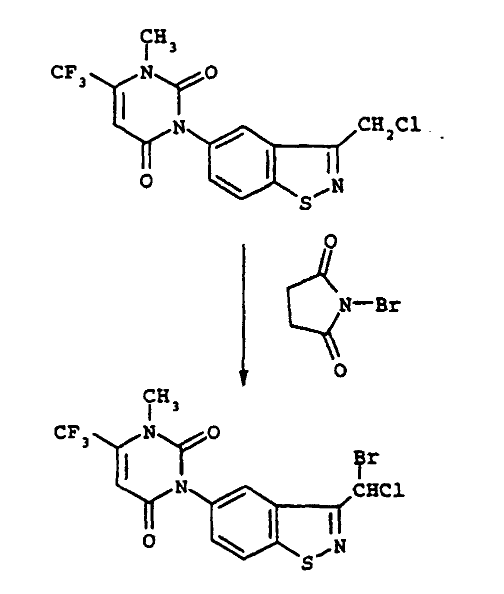

A mixture of 3-[3-(chloromethyl)-1,2-benzisothiazol-5-yl]-1-methyl-6-(trifluoromethyl)-2,4(1H,3H)-pyrimidinedione (1.00 g, 0.00266 mol), azo-bis-isobutyronitrile (0.100 g), N-bromosuccinimide (0.712 g, 0.00400 mol) and 1,2-dichloroethane is stirred one hour at reflux. Additional azo-bis-isobutyronitrile (0.0500 g) and N-bromosuccinimide (0.100 g) are added and the mixture is stirred two hours at reflux, cooled to room temperature, diluted with methylene chloride, washed with water, dried over anhydrous magnesium sulfate, and concentrated *in vacuo* to an orange gum. Chromatography of the gum on silica gel with methylene chloride:ether gives the title compound as a white foam (0.950 g, 80.2%, mp 93-96 °C) which is identified by NMR spectral analysis.

### EXAMPLE 130

### Preparation of Methyl 5-[3,6-dihydro-3-methyl-2,6-dioxo-4-(trifluoromethyl)-1(2H)-pyrimidinyl]-α-bromo-1,2-benzisothiazole-3-acetate

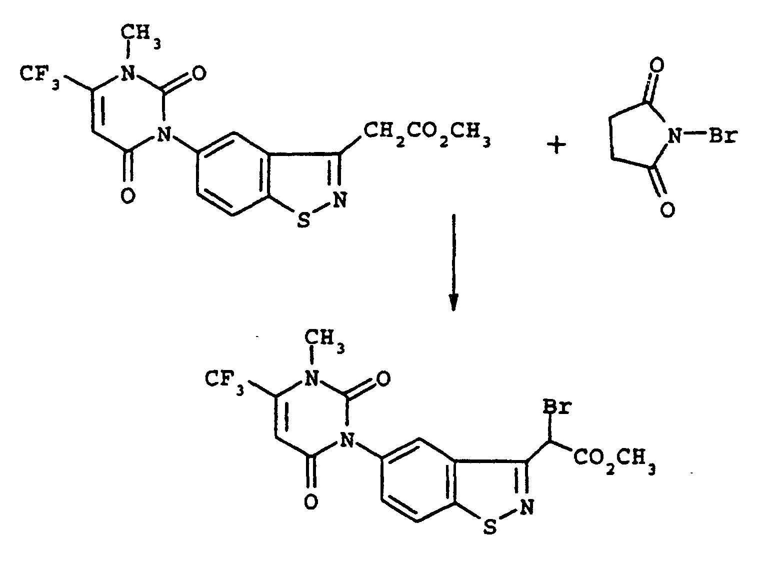

A mixture of methyl 5-[3,6-dihydro-3-methyl-2,6-dioxo-4-(trifluoromethyl)-1(2H)-pyrimidinyl]-1,2-benzisothiazole-3-acetate (0.400 g, 0.00100 mol), N-bromosuccinimide (0.370 g, 0.00210 mol), benzoyl peroxide (0.0100 g) and tetrachloroethane is stirred 2.5 hours at reflux, cooled to room temperature, and chromatographed on silica gel with methylene chloride to afford the title compound as an off-white solid (0.430 g, 90.0%, mp 84-87 °C) which is identified by NMR spectral analysis.

### EXAMPLE 131

### Preparation of 3-[3-(2-Bromoethyl)-1,2-benzisothiazol-5-yl]-1-methyl-6-(trifluoromethyl)-2,4(1H,3H)-pyrimidinedione

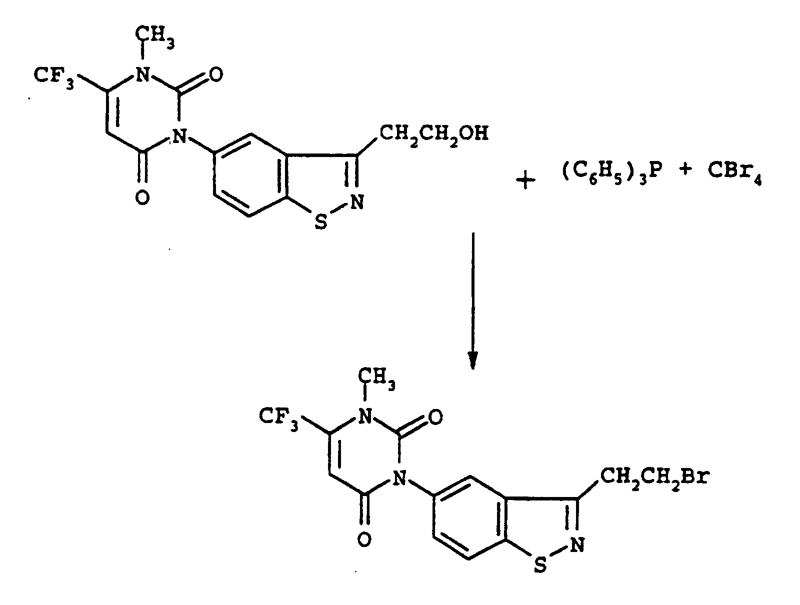

To a solution of 3-[3-(2;hydroxyethyl)-1,2-benzisothiazol-5-yl]-1-methyl-6-(trifluoromethyl)-2,4(1H,3H)-pyrimidinedione (0.690 g, 0.00185 mol) in methylene chloride is added carbon tetrabromide (0.655 g, 0.00197 mol) followed by triphenylphosphine (0.517 g, 0.00197 mol). The mixture is stirred three hours at ambient temperature and treated with additional carbon tetrabromide (0.328 g, 0.000988 mol) and triphenyl phosphine (0.259 g, 0.000987 mol). The resultant mixture is stirred 85 minutes, diluted with methanol, and concentrated *in vacuo*. The resultant solid is taken up in methylene chloride, washed with saturated sodium bicarbonate and water, dried over anhydrous sodium sulfate, filtered, and concentrated *in vacuo* to a yellow solid. The yellow solid is chromatographed on silica gel with methylene chloride to give the title product as an off-white solid (0.530 g, 66.0%) which is identified by NMR and elemental analyses.

### EXAMPLE 132

### Preparation of 3-[3-(2,2-Dibromovinyl)-1,2-benzisothiazol-5-yl]-1-methyl-6-(trifluoromethyl)-2,4(1H,3H)-pyrimidinedione

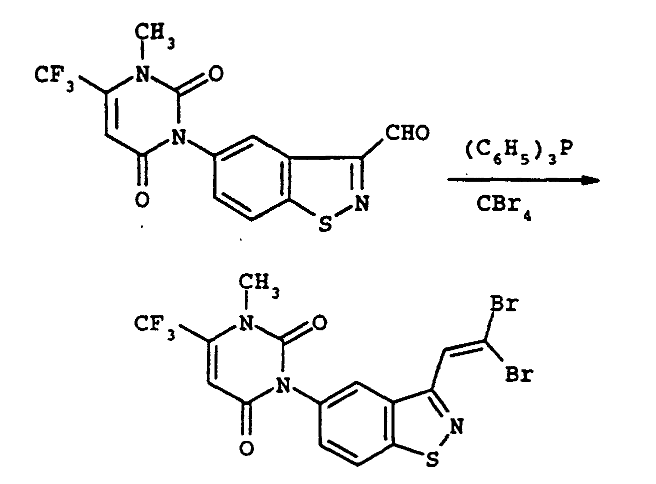

Triphenylphosphine (0.354 g, 0.00138 mol) is added to a solution of carbon tetrabromide (0.223 g, 0.000676 mol) in methylene chloride. The resultant mixture is stirred at room temperature for 10 minutes and treated dropwise with a solution of 5-[3,6-dihydro-3-methyl-2,6-dioxo-4-(trifluoromethyl)-1(2H)-pyrimidinyl]-1,2-benzisothiazole-3-carboxaldehyde (0.200 g, 0.000563 mol) in methylene chloride. The resultant mixture is stirred 20 minutes and concentrated *in vacuo*. The resultant residue is purified by flash column chromatography using silica gel and 10:1 methylene chloride/ethyl acetate to give the title product as a white solid (0.240 g, 83.6%, mp 260-262 °C) which is identified by NMR and IR spectral analyses.

Using essentially the same procedure, the following compounds are obtained:

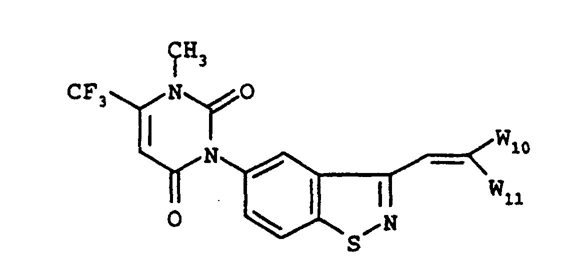

| **W**_{**10**} | **W**_{**11**} | **mp** °**C** |
|---|---|---|
| Br | F | |
| Cl | Cl | 254-256 |

### EXAMPLE 133

### Preparation of (1R,2S)-3-[3-(1,2-Dibromopropyl)-1,2-benzisothiazol-5-yl]-1-methyl-6-(trifluoromethyl)-2,4-(1H,3H)-pyrimidinedione and (1R,2R)-3-[3-(1,2-Dibromopropyl)-1,2-benzisothiazol-5-yl]-1-methyl-6-(trifluoromethyl)-2,4(1H,3H)-pyrimidinedione

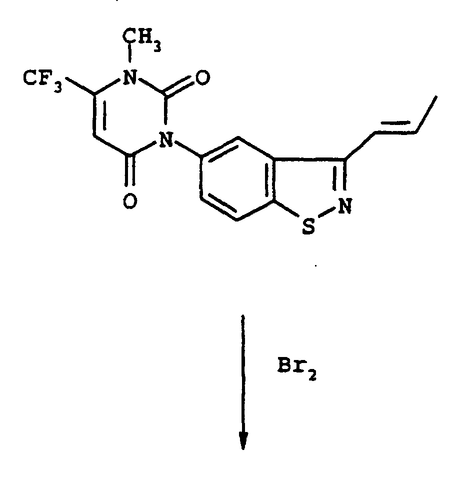

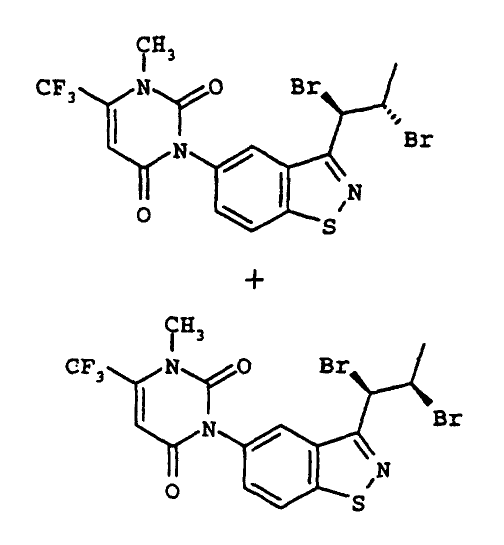

To a mixture of 1-methyl-(3-propenyl-1,2-benzisothiazol-5-yl)-6-(trifluoromethyl)-2,4(1H,3H)-pyrimidinedione (0.500 g, 0.00136 mol) and carbon tetrachloride is added bromine (0.220 g, 0.00138 mol). The resultant mixture is stirred 2.5 hours at room temperature and concentrated *in vacuo* to a yellow foam, which is chromatographed on silica gel with methylene chloride:ether. The (1R,2S)-isomer elutes first and is isolated as an off-white solid (0.310 g 41.9%, mp 214 °C) which is identified by NMR spectral analysis. The (1R,2R)-isomer elutes second and is isolated as an off-white foam (0.290 g 39.2%, mp 188-189 °C) which is identified by NMR spectral analysis.

### EXAMPLE 134

### Preparation of 3-[3-(2(and 1)-Bromo-1(and 2)-hydroxypropyl)-1,2-benzisothiazol-5-yl]-1-methyl-6-(trifluoromethyl)-2,4(1H,3H)-pyrimidinedione, (4:1) mixture of diastereomers

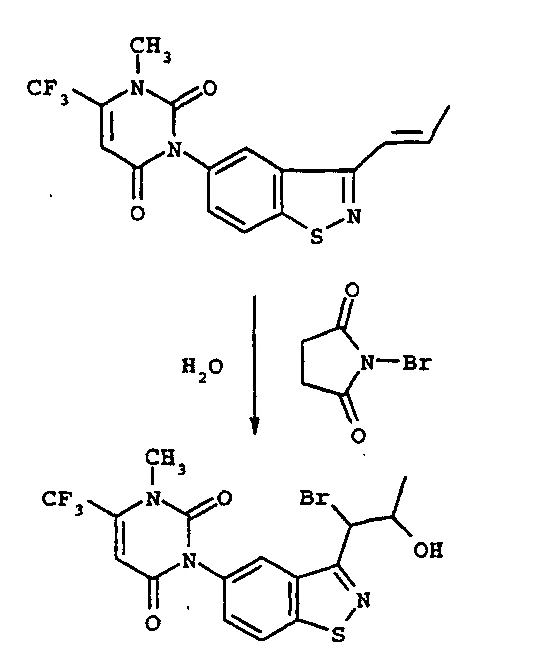

To a mixture of 1-methyl-(3-propenyl-1,2-benzisothiazol-5-yl)-6-(trifluoromethyl)-2,4(1H,3H)-pyrimidinedione (1.00 g, 0.00272 mol), dioxane and water is added N-bromosuccinimide (0.570 g, 0.00321 mol). The resultant mixture is stirred 23 hours at room temperature, poured into water and extracted with ether. The organic layers are washed with water, dried over anhydrous magnesium sulfate, filtered, and concentrated *in vacuo* to a yellow foam. The foam is chromatographed on silica gel with methylene chloride:ether to afford the title mixture of isomers as a white foam (1.03 g, 82.4%, mp 100-103 °C) which is identified by NMR spectral analysis.

### EXAMPLE 135

### Preparation of Methyl 5-[3,6-dihydro-5-bromo-3-methyl-2,6-dioxo-4-(trifluoromethyl)-1(2H)-pyrimidinyl]-α,α-dibromo-1,2-benzisothiazole-3-acetate and Methyl-5-[3,6-dihydro-3-methyl-2,6-dioxo-4-(trifluoromethyl)-1(2H)-pyrimidinyl]-α,α-dibromo-1,2-benzisothiazole-3-acetate

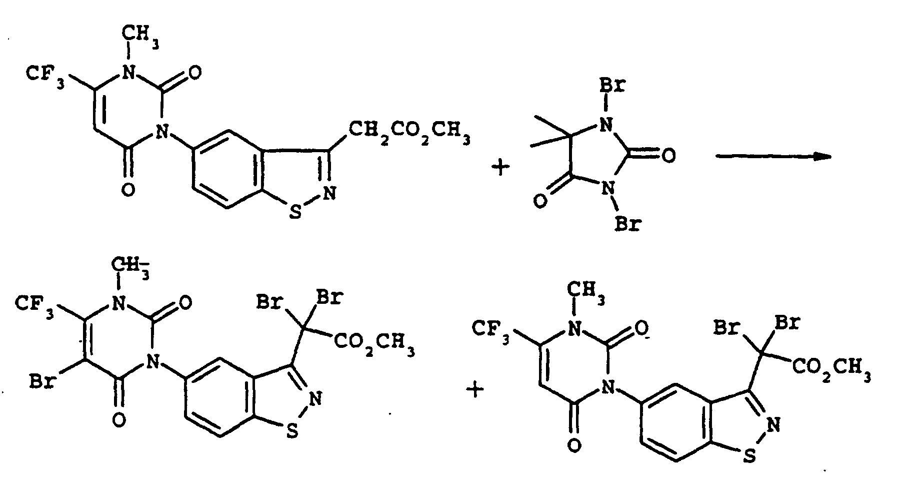

A mixture of methyl 5-[3,6-dihydro-5-bromo-3-methyl-2,6-dioxo-4-(trifluoromethyl)-1(2H)-pyrimidinyl]-1,2-benzisothiazole-3-acetate (1.20 g, 0.00300 mol), dimethyl-N/N-dibromohydantoin (4.20 g, 0.0150 mol), and 1,2-dichloroethane is stirred 3 days at reflux, cooled to room temperature, diluted with methylene chloride, washed with 10% sodium thiosulfate, water and brine, dried over anhydrous magnesium sulfate, filtered, and concentrated *in vacuo*. The resultant residue is chromatographed on silica gel with methylene chloride. The bromouracil substituted α,α-dibromo-1,2-benzisothiazole-3-acetate elutes first and is isolated as a white solid (0.400 g, 30.0%, mp 241 °C) which is identified by NMR spectral analysis. The α,α-dibromo-1,2-benzisothiazole-3-acetate elutes second and is isolated as a white solid (0.520 g, 31.1%, mp 194-196 °C) which is identified by NMR spectral analysis.

### EXAMPLE 136

### Preparation of α-Bromo-5-[3,6-dihydro-3-methyl-2,6-dioxo-4-(trifluoromethyl)-1(2H)-pyrimidyl]-1,2-benzisothiazole-3-acetonitrile

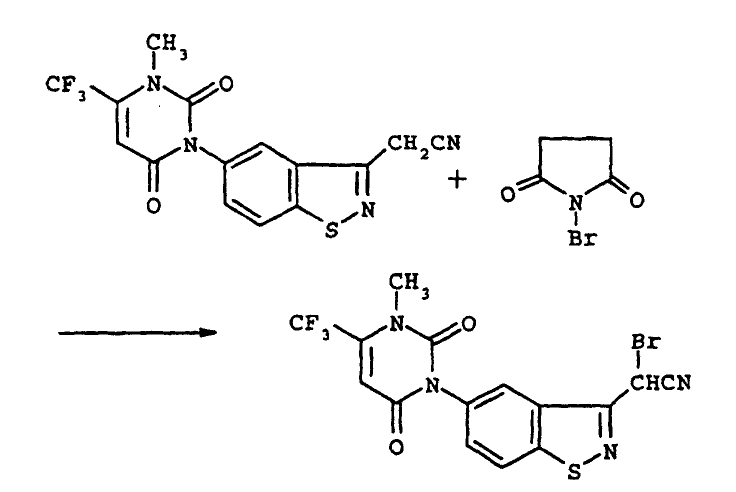

To a solution of 5-[3,6-dihydro-3-methyl-2,6-dioxo-4-(trifluoromethyl)-1(2H)-pyrimidinyl]-1,2-benzisothiazole-3-acetonitrile (0.500 g, 0.00140 mol) in 1,2-dichloroethane is added N-bromosuccinimide (0.250 g, 0.00140 mol). The resultant mixture is irradiated with a UV lamp and stirred at reflux six hours. Additional N-bromosuccinimide (0.250 g, 0.00140 mol) is added and the resultant mixture is stirred 1.5 hours at reflux under a UV lamp. The mixture is cooled to room temperature and concentrated *in vacuo.* The resultant residue is taken up in methylene chloride, washed with water, treated with charcoal, dried over anhydrous magnesium sulfate, filtered, and concentrated *in vacuo* to give the title product as a red glass (0.350 g, 56.2%, mp 185-187 °C) which is identified by IR and NMR spectral analyses.

### EXAMPLE 137

### Preparation of {5-[3,6-Dihydro-3-methyl-2,6-dioxo-4-(trifluoromethyl)-1(2H)-pyrimidinyl]-1,2-benzisothiazol-3-yl}-iso-thiocyanic acid, methyl ester

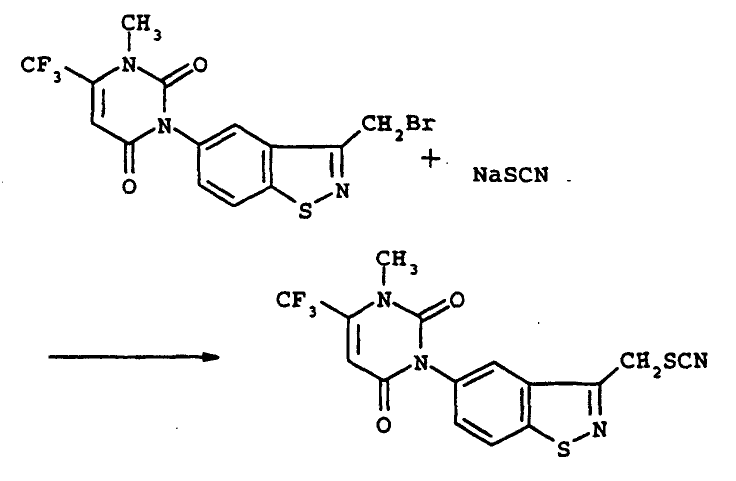

To a solution of potassium thiocyanate (0.300 g, 0.00309 mol) in dimethyl sulfoxide is added 3-[3-(bromomethyl)-1,2-benzisothiazol-5-yl]-1-methyl-6-(trifluoromethyl)-2,4(1H,3H)-pyrimidinedione (0.500 g, 0.00119 mol). The resultant mixture is stirred two hours at room temperature, diluted with ethyl acetate, washed with water, dried over anhydrous magnesium sulfate, filtered, and concentrated *in vacuo* to give the title compound as a white solid (0.475 g, 100%, mp 239-241 °C) which is identified by NMR and IR spectral analyses.

### EXAMPLE 138

### Preparation of 3-[3-Hydroxymethyl)-1,2-benzisothiazol-5-yl]-1-methyl-6-(trifluoromethyl)-2,4(1H,3H)-pyrimidinedione, nitrate (ester)

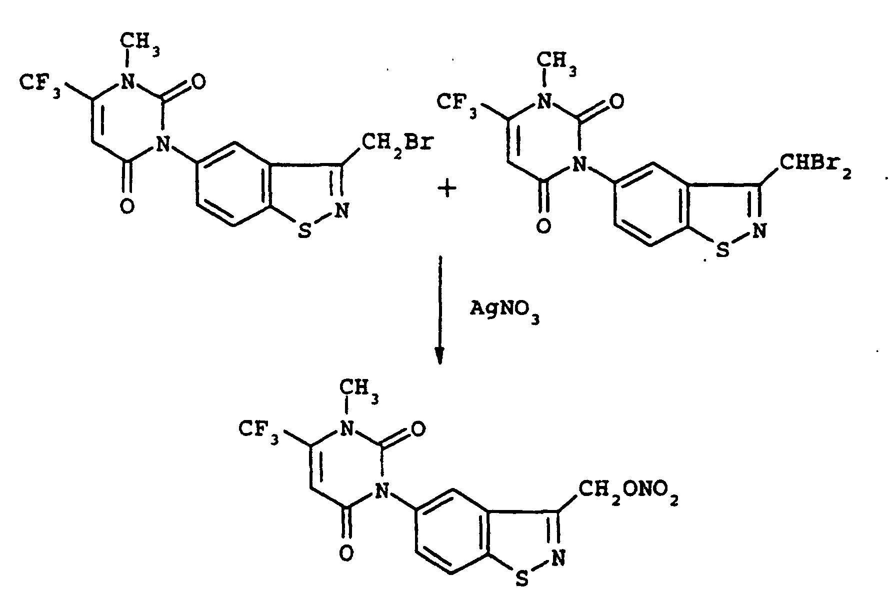

A mixture of 3-[3-(dibromo- (33%) and -bromomethyl (67%))-1,2-benzisothiazol-5-yl]-1-methyl-6-(trifluoromethyl)-2,4(1H,3H)-pyrimidinedione (2.00 g, 0.00434 mol), silver nitrate (1.50 g, 0.00887 mol), dioxane and water is stirred one hour at reflux, cooled to room temperature, and filtered. The filtrate is concentrated *in vacuo*. The residue is partitioned between methylene chloride and water. The organic layer is washed with brine, dried over anhydrous magnesium sulfate, filtered, and concentrated *in vacuo.* The residue is chromatographed on silica gel with methylene chloride: methanol to give the title compound as a white solid (0.450 g, 25.9%, mp 188-190 °C) which is identified by NMR spectral analysis.

### EXAMPLE 139

### Preparation of 3-[3-(1-Methoxyethyl)-1,2-benzisothiazol-5-yl]-1-methyl-6-(trifluoromethyl)-2,4(1H,3H)-pyrimidinedione

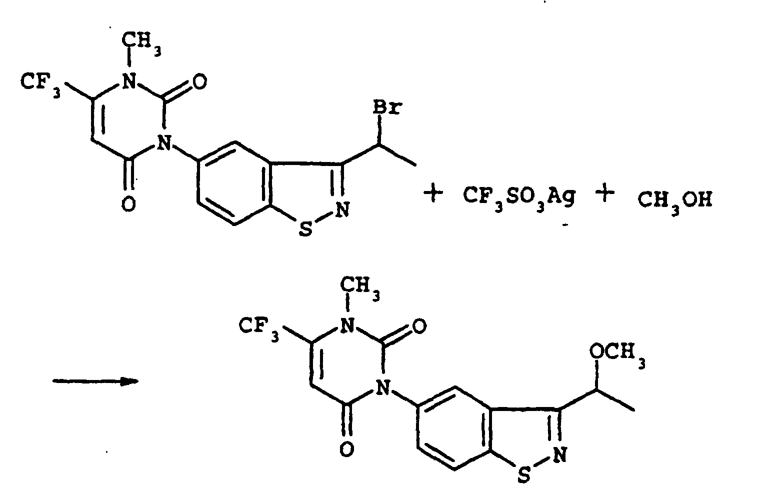

To a mixture of 3-[3-(1-bromoethyl)-1,2-benzisothiazol-5-yl]-1-methyl-6-(trifluoromethyl)-2,4(1H,3H)-pyrimidinedione (0.720 g, 0.00166 mol), methanol and 1,2-dichloroethane is added silver trifluoromethane sulfonate (0.510 g, 0.00199 mol). The resultant mixture is stirred 23 hours at room temperature and filtered through diatomaceous earth with ethyl acetate. Saturated sodium bicarbonate is added to the filtrate and the resultant mixture is stirred vigorously for one hour. The organic layer is saved while the aqueous layer is extracted with ethyl acetate. The combined organic layers are dried over magnesium sulfate, filtered and concentrated *in vacuo* to a syrup, which is chromatographed on silica gel with hexanes:ethyl acetate to give the title compound as a white solid (0.660 g, 100%, mp 133-135 °C) which is identified by NMR spectral analysis.

Using essentially the same procedure, but substituting the appropriate alcohol for methanol, the following compound is prepared:

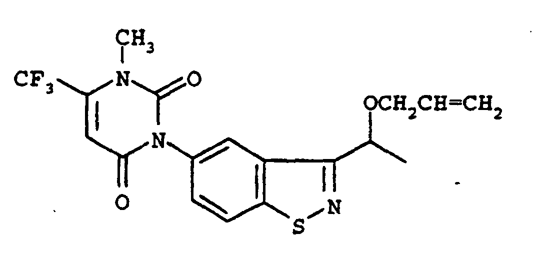

### EXAMPLE 140

### Preparation of Methyl 5-[3,6-dihydro-3-methyl-2,6-dioxo-4-(trifluoromethyl)-1(2H)-pyrimidinyl]-α-methoxy-1,2-benzisothiazole-3-acetate

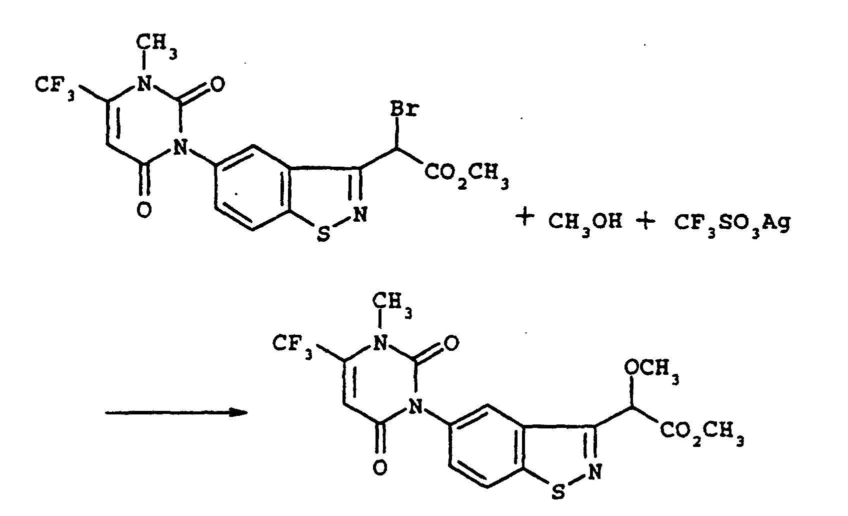

A mixture of methyl 5-[3,6-dioxo-3-methyl-2,6-dioxo-4-(trifluoromethyl)-1(2H)-pyrimidinyl]-α-bromo-1,2-benzisothiazole-3-acetate (0.600 g, 0.00125 mol), silver trifluoromethane sulfonate (0.400 g, 0.00156 mol) and methanol is stirred 36 hours at reflux, cooled and concentrated *in vacuo.* The residue is partitioned between methylene chloride and saturated sodium bicarbonate. The organic layer is saved and the aqueous layer extracted with methylene chloride. The combined organic layers are washed with brine, dried over anhydrous magnesium sulfate, filtered, and concentrated *in vacuo*. The residue is chromatographed on silica gel with methylene chloride to give the title compound as a white solid (0.220 g, 41.0%, mp 166-168 °C) which is identified by NMR spectral analysis.

Using essentially the same procedure, methyl 5-[3,6-dihydro-3-methyl-2,6-dioxo-4-(trifluoromethyl)-1(2H)-pyrimidinyl]-α,α-dimethoxy-1,2-benzisothiazole-3-acetate is obtained from methyl 5-[3,6-dihydro-3-methyl-2,6-dioxo-4-(trifluoromethyl)-1(2H)-pyrimidinyl]-α,α-dibromo-1,2-benzisothiazole-3-acetate.

### EXAMPLE 141

### Preparation of 5-[3,6-Dihydro-3-methyl-2,6-dioxo-4-(trifluoromethyl)-1(2H)-pyrimidinyl]-6-fluoro-1,2-benzisothiazole-3-carboxaldehyde, 3-(dimethylacetal)

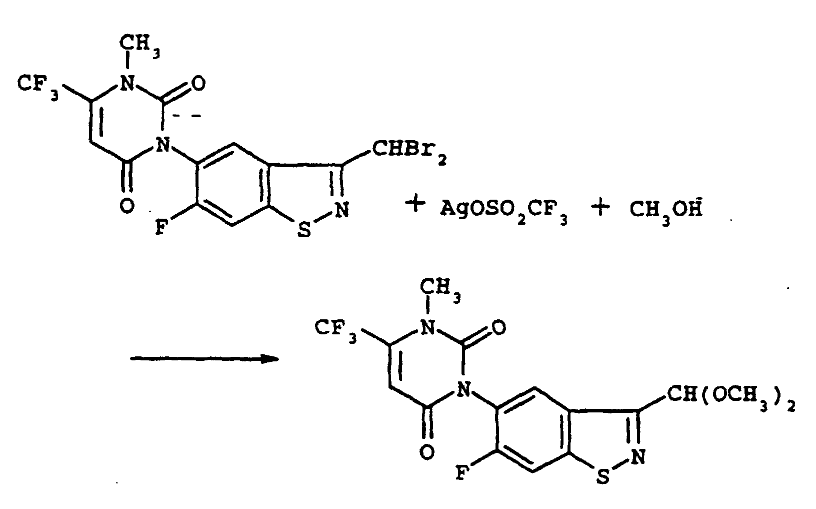

To a mixture of 3-[6-fluoro-3-(dibromomethyl)-1,2-benzisothiazol-5-yl]-1-methyl-6-(trifluoromethyl)-2,4(1H,3H)-pyrimidinedione (0.500 g, 0.000967 mol), 1,2-dichloroethane and methanol (1.00 ml) is added silver trifluoromethyl sulfonate (0.620 g, 0.00241 mol). The resultant mixture is stirred overnight at room temperature with the exclusion of light. The mixture is filtered with methylene chloride wash. The filtrate is washed with saturated sodium bicarbonate, water and brine, dried over anhydrous magnesium sulfate, filtered, and concentrated *in vacuo* to an oil. The oil is chromatographed on silica gel with ethyl acetate:hexanes to give the title compound as a white solid (0.330 g, 81.4%, mp 63-65 °C) which is identified by NMR spectral analysis.

Using essentially the same procedure, the following compound is obtained:

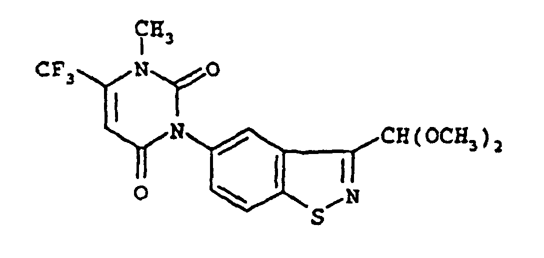

### EXAMPLE 142

### Preparation of {{5-[3,6-Dihydro-3-methyl-2,6-dioxo-4-(trifluoromethyl)-1(2H)-pyrimidinyl]-1,2-benzisothiazol-3-yl}methyl}(methylsulfonyl)carbamic acid, tert-butyl ester

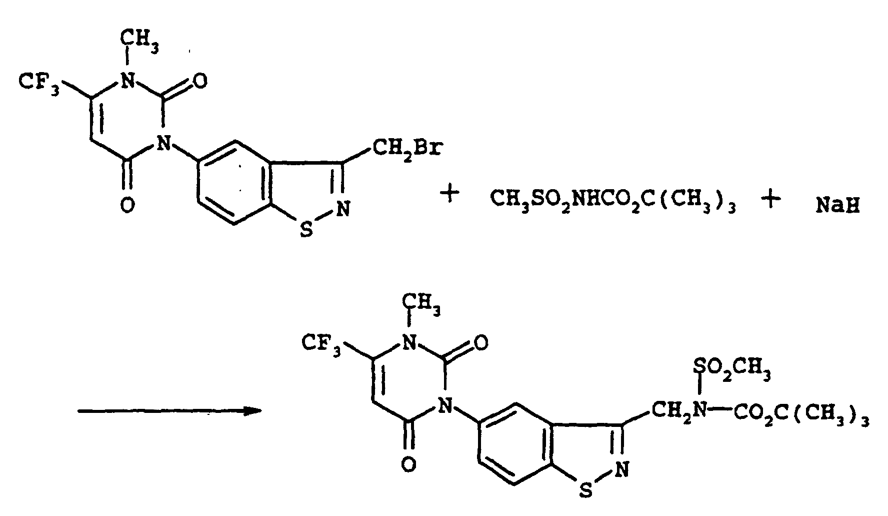

*tert*-Butyl(methylsulfonyl)carbamate (1.52 g, 0.00780 mol) is added to a mixture of sodium hydride (60%, 0.312 g, 0.00780 mol) and tetrahydrofuran at 0°C. The mixture is allowed to come to room temperature. A solution of 3-[3-(bromomethyl)-1,2-benzisothiazol-5-yl]-1-methyl-6-(trifluoromethyl)-2,4(1H,3H)-pyrimidinedione (3.00 g, 0.00714 mol) in dimethyl sulfoxide is added dropwise. The resultant mixture is stirred overnight at room temperature, poured into dilute hydrochloric acid, and extracted with methylene chloride. The combined organic layers are washed with water, dried over anhydrous magnesium sulfate, filtered, and concentrated *in vacuo.* The resultant residue is chromatographed on silica gel with ether:methylene chloride to give the title compound as a white solid (3.00 g, 78.7%, mp 109-111 °C) which is identified by NMR spectral analysis.

### EXAMPLE 143

### Preparation of 3-[3-(Hydroxymethyl)-1,2-benzisothiazol-5-yl]-1-methyl-6-(trifluoromethyl)-2,4(1H,3H)-pyrimidinedione, benzoate (ester)

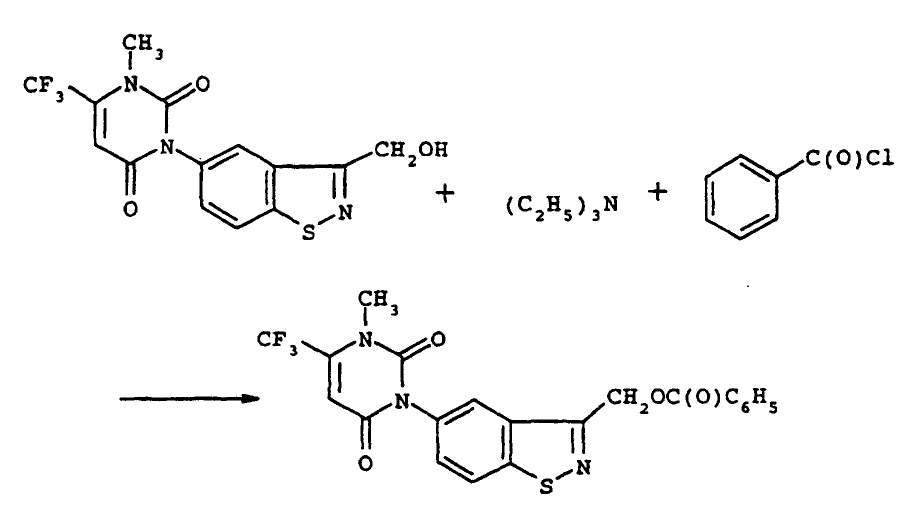

To a mixture of 3-[3-(hydroxymethyl)-1,2-benzisothiazol-5-yl]-1-methyl-6-(trifluoromethyl)-2,4(1H,3H)-pyrimidinedione (0.650 g, 0.00182 mol) and methylene chloride at 0°C is added triethylamine (0.330 ml, 0.00236 mol) followed by benzoyl chloride (0.210 ml, 0.00182 mol). The mixture is stirred one hour at 0°C and quenched with saturated sodium bicarbonate with stirring over a 15 minute period. The resultant mixture is diluted with ether and the organic layer is washed with saturated sodium bicarbonate and brine, dried over anhydrous magnesium sulfate, filtered, and concentrated *in vacuo* to an orange syrup. The syrup is chromatographed on silica gel with hexanes:ethyl acetate to afford a solid. The solid is recrystallized from ethyl acetate and hexanes to give the title compound as a white solid (0.340 g, 40.5%, mp 140-142 °C).

Following essentially the same procedure, but using the appropriate acid chloride and hydroxyalkylbenzisothiazole, the following compounds are obtained:

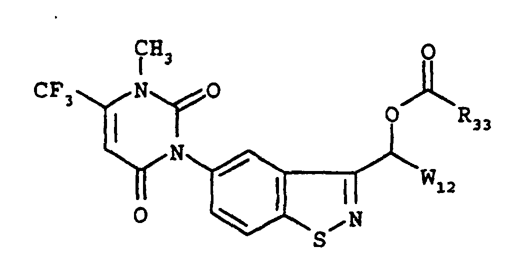

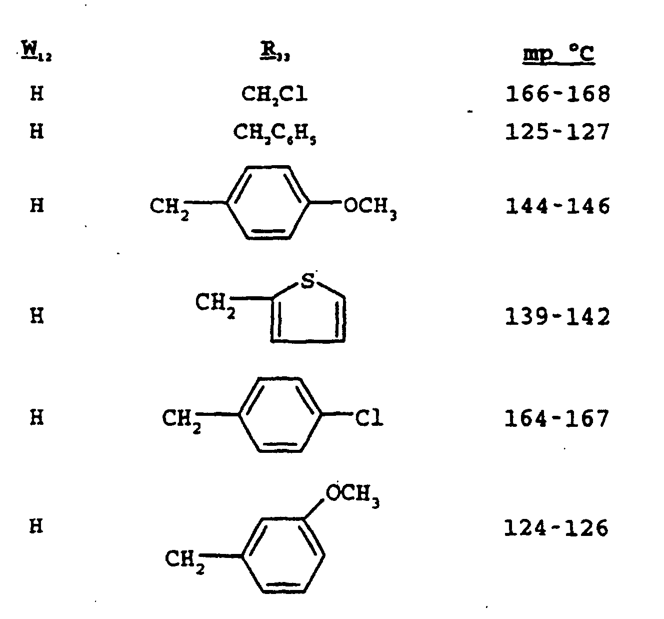

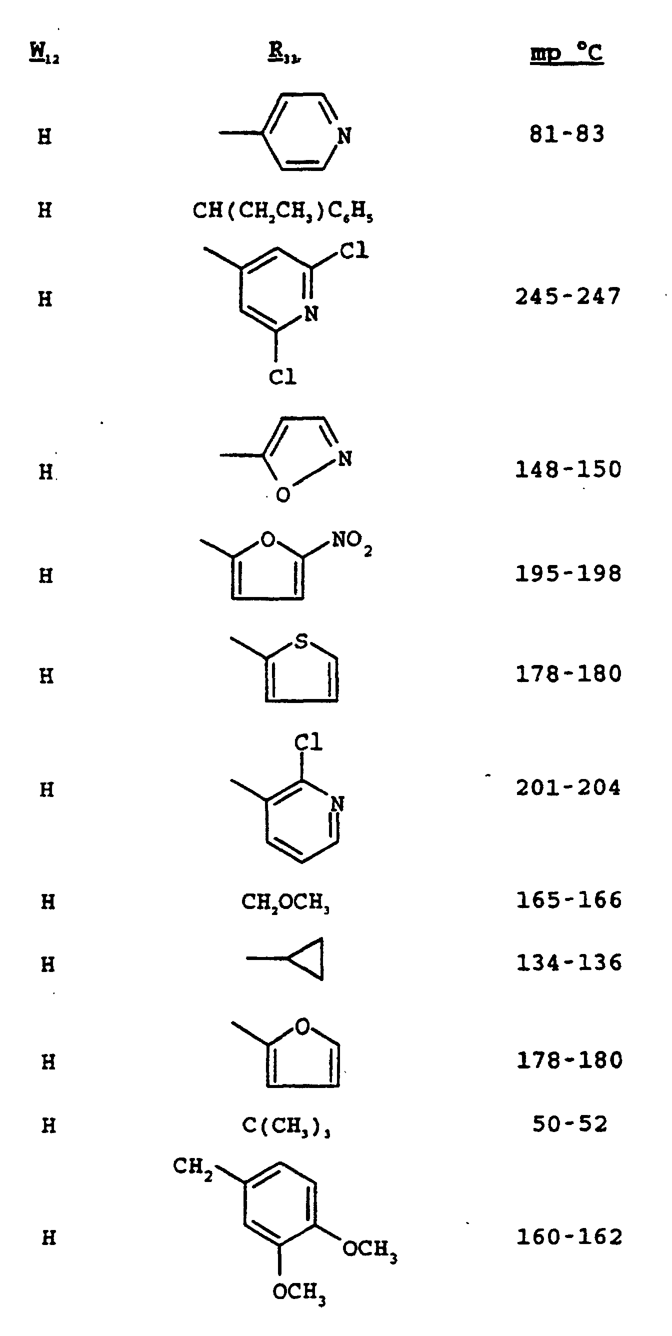

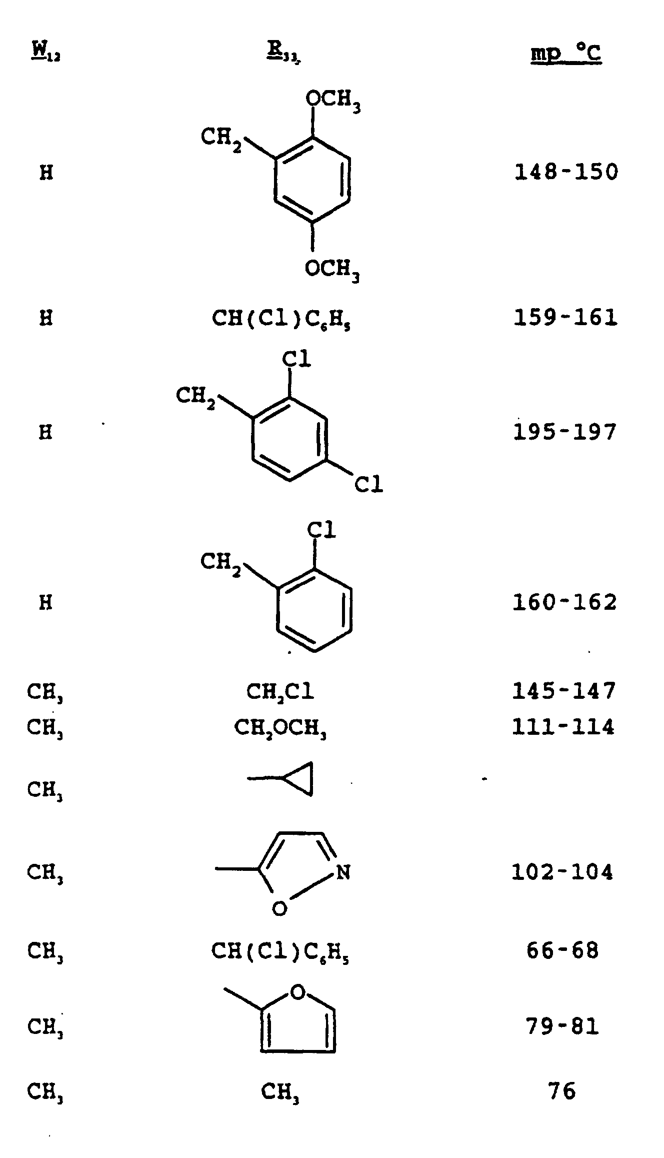

### EXAMPLE 144

### Preparation of 3-[3-(1-Hydroxyethyl)-1,2-benzisothiazol-5-yl]-1-methyl-6-(trifluoromethyl)-2,4(1H,3H)-pyrimidinedione

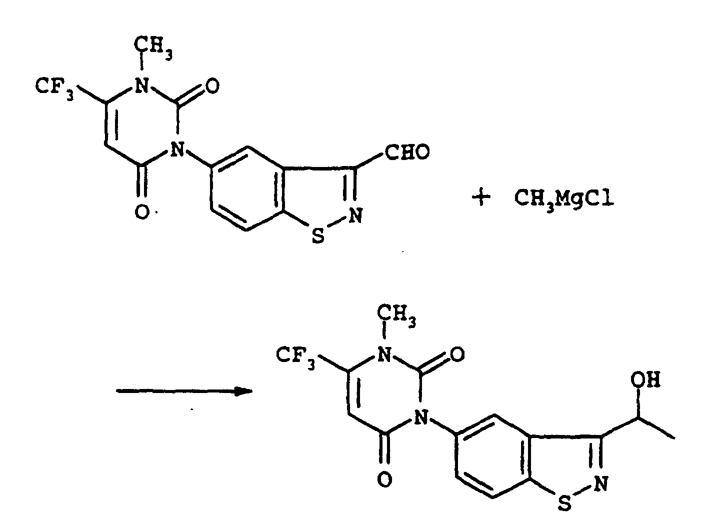

Methyl magnesium chloride (14.1 ml, 0.0422 mol) is added dropwise to a solution of 5-[3,6-dihydro-3-methyl-2,6-dioxo-4-(trifluoromethyl)-1(2H)-pyrimidinyl]-1,2-benzisothiazole-3-carboxaldehyde (6.00 g, 0.00170 mol) in tetrahydrofuran at -78 °C over 15 minutes. The resultant mixture is stirred at -78 °C for 30 minutes, quenched with saturated ammonium chloride and allowed to warm to room temperature. After dilution with ethyl acetate, the mixture is washed with saturated ammonium chloride, water and brine, dried over anhydrous magnesium sulfate, filtered, and concentrated *in vacuo* to a solid. The solid is chromatographed on silica gel with hexanes/ethyl acetate to give the title compound as a white crystalline solid (4.49 g, 71.1%) which is identified by NMR spectral analysis.

### EXAMPLE 145

### Preparation of 3-(3-Acetyl-1,2-benzisothiazol-5-yl)-1-methyl-6-(trifluoromethyl)-2,4(1H,3H)-pyrimidinedione

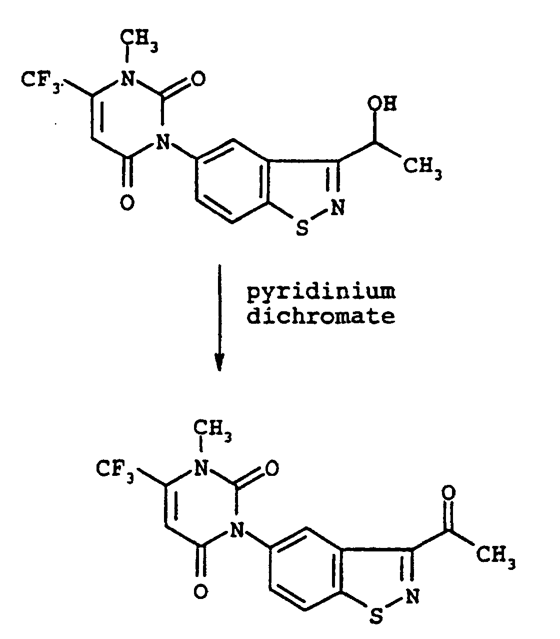

To a solution of 3-[3-(1-hydroxyethyl)-1,2-benzisothiazol-5-yl]-1-methyl-6-(trifluoromethyl)-2,4(1H,3H)-pyrimidinedione (3.99 g, 0.0107 mol) in dry methylene chloride under a nitrogen atmosphere is added powdered 4Å molecular sieves followed by pyridinium dichromate (4.85 g, 0.0129 mol). The resultant mixture is stirred 22 hours at room temperature, diluted with ether, filtered through silica gel and diatomaceous earth, and concentrated *in vacuo* to a solid. The solid is recrystallized from ethyl acetate:hexanes to afford the title compound as a white crystalline solid (2.34 g, 59.2%, mp 149 °C) which is identified by NMR spectral analysis.

### EXAMPLE 146

### Preparation of 3-[3-(Hydroxymethyl)-1,2-benzisothiazol-5-yl)-1-methyl-6-(trifluoromethyl)-2,4(1H,3H)-pyrimidinedione, methylcarbamate (ester)

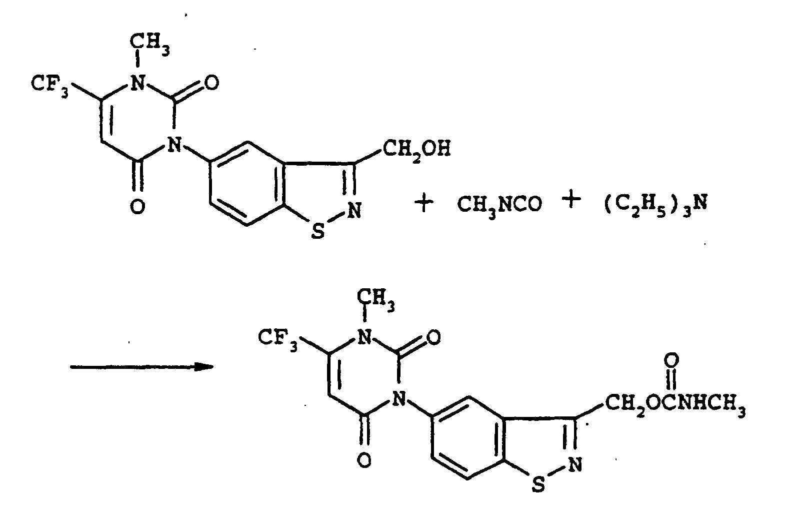

To a mixture of 3-[3-(hydroxymethyl)-1,2-benzisothiazol-5-yl]-6-(trifluoromethyl)-2,4(1H,3H)-pyrimidinedione (1.00 g, 0.00280 mol) and methylene chloride at 0°C is added triethylamine (0.590 ml, 0.00420 mol) followed by methyl isocyanate (0.190 ml, 0.00320 mol). The resultant mixture is stirred 27 hours at ambient temperature, diluted with ethyl acetate and saturated sodium bicarbonate, and stirred 15 minutes. The organic layer is washed with saturated sodium bicarbonate and brine, dried over anhydrous magnesium sulfate, filtered and concentrated *in vacuo* to a yellow solid. The solid is recrystallized from ethyl acetate to give the title compound as tan crystals (0.440 g, 37.9%, mp >230 °C).

Following essentially the same procedure, but using the appropriate isocyanate, the following compounds are obtained:

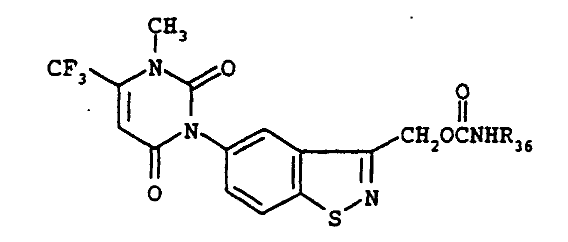

| **R**_{**36**} | **mp °C** |
|---|---|
| C₆H₅ | 209 |
| CH₂C₆H₅ | 174-177 |

### EXAMPLE 147

### Preparation of 3-[3-(1-Hydroxyethyl)-1,2-benzisothiazol-5-yl)-1-methyl-6-(trifluoromethyl)-2,4(1H,3H)-pyrimidinedione, methane sulfonate (ester)

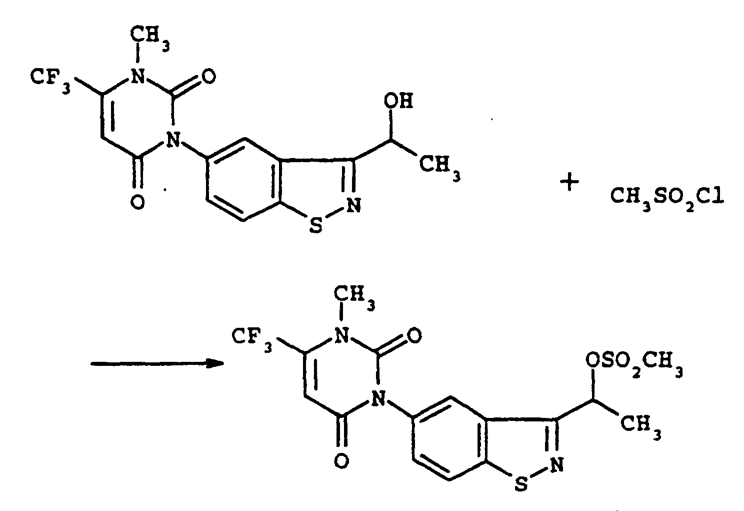

To a solution of 3-[3-(1-hydroxyethyl)-1,2-benzisothiazol-5-yl]-1-methyl-6-(trifluoromethyl)-2,4(1H,3H)-pyrimidinedione (0.650 g, 0.00175 mol) in methylene chloride at 0°C is added methane sulfonyl chloride (0.150 ml, 0.00193 mol) followed by triethylamine (0.320 ml, 0.00228 mol). The resultant mixture is stirred 80 minutes at 0 °C and quenched with pH:7 buffer. The organic layer, after dilution with ethyl acetate and ether, is washed with pH:7 buffer and brine, dried over anhydrous magnesium sulfate, filtered, and concentrated *in vacuo* to a colorless syrup. The syrup is dissolved in ethyl acetate and crystallized by the addition of hexanes to give the title compound as a white, crystalline solid (0.640 g, 81.4%, mp 157 °C) which is identified by NMR spectral analysis.

Following essentially the same procedure, but using the appropriate sulfonyl chloride, the following compounds are prepared:

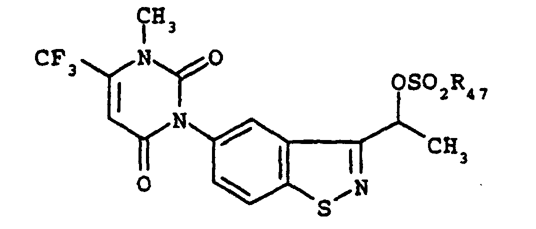

| **R**_{**47**} | **mp °C** |
|---|---|
| CH(CH₃)₂ | 113-115 |
| (CR₂)₃CH₃ | 124-125 |
| C₆H₅ | 92-94 |

Following essentially the same procedure, but using the appropriate sulfonyl chloride on 3-[3-(hydroxymethyl)-1,2-benzisothiazol-5-yl]-1-methyl-6-(trifluoromethyl)-2,4(1H,3H)-pyrimidinedione, the following compounds are obtained:

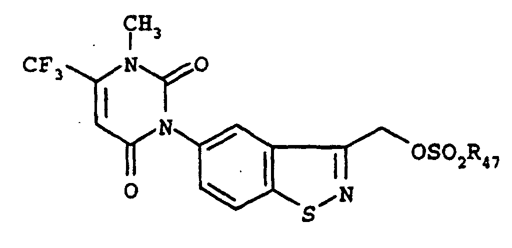

| **R**_{**47**} | **mp °C** |
|---|---|
| CH₃ | 178-180 |
| CH(CH₃)₂ | 141-143 |
| (CH₂)₃CH₃ | 142-145 |
| C₆H₅ | 150-152 |

### EXAMPLE 148

### Preparation of 5-[3,6-Dihydro-3-methyl-2,6-dioxo-4-(trifluoromethyl)-1(2H)-pyrimidinyl]-1,2-benzisothiazole-3-carboxaldehyde, 3-(diethylacetal)

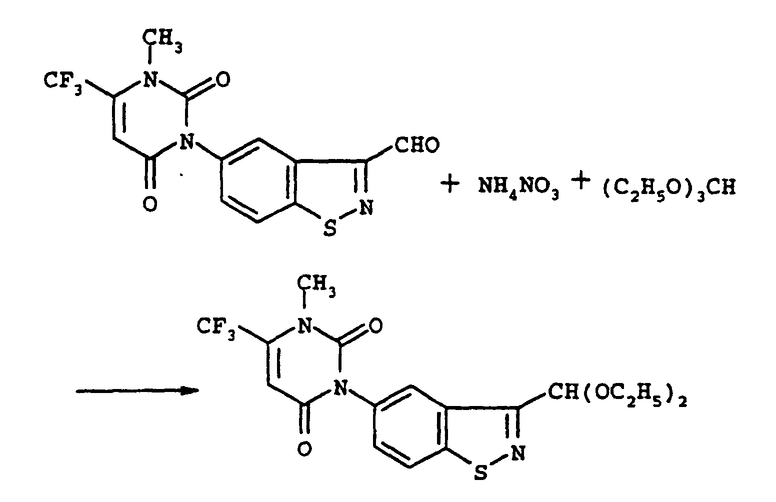

To a suspension of 5-[3,6-dihydro-3-methyl-2,6-dioxo-4-(trifluoromethyl)-1(2H)-pyrimidinyl]-1,2-benzisothiazole-3-carboxaldehyde (0.800 g, 0.00225 mol) in ethanol is added triethyl orthoformate (0.560 ml, 0.00338 mol), followed by ammonium nitrate (0.0100 g). The resultant mixture is stirred 19 hours at room temperature and three hours at reflux. After cooling to room temperature, the mixture is concentrated *in vacuo* to a yellow oil. The oil is chromatographed on silica gel with hexanes:ethyl acetate to give the title compound as a yellow foam (0.690 g, 71.4%, mp 49 °C) which is identified by NMR spectral analysis.

### EXAMPLE 149

### Preparation of 5-[3,6-Dihydro-3-methyl-2,6-dioxo-4-(trifluoromethyl)-1(2H)-pyrimidinyl]-1,2-benzisothiazole-3-carboxaldehyde, 3-[bis(3-hydroxypropyl)dithioacetal]

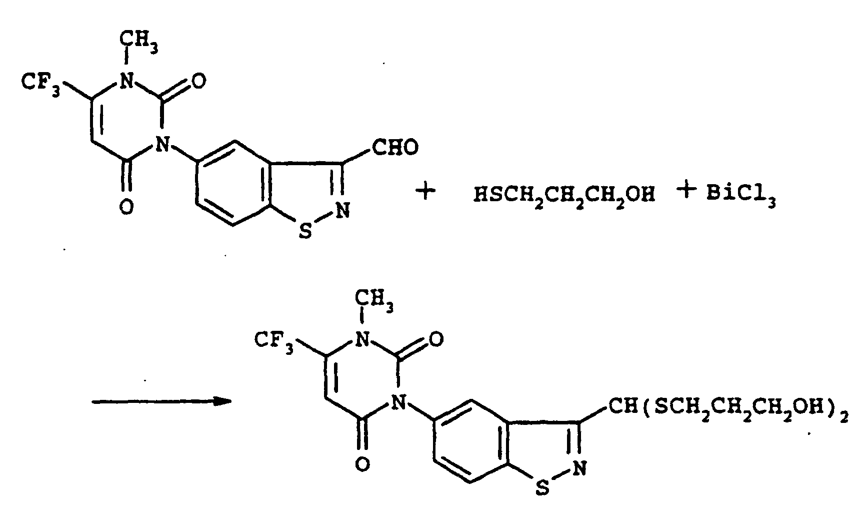

To a mixture of 5-[3,6-dihydro-3-methyl-2,6-dioxo-4-(trifluoromethyl)-1(2H)-pyrimidinyl]-1,2-benzisothiazole-3-carboxaldehyde (2.86 g, 0.00805 mol) and acetonitrile is added 3-mercapto-1-propanol (0.600 ml, 0.00684 mol) followed by bismuth(III) chloride (0.0900 g). The resultant mixture is stirred overnight at room temperature. Additional 3-mercapto-1-propanol (0.200 ml, 0.00228 mol) is added and the mixture is concentrated *in vacuo*. The residue is chromatographed on silica gel with acetone:ethyl acetate; the second component, isolated as a yellow oil, is taken up in ether, washed with water and brine, dried over anhydrous magnesium sulfate, filtered, and concentrated *in vacuo* to give the title compound as a yellow solid (1.00 g, 23.8%) which is identified by NMR spectral analysis.

### EXAMPLE 150

### Preparation of 5-[3,6-Dihydro-3-methyl-2,6-dioxo-4-(trifluoromethyl)-1(2H)-pyrimidinyl]-1,2-benzisothiazole-3-carboxaldehyde, 3-[bis(2-hydroxyethyl)dithioacetal], diacetate (diester)

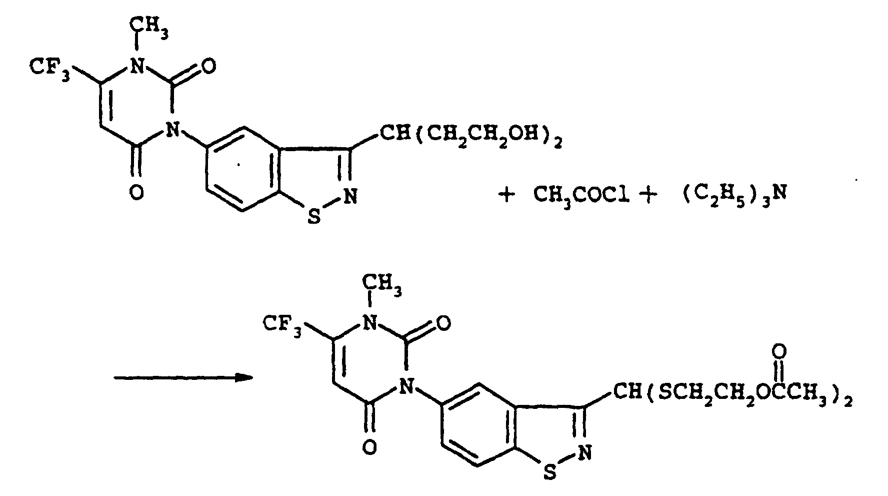

To a solution of 5-[3,6-dihydro-3-methyl-2,6-dioxo-4-(trifluoromethyl)-1(2H)-pyrimidinyl]-1,2-benzisothiazole-3-carboxaldehyde, 3-[bis(2-hydroxyethyl)-dithioacetal (0.500 g, 0.00101 mol) in methylene chloride is added triethylamine (0.400 ml, 0.00300 mol) followed by acetyl chloride (0.160 ml, 0.00220 mol). The resultant mixture is stirred one hour at room temperature, poured into water and diluted with methylene chloride. The organic layer is saved and the aqueous layer is extracted with methylene chloride. The combined organic layers are washed with water and brine, dried over anhydrous magnesium sulfate, filtered, and concentrated *in vacuo* to a yellow oil. The oil is chromatographed on silica gel with hexanes:ethyl acetate to give the title compound as a yellow oil (0.340 g, 58.3%) which is identified by NMR spectral analysis.

Using essentially the same procedure 5-[3,6-dihydro-3-methyl-2,6-dioxo-4-(trifluoromethyl)-1(2H)-pyrimidinyl]-1,2-benzisothiazole-3-carboxaldehyde, 3-[bis(3-hydroxypropyl)dithioacetal], diacetate (diester), is obtained from 5-[3,6-dihydro-3-methyl-2,6-dioxo-4-(trifluoromethyl)-1(2H)-pyrimidinyl]-1,2-benzisothiazole-3-carboxaldehyde, 3-[bis(3-hydroxypropyl)dithioacetal.

### EXAMPLE 151

### Preparation of 5-[3,6-Dihydro-3-methyl-2,6-dioxo-4-(trifluoromethyl)-1(2H)-pyrimidinyl]-1,2-benzisothiazole-3-carboxaldehyde, 3-[bis(2-hydroxyethyl)dithioacetal]

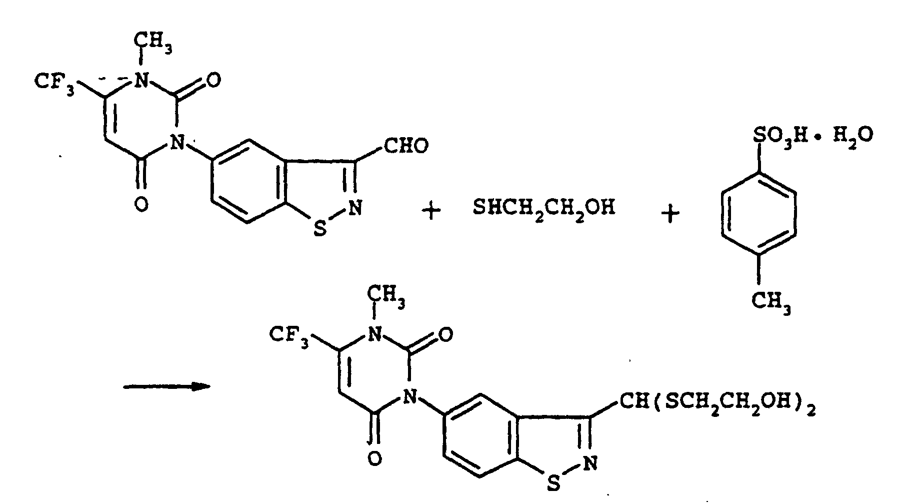

A mixture of 5-[3,6-dihydro-3-methyl-2,6-dioxo-4-(trifluoromethyl)-1(2H)-pyrimidinyl]-1,2-benzisothiazole-3-carboxaldehyde (3.00 g, 0.00844 mol), 2-mercaptoethanol (1.80 ml, 0.0252 mol), *p*-toluenesulfonic acid (0.0800 g, 0.000420 mol) and toluene is stirred two hours at reflux with azeotropic removal of water. After cooling to room temperature, the mixture is stirred overnight, diluted with ethyl acetate, washed with water and brine, dried over anhydrous magnesium sulfate, filtered, and concentrated *in vacuo* to obtain a brown oil. The oil is chromatographed on silica gel to give the title compound as a white solid (1.10 g, 31.3%) which is identified by NMR spectral analysis.

### EXAMPLE 152

### Preparation of 5-[3,6-Dihydro-3-methyl-2,6-dioxo-4-(trifluoromethyl)-1(2H)-pyrimidinyl]-1,2-benzisothiazole-3-carboxaldehyde, 3-oxime, (E)-isomer

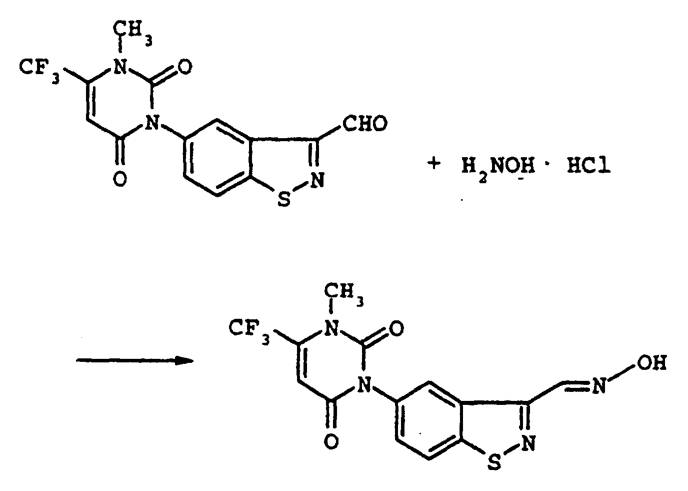

A mixture of 5-[3,6-dihydro-3-methyl-2,6-dioxo-4-(trifluoromethyl)-1(2H)-pyrimidinyl]-1,2-benzisothiazole-3-carboxaldehyde (0.200 g, 0.000563 mol), hydroxylamine hydrochloride (0.0700 g, 0.00100 mol), sodium acetate (0.150g, 0.00183 mol) and ethanol is stirred one hour at 50-60 °C, and concentrated *in vacuo*. The residue is partitioned between methylene chloride and water. The organic layer is separated, washed with brine, dried over anhydrous magnesium sulfate, filtered, and concentrated *in vacuo.* The residue is chromatographed on silica gel with methylene chloride:ethyl acetate to give the title compound as a white solid (0.140 g, 67.3%, mp 234-236 °C) which is identified by NMR spectral analysis.

Following essentially the same procedure, but using the appropriate aldehyde with various hydroxylamines or hydrazines, the following oximes and hydrazones are obtained:

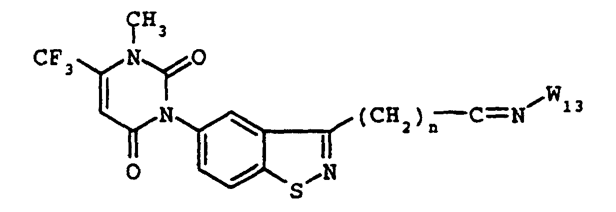

| **n** | **W**_{**13**} | **mp °C** |
|---|---|---|
| 1 | OH | 186-188 |
| 0 | OCH₃ | 235-237 |
| 0 | NHC₆H₅ | >220 |
| 0 | N(CH₃)₂ | 192-192.5 |
| 0 | N(CH₃)C₆H₅ | 198.5 |

### EXAMPLE 153

### Preparation of 3-[3-(Chloroacetyl)-1,2-benzisothiazol-5-yl]-1-methyl-6-(trifluoromethyl)-2,4(1H,3H)-pyrimidinedione

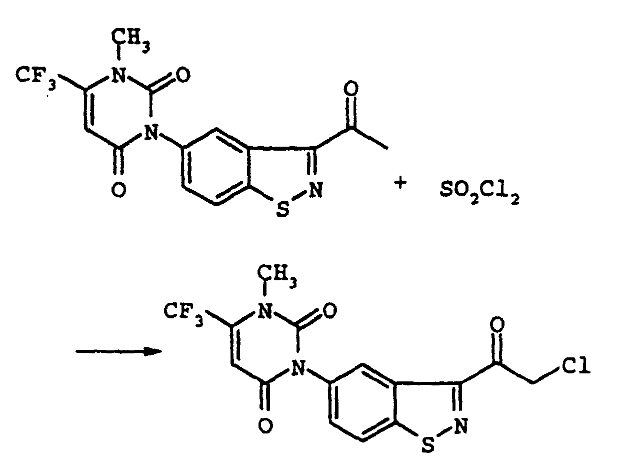

To a solution of 3-(3-acetyl-1,2-benzisothiazol-5-yl)-1-methyl-6-(trifluoromethyl)-2,4(1H,3H)-pyrimidinedione (0.570 g, 0.00154 mol) in chloroform is added sulfuryl chloride (0.350 ml, 0.00433 mol). The resultant mixture is stirred one hour at reflux. Concentrated hydrochloric acid (one drop) is added followed by sulfuryl chloride (1.05 ml, 0.0130 mol) and the resultant mixture is stirred three days at reflux. The mixture is cooled to room temperature, diluted with ethyl acetate and ether, washed with saturated sodium bicarbonate and brine, dried over anhydrous magnesium sulfate, and concentrated *in vacuo* to a solid. The resultant solid is chromatographed on silica gel with hexanes: ethyl acetate to afford a white solid. The solid is chromatographed on silica gel with chloroform to afford a white solid, which is recrystallized from ethyl acetate and hexanes to give the title compound as a white crystalline solid (mp 215-215 °C) which is identified by NMR spectral analysis.

### EXAMPLE 154

### Preparation of 3-[3-(Bromoacetyl)-1,2-benzisothiazol-5-yl]-1-methyl-6-(trifluoromethyl)-2,4(1H,3H)-pyrimidinedione

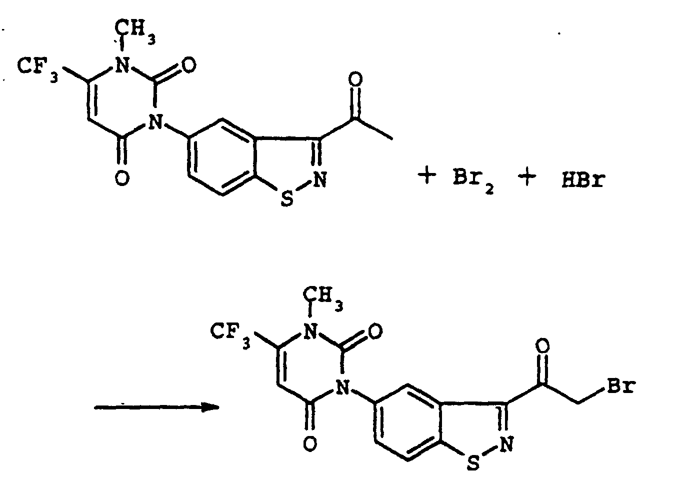

To a solution of 3-(3-acetyl-1,2-benzisothiazol-5-yl)-1-methyl-6-(trifluoromethyl)-2,4(1H,3H)-pyrimidinedione (0.800 g, 0.00217 mol) in ethyl acetate at room temperature is added hydrobromic acid (48%, 3 drops) followed by bromine in ethyl acetate (0.522 M, 4.35 ml, 0.00277 mol). The resultant mixture is stirred two hours at reflux, cooled to room temperature, diluted with ethyl acetate, washed sequentially with sodium thiosulfate, saturated sodium bicarbonate and brine, dried over anhydrous magnesium sulfate, filtered, and concentrated *in vacuo* to a white solid. The solid is recrystallized from hexanes:ethyl acetate to give the title compound as a white, crystalline solid (mp 191-193 °C) which is identified by NMR spectral analysis.

### EXAMPLE 155

### Preparation of (E)- and (Z)- α-(Cyclopropylmethylene)-5-[3,6-dihydro-3-methyl-2,6-dioxo-4-(trifluoromethyl)-1(2H)-pyrimidinyl]-1,2-benzisothiazole-3-acetonitrile

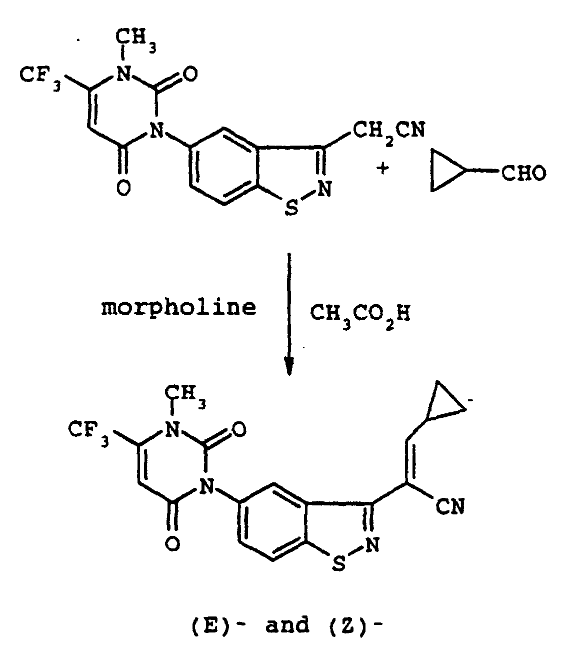

A mixture of 5-[3,6-dihydro-3-methyl-2,6-dioxo-4-(trifluoromethyl)-1(2H)-pyrimidinyl]-1,2-benzisothiazole-3-acetonitrile (0.730 g, 0.00199 mol), cyclopropane aldehyde (0.300 g, 0.00428 mol), morpholine (0.100 ml), acetic acid (0.0500 ml) and ethanol is stirred 45 minutes at reflux, cooled and poured into 10% aqueous hydrochloric acid. The resultant precipitate is filtered, washed with water, and dried *in vacuo* to give the title compound (0.780 g, 93.8%, mp 201-205 °C) which is identified by IR and NMR spectral analysis.

Using essentially the same procedure, the following compounds are obtained:

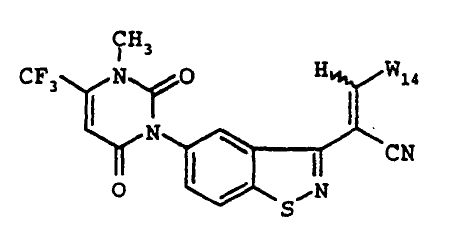

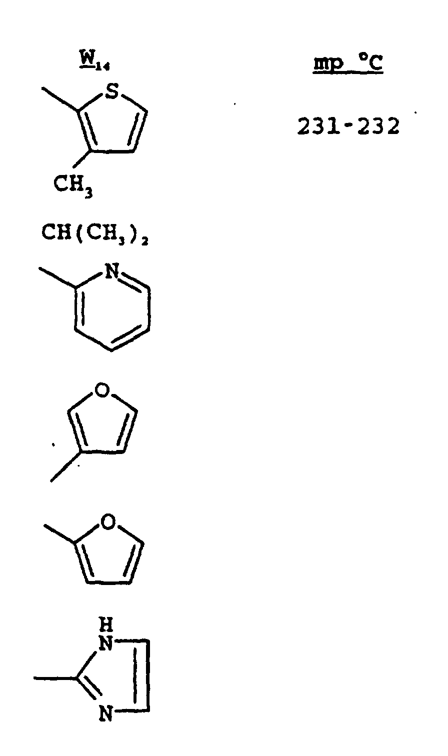

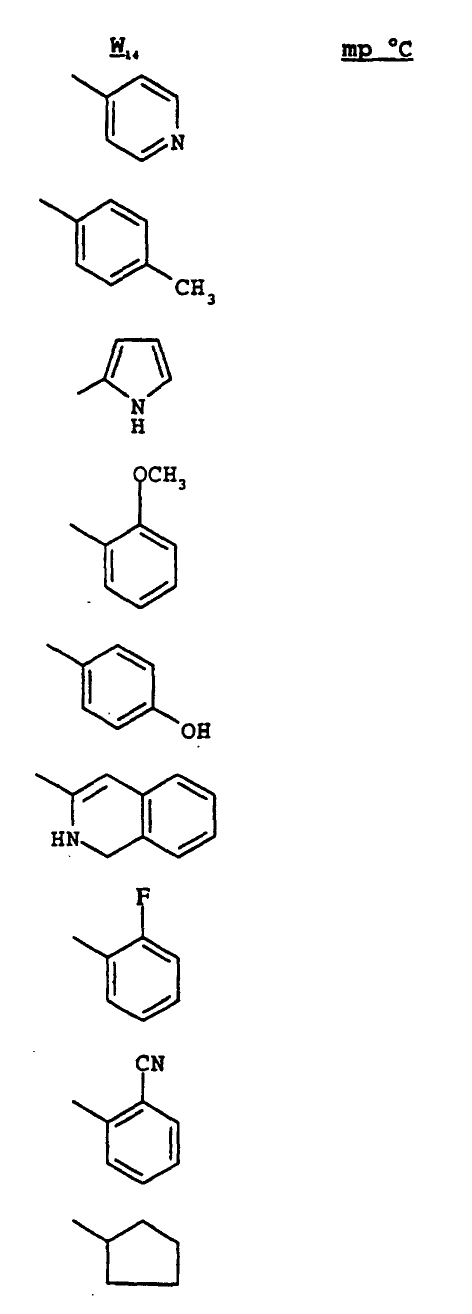

Using essentially the same procedure with methyl 5-[3,6-dihydro-3-methyl-2,6-dioxo-4-(trifluoromethyl)-1(2H)-pyrimidinyl]-1,2-benzisothiazole-3-acetate as substrate and the appropriate aldehyde, the following compounds are obtained:

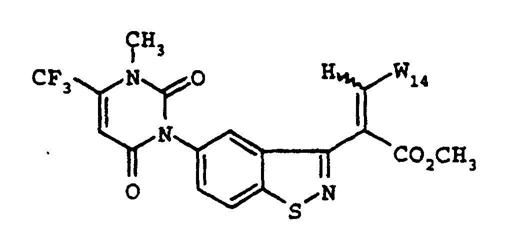

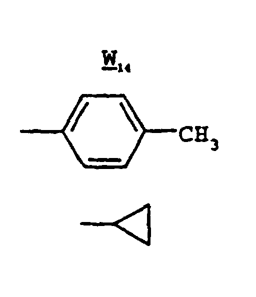

### EXAMPLE 156

### Preparation of 5-[3,6-Dihydro-3-methyl-2,6-dioxo-4-(trifluoromethyl)-1(2H)-pyrimidinyl]-α-ethyl-1,2-benzisothiazole-3-acetonitrile

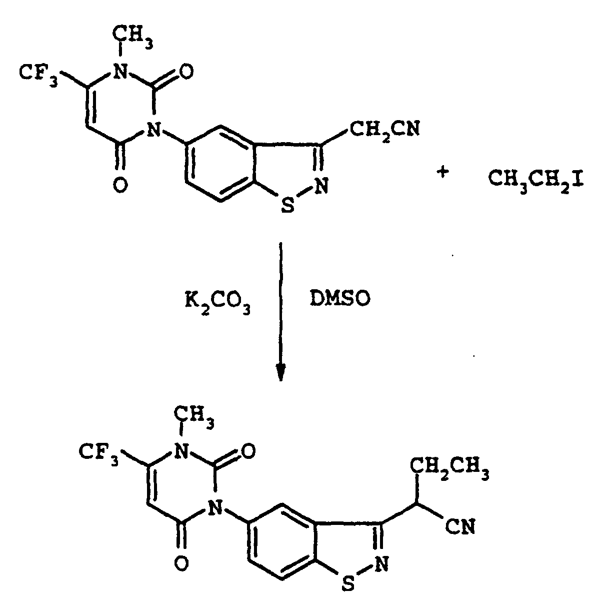

To a solution of 5-[3,6-dihydro-3-methyl-2,6-dioxo-4-(trifluoromethyl)-1(2H)-pyrimidinyl]-1,2-benzisothiazole-3-acetonitrile (0.730 g, 0.00199 mol) in dimethyl sulfoxide is added iodoethane (0.170 ml, 0.00210 mol) followed by potassium carbonate (0.750 g, 0.00543 mol). The resultant mixture is stirred overnight at room temperature and poured onto a mixture of ice and 5% aqueous hydrochloric acid. The resultant solid is filtered, dried and taken up in a minimal amount of methylene chloride. Flash column chromatography using silica gel and 100% to 99:1 methylene chloride/diethyl ether gives the title compound as a white solid (0.220 g, 28.0%, mp 107-109 °C) which is identified by NMR spectral analysis.

Using essentially the same procedure and iodomethane as alkylating agent 5-[3,6-dihydro-3-methyl-2,6-dioxo-4-(trifluoromethyl)-1(2H)-pyrimidinyl]-α-methyl-1,2-benzisothiazole-3-acetonitrile is obtained.

Using essentially the same procedure with the appropriate alkylating agent and methyl 5-[3,6-dihydro-3-methyl-2,6-dioxo-4-(trifluoromethyl)-1(2H)-pyrimidinyl]-1,2-benzisothiazole-3-acetate as substrate, the following compounds are obtained:

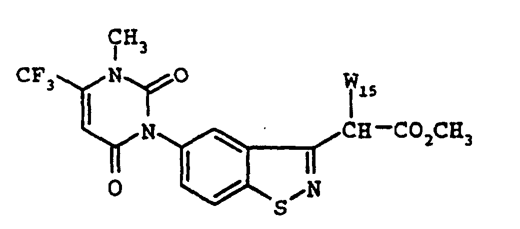

| **W**_{**15**} |
|---|
| CH₃ |
| CH₂CH(CH₃)₂ |

### EXAMPLE 157

### Preparation of 5-[3,6-Dihydro-3-methyl-2,6-dioxo-4-(trifluoromethyl)-1(2H)-pyrimidinyl]-α,α-diethyl-1,2-benzisothiazole-3-acetonitrile

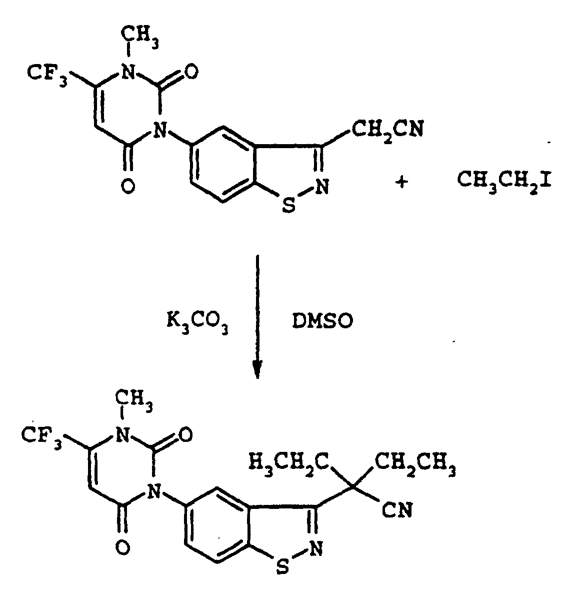

To a solution of 5-[3,6-dihydro-3-methyl-2,6-dioxo-4-(trifluoromethyl)-1(2H)-pyrimidinyl]-1,2-benzisothiazole-3-acetonitrile (0.730 g, 0.00199 mol) in dimethyl sulfoxide is added iodoethane (0.326 ml, 0.00408 mol) followed by potassium carbonate (0.750 g, 0.00543 mol). The resultant mixture is stirred overnight at room temperature, poured into water, and extracted with methylene chloride. The combined organic layers are dried over anhydrous magnesium sulfate, and concentrated *in vacuo* to a yellow oil. The oil is flash chromatographed on silica gel with 100% to 99:1 methylene chloride/ethyl ether to afford the title compound as a white solid (0.430 g, 51.2%, mp 76-78 °C) which is identified by NMR spectral analysis.

Using the same starting material, different alkylating agents and essentially the same procedure, the following compounds are obtained:

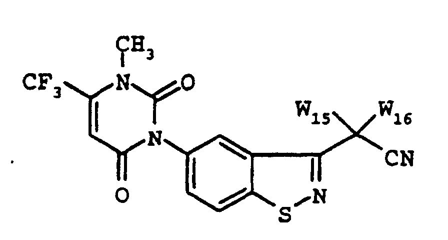

| **W**_{**15**} | **W**_{**16**} |
|---|---|
| CH₃ | CH₃ |
| -CH₂CH₂- | |
| CH₂CH=CH₂ | CH₂CH=CH₂ |
| CH₂C≡CH | CH₂C≡CH |
| CH₂C₆H₅ | CH₂C₆H₅ |

### EXAMPLE 158

### Preparation of 5-[3,6-Dihydro-3-methyl-2,6-dioxo-4-(trifluoromethyl)-1(2H)-pyrimidinyl]-1,2-benzisothiazole-3-carboxylic acid, 2-butynyl ester

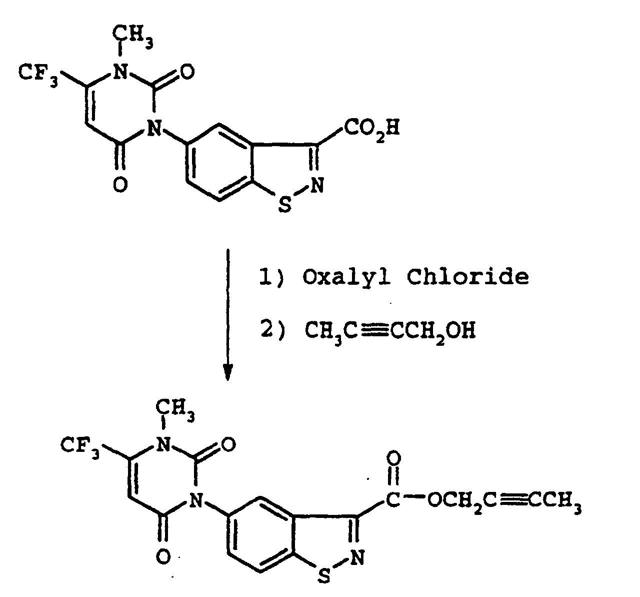

To a mixture of 5-[3,6-dihydro-3-methyl-2,6-dioxo-4-(trifluoromethyl-1(2H)-pyrimidinyl]-1,2-benzisothiazole-3-carboxylic acid (2.00 g, 0.00538 mol), methylene chloride and tetrahdrofuran is added N,N-dimethylformamide (8 drops). The resultant mixture is cooled on an ice bath, treated with oxalyl chloride (6.70 ml, 0.0135 mol), stirred one hour on an ice bath, and concentrated *in vacuo.* 12.5% of the residue as a solution in tetrahydrofuran is added to a mixture of 2-butyn-1-ol (0.200 ml), triethylamine and tetrahydrofuran. The resultant mixture is stirred overnight at ambient temperature and poured into cold 10% hydrochloric acid. The mixture is extracted with methylene chloride and the combined organic layers are dried over anhydrous magnesium sulfate, filtered, and concentrated *in vacuo* to give the title compound as an off-white solid (0.140 g, 49.1%, mp 138-139 °C) which is identified by NMR spectral analysis.

Using essentially the same procedure, with the appropriate alcohols or thioalcohols, the following compounds are obtained:

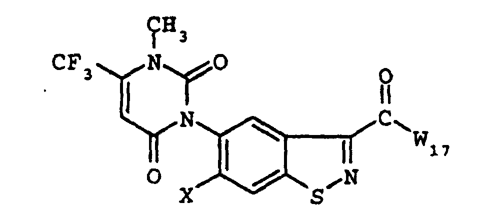

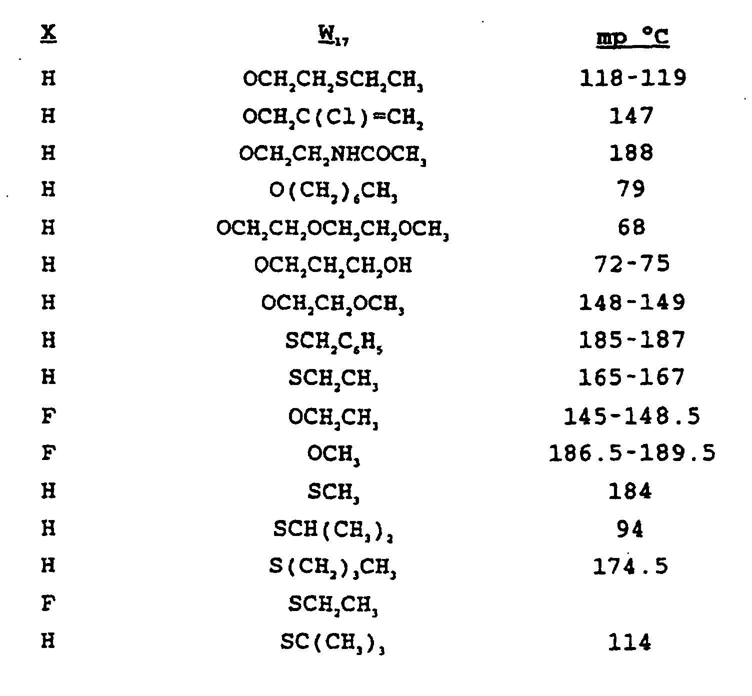

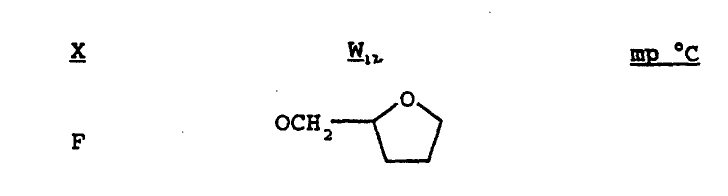

### EXAMPLE 159

### Preparation of 5-[3,6-Dihydro-3-methyl-2,6-dioxo-4-(trifluoromethyl)-1(2H)-pyrimidinyl]-1,2-benzisothiazole-3-carboxylic acid, methyl ester

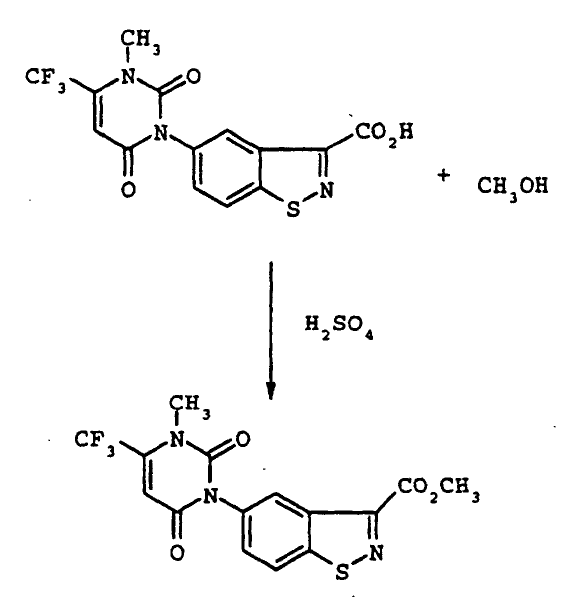

To a solution of 5-[3,6-dihydro-3-methyl-2,6-dioxo-4-(trifluoromethyl)-1(2H)-pyrimidinyl]-1,2-benzisothiazole-3-carboxylic acid (0.300 g, 0.000809 mol) in methanol is added sulfuric acid (98%, 5 drops) at room temperature. The resultant mixture is stirred overnight at room temperature and concentrated *in vacuo.* The residue is taken up in methylene chloride, washed with saturated sodium bicarbonate and brine, dried over anhydrous sodium sulfate, filtered, and concentrated *in vacuo.* Column chromatography of the residue on silica gel with methylene chloride:ethyl acetate gives the title compound as a white solid (0.235 g, 75.3%, mp 153-156 °C).

### EXAMPLE 160

### Preparation of 5-[3,6-Dihydro-3-methyl-2,6-dioxo-4-(trifluoromethyl)-1(2H)-pyrimidinyl]-1,2-benzisothiazole-3-carboxylic acid, isopropyl ester

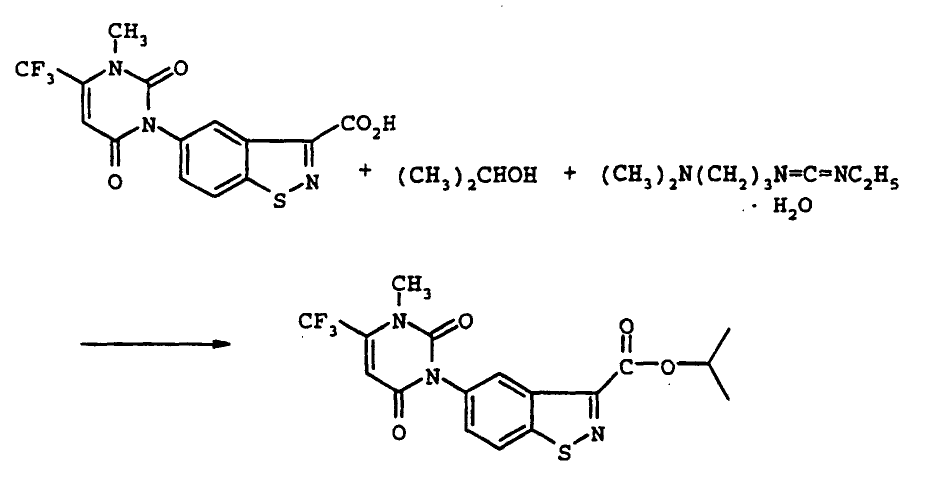

1-(3-Dimethylaminopropyl)-3-ethyl carbodiimide hydrochloride (0.200 g, 0.00105 mol) is added to a mixture of 5-[3,6-dihydro-3-methyl-2,6-dioxo-4-(trifluoromethyl)-1(2H)-pyrimidinyl-1,2-benzisothiazole-3-carboxylic acid (0.200 g, 0.000539 mol), dimethylaminopyridine (0.0100 mg), isopropanol (0.200 g, 0.00330 mol) and methylene chloride at room temperature. The resultant mixture is stirred overnight at room temperature and partitioned between water and methylene chloride. The organic layer is separated, washed with water and brine; dried over anhydrous sodium sulfate, filtered, and concentrated *in vacuo.* The resultant residue is chromatographed on silica gel with methylene chloride:ethyl acetate to give the title compound as a white solid (0.180 g, 80.7%, mp 94-97 °C).

Following essentially the same procedure, but using the appropriate alcohol, the following compounds are obtained:

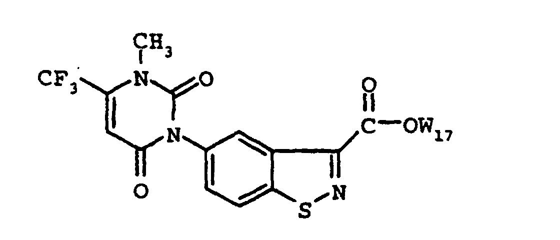

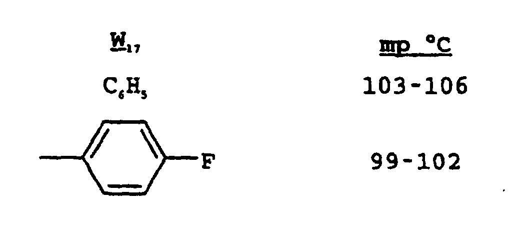

### EXAMPLE 161

### Preparation of 5-[3,6-Dihydro-3-methyl-2,6-dioxo-4-(trifluoromethyl)-1(2H)-pyrimidinyl]-N-(diisopropylsulfamoyl)-1,2-benzisothiazole-3-carboxamide

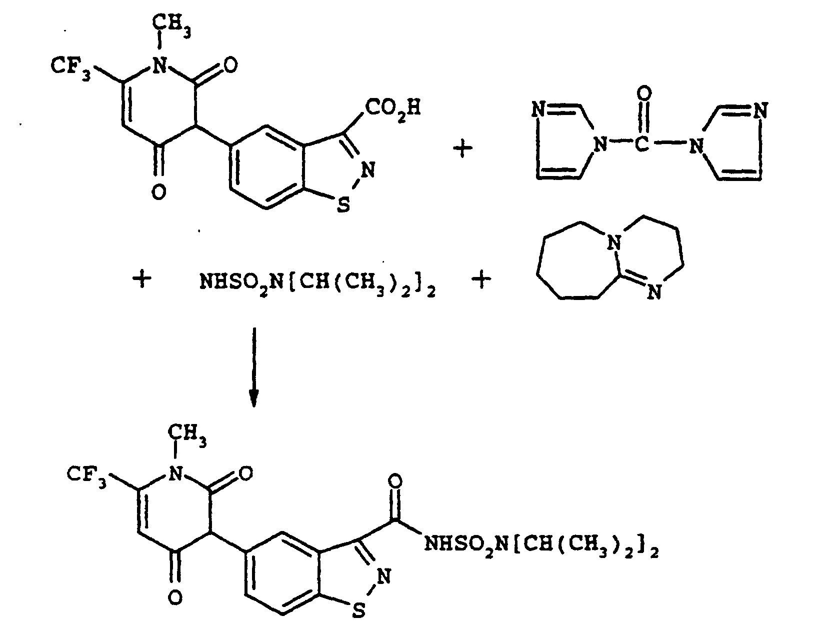

A solution of 5-[3,6-dihydro-3-methyl-2,6-dioxo-4-(trifluoromethyl)-1(2H)-pyrimidinyl-1,2-benzisothiazole-3-carboxylic acid (1.00 g, 0.00270 mol) and carbonyldiimidazole (0.480 g, 0.00296 mol) in tetrahydrofuran is stirred at reflux. After 30 minutes, the reaction mixture is cooled to room temperature, treated with N,N-diisopropylsulfamide, stirred for 15 minutes, and treated with 1,8-diazabicyclo(5.4.0]undec-7-ene (0.400 ml, 0.00270 mol). After stirring overnight at room temperature, the reaction mixture is concentrated *in vacuo*, and partitioned between water and ethyl acetate. The aqueous layer is extracted with ethyl acetate and the combined organic layers are washed with brine, dried over anhydrous magnesium sulfate, and concentrated *in vacuo.* Flash chromatography of the resultant residue on silica gel using 1:2 ethyl acetate/hexanes gives the title compound as a white solid (0.200 g, mp 214 °C).

Using essentially the same procedure, the following compounds are obtained:

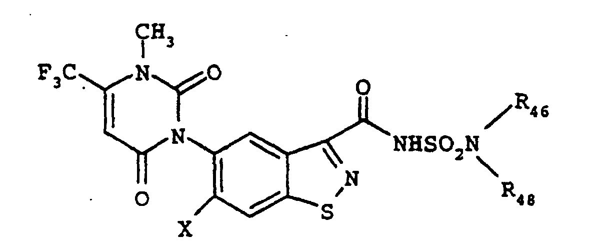

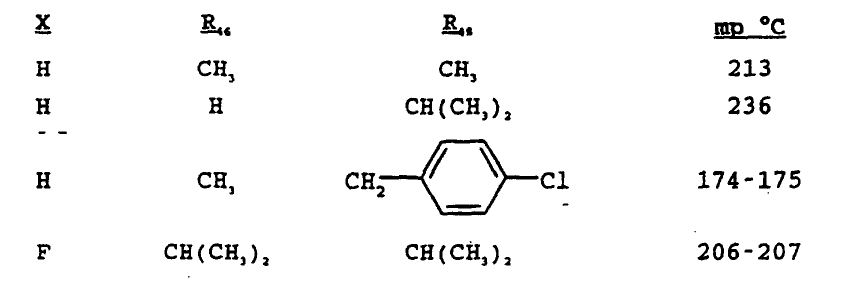

Using essentially the same procedure but starting with the appropriate C-3 phenyl acid, the following compounds are prepared:

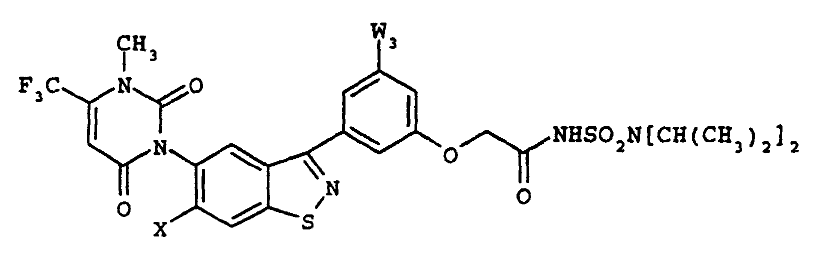

| **X** | **W**_{**3**} | **mp °C** |
|---|---|---|
| H | H | 121-123 |
| H | CH₃ | 193-195 |
| F | CH₃ | 192 |

### EXAMPLE 162

### Preparation of 5-[3,6-Dihydro-3-methyl-2.6-dioxo-4-(trifluoromethyl)-1(2H)-pyrimidinyl]-N-sulfamoyl-1,2-benzisothiazole-3-carboxamide

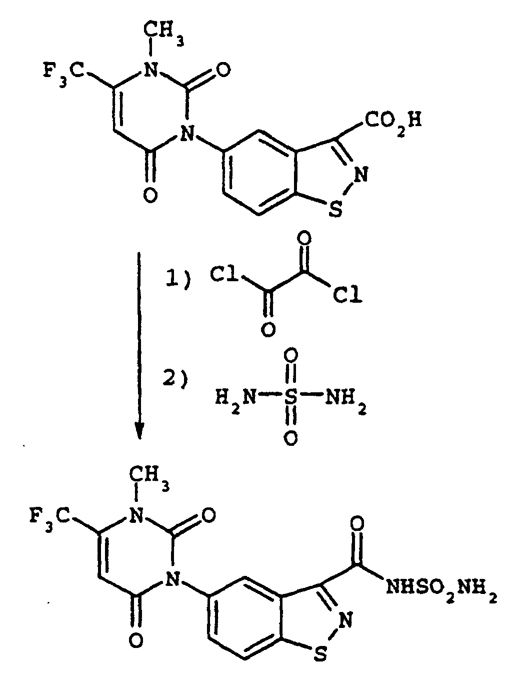

A solution of 5-[3,6-dihydro-3-methyl-2,6-dioxo-4-(trifluoromethyl)-1(2H)-pyrimidinyl-1,2-bnezisothiazole-3-carboxylic acid (0.500 g, 0.00135 mol) in 9:1 methylene chloride/tetrahydrofuran at 10 °C is treated with oxalyl chloride (1.68 ml, 0.00337 mol) and N,N-dimethylformamide (1 drop). After 1 hour, the reaction mixture is concentrated *in vacuo*, taken up into 1,4-dioxane, and added dropwise to a solution of sulfamide (1.29 g, 0.0135 mol) in 1,4-dioxane at 0°C. After stirring overnight at room temperature, the reaction mixture is concentrated *in vacuo*, taken up into ethyl acetate, washed sequentially with brine and 10% aqueous HCl, dried over anhydrous magnesium sulfate, and concentrated *in vacuo* to give a crude product. The crude product is filtered through silica gel, using 6:4 ethyl acetate/hexanes to give the title product as an off-white solid, mp 221 °C.

### EXAMPLE 163

### Preparation of 5-[3,6-Dihydro-3-methyl-2,6-dioxo-4-(trifluoromethyl)-1(2H)-pyrimidinyl]-N-isopropyl-1,2-benzisothiazole-3-carboxamide

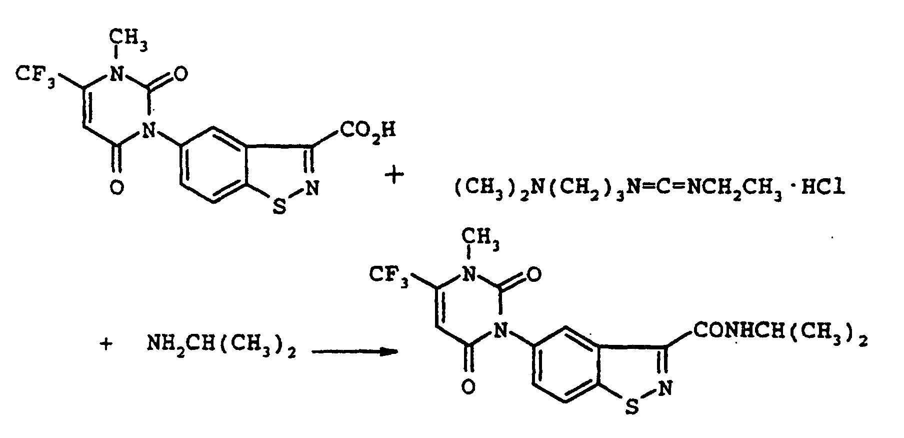

To a mixture of 5-[3,6-dihydro-3-methyl-2,6-dioxo-4-(trifluoromethyl)-1(2H)-pyrimidinyl-1,2-benzisothiazole-3-carboxylic acid (0.400 g, 0.00108 mol), isopropylamine (0.140 g, 0.00237 mol), dimethylaminopyridine (0.000100 g) and methylene chloride is added 1-(3-dimethylaminopropyl)-3-ethylcarbodiimide hydrochloride (0.300 g, 0.00157 mol) at room temperature. The resultant mixture is stirred overnight at room temperature and partitioned between methylene chloride and water. The organic layers are washed with 0.5 M hydrochloric acid and brine, dried over anhydrous sodium sulfate, filtered, and concentrated *in vacuo.* The resultant residue is chromatographed on silica gel with methylene chloride:ethyl acetate to give the title compound as a white solid (0.250 g, 56.2%, mp 110-113 °C).

Following essentially the same procedure, but using the appropriately substituted amine, the following compounds are obtained:

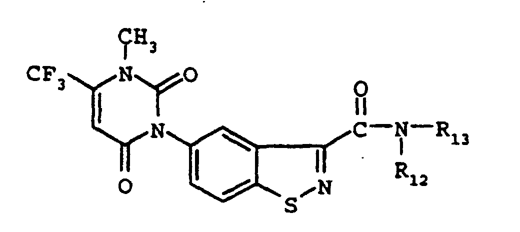

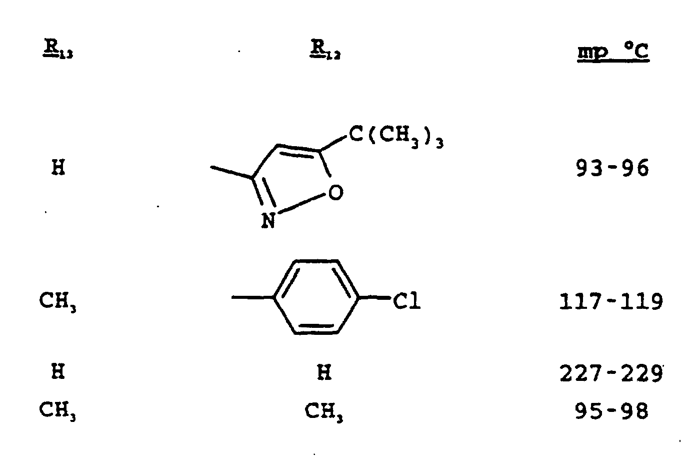

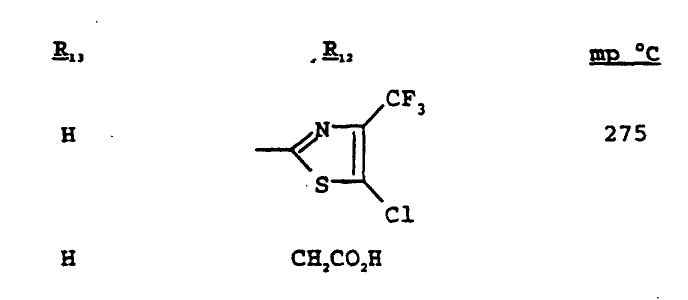

### EXAMPLE 164

### Preparation of 5-[3,6-Dihydro-3-methyl-2,6-dioxo-4-(trifluoromethyl)-1(2H)-pyrimidinyl]-N-methoxy-1,2-benzisothiazole-3-carboxamide

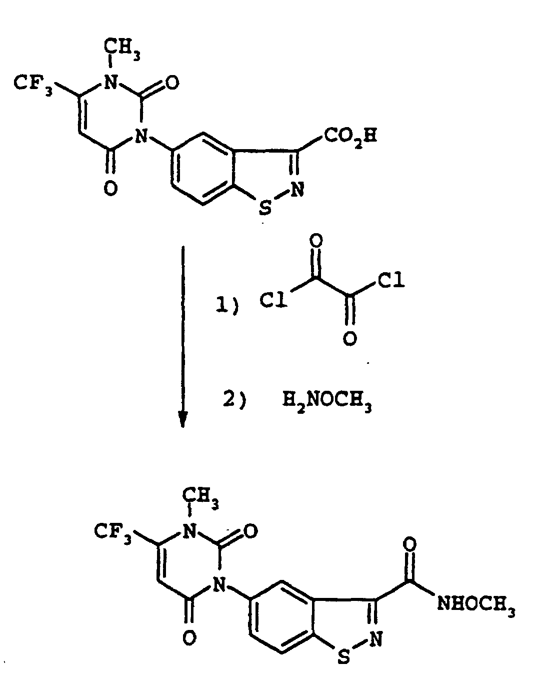

To a mixture of 5-[3,6-dihydro-3-methyl-2,6-dioxo-4-(trifluoromethyl)-1(2H)-pyrimidinyl]-1,2-benzisothiazole-3-carboxylic acid (4.00 g, 0.0108 mol), methylene chloride and tetrahydrofuran on an ice bath is added N,N-dimethylformamide (5 drops) followed by oxalyl chloride (13.5 ml, 0.0269 mol). The resultant mixture is stirred 90 minutes at ambient temperature and concentrated *in vacuo.* The residue is taken up in tetrahydrofuran and 25% of the solution is added to a mixture of O-methyl hydroxylamine hydrochloride (0.900 g, 0.0108 mol), tetrahydrofuran and triethylamine (2.25 ml, 0.0162 mol). The resultant mixture is stirred overnight at room temperature, poured into ice water, and extracted with methylene chloride. The organic layers are dried over anhydrous magnesium sulfate, filtered, and concentrated *in vacuo.* The residue is chromatographed on silica gel with methylene chloride to give the title compound as a light yellow solid (0.240 g, 22.3%, mp 125-127 °C).

Using essentially the same procedure, with the appropriate hydroxylamines, the following compounds are obtained:

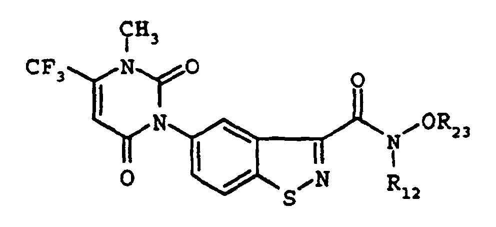

| **R**_{**12**} | **R**_{**23**} | **mp °C** |
|---|---|---|
| H | CH₂CH=CH₂ | 91-92 |
| CH₃ | H | 118-120 |
| H | CH₂C₆H₅ | 96-97 |
| H | SO₂OH | 221 |
| H | H | 234 |

### EXAMPLE 165

### Preparation of 1-Methyl-6-(trifluoromethyl)-3-(3-valeryl-1,2-benzisothiazol-5-yl)-2,4(1H,3H)-pyrimidinedione

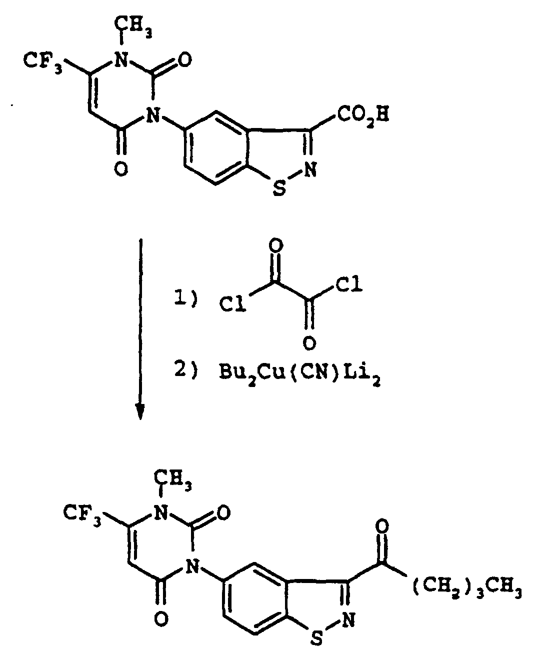

To a solution of 5-[3,6-dihydro-3-methyl-2,6-dioxo-4-(trifluoromethyl)-1(2H)-pyrimidinyl]-1,2-benzisothiazole-3-carboxylic acid (0.200 g, 0.000540 mol) in methylene chloride is added N,N-dimethylformamide (one drop) followed by oxalyl chloride in methylene chloride (2.0 M, 0.540 ml, 0.00108 mol). The resultant mixture is stirred at room temperature for 90 minutes and concentrated *in vacuo* to a white solid, which is saved. To a suspension of copper cyanide (0.061 g, 0.000675 mol) in tetrahydrofuran, is added dropwise *n*-butyllithium (2.5 M in hexanes, 0.540 ml, 0.00135 mol) at -78°C. The reaction mixture is allowed to warm for 5 minutes and then cooled back to -78°C. The mixture is then treated with a solution of the white solid from the first step in tetrahydrofuran, and the resultant mixture stirred one hour at -78°C. The reaction mixture is quenched with saturated ammonium chloride and diluted with ethyl acetate. The organic layer is washed sequentially with saturated ammonium chloride, water and brine, dried over anhydrous magnesium sulfate, filtered, and concentrated *in vacuo* to a yellow syrup. Chromatography of the syrup on silica gel using hexanes:ethyl acetate gives the title compound as a colorless syrup (0.142 g, 64.0%) which is identified by NMR spectral analysis.

Using essentially the same procedure, the following compounds are obtained:

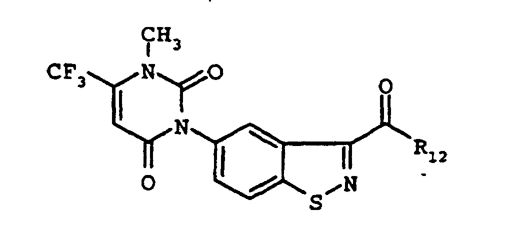

| **R**_{**12**} | **mp °C** |
|---|---|
| C(CH₃)₃ | 83-85 |
| C₆H₅ | 93-95 |
| CH(CH₃)CH₂CH₃ | 75-77 |

### EXAMPLE 166

### Preparation of 5-[3,6-Dihydro-3-methyl-2,6-dioxo-4-(trifluoromethyl)-1(2H)-pyrimidinyl]-N-methyl-N-(methylsulfonyl)-1,2-benzisothiazole-3-carboxamide

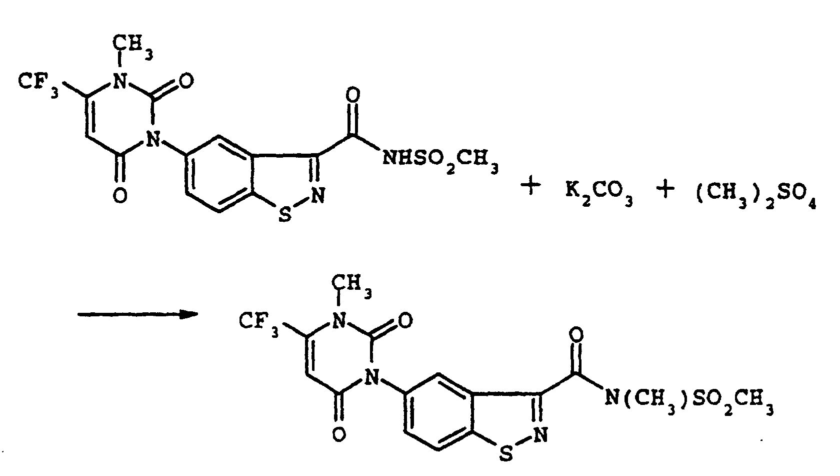

To a solution of 5-[3,6-dihydro-3-methyl-2,6-dioxo-4-(trifluoromethyl)-1(2H)-pyrimidinyl] -N-(methylsulfonyl)-1,2-benzisothiazole-3-carboxamide (0.300 g, 0.000670 mol) in acetone is added dimethyl sulfate (0.180 g, 0.00140 mol) followed by potassium carbonate (0.250 g, 0.00180 mol). The resultant mixture is stirred one hour at reflux and concentrated *in vacuo.* The residue is partitioned between methylene chloride and water. The organic layer is saved and the aqueous layer is extracted with methylene chloride. The combined organic layers are washed with water, dried over anhydrous sodium sulfate, filtered, and concentrated *in vacuo*. The residue is chromatographed on silica gel with ethyl acetate:hexanes to give the title compound as a white solid (0.232 g, 74.9%, mp 120-122 °C).

Using essentially the same procedure, the following compounds are obtained:

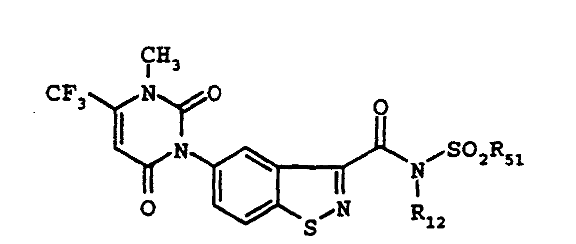

| **R**_{**12**} | **R**_{**51**} | **mp °C** |
|---|---|---|
| CH₂OCH₂CH₃ | CH₃ | 91-93 |
| CH₂OCH₂CH₃ | CH₂C₆H₅ | 185-186 |
| CH₂CO₂CH₃ | CH₂C₆H₅ | 158-160 |
| CH₃ | CH₂C₆H₅ | >225 |

### EXAMPLE 167

### Preparation of 3-(3-Isopropyl-1,2-benzisothiazol-5 yl)-1-methyl-6-(trifluoromethyl)-2,4(1H,3H)-pyrimidinedione

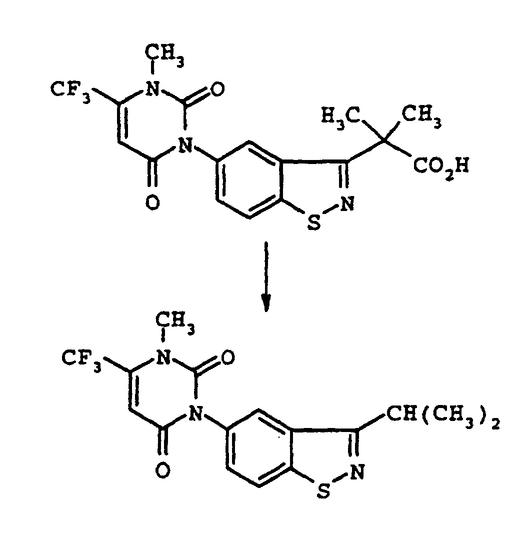

A mixture of 5-[3,6-dihydro-3-methyl-2,6-dioxo-4-(trifluoromethyl)-1(2H)-pyrimidinyl]-α,α-dimethyl-1,2-benzisothiazole-3-acetic acid (2.00 g, 0.00484 mol) and quinoline (20.0 ml) is heated to 140 °C with stirring. After 15 minutes, the mixture is cooled to room temperature, diluted with ethyl acetate, washed sequentially with 2 *N* hydrochloric acid, saturated sodium bicarbonate and brine, dried over anhydrous magnesium sulfate, and concentrated *in vacuo* to a yellow solid. The solid is recrystallized from hexanes:methylene chloride to give the title compound as an off-white solid (0.640 g, 35.8%, mp 139-140 °C) which is identified by NMR and IR spectral analyses.

### EXAMPLE 168

### Preparation of 5-[3,6-Dihydro-3-methyl-2,6-dioxo-4-(trifluoromethyl)-1(2H)-pyrimidinyl]-α,α-dimethyl-1,2-benzisothiazole-3-acetic acid, allyl ester

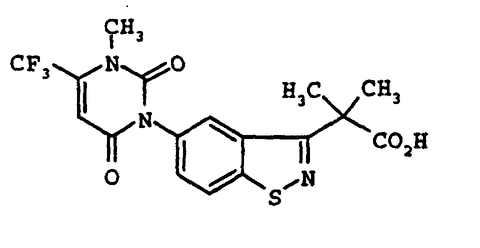

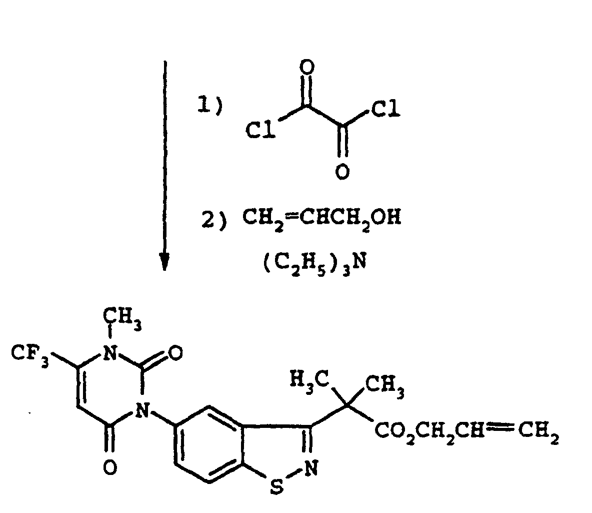

N,N-dimethylformamide (8 drops) is added to a mixture of 5-[3,6-dihydro-3-methyl-2,6-dioxo-4-(trifluoromethyl)-1(2H)-pyrimidinyl]-α,α-dimethyl-1,2-benzisothiazole-3-acetic acid (2.00 g, 0.00484 mol) and methylene chloride. The mixture is cooled on an ice bath and oxalyl chloride (2.0 M in methylene chloride, 6.05 ml, 0.0121 mol) is added. The resultant mixture is stirred one hour on an ice bath and concentrated *in vacuo*. The residue is taken up in tetrahydrofuran. To this solution are added allyl alcohol (0.420 g, 0.0185 mol) and triethylamine (1.50 ml 0.0108 mol). The mixture is stirred three days at room temperature. Dimethylaminopyridine (0.0100 g) is added and the mixture is stirred 24 hours at room temperature and filtered. The filtrate is concentrated *in vacuo.* The residue is taken up in ether, washed with saturated sodium bicarbonate and brine, dried over anhydrous magnesium sulfate, filtered, and concentrated *in vacuo.* The residue is chromatographed on silica gel with hexanes:ethyl acetate to afford the title compound as a white solid (mp 115-116 °C) which is identified by NMR spectral analysis.

Following essentially the same procedure, but using the appropriate alcohol or thioalcohol, the following compounds are obtained:

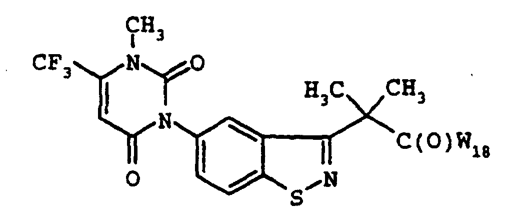

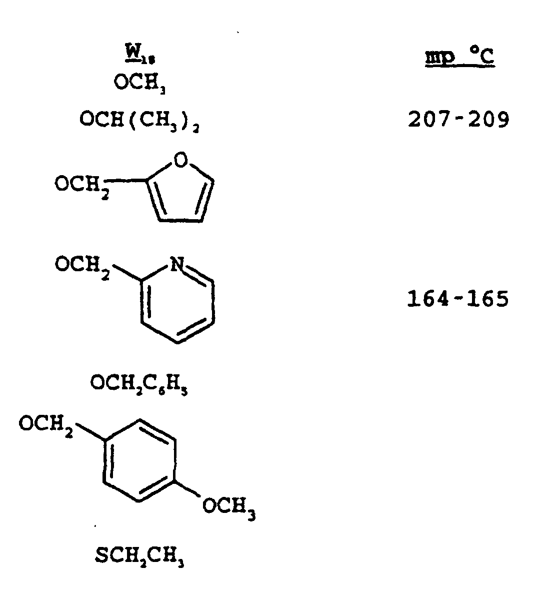

### EXAMPLE 169

### Preparation of 5-[3,6-Dihydro-3-methyl-2,6-dioxo-4-(trifluoromethyl)-1(2H)-pyrimidinyl]-α,α-dimethyl-1,2-benzisothiazole-3-acetic acid

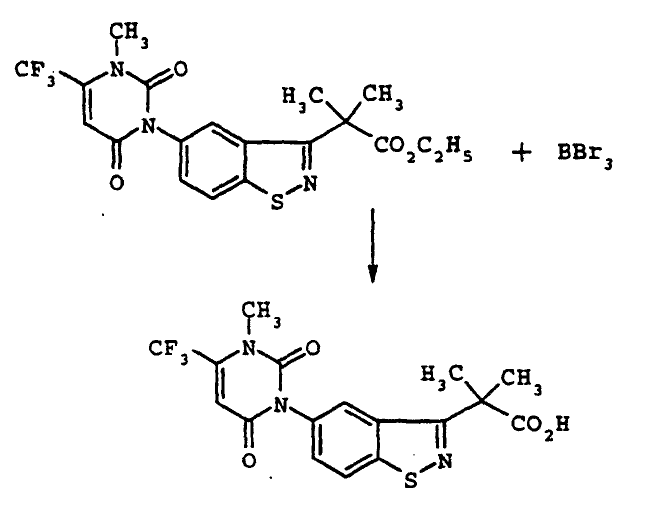

To a solution of ethyl 5-[3,6-dihydro-3-methyl-2,6-dioxo-4-(trifluoromethyl)-1(2H)-pyrimidinyl]-α,α-dimethyl-1,2-benzisothiazole-3-acetate (6.30 g, 0.0143 mol) in methylene chloride at 0 °C is added boron tribromide in methylene chloride (1.0 M, 100 ml, 0.100 mol) such that the temperature does not exceed 15 °C. The resultant mixture is stirred one hour at ambient temperature, quenched with 3 N hydrochloric acid, and diluted with methylene chloride. The organic layer is separated, washed sequentially with water and brine, dried over anhydrous magnesium sulfate, and concentrated *in vacuo* to give the title compound as an off-white solid (mp 155-156 °C) which is identified by NMR and IR spectral analyses.

Using essentially the same procedure, 5-[3,6-dihydro-3-methyl-2,6-dioxo-4-(trifluoromethyl)-1(2H)-pyrimidinyl]-6-fluoro-1,2-benzisothiazole-3-carboxylic acid is prepared from its ethyl ester.

### EXAMPLE 170

### Preparation of 5-[3,6-Dihydro-3-methyl-2,6-dioxo-4-(trifluoromethyl)-1(2H)-pyrimidinyl]-N-(diisopropylsulfamoyl)-α,α-dimethyl-1,2-benzisothiazole-3-acetamide

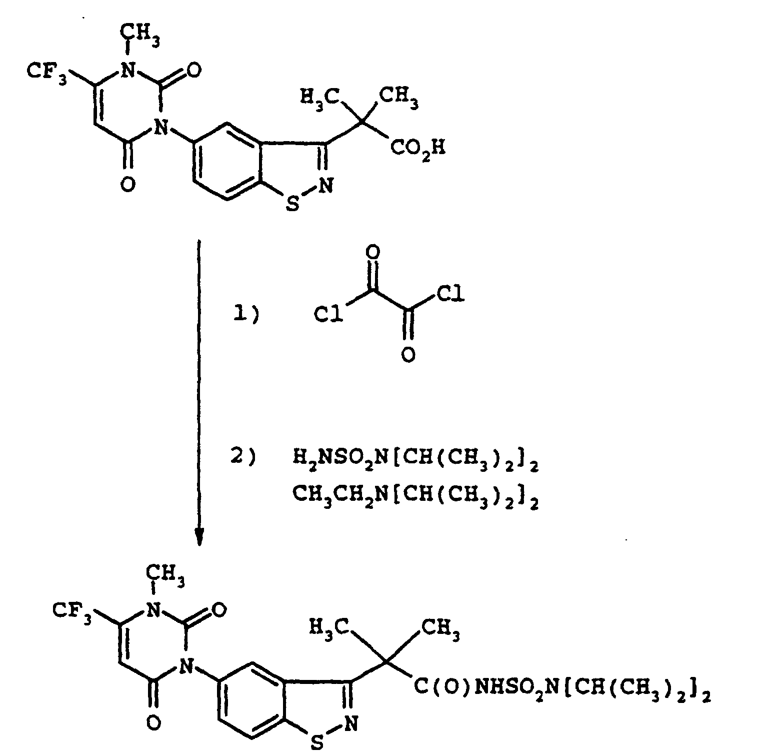

N,N-dimethylformamide (8 drops) is added to a mixture of 5-[3,6-dihydro-3-methyl-2,6-dioxo-4-(trifluoromethyl)-1(2H)-pyrimidinyl]-α,α-dimethyl-1,2-benzisothiazole-3-acetic acid (0.800 g, 0.00194 mol) and methylene chloride. The mixture is cooled on an ice bath and oxalyl chloride (2.0 M in methylene chloride, 2.42 ml, 0.00485 mol) is added. The resultant mixture is stirred one hour on an ice bath and concentrated *in vacuo*. The residue is taken up in tetrahydrofuran. N,N-Diisopropylaminosulfonamide (0.410 g, 0.00278 mol), ethyldiisopropylamine (0.310 g, 0.00240 mol) and dimethylaminopyridine (0.0100 g) are added and the resultant mixture is stirred overnight at room temperature. The mixture is then filtered and the filtrate is concentrated *in vacuo* to afford a yellow resin. The resin is filtered through silica gel to afford the title compound as a tan resin (0.500 g, 44.8%).

### EXAMPLE 171

### Preparation of 3-[3-(1,1-Dimethyl-2-oxopropyl)-1,2-benzisothiazol-5-yl]-1-methyl-6-(trifluoromethyl)-2,4(1H,3H)-pyrimidinedione

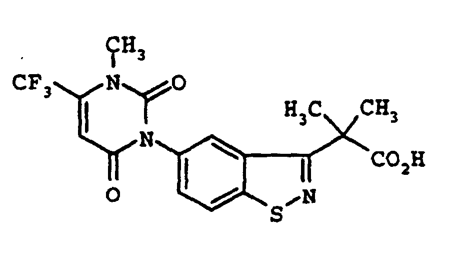

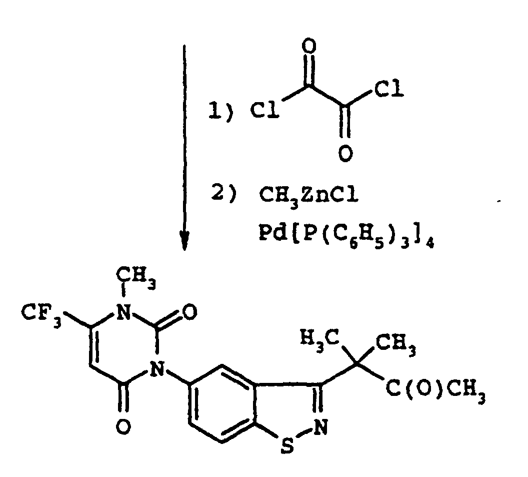

N,N-Dimethylformamide (8 drops) is added to a mixture of 5-[3,6-dihydro-3-methyl-2,6-dioxo-4-(trifluoromethyl)-1(2H)-pyrimidinyl]-α,α-dimethyl-1,2-benzisothiazole-3-acetic acid (0.800 g, 0.00194 mol) and methylene chloride. The mixture is cooled on an ice bath and oxalyl chloride (2.0 M in methylene chloride, 2.42 ml, 0.00484 mol) is added. The resultant mixture is stirred one hour on an ice bath and concentrated *in vacuo*. The residue is taken up in tetrahydrofuran. The resultant solution is added dropwise to a mixture of methyl zinc chloride (2.0 M, 0.970 ml, 0.00194 mol) in tetrahydrofuran, followed by addition of palladium tetrakistriphenylphosphine (0.220 g, 0.000190 mol). The resultant mixture is stirred overnight at room temperature, quenched with 10% hydrochloric acid, and extracted with ethyl acetate. The organic layer is washed with saturated sodium bicarbonate and brine, dried over anhydrous magnesium sulfate, filtered, and concentrated *in vacuo* to an orange solid. The solid is chromatographed on silica gel to afford the title compound as an off-white solid (0.270 g, 65.7%, mp 195-196 °C).

### EXAMPLE 172

### Preparation of 5-[3,6-Dihydro-3-methyl-2,6-dioxo-4-(trifluoromethyl)-1(2H)-pyrimidinyl]-α,α-dimethyl-1,2-benzisothiazole-3-acetamide

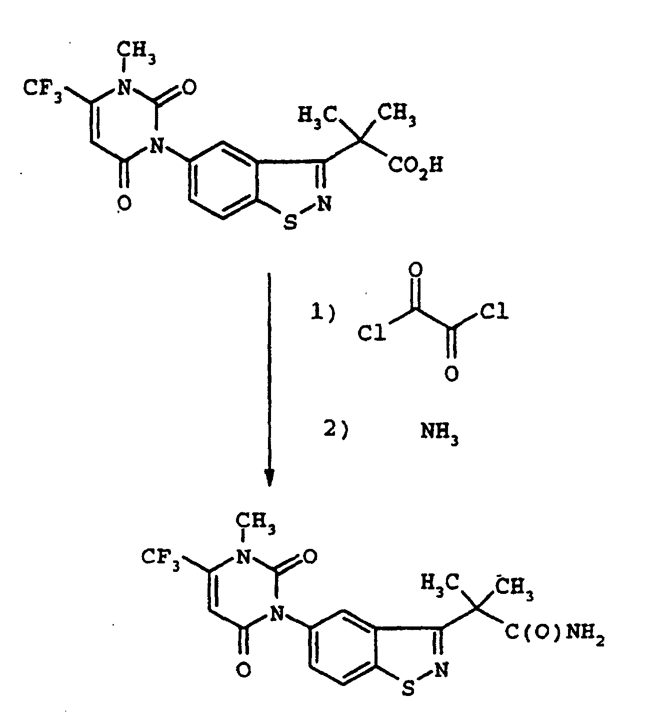

N,N-Dimethylformamide (8 drops) is added to a mixture of 5-[3,6-dihydro-3-methyl-2,6-dioxo-4-(trifluoromethyl)-1(2H)-pyrimidinyl]-α,α-dimethyl-1,2-benzisothiazole-3-acetic acid (2.00 g, 0.00484 mol) and methylene chloride. The mixture is cooled on an ice bath and oxalyl chloride (2.0 M in methylene chloride, 6.05 ml, 0.0121 mol) is added. The resultant mixture is stirred one hour on an ice bath and concentrated *in vacuo.* The residue is taken up in tetrahydrofuran. The resultant solution is saturated with ammonia gas followed by the addition of triethylamine. The mixture is filtered and the filtrate is concentrated *in vacuo* to a solid. The solid is recrystallized from methylene chloride:hexanes to afford the title compound as a tan solid (1.65 g, 82.7%, mp 192-196 °C) which is identified by NMR and IR spectral analyses.

### EXAMPLE 173

### Preparation of 5-[3,6-Dihydro-3-methyl-2,6-dioxo-4-(trifluoromethyl)-1(2H)-pyrimidinyl]-α,α-dimethyl-N-(methylsulfonyl)-1,2-benzisothiazole-3-acetamide

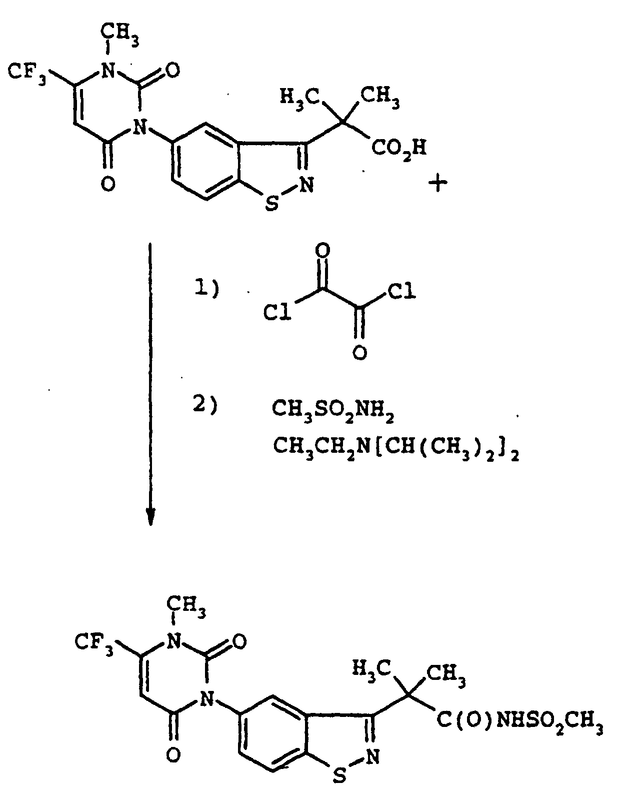

N,N-Dimethylformamide (8 drops) is added to a mixture of 5-[3,6-dihydro-3-methyl-2,6-dioxo-4-(trifluoromethyl)-1(2H)-pyrimidinyl]-α,α-dimethyl-1,2-benzisothiazole-3-acetic acid (2.00 g, 0.00484 mol) and methylene chloride. The mixture is cooled on an ice bath and oxalyl chloride (2.0 M in methylene chloride, 6.05 ml, 0.0121 mol) is added. The resultant mixture is stirred one hour on an ice bath and concentrated *in vacuo.* The residue is taken up in tetrahydrofuran. Methylsulfonamide (0.690 g, 0.00726 mol) is added, followed by ethyldiisopropylamine (0.940 g, 0.00727 mol) and dimethylaminopyridine (0.100 g). The resultant mixture is stirred at room temperature and filtered. The filtrate is concentrated *in vacuo* and the residue is filtered through silica gel with hexanes:ethyl acetate. The filtrate is concentrated *in vacuo* to a tan solid. The solid is suspended in methylene chloride, stirred, and refiltered to give the title compound as a white solid (0.320 g, 13.5%, mp 264-265 °C).

Using essentially the same procedure, the following compounds are obtained:

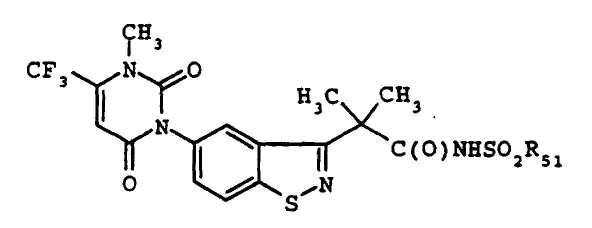

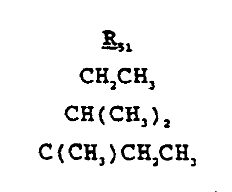

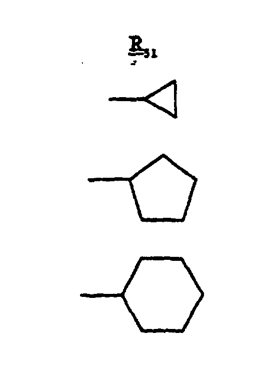

### EXAMPLE 174

### Preparation of {{{5-[3,6-Dihydro-3-methyl-2,6-dioxo-4-(trifluoromethyl)-1(2H)-pyrimidinyl]-1,2-benzisothiazol-3-yl}methyl}sulfonyl}acetic acid

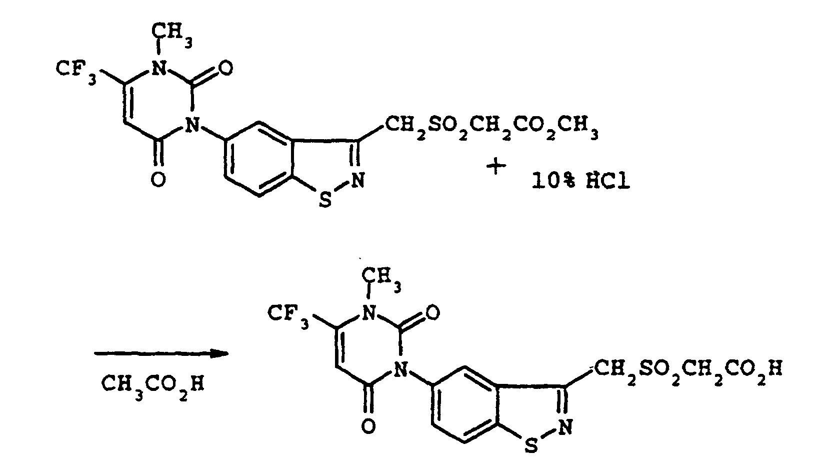

A mixture of methyl {{{5-[3,6-dihydro-3-methyl-2,6-dioxo-4-(trifluoromethyl)-1(2H)-pyrimidinyl]-1,2-benzisothiazol-3-yl}methyl}sulfonyl}acetate (1.00 g, 0.00210 mol), acetic acid and 10% hydrochloric acid (2.00 ml) is stirred overnight at 60 °C. Additional hydrochloric acid (37%, 1.20 ml) is added and the mixture is stirred 5 hours at 80 °C. The mixture is cooled to room temperature, poured into ice water with stirring, and filtered to give the title compound as a white solid (0.760 g, 78.4%, mp 215 °C) which is identified by NMR spectral analysis.

Using essentially the same procedure {{{5-[3,6-dihydro-3-methyl-2,6-dioxo-4-(trifluoromethyl)-1(2H)-pyrimidinyl]-1,2-benzisothiazol-3-yl}methyl}thio}acetic acid is obtained from its methyl ester.

### EXAMPLE 175

### Preparation of Methyl {{{5-[3,6-dihydro-3-methyl-2,6-dioxo-4-(trifluoromethyl)-1(2H)-pyrimidinyl]-1,2-benzisothiazol-3-yl}methyl}sulfonyl}acetate

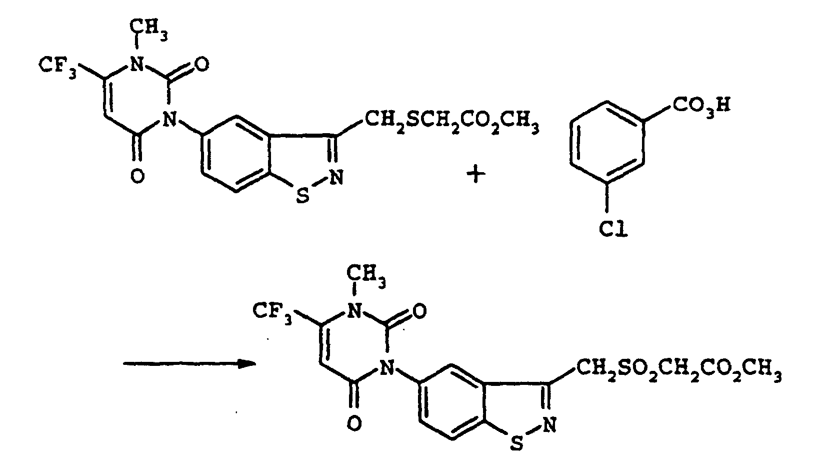

A solution of meta-chloroperbenzoic acid (70% purity, 1.91 g, 0.00774 mol) in methylene chloride is added to a solution of methyl {{{5-[3,6-dihydro-3-methyl-2,6-dioxo-4-(trifluoromethyl)-1(2H)-pyrimidinyl]-1,2-. benzisothiazol-3-yl]methyl}thio]acetate (1.00 g, 0.00225 mol) in methylene chloride at 0 °C. When the reaction is complete by thin layer chromatography analysis, the mixture is washed sequentially with 5% sodium carbonate and brine, and concentrated *in vacuo* to a white foam. The foam is triturated in ether to give the title compound as a white solid (0.500 g, 46.7%, mp 209-210 °C) which is identified by NMR spectral analysis.

### EXAMPLE 176

### Preparation of N-{{5-[3,6-Dihydro-3-methyl-2,6-dioxo-4-(trifluoromethyl)-1(2H)-pyrimidinyl]-1,2-benzisothiazol-3-yl}methyl}-N-(methylsulfonyl)-glycine, methyl ester

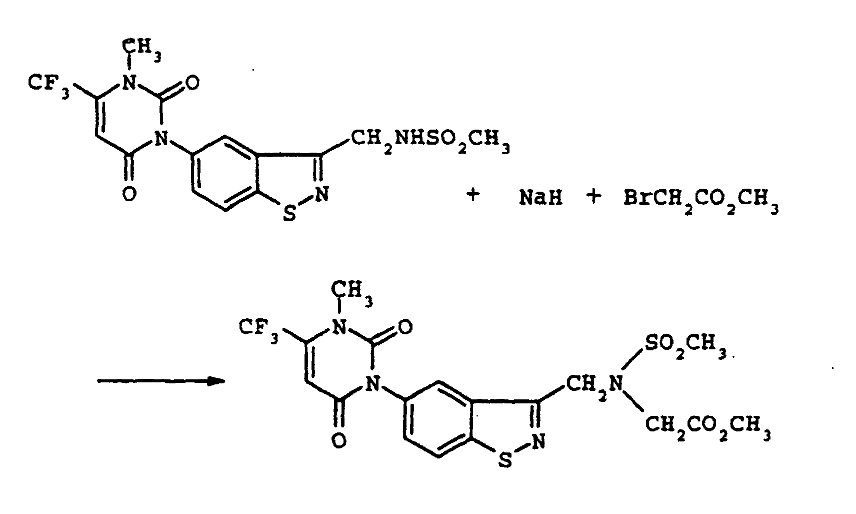

To a mixture of 2-{{5-[3,6-dihydro-3-methyl-2,6-dioxo-4-(trifluoromethyl)-1(2H)-pyrimidinyl]-1,2-benzisothiazol-3-yl}methoxy}-N-(methylsulfonyl)-acetamide (0.500 g, 0.00115 mol) and tetrahydrofuran is added methyl bromoacetate (0.130 ml, 0.00138 mol) followed by sodium hydride (60%, 0.0690 g, 0.00173 mol). The resultant mixture is poured into water and extracted with methylene chloride. The combined organic layers are dried over anhydrous magnesium sulfate, filtered, and concentrated *in vacuo* to an oil. The oil is triturated in ether to give the title compound as a white solid (0.270 g, 46.4%, mp 135-136 °C) which is identified by NMR spectral analysis.

### EXAMPLE 177

### Preparation of 3-[3-[(Ethoxymethoxy)methyl]-1,2-benzisothiazol-5-yl]-1-methyl-6-(trifluoromethyl)-2,4(1H,3H)-pyrimidinedione

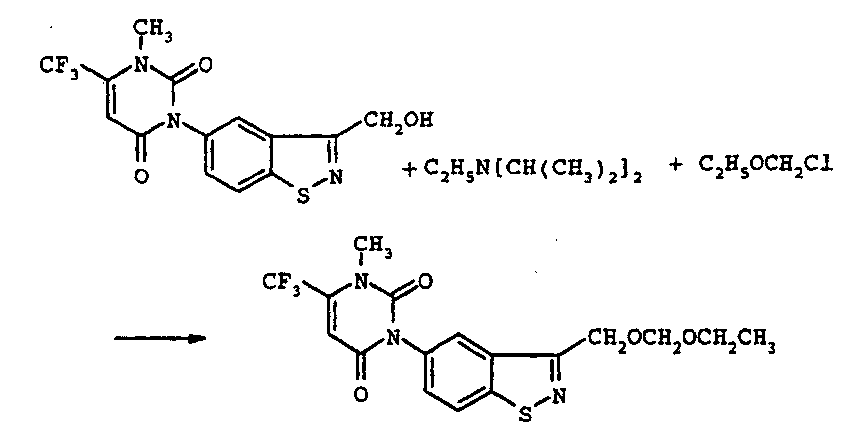

To a mixture of 3-[3-(hydroxymethyl)-1,2-benzisothiazol-5-yl]-1-methyl-6-(trifluoromethyl)-2,4(1H,3H)-pyrimidinedione (0.800 g, 0.00224 mol) and methylene chloride is added ethyldiisopropylamine (0.780 ml, 0.00448 mol) followed by chloromethyl ethyl ether (0.310 ml, 0.00336 mol). The resultant mixture is stirred 42.5 hours at room temperature, diluted with ether, washed sequentially with 1 M aqueous hydrochloric acid, saturated sodium bicarbonate and brine, dried over anhydrous magnesium sulfate, filtered, and concentrated *in vacuo* to a yellow syrup. The syrup is chromatographed on silica gel with hexanes:ethyl acetate to give the product as a white solid which is identified by NMR spectral analysis.

Following essentially the same procedure, but using the appropriate alkylating agent, the following compounds are obtained:

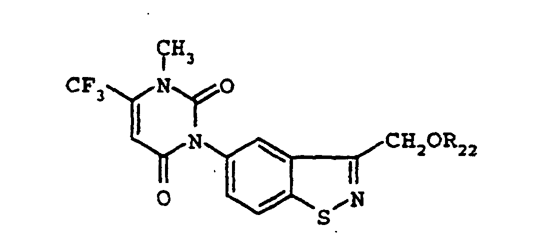

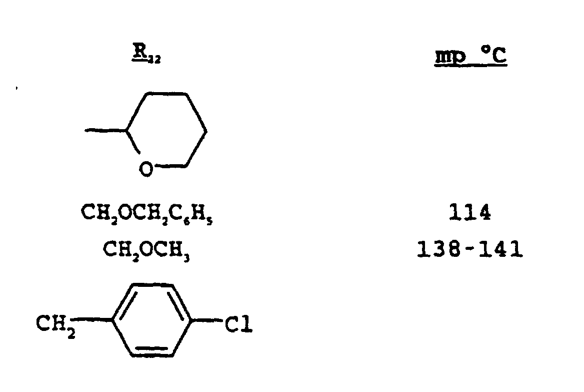

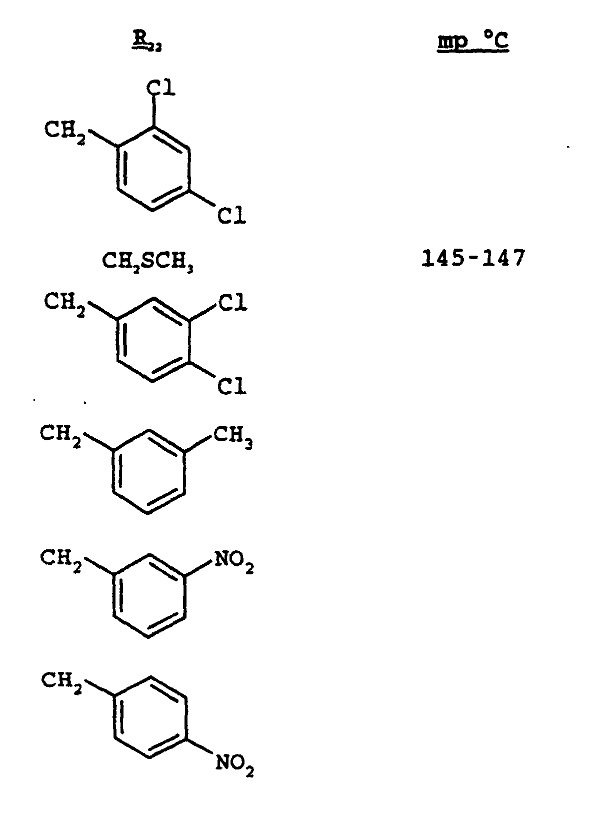

### EXAMPLE 178

### Preparation of {{5-[3,6-Dihydro-3-methyl-2,6-dioxo-4-(trifluoromethyl)-1(2H)-pyrimidinyl]-1,2-benzisothiazol-3-yl}methoxy}acetic acid

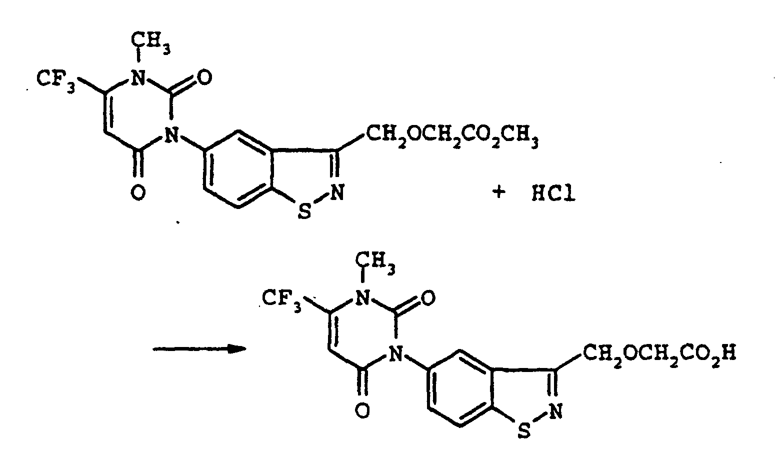

To a mixture of {{5-[3,6-dihydro-3-methyl-2,6-dioxo-4-(trifluoromethyl)-1(2H)-pyrimidinyl]-1,2-benzisothiazol-3-yl}methoxy}acetic acid, methyl ester (4.19 g, 0.00977 mol) and acetic acid is added 37% hydrochloric acid (5.00 ml). The resultant mixture is stirred overnight at 65°C and cooled to room temperature. The mixture is concentrated *in vacuo.* The residue is suspended in water and quenched slowly by the addition of solid sodium bicarbonate to neutral pH. The resultant mixture is partitioned between ether and saturated sodium bicarbonate, and filtered. The filtrate is washed with methylene chloride, acidified with hydrochloric acid, and filtered to give the title product as a white solid (2.87 g, 70.7%, mp 191-192 °C) which is identified by NMR spectral analysis.

### EXAMPLE 179

### Preparation of 2-{{5-[3,6-Dihydro-3-methyl-2,6-dioxo-4-(trifluoromethyl)-1(2H)-pyrimidinyl]-1,2-benzisothiazol-3-yl}methoxy}-N-(methylsulfonyl)acetamide, dimethylamine salt

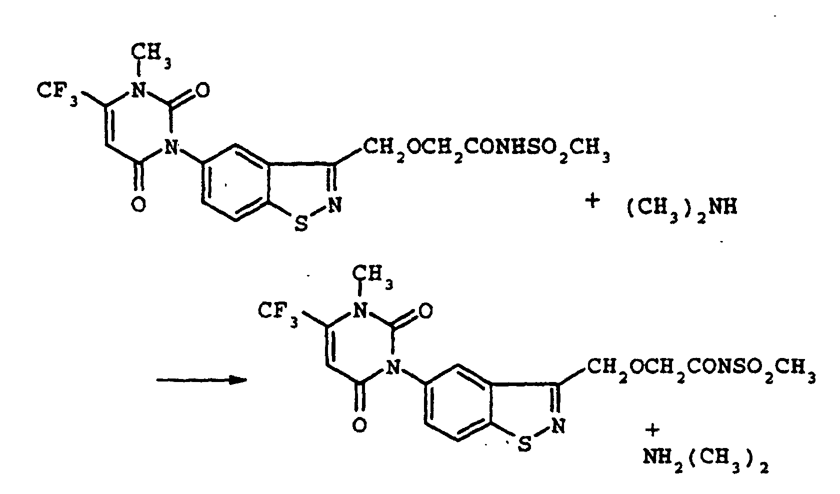

Dimethylamine (2.0 M in tetrahydrofuran, 1.04 ml, 0.00208 mol) is added to a solution of 2-{{5-[3,6-dihydro-3-methyl-2,6-dioxo-4-(trifluoromethyl)-1(2H)-pyrimidinyl]-1,2-benzisothiazol-3-yl}methoxy}-N-(methylsulfonyl)acetamide (0.510 g, 0.00104 mol) in tetrahydrofuran. The resultant mixture is stirred 5 minutes and concentrated *in vacuo.* The residue is taken up in tetrahydrofuran and crystallized by the addition of ether. The resultant off-white solid (0.420 g, 75.1%, mp 102-107 °C) is identified as the title compound by NMR spectral analysis.

Using essentially the same procedure on 2-{{{5-[3,6-dihydro-3-methyl-2,6-dioxo-4-(trifluoromethyl)-1(2H)-pyrimidinyl]-1,2-benzisothiazol-3-yl}methyl}thio}-N-(methylsulfonyl)acetamide; 2-{{{5-[3,6-dihydro-3-methyl-2,6-dioxo-4-(trifluoromethyl)-1(2H)-pyrimidinyl]-1,2-benzisothiazol-3-yl}methyl}thio}-N-(methylsulfonyl)-acetamide, dimethylamine salt is obtained.

### EXAMPLE 180

### Preparation of {{5-[3,6-Dihydro-3-methyl-2,6-dioxo-4-(trifluoromethyl)-1(2H)-pyrimidinyl]-1,2-benzisothiazol-3-yl}methoxy}acetic acid, dimethylamine salt

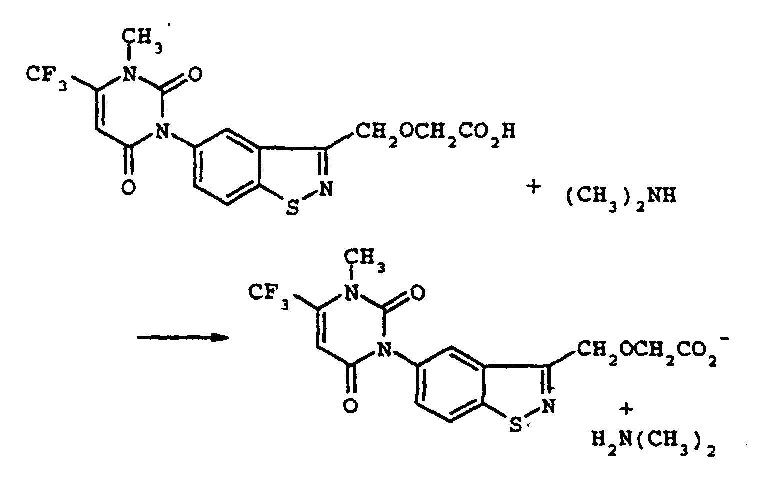

Dimethylamine in tetrahydrofuran (2.0 M, 1.23 ml, 0.00246 mol) is added to a solution of {{5-[3,6-dihydro-3-methyl-2,6-dioxo-4-(trifluoromethyl)-1(2H)-pyrimidinyl]-1,2-benzisothiazol-3-yl}methoxy}acetic acid (0.510 g, 0.00123 mol) in tetrahydrofuran. The mixture is stirred 15 minutes, diluted with ether and the resultant off-white solid is isolated (0.420 g, 74.2%, mp 175-176 °C) and identified as the title compound by NMR spectral analysis.

Using essentially the same procedure on {{{5-[3,6-dihydro-3-methyl-2,6-dioxo-4-(trifluoromethyl)-1(2H)-pyrimidinyl]-1,2-benzisothiazol-3-yl}methyl}thio}acetic acid; {{{5-[3,6-dihydro-3-methyl-2,6-dioxo-4-(trifluoromethyl)-1(2H)-pyrimidinyl]-1,2-benzisothiazol-3-yl}methyl}thio}acetic acid, dimethylamine salt is obtained.

### EXAMPLE 181

### Preparation of 2-{{5-{3,6-Dihydro-3-methyl-2,6-dioxo-4-(trifluoromethyl)-1(2H)-pyrimidinyl]-1,2-benzisothiazol-3-yl}methoxy-N-(methylsulfonyl)acetamide

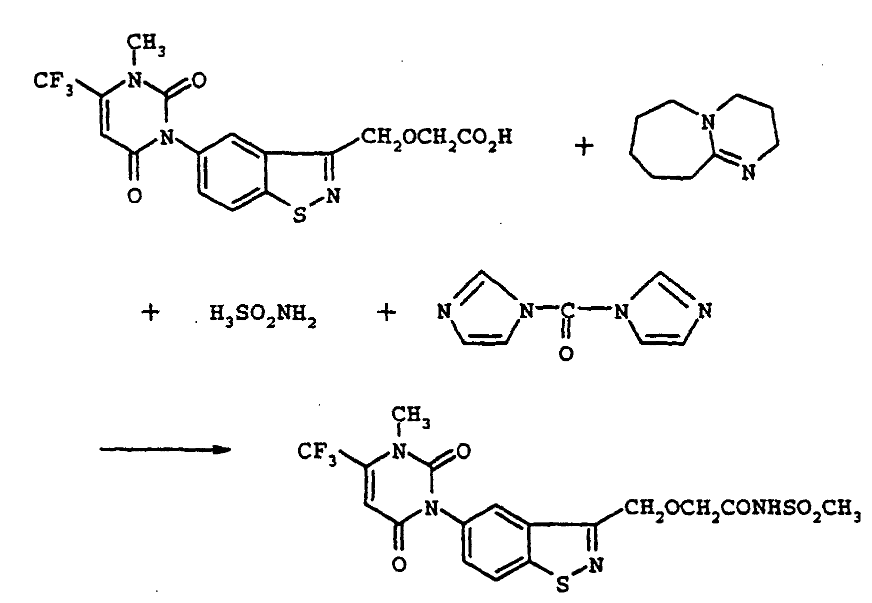

1,1'-Carbonyldiimidazole (1.17 g, 0.00722 mol) is added to a solution of {{5-[3,6-dihydro-3-methyl-2,6-dioxo-4-(trifluoromethyl)-1(2H)-pyrimidinyl]-1,2-benzisothiazol-3-yl}methoxy}acetic acid (1.50 g, 0.00361 mol) in tetrahydrofuran. The mixture is stirred one hour at reflux and cooled to room temperature. Methylsulfonamide (0.755 g, 0.00794 mol) is added, followed by 1,8-diazabicyclo[5.4.0]undec-7-ene (1.19 ml, 0.00794 mol). The resultant mixture is stirred overnight at room temperature, poured into 10% hydrochloric acid, and filtered. The resultant white solid is triturated in tetrahydrofuran, and filtered to give the title compound as an off-white solid (1.00 g, 56.2%, mp >240 °C) which is identified by NMR spectral analysis.

Using essentially the same procedure, 2-{{{5-[3,6-dihydro-3-methyl-2,6-dioxo-4-(trifluoromethyl)-1(2H)-pyrimidinyl]-1,2-benzisothiazol-3-yl}methyl}thio}-N-(methylsulfonyl)acetamide is obtained from {{{5-[3,6-dihydro-3-methyl-2,6-dioxo-4-(trifluoromethyl)-1(2H)-pyrimidinyl]-1,2-benzisothiazol-3-yl}methyl}sulfonyl}acetic acid.

### EXAMPLE 182

### Preparation of 3-{3-[Bromo(phenylsulfonyl)methyl]-1,2-benzisothiazol-5-yl}-1-methyl-6-(trifluoromethyl)-2,4(1H,3H)-pyrimidinedione

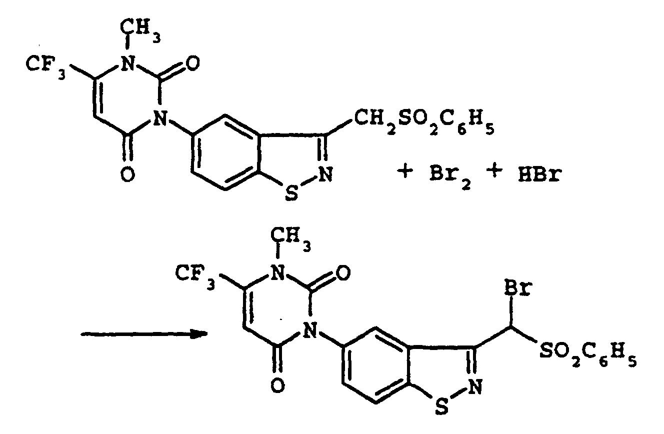

To a solution of 3-[3-(phenylsulfonyl)methyl-1,2-benzisothiazol-5-yl)-1-methyl-6-(trifluoromethyl)-2,4(1H,3H)-pyrimidinedione (0.400 g, 0.000831 mol) in ethyl acetate is added 48% hydrobromic acid (4 drops) followed by bromine in ethyl acetate (0.522 M, 1.67 ml, 0.000872 mol). The resultant mixture is stirred 1.5 hours at reflux. Additional bromine in ethyl acetate (1.67 ml) is added and the resultant mixture is stirred one hour at reflux. The mixture is cooled to room temperature, diluted with ethyl acetate, washed sequentially with 0.1 M sodium sulfite, saturated sodium bicarbonate and brine, dried over anhydrous magnesium sulfate, filtered, and concentrated *in vacuo.* The residue is chromatographed on silica gel with hexanes: ethyl acetate to give the title compound as a white crystalline solid (0.420 g, 90.1%, mp 182-183 °C) which is identified by NMR spectral analysis.

### EXAMPLE 183

### Preparation of Methyl 5-[3,6-dihydro-3-methyl-2,6-dioxo-4-(trifluoromethyl)-1(2H)-pyrimidinyl]-α-ethyl-1,2-benzisothiazole-3-acetate

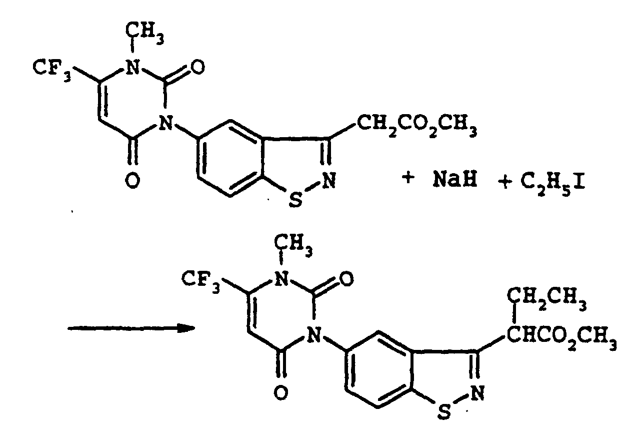

To a mixture of methyl 5-[3,6-dihydro-3-methyl-2,6-dioxo-4-(trifluoromethyl)-1(2H)-pyrimidinyl]-1,2-benzisothiazole-3-acetate (0.400 g, 0.00100 mol), N,N-dimethylformamide and ethyl iodide (0.980 g, 0.00628 mol) is added sodium hydride (60%, 0.0900 g, 0.00225 mol) in portions. The mixture is stirred 5 days at room temperature, diluted with methylene chloride, washed sequentially with 8% hydrochloric acid, water and brine, dried over anhydrous magnesium sulfate, filtered, and concentrated *in vacuo.* The residue is chromatographed on silica gel with ether:hexanes:methylene chloride to give the title compound (0.260 g, 60.9%) which is identified by NMR spectral analysis.

Following essentially the same procedure, but using the appropriate alkylating agent, the following compounds are obtained:

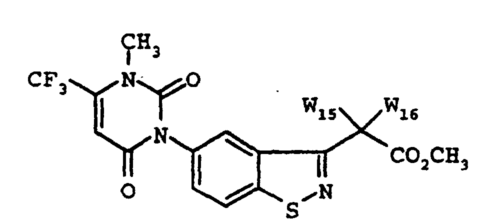

| **W**_{**15**} | **W**_{**16**} |
|---|---|
| H | CH₂CH=CH₂ |
| CH₂CH=CH₂ | CH₂CH=CH₂ |
| H | CH₂C≡CH |
| CH₂C≡CH | CH₂C≡CH |
| H | CH₂C₆H₅ |
| CH₂C₆H₅ | CH₂C₆H₅ |
| -CH₂CH₂- | |

### EXAMPLE 184

### Preparation of 1-Methyl-3-{3-[1-(methylsulfonyl)-ethyl]-1,2-benzisothiazol-5-yl}-6-(trifluoromethyl)-2,4(1H,3H)-pyrimidinedione

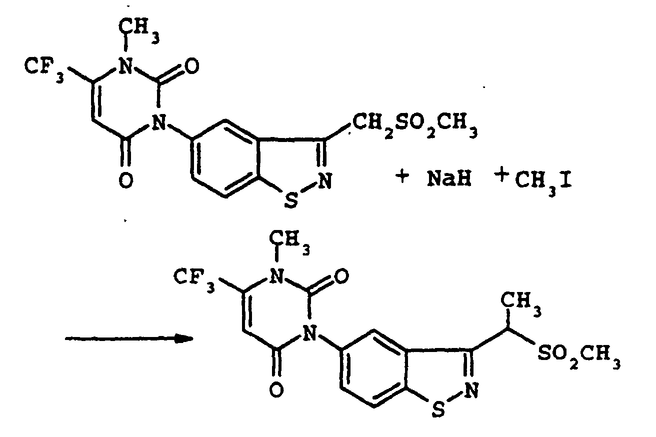

To a suspension of 1-methyl-3-{3-[(methylsulfonyl)-methyl]-1,2-benzisothiazol-5-yl}-6-(trifluoromethyl)-2,4(1H,3H)-pyrimidinedione (1.00 g, 0.00238 mol) in tetrahydrofuran is added sodium hydride (60%, 0.120 g, 0.00300 mol). The resultant mixture is stirred one hour at room temperature and treated with iodomethane (0.220 ml, 0.00360 mol). The mixture is stirred two hours at room temperature, partially concentrated *in vacuo,* diluted with water, and extracted with ethyl acetate. The organic layer is washed with water, filtered through anhydrous magnesium sulfate and concentrated *in vacuo* to a brown foam. The foam is chromatographed on silica gel with methylene chloride/ether to give the title compound as an off-white solid (0.420 g, 40.4%) which is identified by NMR spectral analysis.

Following essentially the same procedure, but using the appropriate alkylating agent, the following compounds are obtained:

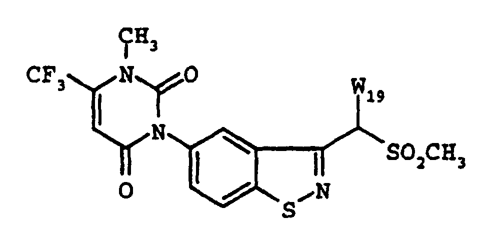

| **W**_{**19**} |
|---|
| CH₂CH=CH₂ |
| CH₂C≡CH |
| CH₂C₆H₅ |

### Example 185

### Postemergence herbicidal evaluation of test compounds

The postemergence herbicidal activity of the compounds of the present invention is demonstrated by the following tests wherein a variety of dicotyledonous and monocotyledonous plants are treated with test compounds. In the tests, seedling plants are grown in jiffy flats for about two weeks. The test compounds are dispersed in 50/50 acetone/water mixtures containing 0.5 % TWEEN®20, a polyoxyethylene sorbitan monolaurate surfactant of Atlas Chemical Industries, in sufficient quantities to provide the equivalent of about 0.0157 kg to 0.500 kg per hectare of active compound when applied to the plants through a spray nozzle operating at 275,8 kilopascals (40 psi) for a predetermined time. After spraying, the plants are placed on greenhouse benches and are cared for in accordance with conventional greenhouse procedures. From four to five weeks after treatment, the seedling plants are examined and rated according to the rating system set forth below. Data obtained are reported in Table I below. Where more than one test is involved for a given compound, the data are averaged.

Plant species employed in these evaluations are reported by header abbreviation, common name and scientific name.

Compounds employed in this postemergence herbicidal evaluation are given a compound number and identified by name. Data in Table I are reported by compound number.

### Herbicide Rating Scale

Results of herbicide evaluation are expressed on a rating scale (0-9). The scale is based upon a visual observation of plant stand, vigor, malformation, size, chlorosis and overall plant appearance as compared with a control.

| **Rating** | **Meaning** | **% Control** **Compared to Check** |
|---|---|---|
| 9 | Complete kill | 100 |
| 8 | Approaching Complete Kill | 91-99 |
| 7 | Good Herbicidal Effect | 80-90 |
| 6 | Herbicidal Effect | 65-79 |
| 5 | Definite Injury | 45-64 |
| 4 | Injury | 30-44 |
| 3 | Moderate Effect | 16-29 |
| 2 | Slight Effect | 6-15 |
| 1 | Trace Effect | 1-5 |
| 0 | No Effect | 0 |

A "blank space" indicates that no evaluation was conducted.

| **PLANT SPECIES EMPLOYED IN HERBICIDAL EVALUATIONS** | | |
|---|---|---|
| **Header Abbr.** | **Common Name** | **Scientific Name** |
| ABUTH | Velvetleaf | *Abutilon theophrasti,* Medic. |
| | | |
| AMBEL | Ragweed, Common | *Ambrosia artemisifolia,* L. |
| | | |
| CASOB | Sicklepod | *Cassia obtusifolia,* L. |
| | | |
| CHEAL | Lambsquarters, Common | *Chenopodium album,* L. |
| | | |
| GALAP | Galium | *Galium aparine* |
| | | |
| IPOHE | Morningglory, Ivyleaf | *Ipomoea hederacea,* (L.) Jacq. |
| | | |
| IPOSS | Morningglory Spp. | *Ipomoea* Spp. |
| | | |
| ECHCG | Barnyardgrass | *Echinochloa crus-galli,* (L.) Beau |
| | | |
| SETVI | Foxtail, Green | *Setaria viridis,* (L.) Beau |
| | | |
| GLXMAW | Soybean, Williams | *Glycine max,* (L.) Merr. cv Williams |
| | | |
| GLXMA | Soybean | *Glycine max,* (L.) Merr. |
| | | |
| ORYSAT | Rice, Tebonnet | *Oryza sativa,* (L.) Tebonnet |
| | | |
| TRZAWR | Wheat, Winter, cv Riband | *Triticum aestivum,* cv Riband |
| | | |
| ZEAMX | Corn, Field | *Zea mays,* L. |

| **COMPOUNDS EVALUATED AS HERBICIDAL AGENTS** | |
|---|---|
| **Compound Number** | |
| 1 | 3-[3-(6-Methoxy-*m*-tolyl)-1,2-benzisothiazol-5-yl]-1-methyl-6-(trifluoromethyl)-2,4(1H,3H)-pyrimidinedione |
| 2 | Methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}acetate |
| 3 | Methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate |
| 4 | 1-Methyl-3-(3-phenyl-1,2-benzisothiazol-5-yl)-6-(trifluoromethyl)-2,4(1H,3H)-pyrimidinedione |
| 5 | 3-[3-(*p*-Ethylphenyl)-1,2-benzisothiazol-5-yl]-1-methyl-6-(trifluoromethyl)-2,4(1H,3H)-pyrimidinedione |
| 6 | 1-Methyl-3-(3-methyl-1,2-benzisothiazol-5-yl)-6-(trifluoromethyl)-2,4(1H,3H)-pyrimidinedione |
| 7 | 3-[3-(2-Methoxy-*p*-tolyl)-1,2-benzisothiazol-5-yl]-1-methyl-6-(trifluoromethyl)-2,4(1H,3H)-pyrimidinedione |
| 8 | Methyl {{6-{5-[3,6-dihydro-3-methyl-4-(trifluoromethyl)-2,6-dioxo-1(2H)-pyrimidinyl]-1,2-benzisothiazol-3-yl}-*m*-tolyl}oxy}acetate |
| 9 | Methyl 2-{{6-{5-[3,6-dihydro-3-methyl-4-(trifluoromethyl)-2,6-dioxo-1(2H)-pyrimidinyl]-1,2-benzisothiazol-3-yl}-*m*-tolyl}oxy}propionate |
| 10 | 2-[3-(6-Methoxy-3,4-xylyl)-1,2-benzisothiazol-5-yl]-1-methyl-6-(trifluoromethyl)-2,4(1H,3H)-pyrimidinedione |
| 11 | 3-[3-(6-Hydroxy-3,4-xylyl)-1,2-benzisothiazol-5-yl]-1-methyl-6-(trifluoromethyl)-2,4(1H,3H)-pyrimidinedione |
| 12 | Methyl {{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}acetate |
| 13 | Methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate |
| 14 | Methyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate, (R)- |
| 15 | Methyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate, (S)- |
| 16 | 3-[3-(Bromomethyl)-1,2-benzisothiazol-5-yl]-1-methyl-6-(trifluoromethyl)-2,4(1H,3H)-pyrimidinedione |
| 17 | 3-[3-(Dibromomethyl)-1,2-benzisothiazol-5-yl]-1-methyl-6-(trifluoromethyl)-2,4(1H,3H)-pyrimidinedione |
| 18 | 3-[3-(2-Methoxy-3,5-xylyl)-1,2-benzisothiazol-5-yl]-1-methyl-6-(trifluoromethyl)-2,4(1H,3H)-pyrimidinedione |
| 19 | 3-(3-Isopropoxy-1,2-benzisoxazol-5-yl)-1-methyl-6-(trifluoromethyl)-2,4(1H,3H)-pyrimidinedione |
| 20 | 3-(3-Chloro-1,2-benzisoxazol-5-yl)-1-methyl-6-(trifluoromethyl)-2,4(1H,3H)-pyrimidinedione |
| 21 | 1-Methyl-3-(3-methyl-1,2-benzisoxazol-5-yl)-6-(trifluoromethyl)-2,4(1H,3H)-pyrimidinedione |
| 22 | Methyl {{5-[3,6-dihydro-3-methyl-2,6-dioxo-4-(trifluoromethyl)-1(2H)-pyrimidinyl]-1,2-benzisoxazol-3-yl}oxy}acetate |
| 23 | 3-(6-Fluoro-3-methyl-1,2-benzisoxazol-5-yl)-1-methyl-6-(trifluoromethyl)-2,4(1H,3H)-pyrimidinedione |
| 24 | 3-[3-(6-Methoxy-*m*-tolyl)-1,2-benzisothiazol-5-yl]-1,6-dimethyl-2,4(1H,3H)-pyrimidinedione |
| 25 | 1-Methyl-3-(3-phenyl-1,2-benzisoxazol-5-yl)-6-(trifluoromethyl)-2,4(1H,3H)-pyrimidinedione |
| 26 | Methyl {{6-{5-[3,6-dihydro-3-methyl-2,6-dioxo-4-(trifluoromethyl)-1(2H)-pyrimidinyl]-1,2-benzisothiazol-3-yl}-2,4-xylyl}oxy}acetate |
| 27 | Methyl 2-{{6-{5-[3,6-dihydro-3-methyl-2,6-dioxo-4-(trifluoromethyl)-1(2H)-pyrimidinyl]-1,2-benzisothiazol-3-yl}-2,4-xylyl}oxy}propionate |
| 28 | Methyl 2-{2-{5-[3,6-dihydro-3-methyl-2,6-dioxo-4-(trifluoromethyl)-1(2H)-pyrimidinyl]-1,2-benzisothiazol-3-yl}-4-ethylphenoxy}propionate |
| 29 | 1-Ethyl-3-[3-(6-methoxy-*m*-tolyl)-1,2-benzisothiazol-5-yl]-6-(trifluoromethyl)-2,4(1H,3H)-pyrimidinedione |
| 30 | Methyl 2-{2-{5-[3,6-dihydro-3-methyl-4-(trifluoromethyl)-2,6-dioxo-1(2H)-pyrimidinyl]-1,2-benzisothiazol-3-yl}-4-isopropylphenoxy}propionate |
| 31 | 3-[3-(Methoxymethyl)-1,2-benzisothiazol-5-yl]-1-methyl-6-(trifluoromethyl)-2,4(1H,3H)-pyrimidinedione |
| 32 | 3-[3-(Hydroxymethyl)-1,2-benzisothiazol-5-yl]-1-methyl-6-(trifluoromethyl)-2,4(1H,3H)-pyrimidinedione, acetate ester |
| 33 | Methyl 2-{{2-{5-[3,6-dihydro-3-methyl-2-oxo-6-thioxo-4-(trifluoromethyl)-1(2H)-pyrimidinyl]-1,2-benzisothiazol-3-yl}-*p*-tolyl}oxy}propionate |
| 34 | 3-[3-(6-Methoxy-2,3,4-trimethylphenyl)-1,2-benzisothiazol-5-yl]-1-methyl-6-(trifluoromethyl)-2,4(1H,3H)-pyrimidinedione |
| 35 | 3-(1,2-Benzisothiazol-5-yl)-1-methyl-6-(trifluoromethyl)-2,4(1H,3H)-pyrimidinedione |
| 36 | 3-(3-Bromo-1,2-benzisothiazol-5-yl)-1-methyl-6-(trifluoromethyl)-2,4(1H,3H)-pyrimidinedione |
| 37 | 3-(3-Chloro-1,2-benzisothiazol-5-yl)-1-methyl-6-(trifluoromethyl)-2,4(1H,3H)-pyrimidinedione |
| 38 | Methyl {{2-[5-(3,6-dihydro-3,4-dimethyl-2,6-dioxo-1(2H)-pyrimidinyl)-1,2-benzisothiazol-3-yl]-*p*-tolyl}oxy}acetate |
| 39 | Methyl 2-{{2-[5-(3,6-dihydro-3,4-dimethyl-2,6-dioxo-1(2H)-pyrimidinyl)-1,2-benzisothiazol-3-yl]-*p*-tolyl}oxy}propionate |
| 40 | 3-[3-(6-Methoxy-*m*-tolyl)-1,2-benzisoxazol-5-yl]-1-methyl-6-(trifluoromethyl)-2,4(1H,3H)-pyrimidinedione |
| 41 | 3-[4-Chloro-3-(6-hydroxy-*m*-tolyl)-1,2-benzisoxazol-5-yl]-1-methyl-6-(trifluoromethyl)-2,4(1H,3H)-pyrimidinedione |
| 42 | 3-(3-Methoxy-1,2-benzisoxazol-5-yl)-1-methyl-6-(trifluoromethyl)-2,4(1H,3H)-pyrimidinedione |
| 43 | 3-[3-(*o*-Methoxyphenoxy)-1,2-benzisoxazol-5-yl]-1-methyl-6-(trifluoromethyl)-2,4(1H,3H)-pyrimidinedione |
| 44 | 1-Methyl-3-(2-methyl-3-oxo-1,2-benzisoxazolin-5-yl)-6-(trifluoromethyl)-2,4(1H,3H)-pyrimidinedione |
| 45 | 3-[3-(5-Ethyl-2-methoxyphenyl)-1,2-benzisothiazol-5-yl]-1-methyl-6-(trifluoromethyl)-2,4(1H,3H)-pyrimidinedione |
| 46 | 3-[3-(5-Isopropyl-2-methoxyphenyl)-1,2-benzisothiazol-5-yl]-1-methyl-6-(trifluoromethyl)-2,4(1H,3H)-pyrimidinedione |
| 47 | Methyl 2-{*o*-{{5-[3,6-dihydro-3-methyl-2,6-dioxo-4-(trifluoromethyl)-1(2H)-pyrimidinyl]-1,2-benzisoxazol-3-yl}oxy}phenoxy}propionate |
| 48 | Methyl {*o*-{{5-[3,6-dihydro-3-methyl-2,6-dioxo-4-(trifluoromethyl)-1(2H)-pyrimidinyl]-1,2-benzisoxazol-3-yl}oxy}phenoxy}acetate |
| 49 | Ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 50 | Isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 51 | 2-Propynyl {{2-{5-[3,6-dihydro-3-methyl-2,6-dioxo-4-(trifluoromethyl)-1(2H)-pyrimidinyl]-1,2-benzisothiazol-3-yl}-p-tolyl}oxy}acetate |
| 52 | 1-Methyl-3-{3-{6-[(morpholinocarbanyl)methoxy]-*m*-tolyl}-1,2-benzisothiazol-5-yl}-6-(trifluoromethyl)-2,4(1H,3H)-pyrimidinedione |
| 53 | 2-Morpholinoethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 54 | 1-Methyl-3-{3-{6-{[(4-phenyl-1-piperazinyl)-carbonyl]methoxy}-*m*-tolyl}1,2-benzisothiazol-5-yl}-6-(trifluoromethyl)-2,4(1H,3H)-pyrimidinedione |
| 55 | Methyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}butyrate |
| 56 | 1-Methyl-3-{3-[6-(2-propynyloxy)-*m*-tolyl]-1,2-benzisothiazol-5-yl}-6-(trifluoromethyl)-2,4(1H,3H)-pyrimidinedione |
| 57 | 3-{3-[6-(Cyanomethoxy)-*m*-tolyl]-1,2-benzisothiazol-5-yl}-1-methyl-6-(trifluoromethyl)-2,4(1H,3H)-pyrimidinedione |
| 58 | 3-{3-[6-(1-Cyanoethoxy)-m-tolyl]-1,2-benzisothiazol-5-yl}-1-methyl-6-(trifluoromethyl)-2,4(1H,3H)-pyrimidinedione |
| 59 | 3-[3-(6-Isopropoxy-*m*-tolyl)-1,2-benzisothiazol-5-yl]-1-methyl-6-(trifluoromethyl)-2,4(1H,3H)-pyrimidinedione |
| 60 | 3-{3-{6-[(Diethylcarbamoyl)methoxy]-*m*-tolyl}-1,2-benzisothiazol-5-yl}-1-methyl-6-(trifluoromethyl)-2,4(1H,3H)-pyrimidinedione |
| 61 | 3-{3-{6-[(Diisopropylcarbamoyl)methoxy]-*m*-tolyl}-1,2-benzisothiazol-5-yl}-1-methyl-6-(trifluoromethyl)-2,4(1H,3H)-pyrimidinedione |
| 62 | 3-{3-{6-[(1-Benzimidazolylcarbonyl)methoxy]-*m*-tolyl}-1,2-benzisothiazol-5-yl}-1-methyl-6-trifluoromethyl)-2,4(1H,3H)-pyrimidinedione |
| 63 | 3-{3-{6-{[(*m*-Cyanophenyl)carbamoyl]methoxy}-*m*-tolyl}-1,2-benzisothiazol-5-yl}-1-methyl-6-(trifluoromethyl)-2,4(1H,3H)-pyrimidinedione |
| 64 | {{2-{5-[3,6-Dihydro-3-methyl-2,6-dioxo-4-(trifluoromethyl)-1(2H)-pyrimidinyl]-1,2-benzisothiazol-3-yl}- *p* -tolyl}oxy}acetic acid |
| 65 | 3-{3-[6-(Allyloxy)-*m*-tolyl]-1,2-benzisothiazol-5-yl}-1-methyl-6-(trifluoromethyl)-2,4(1H,3H)-pyrimidinedione |
| 66 | 3-[3-(6-Ethoxy-*m*-tolyl)-1,2-benzisothiazol-5-yl]-1-methyl-6-(trifluoromethyl)-2,4(1H,3H)-pyrimidinedione |
| 67 | Ethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}butyrate |
| 68 | *tert* -Butyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 69 | Allyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 70 | 2-(Dimethylamino)ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 71 | 2-Chloroethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 72 | 2-Pyridylmethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 73 | 3-Phenyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 74 | 1-Methyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 75 | 2-Butynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 76 | 1-Methyl-2-butynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 77 | 2-Propynyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate |
| 78 | Allyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate |
| 79 | Furfuryl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate |
| 80 | 2-Butynyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate |
| 81 | 2-Chloroethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate |
| 82 | Isopropyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate |
| 83 | 1-Ethylpropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 84 | Cyclopentyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 85 | 1,2,2-Trimethylpropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 86 | 1,2-Dimethylpropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 87 | 2,2-Dimethylpropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 88 | 2-Butenyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate, (E)- or (Z)- |
| 89 | Cinnamyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxylacetate, (E)- or (Z)- |
| 90 | 2,2,2-Trifluoroethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 91 | *tert* -Butyl 2-{{2-{5-[3,6-dihydro-3-methyl-2,6-dioxo-4-(trifluoromethyl)-1(2R)-pyrimidinyl]-1,2-benzisothiazol-3-yl}-*p*-tolyl}oxy}propionate |
| 92 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}propionic acid |
| 93 | Methyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate |
| 94 | 1-Allyl-3-[3-(6-methoxy-*m*-tolyl)-1,2-benzisothiazol-5-yl]-6-(trifluoromethyl)-2,4(1H,3H)pyrimidinedione |
| 95 | 1-Isopropyl-3-[3-(6-methoxy-*m*-tolyl)-1,2-benzisothiazol-5-yl]-6-(trifluoromethyl)-2,4(1H,3H)-pyrimidinedione |
| 96 | Methyl {{2-{5-[3-ethyl-3,6-dihydro-2,6-dioxo-4-(trifluoromethyl)-1(2H)-pyrimidinyl]-1,2-benzisothiazol-3-yl}-*p*-tolyl}oxy}acetate |
| 97 | Methyl 2-{{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate |
| 98 | 1-Methyl-3-{3-[(methylthio)methyl]-1,2-benzisothiazol-5-yl}-6-(trifluoromethyl)-2,4(1H,3H)-pyrimidinedione |
| 99 | Methyl {{{5-[3,6-dihydro-3-methyl-2,6-dioxo-4-(trifluoromethyl)-1(2H)-pyrimidinyl]-1,2-benzisothiazol-3-yl}methyl}thio}acetate |
| 100 | 1-Methyl-3-{3-[(methylsulfonyl)methyl]-1,2-benzisothiazol-5-yl}-6-(trifluoromethyl)-2,4(1H,3H)-pyrimidinedione |
| 101 | 3-{3-[(Dimethylamino)methyl]-1,2-benzisothiazol-5-yl}-1-methyl-6-(trifluoromethyl)-2,4(1H,3H)-pyrimidinedione |
| 102 | Ethyl N-{{5-[3,6-dihydro-3-methyl-2,6-dioxo-4-(trifluoromethyl)-1(2H)-pyrimidinyl]-1,2-benzisothiazol-3-yl}methyl}-N-methylglycinate |
| 103 | 2-Propynyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate |
| 104 | Ethyl 5-[3,6-dihydro-3-methyl-2,6-dioxo-4-(trifluoromethyl)-1(2H)-pyrimidinyl]-1,2-benzisothiazole-3-acetate |
| 105 | 3-[3-(*o*-Methoxyphenyl)-1,2-benzisothiazol-5-yl]-1-methyl-6-(trifluoromethyl)-2,4(1H,3H)-pyrimidinedione |
| 106 | 3-[3-(Imidazol-1-ylmethyl)-1,2-benzisothiazol-5-yl]-1-methyl-6-(trifluoromethyl)-2,4(1H,3H)-pyrimidinedione |
| 107 | 1-Methyl-3-[3-(2-propynyloxy)-1,2-benzisothiazol-5-yl]-6-(trifluoromethyl)-2,4(1H,3H)-pyrimidinedione |
| 108 | {{5-[3,6-Dihydro-3-methyl-2,6-dioxo-4-(trifluoromethyl)-1(2H)-pyrimidinyl]-1,2-benzisothiazol-3-yl}oxy}acetonitrile |
| 109 | 3-[3-(Allyloxy)-1,2-benzisothiazol-5-yl]-1-methyl-6-(trifluoromethyl)-2,4(1H,3H)-pyrimidinedione |
| 110 | 3-(3-Isopropoxy-1,2-benzisothiazol-5-yl)-1-methyl-6-(trifluoromethyl)-2,4(1H,3H)-pyrimidinedione |
| 111 | 3-(3-Methoxy-1,2-benzisothiazol-5-yl)-1-methyl-6-(trifluoromethyl)-2,4(1H,3H)-pyrimidinedione |
| 112 | Methyl {{5-[3,6-dihydro-3-methyl-2,6-dioxo-4-(trifluoromethyl)-1(2H)-pyrimidinyl]-1,2-benzisothiazol-3-yl}oxy}acetate |
| 113 | 5-[3,6-Dihydro-3-methyl-2,6-dioxo-4-(trifluoromethyl)-1(2H)-pyrimidinyl]-1,2-benzisothiazole-3-carboxaldehyde |
| 114 | 3-[3-(Dichloromethyl)-1,2-benzisothiazol-5-yl]-1-methyl-6-(trifluoromethyl)-2,4(1H,3H)-pyrimidinedione |
| 115 | 2-Propynyl {{2-{5-[3-allyl-3,6-dihydro-2,6-dioxo-4-(trifluoromethyl)-1(2H)-pyrimidinyl]-1,2-benzisothiazol-3-yl}-*p*-tolyl}oxy}acetate |
| 116 | 1-Methyl-3-(3-morpholino-1,2-benzisothiazol-5-yl)-6-(trifluoromethyl)-2,4(1H,3H)-pyrimidinedione |
| 117 | N-{5-[3,6-Dihydro-3-methyl-2,6-dioxo-4-(trifluoromethyl)-1(2H)-pyrimidinyl]-1,2-benzisothiazol-3-yl}methanesulfonamide |
| 118 | 3-[3-(Diethylamino)-1,2-benzisothiazol-5-yl]-1-methyl-6-(trifluoromethyl)-2,4(1H,3H)-pyrimidinedione |
| 119 | 3-(3-Ethyl-1,2-benzisothiazol-5-yl)-1-methyl-6-(trifluoromethyl)-2,4(1H,3H)-pyrimidinedione |
| 120 | 3-[3-(*o*-Hydroxyphenyl)-1,2-benzisothiazol-5-yl]-1-methyl-6-(trifluoromethyl)-2,4(1H,3H)-pyrimidinedione |
| 121 | N-{{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetyl}methanesulfonamide |
| 122 | N-{{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetyl}-*p*-toluenesulfonamide |
| 123 | *p*-Chloro-N-{{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetyl}-benzenesulfonamide |
| 124 | N-{{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetyl}-α-toluenesulfonamide |
| 125 | 2-Propynyl 2-{{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate |
| 126 | 3-{3-[5-(Bromomethyl)-2-methoxyphenyl]-1,2-benzisothiazol-5-yl}-1-methyl-6-(trifluoromethyl)-2,4(1H,3H)-pyrimidinedione |
| 127 | 3-{5-[3,6-Dihydro-3-methyl-2,6-dioxo-4-(trifluoromethyl)-1(2H)-pyrimidinyl]-1,2-benzisothiazol-3-yl}-4-methoxybenzaldehyde |
| 128 | 3-[3-(Chloromethyl)-1,2-benzisothiazol-5-yl]- 1-methyl-6-(trifluoromethyl)-2,4(1H,3H)-pyrimidinedione |
| 129 | *tert* -Butyl α-acetyl-5-[3,6-dihydro-3-methyl-2,6-dioxo-4-(trifluoromethyl)-1(2H)-pyrimidinyl]-1,2-benzisothiazole-3-propionate |
| 130 | α-Acetyl-5-[3,6-dihydro-3-methyl-2,6-dioxo-4-(trifluoromethyl)-1(2H)-pyrimidinyl]-1,2-benzisothiazole-3-propionic acid |
| 131 | 1-Methyl-3-[3-(3-oxobutyl)-1,2-benzisothiazol-5-yl]-6-(trifluoromethyl)-2,4(1H,3H)-pyrimidinedione |
| 132 | Methyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate |
| 133 | Methyl 2-{*o*-{5-[3,6-dihydro-3-methyl-2,6-dioxo-4-(trifluoromethyl)-1(2H)-pyrimidinyl]-1,2-benzisothiazol-3-yl}phenoxy}propionate |
| 134 | 2-Propynyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate |
| 135 | 2-Propynyl {{6-{5-[3,6-dihydro-3-methyl-2,6-dioxo-4-(trifluoromethyl)-1(2H)-pyrimidinyl]-1,2-benzisothiazol-3-yl)-3,4-xylyl}oxy}acetate |
| 136 | Methyl {2-{5-[3,6-dihydro-3-methyl-2,6-dioxo-4-(trifluoromethyl)-1(2H)-pyrimidinyl]-1,2-benzisothiazol-3-yl}-4-formylphenoxy}acetate |
| 137 | 3-[3-(Fluoromethyl)-1,2-benzisothiazol-5-yl]-1-methyl-6-(trifluoromethyl)-2,4(1H,3H)-pyrimidinedione |
| 138 | 2-Propynyl {{2-{4-chloro-5-[3,6-dihydro-3-methyl-2,6-dioxo-4-(trifluoromethyl)-1(2H)-pyrimidinyl]-1,2-benzisoxazol-3-yl}-*p*-tolyl}oxylacetate |
| 139 | 3-[3-(*p*-Chlorophenyl)-1,2-benzisothiazol-5-yl]-1-methyl-6-(trifluoromethyl)-2,4(1H,3H)-pyrimidinedione |
| 140 | 3-[3-(Difluoromethyl)-1,2-benzisothiazol-5-yl]-1-methyl-6-(trifluoromethyl)-2,4(1H,3H)-pyrimidinedione |
| 141 | 5-[3,6-Dihydro-3-methyl-2,6-dioxo-4-(trifluoromethyl)-1(2H)-pyrimidinyl]-1,2-benzisothiazole-3-acetonitrile |
| 142 | 2-Propynyl {{6-{5-[3,6-dihydro-3-methyl-2,6-dioxo-4-(trifluoromethyl)-1(2H)-pyrimidinyl]-1,2-benzisoxazol-3-yl}-*m*-tolyl}oxy}acetate |
| 143 | 3-[3-(2-Hydroxyethyl)-1,2-benzisothiazol-5-yl]-1-methyl-6-(trifluoromethyl)-2,4(1H,3H)-pyrimidinedione, acetate (ester) |
| 144 | 2-Propynyl 2-{{2-{4-chloro-5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate |
| 145 | Di- *tert*-butyl {{5-[3,6-dihydro-3-methyl-2,6-dioxo-4-(trifluoromethyl)-1(2H)-pyrimidinyl]-1,2-benzisothiazol-3-yl}methyl}malonate |
| 146 | *tert*-Butyl methyl {{5-[3,6-dihydro-3-methyl-2,6-dioxo-4-(trifluoromethyl)-1(2H)-pyrimidinyl]-1,2-benzisothiazol-3-yl}methyl}malonate |
| 147 | Monomethyl {{5-[3,6-dihydro-3-methyl-2,6-dioxo-4-(trifluoromethyl)-1(2H)-pyrimidinyl]-1,2-benzisothiazol-3-yl}methyl}malonate |
| 148 | Methyl 5-[3,6-dihydro-3-methyl-2,6-dioxo-4-(trifluoromethyl)-1(2H)-pyrimidinyl]-1,2-benzisothiazole-3-propionate |
| 149 | 3-{3-[(2-Chloro-5-nitrophenoxy)methyl]-1,2-benzisothiazol-5-yl}-1-methyl-6-(trifluoromethyl)-2,4(1H,3H)-pyrimidinedione |
| 150 | 5-[3,6-Dihydro-3-methyl-2,6-dioxo-4-(trifluoromethyl)-1(2H)-pyrimidinyl]-1,2-benzisothiazole-3-acetic acid |
| 151 | 1-Methyl-3-[3-(1-piperazinyl)-1,2-benzisothiazol-5-yl]-6-(trifluoromethyl)-2,4(1H,3H)-pyrimidinedione |
| 152 | 3-{3-{[5-Chloro-4-(trifluoromethyl)-2-thiazolyl]amino}-1,2-benzisothiazol-5-yl}-1-methyl-6-(trifluoromethyl)-2,4(1H,3H)-pyrimidinedione |
| 153 | Methyl 2-{{2-{4-chloro-5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate |
| 154 | 3-[3-(2-Hydroxyethyl)-1,2-benzisothiazol-5-yl]-1-methyl-6-(trifluoromethyl)-2,4(1H,3H)-pyrimidinedione |
| 155 | Diethyl {{5-[3,6-dihydro-3-methyl-2,6-dioxo-4-(trifluoromethyl)-1(2H)-pyrimidinyl]-1,2-benzisothiazol-3-yl}methyl}phosphonate |
| 156 | {{5-[3,6-Dihydro-3-methyl-2,6-dioxo-4-(trifluoromethyl)-1(2H)-pyrimidinyl]-1,2-benzisothiazol-3-yl}methyl}trimethylammonium bromide |
| 157 | Methyl 5-[3,6-dihydro-3-methyl-2,6-dioxo-4-(trifluoromethyl)-1(2H)-pyrimidinyl]-1,2-benzisothiazole-3-acetate |
| 158 | N-{{5-[3,6-Dihydro-3-methyl-2,6-dioxo-4-(trifluoromethyl)-1(2H)-pyrimidinyl]-1,2-benzisothiazol-3-yl}methyl}phthalimide |
| 159 | Diethyl {{5-[3,6-dihydro-3-methyl-2,6-dioxo-4-(trifluoromethyl)-1(2H)-pyrimidinyl]-1,2-benzisothiazol-3-yl}methyl}phosphoramidate |
| 160 | {{5-[3,6-Dihydro-3-methyl-2,6-dioxo-4-(trifluoromethyl)-1(2H)-pyrimidinyl]-1,2-benzisothiazol-3-yl}methyl}phosphonic acid |
| 161 | 3-(4-Chloro-6-fluoro-3-methyl-1,2-benzisoxazol-5-yl)-1-methyl-6-(trifluoromethyl)-2,4(1H,3H)-pyrimidinedione |
| 162 | Methyl {{2-{5-[2-(methylthio)-6-oxo-4-(trifluoromethyl)-1(6H)-pyrimidinyl]-1,2-benzisothiazol-3-yl}-*p*-tolyl}oxy}acetate |
| 163 | 2-Propynyl {{2-{5-[2-(methylthio)-6-oxo-4-(trifluoromethyl)-1(6H)-pyrimidinyl]-1,2-benzisothiazol-3-yl}-*p*-tolyl}oxy}acetate |
| 164 | Methyl *o*-{{5-[3,6-dihydro-3-methyl-2,6-dioxo-4-(trifluoromethyl)-1(2H)-pyrimidinyl]-1,2-benzisothiazol-3-yl}cinnamate |
| 165 | 5-[3,6-Dihydro-3-methyl-2,6-dioxo-4-(trifluoromethyl)-1(2H)-pyrimidinyl]-1,2-benzisothiazole-3-malononitrile |
| 166 | Methyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate, S,S-dioxide |
| 167 | 5-[3,6-Dihydro-3-methyl-2,6-dioxo-4-(trifluoromethyl)-1(2H)-pyrimidinyl]-1,2-benzisothiazole-3-acetamide |
| 168 | 1-Methyl-3-{3-[(methylsulfinyl)methyl]-1,2-benzisothiazol-5-yl}-6-(trifluoromethyl)-2,4(1H,3H)-pyrimidinedione |
| 169 | 3-[3-(*p*-Methoxyphenyl)-1,2-benzisothiazol-5-yl]-1-methyl-6-(trifluoromethyl)-2,4(1H,3H)-pyrimidinedione |
| 170 | Methyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methylpropionate |
| 171 | Ethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}valerate, DL- |
| 172 | Methyl {{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetoxy}acetate |
| 173 | Sodium 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate, DL- |
| 174 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}propionic acid, DL-, compound with diisopropylamine |
| 175 | {{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetic acid, compound with diisopropylamine |
| 176 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}propionic acid, ammonium salt, DL- |
| 177 | {{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetic acid, ammonium salt |
| 178 | Sodium {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 179 | 3-{3-[(Isopropoxy)methyl]-1,2-benzisothiazol-5-yl}-1-methyl-6-(trifluoromethyl)-2,4(1H,3H)-pyrimidinedione |
| 180 | 3-{3-[(2-Fluoroethoxy)methyl]-1,2-benzisothiazol-5-yl}-1-methyl-6-(trifluoromethyl)-2,4(1H,3H)-pyrimidinedione |
| 181 | 3-{3-[(Benzyloxy)methyl]-1,2-benzisothiazol-5-yl}-1-methyl-6-(trifluoromethyl)-2,4(1H,3H)-pyrimidinedione |
| 182 | 3-Methyl-3-butenyl {{2-(5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 183 | 2-Methylallyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 184 | 2,4-Hexadienyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 185 | 2-Cyclohexen-1-yl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 186 | 1-Methylallyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 187 | 3-Methyl-2-butenyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 188 | Methyl 4-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-butenoate, (E)- and (Z)- |
| 189 | 1-Methyl-3-{3-{6-[(tetrahydro-5-methyl-2-oxo-3-furyl)oxy]-*m*-tolyl}-1,2-benzisothiazol-5-yl}-6-(trifluoromethyl)-2,4(1H,3H)-pyrimidinedione, DL- |
| 190 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetoxy}-propionic acid, DL- |
| 191 | 2-Methoxyethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 192 | 1-Methyl-2-methoxyethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate, DL- |
| 193 | 2-{{2-{5-[3,6-Dihydro-3-methyl-2,6-dioxo-4-(trifluoromethyl)-1(2H)-pyrimidinyl]-1,2-benzisoxazol-3-yl}-*p*-tolyl}oxy}-N-(2-propynyl)acetamide |
| 194 | O-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}acetoxy}oxime acetone |
| 195 | 3-[3-(Ethoxymethyl)-1,2-benzisothiazol-5-yl]-1-methyl-6-(trifluoromethyl)-2,4(1H,3H)-pyrimidinedione |
| 196 | 3-{3-[(Allyloxy)methyl]-1,2-benzisothiazol-5-yl}-1-methyl-6-(trifluoromethyl)-2,4(1H,3H)-pyrimidinedione |
| 197 | 1-Methyl-3-{3-{[(*m*-methylbenzyl)thio]methyl}-1,2-benzisothiazol-5-yl}-6-(trifluoromethyl)-2,4(1H,3H)-pyrimidinedione |
| 198 | 3-{3-[(2-Chloroethoxy)methyl]-1,2-benzisothiazol-5-yl}-1-methyl-6-(trifluoromethyl)-2,4(1H,3H)-pyrimidinedione |
| 199 | 3-{3-[2-Chloro-1-(chloromethyl)ethyl]-1,2-benzisothiazol-5-yl}-1-methyl-6-(trifluoromethyl)-2,4(1H,3H)-pyrimidinedione |
| 200 | 3-{3-[(2-Ethoxyethoxy)methyl]-1,2-benzisothiazol-5-yl}-1-methyl-6-(trifluoromethyl)-2,4(1H,3H)-pyrimidinedione |
| 201 | 1-Methyl-3-{3-[(2,2,2-trifluoroethoxy)methyl]-1,2-benzisothiazol-5-yl}-6-(trifluoromethyl)-2,4(1H,3H)-pyrimidinedione |
| 202 | 3-{3-[(Isopropylthio)methyl]-1,2-benzisothiazol-5-yl]-1-methyl-6-(trifluoromethyl)-2,4(1H,3H)-pyrimidinedione |
| 203 | 3-{3-[(2,2-Difluoroethoxy)methyl]-1,2-benzisothiazol-5-yl}-1-methyl-6-(trifluoromethyl)-2,4(1H,3H)-pyrimidinedione |
| 204 | 3-[3-(Butoxymethyl)-1,2-benzisothiazol-5-yl]-1-methyl-6-(trifluoromethyl)-2,4(1H,3H)-pyrimidinedione |
| 205 | 3-{3-[(2-Butenyloxy)methyl]-1,2-benzisothiazol-5-yl}-1-methyl-6-(trifluoromethyl)-2,4(1H,3H)-pyrimidinedione |
| 206 | 1-Methyl-3-{3-[(1-methylallyloxy)methyl]-1,2-benzisothiazol-5-yl]-6-(trifluoromethyl)-2,4(1H,3H)-pyrimidinedione |
| 207 | 3-{3-{[2-Fluoro-1-(fluoromethyl)ethoxy]methyl}-1,2-benzisothiazol-5-yl}-1-methyl-6-(trifluoromethyl)-2,4(1H,3H)-pyrimidinedione |
| 208 | 1-Methyl-6-(trifluoromethyl)-3-[(2,2,2-trifluoro-1-methylethoxy)methyl]-1,2-benzisothiazol-5-yl]-2,4(1H,3H)-pyrimidinedione |
| 209 | 3-{3-[(2-Cyclohexen-1-yloxy)methyl]-1,2-benzisothiazol-5-yl}-1-methyl-6-(trifluoromethyl)-2,4(1H,3H)-pyrimidinedione |
| 210 | 1-Methyl-3-{3-[(3-methyl-2-butenyloxy)methyl]-1,2-benzisothiazol-5-yl}-6-(trifluoromethyl)-2,4(1H,3H)-pyrimidinedione |
| 211 | 1-Methyl-3-{3-{{[4-(1-methylvinyl)-1-cyclohexen-1-yl]methoxy}methyl}-1,2-benzisothiazol-5-yl}-2,4(1H,3H)-pyrimidinedione |
| 212 | Methyl 3-{{5-[3,6-dihydro-3-methyl-2,6-dioxo-4-(trifluoromethyl)-1(2H)-pyrimidinyl]-1,2-benzisothiazol-3-yl}methoxy}-2-methylenebutyrate |
| 213 | 3,4-Dihydro-3-(3-methyl-1,2-benzisothiazol-5-yl)-2,4-dioxo-6-(trifluoromethyl)-1(2H)-pyrimidineacetonitrile |
| 214 | 1-Ethyl-3-(3-methyl-1,2-benzisothiazol-5-yl)-6-(trifluoromethyl)-2,4(1H,3H)-pyrimidinedione and 2-Ethoxy-3-(3-methyl-1,2-benzisothiazol-5-yl)-6-(trifluoromethyl)-4(3H)-pyrimidinone (1:1) |
| 215 | 1-Isobutyl-3-(3-methyl-1,2-benzisothiazol-5-yl)-6-(trifluoromethyl)-2,4(1H,3H)-pyrimidinedione and 2-Isobutoxy-3-(3-methyl-1,2-benzisothiazol-5-yl)-6-(trifluoromethyl)-4(3H)-pyrimidinone (55:45) |
| 216 | 1-Allyl-3-(3-methyl-1,2-benzisothiazol-5-yl)-6-(trifluoromethyl)-2,4(1H,3H)-pyrimidinedione and 2-(Allyloxy)-3-(3-methyl-1,2-benzisothiazol-5-yl)-6-(trifluoromethyl)-4(3H)-pyrimidinone (85:15) |
| 217 | 3-(3-Methyl-1,2-benzisothiazol-5-yl)-1-(2-propynyl)-6-(trifluoromethyl)-2,4(1H,3H)-pyrimidinedione |
| 218 | 1-Benzyl-3-(3-methyl-1,2-benzisothiazol-5-yl)-6-(trifluoromethyl)-2,4(1H,3H)-pyrimidinedione |
| 219 | 1-Methyl-3-{3-[(4-pentenyloxy)methyl]-1,2-benzisothiazol-5-yl}-6-(trifluoromethyl)-2,4(1H,3H)-pyrimidinedione |
| 220 | 3-{3-[(3-Hexenyloxy)methyl]-1,2-benzisothiazol-5-yl}-1-methyl-6-(trifluoromethyl)-2,4(1H,3H)-pyrimidinedione, (E)- |
| 221 | 1-Methyl-3-{3-{[(*p*-methylphenethyl)oxy]methyl}-1,2-benzisothiazol-5-yl}-6-(trifluoromethyl)-2,4(1H,3H)-pyrimidinedione |
| 222 | 3-{[(2,4-Dimethylphenethyl)oxy]methyl}-1,2-benzisothiazol-5-yl}-1-methyl-6-(trifluoromethyl)-2,4(1H,3H)-pyrimidinedione |
| 223 | 3-{3-[(3-Butenyloxy)methyl]-1,2-benzisothiazol-5-yl}-1-methyl-6-(trifluoromethyl)-2,4(1H,3H)-pyrimidinedione |
| 224 | 3-{3-[(3-Chloropropoxy)methyl]-1,2-benzisothiazol-5-yl}-1-methyl-6-(trifluoromethyl)-2,4(1H,3H)-pyrimidinedione |
| 225 | 3-[3-(Hydroxymethyl)-1,2-benzisothiazol-5-yl]-1-methyl-6-(trifluoromethyl)-2,4(1H,3H)-pyrimidinedione |
| 226 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-(*o*-tolylsulfonyl)acetamide |
| 227 | 2-{{2-{5-[3,6-Dihydro-3-methyl-2,6-dioxo-4-(trifluoromethyl)-1(2H)-pyrimidinyl]-1,2-benzisothiazol-3-yl} *p*-tolyl}oxy}-N-(styrylsulfonyl)acetamide |
| 228 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(*m*-nitrophenyl)sulfonyl]acetamide |
| 229 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(*p*-nitrophenyl)sulfonyl]acetamide |
| 230 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(*p*-fluorophenyl)sulfonyl]acetamide |
| 231 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(*p*-methoxyphenyl)sulfonyl]acetamide |
| 232 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-(phenylsulfonyl)acetamide |
| 233 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(α,α,α-trifluoro-*m*-tolyl)sulfonyl]acetamide |
| 234 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(*o*-chlorophenyl)sulfonyl]acetamide |
| 235 | 2-{{2-{5-[3,6-Dihydro-3-methyl-2,6-dioxo-4-(trifluoromethyl)-1(2H)-pyrimidinyl]-1,2-benzisothiazol-3-yl}-*p*-tolyl}oxy}-N-[(*m*-chlorophenyl)sulfonyl]acetamide |
| 236 | *o*-{{5-[3,6-Dihydro-3-methyl-2,6-dioxo-4-(trifluoromethyl)-1(2H)-pyrimidinyl]-1,2-benzisothiazol-3-yl}methoxy}benzonitrile |
| 237 | 1-Methyl-3-{3-[(*o*-nitrophenoxy)methyl]-1,2benzisothiazol-5-yl}-6-(trifluoromethyl)-2,4(1H,3H)-pyrimidinedione |
| 238 | 1-Methyl-6-(trifluoromethyl)-3-{3-{[(α,α,α-trifluoro-*o*-tolyl)oxy]methyl}-1,2-benzisothiazol-5-yl}-2,4(1H,3H)-pyrimidinedione |
| 239 | {2-{5-[3,6-Dihydro-3-methyl-2,6-dioxo-4-(trifluoromethyl)-1(2H)-pyrimidinyl]-1,2-benzisothiazol-3-yl}methyl salicylate |
| 240 | 1-Methyl-3-{3-{[*m*-(methylamino)phenoxy]methyl}-1,2-benzisothiazol-5-yl}-6-(trifluoromethyl)-2,4(1H,3H)-pyrimidinedione |
| 241 | 3-{3-[(*m*-Acetylphenoxy)methyl]-1,2-benzisothiazol-5-yl}-1-methyl-6-(trifluoromethyl)-2,4(1H,3H)-pyrimidinedione |
| 242 | *m*-{{5-[3,6-Dihydro-3-methyl-2,6-dioxo-4-(trifluoromethyl)-1(2H)-pyrimidinyl]-1,2-benzisothiazol-3-yl}methoxy}benzonitrile |
| 243 | 1-Methyl-6-(trifluoromethyl)-3-{3-{[(α,α,α-trifluoro-*m*-tolyl)oxy]methyl)-1,2-benzisothiazol-5-yl}-2,4(1H,3H)-pyrimidinedione |
| 244 | 1-Methyl-3-{3-{[*m*-(trifluoromethoxy)phenoxy]methyl}-1,2-benzisothiazol-5-yl}-6-(trifluoromethyl)-2,4(1H,3H)-pyrimidinedione |
| 245 | 2-(Allyloxy)-3-[3-(6-methoxy-*m*-tolyl)-1,2-benzisothiazol-5-yl]-6-(trifluoromethyl)-4(3H)-pyrimidinone |
| 246 | Methyl {{2-{5-[2-ethoxy-6-oxo-4-(trifluoromethyl)-1(6H)-pyrimidinyl]-1,2-benzisothiazol-3-yl}-*p*-tolyl}oxy}acetate |
| 247 | Ethyl 5-[3,6-dihydro-3-methyl-2,6-dioxo-4-(trifluoromethyl)-1(2H)-pyrimidinyl]-α,α-dimethyl-1,2-benzisothiazole-3-acetate |
| 248 | 3-{3-[(*m*-Fluorophenoxy)methyl]-1,2-benzisothiazol-5-yl}-1-methyl-6-(trifluoromethyl)-2,4(1H,3H)-pyrimidinedione |
| 249 | 2-Cyanoethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 250 | 1-Methylhexyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate, DL- |
| 251 | 2,2,3,3,3-Pentafluoropropyl {{2-{5-[3,6-dihydro-3-methyl-2,6-dioxo-4-(trifluoromethyl)-1(2H)pyrimidinyl]-1,2-benzisothiazol-3-yl}-*p*-tolyl}oxy}acetate |
| 252 | Dodecyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 253 | 3-Furylmethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate |
| 254 | Tetrahydrofurfuryl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate, DL- |
| 255 | 1-Methyldecyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy)acetate, DL- |
| 256 | 1,2-Benzisothiazole-3-acetonitrile, 5-[3,6-dihydro-3-methyl-2,6-dioxo-4-(trifluoromethyl)-1(2H)-pyrimidinyl)-alpha,alpha-dimethyl- |
| 257 | Acetic acid, {*p*-{5-[3,6-dihydro-3-methyl-2,6-dioxo-4-(trifluoromethyl)-1(2H)-pyrimidinyl)-1,2-benzisothiazol-3-yl}phenoxy}-, methyl ester |
| 258 | Propionic acid, 2-{*p*-{5-[3,6-dihydro-3-methyl-2,6-dioxo-4-(trifluoromethyl)-1(2H)-pyrimidinyl]-1,2-benzisothiazol-3-yl}phenoxy}-, methyl ester |
| 259 | Acetic acid, [*p*-{5-[3,6-dihydro-3-methyl-2,6-dioxo-4-(trifluoromethyl)-1(2H)-pyrimidinyl]-1,2-benzisothiazol-3-yl}phenoxy)-, 2-propynyl ester |
| 260 | 2,4(1H,3H)-Pyrimidinedione, 3-[3-(2-bromoethyl)-1,2-benzisothiazol-5-yl]-1-methyl-6-(trifluoromethyl)- |
| 261 | Acetic acid, {{2-{4-chloro-5-[3,6-dihydro-3-methyl-2,6-dioxo-4-(trifluoromethyl)-1(2H)-pyrimidinyl]-1,2-benzisoxazol-3-yl}-*p*-tolyl}oxy}-, methyl ester |
| 262 | 1,2-Benzisothiazole-3-methanephosphonic acid, 5-[3,6-dihydro-3-methyl-2,6-dioxo-4-(trifluoromethyl)-1(2H)-pyrimidinyl]-, dimethyl ester |
| 263 | 1,2-Benzisothiazole-3-methanephosphonic acid, 5-[3,6-dihydro-3-methyl-2,6-dioxo-4-(trifluoromethyl)-1(2H)-pyrimidinyl]-, diisopropyl ester |
| 264 | 1,2-Benzisothiazole-3-acetonitrile, alpha-bromo-5-[3,6-dihydro-3-methyl-2,6-dioxo-4-(trifluoromethyl)-1(2H)-pyrimidinyl]- |
| 265 | Acetic acid, {{2-{4-chloro-5-[3,6-dihydro-3-methyl-2,6-dioxo-4-(trifluoromethyl)-1(2H)-pyrimidinyl]-1,2-benzisoxazol-3-yl}-*p*-tolyl}oxy}-, *tert*-butyl ester |
| 266 | 1,2-Benzisothiazole-3-acetaldehyde, 5-[3,6-dihydro-3-methyl-2,6-dioxo-4-(trifluoromethyl)-1(2H)-pyrimidinyl]-, 3-oxime, (E)- and (Z)- |
| 267 | 2,4(1H,3H)-Pyrimidinedione, 3-[3-(2-methoxy-3,4-xylyl)-1,2-benzisothiazol-5-yl]-1-methyl-6-(trifluoromethyl)- |
| 268 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(methylsulfonyl)- |
| 269 | Acetamide, N-[(5-acetamido-1,3,4-thiadiazol-2-yl)sulfonyl]-2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}- |
| 270 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-[(3,5-dimethylisoxazol-4-yl)sulfonyl]- |
| 271 | 2,4(1H,3H)-Pyrimidinedione, 5-chloro-1-methyl-3-(3-methyl-1,2-benzisothiazol-5-yl)-6-(trifluoromethyl)- |
| 272 | Acetic acid, {{{5-[3,6-dihydro-3-methyl-2,6-dioxo-4-(trifluoromethyl)-1(2H)-pyrimidinyl]-1,2-benzisothiazol-3-yl}methyl}sulfonyl}-, methyl ester |
| 273 | Acetic acid, {*p*-{5-[3,6-dihydro-3-methyl-2,6-dioxo-4-(trifluoromethyl)-1(2H)-pyrimidinyl]-1,2-benzisothiazol-3-yl}phenoxy}-, *tert*-butyl ester |
| 274 | Acetic acid, {{6-{5-[3,6-dihydro-3-methyl-2,6-dioxo-4-(trifluoromethyl)-1(2H)-pyrimidinyl]-1,2-benzisothiazol-3-yl}-2,3-xylyl}oxy}-, methyl ester |
| 275 | Propionic acid, 2-{{6-{5-[3,6-dihydro-3-methyl-2,6-dioxo-4-(trifluoromethyl)-1(2H)-pyrimidinyl]-1,2-benzisothiazol-3-yl}-2,3-xylyl}oxy}-, methyl ester |
| 276 | 2,4(1H,3H)-Pyrimidinedione, 1-methyl-6-(trifluoromethyl)-3-(3-vinyl-1,2-benzisothiazol-5-yl)- |
| 277 | 1,2-Benzisothiazole-3-carboxaldehyde, 5-[3,6-dihydro-3-methyl-2,6-dioxo-4-(trifluoromethyl)-1(2H)-pyrimidinyl]-, 3-(O-methyloxime), (E)- |
| 278 | 1,2-Benzisothiazole-3-carboxaldehyde, 5-[3,6-dihydro-3-methyl-2,6-dioxo-4-(trifluoromethyl)-1(2H)-pyrimidinyl]-, 3-oxime, (E)- |
| 279 | 1,2-Benzisothiazole-3-acrylic acid, 5-[3,6-dihydro-3-methyl-2,6-dioxo-4-(trifluoromethyl)-1(2H)-pyrimidinyl]-, methyl ester, (E)- |
| 280 | Acetic acid, {{6-[5-[3,6-dihydro-3-methyl-2,6-dioxo-4-(trifluoromethyl)-1(2H)-pyrimidinyl]-1,2-benzisothiazol-3-yl}-2,3-xylyl}oxy}-, 2-propynyl ester |
| 281 | Acetic acid, {*p*-{5-[3,6-dihydro-3-methyl-2,6-dioxo-4-(trifluoromethyl)-1(2H)-pyrimidinyl]-1,2-benzisothiazol-3-yl}phenoxy}- |
| 282 | Acetic acid, {{{5-[3,6-dihydro-3-methyl-2,6-dioxo-4-(trifluoromethyl)-1(2H)-pyrimidinyl]-1,2-benzisothiazol-3-yl}methyl}sulfonyl}- |
| 283 | Acetic acid, {{{5-[3,6-dihydro-3-methyl-2,6-dioxo-4-(trifluoromethyl)-1(2H)-pyrimidinyl]-1,2-benzisothiazol-3-yl}methyl}thio}- |
| 284 | 2,4(1H,3H)-Pyrimidinedione, 3-[3-(2,2-dibromovinyl)-1,2-benzisothiazol-5-yl]-1-methyl-6-(trifluoromethyl)- |
| 285 | 2,4(1H,3H)-Pyrimidinedione, 1-methyl-3-[3-(3-oxo-1-butenyl)-1,2-benzisothiazol-5-yl]-6-(trifluoromethyl)-, (E)- |
| 286 | 2,4(1H,3H)-Pyrimidinedione, 3-[3-(2-bromo-2-fluorovinyl)-1,2-benzisothiazol-5-yl]-1-methyl-6-(trifluoromethyl)- |
| 287 | 2,4(1H,3H)-Pyrimidinedione, 3-[3-(2,2-dichlorovinyl)-1,2-benzisothiazol-5-yl]-1-methyl-6-(trifluoromethyl)- |
| 288 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-[(2,4-dimethylthiazol-5-yl)sulfonyl]- |
| 289 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-[(5-ethoxybenzothiazol-2-yl)sulfonyl]- |
| 290 | Acetamide, N-[(5-chloro-3-methylbenzo[b]thien-2-yl)sulfonyl]-2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}- |
| 291 | 1,2-Benzisothiazole-3-acrylaldehyde, 5-[3,6-dihydro-3-methyl-2,6-dioxo-4-(trifluoromethyl)-1(2H)-pyrimidinyl]-, (E)- |
| 292 | Acetic acid, {{5-[3,6-dihydro-3-methyl-2,6-dioxo-4-(trifluoromethyl)-1(2H)-pyrimidinyl]-1,2-benzisothiazol-3-yl}methoxy}-, methyl ester |
| 293 | Acetic acid, {{5-[3,6-dihydro-3-methyl-2,6-dioxo-4-(trifluoromethyl)-1(2H)-pyrimidinyl]-1,2-benzisothiazol-3-yl}methoxy}- |
| 294 | Acetamide, 2-{{5-[3,6-dihydro-3-methyl-2,6-dioxo-4-(trifluoromethyl)-1(2H)-pyrimidinyl]-1,2-benzisothiazol-3-yl}methoxy}-N-(methylsulfonyl)- |
| 295 | Acetamide, 2-{{5-[3,6-dihydro-3-methyl-2,6-dioxo-4-(trifluoromethyl)-1(2H)-pyrimidinyl]-1,2-benzisothiazol-3-yl}methoxy}-N-(methylsulfonyl)-, dimethylamine salt |
| 296 | Acetic acid, {{5-[3,6-dihydro-3-methyl-2,6-dioxo-4-(trifluoromethyl)-1(2H)-pyrimidinyl]-1,2-benzisothiazol-3-yl}methoxy}-, dimethylamine salt |
| 297 | Acetamide, 2-{{{5-[3,6-dihydro-3-methyl-2,6-dioxo-4-(trifluoromethyl)-1(2H)-pyrimidinyl]-1,2-benzisothiazol-3-yl}methyl}thio}-N-(methylsulfonyl)- |
| 298 | Acetamide, 2-{{{5-[3,6-dihydro-3-methyl-2,6-dioxo-4-(trifluoromethyl)-1(2H)-pyrimidinyl]-1,2-benzisothiazol-3-yl}methyl}thio}-N-(methylsulfonyl)-, dimethylamine salt |
| 299 | Acetic acid, {{{5-[3,6-dihydro-3-methyl-2,6-dioxo-4-(trifluoromethyl)-1(2H)-pyrimidinyl)-1,2-benzisothiazol-3-yl}methyl}thio}-, dimethylamine salt |
| 300 | 2,4(1H,3H)-Pyrimidinedione, 3-[3-(bromochloromethyl)-1,2-benzisothiazol-5-yl]-1-methyl-6-(trifluoromethyl)- |
| 301 | 2,4(1H,3H)-Pyrimidinedione, 3-[3-(*m*-methoxyphenyl)-1,2-benzisothiazol-5-yl]-1-methyl-6-(trifluoromethyl)- |
| 302 | 2,4(1H,3H)-Pyrimidinedione, 3-[3-(1,2-dibromopropyl)-1,2-benzisothiazol-5-yl]-1-methyl-6-(trifluoromethyl)-, (1R,2S)- |
| 303 | 2,4(1H,3H)-Pyrimidinedione, 3-[3-(1,2-dibromopropyl)-1,2-benzisothiazol-5-yl]-1-methyl-6-(trifluoromethyl)-, (1R,2R)- |
| 304 | Carbamic acid, {{5-[3,6-dihydro-3-methyl-2,6-dioxo-4-(trifluoromethyl)-1(2H)-pyrimidinyl]-1, 2-benzisothiazol-3-yl}methyl}(methylsulfonyl)-, *tert* -butyl ester |
| 305 | Methanesulfonamide, N-{{5-[3,6-dihydro-3-methyl-2,6-dioxo-4-(trifluoromethyl)-1(2H)-pyrimidinyl]-1,2-benzisothiazol-3-ylmethyl}- |
| 306 | Glycine, N-{{5-[3,6-dihydro-3-methyl-2,6-dioxo-4-(trifluoromethyl)-1(2H)-pyrimidinyl]-1,2-benzisothiazol-3-yl}methyl}-N-(methylsulfonyl)-, methyl ester |
| 307 | 2,4(1H,3H)-Pyrimidinedione, 3-(3-acetyl-1,2-benzisothiazol-5-yl)-1-methyl-6-(trifluoromethyl)- |
| 308 | 2,4(1H,3H)-Pyrimidinedione, 3-[3-(1-hydroxyethyl)-1,2-benzisothiazol-5-yl]-1-methyl-6-(trifluoromethyl)- |
| 309 | 2,4(1H,3H)-Pyrimidinedione, 3-[3-(1-hydroxyethyl)-1,2-benzisothiazol-5-yl]-1-methyl-6-(trifluoromethyl)-, methanesulfonate (ester) |
| 310 | 2,4(1H,3H)-Pyrimidinedione, 3-[3-(hydroxymethyl)-1,2-benzisothiazol-5-yl]-1-methyl-6-(trifluoromethyl)-, nitrate (ester) |
| 311 | Isothiocyanic acid, {5-[3,6-dihydro-3-methyl-2,6-dioxo-4-(trifluoromethyl)-1(2H)-pyrimidinyl]-1,2-benzisothiazol-3-yl}methyl ester |
| 312 | Acetic acid, {*o*-{5-[3,6-dihydro-3-methyl-2,6-dioxo-4-(trifluoromethyl)-1(2H)-pyrimidinyl]-1,2-benzisothiazol-3-yl}phenoxy}- |
| 313 | Acetohydroxamic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}- |
| 314 | Propionic acid, 2-{*o*-{5-[3,6-dihydro-3-methyl-2,6-dioxo-4-(trifluoromethyl)-1(2H)-pyrimidinyl]-1,2-benzisothiazol-3-yl}phenoxy}- |
| 315 | 1,2-Benzisothiazole-3-carboxylic acid, 5-[3,6-dihydro-3-methyl-2,6-dioxo-4-(trifluoromethyl)-1(2H)-pyrimidinyl]- |
| 316 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methoxy- |
| 317 | 1,2-Benzisothiazole-3-carboxylic acid, 5-[3,6-dihydro-3-methyl-2,6-dioxo-4-(trifluoromethyl)-1(2H)-pyrimidinyl]-, methyl ester |
| 318 | 1,2-Benzisothiazole-3-carboxylic acid, 5-[3,6-dihydro-3-methyl-2,6-dioxo-4-(trifluoromethyl)-1(2H)-pyrimidinyl]-, methyl ester |
| 319 | 1,2-Benzisothiazole-3-carboxylic acid, 5-[3,6-dihydro-3-methyl-2,6-dioxo-4-(trifluoromethyl)-1(2H)-pyrimidinyl]-, phenyl ester |
| 320 | 1,2-Benzisothiazole-3-carboxylic acid, 5-[3,6-dihydro-3-methyl-2,6-dioxo-4-(trifluoromethyl)-1(2H)-pyrimidinyl]-, *p*-fluorophenyl ester |
| 321 | 2,4(1H,3H)-Pyrimidinedione, 3-[3-(2(and 1)-bromo-1(and 2)-hydroxypropyl)-1,2-benzisothiazol-5-yl]-1-methyl-6-(trifluoromethyl)-, (4:1), mixture of diastereomers |
| 322 | 1,2-Benzisothiazole-3-carboxamide, 5-[3,6-dihydro-3-methyl-2,6-dioxo-4-(trifluoromethyl)-1(2H)-pyrimidinyl]- |
| 323 | 1,2-Benzisothiazole-3-carboxamide, 5-[3,6-dihydro-3-methyl-2,6-dioxo-4-(trifluoromethyl)-1(2H)-pyrimidinyl]-N-(methylsulfonyl)- |
| 324 | 1,2-Benzisothiazole-3-carboxamide, N-[(chloromethyl)sulfonyl]-5-[3,6-dihydro-3-methyl-2,6-dioxo-4-(trifluoromethyl)-1(2H)-pyrimidinyl]- |
| 325 | Propionic acid, 2-{*m*-{5-[3,6-dihydro-3-methyl-2,6-dioxo-4-(trifluoromethyl)-1-(2H)-pyrimidinyl]-1,2-benzisothiazol-3-yl}phenoxy}-, methyl ester |
| 326 | Acetic acid, {*m*-{5-[3,6-dihydro-3-methyl-2,6-dioxo-4-(trifluoromethyl)-1(2H)-pyrimidinyl]-1,2-benzisothiazol-3-yl}phenoxy}-, 2-propynyl ester |
| 327 | 2,4(1H,3H)-Pyrimidinedione, 3-[3-(*m*-ethoxyphenyl)-1,2-benzisothiazol-5-yl]-1-methyl-6-(trifluoromethyl)- |
| 328 | 2,4(1H,3H)-Pyrimidinedione, 3-[3-(*m*-isopropoxyphenyl)-1,2-benzisothiazol-5-yl]-1-methyl-6-(trifluoromethyl)- |
| 329 | 1,2-Benzisothiazole-3-carboxamide, 5-[3,6-dihydro-3-methyl-2,6-dioxo-4-(trifluoromethyl)-1(2H)-pyrimidinyl]-N,N-dimethyl- |
| 330 | 1,2-Benzisothiazole-3-carboxamide, 5-[3,6-dihydro-3-methyl-2,6-dioxo-4-(trifluoromethyl)-1(2H)-pyrimidinyl]-N-isopropyl- |
| 331 | 1,2-Benzisothiazole-3-carboxamide, N-(5-*tert*-butylisoxazol-3-yl)-5-[3,6-dihydro-3-methyl-2,6-dioxo-4-(trifluoromethyl)-1(2H)-pyrimidinyl]- |
| 332 | Acetic acid, {*m*-{5-[3,6-dihydro-3-methyl-2,6-dioxo-4-(trifluoromethyl)-1(2H)-pyrimidinyl]-1,2-benzisothiazol-3-yl}phenoxy}-, methyl ester |
| 333 | 2,4(1H,3H)-Pyrimidinedione, 3-[3-(2,5-dimethoxyphenyl)-1,2-benzisothiazol-5-yl]-1-methyl-6-(trifluoromethyl)- |
| 334 | 1,2-Benzisothiazole-3-carboxamide, N-[(chloromethyl)sulfonyl]-5-[3,6-dihydro-3-methyl-2,6-dioxo-4-(trifluoromethyl)-1(2H)-pyrimidinyl]- |
| 335 | Propionamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(methylsulfonyl)- |
| 336 | Acetohydroxamic acid, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-methyl- |
| 337 | 2,4(1H,3H)-Pyrimidinedione, 3-[3-(bromoacetyl)-1,2-benzisothiazol-5-yl]-1-methyl-6-(trifluoromethyl)- |
| 338 | 2,4(1H,3H)-Pyrimidinedione, 3-[3-(chloroacetyl)-1,2-benzisothiazol-5-yl]-1-methyl-6-(trifluoromethyl)- |
| 339 | 2,4(1H,3H)-Pyrimidinedione, 3-[3-(1-bromoethyl)-1,2-benzisothiazol-5-yl]-1-methyl-6-(trifluoromethyl)- |
| 340 | 2,4(1H,3H)-Pyrimidinedione, 3-[3-(1,1-dibromoethyl)-1,2-benzisothiazol-5-yl]-1-methyl-6-(trifluoromethyl)- |
| 341 | 1,2-Benzisothiazole-3-carboxamide, N-(benzylsulfonyl)-5-[3,6-dihydro-3-methyl-2,6-dioxo-4-(trifluoromethyl)-1(2H)-pyrimidinyl]- |
| 342 | 2,4(1H,3H)-Pyrimidinedione, 1-methyl-3-{3-[6-(methylthio)-*m*-tolyl]-1,2-benzisothiazol-5-yl}-6-(trifluoromethyl)- |
| 343 | Acetamide, N-[(chloromethyl)sulfonyl]-2-{{2-{5-[3,6-dihydro-3-methyl-2,6-dioxo-4-(trifluoromethyl)-1(2H)-pyrimidinyl]-1,2-benzisothiazol-3-yl}-*p*-tolyloxy}- |
| 344 | Acetamide, N-[(chloromethyl)sulfonyl]-2-{*o*-{5-[3,6-dihydro-3-methyl-2,6-dioxo-4-(trifluoromethyl)-1(2H)-pyrimidinyl]-1,2-benzisothiazol-3-yl}phenoxy}- |
| 345 | 2,4(1H,3H)-Pyrimidinedione, 3-[6-fluoro-3-(6-methoxy-*m*-tolyl)-1,2-benzisoxazol-5-yl]-1-methyl-6-(trifluoromethyl)- |
| 346 | 2,4(1H,3H)-Pyrimidinedione, 3-[6-fluoro-3-(6-hydroxy-*m*-tolyl)-1,2-benzisoxazol-5-yl]-1-methyl-6-(trifluoromethyl)- |
| 347 | Acetic acid, {2-{5-[3,6-dihydro-3-methyl-2,6-dioxo-4-(trifluoromethyl)-1(2H)-pyrimidinyl]-1,2-benzisothiazol-3-yl}-4-methoxyphenoxy}-, methyl ester |
| 348 | Propionic acid, 2-{2-{5-[3,6-dihydro-3-methyl-2,6-dioxo-4-(trifluoromethyl)-1(2H)-pyrimidinyl]-1,2-benzisothiazol-3-yl}-4-methoxyphenoxy}-, methyl ester |
| 349 | Acetic acid, {2-{5-[3,6-dihydro-3-methyl-2,6-dioxo-4-(trifluoromethyl)-1(2H)-pyrimidinyl]-1,2-benzisothiazol-3-yl}-4-methoxyphenoxy}-, 2-propynyl ester |
| 350 | 2,4(1H,3H)-Pyrimidinedione, 3-[3-(1-methoxyethyl)-1,2-benzisothiazol-5-yl]-1-methyl-6-(trifluoromethyl)- |
| 351 | 2,4(1H,3H)-Pyrimidinedione, 3-{3-[1-(allyloxy)ethyl]-1,2-benzisothiazol-5-yl}-1-methyl-6-(trifluoromethyl)- |
| 352 | 1,2-Benzisothiazole-3-carboxamide, 5-[3,6-dihydro-3-methyl-2,6-dioxo-4-(trifluoromethyl)-1(2H)-pyrimidinyl]-N-(phenylsulfonyl)- |
| 353 | 1,2-Benzisothiazole-3-carboxamide, 5-[3,6-dihydro-3-methyl-2,6-dioxo-4-(trifluoromethyl)-1(2H)-pyrimidinyl]-N-(*p*-tolylsulfonyl)- |
| 354 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-methoxy-N-methyl- |
| 355 | Acetohydroxamic acid, 2-{{2-{5-[3,6-dihydro-3-methyl-2,6-dioxo-4-(trifluoromethyl)-1(2H)pyrimidinyl]-1,2-benzisothiazol-3-yl}-*p*-tolyl}oxy}-N-methyl- |
| 356 | 1,2-Benzisothiazole-3-carboxamide, N-[(*p*-chlorophenyl)sulfonyl]-5-[3,6-dihydro-3-methyl-2,6-dioxo-4-(trifluoromethyl)-1(2H)-pyrimidinyl]- |
| 357 | 1,2-Benzisothiazole-3-carboxamide, 5-[3,6-dihydro-3-methyl-2,6-dioxo-4-(trifluoromethyl)-1(2H)-pyrimidinyl]-N-(2-naphthylsulfonyl)- |
| 358 | 1,2-Benzisothiazole-3-carboxamide, 5-[3,6-dihydro-3-methyl-2,6-dioxo-4-(trifluoromethyl)-1(2H)-pyrimidinyl]-N-(dodecylsulfonyl)- |
| 359 | Acetic acid, {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}-, methyl ester |
| 360 | 2,4(1H,3H)-Pyrimidinedione, 3-[3-(3-bromopropenyl)-1,2-benzisothiazol-5-yl]-1-methyl-6-(trifluoromethyl)-, (E)- |
| 361 | 2,4(1H,3H)-Pyrimidinedione, 3-[3-(2-hydroxy-5-methoxyphenyl)-1,2-benzisothiazol-5-yl]-1-methyl-6-(trifluoromethyl)- |
| 362 | 1,2-Benzisothiazole-3-carboxamide, 5-[3,6-dihydro-3-methyl-2,6-dioxo-4-(trifluoromethyl)-1(2H)-pyrimidinyl]-N-(isopropylsulfonyl)- |
| 363 | 2,4(1H,3H)-Pyrimidinedione, 3-[3-(hydroxymethyl)-1,2-benzisothiazol-5-yl]-1-methyl-6-(trifluoromethyl)-, methanesulfonate (ester) |
| 364 | 2,4(1H,3H)-Pyrimidinedione, 3-[3-(hydroxymethyl)-1,2-benzisothiazol-5-yl]-1-methyl-6-(trifluoromethyl)-, benzoate (ester) |
| 365 | 1,2-Benzisothiazole-3-carboxanilide, 4'-chloro-5-[3,6-dihydro-3-methyl-2,6-dioxo-4-(trifluoromethyl)-1(2H)-pyrimidinyl]-N-methyl- |
| 366 | 2,4(1H,3H)-Pyrimidinedione, 3-(6-fluoro-3-methyl-1,2-benzisothiazol-5-yl)-1-methyl-6-(trifluoromethyl)- |
| 367 | 1,2-Benzisothiazole-3-carboxamide, 5-[3,6-dihydro-3-methyl-2,6-dioxo-4-(trifluoromethyl)-1(2H)-pyrimidinyl]-N-methyl-N-(methylsulfonyl)- |
| 368 | 2,4(1H,3H)-Pyrimidinedione, 3-[3-(hydroxymethyl)-1,2-benzisothiazol-5-yl]-1-methyl-6-(trifluoromethyl)-, chloroacetate (ester) |
| 369 | 2,4(1H,3H)-Pyrimidinedione, 3-[3-(hydroxymethyl)-1,2-benzisothiazol-5-yl]-1-methyl-6-(trifluoromethyl)-, phenylacetate (ester) |
| 370 | 1,2-Benzisothiazole-3-carboxamide, 5-[3,6-dihydro-3-methyl-2,6-dioxo-4-(trifluoromethyl)-1(2H)-pyrimidinyl]-N-(ethoxymethyl)-N-(methylsulfonyl)- |
| 371 | 2,4(1H,3H)-Pyrimidinedione, 3-[3-(hydroxymethyl)-1,2-benzisothiazol-5-yl]-1-methyl-6-(trifluoromethyl)-, 1-methylethanesulfonate (ester) |
| 372 | 2,4(1H,3H)-Pyrimidinedione, 3-[3-(dibromomethyl)-6-fluoro-1,2-benzisothiazol-5-yl]-1-methyl-6-(trifluoromethyl)- |
| 373 | 1,2-Benzisothiazole-3-carbonitrile, 5-[3,6-dihydro-3-methyl-2,6-dioxo-4-(trifluoromethyl)-1(2H)-pyrimidinyl]- |
| 374 | 2,4(1H,3H)-Pyrimidinedione, 3-[3-(hydroxymethyl)1,2-benzisothiazol-5-yl]-1-methyl-6-(trifluoromethyl)-, methylcarbamate (ester) |
| 375 | 2,4(1H,3H)-Pyrimidinedione, 3-[3-(hydroxymethyl)-1,2-benzisothiazol-5-yl]-1-methyl-6-(trifluoromethyl)-, phenylcarbamate (ester) |
| 376 | 2,4(1H,3H)-Pyrimidinedione, 3-[3-(bromomethyl)-6-fluoro-1,2-benzisothiazol-5-yl]-1-methyl-6-(trifluoromethyl)- |
| 377 | 1,2-Benzisothiazole-3-methanephosphonic acid, 5-[3,6-dihydro-3-methyl-2,6-dioxo-4-(trifluoromethyl)-1(2H)-pyrimidinyl]-6-fluoro-, diethyl ester |
| 378 | 2,4(1H,3H)-Pyrimidinedione, 3-[6-fluoro-3-(methoxymethyl)-1,2-benzisothiazol-5-yl]-1-methyl-6-(trifluoromethyl)- |
| 379 | 1,2-Benzisothiazole-3-acetonitrile, 5-[3,6-dihydro-3-methyl-2,6-dioxo-4-(trifluoromethyl)-1(2H)-pyrimidinyl]-6-fluoro- |
| 380 | 2,4(1H,3H)-Pyrimidinedione, 3-[3-(6-methoxy-5-indanyl)-1,2-benzisothiazol-5-yl]-1-methyl-6-(trifluoromethyl)- |
| 381 | 2,4(1H,3H)-Pyrimidinedione, 3-[3-(hydroxymethyl)-1,2-benzisothiazol-5-yl]-1-methyl-6-(trifluoromethyl)-, butanesulfonate (ester) |
| 382 | 2,4(1H,3H)-Pyrimidinedione, 3-[3-(hydroxymethyl)-1,2-benzisothiazol-5-yl]-1-methyl-6-(trifluoromethyl)-, benzenesulfonate (ester) |
| 383 | 1,2-Benzisothiazole-3-carboxamide, 5-[3,6-dihydro-3-methyl-2,6-dioxo-4-(trifluoromethyl)-1(2H)-pyrimidinyl]-N-(ethylsulfonyl)- |
| 384 | 1,2-Benzisothiazole-3-carboxylic acid, 5-[3,6-dihydro-3-methyl-2,6-dioxo-4-(trifluoromethyl)-1(2H)-pyrimidinyl]-, 2-butynyl ester |
| 385 | 1,2-Benzisothiazole-3-carboxylic acid, 5-[3,6-dihydro-3-methyl-2,6-dioxo-4-(trifluoromethyl)-1(2H)-pyrimidinyl]-, 2-(ethylthio)ethyl ester |
| 386 | 1,2-Benzisothiazole-3-carboxylic acid, 5-[3,6-dihydro-3-methyl-2,6-dioxo-4-(trifluoromethyl)-1(2H)-pyrimidinyl]-, 2-chloroallyl ester |
| 387 | 1,2-Benzisothiazole-3-carboxylic acid, 5-[3,6-dihydro-3-methyl-2,6-dioxo-4-(trifluoromethyl)-1(2H)-pyrimidinyl]-, 2-acetamidoethyl ester |
| 388 | 1,2-Benzisothiazole-3-carboxylic acid, 5-[3,6-dihydro-3-methyl-2,6-dioxo-4-(trifluoromethyl)-1(2H)-pyrimidinyl]-, heptyl ester |
| 389 | 1,2-Benzisothiazole-3-carboxylic acid, 5-[3,6-dihydro-3-methyl-2,6-dioxo-4-(trifluoromethyl)-1(2H)-pyrimidinyl]-, 2-(2-methoxyethoxy)ethyl ester |
| 390 | 2,4(1H,3H)-Pyrimidinedione, 3-[3-(hydroxymethyl)-1,2-benzisothiazol-5-yl]-1-methyl-6-(trifluoromethyl)-, benzylcarbamate (ester) |
| 391 | 1,2-Benzisothiazole-3-carboxamide, 5-[3,6-dihydro-3-methyl-2,6-dioxo-4-(trifluoromethyl)-1(2H)-pyrimidinyl]-N-(2-thienylsulfonyl)- |
| 392 | 2,4(1H,3H)-Pyrimidinedione, 1-methyl-3-{3-[(phenylthio)methyl]-1,2-benzisothiazol-5-yl}-6-(trifluoromethyl)- |
| 393 | 2,4(1H,3H)-Pyrimidinedione, 1-methyl-3-{3-[(phenylsulfonyl)methyl]-1,2-benzisothiazol-5-yl}-6-(trifluoromethyl)- |
| 394 | 2,4(1H,3H)-Pyrimidinedione, 1-methyl-3-{3-[(phenylsulfinyl)methyl]-1,2-benzisothiazol-5-yl}-6-(trifluoromethyl)- |
| 395 | 1,2-Benzisothiazole-3-carboxamide, 5-[3,6-dihydro-3-methyl-2,6-dioxo-4-(trifluoromethyl)-1(2H)-pyrimidinyl]-N-[(1-methylpropyl)sulfonyl]- |
| 396 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, benzyl ester |
| 397 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, *p*-methoxybenzyl ester |
| 398 | 1,2-Benzisothiazole-3-carbohydroxamic acid, 5-[3,6-dihydro-3-methyl-2,6-dioxo-4-(trifluoromethyl)-1(2H)-pyrimidinyl]-N-methyl- |
| 399 | 1,2-Benzisothiazole-3-carboxamide, N-(allyloxy)-5-[3,6-dihydro-3-methyl-2,6-dioxo-4-(trifluoromethyl)-1(2H)-pyrimidinyl]- |
| 400 | 1,2-Benzisothiazole-3-carboxamide, N-(benzyloxy)-5-[3,6-dihydro-3-methyl-2,6-dioxo-4-(trifluoromethyl)-1(2H)-pyrimidinyl]- |
| 401 | 2,4(1H,3H)-Pyrimidinedione, 3-[3-(hydroxymethyl)-1,2-benzisothiazol-5-yl]-1-methyl-6-(trifluoromethyl)-, (2,4-dichlorophenyl)acetate (ester) |
| 402 | 2,4(1H,3H)-Pyrimidinedione, 3-[3-(hydroxymethyl)-1,2-benzisothiazol-5-yl]-1-methyl-6-(trifluoromethyl)-, (*o*-chlorophenyl)acetate (ester) |
| 403 | 1,2-Benzisothiazole-3-carboxamide, 5-[3,6-dihydro-3-methyl-2,6-dioxo-4-(trifluoromethyl)-1(2H)-pyrimidinyl]-N-methoxy- |
| 404 | 1,2-Benzisothiazole-3-carboxamide, 5-[3,6-dihydro-3-methyl-2,6-dioxo-4-(trifluoromethyl)-1(2H)-pyrimidinyl]-N-[(*o*-nitrobenzyl)sulfonyl]- |
| 405 | 1,2-Benzisothiazole-3-carboxamide, N-[(*p*-*tert*-butylbenzyl)sulfonyl]-5-[3,6-dihydro-3-methyl-2,6-dioxo-4-(trifluoromethyl)-1(2H)-pyrimidinyl]- |
| 406 | 1,2-Benzisothiazole-3-carboxamide, 5-[3,6-dihydro-3-methyl-2,6-dioxo-4-(trifluoromethyl)-1(2H)-pyrimidinyl]-N-{[*p*-(trifluoromethoxy)-benzyl]sulfonyl}- |
| 407 | 2,4(1H,3H)-Pyrimidinedione, 3-{3-[bromo(phenylsulfonyl)methyl]-1,2-benzisothiazol-5-yl}-1-methyl-6-(trifluoromethyl)- |
| 408 | 1,2-Benzisothiazole-3-carboxaldehyde, 5-[3,6-dihydro-3-methyl-2,6-dioxo-4-(trifluoromethyl)-1(2H)-pyrimidinyl]-, 3-(phenylhydrazone), (E)- |
| 409 | 1,2-Benzisothiazole-3-carboxaldehyde, 5-[3,6-dihydro-3-methyl-2,6-dioxo-4-(trifluoromethyl)-1(2H)-pyrimidinyl]-, 3-(dimethylhydrazone), (E)- |
| 410 | 1,2-Benzisothiazole-3-carboxylic acid, 5-[3,6-dihydro-3-methyl-2,6-dioxo-4-(trifluoromethyl)-1(2H)-pyrimidinyl]-, 3-hydroxypropyl ester |
| 411 | 1,2-Benzisothiazole-3-carboxylic acid, 5-[3,6-dihydro-3-methyl-2,6-dioxo-4-(trifluoromethyl)-1(2H)-pyrimidinyl]-, 2-methoxyethyl ester |
| 412 | 1,2-Benzisothiazole-3-carboxaldehyde, 5-[3,6-dihydro-3-methyl-2,6-dioxo-4-(trifluoromethyl)-1(2H)-pyrimidinyl]-, 3-(methylphenylhydrazone), (E)- |
| 413 | 2,4(1H,3H)-Pyrimidinedione, 3-[3-(1-hydroxyethyl)-1,2-benzisothiazol-5-yl]-1-methyl-6-(trifluoromethyl)-, 1-methylethanesulfonate (ester) |
| 414 | 2,4(1H,3H)-Pyrimidinedione, 3-[3-(1-hydroxyethyl)-1,2-benzisothiazol-5-yl]-1-methyl-6-(trifluoromethyl)-, butanesulfonate (ester) |
| 415 | 2,4(1H,3H)-Pyrimidinedione, 3-[3-(1-hydroxyethyl)-1,2-benzisothiazol-5-yl]-1-methyl-6-(trifluoromethyl)-, benzenesulfonate (ester) |
| 416 | 2,4(1H,3H)-Pyrimidinedione, 3-[3-(1-chloroethyl)-1,2-benzisothiazol-5-yl]-1-methyl-6-(trifluoromethyl)- |
| 417 | 1,2-Benzisothiazole-3-carbohydroxamic acid, 5-[3,6-dihydro-3-methyl-2,6-dioxo-4-(trifluoromethyl)-1(2H)-pyrimidinyl]-, hydrogen sulfate (ester) |
| 418 | 1,2-Benzisothiazole-3-carboxaldehyde, 5-[3,6-dihydro-3-methyl-2,6-dioxo-4-(trifluoromethyl)-1(2H)-pyrimidinyl]-, 3-[bis(2-hydroxyethyl) dithioacetal], diacetate (diester) |
| 419 | 1,2-Benzisothiazole-3-carboxaldehyde, 5-[3,6-dihydro-3-methyl-2,6-dioxo-4-(trifluoromethyl)-1(2H)-pyrimidinyl]-, 3-[bis(2-hydroxyethyl) dithioacetal] |
| 420 | 1,2-Benzisothiazole-3-carboxamide, N-[(*p*-chlorobenzyl)methylsulfamoyl]-5-[3,6-dihydro-3-methyl-2,6-dioxo-4-(trifluoromethyl)-1(2H)-pyrimidinyl]- |
| 421 | 1,2-Benzisothiazole-3-thiocarboxylic acid, 5-[3,6-dihydro-3-methyl-2,6-dioxo-4-(trifluoromethyl)-1(2H)-pyrimidinyl]-, S-benzyl ester |
| 422 | 1,2-Benzisothiazole-3-thiocarboxylic acid, 5-[3,6-dihydro-3-methyl-2,6-dioxo-4-(trifluoromethyl)-1(2H)-pyrimidinyl]-, S-ethyl ester |
| 423 | 1,2-Benzisothiazole-3-carboxylic acid, 5-[3,6-dihydro-3-methyl-2,6-dioxo-4-(trifluoromethyl)-1(2H)-pyrimidinyl]-6-fluoro-, ethyl ester |
| 424 | 1,2-Benzisothiazole-3-carboxaldehyde, 5-[3,6-dihydro-3-methyl-2,6-dioxo-4-(trifluoromethyl)-1(2H)-pyrimidinyl]-, 3-[bis(3-hydroxypropyl) dithioacetal] |
| 425 | Acetic acid, {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}- |
| 426 | 1,2-Benzisothiazole-3-carboxaldehyde, 5-[3,6-dihydro-3-methyl-2,6-dioxo-4-(trifluoromethyl)-1(2H)-pyrimidinyl]-, 3-[bis(3-hydroxypropyl) dithioacetal], diacetate (diester) |
| 427 | 1,2-Benzisothiazole-3-carboxamide, 5-[3,6-dihydro-3-methyl-2,6-dioxo-4-(trifluoromethyl)-1(2H)-pyrimidinyl]-N-[(alpha-methylbenzyl)-sulfonyl]- |
| 428 | Acetamide, 2-{{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}-N-(methylsulfonyl)- |
| 429 | 2,4(1H,3H)-Pyrimidinedione, 3-[3-(5-iodo-2-methoxy-*p*-tolyl)-1,2-benzisothiazol-5-yl]-1-methyl-6-(trifluoromethyl)- |
| 430 | Methanesulfonanilide, 4'-{5-[3,6-dihydro-3-methyl-2,6-dioxo-4-(trifluoromethyl)-1(2H)-pyrimidinyl]-1,2-benzisothiazol-3-yl}-N-methyl- |
| 431 | 2,4(1H,3H)-Pyrimidinedione, 1-methyl-6-(trifluoromethyl)-3-(3-valeryl-1,2-benzisothiazol-5-yl)- |
| 432 | 2,4(1H,3H)-Pyrimidinedione, 1-methyl-3-(3-pivaloyl-1,2-benzisothiazol-5-yl)-6-(trifluoromethyl)- |
| 433 | 2,4(1H,3H)-Pyrimidinedione, 1-methyl-3-[(2-methylbutyryl)-1,2-benzisothiazol-5-yl]-6-(trifluoromethyl)- |
| 434 | Acetic acid, {{6-{5-[3,6-dihydro-3-methyl-2,6-dioxo-4-(trifluoromethyl)-1(2H)-pyrimidinyl]-1,2-benzisothiazol-3-yl}-5-indanyl}oxy}-, methyl ester |
| 435 | Propionic acid, 2-{{6-{5-[3,6-dihydro-3-methyl-2,6-dioxo-4-(trifluoromethyl)-1(2H)-pyrimidinyl]-1,2-benzisothiazol-3-yl}-5-indanyl}oxy}-, methyl ester |
| 436 | Acetic acid, {{6-{5-[3,6-dihydro-3-methyl-2,6-dioxo-4-(trifluoromethyl)-1(2H)-pyrimidinyl]-1,2-benzisothiazol-3-yl}-5-indanyl}oxy}-, 2-propynyl ester |
| 437 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl-N-(methylsulfonyl)- |
| 438 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-methyl-N-(methylsulfonyl)- |
| 439 | 1,2-Benzisothiazole-3-carboxamide, N-[5-chloro-4-(trifluoromethyl)thiazol-2-yl]-5-[3,6-dihydro-3-methyl-2,6-dioxo-4-(trifluoromethyl)-1(2H)-pyrimidinyl]- |
| 440 | 1,2-Benzisothiazole-3-carboxamide, N-(benzylsulfonyl)-5-[3,6-dihydro-3-methyl-2,6-dioxo-4-(trifluoromethyl)-1(2H)-pyrimidinyl]-N-(ethoxymethyl)- |
| 441 | Glycine, N-(benzylsulfonyl)-N-{{5-[3,6-dihydro-3-methyl-2,6-dioxo-4-(trifluoromethyl)-1(2H)-pyrimidinyl]-1,2-benzisothiazol-3-yl}carbonyl}-, methyl ester |
| 442 | 2,4(1H,3H)-Pyrimidinedione, 3-[6-chloro-3-(6-methoxy- *m* -tolyl)-1,2-benzisothiazol-5-yl]-1-methyl-6-(trifluoromethyl)- |
| 443 | Acetic acid, {{2-{6-chloro-5-[3,6-dihydro-3-methyl-2,6-dioxo-4-(trifluoromethyl)-1(2H)-pyrimidinyl]-1,2-benzisothiazol-3-yl}- *p* -tolyl}oxy}-, methyl ester |
| 444 | 1,2-Benzisothiazole-3-acetonitrile, 5-[3,6-dihydro-3-methyl-2,6-dioxo-4-(trifluoromethyl)-1(2H)-pyrimidinyl]-alpha-ethyl- |
| 445 | 1,2-Benzisothiazole-3-acetonitrile, 5-[3,6-dihydro-3-methyl-2,6-dioxo-4-(trifluoromethyl)-1(2H)-pyrimidinyl]-alpha,alpha-diethyl- |
| 446 | Cyclopropanecarbonitrile, 1-{5-[3,6-dihydro-3-methyl-2,6-dioxo-4-(trifluoromethyl)-1(2H)-pyrimidinyl]-1,2-benzisothiazol-3-yl}- |
| 447 | 2,4(1H,3H)-Pyrimidinedione, 3-[3-(alpha,alpha-dibromo-2-methoxy-*p*-tolyl)-1,2-benzisothiazol-5-yl]-1-methyl-6-(trifluoromethyl)- |
| 448 | 2,4(1H,3H)-Pyrimidinedione, 3-[3-(5-iodo-2-methoxyphenyl)-1,2-benzisothiazol-5-yl]-1-methyl-6-(trifluoromethyl)- |
| 449 | 2,4(1,3H)-Pyrimidinedione, 3-(3-benzoyl-1,2-benzisothiazol-5-yl)-1-methyl-6-(trifluoromethyl)- |
| 450 | 2,4(1H,3H)-Pyrimidinedione, 3-[3-(hydroxymethyl)-1,2-benzisothiazol-5-yl]-1-methyl-6-(trifluoromethyl)-, (*p*-methoxyphenyl)acetate (ester) |
| 451 | 2,4(1H,3H)-Pyrimidinedione, 3-[3-(hydroxymethyl)-1,2-benzisothiazol-5-yl]-1-methyl-6-(trifluoromethyl)-, 2-thienylacetate (ester) |
| 452 | 2,4(1H,3H)-Pyrimidinedione, 3-[3-(hydroxymethyl)-1,2-benzisothiazol-5-yl]-1-methyl-6-(trifluoromethyl)-, (*p*-chlorophenyl)acetate (ester) |
| 453 | 1,2-Benzisothiazole-3-carboxaldehyde, 5-[3,6-dihydro-3-methyl-2,6-dioxo-4-(trifluoromethyl)-1(2H)-pyrimidinyl]-, 3-(dimethyl acetal) |
| 454 | 2,4(1H,3H)-Pyrimidinedione, 3-[3-(hydroxymethyl)-1,2-benzisothiazol-5-yl]-1-methyl-6-(trifluoromethyl)-, (*m*-methoxyphenyl)acetate (ester) |
| 455 | 2,4(1H,3H)-Pyrimidinedione, 3-(3-[(ethoxymethoxy)methyl]-1,2-benzisothiazol-5-yl}-1-methyl-6-(trifluoromethyl)- |
| 456 | 2,4(1H,3H)-Pyrimidinedione, 1-methyl-3-{3-{[(tetrahydro-2H-pyran-2-yl)oxy]methyl}-1,2-benzisothiazol-5-yl}-6-(trifluoromethyl)- |
| 457 | 1,2-Benzisothiazole-3-acetonitrile, 5-[3,6-dihydro-3-methyl-2,6-dioxo-4-(trifluoromethyl)-1(2H)-pyrimidinyl]-alpha-(3-methyl-2-thenylidene)- |
| 458 | 1,2-Benzisothiazole-3-acetonitrile, alpha(cyclopropylmethylene)-5-[3,6-dihydro-3-methyl-2,6-dioxo-4-(trifluoromethyl)-1(2H)-pyrimidinyl]-, (E)- and (Z)- |
| 459 | Acetic acid, {2-{5-[3,6-dihydro-3-methyl-2,6-dioxo-4-(trifluoromethyl)-1(2H)-pyrimidinyl]-1,2-benzisothiazol-3-yl}-4-iodophenoxy}-, methyl ester |
| 460 | Propionic acid, 2-{2-{5-[3,6-dihydro-3-methyl-2,6-dioxo-4-(trifluoromethyl)-1(2H)-pyrimidinyl]-1,2-benzisothiazol-3-yl}-4-iodophenoxy}-, methyl ester |
| 461 | 2,4(1H,3H)-Pyrimidinedione, 3-{3-{[(benzyloxy)-methoxy]methyl}-1,2-benzisothiazol-5-yl}-1-methyl-6-(trifluoromethyl)- |
| 462 | 2,4(1H,3H)-Pyrimidinedione, 3-[6-fluoro-3-(6-methoxy-*m*-tolyl)-1,2-benzisothiazol-5-yl]-1-methyl-6-(trifluoromethyl)- |
| 463 | 2,4(1H,3H)-Pyrimidinedione, 3-[3-(hydroxymethyl)-1,2-benzisothiazol-5-yl]-1-methyl-6-(trifluoromethyl)-, isonicotinate (ester) |
| 464 | 1,2-Benzisothiazole-3-carboxamide, 5-[3,6-dihydro-3-methyl-2,6-dioxo-4-(trifluoromethyl)-1(2H)-pyrimidinyl]-N-[(*p*-methylbenzyl)sulfonyl]- |
| 465 | 1,2-Benzisothiazole-3-carboxamide, 5-[3,6-dihydro-3-methyl-2,6-dioxo-4-(trifluoromethyl)-1(2H)-pyrimidinyl]-N-[(*o*-methylbenzyl)sulfonyl]- |
| 466 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl- |
| 467 | Propionaldehyde, 3-{{2-[5-{3,6-dihydro-3-methyl-2,6-dioxo-4-(trifluoromethyl)-1(2H)-pyrimidinyl]-1,2-benzisothiazol-3-yl}-*p*-tolyl}oxy}-, 1-(dimethyl acetal) |
| 468 | 1,2-Benzisothiazole-3-carboxamide, N-[(*p*-chlorobenzyl)sulfonyl]-5-[3,6-dihydro-3-methyl-2,6-dioxo-4-(trifluoromethyl)-1(2H)-pyrimidinyl]- |
| 469 | Acetic acid, {{6-{5-[3,6-dihydro-3-methyl-2,6-dioxo-4-(trifluoromethyl)-1(2H)-pyrimidinyl]-1,2-benzisothiazol-3-yl}-4-iodo- *m* -tolyl}oxy}-, methyl ester |
| 470 | Propionic acid, 2-{{6-{5-[3,6-dihydro-3-methyl-2,6-dioxo-4-(trifluoromethyl)-1(2H)-pyrimidinyl]-1,2-benzisothiazol-3-yl}-4-iodo- *m* -tolyl}oxy}-, methyl ester |
| 471 | 1,2-Benzisothiazole-3-carbohydroxamic acid, 5-[3,6-dihydro-3-methyl-2,6-dioxo-4-(trifluoromethyl)-1(2H)-pyrimidinyl]- |
| 472 | 1,2-Benzisothiazole-3-carboxamide, 5-[3,6-dihydro-3-methyl-2,6-dioxo-4-(trifluoromethyl)-1(2H)-pyrimidinyl]-N-sulfamoyl- |
| 473 | 2,4(1H,3H)-Pyrimidinedione, 3-[3-(hydroxymethyl)-1,2-benzisothiazol-5-yl]-1-methyl-6-(trifluoromethyl)-, 2-phenylbutyrate (ester) |
| 474 | 2,4(1H,3H)-Pyrimidinedione, 3-[3-(hydroxymethyl)-1,2-benzisothiazol-5-yl]-1-methyl-6-(trifluoromethyl)-, 2,6-dichloroisonicotinate (ester) |
| 475 | 2,4(1H,3H)-Pyrimidinedione, 3-[3-(hydroxymethyl)-1,2-benzisothiazol-5-yl]-1-methyl-6-(trifluoromethyl)-, 5-isoxazolecarboxylate (ester) |
| 476 | 2,4(1H,3H)-Pyrimidinedione, 3-[3-(hydroxymethyl)-1,2-benzisothiazol-5-yl]-1-methyl-6-(trifluoromethyl)-, 5-nitro-2-furoate (ester) |
| 477 | 2,4(1H,3H)-Pyrimidinedione, 3-[3-(hydroxymethyl)-1,2-benzisothiazol-5-yl]-1-methyl-6-(trifluoromethyl)-, 2-thiophenecarboxylate (ester) |
| 478 | 2,4(1H,3H)-Pyrimidinedione, 3-{3-[(methoxymethoxy)methyl]-1,2-benzisothiazol-5-yl}-1-methyl-6-(trifluoromethyl)- |
| 479 | Acetic acid, {{6-{5-[3,6-dihydro-3-methyl-2,6-dioxo-4-(trifluoromethyl)-1(2H)-pyrimidinyl]-6-fluoro-1,2-benzisothiazol-3-yl}-*m*-tolyl}oxy}-, methyl ester |
| 480 | 2,4(1H,3H)-Pyrimidinedione, 3-[3-(hydroxymethyl)-1,2-benzisothiazol-5-yl]-1-methyl-6-(trifluoromethyl)-, 2-chloronicotinate (ester) |
| 481 | 2,4(1H,3H)-Pyrimidinedione, 3-[3-(hydroxymethyl)-1,2-benzisothiazol-5-yl]-1-methyl-6-(trifluoromethyl)-, 6-chloronicotinate (ester) |
| 482 | 1,2-Benzisothiazole-3-carboxamide, N-(benzylsulfonyl)-5-[3,6-dihydro-3-methyl-2,6-dioxo-4-(trifluoromethyl)-1(2H)-pyrimidinyl]-6-fluoro- |
| 483 | 1,2-Benzisothiazole-3-carboxamide, 5-[3,6-dihydro-3-methyl-2,6-dioxo-4-(trifluoromethyl)-1(2H)-pyrimidinyl]-N-[(*m*-methylbenzyl)sulfonyl]- |
| 484 | 2,4(1H,3H)-Pyrimidinedione, 1-methyl-3-{3-{[(methylthio)methoxy]methyl}-1,2-benzisothiazol-5-yl}-6-(trifluoromethyl)- |
| 485 | 1,2-Benzisothiazole-3-carboxamide, 5-[3,6-dihydro-3-methyl-2,6-dioxo-4-(trifluoromethyl)-1(2H)-pyrimidinyl]-N-(phenethylsulfonyl)- |
| 486 | 1,2-Benzisothiazole-3-carboxamide, N-[(*m*-chlorobenzyl)sulfonyl]-5-[3,6-dihydro-3-methyl-2,6-dioxo-4-(trifluoromethyl)-1(2H)-pyrimidinyl]- |
| 487 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, furfuryl ester |
| 488 | 1,2-Benzisothiazole-3-acetamide, 5-[3,6-dihydro-3-methyl-2,6-dioxo-4-(trifluoromethyl)-1(2H)-pyrimidinyl]-alpha,alpha-dimethyl-N-(methylsulfonyl)- |
| 489 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, isopropyl ester |
| 490 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, methyl ester |
| 491 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, allyl ester |
| 492 | Propionic acid, 2-{{5-chloro-2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, methyl ester |
| 493 | Acetic acid, {{5-chloro-2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, methyl ester |
| 494 | 1,2-Benzisothiazole-3-carboxamide, N-(benzylsulfonyl)-5-[3,6-dihydro-3-methyl-2,6-dioxo-4-(trifluoromethyl)-1(2H)-pyrimidinyl]-N-methyl- |
| 495 | 2,4(1H,3H)-Pyrimidinedione, 3-[3-(hydroxymethyl)-1,2-benzisothiazol-5-yl]-1-methyl-6-(trifluoromethyl)-, methoxyacetate (ester) |
| 496 | 2,4(1H,3H)-Pyrimidinedione, 3-[3-(hydroxymethyl)-1,2-benzisothiazol-5-yl]-1-methyl-6-(trifluoromethyl)-, cyclopropanecarboxylate (ester) |
| 497 | 2,4(1H,3H)-Pyrimidinedione, 3-[3-(hydroxymethyl)-1,2-benzisothiazol-5-yl]-1-methyl-6-(trifluoromethyl)-, 2-furoate (ester) |
| 498 | 2,4(1H,3H)-Pyrimidinedione, 3-[3-(hydroxymethyl)-1,2-benzisothiazol-5-yl]-1-methyl-6-(trifluoromethyl)-, pivalate (ester) |
| 499 | 2,4(1H,3H)-Pyrimidinedione, 3-(3-isopropyl-1,2-benzisothiazol-5-yl)-1-methyl-6-(trifluoromethyl)- |
| 500 | 1,2-Benzisothiazole-3-carboxamide, 5-[3,6-dihydro-3-methyl-2,6-dioxo-4-(trifluoromethyl)-1(2H)-pyrimidinyl)-N-(diisopropylsulfamoyl)- |
| 501 | 1,2-Benzisothiazole-3-carboxamide, 5-[3,6-dihydro-3-methyl-2,6-dioxo-4-(trifluoromethyl)-1(2H)-pyrimidinyl]-N-(styrylsulfonyl)-, (E)- |
| 502 | 2,4(1H,3H)-Pyrimidinedione, 3-[3-(hydroxymethyl)-1,2-benzisothiazol-5-yl]-1-methyl-6-(trifluoromethyl)-, (3,4-dimethoxyphenyl)acetate (ester) |
| 503 | 2,4(1H,3H)-Pyrimidinedione, 3-[3-(hydroxymethyl)-1,2-benzisothiazol-5-yl]-1-methyl-6-(trifluoromethyl)-, (2,5-dimethoxyphenyl)acetate (ester) |
| 504 | 2,4(1H,3H)-Pyrimidinedione, 3-[3-(hydroxymethyl)-1,2-benzisothiazol-5-yl]-1-methyl-6-(trifluoromethyl)-, chlorophenylacetate (ester) |
| 505 | 2,4(1H,3H)-Pyrimidinedione, 3-{3-[6-(allyloxy)-5-iodo-*m*-tolyl]-1,2-benzisothiazol-5-yl}-1-methyl-6-(trifluoromethyl)- |
| 506 | 2,4(1H,3H)-Pyrimidinedione, 3-[3-(4-chloro-6-methoxy-*m*-tolyl)-1,2-benzisothiazol-5-yl]-1-methyl-6-(trifluoromethyl)- |
| 507 | 1,2-Benzisothiazole-3-carboxamide, 5-[3,6-dihydro-3-methyl-2,6-dioxo-4-(trifluoromethyl)-1(2H)-pyrimidinyl]-N-(dimethylsulfamoyl)- |
| 508 | 1,2-Benzisothiazole-3-carboxamide, 5-[3,6-dihydro-3-methyl-2,6-dioxo-4-(trifluoromethyl)-1(2H)-pyrimidinyl]-N-(isopropylsulfamoyl)- |
| 509 | 1,2-Benzisothiazole-3-acetamide, 5-[3,6-dihydro-3-methyl-2,6-dioxo-4-(trifluoromethyl)-1(2H)-pyrimidinyl]-alpha,alpha-dimethyl- |
| 510 | 1,2-Benzisothiazole-3-acetic acid, 5-[3,6-dihydro-3-methyl-2,6-dioxo-4-(trifluoromethyl)-1(2H)-pyrimidinyl]-alpha,alpha-dimethyl-, 2-pyridylmethyl ester |
| 511 | 1,2-Benzisothiazole-3-acetamide, 5-[3,6-dihydro-3-methyl-2,6-dioxo-4-(trifluoromethyl)-1(2H)-pyrimidinyl]-N-(diisopropylsulfamoyl)-alpha, alpha-dimethyl- |
| 512 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(diisopropyl-sulfamoyl)- |
| 513 | 1,2-Benzisothiazole-3-carboxylic acid, 5-[3,6-dihydro-3-methyl-2,6-dioxo-4-(trifluoromethyl)-1(2H)-pyrimidinyl]-6-fluoro-, methyl ester |
| 514 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, tert-butyl ester |
| 515 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl- |
| 516 | 2,4(1H,3H)-Pyrimidinedione, 1-(2-fluoroethyl)-3-(3-methyl-1,2-benzisothiazol-5-yl)-6-(trifluoromethyl)- |
| 517 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, (1-phenylcyclopropyl)methyl ester |
| 518 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 4-(methylthio)butyl ester |
| 519 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, nonyl ester |
| 520 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 1-vinylpentyl ester |
| 521 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 2,2-bis-[(allyloxy)methyl]butyl ester |
| 522 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 3-decynyl ester |
| 523 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 3-(3-methoxypropoxy)propyl ester |
| 524 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, ester with ethyl 4-hydroxycyclohexanecarboxylate, isomer A |
| 525 | Acetic acid, {{2-{5-[3,6-dihydro-3-methyl-2,6-dioxo-4-(trifluoromethyl)-1(2H)-pyrimidinyl)-1,2-benzisothiazol-3-yl}-*p*-tolyl}oxy}-, ester with ethyl 4-hydroxycyclohexanecarboxylate, isomer B |
| 526 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 11-tetradecenyl ester, (E)- |
| 527 | Acetic acid, {{2-{5-[3,6-dihydro-3-methyl-2,6-dioxo-4-(trifluoromethyl)-1(2H)-pyrimidinyl)-1,2-benzisothiazol-3-yl}-*p*-tolyl}oxy}-, 2,2-dimethyl-3-phenylpropyl ester |
| 528 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 2-propynyl ester |
| 529 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 2-butenyl ester |
| 530 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, allyl ester |
| 531 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 1-methyl-2-propynyl ester |
| 532 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 2-methoxy-1-methylethyl ester |
| 533 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 2-(5,5-dimethyl-*m*-dioxan-2-yl)ethyl ester |
| 534 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 5-(*tert*-butyldimethylsiloxy)pentyl ester |
| 535 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, (tetrahydro-3-furyl)methyl ester, DL- |
| 536 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 3-(3-methoxypropoxy)propyl ester |
| 537 | 2,4(1H,3H)-Pyrimidinedione, 1-methyl-3-{3-[6-(phenacyloxy)- *m* -tolyl]-1,2-benzisothiazol-5-yl}-6-(trifluoromethyl)- |
| 538 | 2,4(1H,3H)-Pyrimidinedione, 3-{3-{6-[(*p*-chlorophenacyl)oxy]-*m*-tolyl}-1,2-benzisothiazol-5-yl}-1-methyl-6-(trifluoromethyl)- |
| 539 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}- |
| 540 | 2,4(1H,3H)-Pyrimidinedione, 3-{3-[6-(3,3-dimethyl-2-oxobutoxy)-*m*-tolyl]-1,2-benzisothiazol-5-yl}-1-methyl-6-(trifluoromethyl)- |
| 541 | Benzoic acid, *p*-{2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionamido}-, ethyl ester |
| 542 | 2,4(1H,3H)-Pyrimidinedione, 3-{3-{6-[1-(3,4-dimethylbenzoyl)ethoxy]-*m*-tolyl}-1,2-benzisothiazol-5-yl}-1-methyl-6-(trifluoromethyl)- |
| 543 | 2,4(1H,3H)-Pyrimidinedione, 3-{3-{6-{[*p*-(difluoromethoxy)phenacyl]oxy}-*m*-tolyl}-1,2-benzisothiazol-5-yl}-1-methyl-6-(trifluoromethyl)- |
| 544 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 2-chloroethyl ester |
| 545 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 2,2,2-trifluoroethyl ester |
| 546 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, 2-methoxyethyl ester |
| 547 | Propionic acid, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-, isopropyl ester |
| 548 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}- |
| 549 | 2,4(1H,3H)-Pyrimidinedione, 3-{3-[o-(3,3-dimethyl-2-oxobutoxy)phenyl)-1,2-benzisothiazol-5-yl}-1-methyl-6-(trifluoromethyl)- |
| 550 | 2,4(1H,3H)-Pyrimidinedione, 3-{-3-{*o*-[1-(3,4-dimethylbenzoyl)ethoxy]phenyl}-1,2-benzisothiazol-5-yl]-1-methyl-6-(trifluoromethyl)- |
| 551 | Propionamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N,N-dimethyl- |
| 552 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(propylsulfonyl)- |
| 553 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(vinylsulfonyl)- |
| 554 | Acetamide, 2-{{2-{5-[3,6-dihydro-3-methyl-2,6-dioxo-4-(trifluoromethyl)-1(2H)-pyrimidinyl)-1,2-benzisothiazol-3-yl}-*p*-tolyl}oxy}-N-(ethylsulfonyl)- |
| 555 | Acetamide, 2-{*o*-{5-[3,6-dihydro-3-methyl-2,6-dioxo-4-(trifluoromethyl)-1(2H)-pyrimidinyl)-1,2-benzisothiazol-3-yl}phenoxy}-N-(isopropylsulfonyl)- |
| 556 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(propylsulfonyl)- |
| 557 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(vinylsulfonyl)- |
| 558 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(ethylsulfonyl)- |
| 559 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}difluoro-, ethyl ester |
| 560 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(isopropylsulfonyl)- |
| 561 | Acetamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl- |
| 562 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-methyl- |
| 563 | Acetamide, 2-{*o*-{5-[3,6-dihydro-3-methyl-2,6-dioxo-4-(trifluoromethyl)-1(2H)-pyrimidinyl]-1,2-benzisothiazol-3-yl}phenoxy}- |
| 564 | 2,4(1H,3H)-Pyrimidinedione, 1-methyl-3-[3-(3-oxo-3-phenylpropenyl)-1,2-benzisothiazol-5-yl]-6-(trifluoromethyl)-, (E)- |
| 565 | 1,2-Benzisothiazole-3-acrylamide, 5-[3,6-dihydro-3-methyl-2,6-dioxo-4-(trifluoromethyl)-1(2H)-pyrimidinyl]-N-methoxy-N-methyl-, (E)- |
| 566 | 1,2-Benzisothiazole-3-acrylic acid, 5-[3,6-dihydro-3-methyl-2,6-dioxo-4-(trifluoromethyl)-1(2H)-pyrimidinyl]-, allyl ester, (E)- |
| 567 | 1,2-Benzisothiazole-3-acrylaldehyde, 5-[3,6-dihydro-3-methyl-2,6-dioxo-4-(trifluoromethyl)-1(2H)-pyrimidinyl]-alpha-ethyl-, (E)- |
| 568 | Propionamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(isopropylsulfonyl)- |
| 569 | Propionamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(propyl-sulfonyl)- |
| 570 | Propionamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(vinylsulfonyl)- |
| 571 | Propionamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-(ethylsulfonyl) |
| 572 | Propionamide, 2-{{2-{5-[3,6-dihydro-3-methyl-2,6-dioxo-4-(trifluoromethyl)-1(2H)-pyrimidinyl)-1,2-benzisothiazol-3-yl}-*p*-tolyl}oxy}-N-(methylsulfonyl)- |
| 573 | Acetamide, N-(butylsulfonyl)-2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}- |
| 574 | Acetamide, N-(butylsulfonyl)-2-{*o*-{5-[3,6-dihydro-3-methyl-2,6-dioxo-4-(trifluoromethyl)-1(2H)-pyrimidinyl]-1,2-benzisothiazol-3-yl}-phenoxy}- |
| 575 | Propionamide, N-(butylsulfonyl)-2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}- |
| 576 | Propionamide, 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-2-methyl-N-(methylsulfonyl)- |
| 577 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 2-oxobutyl ester |
| 578 | Acetic acid, {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-, 2-hydroxyethyl ester, acetate (ester) |
| 579 | Glycine, N-{{5-[3,6-dihydro-3-methyl-2,6-dioxo-4-(trifluoromethyl)-1(2H)-pyrimidinyl]-1,2-benzisothiazol-3-yl}carbonyl}- |
| 580 | 1,2-Benzisothiazole-3-acetonitrile, alpha,alpha-diallyl-5-[3,6-dihydro-3-methyl-2,6-dioxo-4-(trifluoromethyl)-1(2H)-pyrimidinyl]- |
| 581 | 1,2-Benzisothiazole-3-acetonitrile, 5-[3,6-dihydro-3-methyl-2,6-dioxo-4-(trifluoromethyl)-1(2H)-pyrimidinyl]-alpha,alpha-di-2-propynyl- |
| 582 | 1,2-Benzisothiazole-3-acetonitrile, alpha,alpha-dibenzyl-5-[3,6-dihydro-3-methyl-2,6-dioxo-4-(trifluoromethyl)-1(2H)-pyrimidinyl]- |
| 583 | 1,2-Benzisothiazole-3-acetonitrile, 5-[3,6-dihydro-3-methyl-2,6-dioxo-4-(trifluoromethyl)-1(2H)-pyrimidinyl]-alpha-methyl- |
| 584 | 2,4(1H,3H)-Pyrimidinedione, 3-{3-{[(*p*-chlorobenzyl)oxy]methyl}-1,2-benzisothiazol-5-yl}-1-methyl-6-(trifluoromethyl)- |
| 585 | 2,4(1H,3H)-Pyrimidinedione, 3-{3-{[(2,4-dichlorobenzyl)oxy]methyl}-1,2-benzisothiazol-5-yl}-1-methyl-6-(trifluoromethyl)- |
| 586 | 2,4(1H,3H)-Pyrimidinedione, 3-{3-{[(3,4-dichlorobenzyl)oxy]methyl}-1,2-benzisothiazol-5-yl}-1-methyl-6-(trifluoromethyl)- |
| 587 | 2,4(1H,3H)-Pyrimidinedione, 1-methyl-3-{3-{[(*m*-methylbenzyl)oxy]methyl}-1,2-benzisothiazol-5-yl}-6-(trifluoromethyl)- |
| 588 | 2,4(1H,3H)-Pyrimidinedione, 1-methyl-3-{3-{[(*m*-nitrobenzyl)oxy]methyl}-1,2-benzisothiazol-5-yl}-6-(trifluoromethyl)- |
| 589 | 2,4(1H,3H)-Pyrimidinedione, 1-methyl-3-{3-{[(*p*-nitrobenzyl)oxy]methyl}-1,2-benzisothiazol-5-yl}-6-(trifluoromethyl)- |
| 590 | 2,4(1H,3H)-Pyrimidinedione, 1-methyl-3-{3-[1-(methylsulfonyl)ethyl}-1,2-benzisothiazol-5-yl}-6-(trifluoromethyl)- |
| 591 | 1,2-Benzisothiazole-3-acetonitrile, 5-[3,6-dihydro-3-methyl-2,6-dioxo-4-(trifluoromethyl)-1(2H)-pyrimidinyl]-alpha-(2-methylpropylidene)- |
| 592 | 1,2-Benzisothiazole-3-acetonitrile, 5-[3,6-dihydro-3-methyl-2,6-dioxo-4-(trifluoromethyl)-1(2H)-pyrimidinyl]-alpha-(2-pyridylmethylene)- |
| 593 | 1,2-Benzisothiazole-3-acetonitrile, 5-[3,6-dihydro-3-methyl-2,6-dioxo-4-(trifluoromethyl)-1(2H)-pyrimidinyl]-alpha-(3-furylmethylene)- |
| 594 | 1,2-Benzisothiazole-3-acetonitrile, 5-[3,6-dihydro-3-methyl-2,6-dioxo-4-(trifluoromethyl)-1(2H)-pyrimidinyl]-alpha-furfurylidene- |
| 595 | 1,2-Benzisothiazole-3-acetonitrile, 5-[3,6-dihydro-3-methyl-2,6-dioxo-4-(trifluoromethyl)-1(2H)-pyrimidinyl]-alpha-(imidazol-5-ylmethylene)- |
| 596 | 1,2-Benzisothiazole-3-acetonitrile, 5-[3,6-dihydro-3-methyl-2,6-dioxo-4-(trifluoromethyl)-1(2H)-pyrimidinyl]-alpha-(4-pyridylmethylene)- |
| 597 | 1,2-Benzisothiazole-3-acetonitrile, 5-[3,6-dihydro-3-methyl-2,6-dioxo-4-(trifluoromethyl)-1(2H)-pyrimidinyl]-alpha-(*p*-methylbenzylidene)- |
| 598 | 1,2-Benzisothiazole-3-acetonitrile, 5-[3,6-dihydro-3-methyl-2,6-dioxo-4-(trifluoromethyl)-1(2H)-pyrimidinyl]-alpha-(pyrrol-2-ylmethylene)- |
| 599 | 1,2-Benzisothiazole-3-acetonitrile, 5-[3,6-dihydro-3-methyl-2,6-dioxo-4-(trifluoromethyl)-1(2H)-pyrimidinyl]-alpha-(*o*-methoxybenzylidene)- |
| 600 | 1,2-Benzisothiazole-3-acetonitrile, 5-[3,6-dihydro-3-methyl-2,6-dioxo-4-(trifluoromethyl)-1(2H)-pyrimidinyl]-alpha-(*p*-hydroxybenzylidene)- |
| 601 | 1,2-Benzisothiazole-3-acetonitrile, 5-[3,6-dihydro-3-methyl-2,6-dioxo-4-(trifluoromethyl)-1(2H)-pyrimidinyl]-alpha-(indol-2-ylmethylene)- |
| 602 | 2,4(1H,3H)-Pyrimidinedione, 1-methyl-3-{3-[1-(methylsulfonyl)-3-butenyl]-1,2-benzisothiazol-5-yl}-6-(trifluoromethyl)- |
| 603 | 2,4(1H,3H)-Pyrimidinedione, 1-methyl-3-{3-[1-(methylsulfonyl)-3-butynyl]-1,2-benzisothiazol-5-yl}-6-(trifluoromethyl)- |
| 604 | 2,4(1H,3H)-Pyrimidinedione, 1-methyl-3-{3-[alpha(methylsulfonyl)phenethyl]-1,2-benzisothiazol-5-yl}-6-(trifluoromethyl)- |
| 605 | 1,2-Benzisothiazole-3-acetonitrile, 5-[3,6-dihydro-3-methyl-2,6-dioxo-4-(trifluoromethyl)-1(2H)-pyrimidinyl]-alpha-(*o*-fluorobenzylidene)- |
| 606 | 1,2-Benzisothiazole-3-acetonitrile, alpha-(*o*-cyanobenzylidene)-5-[3,6-dihydro-3-methyl-2,6-dioxo-4-(trifluoromethyl)-1(2H)-pyrimidinyl]-, (E)- and (Z)- |
| 607 | 1,2-Benzisothiazole-3-acetonitrile, alpha(cyclopentylmethylene)-5-[3,6-dihydro-3-methyl-2,6-dioxo-4-(trifluoromethyl)-1(2H)-pyrimidinyl]-, (E)- and (Z)- |

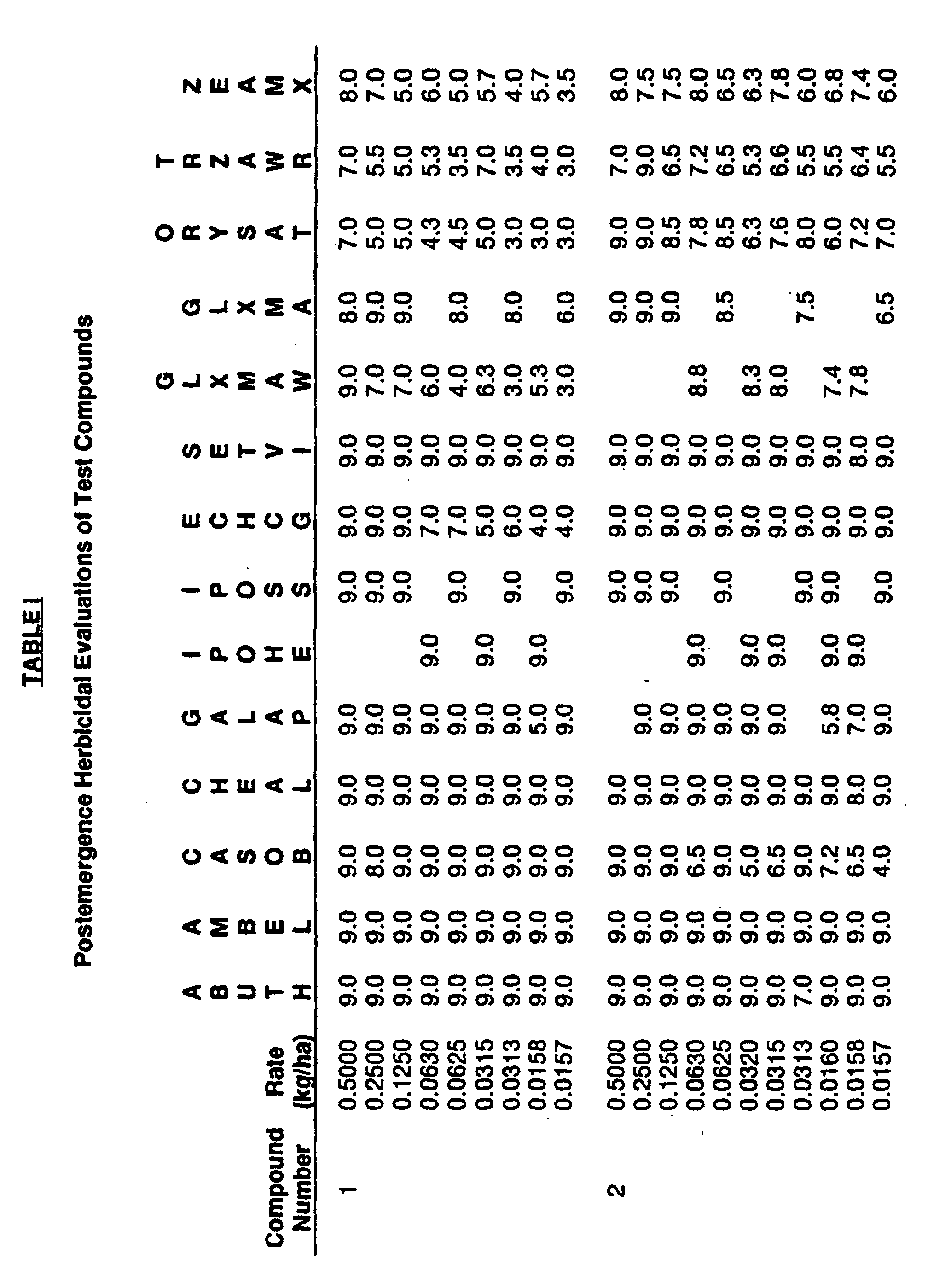

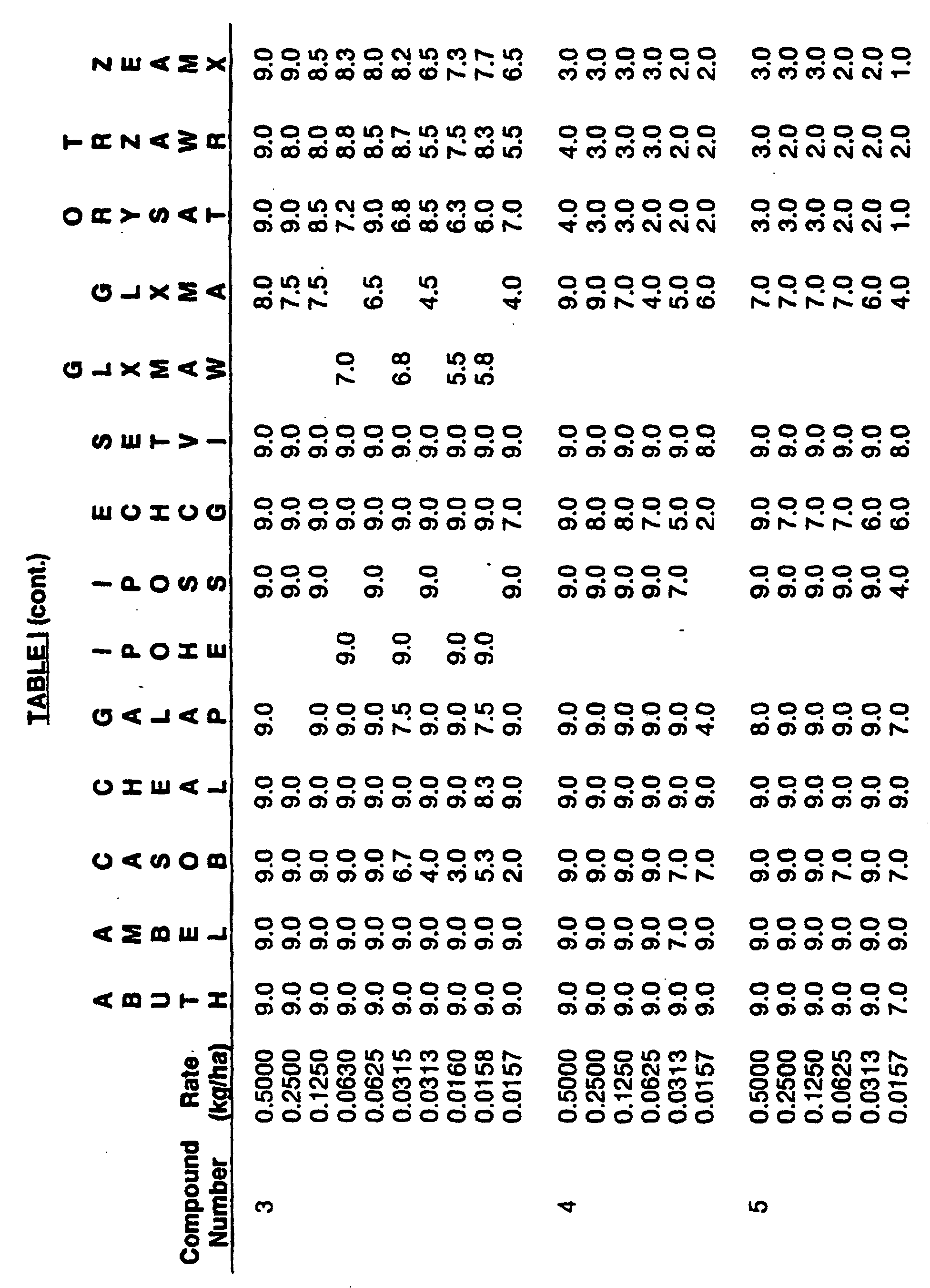

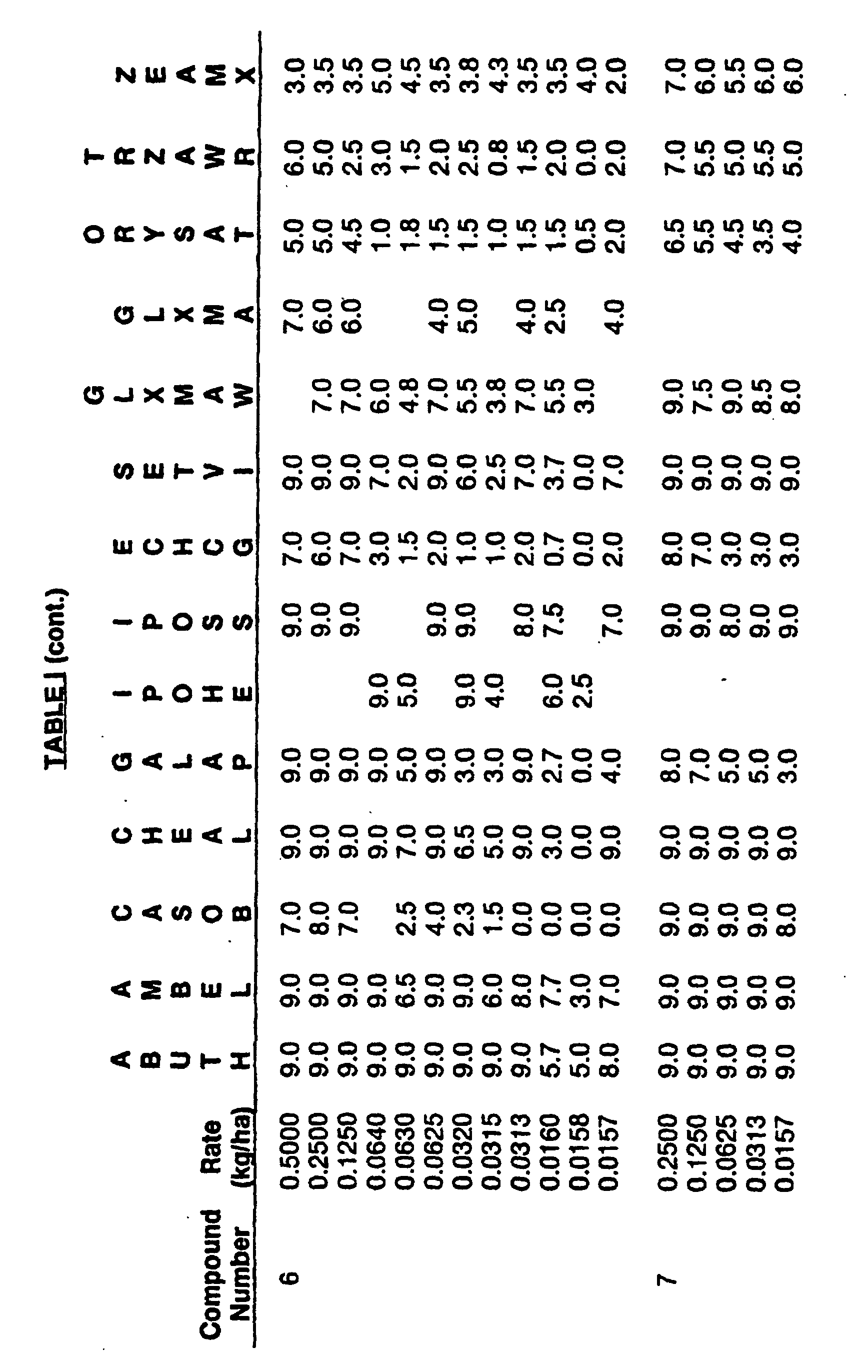

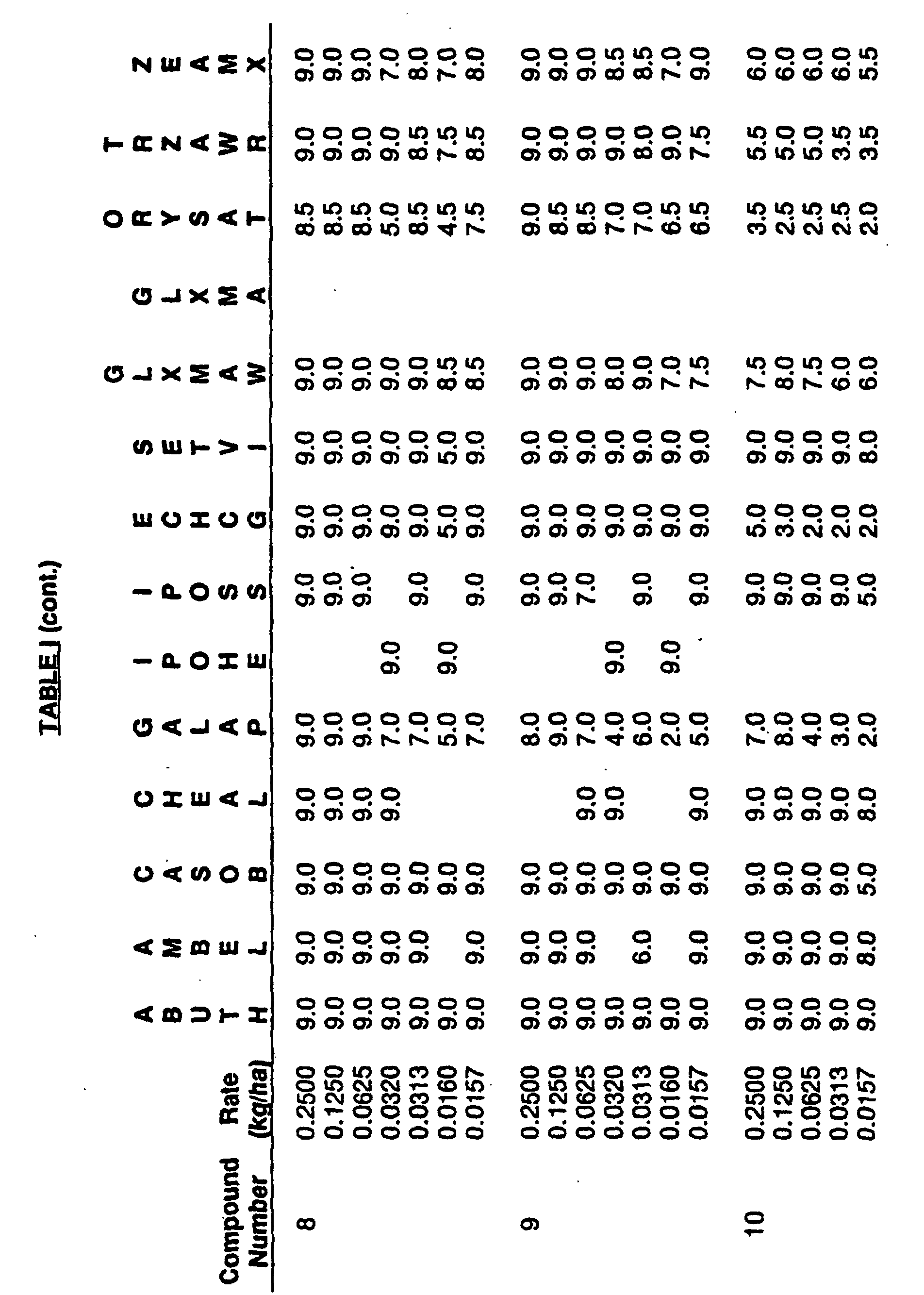

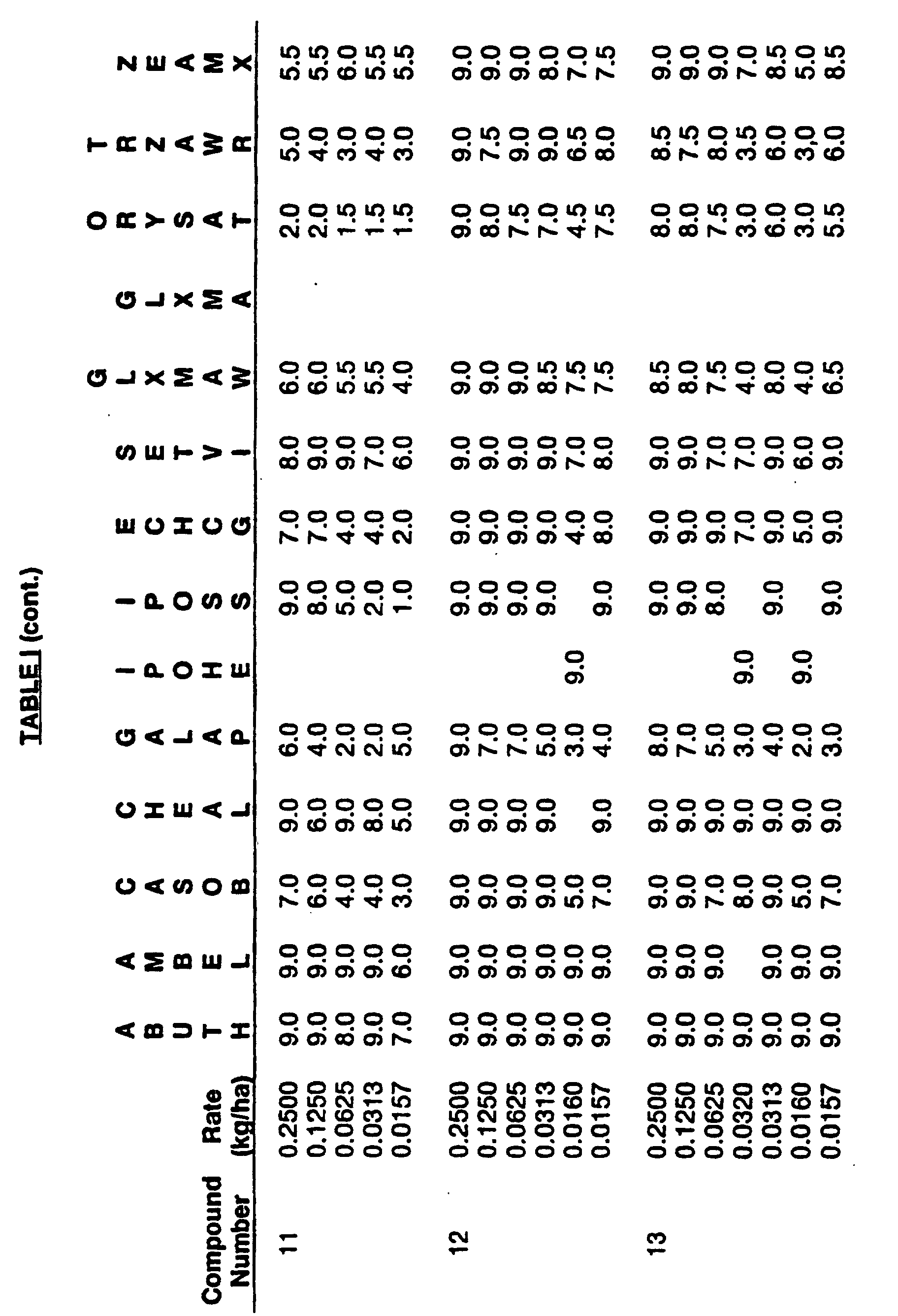

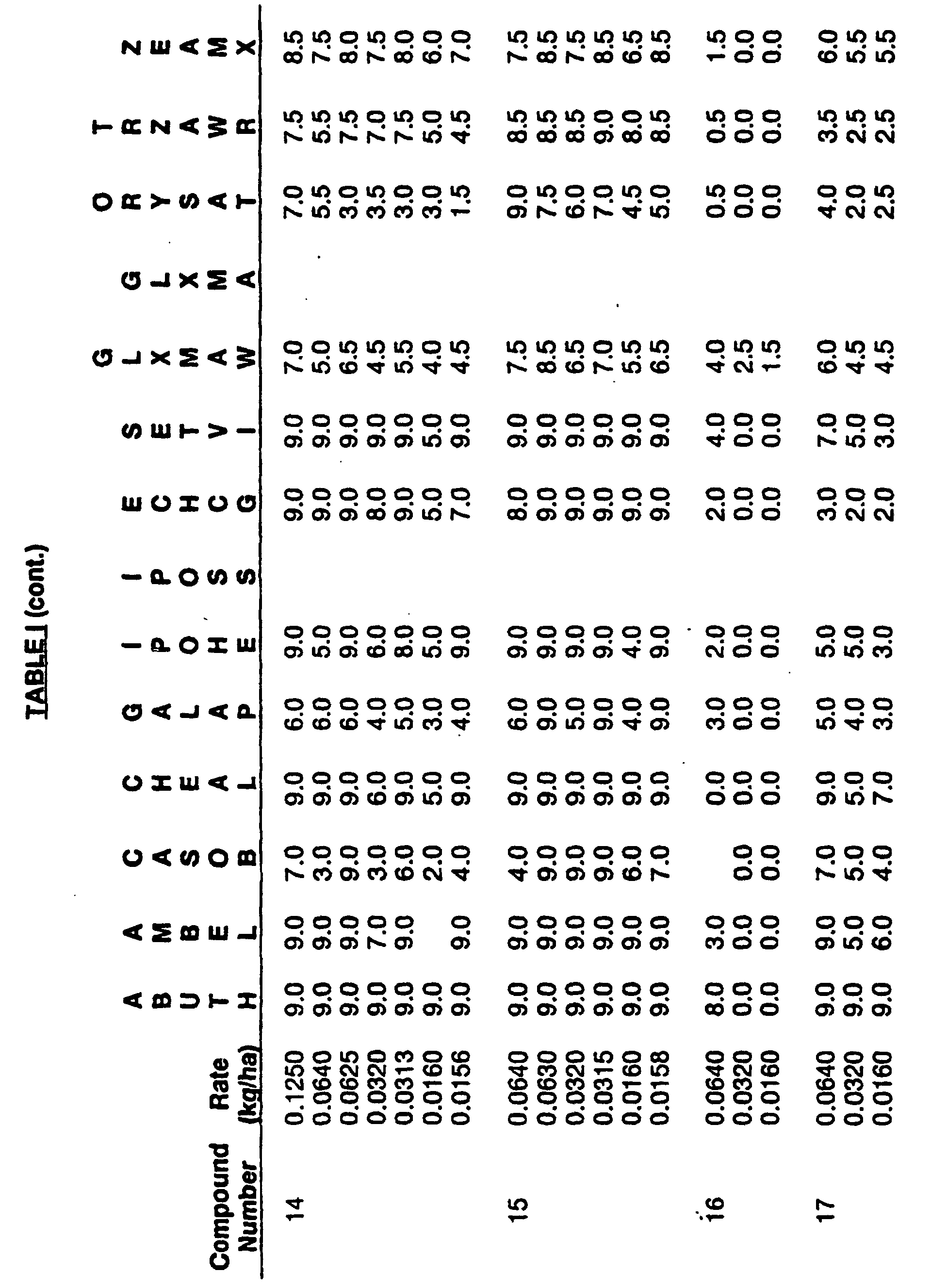

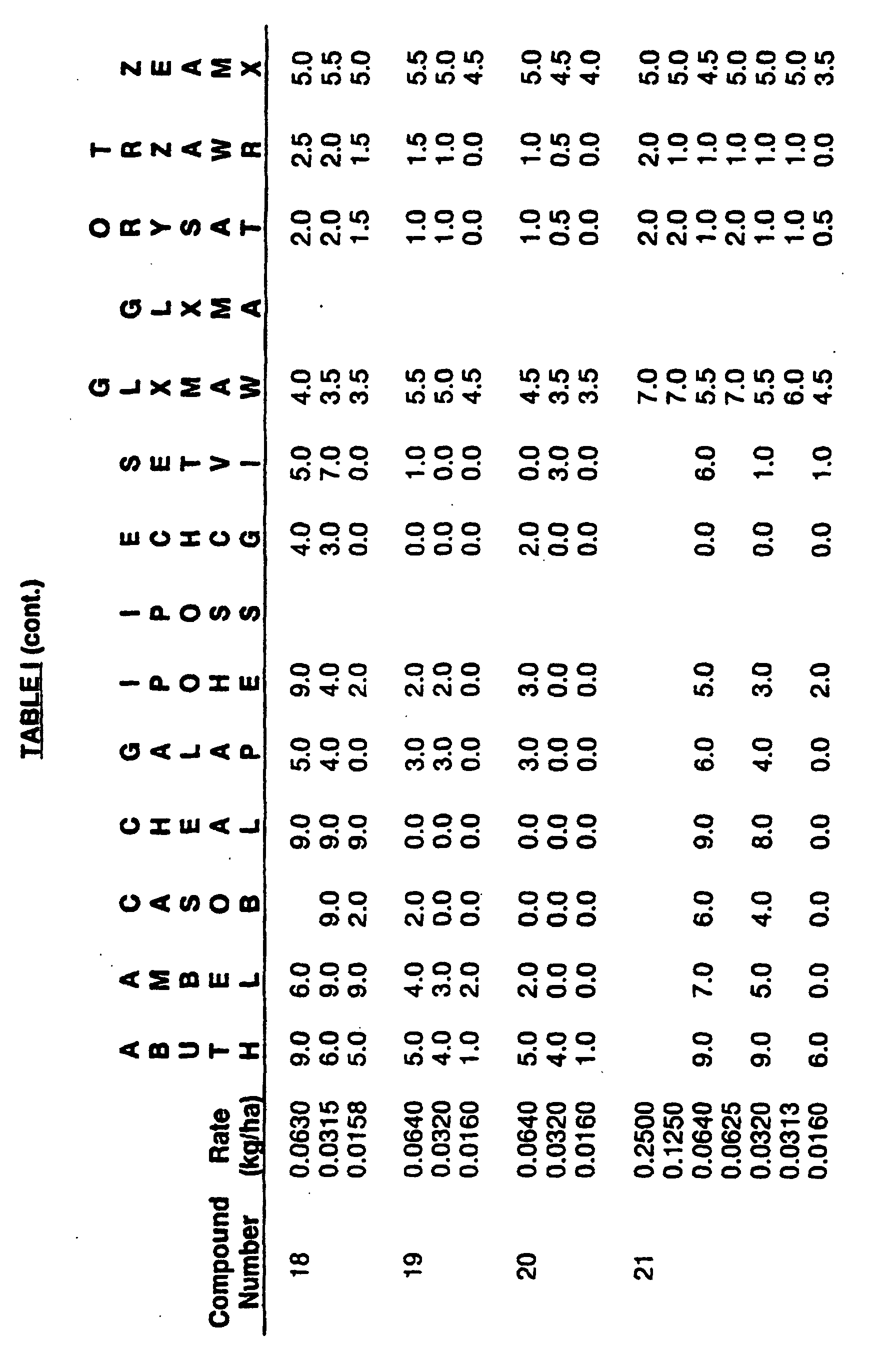

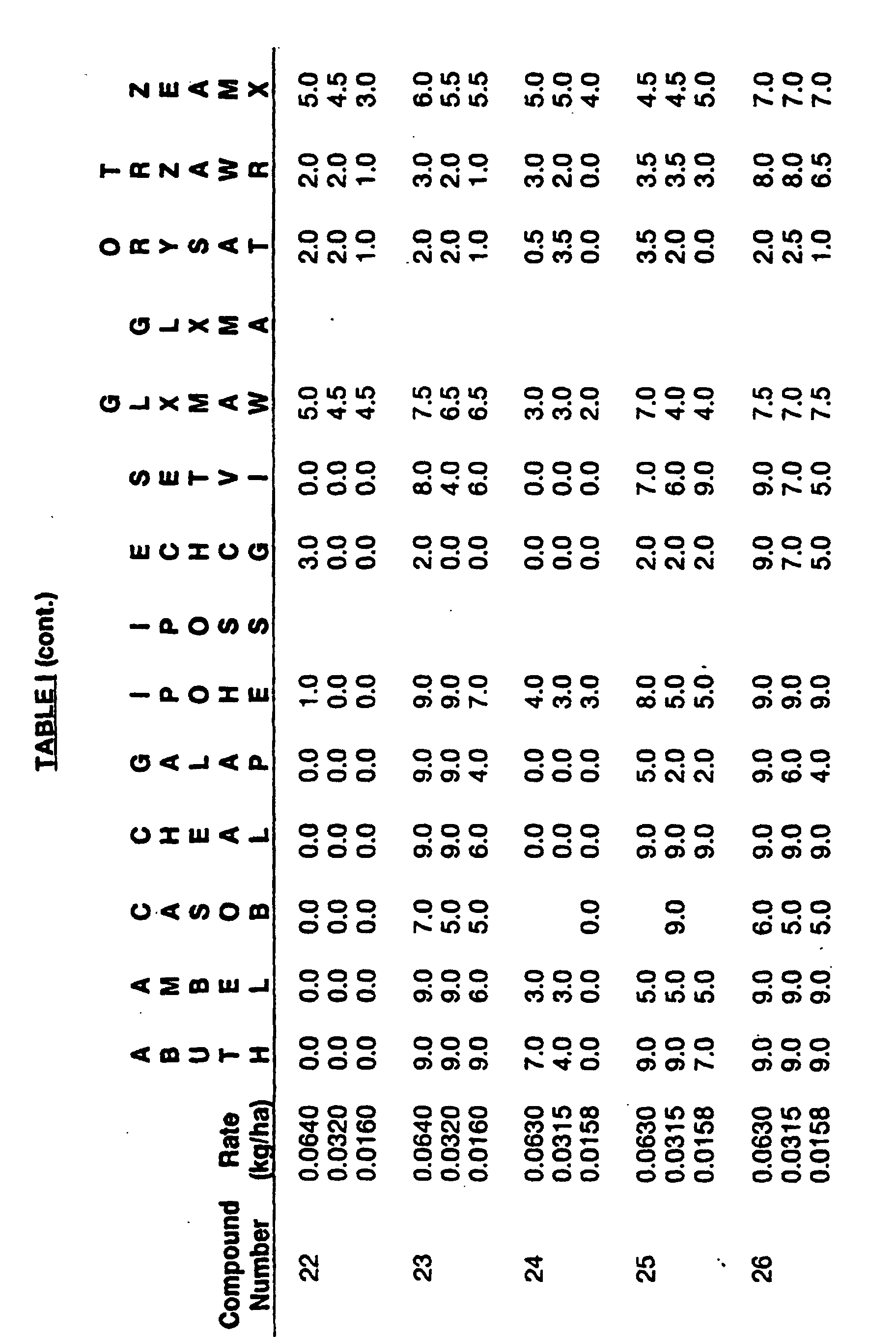

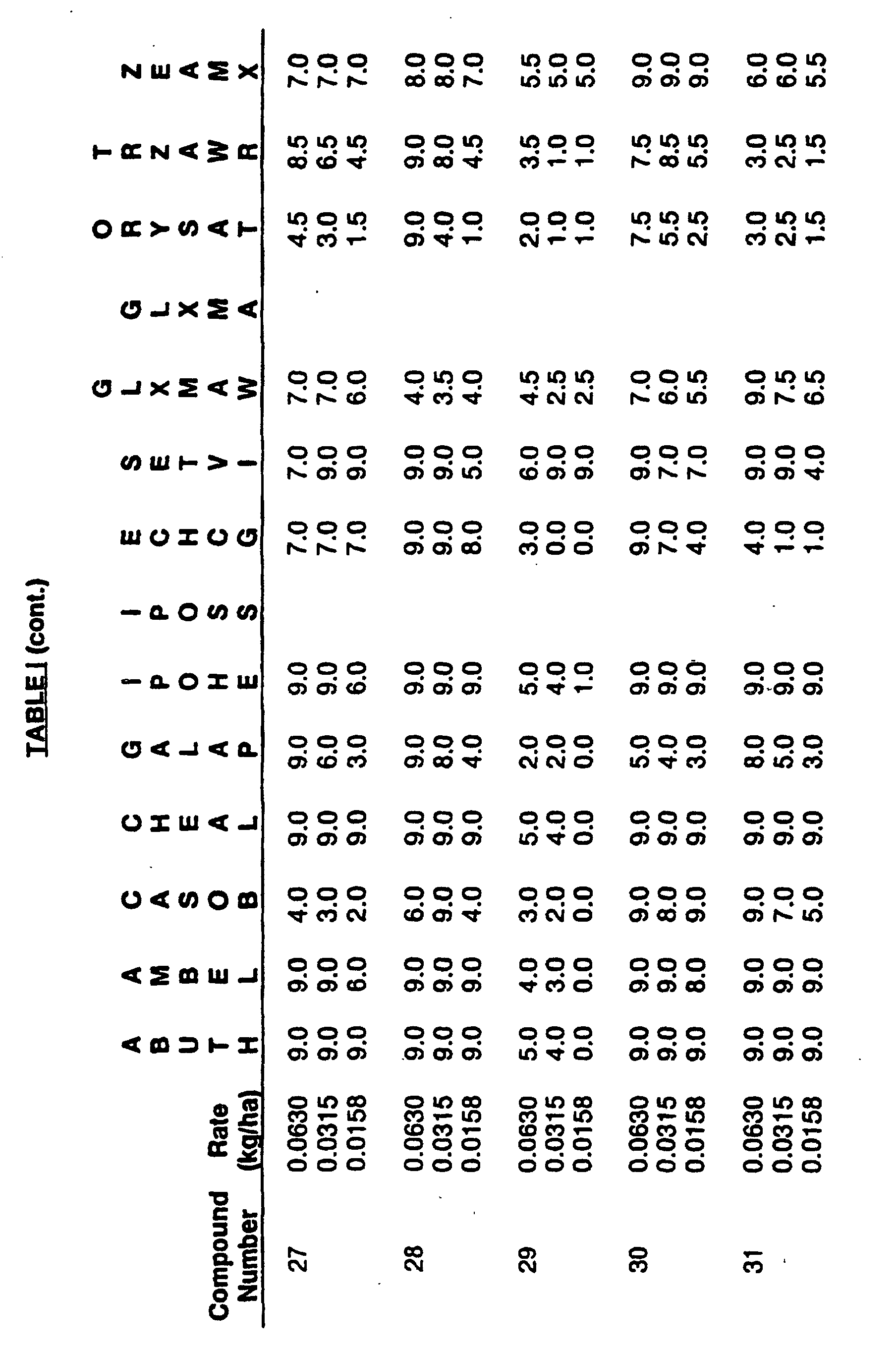

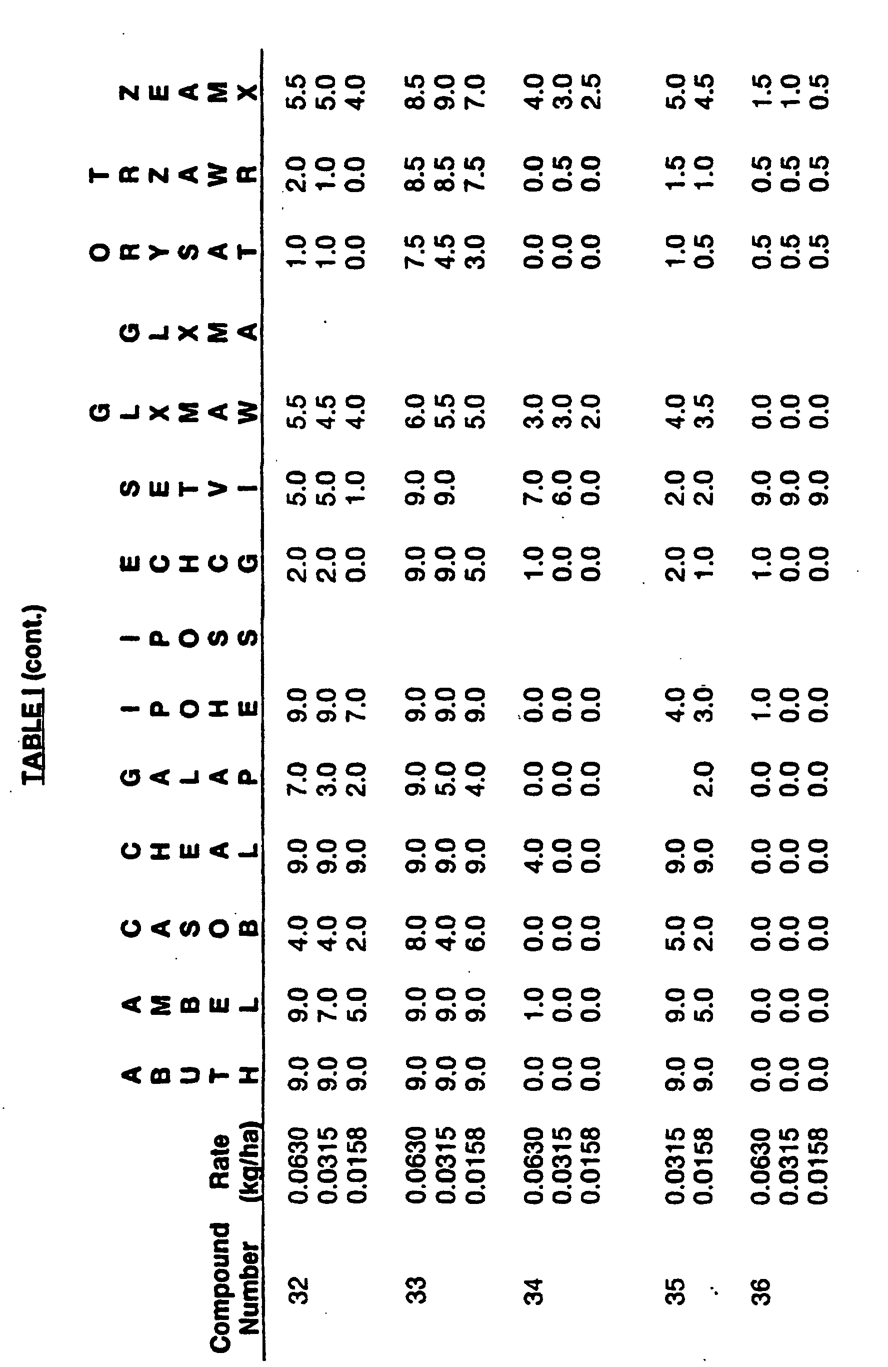

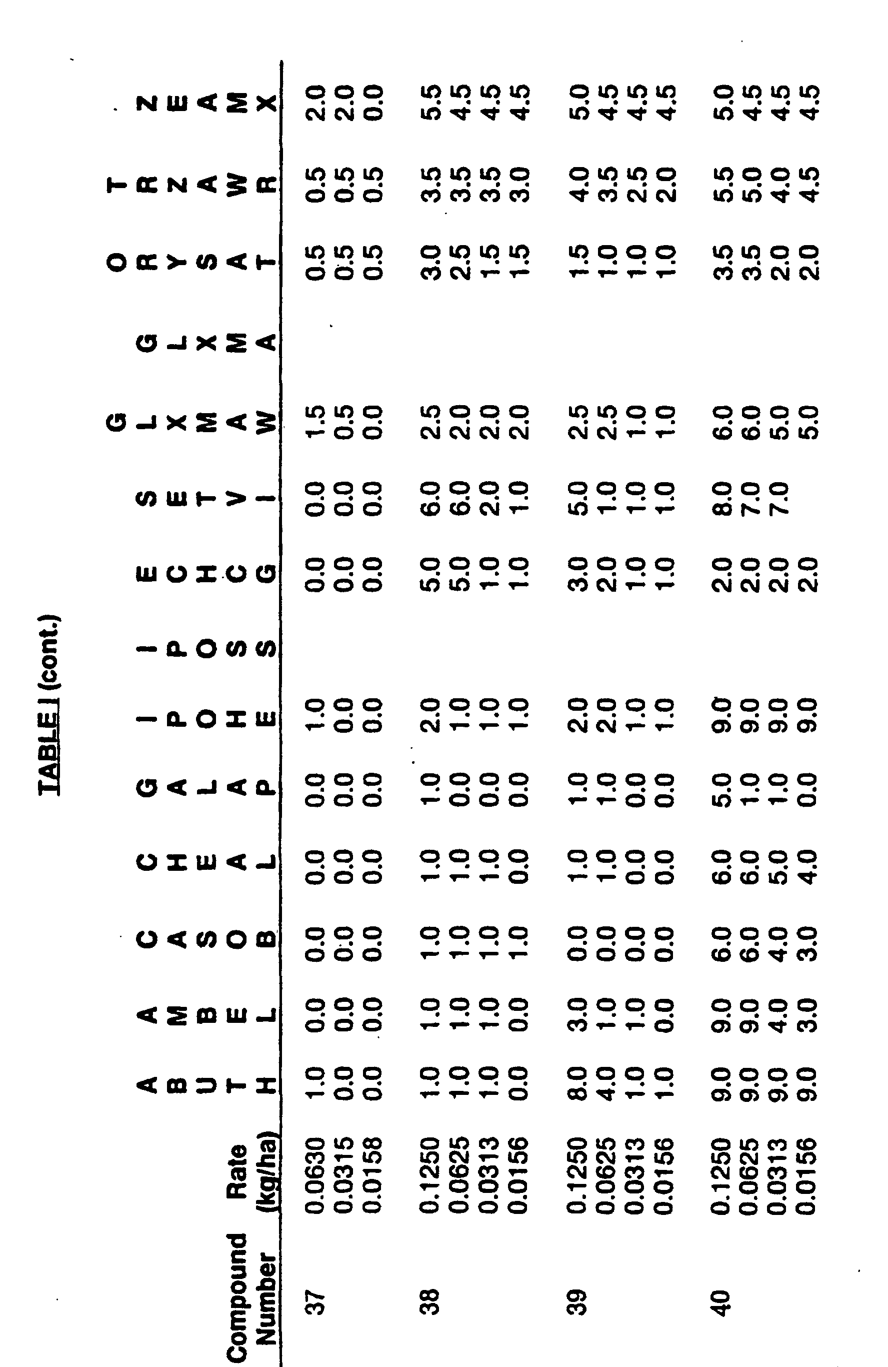

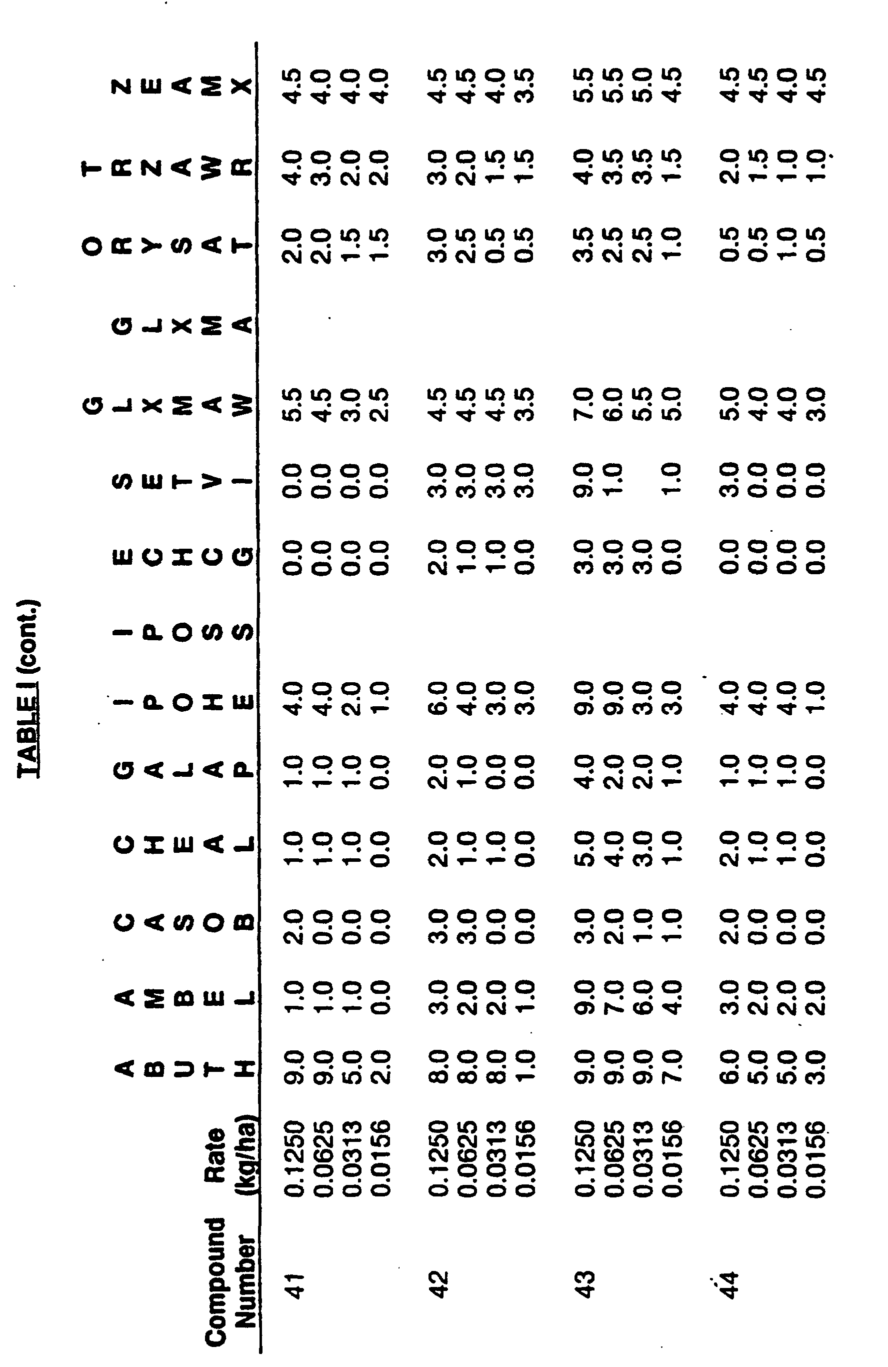

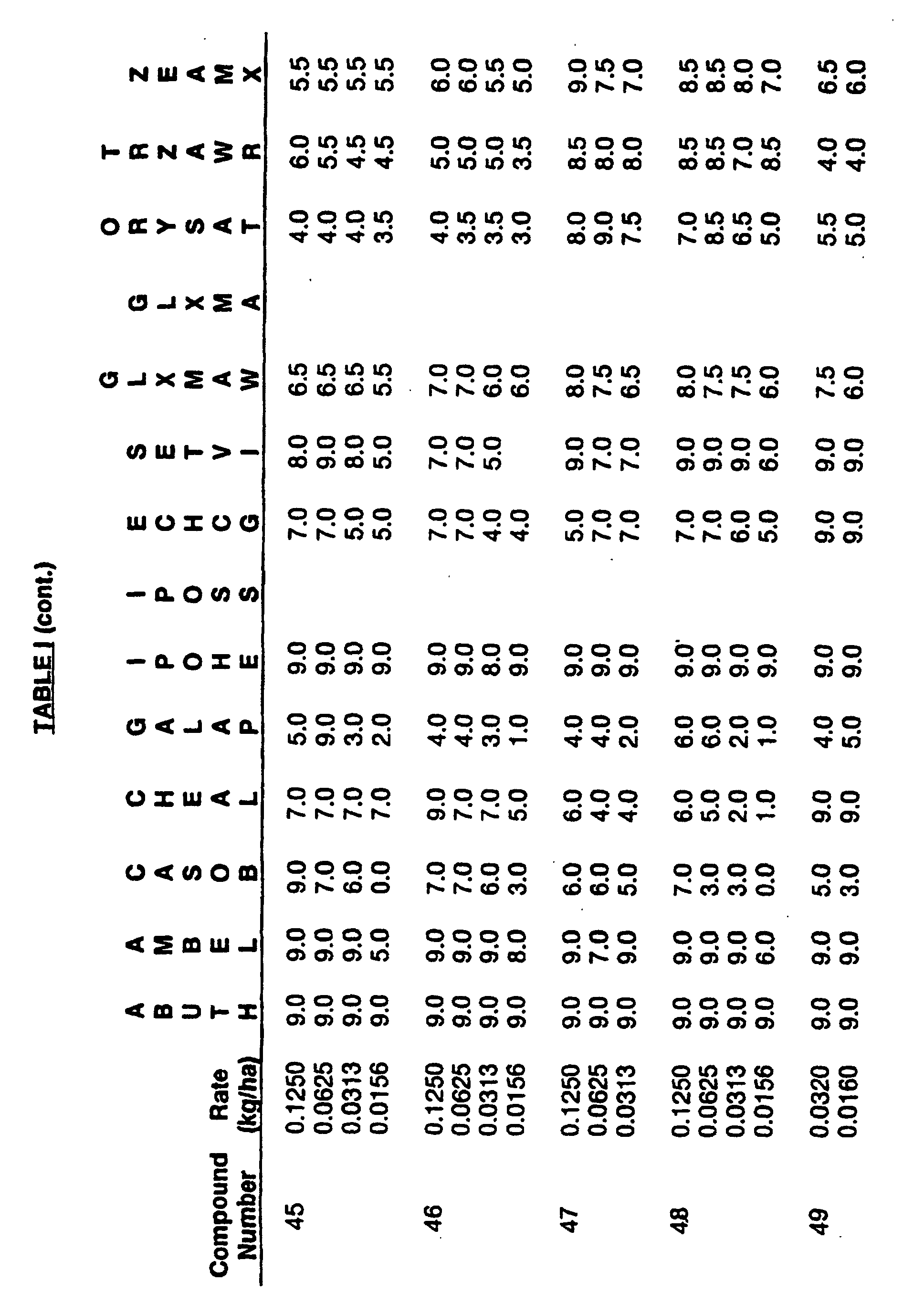

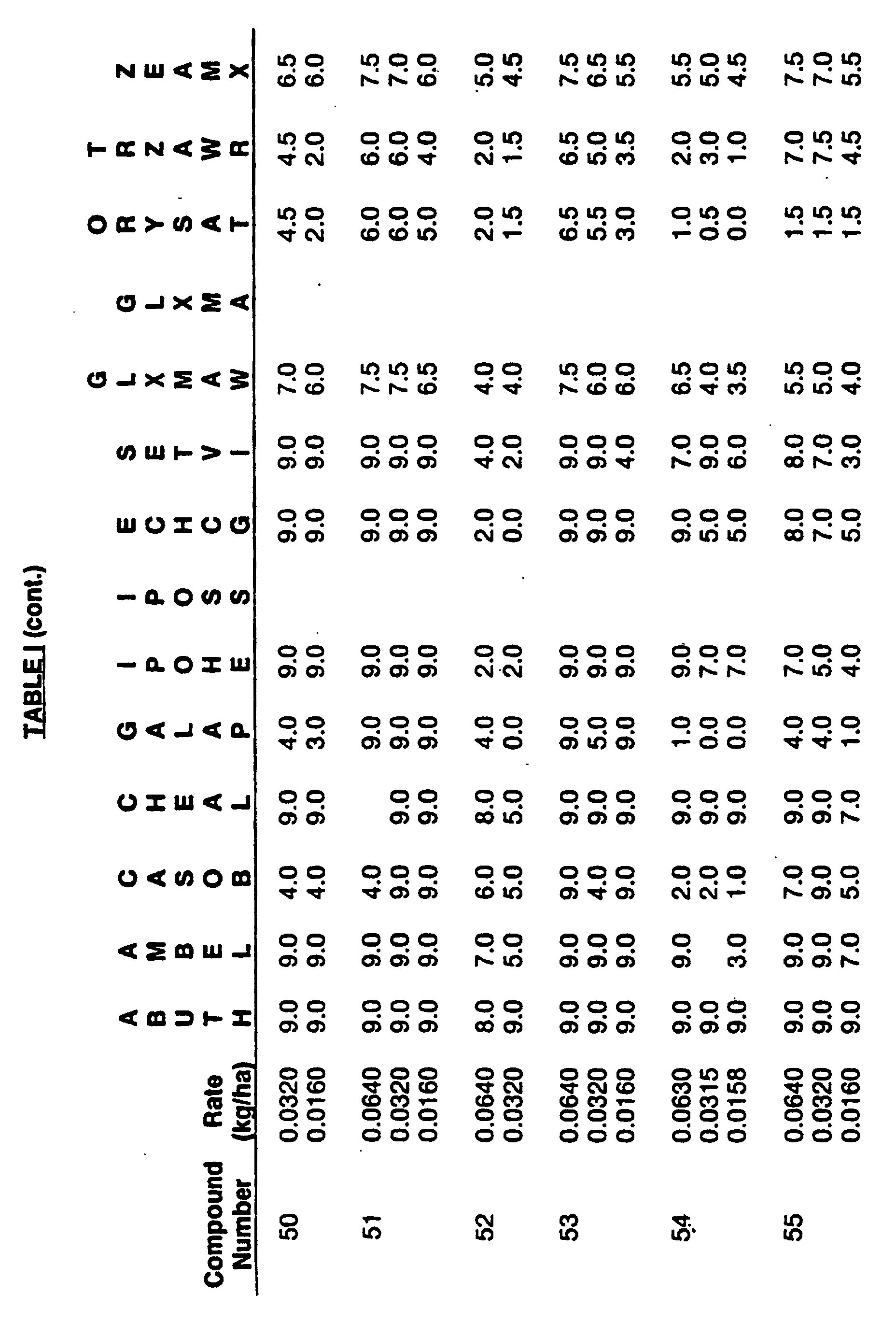

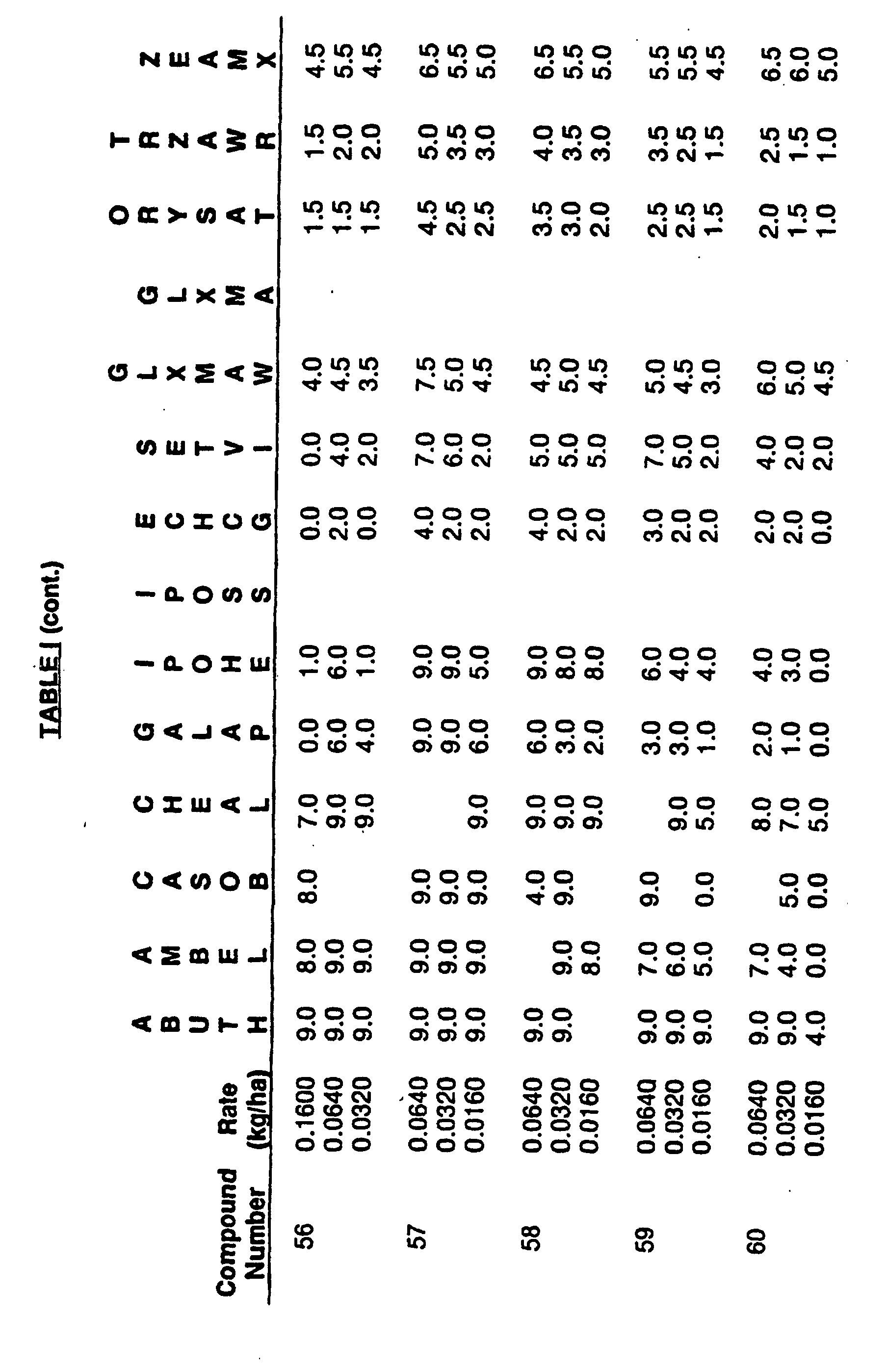

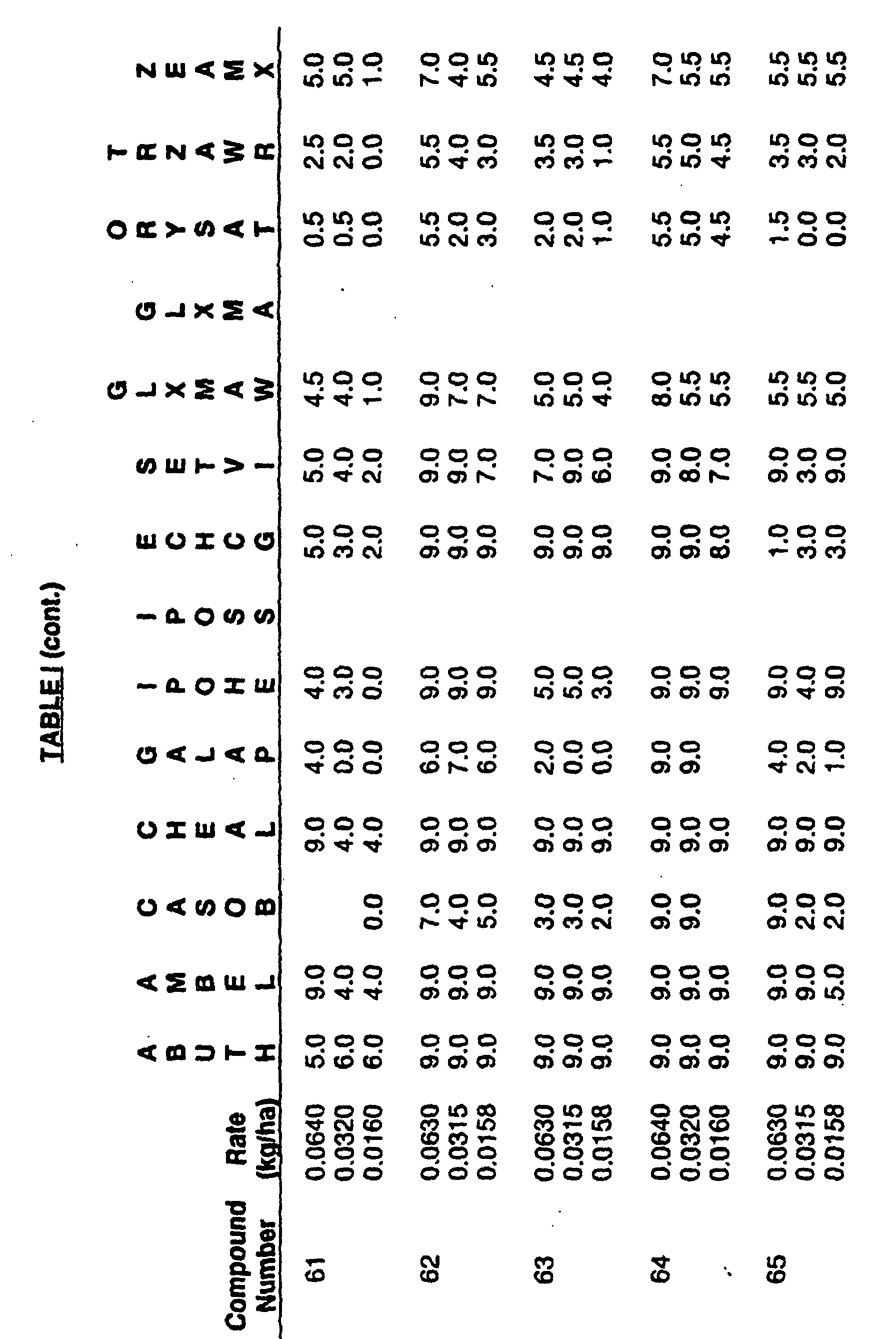

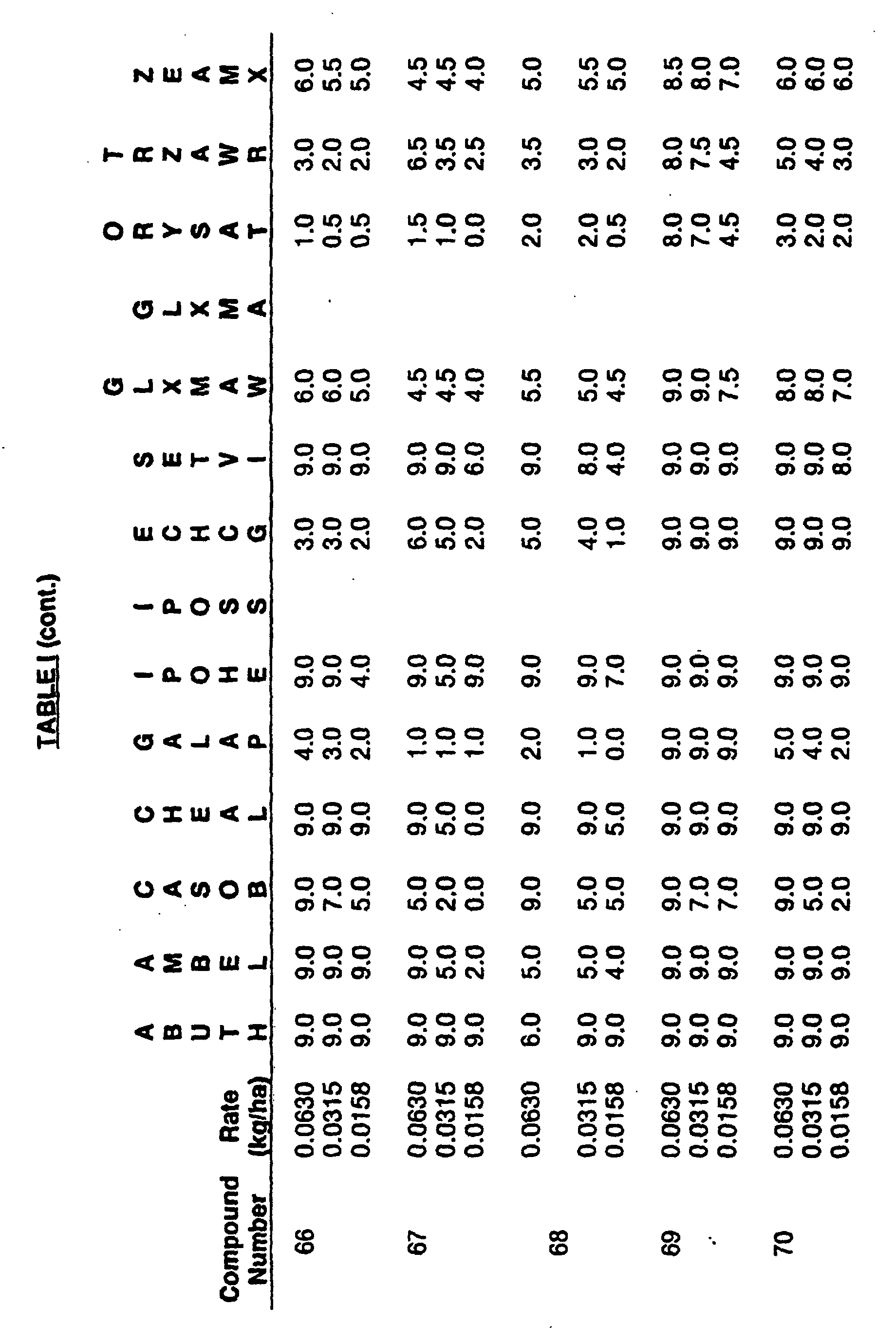

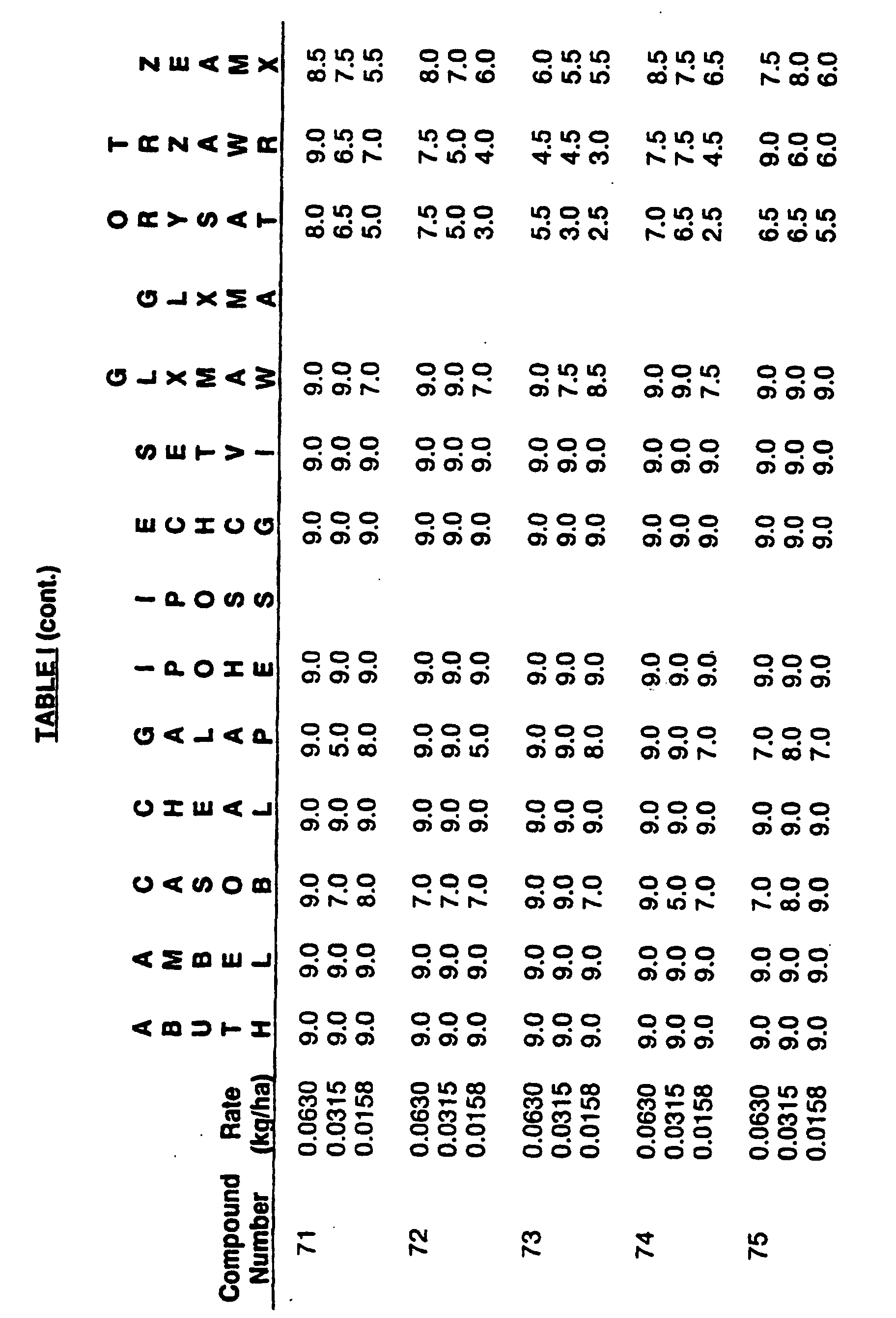

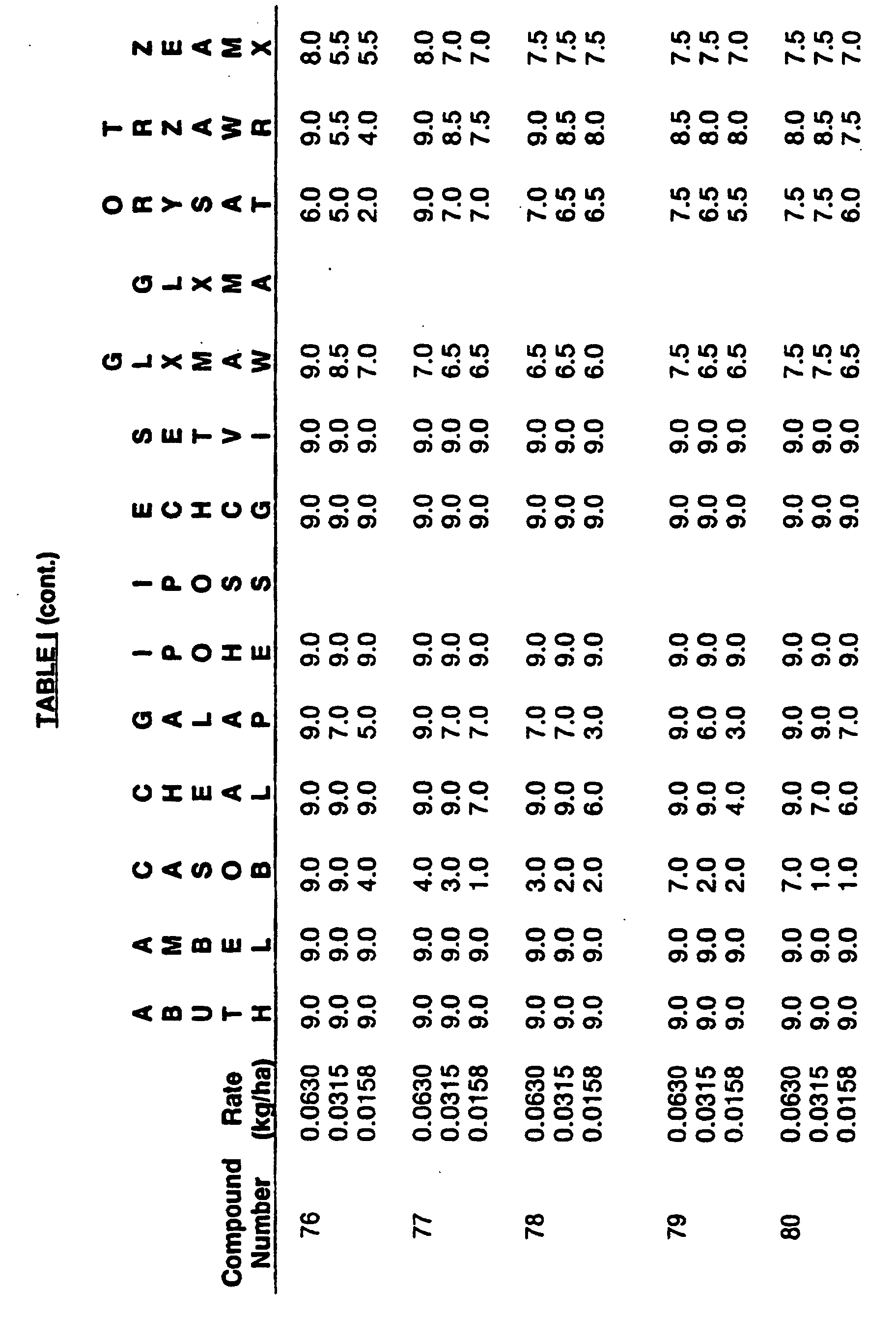

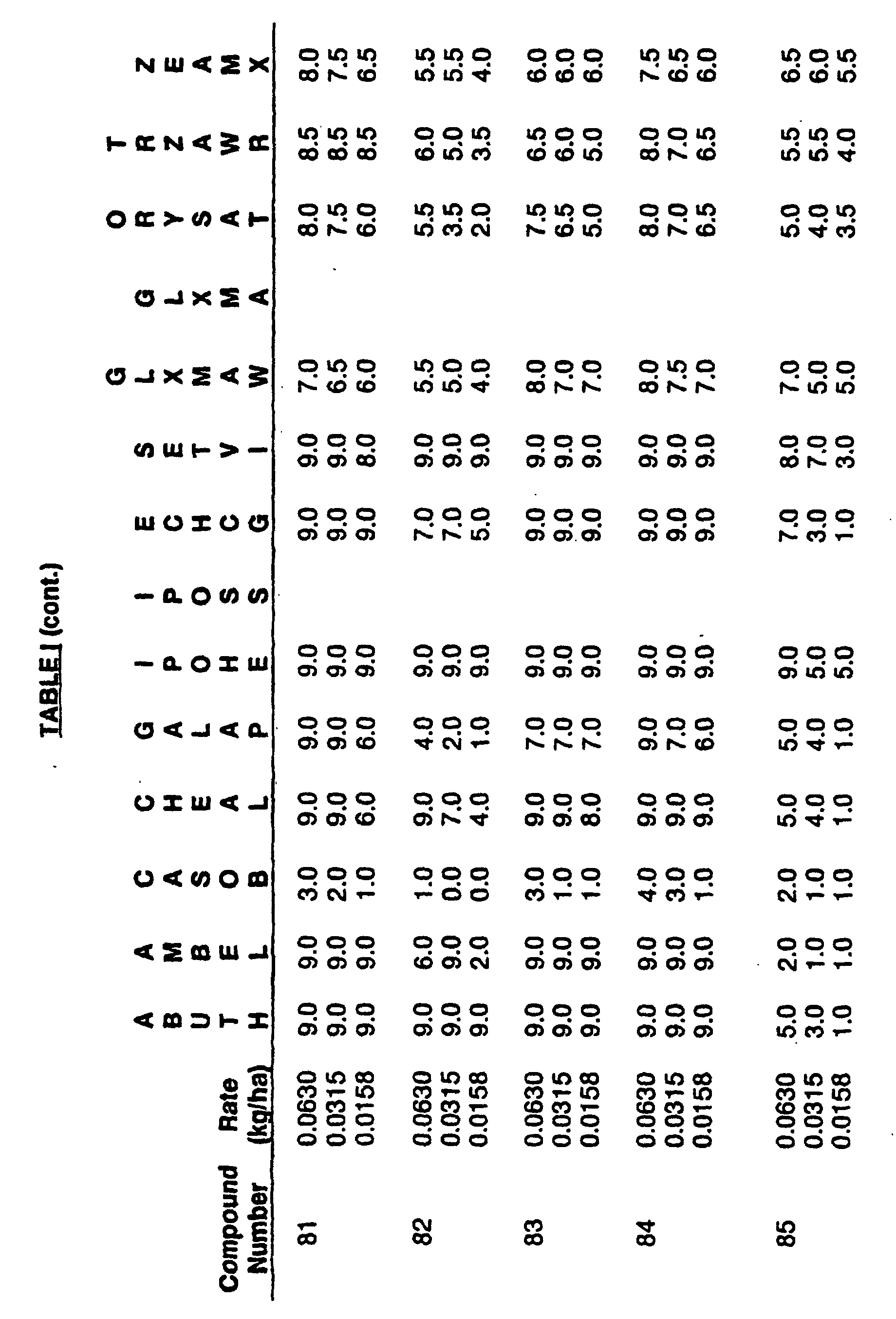

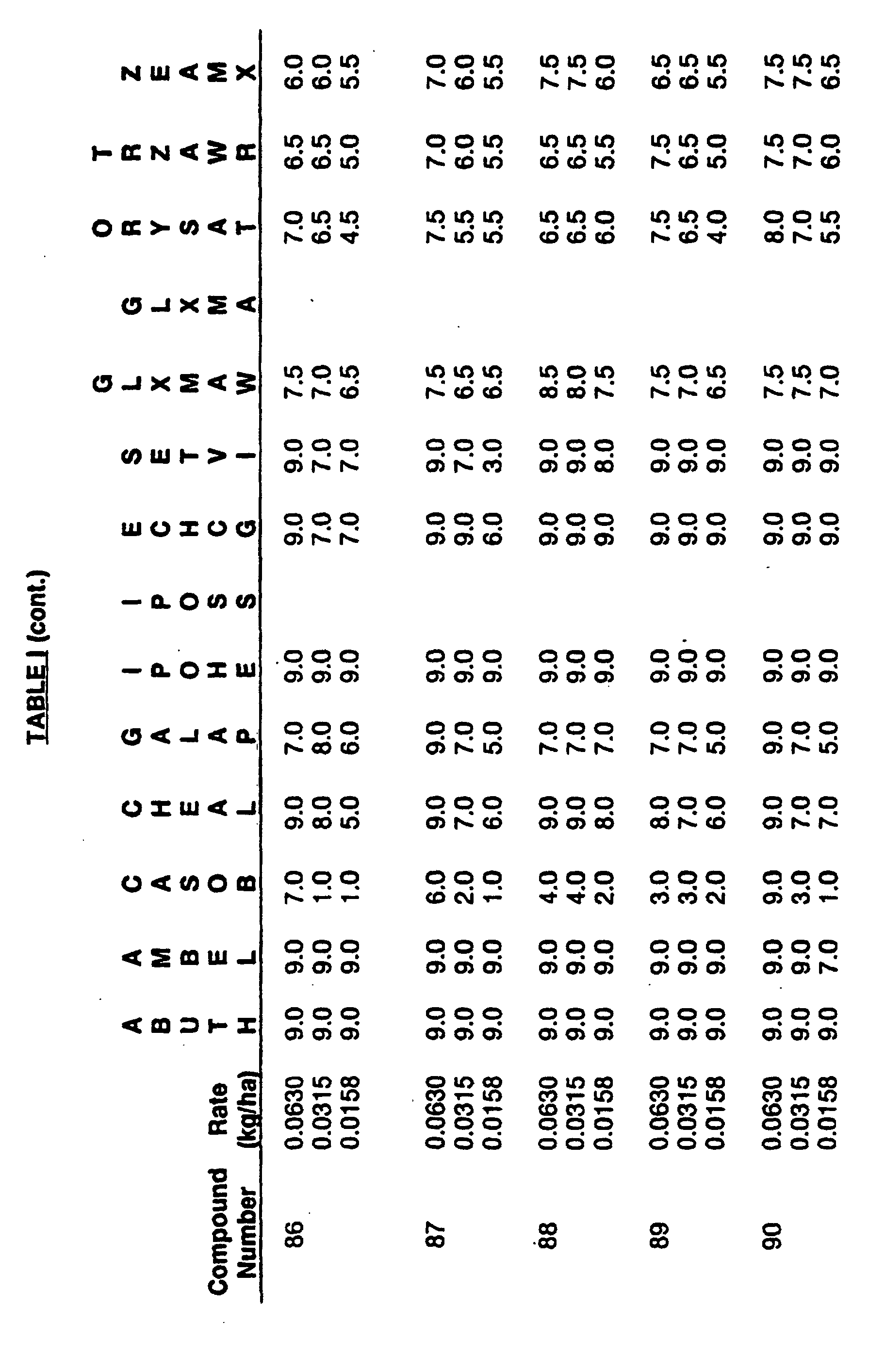

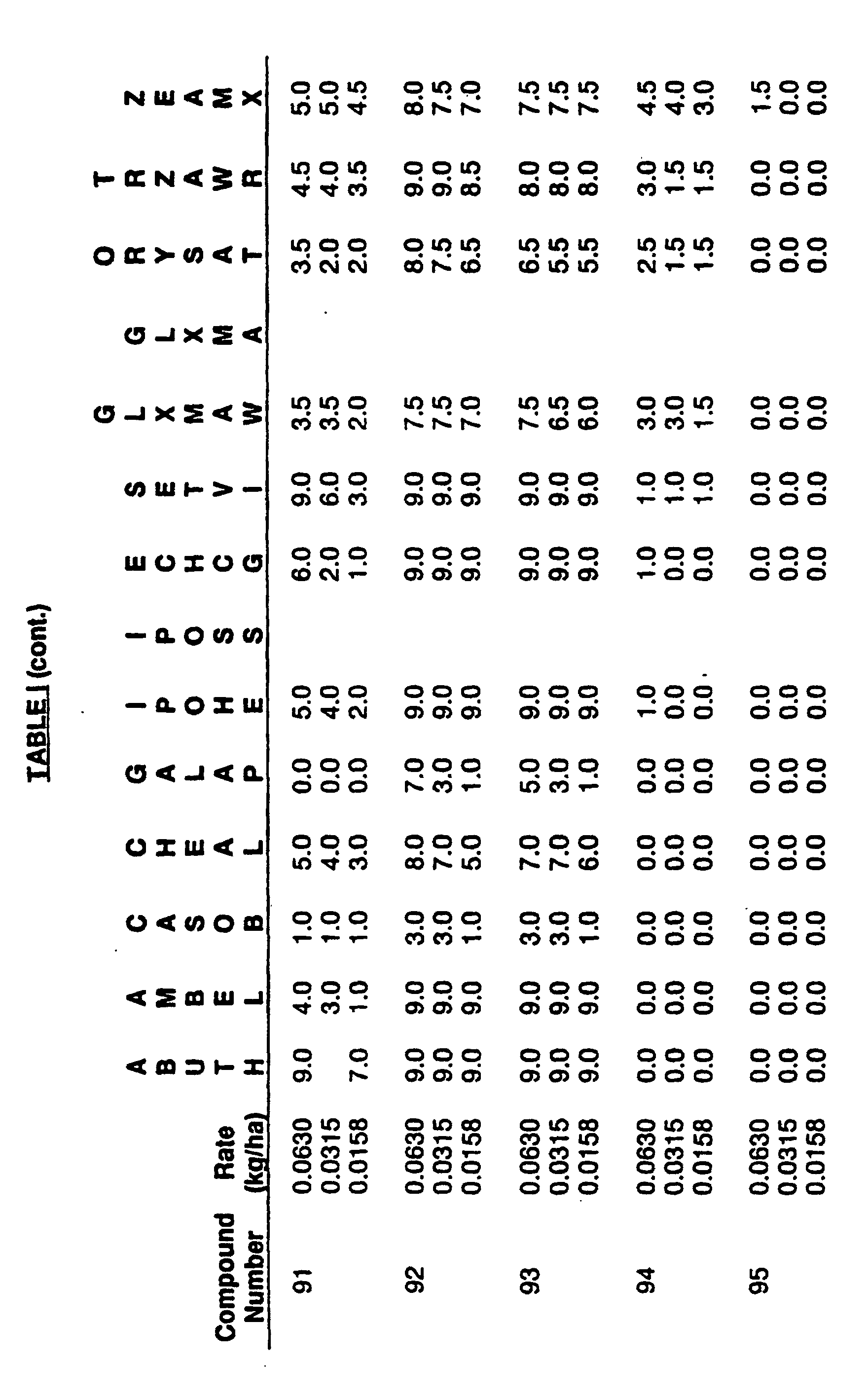

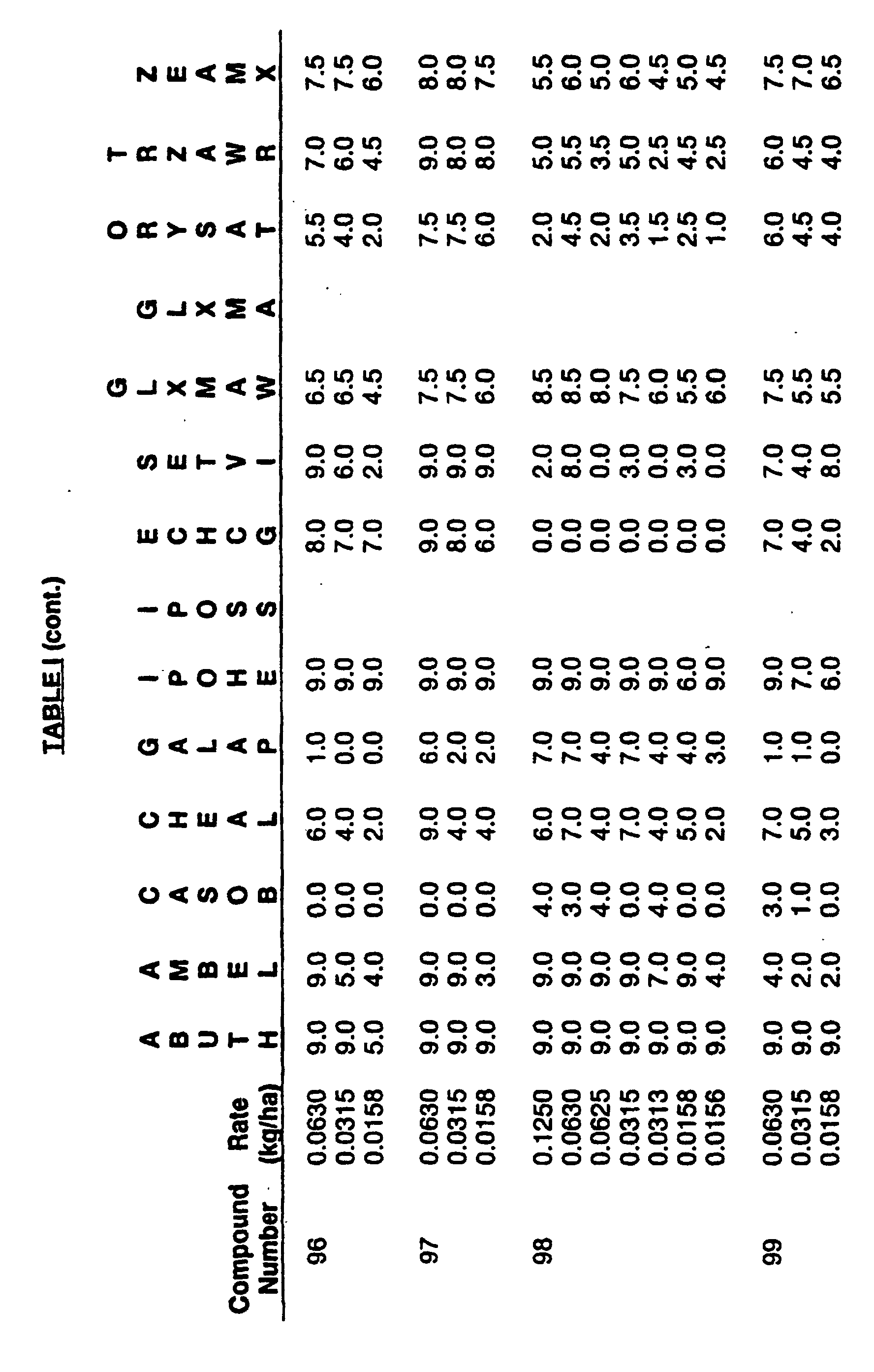

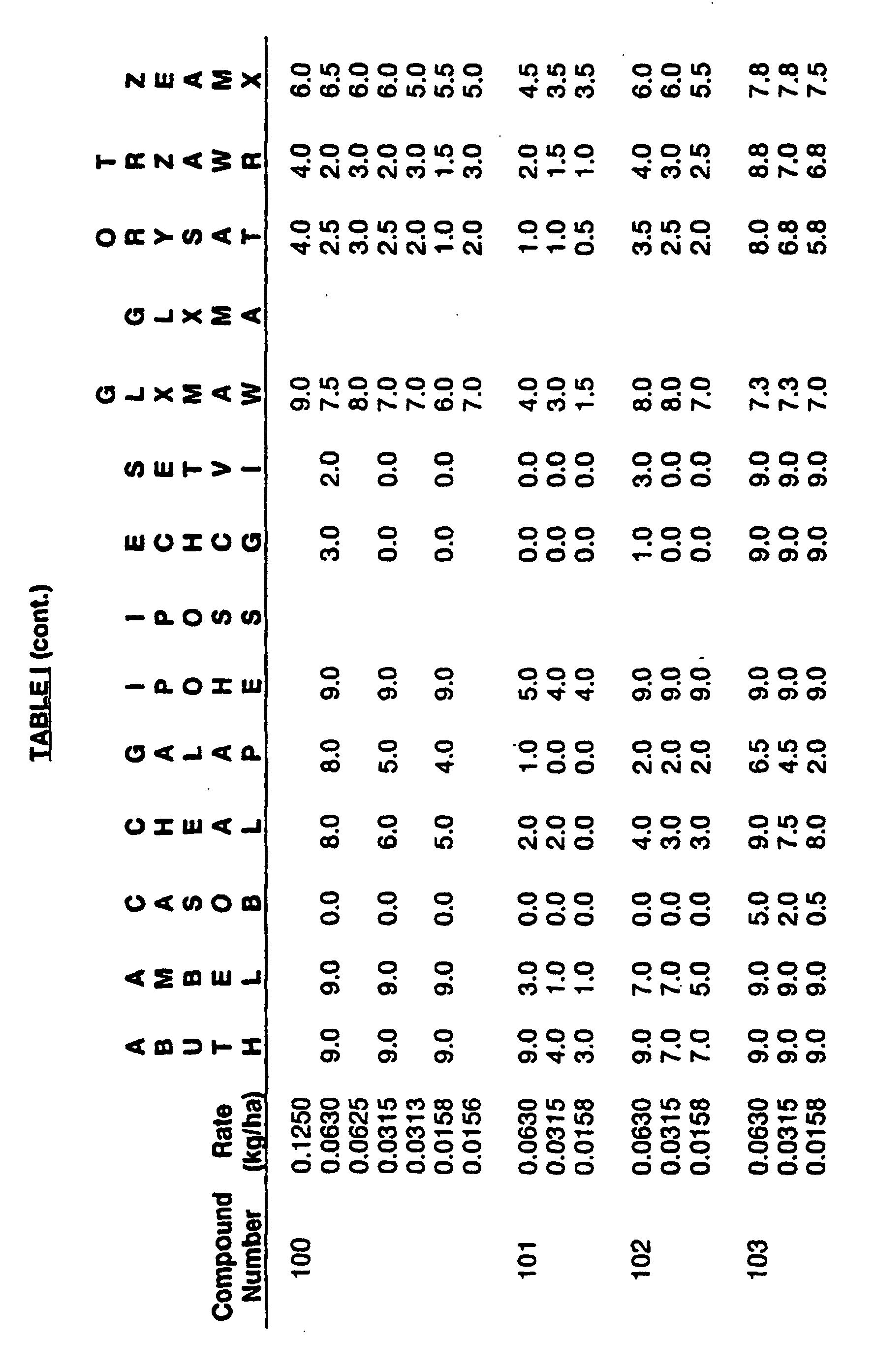

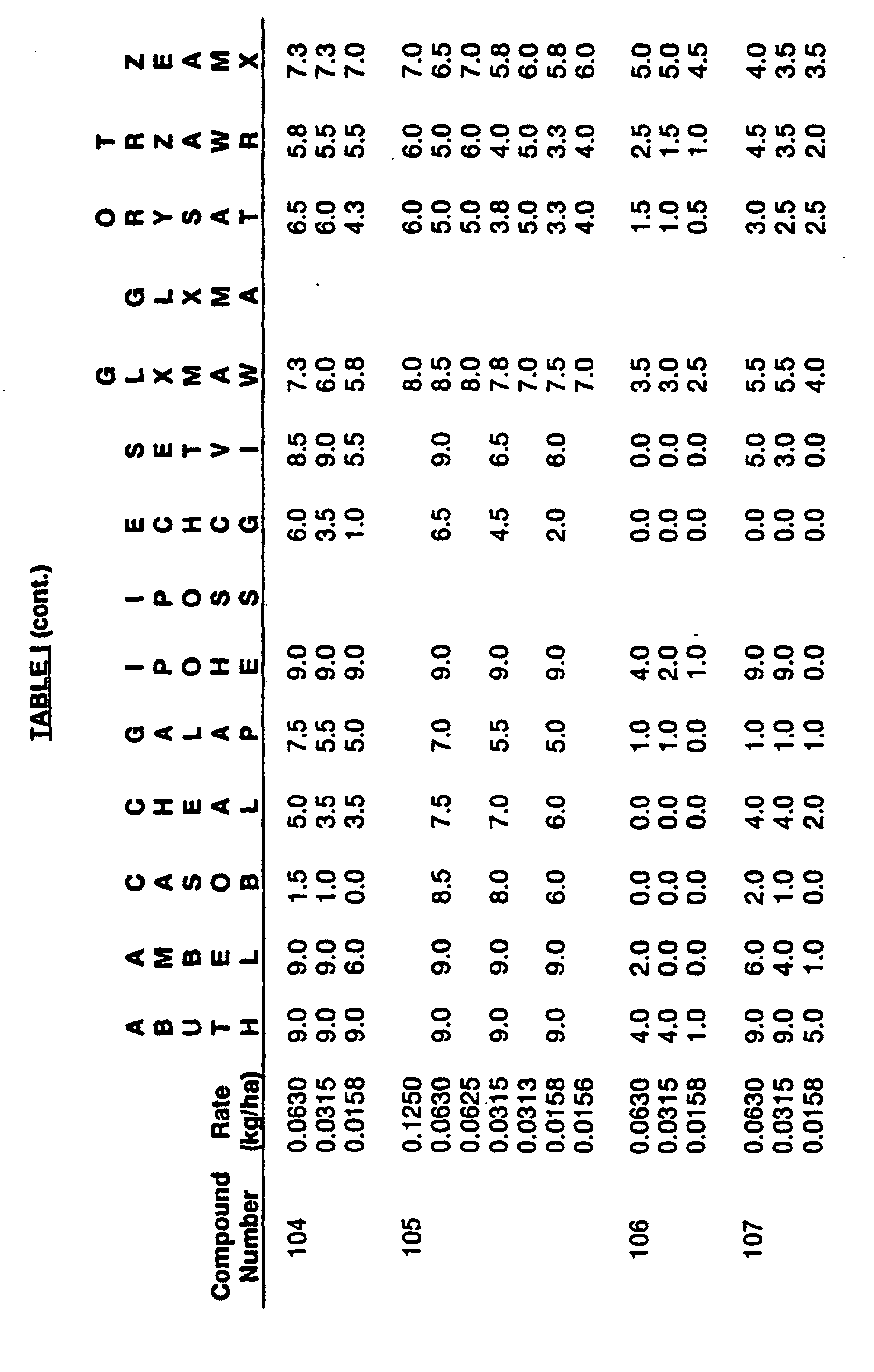

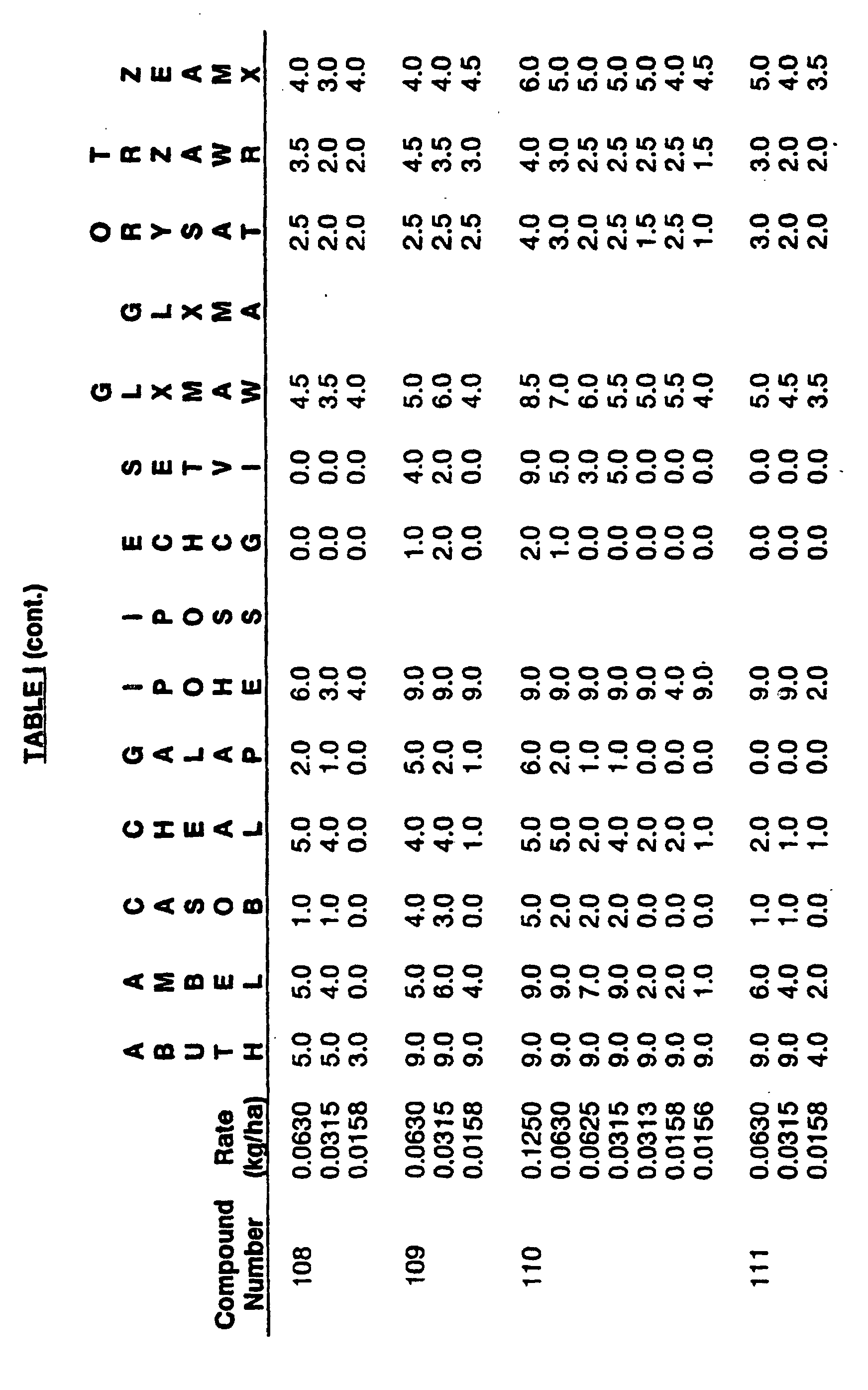

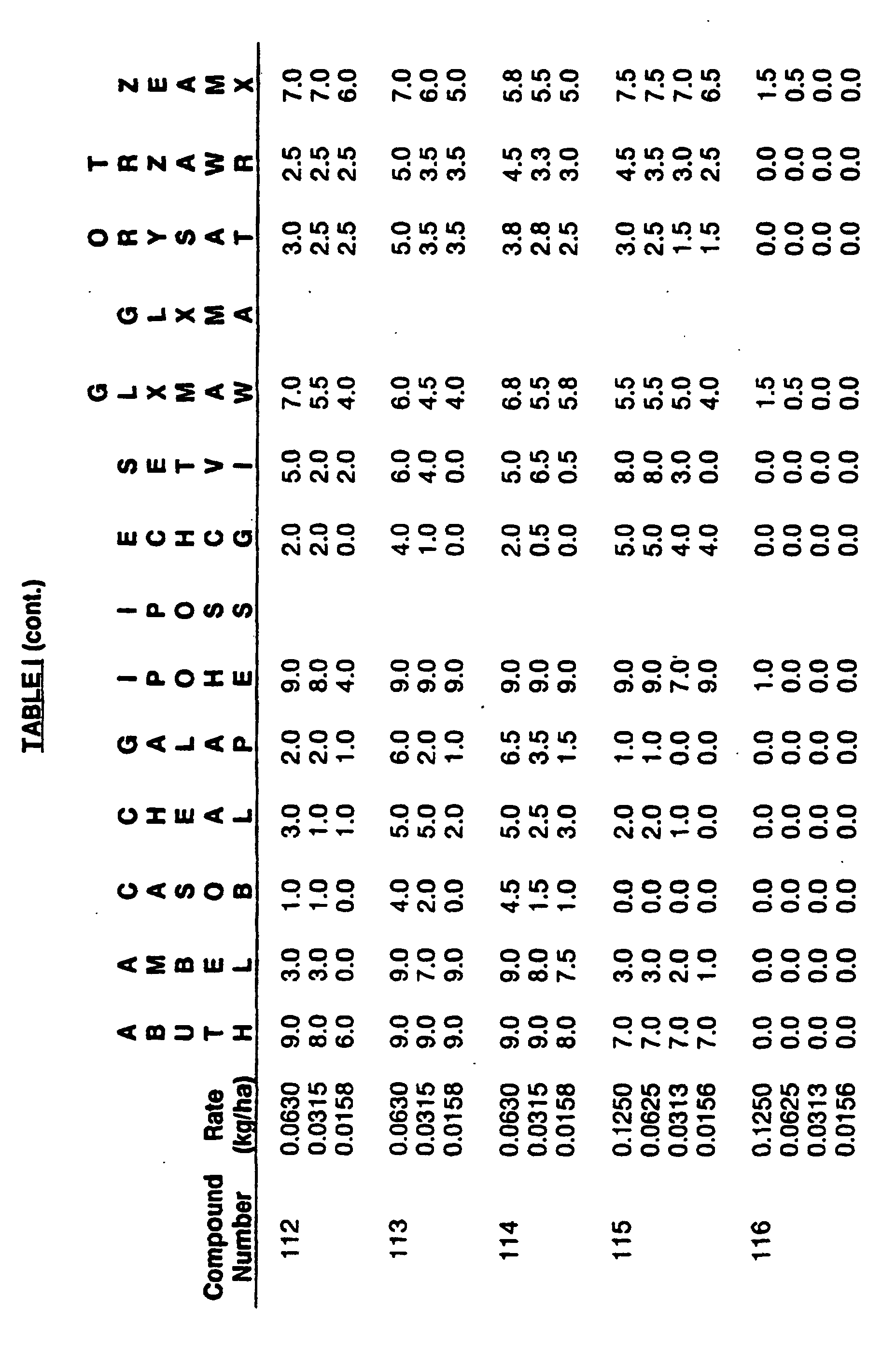

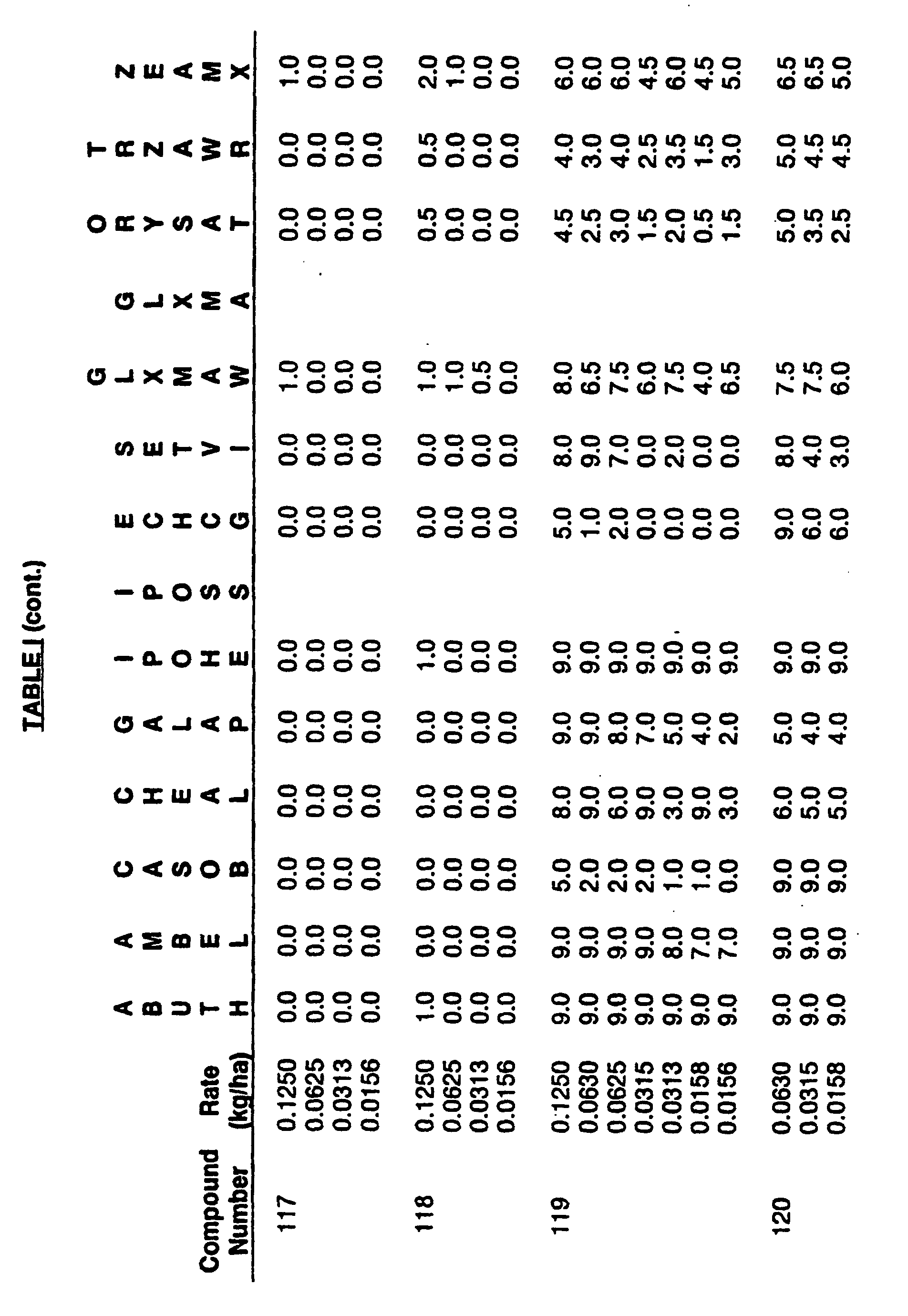

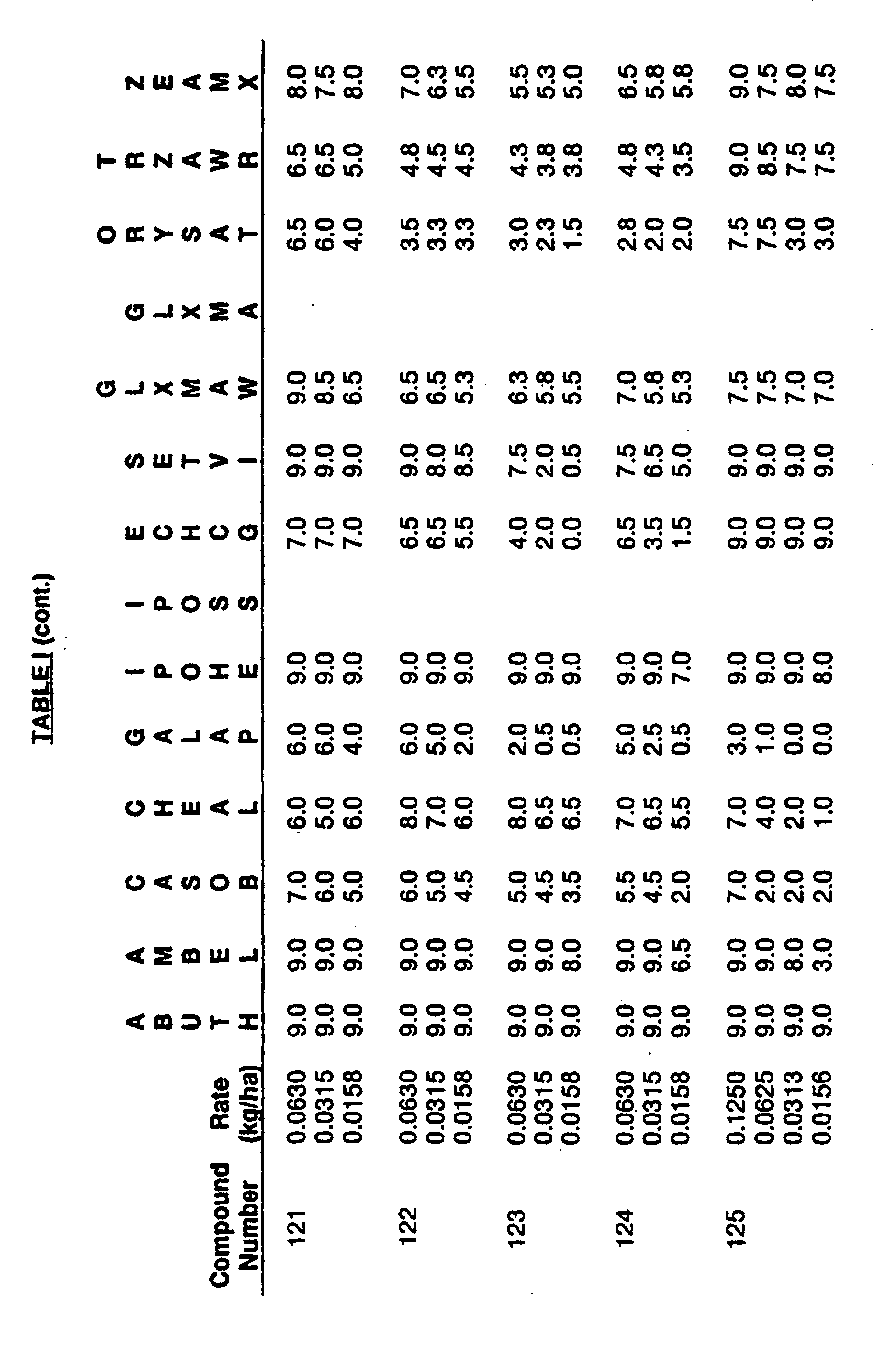

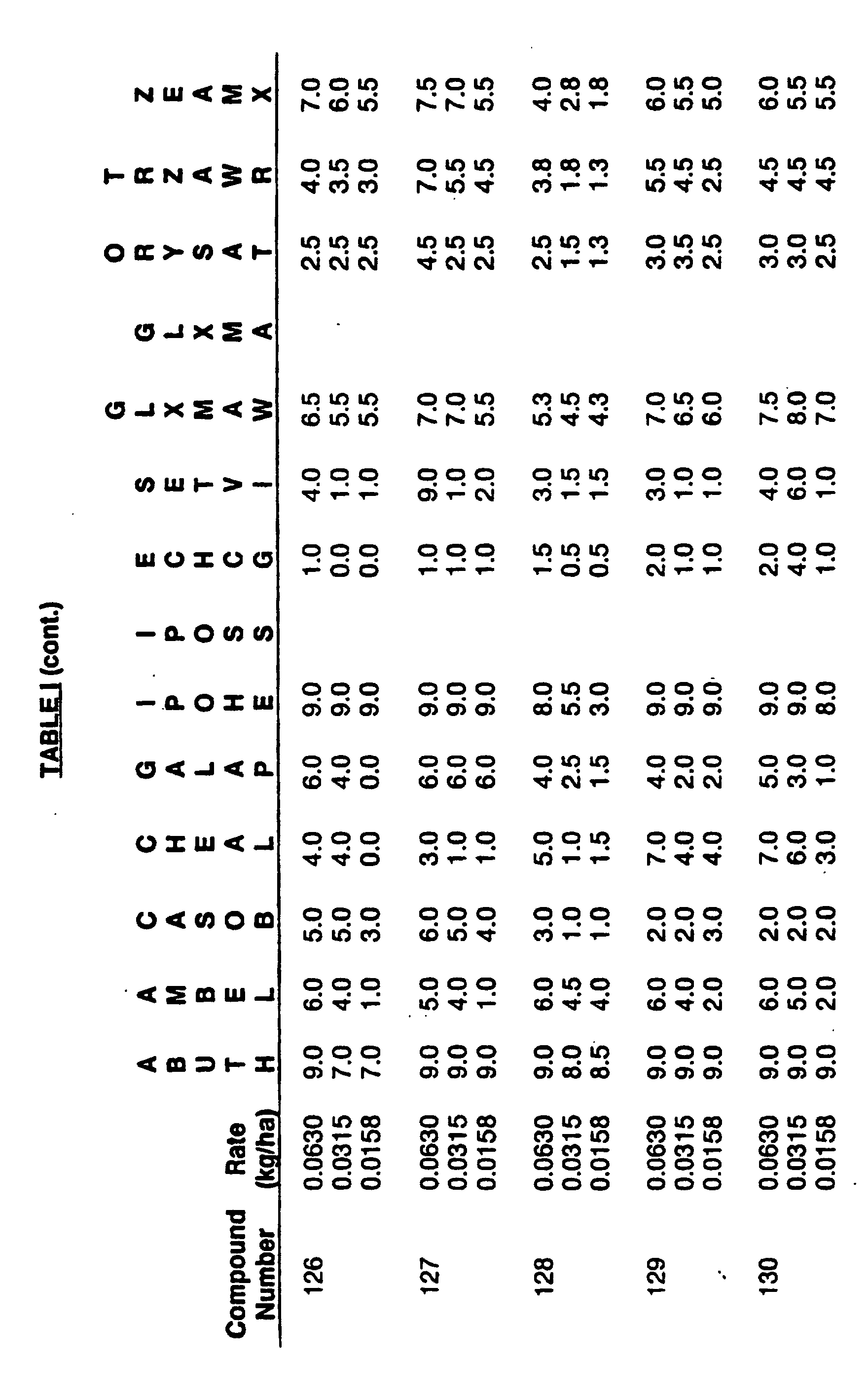

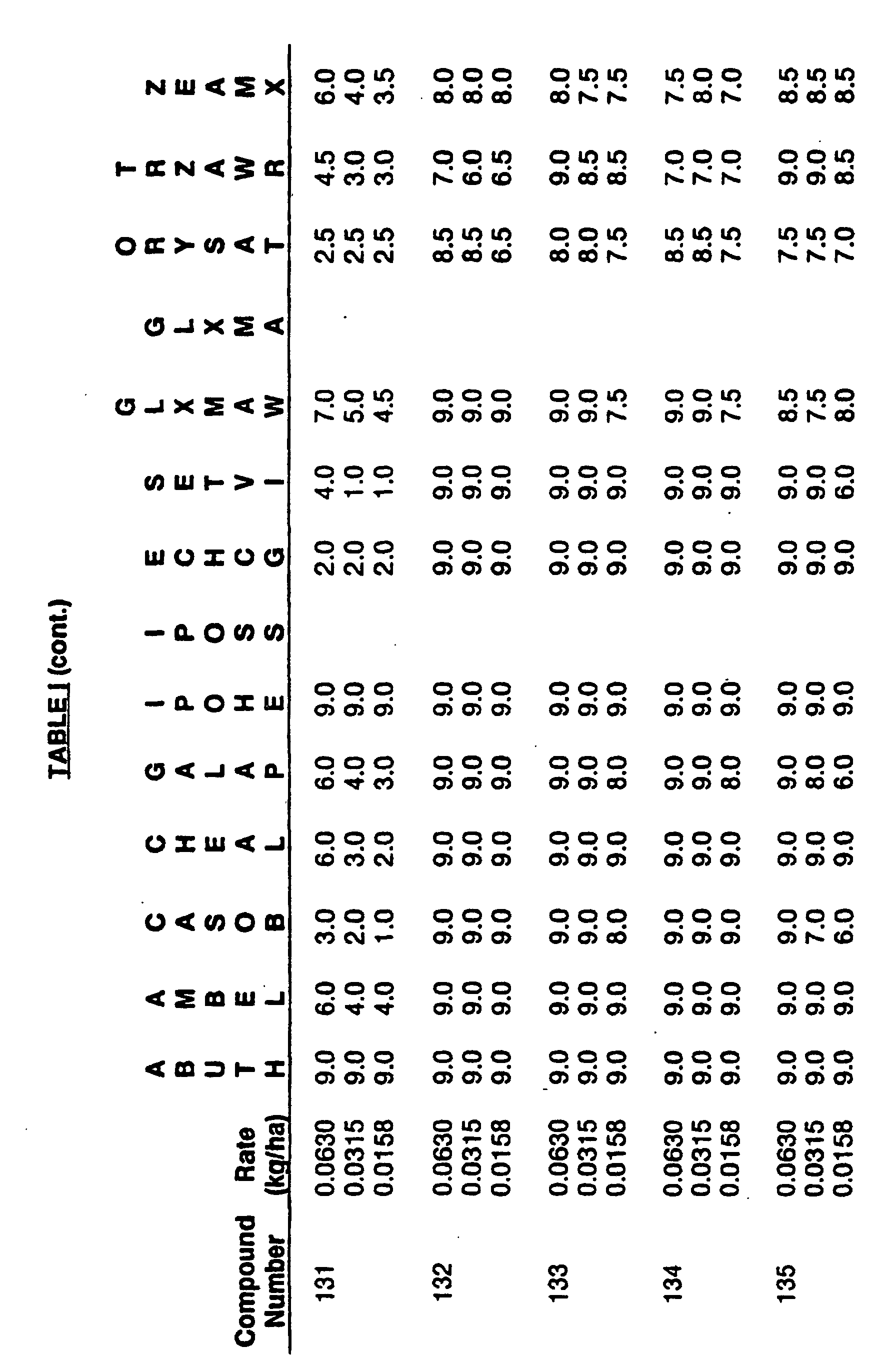

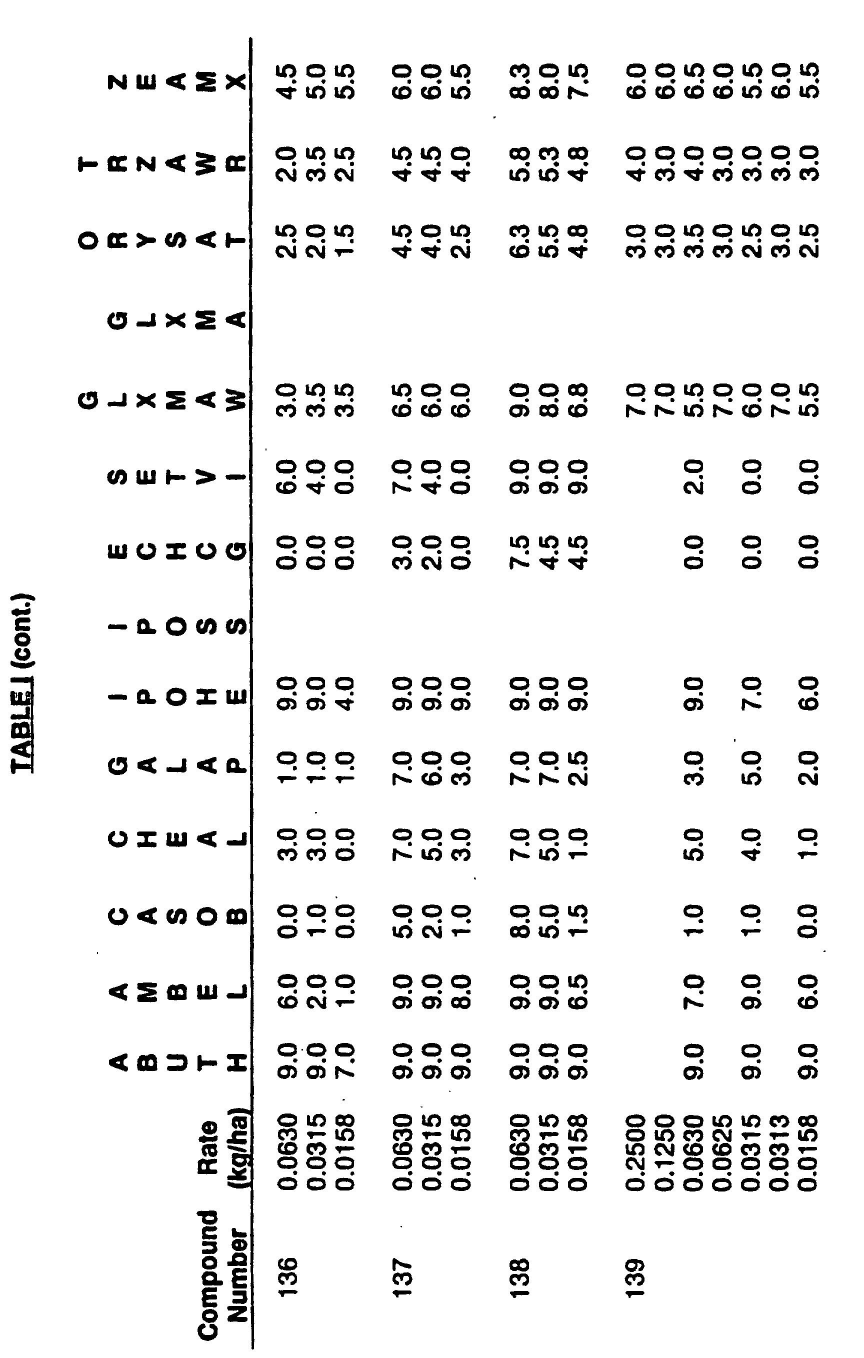

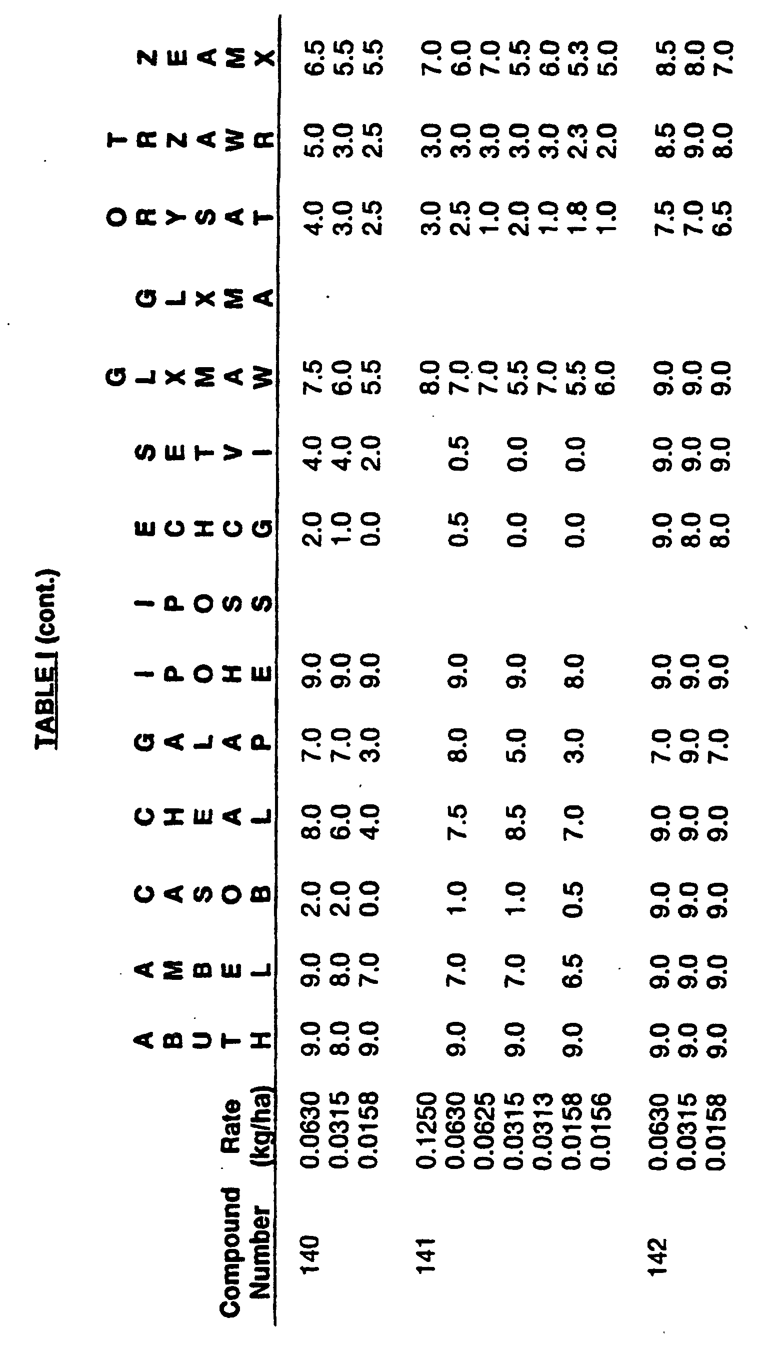

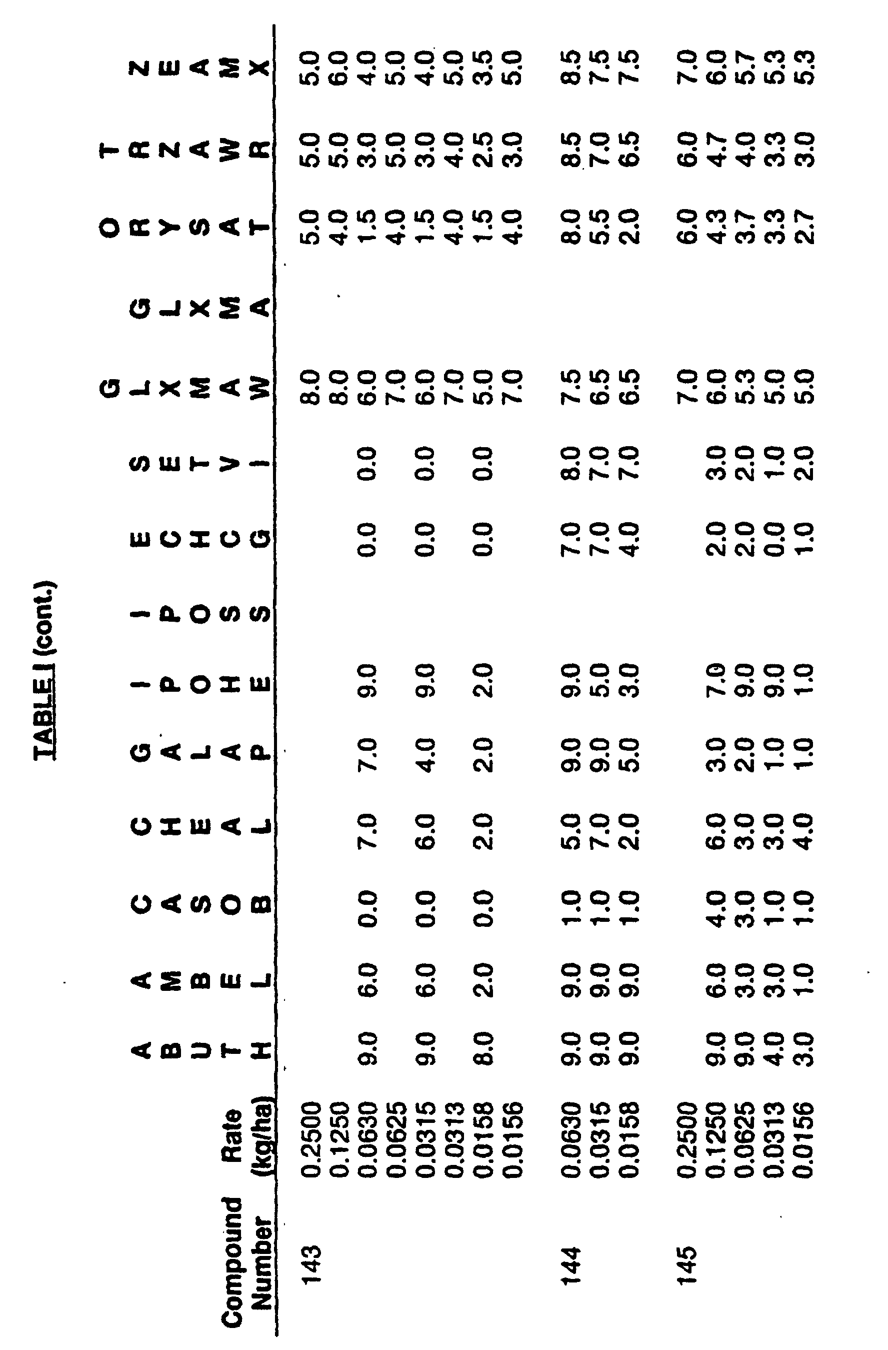

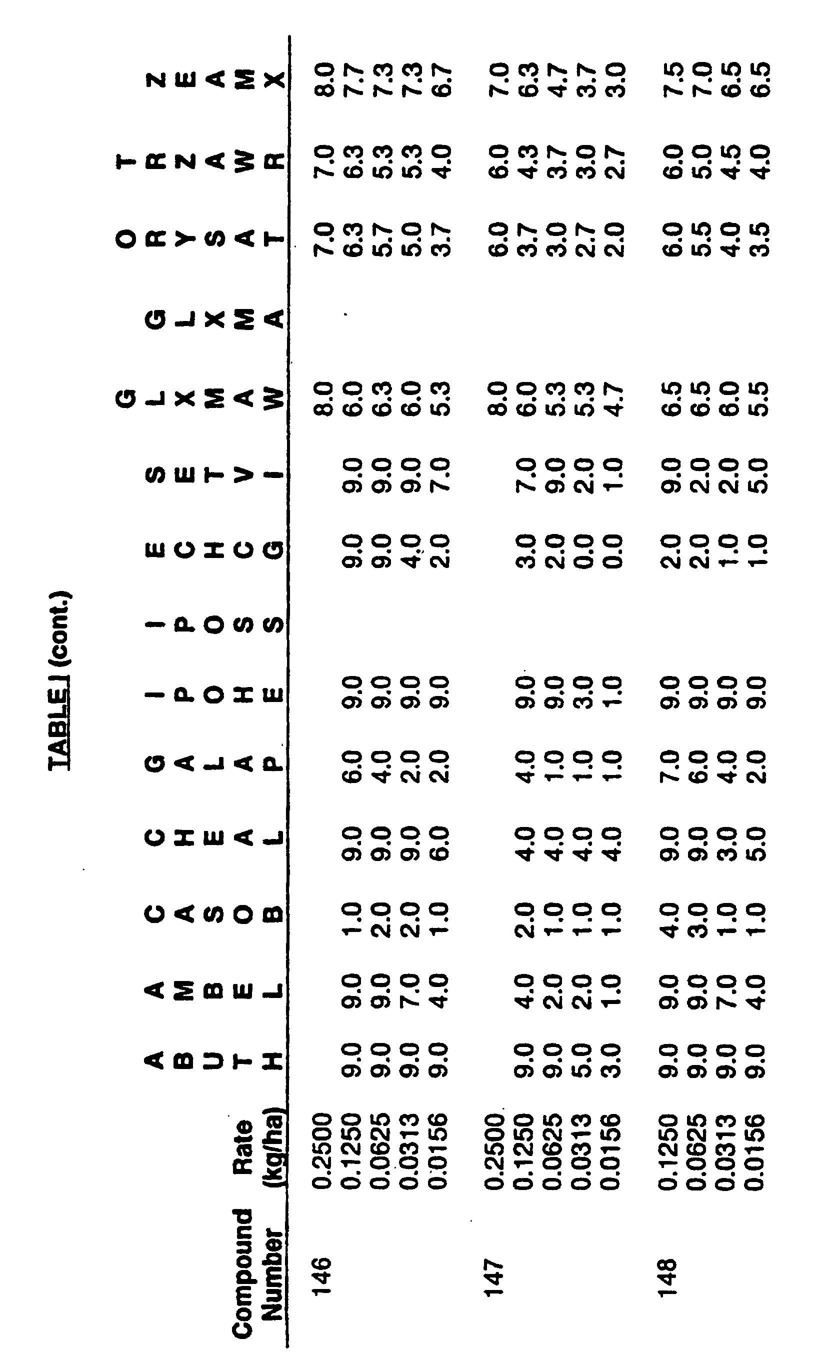

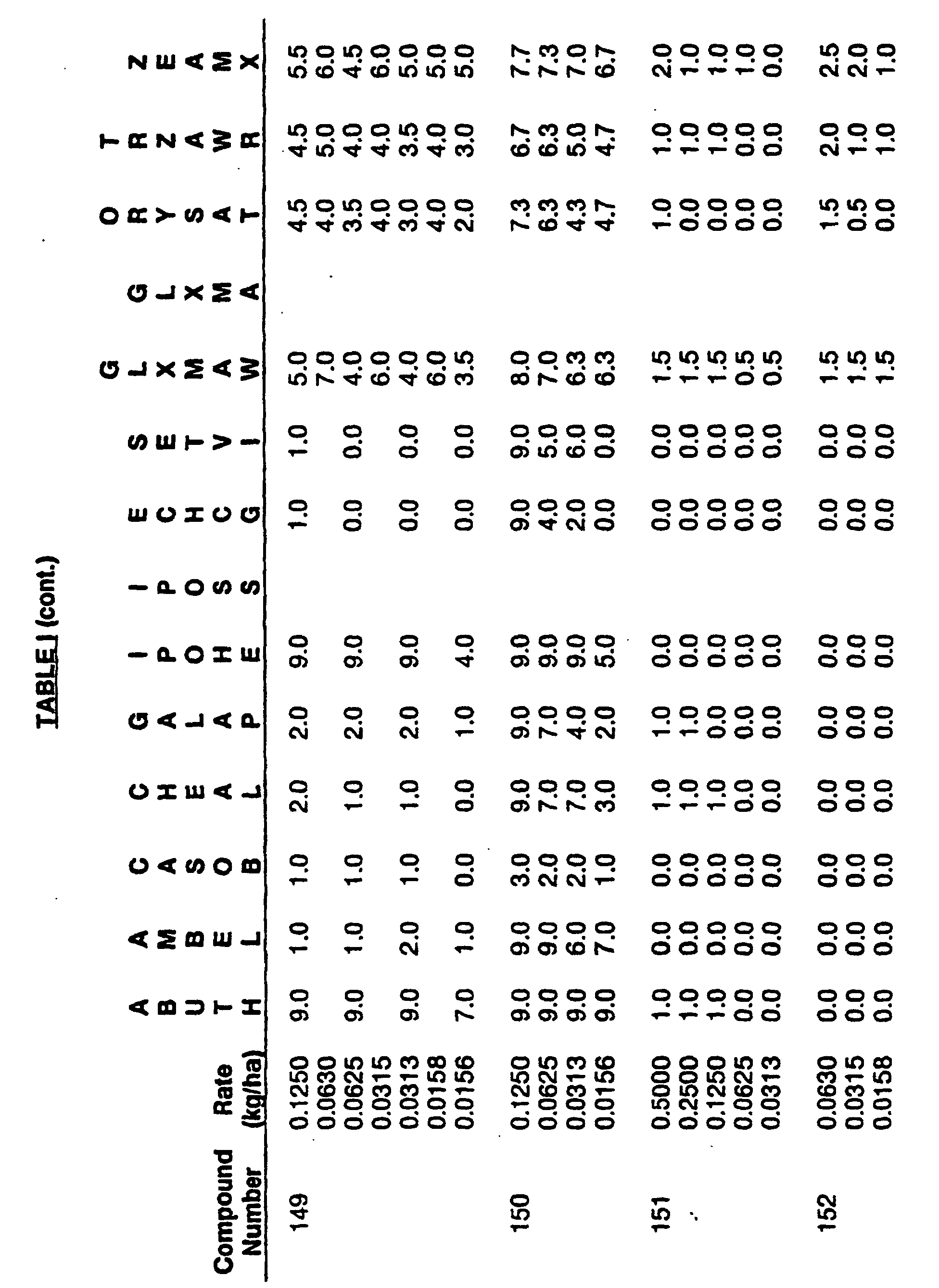

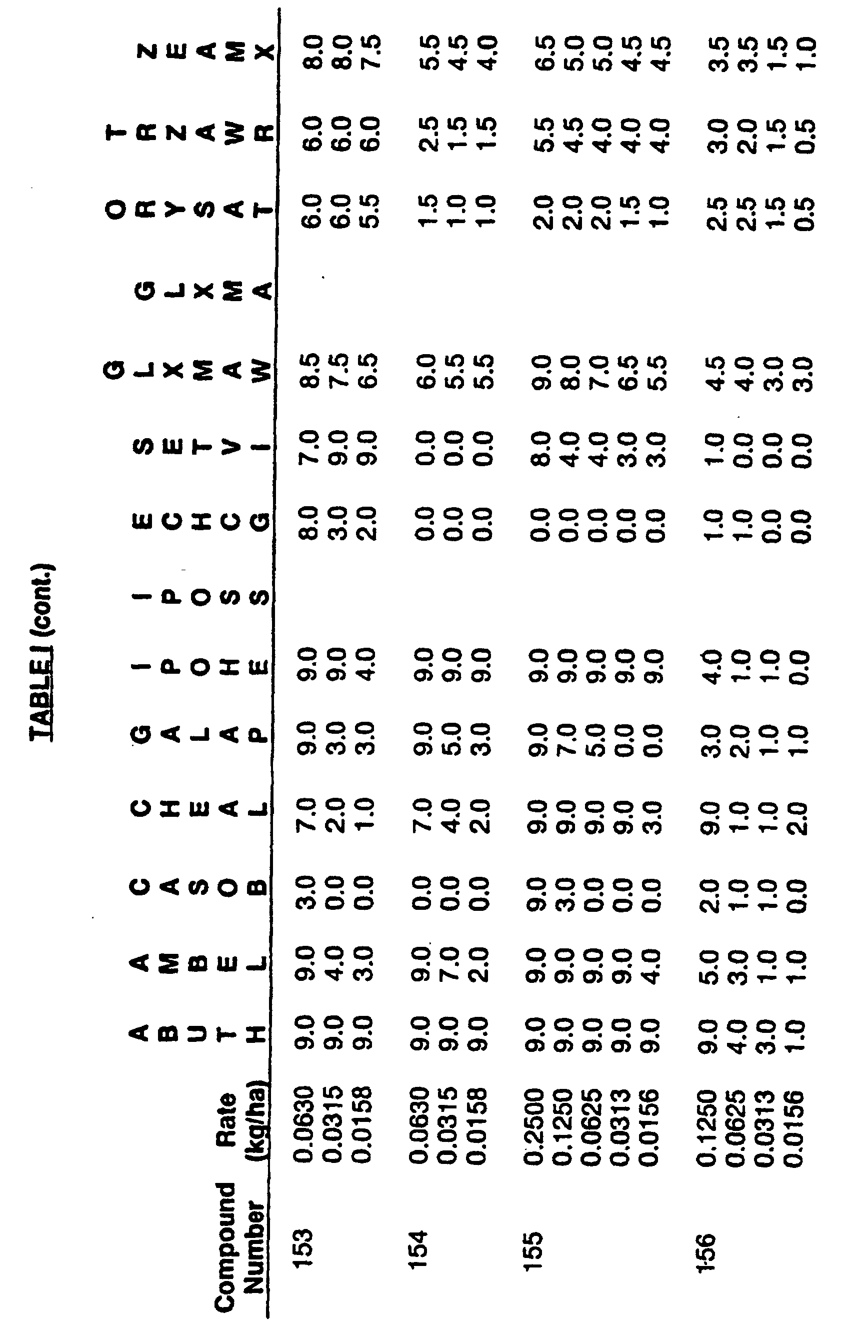

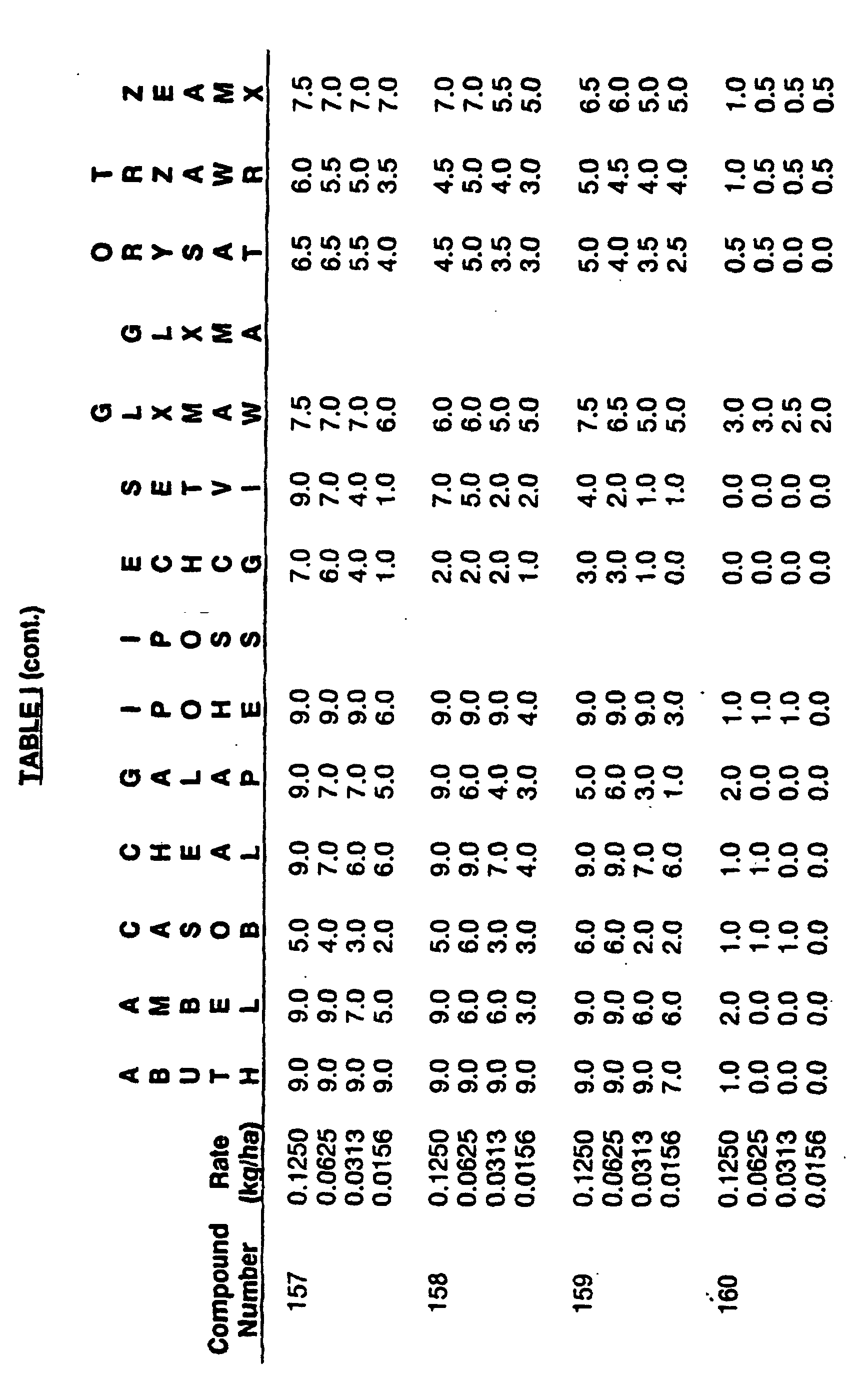

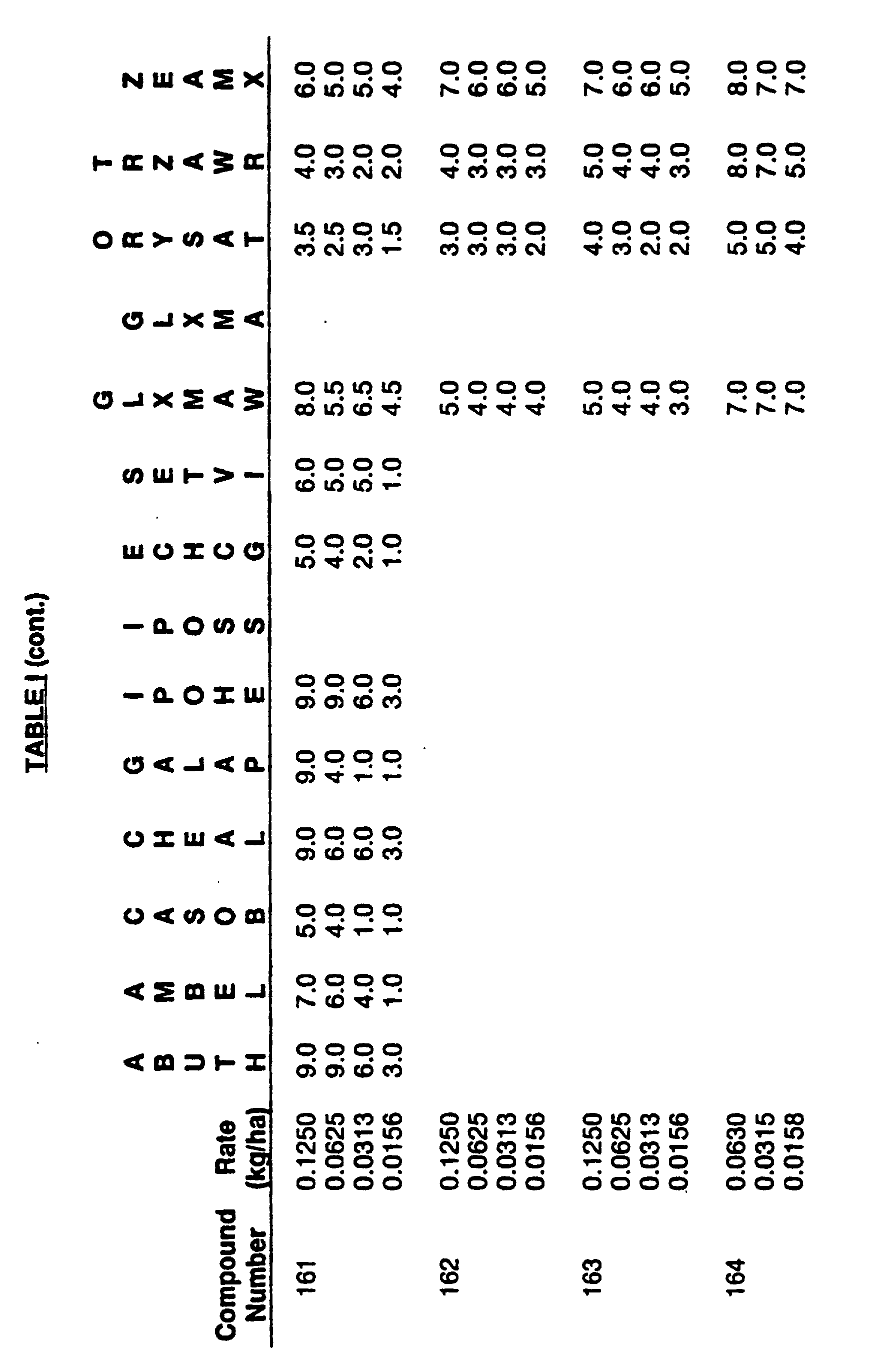

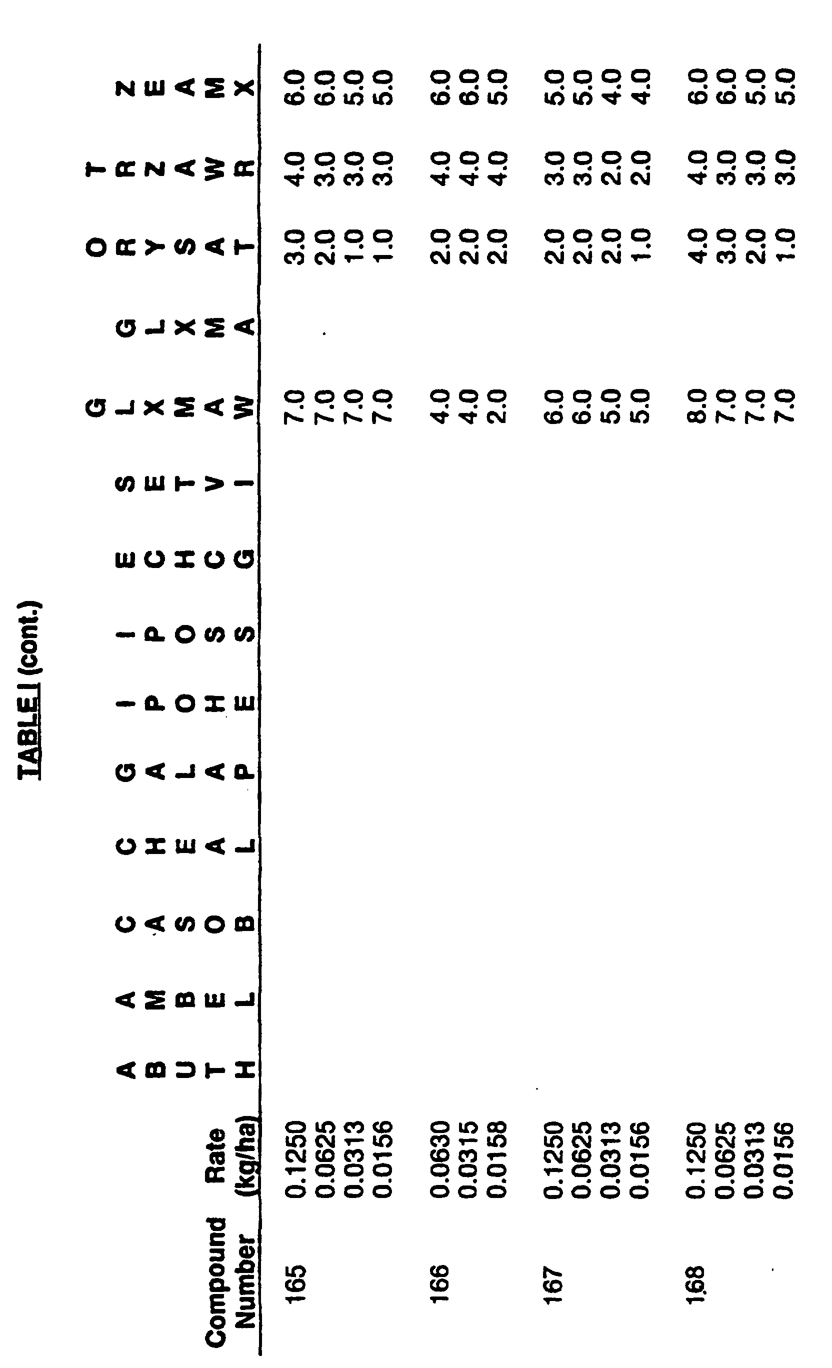

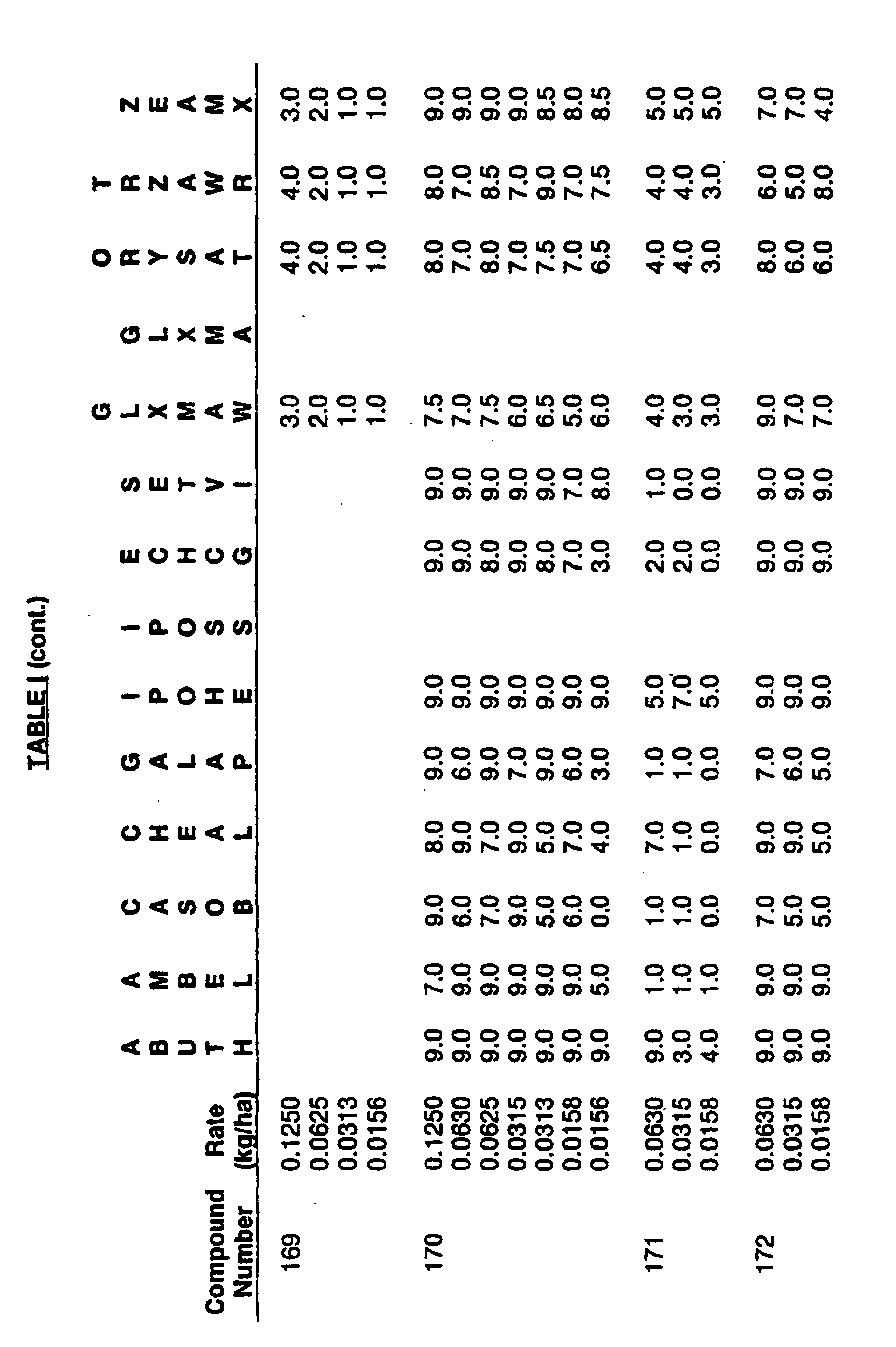

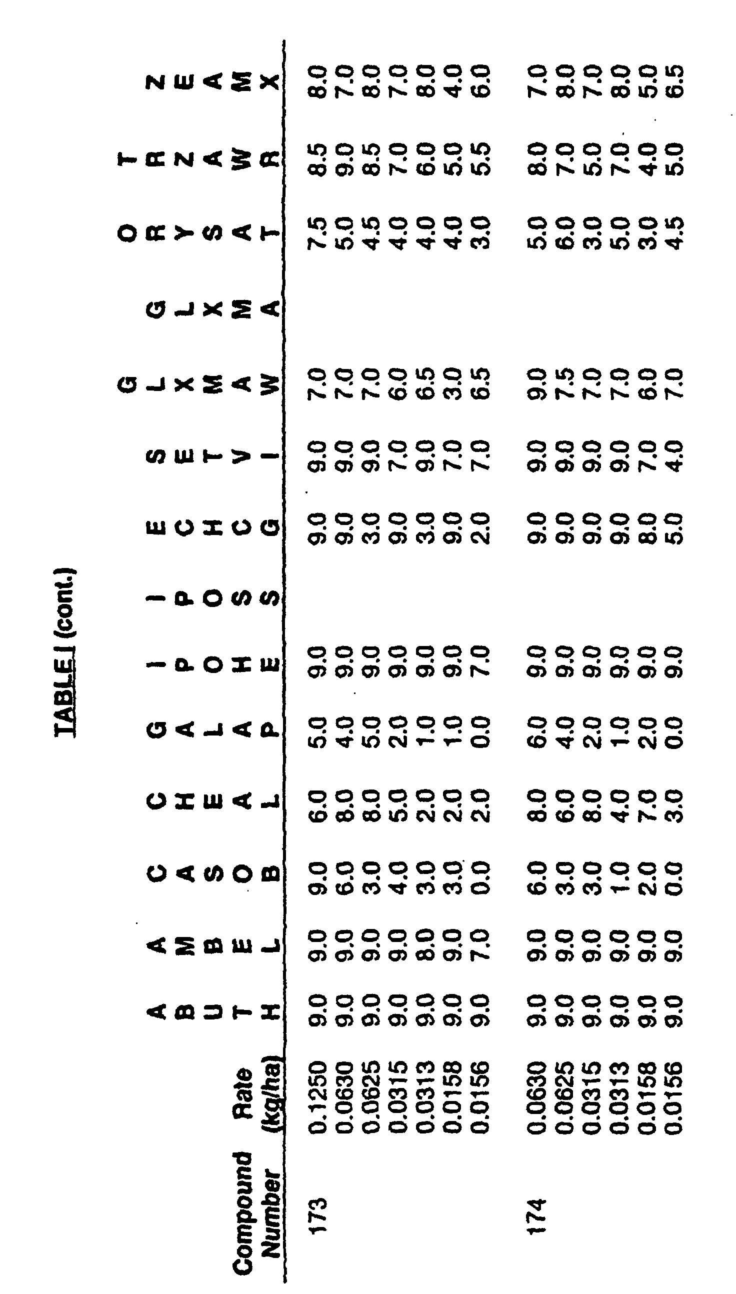

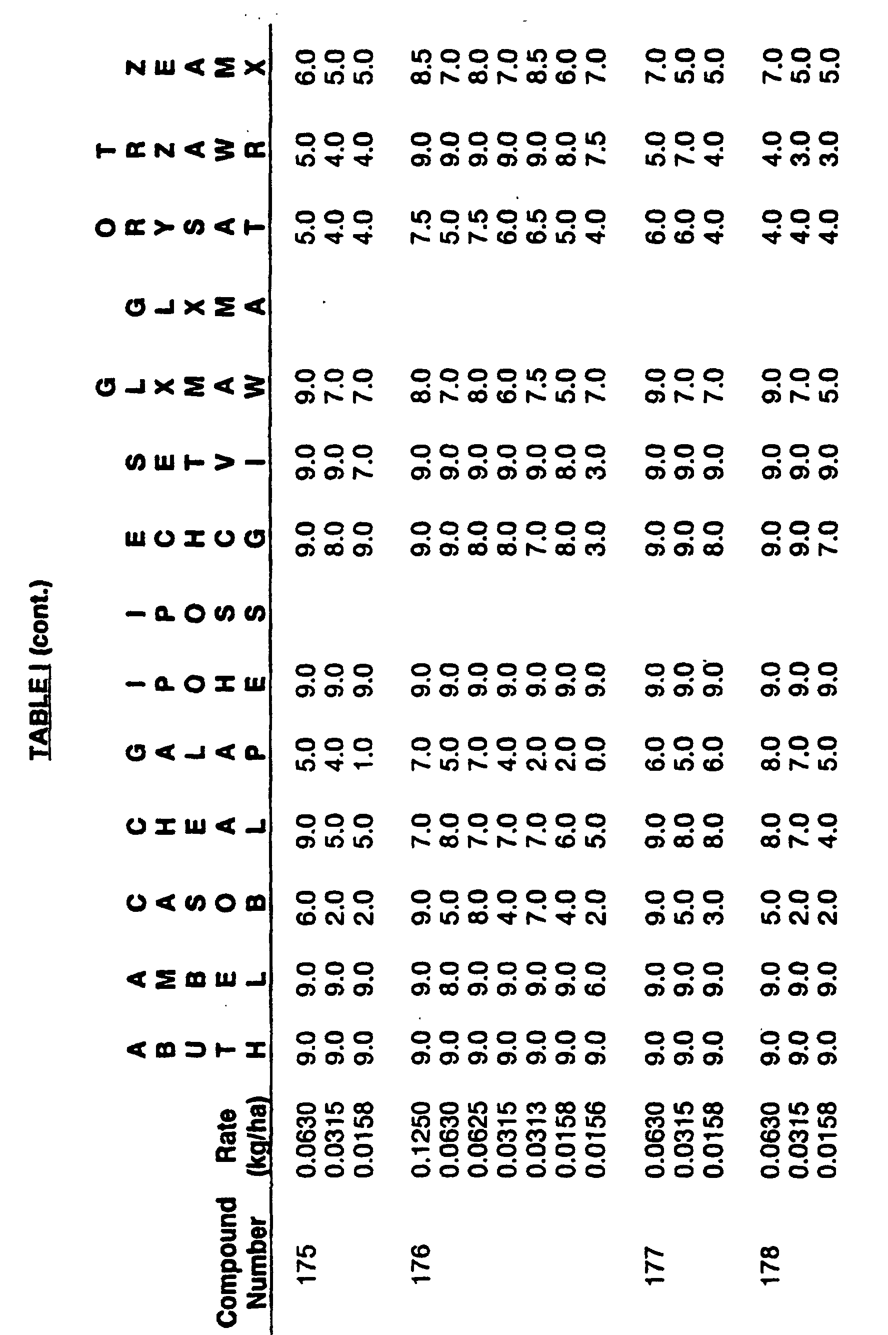

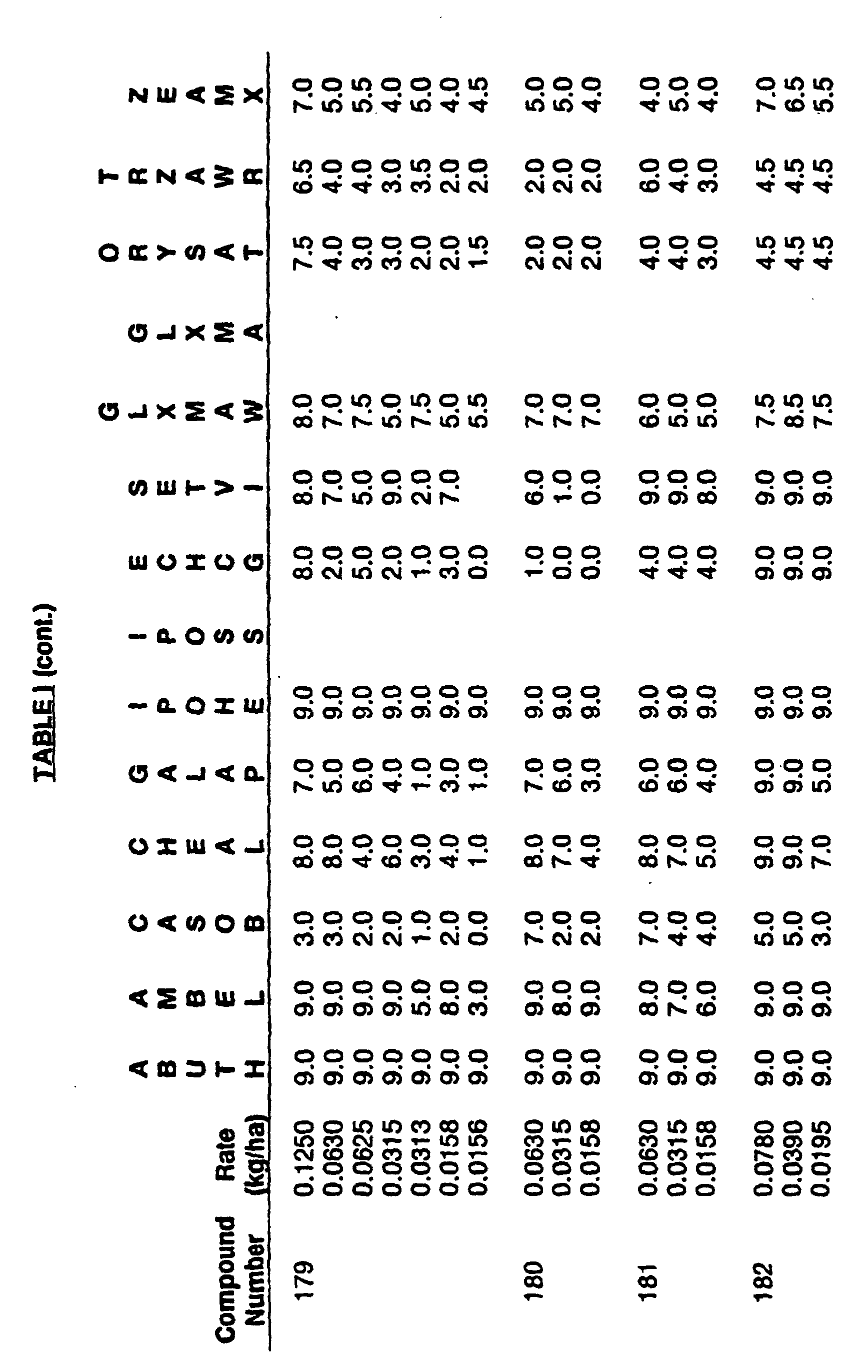

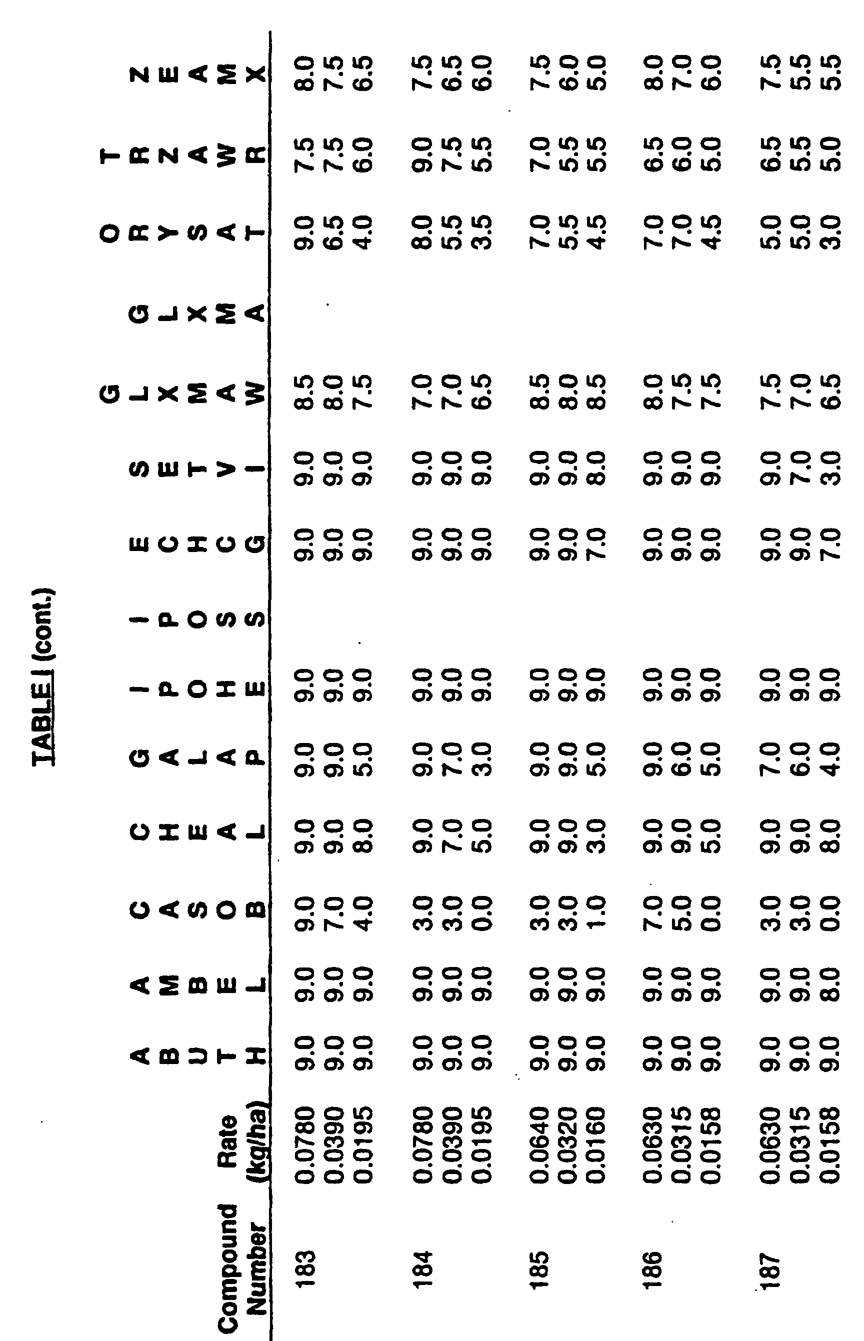

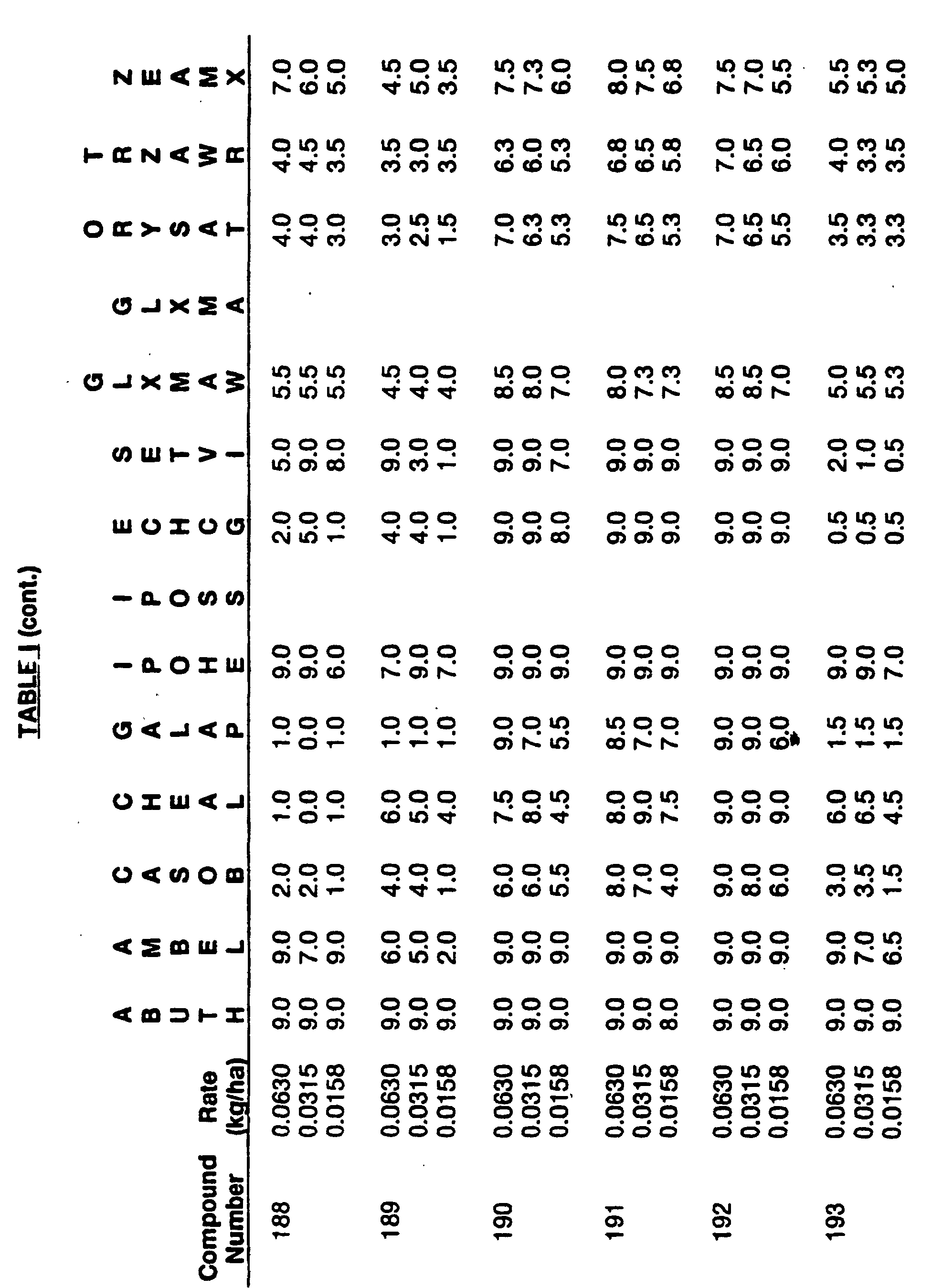

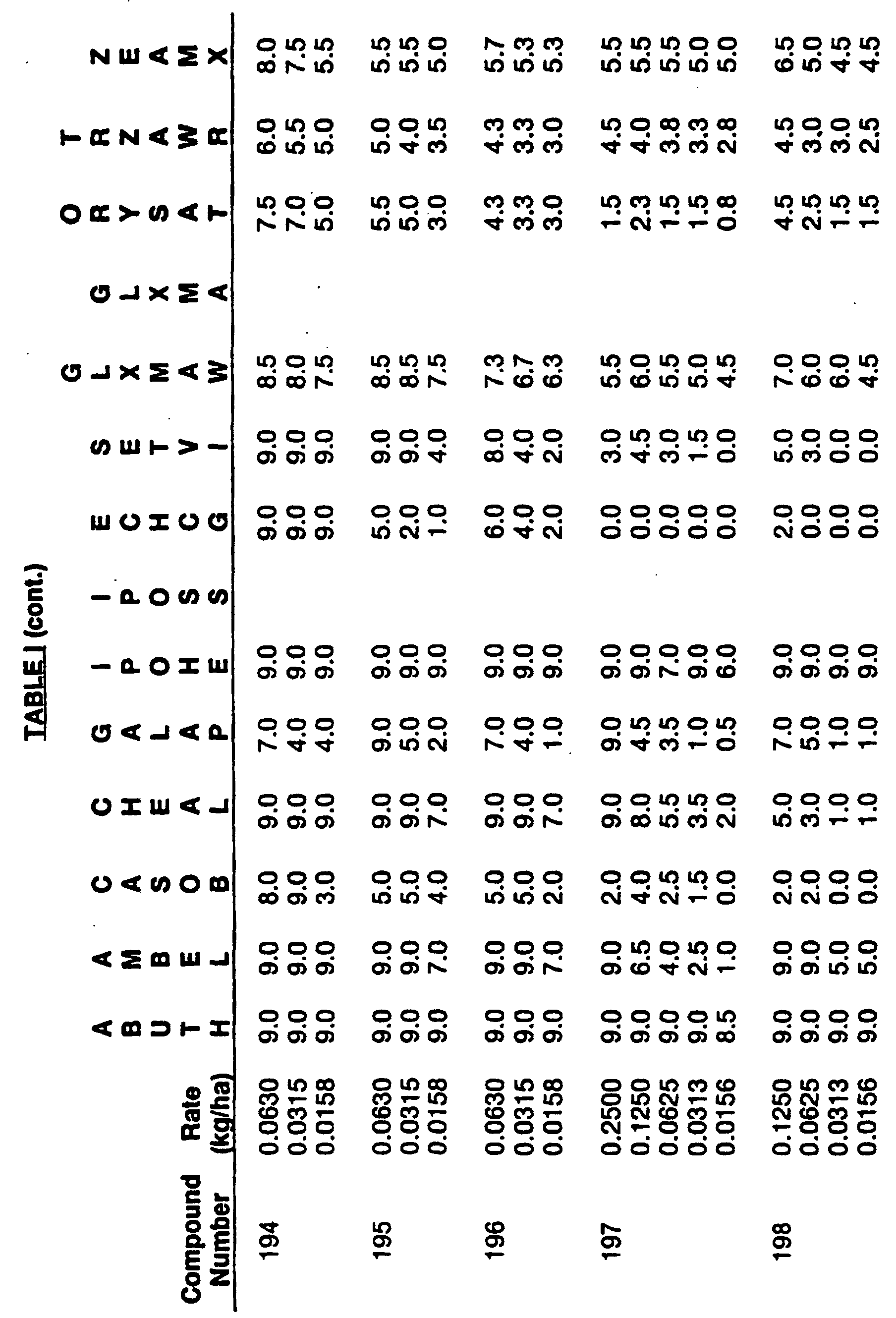

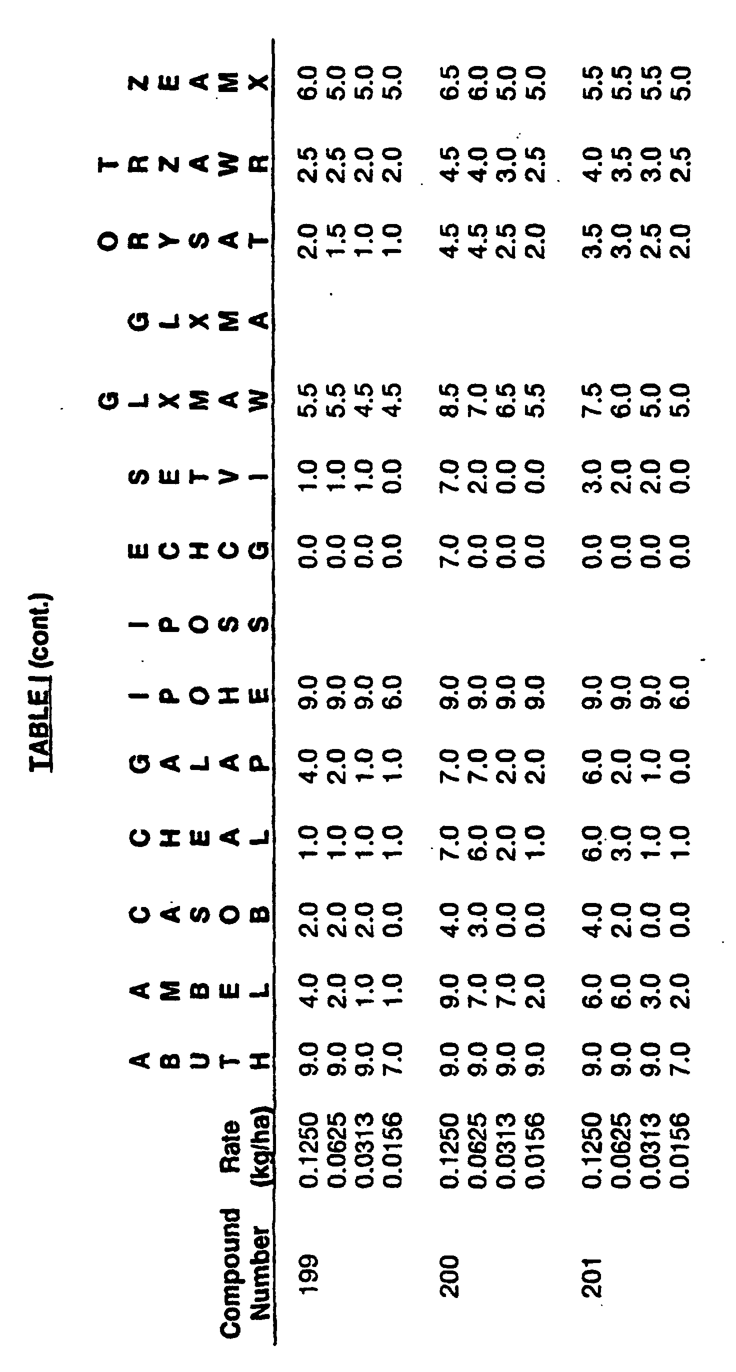

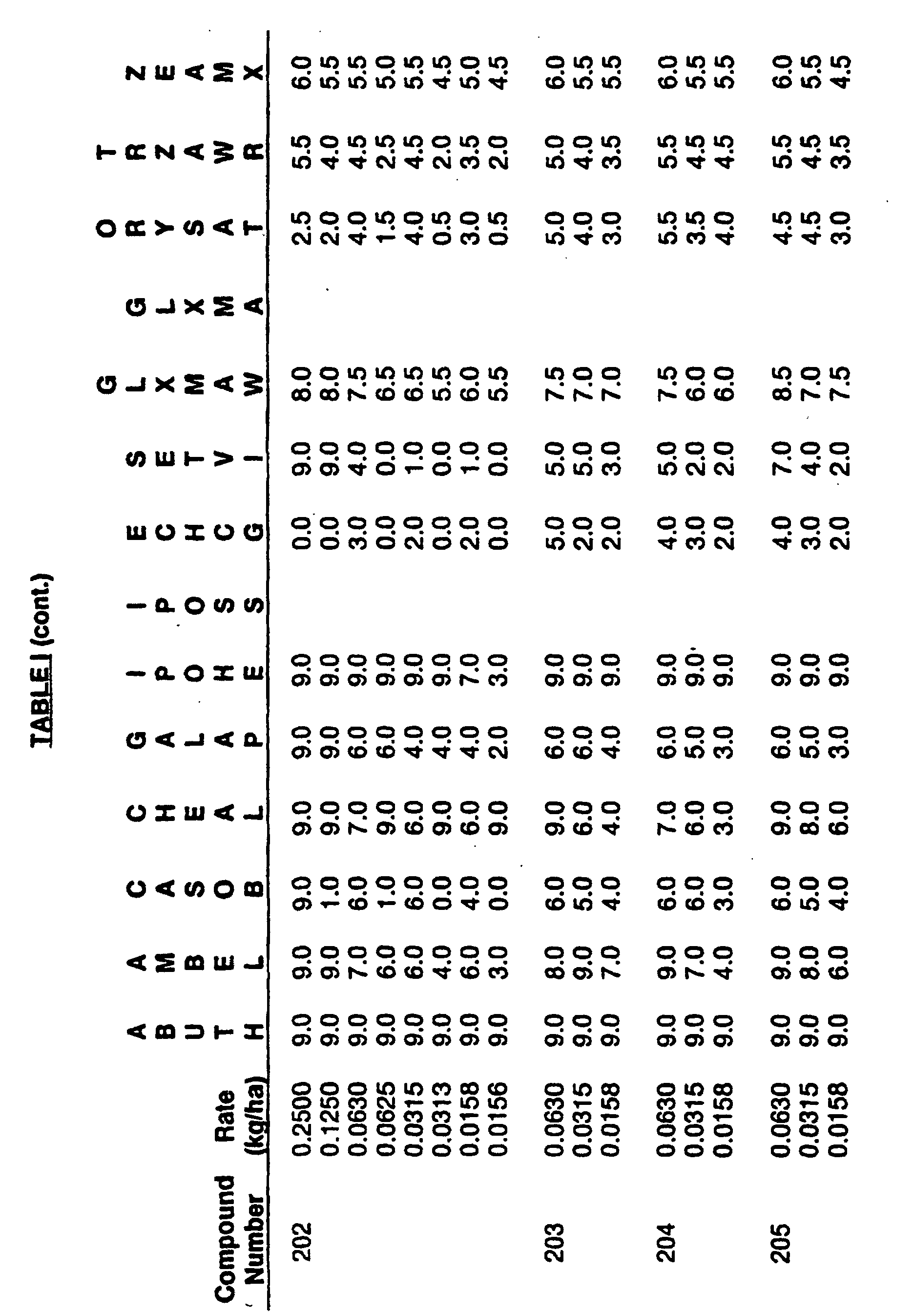

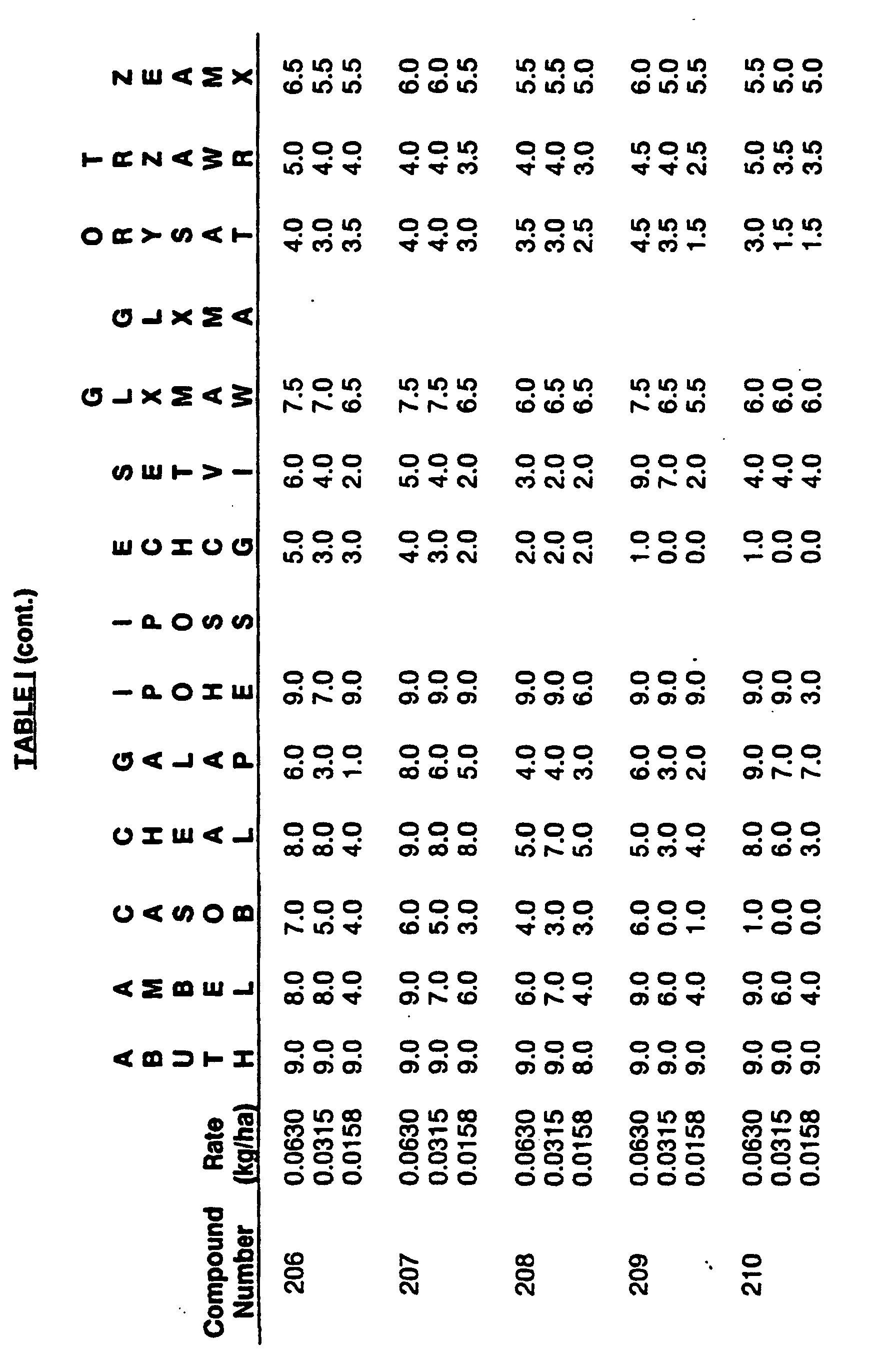

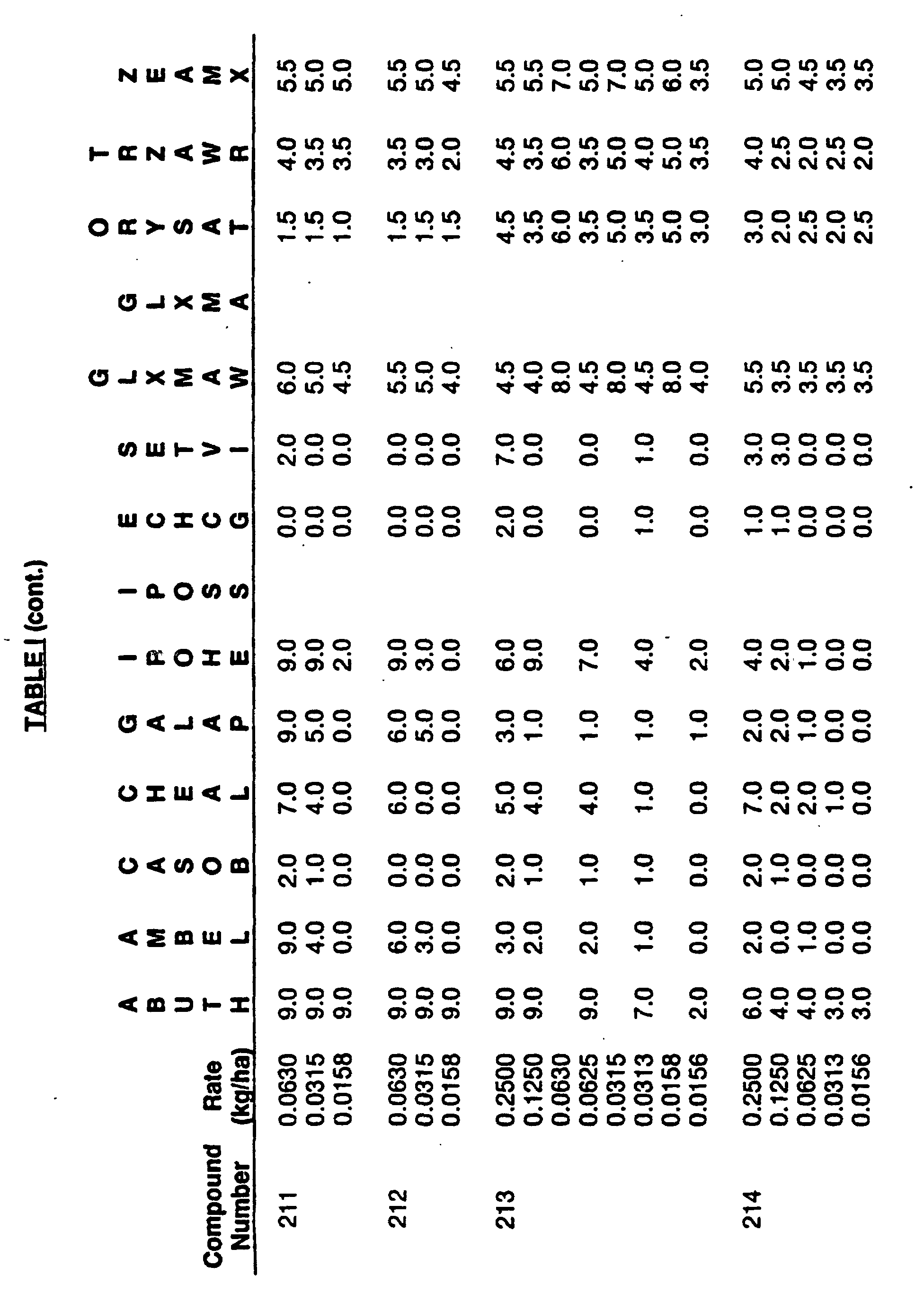

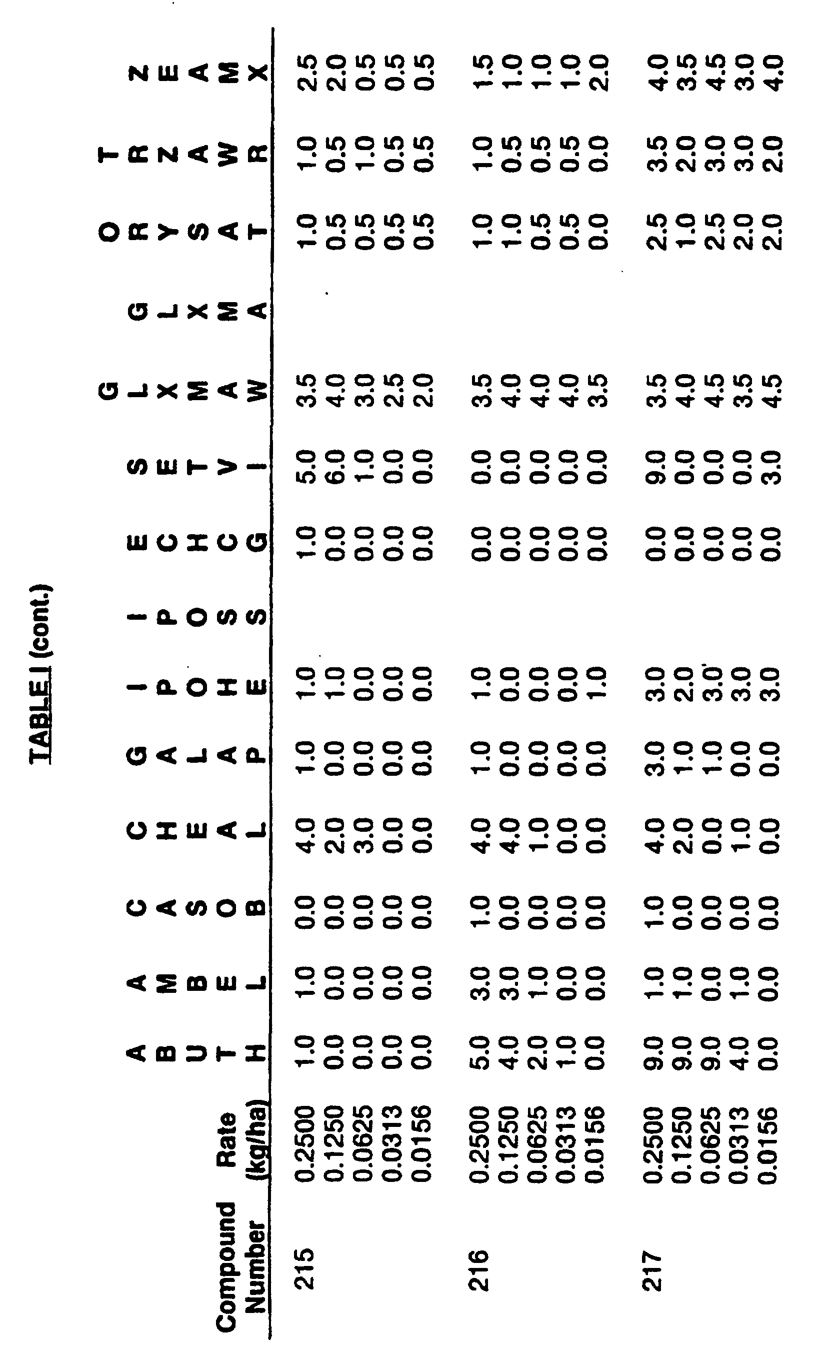

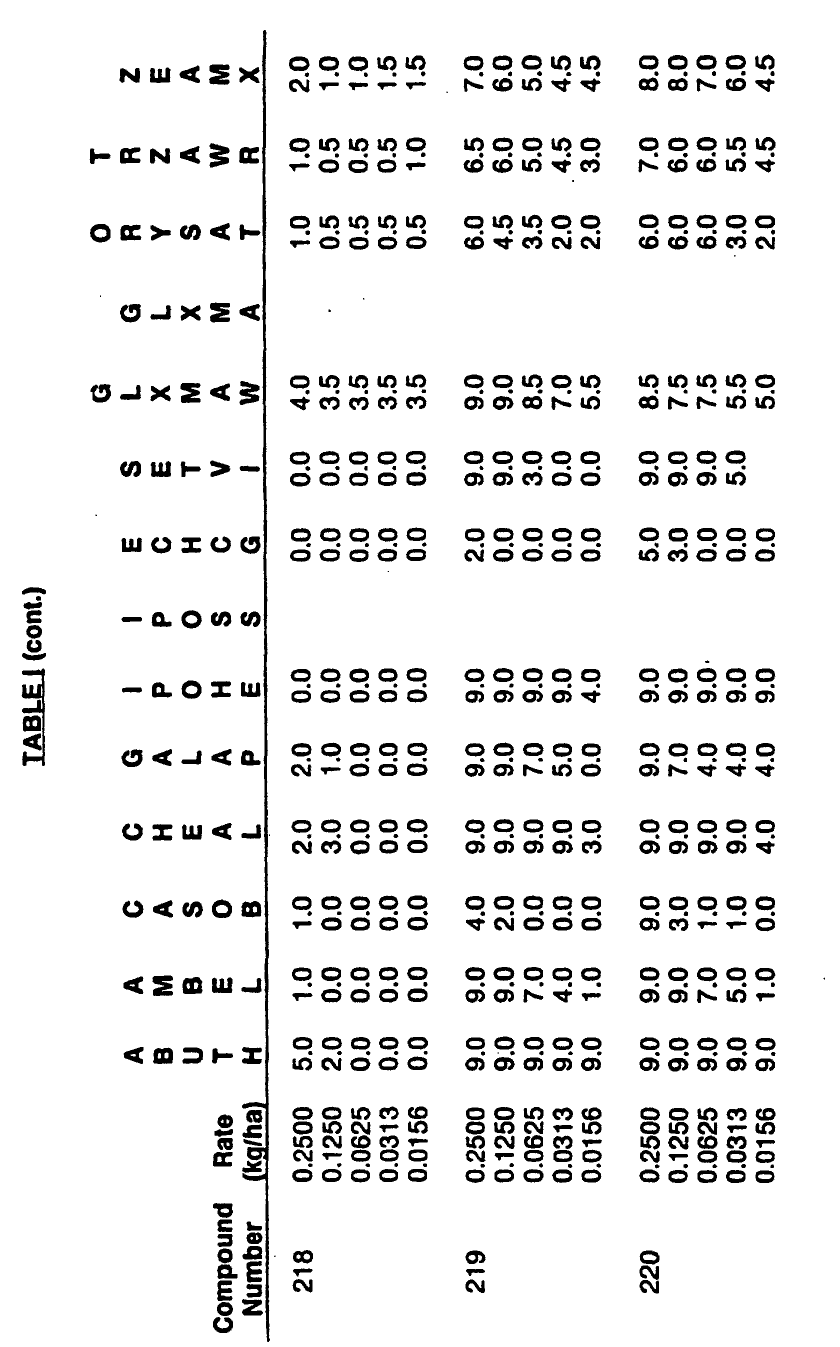

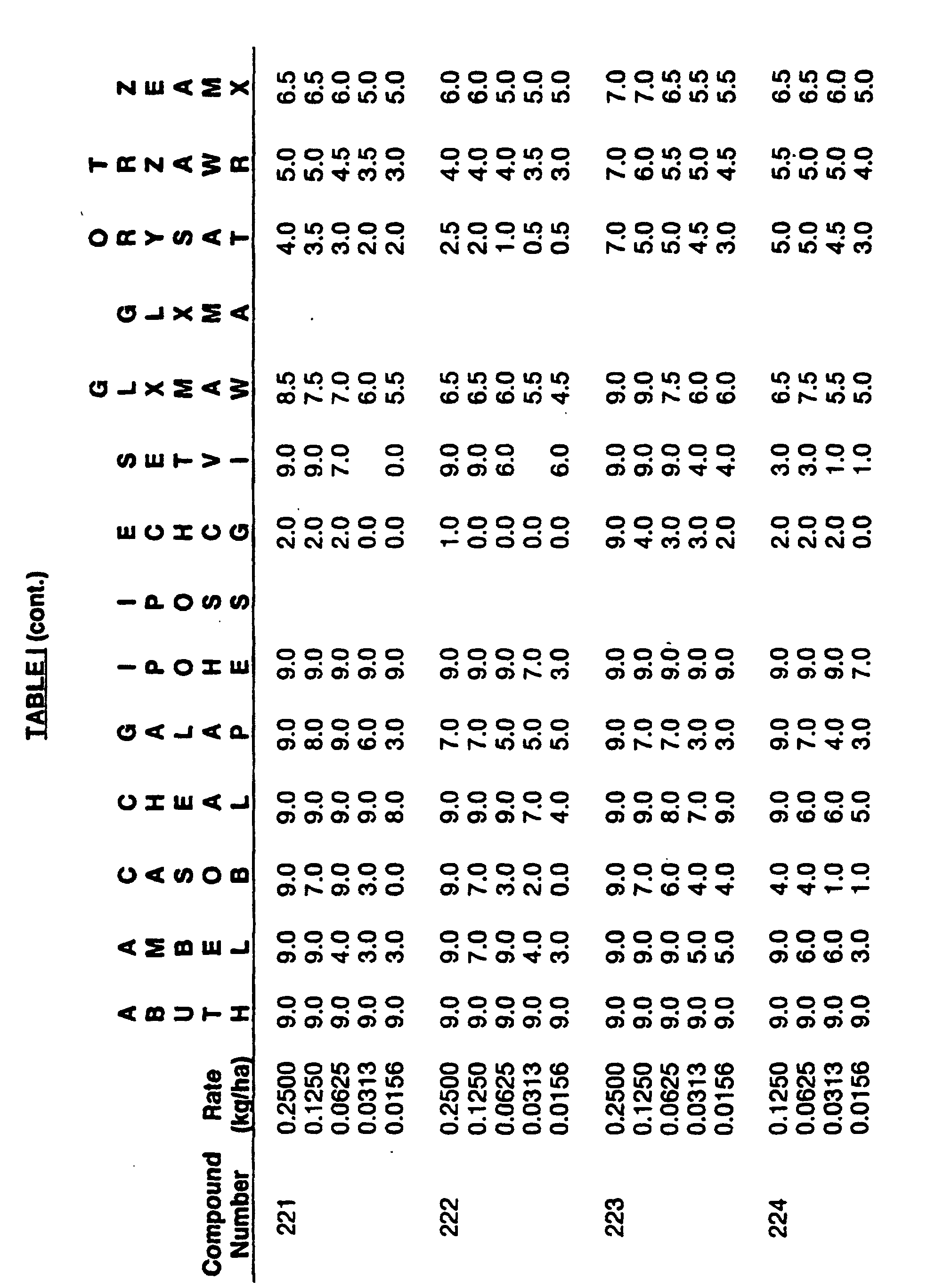

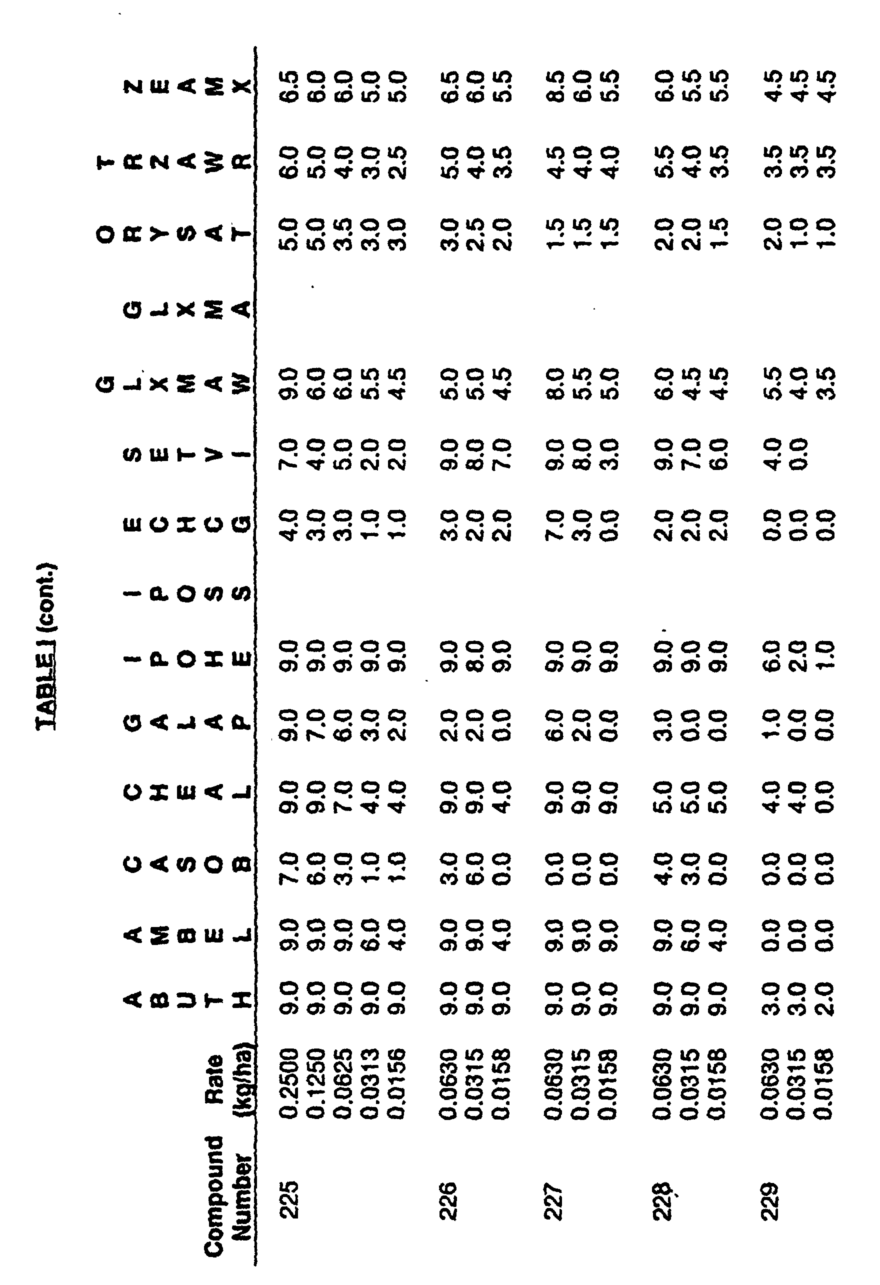

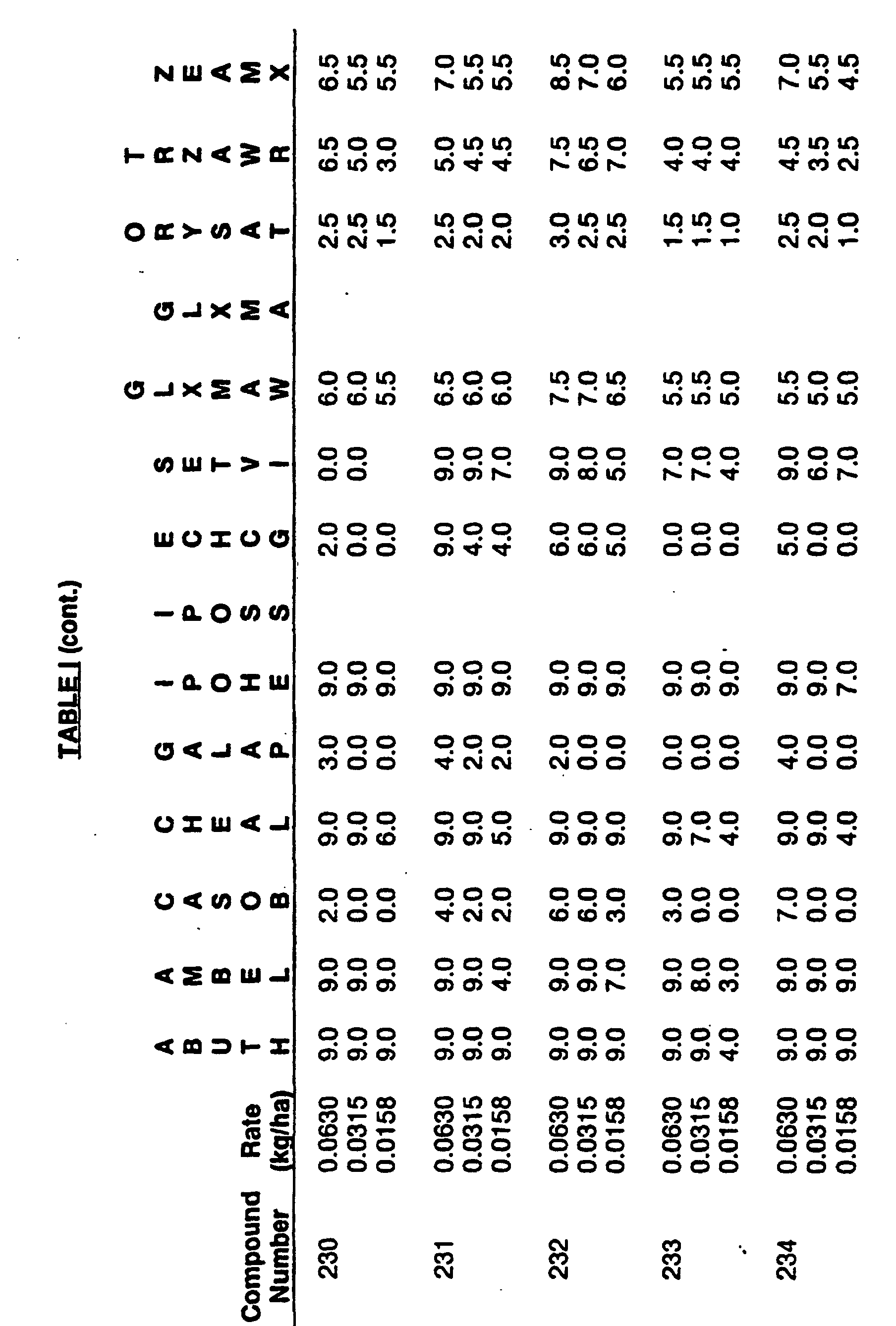

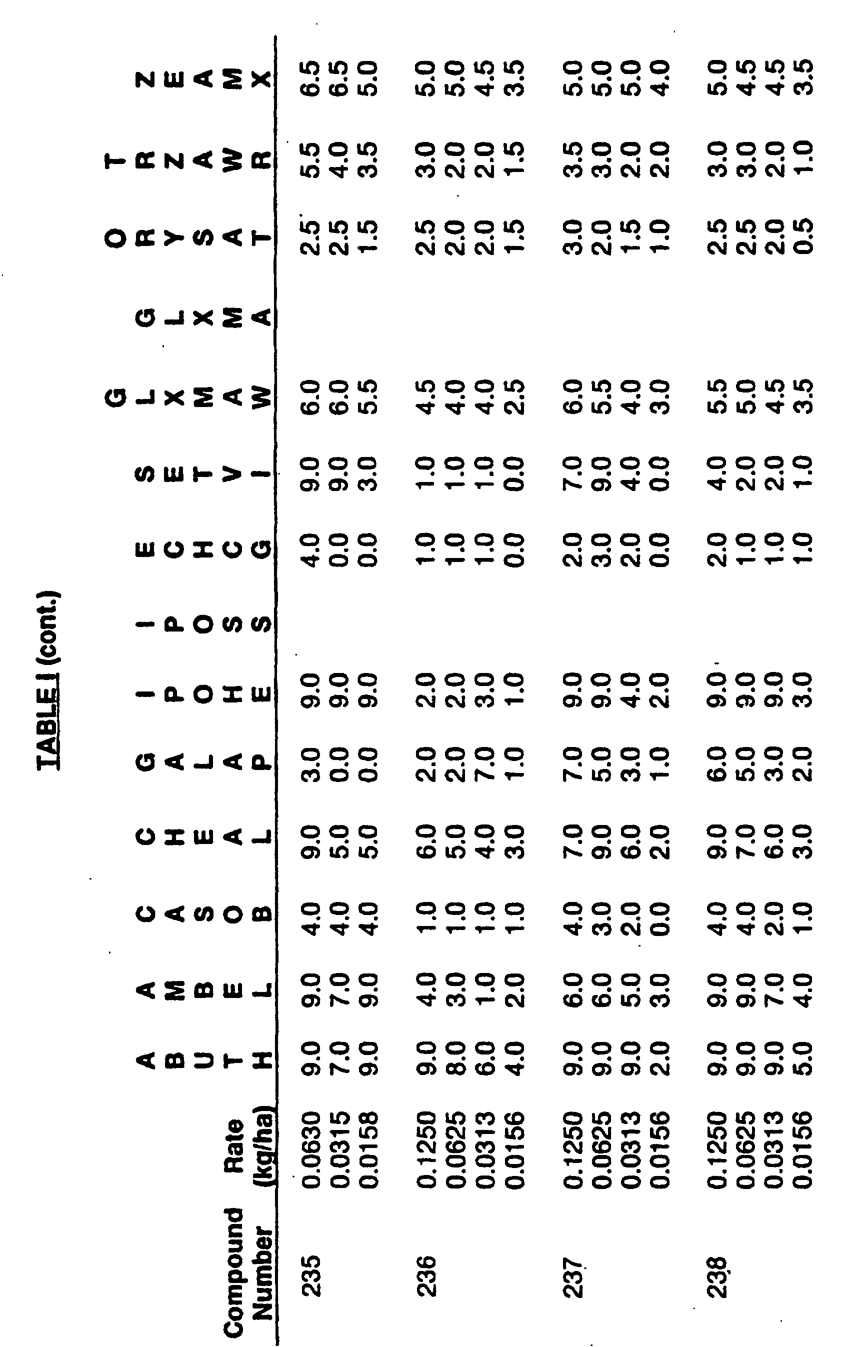

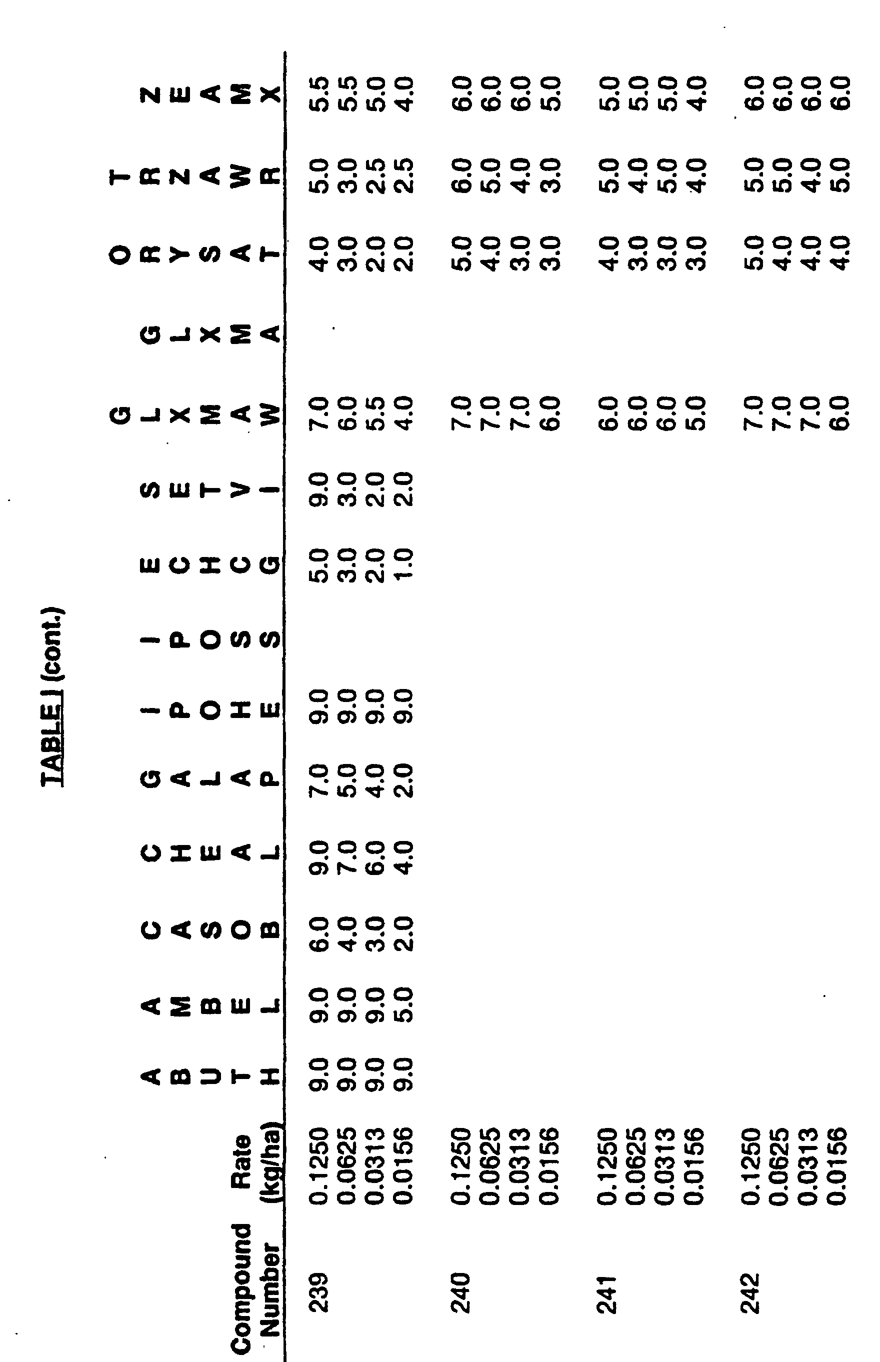

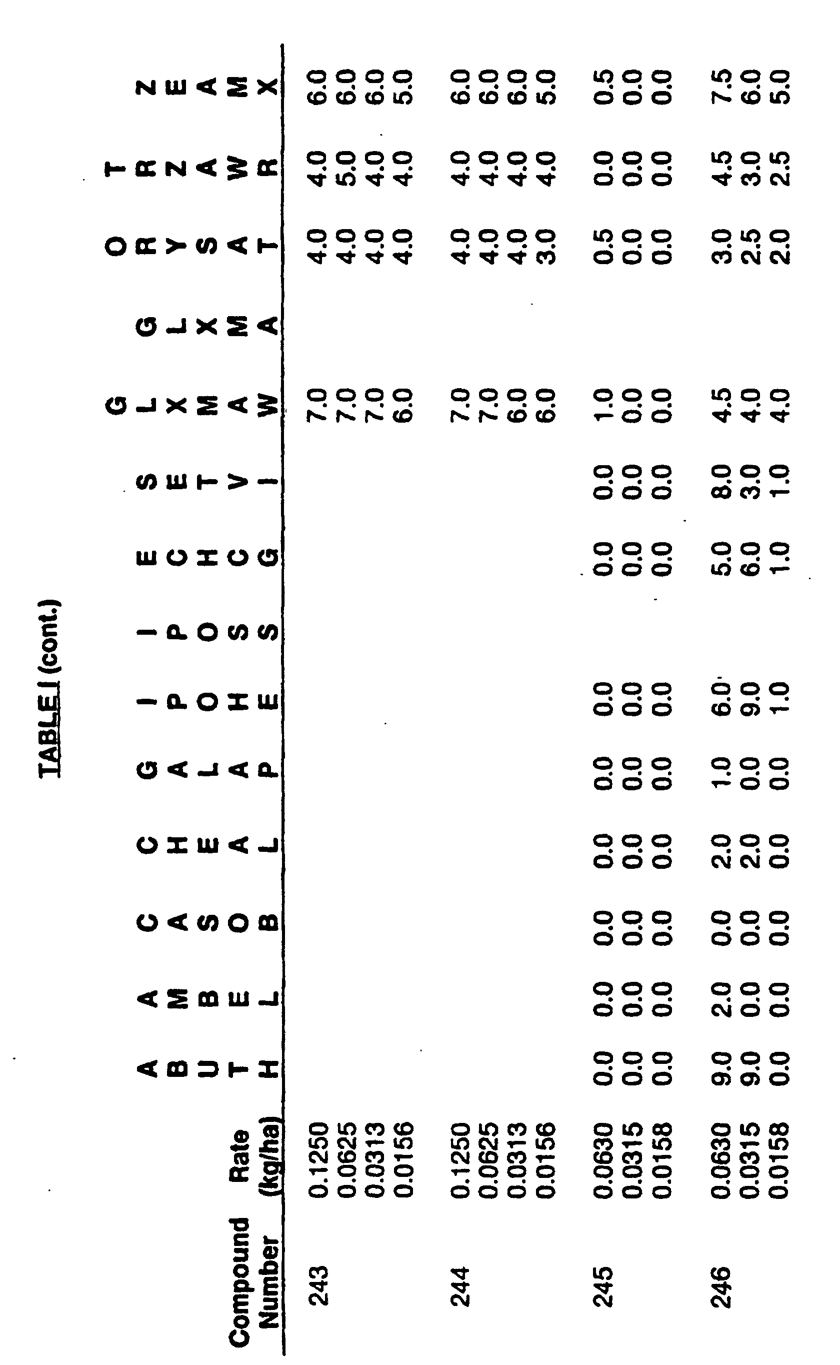

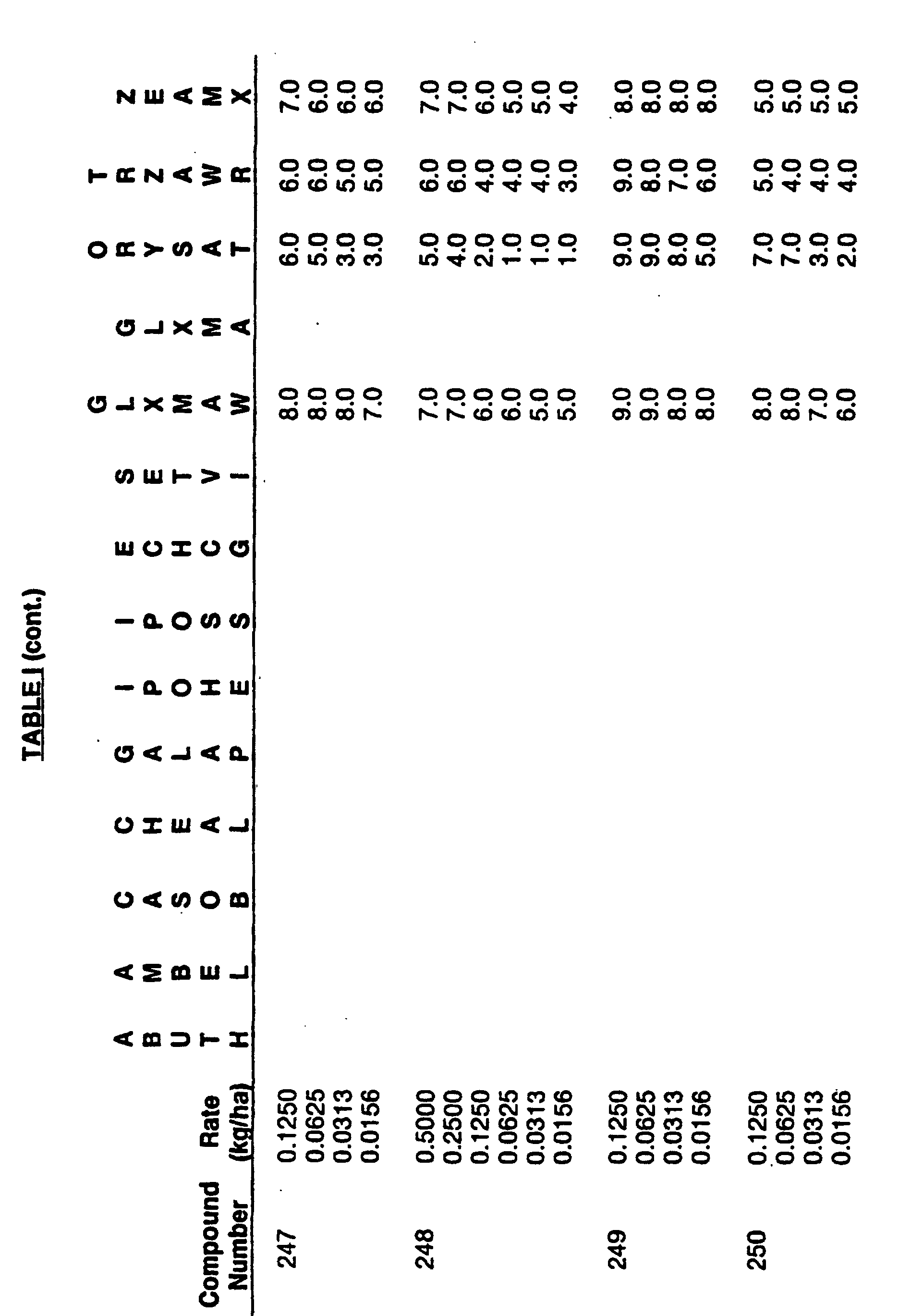

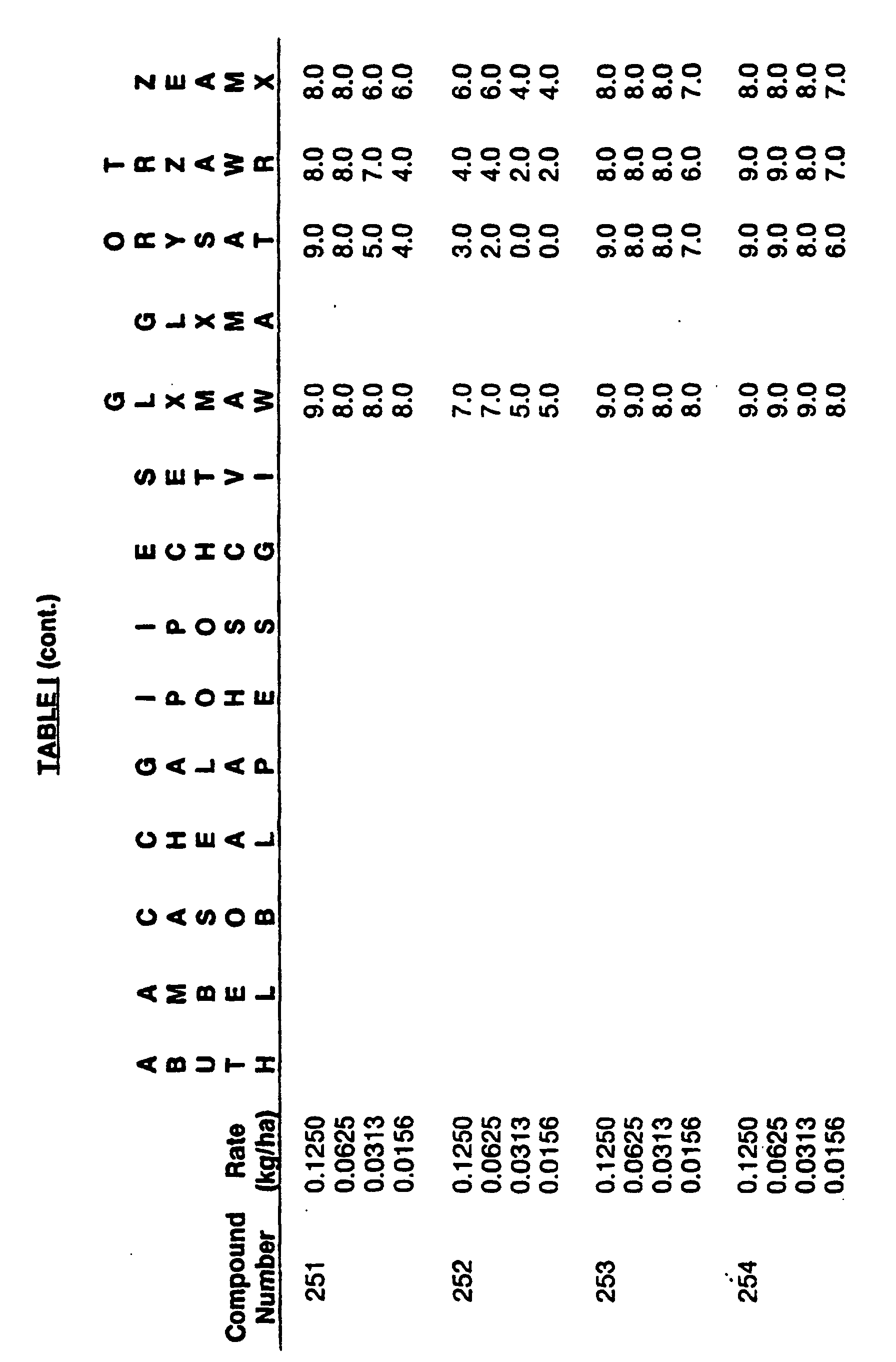

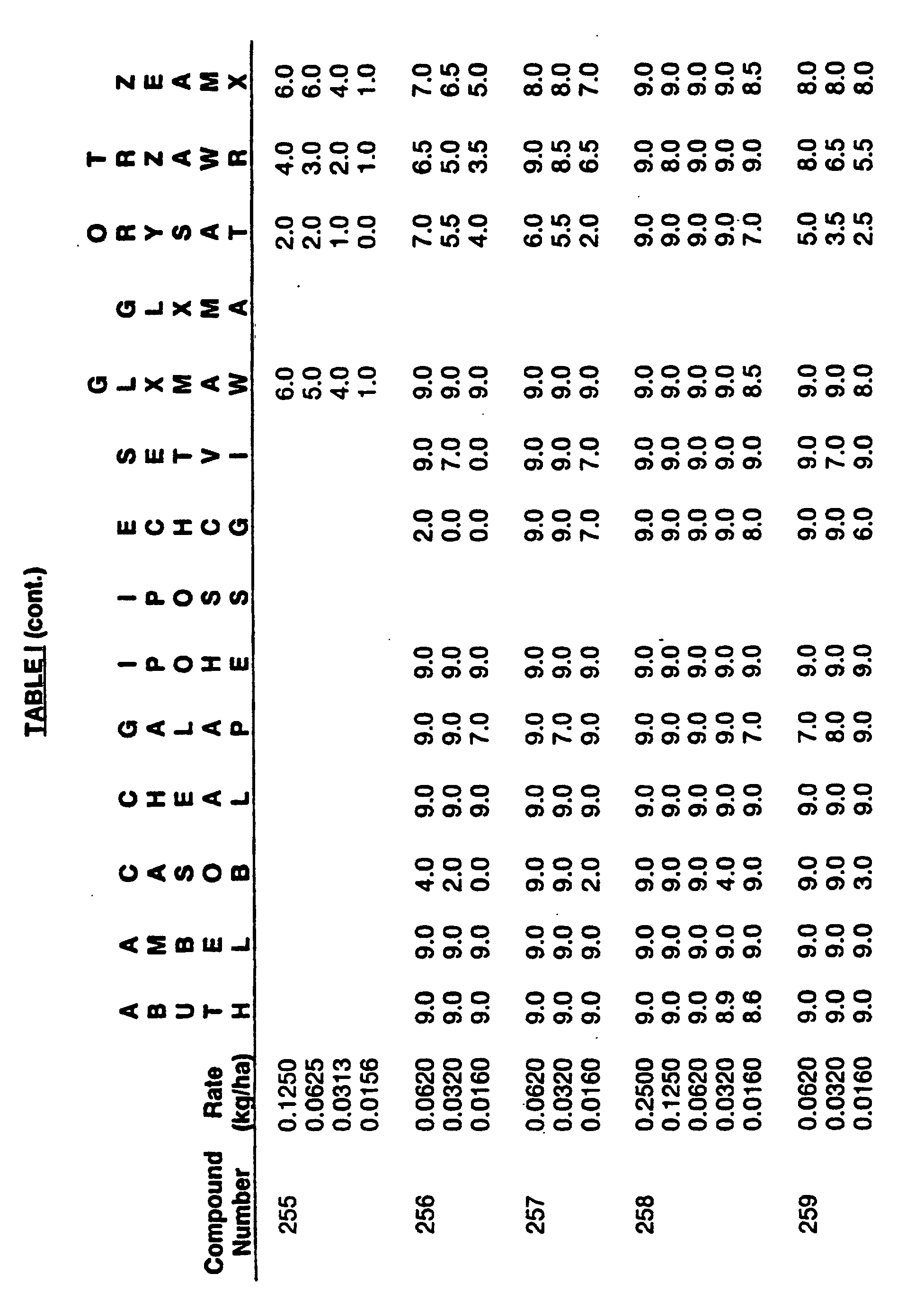

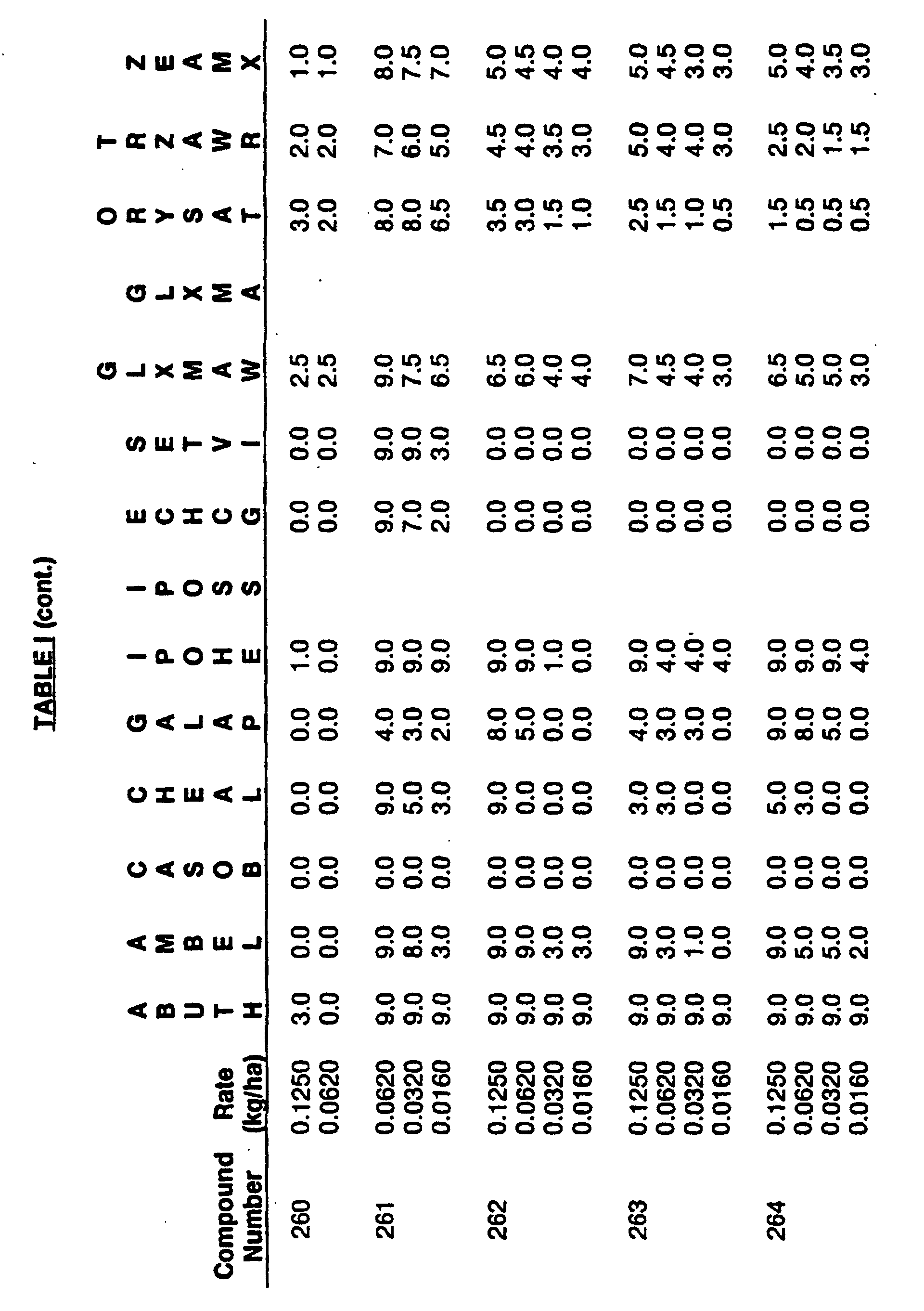

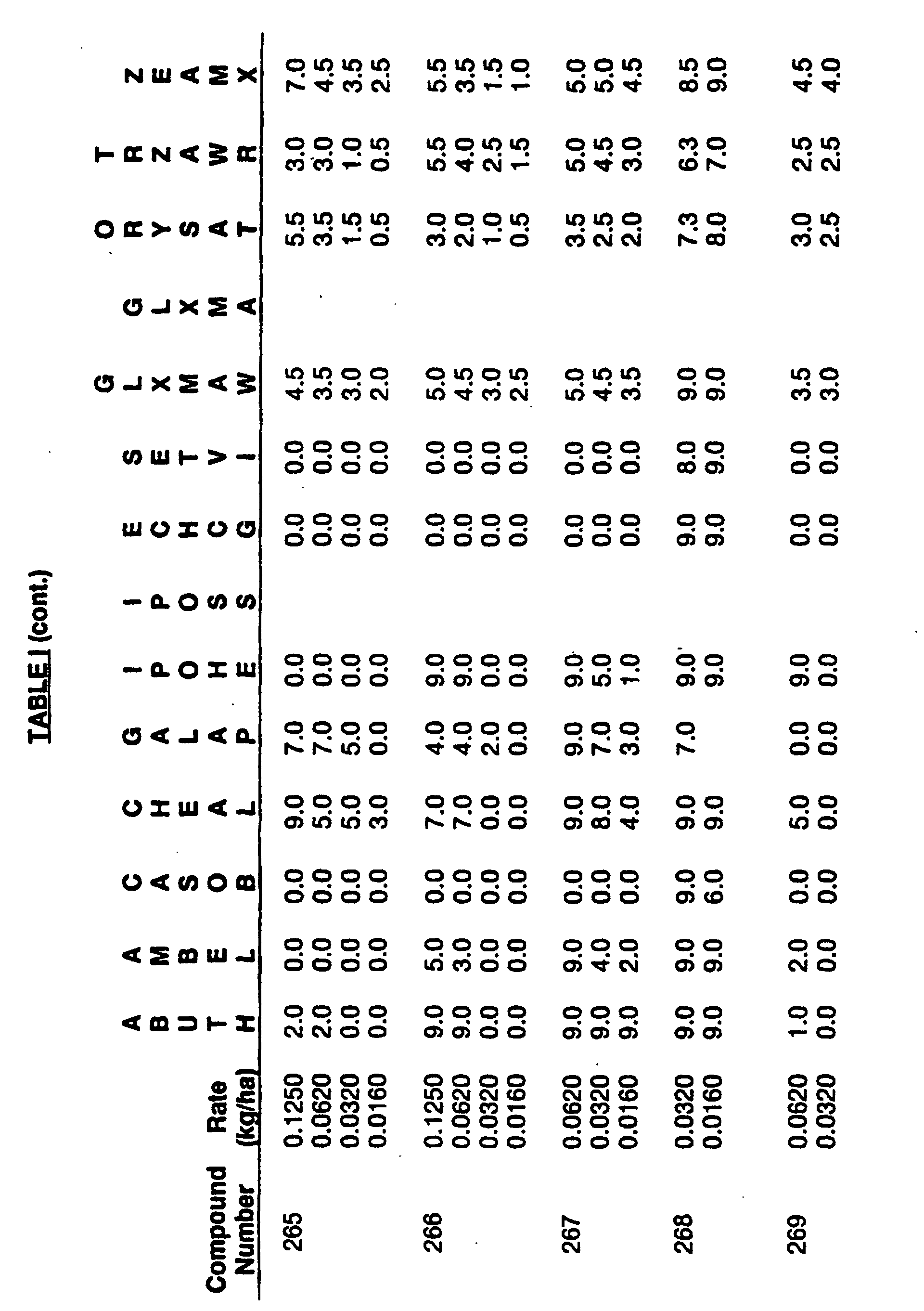

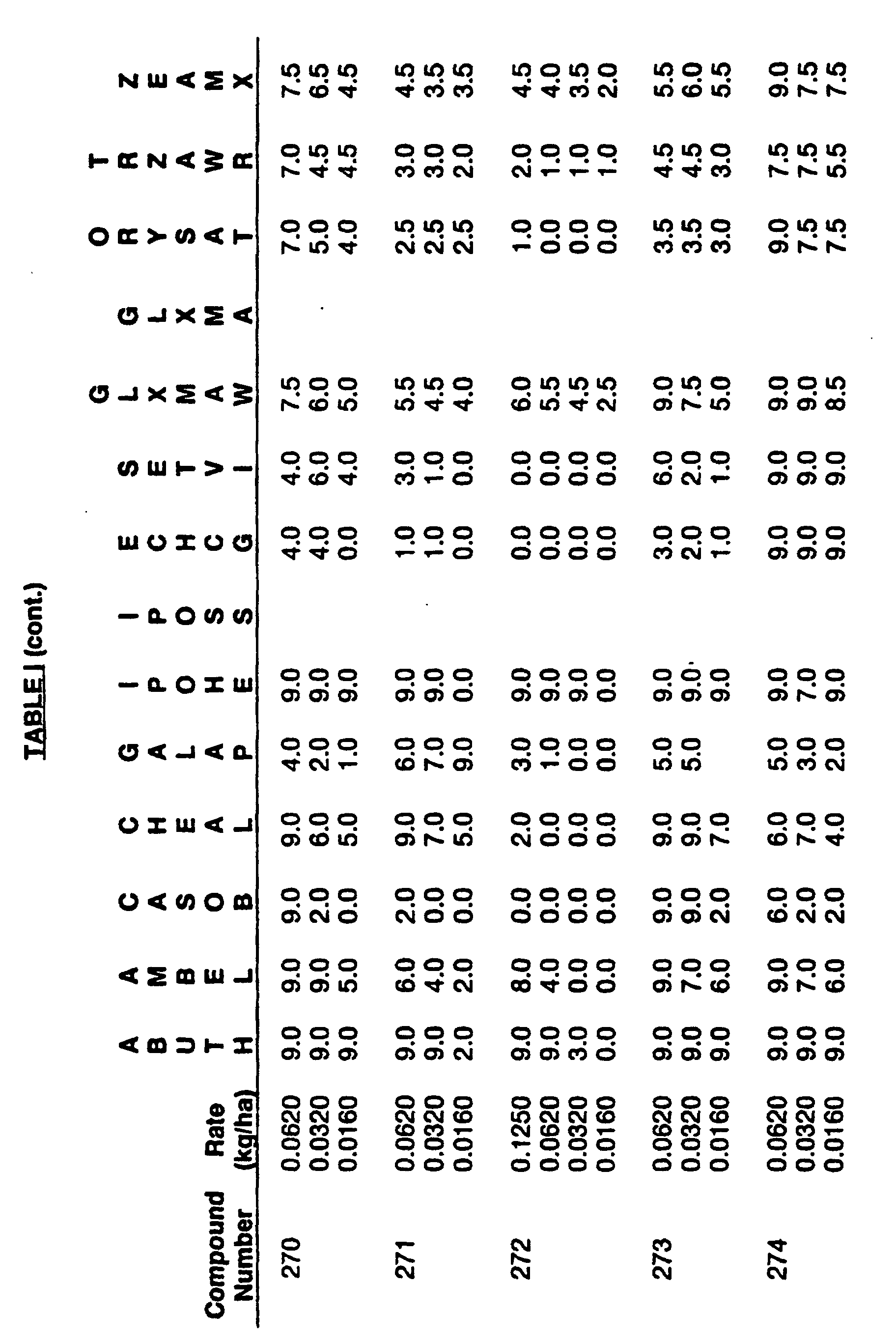

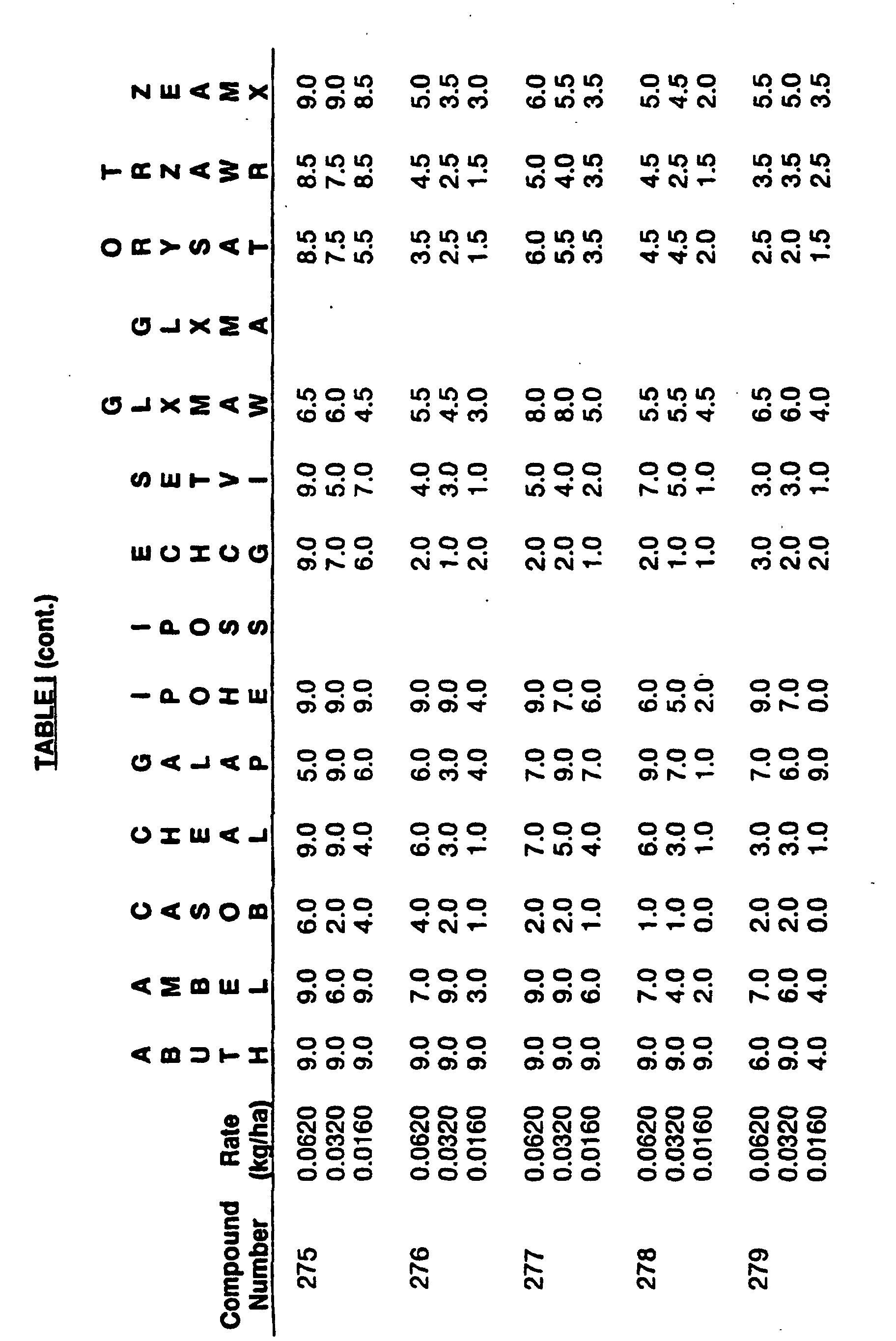

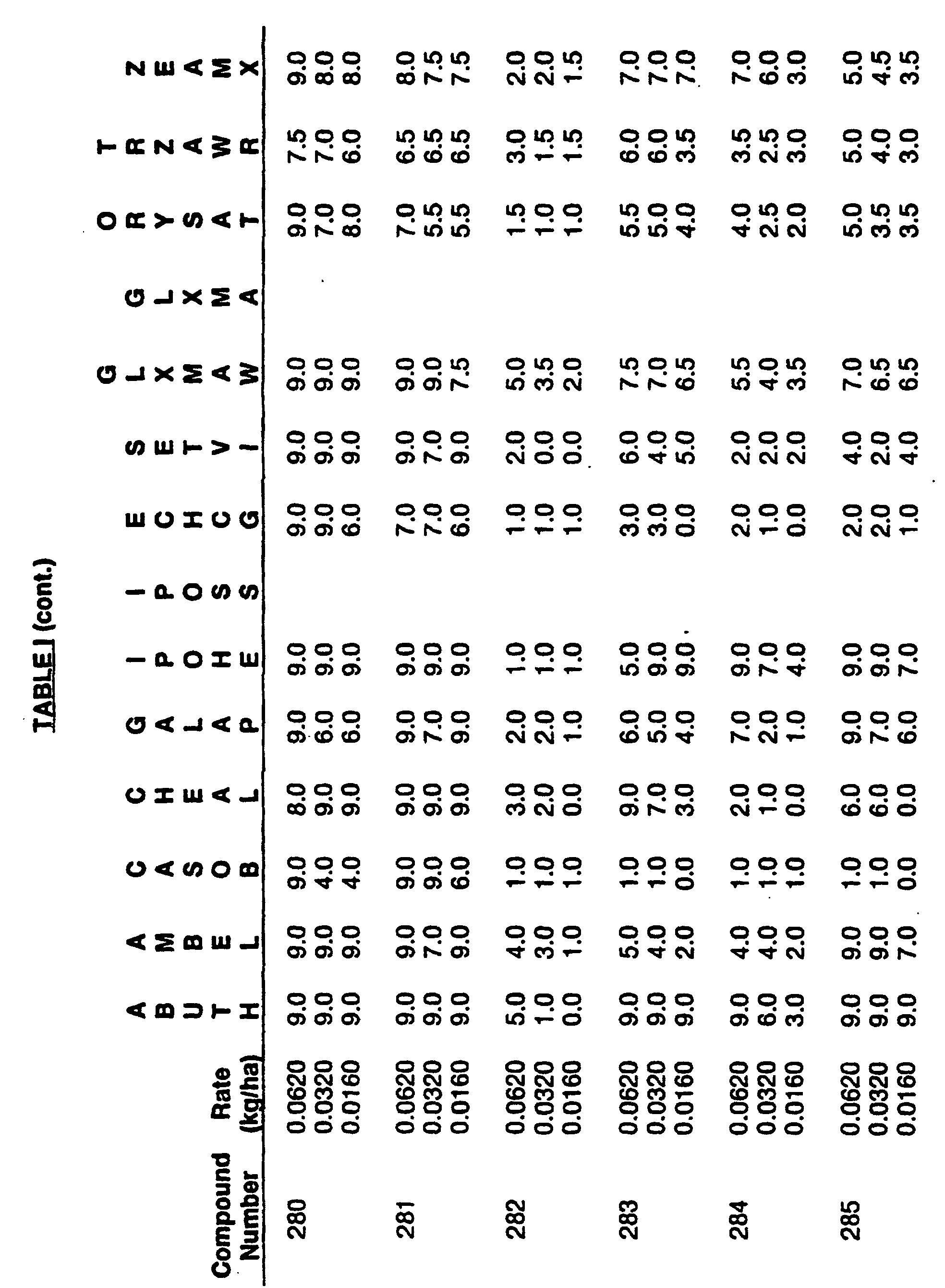

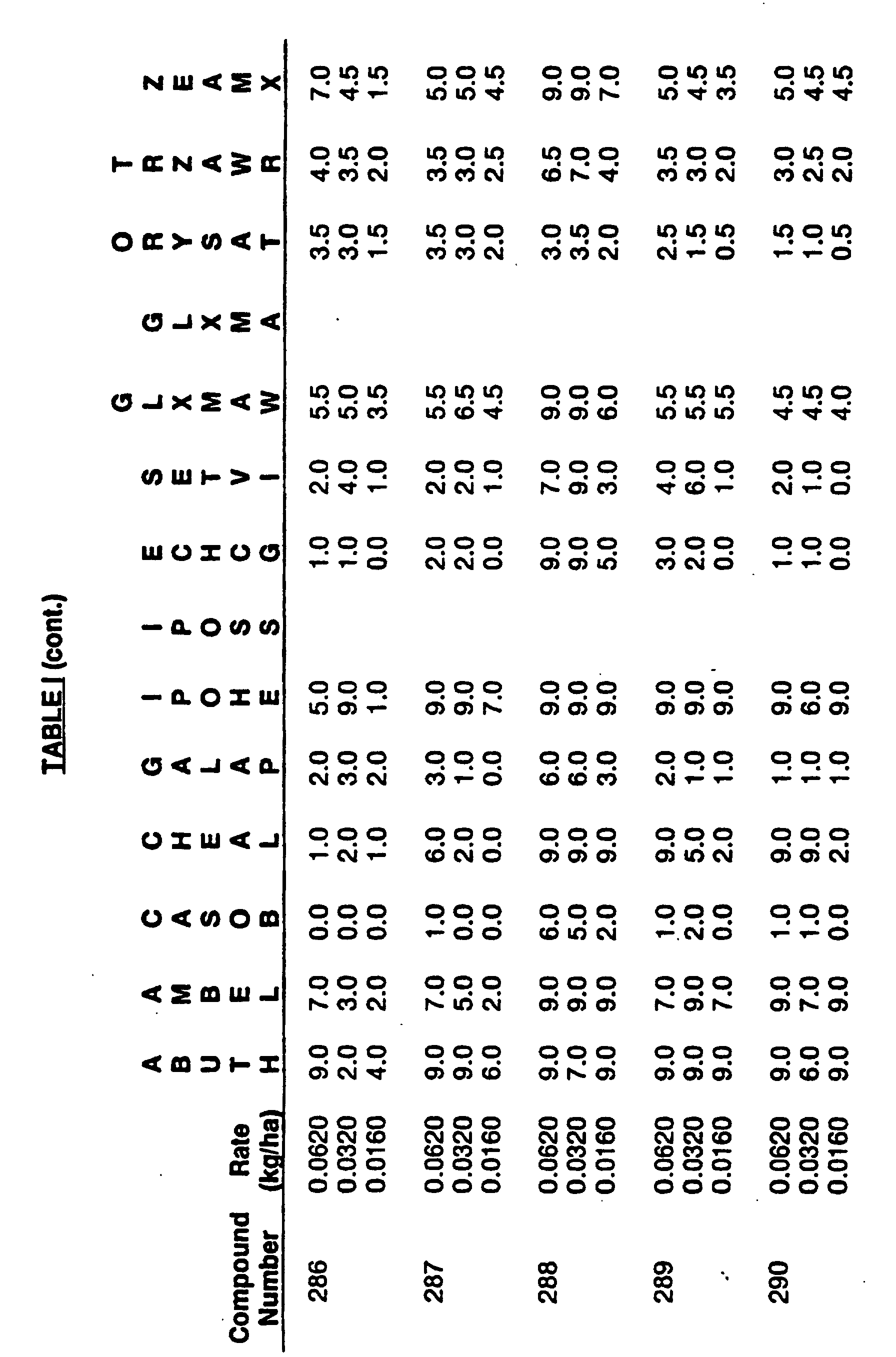

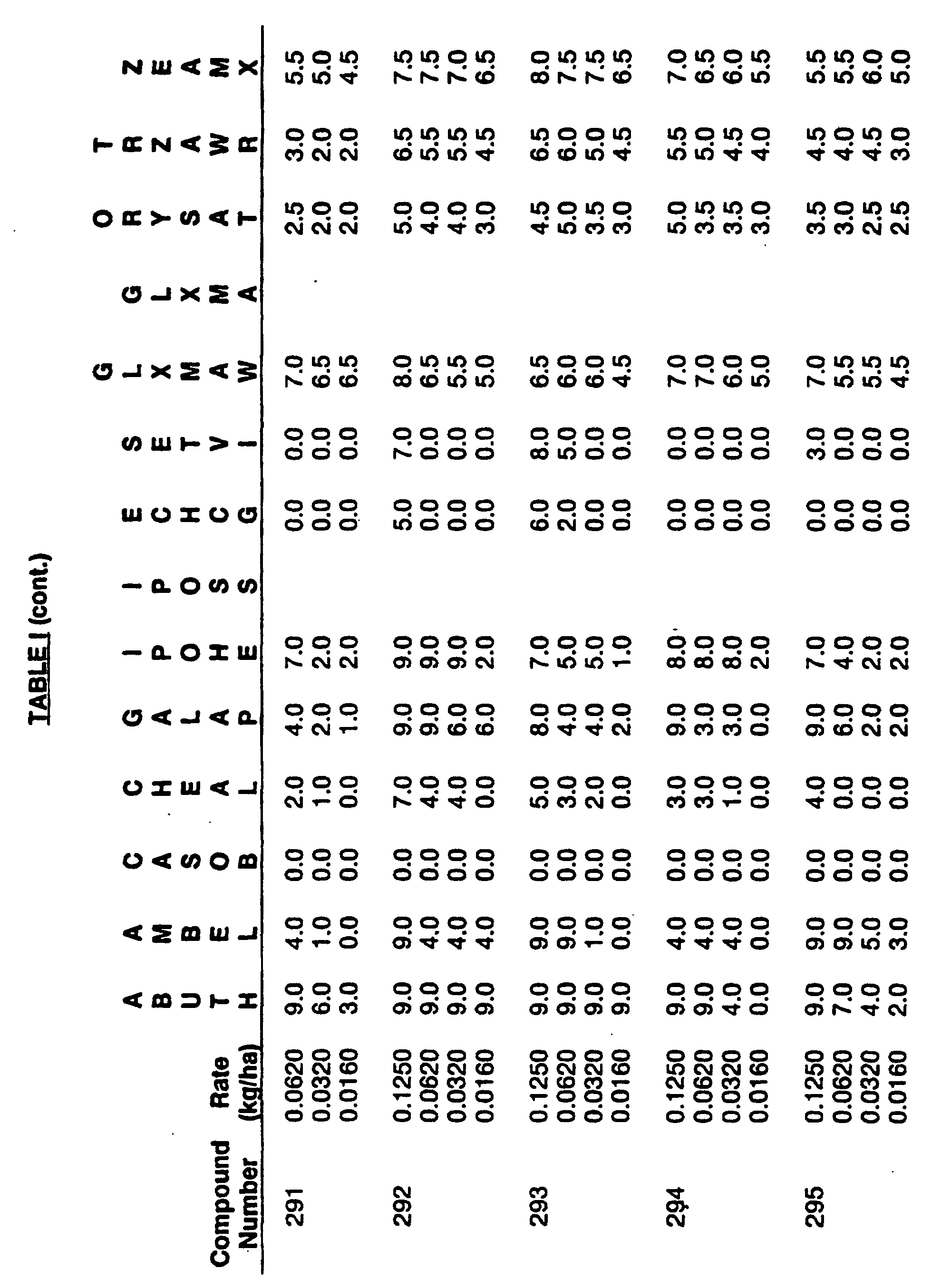

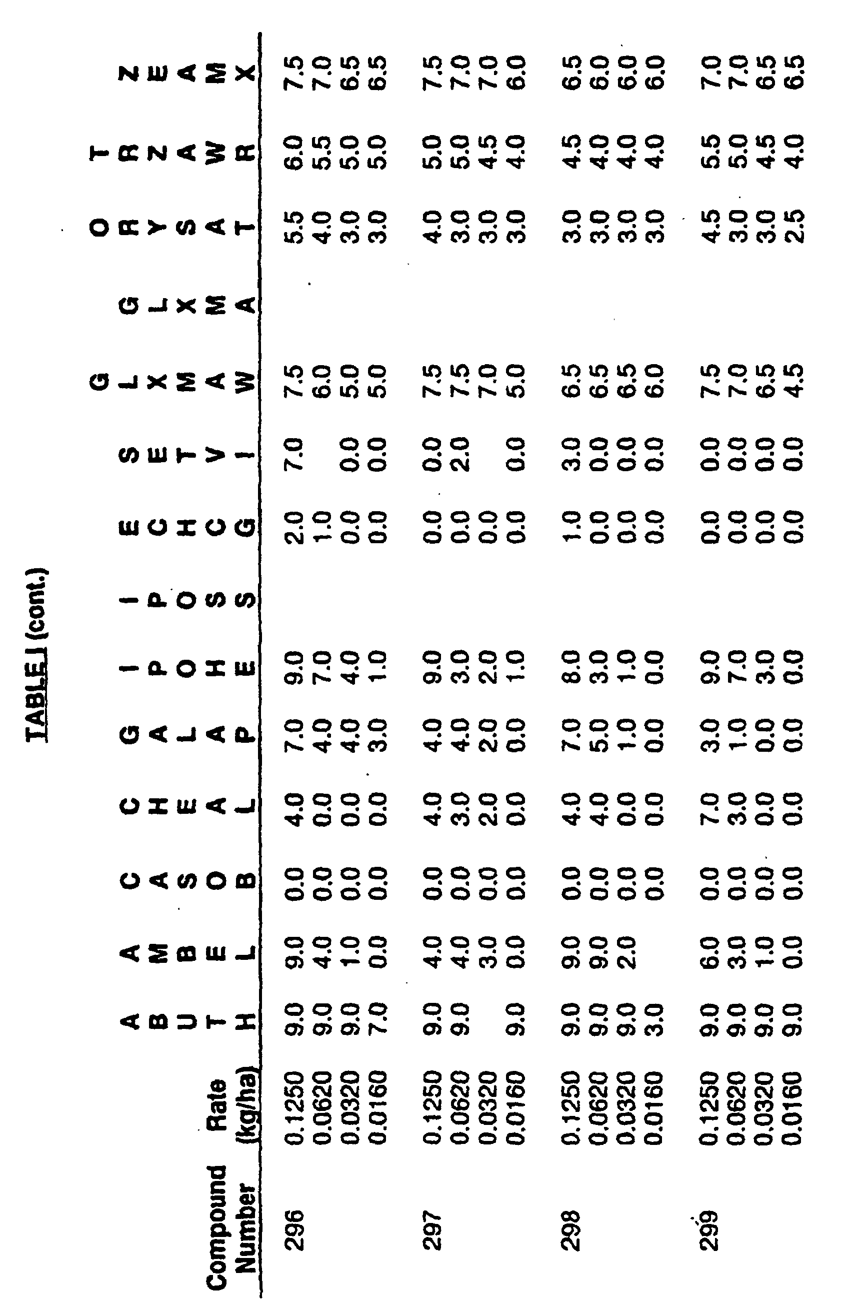

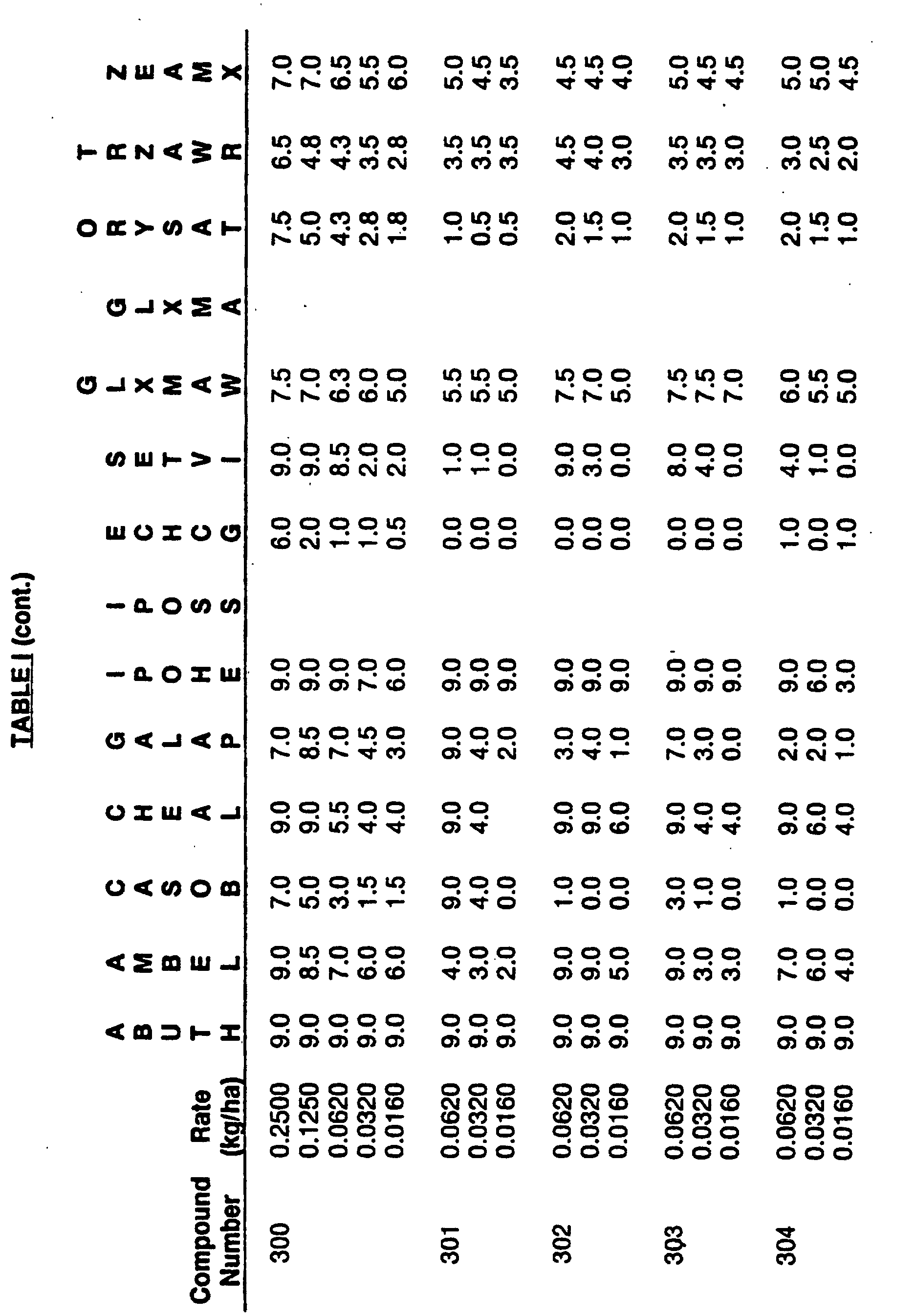

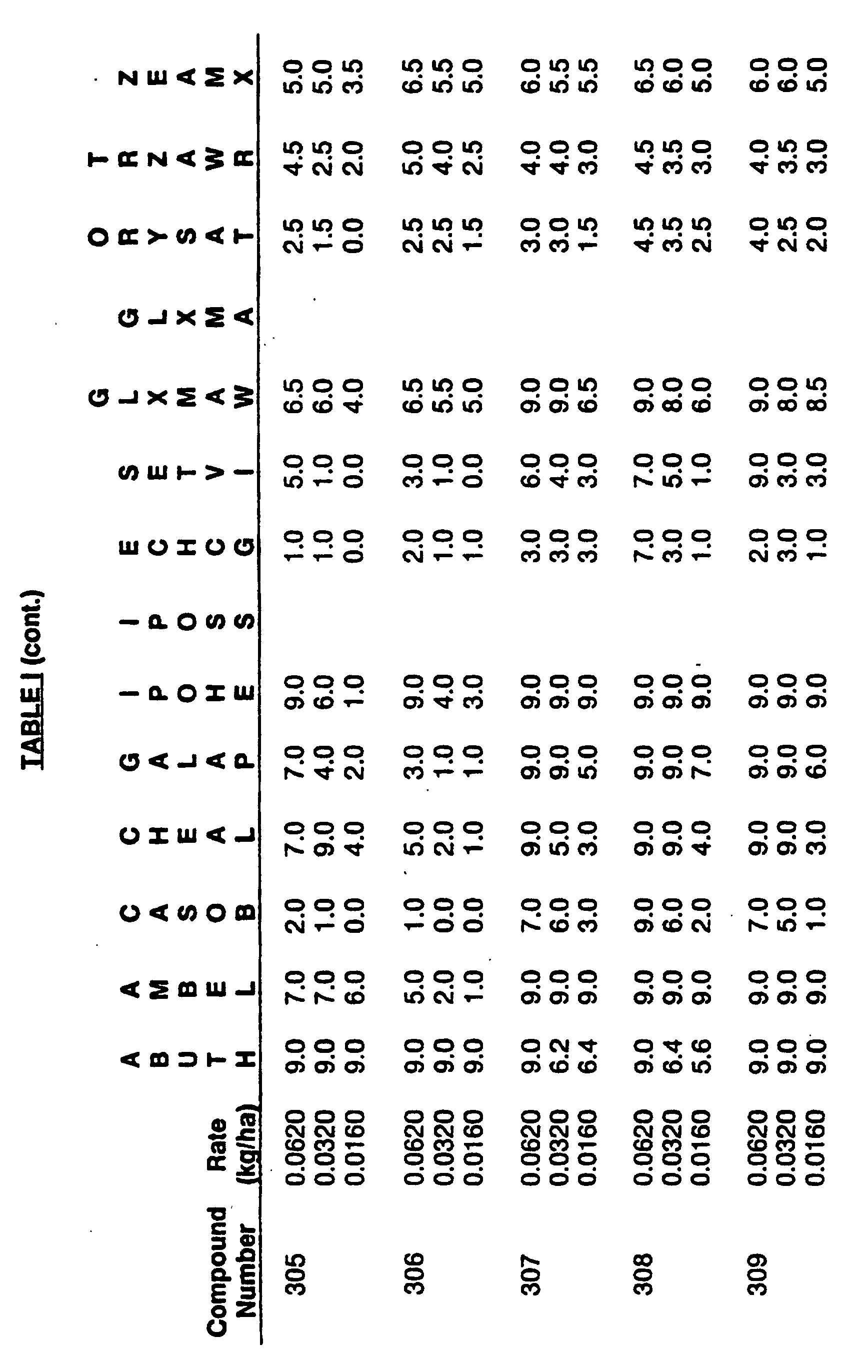

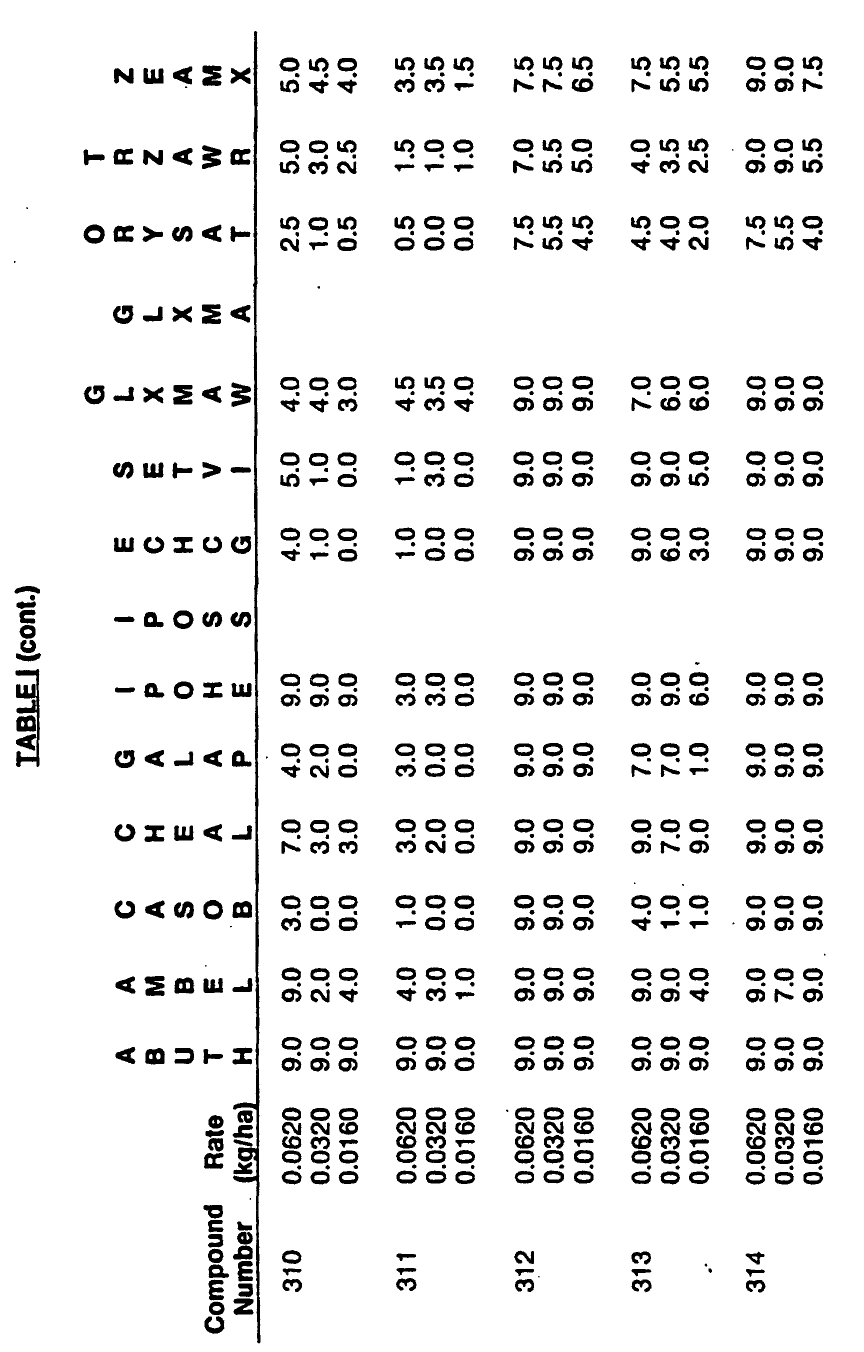

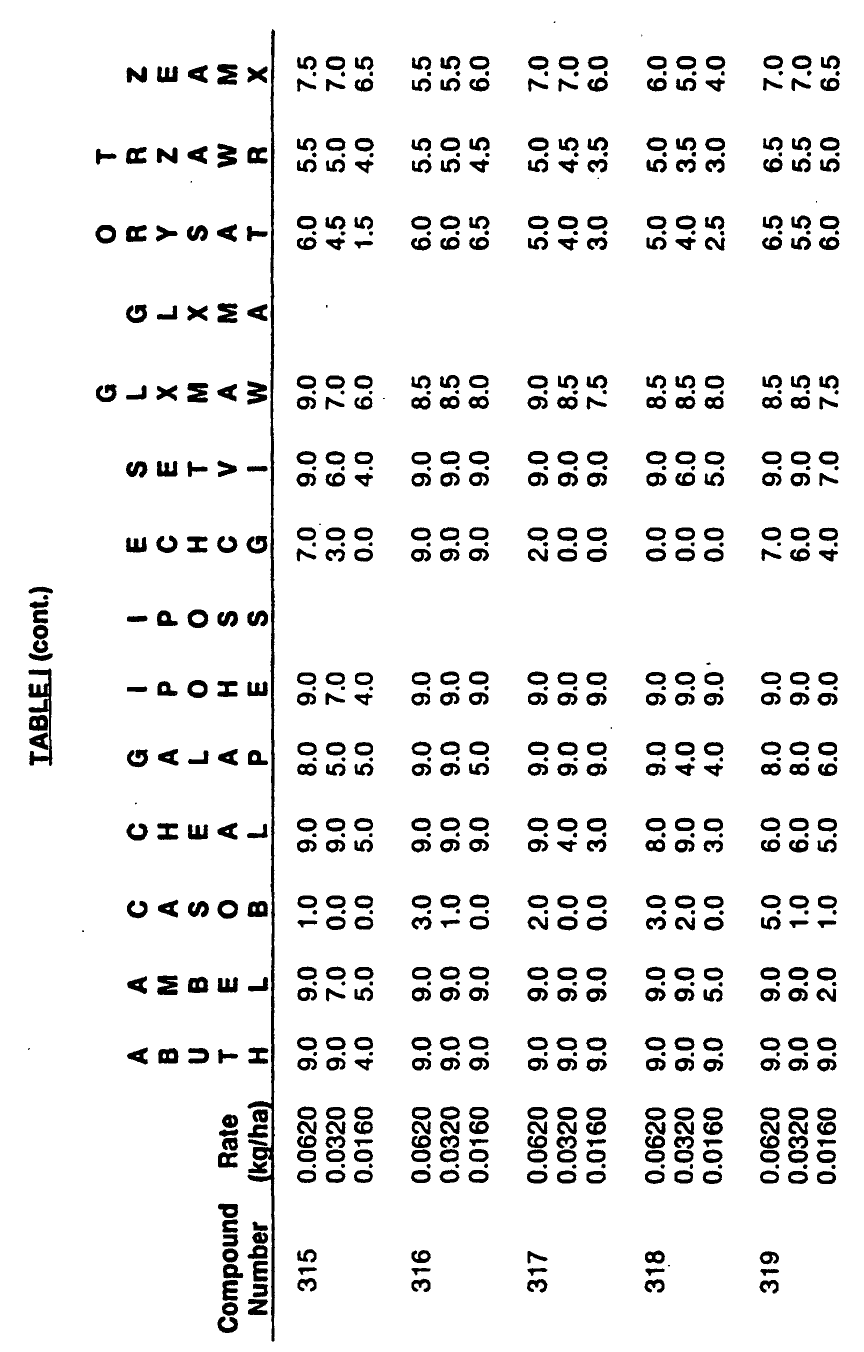

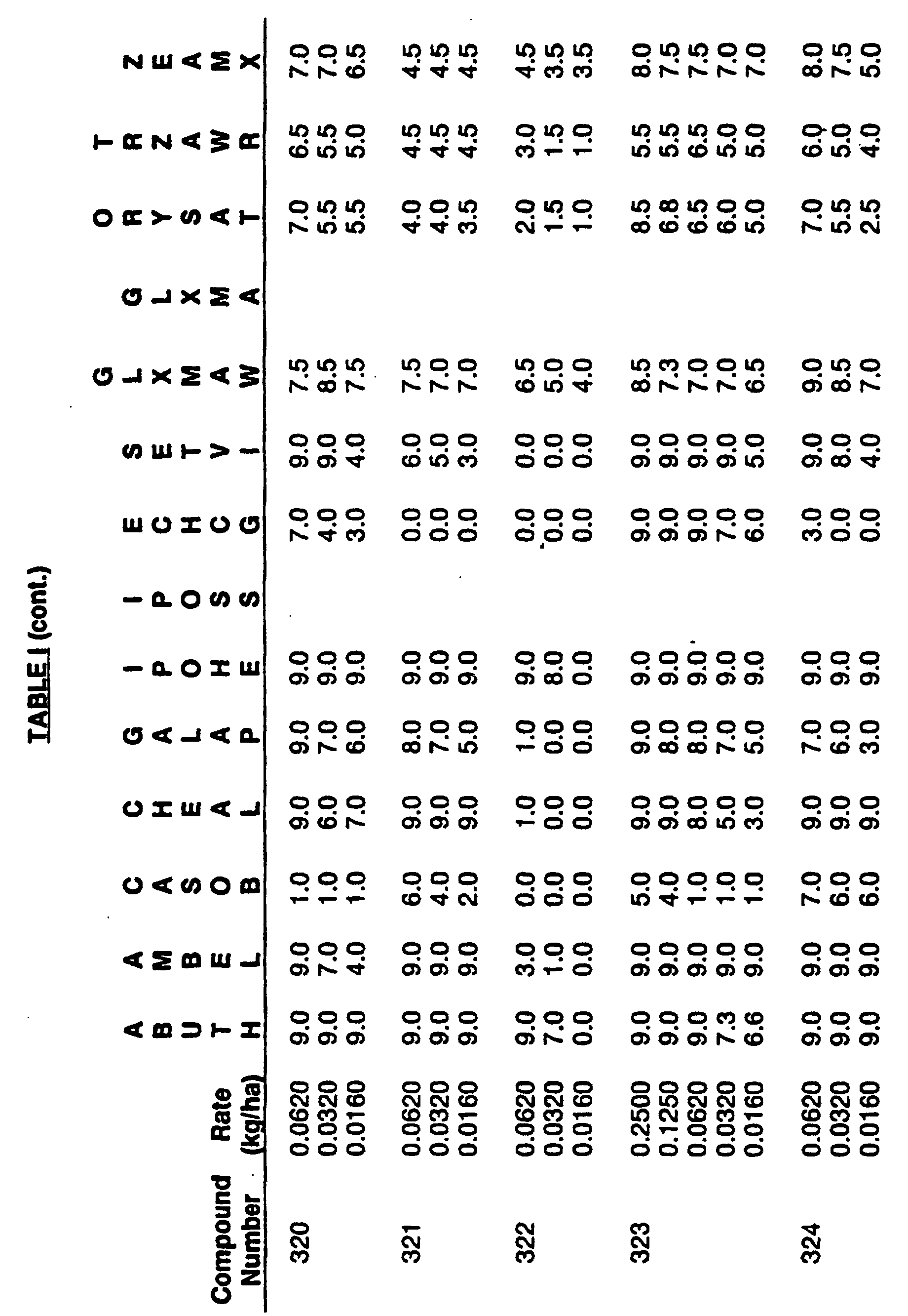

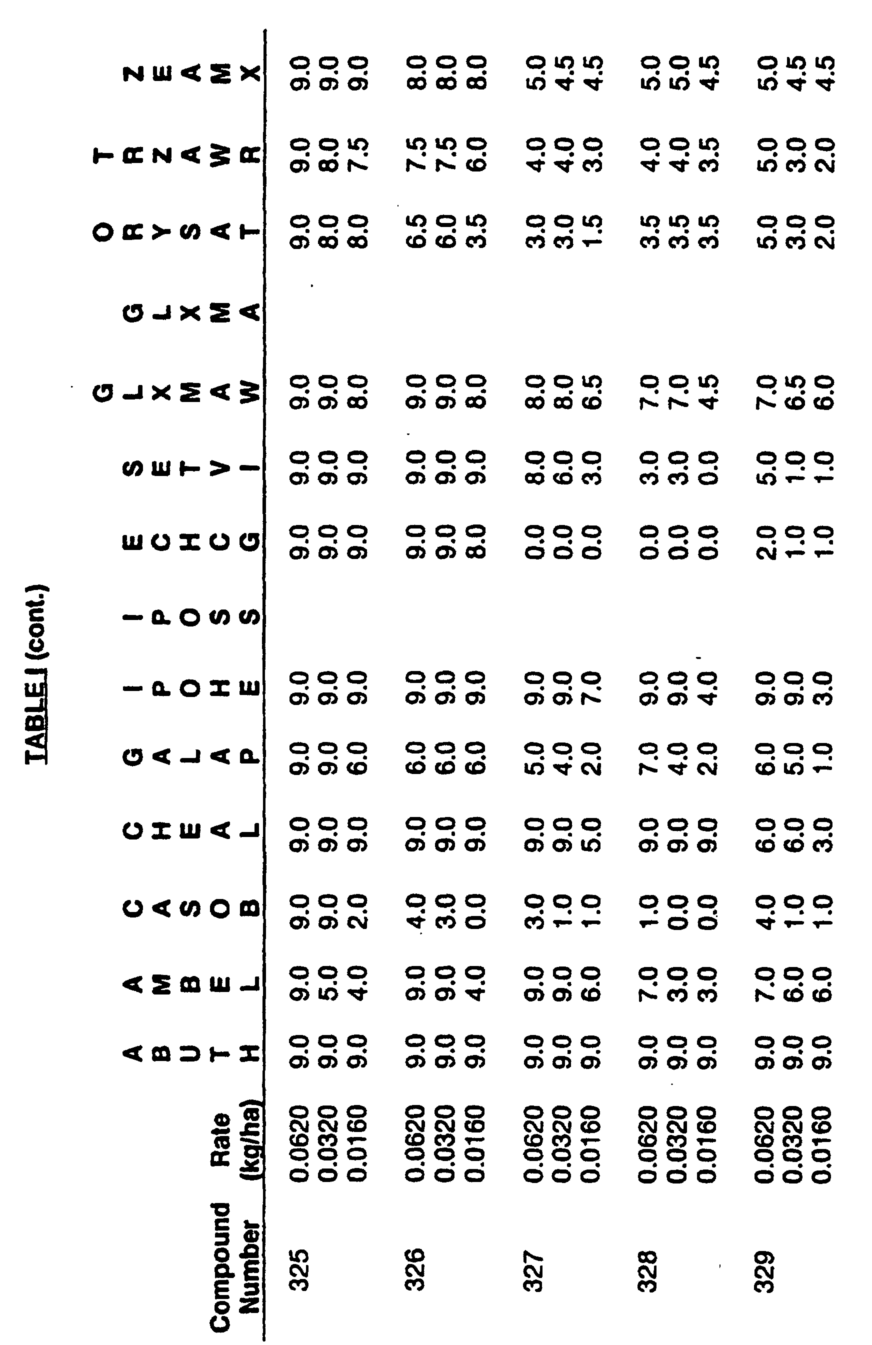

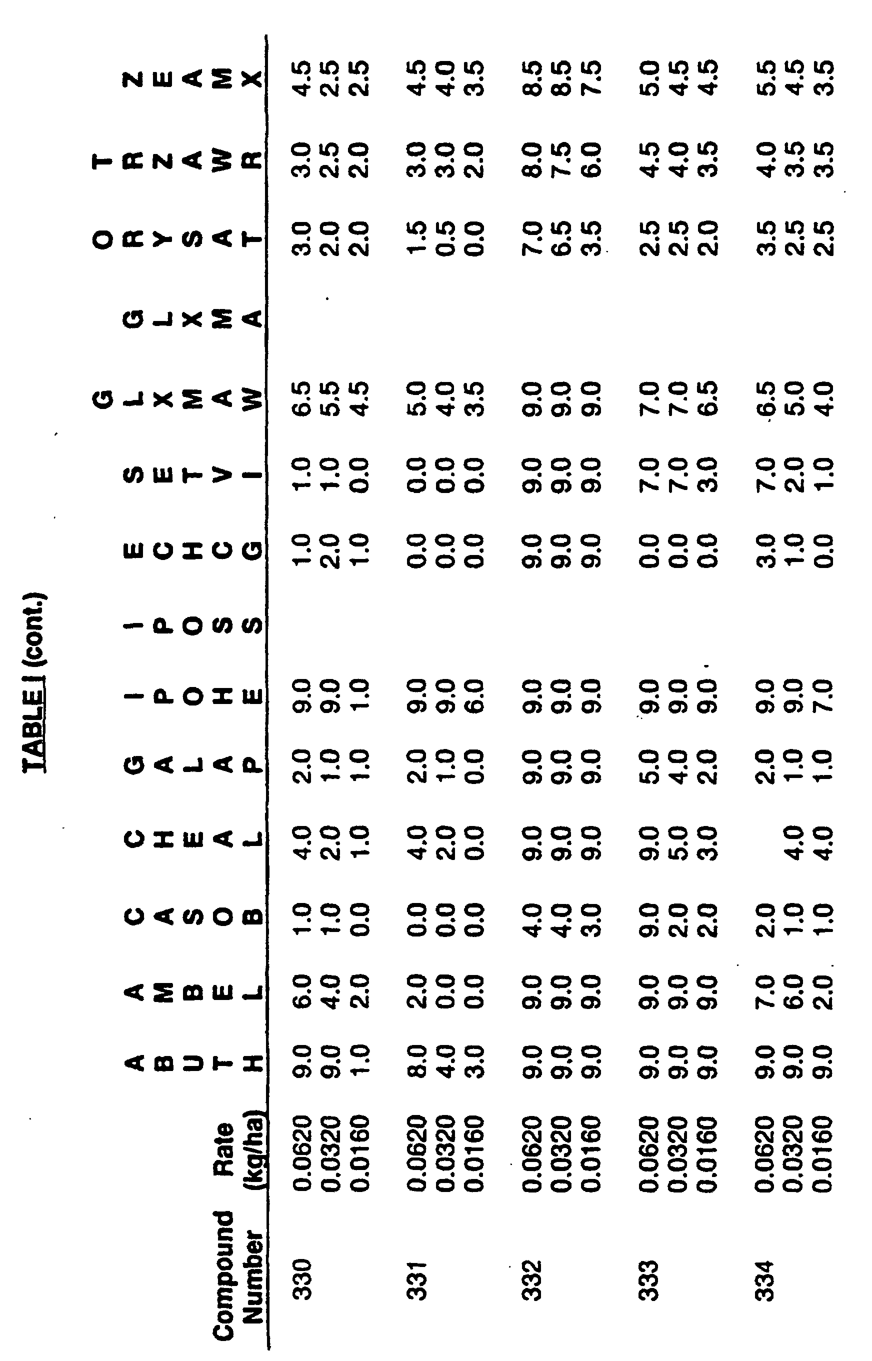

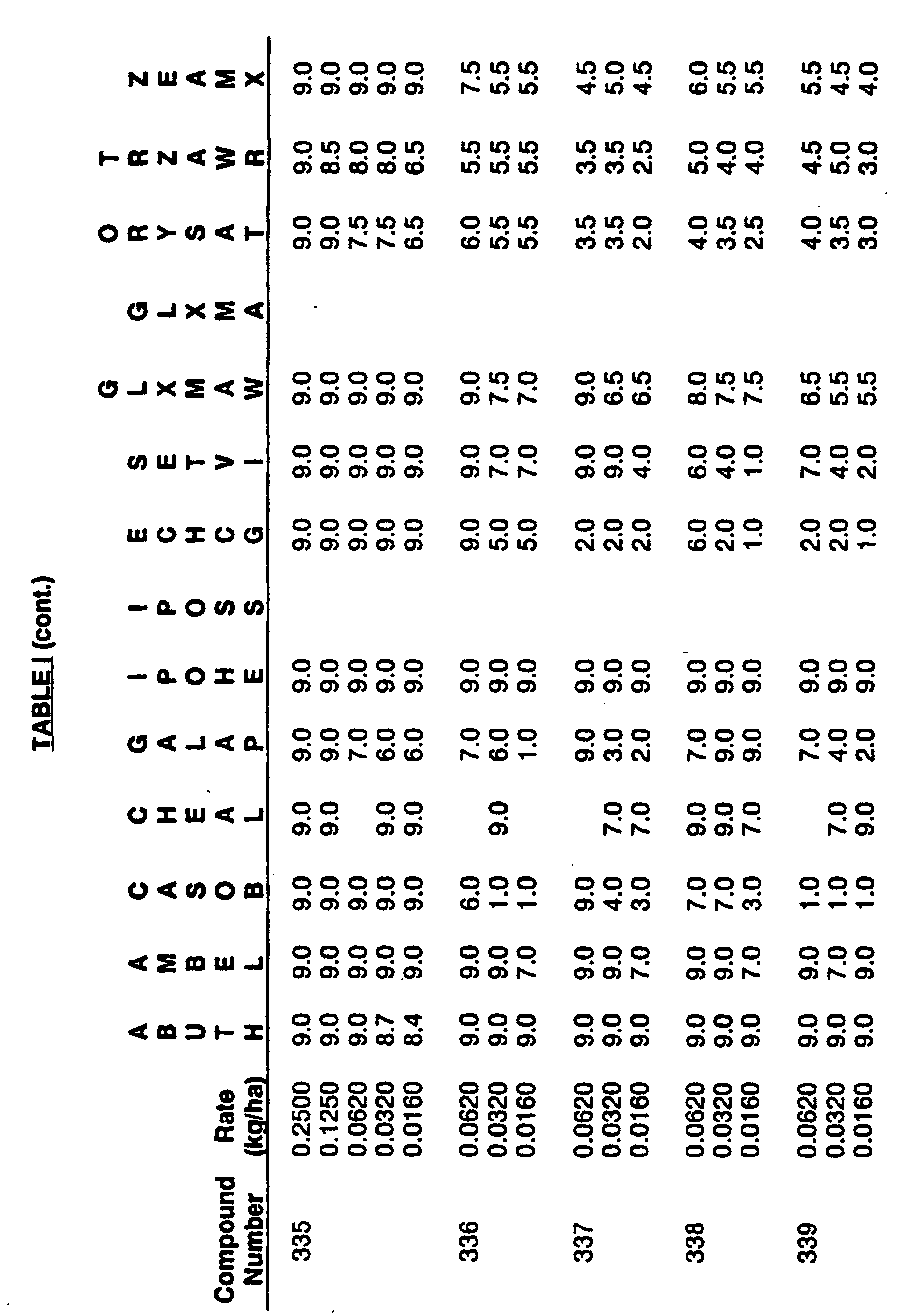

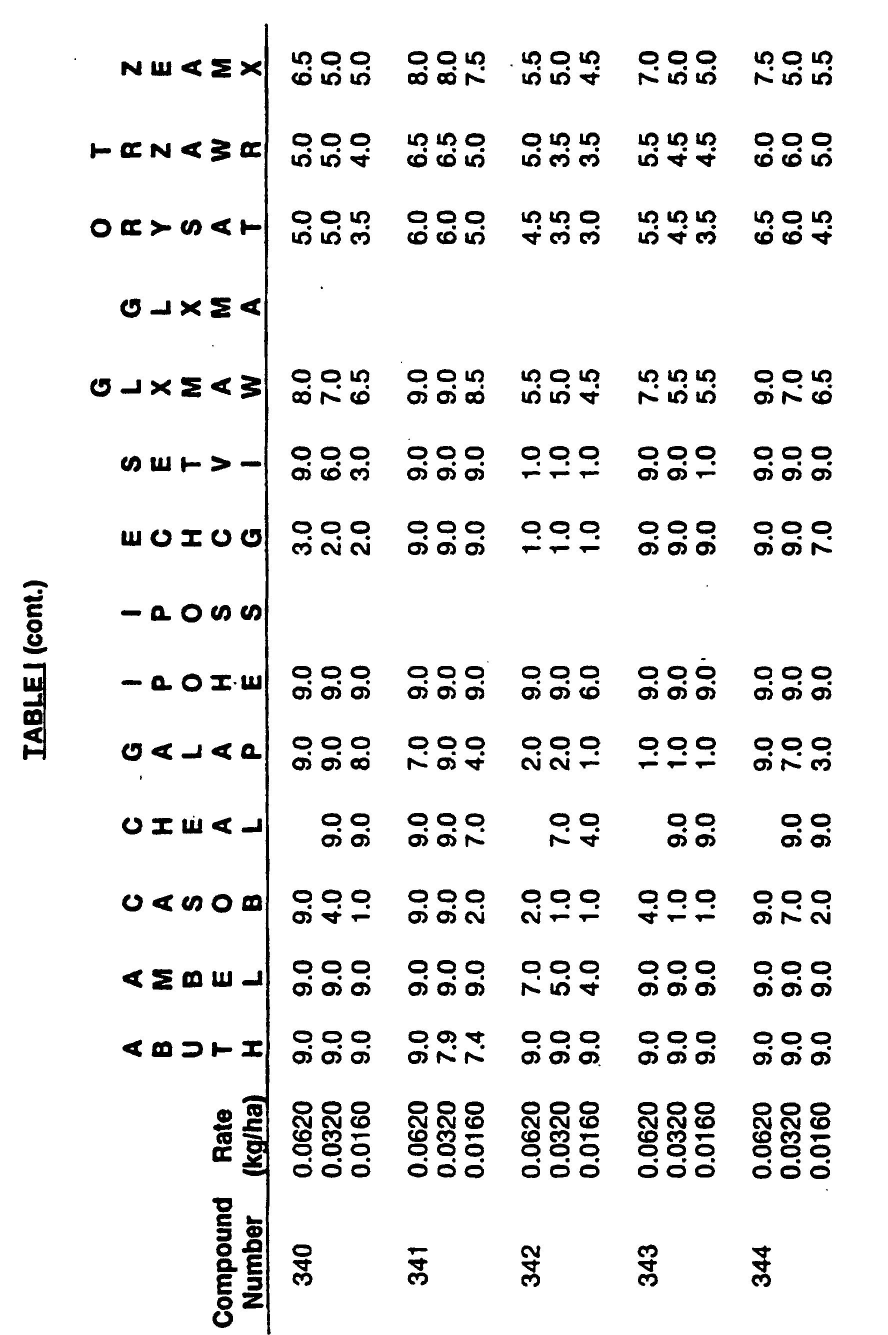

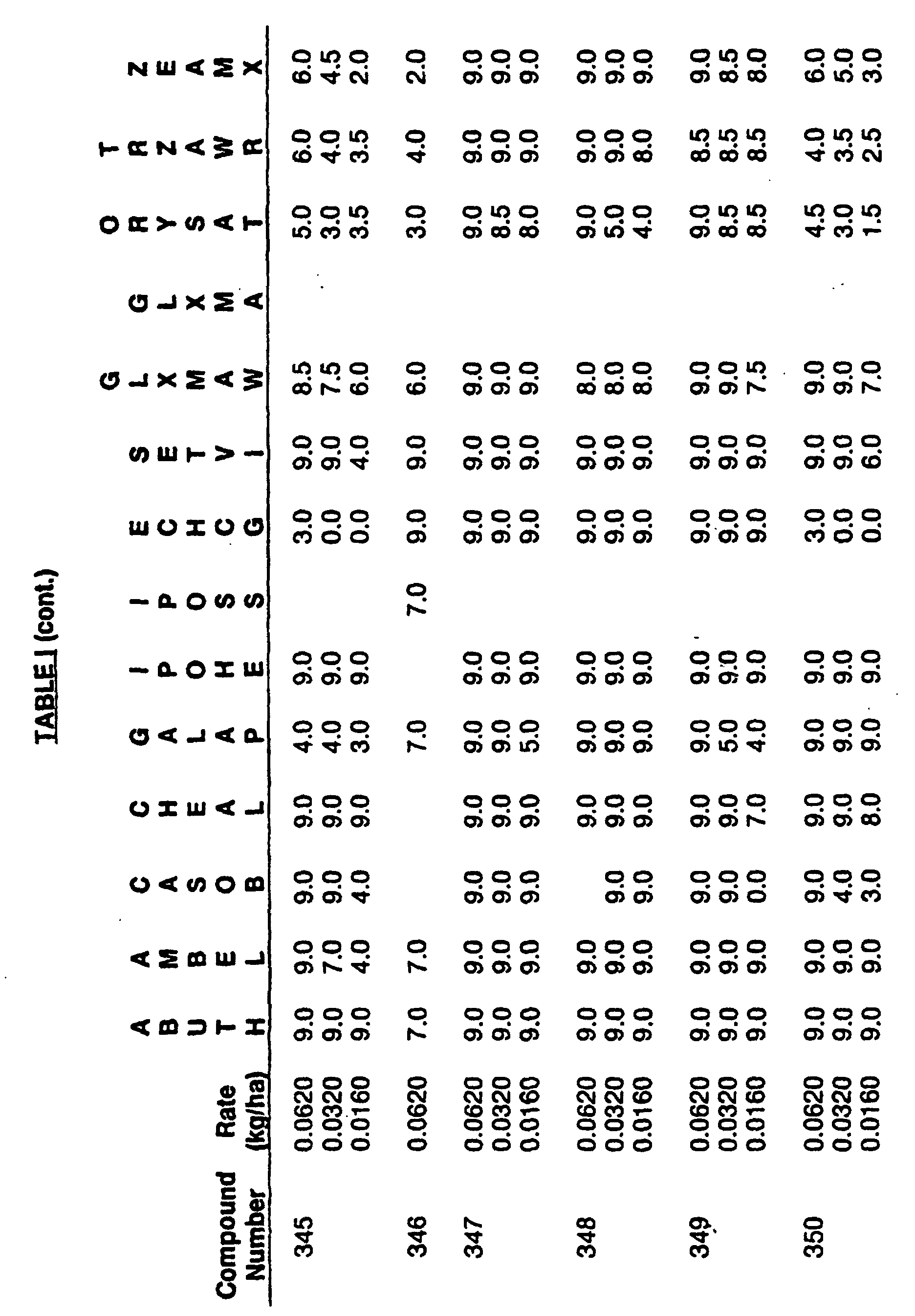

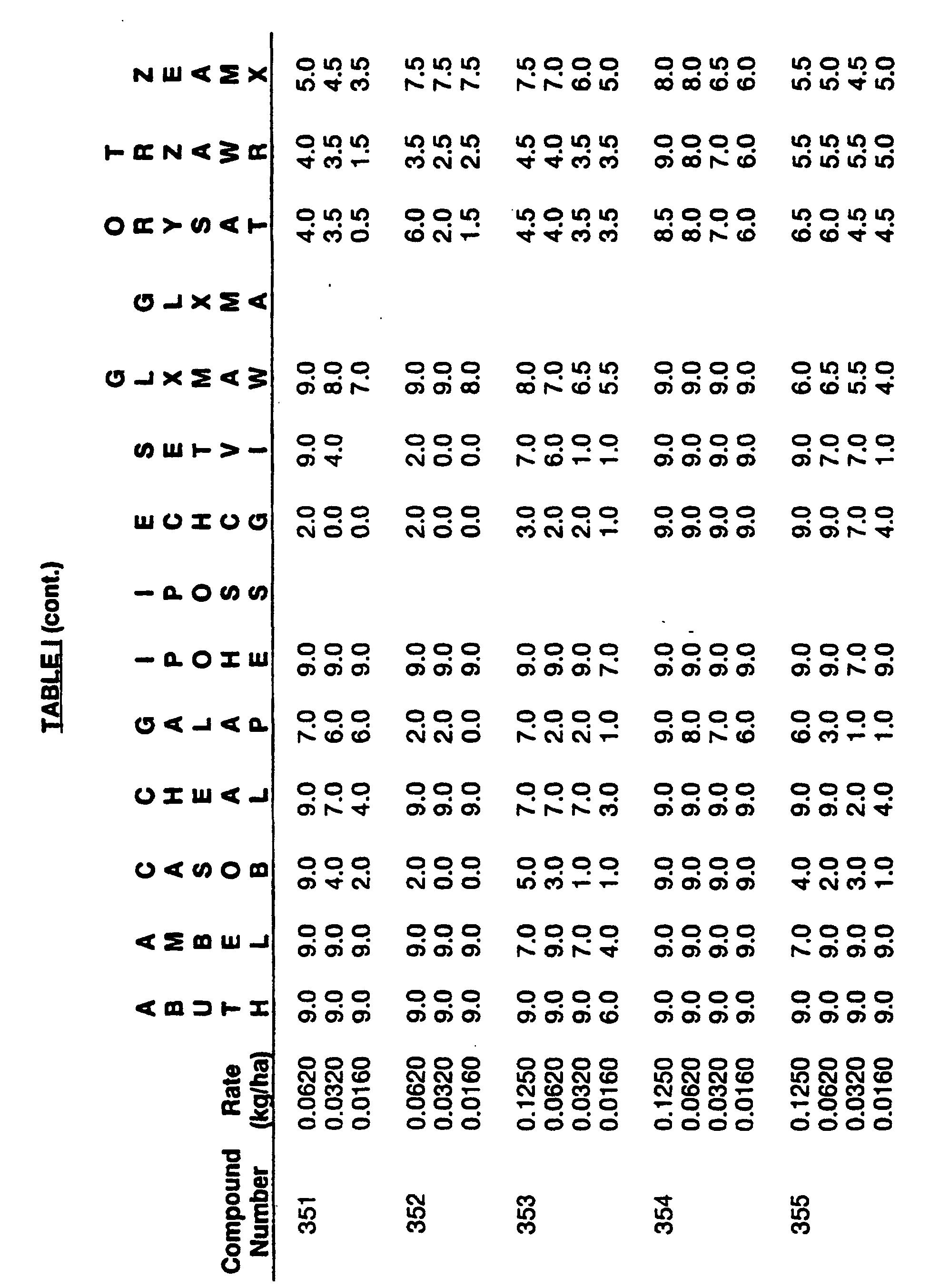

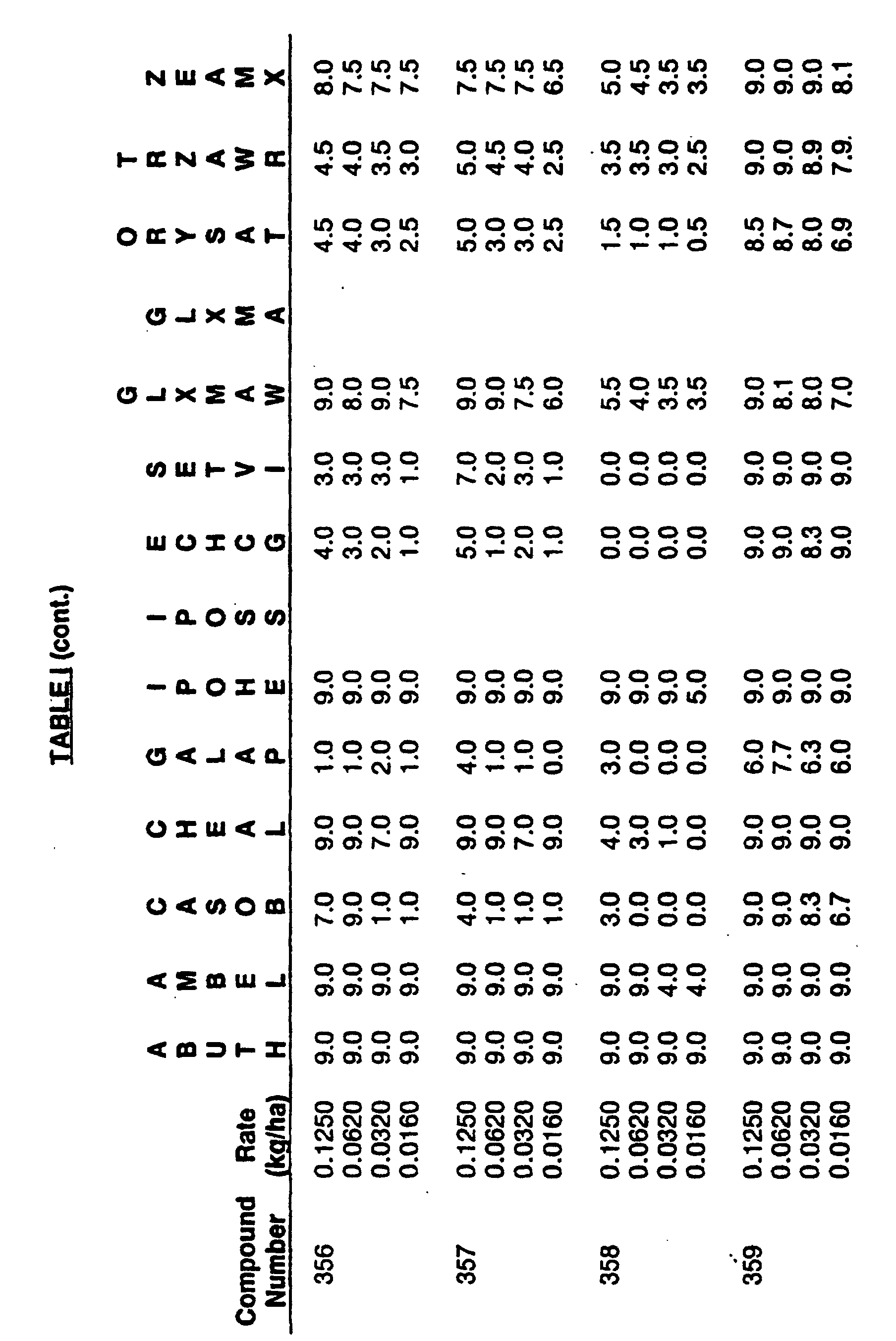

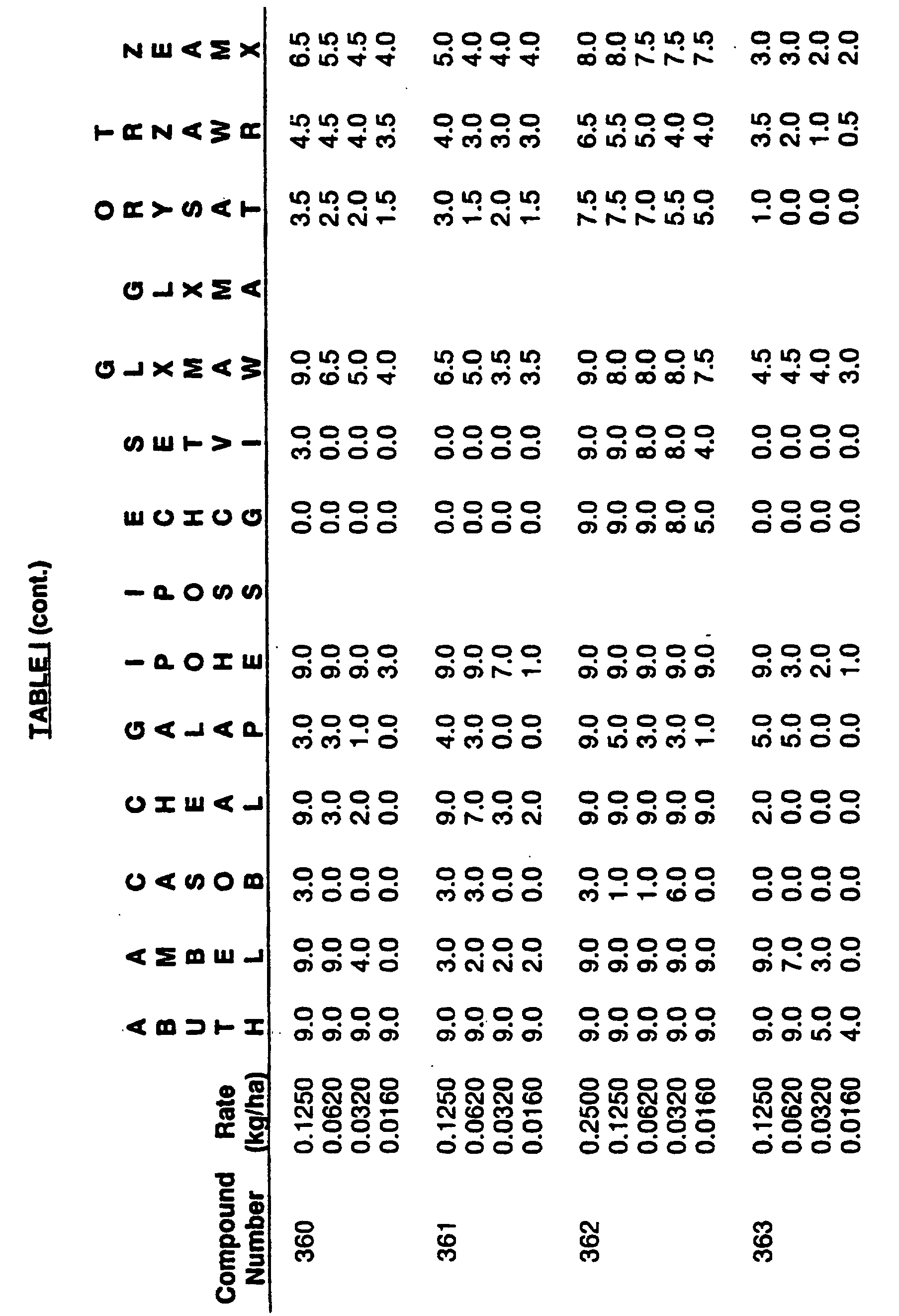

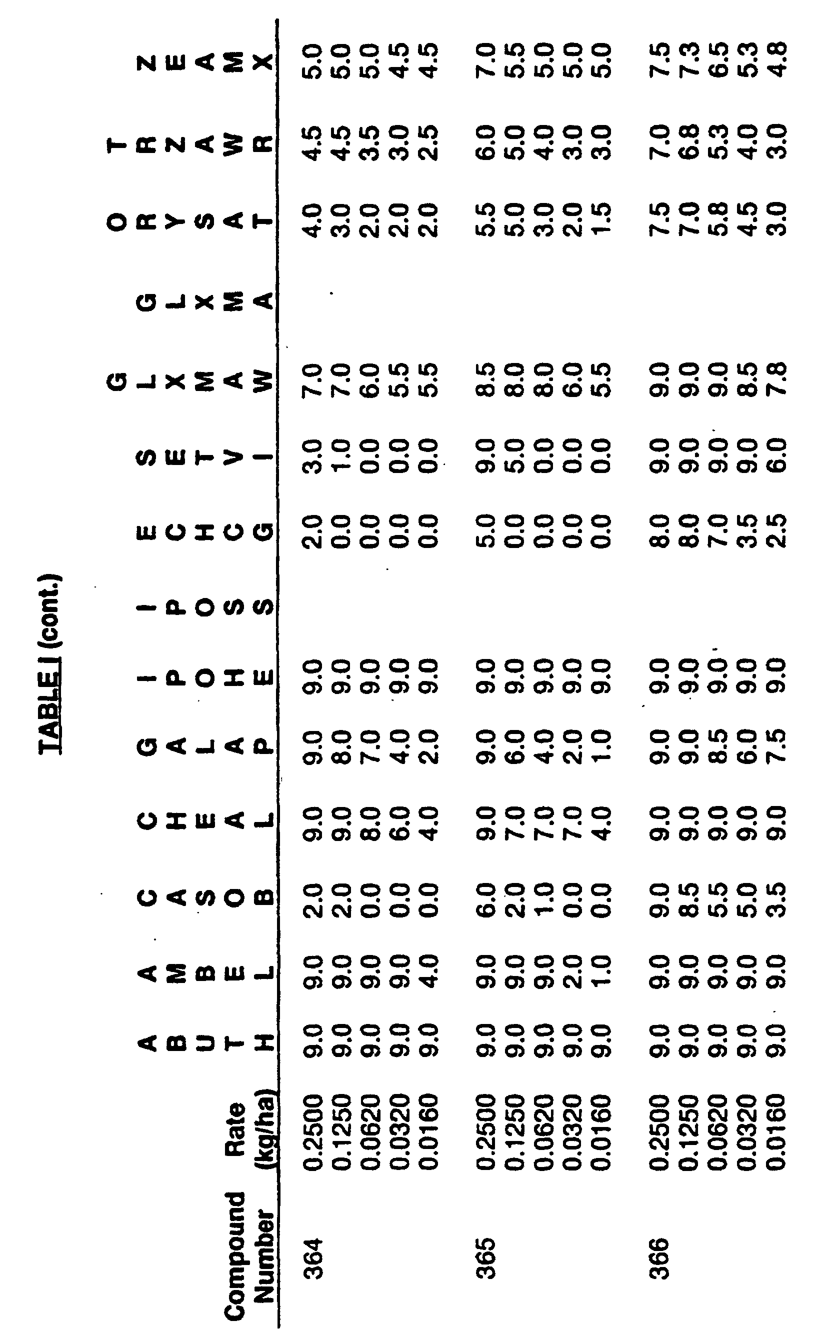

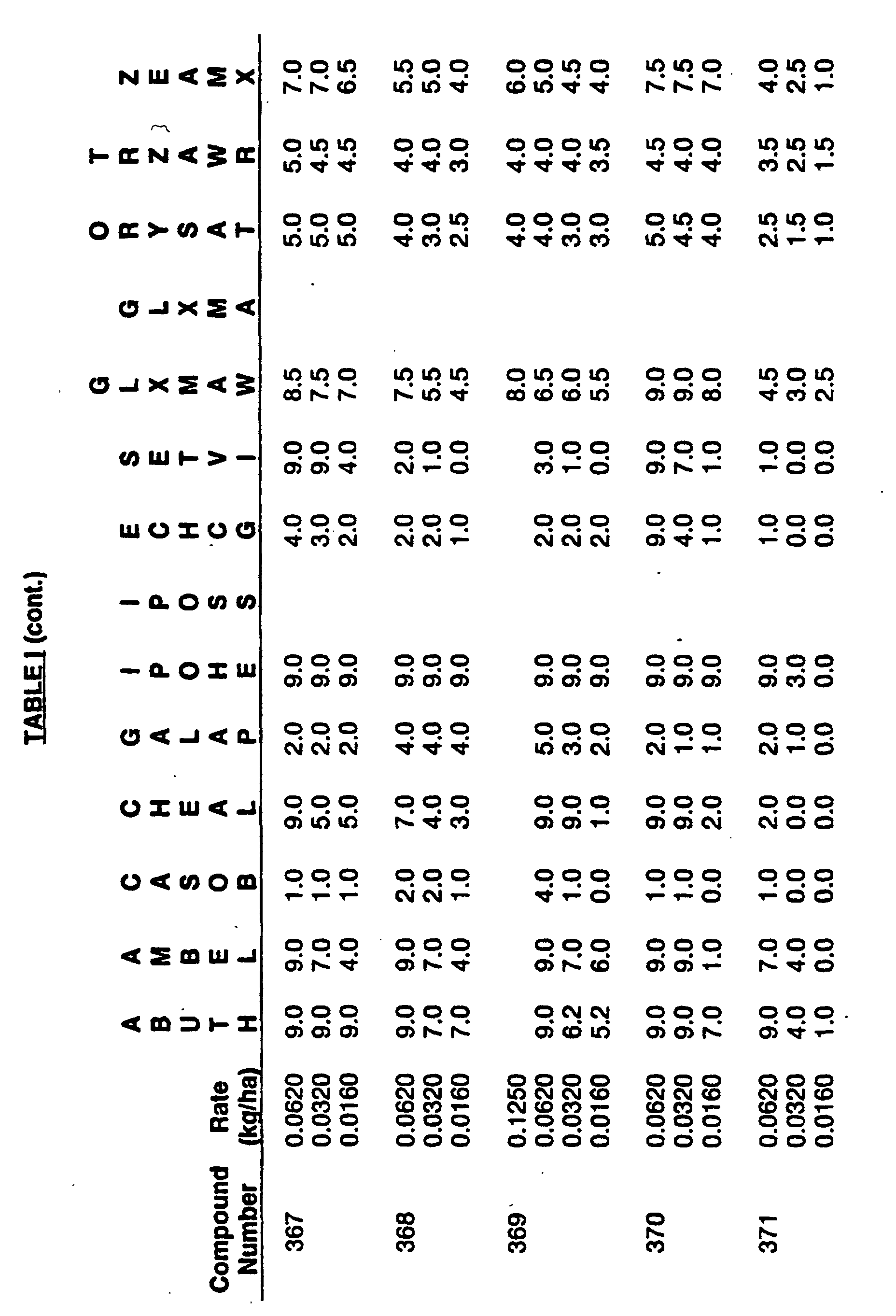

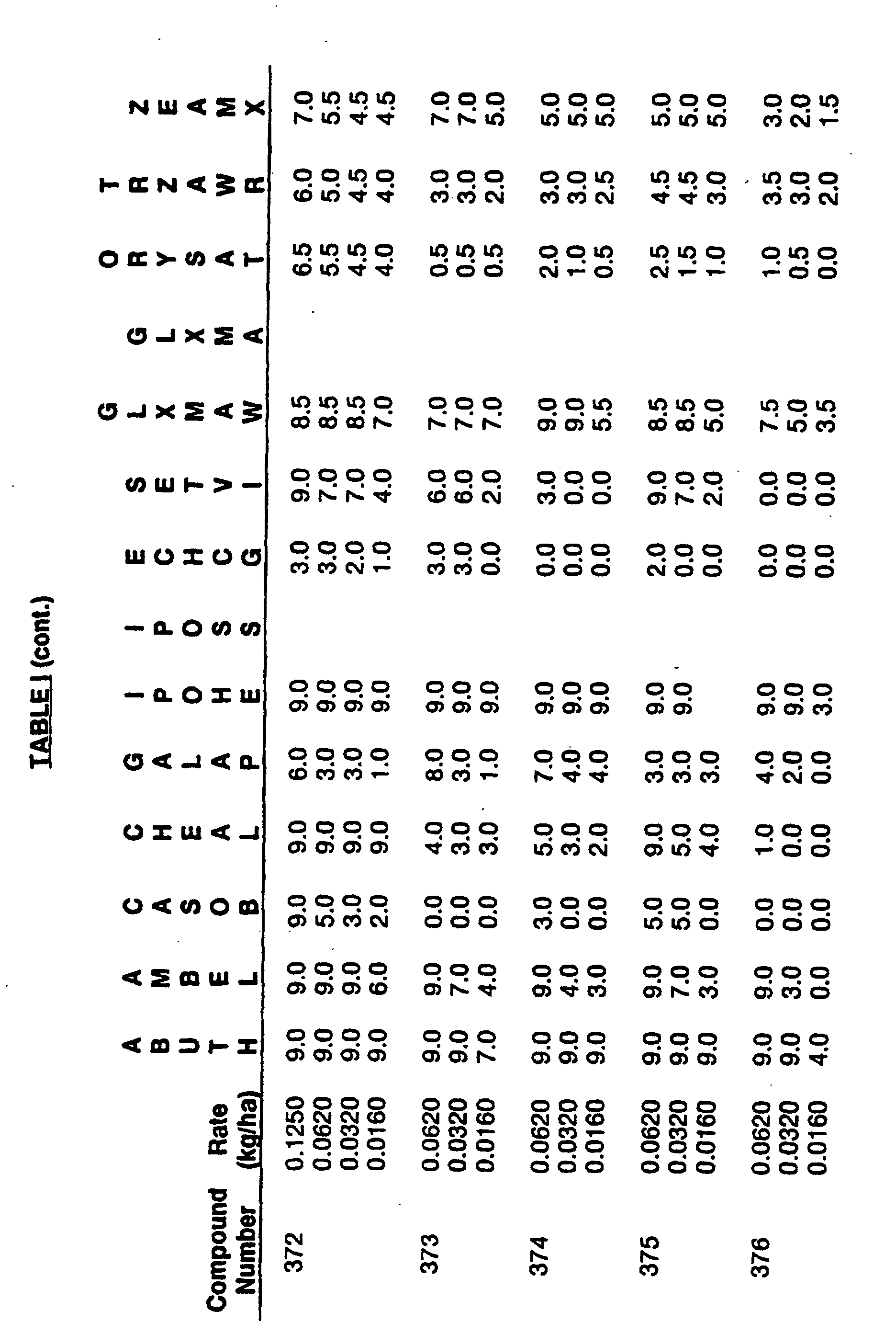

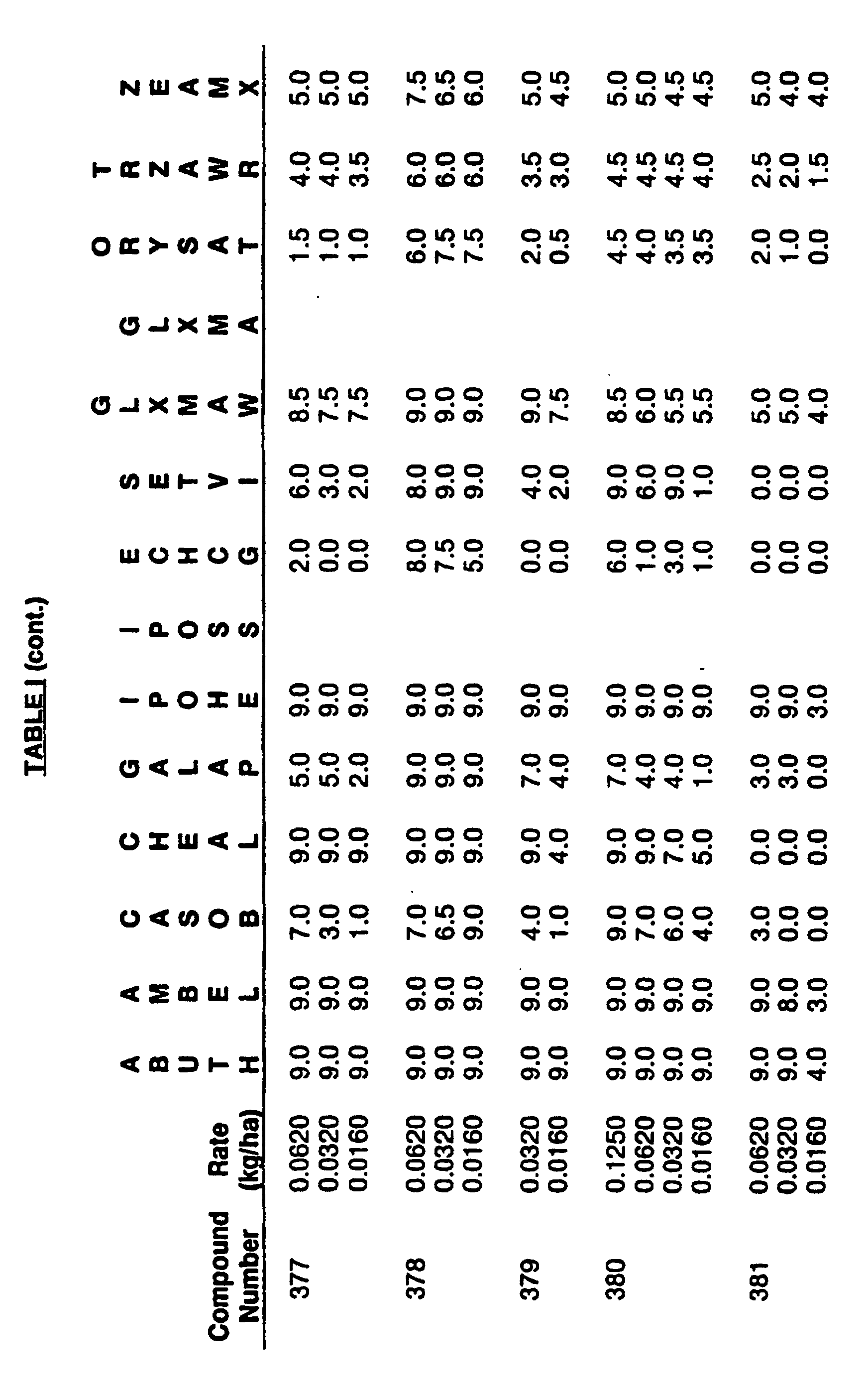

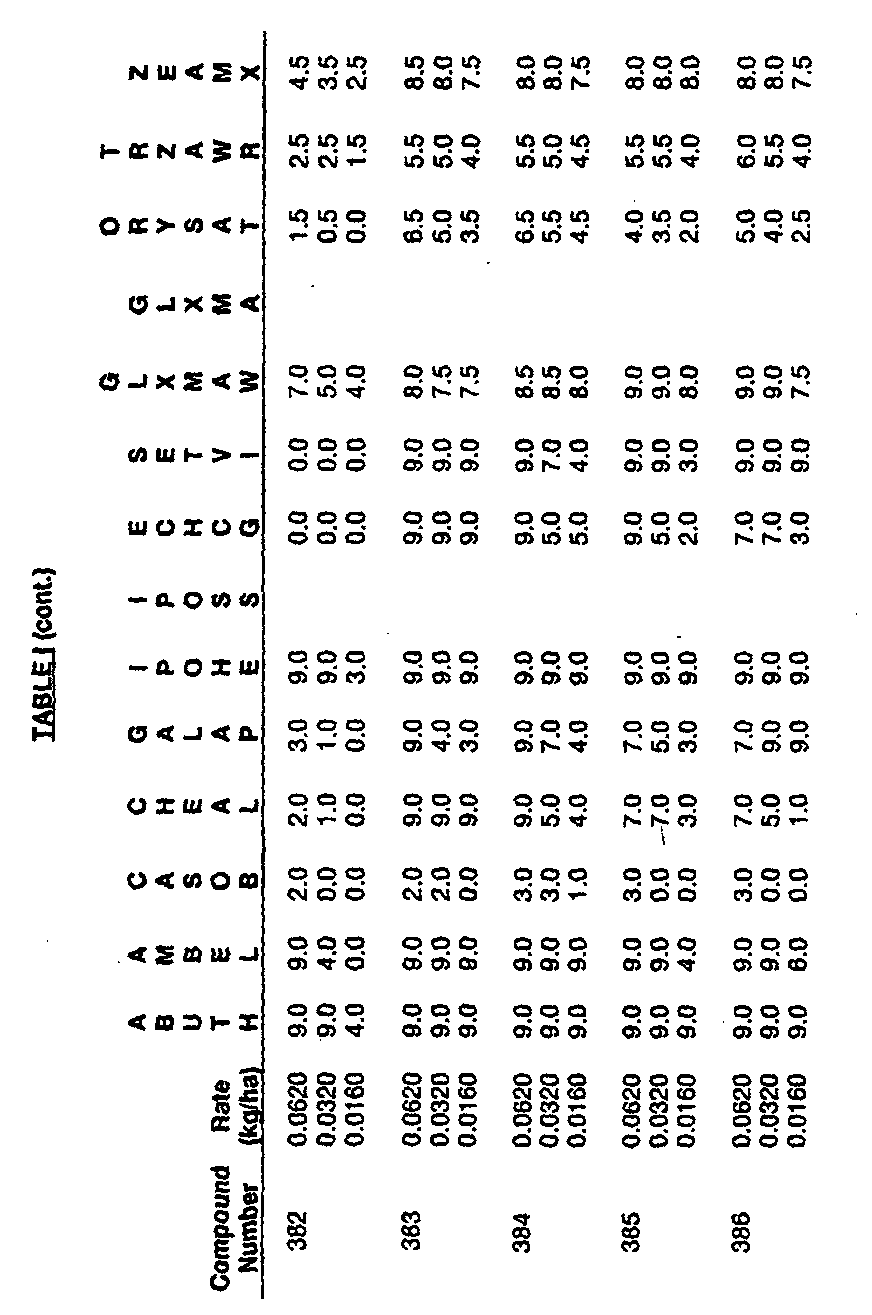

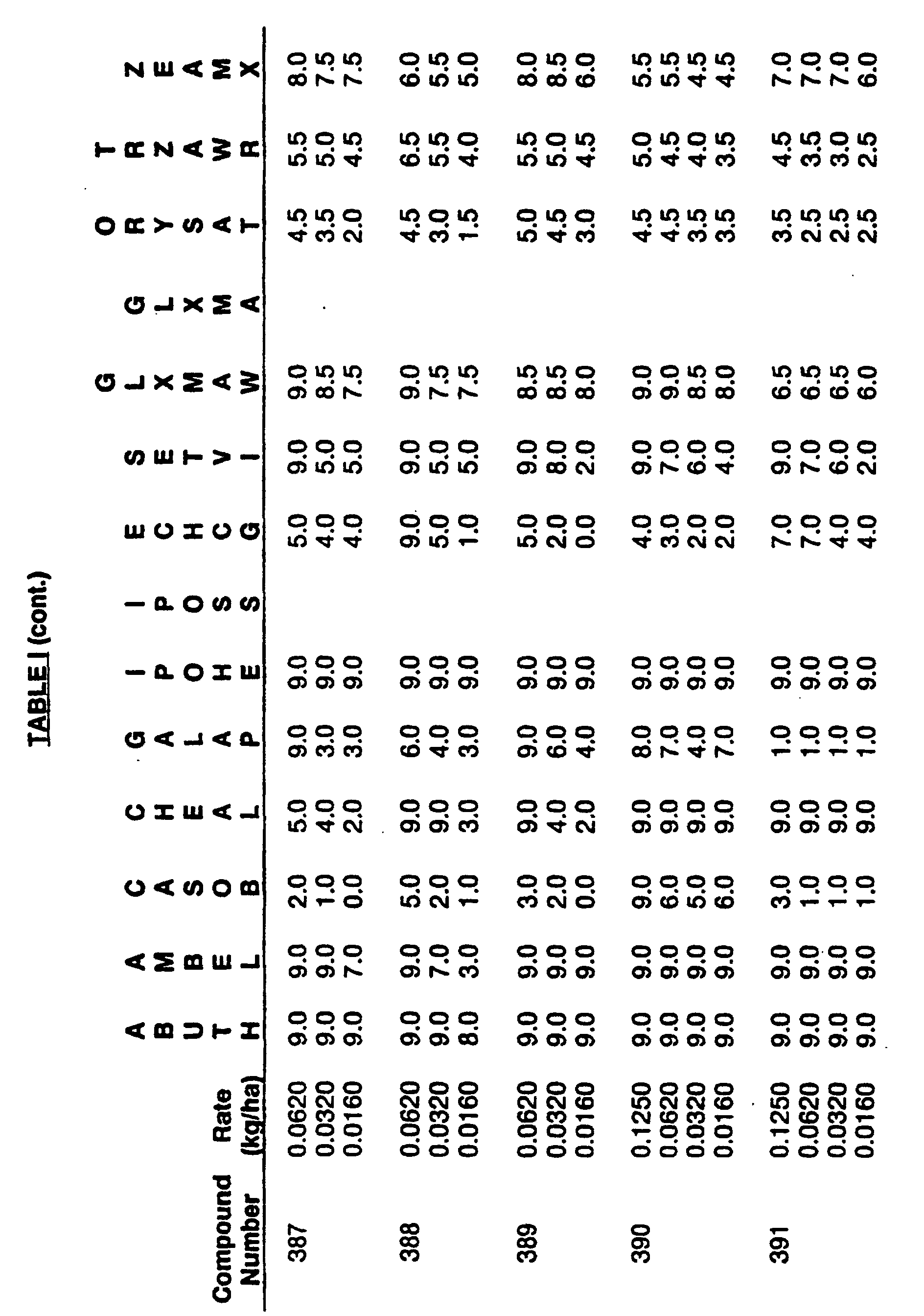

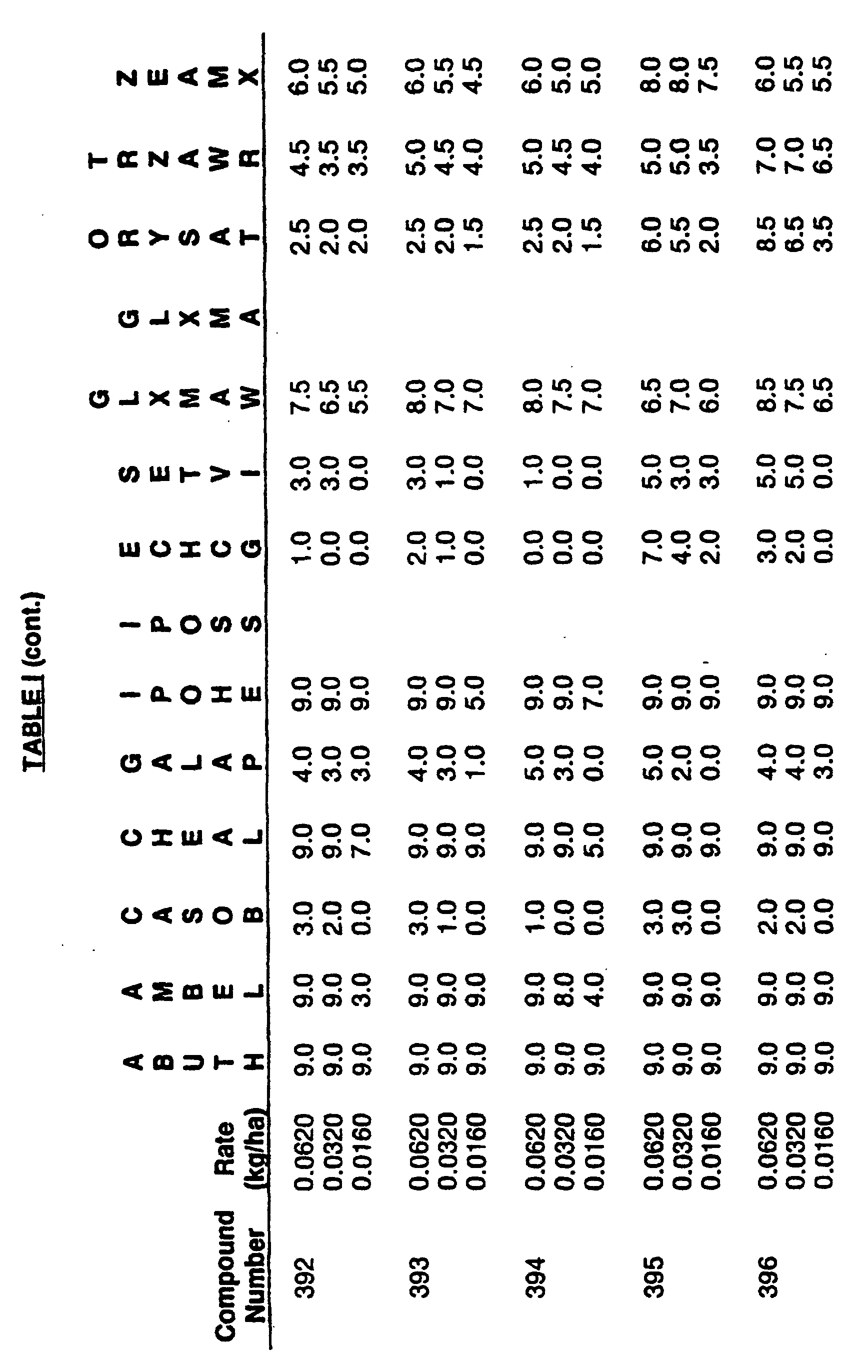

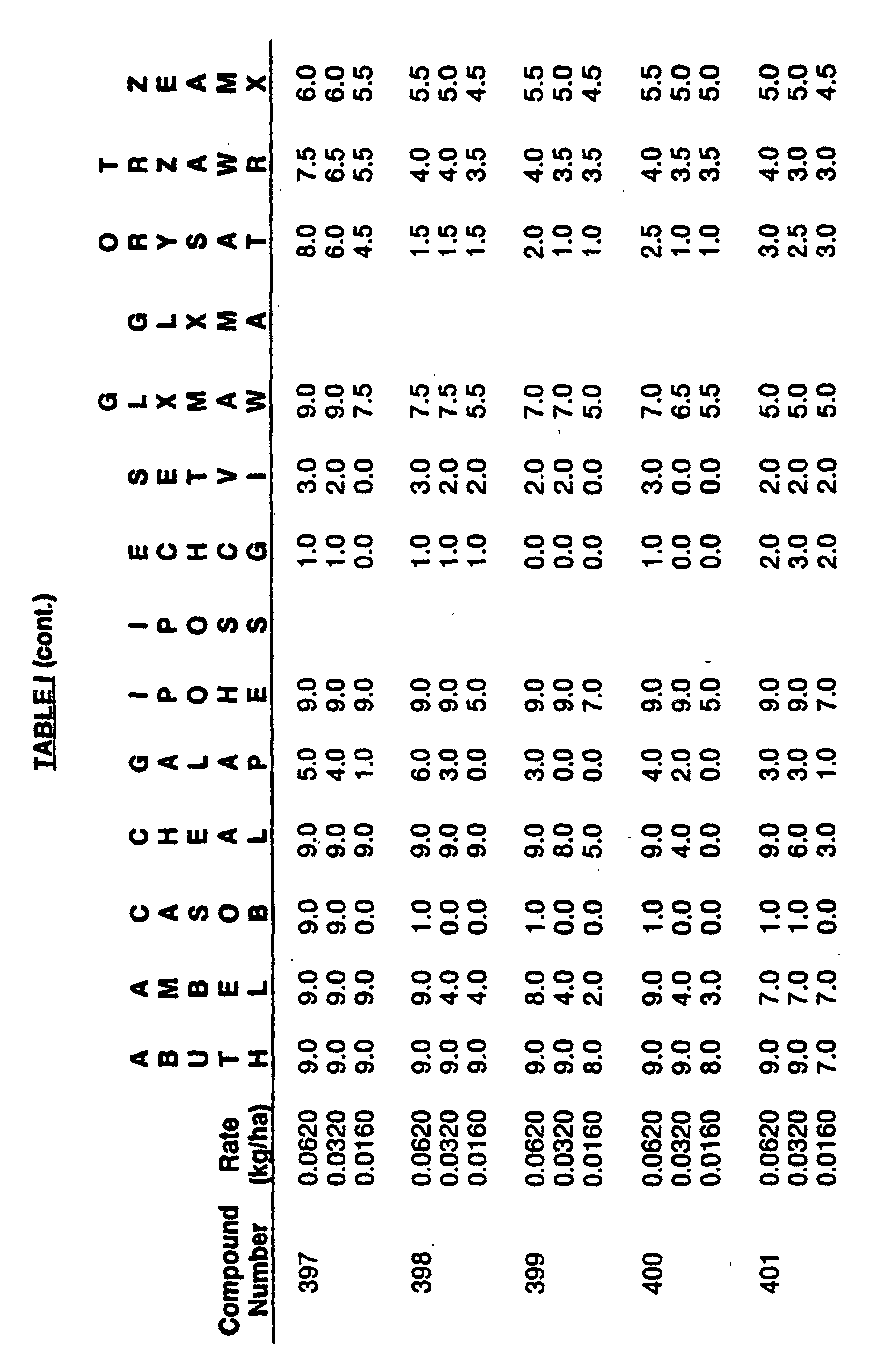

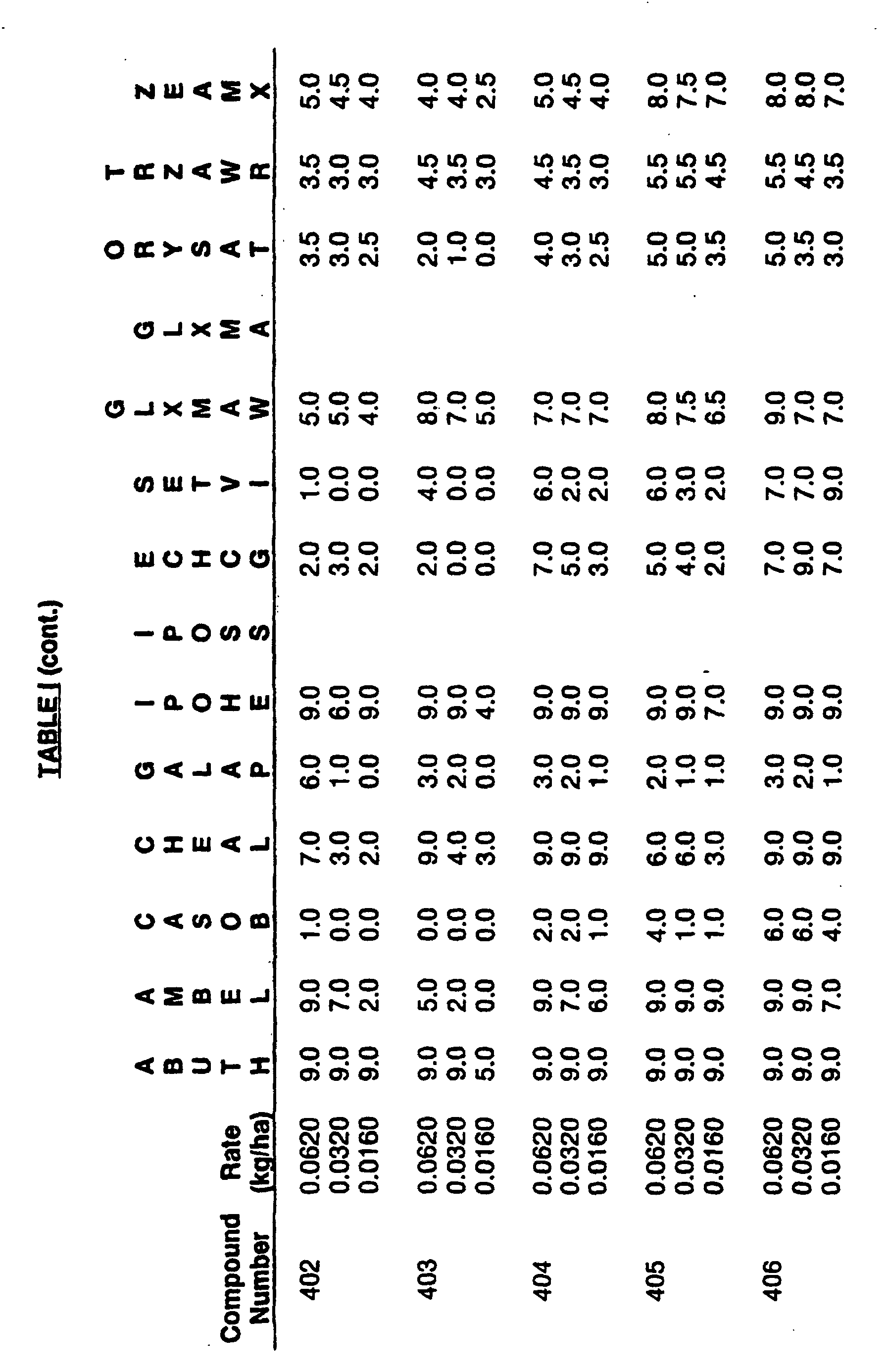

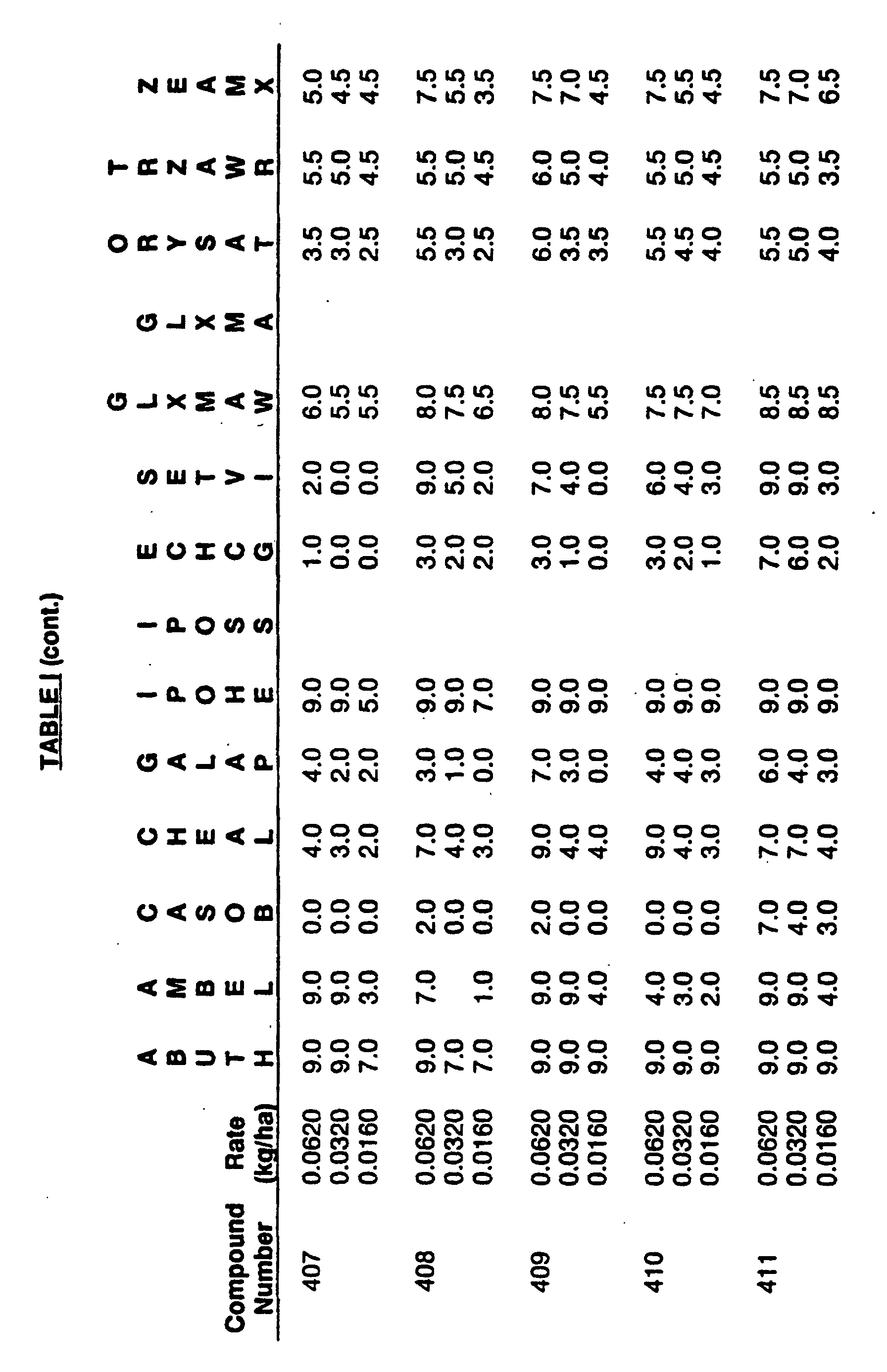

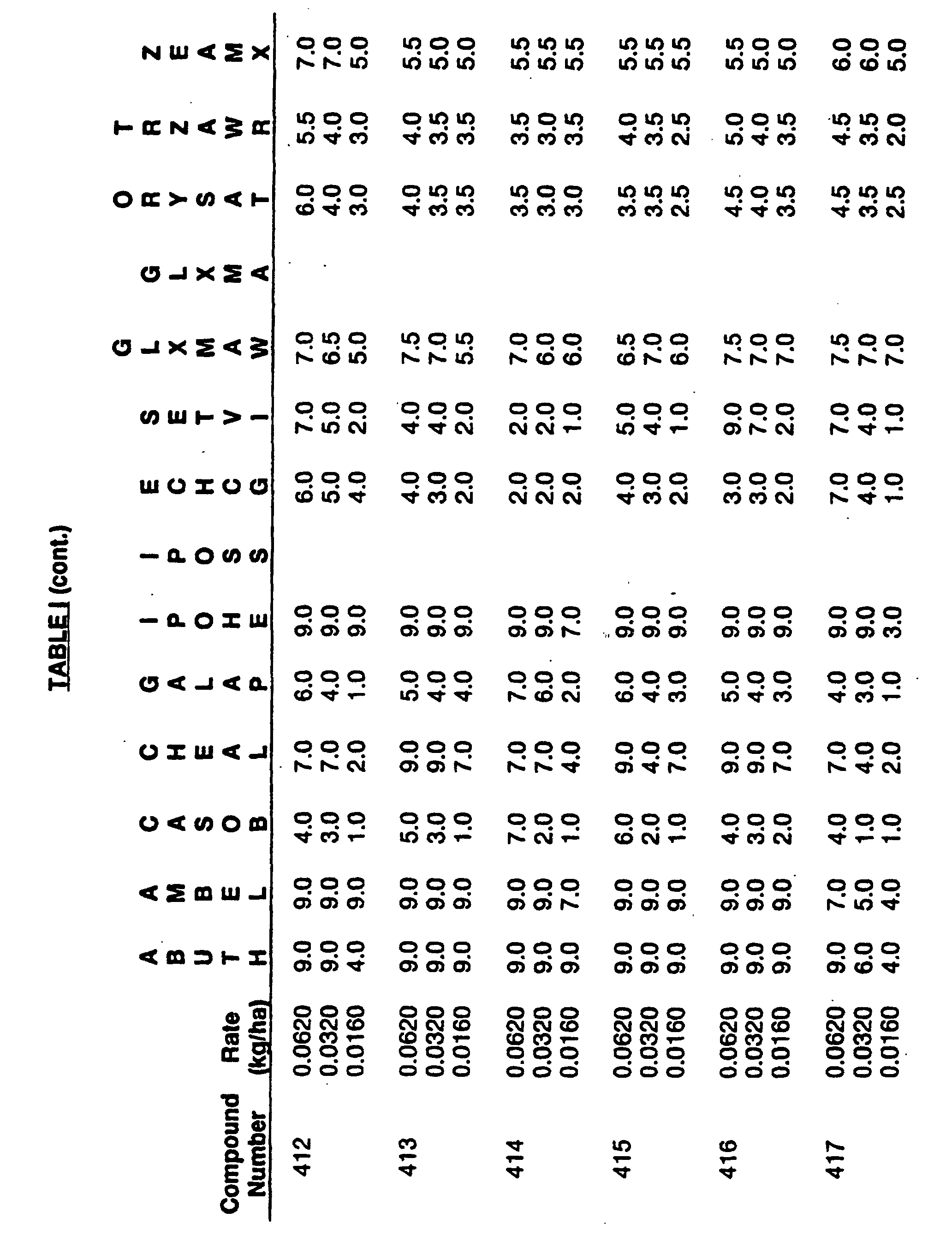

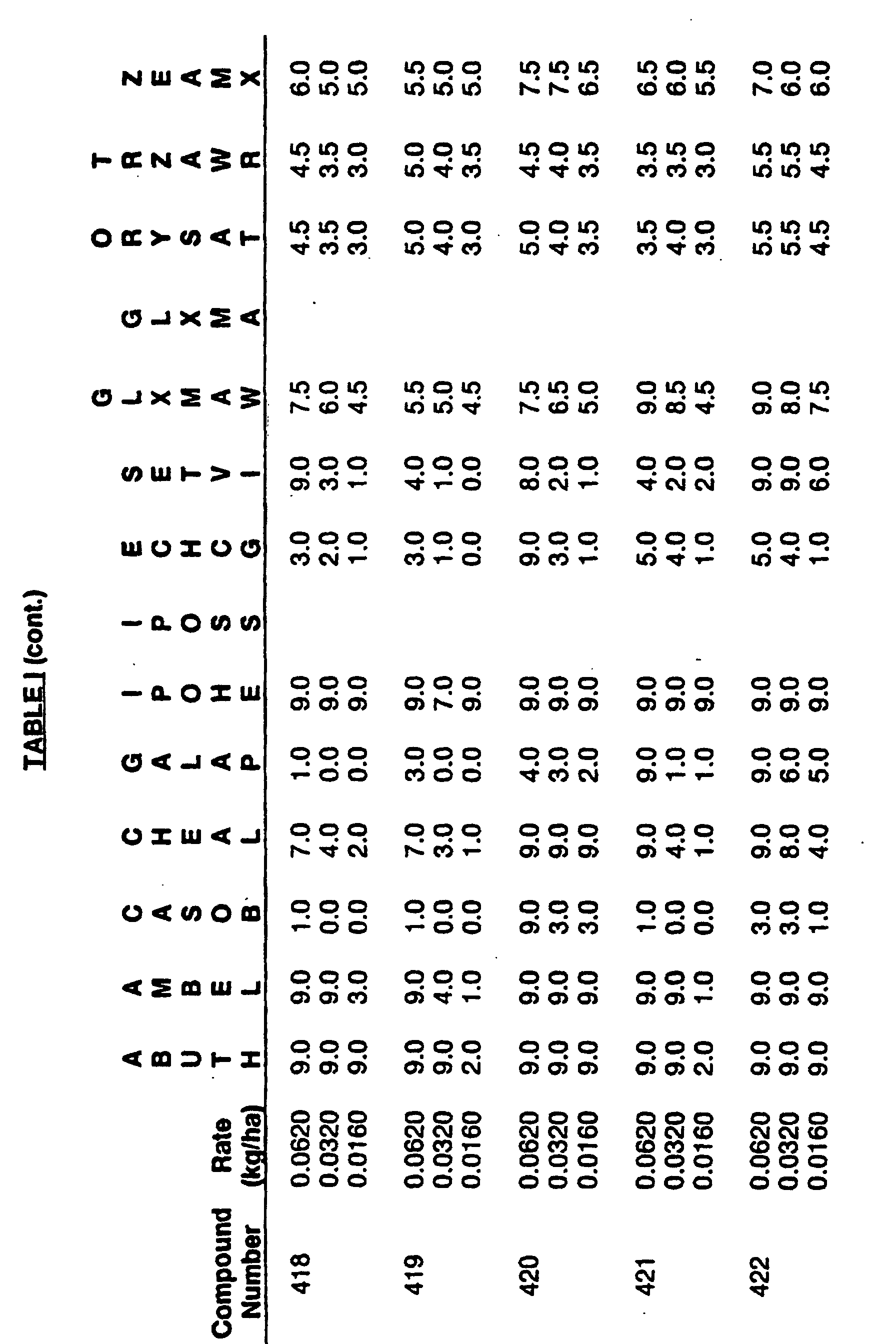

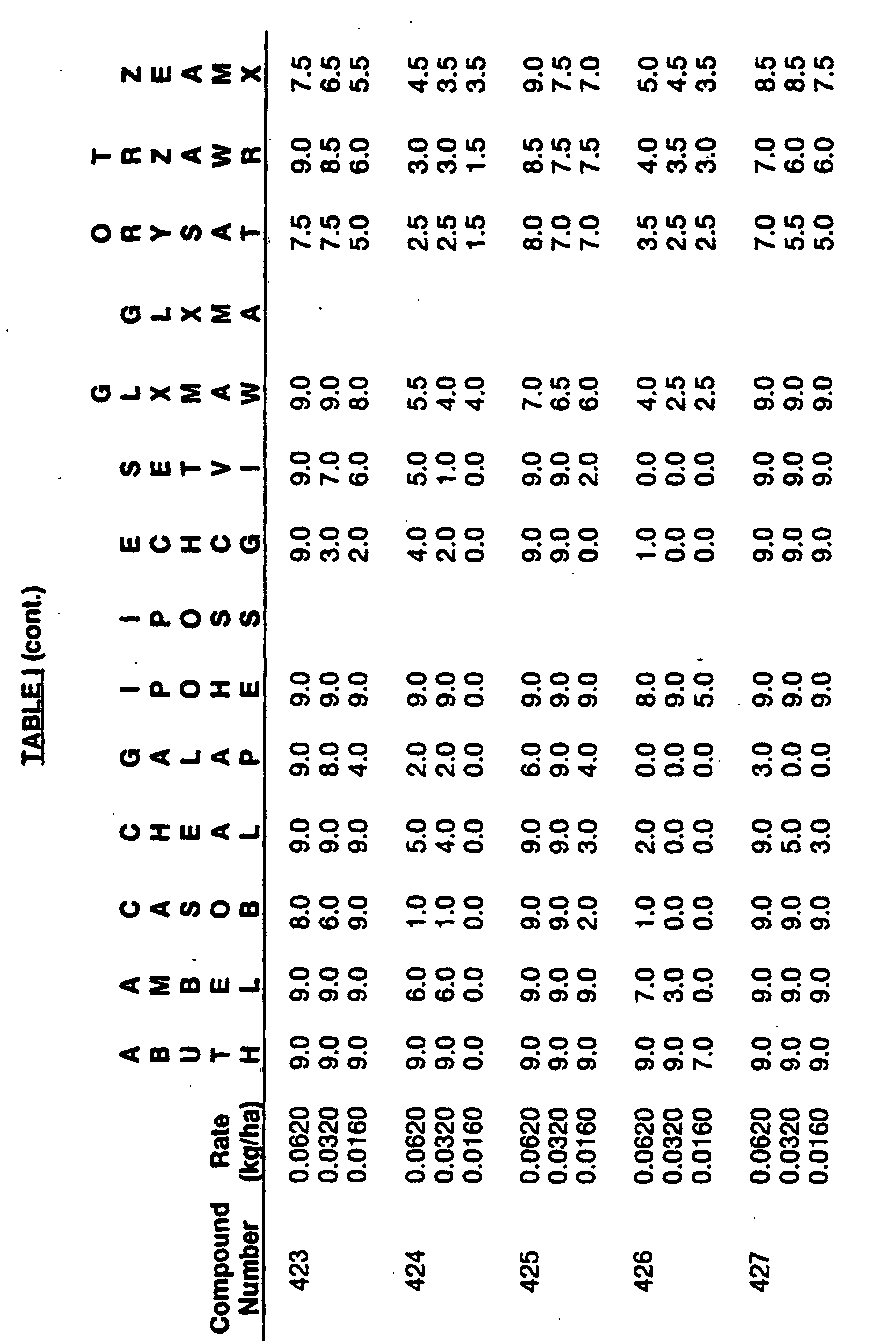

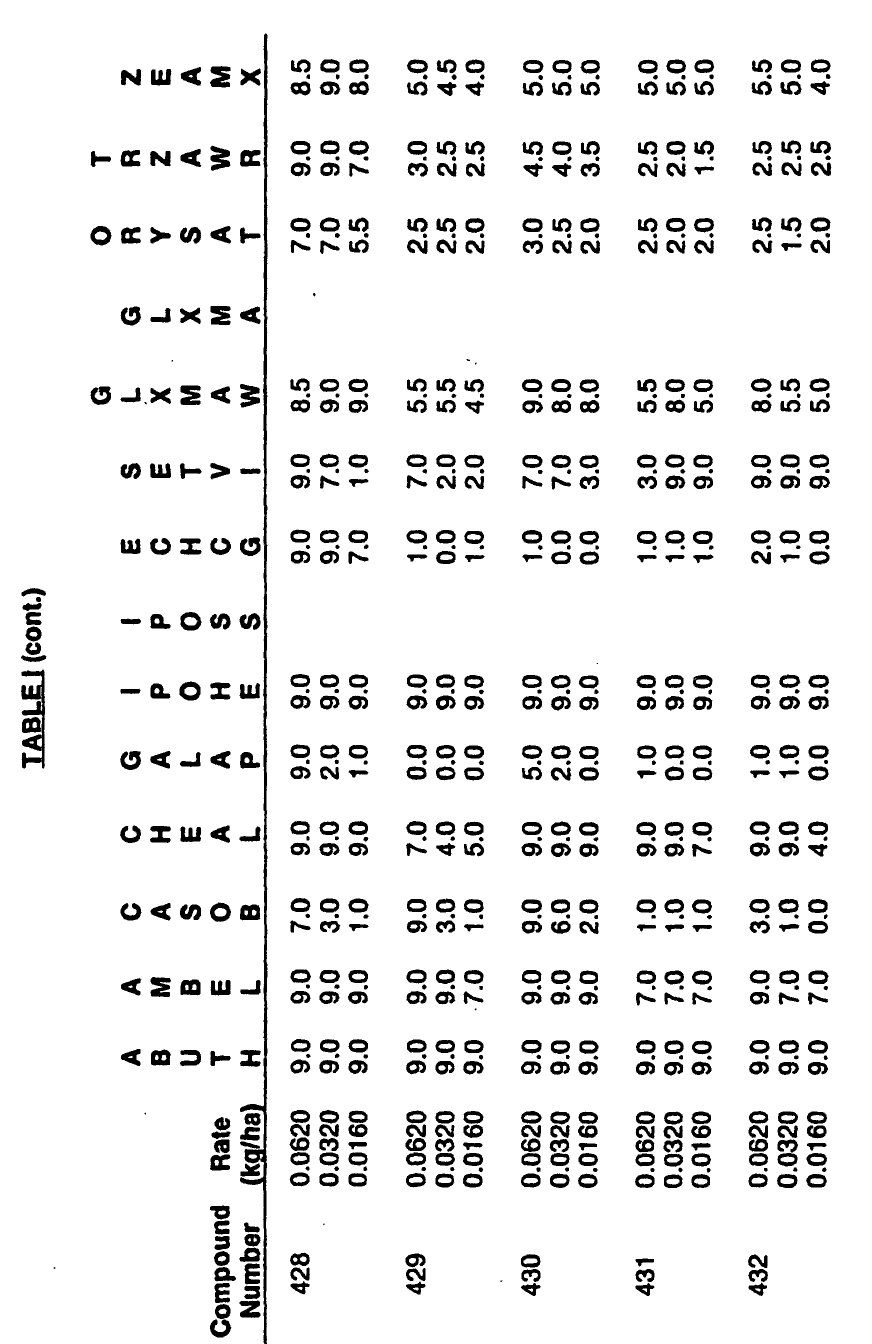

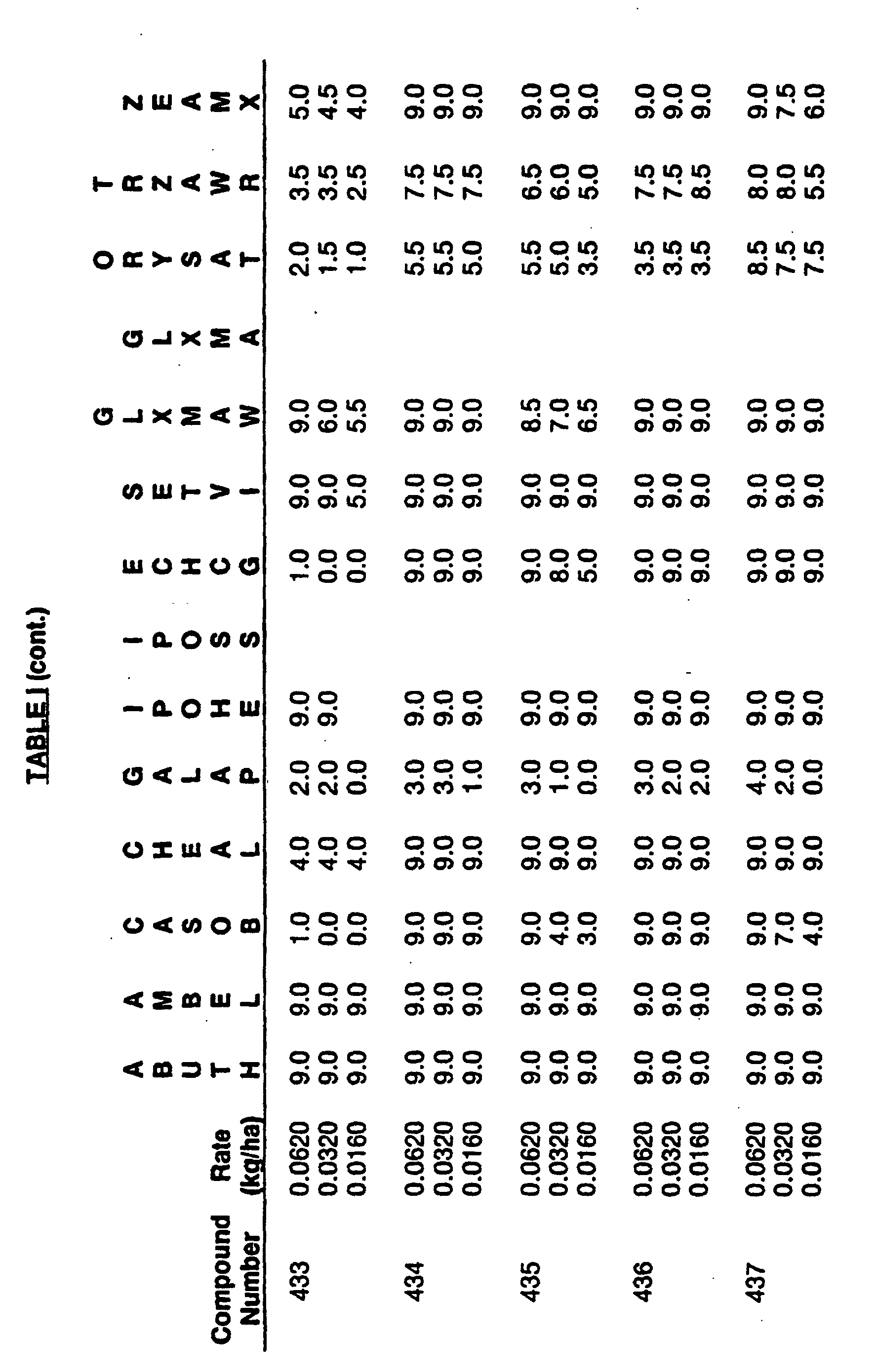

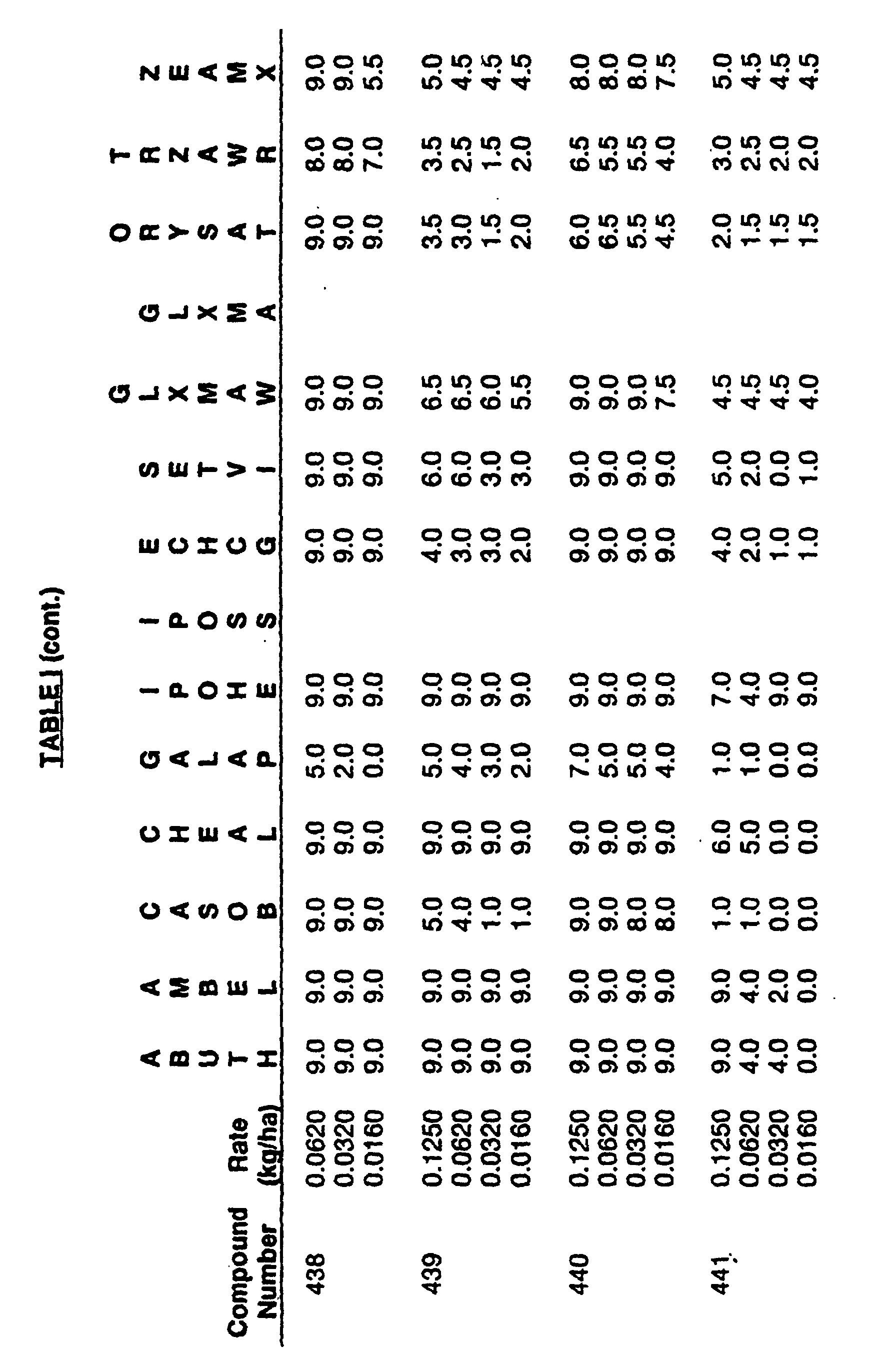

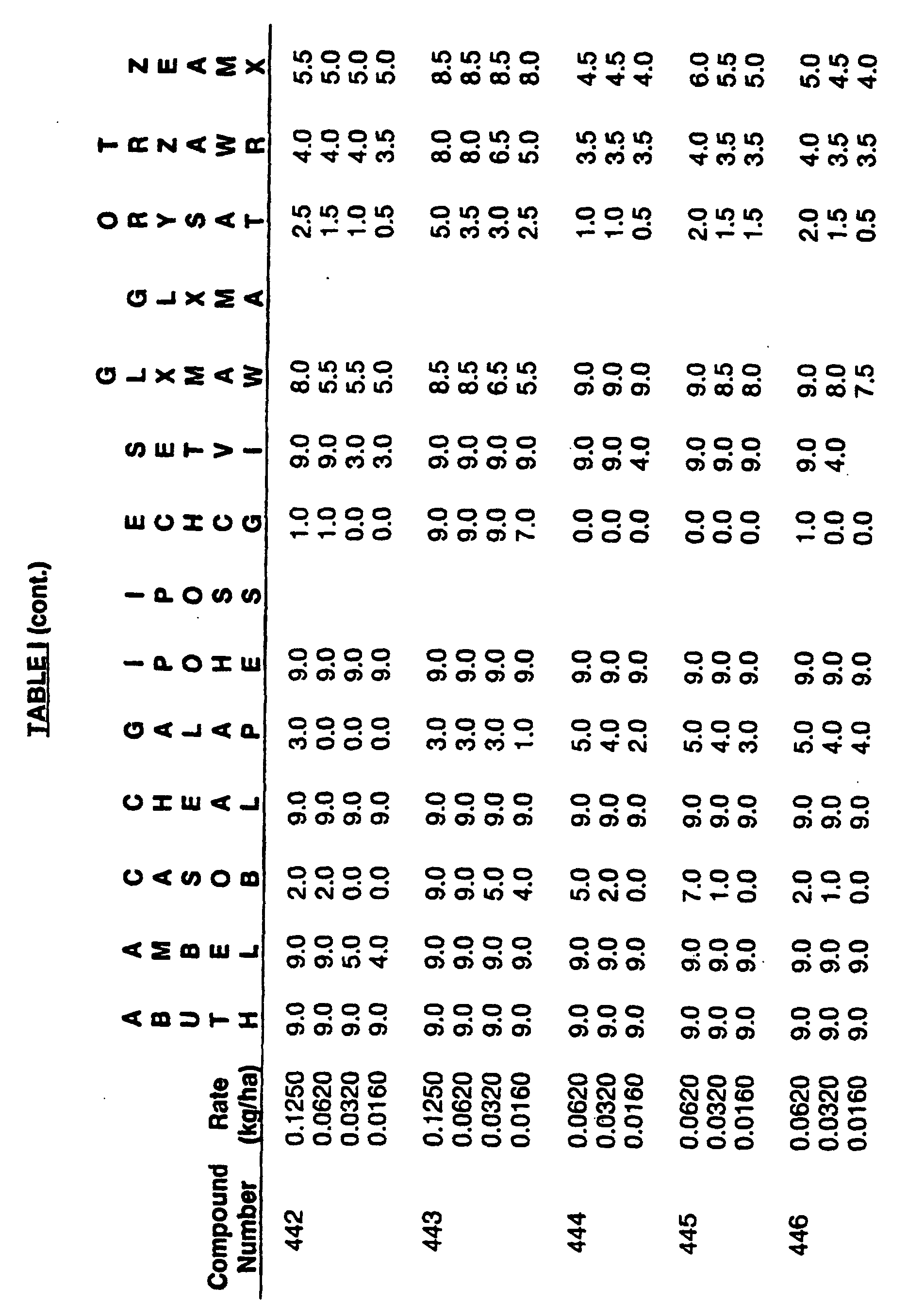

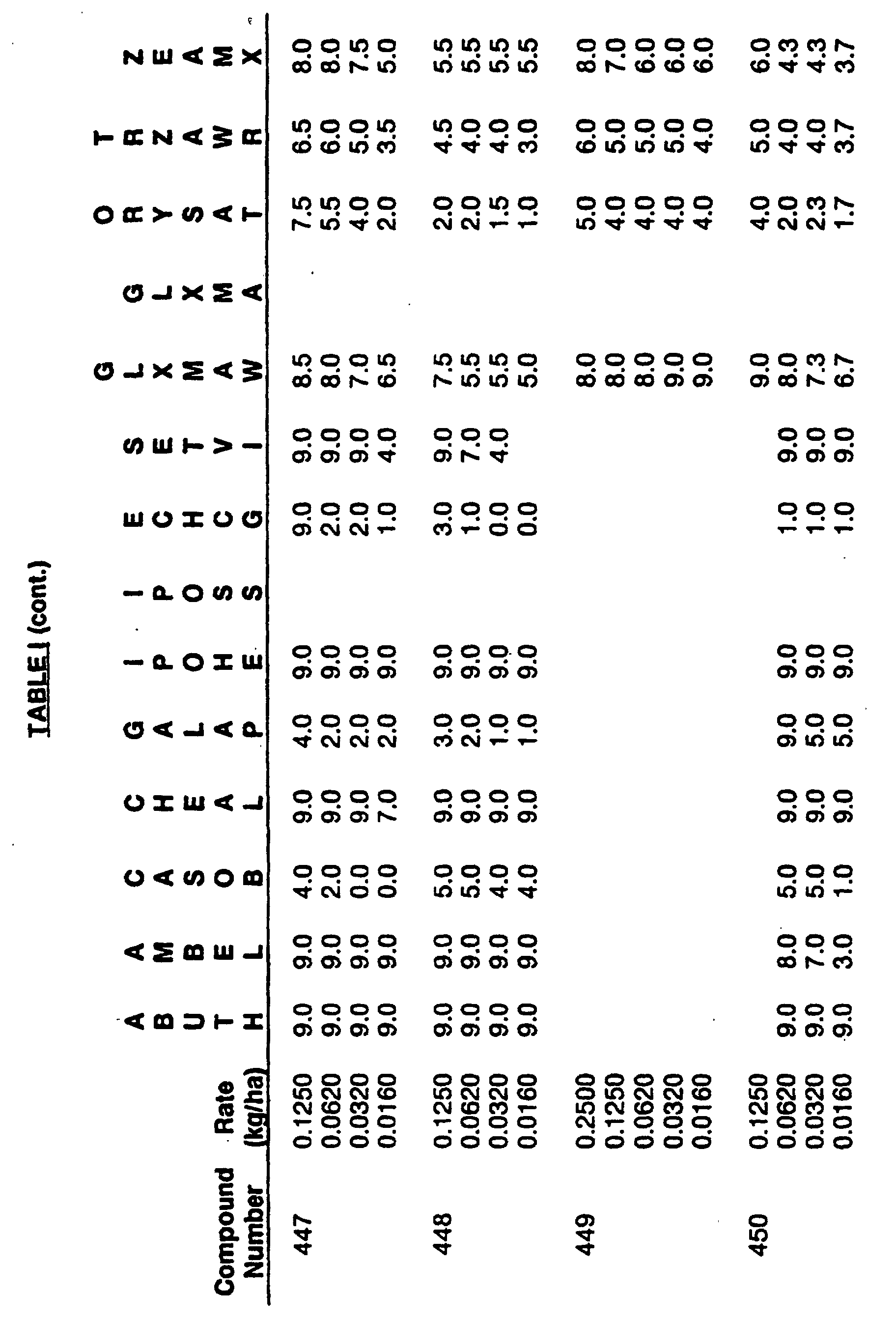

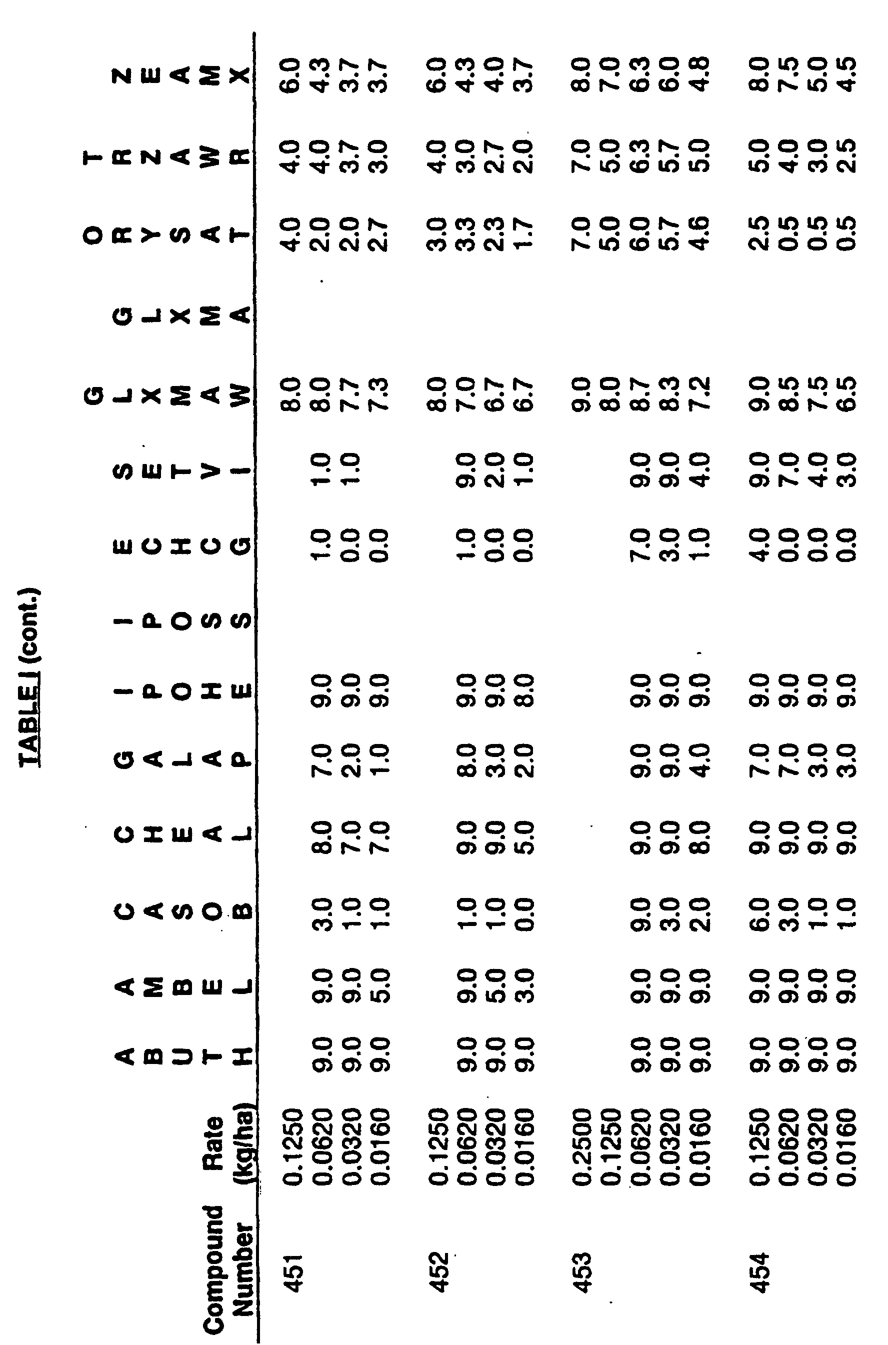

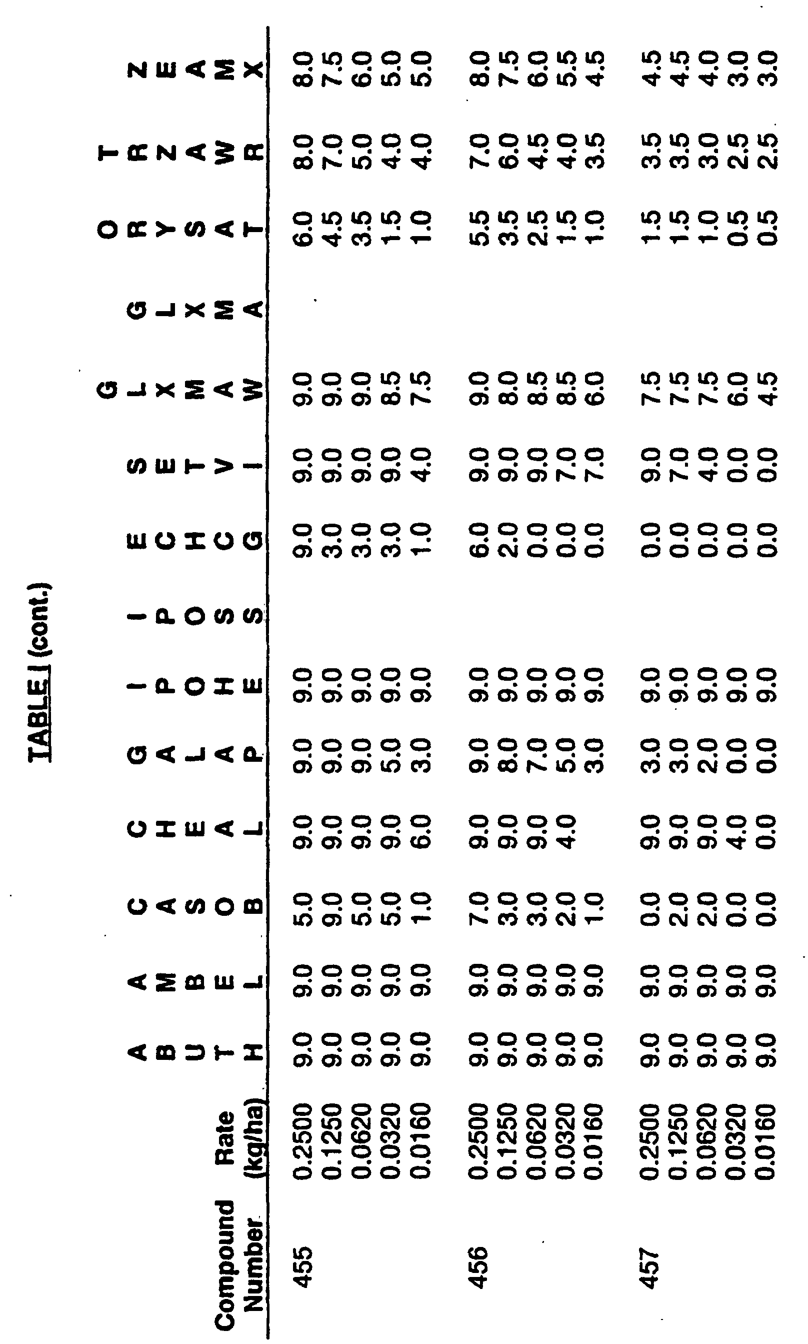

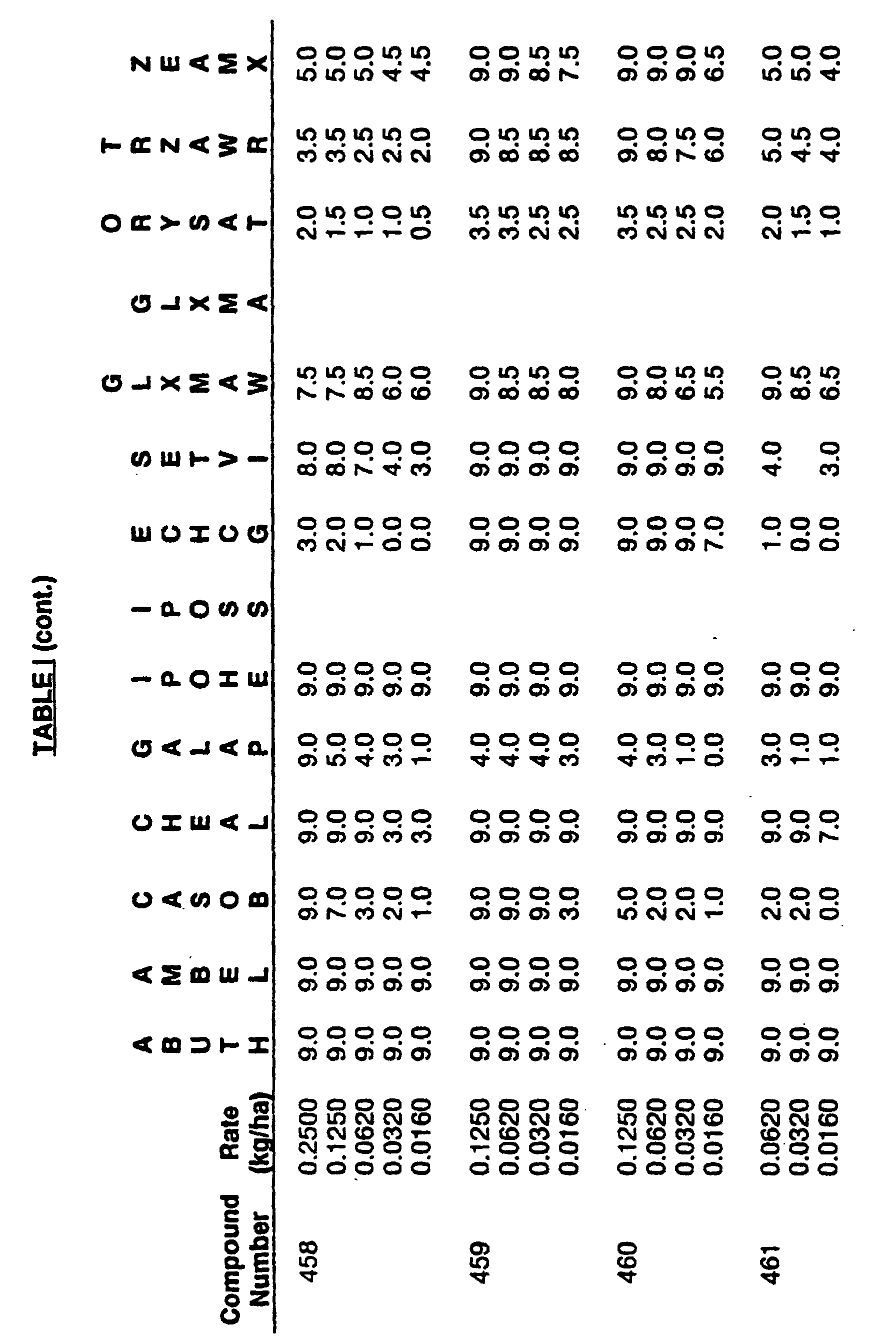

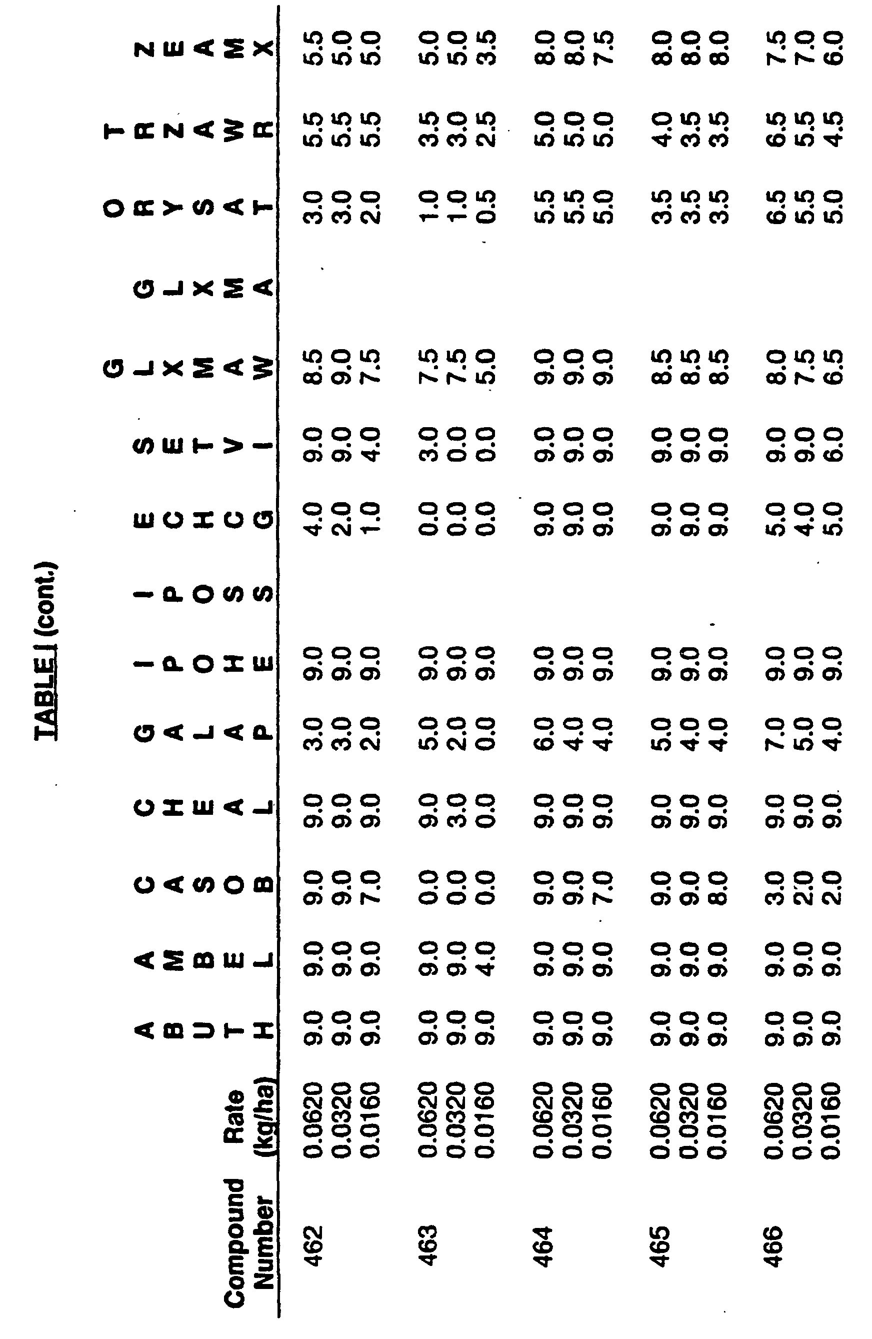

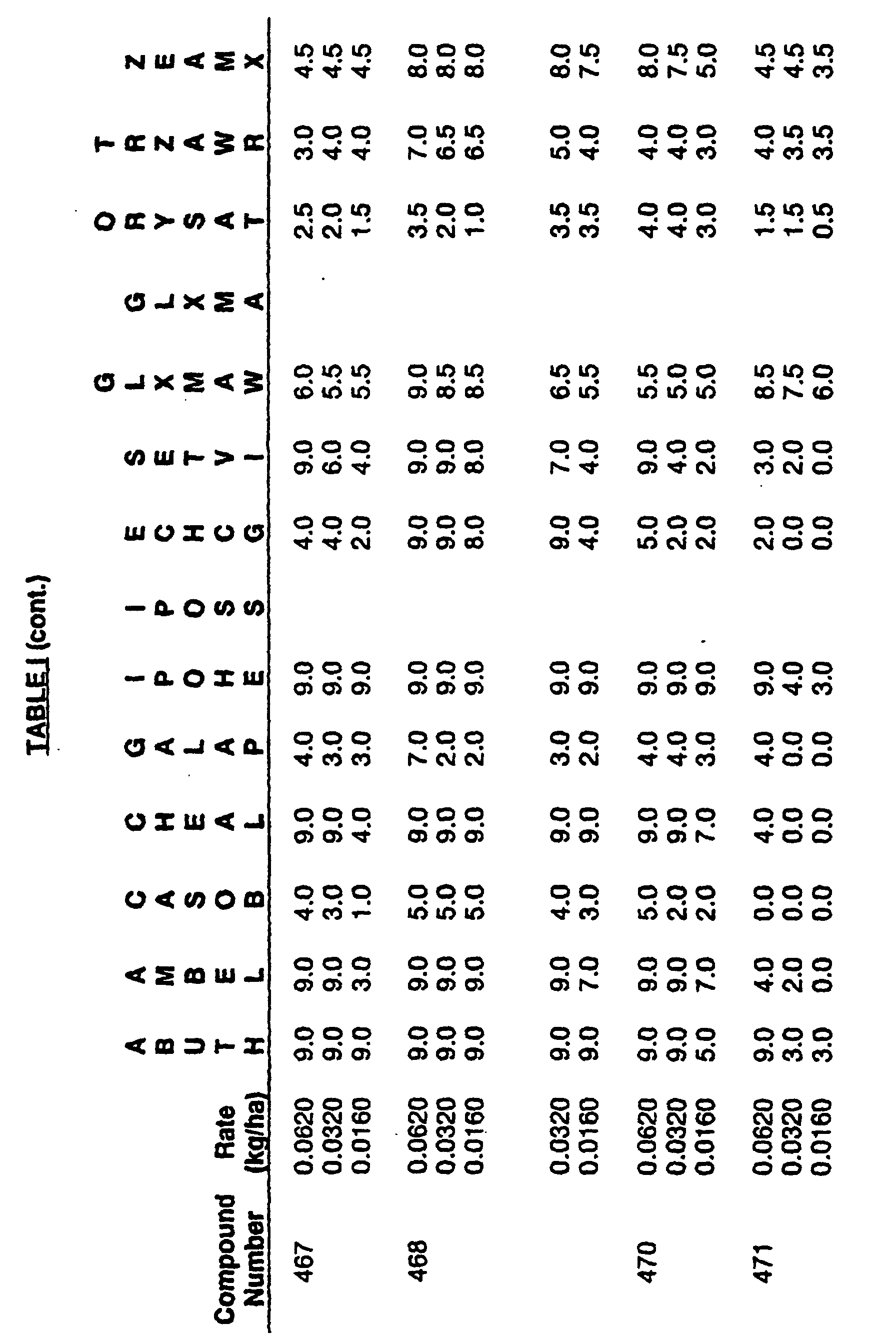

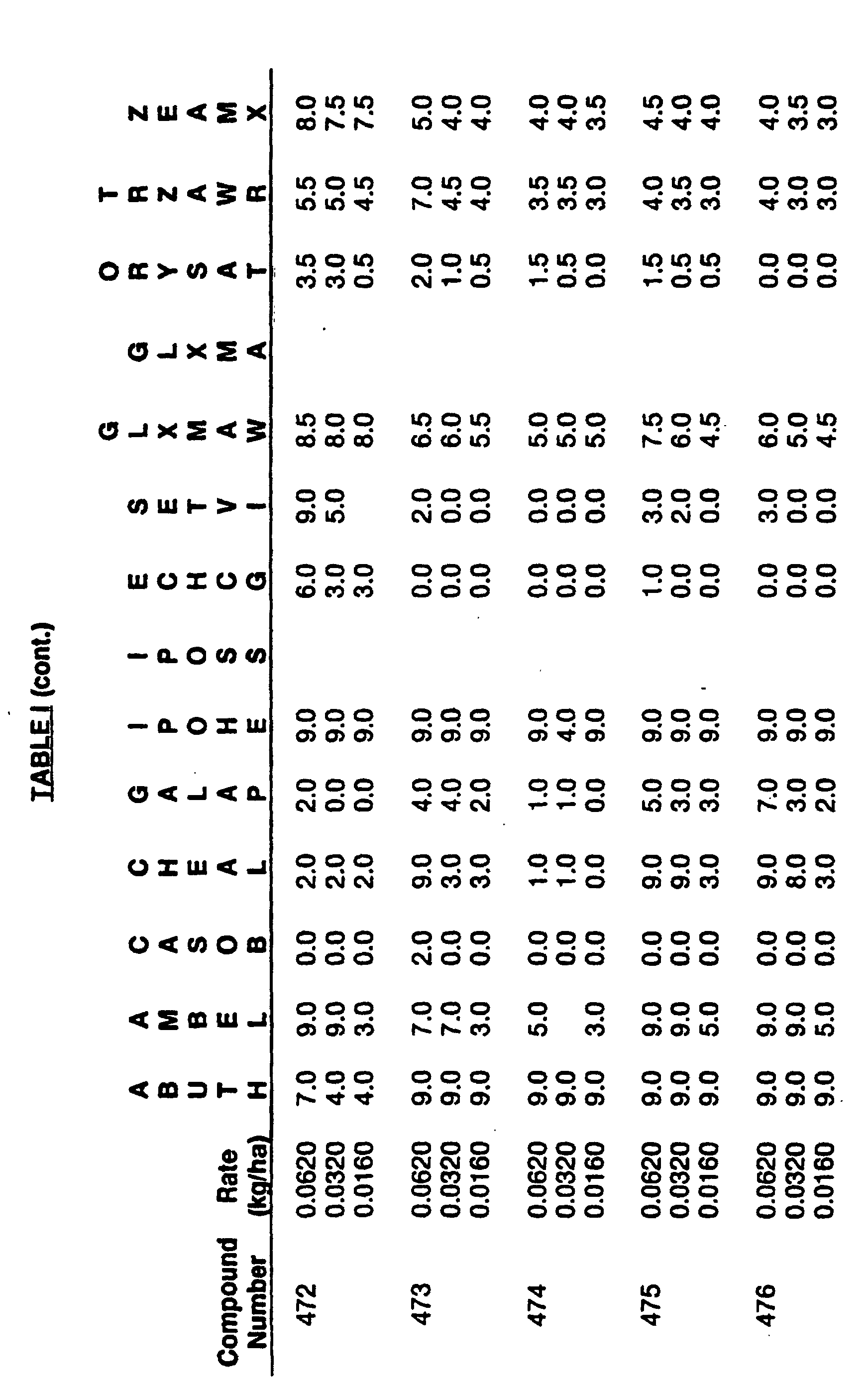

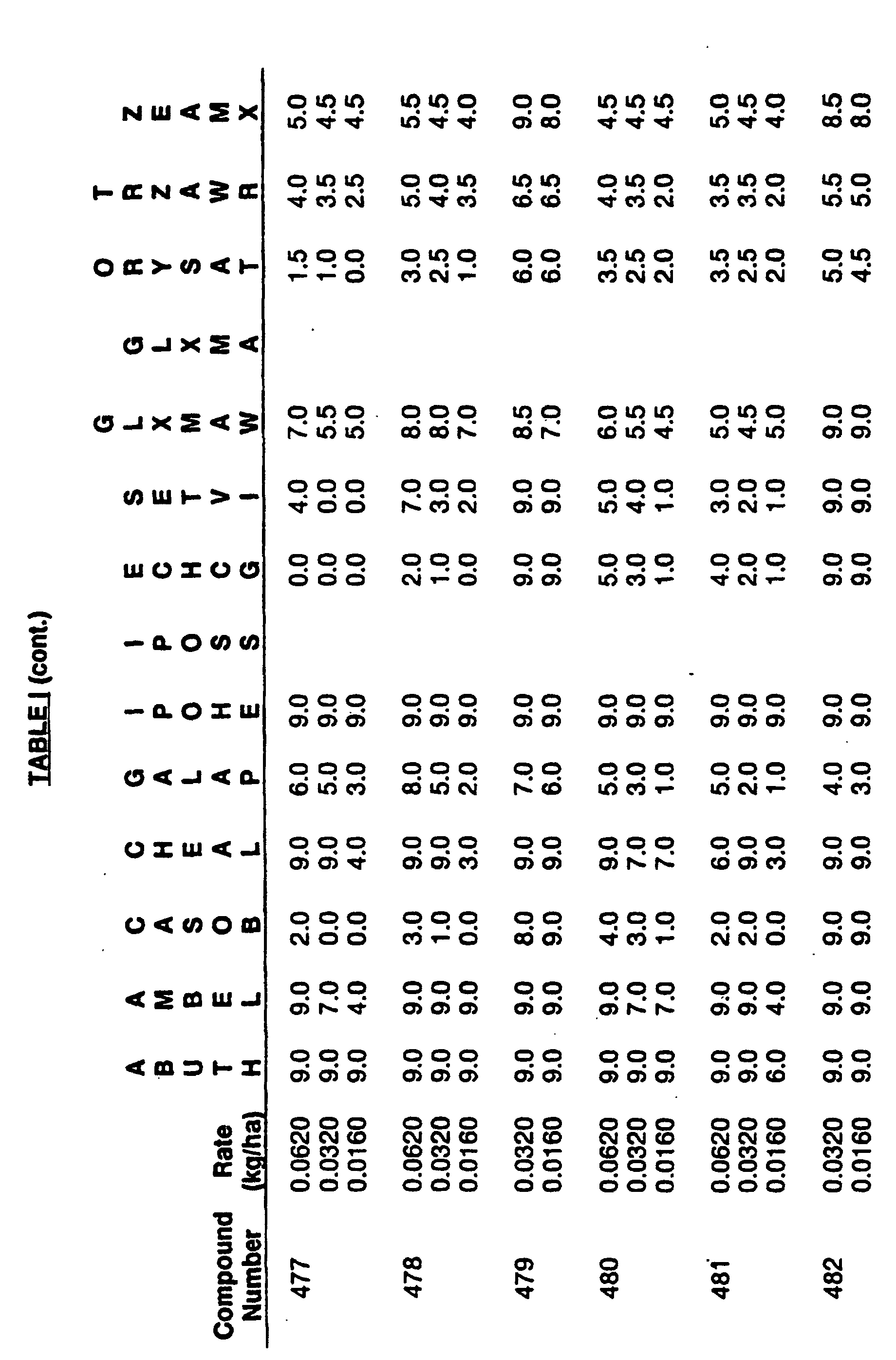

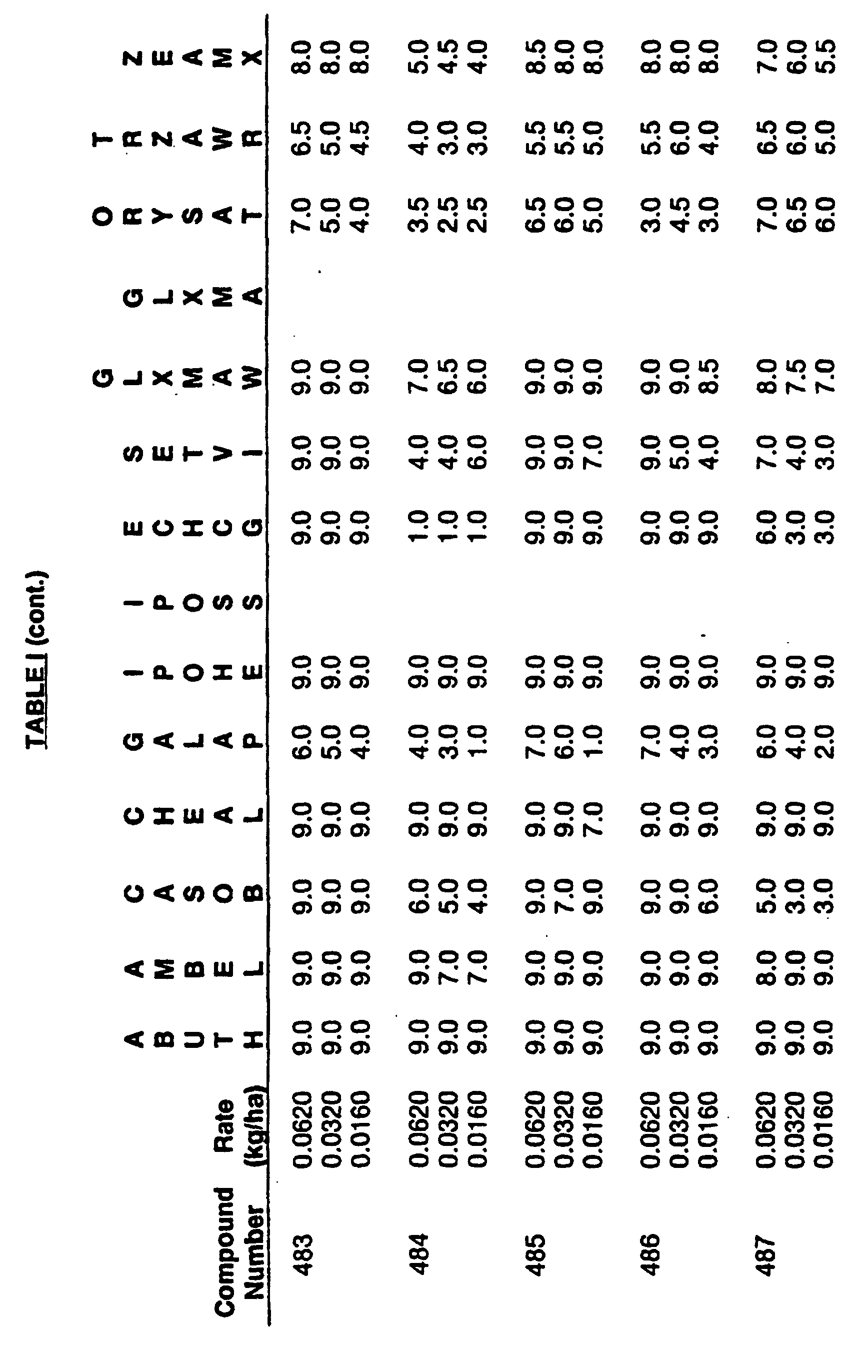

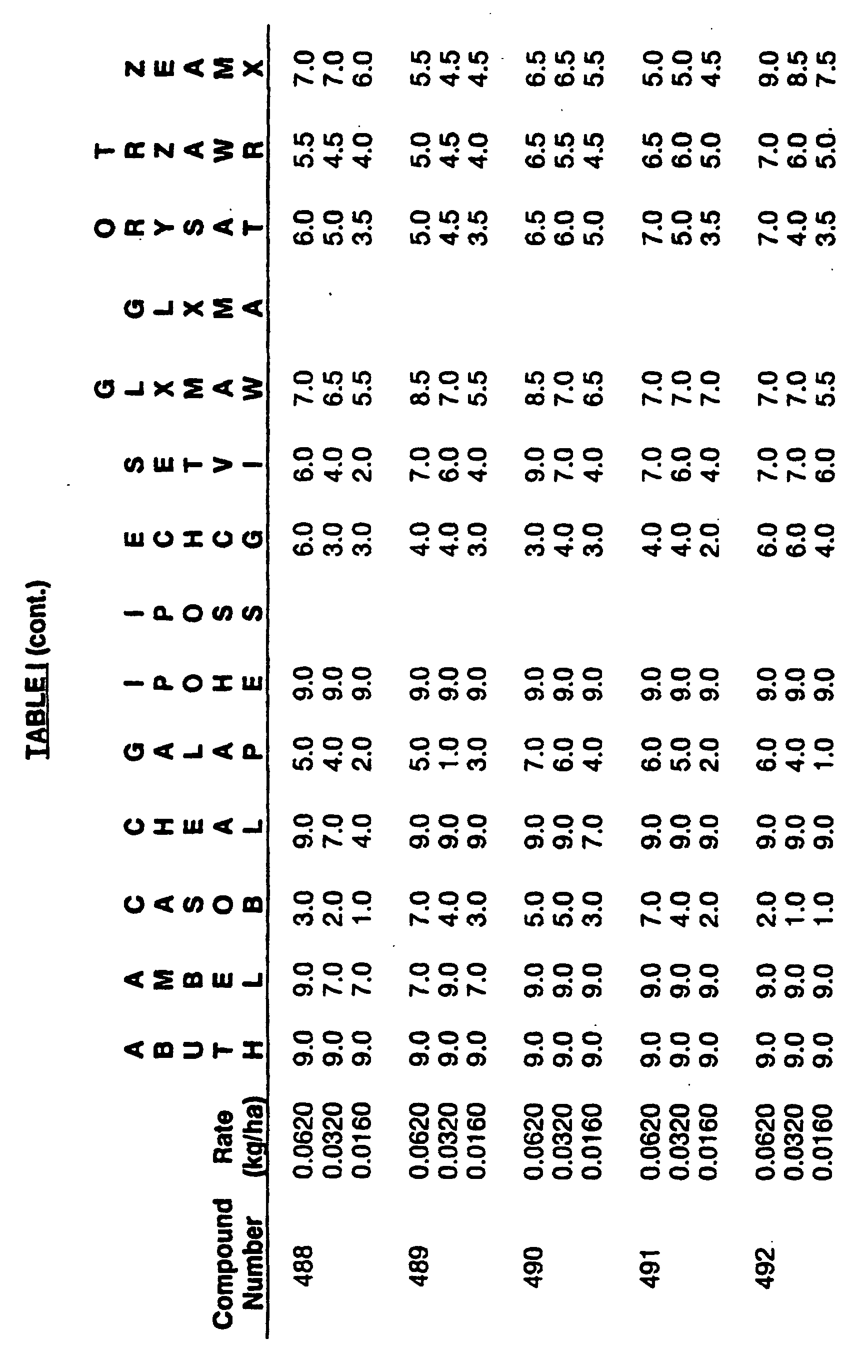

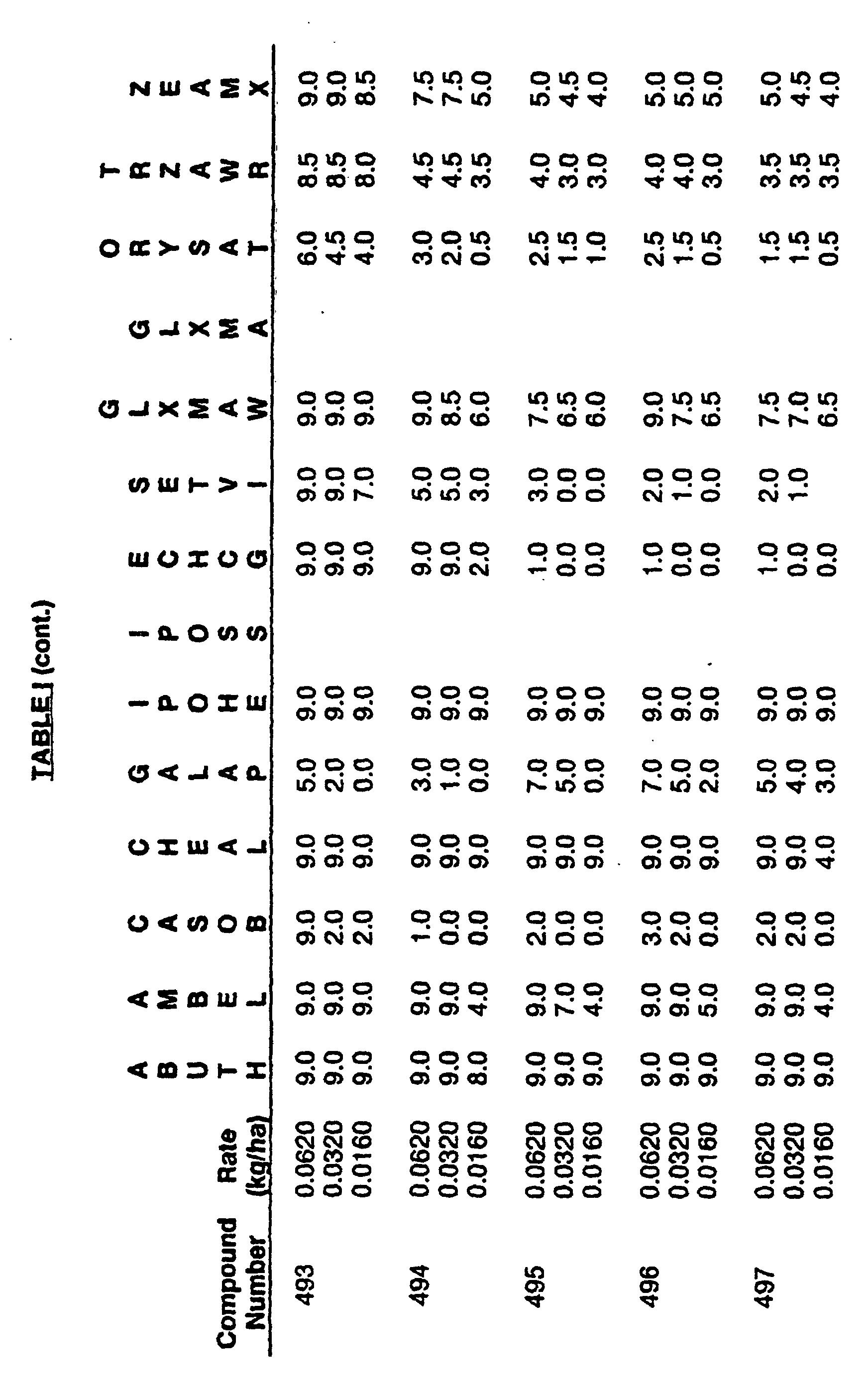

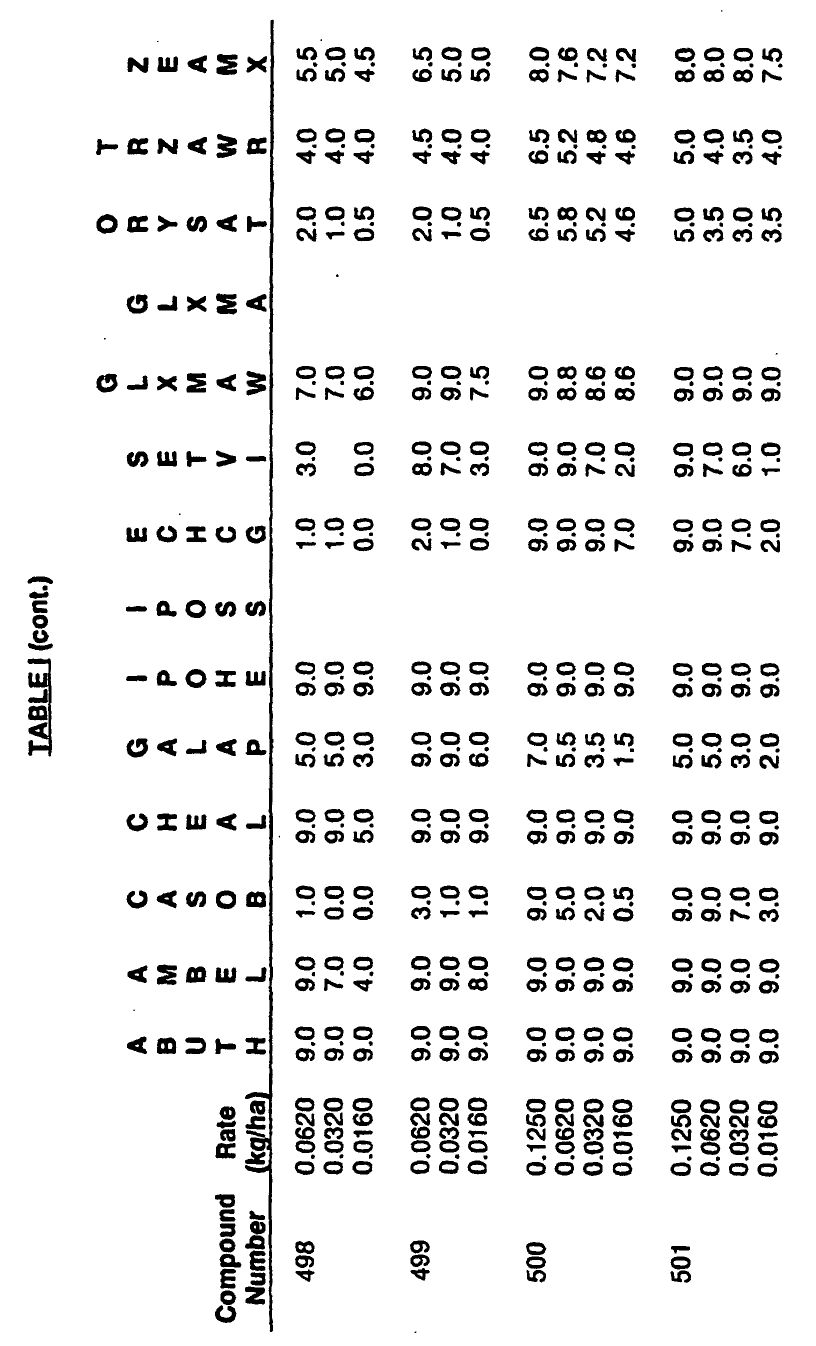

### EXAMPLE 186

### Preemergence herbicidal evaluation of test compounds

The preemergence herbicidal activity of the test compounds of the present invention is exemplified by the following tests in which the seeds of a variety of monocotyledonous and dicotyledonous plants are separately mixed with potting soil and planted on top of approximately one inch of soil in separate pint cups. After planting, the cups are sprayed with the selected aqueous acetone solution containing test compound in sufficient quantity to provide the equivalent of about 0.0156 to 0.500 kg per hectare of test compound per cup. The treated cups are then placed on greenhouse benches, watered and cared for in accordance with conventional greenhouse procedures. From four to five weeks after treatment, the tests are terminated and each cup is examined and rated according to the rating system provided in Example 185.

The data obtained are reported in Table II below. The compounds evaluated are reported by compound number given in Example 185.

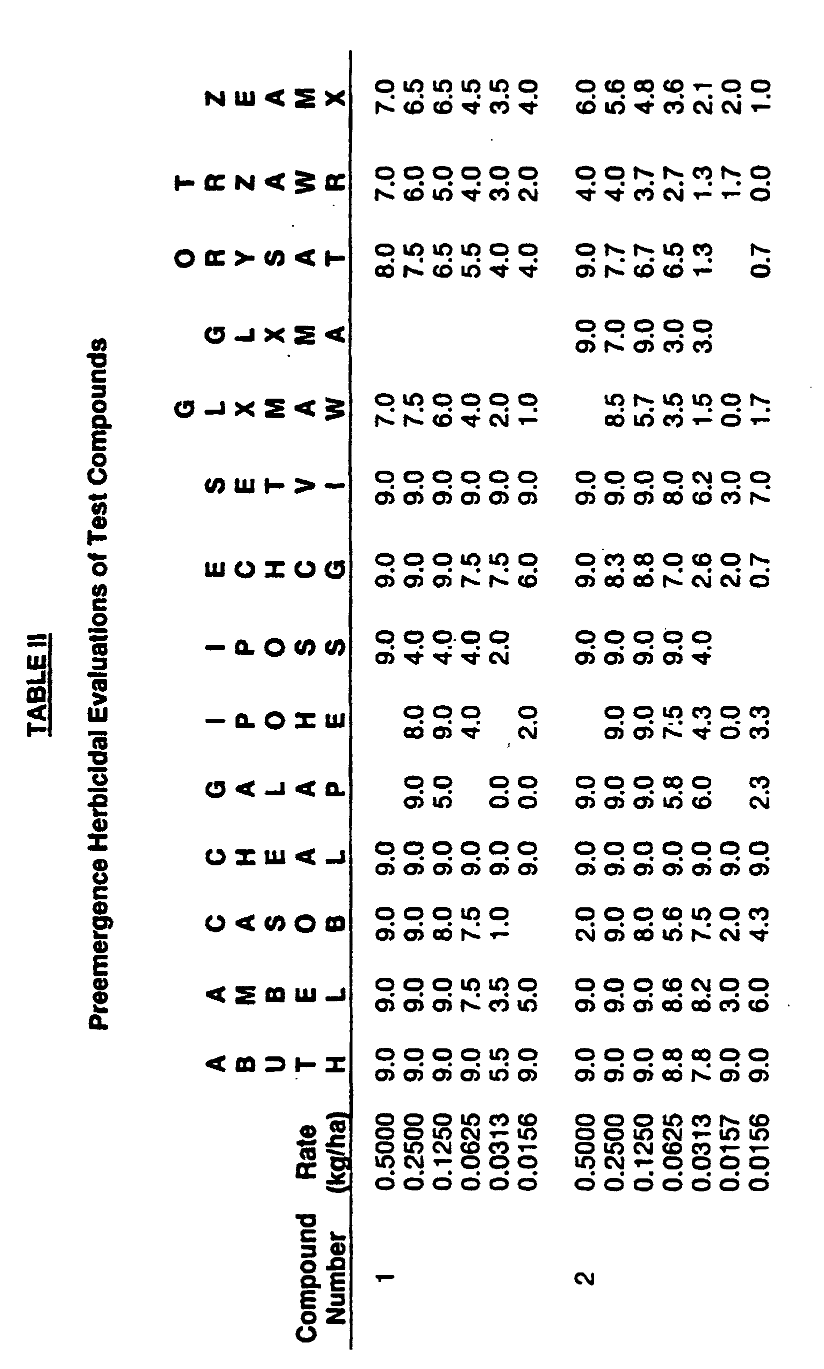

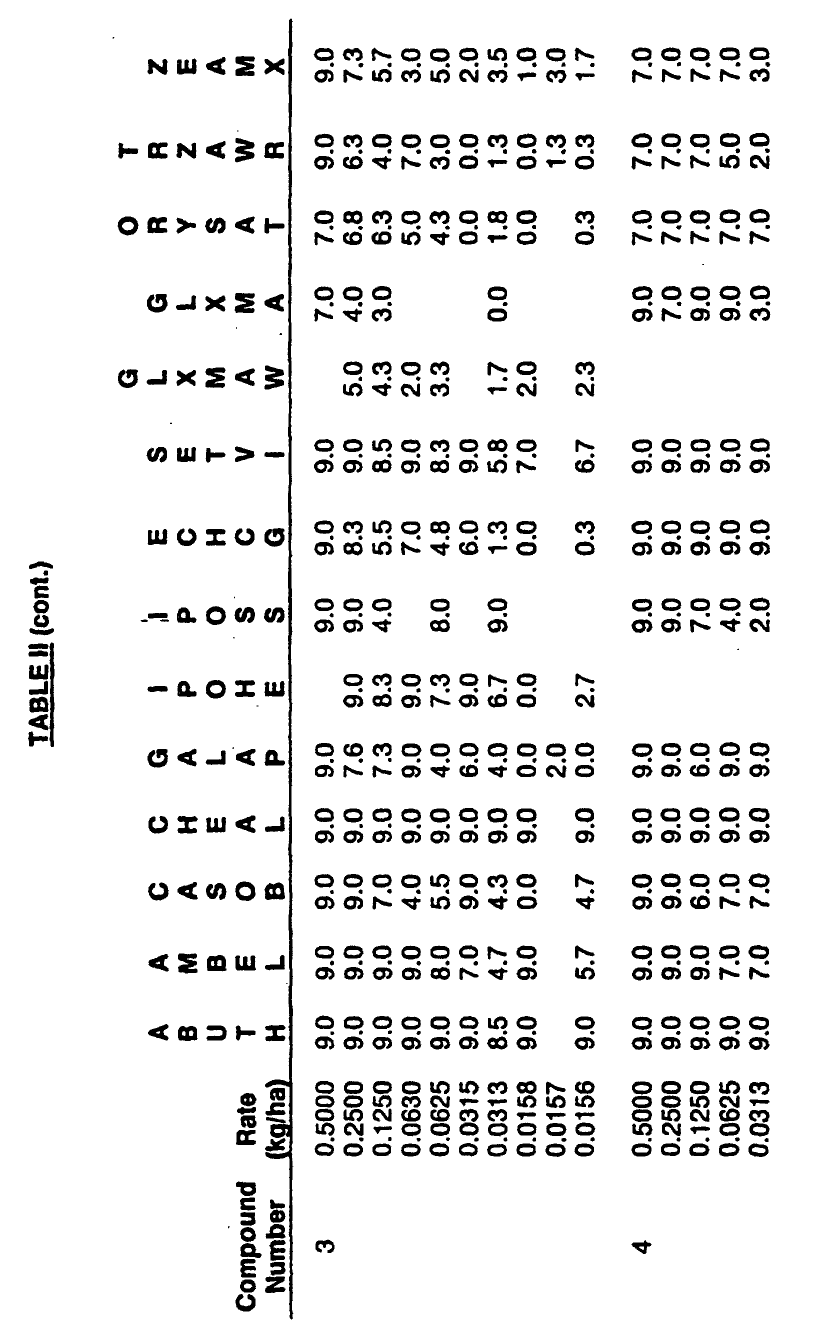

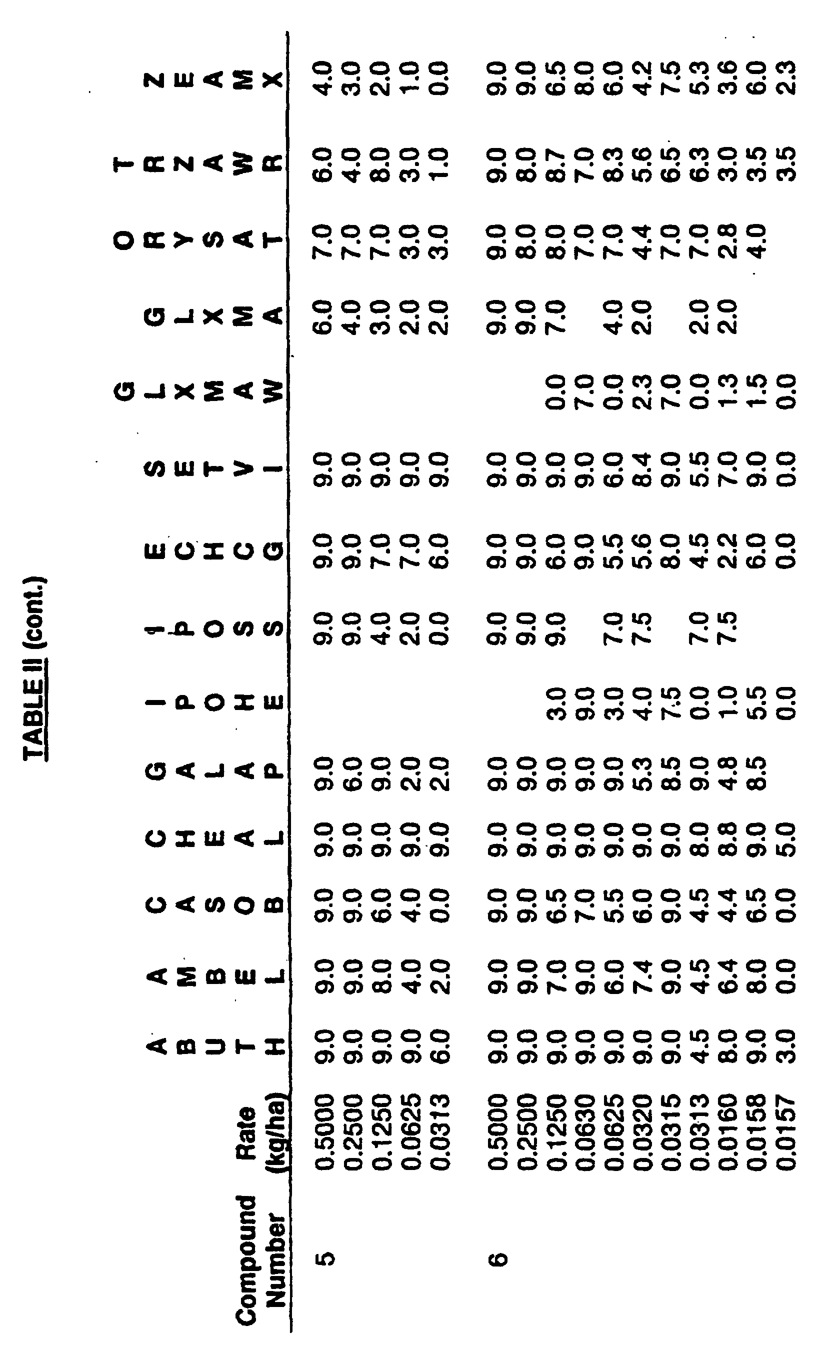

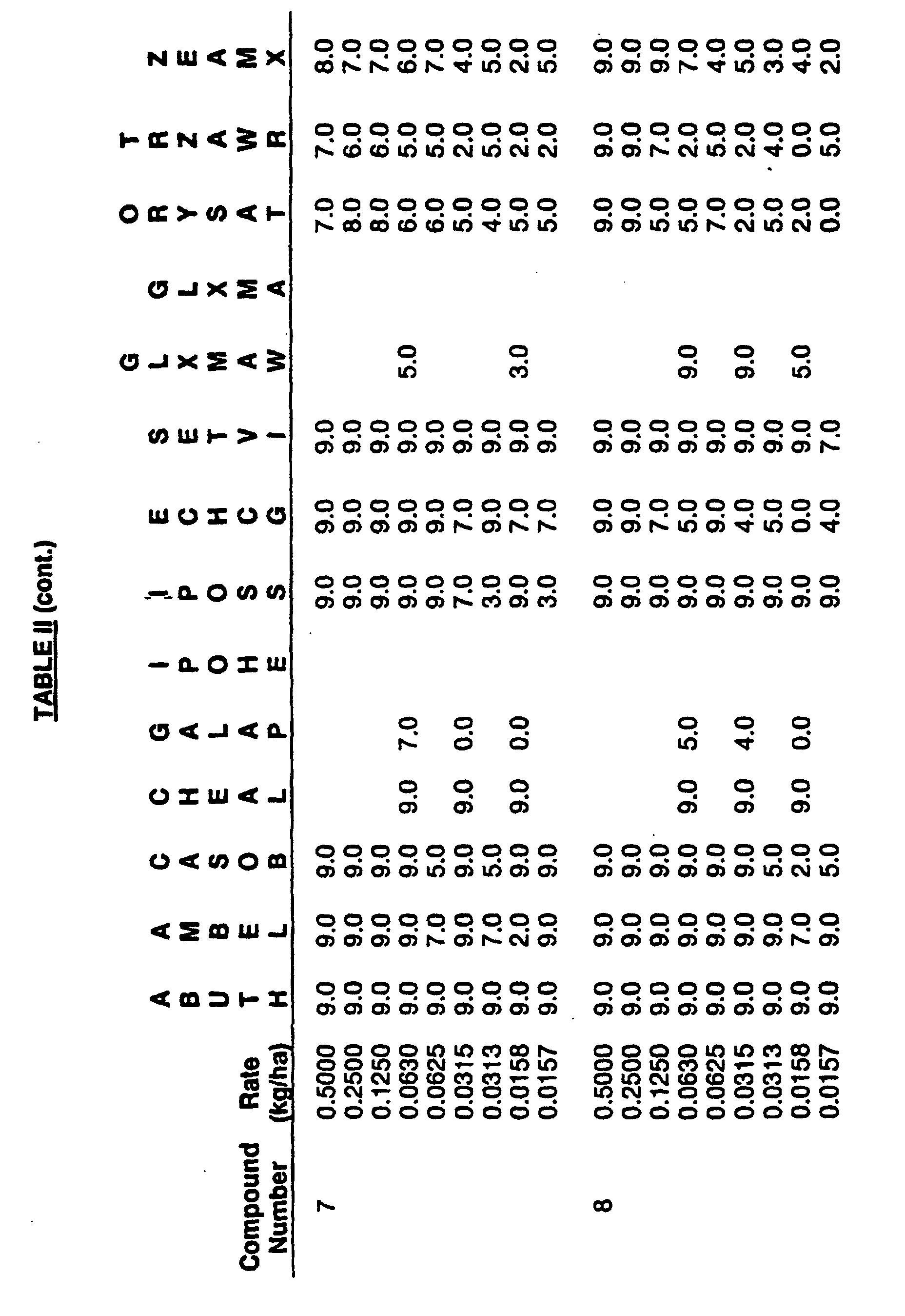

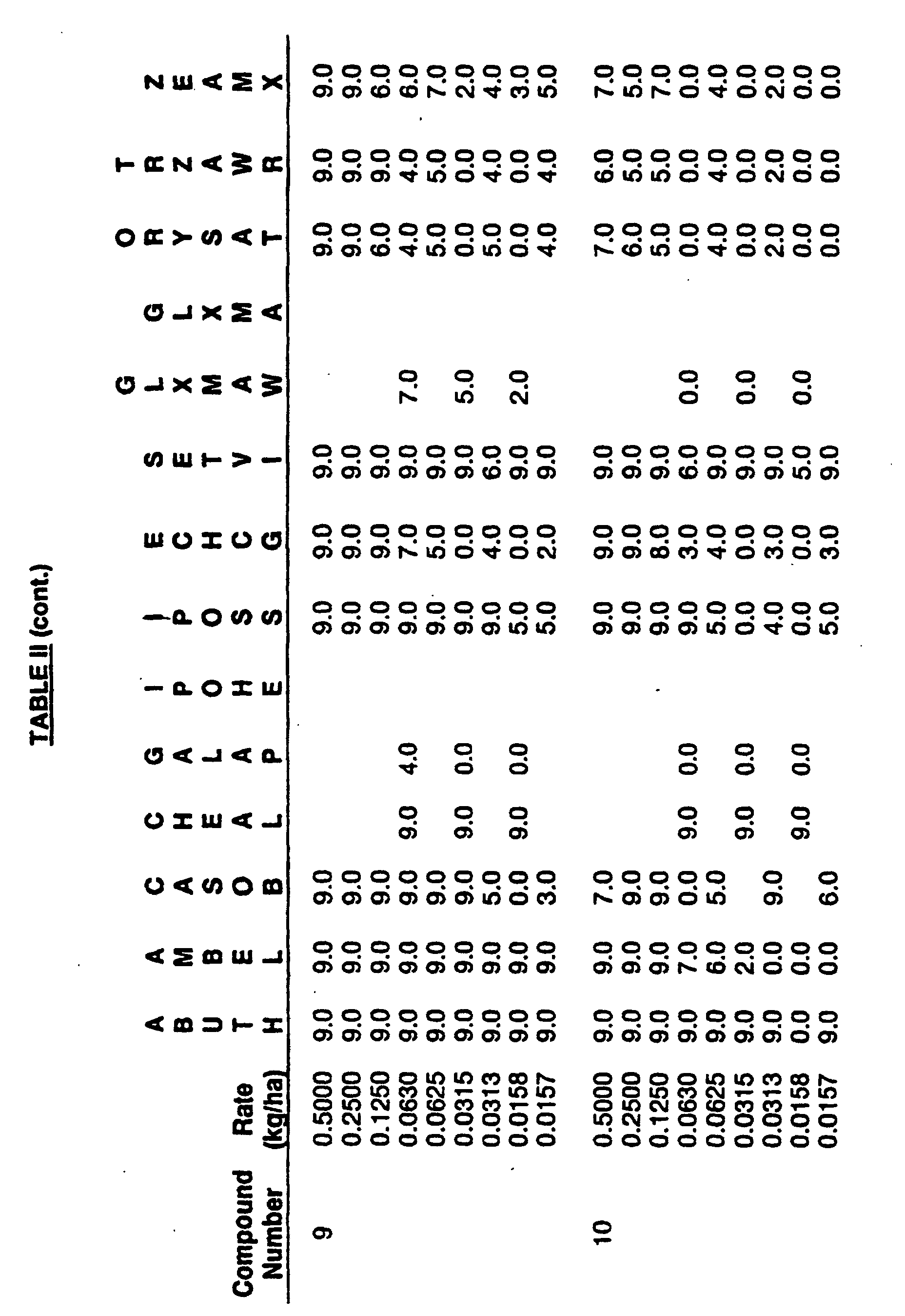

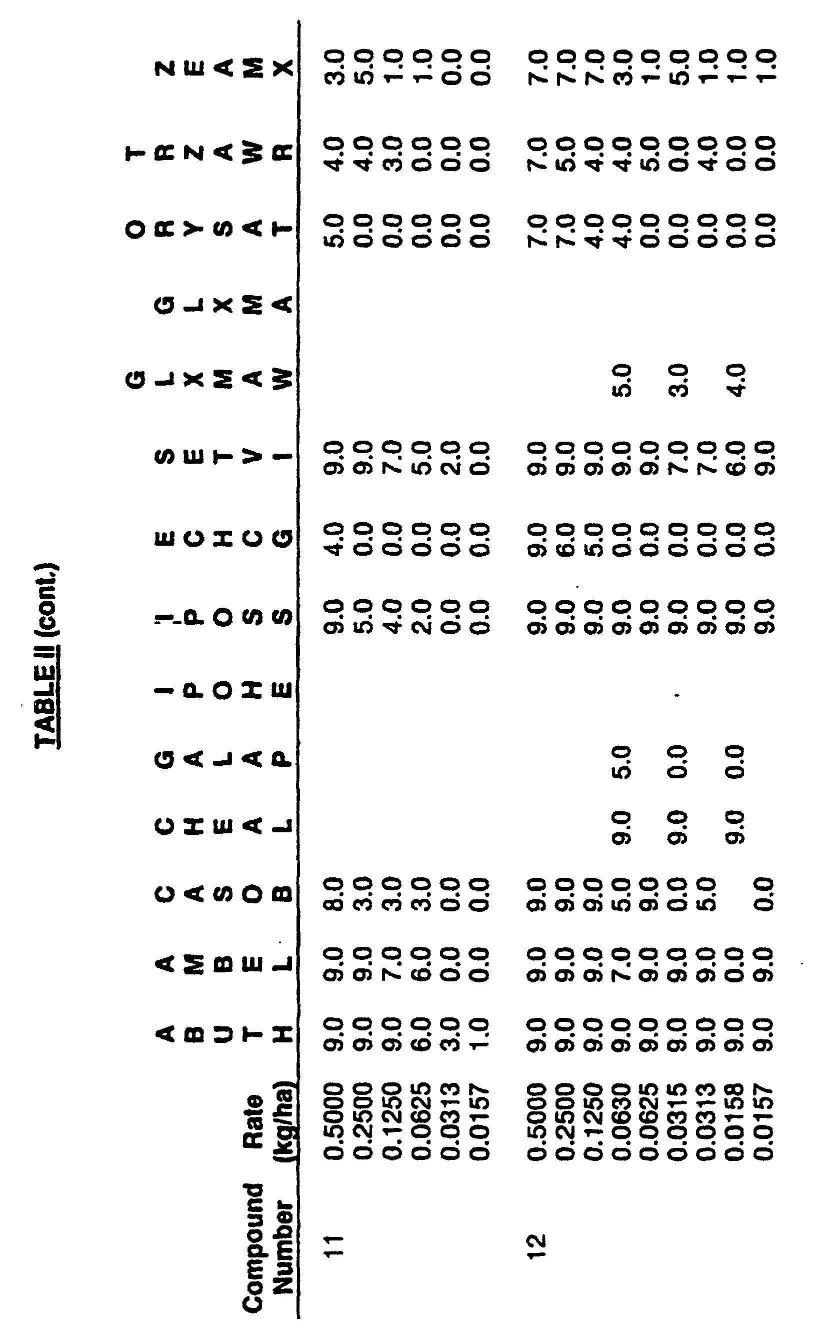

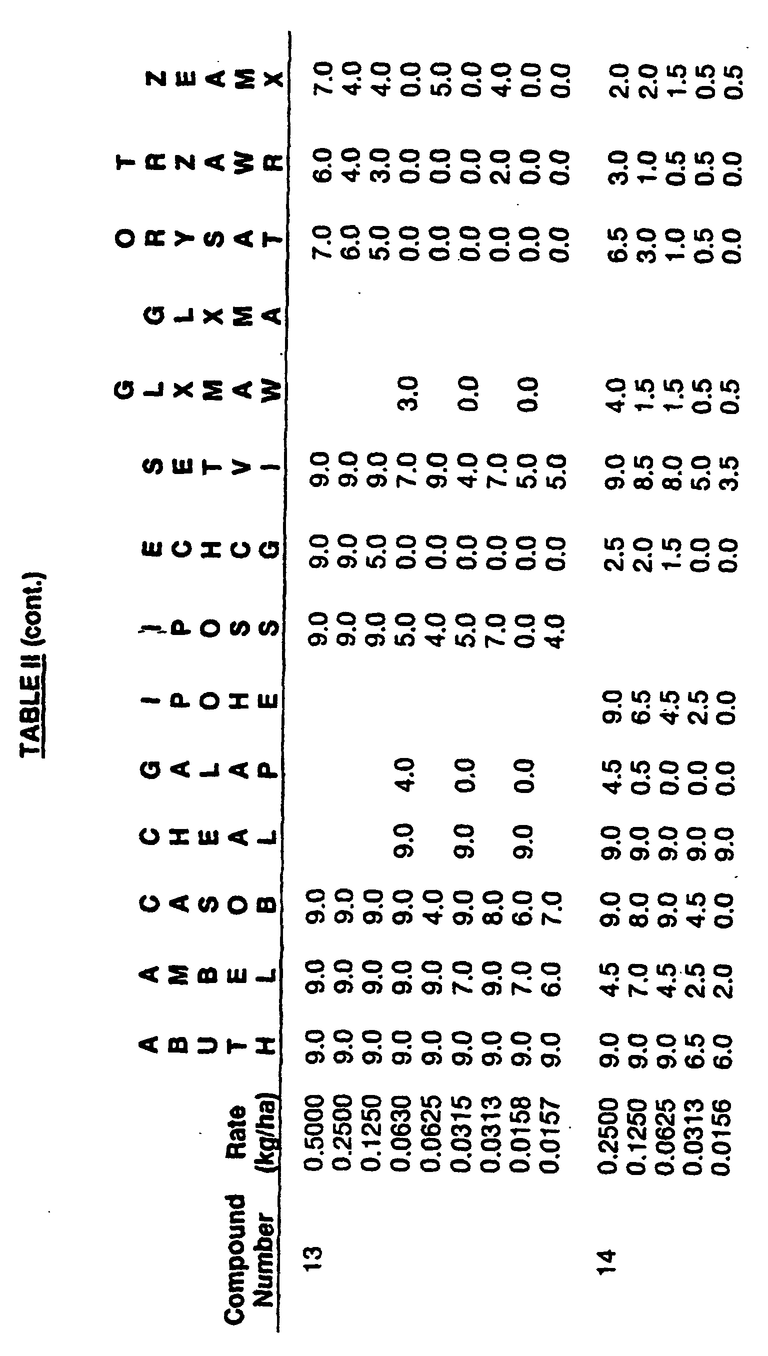

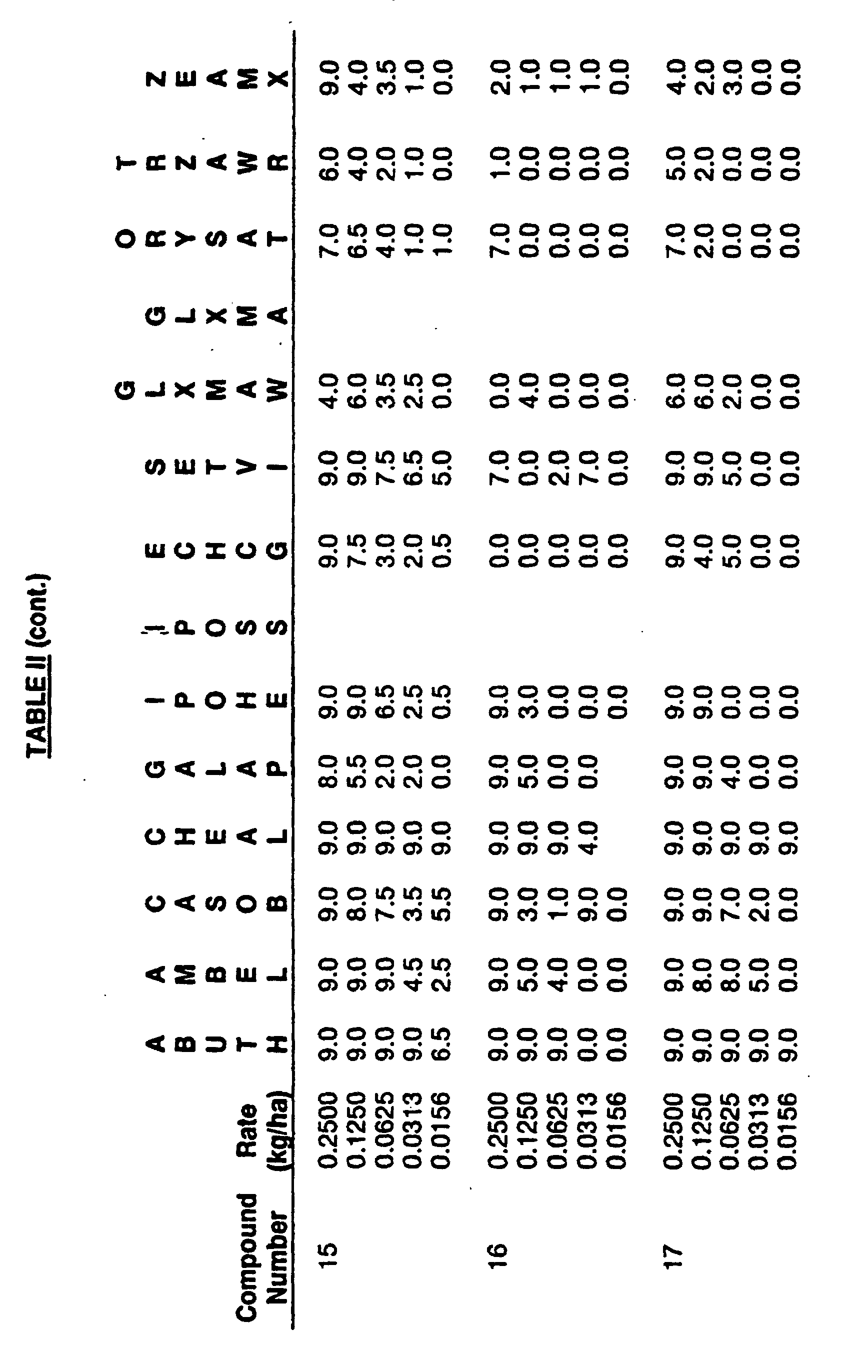

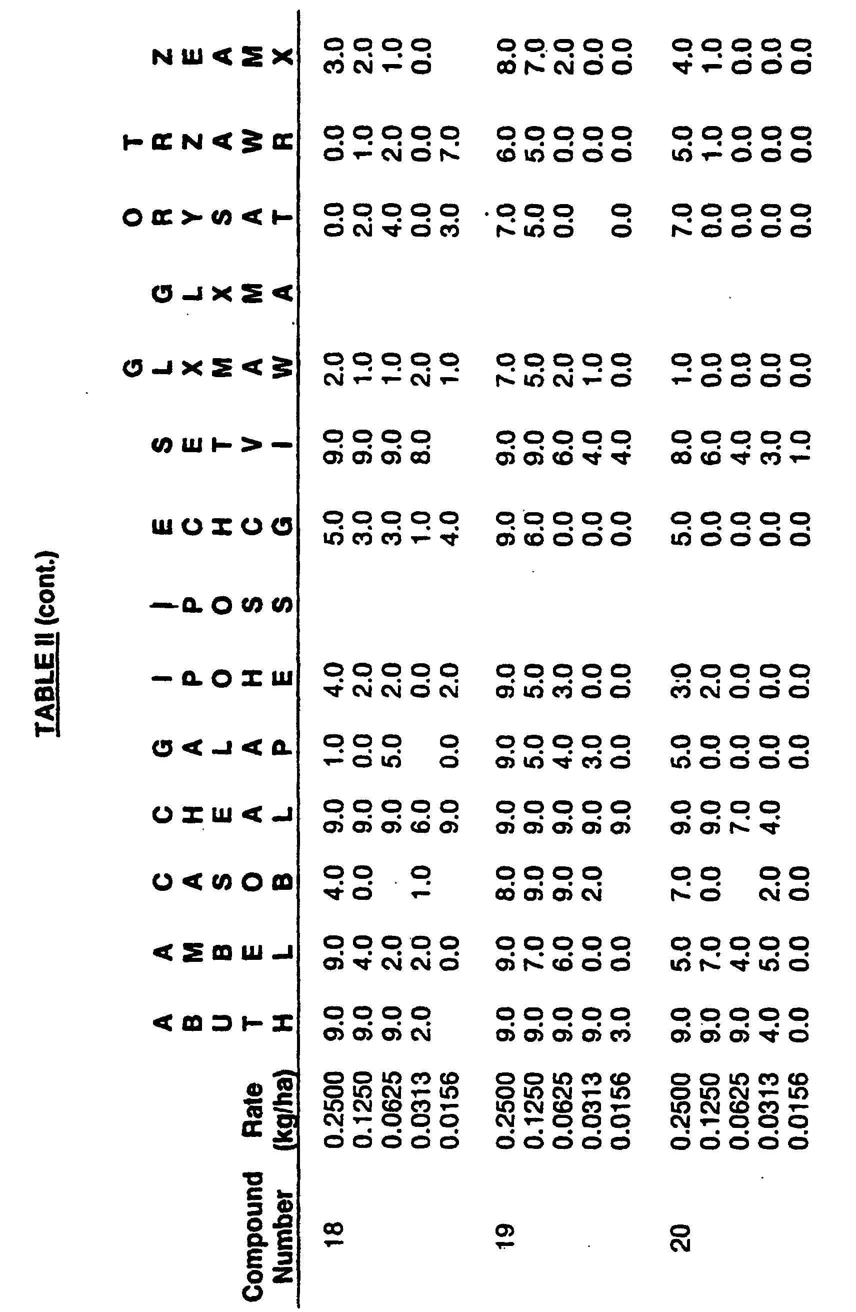

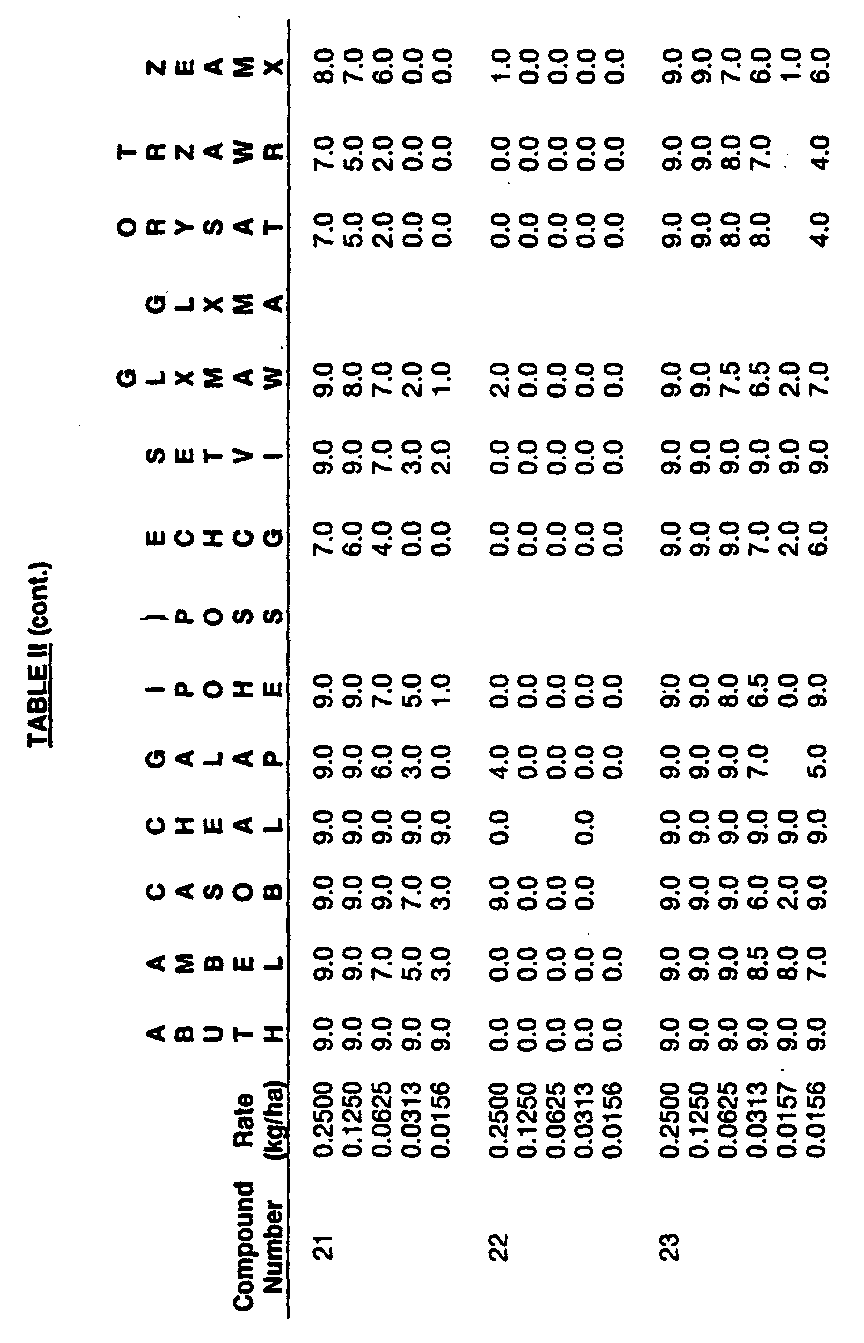

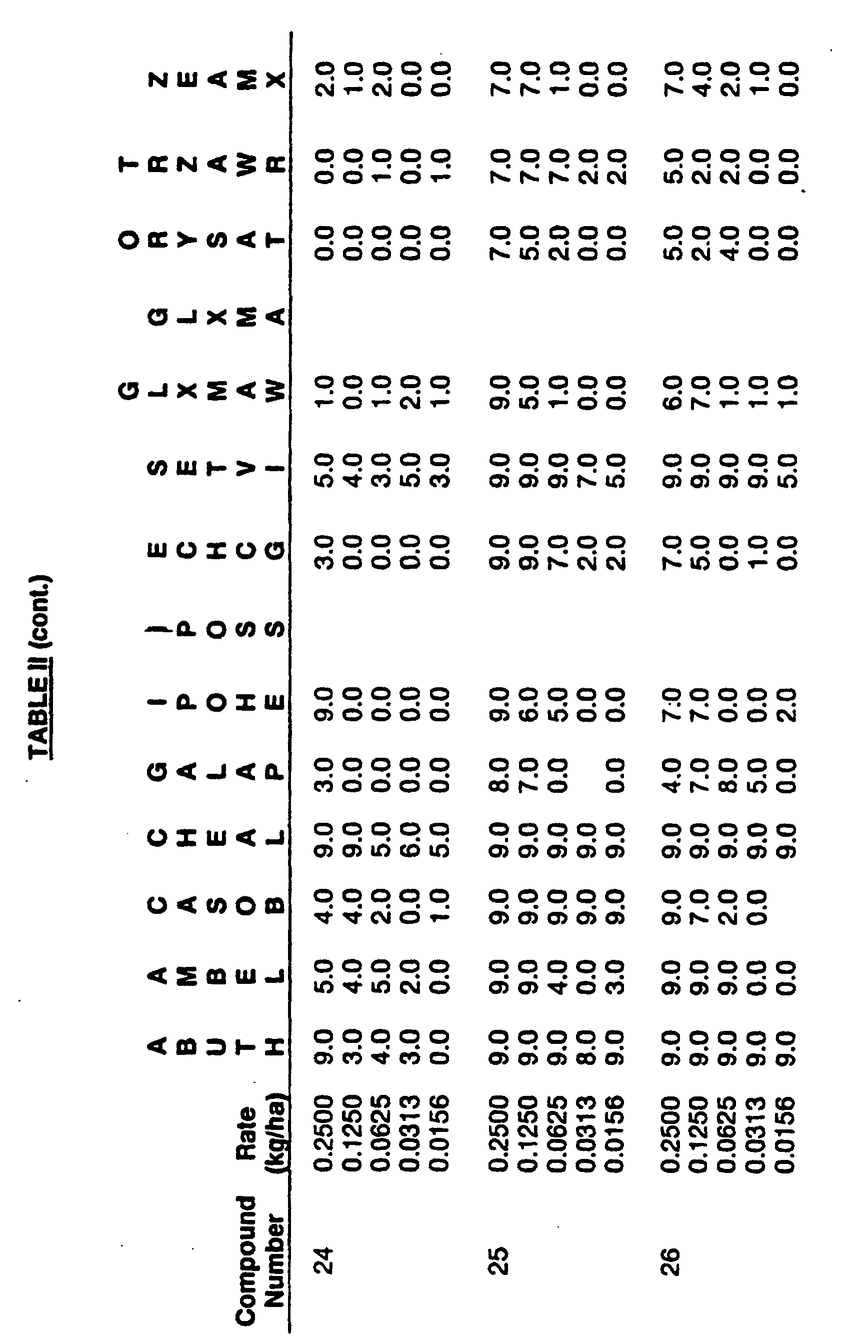

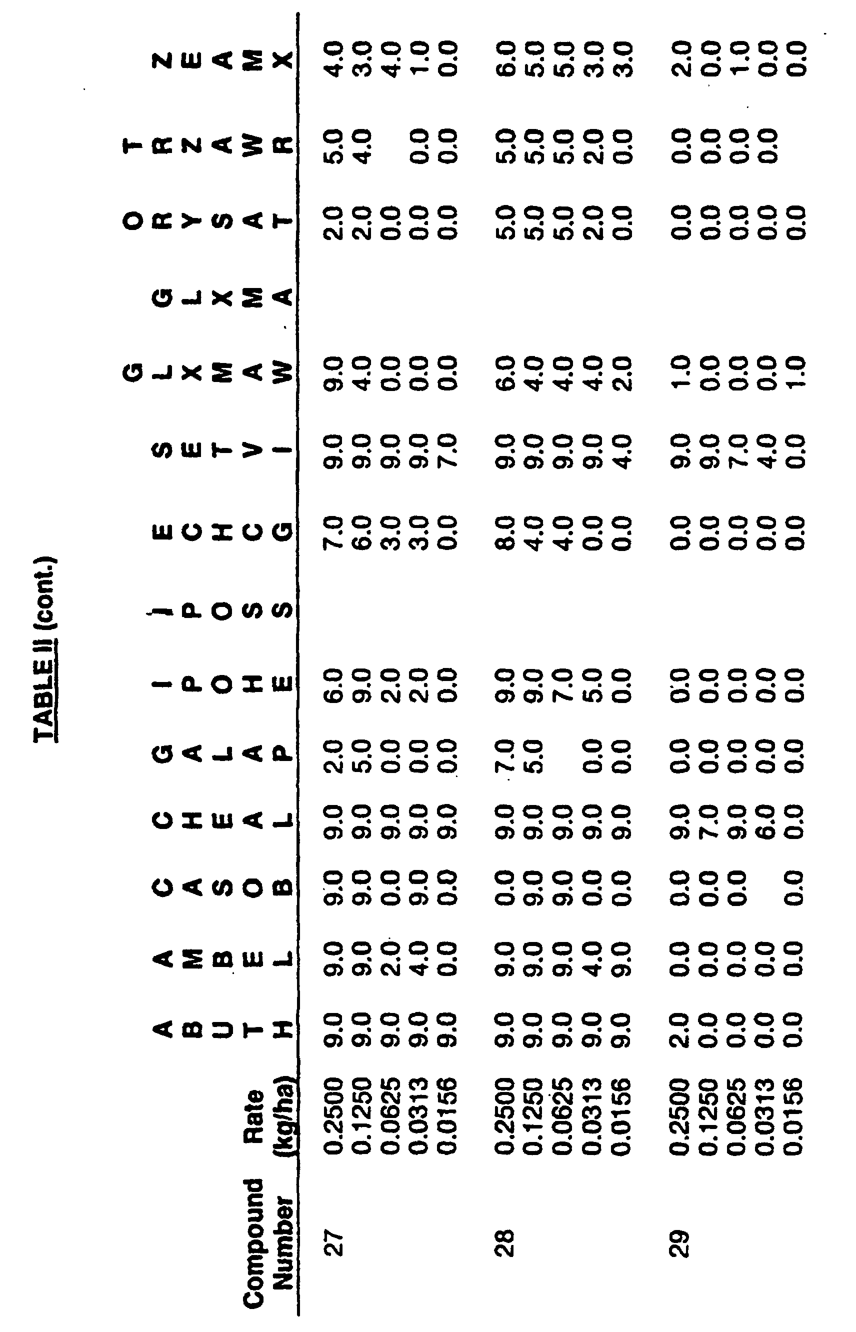

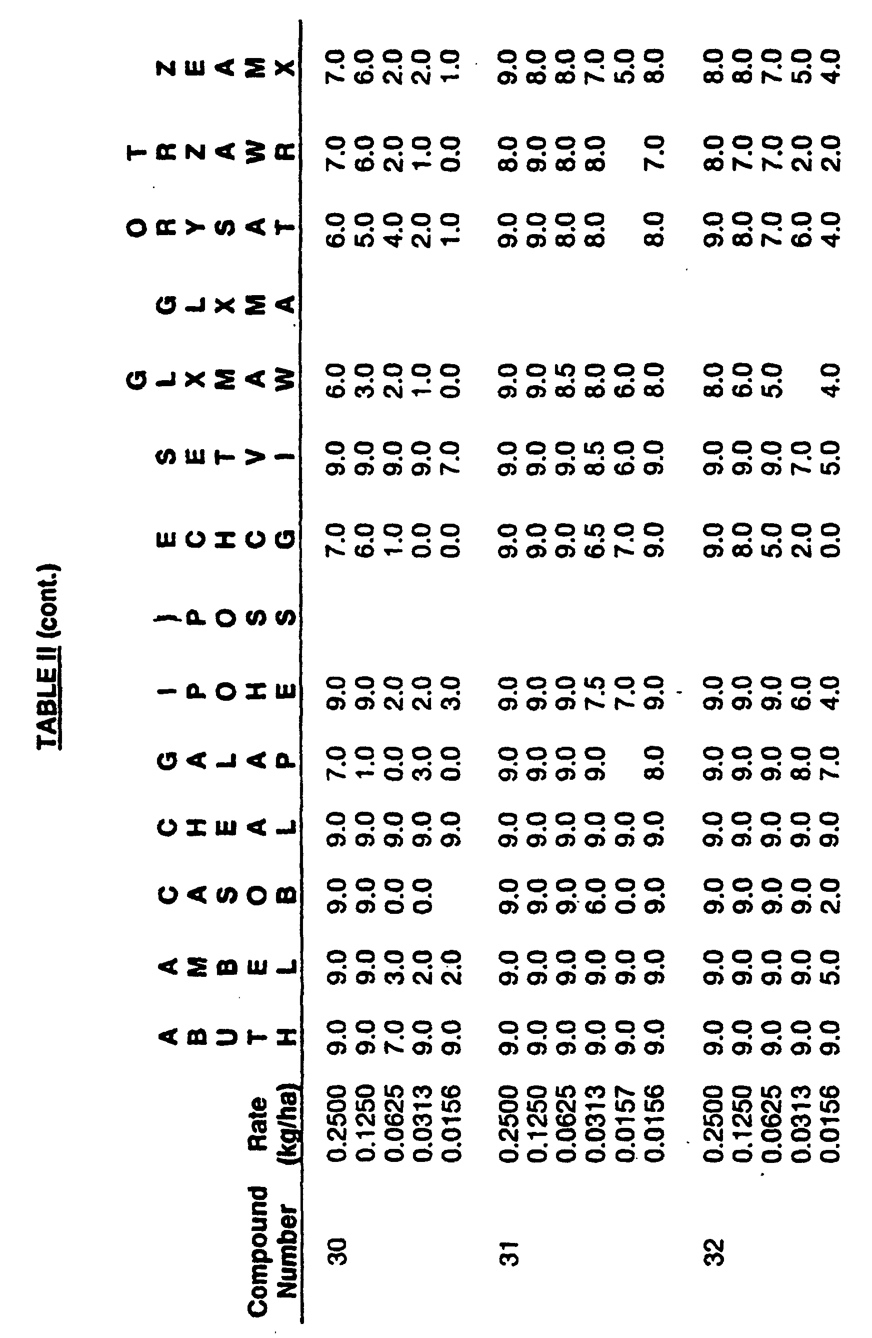

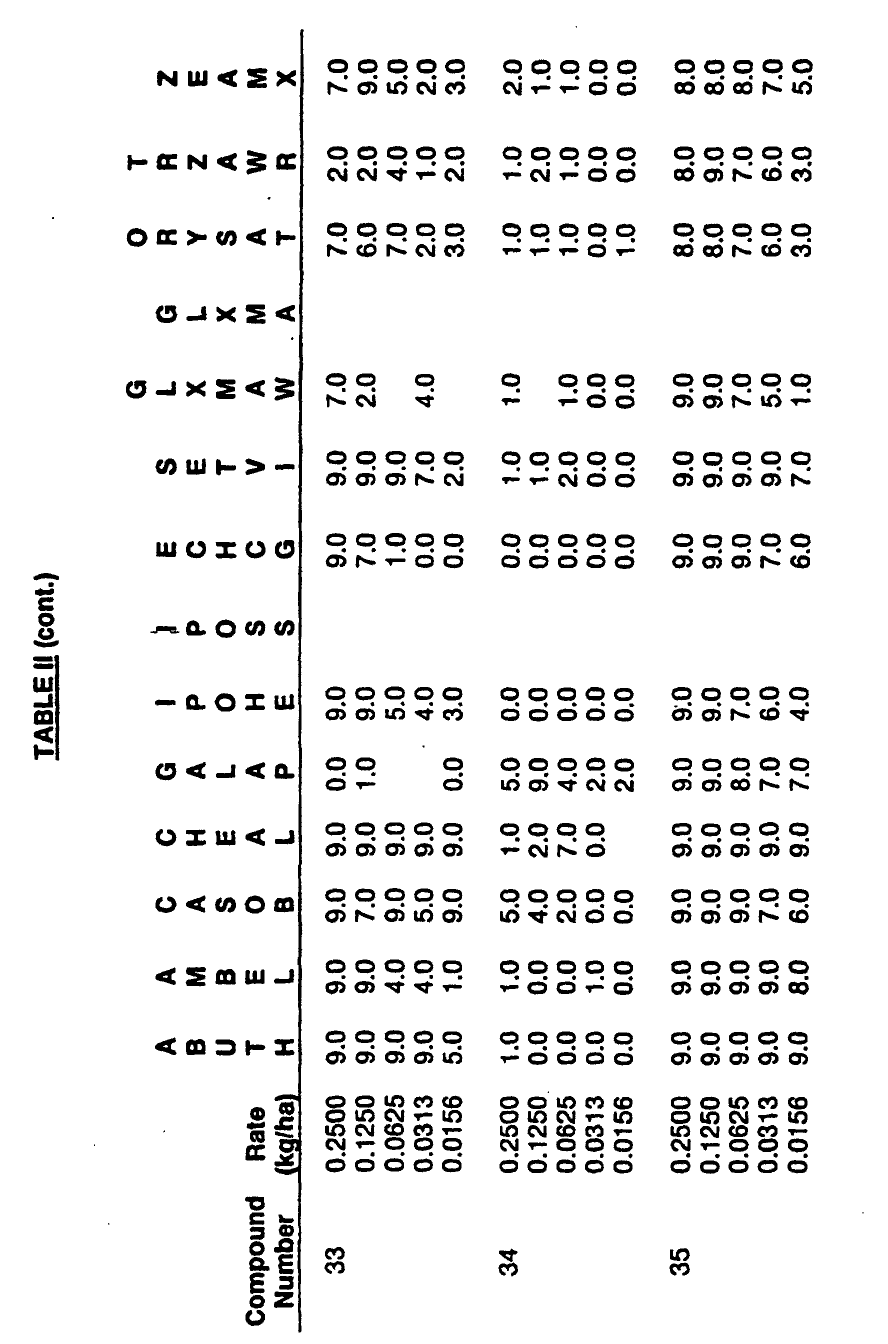

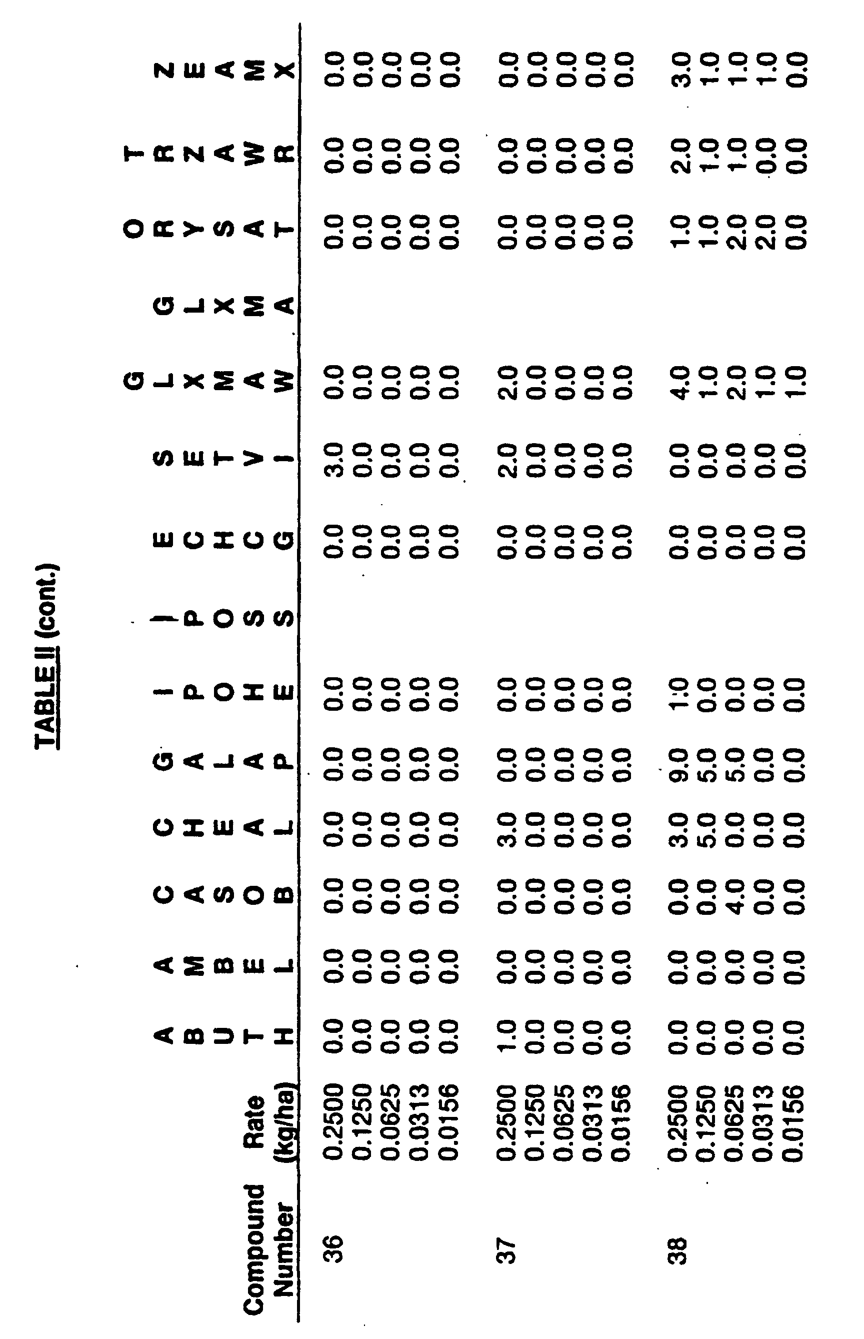

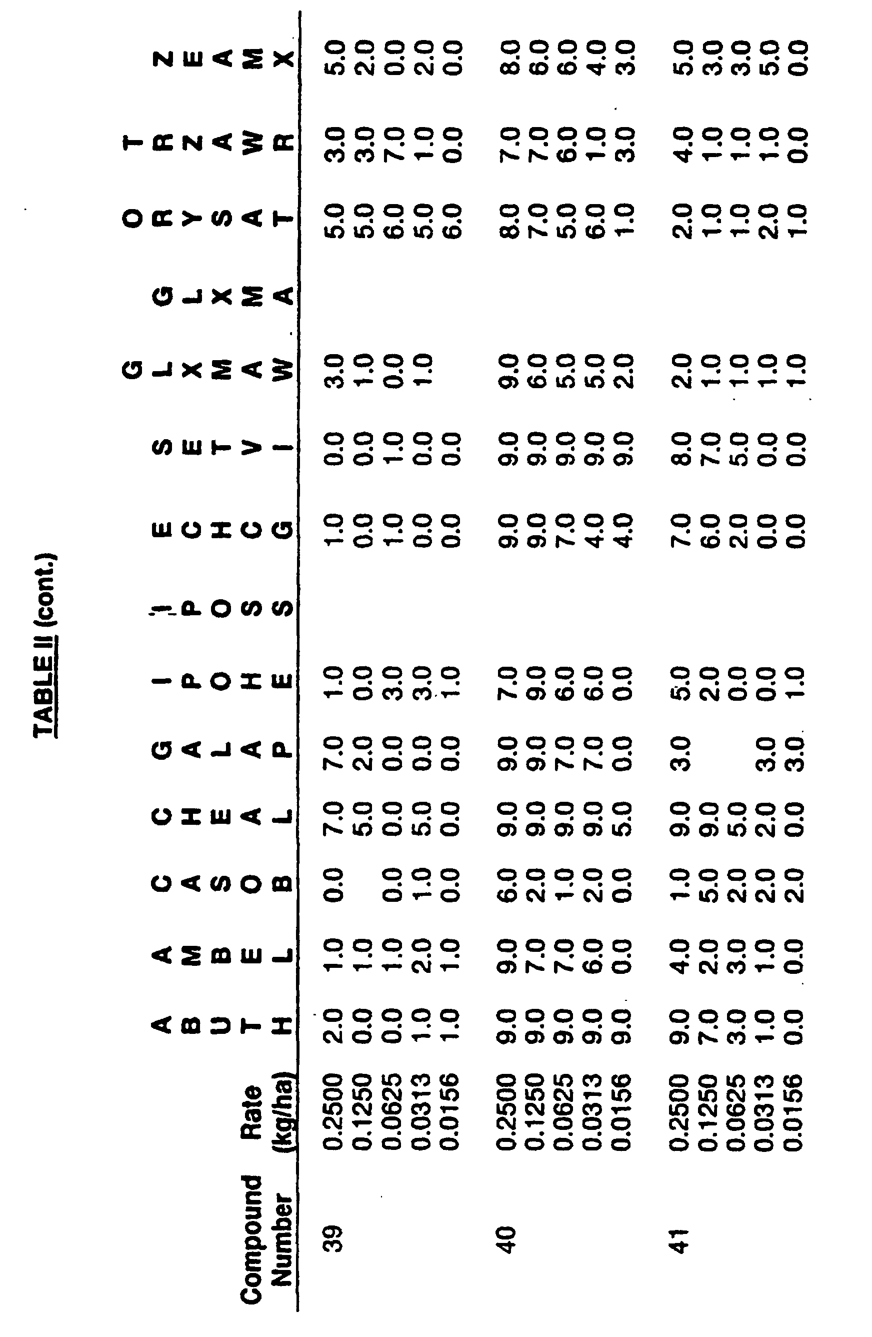

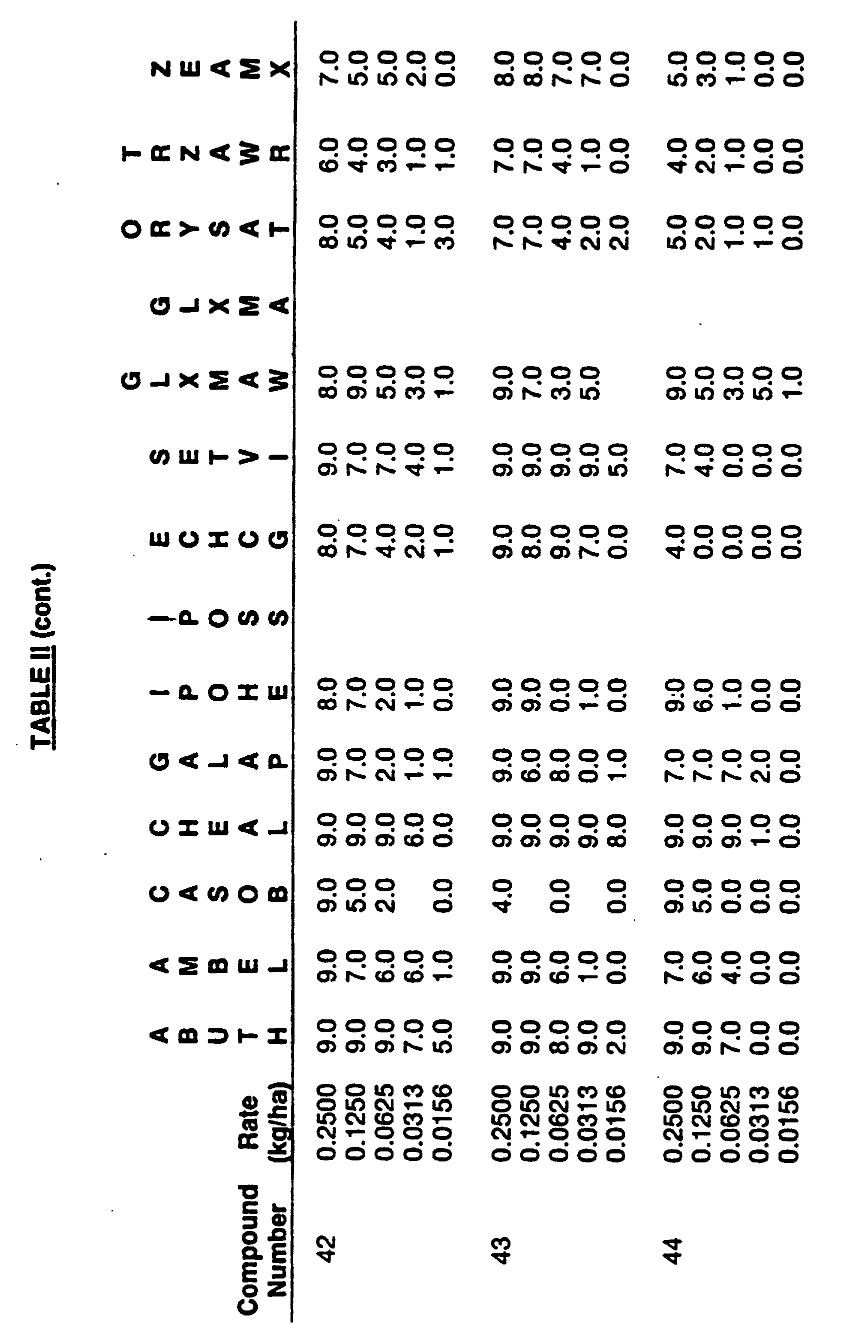

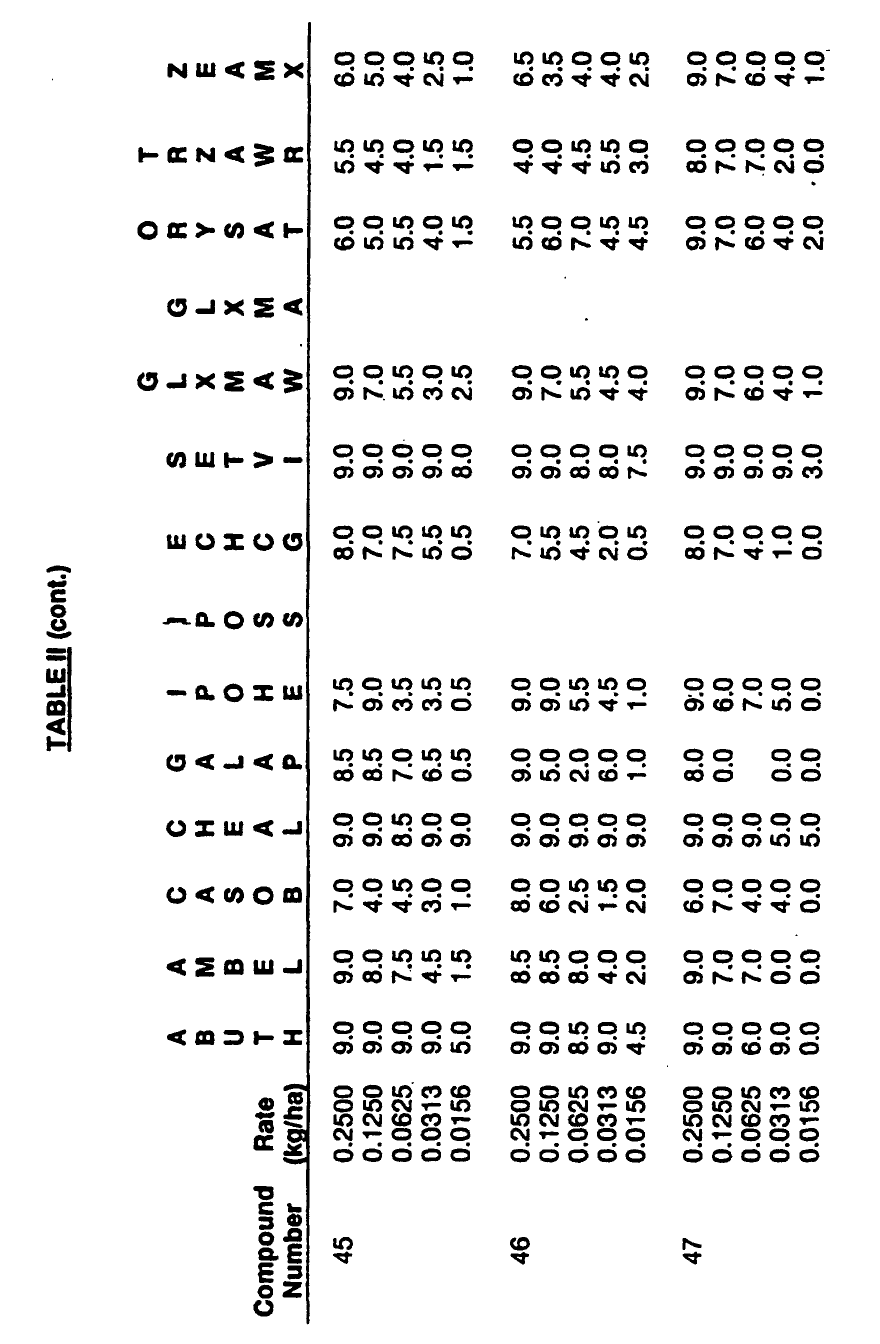

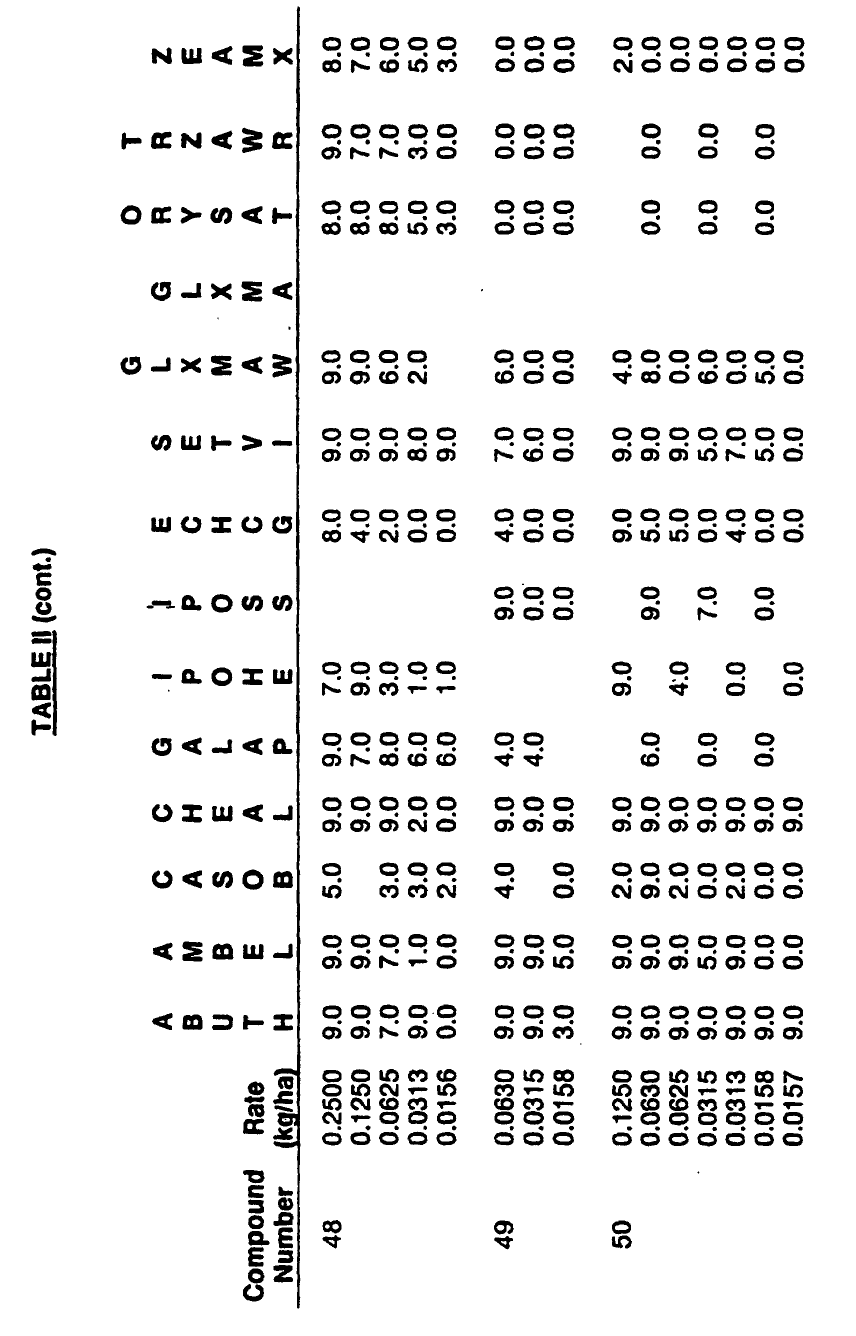

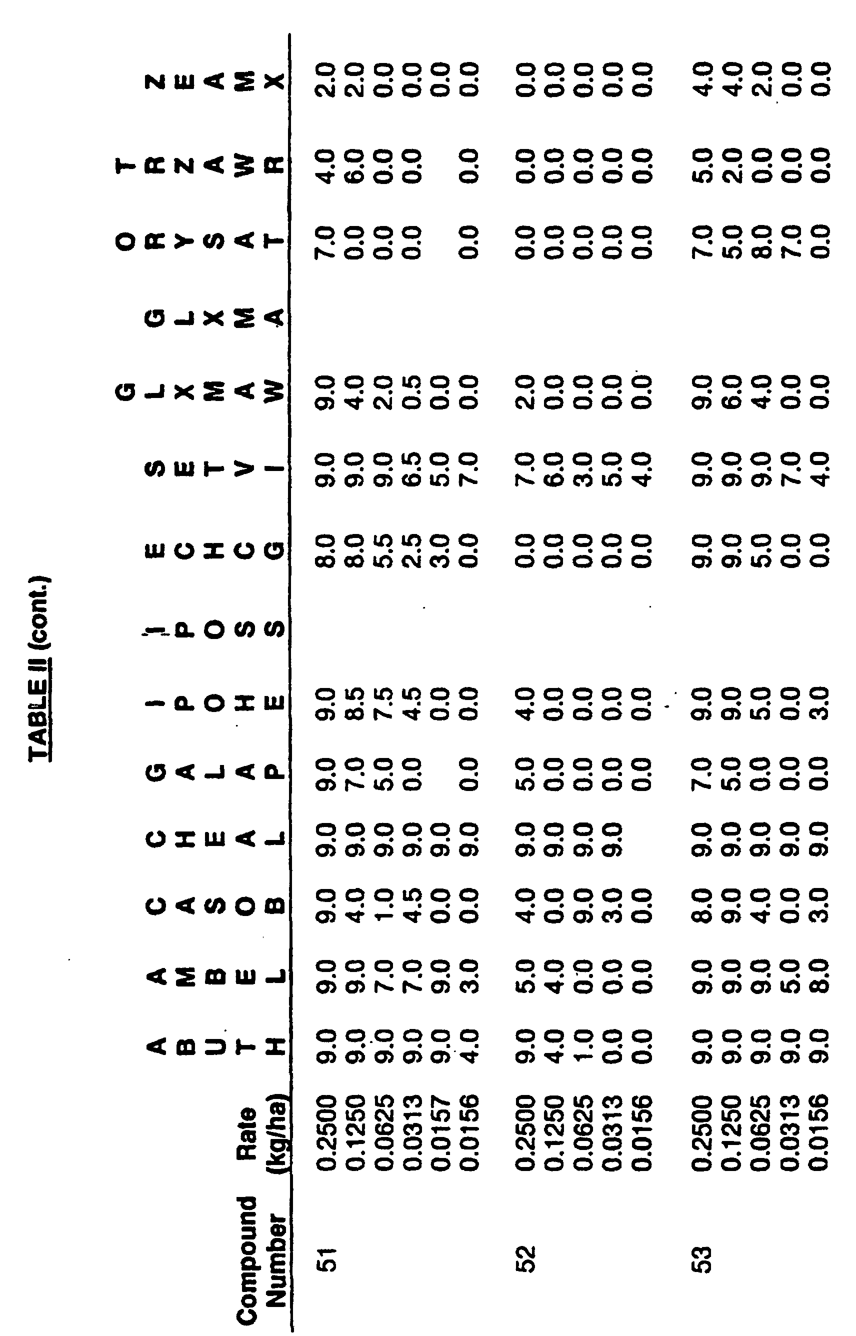

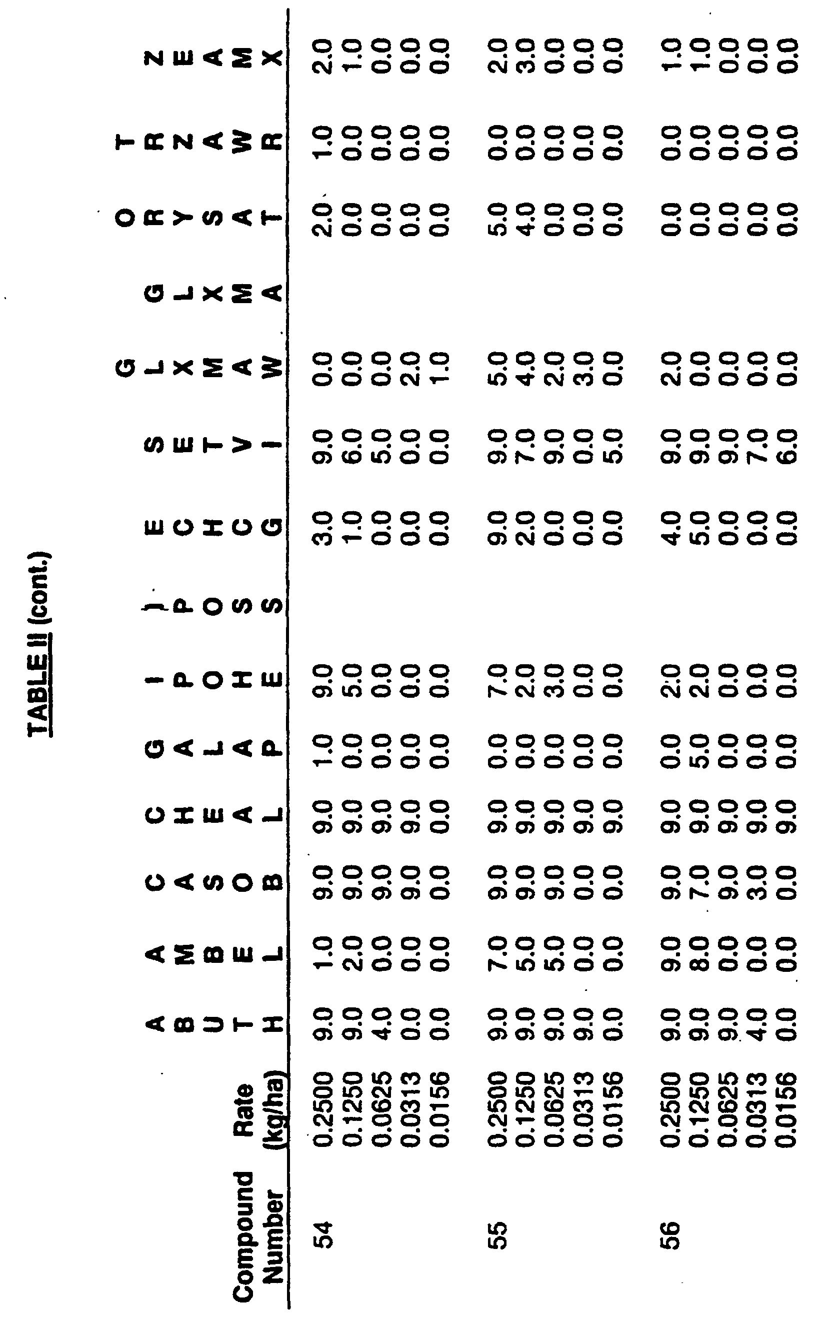

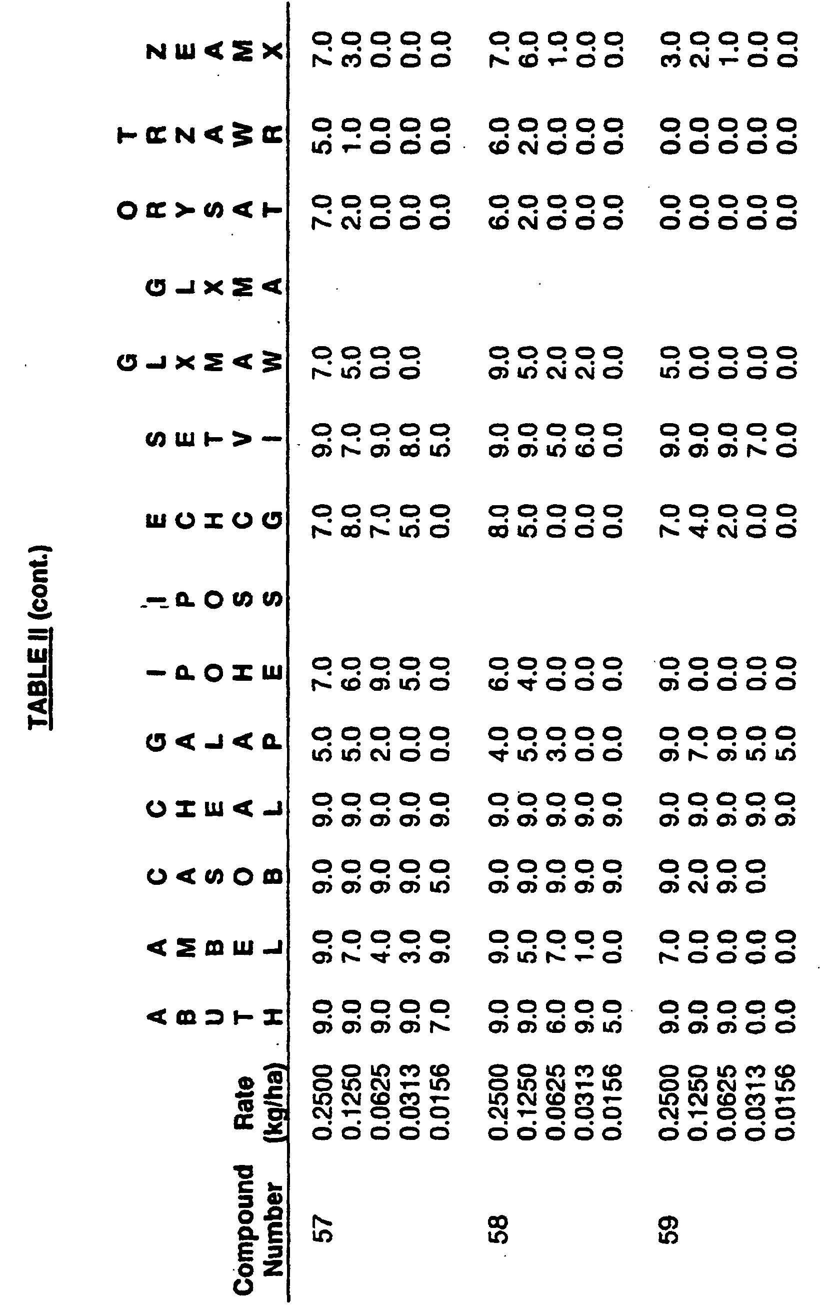

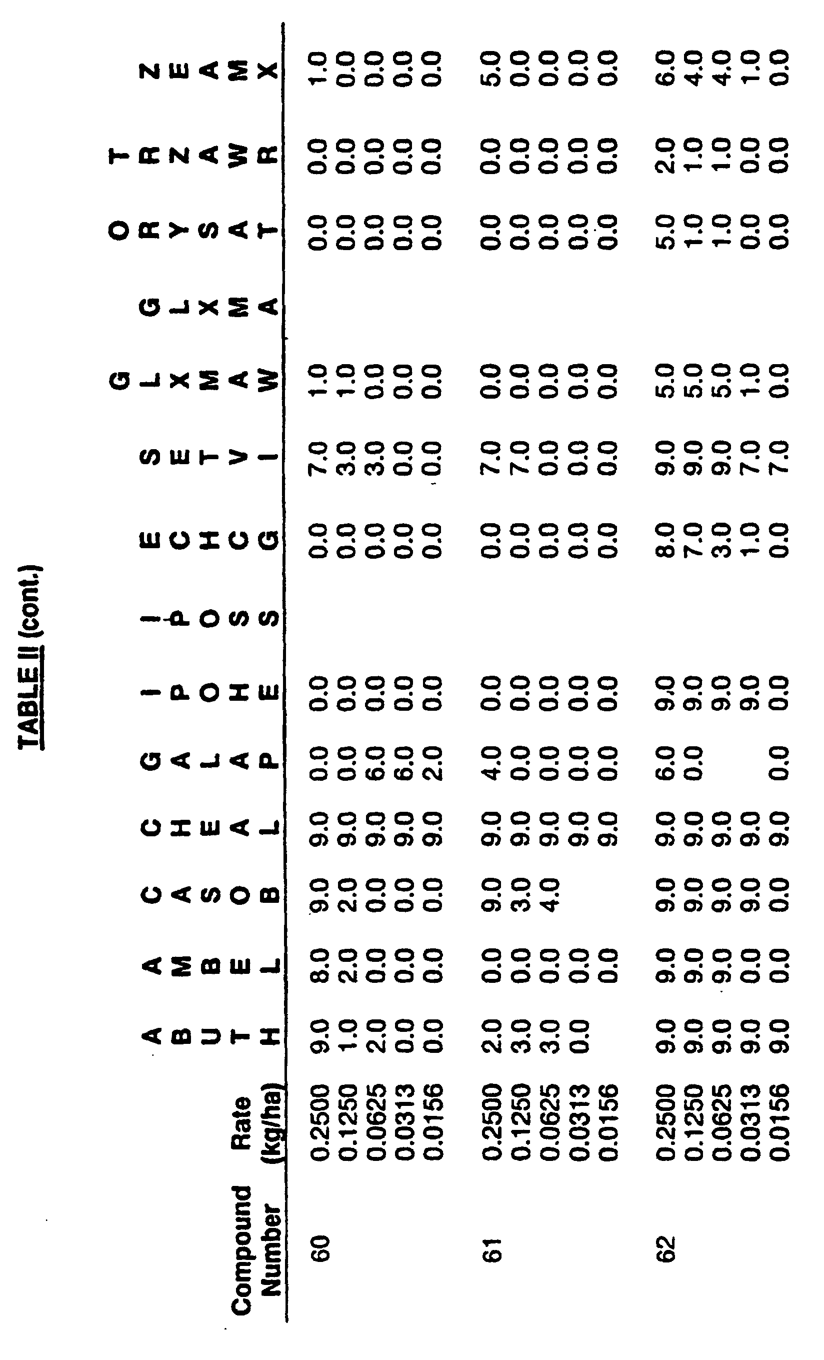

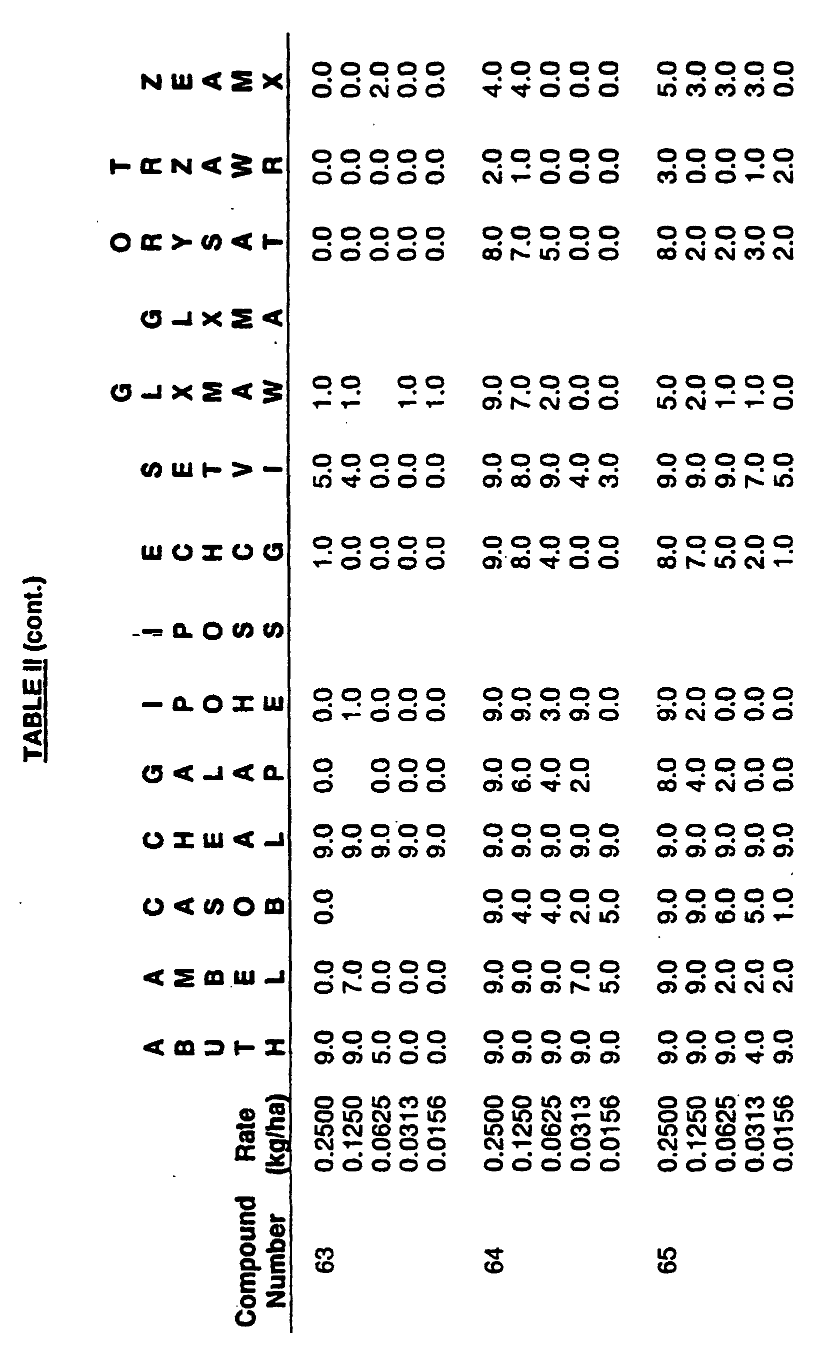

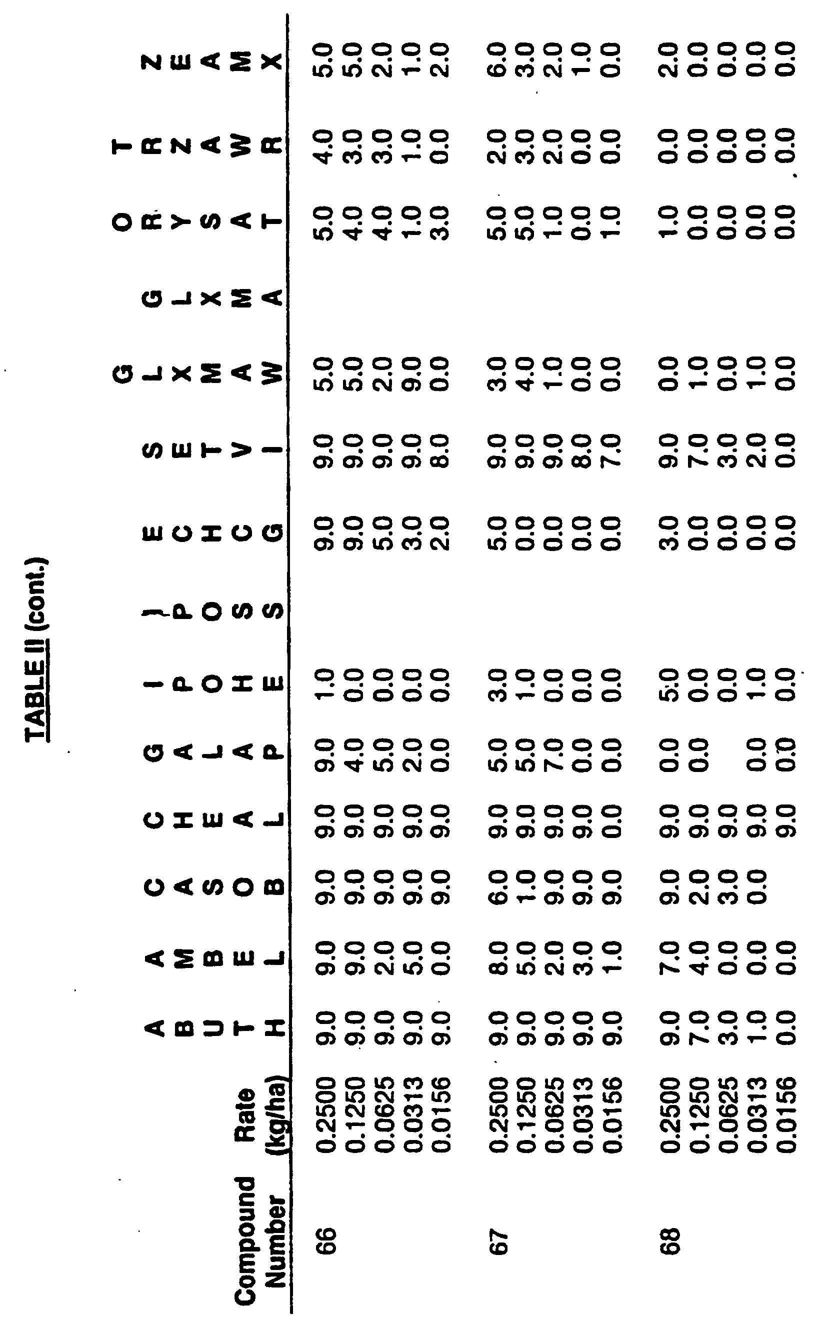

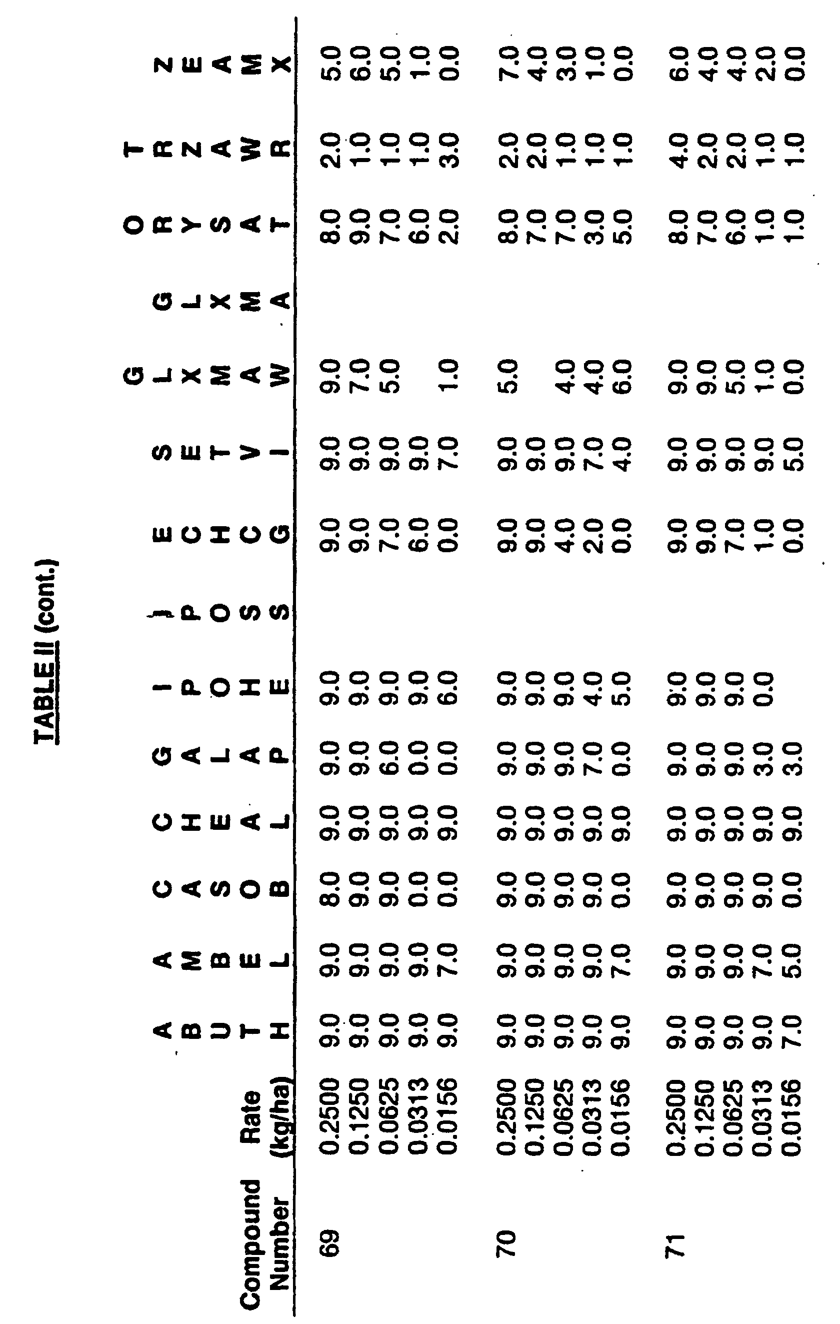

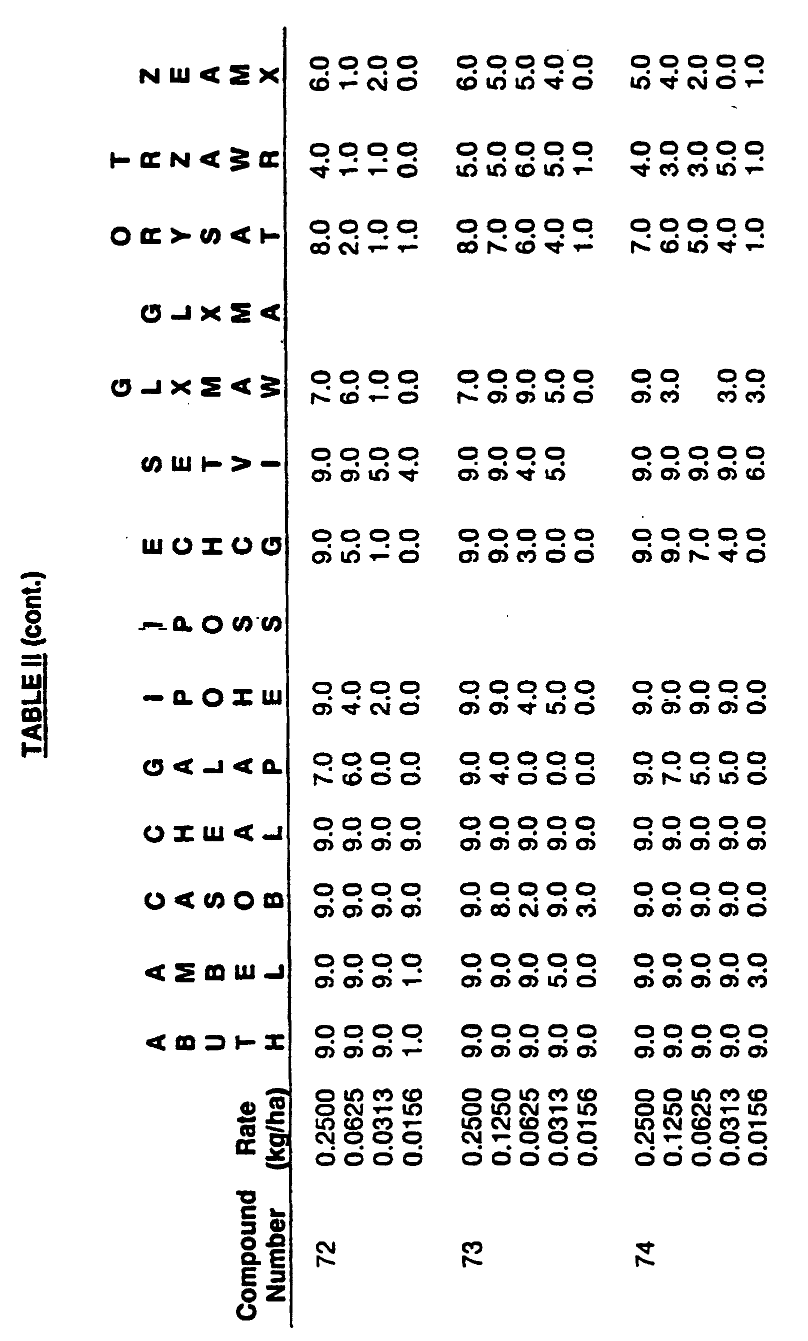

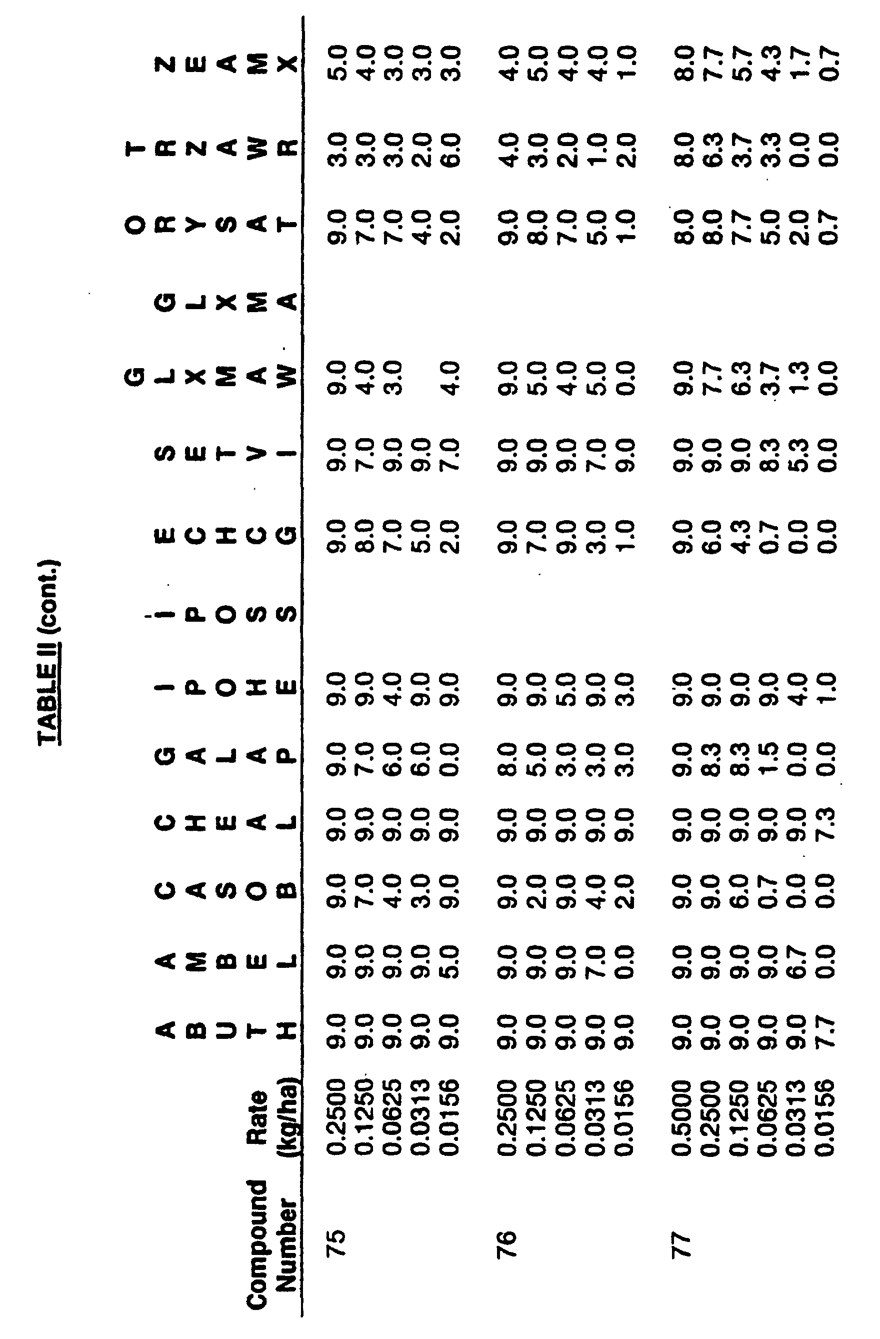

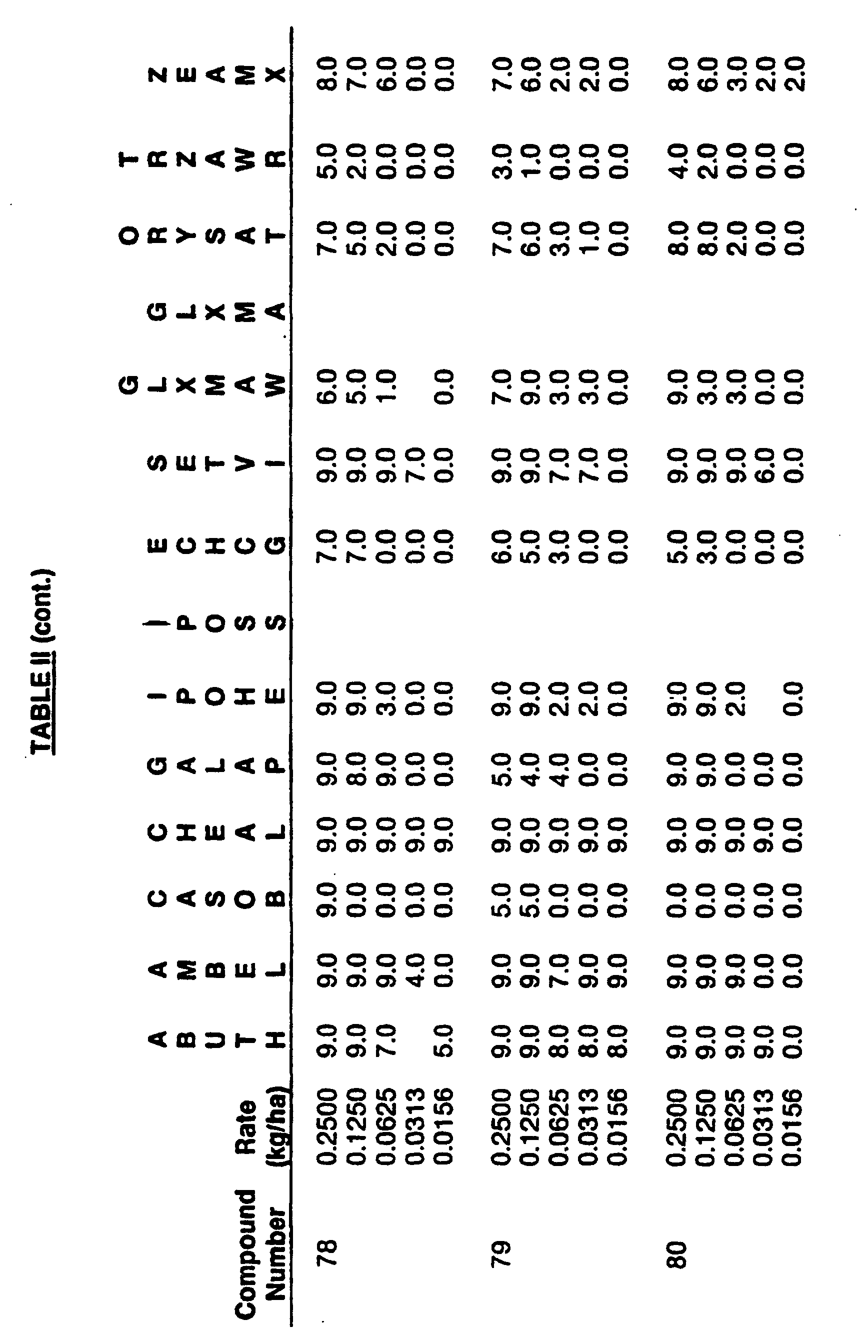

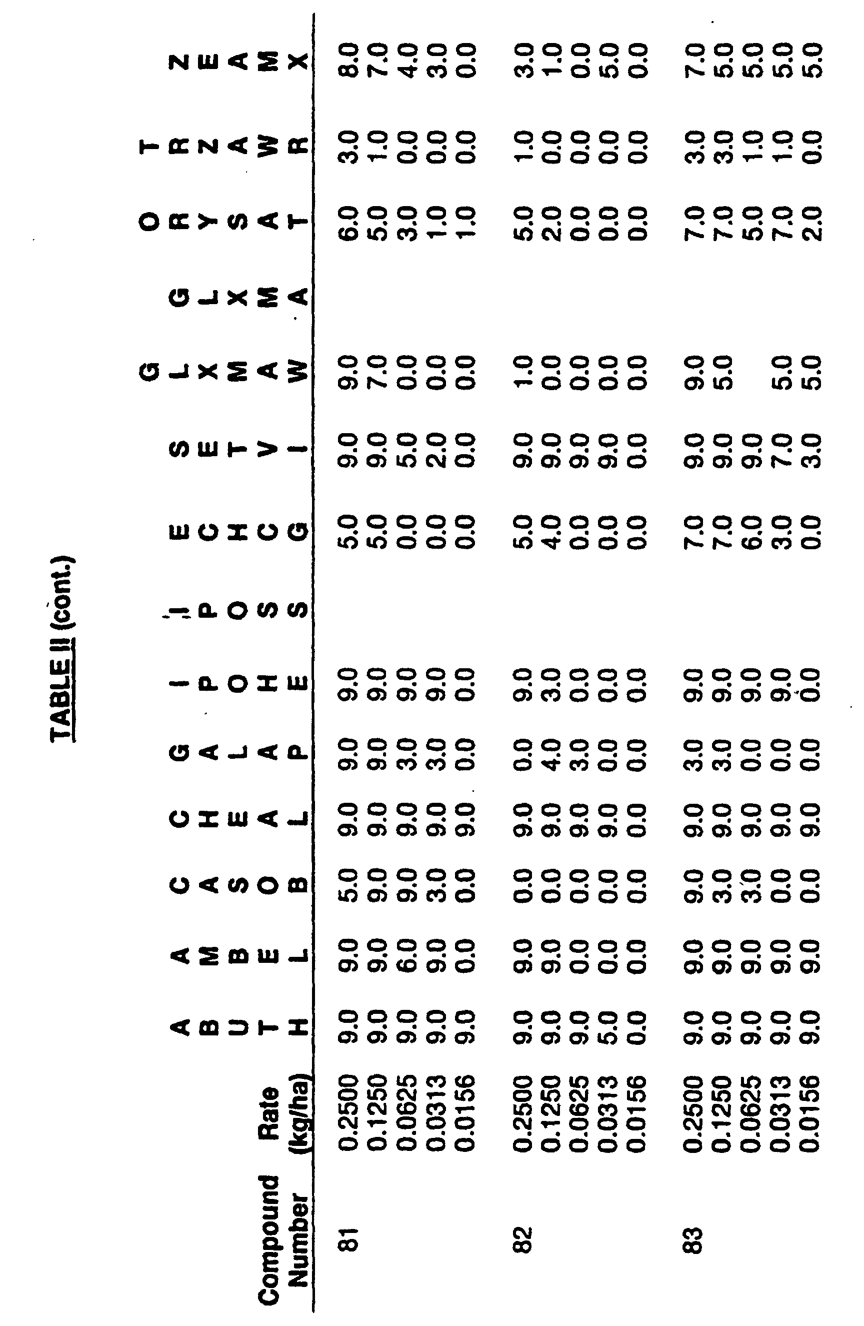

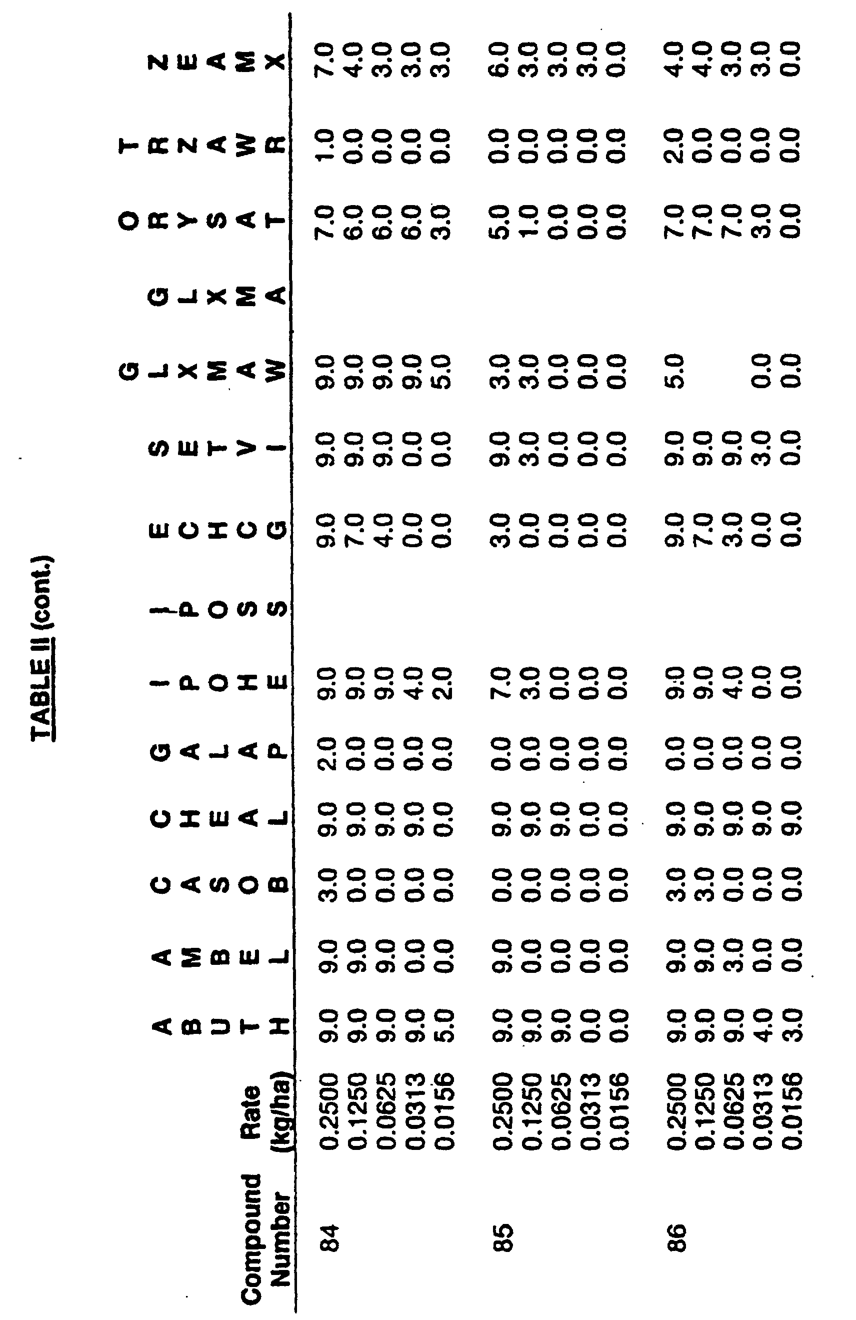

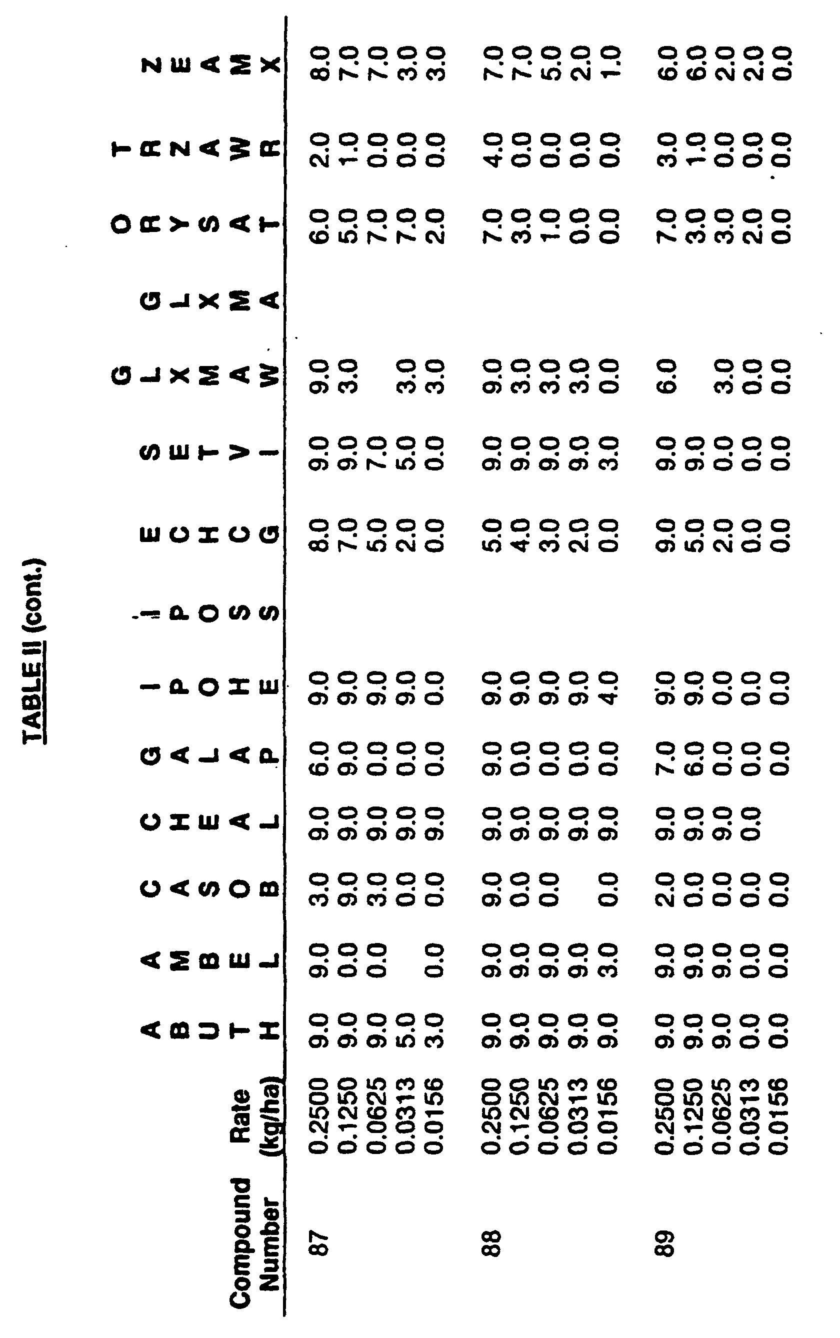

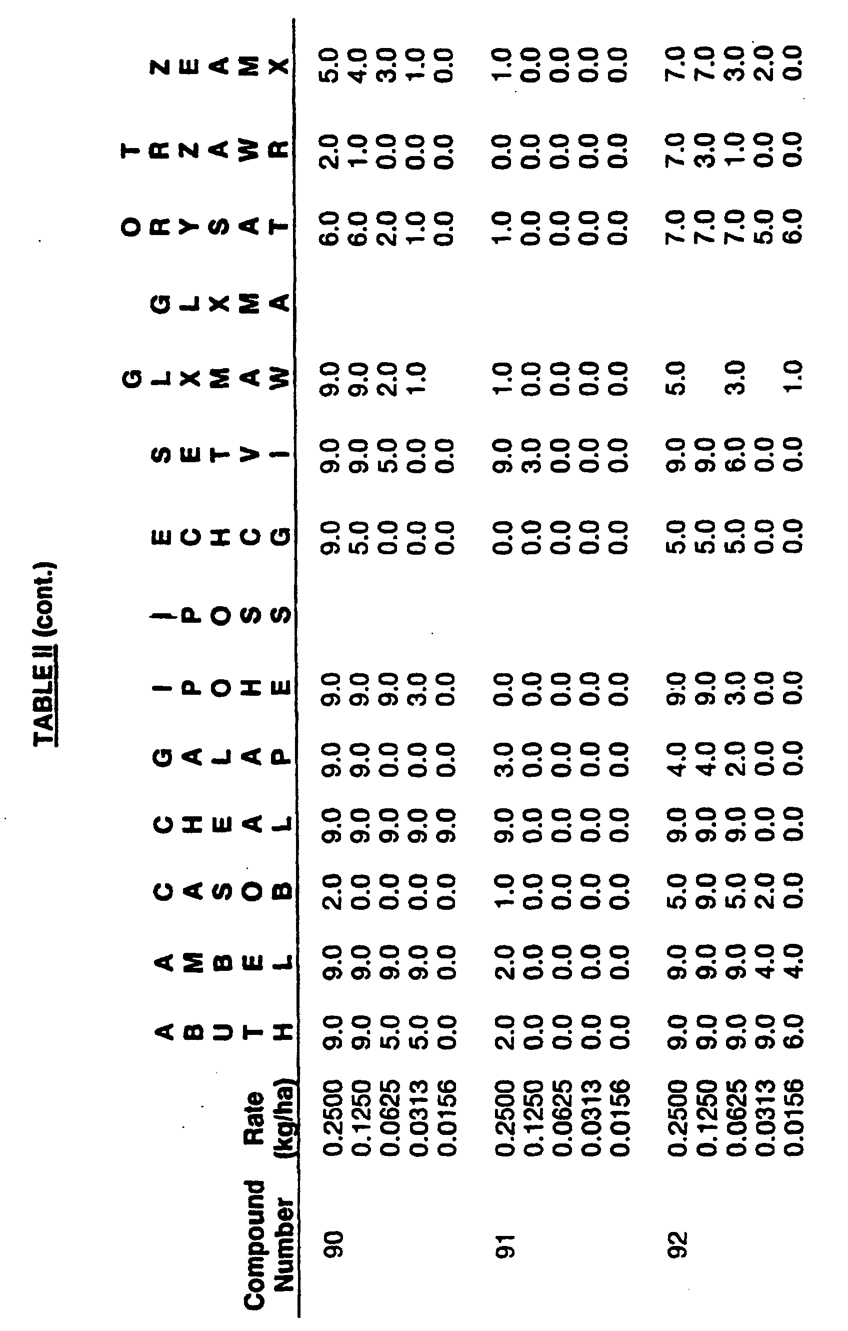

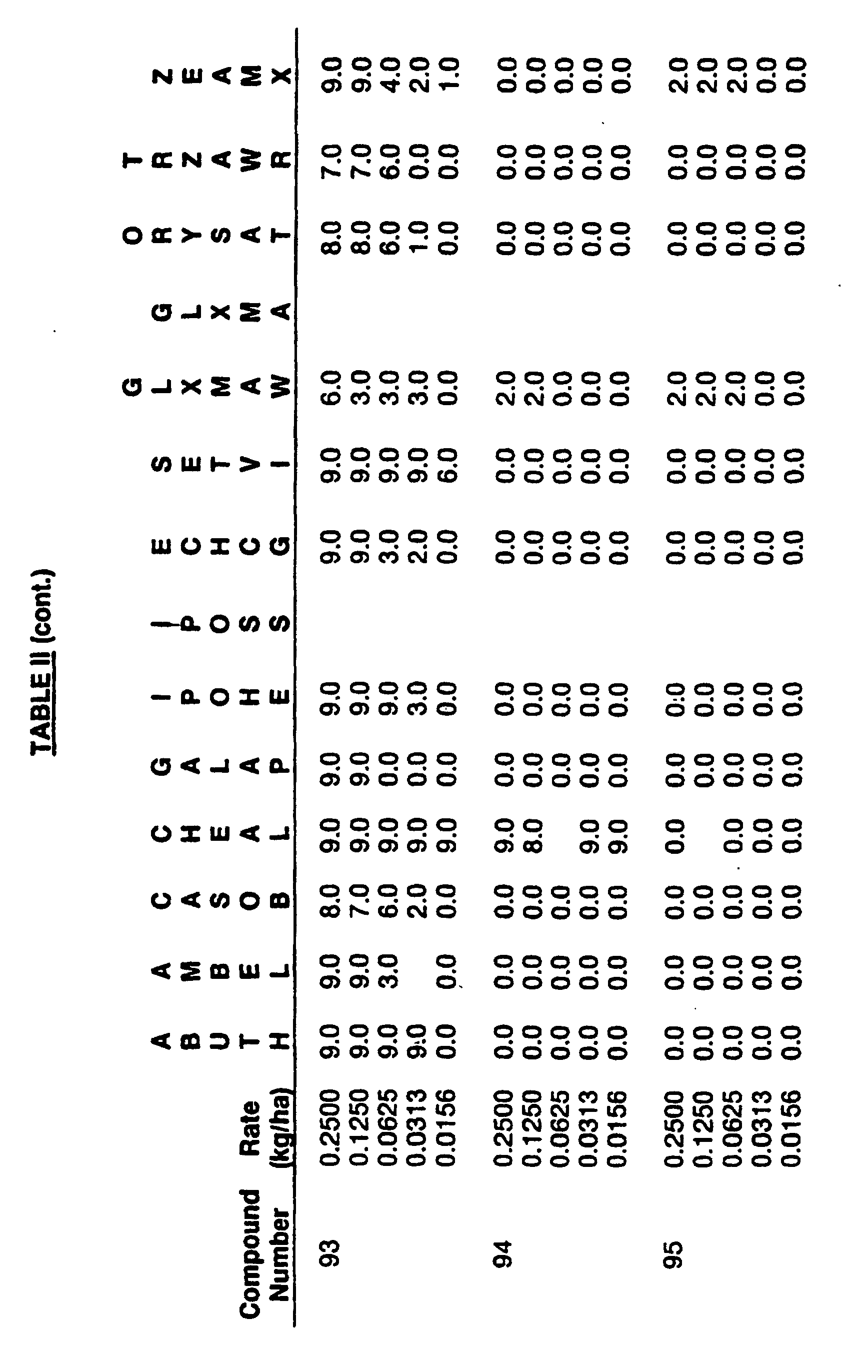

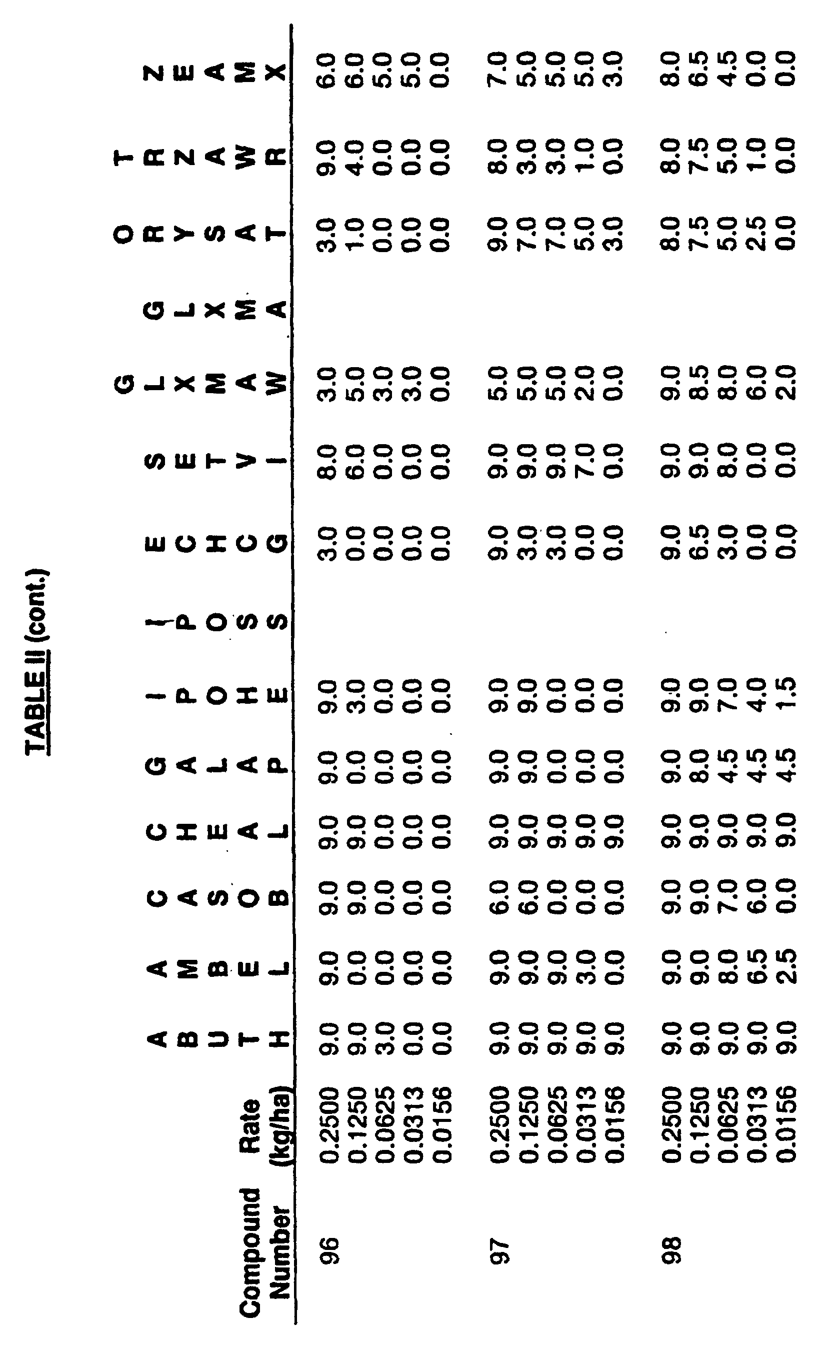

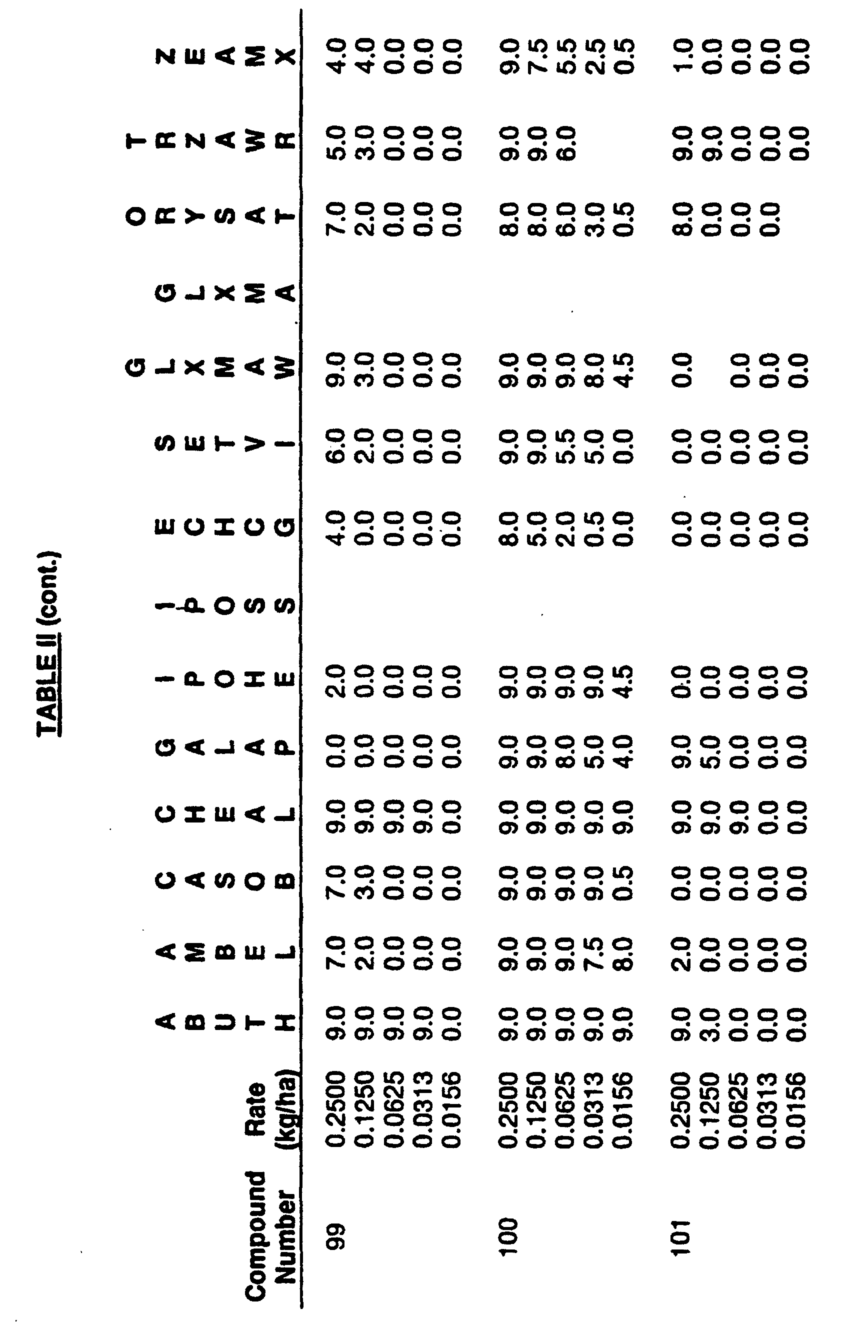

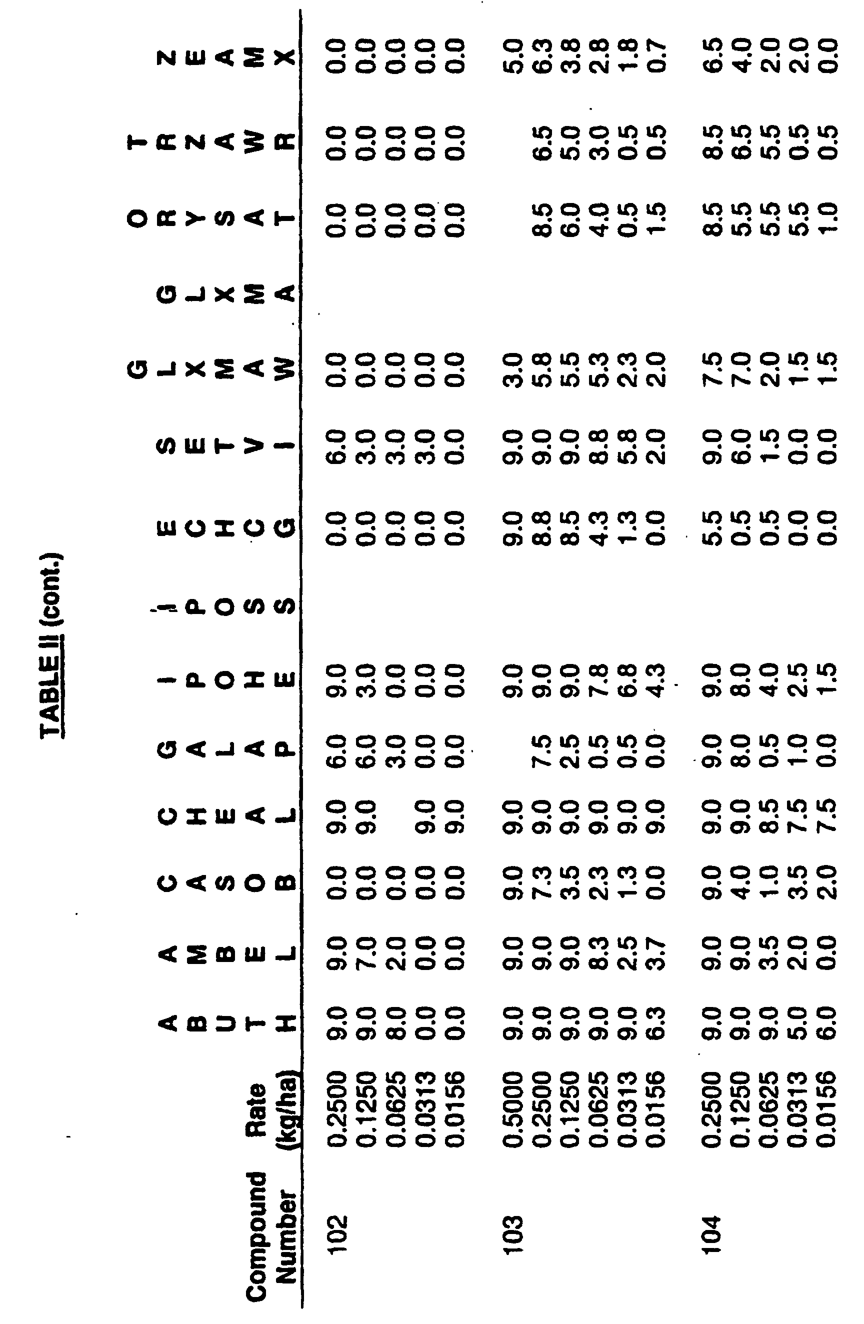

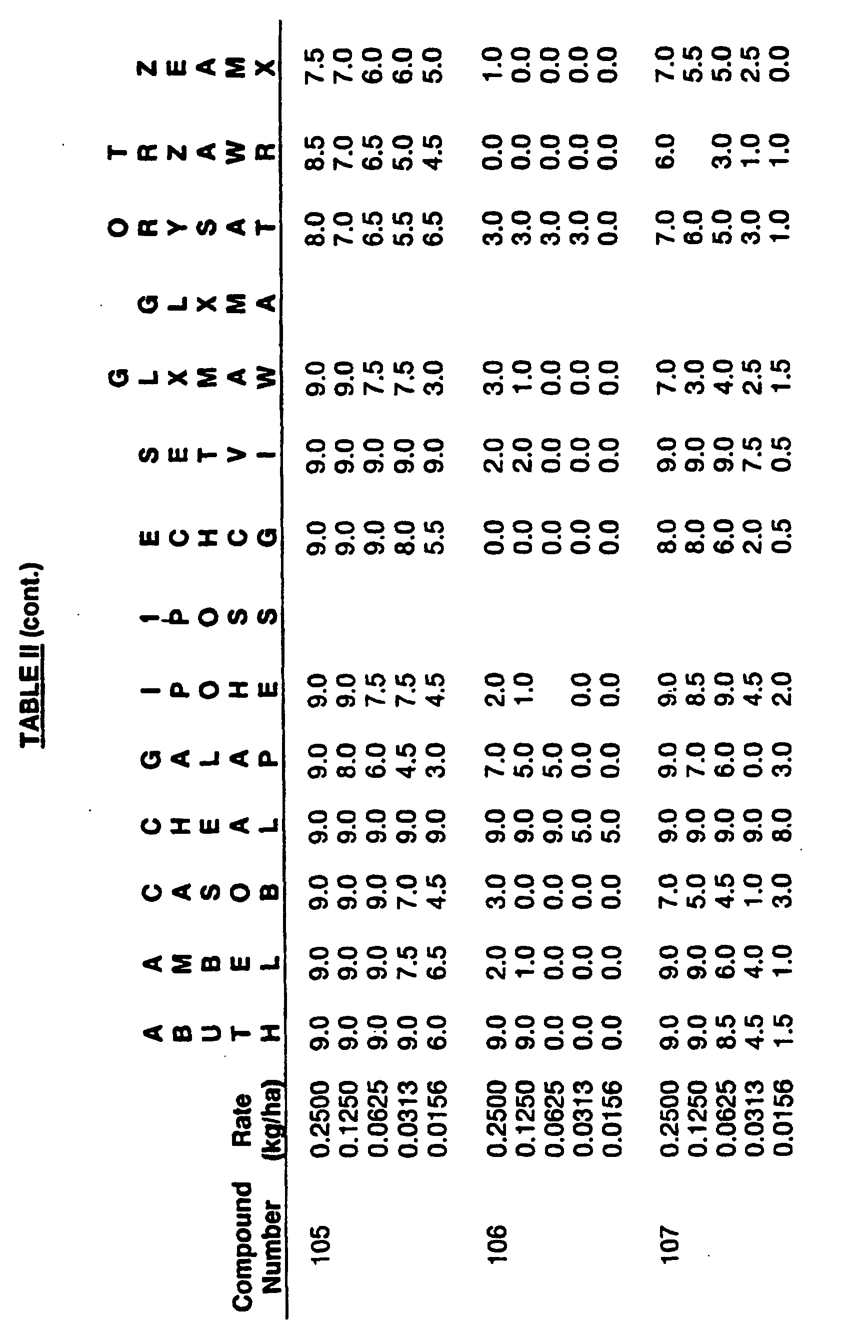

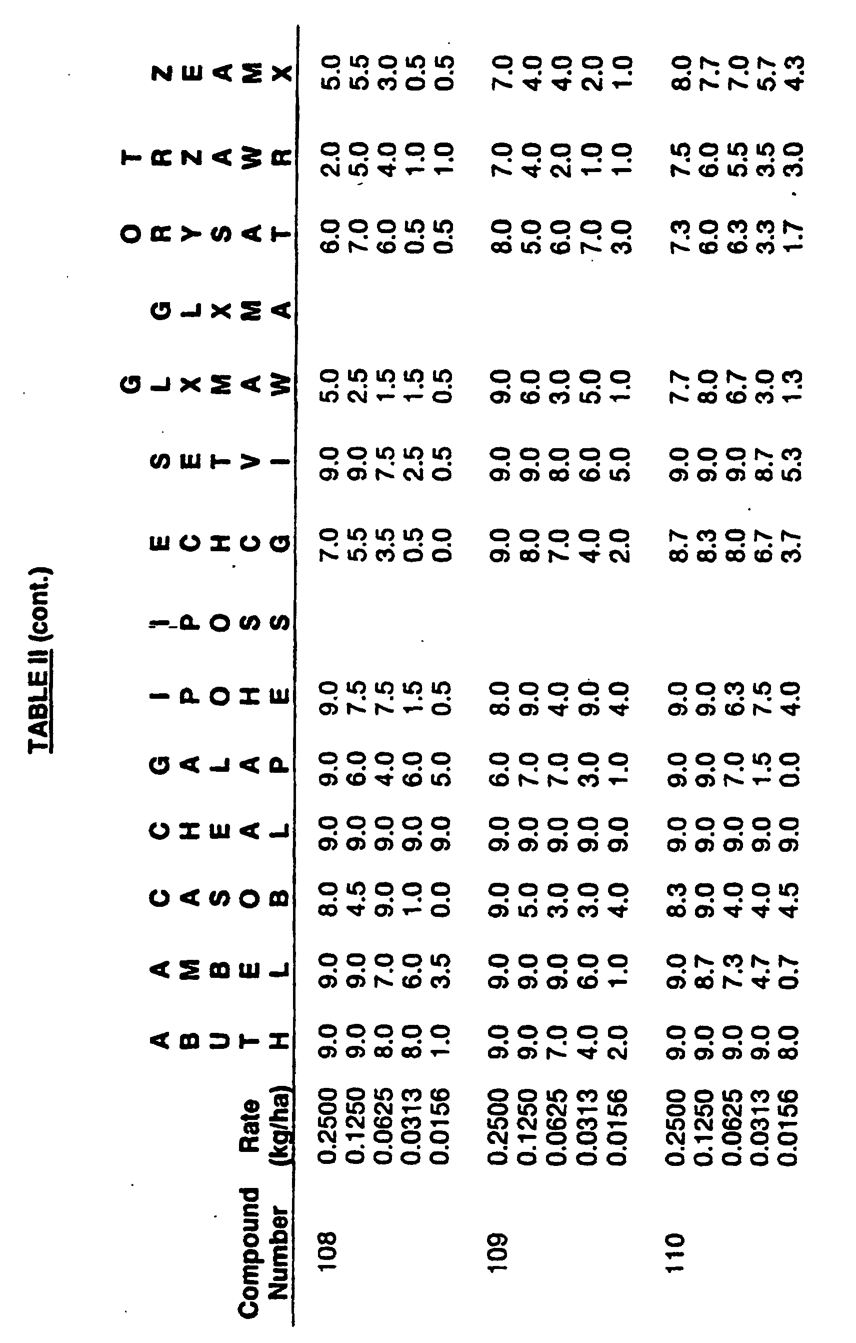

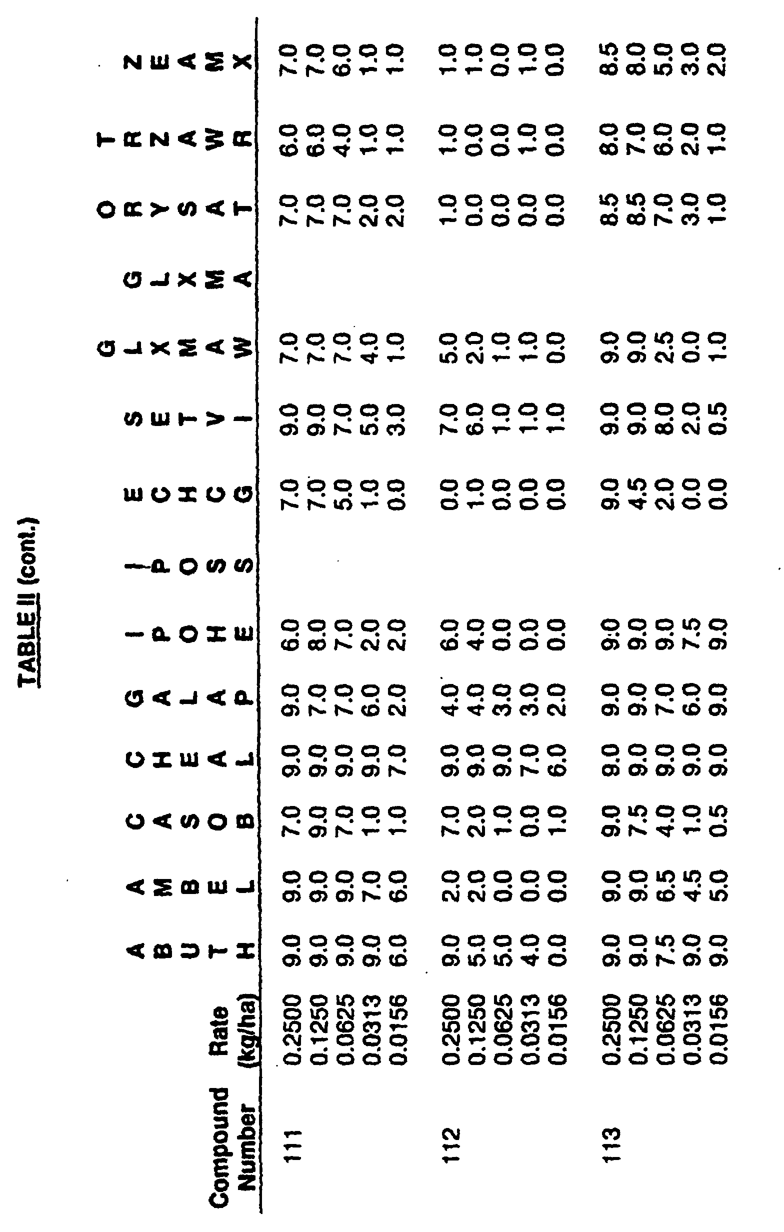

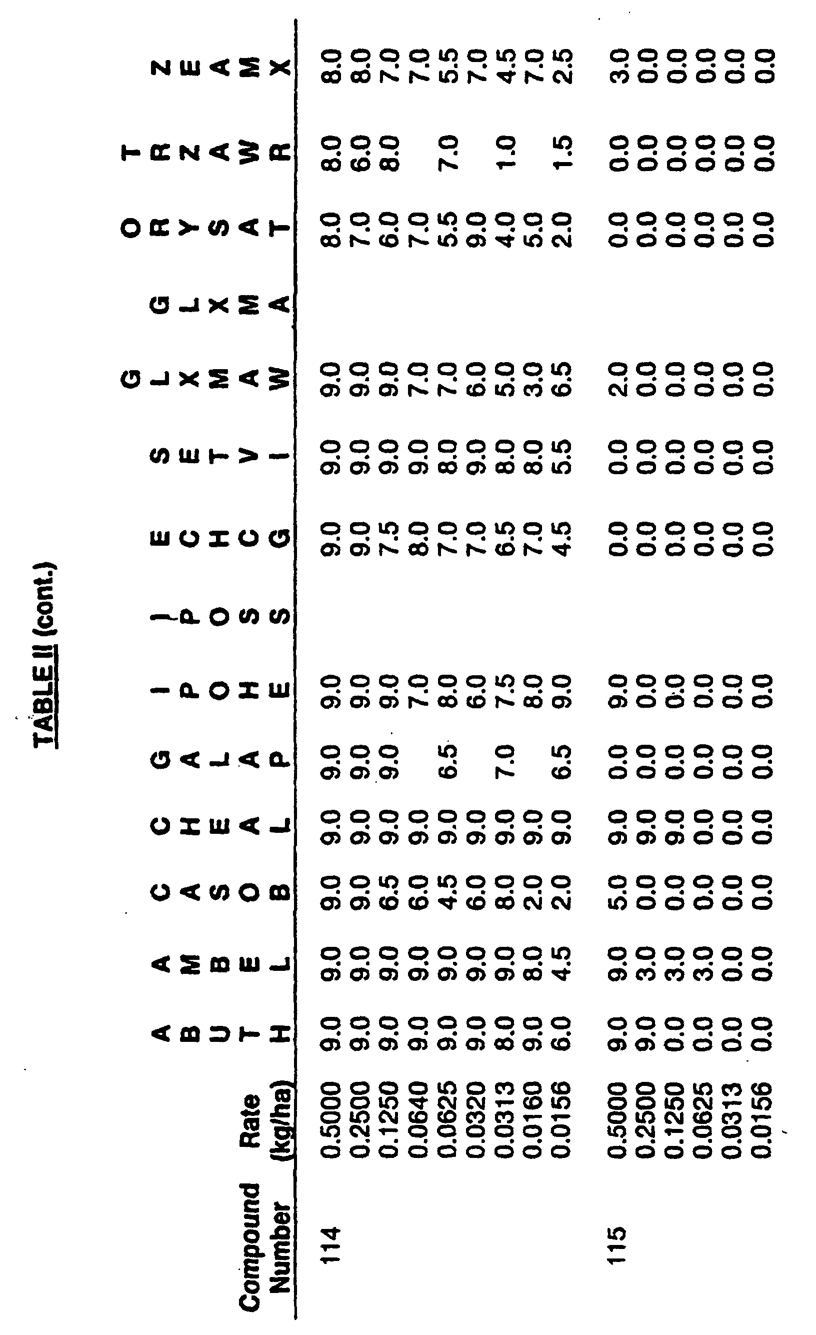

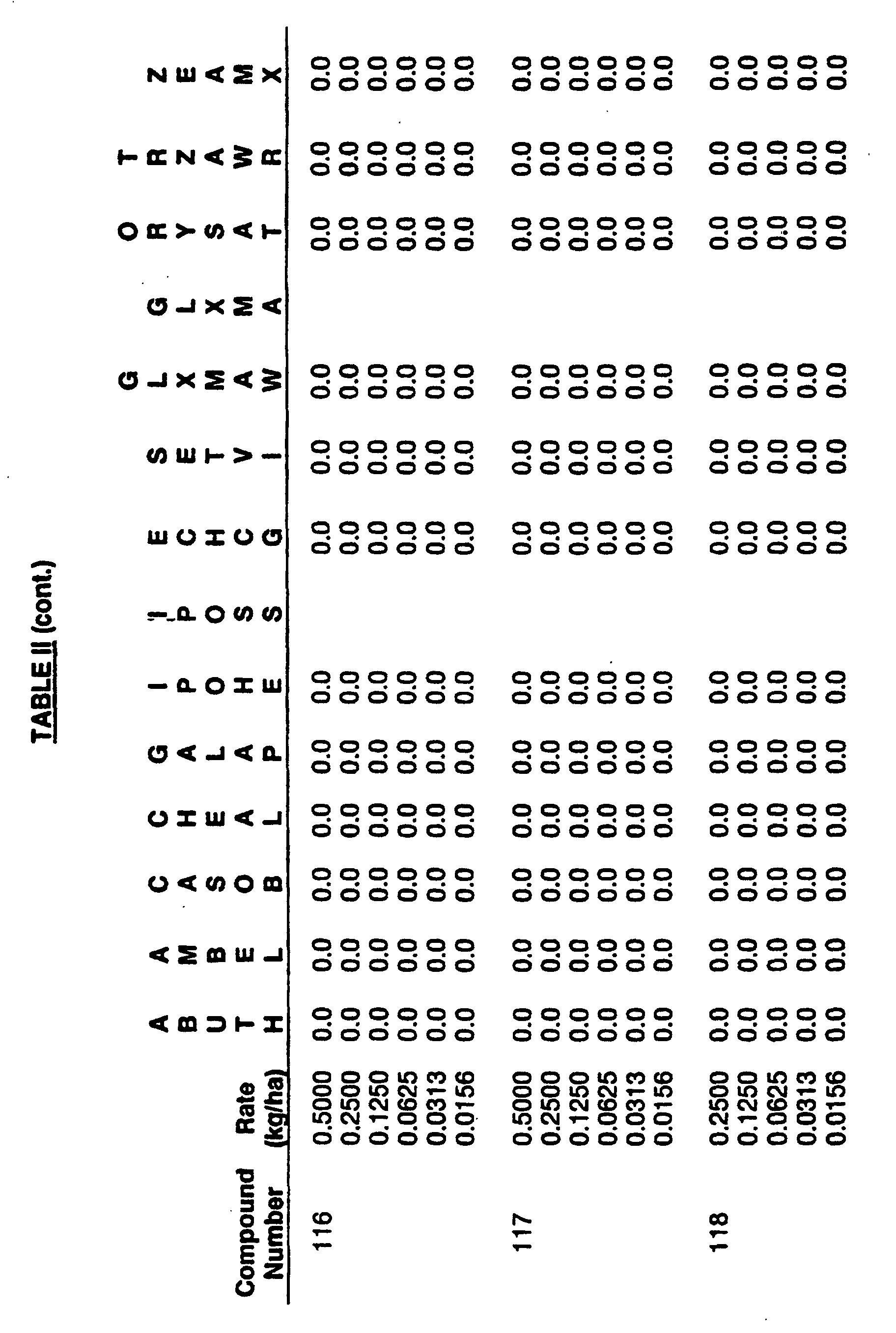

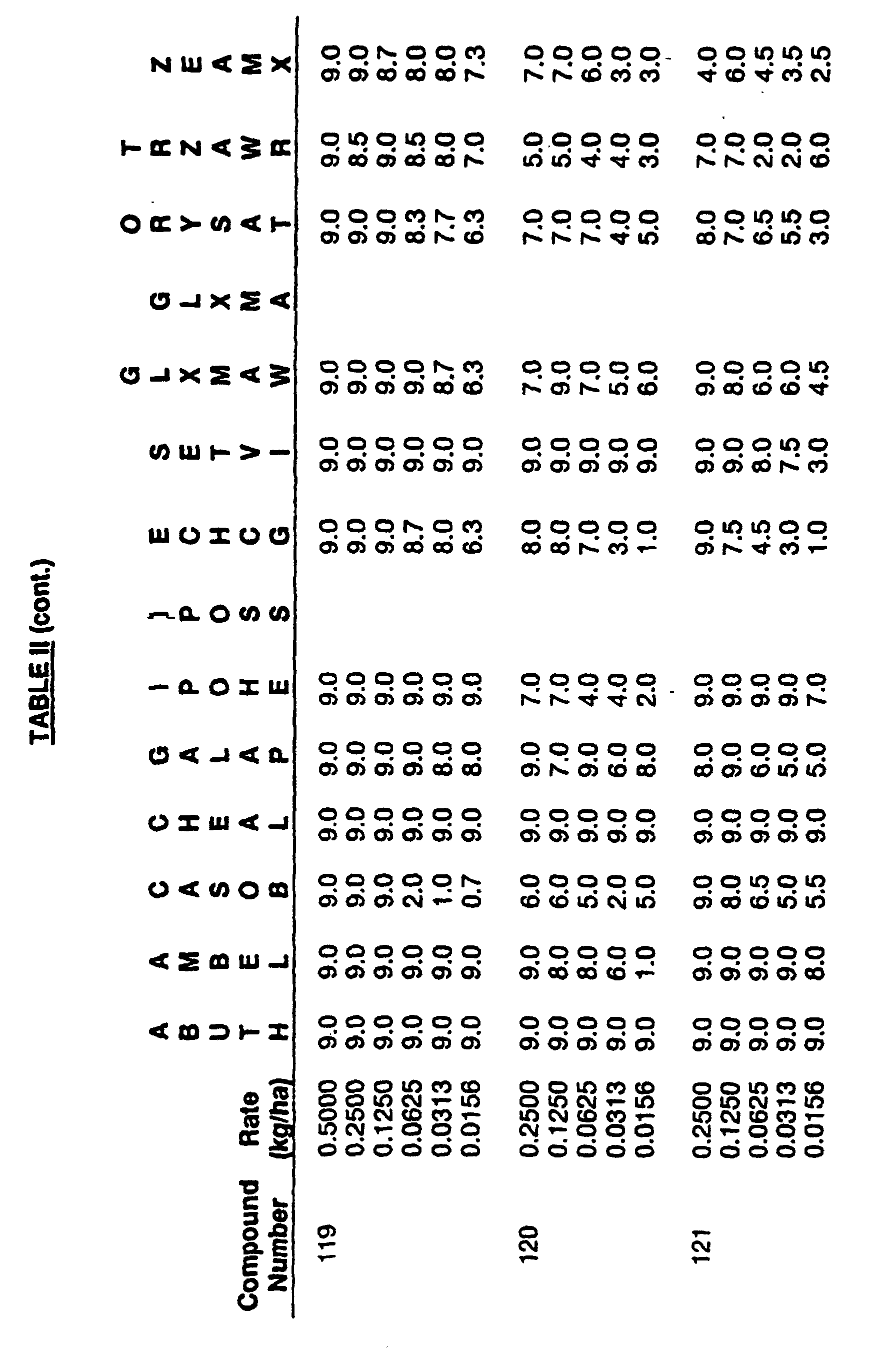

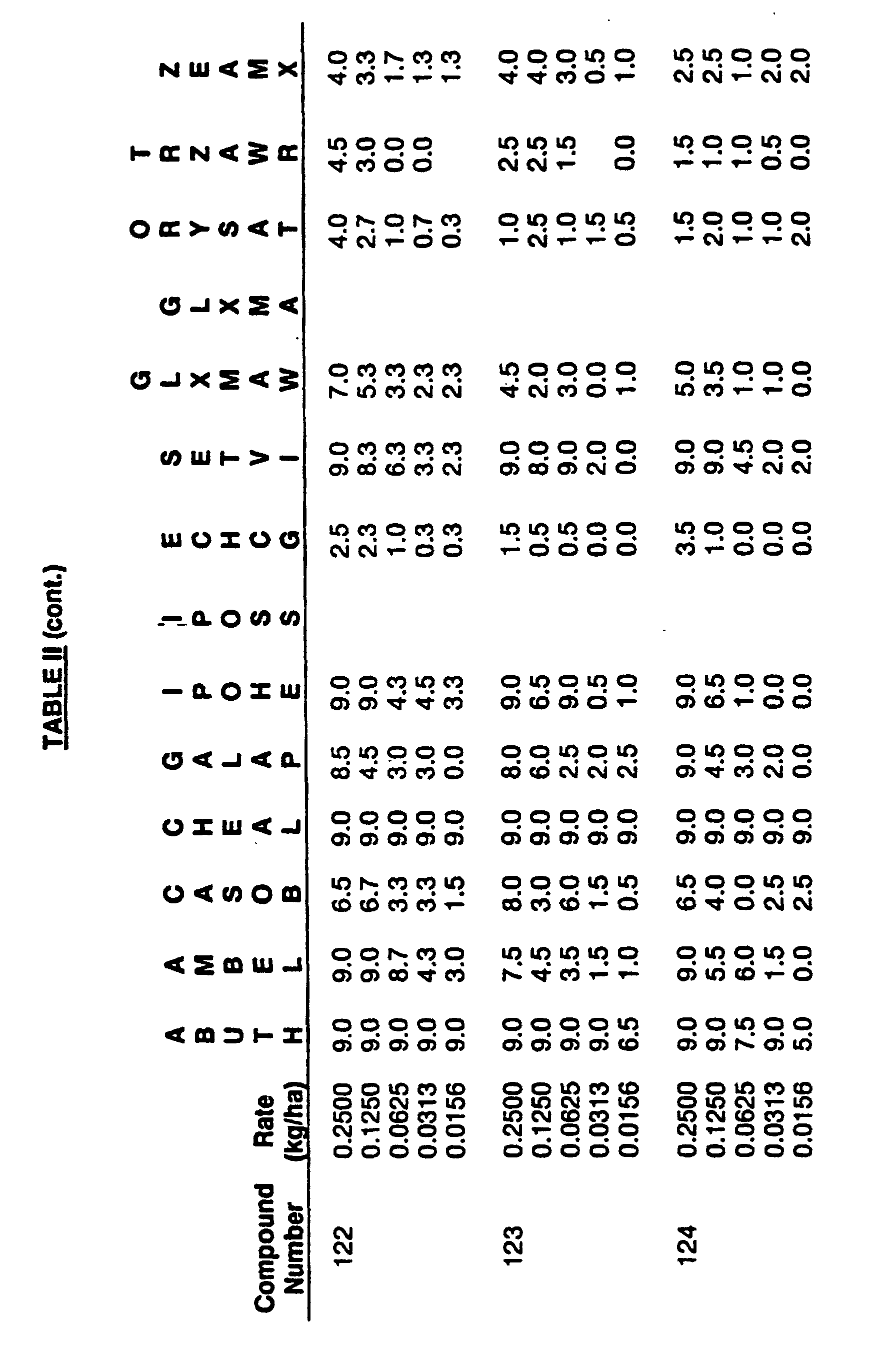

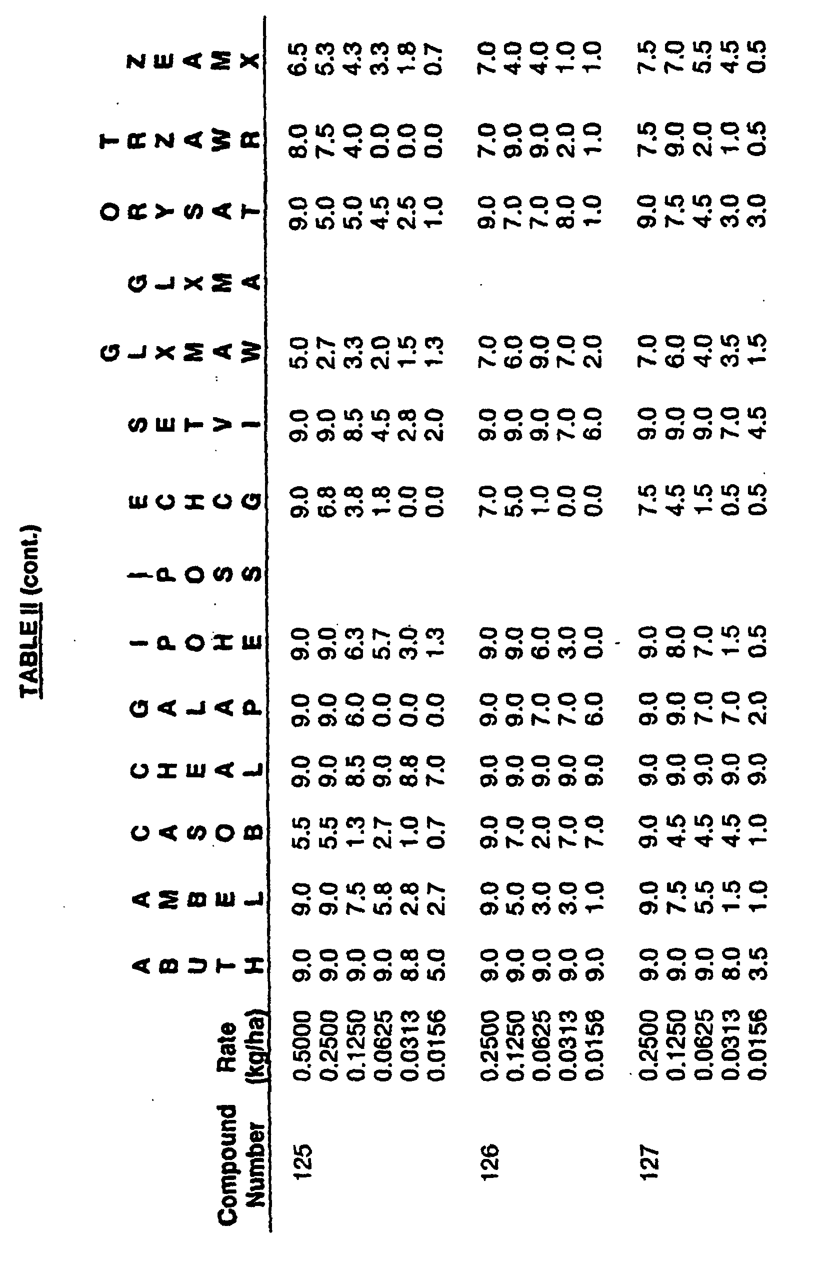

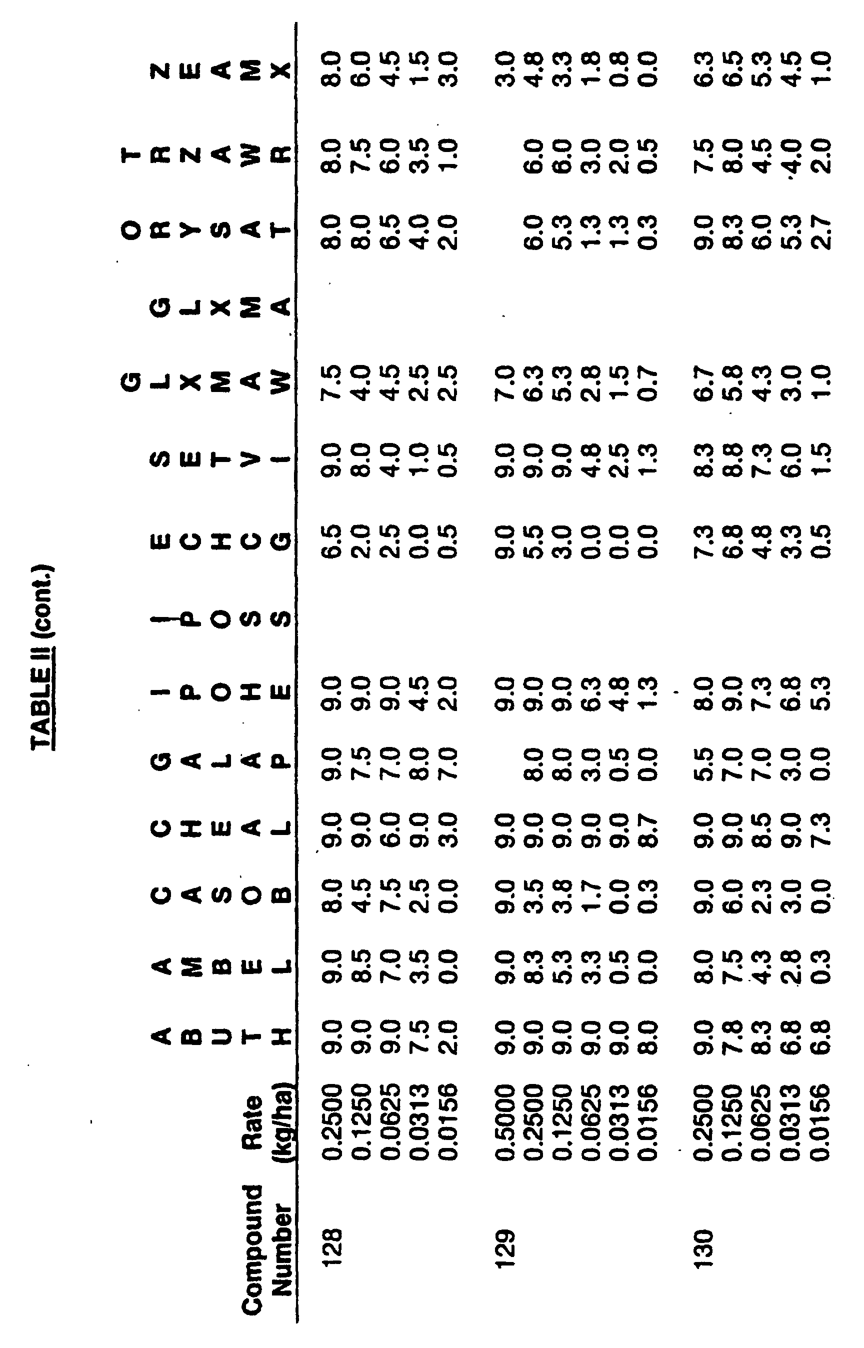

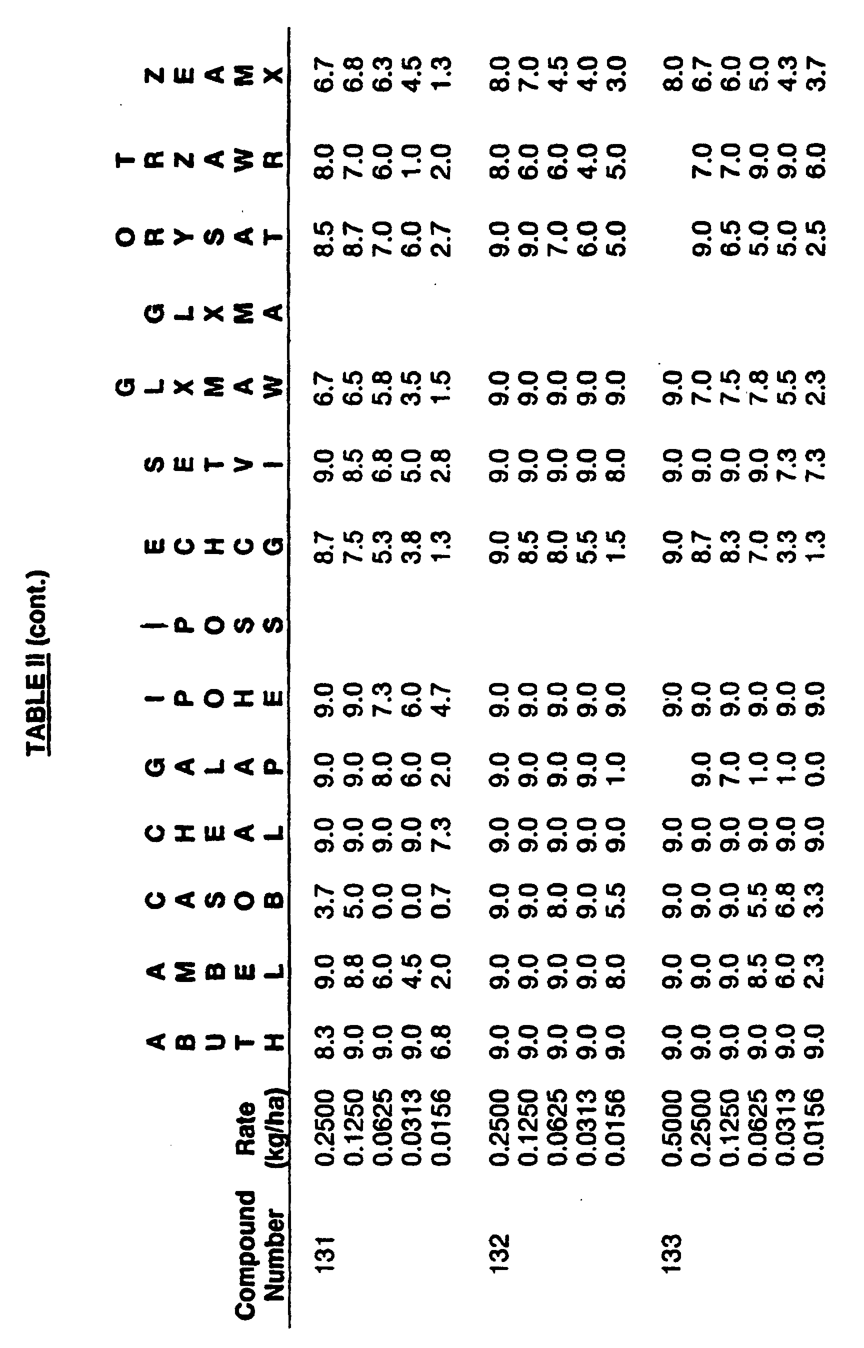

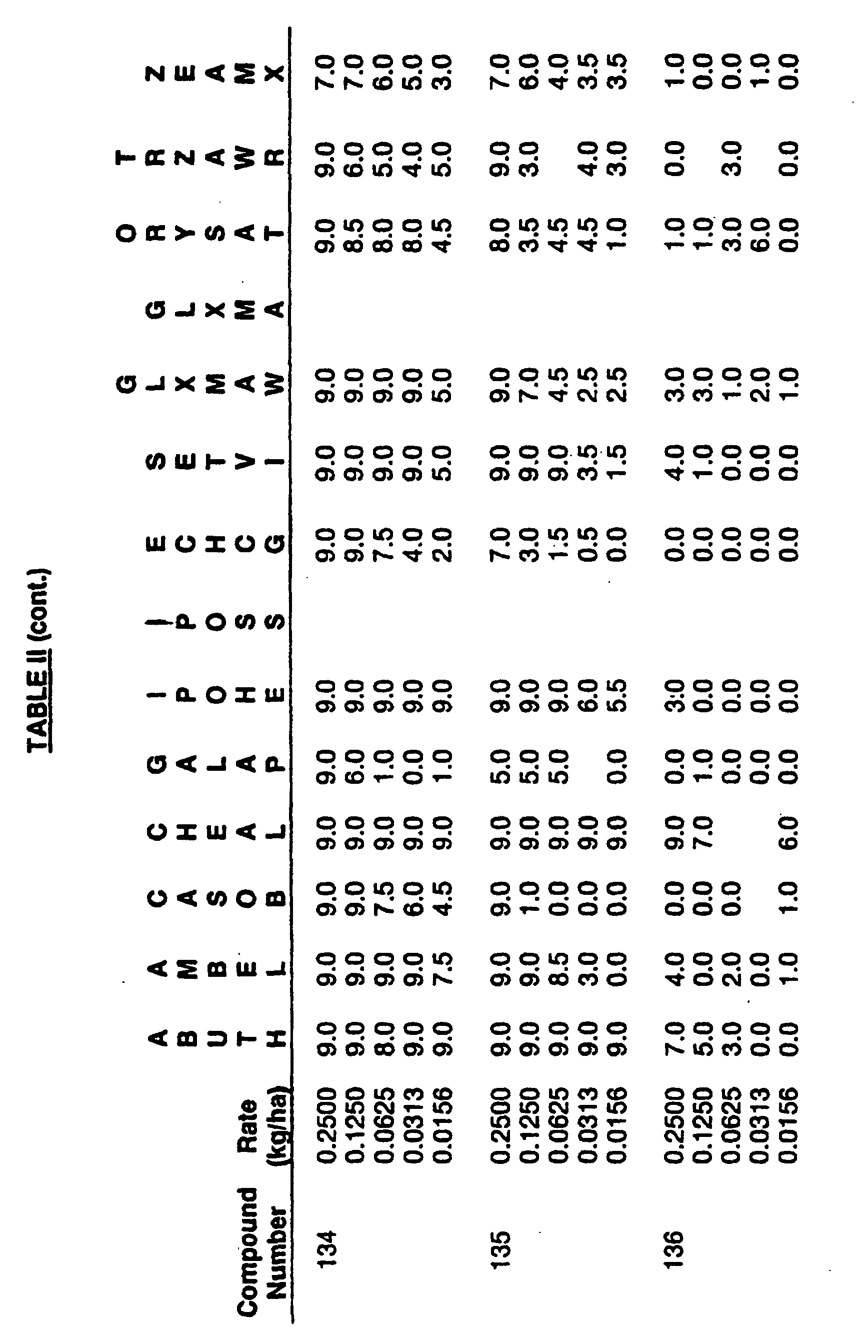

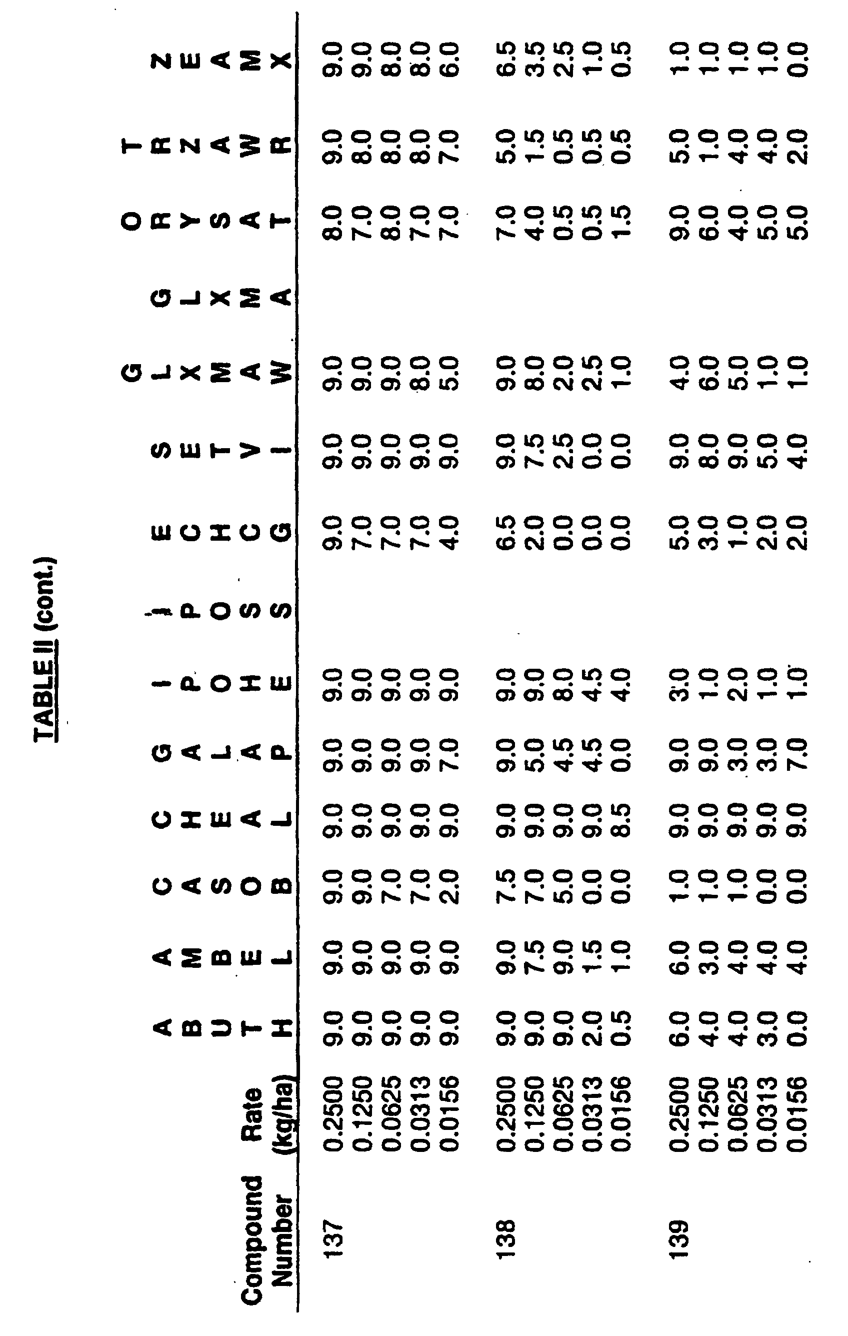

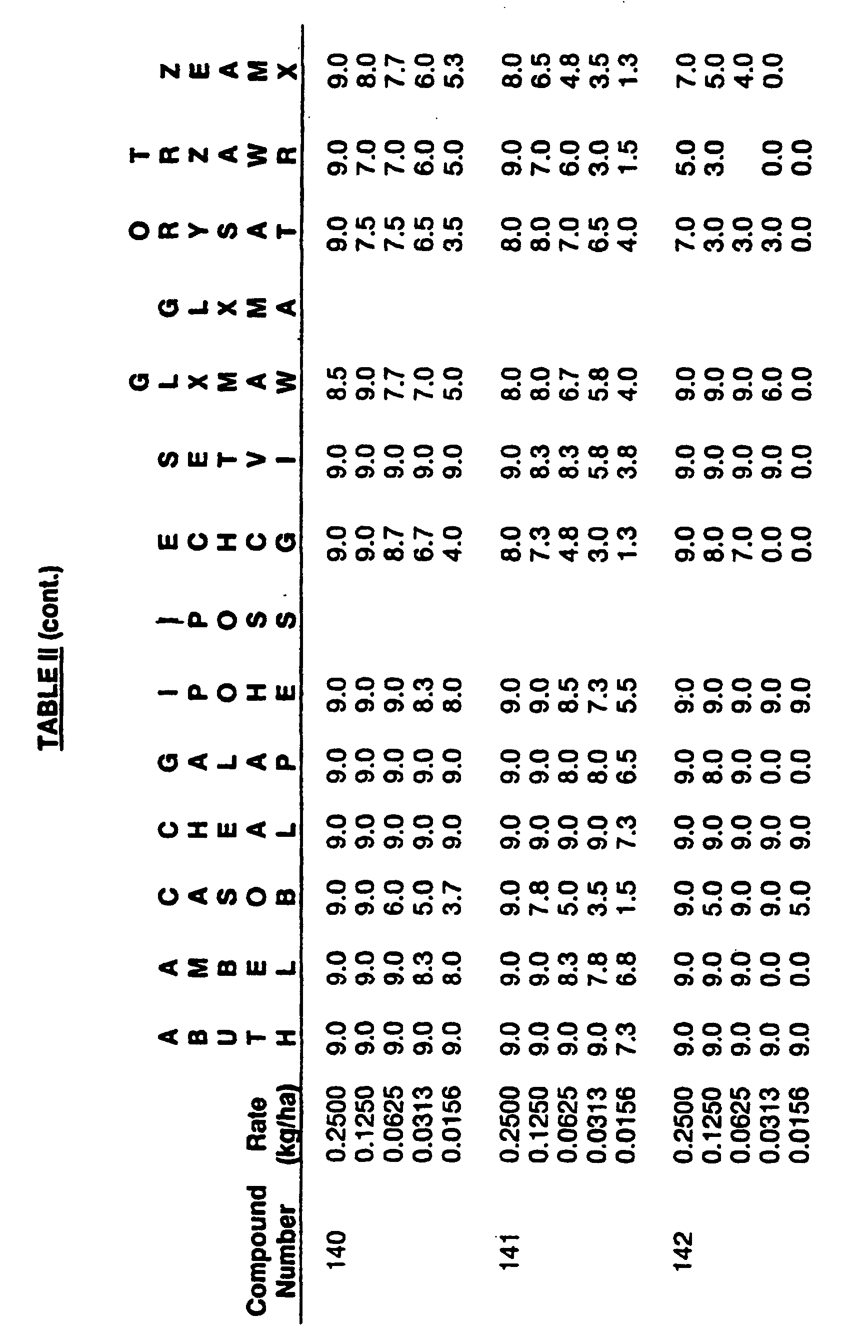

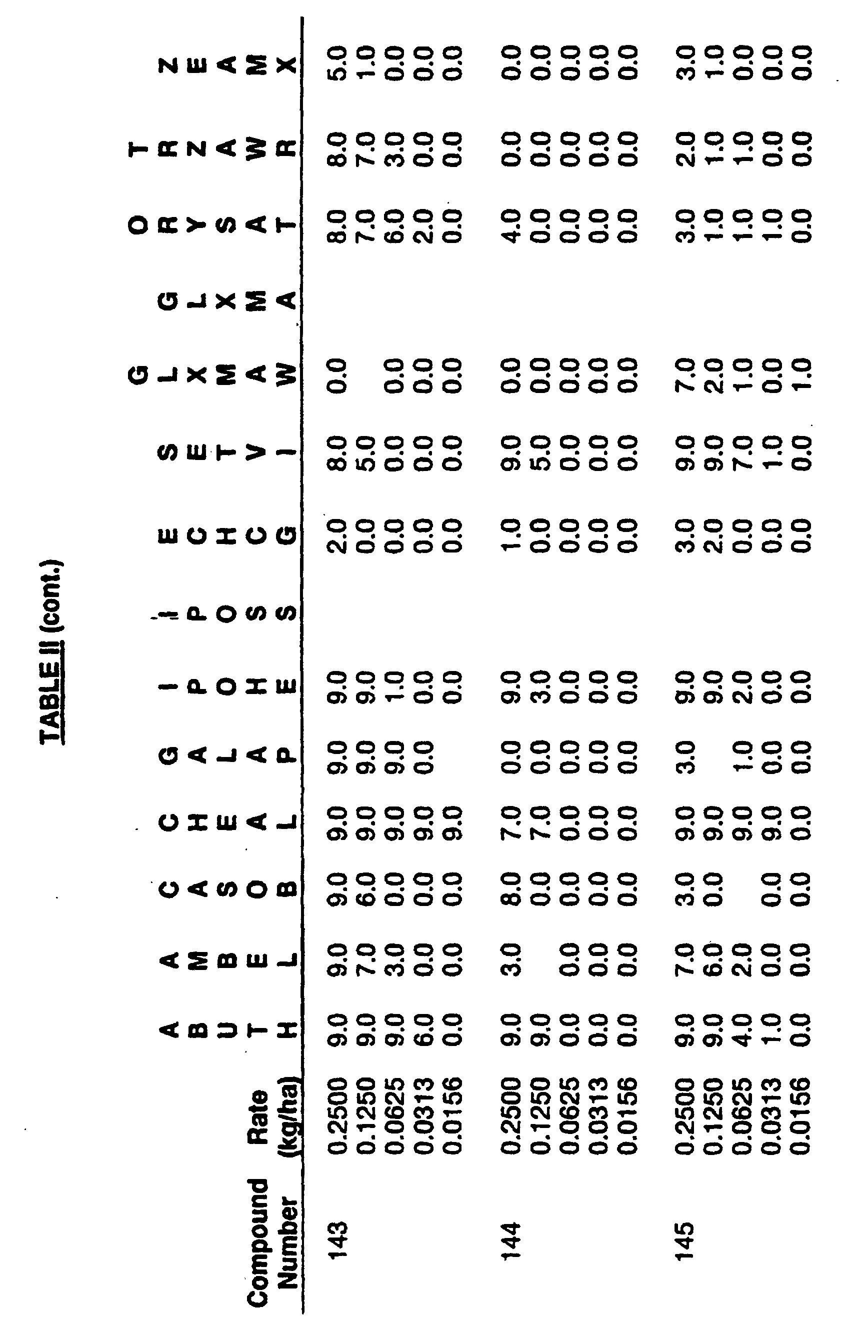

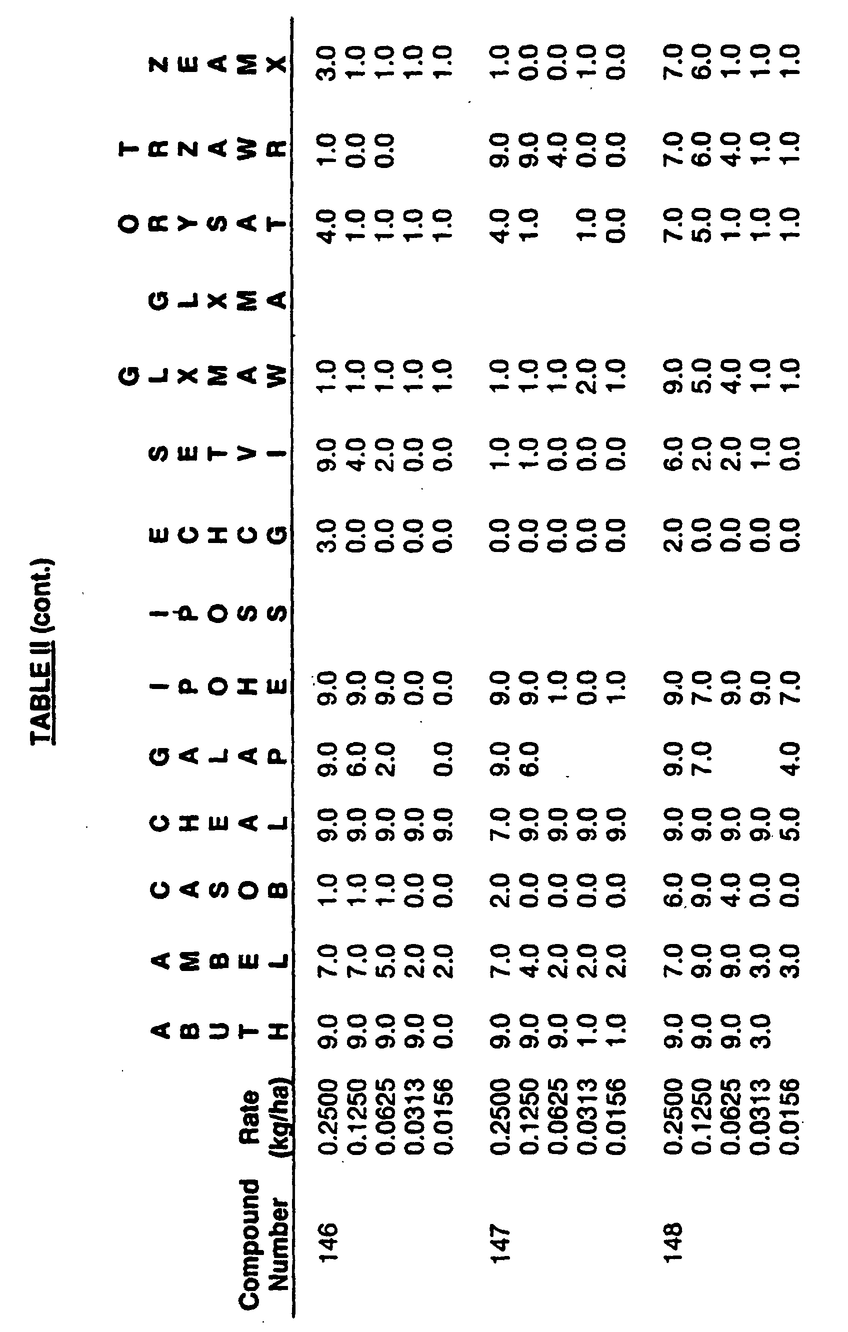

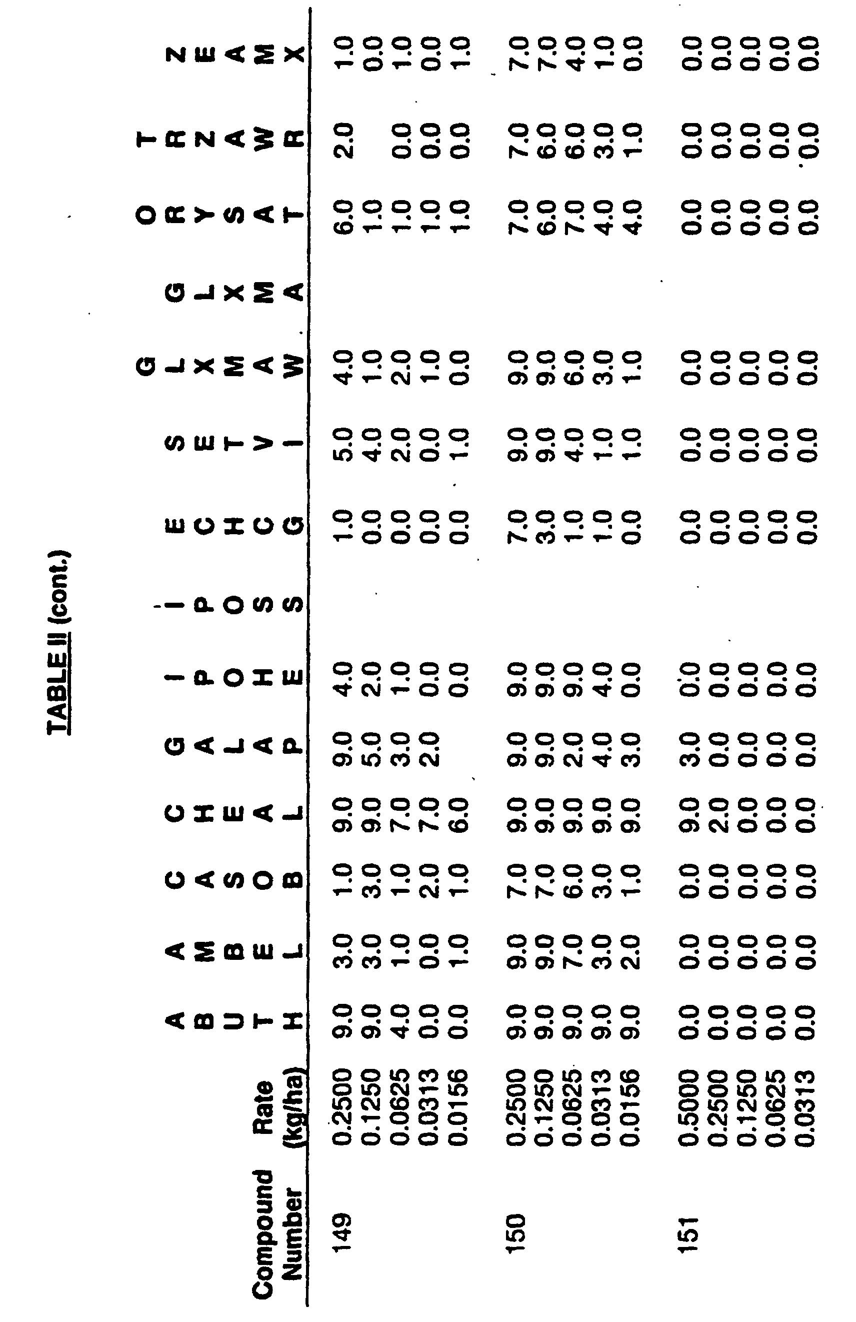

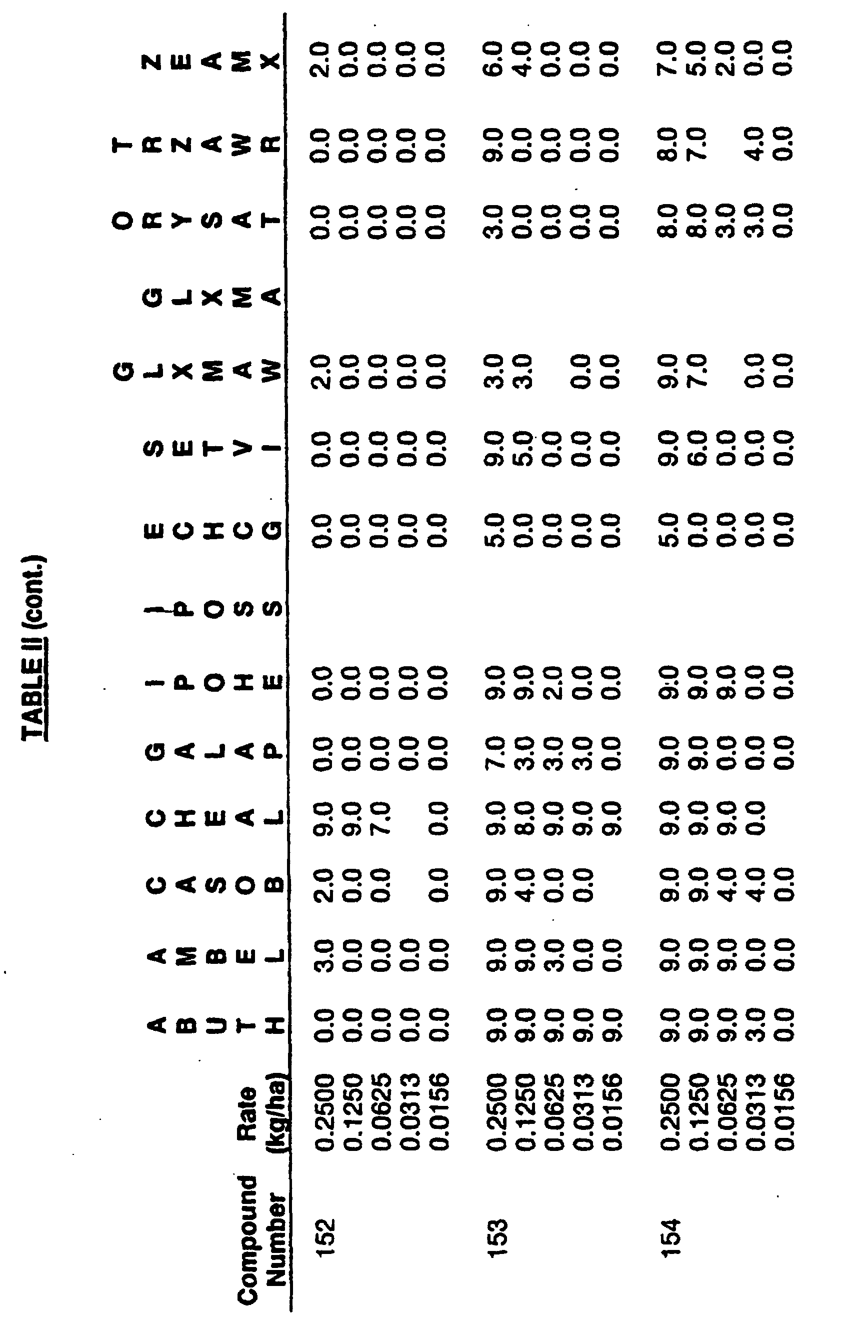

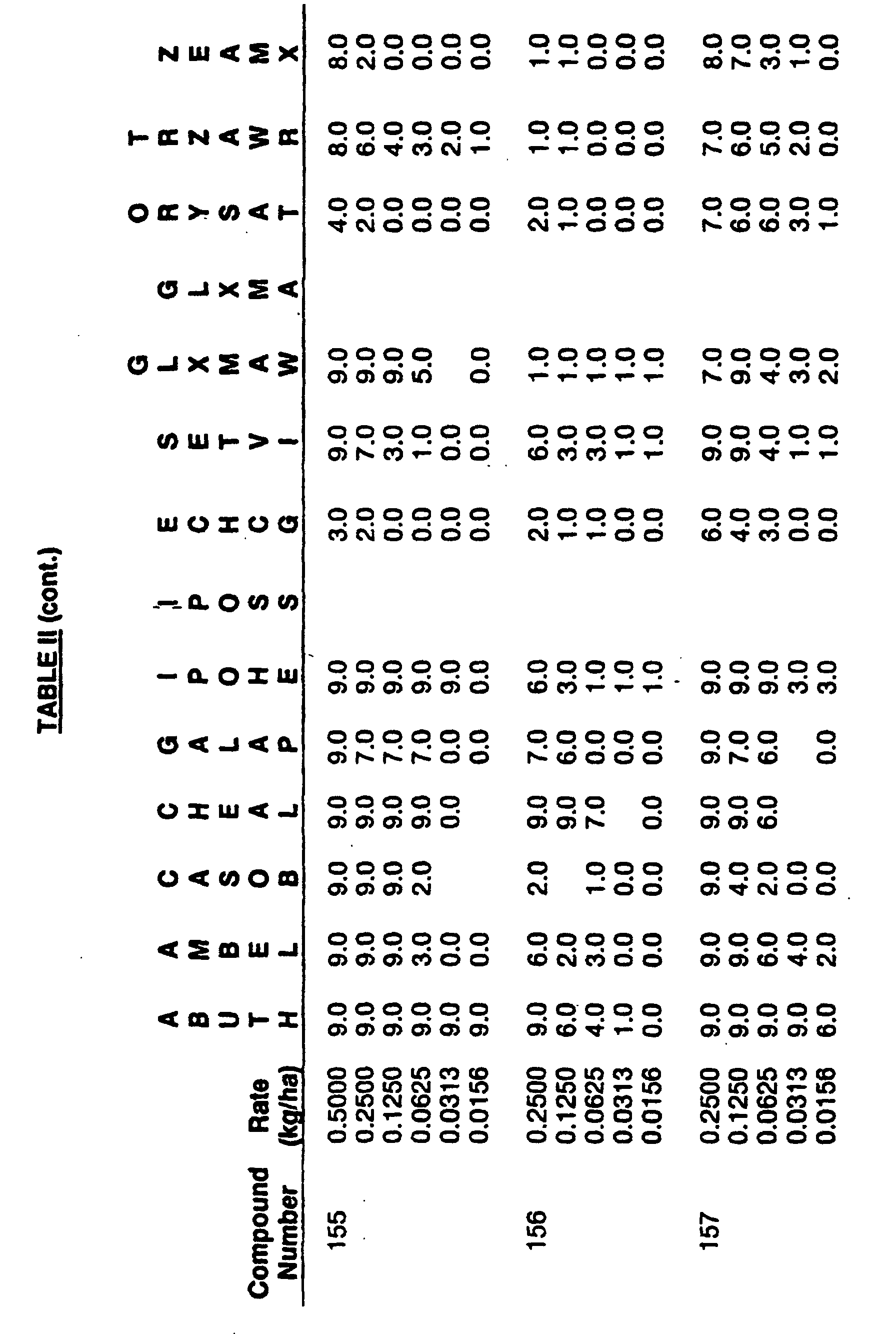

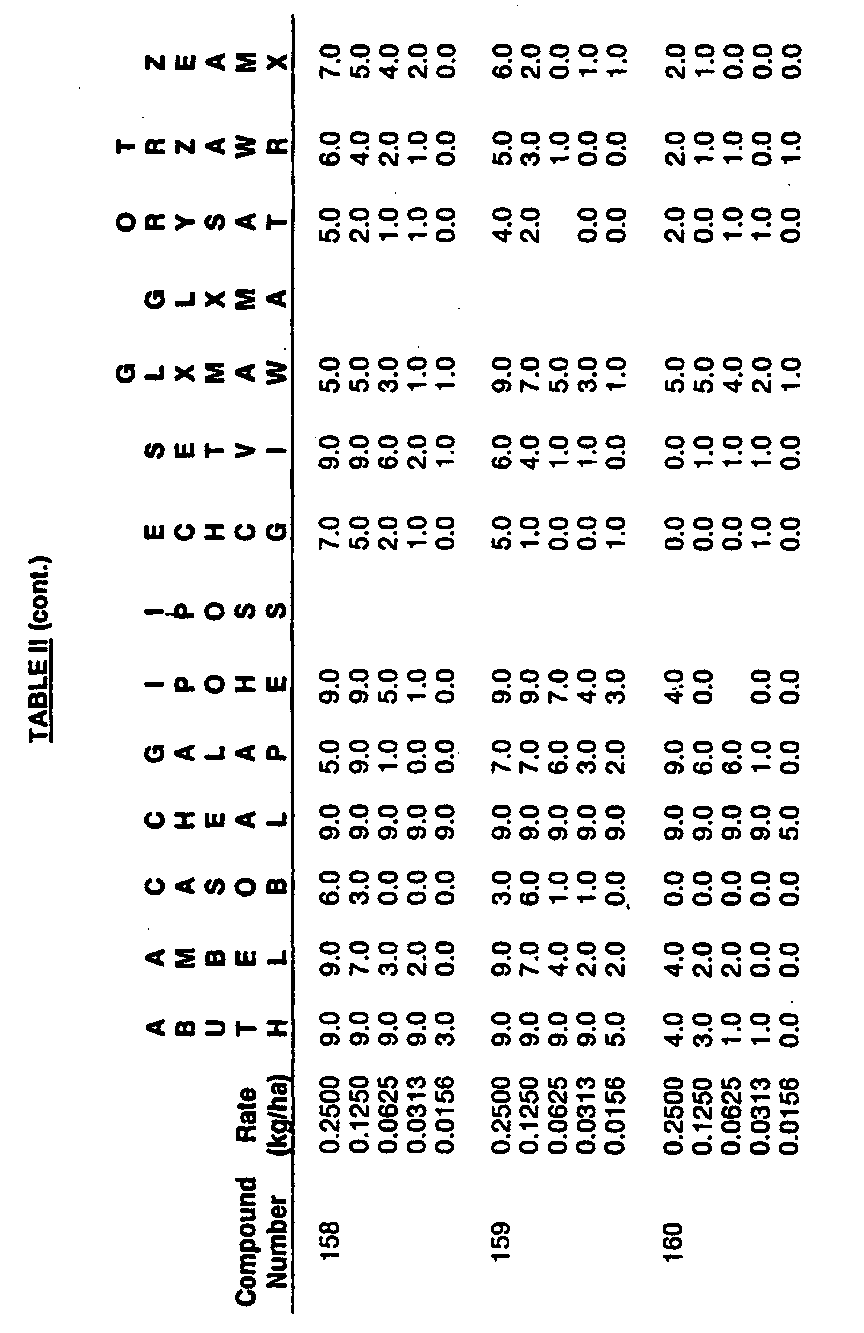

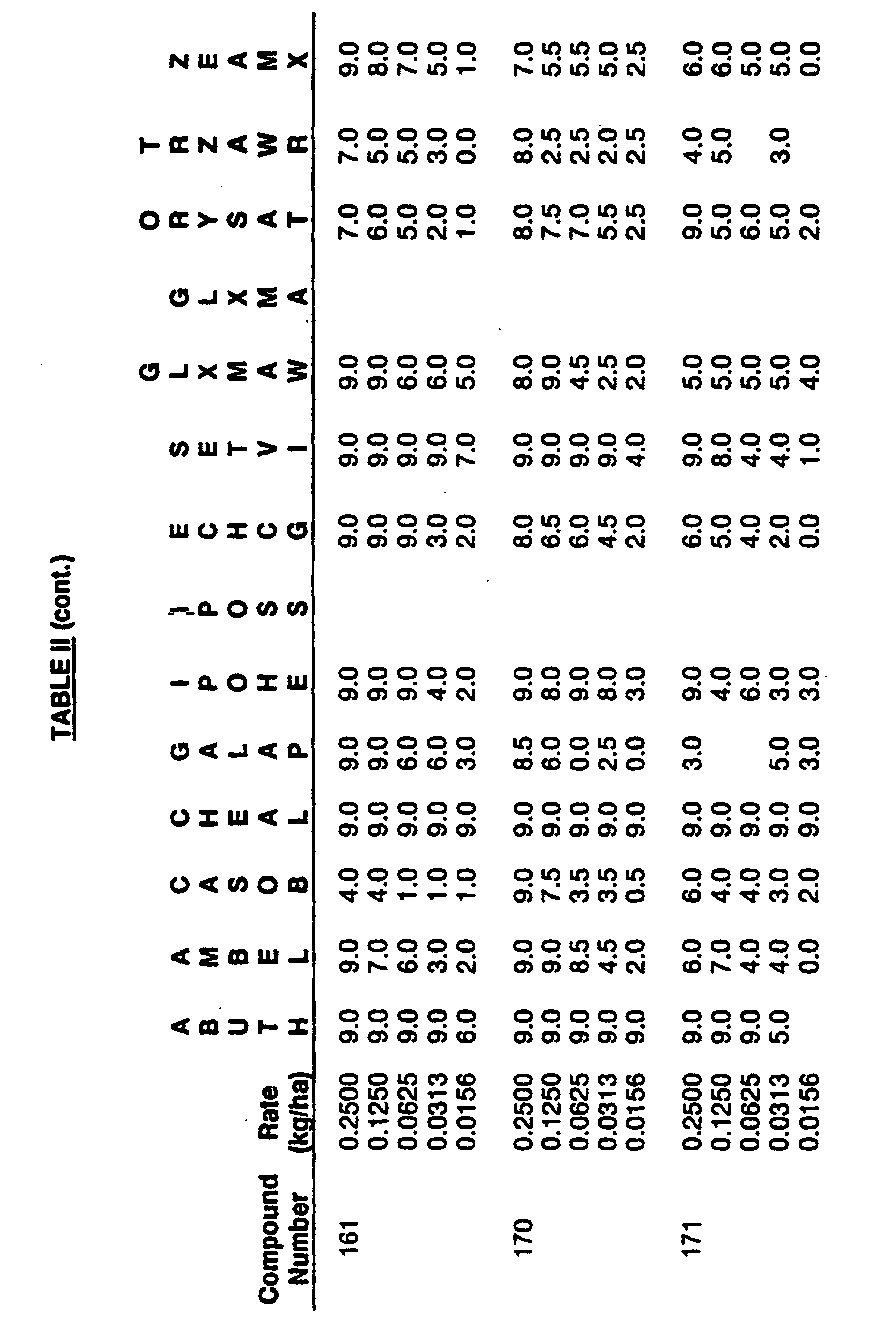

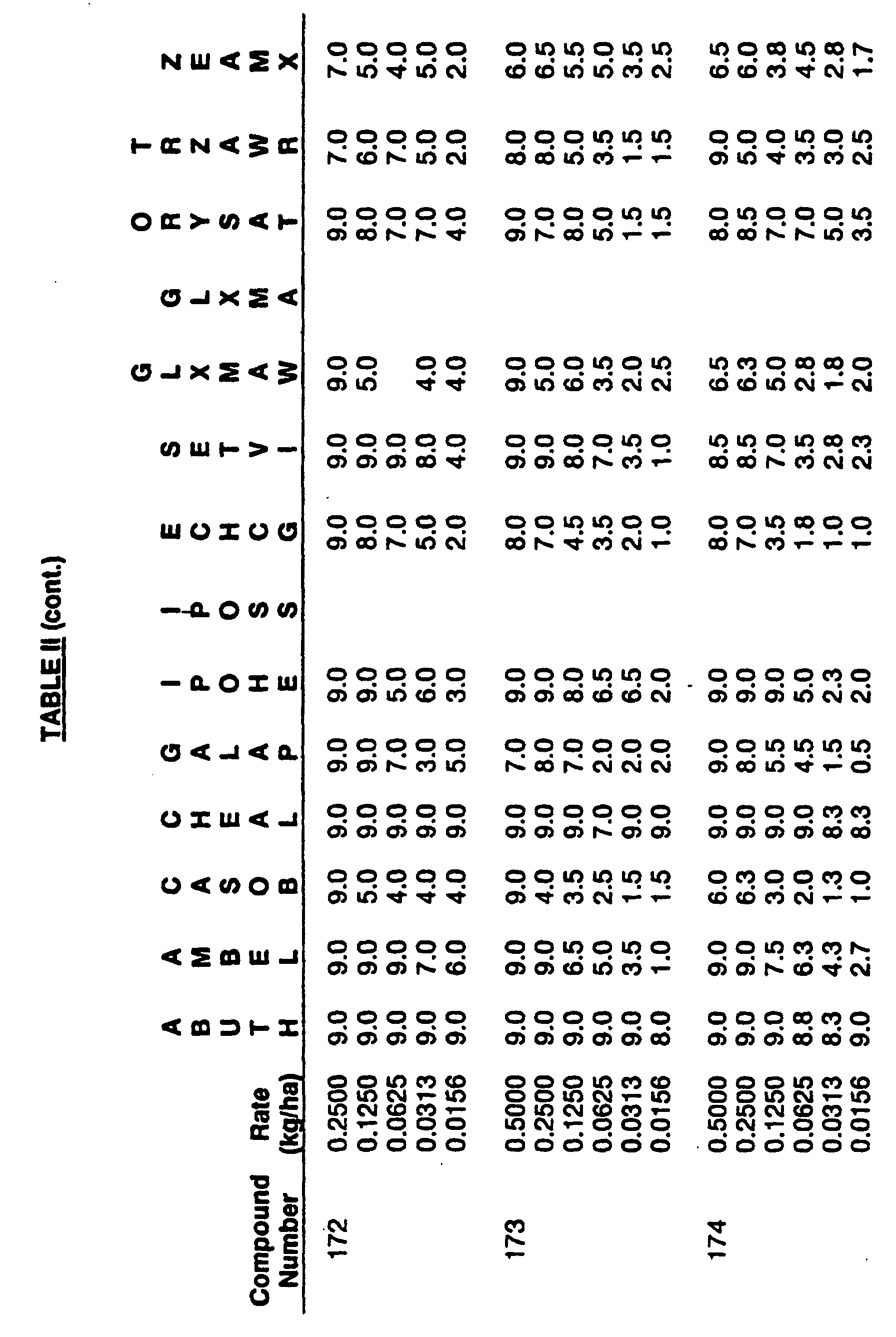

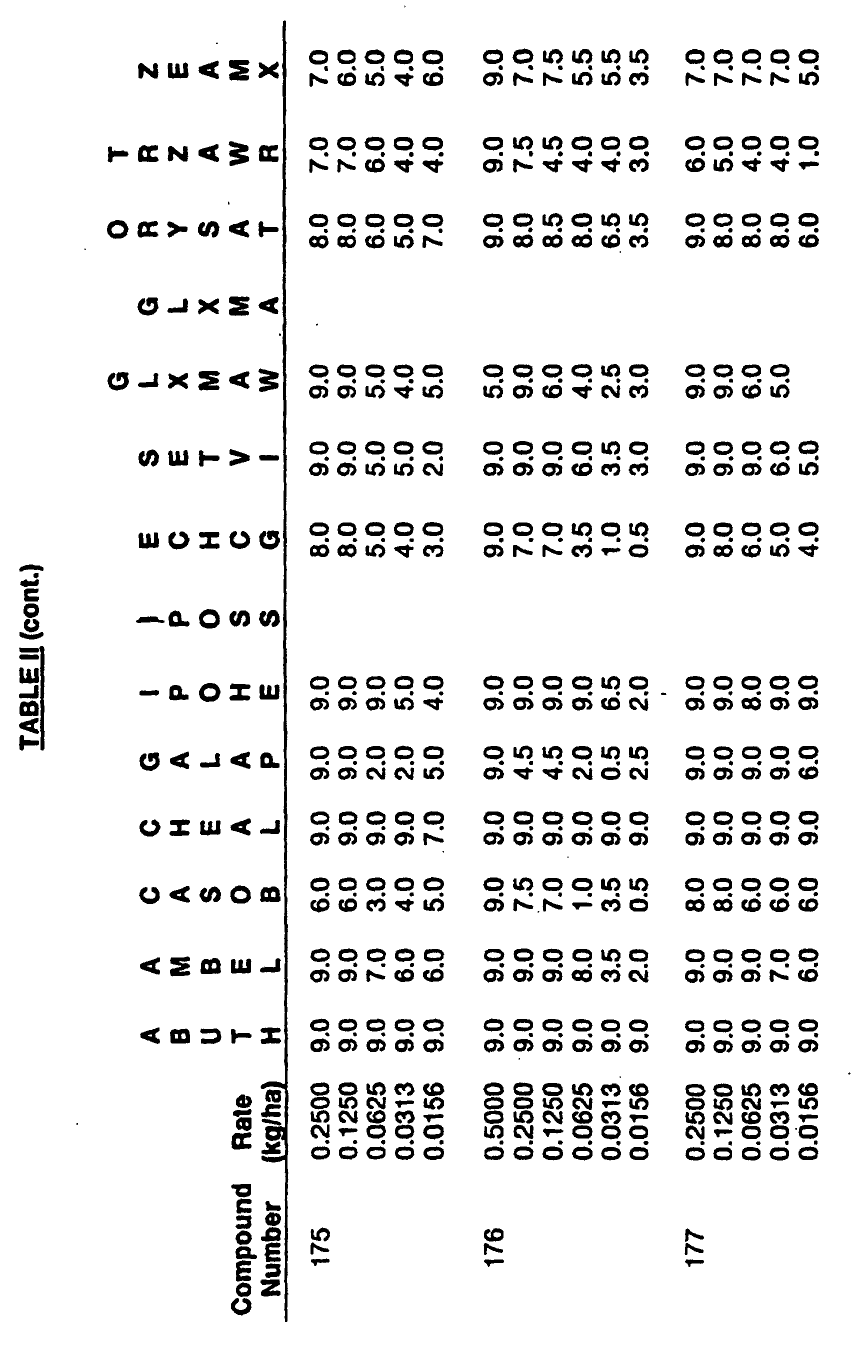

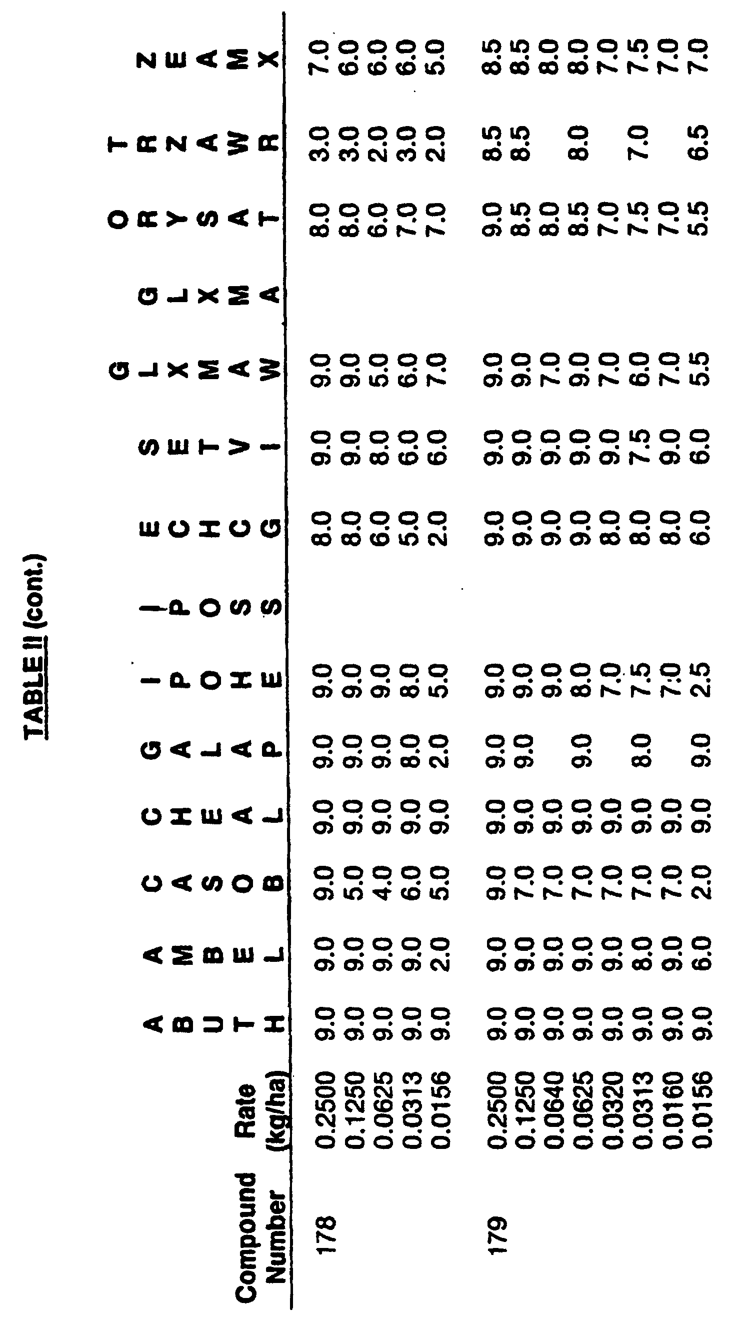

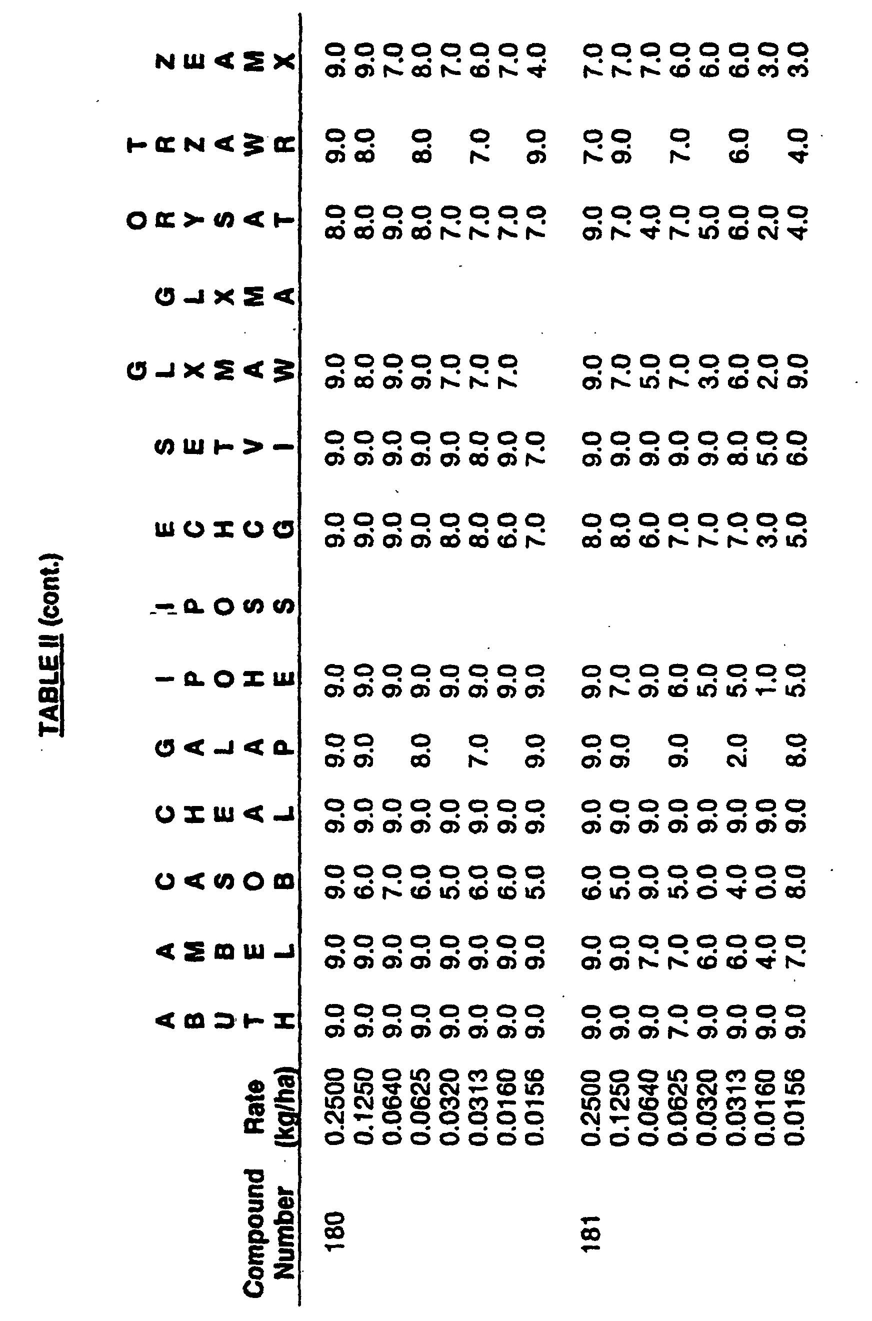

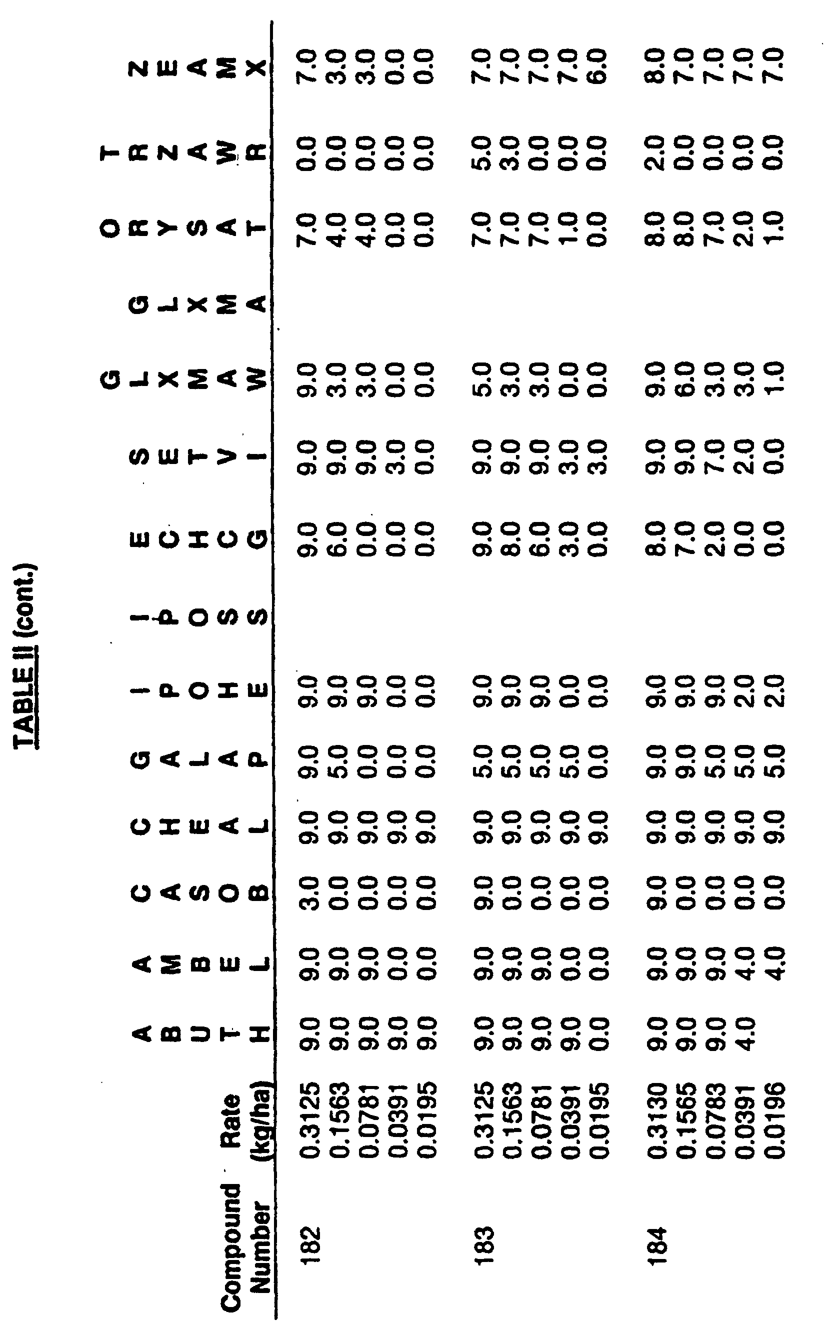

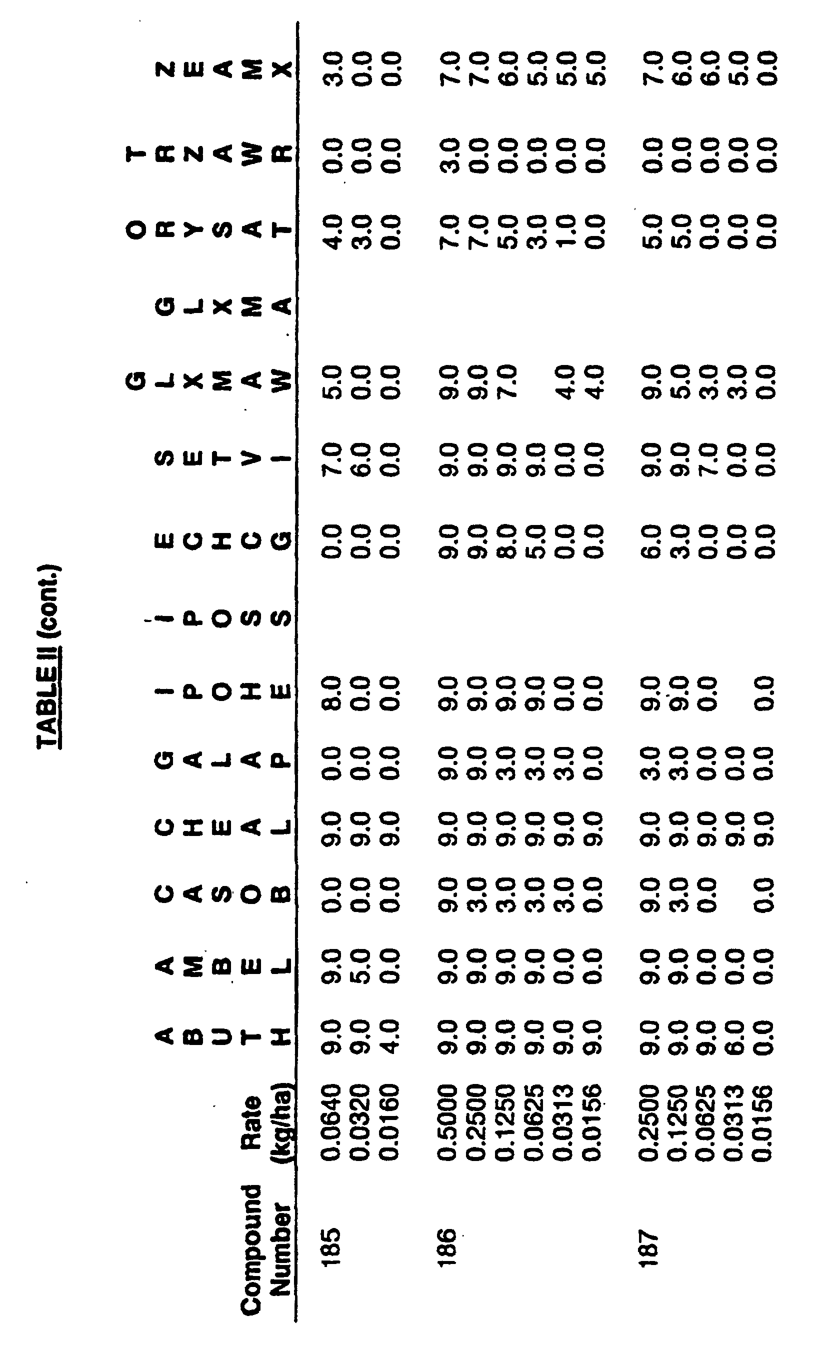

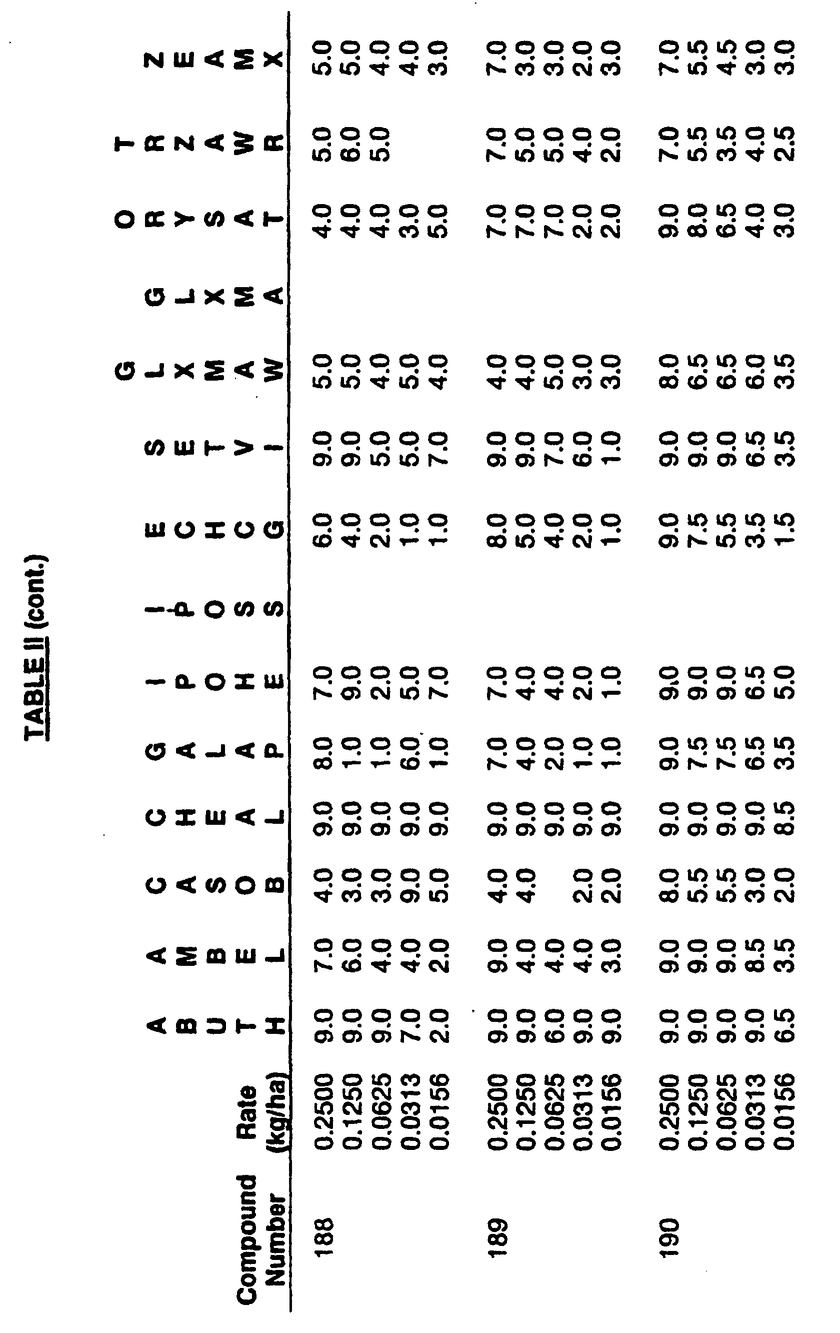

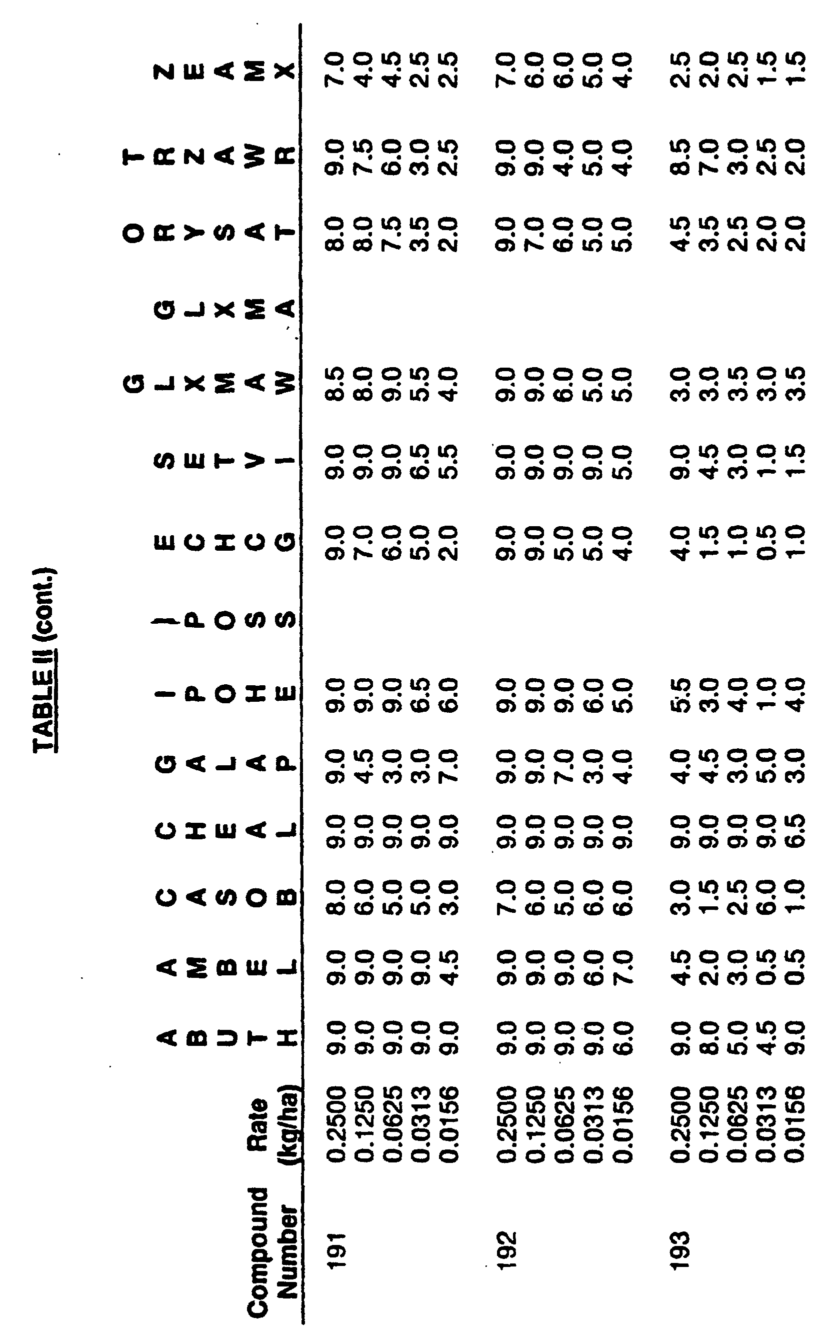

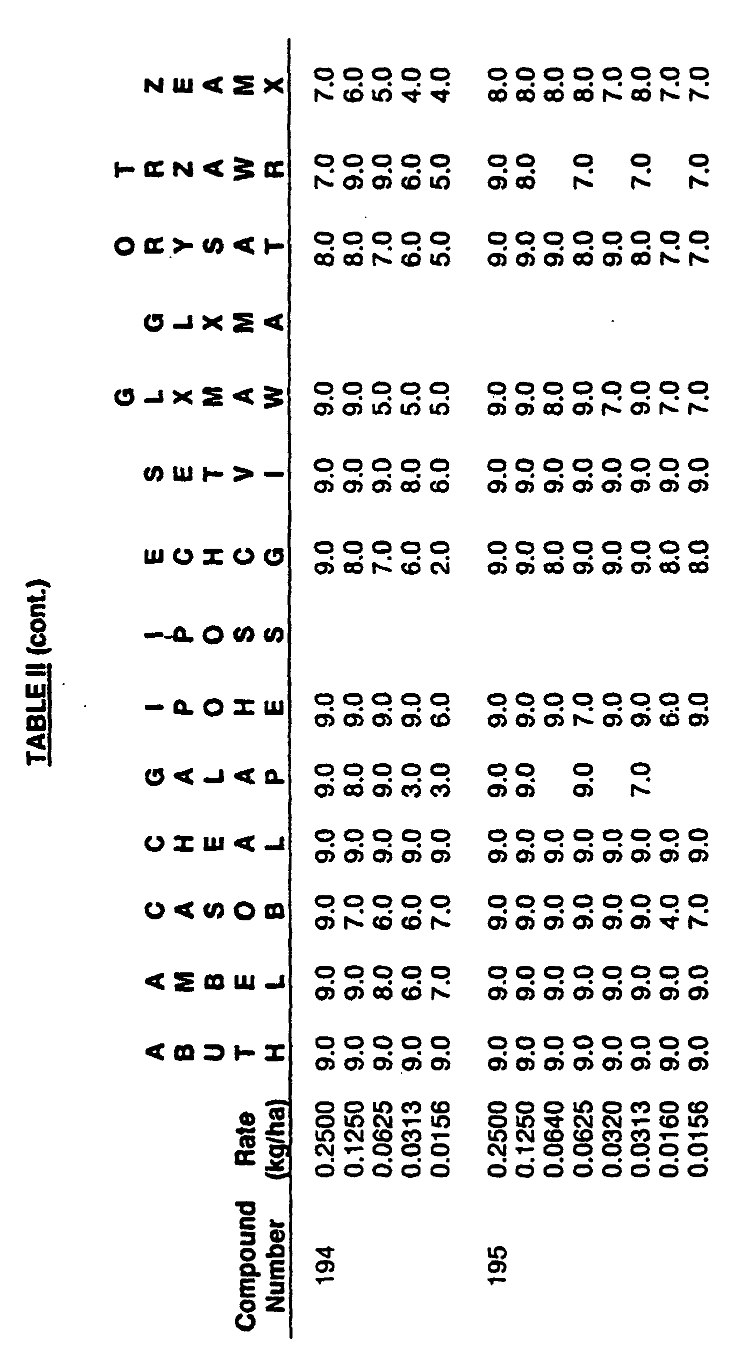

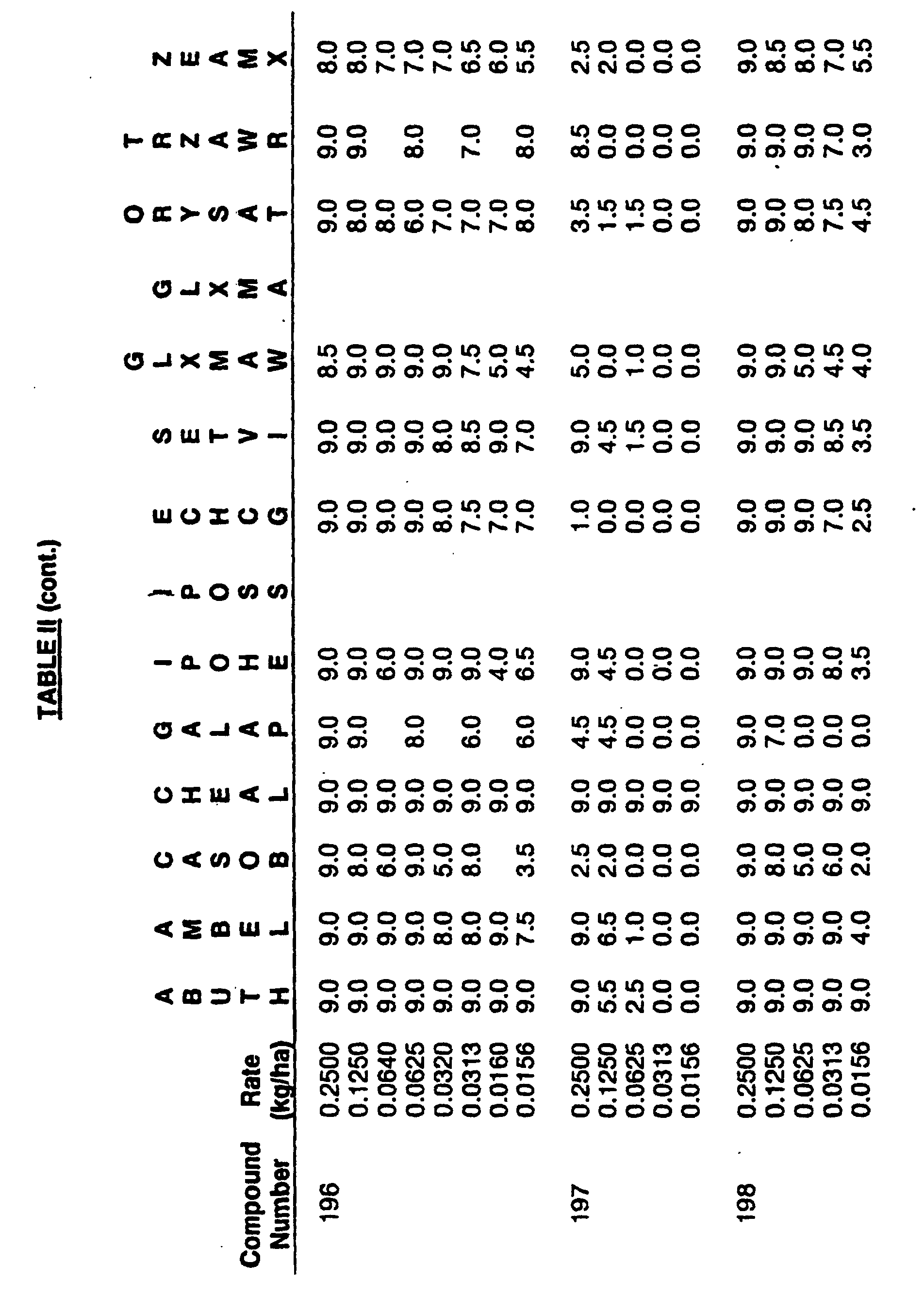

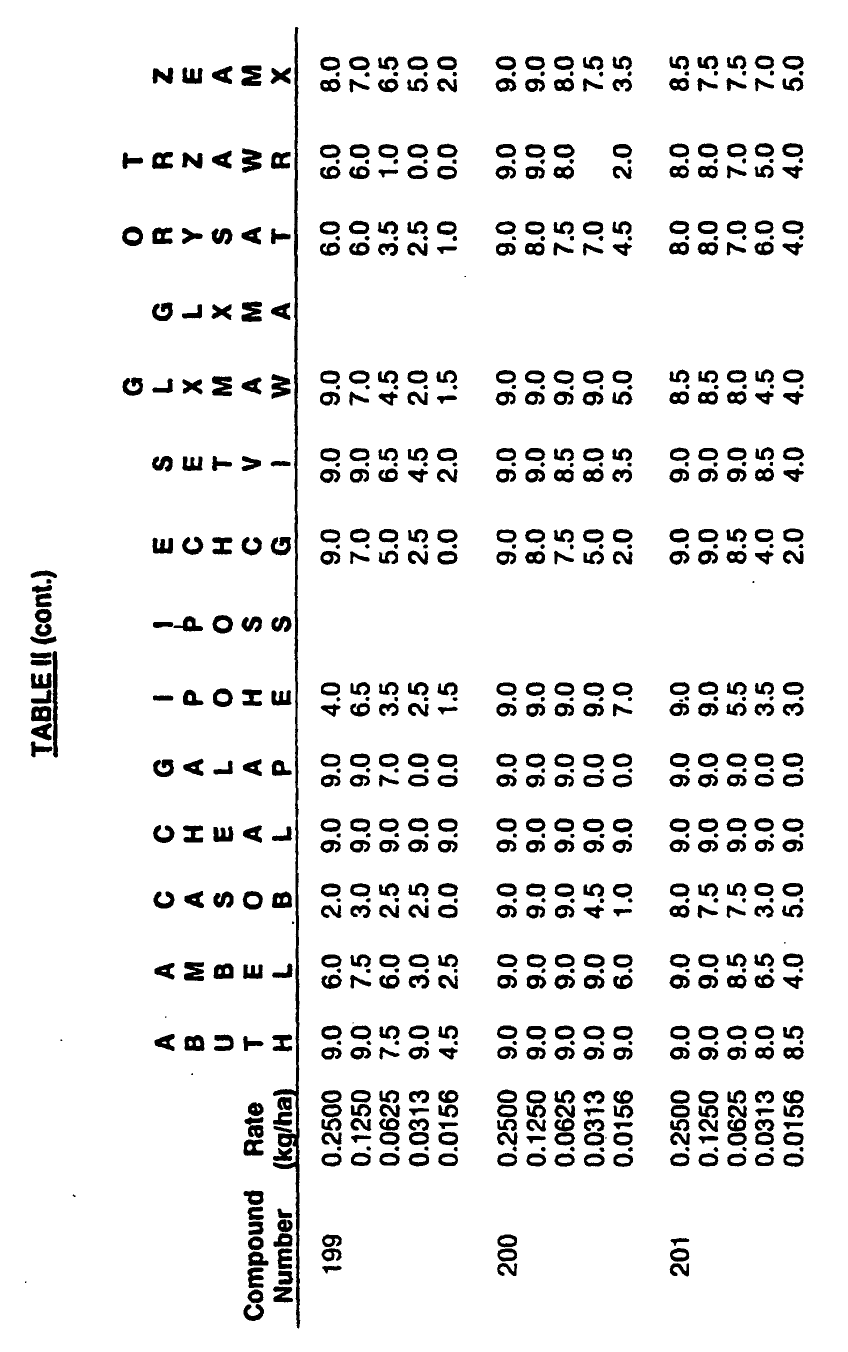

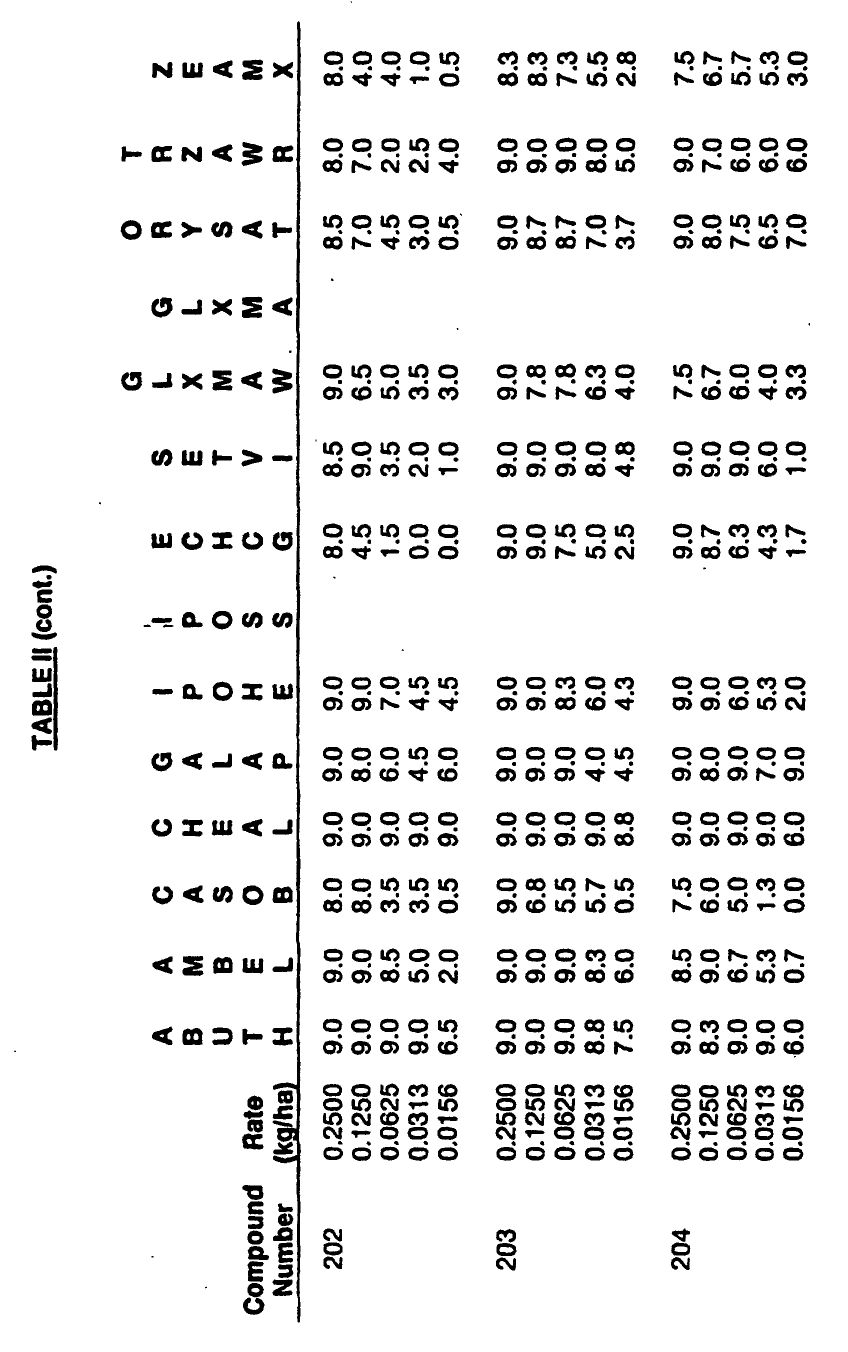

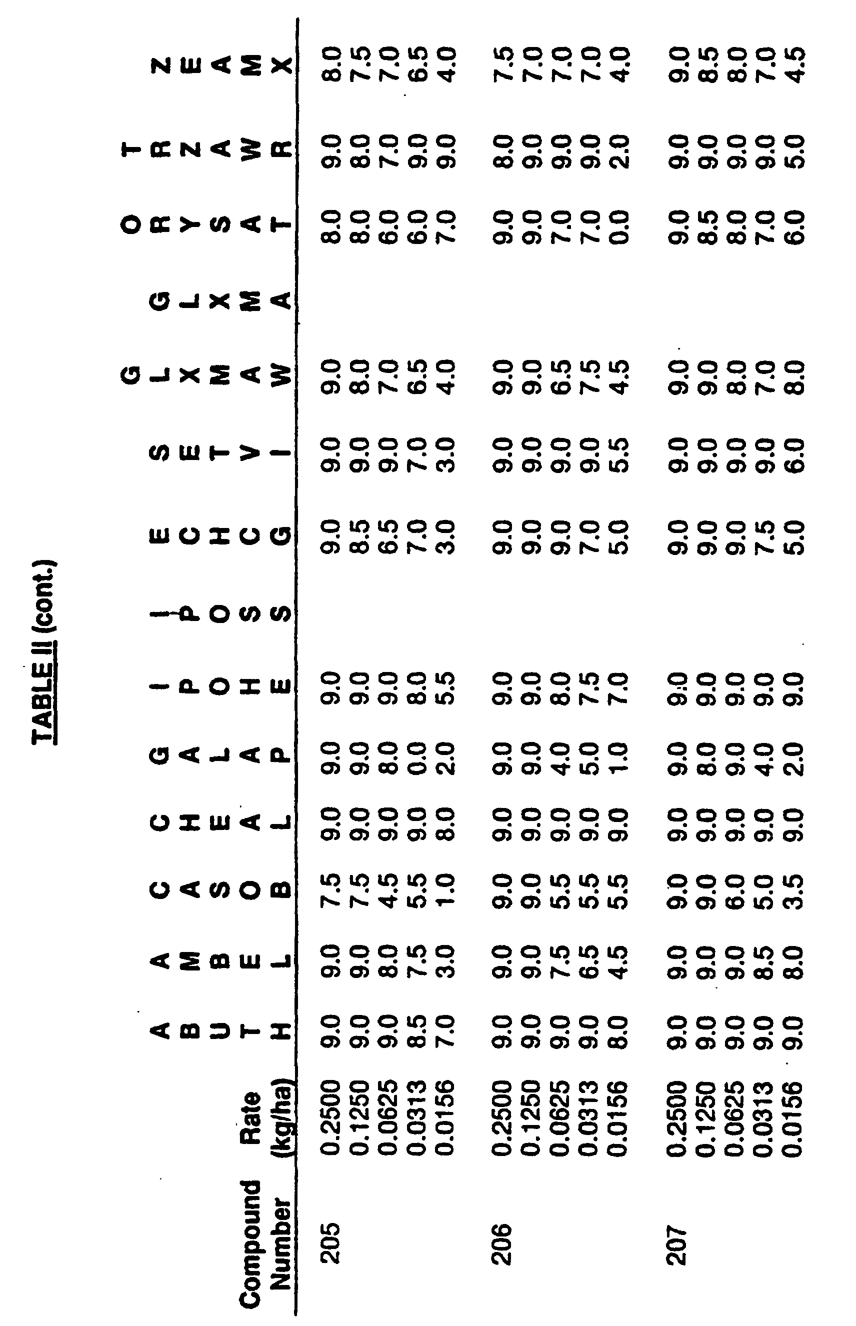

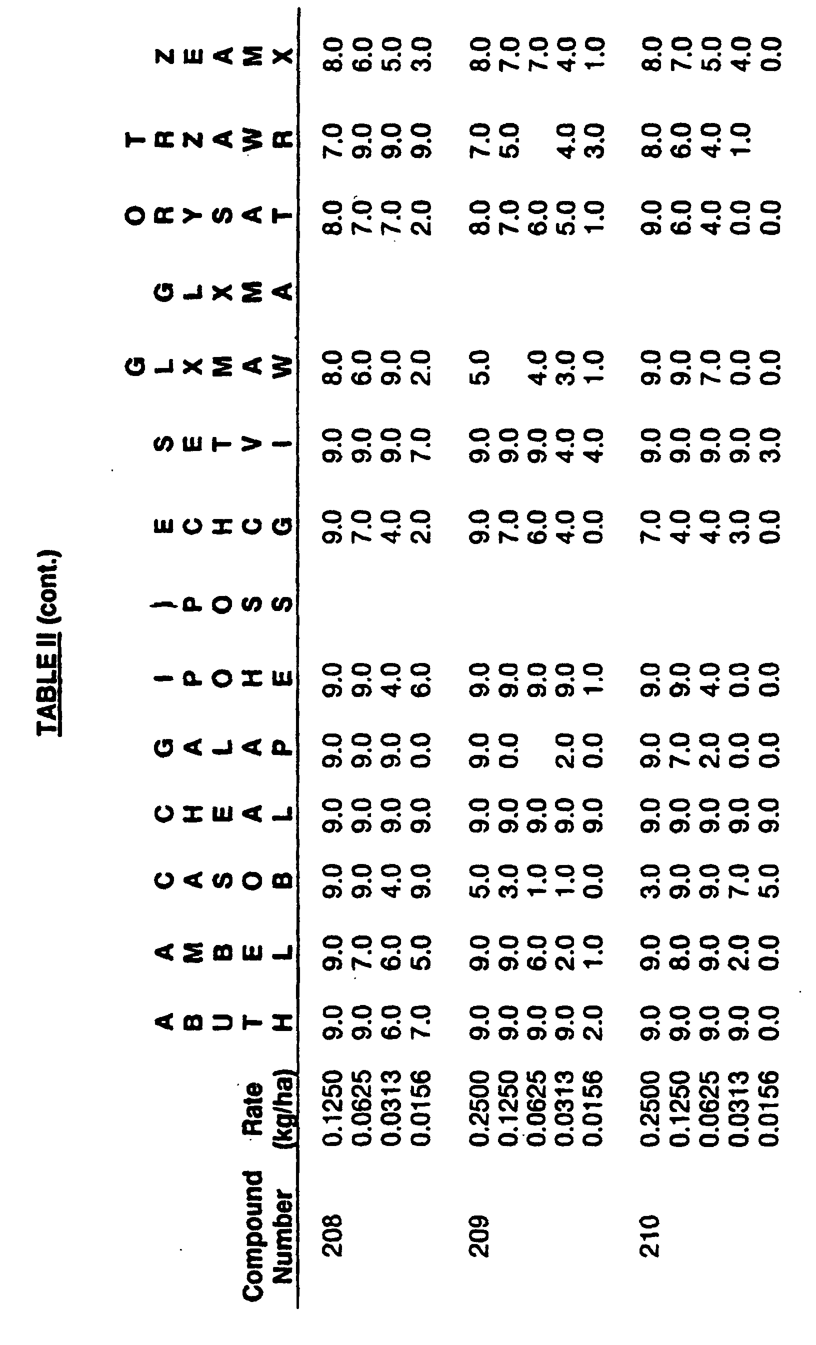

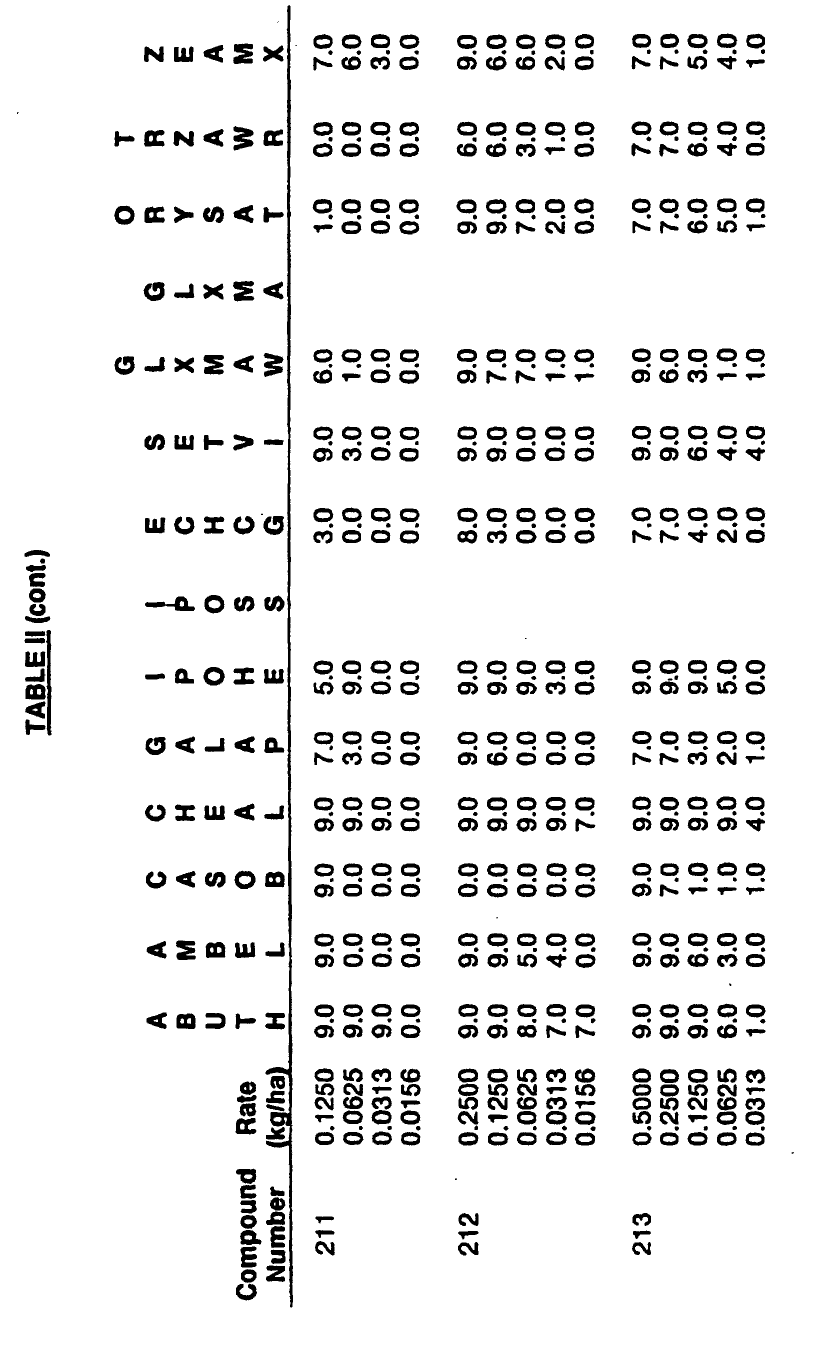

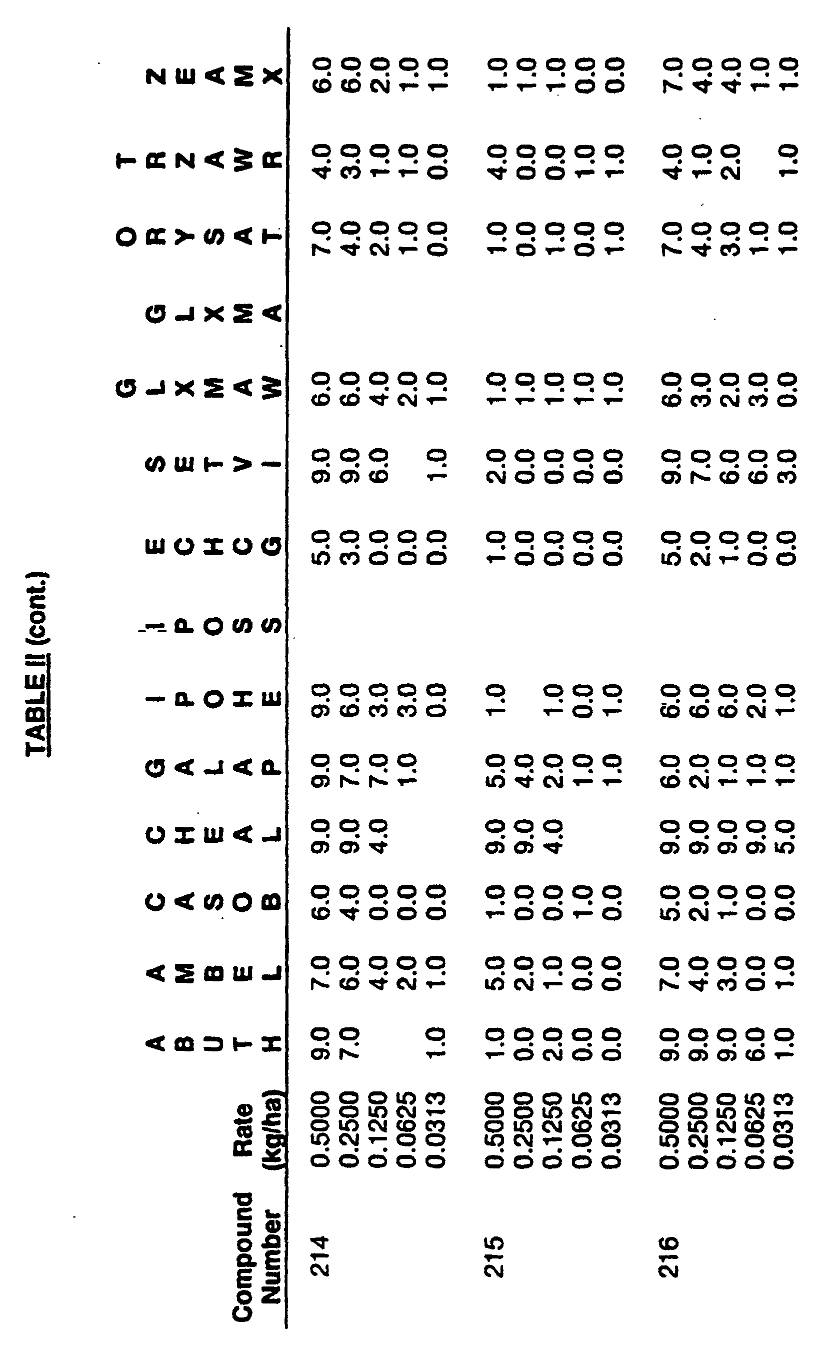

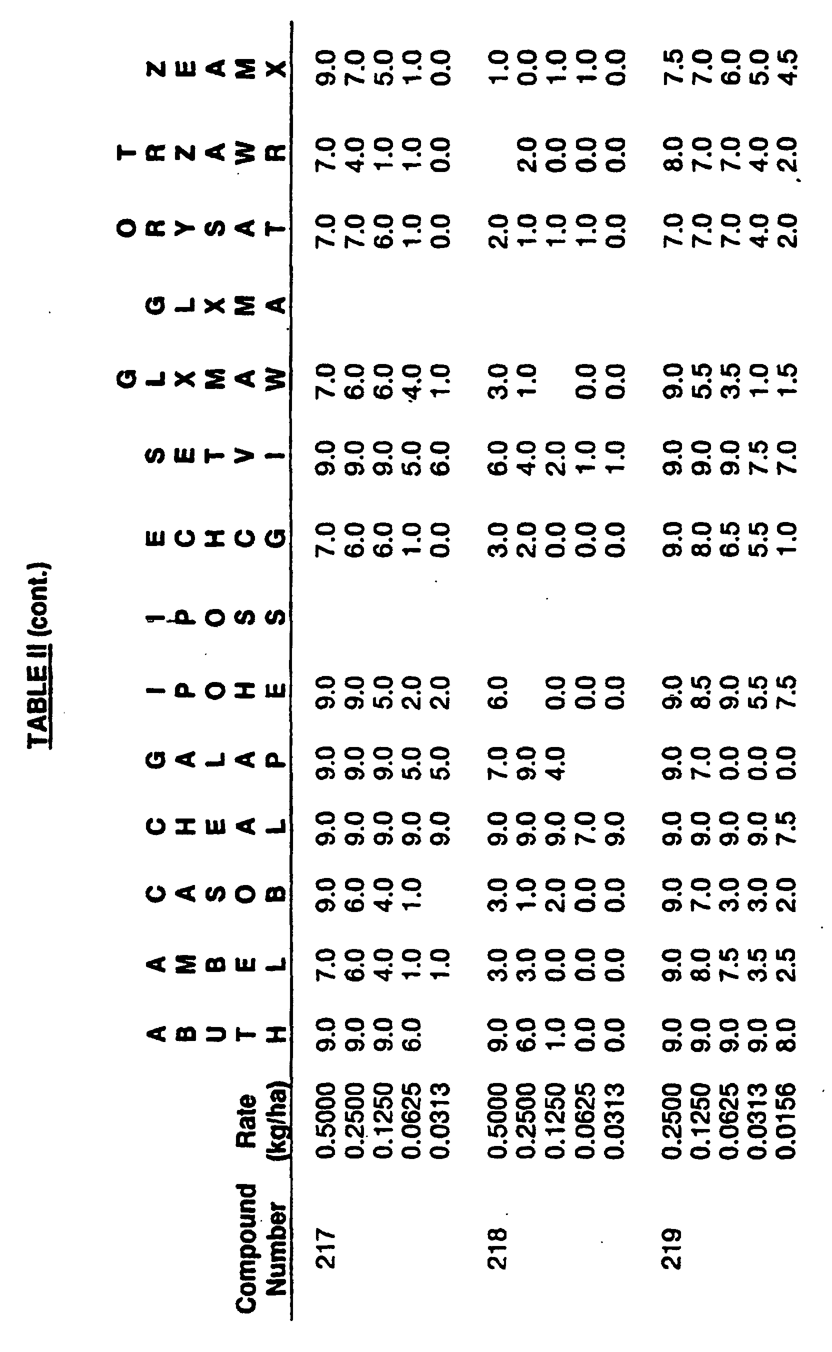

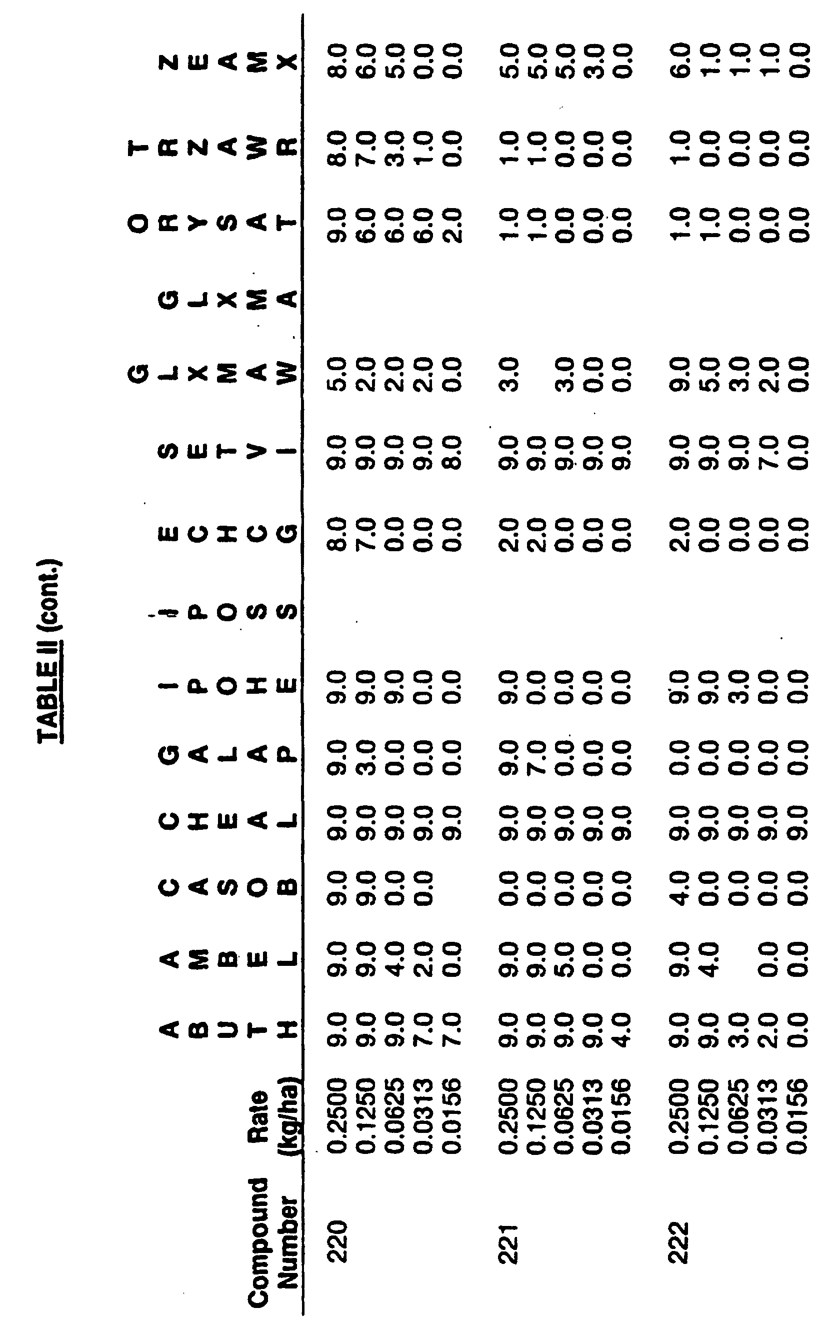

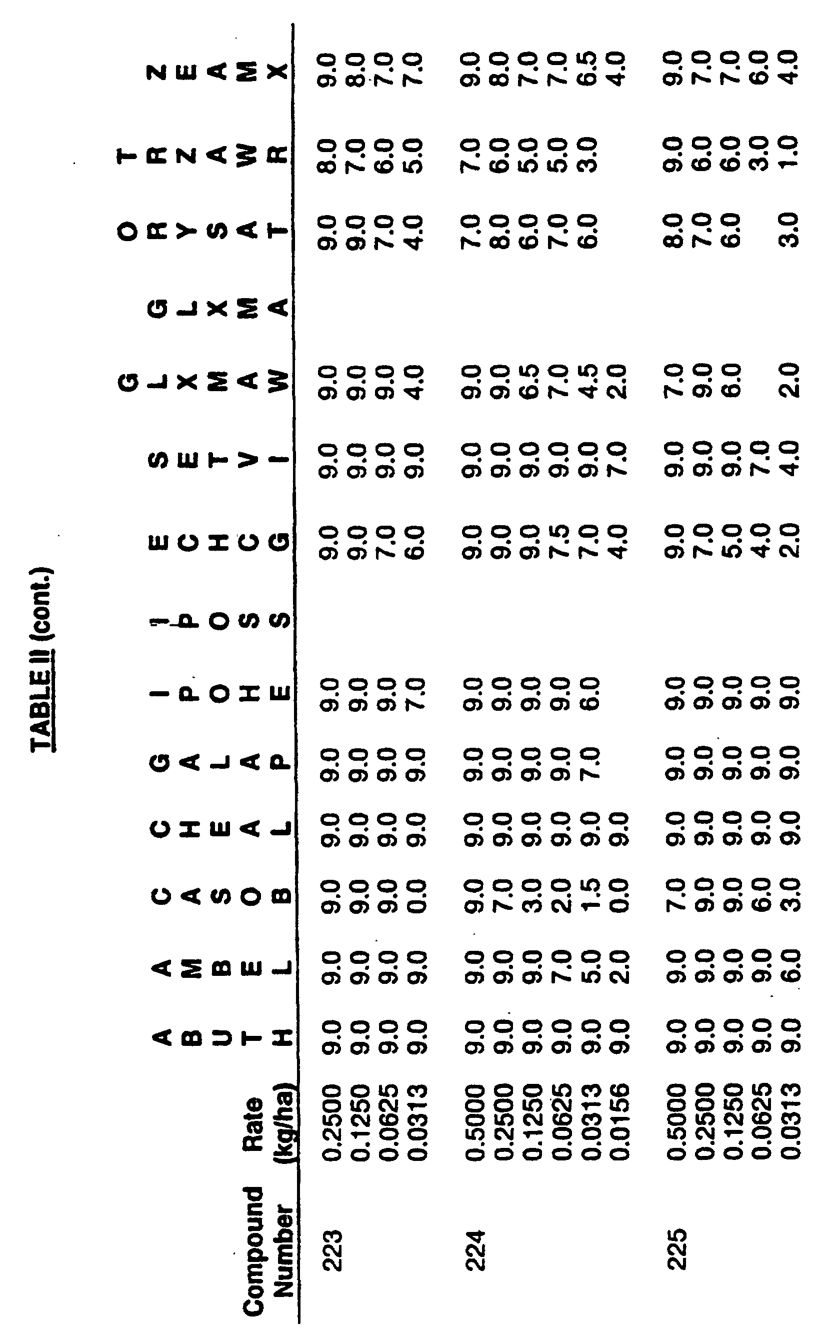

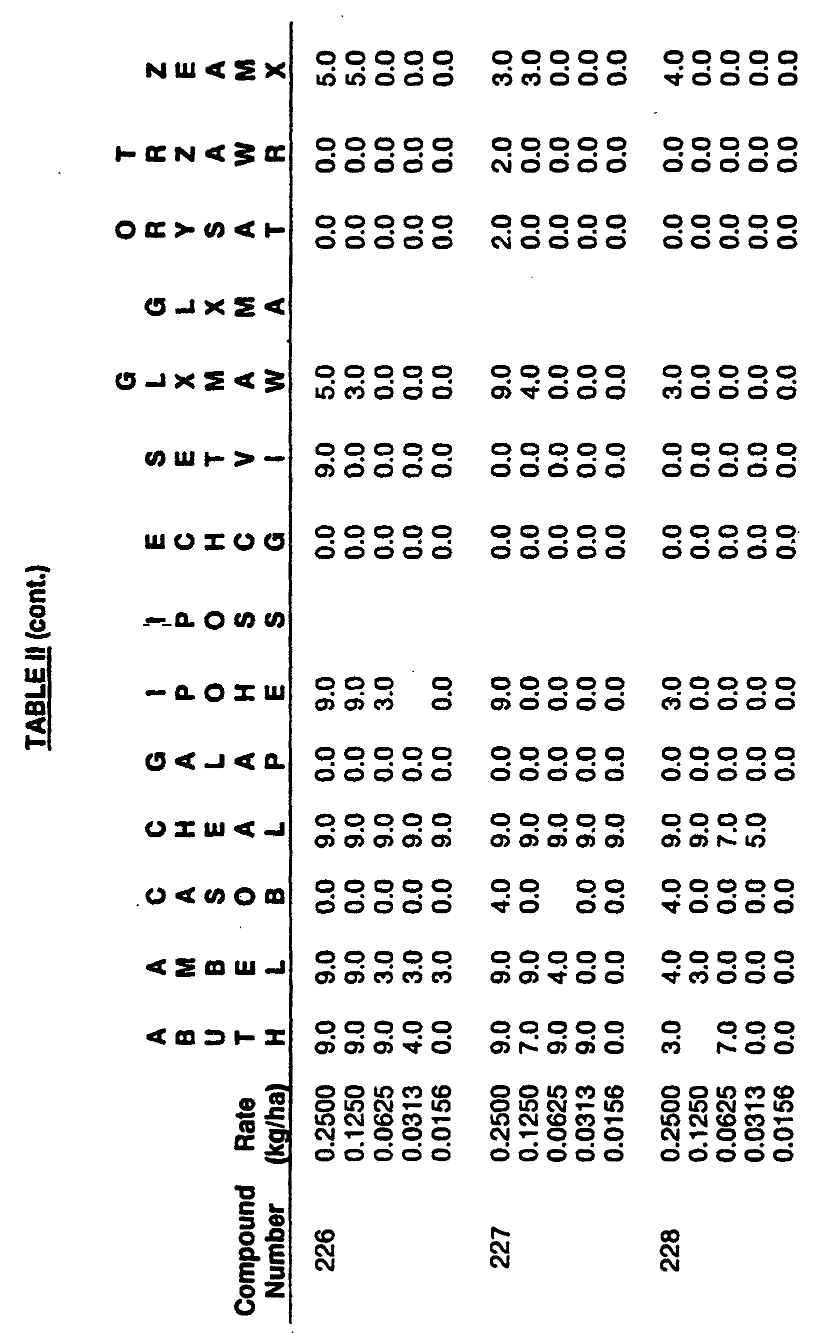

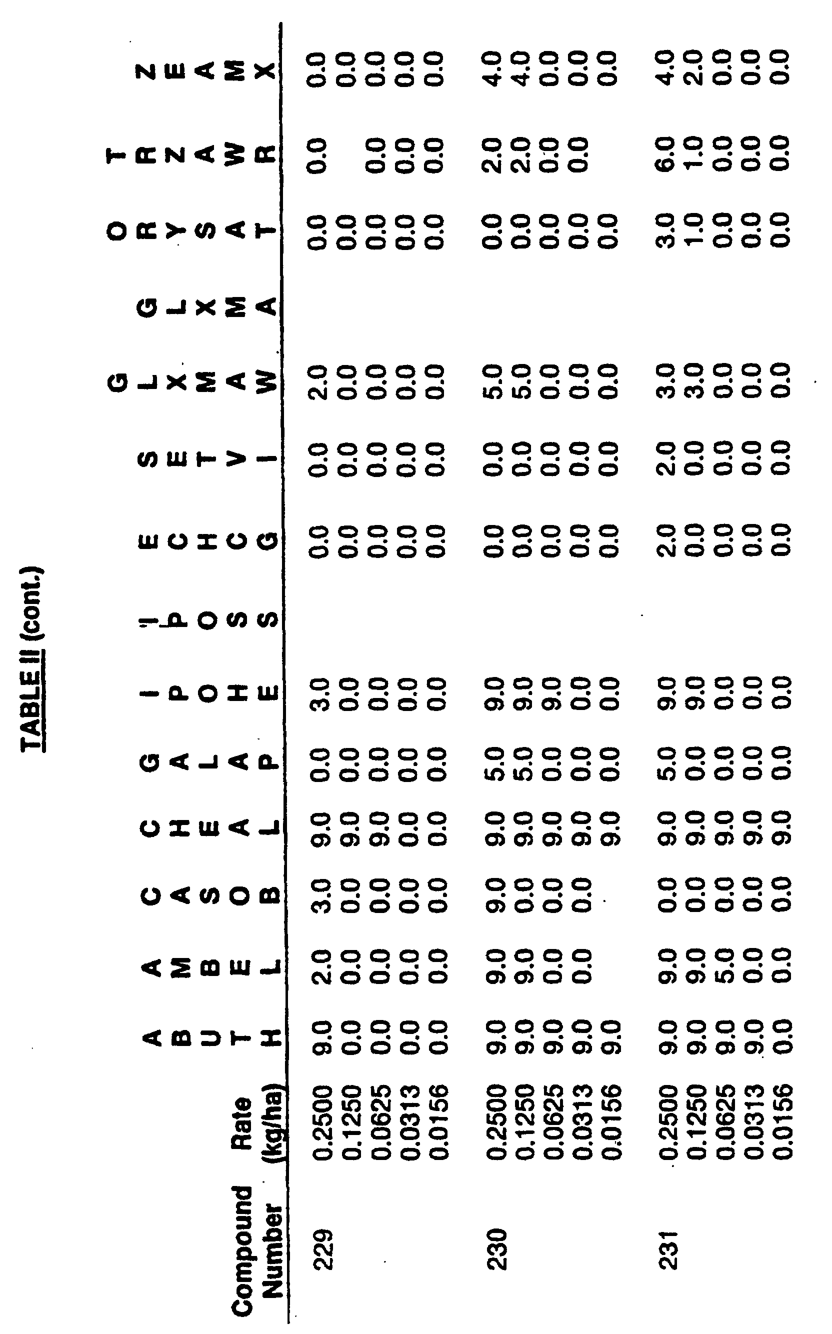

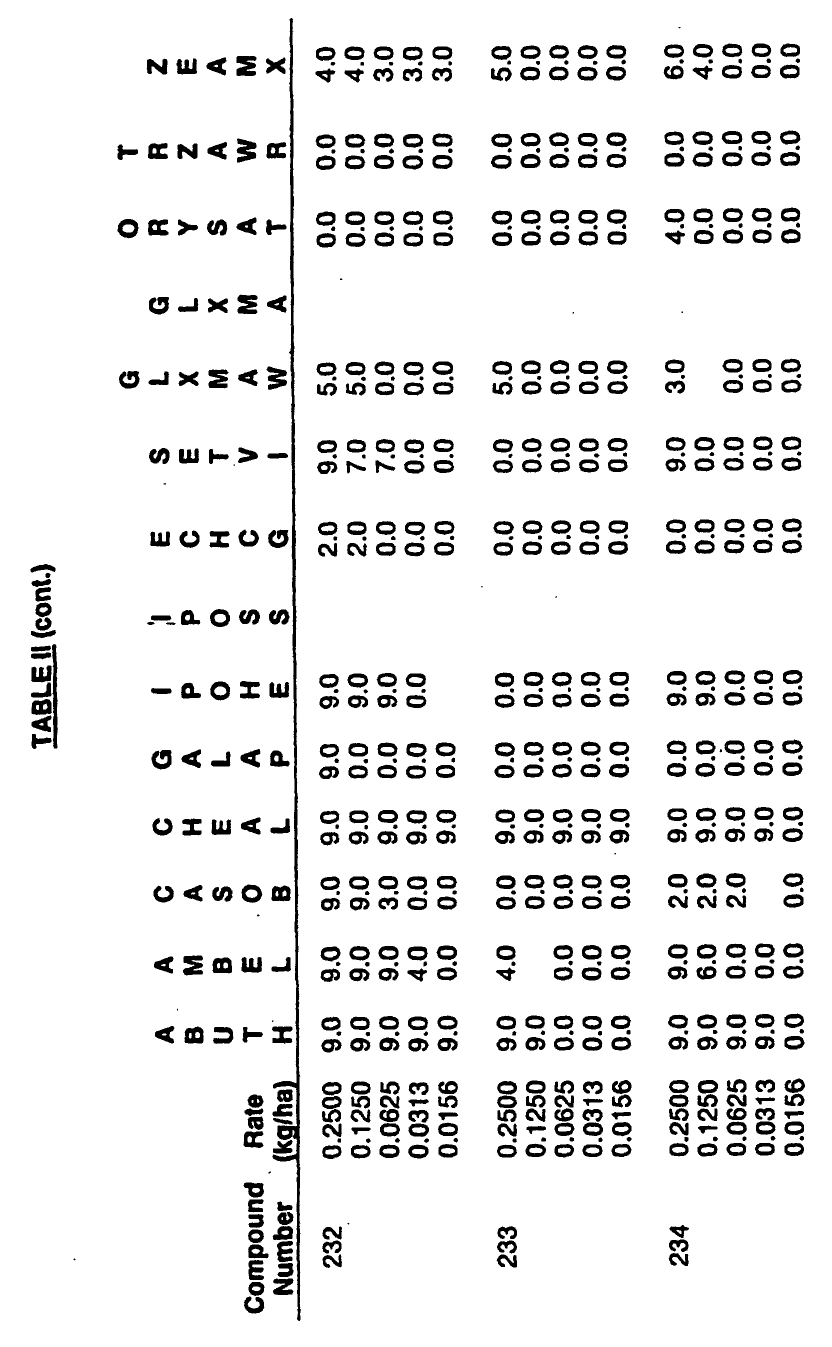

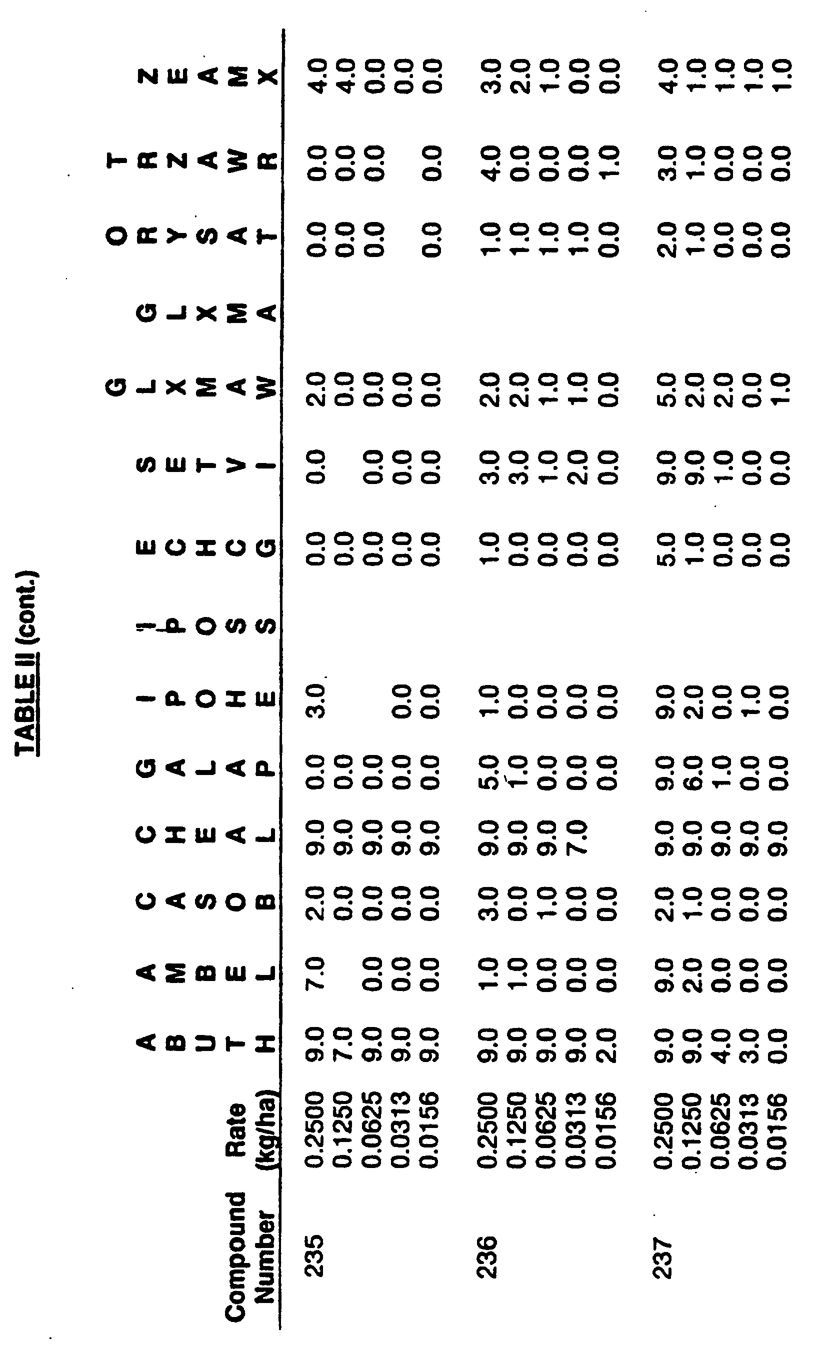

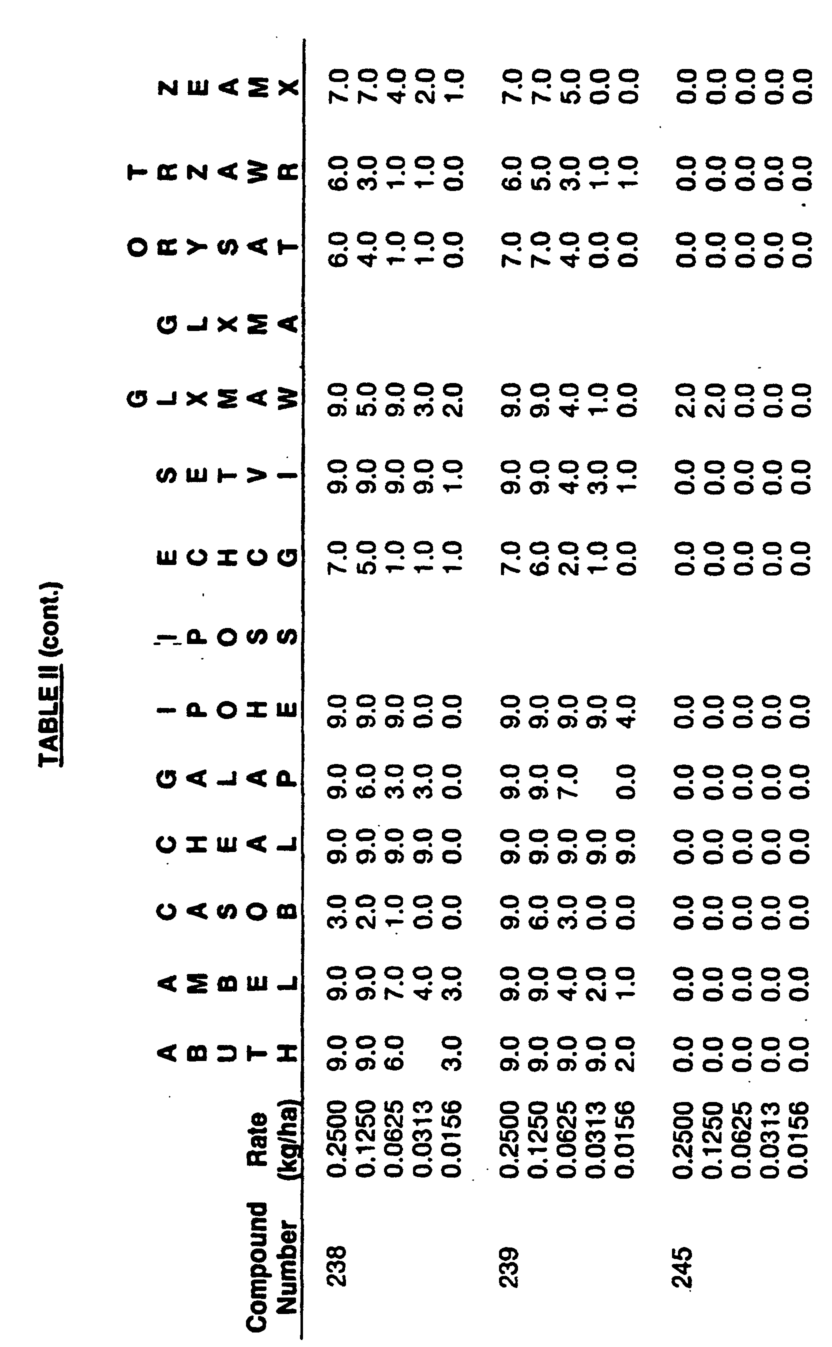

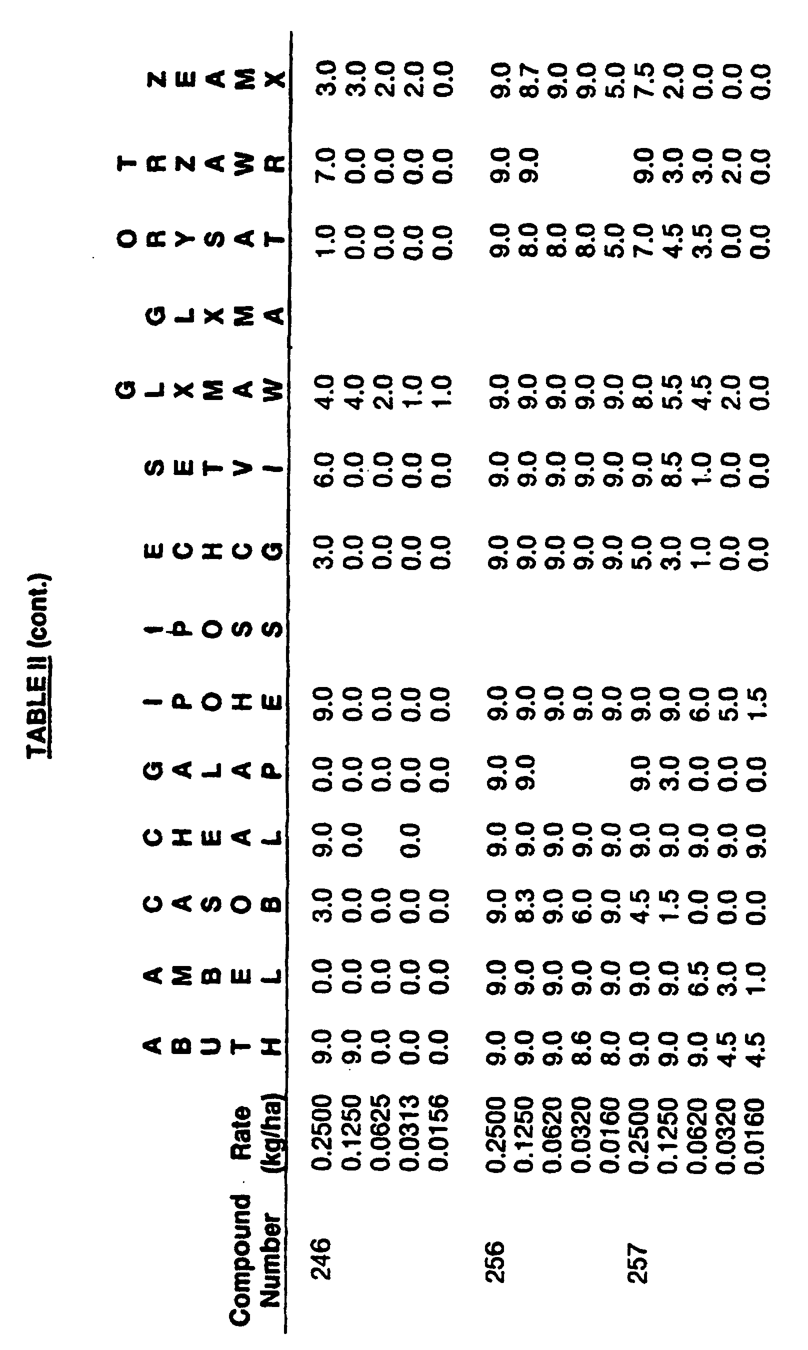

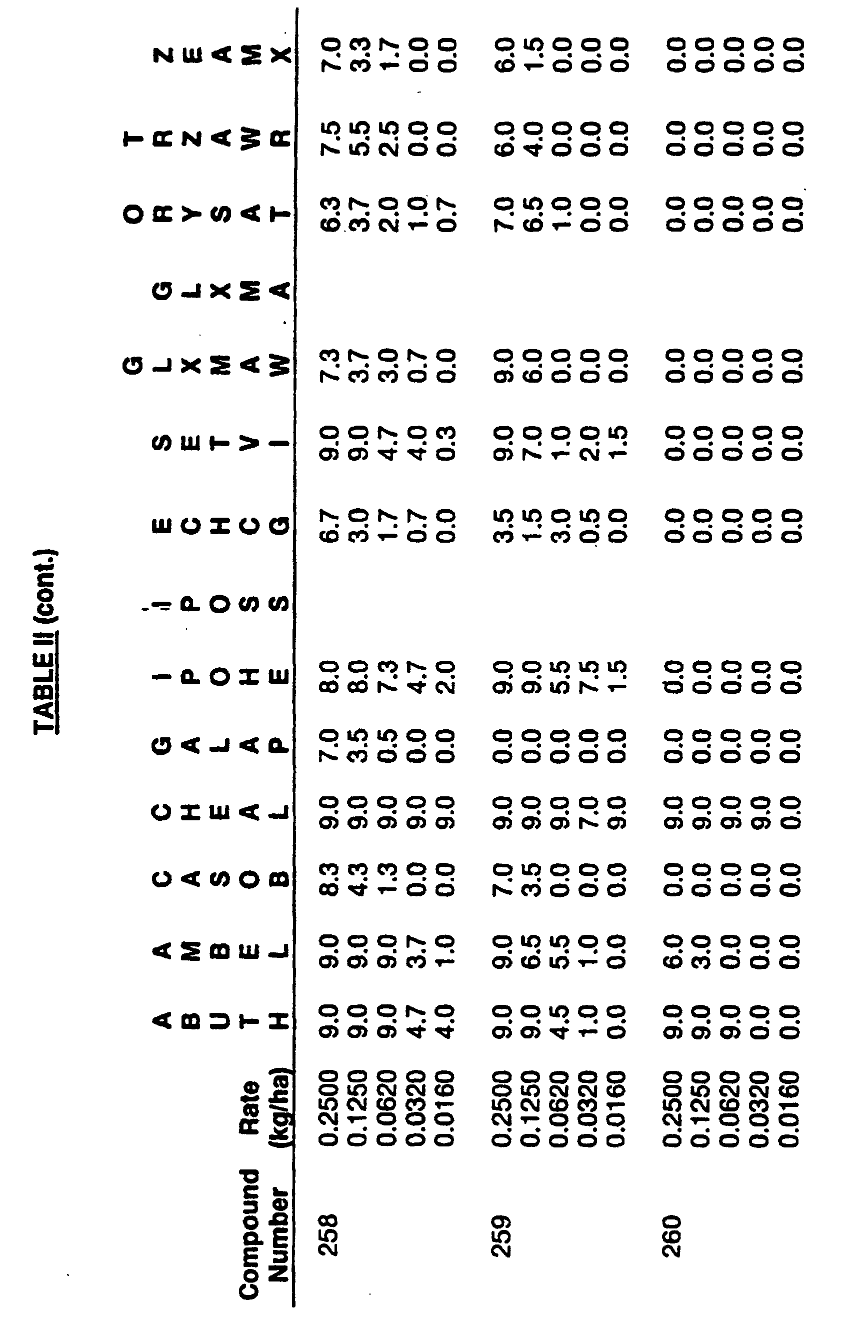

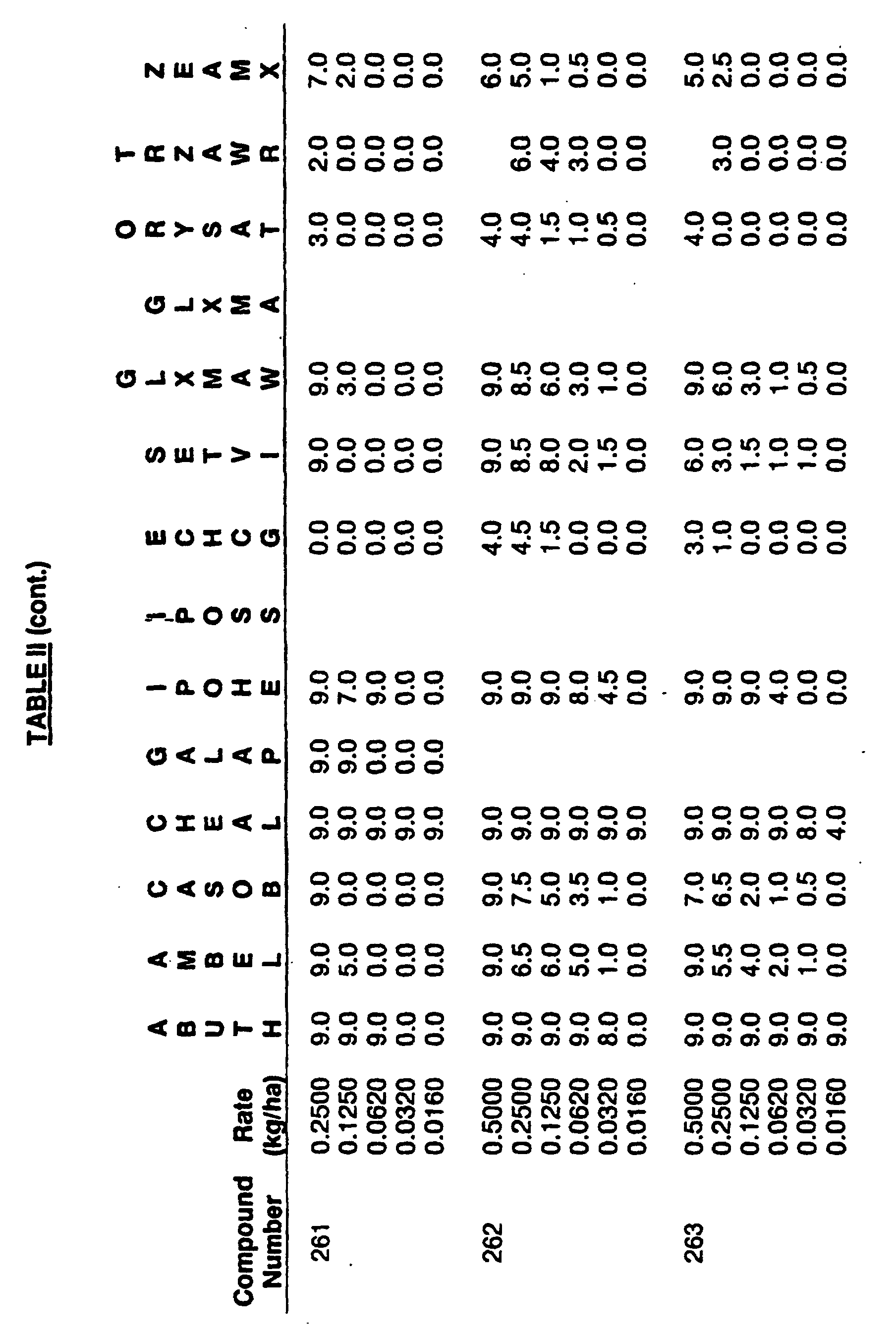

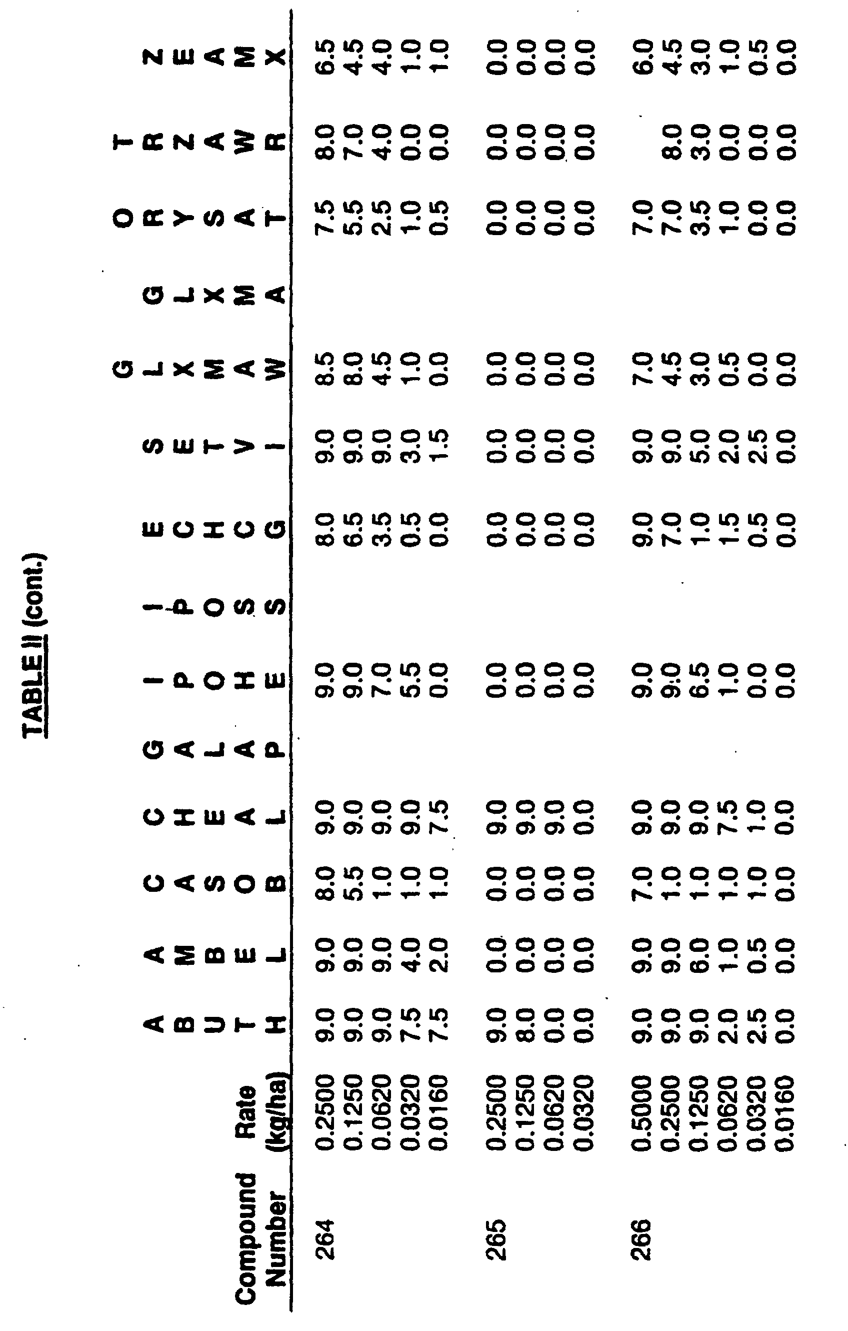

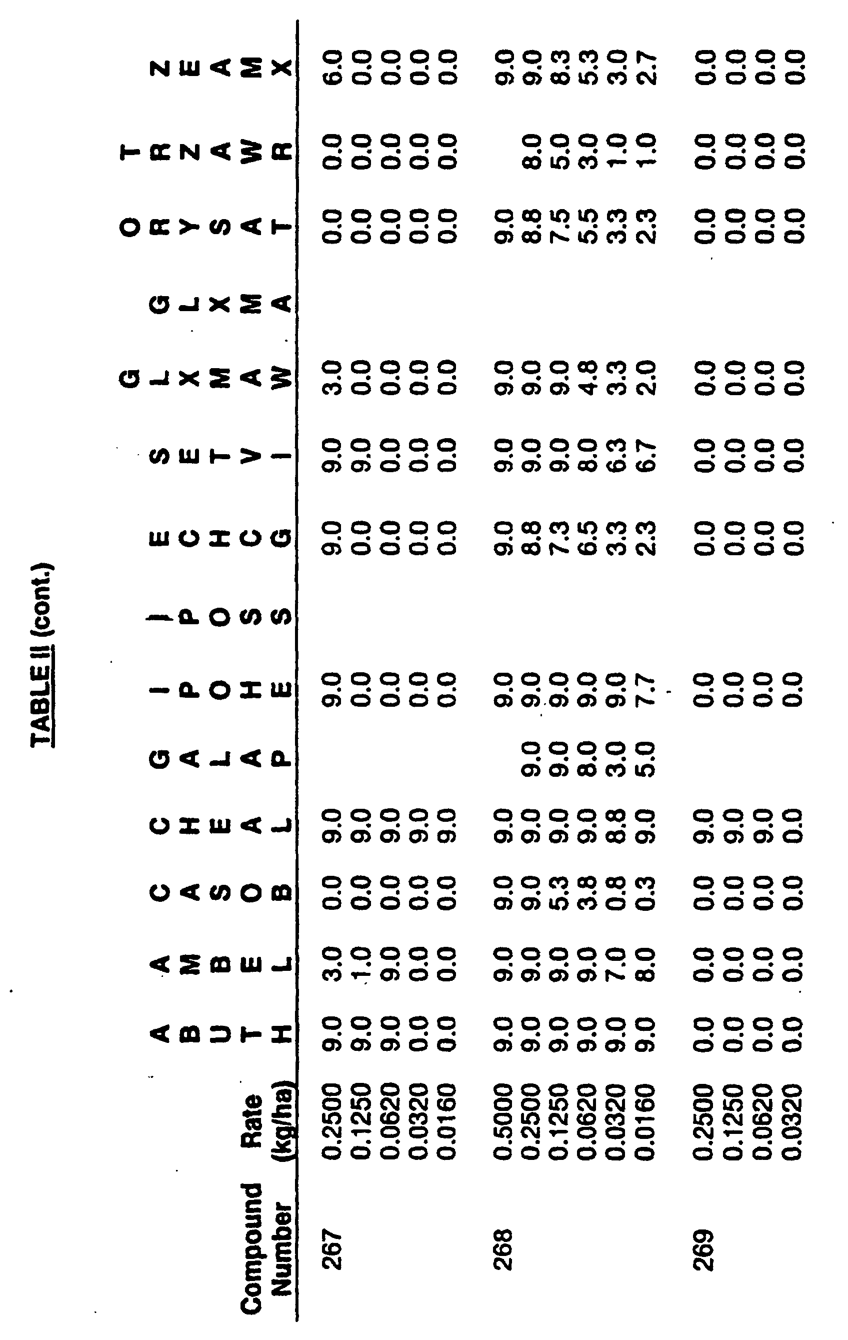

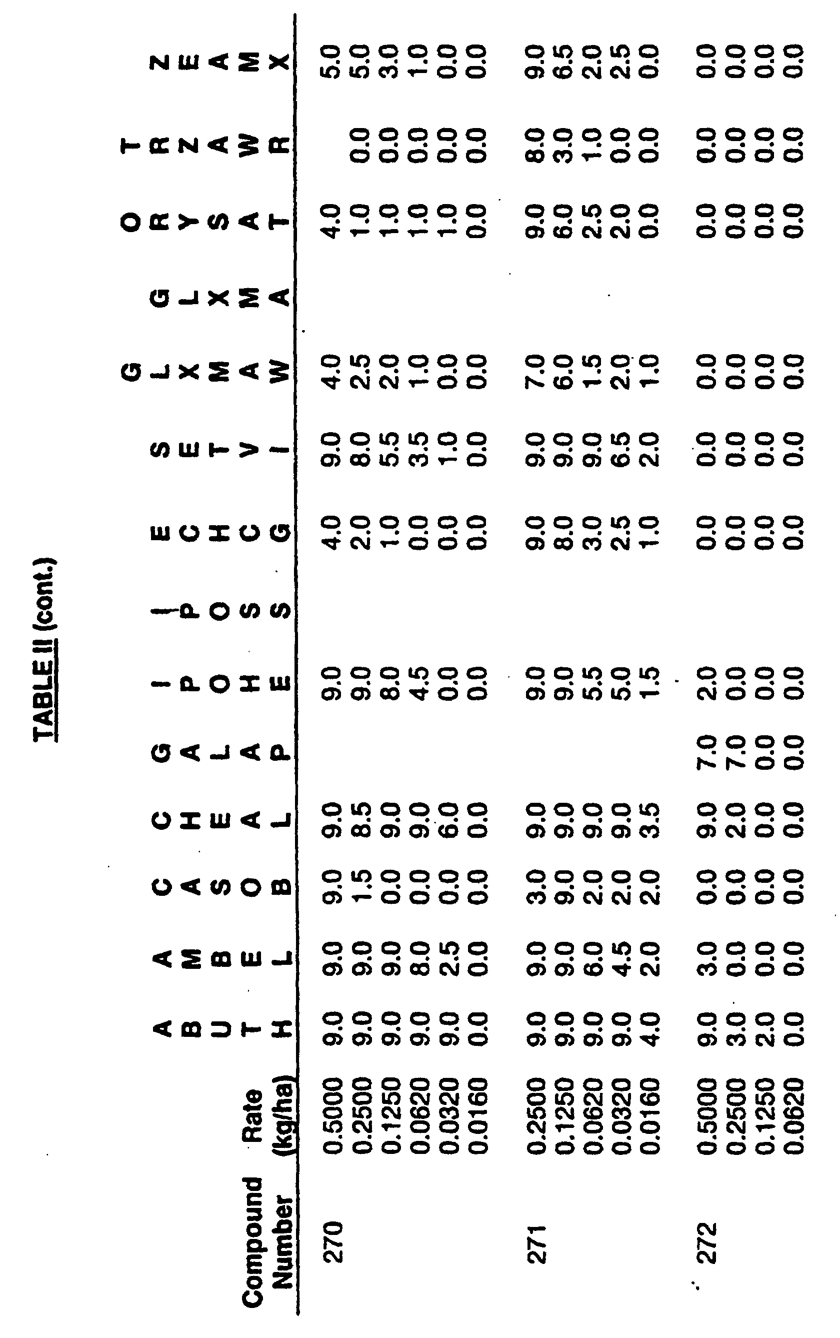

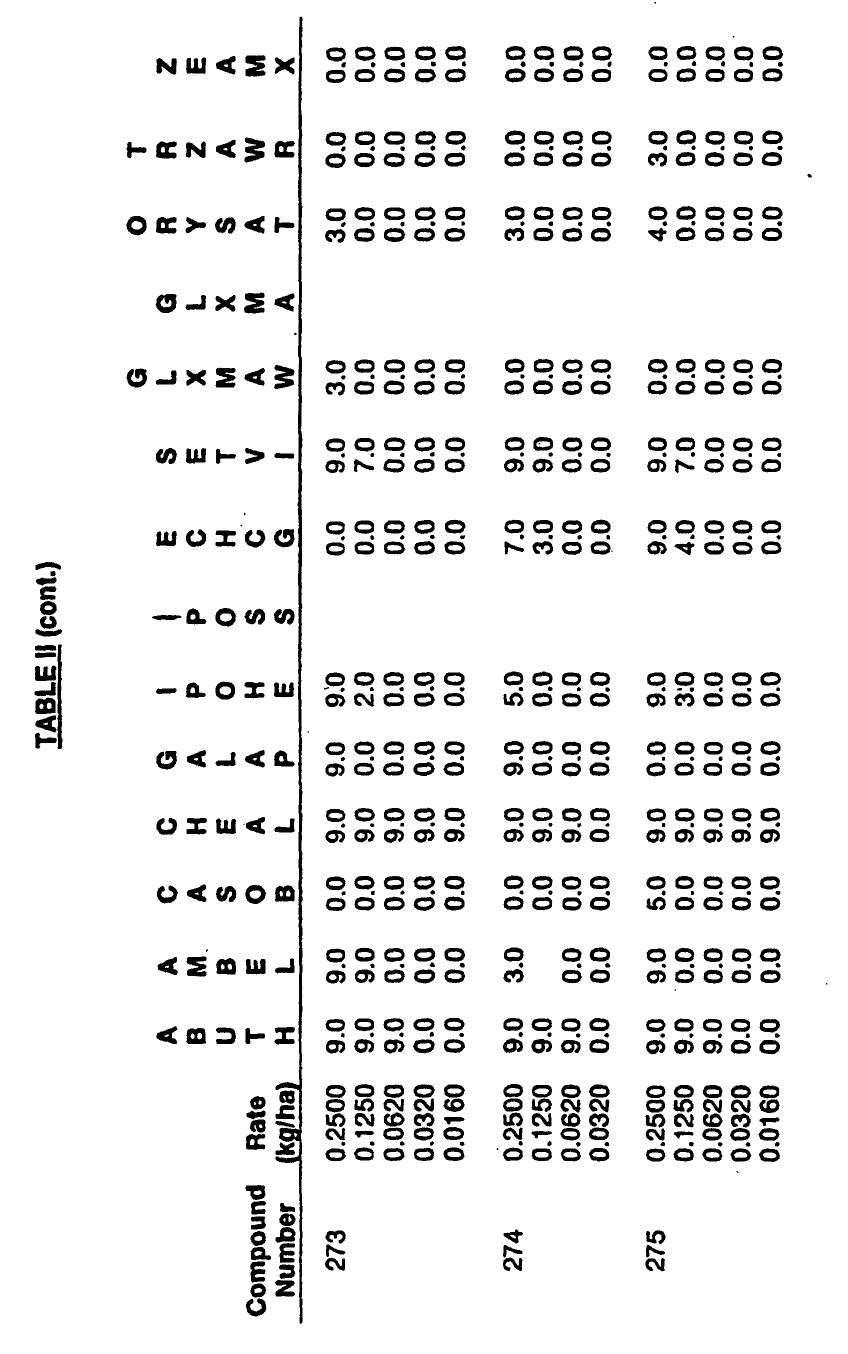

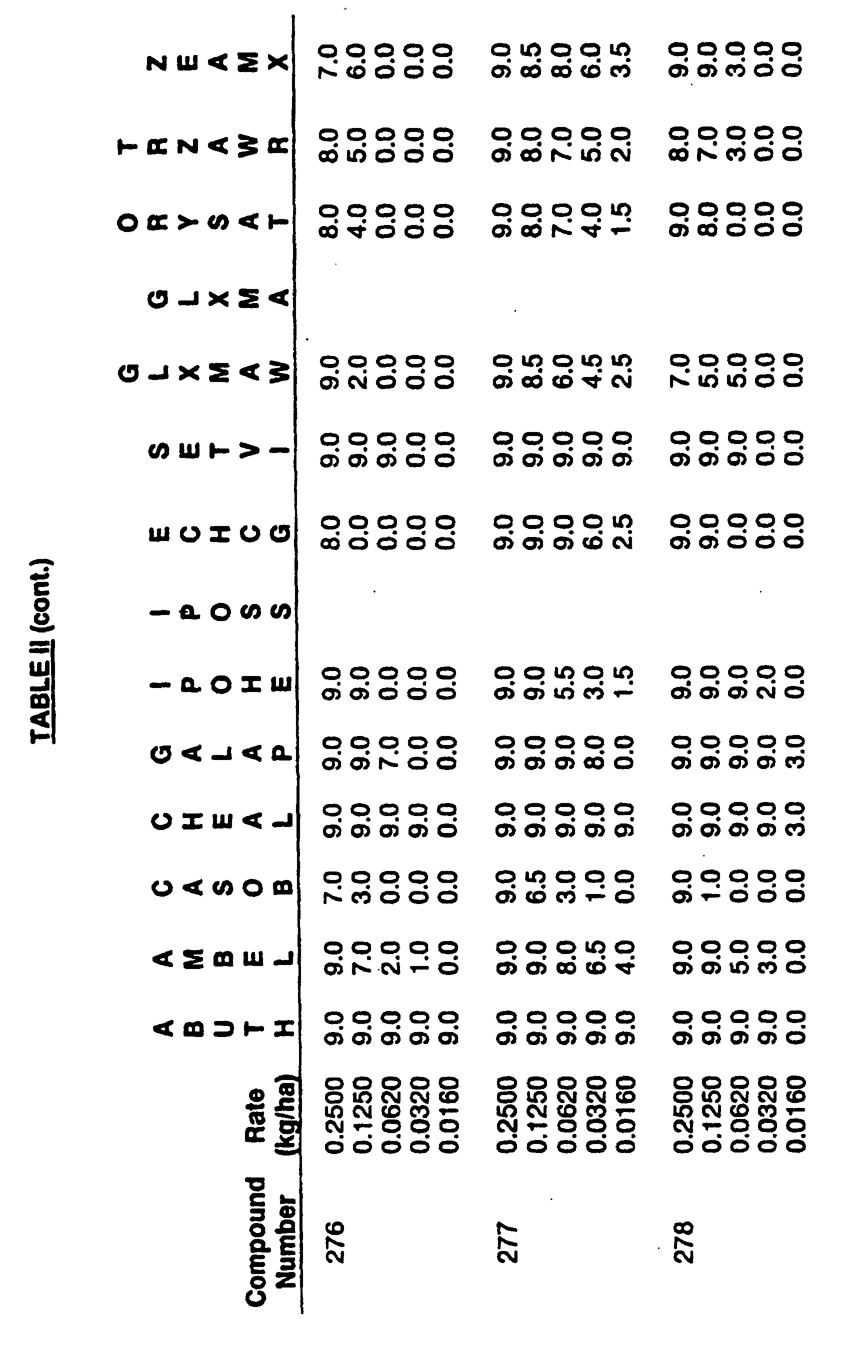

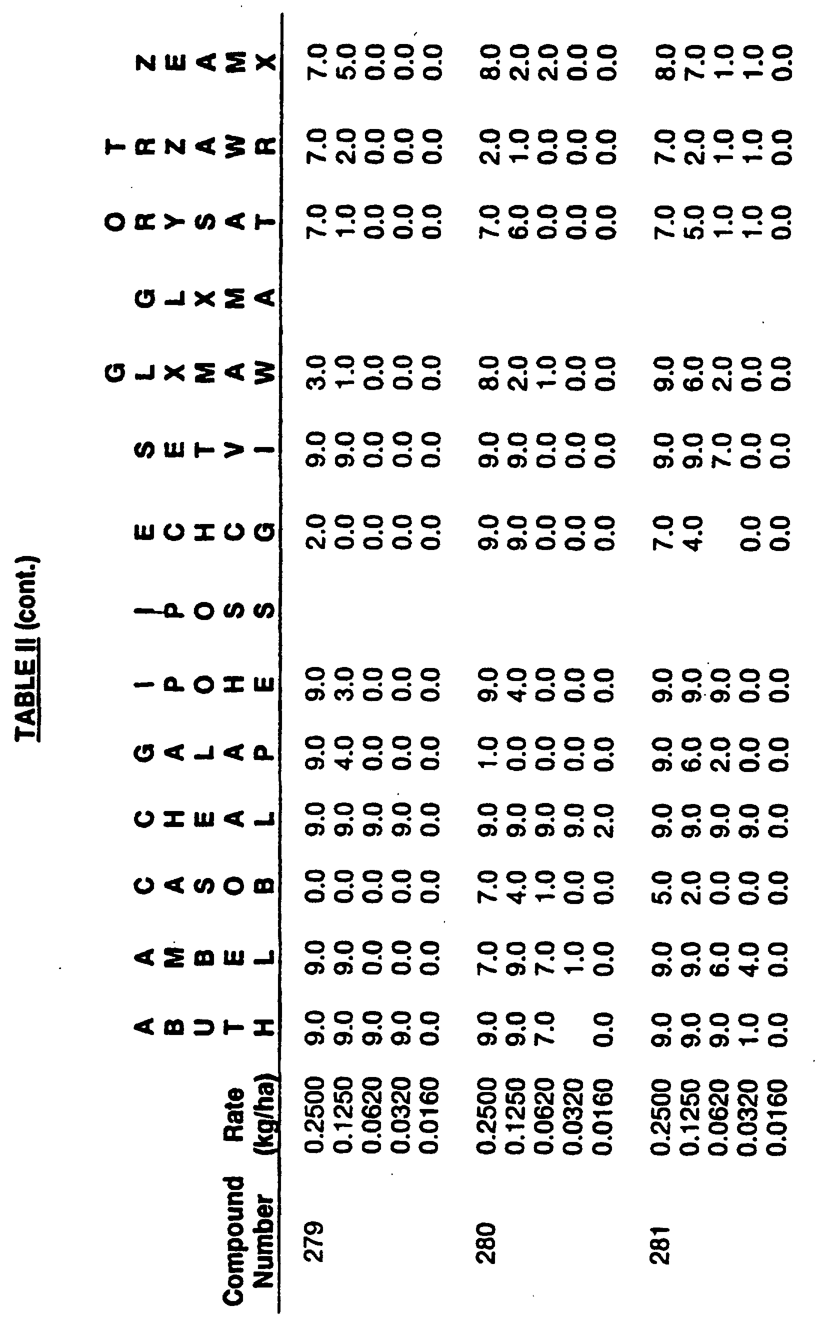

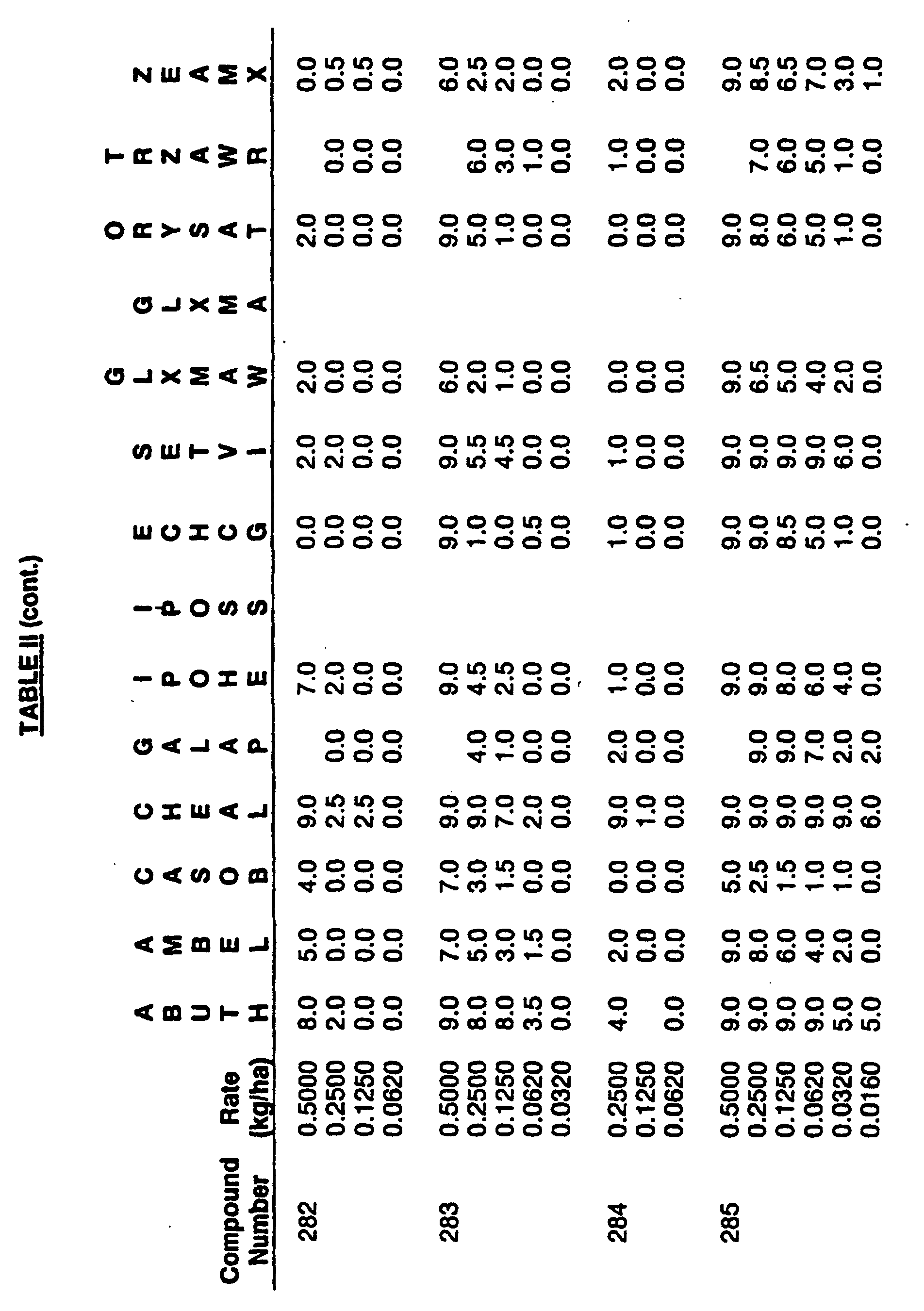

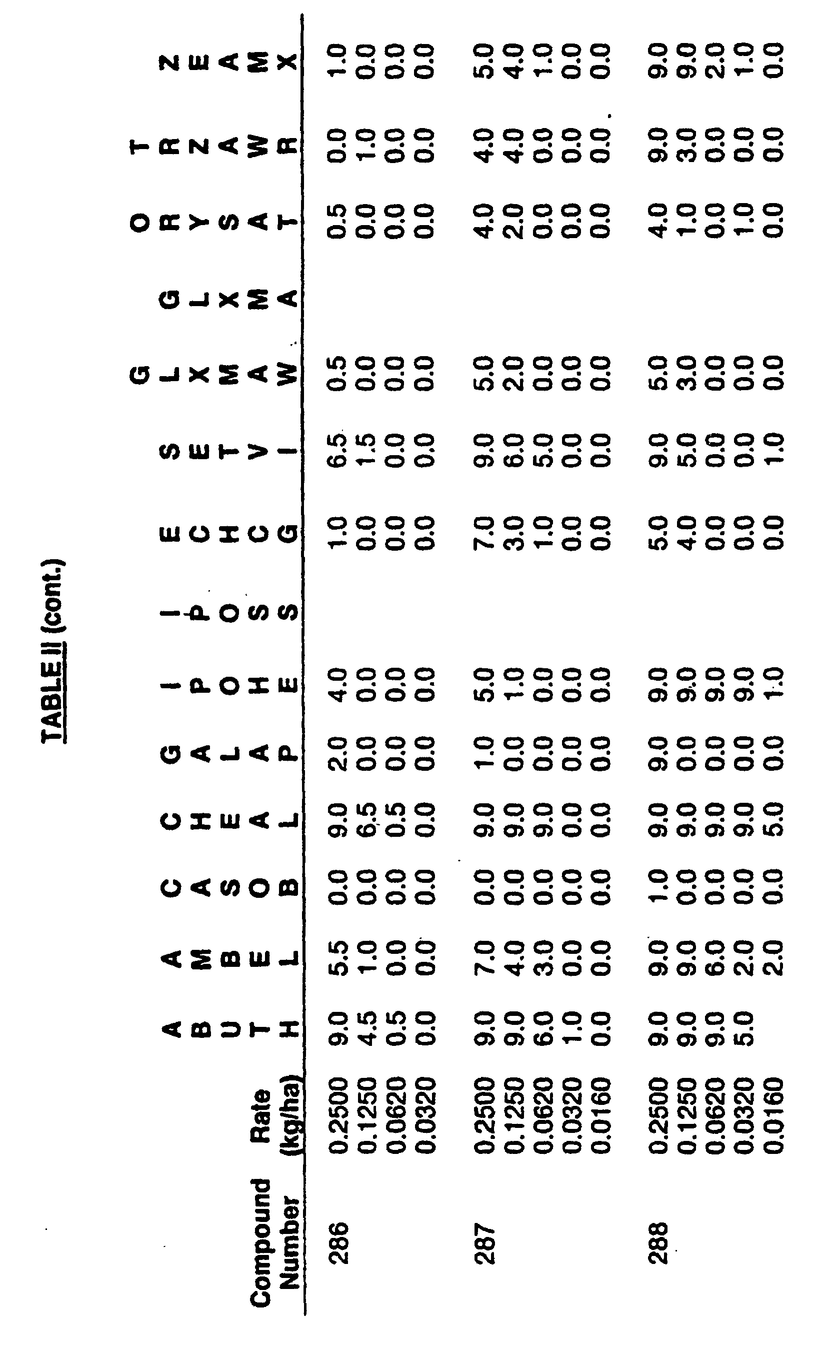

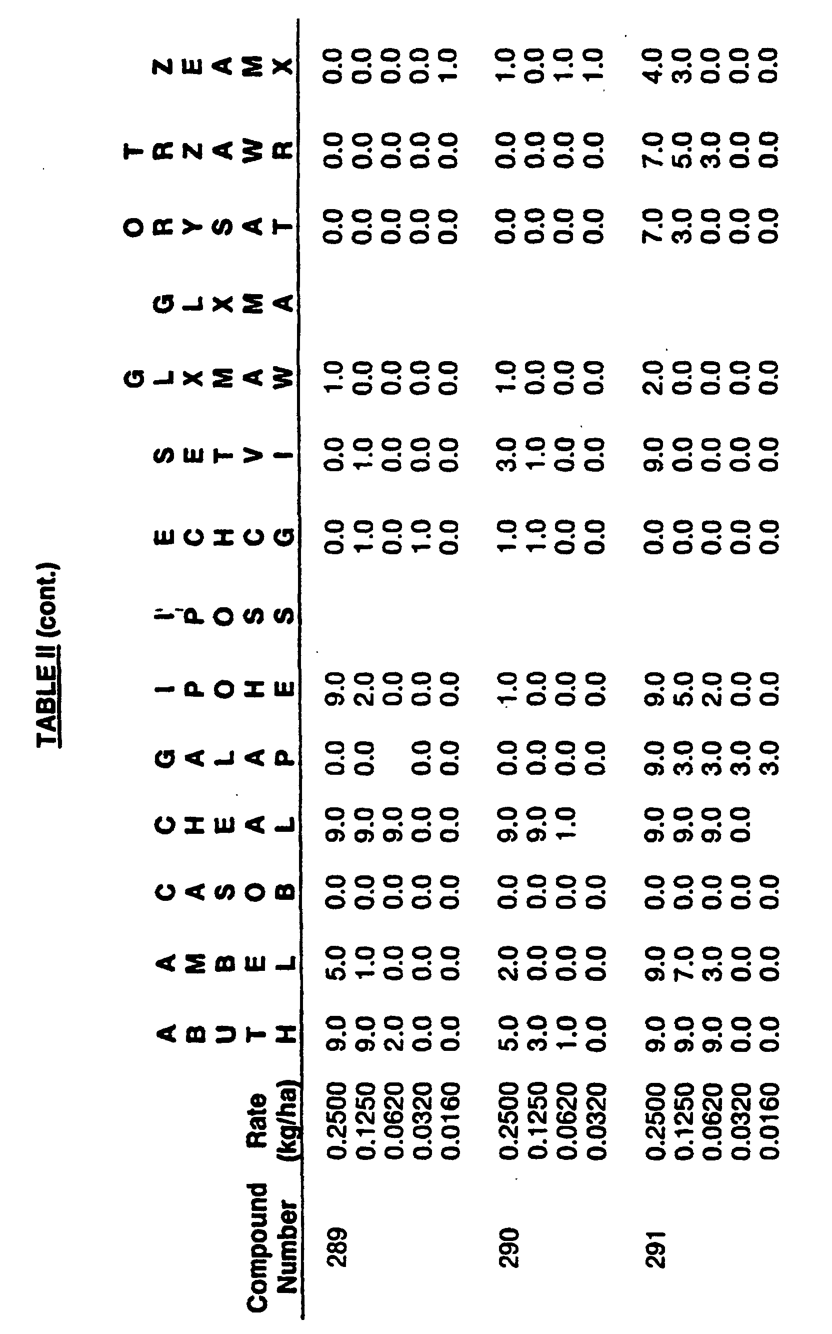

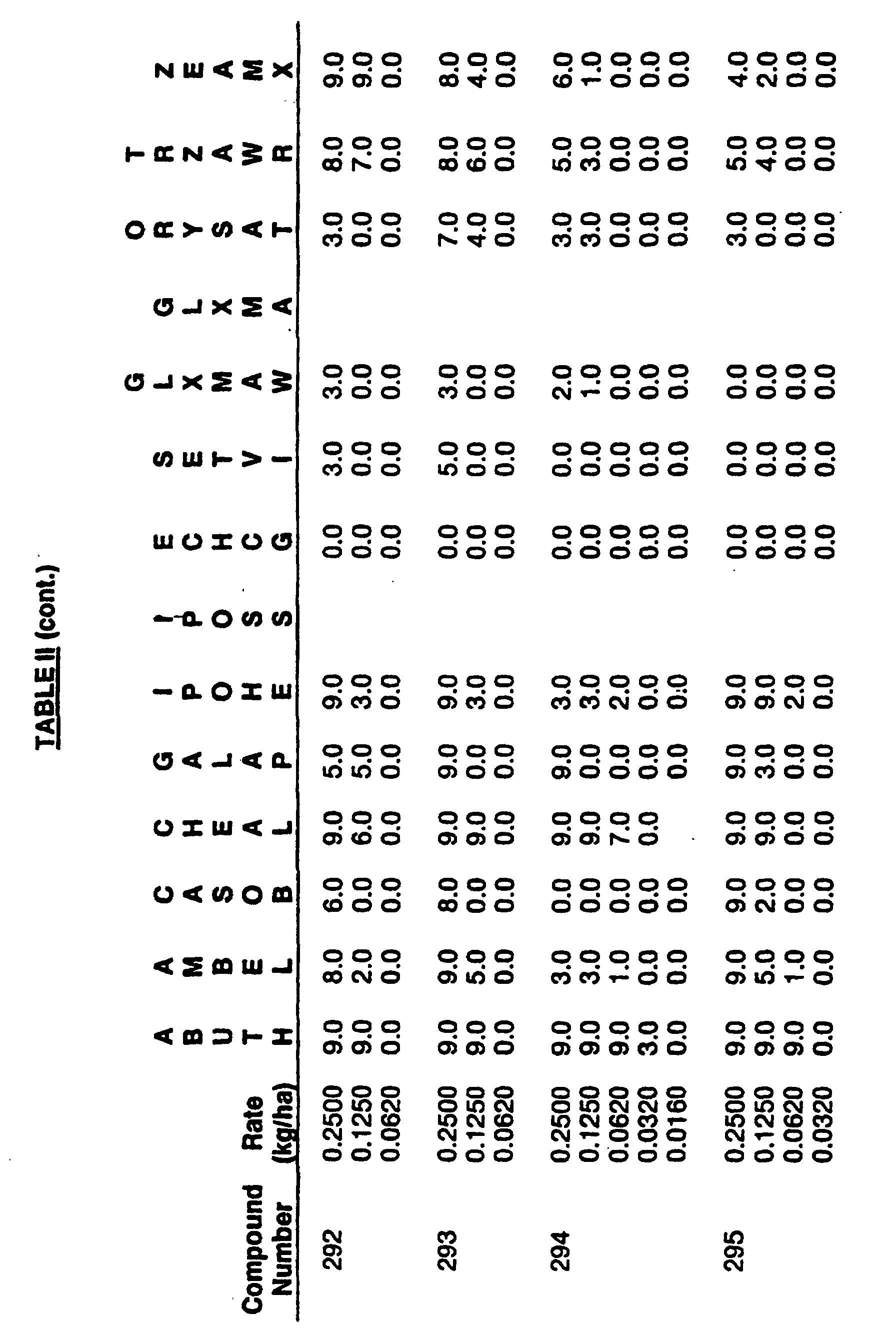

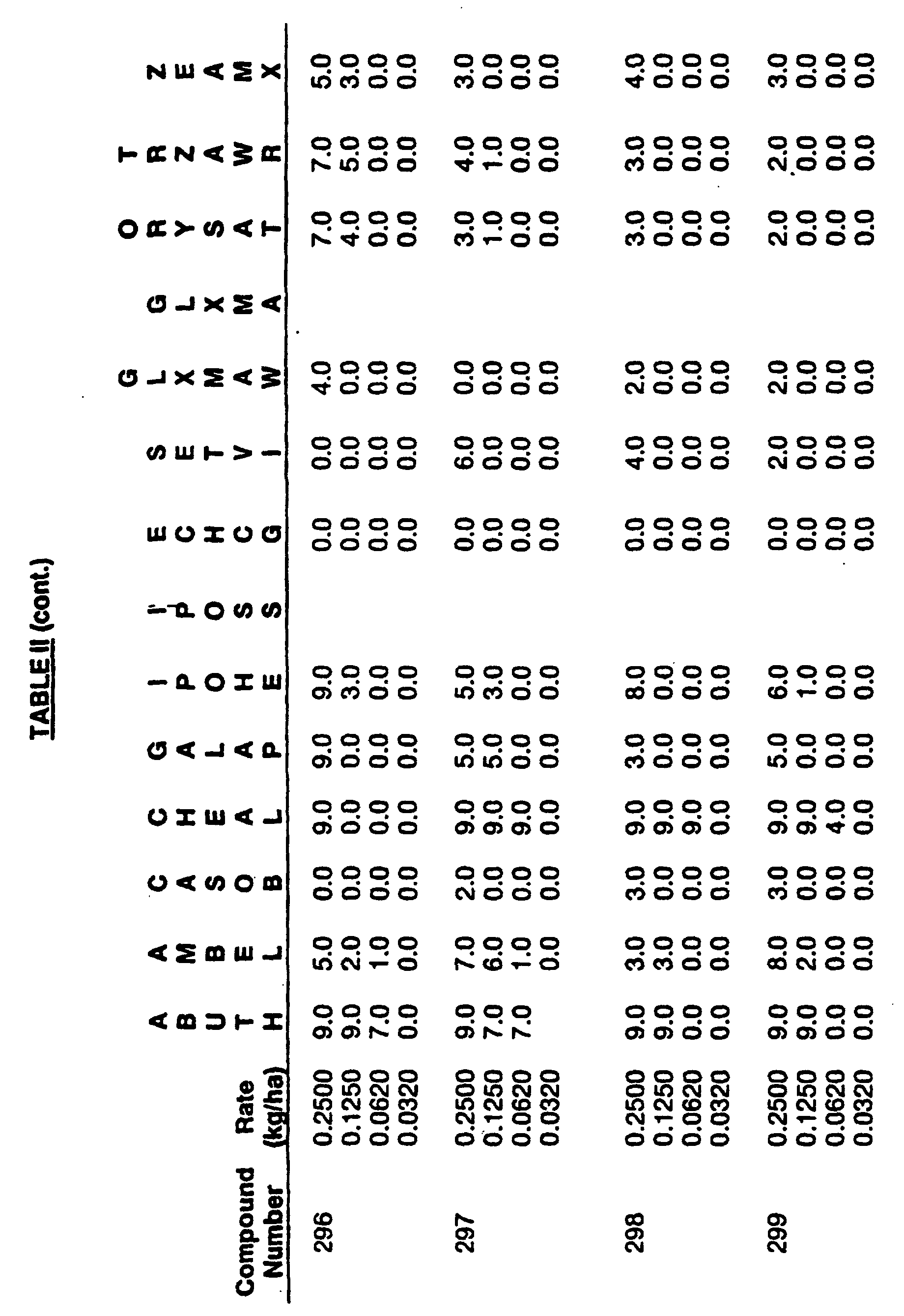

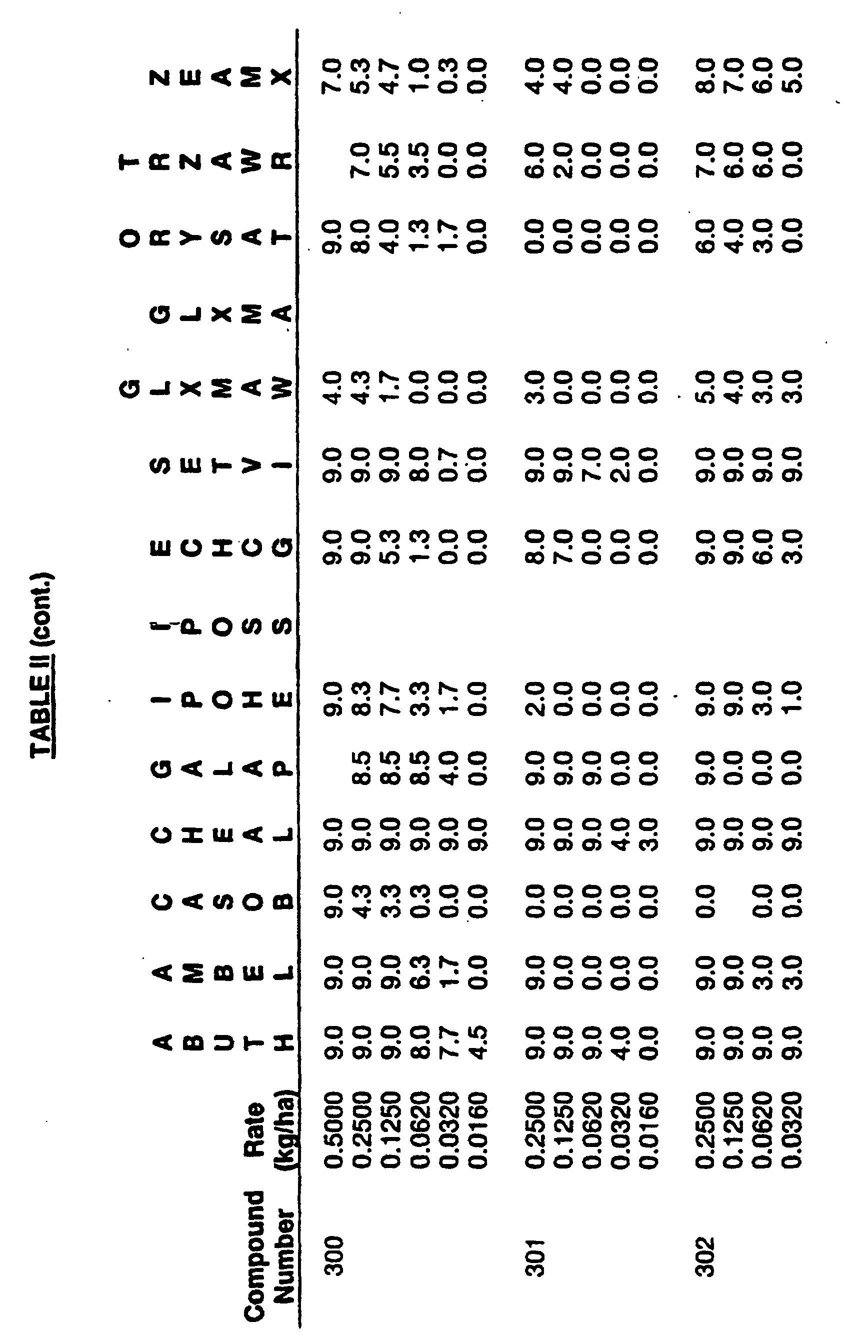

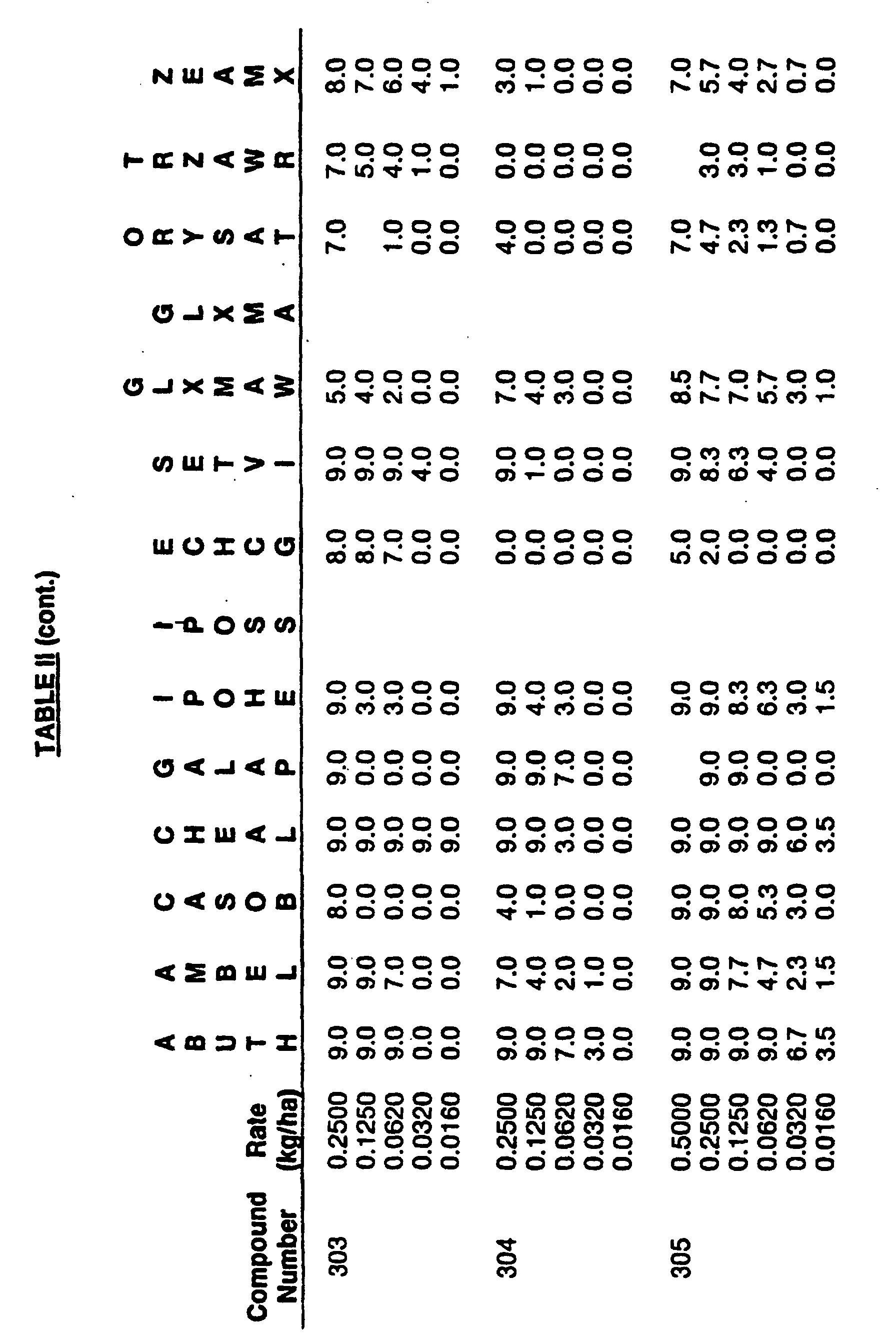

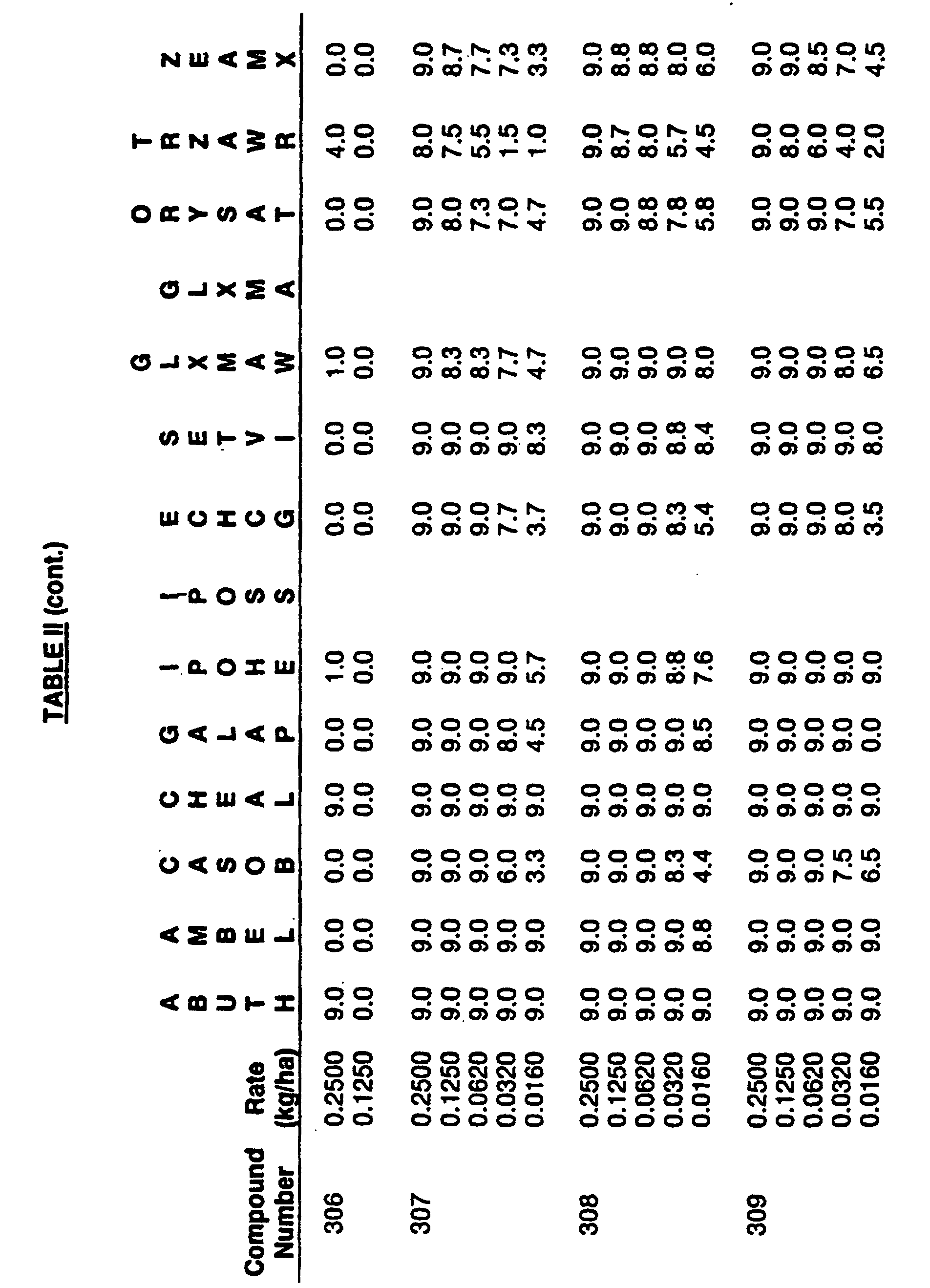

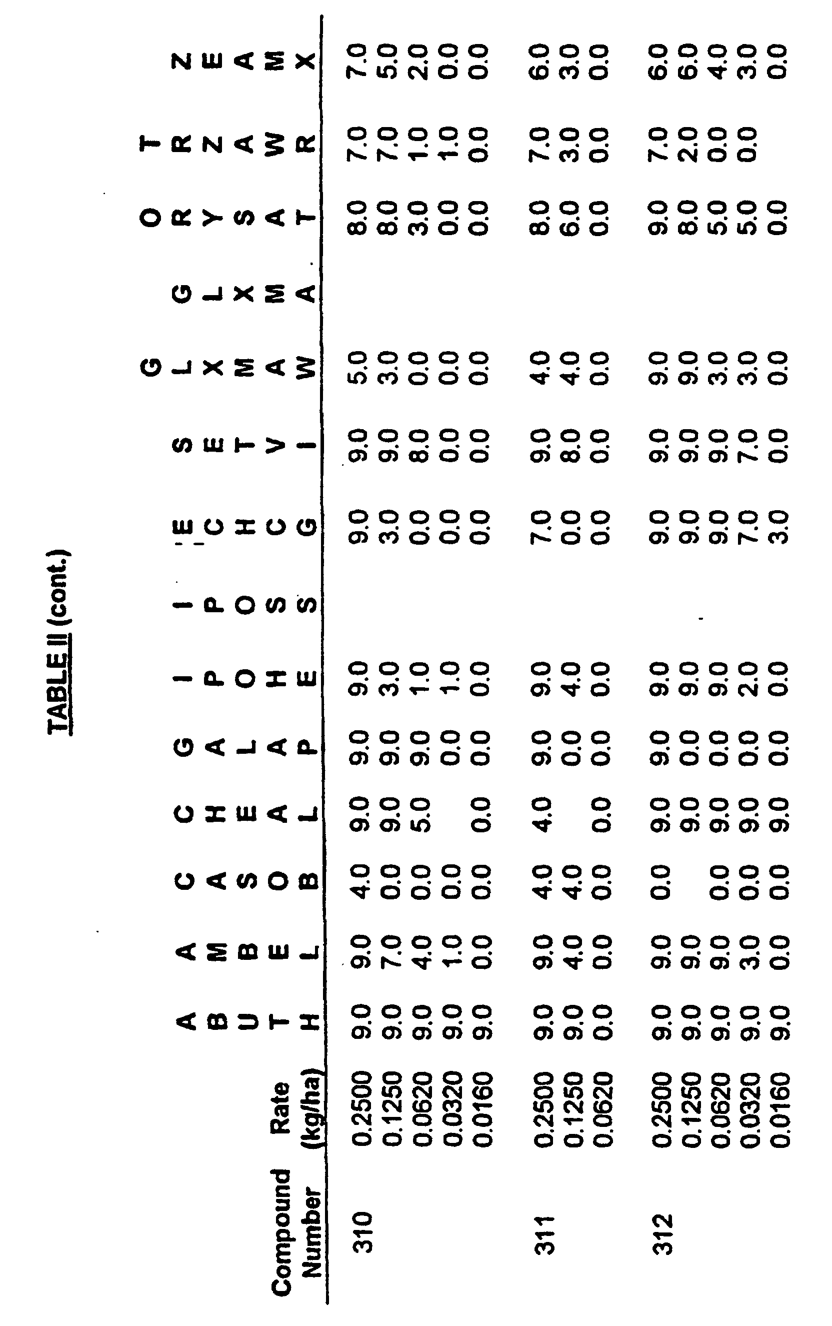

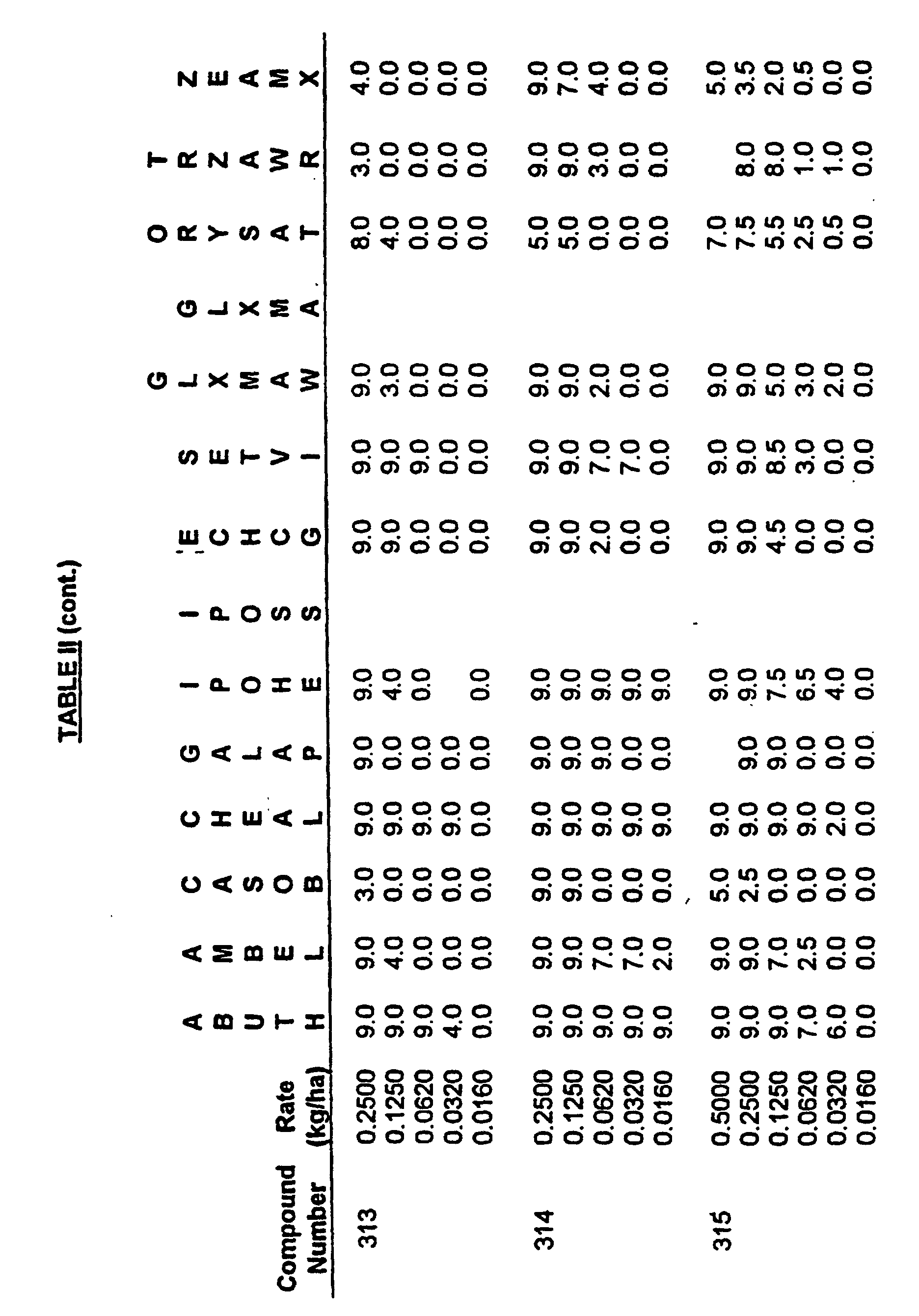

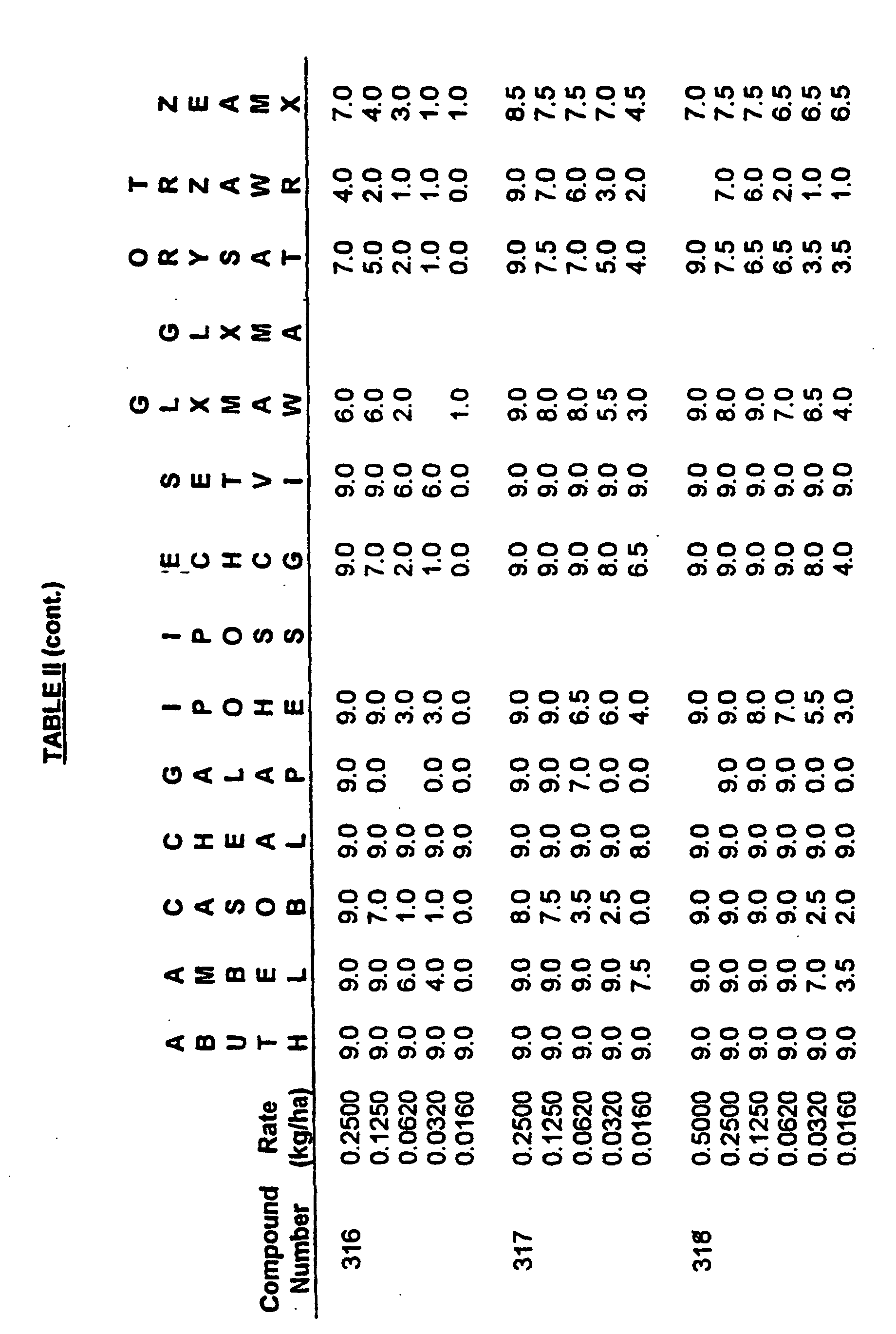

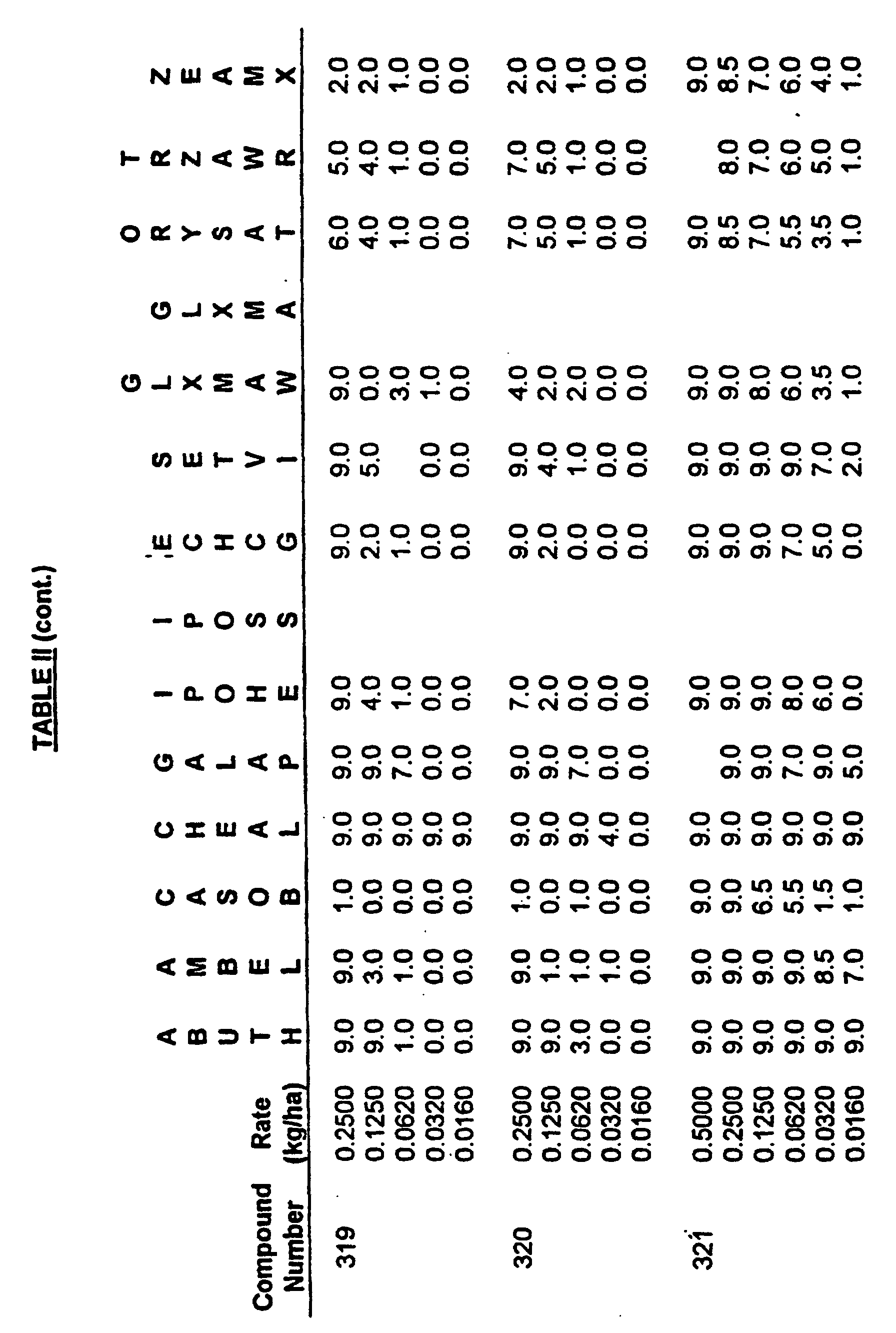

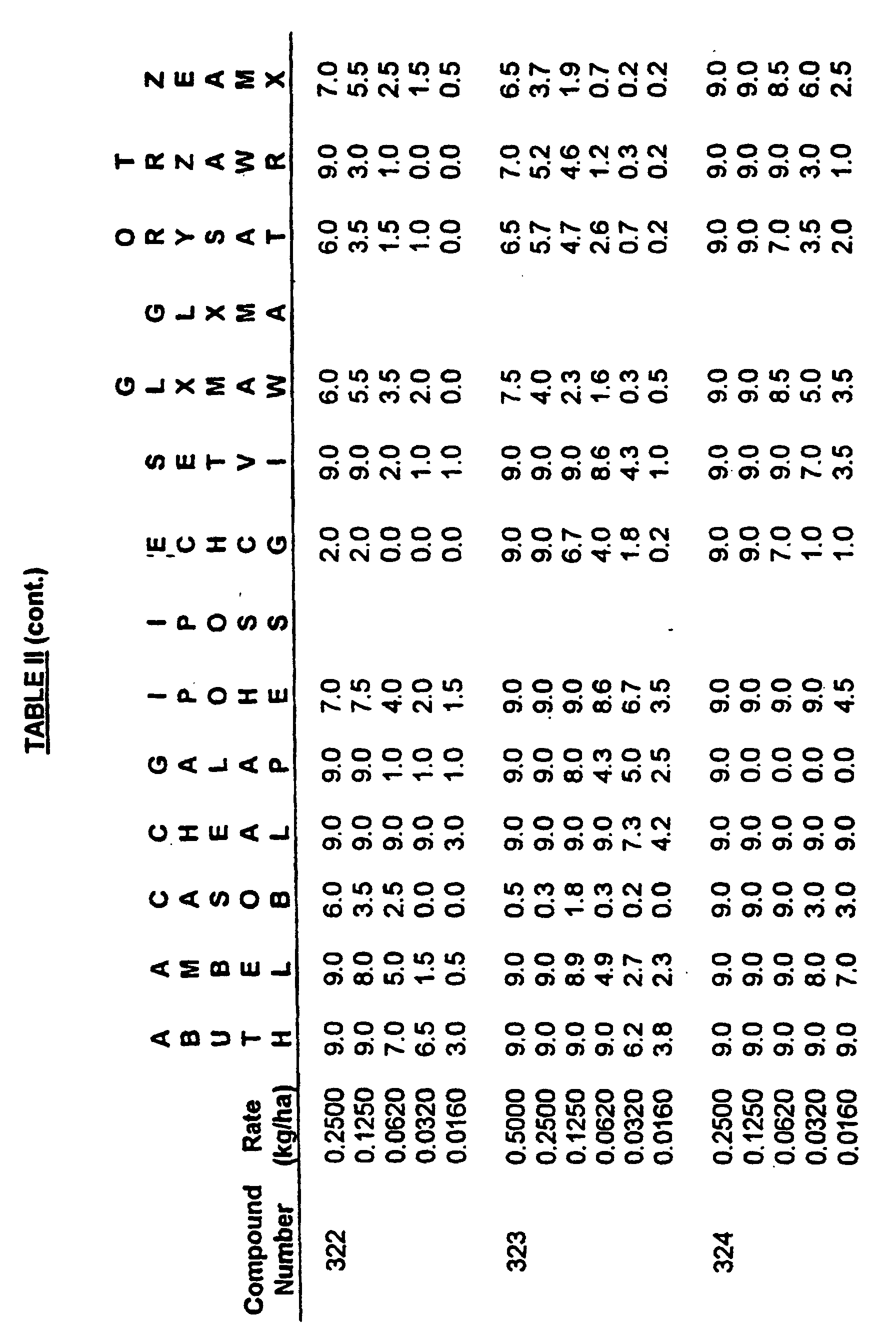

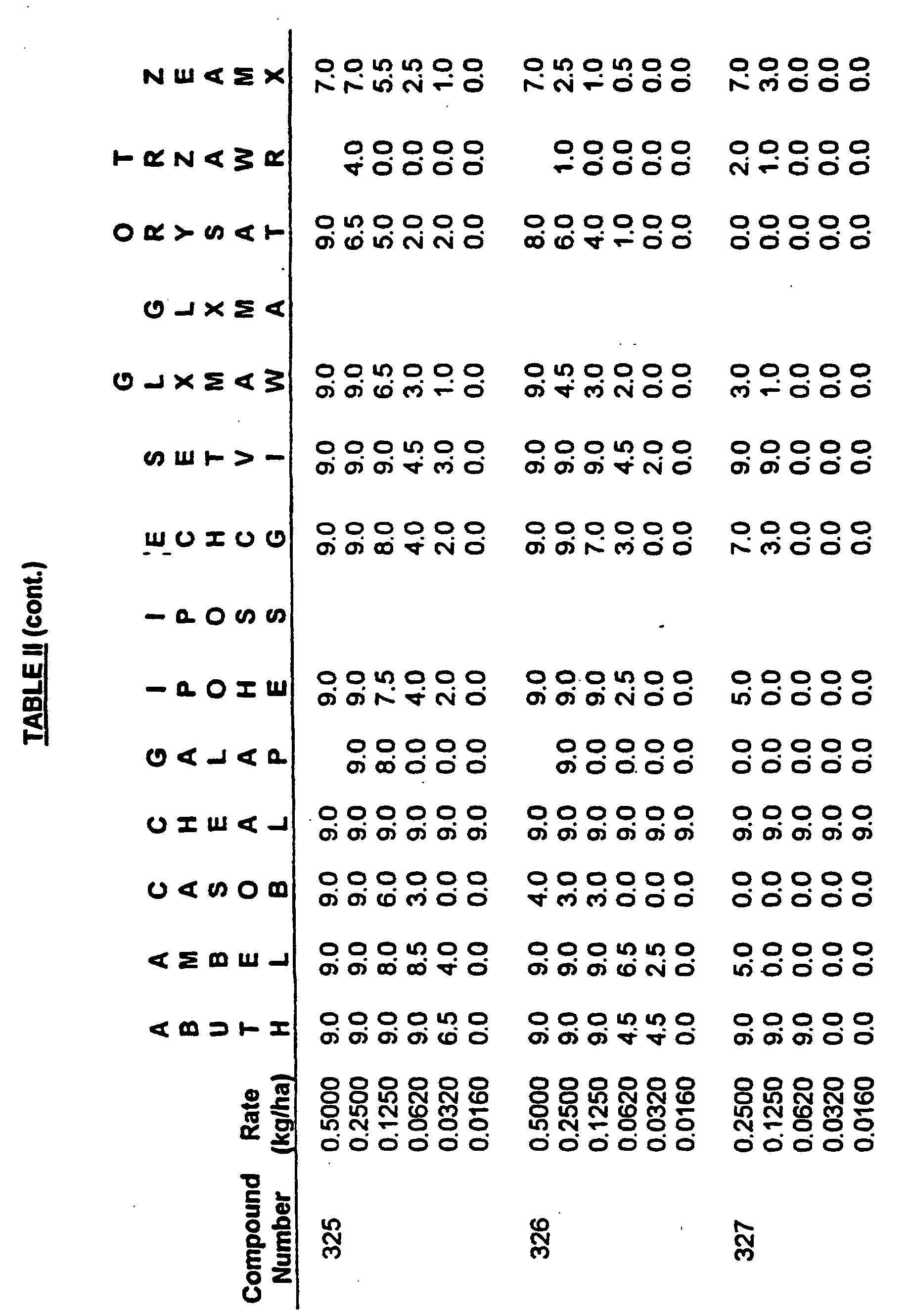

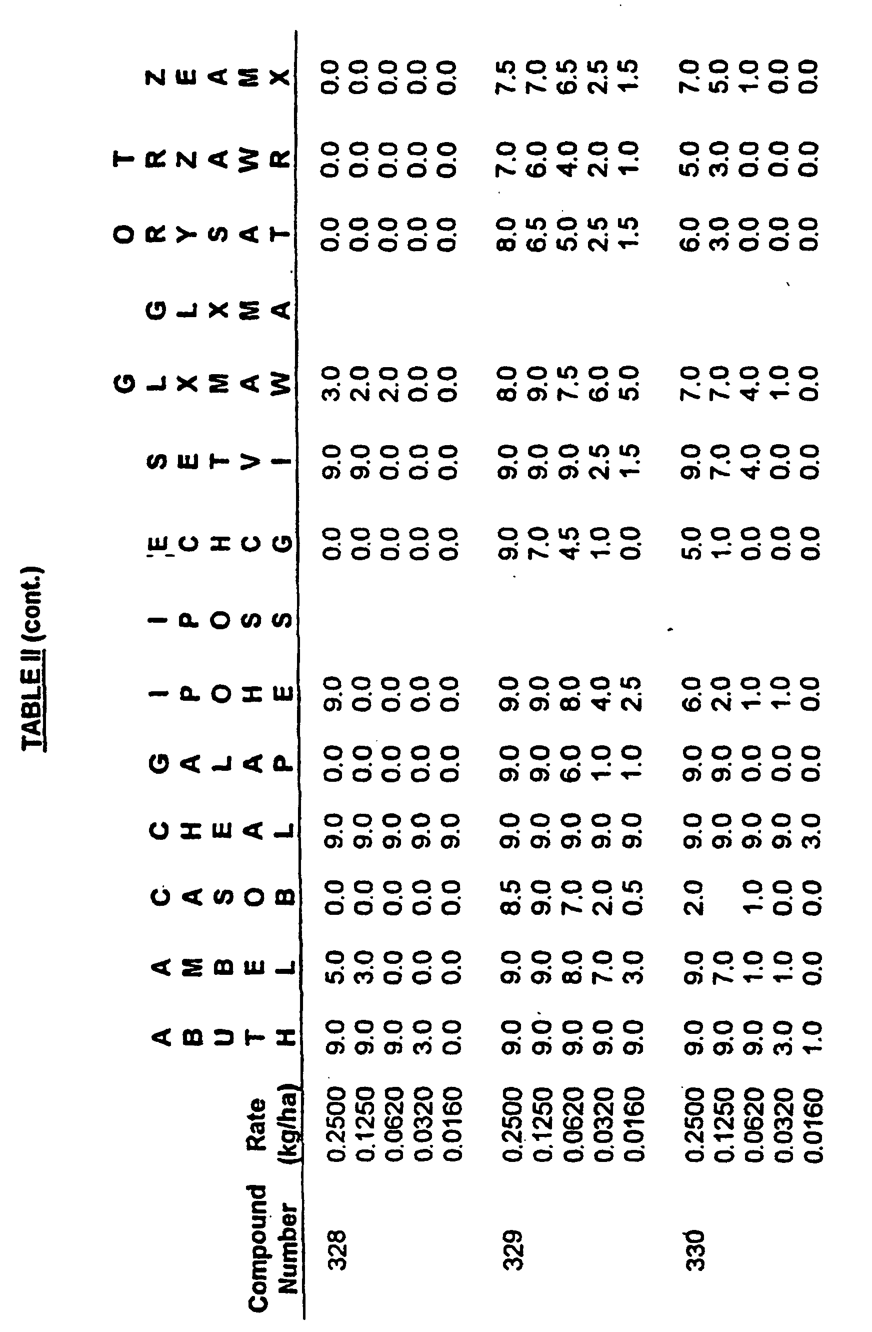

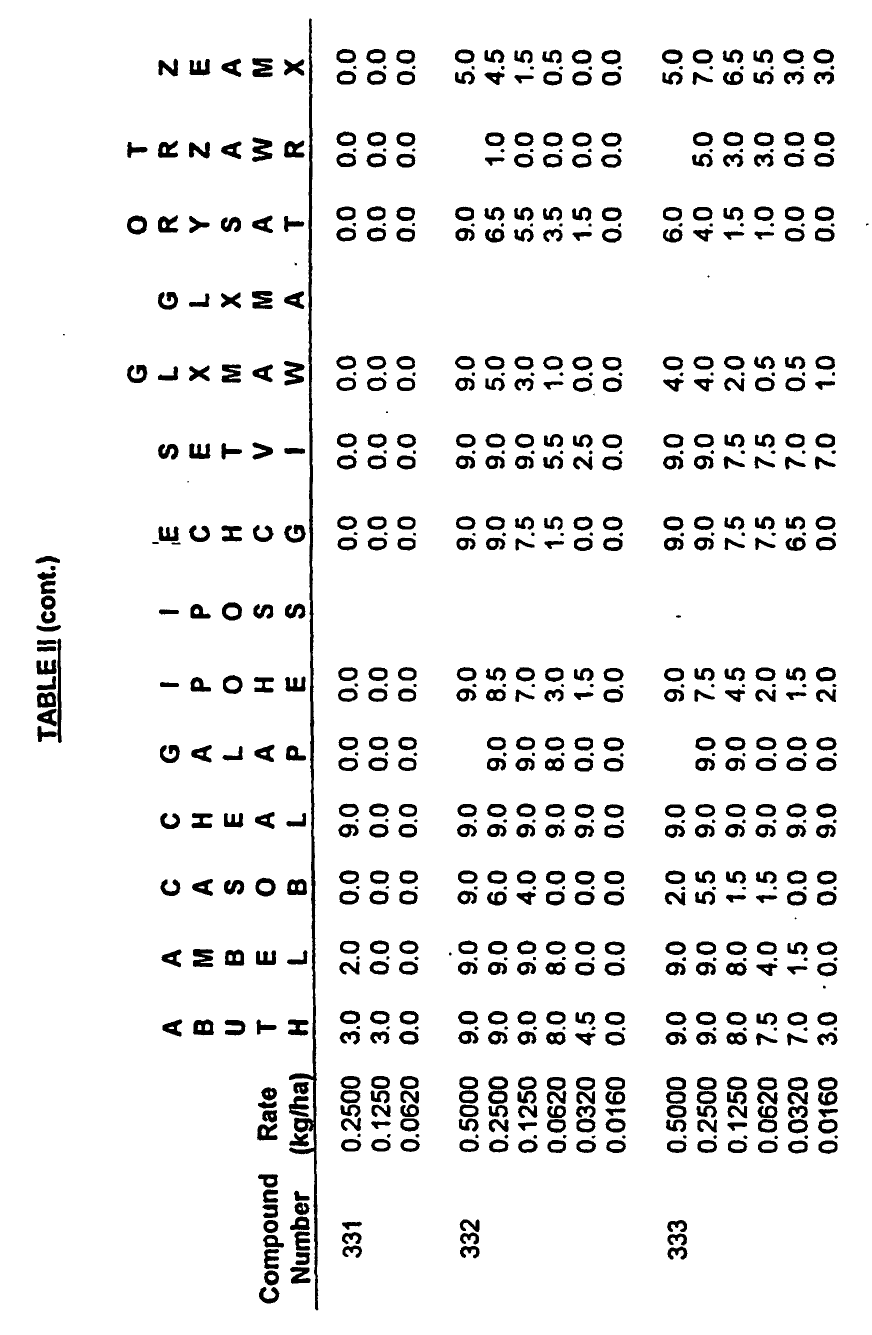

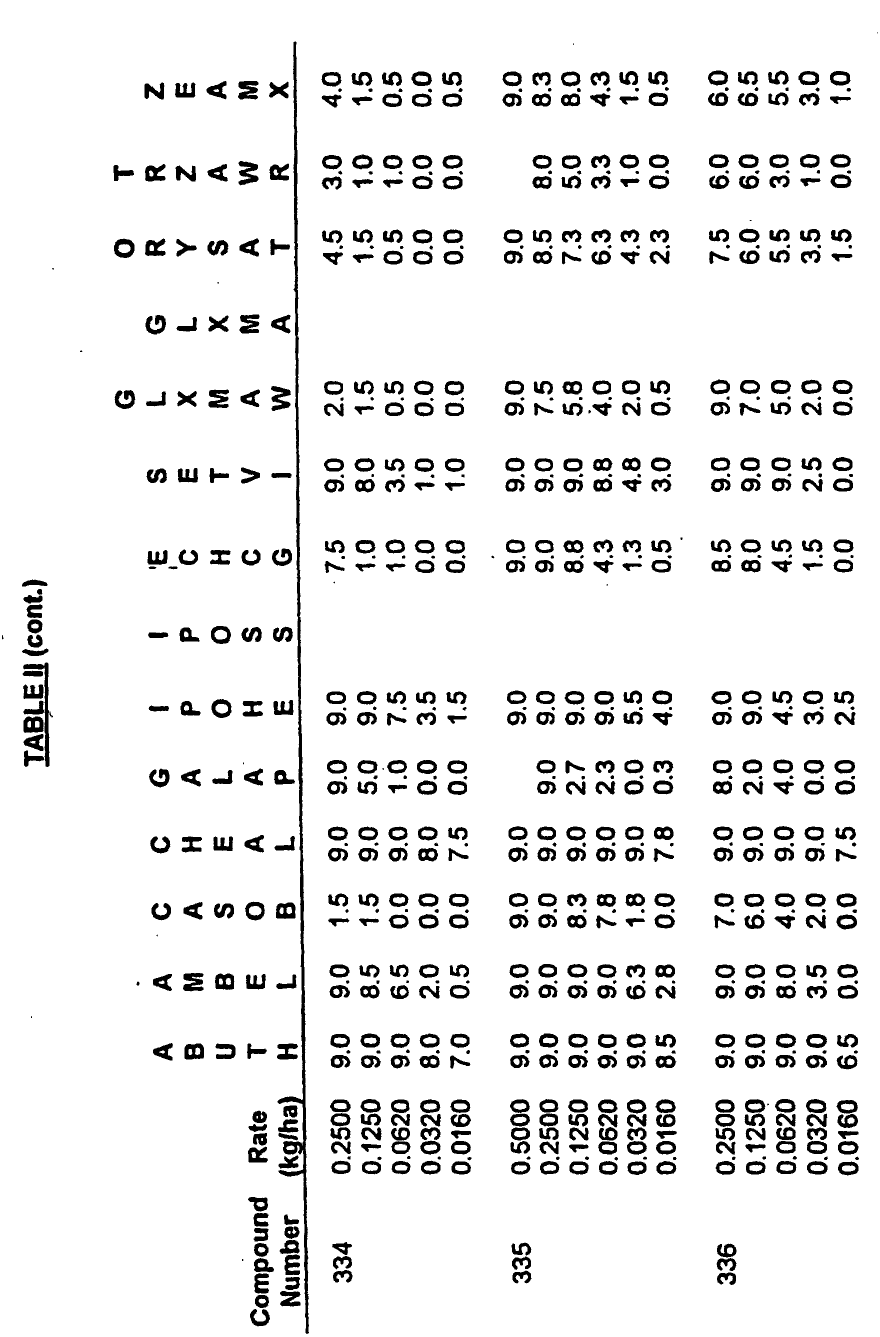

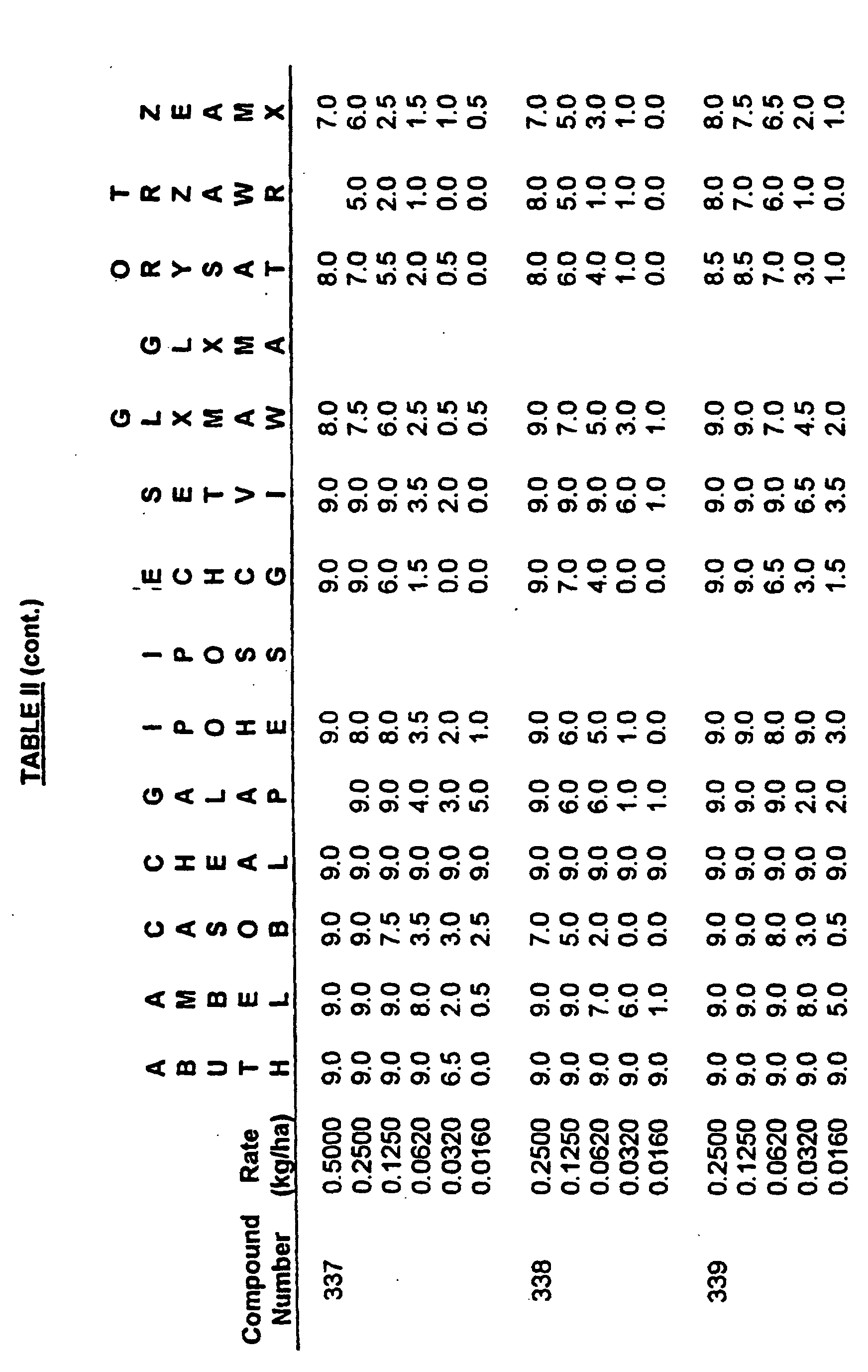

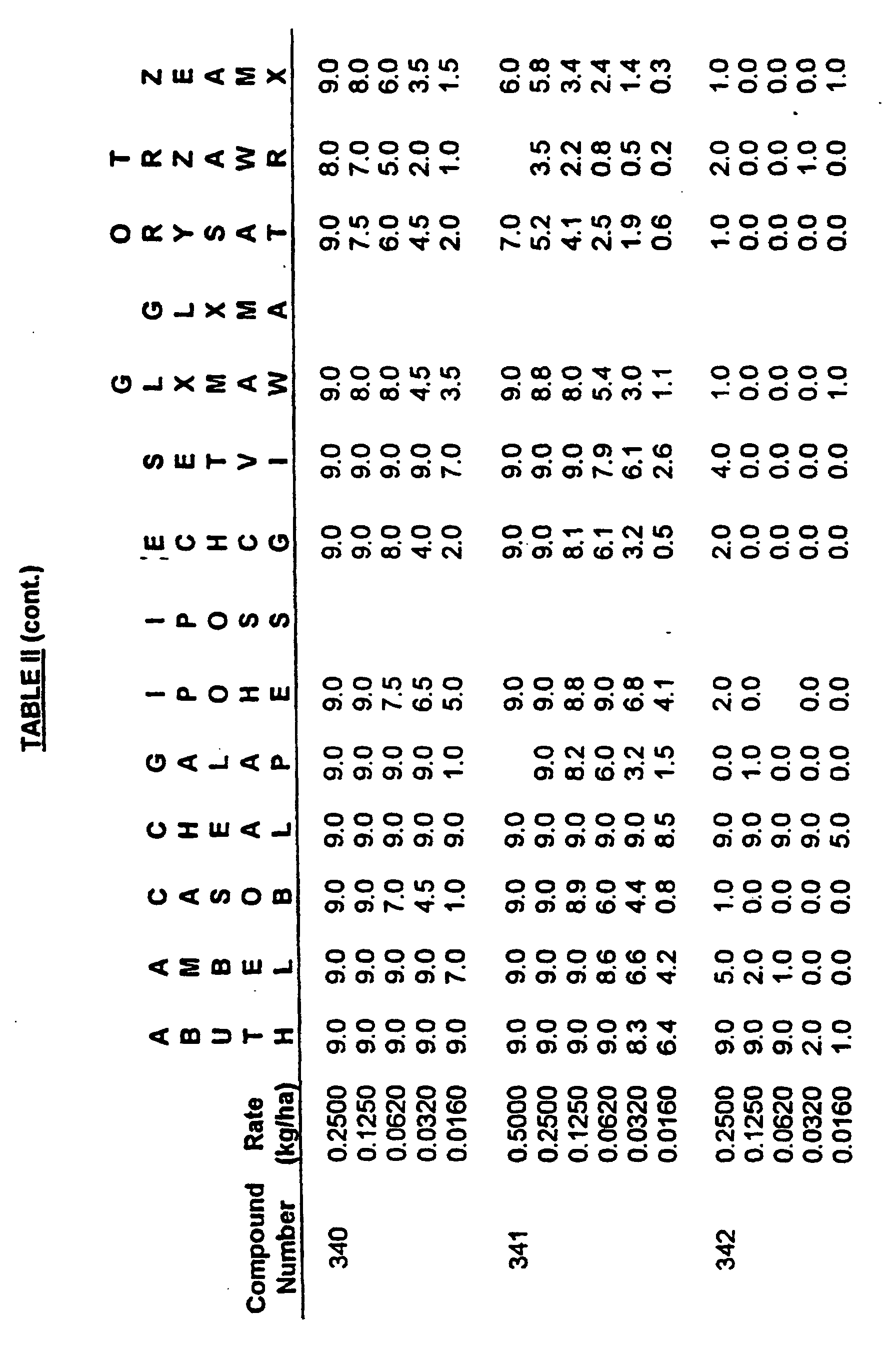

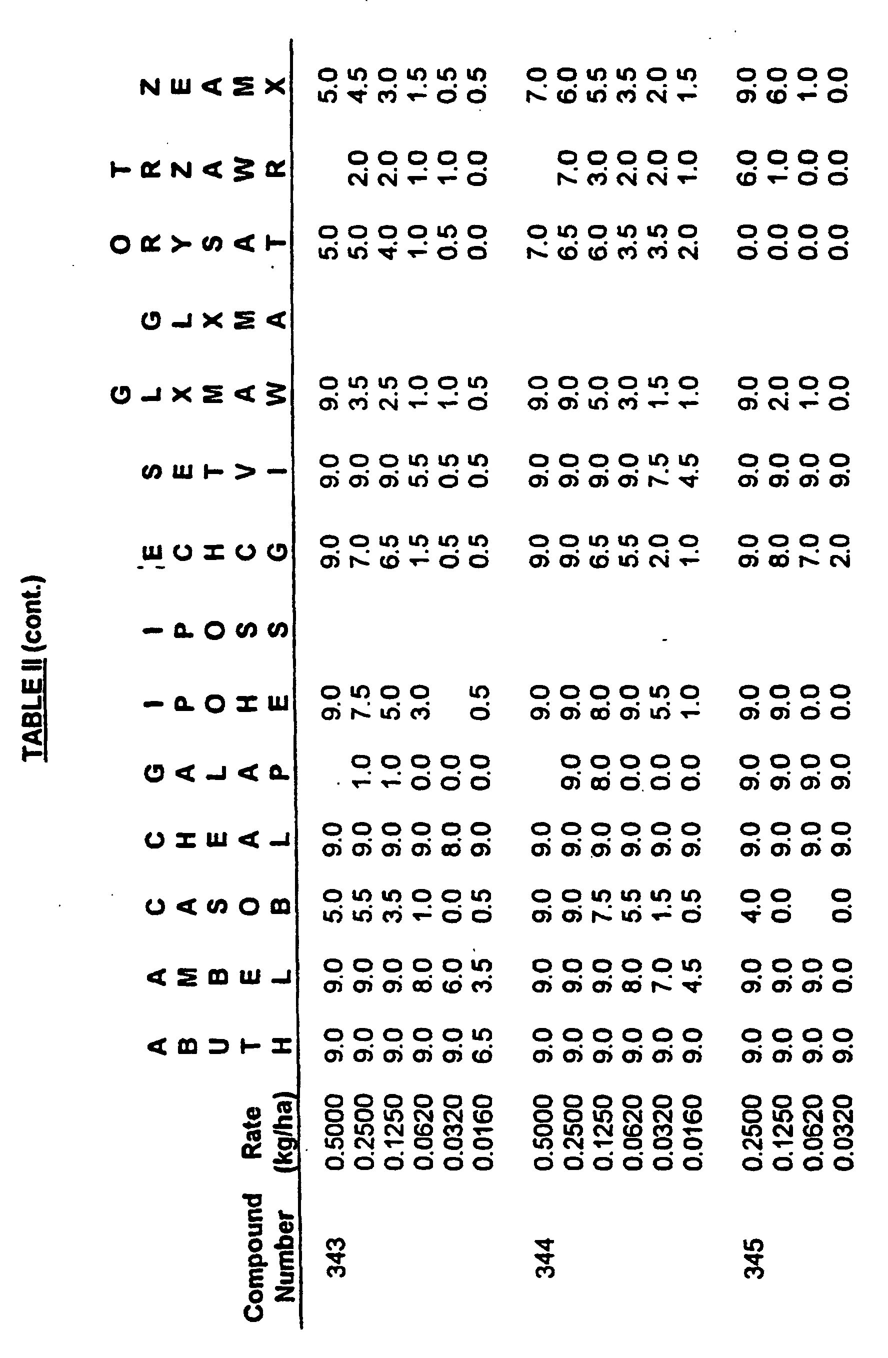

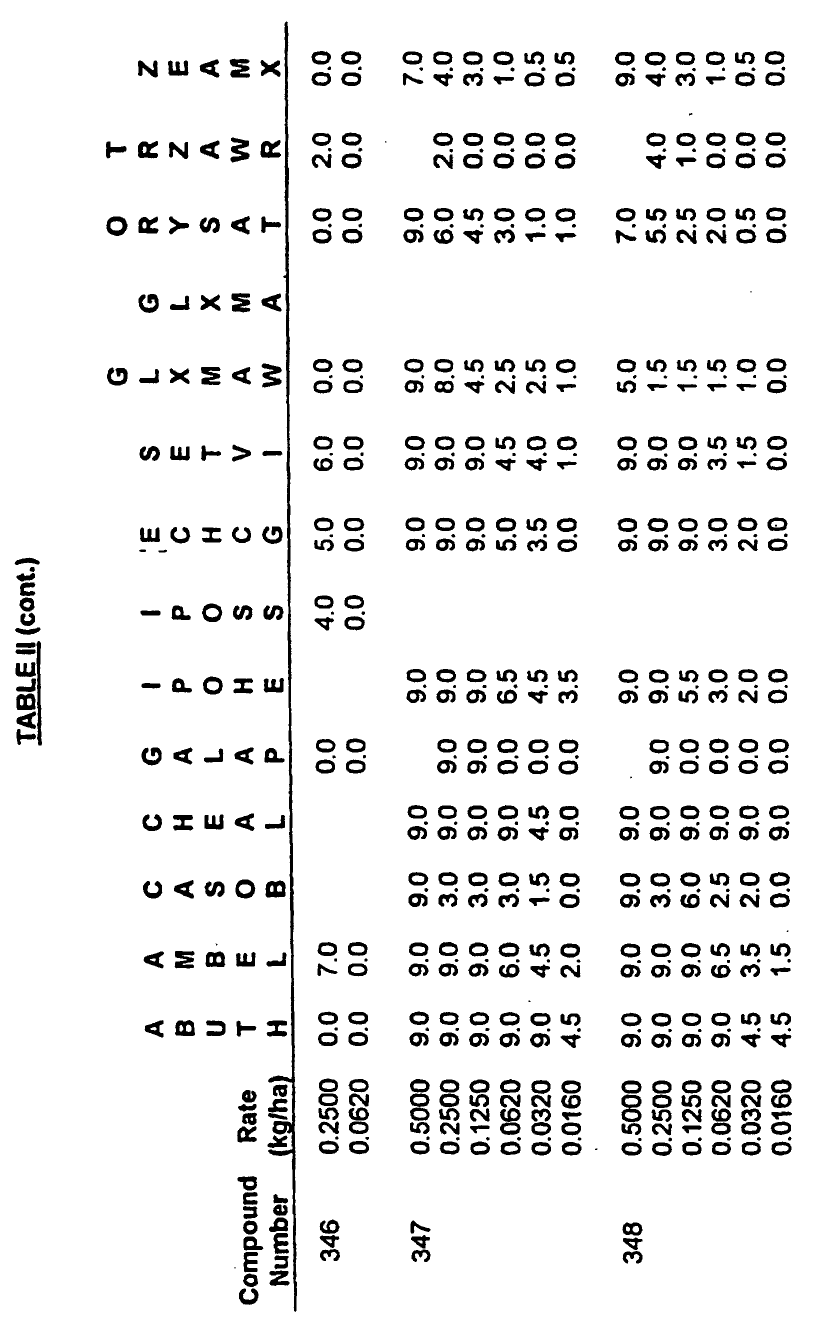

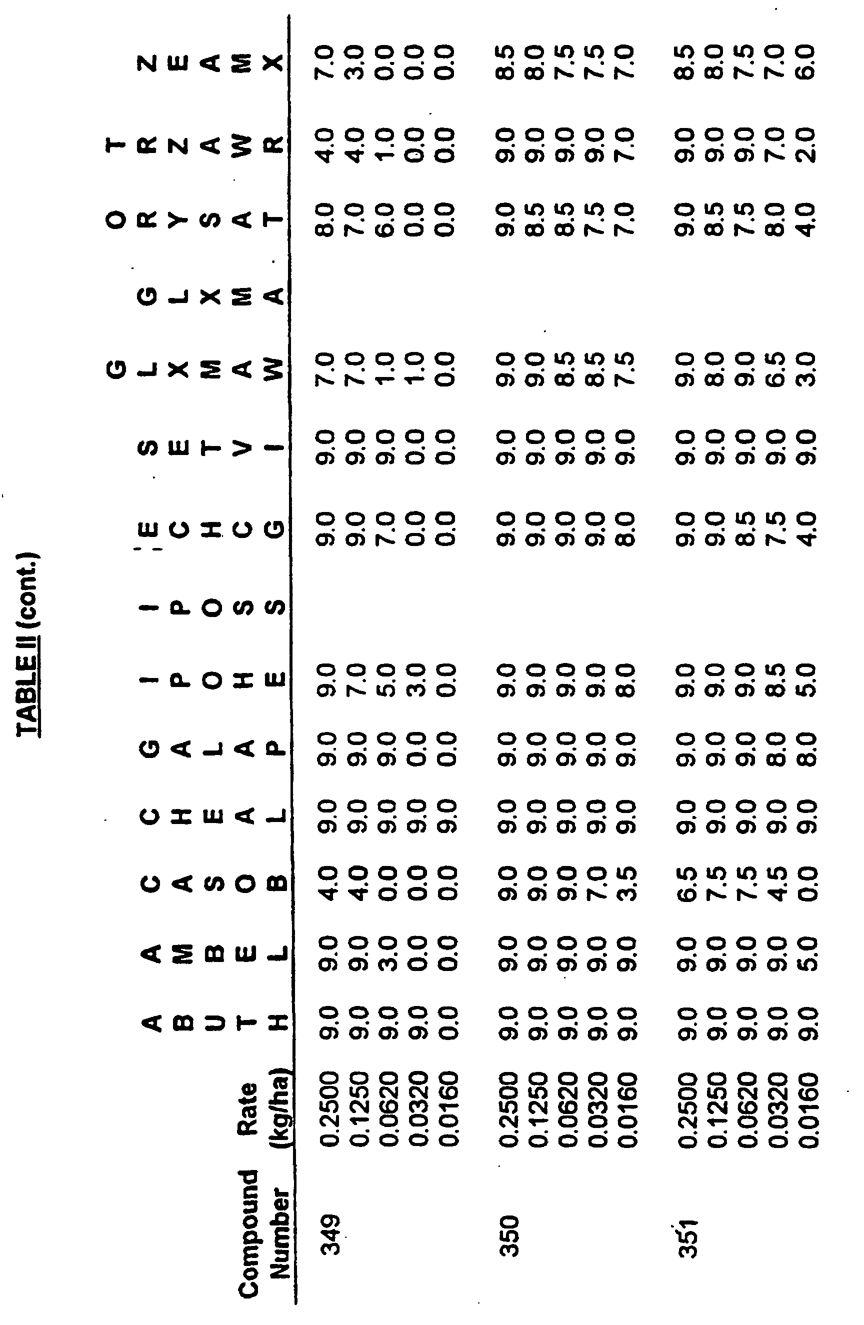

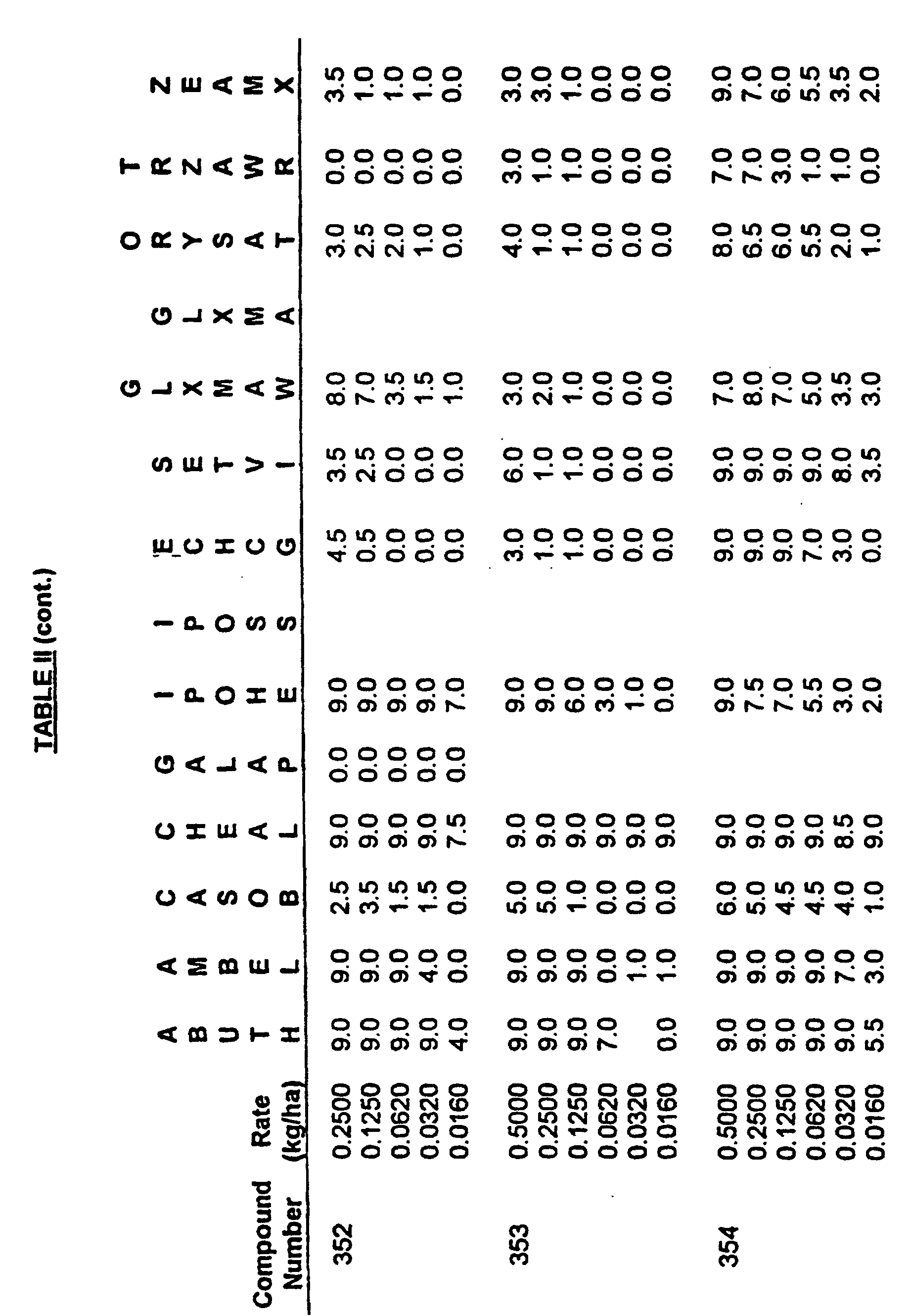

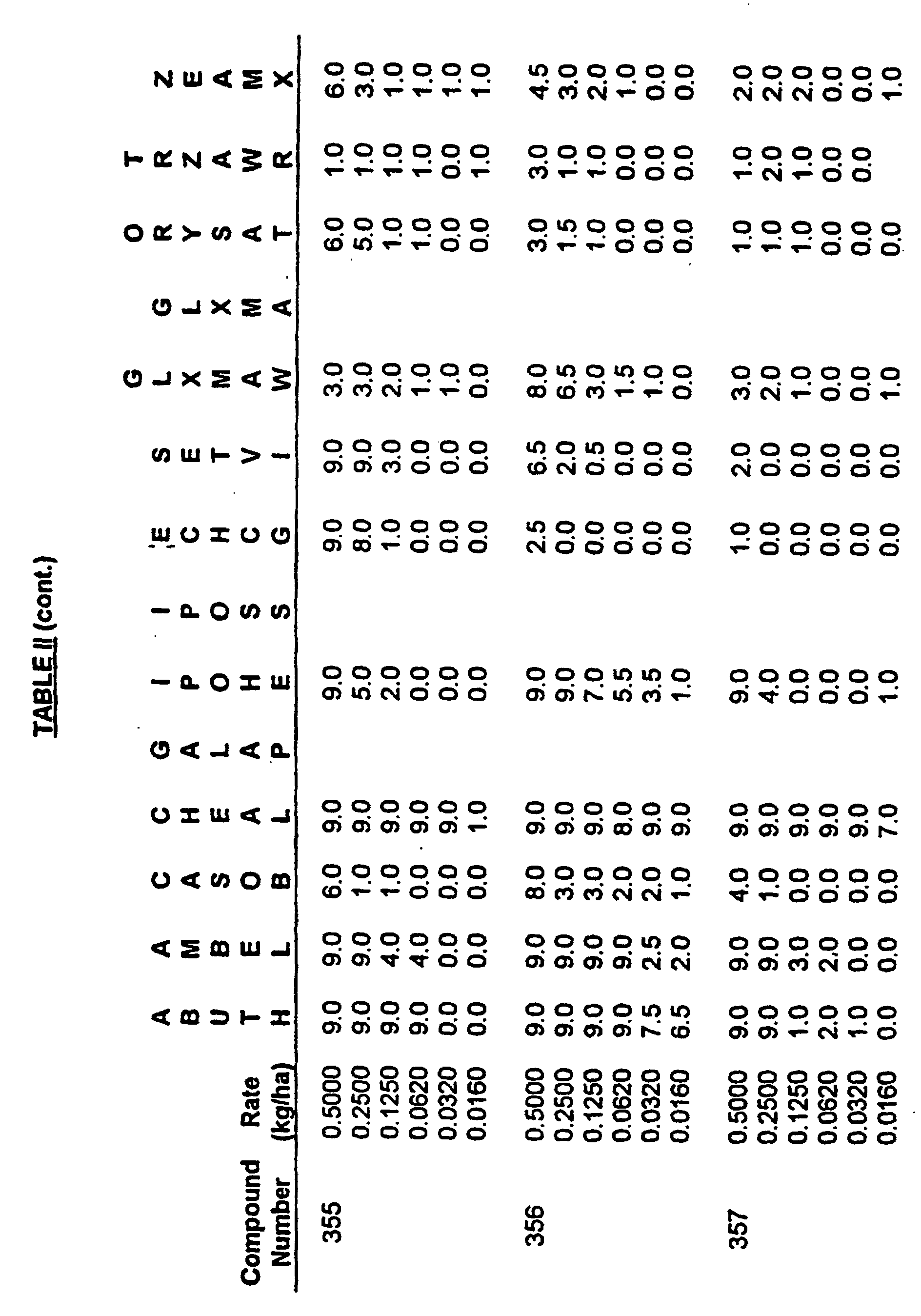

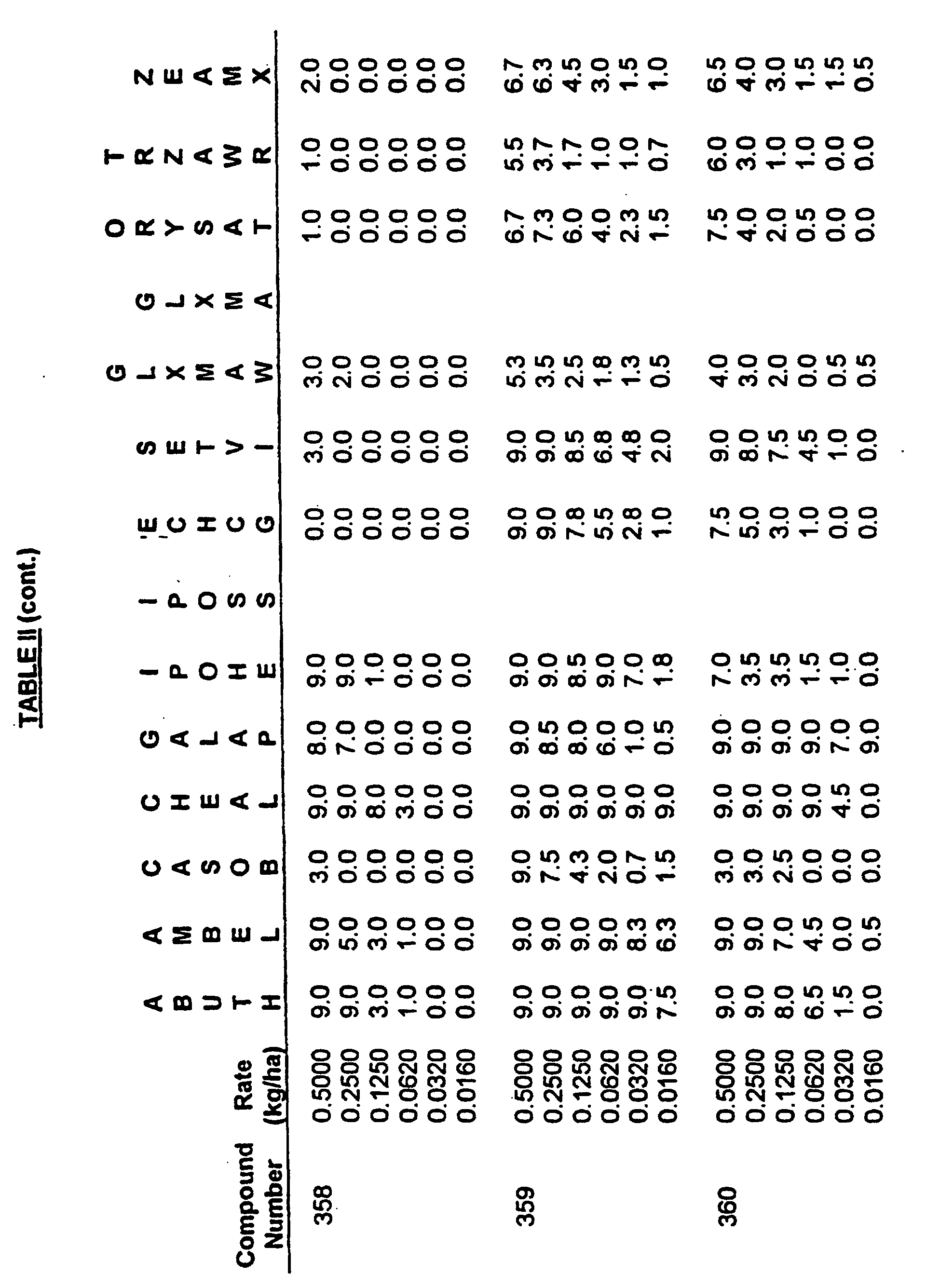

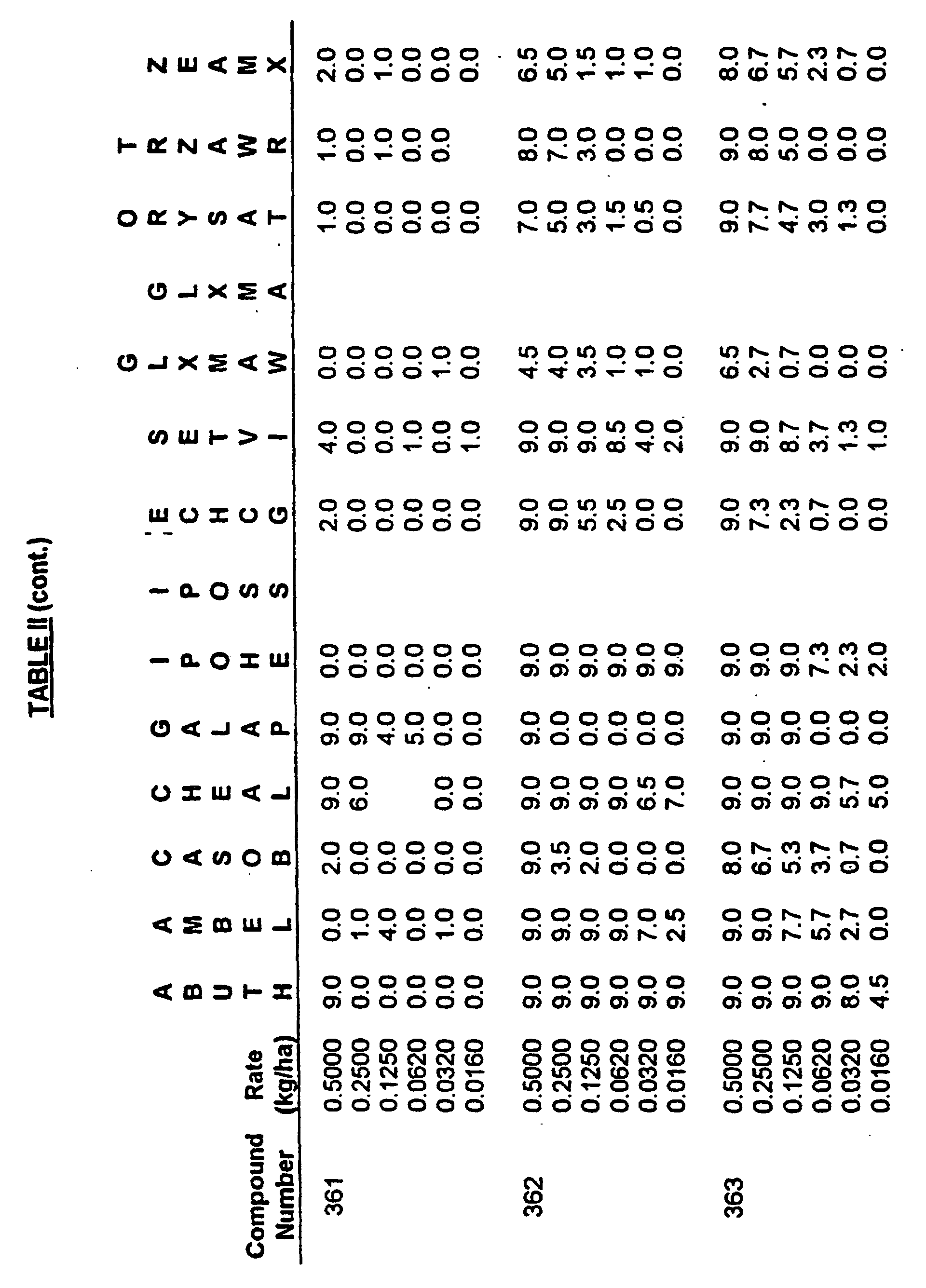

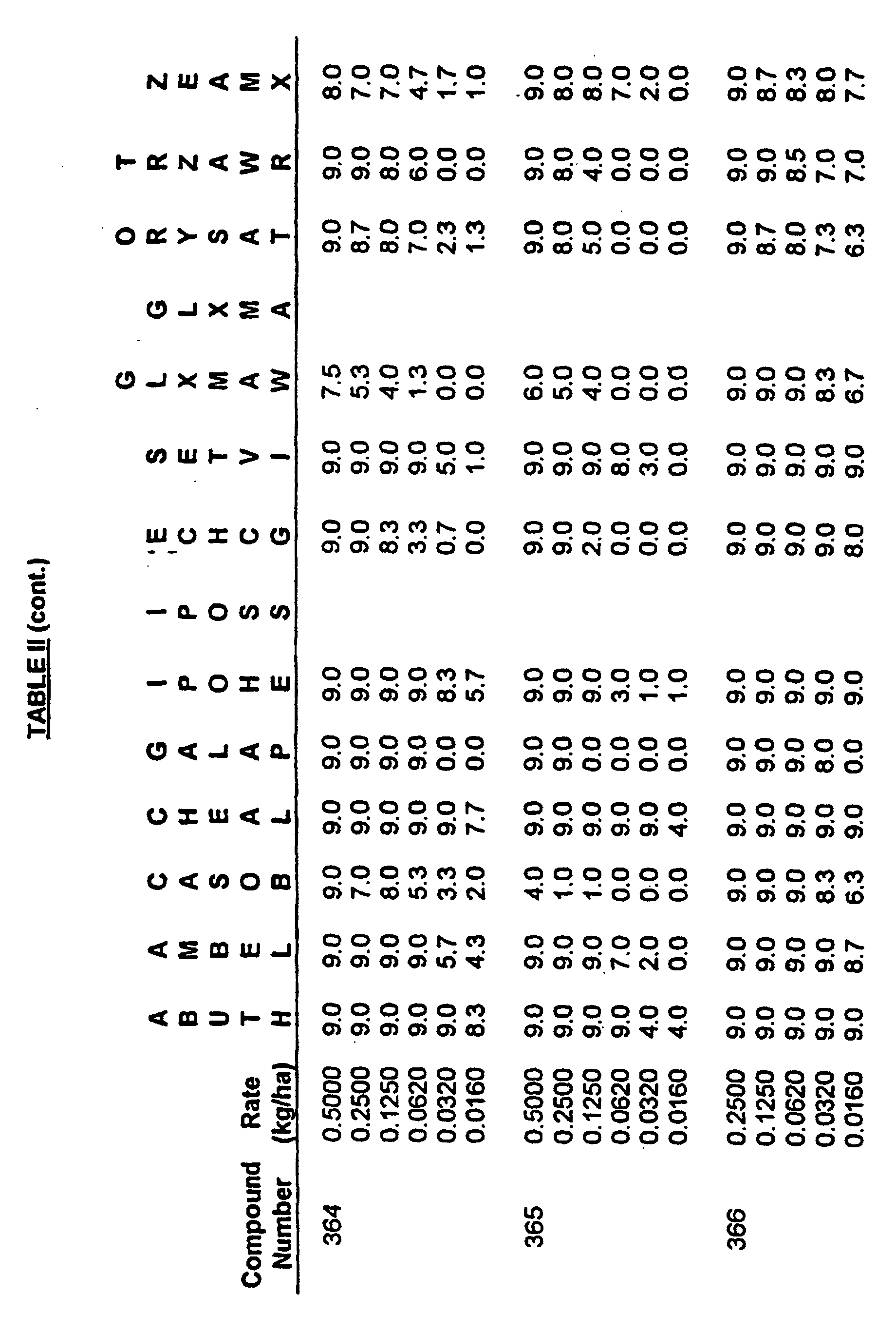

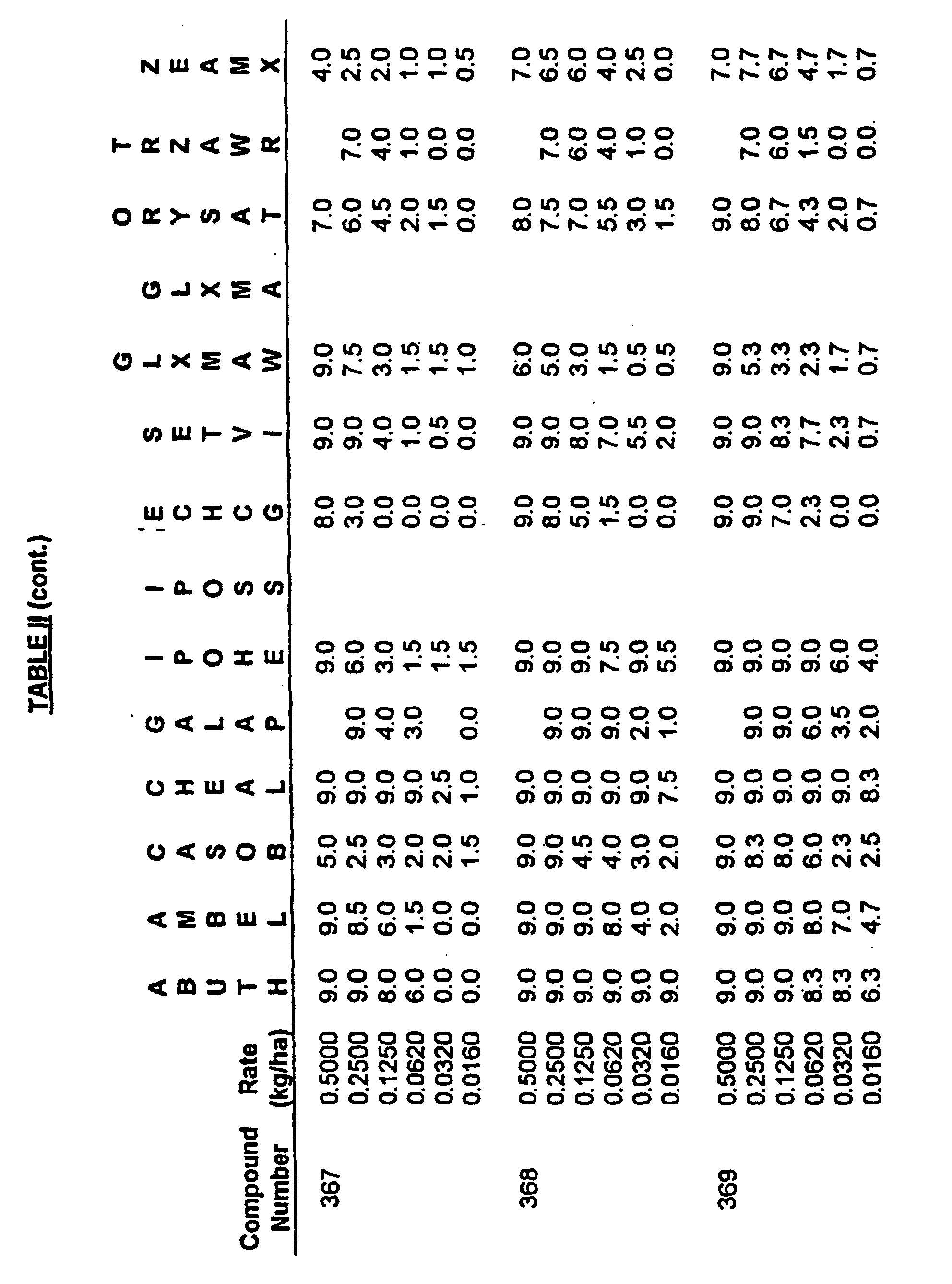

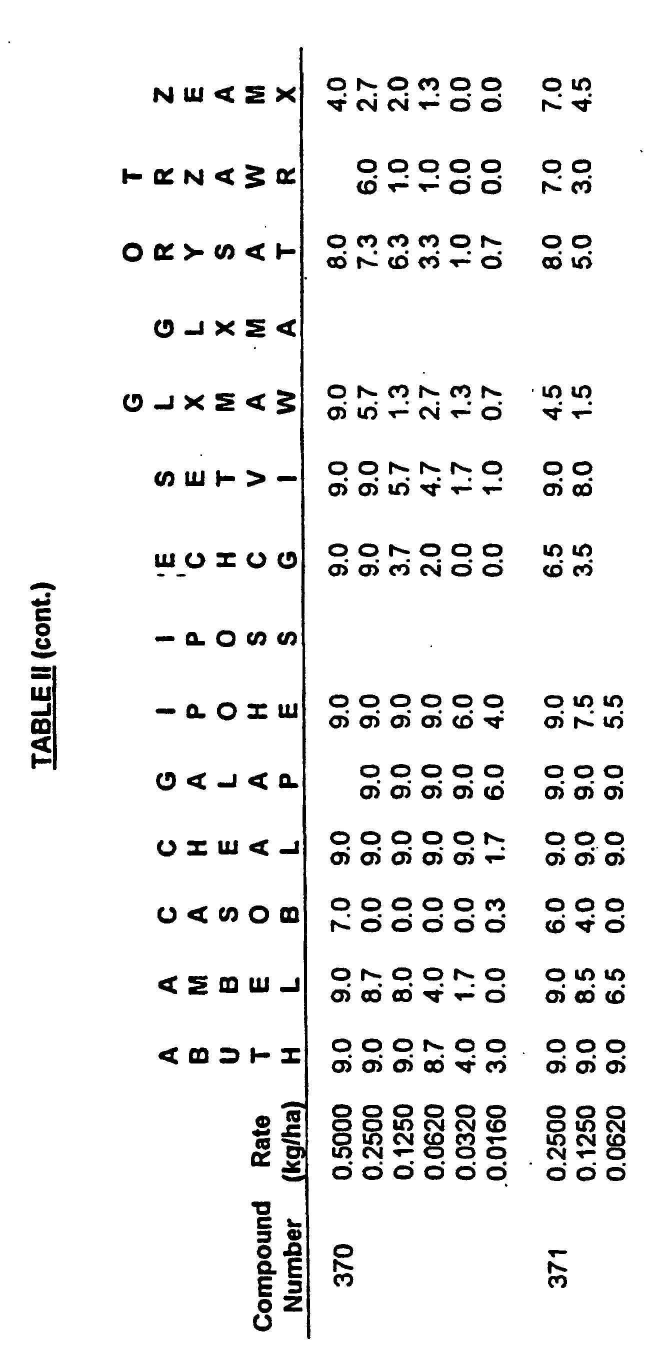

### Example 187

### Rice tolerance to post-transplant applications and preemergence weed control under flooded paddy conditions

The tolerance of transplanted rice to post-transplanted herbicide applications is determined as follows: two ten-day-old rice seedlings (cv. Tebonnet) are transplanted into silt loam soil in 0,95 l (32 oz.) plastic containers with a diameter of 10.5 cm and no drainage holes. After transplanting, the containers are flooded and the water level is maintained at 1.5 to 3 cm above the soil surface. Three days after transplanting, the flooded soil surface of the containers is treated with the selected aqueous/acetone 50/50 v/v mixture containing the test compounds to provide the equivalent of about 0.0313 to 0.500 kg/ha of active ingredient. The treated containers are placed on greenhouse benches, watered such that the water level is maintained as stated above, and cared for in accordance with conventional greenhouse procedures. Three to four weeks after treatment, the test is terminated and each container is examined and herbicidal effect rated according to the rating system provided in Example 185. The data obtained are reported in Table III. The compounds evaluated are reported by compound number given in Example 185.

Preemergence herbicidal activity under flooded paddy conditions is determined as follows: plant seeds or propagating organs are planted in the top 0.5 cm of silt loam soil in 0,95 l (32 oz.) plastic containers with a diameter of 10.5 cm and no drainage holes. Water is added to these containers and maintained at 1.5 to 3 cm above the soil surface for the duration of the experiment. The test compounds are applied as aqueous/acetone mixtures 50/50 v/v pipetted directly into the flood water to give the equivalent of about 0.0313 to 0.500 kg/ha of active ingredient. The treated containers are placed on greenhouse benches and cared for in accordance with conventional greenhouse procedures. Three to four weeks after treatment, the test is terminated and each container is examined and herbicidal effect rated according to the rating system provided in Example 185. The data obtained are reported in Table III. The compounds evaluated are reported by compound number given in Example 185.

Plant species employed in this example are reported by header abbreviation, common name and scientific name.

| **PLANT SPECIES EMPLOYED IN RICE TOLERANCE/PREEMERGENCE WEED CONTROL EVALUATIONS** | | |
|---|---|---|
| **Header Abbr.** | **Common Name** | **Scientific Name** |
| ECHORC | Watergrass (Calif.) | *Echinochloa oryzoides* (Ard.) Fritsch. |
| | | |
| CYPIR | Rice Flatsedge | *Cyperus iria* |
| | | |
| CYPSE | Flatsedge | *Cyperus serotinus,* Rottb. |
| | | |
| MOOVA | Monochoria | *Monochoria vaginalis,* Presl. |
| | | |
| SAGPY | Arrowhead (Pygmaea) | *Sagittaria pygmaea, L.* |
| | | |
| ORYSAT | Rice, Tebonnet | *Oryza sativa,* (L.) Tebonnet |

**TABLE III**

| **PADDY CONDITIONS - POST-TRANSPLANT RICE PREEMERGENCE WEEDS** | | | | | | | |
|---|---|---|---|---|---|---|---|
| **Compound** **Number** | **Rate** **(kg/ha)** | **E** **C** **H** **O** **R** **C** | **C** **Y** **P** **I** **R** | **C** **Y** **P** **S** **E** | **M** **O** **O** **V** **A** | **S** **A** **G** **P** **Y** | **O** **R** **Y** **S** **A** **T** |
| 1 | 0.5000 | 9.0 | 9.0 | 8.0 | 9.0 | 8.5 | 7.0 |
| | 0.2500 | 9.0 | 9.0 | 9.0 | 9.0 | 8.3 | 7.7 |
| | 0.1250 | 9.0 | 9.0 | 6.0 | 9.0 | 6.7 | 5.3 |
| | 0.0625 | 9.0 | 9.0 | 3.0 | 9.0 | 5.0 | 4.7 |
| | 0.0313 | 9.0 | 9.0 | 1.3 | 9.0 | 3.3 | 4.7 |
| | | | | | | | |
| 2 | 0.5000 | 9.0 | 9.0 | 0.0 | 9.0 | 9.0 | 9.0 |
| | 0.2500 | 9.0 | 9.0 | 0.0 | 9.0 | 4.0 | 5.0 |
| | 0.1250 | 9.0 | 9.0 | 0.0 | 9.0 | 2.0 | 3.0 |
| | 0.0625 | 9.0 | 9.0 | 0.0 | 9.0 | 0.0 | 3.0 |
| | 0.0313 | 9.0 | 8.0 | 0.0 | 7.0 | 0.0 | 2.0 |
| | | | | | | | |
| 3 | 0.5000 | 9.0 | 9.0 | 4.0 | 9.0 | 9.0 | 4.0 |
| | 0.2500 | 9.0 | 9.0 | 2.0 | 9.0 | 8.0 | 4.0 |
| | 0.1250 | 9.0 | 9.0 | 0.0 | 9.0 | 6.0 | 3.0 |
| | 0.0625 | 9.0 | 9.0 | 0.0 | 9.0 | 2.0 | 2.0 |
| | 0.0313 | 9.0 | 9.0 | 0.0 | 9.0 | 0.0 | 2.0 |
| | | | | | | | |
| 4 | 0.5000 | 9.0 | 9.0 | 9.0 | 9.0 | 9.0 | 8.0 |
| | 0.2500 | 9.0 | 9.0 | 9.0 | 9.0 | 9.0 | 8.0 |
| | 0.1250 | 9.0 | 9.0 | 9.0 | 9.0 | 6.0 | 7.0 |
| | 0.0625 | 9.0 | 9.0 | 2.0 | 9.0 | 2.0 | 7.0 |
| | 0.0313 | 9.0 | 9.0 | | 9.0 | 2.0 | 4.0 |
| | | | | | | | |
| 5 | 0.5000 | 9.0 | 9.0 | 7.0 | 9.0 | 7.0 | 7.0 |
| | 0.2500 | 9.0 | 9.0 | 8.0 | 9.0 | 7.0 | 5.0 |
| | 0.1250 | 9.0 | 9.0 | 6.0 | 9.0 | 2.0 | 4.0 |
| | 0.0625 | 9.0 | 9.0 | 2.0 | 9.0 | 0.0 | 3.0 |
| | 0.0313 | 9.0 | 9.0 | 0.0 | 9.0 | 0.0 | 2.0 |
| | | | | | | | |
| 6 | 0.5000 | 9.0 | 9.0 | 9.0 | 9.0 | 9.0 | 8.0 |
| | 0.2500 | 9.0 | 9.0 | 9.0 | 9.0 | 7.0 | 8.0 |
| | 0.1250 | 8.0 | 9.0 | 7.0 | 9.0 | 7.0 | 8.0 |
| | 0.0625 | 6.0 | 9.0 | 4.0 | 9.0 | 4.0 | 8.0 |
| | | | | | | | |
| 7 | 0.5000 | 9.0 | 9.0 | 9.0 | 9.0 | 9.0 | 9.0 |
| | 0.2500 | 9.0 | 9.0 | 7.0 | 9.0 | 9.0 | 7.0 |
| | 0.1250 | 9.0 | 9.0 | 8.0 | 9.0 | 9.0 | 7.0 |
| | 0.0625 | 9.0 | 9.0 | 7.0 | 9.0 | 8.0 | 8.0 |
| | 0.0313 | 9.0 | 9.0 | 7.0 | 9.0 | 6.0 | 6.0 |
| | | | | | | | |
| 8 | 0.5000 | 9.0 | 9.0 | 9.0 | 9.0 | 9.0 | 9.0 |
| | 0.2500 | 9.0 | 9.0 | 9.0 | 9.0 | 9.0 | 9.0 |
| | 0.1250 | 9.0 | 9.0 | 9.0 | 9.0 | 9.0 | 9.0 |
| | 0.0625 | 9.0 | 9.0 | 9.0 | 9.0 | 9.0 | 7.0 |
| | 0.0313 | 9.0 | 9.0 | 4.0 | 9.0 | 6.0 | 7.0 |
| | | | | | | | |
| 9 | 0.5000 | 9.0 | 9.0 | 9.0 | 9.0 | 9.0 | 9.0 |
| | 0.2500 | 9.0 | 9.0 | 9.0 | 9.0 | 8.0 | 8.0 |
| | 0.1250 | 9.0 | 9.0 | 9.0 | 9.0 | 8.0 | 7.0 |
| | 0.0625 | 9.0 | 9.0 | 4.0 | 9.0 | 2.0 | 7.0 |
| | 0.0313 | 9.0 | 9.0 | 2.0 | 9.0 | 0.0 | 6.0 |
| | | | | | | | |
| 10 | 0.5000 | 9.0 | 9.0 | 7.0 | 9.0 | 9.0 | 7.0 |
| | 0.2500 | 9.0 | 9.0 | 7.0 | 9.0 | 9.0 | 7.0 |
| | 0.1250 | 9.0 | 9.0 | 7.0 | 9.0 | 9.0 | 6.0 |
| | 0.0625 | 9.0 | 9.0 | 7.0 | 9.0 | 8.0 | 6.0 |
| | 0.0313 | 9.0 | 9.0 | 4.0 | 9.0 | 2.0 | 4.0 |
| | | | | | | | |
| 11 | 0.5000 | 9.0 | 9.0 | 7.0 | 9.0 | 9.0 | 3.0 |
| | 0.2500 | 8.0 | 9.0 | 2.0 | 9.0 | 2.0 | 3.0 |
| | 0.1250 | 8.0 | 9.0 | 2.0 | 9.0 | 0.0 | 2.0 |
| | 0.0625 | 7.0 | 9.0 | 2.0 | 9.0 | 0.0 | 2.0 |
| | | | | | | | |
| 12 | 0.5000 | 9.0 | 9.0 | 7.0 | 9.0 | 9.0 | 9.0 |
| | 0.2500 | 9.0 | 9.0 | 7.0 | 9.0 | 9.0 | 8.0 |
| | 0.1250 | 9.0 | 9.0 | 8.0 | 9.0 | 4.0 | 7.0 |
| | 0.0625 | 9.0 | 9.0 | 7.0 | 9.0 | 2.0 | 7.0 |
| | 0.0313 | 9.0 | 9.0 | 6.0 | 9.0 | 0.0 | 4.0 |
| | | | | | | | |
| 13 | 0.5000 | 9.0 | 9.0 | 7.0 | 9.0 | 9.0 | 9.0 |
| | 0.2500 | 9.0 | 9.0 | 7.0 | 9.0 | 8.0 | 7.0 |
| | 0.1250 | 9.0 | 9.0 | 7.0 | 9.0 | 6.0 | 7.0 |
| | 0.0625 | 9.0 | 9.0 | 2.0 | 9.0 | 2.0 | 6.0 |
| | 0.0313 | 9.0 | 9.0 | 0.0 | 9.0 | 0.0 | 4.0 |
| | | | | | | | |
| 14 | 0.2500 | 7.5 | 8.0 | 2.0 | 9.0 | 0.0 | 3.5 |
| | 0.1250 | 6.5 | 6.0 | 1.0 | 9.0 | 0.0 | 2.5 |
| | 0.0625 | 5.0 | 4.0 | 0.0 | 9.0 | 0.0 | 2.0 |
| | 0.0313 | 4.0 | 2.0 | 0.0 | 9.0 | 0.0 | 2.0 |
| | | | | | | | |
| 16 | 0.2500 | 5.0 | 6.0 | 0.0 | 9.0 | 0.0 | 4.0 |
| | 0.1250 | 3.5 | 4.0 | 0.0 | 9.0 | 0.0 | 2.5 |
| | 0.0625 | 1.0 | 2.0 | 0.0 | 4.0 | 0.0 | 1.5 |
| | 0.0313 | 1.0 | 0.0 | 0.0 | 2.0 | 0.0 | 1.0 |
| | | | | | | | |
| 17 | 0.2500 | 8.0 | 9.0 | 8.0 | 9.0 | 8.0 | 4.0 |
| | 0.1250 | 7.5 | 8.0 | 2.0 | 9.0 | 2.0 | 2.5 |
| | 0.0625 | 5.5 | 8.0 | 0.0 | 9.0 | 0.0 | 3.0 |
| | 0.0313 | 4.0 | 4.0 | 0.0 | 8.0 | 0.0 | 2.0 |
| | | | | | | | |
| 18 | 0.2500 | 9.0 | 9.0 | 8.0 | 9.0 | 0.0 | 9.0 |
| | 0.1250 | 9.0 | 9.0 | 9.0 | 9.0 | 0.0 | 4.0 |
| | 0.0625 | 9.0 | 9.0 | 9.0 | 9.0 | 0.0 | 3.0 |
| | 0.0313 | 9.0 | 9.0 | 6.0 | 9.0 | 0.0 | 3.0 |
| | | | | | | | |
| 19 | 0.2500 | 6.5 | 8.0 | 2.0 | 9.0 | 0.0 | 2.5 |
| | 0.1250 | 5.0 | 8.0 | 0.0 | 9.0 | 0.0 | 2.0 |
| | 0.0625 | 3.5 | 4.0 | 0.0 | 9.0 | 0.0 | 1.5 |
| | 0.0313 | 2.0 | 2.0 | 0.0 | 8.0 | 0.0 | 1.0 |
| | | | | | | | |
| 20 | 0.2500 | 0.0 | 2.0 | 0.0 | 4.0 | 0.0 | 1.0 |
| | 0.1250 | 0.0 | 1.0 | 0.0 | 2.0 | 0.0 | 0.5 |
| | 0.0625 | 0.0 | 0.0 | 0.0 | 0.0 | 0.0 | 0.5 |
| | 0.0313 | 0.0 | 0.0 | 0.0 | 0.0 | 0.0 | 0.5 |
| | | | | | | | |
| 21 | 0.2500 | 4.7 | 1.0 | 0.0 | 9.0 | 0.0 | 4.3 |
| | 0.1250 | 2.7 | 0.5 | 0.0 | 7.5 | 0.0 | 2.3 |
| | 0.0625 | 0.7 | 0.0 | 0.0 | 6.0 | 0.0 | 2.0 |
| | 0.0313 | 0.0 | 0.0 | 0.0 | 4.0 | 0.0 | 1.7 |
| | | | | | | | |
| 22 | 0.2500 | 9.0 | 9.0 | 9.0 | 9.0 | 9.0 | 2.5 |
| | 0.1250 | 9.0 | 9.0 | 7.0 | 9.0 | 4.0 | 2.5 |
| | 0.0625 | 9.0 | 9.0 | 7.0 | 9.0 | 2.0 | 2.0 |
| | 0.0313 | 9.0 | 9.0 | 2.0 | 9.0 | 0.0 | 2.0 |
| | | | | | | | |
| 23 | 0.2500 | 9.0 | 9.0 | 9.0 | 9.0 | 9.0 | 8.5 |
| | 0.1250 | 8.5 | 9.0 | 8.0 | 9.0 | 9.0 | 8.0 |
| | 0.0625 | 7.0 | 9.0 | 7.0 | 9.0 | 6.0 | 6.5 |
| | 0.0313 | 4.5 | 7.0 | 4.0 | 9.0 | 4.0 | 5.5 |
| | | | | | | | |
| 24 | 0.2500 | 7.0 | 8.0 | 4.0 | 9.0 | 0.0 | 4.0 |
| | 0.1250 | 2.0 | 6.0 | 1.0 | 9.0 | 0.0 | 3.0 |
| | 0.0625 | 0.0 | 2.0 | 0.0 | 8.0 | 0.0 | 2.0 |
| | 0.0313 | 0.0 | 0.0 | 0.0 | 4.0 | 0.0 | 2.0 |
| | | | | | | | |
| 25 | 0.2500 | 9.0 | 9.0 | 8.0 | 9.0 | 4.0 | 7.0 |
| | 0.1250 | 9.0 | 9.0 | 7.0 | 9.0 | 1.0 | 7.0 |
| | 0.0625 | 9.0 | 9.0 | 2.0 | 9.0 | 0.0 | 6.0 |
| | 0.0313 | 7.0 | 9.0 | 0.0 | 9.0 | 0.0 | 4.0 |
| | | | | | | | |
| 26 | 0.2500 | 9.0 | 9.0 | 9.0 | 9.0 | 9.0 | 9.0 |
| | 0.1250 | 9.0 | 9.0 | 2.0 | 9.0 | 2.0 | 6.0 |
| | 0.0625 | 9.0 | 8.0 | 4.0 | 9.0 | 0.0 | 4.0 |
| | 0.0313 | 7.0 | 6.0 | 0.0 | 9.0 | 0.0 | 3.0 |
| | | | | | | | |
| 27 | 0.2500 | 9.0 | 9.0 | 2.0 | 9.0 | 4.0 | 6.0 |
| | 0.1250 | 9.0 | 6.0 | 0.0 | 9.0 | 2.0 | 5.0 |
| | 0.0625 | 7.0 | 4.0 | 0.0 | 9.0 | 0.0 | 3.0 |
| | 0.0313 | 6.0 | 2.0 | 0.0 | 8.0 | 0.0 | 2.0 |
| | | | | | | | |
| 28 | 0.2500 | 9.0 | 9.0 | 4.0 | 9.0 | 8.0 | 9.0 |
| | 0.1250 | 9.0 | 6.0 | 0.0 | 9.0 | 4.0 | 7.0 |
| | 0.0625 | 9.0 | 4.0 | 0.0 | 9.0 | 2.0 | 4.0 |
| | 0.0313 | 8.0 | 2.0 | 0.0 | 8.0 | 0.0 | 3.0 |
| | | | | | | | |
| 29 | 0.2500 | 9.0 | 9.0 | 4.0 | 9.0 | 0.0 | 4.0 |
| | 0.1250 | 9.0 | 9.0 | 4.0 | 9.0 | 0.0 | 4.0 |
| | 0.0625 | 9.0 | 7.0 | | 9.0 | 0.0 | 3.0 |
| | 0.0313 | 7.0 | 4.0 | 0.0 | 8.0 | 0.0 | 2.0 |
| | | | | | | | |
| 30 | 0.2500 | 9.0 | 8.0 | 2.0 | 9.0 | 7.0 | 3.5 |
| | 0.1250 | 9.0 | 8.0 | 4.0 | 9.0 | 2.0 | 3.0 |
| | 0.0625 | 8.5 | 6.0 | 0.0 | 9.0 | 0.0 | 3.0 |
| | 0.0313 | 6.5 | 2.0 | 0.0 | 9.0 | 0.0 | 2.0 |
| | | | | | | | |
| 31 | 0.2500 | 9.0 | 9.0 | 9.0 | 9.0 | 9.0 | 8.0 |
| | 0.1250 | 9.0 | 9.0 | 8.0 | 9.0 | 9.0 | 7.5 |
| | 0.0625 | 8.5 | 9.0 | 7.0 | 9.0 | 7.0 | 8.0 |
| | 0.0313 | 7.0 | 9.0 | 7.0 | 9.0 | 4.0 | 7.5 |
| | | | | | | | |
| 32 | 0.2500 | 7.5 | 9.0 | 7.0 | 9.0 | 9.0 | 4.5 |
| | 0.1250 | 4.5 | 8.0 | 2.0 | 9.0 | 2.0 | 4.0 |
| | 0.0625 | 3.5 | 6.0 | 0.0 | 9.0 | 0.0 | 2.5 |
| | 0.0313 | 2.0 | 4.0 | 0.0 | 8.0 | 0.0 | 1.5 |
| | | | | | | | |
| 33 | 0.2500 | 9.0 | 4.0 | 0.0 | 9.0 | 0.0 | 3.5 |
| | 0.1250 | 9.0 | 2.0 | 0.0 | 9.0 | 0.0 | 3.0 |
| | 0.0625 | 8.5 | 0.0 | 0.0 | 9.0 | 0.0 | 3.0 |
| | 0.0313 | 7.5 | 0.0 | 0.0 | 9.0 | 0.0 | 2.0 |
| | | | | | | | |
| 34 | 0.2500 | 4.5 | 0.0 | 0.0 | 0.0 | 0.0 | 0.0 |
| | 0.1250 | 3.5 | 0.0 | 0.0 | 0.0 | 0.0 | 0.0 |
| | 0.0625 | 2.5 | 0.0 | 0.0 | 0.0 | 0.0 | 0.0 |
| | 0.0313 | 2.0 | 0.0 | 0.0 | 0.0 | 0.0 | 0.0 |
| | | | | | | | |
| 35 | 0.2500 | 9.0 | 9.0 | 7.0 | 9.0 | 9.0 | 5.0 |
| | 0.1250 | 7.5 | 9.0 | 7.0 | 9.0 | 2.0 | 4.0 |
| | 0.0625 | 6.5 | 8.0 | 2.0 | 9.0 | 0.0 | 2.5 |
| | 0.0313 | 4.5 | 4.0 | 0.0 | 9.0 | 0.0 | 2.0 |
| | | | | | | | |
| 36 | 0.2500 | 0.0 | | | | | 1.0 |
| | 0.1250 | 0.0 | | | | | 1.0 |
| | 0.0625 | 0.0 | | | | | 1.0 |
| | 0.0313 | 0.0 | | | | | 1.0 |
| | | | | | | | |
| 37 | 0.2500 | 9.0 | | | | | 1.0 |
| | 0.1250 | 8.0 | | | | | 1.0 |
| | 0.0625 | 7.0 | | | | | 1.0 |
| | 0.0313 | 4.0 | | | | | 0.0 |
| | | | | | | | |
| 38 | 0.2500 | 2.0 | 0.0 | 0.0 | 0.0 | 0.0 | 0.0 |
| | 0.1250 | 1.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 39 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 40 | 0.2500 | 9.0 | 9.0 | 9.0 | 9.0 | 6.0 | 4.0 |
| | 0.1250 | 9.0 | 9.0 | 9.0 | 9.0 | 2.0 | 3.0 |
| | 0.0625 | 9.0 | 4.0 | 4.0 | 9.0 | 0.0 | 3.0 |
| | 0.0313 | 9.0 | 2.0 | 2.0 | 9.0 | 0.0 | 2.0 |
| | | | | | | | |
| 41 | 0.2500 | 6.0 | 2.0 | 0.0 | 9.0 | 0.0 | 0.0 |
| | 0.1250 | 2.0 | 1.0 | 0.0 | 4.0 | 0.0 | 0.0 |
| | 0.0625 | 0.0 | 0.0 | 0.0 | 2.0 | 0.0 | 0.0 |
| | 0.0313 | 0.0 | 0.0 | 0.0 | 0.0 | 0.0 | 0.0 |
| | | | | | | | |
| 42 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 43 | 0.2500 | 8.0 | 0.0 | 0.0 | 9.0 | 0.0 | 3.0 |
| | 0.1250 | 2.0 | 0.0 | 0.0 | 9.0 | 0.0 | 3.0 |
| | 0.0625 | 0.0 | 0.0 | 0.0 | 9.0 | 0.0 | 2.0 |
| | 0.0313 | 0.0 | 0.0 | 0.0 | 6.0 | 0.0 | 1.0 |
| | | | | | | | |
| 44 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 45 | 0.2500 | 9.0 | 9.0 | 9.0 | 9.0 | 6.0 | 3.0 |
| | 0.1250 | 9.0 | 9.0 | 9.0 | 9.0 | 4.0 | 3.0 |
| | 0.0625 | 9.0 | 9.0 | 2.0 | 9.0 | 0.0 | 2.0 |
| | 0.0313 | 9.0 | 6.0 | | 9.0 | 0.0 | 2.0 |
| | | | | | | | |
| 46 | 0.2500 | 9.0 | 9.0 | 2.0 | 9.0 | 7.0 | 3.0 |
| | 0.1250 | 9.0 | 9.0 | | 9.0 | 2.0 | 2.0 |
| | 0.0625 | 9.0 | 6.0 | 0.0 | 9.0 | 0.0 | 1.0 |
| | 0.0313 | 9.0 | 2.0 | 0.0 | 9.0 | 0.0 | 1.0 |
| | | | | | | | |
| 47 | 0.2500 | 5.0 | 1.0 | 2.0 | 9.0 | 0.0 | 6.0 |
| | 0.1250 | 3.5 | 0.0 | 1.0 | 5.5 | 0.0 | 4.0 |
| | 0.0625 | 1.5 | 0.0 | 0.0 | 4.5 | 0.0 | 2.5 |
| | 0.0313 | 0.0 | 0.0 | 0.0 | 4.5 | 0.0 | 1.5 |
| | | | | | | | |
| 48 | 0.2500 | 6.0 | 0.0 | | 9.0 | 0.0 | 6.0 |
| | 0.1250 | 2.0 | 0.0 | 0.0 | 9.0 | 0.0 | 4.0 |
| | 0.0625 | 0.0 | 0.0 | 0.0 | 9.0 | 0.0 | 2.0 |
| | 0.0313 | 0.0 | 0.0 | 0.0 | 6.0 | 0.0 | 2.0 |
| | | | | | | | |
| 49 | 0.2500 | 8.0 | 8.0 | 0.0 | 9.0 | 0.0 | 2.0 |
| | 0.1250 | 6.0 | 2.0 | 0.0 | 9.0 | 0.0 | 1.0 |
| | 0.0625 | 4.0 | 0.0 | 0.0 | 9.0 | 0.0 | 1.0 |
| | 0.0313 | 0.0 | 0.0 | 0.0 | 4.0 | 0.0 | 1.0 |
| | | | | | | | |
| 50 | 0.2500 | 9.0 | 6.0 | 0.0 | 9.0 | 0.0 | 3.0 |
| | 0.1250 | 9.0 | 2.0 | 0.0 | 9.0 | 0.0 | 2.0 |
| | 0.0625 | 9.0 | 2.0 | 0.0 | 9.0 | 0.0 | 2.0 |
| | 0.0313 | 9.0 | 0.0 | 0.0 | 9.0 | 0.0 | 2.0 |
| | | | | | | | |
| 51 | 0.2500 | 9.0 | 9.0 | 9.0 | 9.0 | 9.0 | 9.0 |
| | 0.1250 | 9.0 | 9.0 | 7.0 | 9.0 | 4.0 | 9.0 |
| | 0.0625 | 7.0 | 8.0 | 2.0 | 9.0 | 2.0 | 9.0 |
| | 0.0313 | 4.0 | 7.0 | 0.0 | 9.0 | 2.0 | 7.0 |
| | | | | | | | |
| 52 | 0.2500 | 7.0 | 0.0 | | 9.0 | 0.0 | 0.0 |
| | 0.1250 | 4.0 | | | 9.0 | 0.0 | 0.0 |
| | 0.0625 | 2.0 | 0.0 | 0.0 | 9.0 | 0.0 | 0.0 |
| | 0.0313 | 0.0 | 0.0 | 0.0 | 8.0 | 0.0 | 0.0 |
| | | | | | | | |
| 53 | 0.2500 | 9.0 | 9.0 | 0.0 | 9.0 | 0.0 | 7.0 |
| | 0.1250 | 6.0 | 9.0 | 0.0 | 9.0 | 0.0 | 5.0 |
| | 0.0625 | 2.0 | 6.0 | 0.0 | 9.0 | 0.0 | 4.0 |
| | 0.0313 | 0.0 | 2.0 | 0.0 | 8.0 | 0.0 | 2.0 |
| | | | | | | | |
| 54 | 0.2500 | 9.0 | 9.0 | 2.0 | 9.0 | 0.0 | 6.0 |
| | 0.1250 | 7.0 | 7.0 | 1.0 | 9.0 | 0.0 | 3.0 |
| | 0.0625 | 6.0 | 4.0 | 0.0 | 9.0 | 0.0 | 2.0 |
| | 0.0313 | 2.0 | 2.0 | 0.0 | 8.0 | 0.0 | 2.0 |
| | | | | | | | |
| 55 | 0.2500 | 9.0 | 9.0 | 0.0 | 9.0 | 0.0 | 6.0 |
| | 0.1250 | 9.0 | 6.0 | 0.0 | 9.0 | 0.0 | 3.0 |
| | 0.0625 | 7.0 | 2.0 | 0.0 | 9.0 | 0.0 | 1.0 |
| | 0.0313 | 5.0 | 0.0 | 0.0 | 7.0 | 0.0 | 2.0 |
| | | | | | | | |
| 56 | 0.2500 | 9.0 | 9.0 | 2.0 | 9.0 | 0.0 | 6.0 |
| | 0.1250 | 9.0 | 9.0 | 2.0 | 9.0 | 0.0 | 3.0 |
| | 0.0625 | 9.0 | 9.0 | 0.0 | 9.0 | 0.0 | 2.0 |
| | 0.0313 | 9.0 | 6.0 | 0.0 | 9.0 | 0.0 | 1.0 |
| | | | | | | | |
| 57 | 0.2500 | 9.0 | 9.0 | 0.0 | 9.0 | 0.0 | 6.0 |
| | 0.1250 | 9.0 | 9.0 | 0.0 | 9.0 | 0.0 | 5.0 |
| | 0.0625 | 9.0 | 9.0 | 0.0 | 9.0 | 0.0 | 4.0 |
| | 0.0313 | 9.0 | 9.0 | 0.0 | 9.0 | 0.0 | 3.0 |
| | | | | | | | |
| 58 | 0.2500 | 9.0 | 9.0 | 0.0 | 9.0 | 0.0 | 5.0 |
| | 0.1250 | 9.0 | 9.0 | 0.0 | 9.0 | 0.0 | 3.0 |
| | 0.0625 | 9.0 | 9.0 | 0.0 | 9.0 | 0.0 | 2.0 |
| | 0.0313 | 9.0 | 9.0 | 0.0 | 9.0 | 0.0 | 2.0 |
| | | | | | | | |
| 59 | 0.2500 | 9.0 | 9.0 | 0.0 | 9.0 | 0.0 | 4.0 |
| | 0.1250 | 9.0 | 8.0 | 0.0 | 9.0 | 0.0 | 4.0 |
| | 0.0625 | 9.0 | 7.0 | 0.0 | 9.0 | 0.0 | 3.0 |
| | 0.0313 | 9.0 | 7.0 | 0.0 | 9.0 | 0.0 | 2.0 |
| | | | | | | | |
| 60 | 0.2500 | 9.0 | 0.0 | 0.0 | 9.0 | 0.0 | 0.0 |
| | 0.1250 | 8.0 | 0.0 | 0.0 | 9.0 | 0.0 | 0.0 |
| | 0.0625 | 4.0 | 0.0 | 0.0 | 9.0 | 0.0 | 0.0 |
| | 0.0313 | 2.0 | 0.0 | 0.0 | 9.0 | 0.0 | 0.0 |
| | | | | | | | |
| 61 | 0.2500 | 8.0 | 2.0 | 0.0 | 9.0 | 0.0 | 0.0 |
| | 0.1250 | 8.0 | 2.0 | 0.0 | 9.0 | 0.0 | 0.0 |
| | 0.0625 | 7.0 | 0.0 | 0.0 | 8.0 | 0.0 | 0.0 |
| | 0.0313 | 4.0 | 0.0 | 0.0 | 8.0 | 0.0 | 0.0 |
| | | | | | | | |
| 62 | 0.2500 | 9.0 | 9.0 | 9.0 | 9.0 | 7.0 | 9.0 |
| | 0.1250 | 9.0 | 9.0 | 2.0 | 9.0 | 4.0 | 9.0 |
| | 0.0625 | 9.0 | 6.0 | 1.0 | 9.0 | 1.0 | 9.0 |
| | 0.0313 | 6.0 | 4.0 | 0.0 | 9.0 | 0.0 | 4.0 |
| | | | | | | | |
| 63 | 0.2500 | 9.0 | 9.0 | 0.0 | 9.0 | 0.0 | 3.0 |
| | 0.1250 | 9.0 | 9.0 | 0.0 | 9.0 | 0.0 | 2.0 |
| | 0.0625 | 9.0 | 8.0 | 0.0 | 9.0 | 0.0 | 2.0 |
| | 0.0313 | 9.0 | 7.0 | 0.0 | 9.0 | 0.0 | 1.0 |
| | | | | | | | |
| 64 | 0.2500 | 9.0 | 7.0 | 0.0 | 9.0 | 0.0 | 7.0 |
| | 0.1250 | 8.0 | 7.0 | 0.0 | 9.0 | 0.0 | 4.0 |
| | 0.0625 | 8.0 | 4.0 | 0.0 | 9.0 | 0.0 | 3.0 |
| | 0.0313 | 6.0 | 2.0 | 0.0 | 9.0 | 0.0 | 3.0 |
| | | | | | | | |
| 65 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 66 | 0.2500 | 9.0 | 9.0 | 9.0 | 9.0 | 2.0 | 2.0 |
| | 0.1250 | 9.0 | 9.0 | 9.0 | 9.0 | 0.0 | 2.0 |
| | 0.0625 | 9.0 | 9.0 | 2.0 | 9.0 | 0.0 | 1.0 |
| | 0.0313 | 9.0 | 8.0 | 0.0 | 9.0 | 0.0 | 1.0 |
| | | | | | | | |
| 67 | 0.2500 | 9.0 | 0.0 | 6.0 | 9.0 | 0.0 | 1.0 |
| | 0.1250 | 9.0 | 0.0 | 1.0 | 9.0 | 0.0 | 1.0 |
| | 0.0625 | 8.0 | 0.0 | 0.0 | 9.0 | 0.0 | 0.5 |
| | 0.0313 | 5.5 | 0.0 | 0.0 | 8.0 | 0.0 | 0.5 |
| | | | | | | | |
| 68 | 0.2500 | 9.0 | 9.0 | 7.0 | 9.0 | 2.0 | 1.0 |
| | 0.1250 | 9.0 | 9.0 | | 9.0 | 2.0 | 1.5 |
| | 0.0625 | 9.0 | 9.0 | 7.0 | 9.0 | 0.0 | 1.0 |
| | 0.0313 | 7.5 | 7.0 | 6.0 | 9.0 | 0.0 | 0.5 |
| | | | | | | | |
| 69 | 0.2500 | 9.0 | 7.0 | 9.0 | 9.0 | 9.0 | 8.0 |
| | 0.1250 | 9.0 | 4.0 | 2.0 | 9.0 | 2.0 | 6.5 |
| | 0.0625 | 9.0 | 2.0 | 0.0 | 9.0 | 0.0 | 5.0 |
| | 0.0313 | 9.0 | 0.0 | 0.0 | 9.0 | 0.0 | 3.5 |
| | | | | | | | |
| 70 | 0.2500 | 9.0 | 9.0 | 0.0 | 9.0 | 2.0 | 6.5 |
| | 0.1250 | 9.0 | 9.0 | 0.0 | 9.0 | 1.0 | 6.0 |
| | 0.0625 | 8.0 | 7.0 | 0.0 | 9.0 | 0.0 | 4.5 |
| | 0.0313 | 4.0 | 2.0 | 0.0 | 9.0 | 0.0 | 3.0 |
| | | | | | | | |
| 71 | 0.2500 | 9.0 | 2.0 | 9.0 | 9.0 | 4.0 | 7.0 |
| | 0.1250 | 8.5 | 0.0 | 2.0 | 9.0 | 0.0 | 5.5 |
| | 0.0625 | 7.5 | 0.0 | 0.0 | 9.0 | 0.0 | 3.5 |
| | 0.0313 | 5.5 | 0.0 | 0.0 | 8.0 | 0.0 | 2.0 |
| | | | | | | | |
| 72 | 0.2500 | 9.0 | 7.0 | 9.0 | 9.0 | 4.0 | 7.0 |
| | 0.1250 | 8.5 | 4.0 | 2.0 | 9.0 | 0.0 | 6.0 |
| | 0.0625 | 8.0 | 0.0 | 2.0 | 9.0 | 0.0 | 5.0 |
| | 0.0313 | 7.5 | 0.0 | 0.0 | 9.0 | 0.0 | 4.0 |
| | | | | | | | |
| 73 | 0.2500 | 9.0 | 8.0 | 9.0 | 9.0 | 2.0 | 6.0 |
| | 0.1250 | 9.0 | 8.0 | 2.0 | 9.0 | 0.0 | 4.0 |
| | 0.0625 | 6.5 | 6.0 | 0.0 | 9.0 | 0.0 | 3.0 |
| | 0.0313 | 8.0 | 2.0 | 0.0 | 9.0 | 0.0 | 2.5 |
| | | | | | | | |
| 74 | 0.2500 | 9.0 | 8.0 | 6.0 | 9.0 | 0.0 | 7.0 |
| | 0.1250 | 9.0 | 7.0 | 2.0 | 9.0 | 0.0 | 5.0 |
| | 0.0625 | 7.5 | 2.0 | 0.0 | 9.0 | 0.0 | 4.0 |
| | 0.0313 | 6.5 | 0.0 | 0.0 | 8.0 | 0.0 | 2.5 |
| | | | | | | | |
| 75 | 0.2500 | 9.0 | 7.0 | 0.0 | 9.0 | 4.0 | 8.0 |
| | 0.1250 | 8.5 | 4.0 | 0.0 | 9.0 | 0.0 | 5.0 |
| | 0.0625 | 9.0 | 2.0 | 0.0 | 9.0 | 0.0 | 3.0 |
| | 0.0313 | 6.5 | 0.0 | 0.0 | 9.0 | 0.0 | 2.0 |
| | | | | | | | |
| 76 | 0.2500 | 9.0 | 9.0 | 2.0 | 9.0 | 9.0 | 5.5 |
| | 0.1250 | 9.0 | 9.0 | 1.0 | 9.0 | 2.0 | 3.5 |
| | 0.0625 | 8.5 | 6.0 | 0.0 | 9.0 | 0.0 | 2.5 |
| | 0.0313 | 8.0 | 4.0 | 0.0 | 8.0 | 0.0 | 2.5 |
| | | | | | | | |
| 77 | 0.2500 | 9.0 | | | | | 7.0 |
| | 0.1250 | 9.0 | | | | | 7.0 |
| | 0.0625 | 9.0 | | | | | 4.0 |
| | 0.0313 | 9.0 | | | | | 3.0 |
| | | | | | | | |
| 78 | 0.2500 | 9.0 | | | | | 4.0 |
| | 0.1250 | 9.0 | | | | | 4.0 |
| | 0.0625 | 9.0 | | | | | 4.0 |
| | 0.0313 | 9.0 | | | | | 3.0 |
| | | | | | | | |
| 79 | 0.2500 | 9.0 | | | | | 4.0 |
| | 0.1250 | 9.0 | | | | | 4.0 |
| | 0.0625 | 9.0 | | | | | 4.0 |
| | 0.0313 | 9.0 | | | | | 3.0 |
| | | | | | | | |
| 80 | 0.2500 | 9.0 | | | | | 4.0 |
| | 0.1250 | 9.0 | | | | | 3.0 |
| | 0.0625 | 9.0 | | | | | 3.0 |
| | 0.0313 | 9.0 | | | | | 4.0 |
| | | | | | | | |
| 81 | 0.2500 | 9.0 | | | | | 4.0 |
| | 0.1250 | 9.0 | | | | | 4.0 |
| | 0.0625 | 9.0 | | | | | 4.0 |
| | 0.0313 | 9.0 | | | | | 4.0 |
| | | | | | | | |
| 82 | 0.2500 | 9.0 | | | | | 3.0 |
| | 0.1250 | 9.0 | | | | | 3.0 |
| | 0.0625 | 9.0 | | | | | 2.0 |
| | 0.0313 | 9.0 | | | | | 1.0 |
| | | | | | | | |
| 83 | 0.2500 | 9.0 | | | | | 5.0 |
| | 0.1250 | 9.0 | | | | | 6.0 |
| | 0.0625 | 9.0 | | | | | 5.0 |
| | 0.0313 | 9.0 | | | | | 3.0 |
| | | | | | | | |
| 84 | 0.2500 | 9.0 | | | | | 7.0 |
| | 0.1250 | 9.0 | | | | | 4.0 |
| | 0.0625 | 9.0 | | | | | 3.0 |
| | 0.0313 | 9.0 | | | | | 3.0 |
| | | | | | | | |
| 85 | 0.2500 | 9.0 | | | | | 1.0 |
| | 0.1250 | 9.0 | | | | | 0.0 |
| | 0.0625 | 9.0 | | | | | 0.0 |
| | 0.0313 | 8.0 | | | | | 0.0 |
| | | | | | | | |
| 86 | 0.2500 | 9.0 | | | | | 3.0 |
| | 0.1250 | 9.0 | | | | | 2.0 |
| | 0.0625 | 9.0 | | | | | 3.0 |
| | 0.0313 | 9.0 | | | | | 2.0 |
| | | | | | | | |
| 87 | 0.2500 | 9.0 | | | | | 4.0 |
| | 0.1250 | 9.0 | | | | | 4.0 |
| | 0.0625 | 9.0 | | | | | 3.0 |
| | 0.0313 | 9.0 | | | | | 3.0 |
| | | | | | | | |
| 88 | 0.2500 | 9.0 | | | | | 7.0 |
| | 0.1250 | 9.0 | | | | | 6.0 |
| | 0.0625 | 9.0 | | | | | 4.0 |
| | 0.0313 | 9.0 | | | | | 3.0 |
| | | | | | | | |
| 89 | 0.2500 | 9.0 | | | | | 5.0 |
| | 0.1250 | 9.0 | | | | | 4.0 |
| | 0.0625 | 9.0 | | | | | 3.0 |
| | 0.0313 | 9.0 | | | | | 4.0 |
| | | | | | | | |
| 90 | 0.2500 | 9.0 | | | | | 7.0 |
| | 0.1250 | 9.0 | | | | | 6.0 |
| | 0.0625 | 9.0 | | | | | 6.0 |
| | 0.0313 | 9.0 | | | | | 4.0 |
| | | | | | | | |
| 91 | 0.2500 | 9.0 | | | | | 1.0 |
| | 0.1250 | 9.0 | | | | | 0.0 |
| | 0.0625 | 9.0 | | | | | 0.0 |
| | 0.0313 | 9.0 | | | | | 0.0 |
| | | | | | | | |
| 92 | 0.2500 | 9.0 | | | | | 6.0 |
| | 0.1250 | 9.0 | | | | | 6.0 |
| | 0.0625 | 6.0 | | | | | 5.0 |
| | 0.0313 | 4.0 | | | | | 3.0 |
| | | | | | | | |
| 93 | 0.2500 | 9.0 | 7.0 | 9.0 | 9.0 | | 7.0 |
| | 0.1250 | 8.0 | 4.0 | 9.0 | 9.0 | 7.0 | 6.0 |
| | 0.0625 | 8.0 | 2.0 | 2.0 | 9.0 | 0.0 | 4.0 |
| | 0.0313 | 7.0 | 0.0 | | 9.0 | 0.0 | 4.0 |
| | | | | | | | |
| 94 | 0.2500 | 7.0 | 7.0 | 0.0 | 9.0 | 0.0 | 0.0 |
| | 0.1250 | 4.0 | 4.0 | 0.0 | 9.0 | 0.0 | 1.0 |
| | 0.0625 | 2.0 | 2.0 | 0.0 | 9.0 | 0.0 | 0.0 |
| | 0.0313 | 0.0 | 0.0 | 0.0 | 6.0 | 0.0 | 0.0 |
| | | | | | | | |
| 95 | 0.2500 | 0.0 | 8.0 | 0.0 | 9.0 | 0.0 | 4.0 |
| | 0.1250 | 0.0 | 2.0 | 0.0 | 6.0 | 0.0 | 2.0 |
| | 0.0625 | 0.0 | 0.0 | 0.0 | 2.0 | 0.0 | 2.0 |
| | 0.0313 | 0.0 | 0.0 | 0.0 | 0.0 | 0.0 | 0.0 |
| | | | | | | | |
| 96 | 0.2500 | 7.0 | 0.0 | 2.0 | 9.0 | 0.0 | 2.0 |
| | 0.1250 | 4.0 | 0.0 | 0.0 | 9.0 | 0.0 | 3.0 |
| | 0.0625 | 4.0 | 0.0 | 0.0 | 9.0 | 0.0 | 2.0 |
| | 0.0313 | 2.0 | 0.0 | 0.0 | 8.0 | 0.0 | 2.0 |
| | | | | | | | |
| 97 | 0.2500 | 8.5 | 0.0 | 0.0 | 9.0 | 0.0 | 5.5 |
| | 0.1250 | 7.5 | 0.0 | 0.0 | 4.0 | 0.0 | 4.0 |
| | 0.0625 | 5.5 | 0.0 | 0.0 | 2.0 | 0.0 | 2.5 |
| | 0.0313 | 5.5 | 0.0 | 0.0 | 0.0 | 0.0 | 1.5 |
| | | | | | | | |
| 98 | 0.2500 | 6.5 | 6.0 | 0.0 | 9.0 | 0.0 | 7.0 |
| | 0.1250 | 6.5 | 6.0 | 0.0 | 9.0 | 0.0 | 7.0 |
| | 0.0625 | 5.0 | 4.0 | 0.0 | 4.0 | 0.0 | 6.0 |
| | 0.0313 | 3.0 | 0.0 | 0.0 | 2.0 | 0.0 | 4.0 |
| | | | | | | | |
| 99 | 0.2500 | 4.5 | 2.0 | 0.0 | 9.0 | 0.0 | 3.5 |
| | 0.1250 | 4.5 | 0.0 | 0.0 | 8.0 | 0.0 | 2.5 |
| | 0.0625 | 3.0 | 0.0 | 0.0 | | 0.0 | 1.5 |
| | 0.0313 | 1.0 | 0.0 | 0.0 | 2.0 | 0.0 | 0.5 |
| | | | | | | | |
| 100 | 0.2500 | 0.0 | 0.0 | 0.0 | 9.0 | 0.0 | 3.0 |
| | 0.1250 | 0.0 | 0.0 | 0.0 | 8.0 | 0.0 | 3.0 |
| | 0.0625 | 0.0 | 0.0 | 0.0 | 8.0 | 0.0 | 2.0 |
| | 0.0313 | 0.0 | 0.0 | 0.0 | 6.0 | 0.0 | 2.0 |
| | | | | | | | |
| 101 | 0.2500 | 0.0 | 0.0 | 0.0 | 9.0 | 0.0 | 1.0 |
| | 0.1250 | 0.0 | 0.0 | 0.0 | 4.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 102 | 0.2500 | 0.0 | 0.0 | 0.0 | 4.0 | 0.0 | 2.0 |
| | 0.1250 | 0.0 | 0.0 | 0.0 | 2.0 | 0.0 | 1.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 103 | 0.2500 | 6.0 | 0.0 | 0.0 | 9.0 | 0.0 | 4.0 |
| | 0.1250 | 4.0 | 0.0 | 0.0 | 9.0 | 0.0 | 4.0 |
| | 0.0625 | 2.0 | 0.0 | 0.0 | 9.0 | 0.0 | 3.0 |
| | 0.0313 | 0.0 | 0.0 | 0.0 | 8.0 | 0.0 | 2.0 |
| | | | | | | | |
| 104 | 0.2500 | 0.0 | 0.0 | 0.0 | 9.0 | 0.0 | 4.0 |
| | 0.1250 | 0.0 | 0.0 | 0.0 | 8.0 | 0.0 | 3.0 |
| | 0.0625 | 0.0 | 0.0 | 0.0 | 7.0 | 0.0 | 2.0 |
| | 0.0313 | 0.0 | 0.0 | 0.0 | 7.0 | 0.0 | 2.0 |
| | | | | | | | |
| 105 | 0.2500 | 9.0 | 9.0 | 9.0 | 9.0 | 9.0 | 8.0 |
| | 0.1250 | 9.0 | 9.0 | 9.0 | 9.0 | 9.0 | 8.0 |
| | 0.0625 | 9.0 | 8.0 | 7.0 | 9.0 | 4.0 | 6.0 |
| | 0.0313 | 9.0 | 8.0 | 7.0 | 9.0 | 2.0 | 6.0 |
| | | | | | | | |
| 106 | 0.2500 | 0.0 | 4.0 | 0.0 | 8.0 | 0.0 | 0.0 |
| | 0.1250 | 0.0 | 2.0 | 0.0 | 8.0 | 0.0 | 0.0 |
| | 0.0625 | 0.0 | 0.0 | 0.0 | 4.0 | 0.0 | 0.0 |
| | 0.0313 | 0.0 | 0.0 | 0.0 | 2.0 | 0.0 | 0.0 |
| | | | | | | | |
| 107 | 0.2500 | 4.0 | 6.0 | 0.0 | 9.0 | 0.0 | 6.0 |
| | 0.1250 | 1.0 | 2.0 | 0.0 | 9.0 | 0.0 | 5.0 |
| | 0.0625 | 0.0 | 0.0 | 0.0 | 9.0 | 0.0 | 3.0 |
| | 0.0313 | 0.0 | 0.0 | 0.0 | 9.0 | 0.0 | 2.0 |
| | | | | | | | |
| 108 | 0.2500 | 0.0 | 0.0 | 0.0 | 8.0 | 0.0 | 0.0 |
| | 0.1250 | 0.0 | 0.0 | 0.0 | 4.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 109 | 0.2500 | 7.0 | 8.0 | 0.0 | 9.0 | 0.0 | 4.0 |
| | 0.1250 | 4.0 | 4.0 | 0.0 | 9.0 | 0.0 | 4.0 |
| | 0.0625 | 2.0 | 0.0 | 0.0 | 9.0 | 0.0 | 2.0 |
| | 0.0313 | 0.0 | 0.0 | 0.0 | 9.0 | 0.0 | 2.0 |
| | | | | | | | |
| 110 | 0.2500 | 7.0 | 7.0 | 0.0 | 9.0 | 0.0 | 6.0 |
| | 0.1250 | 4.0 | 2.0 | 0.0 | 9.0 | 0.0 | 5.0 |
| | 0.0625 | 1.0 | 0.0 | 0.0 | 9.0 | 0.0 | 4.0 |
| | 0.0313 | 0.0 | 0.0 | 0.0 | 9.0 | 0.0 | 3.0 |
| | | | | | | | |
| 112 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 114 | 0.2500 | 6.0 | 9.0 | 6.0 | 8.0 | 6.0 | 7.0 |
| | 0.1250 | 4.0 | 9.0 | 2.0 | 9.0 | 6.0 | 7.0 |
| | 0.0625 | 0.0 | 9.0 | 0.0 | 9.0 | 2.0 | 6.0 |
| | 0.0313 | 0.0 | 9.0 | 0.0 | 9.0 | 0.0 | 4.0 |
| | | | | | | | |
| 116 | 0.2500 | 0.0 | 0.0 | 0.0 | 8.0 | 0.0 | 0.0 |
| | 0.1250 | 0.0 | 0.0 | 0.0 | 8.0 | 0.0 | 0.0 |
| | 0.0625 | 0.0 | 0.0 | 0.0 | 2.0 | 0.0 | 0.0 |
| | 0.0313 | 0.0 | 0.0 | 0.0 | 0.0 | 0.0 | 0.0 |
| | | | | | | | |
| 117 | 0.2500 | 0.0 | 0.0 | 0.0 | 4.0 | 0.0 | 0.0 |
| | 0.1250 | 0.0 | 0.0 | 0.0 | 2.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 118 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 119 | 0.2500 | 9.0 | 9.0 | 9.0 | 9.0 | 9.0 | 9.0 |
| | 0.1250 | 9.0 | 9.0 | 8.0 | 9.0 | 8.0 | 9.0 |
| | 0.0625 | 9.0 | 9.0 | 4.0 | 9.0 | 8.0 | 8.0 |
| | 0.0313 | 4.0 | 9.0 | 2.0 | 9.0 | 6.0 | 7.0 |
| | | | | | | | |
| 121 | 0.2500 | 9.0 | 4.0 | 4.0 | 9.0 | 0.0 | 9.0 |
| | 0.1250 | 8.0 | 2.0 | 2.0 | 9.0 | 0.0 | 6.0 |
| | 0.0625 | 7.0 | 0.0 | 0.0 | 9.0 | 0.0 | 4.0 |
| | 0.0313 | 6.0 | 0.0 | 0.0 | 9.0 | 0.0 | 3.0 |
| | | | | | | | |
| 122 | 0.2500 | 9.0 | 0.0 | 0.0 | 9.0 | 0.0 | 4.0 |
| | 0.1250 | 8.0 | 0.0 | 0.0 | 9.0 | 0.0 | 4.0 |
| | 0.0625 | 7.0 | 0.0 | 0.0 | 8.0 | 0.0 | 3.0 |
| | 0.0313 | 4.0 | 0.0 | 0.0 | 6.0 | 0.0 | 2.0 |
| | | | | | | | |
| 128 | 0.2500 | 0.0 | 0.0 | 0.0 | 9.0 | 0.0 | 0.0 |
| | 0.1250 | 0.0 | 0.0 | 0.0 | 7.0 | 0.0 | 0.0 |
| | 0.0625 | 0.0 | 0.0 | 0.0 | 4.0 | 0.0 | 0.0 |
| | 0.0313 | 0.0 | 0.0 | 0.0 | 2.0 | 0.0 | 0.0 |
| | | | | | | | |
| 129 | 0.2500 | 2.0 | 7.0 | 0.0 | 9.0 | 0.0 | 3.0 |
| | 0.1250 | 1.0 | 7.0 | 0.0 | 9.0 | 0.0 | 3.0 |
| | 0.0625 | 0.0 | 4.0 | 0.0 | 9.0 | 0.0 | 2.0 |
| | 0.0313 | 0.0 | 0.0 | 0.0 | 8.0 | 0.0 | 1.0 |
| | | | | | | | |
| 130 | 0.2500 | 2.0 | 6.0 | 0.0 | 9.0 | 0.0 | 7.0 |
| | 0.1250 | 0.0 | 2.0 | 0.0 | 9.0 | 0.0 | 7.0 |
| | 0.0625 | 0.0 | 0.0 | 0.0 | 9.0 | 0.0 | 4.0 |
| | 0.0313 | 0.0 | 0.0 | 0.0 | 9.0 | 0.0 | 3.0 |
| | | | | | | | |
| 131 | 0.2500 | 4.0 | 8.0 | 0.0 | 9.0 | 0.0 | 7.0 |
| | 0.1250 | 0.0 | 2.0 | 0.0 | 9.0 | 0.0 | 6.0 |
| | 0.0625 | 0.0 | 0.0 | 0.0 | 9.0 | 0.0 | 3.0 |
| | 0.0313 | 0.0 | 0.0 | 0.0 | 8.0 | 0.0 | 2.0 |
| | | | | | | | |
| 132 | 0.2500 | 9.0 | 9.0 | 2.0 | | 4.0 | 9.0 |
| | 0.1250 | 9.0 | 9.0 | 2.0 | 0.0 | 2.0 | 6.0 |
| | 0.0625 | 6.0 | 9.0 | 0.0 | 0.0 | 0.0 | 3.0 |
| | 0.0313 | 4.0 | 9.0 | 0.0 | 0.0 | 0.0 | 3.0 |
| | | | | | | | |
| 133 | 0.2500 | 9.0 | 9.0 | 0.0 | 2.0 | 0.0 | 5.0 |
| | 0.1250 | 6.0 | 9.0 | 0.0 | 1.0 | 0.0 | 4.0 |
| | 0.0625 | 2.0 | 9.0 | 0.0 | 0.0 | 0.0 | 3.0 |
| | 0.0313 | 1.0 | 9.0 | 0.0 | 0.0 | 0.0 | 2.0 |
| | | | | | | | |
| 134 | 0.2500 | 8.0 | 9.0 | 9.0 | 7.0 | 4.0 | 9.0 |
| | 0.1250 | 9.0 | 9.0 | 4.0 | 4.0 | 2.0 | 7.0 |
| | 0.0625 | 9.0 | 9.0 | 4.0 | 4.0 | 2.0 | 4.0 |
| | 0.0313 | 6.0 | 9.0 | 2.0 | 2.0 | 2.0 | 3.0 |
| | | | | | | | |
| 135 | 0.2500 | 6.0 | 9.0 | 0.0 | 0.0 | 0.0 | 4.0 |
| | 0.1250 | 2.0 | 9.0 | 0.0 | 0.0 | 0.0 | 3.0 |
| | 0.0625 | 0.0 | 8.0 | 0.0 | 0.0 | 0.0 | 3.0 |
| | 0.0313 | 0.0 | 6.0 | 0.0 | 0.0 | 0.0 | 2.0 |
| | | | | | | | |
| 136 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 137 | 0.2500 | 7.0 | 9.0 | 6.0 | 4.0 | 4.0 | 8.0 |
| | 0.1250 | 2.0 | 9.0 | 0.0 | 2.0 | 0.0 | 8.0 |
| | 0.0625 | 0.0 | 9.0 | 0.0 | 0.0 | 0.0 | 7.0 |
| | 0.0313 | 0.0 | 9.0 | 0.0 | 0.0 | 0.0 | 4.0 |
| | | | | | | | |
| 138 | 0.1250 | 0.0 | 8.0 | 0.0 | 0.0 | 0.0 | 3.0 |
| | 0.0625 | 0.0 | 6.0 | 0.0 | 0.0 | 0.0 | 3.0 |
| | 0.0313 | 0.0 | 2.0 | 0.0 | 0.0 | 0.0 | 2.0 |
| | | | | | | | |
| 139 | 0.1250 | 9.0 | 9.0 | 4.0 | 8.0 | 0.0 | 4.0 |
| | 0.0625 | 6.0 | 9.0 | 0.0 | 6.0 | 0.0 | 4.0 |
| | 0.0313 | 4.0 | 9.0 | 0.0 | 2.0 | 0.0 | 3.0 |
| | | | | | | | |
| 140 | 0.1250 | 6.0 | 9.0 | 0.0 | 2.0 | 0.0 | 8.0 |
| | 0.0625 | 4.0 | 9.0 | 0.0 | 2.0 | 0.0 | 7.0 |
| | 0.0313 | 2.0 | 9.0 | 0.0 | 2.0 | 0.0 | 6.0 |
| | | | | | | | |
| 141 | 0.1250 | 2.0 | 9.0 | 0.0 | 0.0 | 0.0 | 3.0 |
| | 0.0625 | 1.0 | 4.0 | 0.0 | 0.0 | 0.0 | 3.0 |
| | 0.0313 | 0.0 | 2.0 | 0.0 | 0.0 | 0.0 | 2.0 |
| | | | | | | | |
| 142 | 0.2500 | 9.0 | 8.0 | 9.0 | 9.0 | 0.0 | 9.0 |
| | 0.1250 | 9.0 | 6.0 | 2.0 | 9.0 | 0.0 | 7.0 |
| | 0.0625 | 9.0 | 6.0 | 0.0 | 9.0 | 0.0 | 6.0 |
| | 0.0313 | 9.0 | 4.0 | 0.0 | 9.0 | 0.0 | 3.0 |
| | | | | | | | |
| 143 | 0.2500 | 6.0 | 0.0 | 0.0 | 9.0 | 0.0 | 4.0 |
| | 0.1250 | 2.0 | 0.0 | 0.0 | 9.0 | 0.0 | 3.0 |
| | 0.0625 | 0.0 | 0.0 | 0.0 | 9.0 | 0.0 | 3.0 |
| | 0.0313 | 0.0 | 0.0 | 0.0 | 9.0 | 0.0 | 3.0 |
| | | | | | | | |
| 144 | 0.2500 | 9.0 | 8.0 | 0.0 | 9.0 | 0.0 | 2.0 |
| | 0.1250 | 9.0 | 4.0 | 0.0 | 9.0 | 0.0 | 2.0 |
| | 0.0625 | 6.0 | 2.0 | 0.0 | 4.0 | 0.0 | 2.0 |
| | 0.0313 | 2.0 | 0.0 | 0.0 | 2.0 | 0.0 | 1.0 |
| | | | | | | | |
| 170 | 0.2500 | 9.0 | 6.0 | 0.0 | 9.0 | 0.0 | 8.0 |
| | 0.1250 | 9.0 | 4.0 | 0.0 | 9.0 | 0.0 | 6.0 |
| | 0.0625 | 9.0 | 0.0 | 0.0 | 2.0 | 0.0 | 5.0 |
| | 0.0313 | 9.0 | 0.0 | 0.0 | 0.0 | 0.0 | 3.0 |
| | | | | | | | |
| 171 | 0.2500 | 9.0 | 2.0 | 0.0 | 9.0 | 0.0 | 2.0 |
| | 0.1250 | 9.0 | 2.0 | 0.0 | 9.0 | 0.0 | 1.0 |
| | 0.0625 | 9.0 | 0.0 | 0.0 | 9.0 | 0.0 | 0.0 |
| | 0.0313 | 8.5 | 0.0 | 0.0 | 9.0 | 0.0 | 0.0 |
| | | | | | | | |
| 172 | 0.2500 | 9.0 | 4.0 | 2.0 | 9.0 | 0.0 | 8.0 |
| | 0.1250 | 9.0 | 4.0 | 1.0 | 9.0 | 0.0 | 6.0 |
| | 0.0625 | 9.0 | 2.0 | 0.0 | 9.0 | 0.0 | 3.5 |
| | 0.0313 | 6.5 | 2.0 | 0.0 | 9.0 | 0.0 | 2.5 |
| | | | | | | | |
| 173 | 0.2500 | 9.0 | 2.0 | | 9.0 | 0.0 | 3.5 |
| | 0.1250 | 6.5 | 1.0 | 0.0 | 9.0 | 0.0 | 3.0 |
| | 0.0625 | 4.5 | 0.0 | 0.0 | 9.0 | 0.0 | 2.5 |
| | 0.0313 | 2.0 | 0.0 | 0.0 | 8.0 | 0.0 | 2.0 |
| | | | | | | | |
| 174 | 0.2500 | 8.5 | 2.0 | 0.0 | 9.0 | 0.0 | 4.0 |
| | 0.1250 | 6.5 | 2.0 | 0.0 | 9.0 | 0.0 | 2.5 |
| | 0.0625 | 5.5 | 2.0 | 0.0 | 9.0 | 0.0 | 2.5 |
| | 0.0313 | 4.5 | 1.0 | 0.0 | 9.0 | 0.0 | 1.5 |
| | | | | | | | |
| 175 | 0.2500 | 8.0 | 2.0 | 0.0 | 9.0 | 0.0 | 6.0 |
| | 0.1250 | 8.0 | 1.0 | 0.0 | 9.0 | 0.0 | 5.0 |
| | 0.0625 | 7.5 | 6.0 | 0.0 | 9.0 | 0.0 | 3.5 |
| | 0.0313 | 6.5 | 0.0 | 0.0 | 8.0 | 0.0 | 2.5 |
| | | | | | | | |
| 176 | 0.2500 | 8.0 | 8.0 | 0.0 | 9.0 | 0.0 | 6.5 |
| | 0.1250 | 8.0 | 4.0 | 0.0 | 9.0 | 0.0 | 5.0 |
| | 0.0625 | 5.5 | 2.0 | 0.0 | 9.0 | 0.0 | 3.5 |
| | 0.0313 | 4.5 | 2.0 | 0.0 | 9.0 | 0.0 | 2.5 |
| | | | | | | | |
| 177 | 0.2500 | 9.0 | 4.0 | 8.0 | 9.0 | 0.0 | 6.5 |
| | 0.1250 | 9.0 | 4.0 | 7.0 | 9.0 | 0.0 | 4.5 |
| | 0.0625 | 6.5 | 2.0 | 0.0 | 9.0 | 0.0 | 3.5 |
| | 0.0313 | 3.0 | 0.0 | 0.0 | 9.0 | 0.0 | 2.5 |
| | | | | | | | |
| 178 | 0.2500 | 8.5 | 2.0 | 0.0 | 9.0 | 0.0 | 6.0 |
| | 0.1250 | 7.5 | 1.0 | 0.0 | 9.0 | 0.0 | 5.0 |
| | 0.0625 | 7.5 | 0.0 | 0.0 | 9.0 | 0.0 | 3.5 |
| | 0.0313 | 4.0 | 0.0 | 0.0 | 9.0 | 0.0 | 2.5 |
| | | | | | | | |
| 179 | 0.2500 | 9.0 | 6.0 | 6.0 | 9.0 | 4.0 | 9.0 |
| | 0.1250 | 9.0 | 6.0 | 6.0 | 9.0 | 0.0 | 8.5 |
| | 0.0625 | 8.0 | 4.0 | 2.0 | 9.0 | 0.0 | 8.0 |
| | 0.0313 | 4.5 | 2.0 | 0.0 | 9.0 | 0.0 | 6.5 |
| | | | | | | | |
| 180 | 0.2500 | 8.5 | 8.0 | 6.0 | 9.0 | 0.0 | 8.5 |
| | 0.1250 | 8.0 | 7.0 | 4.0 | 9.0 | 0.0 | 8.0 |
| | 0.0625 | 5.0 | 4.0 | 0.0 | 9.0 | 0.0 | 7.5 |
| | 0.0313 | 3.5 | 2.0 | 0.0 | 9.0 | 0.0 | 6.0 |
| | | | | | | | |
| 181 | 0.2500 | 9.0 | 9.0 | 8.0 | 9.0 | 0.0 | 7.0 |
| | 0.1250 | 9.0 | 9.0 | 7.0 | 9.0 | 0.0 | 6.0 |
| | 0.0625 | 8.5 | 9.0 | 7.0 | 9.0 | 0.0 | 5.0 |
| | 0.0313 | 8.5 | 7.0 | 4.0 | 9.0 | 0.0 | 3.5 |
| | | | | | | | |
| 182 | 0.0630 | 9.0 | 9.0 | 4.0 | 9.0 | 4.0 | 9.0 |
| | 0.0315 | 9.0 | 9.0 | 2.0 | 9.0 | 4.0 | 7.0 |
| | | | | | | | |
| 183 | 0.0630 | 9.0 | 9.0 | 0.0 | 9.0 | 0.0 | 9.0 |
| | 0.0315 | 9.0 | 9.0 | 0.0 | 9.0 | 0.0 | 8.0 |
| | | | | | | | |
| 184 | 0.0630 | 9.0 | 4.0 | 6.0 | 9.0 | 4.0 | 9.0 |
| | 0.0315 | 9.0 | 1.0 | 2.0 | 9.0 | 2.0 | 7.0 |
| | | | | | | | |
| 185 | 0.0630 | 0.0 | 0.0 | 0.0 | 9.0 | 0.0 | 0.0 |
| | 0.0315 | 0.0 | 0.0 | 0.0 | 9.0 | 0.0 | 0.0 |
| | | | | | | | |
| 186 | 0.0630 | 9.0 | 9.0 | 9.0 | 9.0 | 9.0 | 9.0 |
| | 0.0315 | 9.0 | 9.0 | 9.0 | 9.0 | 9.0 | 9.0 |
| | | | | | | | |
| 187 | 0.2500 | 9.0 | 4.0 | 0.0 | 9.0 | 0.0 | 6.0 |
| | 0.1250 | 6.0 | 2.0 | 0.0 | 9.0 | 0.0 | 3.0 |
| | 0.0625 | 2.0 | 0.0 | 0.0 | 9.0 | 0.0 | 3.0 |
| | 0.0313 | 0.0 | 0.0 | 0.0 | 8.0 | 0.0 | 2.0 |
| | | | | | | | |
| 188 | 0.2500 | 8.0 | 0.0 | | 9.0 | 0.0 | 2.0 |
| | 0.1250 | 2.0 | 0.0 | | 9.0 | 0.0 | 2.0 |
| | 0.0625 | 0.0 | 0.0 | | 9.0 | 0.0 | 1.0 |
| | 0.0313 | 0.0 | 0.0 | 0.0 | 9.0 | 0.0 | 1.0 |
| | | | | | | | |
| 189 | 0.2500 | 0.0 | 0.0 | 0.0 | 8.0 | 0.0 | 0.0 |
| | 0.1250 | 0.0 | 0.0 | 0.0 | 2.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 190 | 0.2500 | 8.0 | 4.0 | 0.0 | 9.0 | 0.0 | 7.0 |
| | 0.1250 | 7.0 | 2.0 | 0.0 | 9.0 | 0.0 | 4.0 |
| | 0.0625 | 4.0 | 0.0 | 0.0 | 9.0 | 0.0 | 3.0 |
| | 0.0313 | 2.0 | 0.0 | 0.0 | 9.0 | 0.0 | 2.0 |
| | | | | | | | |
| 191 | 0.2500 | 7.0 | 4.0 | | 9.0 | 0.0 | 6.0 |
| | 0.1250 | 4.0 | 2.0 | 0.0 | 9.0 | 0.0 | 3.0 |
| | 0.0625 | 2.0 | 0.0 | 0.0 | 9.0 | 0.0 | 2.0 |
| | 0.0313 | 0.0 | 0.0 | 0.0 | 9.0 | 0.0 | 1.0 |
| | | | | | | | |
| 192 | 0.2500 | 9.0 | 7.0 | | 9.0 | 9.0 | 4.0 |
| | 0.1250 | 9.0 | 2.0 | 0.0 | 9.0 | 2.0 | 3.0 |
| | 0.0625 | 4.0 | 0.0 | 0.0 | 9.0 | 0.0 | 2.0 |
| | 0.0313 | 1.0 | 0.0 | 0.0 | 9.0 | 0.0 | 1.0 |
| | | | | | | | |
| 193 | 0.2500 | 0.0 | 0.0 | 0.0 | 9.0 | 0.0 | 1.0 |
| | 0.1250 | 0.0 | 0.0 | 0.0 | 9.0 | 0.0 | 0.0 |
| | 0.0625 | 0.0 | 0.0 | 0.0 | 4.0 | 0.0 | 0.0 |
| | 0.0313 | 0.0 | 0.0 | 0.0 | 2.0 | 0.0 | 0.0 |
| | | | | | | | |
| 194 | 0.2500 | 9.0 | 0.0 | 0.0 | 9.0 | 0.0 | 4.0 |
| | 0.1250 | 6.0 | 0.0 | 0.0 | 9.0 | 0.0 | 3.0 |
| | 0.0625 | 2.0 | 0.0 | 0.0 | 9.0 | 0.0 | 3.0 |
| | 0.0313 | 0.0 | 0.0 | 0.0 | 9.0 | 0.0 | 2.0 |
| | | | | | | | |
| 195 | 0.2500 | 9.0 | 9.0 | 7.0 | 9.0 | 2.0 | 9.0 |
| | 0.1250 | 6.0 | 4.0 | 2.0 | 9.0 | 1.0 | 9.0 |
| | 0.0625 | 3.0 | 2.0 | 0.0 | 9.0 | 0.0 | 7.0 |
| | 0.0313 | 2.0 | 0.0 | 0.0 | 9.0 | 0.0 | 6.0 |
| | | | | | | | |
| 196 | 0.2500 | 4.0 | 4.0 | 9.0 | 9.0 | 0.0 | 8.0 |
| | 0.1250 | 4.0 | 1.0 | 6.0 | 9.0 | 0.0 | 8.0 |
| | 0.0625 | 2.0 | 0.0 | 4.0 | 9.0 | 0.0 | 6.0 |
| | 0.0313 | 0.0 | 0.0 | 2.0 | 9.0 | 0.0 | 4.0 |
| | | | | | | | |
| 198 | 0.2500 | 9.0 | 7.0 | 8.0 | 9.0 | 4.0 | 9.0 |
| | 0.1250 | 9.0 | 4.0 | 4.0 | 9.0 | 2.0 | 8.0 |
| | 0.0625 | 6.0 | 2.0 | 2.0 | 9.0 | 0.0 | 7.0 |
| | 0.0313 | 4.0 | 0.0 | 0.0 | 9.0 | 0.0 | 4.0 |
| | | | | | | | |
| 199 | 0.2500 | 7.0 | 8.0 | 0.0 | 9.0 | 0.0 | 8.0 |
| | 0.1250 | 7.0 | 4.0 | 0.0 | 9.0 | 0.0 | 6.0 |
| | 0.0625 | 4.0 | 2.0 | 0.0 | 9.0 | 0.0 | 4.0 |
| | 0.0313 | 2.0 | 0.0 | 0.0 | 8.0 | 0.0 | 3.0 |
| | | | | | | | |
| 200 | 0.2500 | 9.0 | 8.0 | 7.0 | 9.0 | 4.0 | 9.0 |
| | 0.1250 | 6.0 | 7.0 | 2.0 | 9.0 | 0.0 | 9.0 |
| | 0.0625 | 2.0 | 6.0 | 0.0 | 9.0 | 0.0 | 7.0 |
| | 0.0313 | 0.0 | 4.0 | 0.0 | 9.0 | 0.0 | 7.0 |
| | | | | | | | |
| 202 | 0.2500 | 2.0 | 9.0 | 0.0 | 0.0 | 0.0 | 6.0 |
| | 0.1250 | 2.0 | 9.0 | 0.0 | 0.0 | 0.0 | 5.0 |
| | 0.0625 | 1.0 | 9.0 | 0.0 | 0.0 | 0.0 | 4.0 |
| | 0.0313 | 1.0 | 9.0 | 0.0 | 0.0 | 0.0 | 3.0 |
| | | | | | | | |
| 203 | 0.2500 | 4.0 | 9.0 | 6.0 | 8.0 | 4.0 | 7.0 |
| | 0.1250 | 2.0 | 9.0 | 2.0 | 2.0 | 2.0 | 6.0 |
| | 0.0625 | 0.0 | 9.0 | 0.0 | 0.0 | 0.0 | 6.0 |
| | 0.0313 | 0.0 | 9.0 | 0.0 | 0.0 | 0.0 | 4.0 |
| | | | | | | | |
| 204 | 0.2500 | 9.0 | 9.0 | 7.0 | 7.0 | 0.0 | 7.0 |
| | 0.1250 | 4.0 | 9.0 | 2.0 | 4.0 | 0.0 | 7.0 |
| | 0.0625 | 2.0 | 9.0 | 0.0 | 2.0 | 0.0 | 4.0 |
| | 0.0313 | 0.0 | 9.0 | 0.0 | 0.0 | 0.0 | 3.0 |
| | | | | | | | |
| 206 | 0.2500 | 9.0 | 9.0 | 7.0 | 4.0 | 4.0 | 9.0 |
| | 0.1250 | 2.0 | 9.0 | 7.0 | 1.0 | 1.0 | 7.0 |
| | 0.0625 | 2.0 | 9.0 | 2.0 | 0.0 | 0.0 | 6.0 |
| | 0.0313 | 0.0 | 9.0 | 0.0 | 0.0 | 0.0 | 5.0 |
| | | | | | | | |
| 207 | 0.2500 | 5.5 | 8.5 | 6.0 | 7.5 | 1.0 | 7.5 |
| | 0.1250 | 2.0 | 8.5 | 1.0 | 5.5 | 0.0 | 7.0 |
| | 0.0625 | 1.5 | 6.5 | 0.0 | 4.5 | 0.0 | 6.0 |
| | 0.0313 | 1.0 | 5.5 | 0.0 | 4.0 | 0.0 | 5.0 |
| | | | | | | | |
| 208 | 0.2500 | 0.0 | 9.0 | 0.0 | 4.0 | 0.0 | 5.0 |
| | 0.1250 | 0.0 | 9.0 | 0.0 | 2.0 | 0.0 | 3.0 |
| | 0.0625 | 0.0 | 9.0 | 0.0 | 2.0 | 0.0 | 3.0 |
| | 0.0313 | 0.0 | 9.0 | 0.0 | 0.0 | 0.0 | 2.0 |
| | | | | | | | |
| 209 | 0.1250 | 2.0 | 8.0 | 0.0 | 0.0 | 0.0 | 4.0 |
| | 0.0625 | 2.0 | 4.0 | 0.0 | 0.0 | 0.0 | 3.0 |
| | 0.0313 | 1.0 | 2.0 | 0.0 | 0.0 | 0.0 | 3.0 |
| | | | | | | | |
| 210 | 0.2500 | 9.0 | 7.0 | 0.0 | 9.0 | 0.0 | 7.0 |
| | 0.1250 | 9.0 | | 0.0 | 9.0 | 0.0 | 6.0 |
| | 0.0625 | 9.0 | 7.0 | 0.0 | 9.0 | 0.0 | 4.0 |
| | 0.0313 | 9.0 | 2.0 | 0.0 | 9.0 | 0.0 | 3.0 |
| | | | | | | | |
| 211 | 0.2500 | 9.0 | 4.0 | 0.0 | 9.0 | 0.0 | 3.0 |
| | 0.1250 | 7.0 | 4.0 | 0.0 | 9.0 | 0.0 | 2.0 |
| | 0.0625 | 4.0 | 0.0 | 0.0 | 9.0 | 0.0 | 2.0 |
| | 0.0313 | 2.0 | 0.0 | 0.0 | 9.0 | 0.0 | 2.0 |
| | | | | | | | |
| 212 | 0.2500 | 4.0 | 2.0 | 0.0 | 9.0 | 0.0 | 7.0 |
| | 0.1250 | 2.0 | 0.0 | 0.0 | 9.0 | 0.0 | 6.0 |
| | 0.0625 | 0.0 | 0.0 | 0.0 | 8.0 | 0.0 | 4.0 |
| | 0.0313 | 0.0 | 0.0 | 0.0 | 4.0 | 0.0 | 3.0 |
| | | | | | | | |
| 213 | 0.2500 | 0.0 | | 0.0 | 2.0 | 0.0 | 7.0 |
| | 0.1250 | 0.0 | 0.0 | 0.0 | 0.0 | 0.0 | 6.0 |
| | 0.0625 | 0.0 | 0.0 | 0.0 | 0.0 | 0.0 | 4.0 |
| | 0.0313 | 0.0 | 0.0 | 0.0 | 0.0 | 0.0 | 3.0 |
| | | | | | | | |
| 214 | 0.2500 | 0.0 | 7.0 | 0.0 | 9.0 | 0.0 | 5.0 |
| | 0.1250 | 0.0 | 7.0 | 0.0 | 9.0 | 0.0 | 3.0 |
| | 0.0625 | 0.0 | 4.0 | 0.0 | 9.0 | 0.0 | 2.0 |
| | 0.0313 | 0.0 | 2.0 | 0.0 | 8.0 | 0.0 | 2.0 |
| | | | | | | | |
| 217 | 0.2500 | 0.0 | 0.0 | 0.0 | 7.0 | 0.0 | 4.0 |
| | 0.1250 | 0.0 | 0.0 | 0.0 | 7.0 | 0.0 | 3.0 |
| | 0.0625 | 0.0 | 0.0 | 0.0 | 4.0 | 0.0 | 2.0 |
| | 0.0313 | 0.0 | 0.0 | 0.0 | 2.0 | 0.0 | 2.0 |
| | | | | | | | |
| 218 | 0.2500 | 4.0 | 0.0 | 0.0 | 9.0 | 0.0 | 3.0 |
| | 0.1250 | 2.0 | 0.0 | 0.0 | 9.0 | 0.0 | 3.0 |
| | 0.0625 | 0.0 | 0.0 | 0.0 | 9.0 | 0.0 | 2.0 |
| | 0.0313 | 0.0 | 0.0 | 0.0 | 8.0 | 0.0 | 1.0 |
| | | | | | | | |
| 223 | 0.2500 | 9.0 | 9.0 | 8.0 | 9.0 | 9.0 | 9.0 |
| | 0.1250 | 8.0 | 7.0 | 6.0 | 9.0 | 6.0 | 8.0 |
| | 0.0625 | 6.0 | 7.0 | 4.0 | 9.0 | 2.0 | 8.0 |
| | 0.0313 | 6.0 | 4.0 | 0.0 | 9.0 | 0.0 | 7.0 |
| | | | | | | | |
| 224 | 0.2500 | 9.0 | 8.0 | 7.0 | 9.0 | 8.0 | 9.0 |
| | 0.1250 | 9.0 | 8.0 | 2.0 | 9.0 | 4.0 | 8.0 |
| | 0.0625 | 9.0 | 7.0 | 0.0 | 9.0 | 2.0 | 7.0 |
| | 0.0313 | 6.0 | 4.0 | 0.0 | 9.0 | 0.0 | 6.0 |
| | | | | | | | |
| 225 | 0.2500 | 7.0 | 7.0 | 0.0 | 9.0 | 0.0 | 7.0 |
| | 0.1250 | 4.0 | 2.0 | 0.0 | 9.0 | 0.0 | 6.0 |
| | 0.0625 | 2.0 | 0.0 | 0.0 | 8.0 | 0.0 | 4.0 |
| | 0.0313 | 0.0 | 0.0 | 0.0 | 8.0 | 0.0 | 3.0 |
| | | | | | | | |
| 245 | 0.2500 | 0.0 | 0.0 | 0.0 | 0.0 | 0.0 | 0.0 |
| | 0.1250 | 0.0 | 0.0 | 0.0 | 0.0 | 0.0 | 0.0 |
| | 0.0625 | 0.0 | 0.0 | 0.0 | 0.0 | 0.0 | 0.0 |
| | 0.0313 | 0.0 | 0.0 | 0.0 | 0.0 | 0.0 | 0.0 |
| | | | | | | | |
| 246 | 0.2500 | 9.0 | 4.0 | | 9.0 | 2.0 | 2.0 |
| | 0.1250 | 9.0 | 4.0 | 0.0 | 9.0 | 2.0 | 2.0 |
| | 0.0625 | 7.0 | 2.0 | 0.0 | 9.0 | 0.0 | 1.0 |
| | 0.0313 | 6.0 | 0.0 | 0.0 | 9.0 | 0.0 | 0.0 |
| | | | | | | | |
| 256 | 0.2500 | 9.0 | 9.0 | 9.0 | 9.0 | 8.0 | 9.0 |
| | 0.1250 | 7.0 | 9.0 | 6.0 | 9.0 | 4.0 | 8.0 |
| | 0.0620 | 3.0 | 9.0 | 2.0 | 9.0 | 0.0 | 8.0 |
| | 0.0320 | 1.0 | 8.0 | 0.0 | 8.0 | 0.0 | 6.0 |
| | | | | | | | |
| 257 | 0.2500 | 0.0 | 6.0 | | 9.0 | 0.0 | 2.0 |
| | 0.1250 | 0.0 | 4.0 | | 9.0 | 0.0 | 1.0 |
| | 0.0620 | 0.0 | 2.0 | 0.0 | 9.0 | 0.0 | 0.0 |
| | 0.0320 | 0.0 | 0.0 | 0.0 | 8.0 | 0.0 | 0.0 |
| | | | | | | | |
| 258 | 0.2500 | 0.0 | 8.0 | | 9.0 | 0.0 | 4.0 |
| | 0.1250 | 0.0 | 7.0 | 0.0 | 9.0 | 0.0 | 3.0 |
| | 0.0620 | 0.0 | 2.0 | 0.0 | 9.0 | 0.0 | 2.0 |
| | 0.0320 | 0.0 | 0.0 | 0.0 | 9.0 | 0.0 | 2.0 |
| | | | | | | | |
| 259 | 0.2500 | 2.0 | 4.0 | | 9.0 | 0.0 | 3.0 |
| | 0.1250 | 0.0 | 4.0 | | 9.0 | 0.0 | 2.0 |
| | 0.0620 | 0.0 | 2.0 | 0.0 | 9.0 | 0.0 | 1.0 |
| | 0.0320 | 0.0 | 0.0 | 0.0 | 6.0 | 0.0 | 1.0 |
| | | | | | | | |
| 260 | 0.2500 | 0.0 | 0.0 | 0.0 | 0.0 | 0.0 | 0.0 |
| | | | | | | | |
| 261 | 0.2500 | 0.0 | 2.0 | 0.0 | 3.0 | 0.0 | 0.0 |
| | 0.1250 | 2.0 | 5.5 | 4.5 | 6.0 | 0.0 | 0.0 |
| | 0.0620 | 2.0 | 4.5 | 4.5 | 4.5 | 0.0 | 0.0 |
| | 0.0320 | 2.5 | 4.0 | 4.5 | 4.5 | 0.0 | 0.0 |
| | | | | | | | |
| 262 | 0.2500 | 3.0 | 9.0 | 0.0 | 9.0 | 0.0 | 3.0 |
| | 0.1250 | 0.0 | 6.0 | 0.0 | 9.0 | 0.0 | 0.0 |
| | 0.0620 | 0.0 | 0.0 | 0.0 | 0.0 | 0.0 | 0.0 |
| | | | | | | | |
| 263 | 0.2500 | 2.0 | 9.0 | 9.0 | 9.0 | 0.0 | 4.0 |
| | 0.1250 | 0.0 | 9.0 | 9.0 | 6.0 | 0.0 | 0.0 |
| | 0.0620 | 0.0 | 7.0 | 9.0 | 0.0 | 0.0 | 0.0 |
| | 0.0320 | 0.0 | 4.0 | 9.0 | 0.0 | 0.0 | 0.0 |
| | | | | | | | |
| 264 | 0.2500 | 2.0 | 5.0 | 9.0 | 9.0 | 0.0 | 5.0 |
| | 0.1250 | 0.0 | 5.0 | 9.0 | 9.0 | 0.0 | 0.0 |
| | 0.0620 | 0.0 | 5.0 | 4.0 | 9.0 | 0.0 | 0.0 |
| | 0.0320 | 0.0 | 5.0 | 4.0 | 9.0 | 0.0 | 0.0 |
| | | | | | | | |
| 265 | 0.2500 | 0.0 | 3.0 | 0.0 | 9.0 | 0.0 | 0.0 |
| | 0.1250 | 0.0 | 0.0 | 0.0 | 9.0 | 0.0 | 0.0 |
| | 0.0620 | 0.0 | 0.0 | 0.0 | 9.0 | 0.0 | 0.0 |
| | 0.0320 | 0.0 | 0.0 | 0.0 | 9.0 | 0.0 | 0.0 |
| | | | | | | | |
| 266 | 0.2500 | 0.0 | 9.0 | 3.0 | 9.0 | 0.0 | 0.0 |
| | 0.1250 | 0.0 | 5.0 | 0.0 | 9.0 | 0.0 | 0.0 |
| | 0.0620 | 0.0 | 5.0 | 0.0 | 9.0 | 0.0 | 0.0 |
| | 0.0320 | 0.0 | 5.0 | 0.0 | 9.0 | 0.0 | 0.0 |
| | | | | | | | |
| 267 | 0.2500 | 9.0 | 9.0 | 9.0 | 9.0 | 5.0 | 6.0 |
| | 0.1250 | 9.0 | 9.0 | 5.5 | 9.0 | 3.0 | 4.5 |
| | 0.0620 | 9.0 | 8.0 | 0.0 | 9.0 | 1.0 | 3.0 |
| | 0.0320 | 9.0 | 8.0 | 0.0 | 7.5 | 0.0 | 2.0 |
| | | | | | | | |
| 268 | 0.2500 | 4.5 | 7.5 | 9.0 | 9.0 | 4.0 | 7.5 |
| | 0.1250 | 4.5 | 6.0 | 9.0 | 9.0 | 1.0 | 5.0 |
| | 0.0620 | 3.0 | 3.0 | 5.5 | 9.0 | 1.0 | 4.0 |
| | 0.0320 | 1.0 | 1.0 | 1.0 | 7.5 | 0.0 | 3.5 |
| | | | | | | | |
| 269 | 0.5000 | 4.0 | 0.0 | 0.0 | 9.0 | 0.0 | 0.0 |
| | 0.2500 | 0.0 | 0.0 | 0.0 | 0.0 | 0.0 | 0.0 |
| | | | | | | | |
| 270 | 0.5000 | 9.0 | 9.0 | 3.0 | 9.0 | 5.0 | 2.0 |
| | 0.2500 | 9.0 | 9.0 | 9.0 | 9.0 | 0.0 | 8.0 |
| | 0.1250 | 4.3 | 9.0 | 7.0 | 9.0 | 0.0 | 7.0 |
| | 0.0620 | 2.7 | 9.0 | 0.0 | 9.0 | 0.0 | 4.0 |
| | 0.0320 | 1.3 | 8.0 | 0.0 | 9.0 | 0.0 | 4.0 |
| | | | | | | | |
| 271 | 0.5000 | 0.0 | 0.0 | 0.0 | 9.0 | 0.0 | 0.0 |
| | 0.2500 | 0.0 | 0.0 | 0.0 | 9.0 | 0.0 | 3.0 |
| | 0.1250 | 0.0 | 0.0 | 0.0 | 4.0 | 0.0 | 2.0 |
| | 0.0620 | 0.0 | 0.0 | 0.0 | 0.0 | 0.0 | 1.0 |
| | | | | | | | |
| 272 | 0.5000 | 0.0 | 0.0 | 0.0 | 0.0 | 0.0 | 0.0 |
| | | | | | | | |
| 273 | 0.5000 | 9.0 | 4.0 | 9.0 | 9.0 | 0.0 | 3.0 |
| | 0.2500 | 9.0 | 9.0 | 7.0 | 9.0 | 6.0 | 4.0 |
| | 0.1250 | 6.0 | 9.0 | | 9.0 | 0.0 | 3.0 |
| | 0.0620 | 5.1 | 9.0 | 0.0 | 9.0 | 0.0 | 2.0 |
| | 0.0320 | 3.9 | 6.0 | 0.0 | 9.0 | 0.0 | 0.0 |
| | | | | | | | |
| 274 | 0.5000 | 9.0 | 2.0 | 9.0 | 9.0 | 4.0 | 7.0 |
| | 0.2500 | 9.0 | 9.0 | 9.0 | 9.0 | 9.0 | 8.0 |
| | 0.1250 | 5.9 | 9.0 | 8.0 | 9.0 | 4.0 | 4.0 |
| | 0.0620 | 4.1 | 8.0 | 2.0 | 9.0 | 0.0 | 4.0 |
| | 0.0320 | 3.3 | 7.0 | 0.0 | 9.0 | 0.0 | 2.0 |
| | | | | | | | |
| 275 | 0.5000 | 7.0 | 0.0 | 0.0 | 9.0 | 0.0 | 5.0 |
| | 0.2500 | 9.0 | 7.0 | 9.0 | 9.0 | 9.0 | 4.0 |
| | 0.1250 | 4.1 | 6.0 | 9.0 | 9.0 | 3.0 | 3.0 |
| | 0.0620 | 3.9 | 4.0 | 9.0 | 9.0 | 0.0 | 0.0 |
| | 0.0320 | 3.7 | 0.0 | 9.0 | 6.0 | 0.0 | 0.0 |
| | | | | | | | |
| 276 | 0.5000 | 9.0 | 9.0 | 7.0 | 9.0 | 9.0 | 9.0 |
| | 0.2500 | 9.0 | 9.0 | 7.0 | 9.0 | 9.0 | 7.0 |
| | 0.1250 | 7.9 | 9.0 | 8.0 | 9.0 | 9.0 | 6.0 |
| | 0.0620 | 7.7 | 9.0 | 2.0 | 9.0 | 9.0 | 5.0 |
| | 0.0320 | 4.0 | 8.0 | 2.0 | 9.0 | 8.0 | 4.0 |
| | | | | | | | |
| 277 | 0.5000 | 9.0 | 9.0 | 9.0 | 9.0 | 9.0 | 9.0 |
| | 0.2500 | 9.0 | 9.0 | 9.0 | 9.0 | 9.0 | 8.0 |
| | 0.1250 | 9.0 | 9.0 | 9.0 | 9.0 | 9.0 | 7.0 |
| | 0.0620 | 8.6 | 9.0 | 9.0 | 9.0 | 8.0 | 7.0 |
| | 0.0320 | 7.0 | 9.0 | 2.0 | 9.0 | 6.0 | 5.0 |
| | | | | | | | |
| 278 | 0.5000 | 9.0 | 9.0 | 9.0 | 9.0 | 9.0 | 8.0 |
| | 0.2500 | 8.0 | 9.0 | 4.0 | 9.0 | 2.0 | 7.0 |
| | 0.1250 | 7.4 | 7.0 | 2.0 | 9.0 | 0.0 | 4.0 |
| | 0.0620 | 6.1 | 2.0 | 0.0 | 8.0 | 0.0 | 3.0 |
| | 0.0320 | 0.0 | 0.0 | 0.0 | 6.0 | 0.0 | 2.0 |
| | | | | | | | |
| 279 | 0.5000 | 7.0 | 9.0 | 5.0 | 9.0 | 9.0 | 6.0 |
| | 0.2500 | 6.0 | 6.0 | | 9.0 | 4.0 | 4.0 |
| | 0.1250 | 6.1 | 2.0 | 0.0 | 8.0 | 0.0 | 4.0 |
| | 0.0620 | 5.7 | 0.0 | 0.0 | | 0.0 | 3.0 |
| | 0.0320 | 0.0 | 0.0 | 0.0 | | 0.0 | 2.0 |
| | | | | | | | |
| 280 | 0.5000 | 9.0 | 9.0 | 3.0 | 9.0 | 9.0 | 9.0 |
| | 0.2500 | 9.0 | 9.0 | 2.0 | 9.0 | 9.0 | 8.0 |
| | 0.1250 | 7.6 | 8.0 | 0.0 | 9.0 | 2.0 | 7.0 |
| | 0.0620 | 5.7 | 6.0 | 0.0 | 9.0 | 0.0 | 3.0 |
| | 0.0320 | 0.0 | 2.0 | 0.0 | 9.0 | 0.0 | 3.0 |
| | | | | | | | |
| 281 | 0.5000 | 7.0 | 9.0 | 9.0 | 9.0 | 9.0 | 4.0 |
| | 0.2500 | 9.0 | 9.0 | 9.0 | 9.0 | 8.0 | 6.0 |
| | 0.1250 | 7.1 | 9.0 | 9.0 | 9.0 | 2.0 | 4.0 |
| | 0.0620 | 5.9 | 8.0 | 9.0 | 9.0 | 0.0 | 2.0 |
| | 0.0320 | 0.0 | 4.0 | 9.0 | 9.0 | 0.0 | 0.0 |
| | | | | | | | |
| 282 | 0.5000 | 0.0 | 9.0 | 0.0 | 9.0 | 0.0 | 0.0 |
| | 0.2500 | 0.0 | 8.0 | 0.0 | 9.0 | 0.0 | 4.0 |
| | 0.1250 | 1.9 | 8.0 | 0.0 | 9.0 | 0.0 | 4.0 |
| | 0.0620 | 0.0 | 8.0 | 0.0 | 9.0 | 0.0 | 3.0 |
| | 0.0320 | 0.0 | 4.0 | 0.0 | 6.0 | 0.0 | 2.0 |
| | | | | | | | |
| 283 | 0.5000 | 6.0 | 9.0 | 0.0 | 9.0 | 9.0 | 4.0 |
| | 0.2500 | 6.0 | 9.0 | | 9.0 | | 5.0 |
| | 0.1250 | 3.4 | 8.0 | | 9.0 | 0.0 | 4.0 |
| | 0.0620 | 3.4 | 4.0 | | 8.0 | 0.0 | 2.0 |
| | 0.0320 | 0.0 | 0.0 | | 2.0 | 0.0 | 2.0 |
| | | | | | | | |
| 284 | 0.2500 | 0.0 | 9.0 | 9.0 | 9.0 | 0.0 | 0.0 |
| | 0.1250 | 0.0 | 9.0 | 2.0 | 9.0 | 0.0 | 0.0 |
| | 0.0620 | 0.0 | 9.0 | 0.0 | 9.0 | 0.0 | 0.0 |
| | 0.0320 | 0.0 | 4.0 | 0.0 | 4.0 | 0.0 | 0.0 |
| | | | | | | | |
| 285 | 0.2500 | 7.0 | 9.0 | 0.0 | 9.0 | 3.0 | 7.0 |
| | 0.1250 | 7.0 | 9.0 | 0.0 | 9.0 | 0.0 | 7.0 |
| | 0.0620 | 7.0 | 9.0 | 0.0 | 9.0 | 0.0 | 7.0 |
| | 0.0320 | 3.0 | 5.0 | 0.0 | 4.0 | 0.0 | 3.0 |
| | | | | | | | |
| 286 | 0.2500 | 8.0 | 9.0 | 0.0 | 9.0 | 0.0 | 2.0 |
| | 0.1250 | 3.0 | 7.0 | 0.0 | 9.0 | 0.0 | 1.0 |
| | 0.0620 | 0.0 | 7.0 | 0.0 | 9.0 | 0.0 | 0.0 |
| | 0.0320 | 0.0 | 3.0 | 0.0 | 9.0 | 0.0 | 0.0 |
| | | | | | | | |
| 287 | 0.2500 | 7.0 | 9.0 | 4.0 | 9.0 | 0.0 | 5.0 |
| | 0.1250 | 5.0 | 9.0 | 0.0 | 9.0 | 0.0 | 3.0 |
| | 0.0620 | 2.0 | 9.0 | 0.0 | 9.0 | 0.0 | 3.0 |
| | 0.0320 | 0.0 | 0.0 | 0.0 | 9.0 | 0.0 | 0.0 |
| | | | | | | | |
| 288 | 0.2500 | 5.0 | 6.0 | 0.0 | 6.0 | 0.0 | 2.0 |
| | 0.1250 | 4.0 | 4.0 | 0.0 | 3.0 | 0.0 | 1.0 |
| | 0.0620 | 0.0 | 0.0 | 0.0 | 0.0 | 0.0 | 0.0 |
| | 0.0320 | 0.0 | 0.0 | 0.0 | 0.0 | 0.0 | 0.0 |
| | | | | | | | |
| 289 | 0.2500 | 4.0 | 9.0 | 0.0 | 9.0 | 0.0 | 0.0 |
| | 0.1250 | 3.0 | 9.0 | 0.0 | 9.0 | 0.0 | 0.0 |
| | 0.0620 | 2.0 | 0.0 | 0.0 | 0.0 | 0.0 | 0.0 |
| | 0.0320 | 0.0 | 0.0 | 0.0 | 0.0 | 0.0 | 0.0 |
| | | | | | | | |
| 290 | 0.2500 | 7.0 | 6.0 | 0.0 | 7.0 | 0.0 | 0.0 |
| | 0.1250 | 3.0 | 6.0 | 0.0 | 7.0 | 0.0 | 0.0 |
| | 0.0620 | 0.0 | 6.0 | 0.0 | 7.0 | 0.0 | 0.0 |
| | 0.0320 | 0.0 | 6.0 | 0.0 | 0.0 | 0.0 | 0.0 |
| | | | | | | | |
| 291 | 0.2500 | 0.0 | 9.0 | | 9.0 | 0.0 | 0.0 |
| | 0.1250 | 0.0 | 9.0 | | 9.0 | 0.0 | 0.0 |
| | 0.0620 | 0.0 | 0.0 | | 9.0 | 0.0 | 0.0 |
| | 0.0320 | 0.0 | 0.0 | | 0.0 | 0.0 | 0.0 |
| | | | | | | | |
| 292 | 0.2500 | 0.0 | 9.0 | | 9.0 | 5.0 | 0.0 |
| | 0.1250 | 0.0 | 9.0 | | 9.0 | 0.0 | 0.0 |
| | 0.0620 | 0.0 | 9.0 | | 9.0 | 0.0 | 0.0 |
| | 0.0320 | 0.0 | 9.0 | | 9.0 | 0.0 | 0.0 |
| | | | | | | | |
| 293 | 0.2500 | 0.0 | 9.0 | | 9.0 | 6.0 | 0.0 |
| | 0.1250 | 0.0 | 9.0 | | 9.0 | 0.0 | 0.0 |
| | 0.0620 | 0.0 | 9.0 | | 9.0 | 0.0 | 0.0 |
| | 0.0320 | 0.0 | 9.0 | | 9.0 | 0.0 | 0.0 |
| | | | | | | | |
| 294 | 0.2500 | 0.0 | 9.0 | | 9.0 | 0.0 | 0.0 |
| | 0.1250 | 0.0 | 3.0 | | 9.0 | 0.0 | 0.0 |
| | 0.0620 | 0.0 | 0.0 | | 9.0 | 0.0 | 0.0 |
| | 0.0320 | 0.0 | 0.0 | | 9.0 | 0.0 | 0.0 |
| | | | | | | | |
| 295 | 0.2500 | 0.0 | 9.0 | | 9.0 | 6.0 | 0.0 |
| | 0.1250 | 0.0 | 9.0 | | 9.0 | 0.0 | 0.0 |
| | 0.0620 | 0.0 | 0.0 | | 0.0 | 0.0 | 0.0 |
| | | | | | | | |
| 296 | 0.2500 | 4.0 | 7.0 | | 9.0 | 7.0 | 2.0 |
| | 0.1250 | 2.0 | 7.0 | | 9.0 | 5.0 | 0.0 |
| | 0.0620 | 0.0 | 7.0 | | 9.0 | 0.0 | 0.0 |
| | 0.0320 | 0.0 | 6.0 | | 9.0 | 0.0 | 0.0 |
| | | | | | | | |
| 297 | 0.2500 | 4.0 | 9.0 | | 9.0 | 9.0 | 2.0 |
| | 0.1250 | 3.0 | 7.0 | | 9.0 | 3.0 | 0.0 |
| | 0.0620 | 0.0 | 5.0 | | 9.0 | 0.0 | 0.0 |
| | 0.0320 | 0.0 | 4.0 | | 9.0 | 0.0 | 0.0 |
| | | | | | | | |
| 298 | 0.2500 | 3.0 | 9.0 | | 9.0 | 9.0 | 0.0 |
| | 0.1250 | 0.0 | 9.0 | | 9.0 | 3.0 | 0.0 |
| | 0.0620 | 0.0 | 9.0 | | 9.0 | 0.0 | 0.0 |
| | 0.0320 | 0.0 | 3.0 | | 9.0 | 0.0 | 0.0 |
| | | | | | | | |
| 299 | 0.2500 | 5.0 | 9.0 | | 9.0 | 7.0 | 5.0 |
| | 0.1250 | 2.0 | 9.0 | | 9.0 | 3.0 | 3.0 |
| | 0.0620 | 0.0 | 7.0 | | 9.0 | 0.0 | 0.0 |
| | 0.0320 | 0.0 | 3.0 | | 7.0 | 0.0 | 0.0 |
| | | | | | | | |
| 300 | 0.2500 | 9.0 | 9.0 | | 9.0 | 7.0 | 5.0 |
| | 0.1250 | 7.0 | 9.0 | | 9.0 | 6.0 | 4.0 |
| | 0.0620 | 6.0 | 6.0 | | 9.0 | 5.0 | 4.0 |
| | 0.0320 | 3.0 | 3.0 | | 9.0 | 2.0 | 2.0 |
| | | | | | | | |
| 301 | 0.2500 | 9.0 | 9.0 | 9.0 | 9.0 | 8.5 | 3.5 |
| | 0.1250 | 9.0 | 9.0 | 9.0 | 9.0 | 7.0 | 2.5 |
| | 0.0620 | 9.0 | 9.0 | 9.0 | 9.0 | 4.5 | 1.0 |
| | 0.0320 | 9.0 | 9.0 | 9.0 | 9.0 | 2.0 | 1.0 |
| | | | | | | | |
| 302 | 0.2500 | 8.5 | 8.5 | 6.0 | 9.0 | 9.0 | 8.0 |
| | 0.1250 | 8.0 | 8.5 | 4.5 | 9.0 | 9.0 | 7.0 |
| | 0.0620 | 5.0 | 8.0 | 4.5 | 7.0 | 0.0 | 4.5 |
| | 0.0320 | 4.0 | 8.0 | 4.5 | 7.0 | 0.0 | 1.0 |
| | | | | | | | |
| 303 | 0.2500 | 9.0 | 8.5 | 6.0 | 9.0 | 9.0 | 7.5 |
| | 0.1250 | 9.0 | 8.5 | 5.0 | 9.0 | 8.5 | 5.0 |
| | 0.0620 | 7.5 | 8.0 | 4.0 | 9.0 | 2.0 | 4.5 |
| | 0.0320 | 2.5 | 8.0 | 0.0 | 7.0 | 2.0 | 2.0 |
| | | | | | | | |
| 304 | 0.2500 | 0.0 | 9.0 | 0.0 | 9.0 | 0.0 | 4.0 |
| | 0.1250 | 0.0 | 9.0 | 0.0 | 7.0 | 0.0 | 4.0 |
| | 0.0620 | 0.0 | 4.0 | 0.0 | 7.0 | 0.0 | 2.0 |
| | 0.0320 | 0.0 | 0.0 | 0.0 | 0.0 | 0.0 | 0.0 |
| | | | | | | | |
| 305 | 0.2500 | 0.0 | 0.0 | 0.0 | 0.0 | 0.0 | 0.0 |
| | | | | | | | |
| 306 | 0.2500 | 0.0 | 0.0 | 0.0 | 0.0 | 0.0 | 0.0 |
| | | | | | | | |
| 307 | 0.2500 | 9.0 | 7.5 | 9.0 | 9.0 | 9.0 | 9.0 |
| | 0.1250 | 8.5 | 6.5 | 7.0 | 9.0 | 6.5 | 8.5 |
| | 0.0620 | 5.0 | 6.5 | 3.5 | 8.0 | 1.0 | 6.0 |
| | 0.0320 | 6.0 | 6.5 | 0.0 | 6.0 | 0.0 | 5.0 |
| | | | | | | | |
| 308 | 0.2500 | 9.0 | 0.0 | 9.0 | 0.0 | 9.0 | 7.0 |
| | 0.1250 | 6.0 | 0.0 | 3.0 | 0.0 | 3.0 | 6.0 |
| | 0.0620 | 5.0 | 0.0 | 0.0 | 0.0 | 0.0 | 5.0 |
| | 0.0320 | 5.0 | 0.0 | 0.0 | 0.0 | 0.0 | 5.0 |
| | | | | | | | |
| 309 | 0.2500 | 7.0 | 9.0 | 7.0 | 9.0 | 8.0 | 7.0 |
| | 0.1250 | 4.0 | 9.0 | 0.0 | 9.0 | 4.0 | 5.0 |
| | 0.0620 | 2.0 | 9.0 | 0.0 | 6.0 | 3.0 | 4.0 |
| | 0.0320 | 1.0 | 9.0 | 0.0 | 6.0 | 2.0 | 3.0 |
| | | | | | | | |
| 310 | 0.2500 | 3.0 | 9.0 | 0.0 | 9.0 | 0.0 | 3.0 |
| | 0.1250 | 2.0 | 9.0 | 0.0 | 9.0 | 0.0 | 2.0 |
| | 0.0620 | 0.0 | 9.0 | 0.0 | 9.0 | 0.0 | 0.0 |
| | 0.0320 | 0.0 | 9.0 | 0.0 | 9.0 | 0.0 | 0.0 |
| | | | | | | | |
| 311 | 0.2500 | 0.0 | 9.0 | 0.0 | 9.0 | 0.0 | 2.0 |
| | 0.1250 | 0.0 | 9.0 | 0.0 | 9.0 | 0.0 | 2.0 |
| | 0.0620 | 0.0 | 9.0 | 0.0 | 9.0 | 0.0 | 0.0 |
| | 0.0320 | 0.0 | 0.0 | 0.0 | 0.0 | 0.0 | 0.0 |
| | | | | | | | |
| 312 | 0.2500 | 9.0 | 9.0 | 6.0 | 9.0 | 6.5 | 7.5 |
| | 0.1250 | 9.0 | 9.0 | 4.5 | 9.0 | 5.5 | 7.0 |
| | 0.0620 | 6.5 | 6.5 | 3.5 | 6.0 | 2.0 | 6.5 |
| | 0.0320 | 6.0 | 4.5 | 0.0 | 4.5 | 0.0 | 5.0 |
| | | | | | | | |
| 313 | 0.2500 | 9.0 | 9.0 | 2.0 | 9.0 | 3.0 | 4.0 |
| | 0.1250 | 6.0 | 9.0 | 0.0 | 9.0 | 0.0 | 3.0 |
| | 0.0620 | 5.0 | 0.0 | 0.0 | 9.0 | 0.0 | 1.0 |
| | 0.0320 | 4.0 | 0.0 | 0.0 | 0.0 | 0.0 | 0.0 |
| | | | | | | | |
| 314 | 0.2500 | 9.0 | 9.0 | 0.0 | 9.0 | 0.0 | 5.0 |
| | 0.1250 | 9.0 | 9.0 | 0.0 | 9.0 | 0.0 | 4.0 |
| | 0.0620 | 5.0 | 9.0 | 0.0 | 9.0 | 0.0 | 3.0 |
| | 0.0320 | 5.0 | 9.0 | 0.0 | 9.0 | 0.0 | 3.0 |
| | | | | | | | |
| 315 | 0.2500 | 3.0 | 9.0 | 3.0 | 9.0 | 4.0 | 2.0 |
| | 0.1250 | 0.0 | 5.0 | 3.0 | 9.0 | 3.0 | 0.0 |
| | 0.0620 | 0.0 | 0.0 | 0.0 | 9.0 | 0.0 | 0.0 |
| | 0.0320 | 0.0 | 0.0 | 0.0 | 9.0 | 0.0 | 0.0 |
| | | | | | | | |
| 316 | 0.2500 | 9.0 | 6.0 | 0.0 | 9.0 | 3.0 | 4.0 |
| | 0.1250 | 9.0 | 7.5 | 4.5 | 9.0 | 1.0 | 4.5 |
| | 0.0620 | 9.0 | 7.5 | 4.5 | 9.0 | 3.0 | 4.0 |
| | 0.0320 | 5.5 | 8.5 | 1.0 | 8.0 | 0.0 | 4.5 |
| | | | | | | | |
| 318 | 0.2500 | 5.0 | 6.0 | 9.0 | 9.0 | 4.0 | 9.0 |
| | 0.1250 | 5.0 | 5.0 | 0.0 | 9.0 | 0.0 | 5.0 |
| | 0.0620 | 3.0 | 2.0 | 0.0 | 6.0 | 0.0 | 5.0 |
| | 0.0320 | 0.0 | 0.0 | 0.0 | 4.0 | 0.0 | 5.0 |
| | | | | | | | |
| 319 | 0.2500 | 9.0 | 9.0 | 6.0 | 9.0 | 9.0 | 7.0 |
| | 0.1250 | 9.0 | 9.0 | 6.0 | 9.0 | 9.0 | 6.0 |
| | 0.0620 | 7.0 | 9.0 | 6.0 | 9.0 | 6.0 | 6.0 |
| | 0.0320 | 3.0 | 3.0 | 4.0 | 9.0 | 6.0 | 5.0 |
| | | | | | | | |
| 320 | 0.2500 | 2.5 | 8.0 | | 9.0 | 5.0 | 2.5 |
| | 0.1250 | 0.0 | 5.5 | | 3.0 | 2.5 | 2.0 |
| | 0.0620 | 0.0 | 2.0 | | 0.0 | 2.5 | 2.0 |
| | 0.0320 | 0.0 | 0.0 | | 0.0 | 1.0 | 1.5 |
| | | | | | | | |
| 321 | 0.2500 | 8.0 | 9.0 | 9.0 | 9.0 | 8.5 | 9.0 |
| | 0.1250 | 6.5 | 8.5 | 9.0 | 9.0 | 8.5 | 9.0 |
| | 0.0620 | 5.0 | 7.5 | 1.0 | 9.0 | 6.5 | 8.0 |
| | 0.0320 | 4.0 | 4.0 | 0.0 | 8.0 | 5.5 | 8.0 |
| | | | | | | | |
| 314 | 0.2500 | 9.0 | 9.0 | 0.0 | 9.0 | 0.0 | 5.0 |
| | 0.1250 | 9.0 | 9.0 | 0.0 | 9.0 | 0.0 | 4.0 |
| | 0.0620 | 5.0 | 9.0 | 0.0 | 9.0 | 0.0 | 3.0 |
| | 0.0320 | 5.0 | 9.0 | 0.0 | 9.0 | 0.0 | 3.0 |
| | | | | | | | |
| 315 | 0.2500 | 3.0 | 9.0 | 3.0 | 9.0 | 4.0 | 2.0 |
| | 0.1250 | 0.0 | 5.0 | 3.0 | 9.0 | 3.0 | 0.0 |
| | 0.0620 | 0.0 | 0.0 | 0.0 | 9.0 | 0.0 | 0.0 |
| | 0.0320 | 0.0 | 0.0 | 0.0 | 9.0 | 0.0 | 0.0 |
| | | | | | | | |
| 316 | 0.2500 | 9.0 | 6.0 | 0.0 | 9.0 | 3.0 | 4.0 |
| | 0.1250 | 9.0 | 7.5 | 4.5 | 9.0 | 1.0 | 4.5 |
| | 0.0620 | 9.0 | 7.5 | 4.5 | 9.0 | 3.0 | 4.0 |
| | 0.0320 | 5.5 | 8.5 | 1.0 | 8.0 | 0.0 | 4.5 |
| | | | | | | | |
| 318 | 0.2500 | 5.0 | 6.0 | 9.0 | 9.0 | 4.0 | 9.0 |
| | 0.1250 | 5.0 | 5.0 | 0.0 | 9.0 | 0.0 | 5.0 |
| | 0.0620 | 3.0 | 2.0 | 0.0 | 6.0 | 0.0 | 5.0 |
| | 0.0320 | 0.0 | 0.0 | 0.0 | 4.0 | 0.0 | 5.0 |
| | | | | | | | |
| 319 | 0.2500 | 9.0 | 9.0 | 6.0 | 9.0 | 9.0 | 7.0 |
| | 0.1250 | 9.0 | 9.0 | 6.0 | 9.0 | 9.0 | 6.0 |
| | 0.0620 | 7.0 | 9.0 | 6.0 | 9.0 | 6.0 | 6.0 |
| | 0.0320 | 3.0 | 3.0 | 4.0 | 9.0 | 6.0 | 5.0 |
| | | | | | | | |
| 320 | 0.2500 | 2.5 | 8.0 | | 9.0 | 5.0 | 2.5 |
| | 0.1250 | 0.0 | 5.5 | | 3.0 | 2.5 | 2.0 |
| | 0.0620 | 0.0 | 2.0 | | 0.0 | 2.5 | 2.0 |
| | 0.0320 | 0.0 | 0.0 | | 0.0 | 1.0 | 1.5 |
| | | | | | | | |
| 321 | 0.2500 | 8.0 | 9.0 | 9.0 | 9.0 | 8.5 | 9.0 |
| | 0.1250 | 6.5 | 8.5 | 9.0 | 9.0 | 8.5 | 9.0 |
| | 0.0620 | 5.0 | 7.5 | 1.0 | 9.0 | 6.5 | 8.0 |
| | 0.0320 | 4.0 | 4.0 | 0.0 | 8.0 | 5.5 | 8.0 |
| | | | | | | | |
| 322 | 0.2500 | 9.0 | 9.0 | 9.0 | 9.0 | 9.0 | 7.0 |
| | 0.1250 | 9.0 | 7.0 | 6.0 | 9.0 | 3.0 | 5.0 |
| | 0.0620 | 9.0 | 6.0 | 5.0 | 9.0 | 3.0 | 5.0 |
| | 0.0320 | 9.0 | 4.0 | 5.0 | 5.0 | 3.0 | 5.0 |
| | | | | | | | |
| 323 | 0.2500 | 8.0 | 8.0 | 7.5 | 9.0 | 9.0 | 7.5 |
| | 0.1250 | 8.0 | 8.0 | 6.5 | 9.0 | 3.5 | 6.0 |
| | 0.0620 | 6.5 | 4.5 | 6.0 | 9.0 | 3.5 | 5.0 |
| | 0.0320 | 4.5 | 3.5 | 5.0 | 8.0 | 0.0 | 3.5 |
| | | | | | | | |
| 324 | 0.2500 | 6.0 | 9.0 | 7.0 | 9.0 | 9.0 | 9.0 |
| | 0.1250 | 5.0 | 9.0 | 3.0 | 9.0 | 9.0 | 7.0 |
| | 0.0620 | 4.0 | 9.0 | 0.0 | 9.0 | 3.0 | 4.0 |
| | 0.0320 | 0.0 | 9.0 | 0.0 | 9.0 | 0.0 | 3.0 |
| | | | | | | | |
| 325 | 0.2500 | 9.0 | 9.0 | 9.0 | 9.0 | 9.0 | 4.0 |
| | 0.1250 | 9.0 | 9.0 | 7.3 | 9.0 | 6.3 | 3.7 |
| | 0.0620 | 9.0 | 8.7 | 9.0 | 9.0 | 4.3 | 3.0 |
| | 0.0320 | 7.7 | 8.0 | 4.7 | 8.0 | 4.0 | 2.7 |
| | | | | | | | |
| 326 | 0.2500 | 9.0 | 9.0 | 7.5 | 9.0 | 9.0 | 5.5 |
| | 0.1250 | 9.0 | 7.0 | 4.5 | 9.0 | 5.5 | 5.5 |
| | 0.0620 | 8.5 | 7.0 | 4.5 | 9.0 | 1.0 | 5.5 |
| | 0.0320 | 5.5 | 6.0 | 4.0 | 9.0 | 0.0 | 5.5 |
| | | | | | | | |
| 327 | 0.2500 | 9.0 | 9.0 | 9.0 | 9.0 | 7.5 | 4.0 |
| | 0.1250 | 9.0 | 9.0 | 5.5 | 9.0 | 5.5 | 3.5 |
| | 0.0620 | 9.0 | 9.0 | 5.5 | 9.0 | 5.5 | 2.5 |
| | 0.0320 | 9.0 | 9.0 | 1.0 | 9.0 | 3.0 | 3.0 |
| | | | | | | | |
| 328 | 0.2500 | 9.0 | 9.0 | 9.0 | 9.0 | 4.5 | 4.5 |
| | 0.1250 | 9.0 | 9.0 | 7.5 | 9.0 | 4.5 | 4.0 |
| | 0.0620 | 9.0 | 9.0 | 6.0 | 9.0 | 3.0 | 3.0 |
| | 0.0320 | 9.0 | 9.0 | 6.0 | 9.0 | 2.5 | 2.0 |
| | | | | | | | |
| 329 | 0.2500 | 3.0 | 0.0 | 0.0 | 9.0 | 4.0 | 3.0 |
| | 0.1250 | 3.0 | 0.0 | 0.0 | 9.0 | 4.0 | 3.0 |
| | 0.0620 | 3.0 | 0.0 | 0.0 | 9.0 | 4.0 | 3.0 |
| | 0.0320 | 2.0 | 0.0 | 0.0 | 9.0 | 3.0 | 3.0 |
| | | | | | | | |
| 330 | 0.2500 | 9.0 | 6.0 | 5.0 | 9.0 | 0.0 | 7.0 |
| | 0.1250 | 9.0 | 6.0 | 5.0 | 9.0 | 0.0 | 7.0 |
| | 0.0620 | 7.0 | 0.0 | 5.0 | 9.0 | 0.0 | 7.0 |
| | 0.0320 | 7.0 | 0.0 | 5.0 | 9.0 | 0.0 | 7.0 |
| | | | | | | | |
| 331 | 0.2500 | 8.0 | 9.0 | 0.0 | 9.0 | 0.0 | 1.0 |
| | 0.1250 | 4.0 | 5.0 | 0.0 | 9.0 | 0.0 | 0.0 |
| | 0.0620 | 2.0 | 0.0 | 0.0 | 9.0 | 0.0 | 0.0 |
| | 0.0320 | 0.0 | 0.0 | 0.0 | 9.0 | 0.0 | 0.0 |
| | | | | | | | |
| 332 | 0.2500 | 9.0 | 9.0 | 7.5 | 9.0 | 9.0 | 7.5 |
| | 0.1250 | 9.0 | 9.0 | 4.5 | 9.0 | 6.5 | 5.5 |
| | 0.0620 | 8.5 | 9.0 | 4.5 | 9.0 | 3.5 | 5.5 |
| | 0.0320 | 6.5 | 9.0 | 4.0 | 9.0 | 3.0 | 5.5 |
| | | | | | | | |
| 333 | 0.2500 | 9.0 | 9.0 | 7.5 | 9.0 | 9.0 | 7.0 |
| | 0.1250 | 9.0 | 9.0 | 7.5 | 9.0 | 8.5 | 7.0 |
| | 0.0620 | 9.0 | 9.0 | 7.0 | 9.0 | 8.0 | 6.0 |
| | 0.0320 | 9.0 | 9.0 | 7.0 | 9.0 | 5.5 | 4.0 |
| | | | | | | | |
| 334 | 0.2500 | 3.0 | 6.0 | 2.0 | | 4.0 | 3.0 |
| | 0.1250 | 0.0 | 6.0 | 2.0 | | 4.0 | 3.0 |
| | 0.0620 | 0.0 | 5.0 | 0.0 | | 0.0 | 0.0 |
| | 0.0320 | 0.0 | 3.0 | 0.0 | | 0.0 | 0.0 |
| | | | | | | | |
| 335 | 0.2500 | 9.0 | 2.0 | 6.0 | | 4.0 | 7.0 |
| | 0.1250 | 7.0 | 2.0 | 6.0 | | 4.0 | 5.0 |
| | 0.0620 | 7.0 | 0.0 | 0.0 | | 0.0 | 4.0 |
| | 0.0320 | 6.0 | 0.0 | 0.0 | | 0.0 | 3.0 |
| | | | | | | | |
| 336 | 0.2500 | 9.0 | 9.0 | 3.0 | 9.0 | 9.0 | 9.0 |
| | 0.1250 | 9.0 | 4.0 | 2.0 | 9.0 | 1.0 | 4.5 |
| | 0.0620 | 9.0 | 1.0 | 0.0 | 9.0 | 0.0 | 4.5 |
| | 0.0320 | 6.5 | 0.0 | 0.0 | 9.0 | 0.0 | 2.0 |
| | | | | | | | |
| 337 | 0.2500 | 6.0 | 9.0 | 9.0 | | 3.0 | 3.0 |
| | 0.1250 | 6.0 | 6.0 | 0.0 | | 0.0 | 3.0 |
| | 0.0620 | 2.0 | 0.0 | 0.0 | | 0.0 | 3.0 |
| | 0.0320 | 2.0 | 0.0 | 0.0 | | 0.0 | 3.0 |
| | | | | | | | |
| 338 | 0.2500 | 9.0 | 8.0 | 5.0 | | 5.0 | 6.0 |
| | 0.1250 | 4.0 | 3.0 | 0.0 | | 2.0 | 3.0 |
| | 0.0620 | 2.0 | 3.0 | 0.0 | | 2.0 | 0.0 |
| | 0.0320 | 2.0 | 3.0 | 0.0 | | 0.0 | 0.0 |
| | | | | | | | |
| 339 | 0.2500 | 6.0 | 7.0 | 9.0 | | 9.0 | 9.0 |
| | 0.1250 | 4.0 | 7.0 | 6.0 | | 5.0 | 9.0 |
| | 0.0620 | 0.0 | 4.0 | 0.0 | | 5.0 | 7.0 |
| | 0.0320 | 0.0 | 4.0 | 0.0 | | 5.0 | 5.0 |
| | | | | | | | |
| 340 | 0.2500 | 9.0 | 9.0 | 7.0 | | 7.0 | 9.0 |
| | 0.1250 | 9.0 | 7.0 | 6.0 | | 5.0 | 7.0 |
| | 0.0620 | 6.0 | 5.0 | 3.0 | | 4.0 | 6.0 |
| | 0.0320 | 5.0 | 2.0 | 3.0 | | 4.0 | 5.0 |
| | | | | | | | |
| 341 | 0.2500 | 8.5 | 4.0 | 3.5 | 9.0 | 4.5 | 3.5 |
| | 0.1250 | 8.0 | 3.5 | 2.5 | 9.0 | 4.5 | 3.0 |
| | 0.0620 | 5.5 | 2.0 | 0.0 | 9.0 | 4.5 | 2.5 |
| | 0.0320 | 3.0 | 1.0 | 0.0 | 7.0 | 1.0 | 1.5 |
| | | | | | | | |
| 342 | 0.2500 | 7.0 | 8.0 | 9.0 | | 2.0 | 3.0 |
| | 0.1250 | 7.0 | 4.0 | 0.0 | | 0.0 | 2.0 |
| | 0.0620 | 4.0 | 0.0 | 0.0 | | 0.0 | 0.0 |
| | 0.0320 | 4.0 | 0.0 | 0.0 | | 0.0 | 0.0 |
| | | | | | | | |
| 343 | 0.2500 | 9.0 | 6.0 | 6.0 | | 3.0 | 4.0 |
| | 0.1250 | 7.0 | 0.0 | 4.0 | | 0.0 | 3.0 |
| | 0.0620 | 6.0 | 0.0 | 3.0 | | 0.0 | 2.0 |
| | 0.0320 | 3.0 | 0.0 | 0.0 | | 0.0 | 0.0 |
| | | | | | | | |
| 344 | 0.2500 | 9.0 | 9.0 | 6.0 | | 6.0 | 9.0 |
| | 0.1250 | 7.0 | 7.0 | 6.0 | | 3.0 | 5.0 |
| | 0.0620 | 7.0 | 6.0 | 6.0 | | 0.0 | 5.0 |
| | 0.0320 | 4.0 | 6.0 | 6.0 | | 0.0 | 2.0 |
| | | | | | | | |
| 345 | 0.2500 | 9.0 | 9.0 | 9.0 | 9.0 | 9.0 | 6.0 |
| | 0.1250 | 9.0 | 9.0 | 4.5 | 9.0 | 9.0 | 5.5 |
| | 0.0620 | 9.0 | 9.0 | 9.0 | 9.0 | 9.0 | 5.5 |
| | 0.0320 | 9.0 | 9.0 | 5.0 | 9.0 | 4.5 | 5.5 |
| | | | | | | | |
| 347 | 0.2500 | 9.0 | 9.0 | 7.0 | | 5.0 | 9.0 |
| | 0.1250 | 9.0 | 9.0 | 4.0 | | 4.0 | 7.0 |
| | 0.0620 | 9.0 | 9.0 | 0.0 | | 3.0 | 6.0 |
| | 0.0320 | 7.0 | 7.0 | 0.0 | | 2.0 | 3.0 |
| | | | | | | | |
| 348 | 0.2500 | 9.0 | 9.0 | 0.0 | | 9.0 | 5.0 |
| | 0.1250 | 7.0 | 6.0 | 0.0 | | 7.0 | 4.0 |
| | 0.0620 | 6.0 | 0.0 | 0.0 | | 6.0 | 3.0 |
| | 0.0320 | 6.0 | 0.0 | 0.0 | | 4.0 | 2.0 |
| | | | | | | | |
| 349 | 0.2500 | 7.0 | 7.0 | 4.0 | | 6.0 | 7.0 |
| | 0.1250 | 6.0 | 7.0 | 4.0 | | 5.0 | 7.0 |
| | 0.0620 | 5.0 | 4.0 | 4.0 | | 5.0 | 5.0 |
| | 0.0320 | 5.0 | 0.0 | 4.0 | | 4.0 | 4.0 |
| | | | | | | | |
| 350 | 0.2500 | 9.0 | 9.0 | 9.0 | 9.0 | 9.0 | 9.0 |
| | 0.1250 | 8.0 | 9.0 | 8.5 | 9.0 | 7.5 | 9.0 |
| | 0.0620 | 7.5 | 5.0 | 8.5 | 9.0 | 7.0 | 9.0 |
| | 0.0320 | 6.0 | 4.0 | 7.5 | 9.0 | 5.5 | 9.0 |
| | | | | | | | |
| 351 | 0.2500 | 7.0 | 9.0 | 8.0 | | 7.0 | 9.0 |
| | 0.1250 | 6.0 | 7.0 | 6.0 | | 7.0 | 7.0 |
| | 0.0620 | 6.0 | 7.0 | 6.0 | | 6.0 | 7.0 |
| | 0.0320 | 5.0 | 6.0 | 0.0 | | 5.0 | 7.0 |
| | | | | | | | |
| 352 | 0.2500 | 9.0 | 9.0 | 0.0 | 9.0 | 9.0 | 3.0 |
| | 0.1250 | 6.5 | 7.5 | 1.0 | 9.0 | 9.0 | 2.5 |
| | 0.0620 | 4.0 | 6.5 | 0.0 | 9.0 | 5.0 | 1.0 |
| | 0.0320 | 2.5 | 2.0 | 0.0 | 6.0 | 2.5 | 0.5 |
| | | | | | | | |
| 353 | 0.2500 | 3.0 | 3.0 | 2.5 | 8.0 | 0.0 | 4.0 |
| | 0.1250 | 2.5 | 1.0 | 1.5 | 8.0 | 0.0 | 3.5 |
| | 0.0620 | 2.0 | 0.0 | 0.0 | 6.5 | 0.0 | 3.0 |
| | 0.0320 | 0.0 | 0.0 | 0.0 | 3.5 | 0.0 | 2.5 |
| | | | | | | | |
| 354 | 0.2500 | 9.0 | 9.0 | 9.0 | 9.0 | 9.0 | 9.0 |
| | 0.1250 | 9.0 | 9.0 | 9.0 | 9.0 | 9.0 | 9.0 |
| | 0.0620 | 9.0 | 9.0 | 9.0 | 9.0 | 9.0 | 8.0 |
| | 0.0320 | 9.0 | 8.3 | 8.0 | 9.0 | 6.3 | 7.3 |
| | | | | | | | |
| 355 | 0.2500 | 9.0 | 5.0 | 5.0 | 9.0 | 9.0 | 5.0 |
| | 0.1250 | 9.0 | 6.5 | 3.5 | 9.0 | 4.5 | 5.5 |
| | 0.0620 | 9.0 | 4.5 | 1.0 | 9.0 | 4.5 | 3.0 |
| | 0.0320 | 7.0 | 4.5 | 0.0 | 9.0 | 2.0 | 2.5 |
| | | | | | | | |
| 356 | 0.2500 | 9.0 | 9.0 | 6.5 | 9.0 | 8.0 | 2.0 |
| | 0.1250 | 6.5 | 9.0 | 5.0 | 9.0 | 5.5 | 4.5 |
| | 0.0620 | 5.0 | 9.0 | 0.0 | 9.0 | 4.5 | 2.5 |
| | 0.0320 | 3.5 | 6.5 | 0.0 | 7.5 | 4.5 | 0.5 |
| | | | | | | | |
| 357 | 0.2500 | 7.0 | 6.0 | 0.0 | 9.0 | 4.0 | 3.0 |
| | 0.1250 | 5.0 | 2.0 | 0.0 | 9.0 | 0.0 | 2.0 |
| | 0.0620 | 5.0 | 0.0 | 0.0 | 9.0 | 0.0 | 2.0 |
| | 0.0320 | 4.0 | 0.0 | 0.0 | 9.0 | 0.0 | 2.0 |
| | | | | | | | |
| 358 | 0.2500 | 5.0 | 7.0 | 0.0 | 9.0 | 6.0 | 3.0 |
| | 0.1250 | 5.0 | 0.0 | 0.0 | 9.0 | 6.0 | 3.0 |
| | 0.0620 | 5.0 | 0.0 | 0.0 | 9.0 | 5.0 | 3.0 |
| | 0.0320 | 4.0 | 0.0 | 0.0 | 9.0 | 5.0 | 2.0 |
| | | | | | | | |
| 359 | 0.5000 | 9.0 | 9.0 | | 9.0 | 5.0 | 9.0 |
| | 0.2500 | 9.0 | 9.0 | 9.0 | 9.0 | 4.5 | 9.0 |
| | 0.1250 | 9.0 | 8.5 | 9.0 | 9.0 | 1.8 | 8.8 |
| | 0.0620 | 9.0 | 7.5 | 6.0 | 9.0 | 0.5 | 7.3 |
| | 0.0320 | 8.5 | 6.3 | 9.0 | 8.8 | 0.0 | 6.5 |
| | | | | | | | |
| 360 | 0.5000 | 9.0 | 9.0 | | 9.0 | 9.0 | 9.0 |
| | 0.2500 | 7.5 | 9.0 | 9.0 | 9.0 | 9.0 | 9.0 |
| | 0.1250 | 5.0 | 9.0 | 0.0 | 4.5 | 4.5 | 3.5 |
| | 0.0620 | 2.0 | 2.5 | 0.0 | 4.5 | 4.5 | 2.0 |
| | 0.0320 | 0.0 | 0.0 | 0.0 | 4.5 | 1.5 | 1.0 |
| | | | | | | | |
| 361 | 0.5000 | 4.0 | 6.0 | | 9.0 | 5.0 | 3.0 |
| | 0.2500 | 4.0 | 6.0 | | 9.0 | 5.0 | 5.0 |
| | 0.1250 | 0.0 | 0.0 | | 9.0 | 5.0 | 4.0 |
| | 0.0620 | 0.0 | 0.0 | | 9.0 | 4.0 | 0.0 |
| | 0.0320 | 0.0 | 0.0 | | 9.0 | 4.0 | 0.0 |
| | | | | | | | |
| 362 | 0.5000 | 9.0 | 9.0 | 9.0 | 9.0 | 9.0 | 8.0 |
| | 0.2500 | 9.0 | 7.0 | 9.0 | 9.0 | 9.0 | 6.0 |
| | 0.1250 | 7.0 | 5.0 | 9.0 | 9.0 | 9.0 | 4.0 |
| | 0.0620 | 7.0 | 5.0 | 9.0 | 9.0 | 9.0 | 4.0 |
| | 0.0320 | 4.0 | 3.0 | 6.0 | 9.0 | 9.0 | 4.0 |
| | | | | | | | |
| 363 | 0.5000 | 7.0 | 8.0 | 7.0 | 9.0 | 9.0 | 9.0 |
| | 0.2500 | 6.0 | 7.0 | 7.0 | 9.0 | 9.0 | 7.0 |
| | 0.1250 | 5.0 | 7.0 | 5.0 | 9.0 | 7.0 | 6.0 |
| | 0.0620 | 4.0 | 7.0 | 0.0 | 9.0 | 5.0 | 5.0 |
| | 0.0320 | 3.0 | 6.0 | 0.0 | 9.0 | 3.0 | 4.0 |
| | | | | | | | |
| 364 | 0.5000 | 7.0 | 9.0 | 7.0 | 9.0 | 9.0 | 9.0 |
| | 0.2500 | 6.0 | 9.0 | 6.0 | 9.0 | 9.0 | 9.0 |
| | 0.1250 | 6.0 | 9.0 | 5.0 | 9.0 | 5.0 | 6.0 |
| | 0.0620 | 5.0 | 7.0 | 3.0 | 9.0 | 4.0 | 5.0 |
| | 0.0320 | 3.0 | 7.0 | 3.0 | 9.0 | 4.0 | 4.0 |
| | | | | | | | |
| 365 | 0.5000 | 9.0 | 9.0 | 9.0 | 9.0 | 6.0 | 6.0 |
| | 0.2500 | 9.0 | 9.0 | 9.0 | 9.0 | 5.0 | 5.0 |
| | 0.1250 | 9.0 | 6.0 | 4.0 | 9.0 | 5.0 | 5.0 |
| | 0.0620 | 6.0 | 6.0 | 4.0 | 9.0 | 5.0 | 5.0 |
| | 0.0320 | 4.0 | 4.0 | 4.0 | 9.0 | 3.0 | 3.0 |
| | | | | | | | |
| 366 | 0.5000 | 9.0 | 9.0 | 9.0 | 9.0 | 9.0 | 9.0 |
| | 0.2500 | 9.0 | 9.0 | 9.0 | 9.0 | 9.0 | 9.0 |
| | 0.1250 | 9.0 | 9.0 | 9.0 | 9.0 | 9.0 | 9.0 |
| | 0.0620 | 8.5 | 9.0 | 8.0 | 9.0 | 8.0 | 9.0 |
| | 0.0320 | 8.0 | 8.5 | 8.0 | 9.0 | 7.0 | 7.5 |
| | | | | | | | |
| 367 | 0.2500 | 6.0 | 9.0 | 9.0 | 9.0 | 9.0 | 6.0 |
| | 0.1250 | 3.0 | 7.0 | 9.0 | 9.0 | 5.0 | 5.0 |
| | 0.0620 | 0.0 | 5.0 | 9.0 | 9.0 | 2.0 | 0.0 |
| | 0.0320 | 0.0 | 0.0 | 0.0 | 9.0 | 0.0 | 0.0 |
| | | | | | | | |
| 368 | 0.2500 | 8.0 | 9.0 | 8.0 | 9.0 | 9.0 | 7.0 |
| | 0.1250 | 7.0 | 9.0 | 7.0 | 9.0 | 9.0 | 6.0 |
| | 0.0620 | 7.0 | 9.0 | 4.0 | 9.0 | 6.0 | 5.0 |
| | 0.0320 | 3.0 | 7.0 | 0.0 | 9.0 | 5.0 | 5.0 |
| | | | | | | | |
| 369 | 0.2500 | 8.0 | 9.0 | 9.0 | 9.0 | 9.0 | 7.0 |
| | 0.1250 | 6.0 | 9.0 | 4.0 | 9.0 | 9.0 | 7.0 |
| | 0.0620 | 3.0 | 7.0 | 3.0 | 9.0 | 6.0 | 6.0 |
| | 0.0320 | 3.0 | 7.0 | 0.0 | 9.0 | 0.0 | 6.0 |
| | | | | | | | |
| 370 | 0.2500 | 7.0 | 9.0 | 9.0 | 9.0 | 9.0 | 5.0 |
| | 0.1250 | 6.0 | 9.0 | 7.0 | 9.0 | 7.0 | 4.0 |
| | 0.0620 | 4.0 | 7.0 | 4.0 | 9.0 | 3.0 | 4.0 |
| | 0.0320 | 3.0 | 4.0 | 0.0 | 7.0 | 0.0 | 3.0 |
| | | | | | | | |
| 371 | 0.2500 | 7.0 | 9.0 | 9.0 | 9.0 | 9.0 | 9.0 |
| | 0.1250 | 6.0 | 9.0 | 6.0 | 9.0 | 9.0 | 6.0 |
| | 0.0620 | 4.0 | 6.0 | 0.0 | 9.0 | 4.0 | 6.0 |
| | 0.0320 | 3.0 | 4.0 | 0.0 | 9.0 | 3.0 | 5.0 |
| | | | | | | | |
| 372 | 0.2500 | 9.0 | 9.0 | 9.0 | 9.0 | 9.0 | 9.0 |
| | 0.1250 | 9.0 | 9.0 | 8.5 | 9.0 | 9.0 | 8.0 |
| | 0.0620 | 9.0 | 9.0 | 5.0 | 9.0 | 9.0 | 8.0 |
| | 0.0320 | 9.0 | 8.5 | 1.0 | 9.0 | 8.5 | 6.0 |
| | | | | | | | |
| 373 | 0.2500 | 9.0 | 9.0 | 8.0 | 9.0 | 9.0 | 8.0 |
| | 0.1250 | 9.0 | 9.0 | 7.5 | 9.0 | 9.0 | 6.0 |
| | 0.0620 | 9.0 | 9.0 | 5.0 | 9.0 | 8.5 | 6.5 |
| | 0.0320 | 6.5 | 6.5 | 2.0 | 9.0 | 5.5 | 5.5 |
| | | | | | | | |
| 374 | 0.2500 | 6.0 | 9.0 | 5.0 | 9.0 | 9.0 | 7.0 |
| | 0.1250 | 4.0 | 9.0 | 0.0 | 9.0 | 6.0 | 6.0 |
| | 0.0620 | 3.0 | 9.0 | 0.0 | 9.0 | 3.0 | 4.0 |
| | 0.0320 | 3.0 | 6.0 | 0.0 | 9.0 | 2.0 | 4.0 |
| | | | | | | | |
| 375 | 0.2500 | 9.0 | 9.0 | 7.0 | 9.0 | 9.0 | 6.0 |
| | 0.1250 | 9.0 | 6.0 | 4.0 | 9.0 | 9.0 | 4.0 |
| | 0.0620 | 6.0 | 4.0 | 0.0 | 9.0 | 9.0 | 4.0 |
| | 0.0320 | 6.0 | 3.0 | 0.0 | 9.0 | 7.0 | 2.0 |
| | | | | | | | |
| 376 | 0.2500 | 9.0 | 9.0 | 8.0 | 9.0 | 9.0 | 8.5 |
| | 0.1250 | 6.5 | 9.0 | 5.5 | 9.0 | 9.0 | 6.0 |
| | 0.0620 | 5.5 | 8.0 | 4.0 | 9.0 | 8.5 | 6.0 |
| | 0.0320 | 3.0 | 3.0 | 3.0 | 9.0 | 8.0 | 4.0 |
| | | | | | | | |
| 377 | 0.2500 | 9.0 | 8.0 | 8.5 | 9.0 | 9.0 | 9.0 |
| | 0.1250 | 7.0 | 4.5 | 5.0 | 9.0 | 8.5 | 8.0 |
| | 0.0620 | 5.0 | 3.0 | 3.0 | 9.0 | 4.0 | 6.5 |
| | 0.0320 | 2.0 | 2.0 | 1.0 | 9.0 | 0.0 | 5.5 |
| | | | | | | | |
| 378 | 0.2500 | 9.0 | 9.0 | 9.0 | 9.0 | 9.0 | 9.0 |
| | 0.1250 | 9.0 | 9.0 | 9.0 | 9.0 | 9.0 | 9.0 |
| | 0.0620 | 9.0 | 9.0 | 9.0 | 9.0 | 9.0 | 9.0 |
| | 0.0320 | 9.0 | 9.0 | 9.0 | 9.0 | 9.0 | 9.0 |
| | | | | | | | |
| 379 | 0.2500 | 8.0 | 9.0 | 8.5 | 9.0 | 9.0 | 9.0 |
| | 0.1250 | 7.5 | 9.0 | 8.5 | 9.0 | 9.0 | 9.0 |
| | 0.0620 | 5.0 | 7.5 | 6.5 | 9.0 | 6.5 | 8.5 |
| | 0.0320 | 2.0 | 5.5 | 2.5 | 9.0 | 3.5 | 7.0 |
| | | | | | | | |
| 380 | 0.2500 | 9.0 | 9.0 | 8.0 | 9.0 | 9.0 | 6.5 |
| | 0.1250 | 9.0 | 9.0 | 8.0 | 9.0 | 4.0 | 5.0 |
| | 0.0620 | 9.0 | 9.0 | 3.5 | 9.0 | 0.0 | 4.0 |
| | 0.0320 | 9.0 | 9.0 | 3.5 | 9.0 | 0.0 | 2.5 |
| | | | | | | | |
| 381 | 0.2500 | 6.0 | 8.0 | 7.0 | 8.0 | 7.0 | 7.0 |
| | 0.1250 | 4.0 | 8.0 | 7.0 | 8.0 | 6.0 | 6.0 |
| | 0.0620 | 3.0 | 7.0 | 4.0 | 7.0 | 5.0 | 3.0 |
| | 0.0320 | 0.0 | 6.0 | 0.0 | 6.0 | 3.0 | 3.0 |
| | | | | | | | |
| 382 | 0.2500 | 7.0 | 9.0 | 7.0 | 9.0 | 9.0 | 5.0 |
| | 0.1250 | 4.0 | 7.0 | 7.0 | 9.0 | 7.0 | 6.0 |
| | 0.0620 | 3.0 | 6.0 | 0.0 | 9.0 | 4.0 | 6.0 |
| | 0.0320 | 0.0 | 5.0 | 0.0 | 9.0 | 3.0 | 5.0 |
| | | | | | | | |
| 383 | 0.2500 | 8.0 | 8.0 | 9.0 | 9.0 | 9.0 | 6.5 |
| | 0.1250 | 7.5 | 7.0 | 7.5 | 9.0 | 9.0 | 6.0 |
| | 0.0620 | 6.5 | 7.0 | 5.5 | 9.0 | 7.5 | 4.5 |
| | 0.0320 | 4.0 | 2.5 | 4.5 | 9.0 | 3.0 | 3.5 |
| | | | | | | | |
| 384 | 0.2500 | 6.0 | 8.0 | 7.0 | 9.0 | 6.0 | 6.0 |
| | 0.1250 | 4.0 | 8.0 | 7.0 | 9.0 | 6.0 | 7.0 |
| | 0.0620 | 4.0 | 7.0 | 7.0 | 9.0 | 6.0 | 4.0 |
| | 0.0320 | 2.0 | 4.0 | 7.0 | 9.0 | 5.0 | 4.0 |
| | | | | | | | |
| 385 | 0.2500 | 6.0 | 9.0 | 7.0 | 9.0 | 9.0 | 7.0 |
| | 0.1250 | 3.0 | 6.0 | 7.0 | 9.0 | 9.0 | 7.0 |
| | 0.0620 | 3.0 | 6.0 | 6.0 | 9.0 | 0.0 | 5.0 |
| | 0.0320 | 0.0 | 4.0 | 6.0 | 9.0 | 0.0 | 3.0 |
| | | | | | | | |
| 386 | 0.2500 | 5.0 | 9.0 | 7.0 | 9.0 | 9.0 | 5.0 |
| | 0.1250 | 4.0 | 9.0 | 7.0 | 9.0 | 9.0 | 5.0 |
| | 0.0620 | 3.0 | 9.0 | 7.0 | 9.0 | 7.0 | 5.0 |
| | 0.0320 | 0.0 | 7.0 | 3.0 | 9.0 | 4.0 | 5.0 |
| | | | | | | | |
| 387 | 0.2500 | 3.0 | 7.0 | 5.0 | 9.0 | 6.0 | 2.0 |
| | 0.1250 | 0.0 | 6.0 | 5.0 | 9.0 | 5.0 | 2.0 |
| | 0.0620 | 0.0 | 5.0 | 5.0 | 9.0 | 4.0 | 0.0 |
| | 0.0320 | 0.0 | 0.0 | 2.0 | 4.0 | 3.0 | 0.0 |
| | | | | | | | |
| 388 | 0.2500 | 6.0 | 9.0 | 6.0 | 9.0 | 7.0 | 6.0 |
| | 0.1250 | 5.0 | 7.0 | | 9.0 | 4.0 | 4.0 |
| | 0.0620 | 2.0 | 7.0 | | 9.0 | 4.0 | 4.0 |
| | 0.0320 | 0.0 | 4.0 | 6.0 | 9.0 | 3.0 | 3.0 |
| | | | | | | | |
| 389 | 0.2500 | 7.0 | 9.0 | 8.0 | 9.0 | 9.0 | 5.0 |
| | 0.1250 | 4.0 | 7.0 | 7.0 | 9.0 | 7.0 | 4.0 |
| | 0.0620 | 4.0 | 5.0 | | 9.0 | 4.0 | 4.0 |
| | 0.0320 | 3.0 | 5.0 | 0.0 | 7.0 | 3.0 | 3.0 |
| | | | | | | | |
| 390 | 0.5000 | 9.0 | 9.0 | 6.0 | 9.0 | 9.0 | 5.0 |
| | 0.2500 | 9.0 | 9.0 | 9.0 | 9.0 | 8.0 | 6.5 |
| | 0.1250 | 8.0 | 7.0 | 4.0 | 9.0 | 8.0 | 6.0 |
| | 0.0620 | 7.0 | 4.0 | | 9.0 | 5.5 | 5.5 |
| | 0.0320 | 5.5 | 1.0 | 2.5 | 9.0 | 2.5 | 4.0 |
| | | | | | | | |
| 391 | 0.5000 | 6.0 | 9.0 | 3.0 | 9.0 | 9.0 | 3.0 |
| | 0.2500 | 4.0 | 6.0 | 0.0 | 9.0 | 7.0 | 3.0 |
| | 0.1250 | 2.0 | 4.0 | 0.0 | 9.0 | 7.0 | 3.0 |
| | 0.0620 | 0.0 | 3.0 | 0.0 | 9.0 | 6.0 | 2.0 |
| | 0.0320 | 0.0 | 3.0 | 0.0 | 6.0 | 6.0 | 2.0 |
| | | | | | | | |
| 392 | 0.2500 | 9.0 | 7.0 | 0.0 | 9.0 | 0.0 | |
| | 0.1250 | 9.0 | 3.0 | 0.0 | 7.0 | 0.0 | |
| | 0.0620 | 7.0 | 0.0 | 0.0 | 7.0 | 0.0 | |
| | 0.0320 | 5.0 | 0.0 | 0.0 | 6.0 | 0.0 | |
| | | | | | | | |
| 393 | 0.2500 | 9.0 | 6.0 | 6.0 | 9.0 | 4.0 | |
| | 0.1250 | 4.0 | 3.0 | 3.0 | 8.0 | 3.0 | |
| | 0.0620 | 4.0 | 3.0 | 0.0 | 8.0 | | |
| | 0.0320 | 2.0 | 0.0 | 0.0 | 7.0 | 0.0 | |
| | | | | | | | |
| 394 | 0.2500 | 6.0 | 7.0 | 2.0 | 9.0 | 0.0 | |
| | 0.1250 | 4.0 | 5.0 | 0.0 | 9.0 | 0.0 | |
| | 0.0620 | 0.0 | 3.0 | 0.0 | 8.0 | 0.0 | |
| | 0.0320 | 0.0 | 0.0 | 0.0 | 8.0 | | |
| | | | | | | | |
| 395 | 0.2500 | 3.0 | 3.0 | 0.0 | 9.0 | 5.0 | |
| | 0.1250 | 2.0 | 0.0 | 0.0 | 9.0 | 4.0 | |
| | 0.0620 | 0.0 | 0.0 | 0.0 | 9.0 | 2.0 | |
| | 0.0320 | 0.0 | 0.0 | 0.0 | 9.0 | 0.0 | |
| | | | | | | | |
| 396 | 0.2500 | 9.0 | 9.0 | 6.0 | 9.0 | 8.0 | |
| | 0.1250 | 9.0 | 9.0 | 0.0 | 9.0 | 8.0 | |
| | 0.0620 | 9.0 | 8.0 | 0.0 | 9.0 | 7.0 | |
| | 0.0320 | 9.0 | 7.0 | 0.0 | 9.0 | 7.0 | |
| | | | | | | | |
| 397 | 0.2500 | 9.0 | 9.0 | 0.0 | 9.0 | 5.0 | |
| | 0.1250 | 9.0 | 9.0 | 0.0 | 9.0 | 5.0 | |
| | 0.0620 | 9.0 | 7.0 | | 9.0 | 5.0 | |
| | 0.0320 | 9.0 | 6.0 | 0.0 | 9.0 | 4.0 | |
| | | | | | | | |
| 398 | 0.2500 | 8.0 | 8.0 | 3.0 | 9.0 | 3.0 | |
| | 0.1250 | 3.0 | 6.0 | 3.0 | 9.0 | 0.0 | |
| | 0.0620 | 2.0 | 3.0 | 0.0 | 9.0 | 0.0 | |
| | 0.0320 | 0.0 | 0.0 | 0.0 | 6.0 | 0.0 | |
| | | | | | | | |
| 399 | 0.2500 | 3.0 | 8.0 | 0.0 | 9.0 | 0.0 | |
| | 0.1250 | 2.0 | 7.0 | 0.0 | 8.0 | 0.0 | |
| | 0.0620 | 0.0 | 2.0 | 0.0 | 7.0 | 0.0 | |
| | 0.0320 | 0.0 | 0.0 | 0.0 | 6.0 | 0.0 | |
| | | | | | | | |
| 400 | 0.2500 | 0.0 | 9.0 | 0.0 | 9.0 | 0.0 | |
| | 0.1250 | 0.0 | 9.0 | 0.0 | 7.0 | 0.0 | |
| | 0.0620 | 0.0 | 3.0 | 0.0 | 7.0 | 0.0 | |
| | 0.0320 | 0.0 | 3.0 | 0.0 | 6.0 | 0.0 | |
| | | | | | | | |
| 401 | 0.2500 | 6.0 | 6.0 | 3.0 | 9.0 | 3.0 | 5.0 |
| | 0.1250 | 2.0 | 5.0 | 0.0 | 7.0 | 2.0 | 4.0 |
| | 0.0620 | 0.0 | 4.0 | 0.0 | 7.0 | 0.0 | 4.0 |
| | 0.0320 | 0.0 | 3.0 | | 6.0 | 0.0 | 3.0 |
| | | | | | | | |
| 402 | 0.2500 | 4.0 | 7.0 | 5.0 | 9.0 | 3.0 | 4.0 |
| | 0.1250 | 3.0 | 3.0 | 0.0 | 9.0 | 0.0 | 2.0 |
| | 0.0620 | 0.0 | 2.0 | 0.0 | 7.0 | 0.0 | 0.0 |
| | 0.0320 | 0.0 | 0.0 | | 7.0 | 0.0 | 0.0 |
| | | | | | | | |
| 403 | 0.2500 | 4.0 | 9.0 | 4.0 | 9.0 | 9.0 | |
| | 0.1250 | 4.0 | 7.0 | 2.0 | 9.0 | 9.0 | |
| | 0.0620 | 2.0 | 6.0 | 0.0 | 6.0 | 9.0 | |
| | 0.0320 | 0.0 | 5.0 | 0.0 | 7.0 | 3.0 | |
| | | | | | | | |
| 404 | 0.2500 | 7.0 | 6.0 | 0.0 | 6.0 | 0.0 | 2.0 |
| | 0.1250 | 6.0 | 2.0 | 0.0 | 5.0 | 0.0 | 0.0 |
| | 0.0620 | 3.0 | 0.0 | 0.0 | 5.0 | 0.0 | 0.0 |
| | 0.0320 | 0.0 | 0.0 | 0.0 | 4.0 | 0.0 | 0.0 |
| | | | | | | | |
| 405 | 0.2500 | 9.0 | 8.0 | 0.0 | 7.0 | 6.0 | 2.0 |
| | 0.1250 | 9.0 | 7.0 | 0.0 | 7.0 | 5.0 | 2.0 |
| | 0.0620 | 7.0 | 4.0 | 0.0 | 6.0 | 0.0 | 0.0 |
| | 0.0320 | 7.0 | 4.0 | 0.0 | 6.0 | 0.0 | 0.0 |
| | | | | | | | |
| 406 | 0.2500 | 7.0 | 7.0 | 6.0 | 9.0 | 4.0 | 4.0 |
| | 0.1250 | 7.0 | 4.0 | 3.0 | 9.0 | 4.0 | 2.0 |
| | 0.0620 | 6.0 | 4.0 | 0.0 | 7.0 | 4.0 | 0.0 |
| | 0.0320 | 6.0 | 0.0 | 0.0 | 4.0 | 3.0 | 0.0 |
| | | | | | | | |
| 407 | 0.2500 | 7.0 | 9.0 | 7.0 | 9.0 | 7.0 | 5.0 |
| | 0.1250 | 7.0 | 9.0 | 5.0 | 9.0 | 6.0 | 3.0 |
| | 0.0620 | 3.0 | 4.0 | 0.0 | 7.0 | 2.0 | 2.0 |
| | 0.0320 | 0.0 | 3.0 | 0.0 | 6.0 | 0.0 | 0.0 |
| | | | | | | | |
| 408 | 0.2500 | 7.0 | 9.0 | 6.0 | 9.0 | 6.0 | 3.0 |
| | 0.1250 | 6.0 | 9.0 | 5.0 | 9.0 | 4.0 | 2.0 |
| | 0.0620 | 3.0 | 9.0 | 0.0 | 9.0 | 0.0 | 2.0 |
| | 0.0320 | 2.0 | 6.0 | 0.0 | 7.0 | 0.0 | 0.0 |
| | | | | | | | |
| 409 | 0.2500 | 9.0 | 9.0 | 9.0 | 9.0 | 9.0 | 9.0 |
| | 0.1250 | 9.0 | 9.0 | 9.0 | 9.0 | 7.0 | 7.0 |
| | 0.0620 | 4.0 | 9.0 | 5.0 | 9.0 | 4.0 | 5.0 |
| | 0.0320 | 3.0 | 7.0 | 2.0 | 9.0 | 2.0 | 3.0 |
| | | | | | | | |
| 410 | 0.2500 | 9.0 | 9.0 | 9.0 | 9.0 | 9.0 | 7.0 |
| | 0.1250 | 9.0 | 9.0 | 9.0 | 9.0 | 9.0 | 5.0 |
| | 0.0620 | 0.0 | 9.0 | 5.0 | 9.0 | 7.0 | 3.0 |
| | 0.0320 | 0.0 | 9.0 | 2.0 | 9.0 | 3.0 | 0.0 |
| | | | | | | | |
| 411 | 0.2500 | 6.0 | 9.0 | 9.0 | 9.0 | | 5.0 |
| | 0.1250 | 0.0 | 3.0 | 7.0 | 9.0 | | 2.0 |
| | 0.0620 | 0.0 | 2.0 | 2.0 | 9.0 | | 0.0 |
| | 0.0320 | 0.0 | 0.0 | 0.0 | 9.0 | | 0.0 |
| | | | | | | | |
| 412 | 0.2500 | | 8.0 | 6.0 | 9.0 | | 4.0 |
| | 0.1250 | 0.0 | 4.0 | 4.0 | 9.0 | | 2.0 |
| | 0.0620 | 0.0 | 0.0 | 0.0 | 9.0 | | 0.0 |
| | 0.0320 | 0.0 | 0.0 | 0.0 | 7.0 | | 0.0 |
| | | | | | | | |
| 413 | 0.2500 | 6.0 | 7.0 | 9.0 | 9.0 | | 9.0 |
| | 0.1250 | 0.0 | 5.0 | 5.0 | 9.0 | | 7.0 |
| | 0.0620 | 0.0 | 2.0 | 3.0 | 9.0 | | 5.0 |
| | 0.0320 | 0.0 | 0.0 | 0.0 | 7.0 | | 3.0 |
| | | | | | | | |
| 414 | 0.2500 | 7.0 | 9.0 | 9.0 | 7.0 | | 9.0 |
| | 0.1250 | 2.0 | 6.0 | | 7.0 | | 5.0 |
| | 0.0620 | 0.0 | 2.0 | | 7.0 | | 5.0 |
| | 0.0320 | 0.0 | 0.0 | 0.0 | | | 5.0 |
| | | | | | | | |
| 415 | 0.2500 | 9.0 | 6.0 | | 8.0 | | 9.0 |
| | 0.1250 | 3.0 | 2.0 | | 5.0 | | 5.0 |
| | 0.0620 | 0.0 | 0.0 | 0.0 | 5.0 | | 4.0 |
| | 0.0320 | 0.0 | 0.0 | 0.0 | 5.0 | | 3.0 |
| | | | | | | | |
| 416 | 0.2500 | 9.0 | 8.0 | 3.0 | 9.0 | | 9.0 |
| | 0.1250 | 3.0 | 4.0 | 0.0 | 7.0 | | 9.0 |
| | 0.0620 | 3.0 | 4.0 | 0.0 | 7.0 | | 7.0 |
| | 0.0320 | 0.0 | 4.0 | 0.0 | 7.0 | | 6.0 |
| | | | | | | | |
| 417 | 0.2500 | 3.0 | 6.0 | | 9.0 | | 3.0 |
| | 0.1250 | 3.0 | 5.0 | 3.0 | 9.0 | | 0.0 |
| | 0.0620 | 0.0 | 4.0 | | 6.0 | | 0.0 |
| | 0.0320 | 0.0 | 4.0 | | 6.0 | | 0.0 |
| | | | | | | | |
| 418 | 0.2500 | 3.0 | 0.0 | 0.0 | 0.0 | | 3.0 |
| | 0.1250 | 1.0 | 0.0 | | 0.0 | | 2.0 |
| | 0.0620 | 0.0 | 0.0 | 0.0 | 0.0 | | 0.0 |
| | | | | | | | |
| 419 | 0.2500 | 7.0 | 6.0 | 0.0 | 5.0 | | 3.0 |
| | 0.1250 | 2.0 | 3.0 | 0.0 | 4.0 | | 1.0 |
| | 0.0620 | 0.0 | 0.0 | 0.0 | 0.0 | | 0.0 |
| | | | | | | | |
| 420 | 0.2500 | 7.0 | 6.0 | 0.0 | 9.0 | 3.0 | 5.0 |
| | 0.1250 | 7.0 | 6.0 | | 9.0 | 2.0 | 3.0 |
| | 0.0620 | 7.0 | 4.0 | | 9.0 | 0.0 | 2.0 |
| | 0.0320 | 7.0 | 2.0 | | 9.0 | 0.0 | 0.0 |
| | | | | | | | |
| 421 | 0.2500 | 9.0 | 9.0 | 4.0 | 9.0 | 2.0 | 2.0 |
| | 0.1250 | 7.0 | 8.0 | | 7.0 | 0.0 | 0.0 |
| | 0.0620 | 5.0 | 6.0 | | 6.0 | 0.0 | 0.0 |
| | 0.0320 | 5.0 | 2.0 | | 5.0 | 0.0 | 0.0 |
| | | | | | | | |
| 422 | 0.2500 | 9.0 | 9.0 | | 9.0 | 6.0 | 3.0 |
| | 0.1250 | 9.0 | 9.0 | | 9.0 | 3.0 | 2.0 |
| | 0.0620 | 6.0 | 6.0 | 5.0 | 9.0 | 0.0 | 0.0 |
| | 0.0320 | 5.0 | 5.0 | 0.0 | 7.0 | 0.0 | 0.0 |
| | | | | | | | |
| 423 | 0.2500 | 9.0 | 9.0 | 9.0 | 9.0 | 9.0 | 7.0 |
| | 0.1250 | 9.0 | 9.0 | 9.0 | 9.0 | 9.0 | 7.0 |
| | 0.0620 | 9.0 | 7.0 | 9.0 | 9.0 | 9.0 | 6.0 |
| | 0.0320 | 6.0 | 6.0 | 9.0 | 9.0 | 9.0 | 5.0 |
| | | | | | | | |
| 424 | 0.2500 | 3.0 | 7.0 | 3.0 | 9.0 | 0.0 | 2.0 |
| | 0.1250 | 3.0 | 6.0 | 3.0 | 9.0 | 0.0 | 0.0 |
| | 0.0620 | 0.0 | 5.0 | | 9.0 | 0.0 | 0.0 |
| | 0.0320 | 0.0 | 4.0 | | 7.0 | 0.0 | 0.0 |
| | | | | | | | |
| 425 | 0.2500 | 9.0 | 9.0 | 3.0 | 9.0 | 4.0 | 8.0 |
| | 0.1250 | 9.0 | 6.0 | 3.0 | 9.0 | 3.0 | 7.0 |
| | 0.0620 | 9.0 | 0.0 | 3.0 | 7.0 | 0.0 | 6.0 |
| | 0.0320 | 6.0 | 0.0 | 0.0 | 6.0 | 0.0 | 4.0 |
| | | | | | | | |
| 426 | 0.2500 | 9.0 | 9.0 | 2.0 | | 4.0 | 6.0 |
| | 0.1250 | 5.0 | 7.0 | 0.0 | | 4.0 | 4.0 |
| | 0.0620 | 5.0 | 7.0 | 0.0 | | 2.0 | 2.0 |
| | 0.0320 | 5.0 | 5.0 | 0.0 | | 0.0 | 2.0 |
| | | | | | | | |
| 427 | 0.2500 | 9.0 | 9.0 | 4.0 | | 9.0 | 3.0 |
| | 0.1250 | 9.0 | 7.0 | | | 6.0 | 1.0 |
| | 0.0620 | 7.0 | 7.0 | 0.0 | | 5.0 | 0.0 |
| | 0.0320 | 7.0 | 7.0 | 0.0 | | 4.0 | 0.0 |
| | | | | | | | |
| 428 | 0.2500 | 9.0 | 7.0 | | 9.0 | 6.0 | 6.0 |
| | 0.1250 | 6.0 | 4.0 | | 9.0 | 2.0 | 6.0 |
| | 0.0620 | 3.0 | 2.0 | 0.0 | 9.0 | 0.0 | 2.0 |
| | 0.0320 | 2.0 | 0.0 | 0.0 | 9.0 | 0.0 | 0.0 |
| | | | | | | | |
| 429 | 0.2500 | 9.0 | 9.0 | 1.0 | 9.0 | 3.0 | 3.0 |
| | 0.1250 | 9.0 | 8.3 | 1.0 | 9.0 | 2.3 | 2.3 |
| | 0.0620 | 9.0 | 7.7 | 1.0 | 9.0 | 2.3 | 2.0 |
| | 0.0320 | 9.0 | 7.3 | 0.0 | 9.0 | 2.0 | 1.3 |
| | | | | | | | |
| 430 | 0.2500 | 9.0 | 6.0 | 7.0 | 9.0 | 7.0 | 3.0 |
| | 0.1250 | 7.0 | 7.0 | 2.0 | 9.0 | 6.0 | 4.0 |
| | 0.0620 | 5.0 | 6.0 | 0.0 | 9.0 | | 2.0 |
| | 0.0320 | 5.0 | 2.0 | 0.0 | 9.0 | | 3.0 |
| | | | | | | | |
| 431 | 0.2500 | 9.0 | 9.0 | 9.0 | 9.0 | 9.0 | 6.5 |
| | 0.1250 | 8.5 | 9.0 | 7.5 | 9.0 | 7.5 | 5.0 |
| | 0.0620 | 9.0 | 9.0 | 4.0 | 9.0 | 5.5 | 5.5 |
| | 0.0320 | 8.0 | 8.0 | 0.0 | 9.0 | 3.5 | 3.5 |
| | | | | | | | |
| 432 | 0.2500 | 9.0 | 9.0 | 9.0 | 9.0 | 8.5 | 7.5 |
| | 0.1250 | 9.0 | 9.0 | 6.5 | 9.0 | 7.5 | 6.5 |
| | 0.0620 | 9.0 | 9.0 | 3.5 | 9.0 | 3.5 | 6.0 |
| | 0.0320 | 9.0 | 8.0 | 0.0 | 9.0 | 0.0 | 6.0 |
| | | | | | | | |
| 433 | 0.2500 | 9.0 | 9.0 | 7.5 | 9.0 | 7.0 | 7.5 |
| | 0.1250 | 9.0 | 9.0 | 7.5 | 9.0 | 6.0 | 5.0 |
| | 0.0620 | 9.0 | 8.5 | 6.0 | 9.0 | 4.5 | 4.0 |
| | 0.0320 | 8.0 | 5.5 | 0.0 | 8.5 | 2.0 | 2.0 |
| | | | | | | | |
| 434 | 0.2500 | 9.0 | 8.0 | 7.0 | 9.0 | 6.0 | 7.0 |
| | 0.1250 | 7.0 | 7.0 | 4.0 | 9.0 | 5.0 | 6.0 |
| | 0.0620 | 7.0 | 3.0 | | 9.0 | 4.0 | 6.0 |
| | 0.0320 | 6.0 | 3.0 | 4.0 | 9.0 | 4.0 | 4.0 |
| | | | | | | | |
| 435 | 0.2500 | 9.0 | 3.0 | 2.0 | 9.0 | 6.0 | 2.0 |
| | 0.1250 | 7.0 | 2.0 | 3.0 | 9.0 | 0.0 | 2.0 |
| | 0.0620 | 7.0 | 0.0 | 0.0 | 9.0 | 0.0 | 1.0 |
| | 0.0320 | 5.0 | 0.0 | | 9.0 | 0.0 | 2.0 |
| | | | | | | | |
| 436 | 0.2500 | 9.0 | 7.0 | 6.0 | 9.0 | 3.0 | 6.0 |
| | 0.1250 | 9.0 | 7.0 | | 9.0 | 2.0 | 7.0 |
| | 0.0620 | 9.0 | 2.0 | 6.0 | 9.0 | 0.0 | 5.0 |
| | 0.0320 | 6.0 | 0.0 | 4.0 | 9.0 | 0.0 | 3.0 |
| | | | | | | | |
| 437 | 0.2500 | 8.0 | 6.0 | 7.0 | 9.0 | 7.0 | 7.0 |
| | 0.1250 | 7.0 | 4.0 | 3.0 | 9.0 | 4.0 | 6.0 |
| | 0.0620 | 7.0 | 0.0 | 2.0 | 9.0 | 3.0 | 5.0 |
| | 0.0320 | 5.0 | 0.0 | 0.0 | 9.0 | 0.0 | 4.0 |
| | | | | | | | |
| 438 | 0.2500 | 9.0 | 9.0 | 9.0 | 9.0 | 9.0 | 8.0 |
| | 0.1250 | 9.0 | 8.5 | 7.0 | 9.0 | 8.5 | 7.0 |
| | 0.0620 | 8.0 | 6.5 | 9.0 | 8.5 | 3.5 | 5.0 |
| | 0.0320 | 7.0 | 4.5 | 4.5 | 8.0 | 2.0 | 4.0 |
| | | | | | | | |
| 439 | 0.2500 | 6.0 | 5.0 | 0.0 | 7.0 | 3.0 | 0.0 |
| | 0.1250 | 6.0 | 4.0 | 0.0 | 7.0 | 0.0 | 0.0 |
| | 0.0620 | 3.0 | 3.0 | 0.0 | 6.0 | 0.0 | 0.0 |
| | 0.0320 | 2.0 | 1.0 | 0.0 | 5.0 | 0.0 | 0.0 |
| | | | | | | | |
| 440 | 0.2500 | 9.0 | 9.0 | 9.0 | 9.0 | 9.0 | 4.0 |
| | 0.1250 | 9.0 | 9.0 | 9.0 | 9.0 | 7.0 | 4.0 |
| | 0.0620 | 7.0 | 9.0 | 9.0 | 9.0 | 6.0 | 4.0 |
| | 0.0320 | 5.0 | 5.0 | 6.0 | 7.0 | 4.0 | 3.0 |
| | | | | | | | |
| 441 | 0.2500 | 3.0 | 9.0 | 7.0 | 9.0 | 6.0 | 3.0 |
| | 0.1250 | 0.0 | 9.0 | 4.0 | 9.0 | 3.0 | 2.0 |
| | 0.0620 | 0.0 | 6.0 | 0.0 | 9.0 | 2.0 | 0.0 |
| | 0.0320 | 0.0 | 4.0 | | 9.0 | 0.0 | 0.0 |
| | | | | | | | |
| 442 | 0.2500 | 9.0 | 9.0 | 4.5 | 9.0 | 4.0 | 3.3 |
| | 0.1250 | 9.0 | 8.7 | 0.0 | 9.0 | 2.0 | 1.7 |
| | 0.0620 | 9.0 | 6.3 | 0.0 | 8.3 | 1.7 | 1.3 |
| | 0.0320 | 8.3 | 4.3 | 0.0 | 6.0 | 1.3 | 0.3 |
| | | | | | | | |
| 443 | 0.2500 | 9.0 | 7.0 | | 7.0 | 5.0 | 8.0 |
| | 0.1250 | 7.0 | 7.0 | 0.0 | 7.0 | 4.0 | 5.0 |
| | 0.0620 | 5.0 | 6.0 | 0.0 | 6.0 | 3.0 | 4.0 |
| | 0.0320 | 6.0 | 4.0 | 0.0 | 5.0 | 3.0 | 3.0 |
| | | | | | | | |
| 444 | 0.2500 | 9.0 | 9.0 | 9.0 | 9.0 | 9.0 | 8.5 |
| | 0.1250 | 8.5 | 9.0 | 9.0 | 9.0 | 7.0 | 9.0 |
| | 0.0620 | 6.0 | 8.0 | 9.0 | 9.0 | 6.0 | 6.5 |
| | 0.0320 | 3.5 | 7.5 | 3.5 | 8.0 | 4.5 | 4.5 |
| | | | | | | | |
| 445 | 0.2500 | 9.0 | 9.0 | 8.5 | 9.0 | 9.0 | 8.0 |
| | 0.1250 | 9.0 | 9.0 | 8.0 | 9.0 | 8.5 | 7.0 |
| | 0.0620 | 8.5 | 8.0 | 8.5 | 9.0 | 8.0 | 6.5 |
| | 0.0320 | 8.5 | 6.5 | 7.5 | 9.0 | 4.5 | 5.0 |
| | | | | | | | |
| 446 | 0.2500 | 9.0 | 9.0 | 9.0 | 9.0 | 9.0 | 9.0 |
| | 0.1250 | 7.0 | 9.0 | 9.0 | 9.0 | 9.0 | 9.0 |
| | 0.0620 | 6.0 | 9.0 | 9.0 | 9.0 | 9.0 | 7.0 |
| | 0.0320 | 6.0 | 9.0 | 9.0 | 9.0 | 9.0 | 6.0 |
| | | | | | | | |
| 447 | 0.2500 | 9.0 | 9.0 | 4.0 | 9.0 | 9.0 | 7.0 |
| | 0.1250 | 7.0 | 7.0 | | 9.0 | 5.0 | 5.0 |
| | 0.0620 | 6.0 | 6.0 | 0.0 | 9.0 | 4.0 | 4.0 |
| | 0.0320 | 4.0 | 4.0 | 0.0 | 7.0 | 4.0 | 4.0 |
| | | | | | | | |
| 448 | 0.2500 | 9.0 | 9.0 | 6.5 | 9.0 | 3.5 | 4.0 |
| | 0.1250 | 9.0 | 9.0 | 4.5 | 9.0 | 2.5 | 4.0 |
| | 0.0620 | 9.0 | 9.0 | 4.5 | 9.0 | 1.5 | 4.0 |
| | 0.0320 | 9.0 | 8.5 | 4.5 | 9.0 | 0.0 | 3.5 |
| | | | | | | | |
| 449 | 0.2500 | 9.0 | 9.0 | 4.0 | 9.0 | 6.0 | 9.0 |
| | 0.1250 | 9.0 | 8.0 | | 9.0 | 4.0 | 3.0 |
| | 0.0620 | 7.0 | 5.0 | | 7.0 | 4.0 | 3.0 |
| | 0.0320 | 5.0 | 4.0 | 2.0 | 7.0 | 2.0 | 2.0 |
| | | | | | | | |
| 450 | 0.2500 | 9.0 | 9.0 | | 9.0 | 9.0 | 9.0 |
| | 0.1250 | 6.0 | 9.0 | | 9.0 | 6.0 | 5.0 |
| | 0.0620 | 2.0 | 7.0 | | 7.0 | 5.0 | 4.0 |
| | 0.0320 | 0.0 | 6.0 | 0.0 | 7.0 | 5.0 | 3.0 |
| | | | | | | | |
| 451 | 0.2500 | 7.0 | 9.0 | 0.0 | 9.0 | 9.0 | 6.0 |
| | 0.1250 | 6.0 | 7.0 | | 9.0 | 6.0 | 6.0 |
| | 0.0620 | 0.0 | 5.0 | 0.0 | 9.0 | 5.0 | 4.0 |
| | 0.0320 | 0.0 | 5.0 | | 9.0 | 4.0 | 4.0 |
| | | | | | | | |
| 452 | 0.2500 | 7.0 | 9.0 | 7.0 | 9.0 | 2.0 | 7.0 |
| | 0.1250 | 3.0 | 9.0 | | 9.0 | 0.0 | 6.0 |
| | 0.0620 | 3.0 | 6.0 | | 9.0 | 0.0 | 5.0 |
| | 0.0320 | 0.0 | 4.0 | 0.0 | 9.0 | 0.0 | 3.0 |
| | | | | | | | |
| 453 | 0.2500 | 9.0 | 9.0 | 9.0 | 9.0 | 9.0 | 9.0 |
| | 0.1250 | 9.0 | 9.0 | 9.0 | 9.0 | 9.0 | 9.0 |
| | 0.0620 | 9.0 | 9.0 | 9.0 | 9.0 | 9.0 | 9.0 |
| | 0.0320 | 9.0 | 9.0 | 9.0 | 9.0 | 9.0 | 9.0 |
| | | | | | | | |
| 454 | 0.2500 | 7.0 | 9.0 | 8.0 | 9.0 | 6.0 | 5.0 |
| | 0.1250 | 6.0 | 6.0 | 4.0 | 9.0 | 3.0 | 5.0 |
| | 0.0620 | 3.0 | 1.0 | 0.0 | 7.0 | 2.0 | 2.0 |
| | 0.0320 | 3.0 | 0.0 | 0.0 | 2.0 | 0.0 | 2.0 |
| | | | | | | | |
| 455 | 0.2500 | 9.0 | 9.0 | 9.0 | 9.0 | 9.0 | 9.0 |
| | 0.1250 | 9.0 | 9.0 | 8.0 | 9.0 | 7.0 | 9.0 |
| | 0.0620 | 9.0 | 7.0 | 6.0 | 7.0 | 6.0 | 8.0 |
| | 0.0320 | 9.0 | 6.0 | 2.0 | 6.0 | 4.0 | 6.0 |
| | | | | | | | |
| 456 | 0.2500 | 9.0 | 6.0 | 4.0 | 9.0 | 4.0 | 9.0 |
| | 0.1250 | 7.0 | 6.0 | 2.0 | 9.0 | 2.0 | 8.0 |
| | 0.0620 | 6.0 | 4.0 | 0.0 | 9.0 | 0.0 | 8.0 |
| | 0.0320 | 4.0 | 0.0 | 0.0 | 7.0 | 0.0 | 7.0 |
| | | | | | | | |
| 457 | 0.2500 | 9.0 | 7.0 | 0.0 | 7.0 | 3.0 | 3.0 |
| | 0.1250 | 9.0 | 5.0 | 0.0 | 7.0 | 3.0 | 0.0 |
| | 0.0620 | 7.0 | 3.0 | 0.0 | 4.0 | 0.0 | 0.0 |
| | 0.0320 | 5.0 | 0.0 | 0.0 | 2.0 | 0.0 | 0.0 |
| | | | | | | | |
| 458 | 0.2500 | 8.0 | 7.0 | 6.0 | 9.0 | 8.0 | 5.0 |
| | 0.1250 | 7.0 | 7.0 | 9.0 | 9.0 | 6.0 | 5.0 |
| | 0.0620 | 6.0 | 4.0 | | 6.0 | 2.0 | 3.0 |
| | 0.0320 | 5.0 | 0.0 | 2.0 | 4.0 | 2.0 | 3.0 |
| | | | | | | | |
| 459 | 0.2500 | 9.0 | 4.0 | 3.0 | 7.0 | 0.0 | 5.0 |
| | 0.1250 | 9.0 | 4.0 | 0.0 | 7.0 | 0.0 | 5.0 |
| | 0.0620 | 7.0 | 2.0 | 0.0 | 5.0 | 0.0 | 4.0 |
| | 0.0320 | 7.0 | 0.0 | 0.0 | 3.0 | 0.0 | 2.0 |
| | | | | | | | |
| 460 | 0.2500 | 9.0 | 3.0 | 0.0 | 4.0 | 3.0 | 5.0 |
| | 0.1250 | 9.0 | 3.0 | 0.0 | 4.0 | 3.0 | 4.0 |
| | 0.0620 | 7.0 | 2.0 | | 4.0 | 0.0 | 4.0 |
| | 0.0320 | 6.0 | 0.0 | | 3.0 | 0.0 | 4.0 |
| | | | | | | | |
| 461 | 0.2500 | 9.0 | 9.0 | 7.0 | 9.0 | 6.0 | 6.0 |
| | 0.1250 | 9.0 | 9.0 | | 9.0 | 4.0 | 5.0 |
| | 0.0620 | 9.0 | 9.0 | | 7.0 | 3.0 | 5.0 |
| | 0.0320 | 9.0 | 6.0 | 0.0 | 7.0 | 2.0 | 5.0 |
| | | | | | | | |
| 462 | 0.2500 | 9.0 | 9.0 | | 9.0 | 6.0 | 6.0 |
| | 0.1250 | 9.0 | 9.0 | | 9.0 | 5.0 | 9.0 |
| | 0.0620 | 9.0 | 9.0 | 3.0 | 9.0 | 4.0 | 5.0 |
| | 0.0320 | 9.0 | 9.0 | | 9.0 | 1.0 | 5.0 |
| | | | | | | | |
| 463 | 0.2500 | 9.0 | 9.0 | 7.0 | 9.0 | 7.0 | 6.0 |
| | 0.1250 | 9.0 | 9.0 | 6.0 | 9.0 | 5.0 | 5.0 |
| | 0.0620 | 6.0 | 6.0 | | 9.0 | 3.0 | 2.0 |
| | 0.0320 | 2.0 | 5.0 | 2.0 | 7.0 | 0.0 | 0.0 |
| | | | | | | | |
| 464 | 0.2500 | 9.0 | 5.0 | 7.0 | 6.0 | 4.0 | 4.0 |
| | 0.1250 | 6.0 | 3.0 | 0.0 | 9.0 | 6.0 | 2.0 |
| | 0.0620 | | 3.0 | 0.0 | 9.0 | 0.0 | 2.0 |
| | 0.0320 | 6.0 | 0.0 | 0.0 | 7.0 | 0.0 | 0.0 |
| | | | | | | | |
| 465 | 0.2500 | 9.0 | 8.0 | 9.0 | 9.0 | 6.0 | 5.0 |
| | 0.1250 | 7.0 | 3.0 | 7.0 | 6.0 | 5.0 | 4.0 |
| | 0.0620 | 6.0 | 0.0 | 4.0 | 3.0 | 5.0 | 3.0 |
| | 0.0320 | 6.0 | 0.0 | 0.0 | 2.0 | 2.0 | 2.0 |
| | | | | | | | |
| 466 | 0.2500 | 7.0 | 9.0 | 1.0 | 9.0 | 7.0 | 9.0 |
| | 0.1250 | 5.0 | 7.0 | | 9.0 | 3.0 | 7.0 |
| | 0.0620 | 4.0 | 3.0 | | 7.0 | 0.0 | 5.0 |
| | 0.0320 | 2.0 | 3.0 | | 7.0 | 0.0 | 4.0 |
| | | | | | | | |
| 467 | 0.2500 | 9.0 | 9.0 | 9.0 | 9.0 | 7.0 | 5.0 |
| | 0.1250 | 9.0 | 6.0 | 9.0 | 9.0 | 3.0 | 4.0 |
| | 0.0620 | 7.0 | 5.0 | 7.0 | 9.0 | 0.0 | 2.0 |
| | 0.0320 | 6.0 | 2.0 | 6.0 | 7.0 | 0.0 | 2.0 |
| | | | | | | | |
| 468 | 0.2500 | 9.0 | 5.0 | 5.0 | 5.0 | 5.0 | 5.0 |
| | 0.1250 | 9.0 | 6.0 | 7.0 | 7.0 | 4.0 | 5.0 |
| | 0.0620 | 7.0 | 6.0 | 6.0 | 6.0 | 4.0 | 3.0 |
| | 0.0320 | 7.0 | 5.0 | 2.0 | 5.0 | 3.0 | 3.0 |
| | | | | | | | |
| 469 | 0.2500 | 9.0 | 7.0 | 6.0 | 8.0 | 3.0 | 1.5 |
| | 0.1250 | 9.0 | 6.5 | 9.0 | 5.0 | 2.5 | 0.0 |
| | 0.0620 | 9.0 | 1.5 | 4.5 | 4.5 | 1.5 | 0.0 |
| | 0.0320 | 7.0 | 0.0 | 0.0 | 2.0 | 0.0 | 0.0 |
| | | | | | | | |
| 470 | 0.2500 | 9.0 | 7.0 | 4.5 | 3.0 | 1.5 | 1.5 |
| | 0.1250 | 9.0 | 6.0 | 0.0 | 2.0 | 0.0 | 1.0 |
| | 0.0620 | 9.0 | 0.0 | 0.0 | 2.0 | 0.0 | 0.0 |
| | 0.0320 | 8.5 | 0.0 | 0.0 | 1.5 | 0.0 | 0.0 |
| | | | | | | | |
| 471 | 0.2500 | 9.0 | 9.0 | 7.0 | 9.0 | 7.0 | 5.0 |
| | 0.1250 | 6.0 | 9.0 | | 9.0 | 5.0 | 5.0 |
| | 0.0620 | 3.0 | 6.0 | | 7.0 | 3.0 | 3.0 |
| | 0.0320 | 0.0 | 6.0 | 3.0 | 6.0 | 0.0 | 2.0 |
| | | | | | | | |
| 472 | 0.2500 | 4.0 | 6.0 | 9.0 | 7.0 | 3.0 | 6.0 |
| | 0.1250 | 4.0 | 5.0 | 6.0 | 7.0 | 2.0 | 3.0 |
| | 0.0620 | 3.0 | 4.0 | | 6.0 | 0.0 | 1.0 |
| | 0.0320 | 3.0 | 4.0 | 0.0 | 5.0 | 0.0 | 0.0 |
| | | | | | | | |
| 473 | 0.2500 | 4.0 | 8.0 | 5.0 | 7.0 | 3.0 | 5.0 |
| | 0.1250 | 2.0 | 6.0 | | 7.0 | 2.0 | 4.0 |
| | 0.0620 | 2.0 | 4.0 | 0.0 | 6.0 | 0.0 | 2.0 |
| | 0.0320 | 1.0 | 2.0 | 0.0 | 4.0 | 0.0 | 0.0 |
| | | | | | | | |
| 474 | 0.2500 | 0.0 | 6.0 | 0.0 | 9.0 | 0.0 | 3.0 |
| | 0.1250 | 0.0 | 4.0 | 0.0 | 7.0 | 0.0 | 2.0 |
| | 0.0620 | 0.0 | 5.0 | 0.0 | 3.0 | 0.0 | 0.0 |
| | 0.0320 | 0.0 | 2.0 | 0.0 | 2.0 | 0.0 | 0.0 |
| | | | | | | | |
| 475 | 0.2500 | 3.0 | 9.0 | 7.0 | 9.0 | 7.0 | 9.0 |
| | 0.1250 | 2.0 | 9.0 | 5.0 | 9.0 | 6.0 | 3.0 |
| | 0.0620 | 0.0 | 6.0 | | 9.0 | 4.0 | 0.0 |
| | 0.0320 | 0.0 | 4.0 | 0.0 | 9.0 | 0.0 | 0.0 |
| | | | | | | | |
| 476 | 0.2500 | 3.0 | 9.0 | 6.0 | 9.0 | 5.0 | 6.0 |
| | 0.1250 | 2.0 | 9.0 | 3.0 | 6.0 | 4.0 | 4.0 |
| | 0.0620 | 2.0 | 4.0 | | 6.0 | 2.0 | 3.0 |
| | 0.0320 | 0.0 | 4.0 | 0.0 | 5.0 | 0.0 | 2.0 |
| | | | | | | | |
| 477 | 0.2500 | 3.0 | 6.0 | 3.0 | 7.0 | 2.0 | 6.0 |
| | 0.1250 | 2.0 | 5.0 | | 6.0 | 0.0 | 4.0 |
| | 0.0620 | 0.0 | 4.0 | 3.0 | 3.0 | 0.0 | 4.0 |
| | 0.0320 | 0.0 | 4.0 | 3.0 | 3.0 | 0.0 | 3.0 |
| | | | | | | | |
| 478 | 0.2500 | 9.0 | 9.0 | 9.0 | 9.0 | 9.0 | 9.0 |
| | 0.1250 | 7.0 | 9.0 | 9.0 | 9.0 | 9.0 | 9.0 |
| | 0.0620 | 6.0 | 9.0 | 7.0 | 9.0 | 6.0 | 9.0 |
| | 0.0320 | 4.0 | 4.0 | 6.0 | 7.0 | 6.0 | 7.0 |
| | | | | | | | |
| 479 | 0.2500 | 9.0 | 9.0 | 9.0 | 9.0 | 8.0 | 9.0 |
| | 0.1250 | 9.0 | 5.0 | 9.0 | 9.0 | 6.0 | 9.0 |
| | 0.0620 | 7.0 | 0.0 | 7.0 | 7.0 | 5.0 | 5.0 |
| | 0.0320 | 7.0 | 0.0 | 0.0 | 0.0 | 3.0 | 5.0 |
| | | | | | | | |
| 480 | 0.2500 | 6.0 | 9.0 | | 9.0 | 6.0 | 6.0 |
| | 0.1250 | 5.0 | 9.0 | 3.0 | 9.0 | 5.0 | 5.0 |
| | 0.0620 | 2.0 | 9.0 | | 9.0 | 3.0 | 5.0 |
| | 0.0320 | 0.0 | 6.0 | 3.0 | 7.0 | 0.0 | 4.0 |
| | | | | | | | |
| 481 | 0.2500 | 9.0 | 9.0 | 6.0 | 9.0 | | 7.0 |
| | 0.1250 | 9.0 | 6.0 | | 7.0 | 0.0 | 2.0 |
| | 0.0620 | 2.0 | 2.0 | | 7.0 | 0.0 | 0.0 |
| | 0.0320 | 0.0 | 0.0 | | 6.0 | 0.0 | 0.0 |
| | | | | | | | |
| 482 | 0.2500 | 9.0 | 6.0 | | 7.0 | 7.0 | 7.0 |
| | 0.1250 | 9.0 | 0.0 | | 6.0 | 5.0 | 6.0 |
| | 0.0620 | 7.0 | 0.0 | | 5.0 | 4.0 | 2.0 |
| | 0.0320 | 4.0 | 0.0 | | 4.0 | 3.0 | 0.0 |
| | | | | | | | |
| 483 | 0.2500 | 7.0 | 6.0 | 6.0 | 9.0 | 5.0 | 6.0 |
| | 0.1250 | 7.0 | 5.0 | | 9.0 | 3.0 | 5.0 |
| | 0.0620 | 7.0 | 2.0 | 4.0 | 9.0 | 0.0 | 3.0 |
| | 0.0320 | 6.0 | 0.0 | | 7.0 | 0.0 | 3.0 |
| | | | | | | | |
| 484 | 0.2500 | 9.0 | 9.0 | | 9.0 | | 6.0 |
| | 0.1250 | 7.0 | 6.0 | | 9.0 | | 6.0 |
| | 0.0620 | 4.0 | 4.0 | | 9.0 | | 5.0 |
| | 0.0320 | 5.0 | 4.0 | 0.0 | 7.0 | 5.0 | 5.0 |
| | | | | | | | |
| 485 | 0.2500 | 9.0 | 6.0 | | 8.0 | 3.0 | 5.0 |
| | 0.1250 | 6.0 | 5.0 | | 7.0 | 0.0 | 4.0 |
| | 0.0620 | 6.0 | 4.0 | 0.0 | 6.0 | 0.0 | 2.0 |
| | 0.0320 | 4.0 | 2.0 | 0.0 | 4.0 | 0.0 | 0.0 |
| | | | | | | | |
| 486 | 0.2500 | 9.0 | 6.0 | 3.0 | 9.0 | 6.0 | 6.0 |
| | 0.1250 | 7.0 | 6.0 | | 7.0 | 3.0 | 5.0 |
| | 0.0620 | 6.0 | 5.0 | 0.0 | 6.0 | 0.0 | 3.0 |
| | 0.0320 | 4.0 | 4.0 | 0.0 | 5.0 | 0.0 | 0.0 |
| | | | | | | | |
| 487 | 0.2500 | 9.0 | 9.0 | 3.0 | 9.0 | 7.0 | 7.0 |
| | 0.1250 | 7.0 | 6.0 | | 9.0 | 6.0 | 6.0 |
| | 0.0620 | 7.0 | 5.0 | | 9.0 | 5.0 | 3.0 |
| | 0.0320 | 6.0 | 4.0 | | 7.0 | 4.0 | 2.0 |
| | | | | | | | |
| 488 | 0.2500 | 0.0 | 5.0 | | 9.0 | 0.0 | 4.0 |
| | 0.1250 | 0.0 | 4.0 | 0.0 | 9.0 | 0.0 | 4.0 |
| | 0.0620 | 0.0 | 3.0 | 0.0 | 9.0 | 0.0 | 3.0 |
| | 0.0320 | 0.0 | 3.0 | | 7.0 | 0.0 | 3.0 |
| | | | | | | | |
| 489 | 0.2500 | 9.0 | 9.0 | 9.0 | 9.0 | 9.0 | 6.0 |
| | 0.1250 | 9.0 | 9.0 | 9.0 | 9.0 | 9.0 | 6.0 |
| | 0.0620 | 7.0 | 9.0 | 7.0 | 9.0 | 9.0 | 6.0 |
| | 0.0320 | 7.0 | 6.0 | 6.0 | 9.0 | 6.0 | 5.0 |
| | | | | | | | |
| 490 | 0.2500 | 9.0 | 9.0 | 9.0 | 9.0 | 9.0 | 9.0 |
| | 0.1250 | 9.0 | 9.0 | 9.0 | 9.0 | 9.0 | 8.5 |
| | 0.0620 | 9.0 | 9.0 | 9.0 | 9.0 | 9.0 | 9.0 |
| | 0.0320 | 8.0 | 9.0 | 9.0 | 9.0 | 6.0 | 7.5 |
| | | | | | | | |
| 491 | 0.2500 | 9.0 | 9.0 | 9.0 | 9.0 | 9.0 | 7.5 |
| | 0.1250 | 9.0 | 9.0 | 9.0 | 9.0 | 9.0 | 8.0 |
| | 0.0620 | 9.0 | 9.0 | 9.0 | 9.0 | 5.5 | 6.5 |
| | 0.0320 | 9.0 | 7.5 | 7.0 | 9.0 | 5.0 | 5.0 |
| | | | | | | | |
| 492 | 0.2500 | 9.0 | 5.0 | 0.0 | 7.0 | 2.0 | 3.0 |
| | 0.1250 | 6.0 | 4.0 | | 7.0 | 0.0 | 2.0 |
| | 0.0620 | 6.0 | 4.0 | | 6.0 | 0.0 | 2.0 |
| | 0.0320 | 4.0 | 4.0 | | 6.0 | 0.0 | 2.0 |
| | | | | | | | |
| 493 | 0.2500 | 9.0 | 9.0 | 9.0 | 9.0 | 3.0 | 3.0 |
| | 0.1250 | 9.0 | 7.0 | 9.0 | 7.0 | 0.0 | 3.0 |
| | 0.0620 | 5.0 | 6.0 | | 7.0 | 0.0 | 2.0 |
| | 0.0320 | 4.0 | 4.0 | | 5.0 | 0.0 | 2.0 |
| | | | | | | | |
| 494 | 0.2500 | 3.0 | 5.0 | 3.0 | 6.0 | 3.0 | 0.0 |
| | 0.1250 | 3.0 | 5.0 | 0.0 | 5.0 | 0.0 | 0.0 |
| | 0.0620 | 2.0 | 4.0 | | 4.0 | 0.0 | 0.0 |
| | 0.0320 | 0.0 | 3.0 | | 4.0 | 0.0 | 0.0 |
| | | | | | | | |
| 495 | 0.2500 | 7.0 | 9.0 | | 9.0 | | 7.0 |
| | 0.1250 | 5.0 | 7.0 | | 9.0 | 3.0 | 5.0 |
| | 0.0620 | 4.0 | 6.0 | | 7.0 | 0.0 | 4.0 |
| | 0.0320 | 3.0 | 6.0 | | 7.0 | 0.0 | 2.0 |
| | | | | | | | |
| 496 | 0.2500 | 7.0 | 9.0 | 7.0 | 8.0 | 6.0 | 5.0 |
| | 0.1250 | 6.0 | 7.0 | | 6.0 | 3.0 | 3.0 |
| | 0.0620 | 4.0 | 7.0 | | 6.0 | 0.0 | 3.0 |
| | 0.0320 | 4.0 | 0.0 | | 0.0 | 0.0 | 0.0 |
| | | | | | | | |
| 497 | 0.2500 | 7.0 | 9.0 | 9.0 | 9.0 | 6.0 | 8.0 |
| | 0.1250 | 4.0 | 9.0 | 6.0 | 9.0 | 2.0 | 7.0 |
| | 0.0620 | 0.0 | 6.0 | | 7.0 | 0.0 | 3.0 |
| | 0.0320 | 0.0 | 5.0 | | 6.0 | 0.0 | 0.0 |
| | | | | | | | |
| 498 | 0.2500 | 6.0 | 9.0 | | 8.0 | 6.0 | 6.0 |
| | 0.1250 | 5.0 | 9.0 | 0.0 | 7.0 | 3.0 | 3.0 |
| | 0.0620 | 3.0 | 5.0 | | 5.0 | 0.0 | 2.0 |
| | 0.0320 | 0.0 | 2.0 | | 4.0 | 0.0 | 0.0 |
| | | | | | | | |
| 499 | 0.2500 | 9.0 | 9.0 | 9.0 | 9.0 | 9.0 | 9.0 |
| | 0.1250 | 9.0 | 9.0 | 9.0 | 9.0 | 9.0 | 7.5 |
| | 0.0620 | 9.0 | 9.0 | 9.0 | 9.0 | 9.0 | 8.0 |
| | 0.0320 | 7.5 | 8.0 | 9.0 | 8.0 | 8.0 | 6.5 |
| | | | | | | | |
| 500 | 0.2500 | 9.0 | 9.0 | 0.0 | 9.0 | 9.0 | 2.0 |
| | 0.1250 | 7.0 | 6.0 | 0.0 | 9.0 | 7.0 | 2.0 |
| | 0.0620 | 6.0 | 6.0 | 0.0 | 9.0 | 7.0 | 2.0 |
| | 0.0320 | 4.0 | 6.0 | 0.0 | 7.0 | 5.0 | 2.0 |
| | | | | | | | |
| 501 | 0.2500 | 7.0 | 6.0 | 5.0 | 9.0 | 6.0 | 4.0 |
| | 0.1250 | 5.0 | 4.0 | 3.0 | 7.0 | 5.0 | 3.0 |
| | 0.0620 | 5.0 | 2.0 | 0.0 | 6.0 | 3.0 | 1.0 |
| | 0.0320 | 4.0 | 0.0 | 0.0 | 6.0 | 2.0 | 0.0 |
| | | | | | | | |
| 502 | 0.2500 | 7.0 | 9.0 | 5.0 | 9.0 | 9.0 | 6.0 |
| | 0.1250 | 5.0 | 9.0 | | 7.0 | 5.0 | 5.0 |
| | 0.0620 | 3.0 | 6.0 | 2.0 | 6.0 | 2.0 | 2.0 |
| | 0.0320 | 3.0 | 4.0 | 0.0 | 6.0 | 0.0 | 2.0 |
| | | | | | | | |
| 503 | 0.2500 | 6.0 | 7.0 | 6.0 | 9.0 | 8.0 | 6.0 |
| | 0.1250 | 6.0 | 9.0 | 5.0 | 9.0 | 4.0 | 7.0 |
| | 0.0620 | 4.0 | 6.0 | | 9.0 | 3.0 | 3.0 |
| | 0.0320 | 2.0 | 0.0 | | 7.0 | 0.0 | 1.0 |
| | | | | | | | |
| 504 | 0.2500 | 7.0 | 9.0 | | 9.0 | 7.0 | 5.0 |
| | 0.1250 | 6.0 | 9.0 | 0.0 | 9.0 | | 3.0 |
| | 0.0620 | 3.0 | 7.0 | 0.0 | 8.0 | | |
| | 0.0320 | 2.0 | 4.0 | 0.0 | 6.0 | 0.0 | 3.0 |
| | | | | | | | |
| 505 | 0.2500 | 9.0 | 4.0 | | 8.0 | 4.0 | 6.0 |
| | 0.1250 | 7.0 | 3.0 | 0.0 | 7.0 | 2.0 | 4.0 |
| | 0.0620 | 6.0 | | 0.0 | 7.0 | 2.0 | 4.0 |
| | 0.0320 | 6.0 | 0.0 | | 7.0 | 0.0 | 4.0 |
| | | | | | | | |
| 506 | 0.2500 | 9.0 | 9.0 | 7.0 | 9.0 | 4.5 | 2.0 |
| | 0.1250 | 9.0 | 9.0 | 4.5 | 9.0 | 3.0 | 1.5 |
| | 0.0620 | 9.0 | 8.0 | 4.5 | 9.0 | 3.0 | 1.5 |
| | 0.0320 | 7.5 | 6.5 | 4.5 | 8.0 | 2.5 | 1.5 |
| | | | | | | | |
| 507 | 0.2500 | 7.0 | 9.0 | | 9.0 | 9.0 | 5.0 |
| | 0.1250 | 4.0 | 9.0 | | 9.0 | 9.0 | 7.0 |
| | 0.0620 | 2.0 | 6.0 | | 9.0 | 9.0 | 5.0 |
| | 0.0320 | 2.0 | 5.0 | 0.0 | 9.0 | 7.0 | 3.0 |
| | | | | | | | |
| 508 | 0.2500 | 9.0 | 9.0 | | 9.0 | 9.0 | 6.0 |
| | 0.1250 | 6.0 | 7.0 | | 9.0 | 9.0 | 5.0 |
| | 0.0620 | 5.0 | 7.0 | | 9.0 | 6.0 | 2.0 |
| | 0.0320 | 2.0 | 6.0 | 0.0 | 9.0 | 5.0 | 0.0 |
| | | | | | | | |
| 509 | 0.2500 | 7.0 | 7.0 | 7.0 | 9.0 | 6.0 | 7.0 |
| | 0.1250 | 2.0 | 4.0 | 0.0 | 4.0 | 3.0 | 3.0 |
| | 0.0620 | 0.0 | 0.0 | 0.0 | 0.0 | 0.0 | 0.0 |
| | | | | | | | |
| 510 | 0.2500 | 9.0 | 8.0 | 5.0 | 9.0 | 6.0 | 8.0 |
| | 0.1250 | 9.0 | 7.0 | 3.0 | 9.0 | 5.0 | 4.0 |
| | 0.0620 | 9.0 | 3.0 | 0.0 | 7.0 | 4.0 | 2.0 |
| | 0.0320 | 9.0 | 0.0 | 0.0 | 6.0 | 3.0 | 0.0 |
| | | | | | | | |
| 511 | 0.2500 | 7.0 | 7.0 | 3.0 | | 7.0 | 6.0 |
| | 0.1250 | 5.0 | 2.0 | 3.0 | | 0.0 | 5.0 |
| | 0.0620 | 2.0 | 0.0 | 0.0 | | 0.0 | 4.0 |
| | 0.0320 | 0.0 | 0.0 | 0.0 | | 0.0 | 3.0 |
| | | | | | | | |
| 512 | 0.2500 | 9.0 | 5.0 | 0.0 | 9.0 | 4.0 | 2.0 |
| | 0.1250 | 9.0 | 5.0 | 0.0 | 9.0 | 2.0 | 1.0 |
| | 0.0620 | 7.0 | 2.0 | 0.0 | 9.0 | 0.0 | 0.0 |
| | 0.0320 | 7.0 | 0.0 | 0.0 | 9.0 | 0.0 | 0.0 |
| | | | | | | | |
| 513 | 0.2500 | 8.0 | 9.0 | 9.0 | 9.0 | 9.0 | 9.0 |
| | 0.1250 | 7.0 | 6.0 | 9.0 | 9.0 | 9.0 | 9.0 |
| | 0.0620 | 5.0 | 4.0 | 9.0 | 9.0 | 9.0 | 7.0 |
| | 0.0320 | 0.0 | 4.0 | 9.0 | 9.0 | 9.0 | 9.0 |
| | | | | | | | |
| 514 | 0.2500 | 8.0 | 7.0 | 0.0 | 9.0 | 0.0 | 4.0 |
| | 0.1250 | 2.0 | 2.0 | 0.0 | 9.0 | 0.0 | 1.0 |
| | 0.0620 | 1.0 | 0.0 | 0.0 | 9.0 | 0.0 | 2.0 |
| | 0.0320 | 0.0 | 0.0 | 0.0 | 9.0 | 0.0 | 2.0 |
| | | | | | | | |
| 515 | 0.2500 | 9.0 | 7.0 | 0.0 | 9.0 | 0.0 | 6.0 |
| | 0.1250 | 8.0 | 4.0 | 0.0 | 9.0 | 0.0 | 4.0 |
| | 0.0620 | 6.0 | 2.0 | 0.0 | 9.0 | 0.0 | 2.0 |
| | 0.0320 | 2.0 | 0.0 | 0.0 | 9.0 | 0.0 | 0.0 |
| | | | | | | | |
| 516 | 0.2500 | 0.0 | 2.0 | 0.0 | 9.0 | 0.0 | 3.0 |
| | 0.1250 | 0.0 | 0.0 | 0.0 | 8.0 | 0.0 | 2.0 |
| | 0.0620 | 0.0 | 0.0 | 0.0 | 6.0 | 0.0 | 1.0 |
| | 0.0320 | 0.0 | 0.0 | 0.0 | 4.0 | 0.0 | 0.0 |
| | | | | | | | |
| 517 | 0.2500 | 9.0 | 7.0 | 0.0 | 9.0 | 0.0 | 3.0 |
| | 0.1250 | 8.0 | 5.5 | 4.5 | 9.0 | 0.0 | 1.5 |
| | 0.0620 | 9.0 | 6.5 | 4.5 | 9.0 | 0.0 | 0.0 |
| | 0.0320 | 9.0 | 5.5 | 3.0 | 9.0 | 0.0 | 0.0 |
| | | | | | | | |
| 518 | 0.2500 | 7.0 | 4.0 | 0.0 | 9.0 | 0.0 | 4.0 |
| | 0.1250 | 4.0 | 2.0 | 0.0 | 9.0 | 0.0 | 4.0 |
| | 0.0620 | 2.0 | 2.0 | 0.0 | 7.0 | 0.0 | 2.0 |
| | 0.0320 | 0.0 | 0.0 | 0.0 | 4.0 | 0.0 | 1.0 |
| | | | | | | | |
| 519 | 0.2500 | 9.0 | 6.0 | 0.0 | 9.0 | 0.0 | 4.0 |
| | 0.1250 | 6.5 | 2.0 | 0.0 | 9.0 | 0.0 | 4.5 |
| | 0.0620 | 4.0 | 0.0 | 0.0 | 9.0 | 0.0 | 4.0 |
| | 0.0320 | 2.0 | 0.0 | 0.0 | 9.0 | 0.0 | 2.0 |
| | | | | | | | |
| 520 | 0.2500 | 9.0 | 4.0 | 0.0 | 9.0 | 0.0 | 3.0 |
| | 0.1250 | 8.5 | 2.0 | 0.0 | 9.0 | 0.0 | 4.5 |
| | 0.0620 | 8.0 | 0.0 | 0.0 | 9.0 | 0.0 | 3.5 |
| | 0.0320 | 6.5 | 0.0 | 0.0 | 9.0 | 0.0 | 2.0 |
| | | | | | | | |
| 521 | 0.2500 | 4.0 | 2.0 | 0.0 | 9.0 | 0.0 | 0.0 |
| | 0.1250 | 4.0 | 0.0 | 0.0 | 9.0 | 0.0 | 0.0 |
| | 0.0620 | 2.0 | 0.0 | 0.0 | 9.0 | 0.0 | 0.0 |
| | 0.0320 | 2.0 | 0.0 | 0.0 | 6.0 | 0.0 | 0.0 |
| | | | | | | | |
| 522 | 0.2500 | 9.0 | 2.0 | 0.0 | 9.0 | 0.0 | 4.5 |
| | 0.1250 | 8.0 | 0.0 | 0.0 | 9.0 | 0.0 | 4.0 |
| | 0.0620 | 6.5 | 0.0 | 0.0 | 9.0 | 0.0 | 4.0 |
| | 0.0320 | 5.5 | 0.0 | 0.0 | 9.0 | 0.0 | 2.5 |
| | | | | | | | |
| 523 | 0.2500 | 9.0 | 8.0 | 9.0 | 9.0 | 0.0 | 7.0 |
| | 0.1250 | 8.0 | 4.0 | 2.0 | 9.0 | 0.0 | 5.5 |
| | 0.0620 | 8.0 | 2.0 | 0.0 | 9.0 | 0.0 | 4.5 |
| | 0.0320 | 6.0 | 0.0 | 0.0 | 8.0 | 0.0 | 4.5 |
| | | | | | | | |
| 524 | 0.2500 | 9.0 | 7.0 | 6.0 | 9.0 | 0.0 | 9.0 |
| | 0.1250 | 9.0 | 4.0 | 2.0 | 9.0 | 0.0 | 7.0 |
| | 0.0620 | 9.0 | 2.0 | 0.0 | 9.0 | 0.0 | 4.0 |
| | 0.0320 | 7.0 | 2.0 | 0.0 | 9.0 | 0.0 | 2.0 |
| | | | | | | | |
| 525 | 0.2500 | 9.0 | 4.0 | 0.0 | 9.0 | 0.0 | 4.0 |
| | 0.1250 | 9.0 | 2.0 | 0.0 | 9.0 | 0.0 | 2.0 |
| | 0.0620 | 7.0 | 2.0 | 0.0 | 9.0 | 0.0 | 2.0 |
| | 0.0320 | 6.0 | 0.0 | 0.0 | 9.0 | 0.0 | 0.0 |
| | | | | | | | |
| 526 | 0.2500 | 6.0 | 9.0 | 0.0 | 9.0 | 0.0 | 3.0 |
| | 0.1250 | 4.0 | 7.0 | 0.0 | 9.0 | 0.0 | 2.0 |
| | 0.0620 | 2.0 | 4.0 | 0.0 | 9.0 | 0.0 | 2.0 |
| | 0.0320 | 2.0 | 2.0 | 0.0 | 8.0 | 0.0 | 1.0 |
| | | | | | | | |
| 527 | 0.2500 | 9.0 | 4.0 | 0.0 | 9.0 | 0.0 | 4.0 |
| | 0.1250 | 9.0 | 4.0 | 0.0 | 9.0 | 0.0 | 2.5 |
| | 0.0620 | 8.0 | 2.0 | 0.0 | 9.0 | 0.0 | 2.5 |
| | 0.0320 | 3.0 | 2.0 | 0.0 | 9.0 | 0.0 | 2.0 |
| | | | | | | | |
| 528 | 0.2500 | 9.0 | 9.0 | 0.0 | 9.0 | 3.0 | 9.0 |
| | 0.1250 | 9.0 | 5.0 | 0.0 | 9.0 | 1.5 | 7.0 |
| | 0.0620 | 9.0 | 5.0 | 0.0 | 9.0 | 0.0 | 3.5 |
| | 0.0320 | 9.0 | 4.5 | 0.0 | 9.0 | 1.0 | 1.5 |
| | | | | | | | |
| 529 | 0.2500 | 9.0 | 0.0 | 0.0 | 0.0 | 9.0 | 9.0 |
| | 0.1250 | 9.0 | 3.0 | 0.0 | 3.0 | 9.0 | 3.0 |
| | 0.0620 | 9.0 | 3.0 | 0.0 | 3.0 | 9.0 | 0.0 |
| | 0.0320 | 7.0 | 3.0 | 0.0 | 3.0 | 9.0 | 0.0 |
| | | | | | | | |
| 530 | 0.2500 | 9.0 | 9.0 | 0.0 | 9.0 | 9.0 | 9.0 |
| | 0.1250 | 9.0 | 9.0 | 0.0 | 9.0 | 3.0 | 3.0 |
| | 0.0620 | 9.0 | 9.0 | 0.0 | 9.0 | 0.0 | 0.0 |
| | 0.0320 | 9.0 | 0.0 | 0.0 | 9.0 | 0.0 | 0.0 |
| | | | | | | | |
| 531 | 0.2500 | 9.0 | 4.0 | 3.0 | 9.0 | 3.0 | 9.0 |
| | 0.1250 | 9.0 | 4.0 | 3.0 | 9.0 | 3.0 | 3.0 |
| | 0.0620 | 9.0 | 0.0 | 0.0 | 9.0 | 0.0 | 0.0 |
| | 0.0320 | 9.0 | 0.0 | 0.0 | 9.0 | 0.0 | 0.0 |
| | | | | | | | |
| 532 | 0.2500 | 9.0 | 9.0 | 0.0 | 9.0 | 9.0 | 2.0 |
| | 0.1250 | 9.0 | 9.0 | 0.0 | 9.0 | 0.0 | 0.0 |
| | 0.0620 | 9.0 | 9.0 | 0.0 | 9.0 | 0.0 | 0.0 |
| | 0.0320 | 9.0 | 9.0 | 0.0 | 9.0 | 0.0 | 0.0 |
| | | | | | | | |
| 533 | 0.2500 | 9.0 | 9.0 | 9.0 | 9.0 | 4.0 | 8.0 |
| | 0.1250 | 9.0 | 4.0 | 3.0 | 9.0 | 0.0 | 5.0 |
| | 0.0620 | 9.0 | 3.0 | 0.0 | 9.0 | 0.0 | 4.5 |
| | 0.0320 | 7.5 | 2.0 | 0.0 | 9.0 | 0.0 | 3.0 |
| | | | | | | | |
| 534 | 0.2500 | 9.0 | 9.0 | 0.0 | 9.0 | 0.0 | 4.5 |
| | 0.1250 | 9.0 | 3.0 | 0.0 | 9.0 | 0.0 | 4.0 |
| | 0.0620 | 4.5 | 3.0 | 0.0 | 9.0 | 0.0 | 4.0 |
| | 0.0320 | 3.5 | 0.0 | 0.0 | 4.0 | 0.0 | 2.0 |
| | | | | | | | |
| 535 | 0.2500 | 4.0 | 9.0 | 5.0 | 9.0 | 3.0 | 9.0 |
| | 0.1250 | 3.0 | 9.0 | 5.0 | 9.0 | 3.0 | 4.0 |
| | 0.0620 | 3.0 | 6.0 | 3.0 | 9.0 | 0.0 | 3.0 |
| | 0.0320 | 0.0 | 0.0 | 0.0 | 6.0 | 0.0 | 0.0 |
| | | | | | | | |
| 536 | 0.2500 | 9.0 | 4.0 | 0.0 | 9.0 | 0.0 | 4.0 |
| | 0.1250 | 5.0 | 0.0 | 0.0 | 9.0 | 0.0 | 1.0 |
| | 0.0620 | 4.0 | 0.0 | 0.0 | 9.0 | 0.0 | 0.0 |
| | 0.0320 | 3.0 | 0.0 | 0.0 | 9.0 | 0.0 | 0.0 |
| | | | | | | | |
| 537 | 0.5000 | 9.0 | 9.0 | 0.0 | 9.0 | 0.0 | 0.0 |
| | 0.2500 | 9.0 | 4.0 | 0.0 | 9.0 | 0.0 | 0.0 |
| | 0.1250 | 5.0 | 1.0 | 0.0 | 9.0 | 0.0 | 0.0 |
| | 0.0620 | 4.4 | 0.0 | 0.0 | 9.0 | 0.0 | 0.0 |
| | 0.0320 | 3.4 | 0.0 | 0.0 | 9.0 | 0.0 | 0.0 |
| | | | | | | | |
| 538 | 0.5000 | 9.0 | 9.0 | 0.0 | 9.0 | 0.0 | 1.0 |
| | 0.2500 | 9.0 | 0.0 | 0.0 | 9.0 | 0.0 | 1.0 |
| | 0.1250 | 4.7 | 0.0 | 0.0 | 9.0 | 0.0 | 1.0 |
| | 0.0620 | 3.4 | 0.0 | 0.0 | 9.0 | 0.0 | 0.0 |
| | 0.0320 | 2.1 | 0.0 | 0.0 | 9.0 | 0.0 | 0.0 |
| | | | | | | | |
| 539 | 0.5000 | 9.0 | 9.0 | 2.0 | 9.0 | 9.0 | 5.0 |
| | 0.2500 | 7.0 | 8.0 | 0.0 | 9.0 | 4.0 | 1.0 |
| | 0.1250 | 4.7 | 6.0 | 0.0 | 9.0 | 1.0 | 1.0 |
| | 0.0620 | 3.0 | 2.0 | 0.0 | 9.0 | 0.0 | 1.0 |
| | 0.0320 | 2.1 | 0.0 | 0.0 | 8.0 | 0.0 | 0.0 |
| | | | | | | | |
| 540 | 0.5000 | 9.0 | 9.0 | 3.0 | 9.0 | 0.0 | 3.0 |
| | 0.2500 | 9.0 | 9.0 | 0.0 | 9.0 | 0.0 | 1.0 |
| | 0.1250 | 4.7 | 9.0 | 0.0 | 9.0 | 0.0 | 0.0 |
| | 0.0620 | 3.4 | 8.0 | 0.0 | 9.0 | 0.0 | 0.0 |
| | 0.0320 | 2.3 | 6.0 | 0.0 | 8.0 | 0.0 | 0.0 |
| | | | | | | | |
| 541 | 0.5000 | 6.0 | 9.0 | 0.0 | 9.0 | 0.0 | 2.0 |
| | 0.2500 | 4.0 | 6.0 | 0.0 | 9.0 | 0.0 | 0.0 |
| | 0.1250 | 0.0 | 4.0 | 0.0 | 9.0 | 0.0 | 0.0 |
| | 0.0620 | 0.0 | 3.0 | 0.0 | 9.0 | 0.0 | 0.0 |
| | | | | | | | |
| 542 | 0.5000 | 9.0 | 9.0 | 0.0 | 9.0 | 9.0 | 2.0 |
| | 0.2500 | 8.0 | 4.0 | | 9.0 | 0.0 | 0.0 |
| | 0.1250 | 4.3 | 2.0 | 0.0 | 9.0 | 0.0 | 0.0 |
| | 0.0620 | 2.7 | 0.0 | 0.0 | 9.0 | 0.0 | 0.0 |
| | 0.0320 | 1.7 | 0.0 | 0.0 | 7.0 | 0.0 | 0.0 |
| | | | | | | | |
| 543 | 0.5000 | 9.0 | 3.0 | 0.0 | 9.0 | 0.0 | 2.0 |
| | 0.2500 | 7.0 | 8.0 | 0.0 | 9.0 | 0.0 | 2.0 |
| | 0.1250 | 4.0 | 7.0 | 0.0 | 9.0 | 0.0 | 1.0 |
| | 0.0620 | 2.9 | 6.0 | 0.0 | 9.0 | 0.0 | 0.0 |
| | 0.0320 | 2.1 | 2.0 | 0.0 | 9.0 | 0.0 | 0.0 |
| | | | | | | | |
| 544 | 0.5000 | 9.0 | 9.0 | 4.0 | 9.0 | 9.0 | 8.0 |
| | 0.2500 | 9.0 | 9.0 | 9.0 | 9.0 | 9.0 | 9.0 |
| | 0.1250 | 6.4 | 9.0 | 7.0 | 9.0 | 8.0 | 9.0 |
| | 0.0620 | 5.6 | 9.0 | | 9.0 | 4.0 | 9.0 |
| | 0.0320 | 5.4 | 9.0 | 9.0 | 9.0 | 7.0 | 4.0 |
| | | | | | | | |
| 545 | 0.5000 | 9.0 | 9.0 | 6.0 | 9.0 | 9.0 | 9.0 |
| | 0.2500 | 9.0 | 7.0 | 9.0 | 9.0 | 7.0 | 9.0 |
| | 0.1250 | 7.0 | 6.0 | 9.0 | 9.0 | 4.0 | 4.0 |
| | 0.0620 | 6.6 | 5.0 | 0.0 | 7.0 | 0.0 | 2.0 |
| | 0.0320 | 5.6 | 4.0 | 0.0 | 6.0 | 0.0 | 2.0 |
| | | | | | | | |
| 546 | 0.5000 | 9.0 | 2.0 | 0.0 | 9.0 | 9.0 | 6.0 |
| | 0.2500 | 9.0 | 9.0 | 9.0 | 9.0 | 9.0 | 7.0 |
| | 0.1250 | 4.6 | 6.0 | 9.0 | 7.0 | 4.0 | 4.0 |
| | 0.0620 | 4.3 | 4.0 | 0.0 | 7.0 | 0.0 | 3.0 |
| | 0.0320 | 4.1 | 3.0 | 0.0 | 6.0 | 0.0 | 2.0 |
| | | | | | | | |
| 547 | 0.5000 | 9.0 | 9.0 | 0.0 | 9.0 | 3.0 | 3.0 |
| | 0.2500 | 9.0 | 9.0 | 7.0 | 9.0 | 7.0 | 4.0 |
| | 0.1250 | 5.6 | 9.0 | 8.0 | 9.0 | 4.0 | 4.0 |
| | 0.0620 | 5.4 | 7.0 | 8.0 | 9.0 | 4.0 | 3.0 |
| | 0.0320 | 5.4 | 7.0 | 6.0 | 9.0 | 2.0 | 2.0 |
| | | | | | | | |
| 548 | 0.5000 | 6.0 | 0.0 | 9.0 | 9.0 | 0.0 | 4.0 |
| | 0.2500 | 9.0 | 9.0 | 9.0 | 9.0 | 4.0 | 6.0 |
| | 0.1250 | 4.9 | 8.0 | 9.0 | 9.0 | 2.0 | 4.0 |
| | 0.0620 | 3.7 | 6.0 | 9.0 | 9.0 | 0.0 | 2.0 |
| | 0.0320 | 2.9 | 2.0 | | 8.0 | 0.0 | 3.0 |
| | | | | | | | |
| 549 | 0.5000 | 9.0 | 9.0 | 9.0 | 9.0 | 0.0 | 2.0 |
| | 0.2500 | 9.0 | 9.0 | 9.0 | 9.0 | 4.0 | 4.0 |
| | 0.1250 | 5.1 | 9.0 | 2.0 | 9.0 | 4.0 | 4.0 |
| | 0.0620 | 3.6 | 9.0 | 0.0 | 9.0 | 0.0 | 4.0 |
| | 0.0320 | 3.0 | 7.0 | 0.0 | 9.0 | 0.0 | 4.0 |
| | | | | | | | |
| 550 | 0.5000 | 9.0 | 5.0 | 9.0 | 9.0 | 0.0 | 0.0 |
| | 0.2500 | 9.0 | 7.0 | 0.0 | 9.0 | 0.0 | 0.0 |
| | 0.1250 | 5.9 | 8.0 | 0.0 | 9.0 | 0.0 | 0.0 |
| | 0.0620 | 5.9 | 8.0 | 0.0 | 9.0 | 0.0 | 0.0 |
| | 0.0320 | 4.7 | 2.0 | 0.0 | 9.0 | 0.0 | 0.0 |
| | | | | | | | |
| 551 | 0.5000 | 9.0 | 9.0 | 0.0 | 9.0 | 2.0 | 4.0 |
| | 0.2500 | 7.0 | 2.0 | 0.0 | 9.0 | 0.0 | 3.0 |
| | 0.1250 | 7.0 | 0.0 | 0.0 | 9.0 | 0.0 | 3.0 |
| | 0.0620 | 4.0 | 0.0 | 0.0 | 0.0 | 0.0 | 0.0 |
| | | | | | | | |
| 552 | 0.5000 | 9.0 | 9.0 | 9.0 | 9.0 | 5.0 | 4.0 |
| | 0.2500 | 9.0 | 9.0 | 9.0 | 9.0 | 0.0 | 7.0 |
| | 0.1250 | 4.7 | 7.0 | 4.0 | 9.0 | 0.0 | 6.0 |
| | 0.0620 | 3.7 | 8.0 | | 8.0 | 0.0 | 6.0 |
| | 0.0320 | 2.6 | 6.0 | 0.0 | 6.0 | 0.0 | 4.0 |
| | | | | | | | |
| 553 | 0.5000 | 7.0 | 4.0 | 9.0 | 9.0 | 0.0 | 4.0 |
| | 0.2500 | 9.0 | 9.0 | 2.0 | 9.0 | 0.0 | 6.0 |
| | 0.1250 | 6.4 | 7.0 | | 9.0 | 0.0 | 4.0 |
| | 0.0620 | 2.1 | 6.0 | 0.0 | 9.0 | 0.0 | 4.0 |
| | 0.0320 | 1.3 | 2.0 | 0.0 | 7.0 | 0.0 | 3.0 |
| | | | | | | | |
| 554 | 0.5000 | 7.0 | 5.0 | 9.0 | 9.0 | 0.0 | 6.0 |
| | 0.2500 | 9.0 | 9.0 | 9.0 | 9.0 | 9.0 | 7.0 |
| | 0.1250 | 5.7 | 9.0 | 4.0 | 9.0 | 0.0 | 6.0 |
| | 0.0620 | 4.1 | 7.0 | 0.0 | 9.0 | 0.0 | 4.0 |
| | 0.0320 | 3.0 | 2.0 | 0.0 | 9.0 | 0.0 | 4.0 |
| | | | | | | | |
| 555 | 0.5000 | 9.0 | 3.0 | 9.0 | 9.0 | 0.0 | 3.0 |
| | 0.2500 | 9.0 | 9.0 | 8.0 | 9.0 | 7.0 | 9.0 |
| | 0.1250 | 4.6 | 9.0 | 8.0 | 9.0 | 2.0 | 8.0 |
| | 0.0620 | 2.3 | 6.0 | 6.0 | 9.0 | 0.0 | 6.0 |
| | 0.0320 | 1.6 | 4.0 | 4.0 | 9.0 | 0.0 | 6.0 |
| | | | | | | | |
| 556 | 0.5000 | 9.0 | 3.0 | 0.0 | 9.0 | 3.0 | 5.0 |
| | 0.2500 | 9.0 | 9.0 | 9.0 | 9.0 | 4.0 | 9.0 |
| | 0.1250 | 4.4 | 7.0 | 9.0 | 9.0 | 6.0 | 8.0 |
| | 0.0620 | 3.6 | 2.0 | 2.0 | 9.0 | 0.0 | 7.0 |
| | 0.0320 | 2.9 | 0.0 | 2.0 | 9.0 | 0.0 | 4.0 |
| | | | | | | | |
| 557 | 0.5000 | 9.0 | 9.0 | 5.0 | 9.0 | 4.0 | 9.0 |
| | 0.2500 | 9.0 | 9.0 | 9.0 | 9.0 | 4.0 | 9.0 |
| | 0.1250 | 6.3 | 9.0 | 6.0 | 9.0 | 2.0 | 8.0 |
| | 0.0620 | 5.1 | 8.0 | | 9.0 | 0.0 | 8.0 |
| | 0.0320 | 4.3 | 4.0 | 0.0 | 9.0 | 0.0 | 6.0 |
| | | | | | | | |
| 558 | 0.5000 | 9.0 | 9.0 | 9.0 | 9.0 | 4.0 | 9.0 |
| | 0.2500 | 9.0 | 9.0 | 9.0 | 9.0 | 8.0 | 9.0 |
| | 0.1250 | 7.0 | 8.0 | 9.0 | 9.0 | 4.0 | 9.0 |
| | 0.0620 | 4.7 | 6.0 | | 9.0 | 2.0 | 7.0 |
| | 0.0320 | 4.3 | 2.0 | | 9.0 | 2.0 | 4.0 |
| | | | | | | | |
| 559 | 0.5000 | 9.0 | 4.0 | 9.0 | 9.0 | 0.0 | 2.0 |
| | 0.2500 | 6.0 | 0.0 | 9.0 | 9.0 | 0.0 | 2.0 |
| | 0.1250 | 3.0 | 0.0 | 0.0 | 9.0 | 0.0 | 1.0 |
| | 0.0620 | 3.0 | 0.0 | 0.0 | 9.0 | 0.0 | 1.0 |
| | | | | | | | |
| 560 | 0.5000 | 7.0 | 3.0 | 9.0 | 9.0 | 0.0 | 2.0 |
| | 0.2500 | 9.0 | 9.0 | 9.0 | 9.0 | 0.0 | 4.0 |
| | 0.1250 | 4.6 | 7.0 | 7.0 | 9.0 | 0.0 | 2.0 |
| | 0.0620 | 3.0 | 2.0 | 2.0 | 9.0 | 0.0 | 3.0 |
| | 0.0320 | 2.0 | 0.0 | 0.0 | 8.0 | 0.0 | 3.0 |
| | | | | | | | |
| 561 | 0.5000 | 9.0 | 6.0 | 9.0 | 9.0 | 0.0 | 2.0 |
| | 0.2500 | 9.0 | 9.0 | 0.0 | 9.0 | 3.0 | 4.5 |
| | 0.1250 | 6.4 | 8.5 | 0.0 | 9.0 | 1.0 | 3.5 |
| | 0.0620 | 4.8 | 6.0 | 0.0 | 9.0 | 0.0 | 4.0 |
| | 0.0320 | 3.6 | 3.0 | 0.0 | 9.0 | 0.0 | 2.5 |
| | | | | | | | |
| 562 | 0.5000 | 9.0 | 3.0 | 9.0 | 9.0 | 0.0 | 6.0 |
| | 0.2500 | 9.0 | 9.0 | 9.0 | 9.0 | 7.0 | 9.0 |
| | 0.1250 | 5.1 | 9.0 | 9.0 | 9.0 | 5.0 | 4.0 |
| | 0.0620 | 5.1 | 7.0 | 4.0 | 9.0 | 0.0 | 3.0 |
| | 0.0320 | 2.7 | 2.0 | | 9.0 | 0.0 | 2.0 |
| | | | | | | | |
| 563 | 0.5000 | 9.0 | 9.0 | 0.0 | 9.0 | 4.0 | 4.0 |
| | 0.2500 | 9.0 | 6.0 | 4.0 | 9.0 | 2.0 | 7.0 |
| | 0.1250 | 5.6 | 6.0 | 0.0 | 9.0 | 0.0 | 7.0 |
| | 0.0620 | 5.1 | 2.0 | 0.0 | 9.0 | 0.0 | 7.0 |
| | 0.0320 | 3.6 | 0.0 | 0.0 | 8.0 | 0.0 | 4.0 |
| | | | | | | | |
| 564 | 0.2500 | 7.0 | 9.0 | 4.0 | 9.0 | 0.0 | 0.0 |
| | 0.1250 | 5.0 | 7.0 | 0.0 | 9.0 | 0.0 | 0.0 |
| | 0.0620 | 4.0 | 6.0 | 0.0 | 9.0 | 0.0 | 0.0 |
| | 0.0320 | 4.0 | 6.0 | 0.0 | 9.0 | 0.0 | 0.0 |
| | | | | | | | |
| 565 | 0.2500 | 0.0 | 6.0 | 0.0 | 9.0 | 0.0 | 3.0 |
| | 0.1250 | 0.0 | 6.0 | 0.0 | 9.0 | 0.0 | 2.0 |
| | 0.0620 | 0.0 | 3.0 | 0.0 | 9.0 | 0.0 | 0.0 |
| | 0.0320 | 0.0 | 0.0 | 0.0 | 9.0 | 0.0 | 0.0 |
| | | | | | | | |
| 566 | 0.2500 | 6.0 | 9.0 | 3.0 | 9.0 | 9.0 | 6.0 |
| | 0.1250 | 3.0 | 0.0 | 0.0 | 9.0 | 0.0 | 5.0 |
| | 0.0620 | 3.0 | 0.0 | 0.0 | 9.0 | 0.0 | 3.0 |
| | 0.0320 | 3.0 | 0.0 | 0.0 | 9.0 | 0.0 | 2.0 |
| | | | | | | | |
| 567 | 0.2500 | 4.0 | 9.0 | 0.0 | 9.0 | 0.0 | 2.0 |
| | 0.1250 | 2.0 | 4.0 | 0.0 | 9.0 | 0.0 | 1.0 |
| | 0.0620 | 0.0 | 9.0 | 0.0 | 3.0 | 0.0 | 0.0 |
| | 0.0320 | 0.0 | 4.0 | 0.0 | 0.0 | 0.0 | 0.0 |
| | | | | | | | |
| 568 | 0.2500 | 6.0 | 7.0 | 0.0 | 9.0 | 0.0 | 3.0 |
| | 0.1250 | 5.0 | 5.0 | 0.0 | 9.0 | 0.0 | 1.0 |
| | 0.0620 | 3.0 | 0.0 | 0.0 | 9.0 | 0.0 | 0.0 |
| | 0.0320 | 0.0 | 0.0 | 0.0 | 0.0 | 0.0 | 0.0 |
| | | | | | | | |
| 569 | 0.2500 | 5.0 | 0.0 | 0.0 | 6.0 | 9.0 | 1.0 |
| | 0.1250 | 5.0 | 0.0 | 0.0 | 6.0 | 0.0 | 0.0 |
| | 0.0620 | 4.0 | 0.0 | 0.0 | 6.0 | 0.0 | 0.0 |
| | 0.0320 | 2.0 | 0.0 | 0.0 | 4.0 | 0.0 | 0.0 |
| | | | | | | | |
| 570 | 0.2500 | 6.0 | 9.0 | 0.0 | 9.0 | 0.0 | 3.0 |
| | 0.1250 | 4.0 | 0.0 | 0.0 | 0.0 | 0.0 | 2.0 |
| | 0.0620 | 2.0 | 0.0 | 0.0 | 0.0 | 0.0 | 1.0 |
| | 0.0320 | 0.0 | 0.0 | 0.0 | 0.0 | 0.0 | 0.0 |
| | | | | | | | |
| 571 | 0.2500 | 6.0 | 9.0 | 0.0 | 9.0 | 0.0 | 2.0 |
| | 0.1250 | 3.0 | 3.0 | 0.0 | 7.0 | 0.0 | 1.0 |
| | 0.0620 | 2.0 | 3.0 | 0.0 | 6.0 | 0.0 | 0.0 |
| | 0.0320 | 0.0 | 0.0 | 0.0 | 0.0 | 0.0 | 0.0 |
| | | | | | | | |
| 572 | 0.2500 | 6.0 | 5.0 | 2.0 | 9.0 | 3.0 | 6.0 |
| | 0.1250 | 2.0 | 2.0 | 0.0 | 6.0 | 0.0 | 3.0 |
| | 0.0620 | 0.0 | 0.0 | 0.0 | 6.0 | 0.0 | 1.0 |
| | 0.0320 | 0.0 | 0.0 | 0.0 | 0.0 | 0.0 | 0.0 |
| | | | | | | | |
| 573 | 0.2500 | 7.0 | 5.0 | 4.0 | 9.0 | 2.0 | 1.0 |
| | 0.1250 | 7.0 | 5.0 | 0.0 | 9.0 | 0.0 | 0.0 |
| | 0.0620 | 7.0 | 3.0 | 0.0 | 9.0 | 0.0 | 0.0 |
| | 0.0320 | 6.0 | 0.0 | 0.0 | 9.0 | 0.0 | 0.0 |
| | | | | | | | |
| 574 | 0.2500 | 9.0 | 7.0 | 6.5 | 9.0 | 6.0 | 8.0 |
| | 0.1250 | 8.5 | 6.0 | 4.5 | 9.0 | 2.0 | 6.5 |
| | 0.0620 | 6.5 | 5.5 | 2.0 | 9.0 | 1.0 | 4.5 |
| | 0.0320 | 4.0 | 2.0 | 0.0 | 9.0 | 0.0 | 3.0 |
| | | | | | | | |
| 575 | 0.2500 | 9.0 | 6.0 | 2.0 | 9.0 | 3.0 | 2.0 |
| | 0.1250 | 6.0 | 4.0 | 0.0 | 9.0 | 0.0 | 0.0 |
| | 0.0620 | 5.0 | 0.0 | 0.0 | 4.0 | 0.0 | 0.0 |
| | 0.0320 | 2.0 | 0.0 | 0.0 | 4.0 | 0.0 | 0.0 |
| | | | | | | | |
| 576 | 0.2500 | 9.0 | 5.0 | 4.0 | 9.0 | 4.0 | 9.0 |
| | 0.1250 | 7.0 | 3.0 | 0.0 | 9.0 | 3.0 | 7.0 |
| | 0.0620 | 7.0 | 3.0 | 0.0 | 9.0 | 0.0 | 7.0 |
| | 0.0320 | 6.0 | 0.0 | 0.0 | 7.0 | 0.0 | 4.0 |
| | | | | | | | |
| 577 | 0.2500 | 9.0 | 9.0 | 3.0 | | 6.0 | 9.0 |
| | 0.1250 | 7.0 | 7.0 | 3.0 | | 6.0 | 5.0 |
| | 0.0620 | 7.0 | 4.0 | 3.0 | | 6.0 | 5.0 |
| | 0.0320 | 6.0 | 2.0 | 0.0 | | 4.0 | 4.0 |
| | | | | | | | |
| 578 | 0.2500 | 9.0 | 7.0 | 9.0 | | 9.0 | 7.0 |
| | 0.1250 | 7.0 | 5.0 | 6.0 | | 6.0 | 6.0 |
| | 0.0620 | 6.0 | 5.0 | 6.0 | | 6.0 | 5.0 |
| | 0.0320 | 5.0 | 4.0 | 3.0 | | 3.0 | 4.0 |
| | | | | | | | |
| 579 | 0.2500 | 5.0 | 6.0 | 0.0 | 9.0 | 2.0 | 3.0 |
| | 0.1250 | 4.0 | 4.0 | 0.0 | 7.0 | 0.0 | 3.0 |
| | 0.0620 | 4.0 | 0.0 | 0.0 | 7.0 | 0.0 | 2.0 |
| | 0.0320 | 2.0 | 0.0 | 0.0 | 6.0 | 0.0 | 0.0 |
| | | | | | | | |
| 580 | 0.2500 | 9.0 | 9.0 | 8.5 | 9.0 | 8.5 | 6.5 |
| | 0.1250 | 9.0 | 9.0 | 9.0 | 9.0 | 8.0 | 4.0 |
| | 0.0620 | 9.0 | 9.0 | 9.0 | 9.0 | 8.0 | 4.0 |
| | 0.0320 | 8.5 | 3.0 | 5.5 | 4.5 | 5.5 | 1.5 |
| | | | | | | | |
| 581 | 0.2500 | 9.0 | 9.0 | 8.0 | 9.0 | 9.0 | 7.0 |
| | 0.1250 | 9.0 | 9.0 | 6.0 | 9.0 | 9.0 | 6.0 |
| | 0.0620 | 8.5 | 4.5 | 1.5 | 4.5 | 9.0 | 3.5 |
| | 0.0320 | 6.0 | 3.0 | 1.0 | 4.5 | 3.0 | 1.5 |
| | | | | | | | |
| 582 | 0.2500 | 9.0 | 6.0 | 7.0 | 7.0 | 6.0 | 3.0 |
| | 0.1250 | 7.0 | 6.0 | 0.0 | 7.0 | 6.0 | 0.0 |
| | 0.0620 | 7.0 | 4.0 | 0.0 | 7.0 | 5.0 | 0.0 |
| | 0.0320 | 6.0 | 3.0 | 0.0 | 7.0 | 5.0 | 0.0 |
| | | | | | | | |
| 583 | 0.2500 | 9.0 | 9.0 | 9.0 | 9.0 | 9.0 | 9.0 |
| | 0.1250 | 9.0 | 9.0 | 9.0 | 9.0 | 9.0 | 9.0 |
| | 0.0620 | 7.5 | 9.0 | 9.0 | 9.0 | 9.0 | 7.0 |
| | 0.0320 | 3.0 | 9.0 | 8.5 | 6.0 | 9.0 | 5.0 |
| | | | | | | | |
| 584 | 0.2500 | 9.0 | 9.0 | 0.0 | 9.0 | 2.0 | 3.5 |
| | 0.1250 | 9.0 | 9.0 | | 9.0 | 4.0 | 1.0 |
| | 0.0620 | 9.0 | 6.0 | 0.0 | 8.0 | 1.5 | 1.0 |
| | 0.0320 | 6.0 | 1.5 | 0.0 | 3.0 | 0.0 | 0.0 |
| | | | | | | | |
| 585 | 0.2500 | 9.0 | 9.0 | 3.0 | 9.0 | 2.5 | 1.5 |
| | 0.1250 | 8.5 | 9.0 | 0.0 | 4.5 | 2.0 | 1.5 |
| | 0.0620 | 8.5 | 8.5 | 0.0 | 3.5 | 1.5 | 1.5 |
| | 0.0320 | 5.0 | 2.5 | 0.0 | 3.5 | 0.0 | 1.0 |
| | | | | | | | |
| 586 | 0.2500 | 9.0 | 9.0 | 4.5 | 9.0 | 0.0 | 2.0 |
| | 0.1250 | 9.0 | 9.0 | 4.5 | 9.0 | 0.0 | 2.0 |
| | 0.0620 | 9.0 | 8.5 | 0.0 | 6.5 | 0.0 | 2.0 |
| | 0.0320 | 8.0 | 8.0 | 0.0 | 9.0 | 0.0 | 1.0 |
| | | | | | | | |
| 587 | 0.2500 | 9.0 | 9.0 | | 9.0 | 5.0 | 5.0 |
| | 0.1250 | 9.0 | 9.0 | 0.0 | 9.0 | 5.0 | 4.0 |
| | 0.0620 | 7.0 | 8.0 | | 5.0 | 2.0 | 4.0 |
| | 0.0320 | 7.0 | 4.0 | 0.0 | 5.0 | 0.0 | 4.0 |
| | | | | | | | |
| 588 | 0.2500 | 7.0 | 6.0 | 5.0 | 8.0 | 0.0 | 3.0 |
| | 0.1250 | 9.0 | 6.0 | 5.0 | 7.0 | 0.0 | 3.0 |
| | 0.0620 | 7.0 | 2.0 | 0.0 | | 0.0 | 3.0 |
| | 0.0320 | 4.0 | 0.0 | 0.0 | | 0.0 | 0.0 |
| | | | | | | | |
| 589 | 0.2500 | 4.0 | 4.0 | | 9.0 | 3.0 | 0.0 |
| | 0.1250 | 4.0 | 3.0 | 0.0 | 8.0 | 2.0 | 0.0 |
| | 0.0620 | 4.0 | 2.0 | 0.0 | 8.0 | 2.0 | 0.0 |
| | 0.0320 | 4.0 | 0.0 | 0.0 | 7.0 | 0.0 | 0.0 |
| | | | | | | | |
| 590 | 0.2500 | 9.0 | 9.0 | 5.0 | 9.0 | 9.0 | 6.0 |
| | 0.1250 | 6.0 | 7.0 | | 7.0 | 8.0 | 5.0 |
| | 0.0620 | 5.0 | 7.0 | | 7.0 | 4.0 | 5.0 |
| | 0.0320 | 3.0 | 2.0 | 0.0 | | 3.0 | 3.0 |
| | | | | | | | |
| 591 | 0.2500 | 9.0 | 7.0 | | 9.0 | 7.0 | 5.0 |
| | 0.1250 | 7.0 | 7.0 | 0.0 | 9.0 | 7.0 | 4.0 |
| | 0.0620 | 3.0 | 4.0 | | 9.0 | 3.0 | 4.0 |
| | 0.0320 | 3.0 | 3.0 | 0.0 | 7.0 | 0.0 | 2.0 |
| | | | | | | | |
| 592 | 0.2500 | 5.0 | 6.0 | | 9.0 | | 4.0 |
| | 0.1250 | 4.0 | 4.0 | 0.0 | 9.0 | 3.0 | 4.0 |
| | 0.0620 | 3.0 | 3.0 | | 9.0 | 3.0 | 2.0 |
| | 0.0320 | 0.0 | 2.0 | 0.0 | 8.0 | 0.0 | 2.0 |
| | | | | | | | |
| 593 | 0.2500 | 0.0 | 5.0 | 0.0 | 7.0 | 0.0 | 0.0 |
| | 0.1250 | 0.0 | 4.0 | 0.0 | 7.0 | 0.0 | 0.0 |
| | 0.0620 | 0.0 | 3.0 | | 7.0 | 0.0 | 0.0 |
| | 0.0320 | 0.0 | 3.0 | | | 0.0 | 0.0 |
| | | | | | | | |
| 594 | 0.2500 | 7.0 | 9.0 | | 9.0 | | 3.0 |
| | 0.1250 | 6.0 | 5.0 | | 9.0 | 5.0 | 2.0 |
| | 0.0620 | 5.0 | 5.0 | | 7.0 | 4.0 | 2.0 |
| | 0.0320 | 3.0 | 3.0 | 0.0 | 6.0 | 2.0 | 1.0 |
| | | | | | | | |
| 595 | 0.2500 | 4.0 | 4.0 | | | 0.0 | 2.0 |
| | 0.1250 | 3.0 | 2.0 | 0.0 | | 0.0 | 0.0 |
| | 0.0620 | 0.0 | 0.0 | | | 0.0 | 0.0 |
| | | | | | | | |
| 596 | 0.2500 | 7.0 | 0.0 | 0.0 | 9.0 | 6.0 | 6.0 |
| | 0.1250 | 5.0 | 0.0 | 0.0 | 7.0 | 2.0 | 4.0 |
| | 0.0620 | 4.0 | 0.0 | 0.0 | 4.0 | 0.0 | 3.0 |
| | 0.0320 | 0.0 | 0.0 | 0.0 | 3.0 | 0.0 | 0.0 |
| | | | | | | | |
| 597 | 0.2500 | 9.0 | 0.0 | 0.0 | 4.0 | 0.0 | 0.0 |
| | 0.1250 | 7.0 | 0.0 | 0.0 | | 0.0 | 0.0 |
| | 0.0620 | 6.0 | 0.0 | 0.0 | 4.0 | 0.0 | 0.0 |
| | 0.0320 | 4.0 | 0.0 | 0.0 | | 0.0 | 0.0 |
| | | | | | | | |
| 598 | 0.2500 | 0.0 | 3.0 | 0.0 | 7.0 | 0.0 | 3.0 |
| | 0.1250 | 0.0 | 2.0 | | 7.0 | 0.0 | 3.0 |
| | 0.0620 | 0.0 | 0.0 | | | 0.0 | 0.0 |
| | 0.0320 | 0.0 | 0.0 | 0.0 | | 0.0 | 0.0 |
| | | | | | | | |
| 599 | 0.2500 | 4.0 | 3.0 | 0.0 | 7.0 | 0.0 | 5.0 |
| | 0.1250 | 0.0 | | | | 0.0 | 3.0 |
| | 0.0620 | 0.0 | 0.0 | 0.0 | 7.0 | 0.0 | 3.0 |
| | 0.0320 | 0.0 | 0.0 | | 6.0 | 0.0 | 2.0 |
| | | | | | | | |
| 600 | 0.2500 | 2.0 | 2.0 | 0.0 | 7.0 | 0.0 | 4.0 |
| | 0.1250 | 0.0 | 0.0 | | 7.0 | 0.0 | 4.0 |
| | 0.0620 | 0.0 | 0.0 | 0.0 | 6.0 | 0.0 | 3.0 |
| | 0.0320 | 0.0 | 0.0 | 0.0 | 6.0 | 0.0 | 3.0 |
| | | | | | | | |
| 601 | 0.2500 | 6.0 | 4.0 | 3.0 | 9.0 | 0.0 | 7.0 |
| | 0.1250 | 2.0 | 4.0 | 0.0 | 9.0 | 0.0 | 6.0 |
| | 0.0620 | 0.0 | 0.0 | 0.0 | 7.0 | 0.0 | 5.0 |
| | 0.0320 | 0.0 | 0.0 | 0.0 | 6.0 | 0.0 | 4.0 |
| | | | | | | | |
| 602 | 0.2500 | 9.0 | 5.0 | 9.0 | 9.0 | 3.0 | |
| | 0.1250 | 9.0 | 5.0 | 7.0 | 8.0 | 0.0 | |
| | 0.0620 | 5.0 | 3.0 | 0.0 | 7.0 | 0.0 | |
| | 0.0320 | 0.0 | 3.0 | | 7.0 | 0.0 | |
| | | | | | | | |
| 603 | 0.2500 | 8.0 | 7.0 | 9.0 | 9.0 | 4.0 | |
| | 0.1250 | 6.0 | 6.0 | 8.0 | 9.0 | 2.0 | |
| | 0.0620 | 4.0 | | | 7.0 | 0.0 | |
| | 0.0320 | 0.0 | 5.0 | 0.0 | 7.0 | 0.0 | |
| | | | | | | | |
| 604 | 0.2500 | 9.0 | 6.0 | 0.0 | 8.0 | 0.0 | |
| | 0.1250 | 7.0 | 4.0 | 0.0 | 7.0 | 0.0 | |
| | 0.0620 | 7.0 | 3.0 | 0.0 | 6.0 | 0.0 | |
| | 0.0320 | 7.0 | 0.0 | 0.0 | 5.0 | 0.0 | |
| | | | | | | | |
| 605 | 0.2500 | 7.0 | 7.0 | 0.0 | 9.0 | 7.0 | |
| | 0.1250 | 7.0 | 7.0 | 0.0 | 9.0 | 5.0 | |
| | 0.0620 | 4.0 | 3.0 | 0.0 | 9.0 | 4.0 | |
| | 0.0320 | 4.0 | 1.0 | 0.0 | 9.0 | 4.0 | |
| | | | | | | | |
| 606 | 0.2500 | 6.0 | 9.0 | 2.0 | 9.0 | 4.0 | |
| | 0.1250 | 5.0 | 6.0 | 2.0 | 7.0 | 3.0 | |
| | 0.0620 | 5.0 | 6.0 | 2.0 | 7.0 | | |
| | 0.0320 | 5.0 | 4.0 | 0.0 | 6.0 | 0.0 | |
| | | | | | | | |
| 607 | 0.2500 | 9.0 | 5.0 | 0.0 | 9.0 | 3.0 | 3.0 |
| | 0.1250 | 9.0 | 5.0 | 0.0 | 9.0 | 0.0 | 2.0 |
| | 0.0620 | 7.0 | 2.0 | 0.0 | 7.0 | 0.0 | 0.0 |
| | 0.0320 | 4.0 | 0.0 | | 6.0 | 0.0 | 0.0 |

### EXAMPLE 188

### Rice tolerance to post-transplant applications and postemergence weed control under flooded paddy conditions

Plastic containers containing weeds which are 3 to 5 cm tall and rice seedlings at the 1.5 to 2.5 leaf stage are flooded with water. The water is maintained at 1.5 to 3 cm above the soil surface for the duration of the experiment. Test compounds are applied as aqueous/acetone mixtures 50/50⁻ v/v directly into the flood water to provide the equivalent of about 0.0313 to 0.500 kg/ha of active ingredient. The treated containers are placed on greenhouse benches and cared for in accordance with conventional greenhouse procedures. Three to four weeks after treatment, the test is terminated and each container is examined and herbicidal effect rated according to the rating system provided in Example 185. The results are summarized in Table IV. The compounds evaluated are reported by compound number given in Example 185.

**TABLE IV**

| **Compound** **Number** | **Rate** **(kg/ha)** | **E** **C** **H** **O** **R** **C** | **C** **Y** **P** **I** **R** | **C** **Y** **P** **S** **E** | **M** **O** **O** **V** **A** | **S** **A** **G** **P** **Y** | **O** **R** **Y** **S** **A** **T** |
|---|---|---|---|---|---|---|---|
| 1 | 0.5000 | 9.0 | 9.0 | 6.5 | 6.5 | 3.0 | 7.0 |
| | 0.2500 | 8.0 | 9.0 | 5.5 | 5.5 | 1.0 | 6.0 |
| | 0.1250 | 6.5 | 9.0 | 3.0 | 4.5 | 0.0 | 4.0 |
| | 0.0625 | 2.5 | 4.0 | 1.0 | 1.0 | 0.0 | 3.0 |
| | 0.0313 | 1.0 | 0.0 | 1.0 | 0.0 | 0.0 | 3.0 |
| | | | | | | | |
| 2 | 0.5000 | 9.0 | 9.0 | 0.0 | 9.0 | 4.0 | 8.0 |
| | 0.2500 | 9.0 | 9.0 | 0.0 | 9.0 | 2.0 | 7.0 |
| | 0.1250 | 7.0 | 9.0 | 0.0 | 9.0 | 0.0 | 3.0 |
| | 0.0625 | 4.0 | 9.0 | 0.0 | 9.0 | 0.0 | 2.0 |
| | 0.0313 | 4.0 | 9.0 | 0.0 | 9.0 | 0.0 | 2.0 |
| | | | | | | | |
| 3 | 0.5000 | 4.0 | 9.0 | 0.0 | 9.0 | 0.0 | 3.0 |
| | 0.2500 | 2.0 | 9.0 | 0.0 | 9.0 | 0.0 | 3.0 |
| | 0.1250 | 0.0 | 9.0 | 0.0 | 9.0 | 0.0 | 3.0 |
| | 0.0625 | 0.0 | 9.0 | 0.0 | 9.0 | 0.0 | 2.0 |
| | 0.0313 | 0.0 | 9.0 | 0.0 | 9.0 | 0.0 | 2.0 |
| | | | | | | | |
| 4 | 0.5000 | 9.0 | 9.0 | 9.0 | 9.0 | 9.0 | 8.0 |
| | 0.2500 | 9.0 | 9.0 | 7.0 | 9.0 | 9.0 | 8.0 |
| | 0.1250 | 9.0 | 9.0 | 7.0 | 9.0 | 6.0 | 7.0 |
| | 0.0625 | 9.0 | 9.0 | 4.0 | 9.0 | 2.0 | 4.0 |
| | 0.0313 | 7.0 | 8.0 | 2.0 | 8.0 | 3.0 | 3.0 |
| | | | | | | | |
| 5 | 0.5000 | 9.0 | 9.0 | 0.0 | 9.0 | 8.0 | 8.0 |
| | 0.2500 | 9.0 | 9.0 | 0.0 | 9.0 | 6.0 | 6.0 |
| | 0.1250 | 6.0 | 9.0 | 0.0 | 9.0 | 4.0 | 4.0 |
| | 0.0625 | 7.0 | 9.0 | 0.0 | 9.0 | 2.0 | 2.0 |
| | 0.0313 | 2.0 | 9.0 | 0.0 | 9.0 | 2.0 | 2.0 |
| | | | | | | | |
| 6 | 0.5000 | 9.0 | 9.0 | 9.0 | 9.0 | 9.0 | 9.0 |
| | 0.2500 | 9.0 | 9.0 | 7.0 | 9.0 | 8.0 | 9.0 |
| | 0.1250 | 9.0 | 9.0 | 7.0 | 9.0 | 6.0 | 9.0 |
| | 0.0625 | 7.0 | 9.0 | 2.0 | 9.0 | 4.0 | 4.0 |
| | 0.0313 | 4.0 | 9.0 | 0.0 | 9.0 | 2.0 | 4.0 |
| | | | | | | | |
| 7 | 0.5000 | 9.0 | 9.0 | 8.0 | 9.0 | 9.0 | 8.0 |
| | 0.2500 | 9.0 | 9.0 | 9.0 | 9.0 | 9.0 | 7.0 |
| | 0.1250 | 9.0 | 9.0 | 9.0 | 9.0 | 4.0 | 7.0 |
| | 0.0625 | 9.0 | 9.0 | 6.0 | 9.0 | 2.0 | 7.0 |
| | 0.0313 | 9.0 | 9.0 | 2.0 | 9.0 | 0.0 | 7.0 |
| | | | | | | | |
| 8 | 0.5000 | 9.0 | 9.0 | 9.0 | 9.0 | 9.0 | 9.0 |
| | 0.2500 | 9.0 | 9.0 | 7.0 | 9.0 | 9.0 | 8.0 |
| | 0.1250 | 9.0 | 9.0 | 7.0 | 9.0 | 4.0 | 6.0 |
| | 0.0625 | 9.0 | 9.0 | 2.0 | 9.0 | 4.0 | 6.0 |
| | 0.0313 | 6.0 | 9.0 | 0.0 | 9.0 | 2.0 | 4.0 |
| | | | | | | | |
| 9 | 0.5000 | 9.0 | 9.0 | 9.0 | 9.0 | 9.0 | 9.0 |
| | 0.2500 | 9.0 | 9.0 | 7.0 | 9.0 | 9.0 | 9.0 |
| | 0.1250 | 9.0 | 9.0 | 2.0 | 9.0 | 2.0 | 7.0 |
| | 0.0625 | 4.0 | 9.0 | 0.0 | 9.0 | 0.0 | 6.0 |
| | 0.0313 | 0.0 | 9.0 | 0.0 | 9.0 | 0.0 | 4.0 |
| | | | | | | | |
| 10 | 0.5000 | 9.0 | 9.0 | 7.0 | 9.0 | 4.0 | 4.0 |
| | 0.2500 | 9.0 | 9.0 | 6.0 | 9.0 | 4.0 | 3.0 |
| | 0.1250 | 9.0 | 9.0 | 2.0 | 9.0 | 4.0 | 4.0 |
| | 0.0625 | 9.0 | 9.0 | 0.0 | 9.0 | 2.0 | 2.0 |
| | 0.0313 | 7.0 | 9.0 | 0.0 | 9.0 | 2.0 | 2.0 |
| | | | | | | | |
| 11 | 0.5000 | 7.0 | 9.0 | 0.0 | 9.0 | 7.0 | 1.0 |
| | 0.2500 | 4.0 | 9.0 | 0.0 | 9.0 | 4.0 | 1.0 |
| | 0.1250 | 2.0 | 9.0 | 0.0 | 9.0 | 0.0 | 0.0 |
| | 0.0625 | 0.0 | 9.0 | 0.0 | 9.0 | 0.0 | 0.0 |
| | 0.0313 | 0.0 | 8.0 | 0.0 | 9.0 | 0.0 | 0.0 |
| | | | | | | | |
| 12 | 0.5000 | 9.0 | 9.0 | 2.0 | 9.0 | 9.0 | 7.0 |
| | 0.2500 | 9.0 | 9.0 | | 9.0 | 4.0 | 7.0 |
| | 0.1250 | 6.0 | 9.0 | | 9.0 | 0.0 | 7.0 |
| | 0.0625 | 2.0 | 9.0 | 0.0 | 9.0 | 0.0 | 4.0 |
| | 0.0313 | 0.0 | 9.0 | 0.0 | 9.0 | 0.0 | 4.0 |
| | | | | | | | |
| 13 | 0.5000 | 9.0 | 9.0 | 2.0 | 9.0 | 9.0 | 7.0 |
| | 0.2500 | 8.0 | 9.0 | 4.0 | 9.0 | 2.0 | 4.0 |
| | 0.1250 | 6.0 | 9.0 | 2.0 | 9.0 | 2.0 | 4.0 |
| | 0.0625 | 2.0 | 9.0 | 0.0 | 9.0 | 0.0 | 2.0 |
| | 0.0313 | 0.0 | 9.0 | 0.0 | 9.0 | 0.0 | 2.0 |
| | | | | | | | |
| 49 | 0.2500 | 9.0 | 6.0 | 0.0 | 9.0 | 0.0 | 0.0 |
| | 0.1250 | 9.0 | 4.0 | 0.0 | 9.0 | 0.0 | 0.0 |
| | | | | | | | |
| 50 | 0.2500 | 9.0 | 9.0 | 0.0 | 9.0 | 0.0 | 0.0 |
| | 0.1250 | 9.0 | 9.0 | 0.0 | 9.0 | 0.0 | 0.0 |
| | | | | | | | |
| 63 | 0.5000 | 4.0 | | 0.0 | 9.0 | 0.0 | 0.0 |
| | 0.2500 | 2.0 | | 0.0 | 9.0 | 0.0 | 0.0 |
| | 0.1250 | 0.0 | | 0.0 | 2.0 | 0.0 | 0.0 |
| | | | | | | | |
| 261 | 0.2500 | 0.0 | 6.0 | 0.0 | 9.0 | 0.0 | 0.0 |
| | 0.1250 | 0.0 | 6.0 | 0.0 | 9.0 | 0.0 | 0.0 |
| | 0.0620 | 0.0 | 6.0 | 0.0 | 9.0 | 0.0 | 0.0 |
| | 0.0320 | 0.0 | 5.0 | 0.0 | 9.0 | 0.0 | 0.0 |
| | | | | | | | |
| 267 | 0.2500 | 9.0 | 9.0 | 0.0 | 9.0 | 0.0 | 7.0 |
| | 0.1250 | 9.0 | 9.0 | 0.0 | 9.0 | 0.0 | 6.0 |
| | 0.0620 | 8.0 | 9.0 | 0.0 | 9.0 | 0.0 | 5.0 |
| | 0.0320 | 4.0 | 9.0 | 0.0 | 9.0 | 0.0 | 2.0 |
| | | | | | | | |
| 327 | 0.2500 | 9.0 | 9.0 | 2.0 | 9.0 | 4.0 | 4.0 |
| | 0.1250 | 9.0 | 9.0 | 0.0 | 9.0 | 4.0 | 5.0 |
| | 0.0620 | 7.0 | 9.0 | 0.0 | 9.0 | 0.0 | 3.0 |
| | 0.0320 | 9.0 | 9.0 | 0.0 | 9.0 | 0.0 | 2.0 |
| | | | | | | | |
| 328 | 0.2500 | 9.0 | 9.0 | 6.0 | 9.0 | 6.0 | 4.0 |
| | 0.1250 | 9.0 | 9.0 | 6.0 | 9.0 | 2.0 | 4.0 |
| | 0.0620 | 9.0 | 9.0 | 2.0 | 9.0 | 0.0 | 4.0 |
| | 0.0320 | 7.0 | 9.0 | 0.0 | 9.0 | 0.0 | 2.0 |
| | | | | | | | |
| 333 | 0.2500 | 9.0 | 9.0 | 8.0 | 9.0 | 4.0 | 6.0 |
| | 0.1250 | 9.0 | 9.0 | 4.0 | 9.0 | 4.0 | 6.0 |
| | 0.0620 | 9.0 | 9.0 | 2.0 | 9.0 | 2.0 | 5.0 |
| | 0.0320 | 9.0 | 9.0 | 0.0 | 9.0 | 0.0 | 4.0 |
| | | | | | | | |
| 354 | 0.1250 | 9.0 | 9.0 | 9.0 | 9.0 | 9.0 | 6.0 |
| | 0.0620 | 7.0 | 9.0 | 2.0 | 9.0 | 2.0 | 6.0 |
| | 0.0320 | 4.0 | | 1.0 | 6.0 | 0.0 | |
| | | | | | | | |
| 359 | 0.1250 | 6.0 | 9.0 | 0.0 | 9.0 | 0.0 | 4.0 |
| | 0.0620 | 6.0 | 7.0 | 0.0 | 7.0 | 0.0 | 3.0 |
| | 0.0320 | 2.0 | 4.0 | 0.0 | 7.0 | 0.0 | 2.0 |
| | | | | | | | |
| 362 | 0.2500 | 9.0 | 4.0 | 9.0 | 9.0 | 8.0 | 6.0 |
| | 0.1250 | 7.0 | 8.0 | 9.0 | 9.0 | 4.0 | 3.0 |
| | 0.0620 | 2.0 | 7.0 | 9.0 | 9.0 | 0.0 | 3.0 |
| | 0.0320 | 0.0 | 4.0 | 0.0 | 9.0 | 0.0 | 3.0 |
| | | | | | | | |
| 378 | 0.1250 | 9.0 | 9.0 | 9.0 | 9.0 | 9.0 | 9.0 |
| | 0.0620 | 9.0 | 9.0 | 9.0 | 9.0 | 9.0 | 8.0 |
| | 0.0320 | 9.0 | 9.0 | 9.0 | 9.0 | 9.0 | 8.0 |
| | | | | | | | |
| 380 | 0.2500 | 9.0 | 9.0 | 8.0 | 9.0 | 8.0 | 7.0 |
| | 0.1250 | 9.0 | 9.0 | 2.0 | 9.0 | 2.0 | 7.0 |
| | 0.0620 | 8.0 | 9.0 | 2.0 | 9.0 | 0.0 | 7.0 |
| | 0.0320 | 7.0 | 9.0 | 0.0 | 9.0 | 0.0 | 3.0 |
| | | | | | | | |
| 429 | 0.2500 | 9.0 | 9.0 | 0.0 | 7.0 | 0.0 | 2.0 |
| | 0.1250 | 6.0 | 7.0 | 0.0 | 7.0 | 0.0 | 2.0 |
| | 0.0620 | 6.0 | 6.0 | 0.0 | 4.0 | 0.0 | 1.0 |
| | 0.0320 | 4.0 | 4.0 | 0.0 | 2.0 | 0.0 | 1.0 |
| | | | | | | | |
| 432 | 0.2500 | 9.0 | 9.0 | 9.0 | 9.0 | 4.0 | 7.0 |
| | 0.1250 | 9.0 | 9.0 | 7.0 | 9.0 | 2.0 | 7.0 |
| | 0.0620 | 9.0 | 8.0 | 4.0 | 9.0 | 0.0 | 4.0 |
| | 0.0320 | 9.0 | 8.0 | 0.0 | 9.0 | 0.0 | 4.0 |
| | | | | | | | |
| 431 | 0.2500 | 9.0 | 9.0 | 4.0 | 9.0 | 4.0 | 7.0 |
| | 0.1250 | 9.0 | 9.0 | 0.0 | 9.0 | 0.0 | 3.0 |
| | 0.0620 | 9.0 | 7.0 | 0.0 | 9.0 | 0.0 | 3.0 |
| | 0.0320 | 7.0 | 4.0 | 0.0 | 6.0 | 0.0 | 3.0 |
| | | | | | | | |
| 445 | 0.2500 | 9.0 | 9.0 | 4.0 | 9.0 | 9.0 | 7.0 |
| | 0.1250 | 9.0 | 9.0 | 4.0 | 2.0 | 4.0 | 7.0 |
| | 0.0620 | 7.0 | 7.0 | 2.0 | 0.0 | 0.0 | 6.0 |
| | 0.0320 | 4.0 | 4.0 | 0.0 | 0.0 | 0.0 | 3.0 |
| | | | | | | | |
| 448 | 0.2500 | 2.0 | 9.0 | 0.0 | 4.0 | 0.0 | 2.0 |
| | 0.1250 | 1.0 | 7.0 | 0.0 | 1.0 | 0.0 | 1.0 |
| | 0.0620 | 0.0 | | 0.0 | 0.0 | 0.0 | 0.0 |
| | | | | | | | |
| 517 | 0.2500 | 0.0 | 9.0 | 0.0 | 9.0 | 0.0 | 0.0 |
| | 0.1250 | 0.0 | 9.0 | 0.0 | 9.0 | 0.0 | 0.0 |
| | 0.0620 | 0.0 | 5.0 | 0.0 | 9.0 | 0.0 | 0.0 |
| | 0.0320 | 0.0 | 3.0 | 0.0 | 9.0 | 0.0 | 0.0 |
| | | | | | | | |
| 528 | 0.2500 | 9.0 | 6.0 | 9.0 | 9.0 | 3.0 | 3.0 |
| | 0.1250 | 9.0 | 6.0 | 0.0 | 9.0 | 0.0 | 2.0 |
| | 0.0620 | 7.0 | 6.0 | 0.0 | 0.0 | 0.0 | 0.0 |
| | 0.0320 | 0.0 | 6.0 | 0.0 | 0.0 | 0.0 | 0.0 |

### EXAMPLE 189

### Comparative herbicidal evaluations

The preemergence and postemergence herbicidal properties of certain compounds of the present invention are compared with certain compounds disclosed in U.S. Patent Nos. 5,484,763 and 5,523,278. The evaluations are performed as described in Examples 185 and 186. The results are reported in Tables V and VI. Data in Tables V and VI are reported by the compound number given in Example 185 for the compounds of this invention or by comparative compound letter for the compounds disclosed in U.S. Patent Nos. 5,484,763 and 5,523,278.

As can be seen from the data in Tables V and VI, the compounds of this invention are more effective herbicidal agents than the compounds disclosed in U.S. Patent Nos. 5,484,763 and 5,523,278.

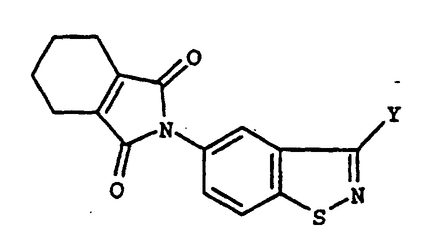

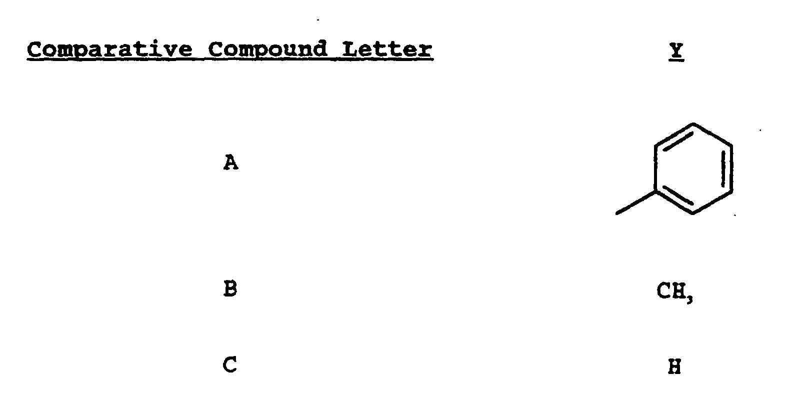

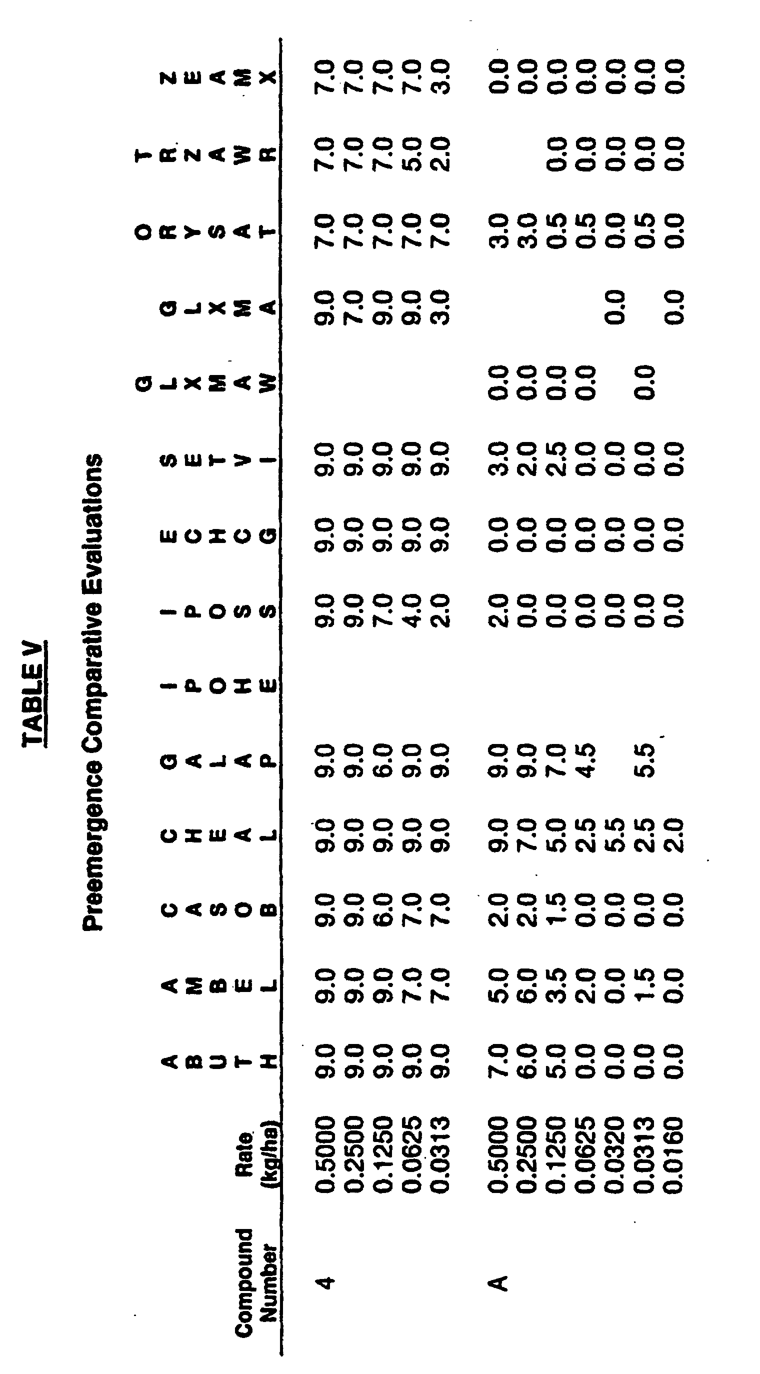

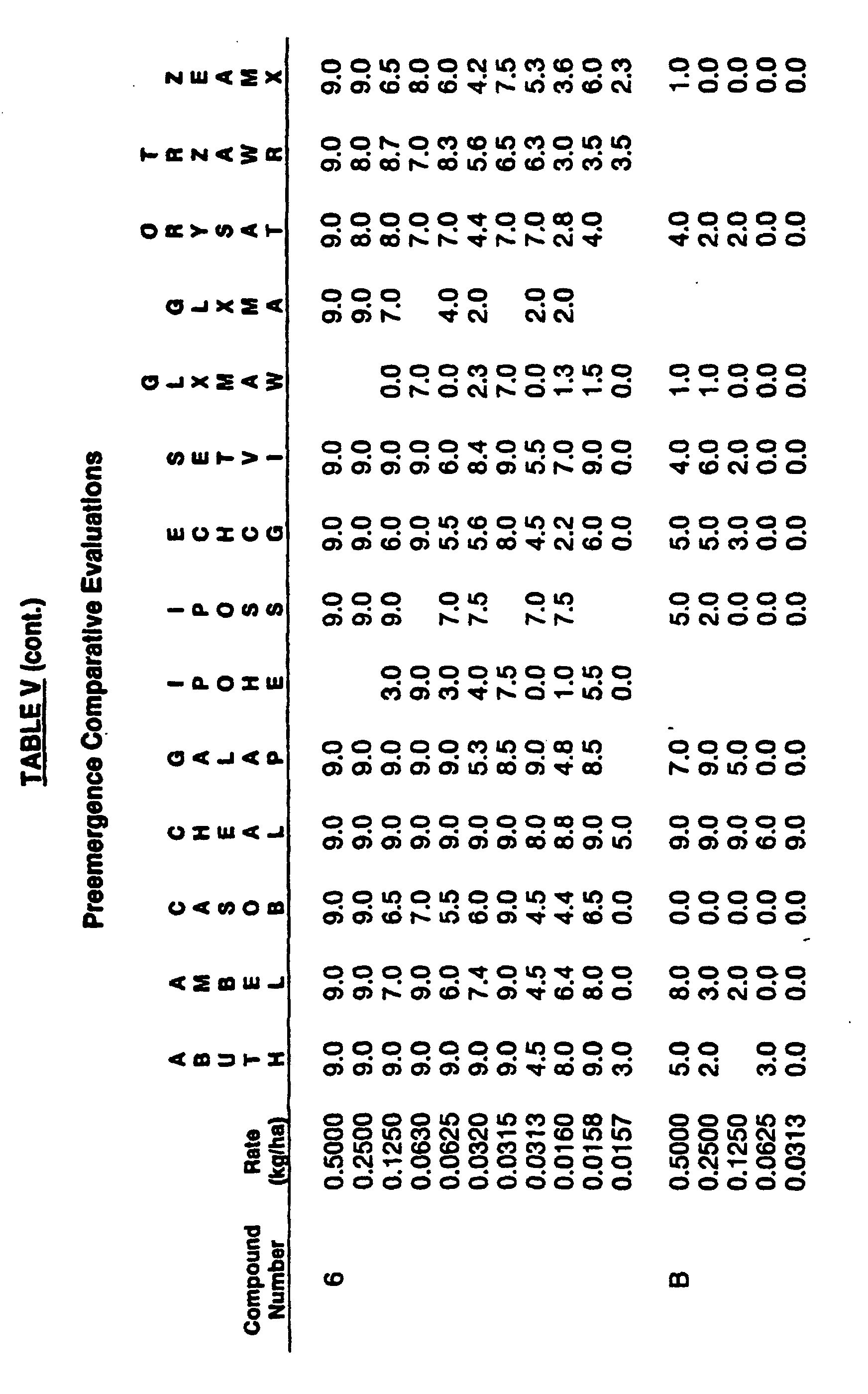

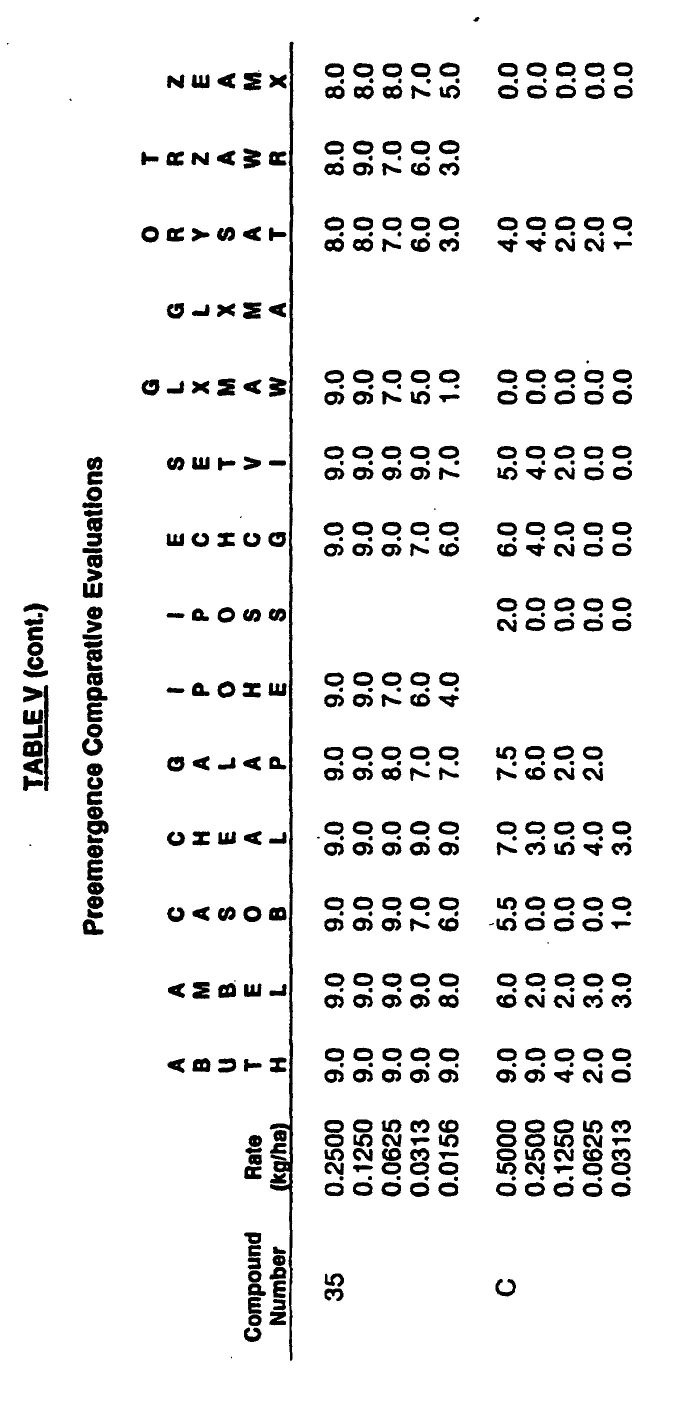

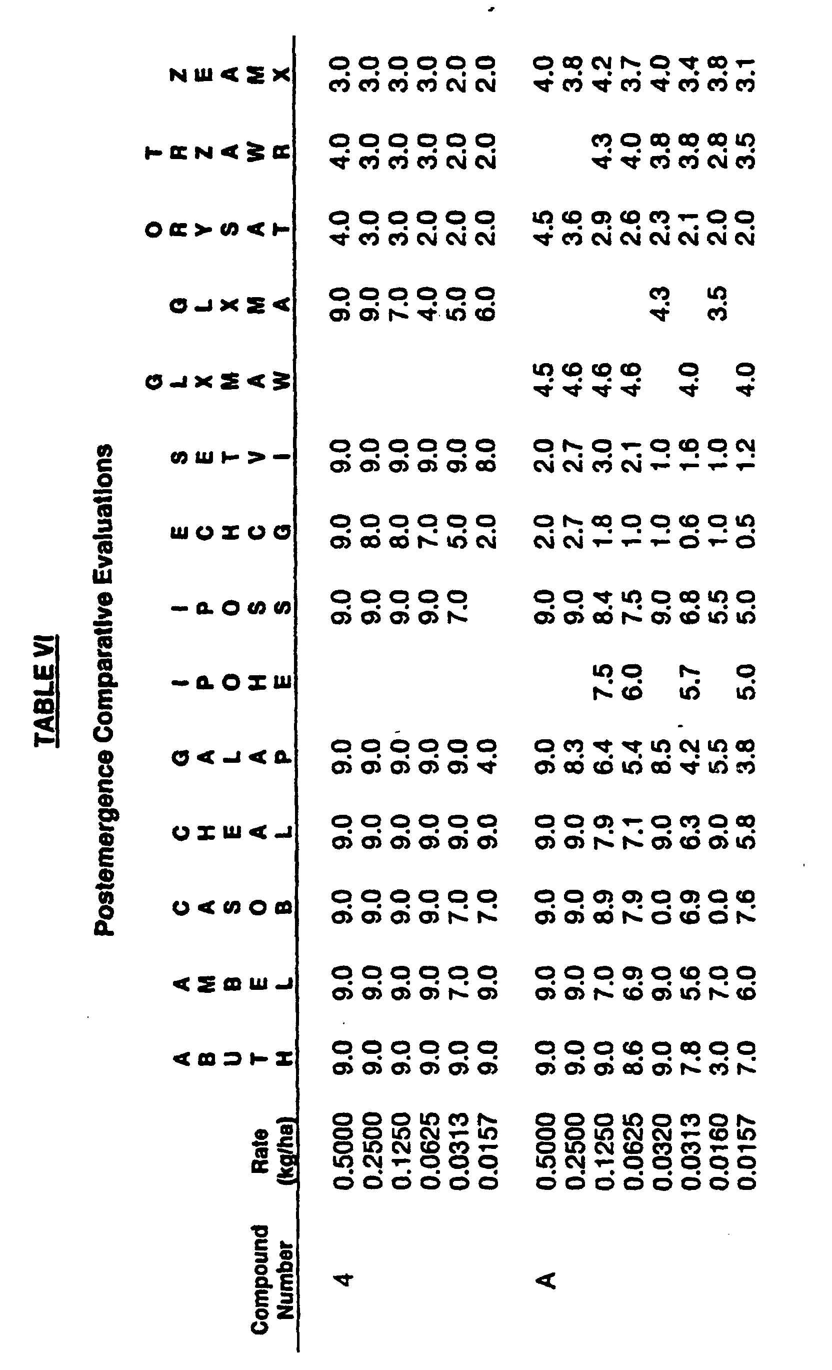

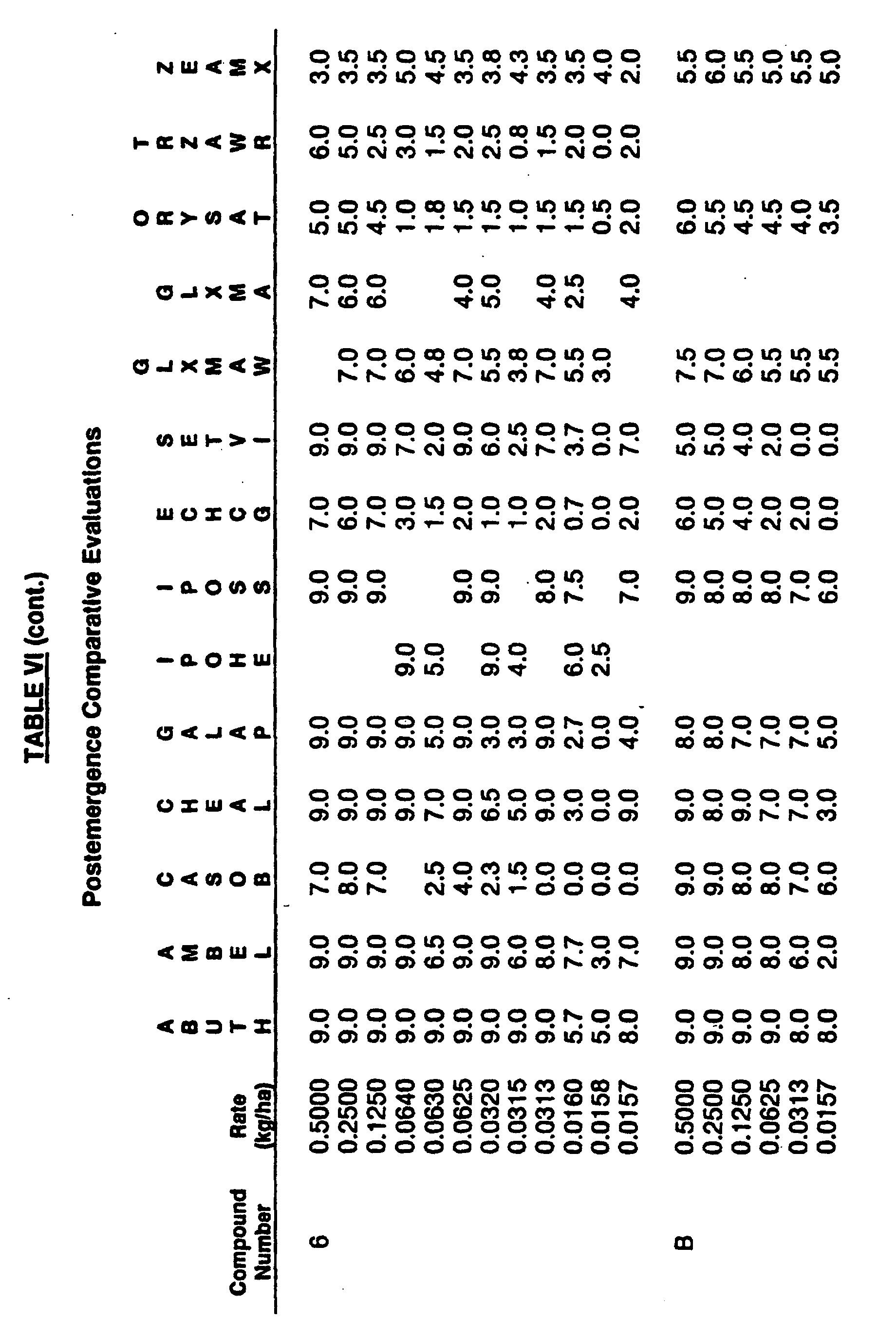

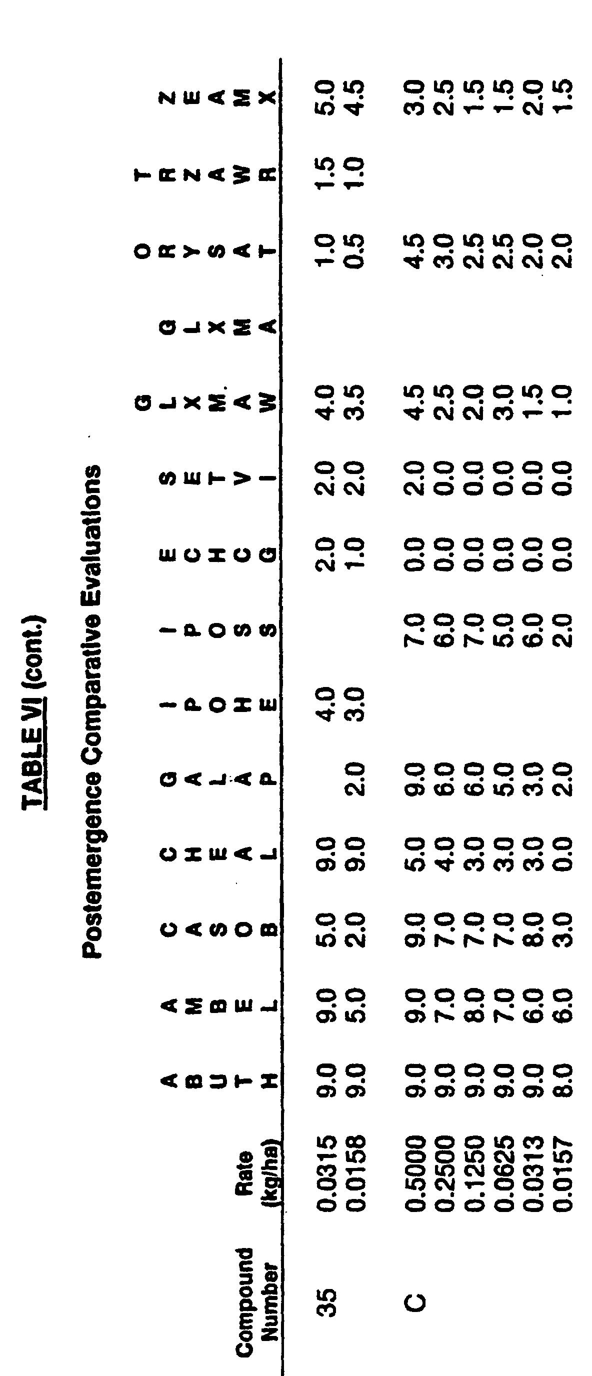

## Claims

1. A compound having the structural formula 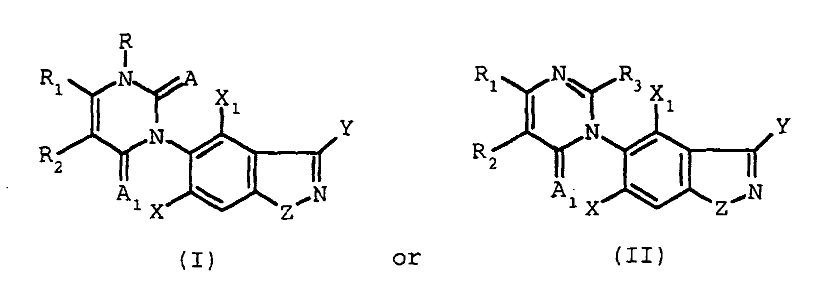 wherein
R is C₁-C₆alkyl, C₁-C₆haloalkyl, C₃-C₇cycloalkyl, C₃-C₆alkenyl, C₃-C₆alkynyl, benzyl, OR₄ or NR₅R₆;
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally interrupted by oxygen, sulfur or nitrogen, and optionally substituted with one to three methyl groups or one or more halogen atoms;
R₃ is halogen or A₂R₁₁;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is, O, S(O)ₚ or NR₁₃;
X₃ is -C(=A₃)-, -C(=NOR₁₄)- or -C(=N-NR₁₃R₅₀);
R₄, R₅, R₆, R₇, R₈, R₉, R₁₀, R₁₁ and R₁₄ are each independently hydrogen, C₁-C₆alkyl, C₃-C₇cycloalkyl, C₂-C₈alkoxyalkyl, C₂-C₆alkenyl, C₂-C₆alkynyl or benzyl;
R₁₂ is hydrogen,
a C₁-C₈alkyl, C₃-C₇cycloalkyl, C₂-C₈alkenyl, C₅-C₆cycloalkenyl or C₂-C₈alkynyl group, wherein each group is optionally substituted with any combination of one to six halogen atoms, one to three C₁-C₂₀alkoxy groups optionally substituted with one C₁-C₆alkoxy, benzyloxy or C₁-C₆alkylthio group, one or two C₁-C₁₀haloalkoxy groups, one or two NR₁₅R₁₆ groups, one to three S(O)_{q}R₁₇ groups, one or two cyano groups, one or two C₃-C₇cycloalkyl groups, one thiocyano group, one O(SO₂)R₁₇ group, one O(NO₂) group, one or two C(O)R₁₈ groups, one or two C(O)SR₁₉ groups, one or two C(O)SR₁₉ groups, one or two C(O)NR₂₀R₂₁ groups, one to three X₄R₂₂ groups, one or two P(O)(OR₂₃)₂ groups, one or two Si(R₂₄)₃ groups,
one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
one quaternary organic ammonium group, or
one or two phenyl groups wherein each phenyl group is independently optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group,
phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group, or
indan optionally substituted with any combination of one or two halogen atoms, one or two C₁-C₆alkyl groups, one C₁-C₆alkoxy group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₁-C₈alkoxyalkyl, C₁-C₆alkyl, C₃-C₇cycloalkyl, C₃-C₆alkenyl, C₃-C₆alkynyl, benzyl, OR₂₃, cyano, SO₂R₅₁ or one 5- to 12-membered
monocyclic or fused bicyclic heterocycling ring optionally substituted with one or more groups independently selected from halogen, cyano, nitro, amino, hydroxyl, C₁-C₄alkyl, C₁-C₄haloalkyl, C₁-C₄alkoxy or
C₁-C₄haloalkoxy, and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃, C(O)NR₃₅R₃₆, C(O)R₃₃, SO₂R₄₇,
C₁-C₂₀alkyl substituted with one hydroxyl, benzyloxy, nitro, OC(O)R₃₃, C₁-C₆alkoxy, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano or 2-dioxolanyl group, one phenyl,
dihydrofuranone or furanone ring wherein each ring is optionally substituted with any combination of one to three halogen atoms, on to three C₁-C₄alkyl groups, one to three C₁-C₄haloalkyl groups, one to three C₁-C₄alkoxy groups or one to three C₁-C₄haloalkoxy groups, or
one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
C₃-C₂₀alkenyl optionally substituted with one to three halogen atoms, one hydroxyl group, one C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
C₅-C₈cycloalkenyl optionally substituted with one hydroxyl, C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
C₃-C₁₀alkynyl optionally substituted with one hydroxyl, C₁-C₆alkoxy, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
a C₁-C₆alkyl, C₃-C₇cycloalkyl, C₂-C₆alkenyl or C₂-C₆alkynyl group wherein each group is optionally substituted with one CO₂R₄₂ group,
benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one CO₂R₂₆ group,
C(O)R₄₃, or
CO₂R₄₂;
R₁₆ and R₂₁ are each independently hydrogen, C₁-C₆alkyl,
C₁-C₄alkoxy, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently NR₄₆R₄₈, -N=CR₄₆R₄₈, C₁-C₈alkyl optionally substituted with one C₁-C₆alkoxy, thiophene or furan group, C₁-C₆haloalkyl, C₃-C₈cycloalkyl, CO₂R₂₃, C₂-C₆alkenyl optionally substituted with phenyl,
benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
Si(R₄₇)₃,
di(C₁-C₄alkyl)amino,
C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)amino group,
C₁-C₁₅haloalkyl,
C₃-C₈cycloalkyl optionally substituted with one CO₂R₄₈ group,
C₃-C₈halocycloalkyl,
C₃-C₂₀alkenyl optionally substituted with one phenyl group,
C₃-C₁₅haloalkenyl,
C₅-C₈cycloalkenyl,
C₅-C₈halocycloalkenyl,
C₃-C₂₀alkynyl optionally substituted with one phenyl group,
C₃-C₂₀haloalkynyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
2-, 3- or 4-pyridyl,
2- or 3-furyl,
2- or 3-thienyl,
2-tetrahydrofuranyl,
C₁-C₄alkyl substituted with one pyridyl, furyl, thienyl, tetrahydrofuryl, C₃-C₈cycloalkyl, dioxane or NR₄₉R₅₀ group, or
an alkali metal, alkaline earth metal, maganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₂-C₆alkenyl, C₃-C₈cycloalkyl,
C₁-C₈alkyl optionally substituted with one or two C₁-C₄alkoxy groups,
benzyl optionally substituted with one or more groups independently selected from halogen,
cyano, nitro, C₁-C₄alkyl, C₁-C₄haloalkyl, C₁-C₄alkoxy and
C₁-C₄haloalkoxy groups, or
phenyl optionally substituted with one or more groups independently selected from halogen, cyano, nitro, C₁-C₄alkyl, C₁-C₄haloalkyl, C₁-C₄alkoxy and C₁-C₄haloalkoxy groups, and
when R₄₆ and R₄₈ are taken together with the atom to which they are attached, they form a 5- to 12-membered monocyclic or fused bicyclic, heterocyclic or cycloalkyl ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups; and
A, A₁, A₂, A₃, A₄, X₄ and X₅ are each independently O or S; and
the optical isomers, diastereomers and/or tautomers thereof.

2. The compound according to claim 1 wherein
R is C₁-C₆alkyl, C₁-C₆haloalkyl C₃-C₇cycloalkyl, C₃-C₆alkenyl, C₃-C₆alkynyl, benzyl, OR₄ or NR₅R₆;
R₁ and R₂ are each independently hydrogen, halogen, C₁-C₆alkyl, C₃-C₇cycloalkyl, C₁-C₆haloalkyl, C₃-C₆alkenyl, C₃-C₆haloalkenyl, C₃-C₆alkynyl, OR₇, S(O)ₙR₈ or NR₉R₁₀, and when R₁ and R₂ are taken together with the atoms to which they are attached, they represent a four- to seven-membered saturated or unsaturated ring optionally interrupted by oxygen, sulfur or nitrogen, and optionally substituted with one to three methyl groups or one or more halogen atoms;
R₃ is halogen or A₂R₁₁;
X is hydrogen, halogen or C₁-C₄alkyl;
X₁ is hydrogen, halogen, C₁-C₄alkyl, C₁-C₄haloalkyl, C₁-C₄alkoxy or C₁-C₄haloalkoxy;
Z is O or S(O)ₘ;
Y is halogen, cyano, R₁₂, X₂R₁₂, X₃R₁₂, X₂X₃R₁₂ or X₃X₂R₁₂;
X₂ is O, S(O)ₚ or NR₁₃;
X₃ is -C(=A₃)- or -C(=NOR₁₄)-;
R₄, R₅, R₆, R₇, R₈, R₉, R₁₀, R₁₁ and R₁₄ are each independently hydrogen, C₁-C₆alkyl, C₃-C₇cycloalkyl, C₂-C₆alkenyl, C₂-C₆alkynyl or benzyl;
R₁₂ is hydrogen,
a C₁-C₆alkyl, C₃-C₇cycloalkyl, C₂-C₆alkenyl, C₅-C₆cycloalkenyl or C₂-C₆alkynyl group, wherein each group is optionally substituted with any combination of one to six halogen atoms, one to three C₁-C₂₀alkoxy groups, one or two C₁-C₁₀haloalkoxy groups, one or two NR₁₅R₁₆groups, one to three S(O)_{q}R₁₇ groups, one or two cyano groups, one or two C(O)R₁₈ groups, one or two CO₂R₁₉ groups, one or two C(O)NR₂₀R₂₁ groups, one to three X₄R₂₂ groups, one or two P(O)(OR₂₃)₂ groups, one or two Si(R₂₄)₃ groups, one 4- to 10-membered monocyclic or fused bicyclic heterocyclic
ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
one quaternary organic ammonium group, or
one phenyl group optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₂₅ group, one CO₂R₂₆ group or one X₅R₂₇ group, or
phenyl optionally substituted with any combination of one to three halogen atoms, one to three C₁-C₄alkyl groups, one to three C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group,
and when R₁₂ and R₁₃ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₃ is hydrogen, C₁-C₆alkyl, C₃-C₇cycloalkyl, C₃-C₆alkenyl, C₃-C₆alkynyl, benzyl, OR₂₃, cyano or SO₂R₅₁, and when R₁₃ and R₁₂ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₂₈ is halogen;
R₂₂ and R₂₇ are each independently hydrogen, Si(R₃₁)₃, C₁-C₂₀alkyl substituted with one C₁-C₆alkoxy, CO₂R₃₂,
C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NHOR₂₃, cyano or 2-dioxolanyl group,
one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
one 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups,
C₃-C₂₀alkenyl optionally substituted with one to three halogen atoms, one C₁-C₆alkoxy, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
C₅-C₈cycloalkenyl optionally substituted with one C₁-C₆alkoxy, C₁-C₄alkyl, C₂-C₄alkenyl, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄alkoxy groups or one to three C₁-C₄haloalkoxy groups,
C₃-C₁₀alkynyl optionally substituted with one C₁-C₆alkoxy, CO₂R₃₂, C(O)R₃₃, C(A₄R₃₄)₂, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
phenyl optionally substituted with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group,
benzyl optionally substituted on the phenyl ring with any combination of one to three halogen atoms, one to three C₁-C₆alkyl groups, one to three C₁-C₆alkoxy groups, one C₁-C₄haloalkyl group, one C₁-C₄haloalkoxy group, one cyano group, one nitro group, one NR₃₉R₄₀ group or one C(O)R₃₃ group, or
a 4- to 10-membered heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups or one to three C₁-C₄haloalkoxy groups;
R₁₅ and R₂₀ are each independently hydrogen,
a C₁-C₆alkyl, C₃-C₇cycloalkyl, C₂-C₆alkenyl or C₂-C₆alkynyl group wherein each group is optionally substituted with one CO₂R₄₂ group,
benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
C(O)R₄₃;
R₁₆ and R₂₁ are each independently hydrogen, C₁-C₆alkyl, C₃-C₇cycloalkyl, C₂-C₆alkenyl, C₃-C₇cycloalkenyl, C₂-C₆alkynyl, P(O)(OR₄₄)₂, SO₂R₄₅, C(O)R₄₆,
benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group, or
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups or one X₄R₂₂ group;
R₁₇, R₁₈, R₂₅, R₃₀, R₃₃, R₄₅ and R₅₁ are each independently C₁-C₄alkyl, C₁-C₄haloalkyl, CO₂R₂₃,
C₂-C₆alkenyl optionally substituted with phenyl,
benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group,
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
a 5- to 12- membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, C₁-C₄haloalkylsulfonyl or X₄R₂₂ groups;
R₁₉ and R₂₆ are each independently hydrogen, C₁-C₂₀alkyl, C₁-C₈haloalkyl, C₃-C₇cycloalkyl, C₃-C₇halocycloalkyl, C₃-C₂₀alkenyl, C₃-C₈haloalkenyl, C₅-C₈cycloalkenyl, C₅-C₈halocycloalkenyl, C₃-C₈alkynyl, C₃-C₈haloalkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen
Si(R₄₇)₃,
di(C₁-C₄alkyl)amino
C₁-C₂₀alkyl optionally substituted with one CO₂R₄₈, C₁-C₆alkoxy or di(C₁-C₄alkyl)amino group,
C₁-C₁₅haloalkyl,
C₃-C₈cycloalkyl,
C₃-C₈halocycloalkyl,
C₃-C₂₀alkenyl optionally substituted with one phenyl group,
C₃-C₁₅haloalkenyl,
C₅-C₈cycloalkenyl,
C₅-C₈halocycloalkenyl,
C₃-C₂₀alkynyl optionally substituted with one phenyl group,
C₃-C₂₀haloalkynyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
2-, 3- or 4-pyridyl,
3- or 3-furyl,
2- or 3-thienyl,
2-tetrahydrofuranyl,
C₁-C₄alkyl substituted with one pyridyl, furyl, thienyl, tetrahydrofuryl or NR₄₉R₅₀ group, or
an alkali metal, alkaline earth metal, maganese, copper, zinc, cobalt, silver, nickel, ammonium or organic ammonium cation;
R₃₄, R₃₇ and R₃₈ are each independently C₁-C₆alkyl;
R₃₅ and R₄₉ are each independently hydrogen or C₁-C₆alkyl and R₃₆ and R₅₀ are each independently hydrogen, cyano, C₁-C₆alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
m, n, p and q are each independently an integer of 0, 1 or 2;
R₂₉ is hydrogen, C₁-C₄alkyl or C₁-C₄haloalkyl;
R₂₄, R₃₁, R₄₁ and R₄₇ are each independently C₁-C₄alkyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
phenyl optionally substituted with one or more groups independently selected from halogen, cyano, nitro, C₁-C₄alkyl, C₁-C₄haloalkyl, C₁-C₄alkoxy and C₁-C₄haloalkoxy groups;
R₂₃, R₃₉, R₄₀, R₄₂, R₄₃, R₄₄, R₄₆ and R₄₈ are each independently hydrogen, C₁-C₄alkyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups, or
phenyl optionally substituted with one or more groups independently selected from halogen, cyano, nitro, C₁-C₄alkyl, C₁-C₄haloalkyl, C₁-C₄alkoxy and C₁-C₄haloalkoxy groups; and
A, A₁, A₂, A₃, A₄, X₄ and X₅ are each independently O or S; and
the optical isomers, diastereomers and/or tautomers thereof.

3. The compound according to claim 1 having the structural formula

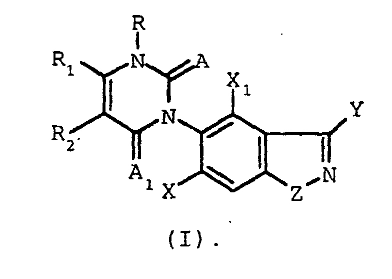

4. The compound according to claim 3 wherein
R is C₁-C₄alkyl, C₁-C₄haloalkyl, C₃-C₄alkenyl, C₃-C₄alkynyl or benzyl;
R₁ is halogen or C₁-C₄haloalkyl;
R₂ is hydrogen, halogen or C₁-C₄haloalkyl;
X and X₁ are each independently hydrogen or halogen;
Z is O or S;
Y is cyano, R₁₂, X₂R₁₂ or X₃X₂R₁₂;
X₂ is O, S or NR₁₃;
R₁₂ is hydrogen,
a C₁-C₆alkyl, C₃-C₇cycloalkyl, C₂-C₆alkenyl, C₅-C₆cycloalkenyl or C₂-C₆alkynyl group, wherein each group is optionally substituted with any combination of one to three halogen atoms, one or two C₁-C₂₀alkoxy groups, one or two C₁-C₁₀haloalkoxy groups, one NR₁₅R₁₆ group, one S(O)_{q}R₁₇ group, one or two cyano groups, one or two C(O)R₁₈ groups, one or two CO₂R₁₉ groups, one or two C(O)SR₁₉ groups, one or two C(O)NR₂₀R₂₁ groups, one or two X₄R₂₂ groups, one P(O)(OR₂₃)₂ group, one Si(R₂₄)₃ group,
one 4- to 10-membered monocyclic or fused bicyclic heterocyclic ring optionally substituted with any combination of one or two oxo groups, one or two thioxo groups, one to three halogen atoms, one to three C₁-C₄alkyl groups, one to three C₁-C₄haloalkyl groups, one to three C₁-C₄alkoxy groups, one to three C₁-C₄haloalkoxy groups or one or two X₄R₂₂ groups,
one quaternary organic ammonium group, or
one phenyl group optionally substituted with any combination of one to three halogen atoms, one C₁-C₆alkyl group, one C₁-C₆alkoxy group, one C₃-C₇cycloalkyl group, one cyano group, one nitro group, one C(O)R₂₅ group, one CO₂R₂₆ group or one X₅R₂₇ group, or
phenyl optionally substituted with any combination of one or two halogen atoms, one to three C₁-C₄alkyl groups, one or two C₁-C₄alkoxy groups, one C₃-C₆cycloalkyl group, one C₁-C₄haloalkyl group, one C₁-C₄alkylthio group, one cyano group, one nitro group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₂-C₆alkoxyalkyl, C₁-C₄alkyl, C₃-C₇cycloalkyl, C₃-C₆alkenyl, C₃-C₆alkynyl, benzyl, OR₂₃, cyano or SO₂R₅₁;
R₁₄ is hydrogen, C₁-C₆alkyl, C₃-C₇cycloalkyl, C₂-C₈alkoxyalkyl, C₂-C₆alkenyl, C₂-C₆alkynyl or benzyl;
R₁₆ and R₂₁ are each independently hydrogen, C₁-C₆alkyl,
C₁-C₄alkoxy, C₃-C₇cycloalkyl, C₂-C₆alkenyl, C₃-C₇cycloalkenyl,
C₂-C₆alkynyl, P(O)(OR₄₄)₂, SO₂R₄₅, C(O)R₄₆,
benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups, or
phenyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups; and
R₃₂ is hydrogen,
Si(R₄₇)₃,
C₁-C₂₀alkyl optionally substituted with one CO₂R₄₈, C₁-C₆alkoxy, C₁-C₆thioalkyl, C₂-C₂₀alkoxyalkoxy, phenyl, OSi(R₄₇)₃, OC(O)R₃₃, C₃-C₇cycloalkyl or di(C₁-C₄alkyl)amino group,
C₁-C₁₅haloalkyl,
C₃-C₈cycloalkyl optionally substituted with one CO₂R₄₈ group,
C₃-C₈halocycloalkyl,
C₃-C₂₀alkenyl optionally substituted with one phenyl group,
C₃-C₁₅haloalkenyl,
C₅-C₈cycloalkenyl,
C₅-C₈halocycloalkenyl,
C₃-C₂₀alkynyl optionally substituted with one phenyl group,
C₃-C₂₀haloalkynyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
phenyl optionally substituted with one or more groups independently selected halogen, cyano, nitro, C₁-C₄alkyl, C₁-C₄haloalkyl, C₁-C₄alkoxy and C₁-C₄haloalkoxy groups,
C₁-C₄alkyl substituted with one pyridyl, furyl, thienyl, tetrahydrofuryl, C₃-C₈cycloalkyl, dioxane or NR₄₉R₅₀ group, or
an alkali metal, alkaline earth metal, maganese, copper, zinc, cobalt, silver, nickel, ammonium or organic ammonium cation.

5. The compound according to claim 4 wherein
R is C₁-C₄alkyl;
R₁ is C₁-C₄haloalkyl;
R₂ is hydrogen,
Y is R₁₂, OR₁₂, C(O)NR₁₃R₁₂, C(O)R₁₂, CO₂R₁₂, C(O)SR₁₂ or
C(=NOR₁₄);
R₁₂ is hydrogen,
a C₁-C₆alkyl or C₂-C₆alkenyl group, wherein each group is optionally substituted with any combination of one to three halogen atoms, one or two C₁-C₄₋ alkoxy groups, one C₁-C₁₀haloalkoxy group, one NR₁₅R₁₆ group, one S(O)_{q}R₁₇ group, one or two cyano groups, one or two C(O)R₁₈ groups, one or two CO₂R₁₉ groups, one or two C(O)NR₂₀R₂₁ groups, one X₄R₂₂ group, one P(O)(OR₂₃)₂ group, one OC(O)R₃₃ group,
one imidazole ring optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
one phthalimide ring optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
one quaternary organic ammonium group, or
one phenyl group optionally substituted with any combination of one to three halogen atoms, one C₁-C₄alkyl group, one C₁-C₄alkoxy group or one X₅R₂₇ group, or
phenyl optionally substituted with any combination of one or two halogen atoms, one to three C₁-C₄alkyl groups, one C₁-C₄alkoxy group, one C(O)R₂₅ group, one CO₂R₂₆ group, one X₄R₂₂ group, one CH=CHR₂₂ group, one CH₂CH(R₂₈)R₂₂ group or one N(R₂₉)SO₂R₃₀ group;
R₁₃ is hydrogen, C₁-C₆alkoxyalkyl, C₁-C₄ alkyl, OR₂₃, cyano or SO₂R₅₁;
R₂₂ and R₂₇ are each independently hydrogen, C(O)R₃₃, SO₂R₄₇, C₁-C₄alkyl substituted with one C₁-C₆alkoxy, CO₂R₃₂,
C(O)R₃₃, C(O)NR₃₅R₃₆, C(O)ON=CR₃₇R₃₈, C(O)NHOR₂₃, cyano or 2-dioxolanyl group, or one phenyl, dihydrofuranone or furanone ring wherein each ring is optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups,
C₃-C₆alkenyl optionally substituted with one C₁-C₆alkoxy, CO₂R₃₂, C(O)R₃₃ or C(OR₃₄)₂ group,
C₃-C₆alkynyl,
phenyl optionally substituted with any combination of one to three halogen atoms, one or two C₁-C₄alkyl groups, one or two C₁-C₄alkoxy groups, one C₁-C₄haloalkyl group, one C₁-C₄haloalkoxy group, one cyano group, one nitro group, one NR₃₉R₄₀ group or one C(O)R₃₃ group, or
benzyl optionally substituted on the phenyl ring with any combination of one to three halogen atoms, one or two C₁-C₄alkyl groups, one or two C₁-C₄alkoxy groups, one C₁-C₄haloalkyl group, one C₁-C₄haloalkoxy group, one cyano group, one nitro group, one NR₃₉R₄₀ group or one C(O)R₃₃ group;
R₁₅ is hydrogen,
C₁-C₄alkyl optionally substituted with one CO₂R₄₂ group,
benzyl optionally substituted with any combination of one to three halogen atoms, one to three
C₁-C₄alkyl groups, one to three C₁-C₄haloalkyl groups, one to three C₁-C₄alkoxy groups or one to three C₁-C₄haloalkoxy groups, or
C(O)R₄₃;
R₁₆ is hydrogen, C₁-C₄alkyl, P(O)(OR₄₄)₂, SO₂R₄₅, C(O)R₄₆, or
benzyl optionally substituted with any combination of one to three halogen atoms, one to three C₁-C₄alkyl groups, one to three C₁-C₄haloalkyl groups, one to three C₁-C₄alkoxy groups or one to three C₁-C₄haloalkoxy groups;
R₁₇, R₁₈, R₂₅, R₃₀, R₃₃, R₄₅ and R₅₁ are each independently NR₄₆R₄₈, C₁-C₄alkyl, C₁-C₄haloalkyl,
C₂-C₆alkenyl optionally substituted with phenyl,
benzyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group, or
phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one nitro group, one cyano group or one X₄R₂₂ group;
R₁₉ and R₂₆ are each independently hydrogen, C₁-C₄alkyl, C₃-C₆alkenyl, C₃-C₆alkynyl, Si(R₄₁)₃, benzyl, phenyl, furfuryl, pyridyl, thienyl, oximino or an alkali metal, alkaline earth metal, manganese, copper, zinc, cobalt, silver, nickel, ammonium or organic ammonium cation;
R₃₂ is hydrogen,
Si(R₄₇)₃,
C₁-C₄alkyl optionally substituted with one CO₂R₄₈, OC(O)R₃₃ or C₁-C₄alkoxy group,
C₁-C₄haloalkyl,
C₃-C₈cycloalkyl,
C₃-C₈halocycloalkyl,
C₃-C₆alkenyl optionally substituted with one phenyl group,
C₃-C₆haloalkenyl,
C₅-C₈cycloalkenyl,
C₅-C₈halocycloalkenyl,
C₃-C₆alkynyl optionally substituted with one phenyl group,
C₃-C₆haloalkynyl,
benzyl optionally substituted with one or more groups independently selected from halogen, cyano, nitro, C₁-C₄alkyl, C₁-C₄haloalkyl, C₁-C₄alkoxy and C₁-C₄haloalkoxy groups,
phenyl optionally substituted with one or more groups independently selected from halogen, cyano, nitro, C₁-C₄alkyl, C₁-C₄haloalkyl, C₁-C₄alkoxy and C₁-C₄haloalkoxy groups,
C₁-C₄alkyl substituted with one pyridyl, furyl, thienyl, tetrahydrofuryl or NR₄₉R₅₀ group, or
an alkali metal, alkaline earth metal, manganese, copper, zinc, cobalt, silver, nickel, ammonium or organic ammonium cation;
R₃₅ and R₄₉ are each independently hydrogen or C₁-C₄alkyl and R₃₆ and R₅₀ are each independently hydrogen, cyano, C₁-C₄alkyl, SO₂R₅₁ or phenyl optionally substituted with one or more groups independently selected from halogen, cyano, nitro, C₁-C₄alkyl, C₁-C₄haloalkyl, C₁-C₄alkoxy and C₁-C₄haloalkoxy groups, or when R₃₅ and R₃₆ or R₄₉ and R₅₀ are taken together with the nitrogen atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy or C₁-C₄haloalkylsulfonyl groups;
R₂₃ is hydrogen or C₁-C₄ alkyl; and
A is O.

6. The compound according to claim 5 wherein
R is methyl;
R₁ is trifluoromethyl;
X₁ is hydrogen;
X is hydrogen or fluorine;
Y is R₁₂, CO₂R₁₂, C(O)R₁₂ or C(O)NR₁₃R₁₂;
R₁₂ is hydrogen,
C₁-C₆alkyl optionally substituted with any combination of one to three halogen atoms, one hydroxyl group, one or two C₁-C₄alkoxy groups, one C₁-C₄haloalkoxy group, one SO₂R₁₇ group, one or two cyano groups, one C(O)R₁₈ group, one or two CO₂R₁₉ groups, one or two C(O)NR₂₀R₂₁ groups, one P(O)(OR₂₃)₂ group or one OC(O)R₃₃ group,
C₂-C₆alkenyl optionally substituted with any combination of one C(O)R₁₈ group or one CO₂R₁₉ group, 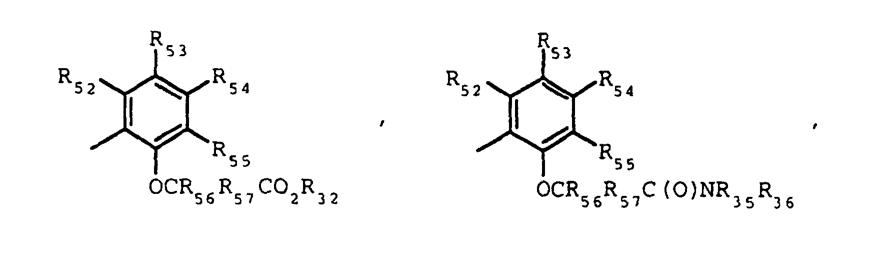 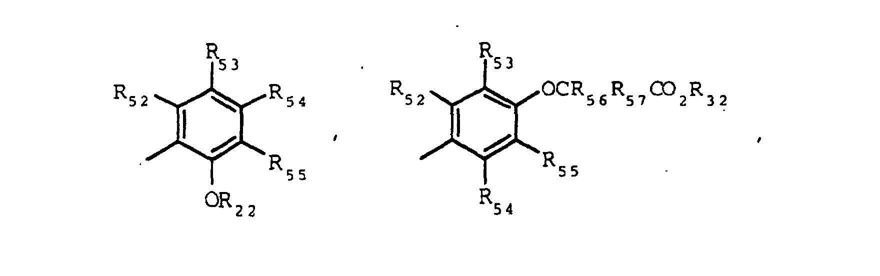 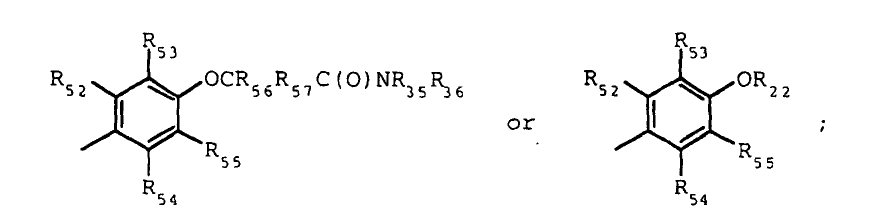
R₁₃ is hydrogen, C₁-C₄ alkyl or SO₂R₅₁;
R₁₄ is hydrogen, C₁-C₄alkyl or benzyl;
R₁₈, R₂₃, R₅₆ and R₅₇ are each independently hydrogen or C₁-C₃alkyl;
R₅₂, R₅₃, R₅₄ and R₅₅ are each independently hydrogen, C₁-C₃alkyl, C₂-C₃alkenyl, C₁-C₃alkoxy or halogen, provided that at least one of R₅₂, R₅₃, R₅₄ and R₅₅ is hydrogen;
R₁₉ is hydrogen, C₁-C₄alkyl, C₃-C₆alkenyl, C₃-C₆alkynyl, benzyl, pyridyl or an alkali metal, alkaline earth metal, ammonium or organic ammonium cation;
R₂₂ is hydrogen, C(O)R₃₃, SO₂R₄₇,
C₁-C₄alkyl optionally substituted with one cyano group,
C₃-C₆alkenyl, or
C₃-C₆alkynyl;
R₃₂ is hydrogen,
C₁-C₄alkyl optionally substituted with one CO₂R₄₈ or C₁-C₄alkoxy group,
C₁-C₄haloalkyl,
C₃-C₆alkenyl optionally substituted with one phenyl group,
C₃-C₆alkynyl optionally substituted with one phenyl group,
C₁-C₄alkyl substituted with one pyridyl, furyl, thienyl, tetrahydrofuryl or NR₄₉R₅₀ group, or
an alkali metal, alkaline earth metal, ammonium or organic ammonium cation;
R₃₅ and R₄₉ are each independently hydrogen or C₁-C₄alkyl;
R₃₆ is SO₂R₅₁ or C₁-C₃alkoxy;
R₁₇, R₃₃ and R₅₁ are each independently C₁-C₄alkyl, C₁-C₄haloalkyl, NR₄₆R₄₈, C₃-C₆cycloalkyl or an isoxazole ring;
R₄₆ and R₄₈ are each independently hydrogen, C₁-C₄alkyl or
benzyl;
R₄₇ is C₁-C₄alkyl;
R₅₀ is C₁-C₄alkyl or
phenyl optionally substituted with one or more groups independently selected from halogen, cyano, C₁-C₄alkyl, C₁-C₄haloalkyl, C₁-C₄alkoxy and C₁-C₄haloalkoxy groups; and
A₁ is O.

7. The compound according to claim 6 wherein
Y is C₁-C₃alkyl, CO₂R₃₂, halomethyl, cyanomethyl, C(CH₃)₂CO₂R₃₂, C₁-C₃alkoxyethyl, C₁-C₃alkoxymethyl, hydroxymethyl, CHO, C(O)CH₃, CH(CH₃)(C₁-C₄alkoxy), C(CH₃)₂CN, CH[O(C₁-C₃alkyl)]₂, CH₂SO₂R₁₇, C(O)NHSO₂R₅₁, 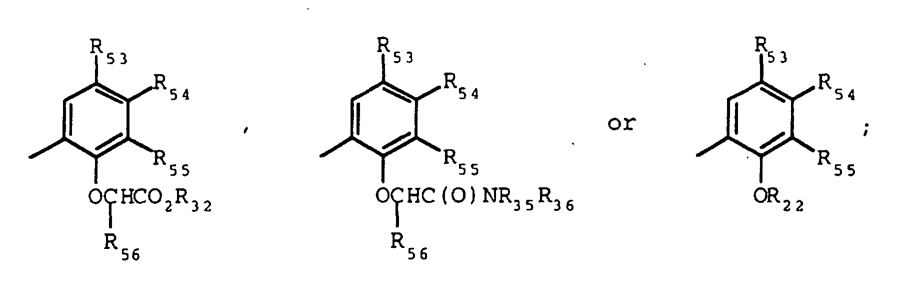
R₅₃, R₅₄, R₅₅ and R₅₆ are each independently hydrogen or methyl, provided that at least one of R₅₃, R₅₄ and R₅₅ is hydrogen;
R₂₂ is cyanomethyl, methyl, ethyl, allyl or propargyl;
R₃₂ is hydrogen,
C₁-C₄alkyl optionally substituted with one CO₂R₄₈ group,
C₁-C₄haloalkyl,
C₃-C₆alkenyl, or
C₃-C₆alkynyl; and
R₃₅ is hydrogen.

8. The compound according to claim 1 selected from the group consisting of
isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy} acetate;
2-(dimethylamino)ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate;
methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate;
2-propynyl {{2-{5-[3,6-dihydro-3-methyl-2,6-dioxo-4-(trifluoromethyl)-1(2H)-pyrimidinyl]-1,2-benzisothiazol-3-yl}-p-tolyl}oxy}acetate;
3-[3-(6-methoxy-*m*-tolyl)-1,2-benzisothiazol-5-yl]-1-methyl-6-(trifluoromethyl)-2,4(1H,3H)-pyrimidinedione;
3-[3-(*p*-ethylphenyl)-1,2-benzisothiazol-5-yl]-1-methyl-6-(trifluoromethyl)-2,4(1H,3H)-pyrimidinedione;
1-methyl-3-(3-methyl-1,2-benzisothiazol-5-yl)-6-(trifluoromethyl)-2,4(1H,3H)-pyrimidinedione;
methyl 2-{{6-{5-[3,6-dihydro-3-methyl-4-(trifluoromethyl)-2,6-dioxo-1(2H)-pyrimidinyl]-1,2-benzisothiazol-3-yl}-*m*-tolyl}oxy}propionate;
methyl {{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}acetate;
3-[3-(dibromomethyl)-1,2-benzisothiazol-5-yl]-1-methyl-6-(trifluoromethyl)-2,4(1H,3H)-pyrimidinedione;
3-(6-fluoro-3-methyl-1,2-benzisoxazol-5-yl)-1-methyl-6-(trifluoromethyl)-2,4(1H,3H)-pyrimidinedione;
ethyl {{2-{5-[3,6-dihydro-3-methyl-2,6-dioxo-4-(trilluoromethyl)-1(2H)-pyrimidinyl]-1,2-benzisothiazol-3-yl}-*p*-tolyl}oxy}acetate;
1-methyl-3-{3-[6-(2-propynyloxy)-*m*-tolyl]-1,2-benzisothiazol-5-yl}-6-(trifluoromethyl)-2,4(1H,3H)-pyrimidinedione;
3-{3-{6-[(1-benzimidazolylcarbonyl)methoxy]-*m*-tolyl}-1,2-benzisothiazol-5-yl}-1-methyl-6-trifluoromethyl)-2,4(1H,3H)-pyrimidinedione;
3-{3-{6-{[(*m*-cyanophenyl)carbamoyl]methoxy}-*m*-tolyl}-1,2-benzisothiazol-5-yl}-1-methyl-6-(trifluoromethyl)-2,4(1H,3H)pyrimidinedione;
allyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
3-phenyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
1-methyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-chloroethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate;
2,2,2-trifluoroethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
3-[3-(2-methoxy-*p*-tolyl)-1,2-benzisothiazol-5-yl]-1-methyl-6-(trifluoromethyl)-2,4(1H,3H)-pyrimidinedione;
2-[3-(6-methoxy-3,4-xylyl)-1,2-benzisothiazol-5-yl]-1-methyl-6-(trifluoromethyl)-2,4(1H,3H)-pyrimidinedione;
methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate;
methyl {{6-{5-[3,6-dihydro-3-methyl-2,6-dioxo-4-(trifluoromethyl)-1(2H)-pyrimidinyl]-1,2-benzisothiazol-3-yl}-2,4-xylyl}oxy}acetate;
3-{3-{6-{[(*m*-cyanophenyl)carbamoyl]methoxy}-*m*-tolyl}-1,2-benzisothiazol-5-yl}-1-methyl-6-(trifluoromethyl)-2,4(1H,3H)-pyrimidinedione;
methyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
1-methyl-3-{3-[(methylsulfonyl)methyl]-1,2-benzisothiazol-5-yl}-6-(trifluoromethyl)-2,4(1H,3H)-pyrimidinedione;
2-propynyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
3-[3-(*o*-methoxyphenyl)-1,2-benzisothiazol-5-yl]-1-methyl-6-(trifluoromethyl)-2,4( 1H,3H)-pyrimidinedione;
N-{{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetyl}methanesulfonamide;
2-propynyl 2-{{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate;
2-propynyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate;
5-[3,6-dihydro-3-methyl-2,6-dioxo-4-(trifluoromethyl)-1(2H)-pyrimidinyl]-1,2-benzisothiazole-3-acetonitrile;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
methyl {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetate;
methyl {{6-(5-[3,6-dihydro-3-methyl-2,6-dioxo-4-(trifluoromethyl)-1(2H)-pyrimidinyl]-6-fluoro-1,2-benzisothiazol-3-yl}-*m*-tolyl}oxy}acetate;
{*o*-{5-[3,6-dihydro-3-methyl-2,6-dioxo-4-(trifluoromethyl)-1(2H)-pyrimidinyl]-1,2-benzisothiazol-3-yl}phenoxy}acetic acid;
2-{*o*-{5-[3,6-dihydro-3-methyl-2,6-dioxo-4-(trifluoromethyl)-1(2H)-pyrimidinyl]-1,2-benzisothiazol-3-yl}phenoxy}propionic acid;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methoxyacetamide;
methyl 2-{2-{5-[3,6-dihydro-3-methyl-2,6-dioxo-4-(trifluoromethyl)-1(2H)-pyrimidinyl]-1,2-benzisothiazol-3-yl}-4-methoxyphenoxy}propionate;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(methylsulfonyl)acetamide;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(methylsulfonyl)propionamide;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl-N-(methylsulfonyl)acetamide;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-methyl-N-(methylsulfonyl)acetamide;
2-{*o*-{5-[3,6-dihydro-3-methyl-2,6-dioxo-4-(trifluoromethyl)-1(2H)-pyrimidinyl]-1,2-benzisothiazol-3-yl}phenoxy}-N-(ethylsulfonyl)acetamide;
3-[3-(hydroxymethyl)-1,2-benzisothiazol-5-yl]-1-methyl-6-(trifluoromethyl)-2,4(1H,3H)-pyrimidinedione, 2-thiophenecarboxylate (ester);
3-[3-(hydroxymethyl)-1,2-benzisothiazol-5-yl]-1-methyl-6-(trifluoromethyl)-2,4(1H,3H)-pyrimidinedione, benzoate (ester);
3-[3-(hydroxymethyl)-1,2-benzisothiazol-5-yl]-1-methyl-6-(trifluoromethyl)-2,4(1H,3H)-pyrimidinedione, methoxyacetate (ester);
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-α,α-dimethyl-1,2-benzisothiazole-3-acetonitrile;
5-[3,6-dihydro-3-methyl-2,6-dioxo-4-(trifluoromethyl)-1(2H)-pyrimidinyl]-6-floro-1,2-benzisothiazole-3-acetonitrile;
5-[3,6-dihydro-3-methyl-2,6-dioxo-4-(trifluoromethyl)-1(2H)-pyrimidinyl]-α-ethyl-1,2-benzisothiazole-3-acetonitrile;
5-[3,6-dihydro-3-methyl-2,6-dioxo-4-(trifluoromethyl)-1(2H)-pyrimidinyl]-α,α-diethyl-1,2-benzisothiazole-3-acetonitrile;
3-[6-fluoro-3-(methoxymethyl)-1,2-benzisothiazol-5-yl]-1-methyl-6-(trifluoromethyl)-2,4(1H,3H)-pyrimidinedione;
3-[3-(hydroxymethyl)-1,2-benzisothiazol-5-yl]-1-methyl-6-(trifluoromethyl)-2,4(1H,3H)-pyrimidinedione, 5-isoxazolecarboxylate (ester);
3-[3-(hydroxymethyl)-1,2-benzisothiazol-5-yl]-1-methyl-6-(trifluoromethyl)-2,4(1H,3H)-pyrimidinedione, cyclopropanecarboxylate (ester);
3-(3-acetyl-1,2-benzisothiazol-5-yl]-1-methyl-6-(trifluoromethyl)-2,4(1H,3H)-pyrimidinedione;
3-[3-(1-methoxyethyl)-1,2-benzisothiazol-5-yl]-1-methyl-6-(trifluoromethyl)-2,4(1H,3H)-pyrimidinedione;
N-(benzylsulfonyl)-5-[3,6-dihydro-3-methyl-2,6-dioxo-4-(trifluoromethyl)-1(2H)-pyrimidinyl]-1,2-benzisothiazole-3-carboxamide;
methyl 5-[3,6-dihydro-3-methyl-2,6-dioxo-4-(trifluoromethyl)-1(2H)-pyrimidinyl]-1,2-benzisothiazole-3-carboxylate;
benzyl 5-[3,6-dihydro-3-methyl-2,6-dioxo-4-(trifluoromethyl)-1 (2H)-pyrimidinyl]-α,α-dimethyl-1,2-benzisothiazole-3-acetate;
N-(benzylsulfonyl)-5-[3,6-dihydro-3-methyl-2,6-dioxo-4-(trifluoromethyl)-1(2H)-pyrimidinyl]-6-fluoro-1,2-benzisothiazole-3-carboxamide;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-N-[(*p*-methylbenzyl)sulfonyl]-1,2-benzisothiazole-3-carboxamide;
N-(benzylsulfonyl)-5-[3,6-dihydro-3-methyl-2,6-dioxo-4-(trifluoromethyl)-1(2H)-pyrimidinyl]-6-fluoro-1,2-benzisothiazole-3-carboxamide;
3-[3-(1-hydroxyethyl)-1,2-benzisothiazol-5-yl]-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-1,2-benzisothiazole-3-carboxaldehyde, 3-(dimethyl acetal);
3-(6-fluoro-3-methyl-1,2-benzisothiazol-5-yl)-1-methyl-6-(trifluoromethyl)-2,4(1H,3H)pyrimidinedione;
5-[3,6-dihydro-3-methyl-2,6-dioxo-4-(trifluoromethyl)-1(2H)-pyrimidinyl]-6-fluoro-1,2-benzisothiazole-3-carboxaldehyde, 3-(dimethyl acetal);
5-[3,6-dihydro-3-methyl-2,6-dioxo-4-(trifluoromethyl)-1(2H)-pyrimidinyl]-6-fluoro-1,2-benzisothiazole-3-carboxylic acid;
5-[3,6-dihydro-3-methyl-2,6-dioxo-4-(trifluoromethyl)-1(2H)-pyrimidinyl]-α-methyl-1,2-benzisothiazole-3-acetonitrile;
N-(benzylsulfonyl)-5-[3,6-dihydro-3-methyl-2,6-dioxo-4-(trifluoromethyl)-1(2H)-pyrimidinyl)-N-(ethoxymethyl)-1,2-benzisothiazole-3-carboxamide;
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide;
methyl 5-[3,6-dihydro-3-methyl-2,6-dioxo-4-(trifluoromethyl)-1 (2H)-pyrimidinyl]-α,α-dimethyl-1,2-benzisothiazole-3-acetate;
3-[3-(hydroxymethyl)-1,2-benzisothiazol-5-yl]-1-methyl-6-(trifluoromethyl)-2,4(1H,3H)-pyrimidinedione, 2-furoate (ester);
N-[(*m*-chlorobenzyl)sulfonyl]-5-[3,6-dihydro-3-methyl-2,6-dioxo-4-(trifluoromethyl)-1(2H)-pyrimidinyl]-1,2-benzisothiazole-3-carboxamide;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide; and
3-[3-(hydroxymethyl)-1,2-benzisothiazol-5-yl]-1-methyl-6-(trifluoromethyl)-2,4(1H,3H)-pyrimidinedione, methylcarbamate (ester).

9. A method for the control of undesirable plant species which comprises applying to the foliage of said plants or to the soil or water containing seeds or other propagating organs thereof, a herbicidally effective amount of a compound according to any one of claims 1 to 8.

10. The method according to claim 9 wherein the compound is applied to the foliage of the undesirable plant species or to the soil or water containing seeds or other propagating organs thereof in the presence of crop plants, crop seeds or other crop propagating organs.

11. The method according to claim 10 wherein the crop is a cereal crop or soybean.

12. The method according to claim 11 wherein the cereal crop is selected from the group consisting of corn, wheat and rice.

13. The method according to claim 12 wherein the cereal crop is corn and the compound is selected from the group consisting of
isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}acetate;
2-(dimethylamino)ethyl{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
3-[3-(*p*-ethylphenyl)-1,2-benzisothiazol-5-yl]-1-methyl-6-(trifluoromethyl)-2,4(1H,3H)-pyrimidinedione;
methyl {{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}acetate;
3-[3-(dibromomethyl)-1,2-benzisothiazol-5-yl]-1-methyl-6-(trifluoromethyl)-2,4( 1H,3H)-pyrimidinedione;
1-methyl-2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
1-methyl-3-{3-[(methylsulfonyl)methyl]-1,2-benzisothiazol-5-yl}-6-(trifluoromethyl)-2,4(1H,3H)-pyrimidinedione;
2-propynyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}}oxy}acetate;
3-[3-(*o*-methoxyphenyl)-1,2-benzisothiazol-5-yl]-1-methyl-6-(trifluoromethyl)-2,4(1H,3H)-pyrimidinedione;
N-{{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetyl}methanesulfonamide;
2-propynyl {*o-*{5-[3,6-dihydro-3-methyl-2,6-dioxo-4-(trifluoromethyl)-1(2H)-pyrimidinyl]-1,2-benzisothiazol-3-yl}phenoxy}acetate;
5-[3,6-dihydro-3-methyl-2,6-dioxo-4-(trifluoromethyl)-1(2H)-pyrimidinyl]-1,2-benzisothiazole-3-acetonitrile;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
2-{{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl-N-(methylsulfonyl)acetamide;
3-[3-(hydroxymethyl)-1,2-benzisothiazol-5-yl]-1-methyl-6-(trifluoromethyl)-2,4(1H,3H)-pyrimidinedione, methylcarbamate (ester);
N-(benzylsulfonyl)-5-[3,6-dihydro-3-methyl-2,6-dioxo-4-(trifluoromethyl)-1(2H)-pyrimidinyl]-1,2-benzisothiazole-3-carboxamide;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl)-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide;
5-[3,6-dihydro-3-methyl-2,6-dioxo-4-(trifluoromethyl)-1(2H)-pyrimidinyl]-6-fluoro-1,2-benzisothiazole-3-carboxylic acid; and
N-(benzylsulfonyl)-5-[3,6-dihydro-3-methyl-2,6-dioxo-4-(trifluoromethyl)-1(2H)-pyrimidinyl]-6-fluoro-1,2-benzisothiazole-3-carboxamide.

14. The method according to claim 12 wherein the cereal crop is wheat and the compound is selected from the group consisting of
isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}acetate;
2-(dimethylamino)ethyl{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
2-propynyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl 2-{{6-{5-[3,6-dihydro-3-methyl-4-(trifluoromethyl)-2,6-dioxo-1(2H)-pyrimidinyl]-1,2-benzisothiazol-3-yl}-*m*-tolyl}oxy}propionate;
3-(6-fluoro-3-methyl-1,2-benzisoxazol-5-yl)-1-methyl-6-(trifluoromethyl)-2,4(1H,3H)-pyrimidinedione;
3-{3-{6-[(1-benzimidazolylcarbonyl)methoxy]-*m*-tolyl}-1,2-benzisothiazol-5-yl}-1-methyl-6-trifluoromethyl)-2,4(1H,3H)-pyrimidinedione;
allyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
3-phenyl-2-propynyl {{2-{(5-(3,6-dihydro-3-methyl-2,6-dioxo-4-(trifluoromethyl)-1(2H)-pyrimidinyl]-1,2-benzisothiazol-3-yl-*p*-tolyl}oxy}acetate;
2-chloroethyl 2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}propionate;
2,2,2-trifluoroethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{6-{5-[3,6-dihydro-3-methyl-2,6-dioxo-4-(trifluoromethyl)-1(2H)-pyrimidinyl]-1,2-benzisothiazol-3-yl}-2,4-xylyl}oxy}acetate;
{*o*-{5-[3,6-dihydro-3-methyl-2,6-dioxo-4-(trifluoromethyl)-1(2H)-pyrimidinyl]-1,2-benzisothiazol-3-yl}phenoxy} acetic acid;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methoxyacetamide;
2-{{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}-N-methyl-N-(methylsulfonyl)acetamide;
methyl {{6-{5-[3,6-dihydro-3-methyl-2,6-dioxo-4-(trifluoromethyl)-1(2H)-pyrimidinyl]-6-fluoro-1,2-benzisothiazol-3-yl}-*m*-tolyl}oxy}acetate;
3-(3-acetyl-1,2-benzisothiazol-5-yl)-1-methyl-6-(trifluoromethyl)-2,4(1H,3H)-pyrimidinedione;
methyl 5-[3,6-dihydro-3-methyl-2,6-dioxo-4-(trifluoromethyl)-1(2H)-pyrimidinyl]-1,2-benzisothiazole-3-carboxylate;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
N-[(*m*-chlorobenzyl)sulfonyl]-5-[3,6-dihydro-3-methyl-2,6-dioxo-4-(trifluoromethyl)-1(2H)-pyrimidinyl]-1,2-benzisothiazole-3-carboxamide;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-((isopropylsulfamoyl)-1,2-benzisothiazole-3-carboxamide; and
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide.

15. The method according to claim 12 wherein the cereal crop is rice and the compound is selected from the group consisting of
isopropyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
methyl {{2-{5-[3,6-dihydro-3-methyl-4-(trifluoromethyl)-2,6-dioxo-1(2H)-pyrimidinyl]-1,2-benzisothiazol-3-yl}*-p*-tolyl}oxy}acetate;
3-[3-(6-methoxy-*m*-tolyl)-1,2-benzisothiazol-5-yl]-1-methyl-6-(trifluoromethyl)-2,4(1H,3H)-pyrimidinedione;
ethyl {{2-{5-[3,6-dihydro-3-methyl-2,6-dioxo-4-(trifluoromethyl)-1(2H)-pyrimidinyl]-1,2-benzisothiazol-3-yl}-*p*-tolyl}oxy}acetate;
1-methyl-3-{3-[6-(2-propynyloxy)-*m*-tolyl]-1,2-benzisothiazol-5-yl}-6-(trifluoromethyl)-2,4(1H,3H)-pyrimidinedione;
3-{3-{6-{[(*m*-cyanophenyl)carbamoyl]methoxy}-*m*-tolyl}-1,2-benzisothiazol-5-yl}-1-methyl-6-(trifluoromethyl)-2,4(1H,3H)pyrimidinedione;
3-[3-(2-methoxy-*p*-tolyl)-1,2-benzisothiazol-5-yl]-1-methyl-6-(trifluoromethyl)-2,4(1H,3H)-pyrimidinedione;
2-[3-(6-methoxy-3,4-xylyl)-1,2-benzisothiazol-5-yl]-1-methyl-6-(trifluoromethyl)-2,4(1H,3H)-pyrimidinedione;
3-{3-{6-{[(*m*-cyanophenyl)carbamoyl]methoxy]-*m*-tolyl}-1,2-benzisothiazol-5-yl}-1-methyl-6-(trifluoromethyl)-2,4(1H,3H)-pyrimidinedione;
2-propynyl{*o*-{5-[3,6-dihydro-3-methyl-2,6-dioxo-4-(trifluoromethyl)-1(2H)-pyrimidinyl]-1,2-benzisothiazol-3-yl}phenoxy}acetate;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-a-ethyl-1,2-benzisothiazole-3-acetonitrile;
5-[3,6-dihydro-3-methyl-2,6-dioxo-4-(trifluoromethyl)-1(2H)-pyrimidinyl]-a,a-diethyl-1,2-benzisothiazole-3-acetonitrile;
3-[3-(hydroxymethyl)-1,2-benzisothiazol-5-yl]-1-methyl-6-(trifluoromethyl)-2,4(1H,3H)-pyrimidinedione, 5-isoxazolecarboxylate (ester);
3-[3-(hydroxymethyl)-1,2-benzisothiazol-5-yl]-1-methyl-6-(trifluoromethyl)-2,4(1H,3H)-pyrimidinedione, cyclopropanecarboxylate (ester);
methyl 5-[3,6-dihydro-3-methyl-2,6-dioxo-4-(trifluoromethyl)-1(2H)-pyrimidinyl]-1,2-benzisothiazole-3-carboxylate;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
benzyl 5-[3,6-dihydro-3-methyl-2,6-dioxo-4-(trifluoromethyl)-1(2H)-pyrimidinyl]-a,a-dimethyl-1,2-benzisothiazole-3-acetate;
5-[3,6-dihydro-3-methyl-2,6-dioxo-4-(trifluoromethyl)-1(2H)-pyrimidinyl]-N-(diisopropylsulfamoyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide; and
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide.

16. The method according to claim 11 wherein the crop is soybean and the compound is selected from the group consisting of
methyl 2-{{2-{5-[3,6-dihydro-3-methyl-4-(trifluoromethyl)-2,6-dioxo-1(2H)-pyrimidinyl]-1,2-benzisothiazol-3-yl}-*p*-tolyl}oxy}propionate;
methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate;
1-methyl-3-(3-methyl-1,2-benzisothiazol-5-yl)-6-(trifluoromethyl)-2,4(1H,3H)-pyrimidinedione;
methyl 2-{{6-{5-[3,6-dihydro-3-methyl-4-(trifluoromethyl)-2,6-dioxo-1(2H)-pyrimidinyl]-1,2-benzisothiazol-3-yl}-3,4-xylyl}oxy}propionate;
2-propynyl {{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}acetate;
3-[3-(*o*-methoxyphenyl)-1,2-benzisothiazol-5-yl]-1-methyl-6-(trifluoromethyl)-2,4(1H,3H)-pyrimidinedione;
2-propynyl 2-{{2-{5-[3,6-dihydro-3-methyl-2,6-dioxo-4-(trifluoromethyl)-1(2H)-pyrimidinyl]-1,2-benzisoxazol-3-yl}-*p*-tolyl}oxy}propionate;
2-propynyl {*o*-{5-[3,6-dihydro-3-methyl-2,6-dioxo-4-(trifluoromethyl)-1(2H)-pyrimidinyl]-1,2-benzisothiazol-3-yl}phenoxy}acetate;
3-{3-[(allyloxy)methyl]-1,2-benzisothiazol-5-yl}-1-methyl-6-(trifluoromethyl)-2,4(1H,3H)-pyrimidinedione;
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
methyl {{2-{5-[3,6-dihydro-3-methyl-2,6-dioxo-4-(trifluoromethyl)-1(2H)-pyrimidinyl]-6-fluoro-1,2-benzisoxazol-3-yl}-*p*-tolyl}oxy}acetate;
methyl {{6-{5-[3,6-dihydro-3-methyl-2,6-dioxo-4-(trifluoromethyl)-1(2H)-pyrimidinyl}-6-fluoro-1,2-benzisothiazol-3-yl}-*m*-tolyl}oxy}acetate;
3-[3-(hydroxymethyl)-1,2-benzisothiazol-5-yl]-1-methyl-6-(trifluoromethyl)-2,4(1H,3H)-pyrimidinedione, benzoate (ester);
3-[3-(hydroxymethyl)-1,2-benzisothiazol-5-yl]-1-methyl-6-(trifluoromethyl)-2,4(1H,3H)-pyrimidinedione, 2-thiophenecarboxylate (ester);
3-[3-(hydroxymethyl)-1,2-benzisothiazol-5-yl]-1-methyl-6-(trifluoromethyl)-2,4(1H,3H)-pyrimidinedione, methoxyacetate (ester);
3-[3-(hydroxymethyl)-1,2-benzisothiazol-5-yl]-1-methyl-6-(trifluoromethyl)-2,4(1H,3H)-pyrimidinedione, cyclopropanecarboxylate (ester);
3-[3-(hydroxymethyl)-1,2-benzisothiazol-5-yl]-1-methyl-6-(trifluoromethyl)-2,4(1H,3H)-pyrimidinedione, 2-furoate (ester);
5-[3,6-dihydro-3-methyl-2,6-dioxo-4-(trifluoromethyl)-1(2H)-pyrimidinyl]-N-(methylsulfonyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfonyl)-1,2-benzisothiazole-3-carboxamide;
5-[3,6-dihydro-3-methyl-2,6-dioxo-4-(trifluoromethyl)-1(2H)-pyrimidinyl]-N-(ethylsulfonyl)-1,2-benzisothiazole-3-carboxamide;
5-[3,6-dihydro-3-methyl-2,6-dioxo-4-(trifluoromethyl)-1(2H)-pyrimidinyl]-N-(isopropylsulfamoyl)-1,2-benzisothiazole-3-carboxamide; and
5-[3,6-dihydro-3-methyl-2,6-dioxo-4-(trifluoromethyl)-1(2H)-pyrimidinyl]-N-(dimethylsulfamoyl)-1,2-benzisothiazole-3-carboxamide.

17. The method according to any one of claims 9 to 16 wherein the compound is applied to the foliage of the undesirable plant species or to the soil or water containing seeds or other propagating organs thereof at a rate of about 0.001 kg/ha to 1 kg/ha.

18. A herbicidal composition which comprises an inert solid or liquid carrier and a herbicidally effective amount of a compound according to any one of claims 1 to 8.

19. An intermediate compound having the structural formula 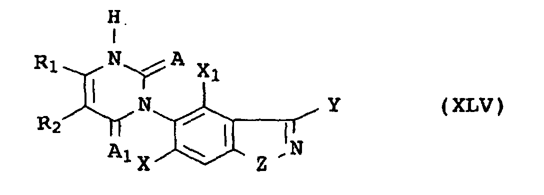 wherein R₁, R₂, X, X₁, Y, A, A₁ and Z are as defined in any one of claims 1, 2 and 4 and the optical isomers, diastereomers and/or tautomers thereof.

20. A process for preparing a compound of formula I or II as claimed in Claim 1 which comprises one of the following:
a) reacting a compound of formula VIII 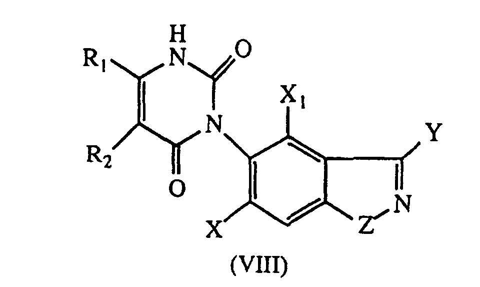 wherein R₁, R₂, X, X₁, Y and Z are as defined in Claim 1, with a compound of formula
**Z**_{**1**}**R** (IX)
wherein R is as defined in Claim 1 and Z₁ is chlorine, bromine or iodine to give a corresponding compound of formula I;
b) reacting a compound of formula XI 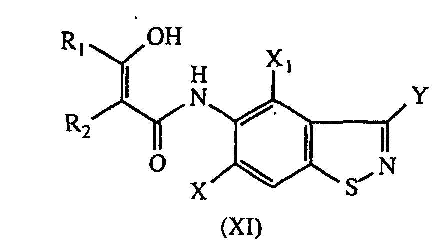 wherein R₁, R₂, X, X₁ and Y are as defined in Claim 1, with
(i) a compound of formula
C(A)Cl₂
wherein A is oxygen or sulphur followed by
(ii) an amine of formula XII
RNH₂ (XII)
wherein R is as defined in Claim 1, in the presence of base, e.g triethylamine or pyridine, to give a corresponding compound of formula I; wherein A is O or S;
c) reacting a compound of formula (I) wherein R, R₁, R₂, A, A₁, X, X₁ and Z are defined in Claim 1 and Y is CH_{3,} with a halogenating agent e.g N-bromosuccinimide, N-chlorosuccinimide, N-iodosuccinimide, bromine, chlorine, sulfuryl chloride and sulfuryl bromide to give a corresponding compound of formula I wherein Y is halomethyl;
d) reacting a compound of formula I, wherein R, R₁, R₂, A, A₁, X, X₁ and Z are defined in Claim 1 and Y is bromomethyl, with a C₁-C₂₀ alcohol, to give a corresponding compound of formula I wherein Y is CH₂O(C₁-C₂₀ alkyl);
e) reacting a compound of formula I wherein R, R₁, R₂, A, A₁, X, X₁ and Z are defined in Claim 1 and Y is bromomethyl with an alcohol or thiol of formula
**HX**_{**4**}**R**_{**22**} (XXI)
wherein X₄ and R₂₂ are as defined in Claim 1 in the presence of base, e.g sodium hydride, to give a corresponding compound of formula I wherein Y is CH₂X₄R₂₂;
f) reacting a compound of formula I wherein R, R₁, R₂, A, A₁, X, X₁ and Z are defined in Claim 1 and Y is bromomethyl, with an alkali metal thioalkoxide to give a corresponding compound of formula I wherein Y is CH₂S(C₁-C₈ alkyl);
g) reacting a compound of formula I wherein R, R₁, R₂, Y, X, X₁ and Z are defined in Claim 1 and A and A₁ are O, with Lawesson's Reagent in the presence of base, e.g NaHCO₃, to give a corresponding compound of formula I wherein A is O and A₁ is S;
h) reacting a compound of formula I wherein R, R₁, R₂, Y, X, X₁ and Z are defined in Claim 1 and A is S and A₁ is O, with Lawesson's Reagent in the presence of a base to give a corresponding compound of formula I wherein A and A₁ are S;
i) hydrolysing a compound of formula I wherein at least one of COOR₁₉, COOR₂₆ or COOR₃₂ is an ester where R₁₉, R₂₆ and R₃₂ are defined in Claim 1 to give a compound of formula I wherein at least one of R₁₉, R₂₆ or R₃₂ is hydrogen;
j) halogenating a compound of formula I wherein at least one of COOR₁₉, COOR₂₆ and COOR₃₂ is a carboxylic acid group, eg using thionyl chloride or oxalyl chloride, to give a corresponding acid chloride; and reacting said acid chloride with:
(i) an alcohol of formula R₁₉OH, R₂₆OH, or R₃₂OH or
(ii) an amine of formula HNR₂₀R₂₁, HNR₃₅R₃₆ or HNR₄₉R₅₀,
to give a corresponding compound of formula I containing a COOR₁₉, COOR₂₆, COOR₃₂, CONR₂₀R₂₁, CONR₃₅R₃₆ or CONR₄₉R₅₀ group wherein R₁₉, R₂₀, R₂₁, R₂₆, R₃₂, R₃₅, R₃₆, R₄₉ and R₅₀ are defined in Claim 1;
k) reacting a compound of formula I wherein at least one of R₁₉, R₂₆ and R₃₂ is hydrogen to give a corresponding compound of formula I wherein at least one of R₁₉, R₂₆ and R₃₂ is an alkali metal, alkaline earth metal, manganese, copper, zinc, cobalt, silver, nickel, ammonium or organic ammonium cation;
l) oxidizing a compound of formula I wherein R, R₁, R₂, A, A₁, X, X₁ and Z are defined in Claim 1 and Y is alkyl, cycloalkyl, alkenyl, cycloalkenyl, alkynyl or phenyl substituted with a CH₂OH group, with an oxidizing agent, e.g chromium (VI) oxide, to give a corresponding compound of formula I where Y is alkyl, cycloalkyl, alkenyl, cycloalkenyl, alkynyl or phenyl substituted with a CHO group.
m) reacting a compound of formula I wherein R, R₁, R₂, A, A₁, X, X₁ and Z are as defined in Claim 1 and Y is methyl, with ceric ammonium nitrate to give a corresponding compound of formula I wherein Y is CHO;
n) reacting a compound of formula I wherein R, R₁, R₂, A, A₁, X, X₁ and Z are as defined in Claim 1 and Y is CH₂Br, with potassium cyanide to give a corresponding compound of formula I wherein Y is CH₂CN;
o) reacting a compound of formula I wherein R, R₁, R₂, A, A₁, X, X₁ and Z are asdefined in Claim 1 and R₁₂ is bromoalkyl, with lithium chloride, sodium iodide, potassium fluoride or silver (I) fluoride to give a corresponding compound of formula I wherein R₁₂ is chloroalkyl, fluoroalkyl or iodoalkyl;
p) reacting a compound of formula: 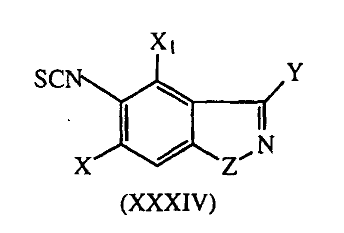 wherein X, X₁, Y and Z are defined in Claim 1 with an alkene of formula: 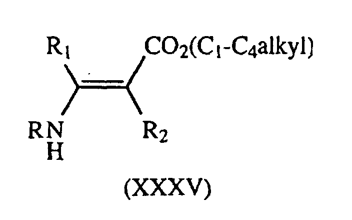 wherein R, R₁, and R₂ are as defined in Claim 1 in the presence of base, e.g sodium hydride, to give a corresponding compound of formula I wherein R, R₁, R₂, X, X₁, Y and Z are defined in Claim 1 and A is S and A₁ is O;
q) reacting a compound of formula I wherein R, R₁, R₂, A, A₁, X, X₁, and Z are as defined in Claim 1 and Y is COOH, with (i) oxalyl chloride or thionyl chloride followed by (ii) HX₂,R₁₂, wherein X₂ and R₁₂ are defined in Claim 1, in the presence of base to give a corresponding compound of formula I wherein Y is C(O)X₂R₁₂ and X₂, R₁₂ are as defined in Claim 1;
r) reacting a compound of formula I wherein R, R₁, R₂, A, A₁, X, X₁ and Z are defined in Claim 1 and Y is COOH, with
(i) a coupling agent, e.g 1-(3-dimethylaminopropyl)-3-ethylcarbodiimide, and
(ii) HNR₁₂,R₁₃, wherein R₁₂ and R₁₃ are defined in Claim 1
to give a corresponding compound of formula I wherein Y is C(O)NR₁₂R₁₃, and R₁₂ and R₁₃ are defined in Claim 1;
s) reacting a compound of formula I wherein R, R₁, R₂, A, A₁, X, X₁ and Z are defined in Claim 1 and Y is COOH, with
(i) oxalyl chloride or thionyl chloride and
(ii) HNR₁₃SO₂NR₄₆R₄₈, wherein R₁₃, R₄₆ and R₄₈ are defined in Claim 1 to give a corresponding compound of formula I wherein Y is C(O)NR₁₃SO₂NR₄₆R₄₈ and R₁₃, R₄₆ and R₄₈ are defined in Claim 1;
t) reacting a compound of formula I wherein R, R₁, R₂, X, X₁, A, A₁, and Z are defined in Claim 1, and Y is COOH, with
(i) oxalyl chloride or thionyl chloride and
(ii) HN(R₁₂)OR₂₃, wherein R₁₂ and R₂₃ are defined in Claim 1,
to give a corresponding compound of formula I wherein Y is C(O)N(R₁₂)OR₂₃ and R₁₂, R₂₃ are defined in Claim 1;
u) reacting a compound of formula I wherein R, R₁, R₂, X, X₁, A, A₁, and Z are defined in Claim 1, and Y is CHO with R₂₃MgBr wherein R₂₃ is defined in Claim I to give a corresponding compound of formula I wherein Y is C(OH)R₂₃;
v) reacting a compound of formula I wherein R, R₁, R₂, A, A₁, X, X₁ and Z are defined in Claim 1 and Y is C(OH)R₂₃, with an oxidizing agent to form a corresponding compound of formula I wherein Y is C(O)R₂₃, wherein R₂₃ is defined in Claim 1;
w) reacting a compound of formula I wherein R, R₁, R₂, A, A₁, X, X₁ and Z are defined in Claim 1 and Y is C(OH)R₂₃, with R₂₂X wherein R₂₂ is defined in Claim 1 and X is chlorine or bromine, in the presence of base to form a corresponding compound of formula I wherein Y is C(OR₂₂)R₂₃, wherein R₂₂ and R₂₃ are defined in Claim 1;
x) reacting a compound of formula I wherein R, R₁, R₂, A, A₁, X, X₁ and Z are defined in Claim 1 and Y is C(OH)R₂₃ wherein R₂₃ is defined in claim 1, with XC(O)R₃₃, wherein R₃₃ is defined in Claim 1 and X is chlorine or bromine, to give a corresponding compound of formula I wherein Y is CH(R₂₃)OC(O)R₃₃, wherein R₂₃ and R₃₃ are defined in claim 1.
or
y) converting a compound of formula (I) or (II) having a reactive substituent group to a different compound of formula (I) or (II) using an appropriate reagent.

21. A process for preparing an intermediate of formula (XLV) as claimed in Claim 19 which comprises one of the following:
a) reacting a compound of formula: 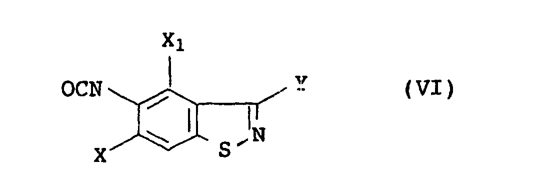 wherein X, X₁ and Y are defined in Claim 1
with a compound of formula:
R₁C(NH₂)=CR₂-CO₂(C₁-C₄-alkyl) (VIII)
wherein R₁ and R₂ are defined in Claim 1
to produce a compound of formula: 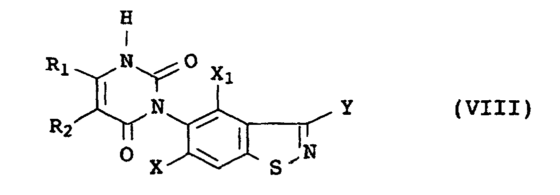 wherein R₁, R₂, X, X₁ and Y are defined in Claim 1;
b) reacting a compound of formula: 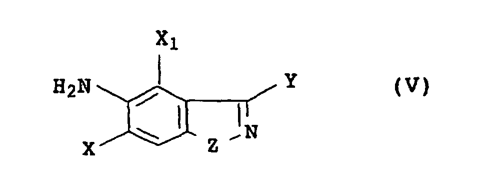 wherein X, X₁, Z and Y are defined in Claim 1
with a compound of formula: 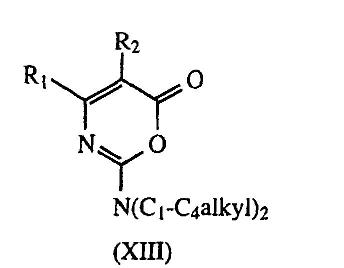 wherein R₁ and R₂ are defined in Claim 1, in the presence of an organic acid e.g acetic acid, to form a compound of formula VIII as shown above wherein R₁, R₂, X, X₁ and Y are defined in Claim 1;
or
c) reacting a compound of formula XXXVIII: 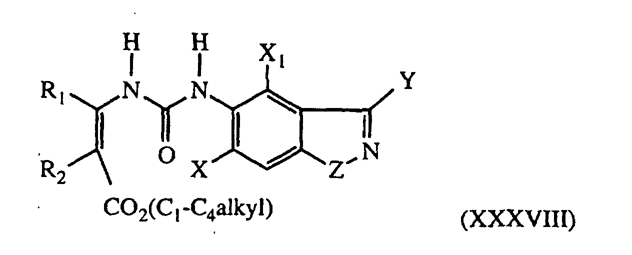 wherein R₁, R₂, X, X₁, Z and Y are defined in Claim 1, with acid to form a compound of formula: 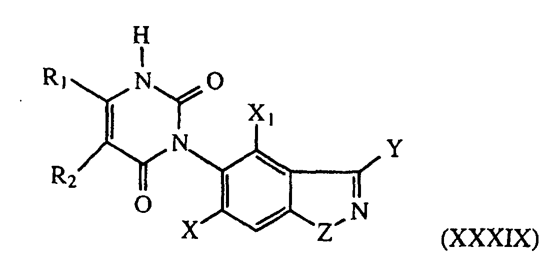 wherein R₁, R₂, X, X₁, Y and Z are defined in Claim 1.

## Patentansprüche

1. Verbindungen mit der Strukturformel 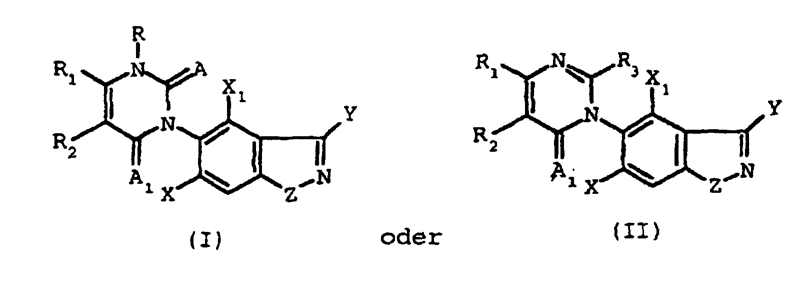 wobei
R für C₁-C₆-Alkyl, C₁-C₆-Halogenalkyl, C₃-C₇-Cycloalkyl, C₃-C₆-Alkenyl, C₃-C₆-Alkinyl, Benzyl, OR₄ oder NR₅R₆ steht;
R₁ und R₂ jeweils unabhängig voneinander für Wasserstoff, Halogen, C₁-C₆-Alkyl, C₃-C₇-Cycloalkyl, C₁-C₆-Halogenalkyl, C₃-C₆-Alkenyl, C₃-C₆-Halogenalkenyl, C₃-C₆-Alkinyl, OR₇, S(O)ₙR₈ oder NR₉R₁₀ stehen und R₁ und R₂ zusammen mit den Atomen, an die sie gebunden sind, für einen vier- bis siebengliedrigen, gesättigten oder ungesättigten Ring, der gegebenenfalls durch Sauerstoff, Schwefel oder Stickstoff unterbrochen und gegebenenfalls durch ein bis drei Methylgruppen oder ein oder mehrere Halogenatome substituiert ist, stehen;
R₃ für Halogen oder A₂R₁₁ steht;
X für Wasserstoff, Halogen oder C₁-C₄-Alkyl steht;
X₁ für Wasserstoff, Halogen, C₁-C₄-Alkyl, C₁-C₄-Halogenalkyl, C₁-C₄-Alkoxy oder C₁-C₄-Halogenalkoxy steht;
Z für O oder S(O)ₘ steht;
Y für Halogen, Cyano, R₁₂, X₂R₁₂, X₃R₁₂, X₂X₃R₁₂ oder X₃X₂R₁₂ steht;
X₂ für O, S(O)ₚ oder NR₁₃ steht;
X₃ für -C(=A₃)-, -C(=NOR₁₄)- oder -C(=N-NR₁₃R₅₀) steht;
R₄, R₅, R₆, R₇, R₈, R₉, R₁₀, R₁₁ und R₁₄ jeweils unabhängig voneinander für Wasserstoff, C₁-C₆-Alkyl, C₃-C₇-Cycloalkyl, C₂-C₈-Alkoxyalkyl, C₂-C₆-Alkenyl, C₂-C₆-Alkinyl oder Benzyl stehen;
R₁₂ für Wasserstoff steht,
eine C₁-C₈-Alkyl-, C₃-C₇-Cycloalkyl-, C₂-C₈-Alkenyl-, C₅-C₆-Cycloalkenyl- oder C₂-C₈-Alkinylgruppe steht, wobei die einzelnen Gruppen jeweils gegebenenfalls substituiert sind durch eine beliebige Kombination von ein bis sechs Halogenatomen, einer bis drei C₁-C₂₀-Alkoxygruppen, gegebenenfalls substituiert durch eine C₁-C₆-Alkoxy-, Benzyloxy- oder C₁-C₆-Alkylthiogruppe, einer oder zwei C₁-C₁₀-Halogenalkoxygruppen, einer oder zwei NR₁₅R₁₆-Gruppen, einer bis drei S(O)_{q}R₁₇-Gruppen, einer oder zwei Cyanogruppen, einer oder zwei C₃-C₇-Cycloalkylgruppen, einer Thiocyanogruppe, einer O(SO₂)R₁₇-Gruppe, einer O(NO₂)-Gruppe, einer oder zwei C(O)R₁₈-Gruppen, einer oder zwei CO₂R₁₉-Gruppen, einer oder zwei C(O)SR₁₉-Gruppen, einer oder zwei C(O)NR₂₀R₂₁-Gruppen, einer bis drei X₄R₂₂-Gruppen, einer oder zwei P(O) (OR₂₃)₂-Gruppen, einer oder zwei Si(R₂₄)₃-Gruppen,
einem 4- bis 10-gliedrigen monocyclischen oder kondensierten bicyclischen heterocyclischen Ring, gegebenenfalls substituiert durch eine beliebige Kombination von einer oder zwei Oxogruppen, einer oder zwei Thioxogruppen, ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen oder einer oder zwei X₄R₂₂-Gruppen,
einer quaternären organischen Ammoniumgruppe, oder
einer oder zwei Phenylgruppen, wobei die einzelnen Phenylgruppen jeweils unabhängig voneinander gegebenenfalls durch eine beliebige Kombination von ein bis drei Halogenatomen, einer bis drei C₁-C₆-Alkylgruppen, einer bis drei C₁-C₆-Alkoxygruppen, einer C₃-C₇-Cycloalkylgruppe, einer C₁-C₄-Halogenalkylgruppe, einer C₁-C₄-Alkylthiogruppe, einer Cyanogruppe, einer Nitrogruppe, einer C(O)R₂₅-Gruppe, einer CO₂R₂₆-Gruppe oder einer X₅R₂₇-Gruppe substituiert sind,
für Phenyl steht, das gegebenenfalls durch eine beliebige Kombination von ein bis drei Halogenatomen. einer bis drei C₁-C₄-Alkylgruppen, einer bis drei C₁-C₄-Alkoxygruppen, einer C₃-C₆-Cycloalkylgruppe, einer C₁-C₄-Halogenalkylgruppe, einer C₁-C₄-Alkylthiogruppe, einer Cyanogruppe, einer Nitrogruppe, einer C(O)R₂₅-Gruppe, einer CO₂R₂₆-Gruppe oder einer X₄R₂₂-Gruppe, einer CH=CHR₂₂-Gruppe, einer CH₂CH (R₂₈) R₂₂-Gruppe oder einer N (R₂₉) SO₂R₃₀-Gruppe substituiert ist, oder
für Indan steht, das gegebenenfalls durch eine beliebige Kombination von ein oder zwei Halogenatomen, einer oder zwei C₁-C₄-Alkylgruppen, einer C₁-C₄-Alkoxygruppe, einer C₁-C₄Halogenalkylgruppe, einer C₁-C₄-Alkylthiogruppe, einer Cyanogruppe, einer Nitrogruppe, einer C(O)R₂₅-Gruppe, einer CO₂R₂₆-Gruppe oder einer X₄R₂₂-Gruppe, einer CH=CHR₂₂-Gruppe, einer CH₂CH(R₂₈)R₂₂-Gruppe oder einer N (R₂₉)SO₂R₃₀-Gruppe substituiert ist;
R₁₃ für Wasserstoff, C₁-C₈-Alkoxyalkyl, C₁-C₆-Alkyl, C₃-C₇-Cycloalkyl, C₃-C₆-Alkenyl, C₃-C₆-Alkinyl, Benzyl, OR₂₃, Cyano, SO₂R₅₁ oder einen 5- bis 12-gliedrigen monocyclischen oder kondensierten bicyclischen heterocyclischen Ring steht, der gegebenenfalls durch ein oder mehrere Gruppen unabhängig voneinander ausgewählt aus Halogen, Cyano, Nitro, Amino, Hydroxyl, C₁-C₄-Alkyl, C₁-C₄-Halogenalkyl, C₁-C₄-Alkoxy oder C₁-C₄-Halogenalkoxy substituiert ist, und R₁₂ und R₁₃ zusammen mit dem Stickstoffatom, an das sie gebunden sind, einen 5- bis 12-gliedrigen monocyclischen oder kondensierten bicyclischen heterocyclischen Ring bilden, der gegebenenfalls durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy-, C₁-C₄-Halogenalkylsulfonyl- oder X₄R₂₂-Gruppen substituiert ist;
R₂₈ für Halogen steht;
R₂₂ und R₂₇ jeweils unabhängig voneinander für Wasserstoff, Si (R₃₁)₃, C(O)NR₃₅R₃₆, C(O)R₃₃, SO₂R₄₇,
C₁-C₂₀-Alkyl, substituiert durch eine Hydroxyl-, Benzyloxy-, Nitro-, OC(O)R₃₃-, C₁-C₆-Alkoxy-, CO₂R₃₂-, C(O)R₃₃-, (C(A₄R₃₄)₂-, C(O)NR₃₅R₃₆-, C(O)ON=OR₃₇R₃₈-, P(O)(OH)NH₂-, P(O)(OH) (OR₂₃)-, C(O)NR₃₅R₂₃-, Cyano- oder 2-Dioxolanylgruppe, einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert sind, oder
einen 5- bis 12-gliedrigen monocyclischen oder kondensierten bicyclischen heterocyclischen Ring, gegebenenfalls substituiert durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy- oder C₁-C₄-Halogenalkylsulfonylgruppen,
C₃-C₂₀-Alkenyl, gegebenenfalls substituiert durch ein bis drei Halogenatome, eine Hydroxylgruppe, eine C₁-C₆-Alkoxy-, CO₂R₃₂-, C(O)R₃₃-, (C(A₄R₃₄)₂-, C(O)NR₃₅R₃₆-, C(O)ON=CR₃₇R₃₈-, C(O)NR₃₅R₂₃-, Cyano- oder 2-Dioxolanylgruppe, oder einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert sind,
C₅-C₈-Cycloalkenyl, gegebenenfalls substituiert durch eine Hydroxyl-, C₁-C₆-Alkoxy-, C₁-C₄-Alkyl-, C₂-C₄-Alkenyl-, CO₂R₃₂-, C(O)R₃₃-, (C(A₄R₃₄)₂-, C(O)NR₃₅R₃₆-, C(O)ON=CR₃₇R₃₈-, C(O)NR₃₅R₂₃-, Cyano- oder 2-Dioxolanylgruppe, oder einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Alkoxygruppen oder einer bis drei C₁-C₄-Halogenalkoxygruppen substituiert sind,
C₃-C₁₀-Alkinyl, gegebenenfalls substituiert durch eine Hydroxyl-, C₁-C₆-Alkoxy-, CO₂R₃₂-, C(O)R₃₃-, (C(A₄R₃₄)₂-, C(O)NR₃₅R₃₆-, C(O)ON=CR₃₇R₃₈-, C(O)NR₃₅R₂₃-, Cyano- oder 2-Dioxolanylgruppe, oder einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert sind,
Phenyl, gegebenenfalls substituiert durch eine beliebige Kombination von ein bis drei Halogenatomen, einer bis drei C₁-C₆-Alkylgruppen, einer bis drei C₁-C₆-Alkoxygruppen, einer C₁-C₄-Halogenalkylgruppe, einer C₁-C₄-Halogenalkoxygruppe, einer Cyanogruppe, einer Nitrogruppe, einer NR₃₉R₄₀-Gruppe oder einer C(O)R₃₃-Gruppe,
Benzyl, am Phenylring gegebenenfalls substituiert durch eine beliebige Kombination von ein bis drei Halogenatomen, einer bis drei C₁-C₆-Alkylgruppen, einer bis drei C₁-C₆-Alkoxygruppen, einer C₁-C₄-Halogenalkylgruppe, einer C₁-C₄-Halogenalkoxygruppe, einer Cyanogruppe, einer Nitrogruppe, einer NR₃₉R₄₀-Gruppe oder einer C(O)R₃₃-Gruppe, oder
einen 4- bis 10-gliedrigen heterocyclischen Ring, gegebenenfalls substituiert durch eine beliebige Kombination von einer oder zwei Oxogruppen, einer oder zwei Thioxogruppen, ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen,
stehen;
R₁₅ und R₂₀ jeweils unabhängig voneinander für Wasserstoff,
eine C₁-C₆-Alkyl, C₃-C₇-Cycloalkyl-, C₂-C₆-Alkenyl- oder C₂-C₆-Alkinylgruppe, wobei jede Gruppe gegebenenfalls durch eine CO₂R₄₂-Gruppe substituiert ist,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen,
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkylgruppen, einer CO₂R₂₆-Gruppe,
C(O)R₄₃, oder
Co₂R₄₂ stehen;
R₁₆ und R₂₁ jeweils unabhängig voneinander für Wasserstoff, C₁-C₆-Alkyl,
C₁-C₄-Alkoxy, C₃-C₇-Cycloalkyl, C₂-C₆-Alkenyl, C₃-C₇-Cycloalkenyl, C₂-C₆-Alkinyl, P(O)(OR₄₄)₂, SO₂R₄₅, C(O)R₄₆,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, oder einer X₄R₂₂-Gruppe, oder
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen oder einer X₄R₂₂-Gruppe, stehen;
R₁₇, R₁₈, R₂₅, R₃₀, R₃₃, R₄₅ und R₅₁ jeweils unabhängig voneinander für NR₄₆R₄₈, -N=CR₄₆R₄₈, C₁-C₈-Alkyl, gegebenenfalls substituiert durch eine C₁-C₆-Alkoxy-, Thiophen- oder Furangruppe, C₁-C₆-Halogenalkyl, C₃-C₈-Cycloalkyl, CO₂R₂₃, C₂-C₆-Alkenyl, gegebenenfalls substituiert durch Phenyl,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Nitrogruppe, einer Cyanogruppe oder einer X₄R₂₂-Gruppe,
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Nitrogruppe, einer Cyanogruppe oder einer X₄R₂₂-Gruppe, oder
einen 5- bis 12-gliedrigen monocyclischen oder kondensierten bicyclischen heterocyclischen Ring, gegebenenfalls substituiert durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy-, C₁-C₄-Halogenalkylsulfonyl- oder X₄R₂₂-Gruppen,
stehen;
R₁₉ und R₂₆ jeweils unabhängig voneinander für Wasserstoff, C₁-C₂₀-Alkyl, C₁-C₈-Halogenalkyl, C₃-C₇-Cycloalkyl, C₃-C₇-Halogencycloalkyl, C₃-C₂₀-Alkenyl, C₃-C₈-Halogenalkenyl, C₅-C₈-Cycloalkenyl, C₅-C₈-Halogencycloalkenyl, C₃-C₈-Alkinyl, C₃-C₈-Halogenalkinyl, Si(R₄₁)₃, Benzyl, Phenyl, Furfuryl, Pyridyl, Thienyl, Oximino oder ein Alkalimetall-, Erdalkalimetall-, Mangan-, Kupfer-, Zink-, Cobalt-, Silber-, Nickel-, Ammonium- oder organisches Ammoniumkation stehen;
R₃₂ für Wasserstoff,
Si(R₄₇)₃,
Di (C₁-C₄-alkyl)amino,
C₁-C₂₀-Alkyl, gegebenenfalls substituiert durch eine CO₂R₄₈-, C₁-C₆-Alkoxy-, C₁-C₆-Thioalkyl-, C₂-C₂₀-Alkoxyalkoxy-, Phenyl-, OSi(R₄₇)₃-, OC(O)R₃₃-, C₃-C₇-Cycloalkyl- oder Di(C₁-C₄-alkyl)aminogruppe,
C₁-C₁₅-Halogenalkyl,
C₃-C₈-Cycloalkyl, gegebenenfalls substituiert durch eine CO₂R₄₈-Gruppe,
C₃-C₈-Halogencycloalkyl,
C₃-C₂₀-Alkenyl, gegebenenfalls substituiert durch eine Phenylgruppe,
C₃-C₁₅-Halogenalkenyl,
C₅-C₈-Cycloalkenyl,
C₅-C₈-Halogencycloalkenyl,
C₃-C₂₀-Alkinyl, gegebenenfalls substituiert durch eine Phenylgruppe,
C₃-C₂₀-Halogenalkinyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
2-, 3- oder 4-Pyridyl,
2- oder 3-Furyl,
2- oder 3-Thienyl,
2-Tetrahydrofuranyl,
C₁-C₄-Alkyl, substituiert durch eine Pyridyl-, Furyl-, Thienyl-, Tetrahydrofuryl-, C₃-C₈-Cycloalkyl-, Dioxan- oder NR₄₉R₅₀-Gruppe, oder
ein Alkalimetall-, Erdalkalimetall-, Mangan-, Kupfer-, Zink-, Cobalt-, Silber-, Nickel-, Ammonium- oder organisches Ammoniumkation, steht;
R₃₄, R₃₇ und R₃₈ jeweils unabhängig voneinander für C₁-C₆-Alkyl stehen;
R₃₅ und R₄₉ jeweils unabhängig voneinander für Wasserstoff oder C₁-C₆-Alkyl stehen und R₃₆ und R₅₀ jeweils unabhängig voneinander für Wasserstoff, Cyano, C₁-C₆-Alkyl, SO₂R₅₁ oder Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen, oder R₃₅ und R₃₆ oder R₄₉ und R₅₀ zusammen mit dem Stickstoffatom, an das sie gebunden sind, einen 5- bis 12-gliedrigen monocyclischen oder kondensierten bicyclischen heterocyclischen Ring bilden, der gegebenenfalls durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy- oder C₁-C₄-Halogenalkylsulfonylgruppen, substituiert ist;
m, n, p und q jeweils unabhängig voneinander für ganze Zahlen 0, 1 oder 2 stehen;
R₂₉ für Wasserstoff, C₁-C₄-Alkyl oder C₁-C₄-Halogenalkyl steht;
R₂₄, R₃₁, R₄₁ und R₄₇ jeweils unabhängig voneinander für C₁-C₄-Alkyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, oder
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen;
R₂₃, R₃₉, R₄₀, R₄₂, R₄₃, R₄₄, R₄₆ und R₄₈ jeweils unabhängig voneinander für Wasserstoff, C₂-C₆-Alkenyl, C₃-C₈-Cycloalkyl,
C₁-C₈-Alkyl, gegebenenfalls substituiert durch ein oder zwei C₁-C₄-Alkoxygruppen,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, oder
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen, und
R₄₆ und R₄₈ zusammen mit dem Atom, an das sie gebunden sind, einen 5- bis 12-gliedrigen monocyclischen oder kondensierten bicyclischen heterocyclischen oder Cycloalkylring bilden, der gegebenenfalls durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy- oder C₁-C₄-Halogenalkylsulfonylgruppen substituiert ist; und
A, A₁, A₂, A₃, A₄, X₄ und X₅ jeweils unabhängig voneinander für O oder S stehen; und
die optischen Isomere, Diastereomere und/oder Tautomere davon.

2. Verbindungen nach Anspruch 1, wobei
R für C₁-C₆-Alkyl, C₁-C₆-Halogenalkyl, C₃-C₇-Cycloalkyl, C₃-C₆-Alkenyl, C₃-C₆-Alkinyl, Benzyl, OR₄ oder NR₅R₆ steht;
R₁ und R₂ jeweils unabhängig voneinander für Wasserstoff, Halogen, C₁₋C₆-Alkyl, C₃₋C₇-Cycloalkyl, C₁-C₆-Halogenalkyl, C₃-C₆-Alkenyl, C₃-C₆-Halogenalkenyl, C₃-C₆-Alkinyl, OR7, S(O)ₙR₈ oder NR₉R₁₀ stehen und R₁ und R₂ zusammen mit den Atomen, an die sie gebunden sind, für einen vier- bis siebengliedrigen, gesättigten oder ungesättigten Ring, der gegebenenfalls durch Sauerstoff, Schwefel oder Stickstoff unterbrochen und gegebenenfalls durch ein bis drei Methylgruppen oder ein oder mehrere Halogenatome substituiert ist, stehen;
R₃ für Halogen oder A₂R₁₁ steht;
X für Wasserstoff, Halogen oder C₁-C₄-Alkyl steht;
X₁ für Wasserstoff, Halogen, C₁-C₄-Alkyl, C₁-C₄-Halogenalkyl, C₁-C₄-Alkoxy oder C₁-C₄-Halogenalkoxy steht;
Z für O oder S(O)ₘ steht;
Y für Halogen, Cyano, R₁₂, X₂R₁₂, X₃R₁₂, X₂X₃R₁₂ oder X₃X₂R₁₂ steht;
X₂ für O, S(O)ₚ oder NR₁₃ steht;
X₃ für -C(=A₃)- oder -C(=NOR₁₄)- steht;
R₄, R₅, R₆, R₇, R₈, R₉, R₁₀, R₁₁ und R₁₄ jeweils unabhängig voneinander für Wasserstoff, C₁-C₆-Alkyl, C₃-C₇-Cycloalkyl, C₂-C₆-Alkenyl, C₂-C₆-Alkinyl oder Benzyl stehen;
R₁₂ für Wasserstoff steht,
eine C₁-C₆-Alkyl-, C₃-C₇-Cycloalkyl-, C₂-C₆-Alkenyl-, C₅-C₆-Cycloalkenyl- oder C₂-C₆-Alkinylgruppe steht, wobei die einzelnen Gruppen jeweils gegebenenfalls substituiert sind durch eine beliebige Kombination von ein bis sechs Halogenatomen, einer bis drei C₁-C₂₀-Alkoxygruppen, einer oder zwei C₁-C₁₀-Halogenalkoxygruppen, einer oder zwei NR₁₅R₁₆-Gruppen, einer bis drei S(O)_{q}R₁₇-Gruppen, einer oder zwei Cyanogruppen, einer oder zwei C(O)R₁₈-Gruppen, einer oder zwei CO₂R₁₉-Gruppen, einer oder zwei C(O)NR₂₀R₂₁-Gruppen, einer bis drei X₄R₂₂-Gruppen, einer oder zwei P(O)(OR₂₃)₂-Gruppen, einer oder zwei Si(R₂₄)₃-Gruppen,
einem 4- bis 10-gliedrigen monocyclischen oder kondensierten bicyclischen heterocyclischen Ring, gegebenenfalls substituiert durch eine beliebige Kombination von einer oder zwei Oxogruppen, einer oder zwei Thioxogruppen, ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogen-alkoxygruppen oder einer oder zwei X₄R₂₂-Gruppen,
einer quaternären organischen Ammoniumgruppe, oder
einer Phenylgruppe, gegebenenfalls substituiert durch eine beliebige Kombination von ein bis drei Halogenatomen, einer bis drei C₁-C₆-Alkylgruppen, einer bis drei C₁-C₆-Alkoxygruppen, einer C₃-C₇-Cycloalkylgruppe, einer C₁-C₄-Halogenalkylgruppe, einer C₁-C₄-Alkylthiogruppe, einer Cyanogruppe, einer Nitrogruppe, einer C(O)R₂₅-Gruppe, einer CO₂R₂₆-Gruppe oder einer X₅R₂₇-Gruppe, oder
für Phenyl steht, das gegebenenfalls durch eine beliebige Kombination von ein bis drei Halogenatomen, einer bis drei C₁-C₄-Alkylgruppen, einer bis drei C₁-C₄-Alkoxygruppen, einer C₃-C₆-Cycloalkylgruppe, einer C₁-C₄-Halogenalkylgruppe, einer C₁-C₄-Alkylthiogruppe, einer Cyanogruppe, einer Nitrogruppe, einer C(O)R₂₅-Gruppe, einer CO₂R₂₆-Gruppe oder einer X₄R₂₂-Gruppe, einer CH=CHR₂₂-Gruppe, einer CH₂CH(R₂₈)R₂₂-Gruppe oder einer N(R₂₉)SO₂R₃₀-Gruppe substituiert ist, oder
und R₁₂ und R₁₃ zusammen mit dem Stickstoffatom, an das sie gebunden sind, einen 5- bis 12-gliedrigen monocyclischen oder kondensierten bicyclischen heterocyclischen Ring bilden, der gegebenenfalls durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy-, C₁-C₄-Halogenalkylsulfonyl- oder X₄R₂₂-Gruppen substituiert ist;
R₁₃ für Wasserstoff, C₁-C₆-Alkyl, C₃-C₇-Cycloalkyl, C₃-C₆-Alkenyl, C₃-C₆-Alkinyl, Benzyl, OR₂₃, Cyano oder SO₂R₅₁ steht, und R₁₃ und R₁₂ zusammen mit dem Stickstoffatom, an das sie gebunden sind, einen 5- bis 12-gliedrigen monocyclischen oder kondensierten bicyclischen heterocyclischen Ring bilden, der gegebenenfalls durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy-, C₁-C₄-Halogenalkylsulfonyl- oder X₄R₂₂-Gruppen substituiert ist;
R₂₈ für Halogen steht;
R₂₂ und R₂₇ jeweils unabhängig voneinander für Wasserstoff, Si(R₃₁)₃,
C₁-C₂₀-Alkyl, substituiert durch eine C₁-C₆-Alkoxy-, CO₂R₃₂-, C(O)R₃₃₋, (C(A₄R₃₄)₂-, C(O)NR₃₅R₃₆-, C(O)ON=CR₃₇R₃₈-, P(O)(OH)NH₂-, P(O)(OH)(OR₂₃)-, C(O)NHOR₂₃-, Cyano- oder 2-Dioxolanylgruppe, einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert sind, oder
einen 5- bis 12-gliedrigen monocyclischen oder kondensierten bicyclischen heterocyclischen Ring, gegebenenfalls substituiert durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy- oder C₁-C₄-Halogenalkylsulfonylgruppen,
C₃-C₂₀-Alkenyl, gegebenenfalls substituiert durch ein bis drei Halogenatome, eine C₁-C₆-Alkoxy-, CO₂R₃₂-, C (O)R₃₃₋, (C(A₄R₃₄)₂-, C(O)NR₃₅R₃₆-, C(O)ON=CR₃₇R₃₈-, C(O)NHOR₂₃-, Cyano- oder 2-Dioxolanylgruppe, oder einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert sind,
C₅-C₈-Cycloalkenyl, gegebenenfalls substituiert durch eine C₁-C₆-Alkoxy-, C₁-C₄-Alkyl-, C₂-C₄-Alkenyl-, CO₂R₃₂-, C(O)R₃₃-, (C(A₄R₃₄)₂-, C(O)NR₃₅R₃₆-, C(O)ON=CR₃₇R₃₈-, C(O)NHOR₂₃-, Cyano- oder 2-Dioxolanylgruppe, oder einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Alkoxygruppen oder einer bis drei C₁-C₄-Halogenalkoxygruppen substituiert sind,
C₃-C₁₀-Alkinyl, gegebenenfalls substituiert durch eine C₁-C₆-Alkoxy-, CO₂R₃₂-, C(O)R₃₃-, (C(A₄R₃₄)₂-, C(O)NR₃₅R₃₆-, C(O)ON=CR₃₇R₃₈-, C(O)NHOR₂₃-, Cyano- oder 2-Dioxolanylgruppe, oder einen Phenyl-, Dihydrofuranon- oder Furanonring, wobei die einzelnen Ringe jeweils gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert sind,
Phenyl, gegebenenfalls substituiert durch eine beliebige Kombination von ein bis drei Halogenatomen, einer bis drei C₁-C₆-Alkylgruppen, einer bis drei C₁-C₆-Alkoxygruppen, einer C₁-C₄-Halogenalkylgruppe, einer C₁-C₄-Halogenalkoxygruppe, einer Cyanogruppe, einer Nitrogruppe, einer NR₃₉R₄₀-Gruppe oder einer C(O)R₃₃-Gruppe,
Benzyl, am Phenylring gegebenenfalls substituiert durch eine beliebige Kombination von ein bis drei Halogenatomen, einer bis drei C₁-C₆-Alkylgruppen, einer bis drei C₁-C₆-Alkoxygruppen, einer C₁-C₄-Halogenalkylgruppe, einer C₁-C₄-Halogenalkoxygruppe, einer Cyanogruppe, einer Nitrogruppe, einer NR₃₉R₄₀-Gruppe oder einer C(O)R₃₃-Gruppe, oder
einen 4- bis 10-gliedrigen heterocyclischen Ring, gegebenenfalls substituiert durch eine beliebige Kombination von einer oder zwei Oxogruppen, einer oder zwei Thioxogruppen, ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen,
stehen;
R₁₅ und R₂₀ jeweils unabhängig voneinander für Wasserstoff,
eine C₁-C₆-Alkyl-, C₃-C₇-Cycloalkyl-, C₂-C₆-Alkenyl- oder C₂-C₆-Alkinylgruppe, wobei jede Gruppe gegebenenfalls durch eine CO₂R₄₂-Gruppe substituiert ist,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen,
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkylgruppen, oder
C(O)R₄₃
stehen;
R₁₆ und R₂₁ jeweils unabhängig voneinander für Wasserstoff, C₁-C₆-Alkyl, C₃-C₇-Cycloalkyl, C₂-C₆-Alkenyl, C₃-C₇-Cycloalkenyl, C₂-C₆-Alkinyl, P(O)(OR₄₄)₂, SO₂R₄₅, C(O)R₄₆,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, oder einer X₄R₂₂-Gruppe, oder
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, oder einer X₄R₂₂-Gruppe, stehen;
R₁₇, R₁₈, R₂₅, R₃₀, R₃₃, R₄₅ und R₅₁ jeweils unabhängig voneinander für C₁-C₄-Alkyl, C₁-C₄-Halogenalkyl,
CO₂R₂₃,
C₂-C₆-Alkenyl, gegebenenfalls substituiert durch Phenyl,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Nitrogruppe, einer Cyanogruppe oder einer X₄R₂₂-Gruppe,
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Nitrogruppe, einer Cyanogruppe oder einer X₄R₂₂-Gruppe, oder
einen 5- bis 12-gliedrigen monocyclischen oder kondensierten bicyclischen heterocyclischen Ring, gegebenenfalls substituiert durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy-, C₁-C₄-Halogenalkylsulfonyl- oder X₄R₂₂-Gruppen,
stehen;
R₁₉ und R₂₆ jeweils unabhängig voneinander für Wasserstoff, C₁-C₂₀-Alkyl, C₁-C₈-Halogenalkyl, C₃-C₇-Cycloalkyl, C₃-C₇-Halogencycloalkyl, C₃-C₂₀-Alkenyl, C₃-C₈-Halogenalkenyl, C₅-C₈-Cycloalkenyl, C₅-C₈-Halogencycloalkenyl, C₃-C₈-Alkinyl, C₃-C₈-Halogenalkinyl, Si(R₄₁)₃, Benzyl, Phenyl, Furfuryl, Pyridyl, Thienyl, Oximino oder ein Alkalimetall-, Erdalkalimetall-, Mangan-, Kupfer-, Zink-, Cobalt-, Silber-, Nickel-, Ammonium- oder organisches Ammoniumkation stehen;
R₃₂ für Wasserstoff,
Si(R₄₇)₃,
Di((C₁-C₄-alkyl)amino,
C₁-C₂₀-Alkyl, gegebenenfalls substituiert durch eine CO₂R₄₈-, C₁-C₆-Alkoxy-, oder Di(C₁-C₄-alkyl) aminogruppe,
C₁-C₁₅-Halogenalkyl,
C₃-C₈-Cycloalkyl,
C₃-C₈-Halogencycloalkyl,
C₃-C₂₀-Alkenyl, gegebenenfalls substituiert durch eine Phenylgruppe,
C₃-C₁₅-Halogenalkenyl,
C₅-C₈-Cycloalkenyl,
C₅-C₈-Halogencycloalkenyl,
C₃-C₂₀-Alkinyl, gegebenenfalls substituiert durch eine Phenylgruppe,
C₃-C₂₀-Halogenalkinyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
2-, 3- oder 4-Pyridyl,
2- oder 3-Furyl,
2- oder 3-Thienyl,
2-Tetrahydrofuranyl,
C₁-C₄-Alkyl, substituiert durch eine Pyridyl-, Furyl-, Thienyl-, Tetrahydrofuryl-, oder NR₄₉R₅₀-Gruppe, oder
ein Alkalimetall-, Erdalkalimetall-, Mangan-, Kupfer-, Zink-, Cobalt-, Silber-, Nickel-, Ammonium- oder organisches Ammoniumkation, steht;
R₃₄, R₃₇ und R₃₈ jeweils unabhängig voneinander für C₁-C₆-Alkyl stehen;
R₃₅ und R₄₉ jeweils unabhängig voneinander für Wasserstoff oder C₁-C₆-Alkyl stehen und R₃₆ und R₅₀ jeweils unabhängig voneinander für Wasserstoff, Cyano, C₁-C₆-Alkyl, SO₂R₅₁ oder Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen, oder R₃₅ und R₃₆ oder R₄₉ und R₅₀ zusammen mit dem Stickstoffatom, an das sie gebunden sind, einen 5- bis 12-gliedrigen monocyclischen oder kondensierten bicyclischen heterocyclischen Ring bilden, der gegebenenfalls durch eine oder mehrere Gruppen unabhängig voneinander ausgewählt aus Halogen-, Cyano-, Nitro-, Amino-, C₁-C₄-Alkyl-, C₁-C₄-Halogenalkyl-, C₁-C₄-Alkoxy-, C₁-C₄-Halogenalkoxy- oder C₁-C₄-Halogenalkylsulfonylgruppen, substituiert ist;
m, n, p und q jeweils unabhängig voneinander für ganze Zahlen 0, 1 oder 2 stehen;
R₂₉ für Wasserstoff, C₁-C₄-Alkyl oder C₁-C₄-Halogenalkyl steht;
R₂₄, R₃₁, R₄₁ und R₄₇ jeweils unabhängig voneinander für C₁-C₄-Alkyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, oder
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen;
R₂₃, R₃₉, R₄₀, R₄₂, R₄₃, R₄₄, R₄₆ und R₄₈ jeweils unabhängig voneinander für Wasserstoff, C₁-C₄-Alkyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, oder
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen, und
A, A₁, A₂, A₃, A₄, X₄ und X₅ jeweils unabhängig voneinander für O oder S stehen;
die optischen Isomere, Diastereomere und/oder Tautomere davon.

3. Verbindungen nach Anspruch 1 mit der Strukturformel

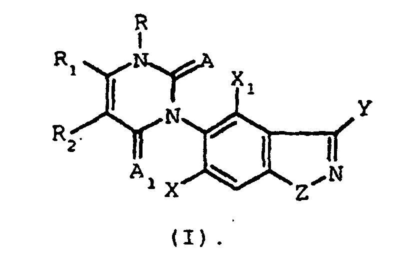

4. Verbindungen nach Anspruch 3, wobei
R für C₁-C₄-Alkyl, C₁-C₄-Halogenalkyl, C₃-C₄-Alkenyl, C₃-C₄-Alkinyl, oder Benzyl, steht;
R₁ für Halogen oder C₁-C₄-Halogenalkyl steht;
R₂ für Wasserstoff, Halogen oder C₁₋C₄-Halogenalkyl steht;
X und X₁ jeweils unabhängig voneinander für Wasserstoff oder Halogen stehen;
Z für O oder S steht;
Y für Cyano, R₁₂, X₂R₁₂ oder X₃X₂R₁₂ steht;
X₂ für O, S oder NR₁₃ steht;
R₁₂ für Wasserstoff,
eine C₁-C₆-Alkyl-, C₃-C₇-Cycloalkyl-, C₂-C₈-Alkenyl-, C₅-C₆-Cycloalkenyl- oder C₂-C₆-Alkinylgruppe, wobei die einzelnen Gruppen jeweils gegebenenfalls substituiert sind durch eine beliebige Kombination von ein bis drei Halogenatomen, einer oder zwei C₁-C₂₀-Alkoxygruppen, einer oder zwei C₁-C₁₀-Halogenalkoxygruppen, einer NR₁₅R₁₆-Gruppe, einer S(O)_{q}R₁₇-Gruppe, einer oder zwei Cyanogruppen, einer oder zwei C(O)R₁₈-Gruppen, einer oder zwei CO₂R₁₉-Gruppen, einer oder zwei C(O)SR₁₉-Gruppen, einer oder zwei C(O)NR₂₀R₂₁-Gruppen, einer oder zwei X₄R₂₂-Gruppen, einer P(O)(OR₂₃)₂-Gruppe, einer Si(R₂₄)₃-Gruppe,
einem 4- bis 10-gliedrigen monocyclischen oder kondensierten bicyclischen heterocyclischen Ring, gegebenenfalls substituiert durch eine beliebige Kombination von einer oder zwei Oxogruppen, einer oder zwei Thioxogruppen, ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen oder einer oder zwei X₄R₂₂-Gruppen,
einer quaternären organischen Ammoniumgruppe, oder
einer Phenylgruppe, gegebenenfalls substituiert durch eine beliebige Kombination von ein bis drei Halogenatomen, einer C₁-C₆-Alkylgruppe, einer C₁-C₆-Alkoxygruppe, einer C₃-C₇-Cycloalkylgruppe, einer Cyanogruppe, einer Nitrogruppe, einer C(O)R₂₅-Gruppe, einer CO₂R₂₆-Gruppe oder einer X₅R₂₇-Gruppe, oder
Phenyl gegebenenfalls substituiert durch eine beliebige Kombination von ein oder zwei Halogenatomen, einer bis drei C₁-C₄-Alkylgruppen, einer oder zwei C₁-C₄-Alkoxygruppen, einer C₃-C₆-Cycloalkylgruppe, einer C₁-C₄-Halogenalkylgruppe, einer C₁-C₄-Alkylthiogruppe, einer Cyanogruppe, einer Nitrogruppe, einer C(O)R₂₅-Gruppe, einer CO₂R₂₆-Gruppe oder einer X₄R₂₂-Gruppe, einer CH=CHR₂₂-Gruppe, einer CH₂CH(R₂₈)R₂₂-Gruppe oder einer N(R₂₉)SO₂R₃₀-Gruppe, steht;
R₁₃ für Wasserstoff, C₂-C₆-Alkoxyalkyl, C₁-C₄-Alkyl, C₃-C₇-Cycloalkyl, C₃-C₆-Alkenyl, C₃-C₆-Alkinyl, Benzyl, OR₂₃, Cyano oder SO₂R₅₁ steht;
R₁₄ für Wasserstoff, C₁-C₆-Alkyl, C₃-C₇-Cycloalkyl, C₂-C₈-Alkoxyalkyl, C₂-C₆-Alkenyl, C₂-C₆-Alkinyl oder Benzyl stehen;
R₁₆ und R₂₁ jeweils unabhängig voneinander für Wasserstoff, C₁-C₆-Alkyl, C₁-C₄-Alkoxy, C₃-C₇-Cycloalkyl, C₂-C₆-Alkenyl, C₃-C₇-Cycloalkenyl, C₂-C₆-Alkinyl, P(O)(OR₄₄)₂, SO₂R₄₅, C(O)R₄₆,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen, oder
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen, stehen;
und
R₃₂ für Wasserstoff,
Si(R₄₇)₃,
C₁-C₂₀-Alkyl, gegebenenfalls substituiert durch eine CO₂R₄₈-, C₁-C₆-Alkoxy-, C₁-C₆-Thioalkyl-, C₂-C₂₀-Alkoxyalkoxy-, Phenyl-, OSi(R₄₇)₃-, OC(O)R₃₃-, C₃-C₇-Cycloalkyl- oder Di(C₁-C₄-alkyl)aminogruppe,
C₁-C₁₅-Halogenalkyl,
C₃-C₈-Cycloalkyl, gegebenenfalls substituiert durch eine CO₂R₄₈-Gruppe,
C₃-C₈-Halogencycloalkyl,
C₃-C₂₀-Alkenyl, gegebenenfalls substituiert durch eine Phenylgruppe,
C₃-C₁₅-Halogenalkenyl,
C₅-C₈-Cycloalkenyl,
C₅-C₈-Halogencycloalkenyl,
C₃-C₂₀-Alkinyl, gegebenenfalls substituiert durch eine Phenylgruppe,
C₃-C₂₀-Halogenalkinyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
C₁-C₄-Alkyl, substituiert durch eine Pyridyl-, Furyl-, Thienyl-, Tetrahydrofuryl-, C₃-C₈-Cycloalkyl-, Dioxan- oder NR₄₉R₅₀-Gruppe, oder
ein Alkalimetall-, Erdalkalimetall-, Mangan-, Kupfer-, Zink-, Cobalt-, Silber-, Nickel-, Ammonium- oder organisches Ammoniumkation, steht.

5. Verbindungen nach Anspruch 4, wobei
R für C₁-C₄-Alkyl steht;
R₁ für C₁₋C₄-Halogenalkyl steht;
R₂ für Wasserstoff steht;
Y für R₁₂, OR₁₂, C(O)NR₁₃R₁₂, C(O)R₁₂, CO₂R₁₂, C(O)SR₁₂ oder C(=NOR₁₄) steht;
R₁₂ für Wasserstoff,
eine C₁-C₆-Alkyl- oder C₂-C₆-Alkenylgruppe, wobei die einzelnen Gruppen jeweils gegebenenfalls substituiert sind durch eine beliebige Kombination von ein bis drei Halogenatomen, einer oder zwei C₁-C₄-Alkoxygruppen, einer C₁-C₁₀-Halogenalkoxygruppe, einer NR₁₅R₁₆-Gruppe, einer S(O)_{q}R₁₇-Gruppe, einer oder zwei Cyanogruppen, einer oder zwei C(O)R₁₈-Gruppen, einer oder zwei CO₂R₁₉-Gruppen, einer oder zwei C(O)NR₂₀R₂₁-Gruppen, einer X₄R₂₂-Gruppe, einer P(O)(OR₂₃)₂-Gruppe, einer OC(O)R₃₃-Gruppe,
einem Imidazolring, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen,
einem Phthalimidring, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen,
einer quaternären organischen Ammoniumgruppe
oder
einer Phenylgruppe, gegebenenfalls durch eine beliebige Kombination von ein bis drei Halogenatomen, einer C₁-C₄-Alkylgruppe, einer C₁-C₄-Alkoxygruppe oder einer X₅R₂₇-Gruppe, substituiert sind, oder
Phenyl gegebenenfalls substituiert durch eine beliebige Kombination von ein oder zwei Halogenatomen, einer bis drei C₁-C₄-Alkylgruppen, einer C₁-C₄-Alkoxygruppe, einer C(O)R₂₅-Gruppe, einer CO₂R₂₆-Gruppe oder einer X₄R₂₂-Gruppe, einer CH=CHR₂₂-Gruppe, einer CH₂CH(R₂₈)R₂₂-Gruppe oder einer N(R₂₉)SO₂R₃₀-Gruppe steht;
R₁₃ für Wasserstoff, C₁-C₆-Alkoxyalkyl, C₁-C₄-Alkyl, OR₂₃, Cyano oder SO₂R₅₁ steht;
R₂₂ und R₂₇ unabhängig voneinander für Wasserstoff, C(O)R₃₃, SO₂R₄₇, C₁-C₄-Alkyl, substituiert durch eine C₁-C₆-Alkoxy-, CO₂R₃₂-,
C(O)R₃₃-, C(O)NR₃₅R₃₆-, C(O)ON=CR₃₇R₃₈-, C(O)NHOR₂₃-, Cyano- oder. 2-Dioxolanylgruppe oder einen Phenyl-, Dihydrofuranon- oder Furanonring, wobei jeder Ring gegebenenfalls durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen substituiert ist,
C₃-C₆-Alkenyl, gegebenenfalls substituiert durch eine C₁-C₆-Alkoxy-, CO₂R₃₂-, C(O)R₃₃- oder C(OR₃₄)₂-Gruppe,
C₃-C₆-Alkinyl,
Phenyl, gegebenenfalls substituiert durch eine beliebige Kombination von ein bis drei Halogenatomen, einer oder zwei C₁-C₄-Alkylgruppen, einer oder zwei C₁-C₄-Alkoxygruppen, einer C₁-C₄-Halogenalkylgruppe, einer C₁-C₄-Halogenalkoxygruppe, einer Cyanogruppe, einer Nitrogruppe, einer NR₃₉R₄₀-Gruppe oder einer C(O)R₃₃-Gruppe, oder
Benzyl, am Phenylring gegebenenfalls substituiert durch eine beliebige Kombination von ein bis drei Halogenatomen, einer oder zwei C₁-C₄-Alkylgruppen, einer oder zwei C₁-C₄-Alkoxygruppen, einer C₁-C₄-Halogenalkylgruppe, einer C₁-C₄-Halogenalkoxygruppe, einer Cyanogruppe, einer Nitrogruppe, einer NR₃₉R₄₀-Gruppe oder einer C(O)R₃₃-Gruppe, stehen;
R₁₅ für Wasserstoff,
C₁-C₄-Alkyl, gegebenenfalls substituiert durch eine CO₂R₄₂-Gruppe,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen, oder
C(O)R₄₃ steht;
R₁₆ für Wasserstoff, C₁-C₄-Alkyl, P(O)(OR₄₄)₂, SO₂R₄₅, C(O)R₄₆ oder
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen oder einer bis drei C₁-C₄-Halogenalkoxygruppen, steht;
R₁₇, R₁₈, R₂₅, R₃₀, R₃₃, R₄₅ und R₅₁ jeweils unabhängig voneinander für NR₄₆R₄₈, C₁-C₄-Alkyl, C₁-C₄-Halogenalkyl,
C₂-C₆-Alkenyl, gegebenenfalls substituiert durch Phenyl,
Benzyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Nitrogruppe, einer Cyanogruppe oder einer X₄R₂₂-Gruppe, oder
Phenyl, gegebenenfalls substituiert durch eine beliebige Kombination von ein bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Nitrogruppe, einer Cyanogruppe oder einer X₄R₂₂-Gruppe, stehen;
R₁₉ und R₂₆ jeweils unabhängig voneinander für Wasserstoff, C₁-C₄-Alkyl, C₃-C₆-Alkenyl, C₃-C₆₋Alkinyl, Si(R₄₁)₃, Benzyl, Phenyl, Furfuryl, Pyridyl, Thienyl, Oximino oder ein Alkalimetall-, Erdalkalimetall-, Mangan-, Kupfer-, Zink-, Cobalt-, Silber-, Nickel-, Ammonium- oder organisches Ammoniumkation stehen;
R₃₂ für Wasserstoff,
Si(R₄₇)₃,
C₁-C₄-Alkyl, gegebenenfalls substituiert durch eine CO₂R₄₈-, OC(O)R₃₃- oder C₁-C₄-Alkoxygruppe,
C₁-C₄-Halogenalkyl,
C₃-C₈-Cycloalkyl,
C₃-C₈-Halogencycloalkyl,
C₃-C₆-Alkenyl, gegebenenfalls substituiert durch eine Phenylgruppe,
C₃-C₆-Halogenalkenyl,
C₅-C₈-Cycloalkenyl,
C₅-C₈-Halogencycloalkenyl,
C₃-C₆-Alkinyl, gegebenenfalls substituiert durch eine Phenylgruppe,
C₃-C₆-Halogenalkinyl,
Benzyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen,
Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, C₁-C₄-Alkyl, substituiert durch eine Pyridyl-, Furyl-, Thienyl-, Tetrahydrofuryl- oder NR₄₉R₅₀-Gruppe, oder
ein Alkalimetall-, Erdalkalimetall-, Mangan-, Kupfer-, Zink-, Cobalt-, Silber-, Nickel-, Ammonium- oder organisches Ammoniumkation, steht;
R₃₅ und R₄₉ jeweils unabhängig voneinander für Wasserstoff oder C₁-C₄-Alkyl stehen und R₃₆ und R₅₀ jeweils unabhängig voneinander für Wasserstoff, Cyano, C₁-C₄-Alkyl, SO₂R₅₁ oder Phenyl, gegebenenfalls substituiert durch eine oder mehrere Gruppen unabhängig voneinander ausgewählt aus Halogen-, Cyano-, Nitro-, C₁-C₄-Alkyl-, C₁-C₄-Halogenalkyl-, C₁-C₄-Alkoxy- und C₁-C₄-Halogenalkoxygruppen, stehen, oder R₃₅ und R₃₆ oder R₄₉ und R₅₀ zusammen mit dem Stickstoffatom, an das sie gebunden sind, einen 5- bis 12-gliedrigen monocyclischen oder kondensierten bicyclischen heterocyclischen Ring bilden, der gegebenenfalls durch eine oder mehrere Gruppen unabhängig voneinander ausgewählt aus Halogen-, Cyano-, Nitro-, Amino-, Hydroxyl-, C₁-C₄-Alkyl-, C₁-C₄-Halogenalkyl-, C₁-C₄-Alkoxy-, C₁-C₄-Halogenalkoxy- oder C₁-C₄-Halogenalkylsulfonylgruppen, substituiert ist;
R₂₃, für Wasserstoff oder C₁-C₄-Alkyl steht;
A für 0 steht.

6. Verbindungen nach Anspruch 5, wobei
R für Methyl steht;
R₁ für Trifluormethyl steht;
X₁ für Wasserstoff steht;
X für Wasserstoff oder Fluor steht;
Y für R₁₂, CO₂R₁₂, C(O)R₁₂ oder C(O)NR₁₃R₁₂ steht;
R₁₂ für Wasserstoff,
C₁-C₆-Alkyl, gegebenenfalls substituiert durch eine beliebige Kombination von ein bis drei Halogenatomen, einer Hydroxylgruppe, einer oder zwei C₁-C₄-Alkoxygruppen, einer C₁-C₄-Halogenalkoxygruppe, einer SO₂R₁₇-Gruppe, einer oder zwei Cyanogruppen, einer C(O)R₁₈-Gruppe, einer oder zwei CO₂R₁₉-Gruppen, einer oder zwei C(O)NR₂₀R₂₁-Gruppen, einer P(O)(OR₂₃)₂-Gruppe oder einer OC(O)R₃₃-Gruppe,
C₂-C₆-Alkenyl, gegebenenfalls substituiert durch eine beliebige Kombination von einer C(O)R₁₈-Gruppe oder einer CO₂R₁₉-Gruppe, 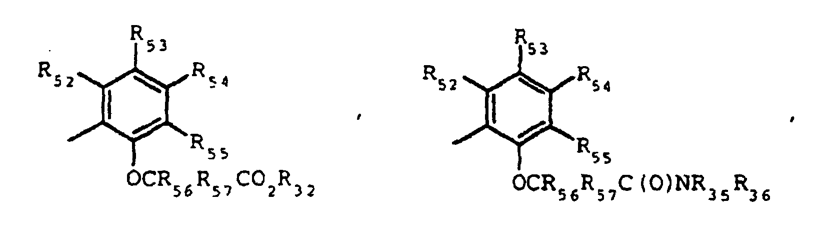 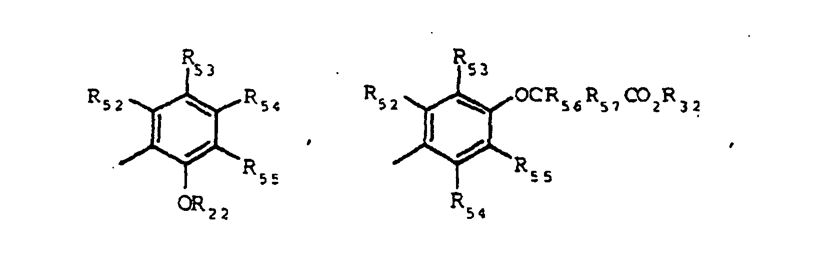 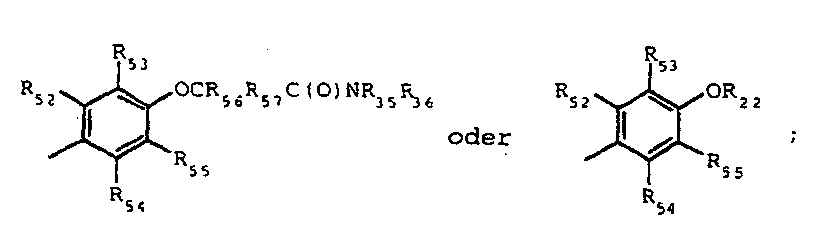 steht;
R₁₃ für Wasserstoff, C₁-C₄-Alkyl oder SO₂R₅₁ steht;
R₁₄ für Wasserstoff, C₁-C₄-Alkyl oder Benzyl steht;
R₁₈, R₂₃, R₅₆ und R₅₇ jeweils unabhängig voneinander für Wasserstoff oder C₁-C₃-Alkyl stehen;
R₅₂, R₅₃, R₅₄ und R₅₅ jeweils unabhängig voneinander für Wasserstoff, C₁-C₃-Alkyl, C₂-C₃-Alkenyl, C₁-C₃-Alkoxy oder Halogen stehen, mit der Maßgabe, daß wenigstens einer der Reste R₅₂, R₅₃, R₅₄ und R₅₅ für Wasserstoff steht;
R₁₉ für Wasserstoff, C₁-C₄-Alkyl, C₃-C₆-Alkenyl, C₃-C₆-Alkinyl, Benzyl, Pyridyl oder ein Alkalimetall-, Erdalkalimetall-, Ammonium- oder organisches Ammoniumkation steht;
R₂₂ für Wasserstoff, C(O)R₃₃, SO₂R₄₇,
C₁-C₄-Alkyl, gegebenenfalls substituiert durch eine Cyanogruppe,
C₃-C₆-Alkenyl oder
C₃-C₆-Alkinyl steht;
R₃₂ für Wasserstoff,
C₁-C₄-Alkyl, gegebenenfalls substituiert durch eine CO₂R₄₈- oder C₁-C₄-Alkoxygruppe,
C₁-C₄-Halogenalkyl,
C₃-C₆-Alkenyl, gegebenenfalls substituiert durch eine Phenylgruppe,
C₃-C₆-Alkinyl, gegebenenfalls substituiert durch eine Phenylgruppe,
C₁-C₄-Alkyl, substituiert durch eine Pyridyl-, Furyl-, Thienyl-, Tetrahydrofuryl- oder NR₄₉R₅₀-Gruppe oder
ein Alkalimetall-, Erdalkalimetall-, Ammonium- oder organisches Ammoniumkation steht;
R₃₅ und R₄₉ jeweils unabhängig voneinander für Wasserstoff oder C₁-C₄-Alkyl stehen;
R₃₆ für SO₂R₅₁ oder C₁-C₃-Alkoxy steht;
R₁₇, R₃₃ und R₅₁ jeweils unabhängig voneinander für C₁-C₄-Alkyl,
C₁-C₄-Halogenalkyl, NR₄₆R₄₈, C₃-C₆-Cycloalkyl oder einen Isoxazolring stehen;
R₄₆ und R₄₈ jeweils unabhängig voneinander für Wasserstoff, C₁-C₄-Alkyl oder Benzyl stehen;
R₄₇ für C₁-C₄-Alkyl steht;
R₅₀ für C₁-C₄-Alkyl oder
Phenyl gegebenenfalls substituiert durch eine oder mehrere Gruppen unabhängig voneinander ausgewählt aus Halogen-, Cyano-, C₁-C₄-Alkyl-, C₁-C₄-Halogenalkyl-, C₁-C₄-Alkoxy- und C₁-C₄-Halogenalkoxygruppen steht; und
A₁ für O steht.

7. Verbindungen nach Anspruch 6, wobei
Y für C₁-C₃-Alkyl, CO₂R₃₂, Halogenmethyl, Cyanomethyl, C (CH₃)₂CO₂R₃₂, C₁-C₃-Alkoxyethyl, C₁-C₃-Alkoxymethyl, Hydroxymethyl, CHO, C(O)CH₃, CH(CH₃) (C₁-C₄-Alkoxy), C(CH₃)₂CN, CH[O(C₁-C₃-Alkyl)]₂, CH₂SO₂R₁₇, C(O)NHSO₂R₅₁, 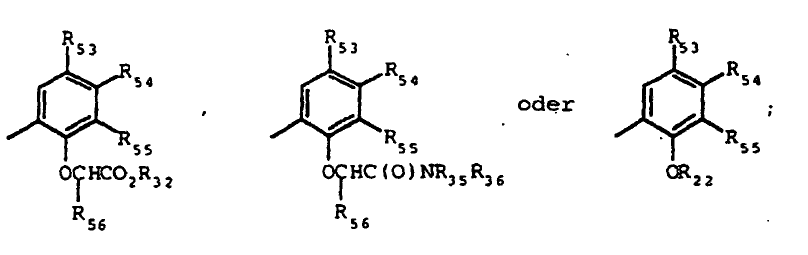 steht;
R₅₃, R₅₄, R₅₅ und R₅₆ jeweils unabhängig voneinander für Wasserstoff oder Methyl stehen, mit der Maßgabe, daß wenigstens einer der Reste R₅₃, R₅₄ und
R₅₅ für Wasserstoff steht;
R₂₂ für Cyanomethyl, Methyl, Ethyl, Allyl oder Propargyl steht;
R₃₂ für Wasserstoff,
C₁-C₄-Alkyl, gegebenenfalls substituiert durch eine CO₂R₄₈-Gruppe,
C₁-C₄-Halogenalkyl,
C₃-C₆-Alkenyl oder
C₃-C₆-Alkinyl steht; und
R₃₅ für Wasserstoff steht.

8. Verbindungen nach Anspruch 1, ausgewählt aus der aus den folgenden Verbindungen bestehenden Gruppe:
{{2-(5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäureisopropylester;
{{2-{5-[3,6-Dihydro-3-methyl-4-(trifluormethyl)-2,6-dioxo-1(2H)-pyrimidinyl]-1,2-benzisothiazol-3-yl}-*p*-tolyl}oxy}essigsäure-isopropylester;
{{2-{5-[3,6-Dihydro-3-methyl-2,6-dioxo-4-(trifluormethyl)-1(2H)-pyrimidinyl]-1,2-benzisothiazol-3-yl]-*p*-tolyl}oxy)essigsäure-2-(dimethylamino)ethylester;
2-{{2-{5-(3,6-Dihydro-3-methyl-4-(trifluormethyl)-2,6-dioxo-1(2H)-pyrimidinyl-1,2-benzisothiazol-3-yl}-*p*-tolyl}oxy}propionsäuremethylester;
2-{{6-{5-[3,6-Dihydro-3-methyl-4-(trifluormethyl)-2,6-dioxo-1(2H)-pyrimidinyl]-1,2-benzisothiazol-3-yl}-3,4-xylyl}propionsäure-methylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2-propinylester;
3-[3-(6-Methoxy-*m*-tolyl)-1,2-benzisothiazol-5-yl]-1-methyl-6-(trifluormethyl)-2,4(1H,3H)-pyrimidindion;
3-[3-(*p*-Ethylphenyl)-1,2-benzisothiazol-5-yl]-1-methyl-6-(trifluormethyl)-2,4(1H,3H)-pyrimidindion;
1-Methyl-3-(3-methyl-1,2-benzisothiazol-5-yl)-6-(trifluormethyl)-2,4(1H,3H)-pyrimidindion;
2-[{{6-{5-[3,6-Dihydro-3-methyl-4-(trifluormethyl)-2,6-dioxo-1(2H)-pyrimidinyl]-1,2-benzisothiazol-3-yl}-*m*-tolyl}oxy}propionsäuremethylester;
{{6-{5-[3,6-Dihydro-3-methyl-4-(trifluormethyl)-2,6-dioxo-1(2H)-pyrimidinyl]-1,2-benzisothiazol-3-yl}-3,4-xylyl}oxy}essigsäure-methylester;
3-[3-(Dibrommethyl]-1,2-benzisothiazol-5-yl]-1-methyl-6-(trifluormethyl)-2,4(1H,3H)-pyrimidindion;
3-(6-Fluor-3-methyl-1,2-benzisoxazol-5-yl)-1-methyl-6-(trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäureethylester;
1-Methyl-3-{3-[6-(2-propinyloxy)-*m*-tolyl]-1,2-benzisothiazol-5-yl}-6-(trifluormethyl]-2,4(1H,3H)-pyrimidindion;
3-{3-{6-[(1-Benzimidazolylcarbonyl)methoxy]-*m*tolyl}-1,2-benzisothiazol-5-yl}-1-methyl-6-trifluormethyl)-2,4(1H,3H)-pyrimidindion;
3-{3-{6-{[(*m*-Cyanophenyl)carbamoyl]methoxy}-*m*tolyl}-1,2-benzisothiazol-5-yl}-1-methyl-6-trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäureallylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-3-phenyl-2-propinylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-1-methyl-2-propinylester;
2-{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}propionsäure-2-chlorethylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2,2,2-trifluorethylester;
3-[3-(2-Methoxy-*p*-tolyl)-1,2-benzisothiazol-5-yl]-1-methyl-6-(trifluormethyl)-2,4(1H,3H)-pyrimidindion;
2-[3-(6-Methoxy-3,4-xylyl)-1,2-benzisothiazol-5-yl]-1-methyl-6-(trifluormethyl)-2,4(1H,3H)-pyrimidindion;
2-{{6-{5-[3,6-Dihydro-3-methyl-4-(trifluormethyl)-2,6-dioxo-1(2H)-pyrimidinyl]-1,2-benzisothiazol-3-yl}-3,4-xylyl}oxy}propionsäure-methylester;
{{6-{5-[3,6-Dihydro-3-methyl-2,6-dioxo-4-(trifluormethyl)-1(2H)-pyrimidinyl]-1,2-benzisothiazol-3-yl}-2,4-xylyl}oxy}essigsäuremethylester;
3-{3-{6-{[(*m*-Cyanophenyl)carbamoyl)methoxy}-*m*tolyl}-1,2-benzisothiazol-5-yl}-1-methyl-6-(trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisoxazol-3-yl}-*p*-tolyl}oxy}essigsäuremethylester;
1-Methyl-3-{3-[(methylsulfonyl)methyl]-1,2-benzisothiazol-5-yl}-6-(trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisoxazol-3-yl}-*p*-tolyl}oxy}essigsäure-2-propinylester;
3-[3-(*o*-Methoxyphenyl)-1,2-benzisothiazol-5-yl] -1-methyl-6-(trifluormethyl)-2,4(1H,3H)-pyrimidindion;
N-{{{-2-{5-[3,6-Dihydro-3-methyl-2,6-dioxo-4-(trifluormethyl)-1(2H)-pyrimidinyl]-1,2-benzisothiazol-3-yl}*-p*-tolyl}oxy}acetyl}methansulfonamid;
2-{{2-{5-[3,6-Dihydro-3-methyl-2,6-dioxo-4-(trifluormethyl)-1(2H)-pyrimidinyl]-1,2-benzisoxazol-3-yl}*-p*-tolyl}oxy}propionsäure-2-propinylester;
{*o*-{5-[3,6-Dihydro-3-methyl-2,6-dioxo-4-(trifluormethyl)-1(2H)-pyrimidinyl]-1,2-benzisothiazol-3-yl}phenoxy}essigsäure-2-propinylester;
5-[3,6-Dihydro-3-methyl-2,6-dioxo-4-(trifluormethyl)-1(2H)-pyrimidinyl]-1,2-benzisothiazol-3-acetonitril;
3-{3-[(Allyloxy)methyl]-1,2-benzisothiazol-5-yl}-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl]-N-(methylsulfonyl)-1,2-benzisothiazol-3-carbonsäureamid;
{{2-{5-[3,6-Dihydro-3-methyl-2,6-dioxo-4-(trifluormethyl)-1(2H)-pyrimidinyl]-6-fluor-1,2-benzisoxazol-3-yl}-*p*-tolyl}oxy}essigsäuremethylester;
{{6-{5-[3,6-Dihydro-3-methyl-2,6-dioxo-4-(trifluormethyl)-1(2H)-pyrimidinyl]-6-fluor-1,2-benzisothiazol-3-yl}-*m*-tolyl}oxy}essigsäuremethylester;
{*o*-{5-[3,6-Dihydro-3-methyl-2,6-dioxo-4-(trifluormethyl)-1(2H)-pyrimidinyl]-1,2-benzisothiazol-3-yl}phenoxy}essigsäure;
2-{*o*-{5-[3,6-Dihydro-3-methyl-2,6-dioxo-4-(trifluormethyl)-1(2H)-pyrimidinyl]-1,2-benzisothiazol-3-yl}phenoxy}propionsäure;
2-{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}-N-methoxyessigsäureamid;
2-{2-{5-[3,6-Dihydro-3-methyl-2,6-dioxo-4-(trifluormethyl)-1(2H)-pyrimidinyl]-1,2-benzisothiazol-3-yl}-4-methoxyphenoxy}propionsäure-methylester
2-{*o*-{5-[3,6-Dihydro-3-methyl-2,6-dioxo-4-(trifluormethyl)-1(2H)-pyrimidinyl}-1,2-benzisothiazol-3-yl}phenoxy}-N-(methylsulfonyl)-essigsäureamid;
2-{*o*-{5-[3,6-Dihydro-3-methyl-2,6-dioxo-4-(trifluormethyl)-1(2H)-pyrimidinyl]-1,2-benzisothiazol-3-yl}phenoxy}-N-(methylsulfonyl)propionsäureamid;
2-{{2-{5-[3,6-Dihydro-3-methyl-2,6-dioxo-4-(trifluormethyl]-1(2H]-pyrimidinyl]-1,2-benzisothiazol-3-yl}-*p*-tolyl}oxy}-N-methyl-N-(methylsulfonyl) essigsäureamid;
2-{*o*-{5-[3,6-Dihydro-3-methyl-2,6-dioxo-4-(trifluormethyl)-1(2H)-pyrimidinyl]-1,2-benzisothiazol-3-yl}phenoxy}-N-methyl-N-(methylsulfonyl)-essigsäureamid;
2-{*o*-{5-[3,6-Dihydro-3-methyl-2,6-dioxo-4-(trifluormethyl)-1(2H)-pyrimidinyl]-1,2-benzisothiazol-3-yl}phenoxy}-N-(ethylsulfonyl)-essigsäureamid;
3-[3-(Hydroxymethyl)-1,2-benzisothiazol-5-yl]-1-methyl-6- (trifluormethyl)-2,4(1H,3H)-pyrimidindion-2-thiophencarboxylatester;
3-[3-(Hydroxymethyl)-1,2-benzisothiazol-5-yl]-1-methyl-6-(trifluormethyl)-2,4(1H,3H)-pyrimidindion-benzoatester;
3-[3-(Hydroxymethyl)-1,2-benzisothiazol-5-yl]-1-methyl-6-(trifluormethyl)-2,4(1H,3H)-pyrimidindion-methoxyessigsäureester;
5-[3,6-Dihydro-3-methyl-2,6-dioxo-4-(trifluormethyl)-1(2H)-pyrimidinyl]-N-(ethylsulfonyl)-1,2-benzisothiazol-3-carbonsäureamid;
5-[3,6-Dihydro-3-methyl-2,6-dioxo-4-(trifluormethyl)-1(2H)-pyrimidinyl]-α,α-dimethyl-1,2-benzisothiazol-3-acetonitril;
5-[3,6-Dihydro-3-methyl-2,6-dioxo-4-(trifluormethyl)-1(2H)-pyrimidinyl]-6-fluor-1,2-benzisothiazol-3-acetonitril;
5-[3,6-Dihydro-3-methyl-2,6-dioxo-4-(trifluormethyl)-1(2H)-pyrimidinyl]-α-ethyl-1,2-benzisothiazol-3-acetonitril;
5-[3,6-Dihydro-3-methyl-2,6-dioxo-4-(trifluormethyl)-1(2H)-pyrimidinyl]-α,α-diethyl-1,2-benzisothiazol-3-acetonitril;
3-[6-Fluor-3-(methoxymethyl)-1,2-benzisothiazol-5-yl]-1-methyl-6-(trifluormethyl)-2,4(1H,3H)-pyrimidindion;
3-[3-(Hydroxymethyl)-1,2-benzisothiazol-5-yl]-1-methyl-6-(trifluormethyl)-2,4(1H,3H)-pyrimidindion-5-isoxazolcarbonsäureester;
3-[3-(Hydroxymethyl)-1,2-benzisothiazol-5-yl]-1-methyl-6-(trifluormethyl)-2,4(1H,3H)-pyrimidindion-cyclopropancarbonsäureester;
3-(3-Acetyl-1,2-benzisothiazol-5-yl]-1-methyl-6-(trifluormethyl)-2,4(1H,3H)-pyrimidindion;
3-[3-(1-Methoxyethyl)-1,2-benzisothiazol-5-yl]-1-methyl-6-(trifluormethyl)-2,4(1H,3H)-pyrimidindion;
N-(Benzylsulfonyl)-5-[3,6-dihydro-3-methyl-2,6-dioxo-4-(trifluormethyl)-1(2H)-pyrimidinyl]-1,2-benzisothiazol-3-carbonsäureamid;
5-[3,6-Dihydro-3-methyl-2,6-dioxo-4-(trifluormethyl)-1(2H)-pyrimidinyl]-1,2-benzisothiazol-3-carbonsäure-methylester;
5-[3,6-Dihydro-3-methyl-2,6-dioxo-4-(trifluormethyl)-1(2H)-pyrimidinyl]-α,α-dimethyl-1,2-benzisothiazol-3-essigsäure-benzylester;
N-(Benzylsulfonyl)-5-[3,6-dihydro-3-methyl-2,6-dioxo-4-(trifluormethyl)-1(2H)-pyrimidinyl]-6-fluor-1,2-benzisothiazol-3-carbonsäureamid;
5-[3,6-Dihydro-3-methyl-2,6-dioxo-4-(trifluormethyl)-1(2H)-pyrimidinyl]-N-(methylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(ethylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-[(*p*methylbenzyl)sulfonyl]-1,2-benzisothiazol-3-carbonsäureamid;
N-(Benzylsulfonyl)-5-[3,6-dihydro-3-methyl-2,6-dioxo-4-(trifluormethyl)-1(2H)-pyrimidinyl]-6-fluor-1,2-benzisothiazol-3-carbonsäureamid;
3-[3-(1-Hydroxyethyl)-1,2-benzisothiazol-5-yl]-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl)-1,2-benzisothiazol-3-carboxaldehyd-3-dimethylacetal;
3-(6-Fluor-3-methyl-1,2-benzisothiazol-5-yl)-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl]-6-fluor-1,2-benzisothiazol-3-carboxaldehyd-3-dimethylacetal;
5-[3,6-Dihydro-3-methyl-2,6-dioxo-4-(trifluormethyl)-1(2H)-pyrimidinyl]-6-fluor-1,2-benzisothiazol-3-carbonsäure;
5-[3,6-Dihydro-3-methyl-2,6-dioxo-4-(trifluormethyl)-1(2H)-pyrimidinyl]-α-methyl-1,2-benzisothiazol-3-acetonitril;
N-(Benzylsulfonyl)-5-[3,6-dihydro-3-methyl-2,6-dioxo-4-(trifluormethyl)-1(2H)-pyrimidinyl]-N-(ethoxymethyl)-1,2-benzisothiazol-3-carbonsäureamid;
5-[3,6-Dihydro-3-methyl-2,6-dioxo-4-(trifluormethyl)-1(2H)-pyrimidinyl]-N-(dimethylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-α,α-dimethyl-1,2-benzisothiazol-3-essigsäuremethylester;
3-[3-(Hydroxymethyl)-1,2-benzisothiazol-5-yl]-1-methyl-6-(trifluormethyl)-2,4(1H,3H)-pyrimidindion-2-furoatester;
N-[(*m*-Chlorbenzyl]sulfonyl]-5-[3,6-dihydro-3-methyl-2,6-dioxo-4-(trifluormethyl)-1(2H)-pyrimidinyl]-1,2-benzisothiazol-3-carbonsäureamid;
5-[3,6-Dihydro-3-methyl-2,6-dioxo-4-(trifluormethyl)-1(2H)-pyrimidinyl]-N-(diisopropylsulfamoyl)-1,2-benzisothiazol-3-carbonsäureamid und
3-[3-(Hydroxymethyl)-1,2-benzisothiazol-5-yl]-1-methyl-6-(trifluormethyl)-2,4(1H,3H)-pyrimidindion-methylcarbamatester.

9. Verfahren zur Kontrolle von unerwünschten Pflanzenspezies, bei dem man auf die Blätter der Pflanzen oder den Boden bzw. das Wasser, das die Samen oder anderes Vermehrungsgut davon enthält, eine herbizid wirksame Menge einer Verbindung nach einem der Ansprüche 1 bis 8 ausbringt.

10. Verfahren nach Anspruch 9, bei dem man die Verbindung auf die Blätter der unerwünschten Pflanzenspezies oder den Boden bzw. das Wasser, das die Samen oder anderes Vermehrungsgut davon enthält, in Gegenwart von Kulturpflanzen, Kulturpflanzensamen oder anderem Kulturpflanzenvermehrungsgut ausbringt.

11. Verfahren nach Anspruch 10, wobei es sich bei der Kulturpflanze um Getreide oder Sojabohnen handelt.

12. Verfahren nach Anspruch 11, bei dem das Getreide aus der aus Mais, Weizen und Reis bestehenden Gruppe ausgewählt ist.

13. Verfahren nach Anspruch 12, wobei es sich bei dem Getreide um Mais handelt und die Verbindung aus der aus den folgenden Verbindungen bestehenden Gruppe ausgewählt ist:
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäureisopropylester;
{{2-{5-[3,6-Dihydro-3-methyl-4-(trifluormethyl)-2,6-dioxo-1(2H)-pyrimidinyl]-1,2-benzisothiazol-3-yl}-*p*-tolyl}oxy}essigsäure-methylester;
{{2-{5-[3,6-Dihydro-3-methyl-2,6-dioxo-4-(trifluormethyl)-1(2H)-pyrimidinyl]-1,2-benzisothiazol-3-yl]-*p*-tolyl}oxy}essigsäure-2-(dimethylamino)ethylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2-propinylester;
3-[3-(*p*-Ethylphenyl)-1,2-benzisothiazol-5-yl]-1-methyl-6-(trifluormethyl)-2,4(1H,3H)-pyrimidindion;
{{6-{5-[3,6-Dihydro-3-methyl-4-(trifluormethyl)-2,6-dioxo-1(2H)-pyrimidinyl]-1,2-benzisothiazol-3-yl}-3,4-xylyl}oxy}essigsäure-methylester;
3-[3-(Dibrommethyl)-1,2-benzisothiazol-5-yl]-1-methyl-6-(trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisothiazol-3-yl}*-p*-tolyl}oxy}essigsäure-1-methyl-2-propinylester;
{{2-{5-[3,6-Dihyro-3-methyl-2,6-dioxo-4-(trifluormethyl)-1(2H)-pyrimdinyl-1,2-benzisoxazol-3-yl}*-p*-tolyl}oxy}essigsäuremethylester;
1-Methyl-3-{3-[(methylsulfonyl)methyl]-1,2-benzisothiazol-5-yl}-6-(trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisoxazol-3-yl}--*p*-tolyl}oxy}essigsäure-2-propinylester;
3-[3-(*o*-Methoxyphenyl)-1,2-benzisothiazol-5-yl]-1-methyl-6-(trifluormethyl)-2,4(1H,3H)-pyrimidindion;
N-{{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}acetyl)methansufonamid;
{*o*-{5-[3,6-Dihydro-3-methyl-2,6-dioxo-4-(trifluormethyl)-1(2H)-pyrimidinyl]-1,2-benzisothiazol-3-yl}phenoxy}essigsäure-2-propinylester;
5-[3,6-Dihydro-3-methyl-2,6-dioxo-4-(trifluormethyl)-1(2H)-pyrimidinyl]-1,2-benzisothiazol-3-acetonitril;
3-{3-[(Allyloxy)methyl]-1,2-benzisothiazol-5-yl}-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl]-N-(methylsulfonyl)-1,2-benzisothiazol-3-carbonsäureamid;
2-{{2-{5-[3,6-Dihydro-3-methyl-2,6-dioxo-4-(trifluormethyl)-1(2H)-pyrimidinyl]-1,2-benzisothiazol-3-yl-*p*-tolyl}oxy}-N-methyl-N-(methylsulfonyl)essigsäureamid;
3-[3-(Hydroxymethyl)-1,2-benzisothiazol-5-yl]-1-methyl-6-(trifluormethyl)-2,4(1H,3H)-pyrimidindion-methylcarbamatester;
N-(Benzylsulfonyl)-5-[3,6-dihydro-3-methyl-2,6-dioxo-4-(trifluormethyl)-1(2H)-pyrimidinyl]-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(ethylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(diisopropylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-N-(dimethylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-6-fluor-1,2-benzisothiazol-3-carbonsäure; und
N-(Benzylsulfonyl)-5-[3,6-dihydro-3-methyl-2,6-dioxo-4-(trifluormethyl)-1(2H)-pyrimidinyl]-6-fluor-1,2-benzisothiazol-3-carbonsäureamid.

14. Verfahren nach Anspruch 12, bei dem es sich bei dem Getreide um Weizen handelt und die Verbindung aus der aus den folgenden Verbindungen bestehenden Gruppe ausgewählt ist:
{{2-[5-[3,6-Dihydro-3-methyl-2,6-dioxo-4-(trifluormethyl)-1(2H)-pyrimidinyl]-1,2-benzisothiazol-3-yl]-*p*-tolyl}oxy}essigsäureisopropylester;
{{2-{5-[3,6-Dihydro-3-methyl-4-(trifluormethyl)-2,6-dioxo-1(2H)-pyrimidinyl]-1,2-benzisothiazol-3-yl}-*p*-tolyl}oxy}-essigsäuremethylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2-(dimethylamino)ethylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2-propinylester;
{{6-{5-[3,6-Dihydro-3-methyl-4-(trifluormethyl)-2,6-dioxo-1(2H)-pyrimidinyl]-1,2-benzisothiazol-3-yl}-*m*-tolyl}oxy}propionsäure-methylester;
3-(6-Fluor-3-methyl-1,2-benzisoxazol-5-yl)-1-methyl-6-(trifluormethyl)-2,4(1H,3H)-pyrimidindion;
3-{3-{6-[(1-Benzimidazolylcarbonyl)methoxy)-*m*tolyl}-1,2-benzisothiazol-5-yl}-1-methyl-6-trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2-allylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-3-phenyl-2-propinylester;
2-{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}propionsäure-2-chlorethylester;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäure-2,2,2-trifluorethylester;
{{6-{5-[3,6-Dihydro-3-methyl-2,6-dioxo-4-(trifluormethyl)-1(2H)-pyrimidinyl]-1,2-benzisothiazol-3-yl}-2,4-xylyl}oxy}-essigsäure-methylester;
{*o*-{5-[3,6-Dihydro-3-methyl-2,6-dioxo-4-(trifluormethyl)-1(2H)-pyrimidinyl]-1,2-benzisothiazol-3-yl}phenoxy}essigsäure;
2-{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}-N-methoxy-essigsäureamid;
2-{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}-N-methyl-N-(methylsulfonyl)essigsäureamid;
{{6-{5-[3,6-Dihydro-3-methyl-2,6-dioxo-4-(trifluormethyl)-1(2H)-pyrimidinyl]-1,2-benzisothiazol-3-yl}-*m*-tolyl}oxy}essigsäure-methylester;
3-(3-Acetyl-1,2-benzisothiazol-5-yl)-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl]-1,2-benzisothiazol-3-carbonsäure-methylester;
5-[3,6-Dihydro-3-methyl-2,6-dioxo-4-(trifluormethyl)-1(2H)-pyrimidinyl]-N-(isopropylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(ethylsulfonyl)-1,2-benzisothiazol-3-carbonsäureamid;
N-[(*m*-Chlorbenzyl)sulfonyl]-5-[3,6-dihydro-3-methyl-2,6-dioxo-4-(trifluormethyl)-1(2H)-pyrimidinyl]-1,2-benzisothiazol-3-carbonsäureamid;
5-[3,6-Dihydro-3-methyl-2,6-dioxo-4-(trifluormethyl)-1(2H)-pyrimidinyl]-N-(diisopropylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfamoyl)-1,2-benzisothiazol-3-carbonsäureamid; und
5-[3,6-Dihydro-3-methyl-2,6-dioxo-4-(trifluormethyl)-1(2H)-pyrimidinyl]-N-(dimethylsulfamoyl)-1,2-benzisothiazol-3-carbonsäureamid.

15. Verfahren nach Anspruch 12, wobei es sich bei dem Getreide um Reis handelt und die Verbindung aus der aus den folgenden Verbindungen bestehenden Gruppe ausgewählt ist:
{{2-{5-[3,6-Dihydro-3-methyl-2,6-dioxo-4-(trifluormethyl)-1(2H)-pyrimidinyl]-1,2-benzisothiazol-3-yl]-*p*-tolyl}oxy}essigsäureisopropylester;
{{2-{5-[3,6-Dihydro-3-methyl-4-(trifluormethyl)-2,6-dioxo-1(2H)-pyrimidinyl]-1,2-benzisothiazol-3-yl}-*p*-tolyl}oxy}-essigsäuremethylester;
3-[3-(6-Methoxy-*m*-tolyl)-1,2-benzisothiazol-5-yl]-1-methyl-6-(trifluormethyl)-2,4(1H,3H)-pyrimidindion;
{{2-{5-[3,6-Dihydro-3-methyl-2,6-dioxo-4-(trifluormethyl)-1(2H)-pyrimidinyl]-1,2-benzisothiazol-3-yl}-*p*-tolyl}oxy}essigsäureethylester;
1-Methyl-3-{3-[6-(2-propinyloxy)-*m*-tolyl]-1,2-benzisothiazol-5-yl}-6-(trifluormethyl)-2,4(1H,3H)-pyrimidindion;
3-{3-{6-{[(*m*-Cyanophenyl)carbamoyl]methoxy}-*m*tolyl}-1,2-benzisothiazol-5-yl}-1-methyl-6-(trifluormethyl)-2,4(1H,3H)-pyrimidindion;
3-[3-(2-Methoxy-*p*-tolyl)-1,2-benzisothiazol-5-yl]-1-methyl-6-(trifluormethyl)-2,4(1H,3H)-pyrimidindion;
2-[3-(6-Methoxy-3,4-xylyl)-1,2-benzisothiazol-5-yl)-1-methyl-6-(trifluormethyl)-2,4(1H,3H)-pyrimidindion;
3-{3-{6-{[(*m*-Cyanophenyl)carbamoyl]methoxy]-*m*tolyl}-1,2-benzisothiazol-5-yl}-1-methyl-6-(trifluormethyl)-2,4(1H,3H)-pyrimidindion;
{*o*-{5-[3,6-Dihydro-3-methyl-2,6-dioxo-4-(trifluormethyl)-1(2H)-pyrimidinyl]-1,2-benzisothiazol-3-yl}phenoxy}essigsäure-2-propinylester;
3-{3-[(Allyloxy)methyl]-1,2-benzisothiazol-5-yl]-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl]-a-ethyl-1,2-benzisothiazol-3-acetonitril;
5-[3,6-Dihydro-3-methyl-2,6-dioxo-4-(trifluormethyl)-1(2H)-pyrimidinyl]-a,a-diethyl-1,2-benzisothiazol-3-acetonitril;
3-[3-(Hydroxymethyl)-1,2-benzisothiazol-5-yl]-1-methyl-6-(trifluormethyl)-2,4(1H,3H)-pyrimidindion-5-isoxazolcarboxylatester;
3-[3-(Hydroxymethyl)-1,2-benzisothiazol-5-yl]-1-methyl-6-(trifluormethyl)-2,4(1H,3H)-pyrimidindion-cyclopropancarboxylatester;
5-[3,6-Dihydro-3-methyl-2,6-dioxo-4-(trifluormethyl)-1(2H)-pyrimidinyl]-1,2-benzisothiazol-3-carboxylat-methylester;
5-[3,6-Dihydro-3-methyl-2,6-dioxo-4-(trifluormethyl)-1(2H)-pyrimidinyl]-N-(isopropylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(ethylsulfonyl)-1,2-benzisothiazol-3-carbonsäureamid;
5-[3,6-Dihydro-3-methyl-2,6-dioxo-4-(trifluormethyl)-1(2H)-pyrimidinyl]-a,a-dimethyl-1,2-benzisothiazol-3-essigsäurebenzylester;
5-[3,6-Dihydro-3-methyl-2,6-dioxo-4-(trifluormethyl)-1(2H)-pyrimidinyl]-N-(diisopropylsulfamoyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfamoyl)-1,2-benzisothiazol-3-carbonsäureamid; und
5-[3,6-Dihydro-3-methyl-2,6-dioxo-4-(trifluormethyl)-1(2H)-pyrimidinyl]-N-(dimethylsulfamoyl)-1,2-benzisothiazol-3-carbonsäureamid.

16. Verfahren nach Anspruch 11, wobei es sich bei dem Getreide um Sojabohnen handelt und die Verbindung aus der aus den folgenden Verbindungen bestehenden Gruppe ausgewählt ist:
2-{{2-{5-[3,6-Dihydro-3-methyl-4-(trifluormethyl)-2,6-dioxo-1(2H)-pyrimidinyl)-1,2-benzisothiazol-3-yl}-*p*-tolyl}oxy}propionsäuremethylester;
2-{{6-{5-[3,6-Dihydro-3-methyl-4-(trifluormethyl)-2,6-dioxo-1(2H)-pyrimidinyl]-1,2-benzisothiazol-3-yl}-3,4-xylyl}oxy}propionsäuremethylester;
1-Methyl-3-(3-methyl-1,2-benzisothiazol-5-yl)-6-(trifluormethyl)-2,4(1H,3H)-pyrimidindion;
2-{(6-{5-[3,6-Dihydro-3-methyl-4-(trifluormethyl)-2,6-dioxo-1(2H)-pyrimidinyl]-1,2-benzisothiazol-3-yl}-3,4-xylyl}oxy}propionsäuremethylester;
{{2-{5-[3,6-Dihydro-3-methyl-2,6-dioxo-4-(trifluormethyl)-1(2H)-pyrimidinyl]-1,2-benzisoxazol-3-yl}-*p*-tolyl}oxy}essigsäure-2-propinylester;
3-[3-(*o*-Methoxyphenyl)-1,2-benzisothiazol-5-yl]-1-methyl-6-(trifluormethyl)-2,4(1H,3H)-pyrimidindion;
2-{{2-{5-[3,6-Dihydro-3-methyl-2,6-dioxo-4-(trifluormethyl)-1(2H)-pyrimidinyl]-1,2-benzisoxazol-3-yl}-*p*-tolyl}oxy}propionsäure-2-propinylester;
{*o*-{5-[3,6-Dihydro-3-methyl-2,6-dioxo-4-(trifluormethyl)-1(2H)-pyrimidinyl]-1,2-benzisothiazol-3-yl}phenoxy}essigsäure-2-propinylester;
3-{3-[(Allyloxy)methyl]-1,2-benzisothiazol-5-yl]-1-methyl-6-(trifluormethyl)-2,4(1H,3H)-pyrimidindion;
5-[3,6-Dihydro-3-methyl-2,6-dioxo-4-(trifluormethyl)-1(2H)-pyrimidinyl]-N-(methylsulfonyl)-1,2-benzisothiazol-3-carbonsäureamid;
{{2-{5-[3,6-Dihydro-3-methyl-2,6-dioxo-4-(trifluormethyl)-1(2H)-pyrimidinyl]-6-fluor-1,2-benzisooxazol-3-yl}-*p*-tolyl}oxy}essigsäuremethylester;
{{6-{5-[3,6-Dihydro-3-methyl-2,6-dioxo-4-(trifluormethyl)-1(2H)-pyrimidinyl]-fluor-1,2-benzisothiazol-3-yl}-*m*-tolyl}oxy}essigsäuremethylester;
3-[3-(Hydroxymethyl)-1,2-benzisothiazol-5-yl]-1-methyl-6-(trifluormethyl)-2,4(1H,3H)-pyrimidindion-benzoatester;
3-[3-(Hydroxymethyl)-1,2-benzisothiazol-5-yl]-1-methyl-6-(trifluormethyl)-2,4(1H,3H)-pyrimidindion-2-thiophencarboxylatester;
3-[3-(Hydroxymethyl)-1,2-benzisothiazol-5-yl]-1-methyl-6-(trifluormethyl)-2,4(1H,3H)-pyrimidindion-methoxyessigsäureester;
3-[3-(Hydroxymethyl)-1,2-benzisothiazol-5-yl]-1-methyl-6-(trifluormethyl)-2,4(1H,3H)-pyrimidindion-cyclopropancarboxylatester;
3-[3-(Hydroxymethyl)-1,2-benzisothiazol-5-yl]-1-methyl-6-(trifluormethyl)-2,4(1H,3H)-pyrimidindion-2-furoatester;
5-[3,6-Dihydro-3-methyl-2,6-dioxo-4-(trifluormethyl)-1(2H)-pyrimidinyl]-N-(methylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(ethylsulfonyl)-1,2-benzisothiazol-3-carbonsäureamid;
5-[3,6-Dihydro-3-methyl-2,6-dioxo-4-(trifluormethyl)-1(2H)-pyrimidinyl]-N-(isopropylsulfamoyl)-1,2-benzisothiazol-3-carbonsäureamid; und
5-[3,6-Dihydro-3-methyl-2,6-dioxo-4-(trifluormethyl)-1(2H)-pyrimidinyl]-N-(dimethylsulfamoyl)-1,2-benzisothiazol-3-carbonsäureamid.

17. Verfahren nach einem der Ansprüche 9 bis 16, bei dem man die Verbindung auf die Blätter der unerwünschten Pflanzenspezies oder den Boden bzw. das Wasser, das Samen oder anderes Vermehrungsgut davon enthält, in einer Aufwandmenge von etwa 0,001 kg/ha bis 1 kg/ha ausbringt.

18. Herbizide Zusammensetzung, enthaltend einen inerten Feststoff oder einen flüssigen Trägerstoff und eine herbizid wirksame Menge einer Verbindung nach einem der Ansprüche 1 bis 8.

19. Zwischenprodukte mit der Strukturformel 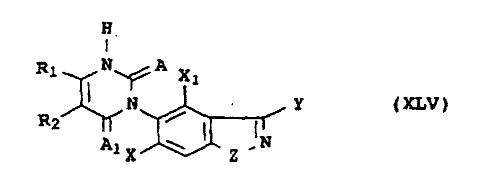 wobei R₁, R₂, X, X₁, Y, A, A₁ und Z wie in einem der Ansprüche 1, 2 und 4 definiert sind, und deren optischen Isomere, Diastereomere und/oder Tautomere.

20. Verfahren zur Herstellung einer Verbindung der Formel I oder II nach Anspruch 1, bei dem man:
a) eine Verbindung der Formel VIII 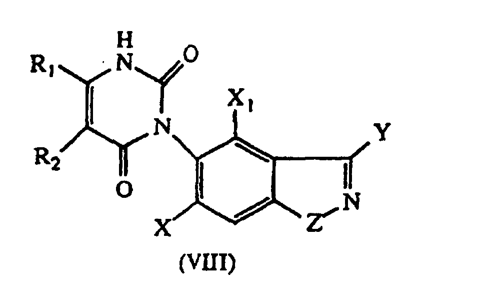 in welcher R₁, R₂, X, X₁, Y und Z wie in Anspruch 1 definiert sind, mit einer Verbindung der Formel
**Z**_{**1**}**R** (IX)
in welcher R wie in Anspruch 1 definiert ist und Z₁ für Chlor, Brom oder Iod steht, zu einer entsprechenden Verbindung der Formel I umsetzt;
b) eine Verbindung der Formel XI 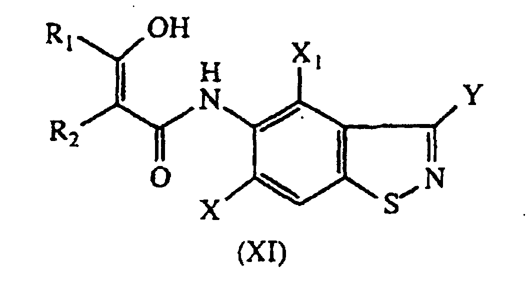 in welcher R₁, R₂, X, X₁ und Y wie in Anspruch 1 definiert sind, mit
(i) einer Verbindung der Formel
C(A)Cl₂
in welcher A für Sauerstoff oder Schwefel steht, und anschließend mit
(ii) einem Amin der Formel XII
RNH₂ (XII)
in welcher R wie in Anspruch 1 definiert ist, in Gegenwart einer Base, z.B. Triethylamin oder Pyridin, zu einer entsprechenden Verbindung der Formel I umsetzt, in welcher A für O oder S steht;
c) eine Verbindung der Formel (I), in welcher R, R₁, R₂, A, A₁ X, X₁ und Z wie in Anspruch 1 definiert sind und Y für CH₃ steht, mit einem Halogenierungsmittel, z.B. N-Bromsuccinimid, N-Chlorsuccinimid, N-Iodsuccinimid, Brom, Chlor, Sulfurylchlorid und Sufurylbromid, zu einer entsprechenden Verbindung der Formel I umsetzt, in welcher Y für Halogenmethyl steht;
d) eine Verbindung der Formel I, in welcher R, R₁, R₂, A, A₁, X, X₁ und Z wie in Anspruch 1 definiert sind und Y für Brommethyl steht, mit einem C₁-C₂₀-Alkohol zu einer entsprechenden Verbindung der Formel I umsetzt, in welcher Y für CH₂O(C₁-C₂₀-Alkyl) steht;
e) eine Verbindung der Formel I, in welcher R, R₁, R₂, A, A₁, X, X₁ und Z wie in Anspruch 1 definiert sind und Y für Brommethyl steht, mit einem Alkohol oder Thio der Formel
**HX**_{**4**}**R**_{**22**} (XXI)
in welcher X₄ und R₂₂ wie in Anspruch 1 definiert sind, in Gegenwart einer Base, z.B. Natriumhydrid, zu einer entsprechenden Verbindung der Formel I umsetzt, in welcher Y für CH₂X₄R₂₂ steht;
f) eine Verbindung der Formel I, in welcher R, R₁, R₂, A, A₁, X, X₁ und Z wie in Anspruch 1 definiert sind und Y für Brommethyl steht, mit einem Alkalithioalkanolat zu einer entsprechenden Verbindung der Formel I umsetzt, in welcher Y für CH₂S(C₁-C₈-Alkyl) steht;
g) eine Verbindung der Formel I, in welcher R, R₁, R₂, Y, X, X₁ und Z wie in Anspruch 1 definiert sind und A und A₁ für O stehen, mit Lawessons Reagens in Gegenwart einer Base, z.B. NaHCO₃, zu einer entsprechenden Verbindung der Formel I umsetzt, in welcher A für O und A₁ für S steht;
h) eine Verbindung der Formel I, in welcher R, R₁, R₂, Y, X, X₁ und Z wie in Anspruch 1 definiert sind und A für S und A₁ für O steht, mit Lawessons Reagens in Gegenwart einer Base zu einer entsprechenden Verbindung der Formel I umsetzt, in welcher A und A₁ für S stehen;
i) eine Verbindung der Formel I, in welcher wenigstens einer der Reste COOR₁₉, COOR₂₆ oder COOR₃₂ ein Ester ist, wobei R₁₉, R₂₆ und R₃₂ wie in Anspruch 1 definiert sind, zu einer Verbindung der Formel I hydrolysiert, in welcher wenigstens einer der Reste R₁₉, R₂₆ oder R₃₂ für Wasserstoff steht;
j) eine Verbindung der Formel I, in welcher wenigstens einer der Reste COOR₁₉, COOR₂₆ und COOR₃₂ für eine Carbonsäuregruppe steht, z.B. unter Verwendung von Thionylchlorid oder Oxalylchlorid, zu einem entsprechenden Säurechlorid halogeniert, und das Säurechlorid mit:
(i) einem Alkohol der Formel R₁₉OH, R₂₆OH oder R₃₂OH
oder
(ii) einem Amin der Formel HNR₂₀R₂₁, HNR₃₅R₃₆ oder HNR₄₉R₅₀
zu einer entsprechenden Verbindung der Formel I umsetzt, die eine COOR₁₉-, COOR₂₆-, COOR₃₂-, CONR₂₀R₂₁-, CONR₃₅R₃₆- oder CONR₄₉R₅₀-Gruppe enthält, wobei R₁₉, R₂₀, R₂₁, R₂₆, R₃₂, R₃₅, R₃₆, R49 und R₅₀ wie in Anspruch 1 definiert sind;
k) eine Verbindung der Formel I, in welcher wenigstens einer der Reste R₁₉, R₂₆ und R₃₂ für Wasserstoff steht, zu einer entsprechenden Verbindung der Formel I umsetzt, in welcher wenigstens einer der Reste R₁₉, R₂₆ und R₃₂ für ein Alkalimetall-, Erdalkalimetall- Mangan-, Kupfer-, Zink-, Cobalt-, Silber-, Nickel-, Ammonium- oder organisches Ammoniumkation steht;
l) eine Verbindung der Formel I, in welcher R, R₁, R₂, A, A₁, X, X₁ und Z wie in Anspruch 1 definiert sind und Y für Alkyl, Cycloalkyl, Alkenyl, Cycloalkenyl, Alkinyl oder Phenyl, substituiert durch eine CH₂OH-Gruppe, steht, mit einem Oxidationsmittel, z.B. Chrom(VI)-oxid, zu einer entsprechenden Verbindung der Formel I oxidiert, in welcher Y für Alkyl, Cycloalkyl, Alkenyl, Cycloalkenyl, Alkinyl oder Phenyl, substituiert durch eine CHO-Gruppe, steht;
m) eine Verbindung der Formel I, in welcher R, R₁, R₂, A, A₁, X, X₁ und Z wie in Anspruch 1 definiert sind und Y für Methyl steht, mit Cerammoniumnitrat zu einer entsprechenden Verbindung der Formel I umsetzt, in welcher Y für CHO steht;
n) eine Verbindung der Formel I, in welcher R, R₁, R₂, A, A₁, X, X₁ und Z wie in Anspruch 1 definiert sind und Y für CH₂Br steht, mit Kaliumcyanid zu einer entsprechenden Verbindung der Formel I umsetzt, in welcher Y für CH₂CN steht;
o) eine Verbindung der Formel I, in welcher R, R₁, R₂, A, A₁, X, X₁ und Z wie in Anspruch 1 definiert sind und R₁₂ für Bromalkyl steht, mit Lithiumchlorid, Natriumiodid, Kaliumfluorid oder Silber(I)-fluorid zu einer entsprechenden Verbindung der Formel I umsetzt, in welcher R₁₂ für Chloralkyl, Fluoralkyl oder Iodalkyl steht;
p) eine Verbindung der Formel: 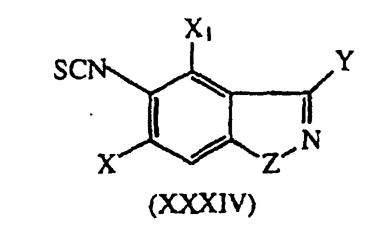 in welcher X, X₁, Y und Z wie in Anspruch 1 definiert sind, mit einem Alken der Formel: 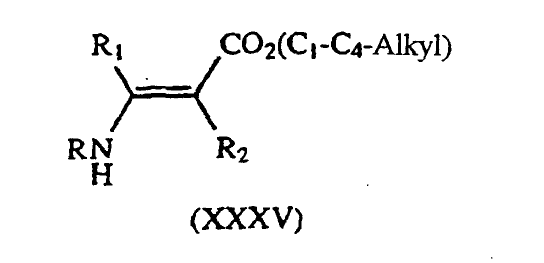 in welcher R, R₁ und R₂ wie in Anspruch 1 definiert sind, in Gegenwart einer Base, z.B. Natriumhydrid, zu einer entsprechenden Verbindung der Formel I umsetzt, in welcher R, R₁, R₂, X, X₁, Y und Z wie in Anspruch 1 definiert sind und A für S und A₁ für O steht;
q) eine Verbindung der Formel I, in welcher R, R₁, R₂, A, A₁, X, X₁ und Z wie in Anspruch 1 definiert sind und Y für COOH steht, mit (i) Oxalylchlorid oder Thionylchlorid und anschließend mit (ii) HX₂,R₁₂, in welcher X₂ und R₁₂ wie in Anspruch 1 definiert sind, in Gegenwart einer Base zu einer entsprechenden Verbindung der Formel I umsetzt, in welcher Y für C(O)X₂R₁₂ steht und X₂, R₁₂ wie in Anspruch 1 definiert sind;
r) eine Verbindung der Formel I, in welcher R, R₁, R₂, A, A₁, X, X₁ und Z wie in Anspruch 1 definiert sind und Y für COOH steht, mit
(i) einem Kupplungsmittel, z.B. 1-(3-Dimethylaminopropyl)-3-ethylcarbodiimid,
und
(ii) HNR₁₂,R₁₃, wobei R₁₂ und R₁₃ wie in Anspruch 1 definiert sind,
zu einer entsprechenden Verbindung der Formel I umsetzt, in welcher Y für C(O)NR₁₂R₁₃ steht und R₁₂ und R₁₃ wie in Anspruch 1 definiert sind;
s) eine Verbindung der Formel I, in welcher R, R₁, R₂, A, A₁, X, X₁ und Z wie in Anspruch 1 definiert sind und Y für COOH steht, mit:
(i) Oxalylchlorid oder Thionylchlord und
(ii) HNR₁₃SO₂NR₄₆R₄₈, wobei R₁₃, R₄₆ und R₄₈ wie in Anspruch 1 definiert sind, zu einer entsprechenden Verbindung der Formel I umsetzt, in welcher Y für C(O)NR₁₃SO₂NR₄₆R₄₈ steht und R₁₃, R₄₆ und R₄₈ wie in Anspruch 1 definiert sind;
t) eine Verbindung der Formel I, in welcher R, R₁, R₂, X, X₁, A, A₁ und Z wie in Anspruch 1 definiert sind und Y für COOH steht, mit:
(i) Oxalylchlorid oder Thionylchlorid und
(ii) HN(R₁₂)OR₂₃, wobei R₁₂ und R₂₃ wie in Anspruch 1 definiert sind, zu einer entsprechenden Verbindung der Formel I umsetzt, in welcher Y für C(O)N(R₁₂)OR₂₃ steht und R₁₂, R₂₃ wie in Anspruch 1 definiert sind;
u) eine Verbindung der Formel I, in welcher R, R₁, R₂, X, X₁, A, A₁ und Z wie in Anspruch 1 definiert sind und Y für CHO steht, mit R₂₃MgBr, in welcher R₂₃ wie in Anspruch 1 definiert ist, zu einer entsprechenden Verbindung der Formel I umsetzt, in welcher Y für C(OH)R₂₃ steht;
v) eine Verbindung der Formel I, in welcher R, R₁, R₂, A, A₁, X, X₁ und Z wie in Anspruch 1 definiert sind und Y für C(OH)R₂₃ steht, mit einem Oxidationsmittel zu einer entsprechenden Verbindung der Formel I umsetzt, in welcher Y für C(O)R₂₃ steht, wobei R₂₃ wie in Anspruch 1 definiert ist;
w) eine Verbindung der Formel I, in welcher R, R₁, R₂, A, A₁, X, X₁ und Z wie in Anspruch 1 definiert sind und Y für C(OH)R₂₃ steht, mit R₂₂X, wobei R₂₂ wie in Anspruch 1 definiert ist und X für Chlor oder Brom steht, in Gegenwart einer Base zu einer entsprechenden Verbindung der Formel I umsetzt, in welcher Y für C(OR₂₂)R₂₃ steht, wobei R₂₂ und R₂₃ wie in Anspruch 1 definiert ist;
x) eine Verbindung der Formel I, in welcher R, R₁, R₂, A, A₁, X, X₁ und Z wie in Anspruch 1 definiert sind und Y für C(OH)R₂₃ steht, wobei R₂₃ wie in Anspruch 1 definiert ist, mit XC(OH)R₃₃, wobei R₃₃ wie in Anspruch 1 definiert ist und X für Chlor oder Brom steht, zu einer entsprechenden Verbindung der Formel I umsetzt, in welcher Y für CH(R₂₃)OC(O)R₃₃ steht, wobei R₂₃ und R₃₃ wie in Anspruch 1 definiert sind;
oder
y) eine Verbindung der Formel (I) oder (II) mit einer reaktiven Substituentengruppe mit einem geeigneten Reagens in eine andere Verbindung der Formel (I) oder (II) umwandelt.

21. Verfahren zur Darstellung eines Zwischenprodukts der Formel (XLV) nach Anspruch 19, bei dem man:
a) eine Verbindung der Formel: 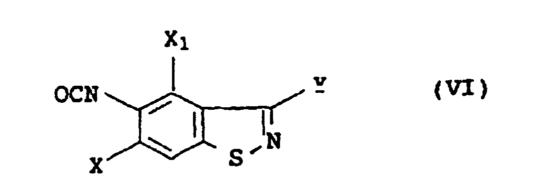 in welcher X, X₁ und Y wie in Anspruch 1 definiert sind, mit einer Verbindung der Formel:
R₁C(NH₂)=CR₂-CO₂(C₁-C₄-Alkyl) (VII)
in welcher R₁ und R₂ wie in Anspruch 1 definiert sind, zu einer Verbindung der Formel: 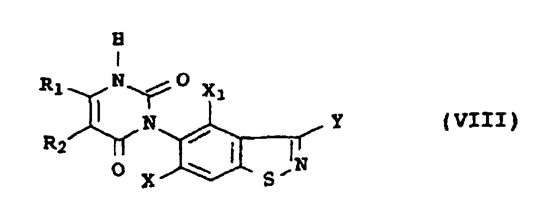 umsetzt, in welcher R₁, R₂, X, X₁ und Y wie in Anspruch 1 definiert sind;
b) eine Verbindung der Formel: 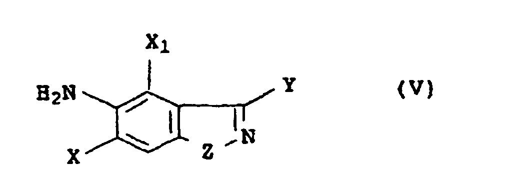 in welcher X, X₁, Z und Y wie in Anspruch 1 definiert sind, mit einer Verbindung der Formel: 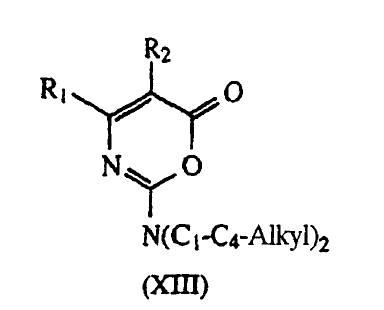 in welcher R₁ und R₂ wie in Anspruch 1 definiert sind, in Gegenwart einer organischen Säure, z.B. Essigsäure, zu einer wie oben gezeigten Verbindung der Formel VIII umsetzt, in welcher R₁, R₂, X, X₁ und Y wie in Anspruch 1 definiert sind;
oder
c) eine Verbindung der Formel XXXVIII: 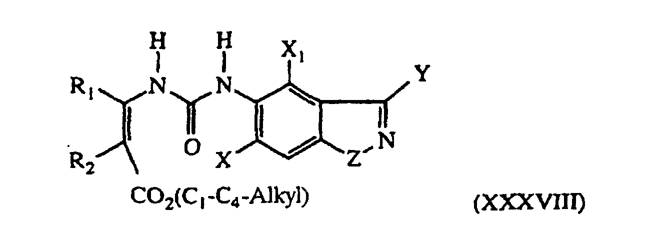 in welcher R₁, R₂, X, X₁, Z und Y wie in Anspruch 1 definiert sind, mit Säure zu einer Verbindung der Formel: 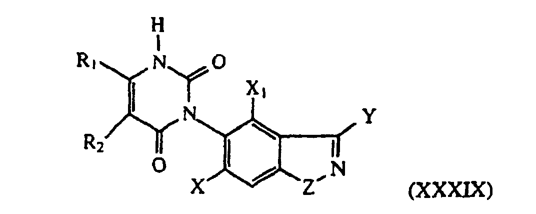 umsetzt, in welcher R₁, R₂, X, X₁, Y und Z wie in Anspruch 1 definiert sind.

## Revendications

1. Composé ayant pour formule développée 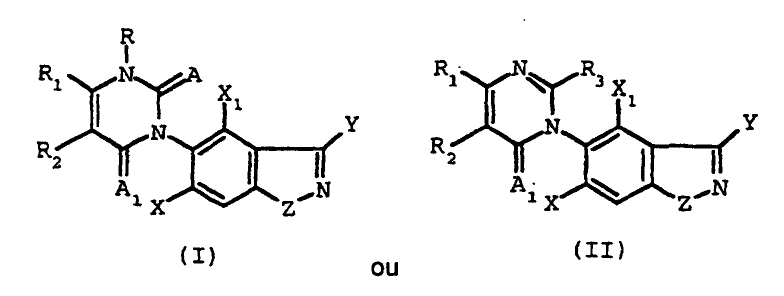 dans laquelle
R est un groupe alkyle en C₁-C₆, halogénoalkyle en C₁-C₆, cycloalkyle en C₃-C₇, alcényle en C₃-C₆, alcynyle en C₃-C₆, benzyle, OR₄ ou NR₅R₆ ;
R₁ et R₂ sont chacun indépendamment un hydrogène, un halogène, un groupe alkyle en C₁-C₆, cycloalkyle en C₃-C₇, halogénoalkyle en C₁-C₆, alcényle en C₃-C₆, halogénoalcényle en C₃-C₆, alcynyle en C₃-C₆, OR₇, S(O)ₙR₈ ou NR₉R₁₀, et lorsque R₁ et R₂ sont pris ensemble avec les atomes auxquels ils sont attachés, ils représentent un cycle saturé ou insaturé ayant quatre à sept chaînons éventuellement interrompu par un oxygène, un soufre ou un azote, et éventuellement substitué par un à trois groupes méthyle ou un ou plusieurs atomes d'halogène ;
R₃ est un halogène ou A₂R₁₁ ;
X est un hydrogène, un halogène ou un groupe alkyle en C₁-C₄ ;
X₁ est un hydrogène, un halogène, un groupe alkyle en C₁-C₄, halogénoalkyle en C₁-C₄, alcoxy en C₁-C₄ ou halogénoalcoxy en C₁-C₄ ;
Z est O ou S(O)ₘ;
Y est un halogène, un groupe cyano, R₁₂, X₂R₁₂, X₃R₁₂, X₂X₃R₁₂ ou X₃X₂R₁₂ ;
X₂ est O, S(O)ₚ ou NR₁₃;
X₃ est -C(=A₃)-, -C(=NOR₁₄)- ou -C(=N-NR₁₃R₅₀) ;
R₄, R₅, R₆, R₇, R₈, R₉, R₁₀, R₁₁ et R₁₄ sont chacun indépendamment un hydrogène, un groupe alkyle en C₁-C₆, cycloalkyle en C₃-C₇, alcoxyalkyle en C₂-C₈, alcényle en C₂-C₆, alcynyle en C₂-C₆ ou benzyle ;
R₁₂ est un hydrogène,
un groupe alkyle en C₁-C₈, cycloalkyle en C₃-C₇, alcényle en C₂-C₈, cycloalcényle en C₅-C₆ ou alcynyle en C₂-C₈, où chaque groupe est éventuellement substitué par toute combinaison d'un à six atomes d'halogène, un à trois groupes alcoxy en C₁-C₂₀ éventuellement substitués par un groupe alcoxy en C₁-C₆, benzyloxy ou alkylthio en C₁-C₆, un ou deux groupes halogénoalcoxy en C₁-C₁₀, un ou deux groupes NR₁₅R₁₆, un à trois groupes S(O)_{q}R₁₇, un ou deux groupes cyano, un ou deux groupes cycloalkyle en C₃-C₇, un groupe thiocyano, un groupe O(SO₂)R₁₇, un groupe O(NO₂), un ou deux groupes C(O)R₁₈, un ou deux groupes CO₂R₁₉, un ou deux groupes C(O)SR₁₉, un ou deux groupes C(O)NR₂₀R₂₁, un à trois groupes X₄R₂₂, un ou deux groupes P(O)(OR₂₃)₂, un ou deux groupes Si(R₂₄)₃,
un noyau hétérocyclique, monocyclique ou bicyclique condensé, ayant 4 à 10 chaînons éventuellement substitué par toute combinaison d'un ou deux groupes oxo, un ou deux groupes thioxo, un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un ou deux groupes X₄R₂₂,
un groupe ammonium organique quaternaire, ou
un ou deux groupes phényle où chaque groupe phényle est indépendamment éventuellement substitué par toute combinaison d'un à trois atomes d'halogène, un à trois groupes alkyle en C₁-C₆, un à trois groupes alcoxy en C₁-C₆, un groupe cycloalkyle en C₃-C₇, un groupe halogénoalkyle en C₁-C₄, un groupe alkylthio en C₁-C₄, un groupe cyano, un groupe nitro, un groupe C(O)R₂₅, un groupe CO₂R₂₆ ou un groupe X₅R₂₇,
un groupe phényle éventuellement substitué par toute combinaison d'un à trois atomes d'halogène, un à trois groupes alkyle en C₁-C₄, un à trois groupes alcoxy en C₁-C₄, un groupe cycloalkyle en C₃-C₆, un groupe halogénoalkyle en C₁-C₄, un groupe alkylthio en C₁-C₄, un groupe cyano, un groupe nitro, un groupe C(O)R₂₅, un groupe CO₂R_{26,} un groupe X₄R₂₂, un groupe CH=CHR₂₂, un groupe CH₂CH(R₂₈)R₂₂ ou un groupe N(R₂₉)SO₂R₃₀, ou
un groupe indane éventuellement substitué par toute combinaison d'un ou deux atomes d'halogène, un ou deux groupes alkyle en C₁-C₄, un groupe alcoxy en C₁-C₄, un groupe halogénoalkyle en C₁-C₄, un groupe alkylthio en C₁-C₄, un groupe cyano, un groupe nitro, un groupe C(O)R₂₅, un groupe CO₂R₂₆, un groupe X₄R₂₂, un groupe CH=CHR₂₂, un groupe CH₂CH(R₂₈)R₂₂ ou un groupe N(R₂₉)SO₂R₃₀ ;
R₁₃ est un hydrogène, un groupe alcoxyalkyle en C₁-C₈, alkyle en C₁-C₆, cycloalkyle en C₃-C₇, alcényle en C₃-C₆, alcynyle en C₃-C₆, benzyle, OR₂₃, cyano, SO₂R₅₁ ou un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄ ou halogénoalcoxy en C₁-C₄, et lorsque R₁₂ et R₁₃ sont pris ensemble avec l'atome d'azote auquel ils sont attachés, ils forment un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄, halogénoalkylsulfonyle en C₁-C₄ ou X₄R₂₂ ;
R₂₈ est un halogène ;
R₂₂ et R₂₇ sont chacun indépendamment un hydrogène, Si(R₃₁)₃, C(O)NR₃₅R₃₆, C(O)R₃₃, SO₂R₄₇.
un groupe alkyle en C₁-C₂₀ substitué par un groupe hydroxyle, benzyloxy, nitro, OC(O)R₃₃, alcoxy en C₁-C₆, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NR₃₅OR₂₃, cyano ou 2-dioxolanyle, un noyau phényle, dihydrofuranone ou furanone
dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄, ou
un noyau hétérocyclique monocyclique ou bicyclique condensé ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄ ou halogénoalkylsulfonyle en C₁-C₄ ;
un groupe alcényle en C₃-C₂₀ éventuellement substitué par un à trois atomes d'halogène, un groupe hydroxyle, un groupe alcoxy en C₁-C₆, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe cycloalcényle en C₅-C₈ éventuellement substitué par un groupe hydroxyle, alcoxy en C₁-C₆, alkyle en C₁-C₄, alcényle en C₂-C₄, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes alcoxy en C₁-C₄ ou un à trois groupes halogénoalcoxy en C₁-C₄,
un groupe alcynyle en C₃-C₁₀ éventuellement substitué par un groupe hydroxyle, alcoxy en C₁-C₆, CO₂R₃₂, C(O)R₃₃, C(A₄R₃₄)₂, C(O)NR₃₅R₃₆, C(O)ON=CR₃₇R₃₈, C(O)NR₃₅OR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe phényle éventuellement substitué par toute combinaison d'un à trois atomes d'halogène, un à trois groupes alkyle en C₁-C₆, un à trois groupes alcoxy en C₁-C₆, un groupe halogénoalkyle en C₁-C₄, un groupe halogénoalcoxy en C₁-C₄, un groupe cyano, un groupe nitro, un groupe NR₃₉R₄₀ ou un groupe C(O)R₃₃,
un groupe benzyle éventuellement substitué sur le noyau phényle par toute combinaison d'un à trois atomes d'halogène, un à trois groupes alkyle en C₁-C₆, un à trois groupes alcoxy en C₁-C₆, un groupe halogénoalkyle en C₁-C₄, un groupe halogénoalcoxy en C₁-C₄, un groupe cyano, un groupe nitro, un groupe NR₃₉R₄₀ ou un groupe C(O)R₃₃, ou
un noyau hétérocyclique ayant 4 à 10 chaînons éventuellement substitué par toute combinaison d'un ou deux groupes oxo, un ou deux groupes thioxo, un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄ ;
R₁₅ et R₂₀ sont chacun indépendamment un hydrogène,
un groupe alkyle en C₁-C₆, cycloalkyle en C₃-C₇, alcényle en C₂-C₆ ou alcynyle en C₂-C₆ où chaque groupe est éventuellement substitué par un groupe CO₂R₄₂,
un groupe benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe CO₂R₂₆,
C(O)R₄₃, ou
CO₂R₄₂;
R₁₆ et R₂₁ sont chacun indépendamment un hydrogène, un groupe alkyle en C₁-C₆, alcoxy en C₁-C₄, cycloalkyle en C₃-C₇, alcényle en C₂-C₆, cycloalcényle en C₃-C₇, alcynyle en C₂-C₆, P(O)(OR₄₄)₂, SO₂R₄₅, C(O)R₄₆,
un groupe benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un groupe X₄R₂₂, ou
un groupe phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un groupe X₄R₂₂ ;
R₁₇, R₁₈, R₂₅, R₃₀, R₃₃, R₄₅ et R₅₁ sont chacun indépendamment NR₄₆R₄₈, -N=CR₄₆R₄₈, un groupe alkyle en C₁-C₈ éventuellement substitué par un groupe alcoxy en C₁-C₆, thiophène ou furane, un groupe halogénoalkyle en C₁-C₆, cycloalkyle en C₃-C₈, CO₂R₂₃, alcényle en C₂-C₆ éventuellement substitué par un groupe phényle,
un groupe benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe nitro, un groupe cyano ou un groupe X₄R₂₂,
un groupe phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe nitro, un groupe cyano ou un groupe X₄R₂₂, ou
un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄, halogénoalkylsulfonyle en C₁-C₄ ou X₄R₂₂ ;
R₁₉ et R₂₆ sont chacun indépendamment un hydrogène, un groupe alkyle en C₁-C₂₀, halogénoalkyle en C₁-C₈, cycloalkyle en C₃-C₇, halogénocycloalkyle en C₃-C₇, alcényle en C₃-C₂₀, halogénoalcényle en C₃-C₈, cycloalcényle en C₅-C₈, halogénocycloalcényle en C₅-C₈, alcynyle en C₃-C₈, halogénoalcynyle en C₃-C₈, Si(R₄₁)₃, benzyle, phényle, furfuryle, pyridyle, thiényle, oximino ou un métal alcalin, métal alcalino-terreux, manganèse, cuivre, zinc, cobalt, argent, nickel, cation ammonium ou ammonium organique ;
R₃₂ est un hydrogène, un groupe
Si(R₄₇)₃,
di(alkyle en C₁-C₄)amino,
alkyle en C₁-C₂₀ éventuellement substitué par un groupe CO₂R₄₈, alcoxy en C₁-C₆, thioalkyle en C₁-C₆, alcoxyalcoxy en C₂-C₂₀, phényle, OSi(R₄₇)₃, OC(O)R₃₃, cycloalkyle en C₃-C₇ ou di(alkyle en C₁-C₄)amino,
halogénoalkyle en C₁-C₁₅,
cycloalkyle en C₃-C₈ éventuellement substitué par un groupe CO₂R₄₈,
halogénocycloalkyle en C₃-C₈,
alcényle en C₃-C₂₀ éventuellement substitué par un groupe phényle,
halogénoalcényle en C₃-C₁₅,
cycloalcényle en C₅-C₈,
halogénocycloalcényle en C₅-C₈,
alcynyle en C₃-C₂₀ éventuellement substitué par un groupe phényle,
halogénoalcynyle en C₃-C₂₀,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
2-, 3- ou 4-pyridyle,
2- ou 3-furyle,
2- ou 3-thiényle,
2-tétrahydrofuranyle,
alkyle en C₁-C₄ substitué par un groupe pyridyle, furyle, thiényle, tétrahydrofuryle, cycloalkyle en C₃-C₈, dioxane ou NR₄₉R₅₀, ou
un métal alcalin, métal alcalino-terreux, manganèse, cuivre, zinc, cobalt, argent, nickel, cation ammonium ou ammonium organique ;
R₃₄, R₃₇ et R₃₈ sont chacun indépendamment un groupe alkyle en C₁-C₆;
R₃₅ et R₄₉ sont chacun indépendamment un hydrogène ou un groupe alkyle en C₁-C₆ et R₃₆ et R₅₀ sont chacun indépendamment un hydrogène, un groupe cyano, alkyle en C₁-C₆, SO₂R₅₁ ou phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou lorsque R₃₅ et R₃₅ ou R₄₉ et R₅₀ sont pris ensemble avec l'atome d'azote auquel ils sont attachés, ils forment un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄ ou halogénoalkylsulfonyle en C₁-C₄ ;
m, n, p et q sont chacun indépendamment un entier valant 0, 1 ou 2 ;
R₂₉ est un hydrogène, un groupe alkyle en C₁-C₄ ou halogénoalkyle en C₁-C₄ ;
R₂₄, R₃₁, R₄₁ et R₄₇ sont chacun indépendamment un groupe alkyle en C₁-C₄,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄;
R₂₃, R₃₉, R₄₀, R₄₂, R₄₃, R₄₄, R₄₆ et R₄₈ sont chacun indépendamment un hydrogène, un groupe alcényle en C₂-C₆, cycloalkyle en C₃-C₈,
alkyle en C₁-C₈ éventuellement substitué par un ou deux groupes alcoxy en C₁-C₄,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, et
lorsque R₄₆ et R₄₈ sont pris ensemble avec l'atome auquel ils sont attachés, ils forment un noyau cycloalkyle ou un noyau hétérocyclique, monocyclique ou bicyclique condensé, ayant 5 à 12 chaînons, noyau éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄ ou halogénoalkylsulfonyle en C₁-C₄ ; et
A, A₁, A₂, A₃, A₄, X₄ et X₅ sont chacun indépendamment O ou S ; et
les isomères optiques, diastéréoisomères et/ou tautomères de celui-ci.

2. Composé selon la revendication 1 dans lequel
R est un groupe alkyle en C₁-C₆, halogénoalkyle en C₁-C₆, cycloalkyle en C₃-C₇, alcényle en C₃-C₆, alcynyle en C₃-C₆, benzyle, OR₄ ou NR₅R₆ ;
R₁ et R₂ sont chacun indépendamment un hydrogène, un halogène, un groupe alkyle en C₁-C₆, cycloalkyle en C₃-C₇, halogénoalkyle en C₁-C₆, alcényle en C₃-C₆, halogénoalcényle en C₃-C₆, alcynyle en C₃-C₆, OR₇, S(O)ₙR₈ ou NR₉R₁₀, et lorsque R₁ et R₂ sont pris ensemble avec les atomes auxquels ils sont attachés, ils représentent un cycle saturé ou insaturé ayant quatre à sept chaînons éventuellement interrompu par un oxygène, un soufre ou un azote, et éventuellement substitué par un à trois groupes méthyle ou un ou plusieurs atomes d'halogène ;
R₃ est un halogène ou A₂R₁₁ ;
X est un hydrogène, un halogène ou un groupe alkyle en C₁-C₄ ;
X₁ est un hydrogène, un halogène, un groupe alkyle en C₁-C₄, halogénoalkyle en C₁-C₄, alcoxy en C₁-C₄ ou halogénoalcoxy en C₁-C₄ ;
Z est O ou S(O)ₘ ;
Y est un halogène, un groupe cyano, R₁₂, X₂R₁₂, X₃R₁₂, X₂X₃R₁₂ ou X₃X₂R₁₂;
X₂ est O, S(O)ₚ ou NR₁₃;
X₃ est -C(=A₃)- ou -C(=NOR₁₄)- ;
R₄, R₅, R₆, R₇, R₈, R₉, R₁₀, R₁₁ et R₁₄ sont chacun indépendamment un hydrogène, un groupe alkyle en C₁-C₆, cycloalkyle en C₃-C₇, alcényle en C₂-C₆, alcynyle en C₂-C₆ ou benzyle ;
R₁₂ est un hydrogène,
un groupe alkyle en C₁-C₆, cycloalkyle en C₃-C₇, alcényle en C₂-C₆, cycloalcényle en C₅-C₆ ou alcynyle en C₂-C₆, où chaque groupe est éventuellement substitué par toute combinaison d'un à six atomes d'halogène, un à trois groupes alcoxy en C₁-C₂₀, un ou deux groupes halogénoalcoxy en C₁-C₁₀, un ou deux groupes NR₁₅R₁₆, un à trois groupes S(O)_{q}R₁₇, un ou deux groupes cyano, un ou deux groupes C(O)R₁₈, un ou deux groupes CO₂R₁₉, un ou deux groupes C(O)NR₂₀R₂₁, un à trois groupes X₄R₂₂, un ou deux groupes P(O)(OR₂₃)₂, un ou deux groupes Si(R₂₄)₃,
un noyau hétérocyclique, monocyclique ou bicyclique condensé, ayant 4 à 10 chaînons éventuellement substitué par toute combinaison d'un ou deux groupes oxo, un ou deux groupes thioxo, un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un ou deux groupes X₄R₂₂,
un groupe ammonium organique quaternaire, ou
un groupe phényle éventuellement substitué par toute combinaison d'un à trois atomes d'halogène, un à trois groupes alkyle en C₁-C₆, un à trois groupes alcoxy en C₁-C₆, un groupe cycloalkyle en C₃-C₇, un groupe halogénoalkyle en C₁-C₄, un groupe alkylthio en C₁-C₄, un groupe cyano, un groupe nitro, un groupe C(O)R₂₅, un groupe CO₂R₂₆ ou un groupe X₅R₂₇, ou
un groupe phényle éventuellement substitué par toute combinaison d'un à trois atomes d'halogène, un à trois groupes alkyle en C₁-C₄, un à trois groupes alcoxy en C₁-C₄, un groupe cycloalkyle en C₃-C₆, un groupe halogénoalkyle en C₁-C₄, un groupe alkylthio en C₁-C₄, un groupe cyano, un groupe nitro, un groupe C(O)R₂₅, un groupe CO₂R₂₆, un groupe X₄R₂₂, un groupe CH=CHR₂₂, un groupe CH₂CH(R₂₈)R₂₂ ou un groupe N(R₂₉)SO₂R₃₀,
et lorsque R₁₂ et R₁₃ sont pris ensemble avec l'atome d'azote auquel ils sont attachés, ils forment un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄, halogénoalkylsulfonyle en C₁-C₄ ou X₄R₂₂ ;
R₁₃ est un hydrogène, un groupe alkyle en C₁-C₆, cycloalkyle en C₃-C₇, alcényle en C₃-C₆, alcynyle en C₃-C₆, benzyle, OR₂₃, cyano ou SO₂R₅₁, et lorsque R₁₃ et R₁₂ sont pris ensemble avec l'atome d'azote auquel ils sont attachés, ils forment un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄, halogénoalkylsulfonyle en C₁-C₄ ou X₄R₂₂ ;
R₂₈ est un halogène ;
R₂₂ et R₂₇ sont chacun indépendamment un hydrogène, Si(R₃₁)₃, un groupe
alkyle en C₁-C₂₀ substitué par un groupe alcoxy en C₁-C₆, CO₂R₃₂, C(O)R₃₃, C(A₄R₃₄)₂, C(O)NR₃₅R₃₆, C(O)ON=CR₃₇R₃₈, P(O)(OH)NH₂, P(O)(OH)(OR₂₃), C(O)NHOR₂₃, cyano ou 2-dioxolanyle,
un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄, ou
un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄ ou halogénoalkylsulfonyle en C₁-C₄,
un groupe alcényle en C₃-C₂₀ éventuellement substitué par un à trois atomes d'halogène, un groupe alcoxy en C₁-C₆, CO₂R₃₂, C(O)R₃₃, C(A₄R₃₄)₂, C(O)NR₃₅R₃₆, C(O)ON=CR₃₇R₃₈, C(O)NHOR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe cycloalcényle en C₅-C₈ éventuellement substitué par un groupe alcoxy en C₁-C₆, alkyle en C₁-C₄, alcényle en C₂-C₄, CO₂R₃₂, C(O)R₃₃, C(A₄R₃₄)₂, C(O)NR₃₅R₃₆, C(O)ON=CR₃₇R₃₈, C(O)NHOR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes alcoxy en C₁-C₄ ou un à trois groupes halogénoalcoxy en C₁-C₄,
un groupe alcynyle en C₃-C₁₀ éventuellement substitué par un groupe alcoxy en C₁-C₆, CO₂R₃₂, C(O)R₃₃, C(A₄R₃₄)₂, C(O)NR₃₅R₃₆, C(O)ON=CR₃₇R₃₈, C(O)NHOR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe phényle éventuellement substitué par toute combinaison d'un à trois atomes d'halogène, un à trois groupes alkyle en C₁-C₆, un à trois groupes alcoxy en C₁-C₆, un groupe halogénoalkyle en C₁-C₄, un groupe halogénoalcoxy en C₁-C₄, un groupe cyano, un groupe nitro, un groupe NR₃₉R₄₀ ou un groupe C(O)R₃₃,
un groupe benzyle éventuellement substitué sur le noyau phényle par toute combinaison d'un à trois atomes d'halogène, un à trois groupes alkyle en C₁-C₆, un à trois groupes alcoxy en C₁-C₆, un groupe halogénoalkyle en C₁-C₄, un groupe halogénoalcoxy en C₁-C₄, un groupe cyano, un groupe nitro, un groupe NR₃₉R₄₀ ou un groupe C(O)R₃₃, ou
un noyau hétérocyclique ayant 4 à 10 chaînons éventuellement substitué par toute combinaison d'un ou deux groupes oxo, un ou deux groupes thioxo, un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄ ;
R₁₅ et R₂₀ sont chacun indépendamment un hydrogène,
un groupe alkyle en C₁-C₆, cycloalkyle en C₃-C₇, alcényle en C₂-C₆ ou alcynyle en C₂-C₆ où chaque groupe est éventuellement substitué par un groupe CO₂R₄₂,
un groupe benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄, ou
C(O)R₄₃ ;
R₁₆ et R₂₁ sont chacun indépendamment un hydrogène, un groupe alkyle en C₁-C₆, cycloalkyle en C₃-C₇, alcényle en C₂-C₆, cycloalcényle en C₃-C₇, alcynyle en C₂-C₆, P(O)(OR₄₄)₂, SO₂R₄₅, C(O)R₄₆,
benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un groupe X₄R₂₂, ou
phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un groupe X₄R₂₂ ;
R₁₇, R₁₈, R₂₅, R₃₀, R₃₃, R₄₅ et R₅₁ sont chacun indépendamment un groupe alkyle en C₁-C₄, halogénoalkyle en C₁-C₄, CO₂R₂₃,
alcényle en C₂-C₆ éventuellement substitué par un groupe phényle,
benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe nitro, un groupe cyano ou un groupe X₄R₂₂,
phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe nitro, un groupe cyano ou un groupe X₄R₂₂, ou
un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄, halogénoalkylsulfonyle en C₁-C₄ ou X₄R₂₂ ;
R₁₉ et R₂₆ sont chacun indépendamment un hydrogène, un groupe alkyle en C₁-C₂₀, halogénoalkyle en C₁-C₈, cycloalkyle en C₃-C₇, halogénocycloalkyle en C₃-C₇, alcényle en C₃-C₂₀, halogénoalcényle en C₃-C₈, cycloalcényle en C₅-C₈, halogénocycloalcényle en C₅-C₈, alcynyle en C₃-C₈, halogénoalcynyle en C₃-C₈, Si(R₄₁)₃, benzyle, phényle, furfuryle, pyridyle, thiényle, oximino ou un métal alcalin, métal alcalino-terreux, manganèse, cuivre, zinc, cobalt, argent, nickel, cation ammonium ou ammonium organique ;
R32 est un hydrogène, un groupe
Si(R₄₇)₃,
di(alkyle en C₁-C₄)amino,
alkyle en C₁-C₂₀ éventuellement substitué par un groupe CO₂R₄₈, alcoxy en C₁-C₆ ou di(alkyle en C₁-C₄)amino,
halogénoalkyle en C₁-C₁₅,
cycloalkyle en C₃-C₈,
halogénocycloalkyle en C₃-C₈,
alcényle en C₃-C₂₀ éventuellement substitué par un groupe phényle,
halogénoalcényle en C₃-C₁₅,
cycloalcényle en C₅-C₈,
halogénocycloalcényle en C₅-C₈,
alcynyle en C₃-C₂₀ éventuellement substitué par un groupe phényle,
halogénoalcynyle en C₃-C₂₀,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
2-, 3- ou 4-pyridyle,
2- ou 3-furyle,
2- ou 3-thiényle,
2-tétrahydrofuranyle,
alkyle en C₁-C₄ substitué par un groupe pyridyle, furyle, thiényle, tétrahydrofuryle ou NR₄₉R₅₀, ou
un métal alcalin, métal alcalino-terreux, manganèse, cuivre, zinc, cobalt, argent, nickel, cation ammonium ou ammonium organique ;
R₃₄, R₃₇ et R₃₈ sont chacun indépendamment un groupe alkyle en C₁-C₆;
R₃₅ et R₄₉ sont chacun indépendamment un hydrogène ou un groupe alkyle en C₁-C₆ et R₃₆ et R₅₀ sont chacun indépendamment un hydrogène, un groupe cyano, alkyle en C₁-C₆, SO₂R₅₁ ou phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou lorsque R₃₅ et R₃₆ ou R₄₉ et R₅₀ sont pris ensemble avec l'atome d'azote auquel ils sont attachés, ils forment un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄ ou halogénoalkylsulfonyle en C₁-C₄ ;
m, n, p et q sont chacun indépendamment un entier valant 0, 1 ou 2 ;
R₂₉ est un hydrogène, un groupe alkyle en C₁-C₄ ou halogénoalkyle en C₁-C₄ ;
R₂₄, R₃₁, R₄₁ et R₄₇ sont chacun indépendamment un groupe alkyle en C₁-C₄,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄;
R₂₃, R₃₉, R₄₀, R₄₂, R₄₃, R₄₄, R₄₆ et R₄₈ sont chacun indépendamment un hydrogène, un groupe alkyle en C₁-C₄,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄ ; et
A, A₁, A₂, A₃, A₄, X₄ et X₅ sont chacun indépendamment O ou S ; et
les isomères optiques, diastéréoisomères et/ou tautomères de celui-ci.

3. Composé selon la revendication 1 ayant pour formule développée

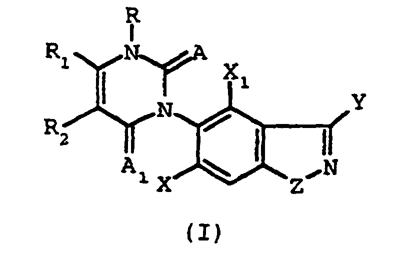

4. Composé selon la revendication 3 dans lequel
R est un groupe alkyle en C₁-C₄, halogénoalkyle en C₁-C₄, alcényle en C₃-C₄, alcynyle en C₃-C₄ ou benzyle ;
R₁ est un halogène ou un groupe halogénoalkyle en C₁-C₄ ;
R₂ est un hydrogène, un halogène ou un groupe halogénoalkyle en C₁-C₄ ;
X et X₁ sont chacun indépendamment un hydrogène ou un halogène ;
Z est O ou S ;
Y est un groupe cyano, R₁₂, X₂R₁₂ ou X₃X₂R₁₂ ;
X₂ est O, S ou NR₁₃ ;
R₁₂ est un hydrogène,
un groupe alkyle en C₁-C₆, cycloalkyle en C₃-C₇, alcényle en C₂-C₆, cycloalcényle en C₅-C₆ ou alcynyle en C₂-C₆, où chaque groupe est éventuellement substitué par toute combinaison d'un à trois atomes d'halogène, un ou deux groupes alcoxy en C₁-C₂₀, un ou deux groupes halogénoalcoxy en C₁-C₁₀, un groupe NR₁₅R₁₆, un groupe S(O)_{q}R₁₇, un ou deux groupes cyano, un ou deux groupes C(O)R₁₈, un ou deux groupes CO₂R₁₉, un ou deux groupes C(O)SR₁₉, un ou deux groupes C(O)NR₂₀R₂₁, un ou deux groupes X₄R₂₂, un groupe P(O)(OR₂₃)₂, un groupe Si(R₂₄)₃,
un noyau hétérocyclique, monocyclique ou bicyclique condensé, ayant 4 à 10 chaînons éventuellement substitué par toute combinaison d'un ou deux groupes oxo, un ou deux groupes thioxo, un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄ ou un ou deux groupes X₄R₂₂,
un groupe ammonium organique quaternaire, ou
un groupe phényle éventuellement substitué par toute combinaison d'un à trois atomes d'halogène, un groupe alkyle en C₁-C₆, un groupe alcoxy en C₁-C₆, un groupe cycloalkyle en C₃-C₇, un groupe cyano, un groupe nitro, un groupe C(O)R₂₅, un groupe CO₂R₂₆ ou un groupe X₅R₂₇, ou
un groupe phényle éventuellement substitué par toute combinaison d'un ou deux atomes d'halogène, un à trois groupes alkyle en C₁-C₄, un ou deux groupes alcoxy en C₁-C₄, un groupe cycloalkyle en C₃-C₆, un groupe halogénoalkyle en C₁-C₄, un groupe alkylthio en C₁-C₄, un groupe cyano, un groupe nitro, un groupe C(O)R₂₅, un groupe CO₂R₂₆, un groupe X₄R₂₂, un groupe CH=CHR₂₂, un groupe CH₂CH(R₂₈)R₂₂ ou un groupe N(R₂₉)SO₂R₃₀ ;
R₁₃ est un hydrogène, un groupe alcoxyalkyle en C₂-C₆, alkyle en C₁-C₄, cycloalkyle en C₃-C₇, alcényle en C₃-C₆, alcynyle en C₃-C₆, benzyle, OR₂₃, cyano, ou SO₂R₅₁ ;
R₁₄ est un hydrogène, un groupe alkyle en C₁-C₆, cycloalkyle en C₃-C₇, alcoxyalkyle en C₂-C₈, alcényle en C₂-C₆, alcynyle en C₂-C₆ ou benzyle ;
R₁₆ et R₂₁ sont chacun indépendamment un hydrogène, un groupe alkyle en C₁-C₆, alcoxy en C₁-C₄, cycloalkyle en C₃-C₇, alcényle en C₂-C₆, cycloalcényle en C₃-C₇, alcynyle en C₂-C₆, P(O)(OR₄₄)₂, SO₂R₄₅, C(O)R₄₆,
benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄, ou
phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄ ;
R₃₂ est un hydrogène, un groupe
Si(R₄₇)₃,
alkyle en C₁-C₂₀ éventuellement substitué par un groupe CO₂R₄₈, alcoxy en C₁-C₆, thioalkyle en C₁-C₆, alcoxyalcoxy en C₂-C₂₀, phényle, OSi(R₄₇)₃, OC(O)R₃₃, cycloalkyle en C₃-C₇ ou di(alkyle en C₁-C₄)amino,
halogénoalkyle en C₁-C₁₅,
cycloalkyle en C₃-C₈ éventuellement substitué par un groupe CO₂R₄₈,
halogénocycloalkyle en C₃-C₈,
alcényle en C₃-C₂₀ éventuellement substitué par un groupe phényle,
halogénoalcényle en C₃-C₁₅,
cycloalcényle en C₅-C₈,
halogénocycloalcényle en C₅-C₈,
alcynyle en C₃-C₂₀ éventuellement substitué par un groupe phényle,
halogénoalcynyle en C₃-C₂₀,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
alkyle en C₁-C₄ substitué par un groupe pyridyle, furyle, thiényle, tétrahydrofuryle, cycloalkyle en C₃-C₈, dioxane ou NR₄₉R₅₀, ou
un métal alcalin, métal alcalino-terreux, manganèse, cuivre, zinc, cobalt, argent, nickel, cation ammonium ou ammonium organique.

5. Composé selon la revendication 4 dans lequel
R est un groupe alkyle en C₁-C₄ ;
R₁ est un groupe halogénoalkyle en C₁-C₄ ;
R₂ est un hydrogène ;
Y est R₁₂, OR₁₂, C(O)NR₁₃R₁₂, C(O)R₁₂, CO₂R₁₂, C(O)SR₁₂ ou C(=NOR₁₄) ;
R₁₂ est un hydrogène,
un groupe alkyle en C₁-C₆ ou alcényle en C₂-C₆, où chaque groupe est éventuellement substitué par toute combinaison d'un à trois atomes d'halogène, un ou deux groupes alcoxy en C₁-C₄, un groupe halogénoalcoxy en C₁-C₁₀, un groupe NR₁₅R₁₆, un groupe S(O)_{q}R₁₇, un ou deux groupes cyano, un ou deux groupes C(O)R₁₈, un ou deux groupes CO₂R₁₉, un ou deux groupes C(O)NR₂₀R₂₁, un groupe X₄R₂₂, un groupe P(O)(OR₂₃)_{2,} un groupe OC(O)R₃₃,
un noyau imidazole éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un noyau phtalimide éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe ammonium organique quaternaire, ou
un groupe phényle éventuellement substitué par toute combinaison d'un à trois atomes d'halogène, un groupe alkyle en C₁-C₄, un groupe alcoxy en C₁-C₄ ou un groupe X₅R₂₇, ou
un groupe phényle éventuellement substitué par toute combinaison d'un ou deux atomes d'halogène, un à trois groupes alkyle en C₁-C₄, un groupe alcoxy en C₁-C₄, un groupe C(O)R₂₅, un groupe CO₂R₂₆, un groupe X₄R₂₂, un groupe CH=CHR₂₂, un groupe CH₂CH(R₂₈)R₂₂ ou un groupe N(R₂₉)SO₂R₃₀;
R₁₃ est un hydrogène, un groupe alcoxyalkyle en C₁-C₆, alkyle en C₁-C₄, OR₂₃, cyano ou SO₂R₅₁ ;
R₂₂ et R₂₇ sont chacun indépendamment un hydrogène, C(O)R₃₃, SO₂R₄₇,
un groupe alkyle en C₁-C₄ substitué par un groupe alcoxy en C₁-C₆, CO₂R₃₂,
C(O)R₃₃, C(O)NR₃₅R₃₆, C(O)ON=CR₃₇R₃₈, C(O)NHOR₂₃, cyano ou 2-dioxolanyle, ou un noyau phényle, dihydrofuranone ou furanone dans lequel chaque noyau est éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄,
un groupe alcényle en C₃-C₆ éventuellement substitué par un groupe alcoxy en C₁-C₆, CO₂R₃₂, C(O)R₃₃ ou C(OR₃₄)₂,
un groupe alcynyle en C₃-C₆,
un groupe phényle éventuellement substitué par toute combinaison d'un à trois atomes d'halogène, un ou deux groupes alkyle en C₁-C₄, un ou deux groupes alcoxy en C₁-C₄, un groupe halogénoalkyle en C₁-C₄, un groupe halogénoalcoxy en C₁-C₄, un groupe cyano, un groupe nitro, un groupe NR₃₉R₄₀ ou un groupe C(O)R₃₃, ou
un groupe benzyle éventuellement substitué sur le noyau phényle par toute combinaison d'un à trois atomes d'halogène, un ou deux groupes alkyle en C₁-C₄, un ou deux groupes alcoxy en C₁-C₄, un groupe halogénoalkyle en C₁-C₄, un groupe halogénoalcoxy en C₁-C₄, un groupe cyano, un groupe nitro, un groupe NR₃₉R₄₀ ou un groupe C(O)R₃₃ ;
R₁₅ est un hydrogène,
un groupe alkyle en C₁-C₄ éventuellement substitué par un groupe CO₂R₄₂,
un groupe benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄, ou
C(O)R₄₃ ;
R₁₆ est un hydrogène, un groupe alkyle en C₁-C₄, P(O)(OR₄₄)₂, SO₂R₄₅, C(O)R₄₆, ou
benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄ ou un à trois groupes halogénoalcoxy en C₁-C₄ ;
R₁₇, R₁₈, R₂₅, R₃₀, R₃₃, R₄₅ et R₅₁ sont chacun indépendamment un groupe NR₄₆R₄₈, alkyle en C₁-C₄, halogénoalkyle en C₁-C₄,
alcényle en C₂-C₆ éventuellement substitué par un groupe phényle,
benzyle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe nitro, un groupe cyano ou un groupe X₄R₂₂, ou
phényle éventuellement substitué par toute combinaison d'un à trois atomes d'halogène, un à trois groupes alkyle en C₁-C₄, un à trois groupes halogénoalkyle en C₁-C₄, un à trois groupes alcoxy en C₁-C₄, un à trois groupes halogénoalcoxy en C₁-C₄, un groupe nitro, un groupe cyano ou un groupe X₄R₂₂ ;
R₁₉ et R₂₆ sont chacun indépendamment un hydrogène, un groupe alkyle en C₁-C₄, alcényle en C₃-C₆, alcynyle en C₃-C₆, Si(R₄₁)₃, benzyle, phényle, furfuryle, pyridyle, thiényle, oximino ou un métal alcalin, métal alcalino-terreux, manganèse, cuivre, zinc, cobalt, argent, nickel, cation ammonium ou ammonium organique ;
R₃₂ est un hydrogène, un groupe
Si(R₄₇)₃,
alkyle en C₁-C₄ éventuellement substitué par un groupe CO₂R₄₈, OC(O)R₃₃ ou alcoxy en C₁-C₄,
halogénoalkyle en C₁-C₄,
cycloalkyle en C₃-C₈,
halogénocycloalkyle en C₃-C₈,
alcényle en C₃-C₆ éventuellement substitué par un groupe phényle,
halogénoalcényle en C₃-C₆,
cycloalcényle en C₅-C₈,
halogénocycloalcényle en C₅-C₈,
alcynyle en C₃-C₆ éventuellement substitué par un groupe phényle,
halogénoalcynyle en C₃-C₆,
benzyle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄,
alkyle en C₁-C₄ substitué par un groupe pyridyle, furyle, thiényle, tétrahydrofuryle ou NR₄₉R₅₀, ou
un métal alcalin, métal alcalino-terreux, manganèse, cuivre, zinc, cobalt, argent, nickel, cation ammonium ou ammonium organique.
R₃₅ et R₄₉ sont chacun indépendamment un hydrogène ou un groupe alkyle en C₁-C₄ et R₃₆ et R₅₀ sont chacun indépendamment un hydrogène, un groupe cyano, alkyle en C₁-C₄, SO₂R₅₁ ou phényle éventuellement substitué par un ou plusieurs groupes indépendamment choisis parmi un halogène, un groupe cyano, nitro, alkyle en C₁-C₄, halogénoalkyle en C₁-C₄, alcoxy en C₁-C₄ et halogénoalcoxy en C₁-C₄, ou lorsque R₃₅ et R₃₆ ou R₄₉ et R₅₀ sont pris ensemble avec l'atome d'azote auquel ils sont attachés, ils forment un noyau hétérocyclique, monocyclique ou bicyclique condensé, ayant 5 à 12 chaînons éventuellement substitué par un ou plusieurs groupes indépendamment choisis parmi un halogène, un groupe cyano, nitro, amino, hydroxyle, alkyle en C₁-C₄, halogénoalkyle en C₁-C₄, alcoxy en C₁-C₄, halogénoalcoxy en C₁-C₄ ou halogénoalkylsulfonyle en C₁-C₄ ;
R₂₃ est un hydrogène ou un groupe alkyle en C₁-C₄ ; et
A est O.

6. Composé selon la revendication 5 dans lequel
R est un groupe méthyle ;
R₁ est un groupe trifluorométhyle ;
X₁ est un hydrogène ;
X est un hydrogène ou un fluor ;
Y est R₁₂, CO₂R₁₂, C(O)R₁₂ ou C(O)NR₁₃R₁₂ ;
R₁₂ est un hydrogène,
un groupe alkyle en C₁-C₆ éventuellement substitué par toute combinaison d'un à trois atomes d'halogène, un groupe hydroxyle, un ou deux groupes alcoxy en C₁-C₄, un groupe halogénoalcoxy en C₁-C₄, un groupe SO₂R₁₇, un ou deux groupes cyano, un groupe C(O)R₁₈, un ou deux groupes CO₂R₁₉, un ou deux groupes C(O)NR₂₀R₂₁, un groupe P(O)(OR₂₃)₂ ou un groupe OC(O)R₃₃,
un groupe alcényle en C₂-C₆ éventuellement substitué par toute combinaison d'un groupe C(O)R₁₈ ou d'un groupe CO₂R₁₉, 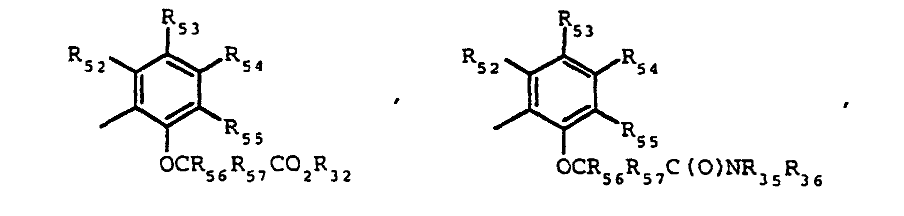 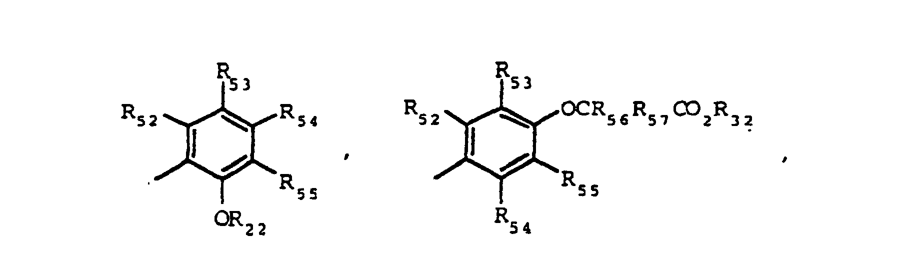 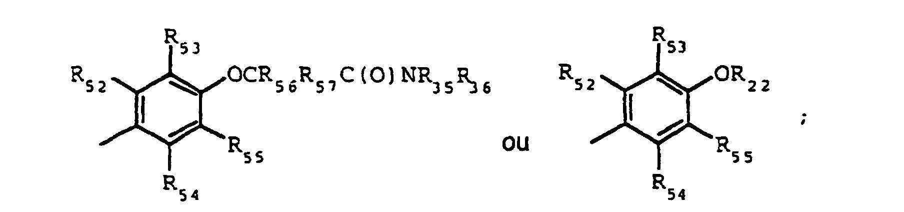
R₁₃ est un hydrogène, un groupe alkyle en C₁-C₄ ou SO₂R₅₁ ;
R₁₄ est un hydrogène, un groupe alkyle en C₁-C₄ ou benzyle ;
R₁₈, R₂₃, R₅₆ et R₅₇ sont chacun indépendamment un hydrogène ou un groupe alkyle en C₁-C₃ ;
R₅₂, R₅₃, R₅₄ et R₅₅ sont chacun indépendamment un hydrogène, un groupe alkyle en C₁-C₃, alcényle en C₂-C₃, alcoxy en C₁-C₃ ou un halogène, à condition qu'au moins un des R₅₂, R₅₃, R₅₄ et R₅₅ soit un hydrogène ;
R₁₉ est un hydrogène, un groupe alkyle en C₁-C₄, alcényle en C₃-C₆, alcynyle en C₃-C₆, benzyle, pyridyle ou un métal alcalin, métal alcalino-terreux, cation ammonium ou ammonium organique ;
R₂₂ est un hydrogène, un groupe C(O)R₃₃, SO₂R₄₇,
alkyle en C₁-C₄ éventuellement substitué par un groupe cyano,
alcényle en C₃-C₆, ou
alcynyle en C₃-C₆;
R₃₂ est un hydrogène, un groupe
alkyle en C₁-C₄ éventuellement substitué par un groupe CO₂R₄₈ ou alcoxy en C₁-C₄,
halogénoalkyle en C₁-C₄,
alcényle en C₃-C₆ éventuellement substitué par un groupe phényle,
alcynyle en C₃-C₆ éventuellement substitué par un groupe phényle,
alkyle en C₁-C₄ substitué par un groupe pyridyle, furyle, thiényle, tétrahydrofuryle ou NR₄₉R₅₀, ou
un métal alcalin, métal alcalino-terreux, cation ammonium ou ammonium organique ;
R₃₅ et R₄₉ sont chacun indépendamment un hydrogène ou un groupe alkyle en C₁-C₄;
R₃₆ est SO₂R₅₁ ou un groupe alcoxy en C₁-C₃,
R₁₇, R₃₃ et R₅₁ sont chacun indépendamment un groupe alkyle en C₁-C₄, halogénoalkyle en C₁-C₄, NR₄₆R₄₈, cycloalkyle en C₃-C₆ ou un noyau isoxazole ;
R₄₆ et R₄₈ sont chacun indépendamment un hydrogène, un groupe alkyle en C₁-C₄ ou benzyle;
R₄₇ est un groupe alkyle en C₁-C₄;
R₅₀ est un groupe alkyle en C₁-C₄ ou
phényle éventuellement substitué par un ou plusieurs groupes indépendamment choisis parmi un halogène, un groupe cyano, alkyle en C₁-C₄, halogénoalkyle en C₁-C₄, alcoxy en C₁-C₄ et halogénoalcoxy en C₁-C₄ ; et
A₁ est O.

7. Composé selon la revendication 6 dans lequel
Y est un groupe alkyle en C₁-C₃, CO₂R₃₂, halogénométhyle, cyanométhyle, C(CH₃)₂CO₂R₃₂, alcoxyéthyle en C₁-C₃, alcoxyméthyle en C₁-C₃, hydroxyméthyle, CHO, C(O)CH₃, CH(CH₃)(alcoxy en C₁-C₄), C(CH₃)₂CN, CH[O(alkyle en C₁-C₃)]₂, CH₂SO₂R₁₇, C(O)NHSO₂R₅₁, 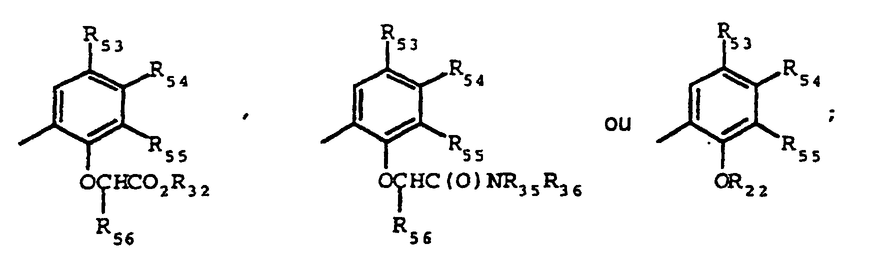
R₅₃, R₅₄, R₅₅ et R₅₆ sont chacun indépendamment un hydrogène ou un groupe méthyle, à condition qu'au moins un des R₅₃, R₅₄ et R₅₅ soit un hydrogène ;
R₂₂ est un groupe cyanométhyle, méthyle, éthyle, allyle ou propargyle ;
R₃₂ est un hydrogène, un groupe
alkyle en C₁-C₄ éventuellement substitué par un groupe CO₂R₄₈,
halogénoalkyle en C₁-C₄,
alcényle en C₃-C₆, ou
alcynyle en C₃-C₆ ; et
R₃₅ est un hydrogène.

8. Composé selon la revendication 1 choisi dans le groupe constitué des composés suivants
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'isopropyle ;
{{2-{5-[3,6-dihydro-3-méthyl-4-(trifluorométhyl)-2,6-dioxo-1(2H)-pyrimidinyl]-1,2-benzisothiazol-3-yl}-p-tolyl}oxy}acétate de méthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2-(diméthylamino)-éthyle ;
2-{{2-{5-[3,6-dihydro-3-méthyl-4-(trifluorométhyl)-2,6-dioxo-1(2H)-pyrimidinyl]-1,2-benzisothiazol-3-yl}-p-tolyl}oxy}propionate de méthyle ;
2-{{6-{5-[3,6-dihydro-3-méthyl-4-(trifluorométhyl)-2,6-dioxo-1(2H)-pyrimidinyl]-1,2-benzisothiazol-3-yl}-3,4-xylyl}oxy}propionate de méthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2-propynyle ;
1-[3-(6-méthoxy-m-tolyl)-1,2-benzisothiazol-5-yl]-1-méthyl-6-(trifluorométhyl)-2,4(1H,3H)-pyrimidinedione ;
3-[3-p-éthylphényl)-1,2-benzisothiazol-5-yl]-1-méthyl-6-(trifluorométhyl)-2,4(1H,3H)-pyrimidinedione ;
1-méthyl-3-(3-méthyl-1,2-benzisothiazol-5-yl)-6-(trifluorométhyl)-2,4(1H,3H)-pyrimidinedione;
2-{{6-{5-[3,6-dihydro-3-méthyl-4-(trifluorométhyl)-2,6-dioxo-1(2H)-pyrimidinyl]-1,2-benzisothiazol-3-yl}-m-tolyl}oxy}propionate de méthyle ;
{{6-{5-[3,6-dihydro-3-méthyl-4-{trifluorométhyl)-2,6-dioxo-1(2H)-pyrimidinyl]-1,2-benzisothiazol-3-yl}-3,4-xylyl}oxy}acétate de méthyle ;
3-[3-(dibromométhyl)-1,2-benzisothiazol-5-yl]-1-méthyl-6-(trifluorométhyl)-2,4(1H,3H)-pyrimidinedione ;
3-(6-fluoro-3-méthyl-1,2-benzisoxazol-5-yl)-1-méthyl-6-(trifluorométhyl)-2,4(1H,3H)-pyrimidinedione ;
{{2-{5-[3,6-dihydro-3-méthyl-2,6-dioxo-4-(trifluorométhyl)-1(2H)-pyrirnidinyl]-1,2-benzisothiazol-3-yl}-p-tolyl}oxy)acétate d'éthyle;
1-méthyl-3-{3-[6-(2-propynyloxy)-m-tolyl]-1,2-benzisothiazol-5-yl}-6-(trifluorométhyl)-2,4(1H,3H)-pyrimidinedione ;
3-{3-{6-[(1-benzimidazolylcarbonyl)méthoxy]-m-tolyl}-1,2-benzisothiazol-5-yl}-1-méthyl-6-trifluorométhyl)-2,4(1H,3H)-pyrimidinedione ;
3-{3-{6-{[(m-cyanophényl)carbamoyl]méthoxy}-m-tolyl}-1,2-benzisothiazol-5-yl}-1-méthyl-6-(trifluorométhyl)-2,4(1H,3H)pyrimidinedione ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'allyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 3-phényl-2-propynyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 1-méthyl-2-propynyle ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}propionate de 2-chloroéthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2,2,2-trifluoroéthyle ;
3-[3-(2-méthoxy-p-tolyl)-1,2-benzisothiazol-5-yl]-1-méthyl-6-(trifluorométhyl)-2,4(1H,3H)-pyrimidinedione ;
2-[3-(6-méthoxy-3,4-xylyl)-1,2-benzisothiazol-5-yl]-1-méthyl-6-(trifluorométhyl)-2,4(1H,3H)-pyrimidinedione ;
2-{{6-{5-[3,6-dihydro-3-méthyl-4-(trifluorométhyl)-2,6-dioxo-1(2H)-pyrimidinyl]-1,2-benzisothiazol-3-yl}-3,4-xylyl}oxy}propionate de méthyle ;
{{6-{5-[3,6-dihydro-3-méthyl-2,6-dioxo-4-(trifluorométhyl)-1(2H)-pyrimidinyl]-1,2-benzisothiazol-3-yl}-2,4-xylyl}oxy}acétate de méthyle ;
3-{3-{6-{[(m-cyanophényl)carbamoyl]méthoxy}-m-tolyl}-1,2-benzisothiazul-5-yl}-1-méthyl-6-(trifluorométhyl)-2,4(1H,3H)-pyrimidinedione ;
{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}acétate de méthyle ;
1-méthyl-3-{3-[(méthylsulfonyl)méthyl]-1,2-benzisothiazol-5-yl}-6-(trifluorométhyl)-2,4(1H,3H)-pyrimidinedione ;
{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}acétate de 2-propynyle ;
3-[3-(o-méthoxyphényl)-1,2-benzisothiazol-5-yl]-1-méthyl-6-(trifluorométhyl)-2,4(1H,3H)-pyrimidinedione ;
N-{{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétyl}méthane sulfonamide ;
2-{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}propionate de 2-propynyle ;
{o-{5-[3,6-dihydro-3-méthyl-2,6-dioxo-4-(trifluorométhyl)-1(2H)-pyrimidinyl]-1,2-benzisothiazol-3-yl}phénoxy}acétate de 2-propynyle ;
5-[3,6-dihydro-3-méthyl-2,6-dioxo-4-(trifluorométhyl)-1(2H)-pyrimidinyl]-1,2-benzisothiazole-3-acétonitrile ;
3-{3-[(allyloxy)méthyl]-1,2-benzisothiazol-5-yl}-1-méthyl-6-(trifluorométhyl)-2,4(1H,3H)-pyrimidinedione ;
5-[3,6-dihydro-3-méthyl-2,6-dioxo-4-(trifluorométhyl]-1(2H)-pyrimidinyl]-N-(méthylsulfonyl)-1,2-benzisothiazole-3-carboxamide ;
{{2-{5-[3,6-dihydro-3-méthyl-2,6-dioxo-4-(trifluorométhyl)-1(2H)-pyrimidinyl]-6-fluoro-1,2-benzisoxazol-3-yl}-p-tolyl}oxy}acétate de méthyle ;
{{6-{5-[3,6-dihydro-3-méthyl-2,6-dioxo-4-(trifluorométhyl)-1(2H)-pyrimidinyl]-6-fluoro-1,2-benzisothiazol-3-yl}-m-tolyl}oxy}acétate de méthyle ;
acide {o-{5-[3,6-dihydro-3-méthyl-2,6-dioxo-4-(trifluorométhyl)-1(2H)-pyrimidinyl]-1,2-benzisothiazol-3-yl}phénoxy}acétique ;
acide 2-{o-{5-[3,6-dihydro-3-méthyl-2,6-dioxo-4-(trifluorométhyl)-1(2H)-pyrimidinyl]-1,2-benzisothiazol-3-yl}phénoxy}propionique ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}-N-méthoxyacétamide ;
2-{2-{5-[3,6-dihydro-3-méthyl-2,6-dioxo-4-(trifluorométhyl)-1(2H)-pyrimidinyl]-1,2-benzisothiazol-3-yl}-4-méthoxyphénoxy}propionate de méthyle ;
2-{o-{5-[3,6-dihydro-3-méthyl-2,6-dioxo-4-(trifluorométhyl)-1(2H)-pyrimidinyl]-1,2-benzisothiazol-3-yl}phénoxy}-N-(méthylsulfonyl)acétamide ;
2-{o-{5-[3,6-dihydro-3-méthyl-2,6-dioxo-4-(trifluorométhyl)-1(2H)-pyrimidinyl]-1,2-benzisothiazol-3-yl}phénoxy}-N-(méthylsulfonyl)-propionamide ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}-N-méthyl-N-(méthylsulfonyl)-acétamide ;
2-{o-{5-[3,6-dihydro-3-méthyl-2,6-dioxo-4-(trifluorométhyl)-1(2H)-pyrimidinyl]-1,2-benzisothiazol-3-yl}phénoxy}-N-méthyl-N-(méthylsulfonyl)acétamide ;
2-{o-{5-[3,6-dihydro-3-méthyl-2,6-dioxo-4-(trifluorométhyl)-1(2H)-pyrimidinyl]-1,2-benzisothiazol-3-yl}phénoxy}-N-(éthylsulfonyl)acétamide ;
3-[3-(hydroxyméthyl)-1,2-benzisothiazol-5-yl]-1-méthyl-6-(trifluorométhyl)-2,4(1H,3H)-pyrimidinedione, 2-thiophénecarboxylate (ester) ;
3-[3-(hydroxyméthyl)-1,2-benzisothiazol-5-yl]-1-méthyl-6-(trifluorométhyl)-2,4(1H,3H)-pyrimidinedione, benzoate (ester) ;
3-[3-(hydroxyméthyl)-1,2-benzisothiazol-5-yl]-1-méthyl-6-(trifluorométhyl)-2,4(1H,3H)-pyrimidinedione, méthoxyacétate (ester) ;
5-[3,6-dihydro-3-méthyl-2,6-dioxo-4-(trifluorométhyl)-1(2H)-pyrimidinyl]-N-(éthylsulfonyl)-1,2-benzisothiazole-3-carboxamide ;
5-[3,6-dihydro-3-méthyl-2,6-dioxo-4-(trifluorométhyl)-1(2H)-pyrimidinyl]-α,α-diméthyl-1,2-benzisothiazole-3-acétonitrile ;
5-[3,6-dihydro-3-méthyl-2,6-dioxo-4-(trifluorométhyl)-1(2H)-pyrimidinyl]-6-fluoro-1,2-benzisothiazole-3-acétonitrile ;
5-[3,6-dihydro-3-méthyl-2,6-dioxo-4-(trifluorométhyl)-1(2H)-pyrimidinyl]-α-éthyl-1,2-benzisothiazole-3-acétonitrile;
5-[3,6-dihydro-3-méthyl-2,6-dioxo-4-(trifluorométhyl)-1(2H)-pyrimidinyl]-α,α-diéthyl-1,2-benzisothiazole-3-acétonitrile ;
3-[6-fluoro-3-(méthoxyméthyl)-1,2-benzisothiazol-5-yl]-1-méthyl-6-(trifluorométhyl)-2,4(1H,3H)-pyrimidinedione ;
3-[3-(hydroxyméthyl)-1,2-benzisothiazol-5-yl]-1-méthyl-6-(trifluorométhyl)-2,4(1H,3H)-pyrimidinedione, 5-isoxazolecarboxylate (ester) ;
3-[3-(hydroxyméthyl)-1,2-benzisothiazol-5-yl]-1-méthyl-6-(trifluorométhyl)-2,4(1H,3H)-pyrimidinedione, cyclopropanecarboxylate (ester) ;
3-(3-acétyl-1,2-benzisothiazol-5-yl)-1-méthyl-6-(trifluorométhyl)-2,4(1H,3H)-pyrimidinedione ;
3-[3-(1-méthoxyéthyl)-1,2-benzisothiazol-5-yl]-1-méthyl-6-(trifluorométhyl)-2,4(1H,3H)-pyrimidinedione ;
N-(benzylsulfonyl)-5-[3,6-dihydro-3-méthyl-2,6-dioxo-4-(trifluorométhyl)-1(2H)-pyrimidinyl]-1,2-benzisothiazole-3-carboxamide ;
5-[3,6-dihydro-3-méthyl-2,6-dioxo-1-(trifluorométhyl)-1(2H)-pyrimidinyl]-1,2-benzisothiazole-3-carboxylate de méthyle ;
5-[3,6-dihydro-3-méthyl-2,6-dioxo-4-(trifluorométhyl)-1(2H)-pyrimidinyl]-α,α-diméthyl-1,2-benzisothiazole-3-acétate de benzyle ;
N-(benzylsulfonyl)-5-[3,6-dihydro-3-méthyl-2,6-dioxo-4-(trifluorométhyl)-1(2H)-pyrimidinyl]-6-fluoro-1,2-benzisothiazole-3-carboxamide ;
5-[3,6-dihydro-3-méthyl-2,6-dioxo-4-(trifluorométhyl)-1(2H)-pyrimidinyl]-N-(méthylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(isopropylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(éthylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-[(p-méthylbenzyl)sulfonyl]-1,2-benzisothiazole-3-carboxamide ;
N-(benzylsulfonyl)-5-[3,6-dihydro-3-méthyl-2,6-dioxo-4-(trifluorométhyl)-1(2H)-pyrimidinyl]-6-fluoro-1,2-benzisothiazole-3-carboxamide ;
3-[3-(1-hydroxyéthyl)-1,2-benzisothiazol-5-yl]-1-méthyl-6-(trifluorométhyl)-2,4(1H,3H)-pyrimidinedione ;
5-[3,6-dihydro-3-méthyl-2,6-dioxo-4-(trifluorométhyl)-1(2H)-pyrimidinyl]-1,2-benzisothiazole-3-carboxaldéhyde, 3-(diméthylacétal) ;
3-(6-fluoro-3-méthyl-1,2-benzisothiazol-5-yl)-1-méthyl-6-(trifluorométhyl)-2,4(1H,3H)-pyrimidinedione ;
5-[3,6-dihydro-3-méthyl-2,6-dioxo-4-(trifluorométhyl)-1(2H)-pyrimidinyl]-6-fluoro-1,2-benzisothiazole-3-carboxaldéhyde, 3-(diméthyl-acétal) ;
acide5-[3,6-dihydro-3-méthyl-2,6-dioxo-4-(trifluorométhyl)-1(2H)-pyrirnidinyl]-6-fluoro-1,2-benzisothiazole-3-carboxylique ;
5-[3,6-dihydro-3-méthyl-2,6-dioxo-4-(trifluorométhyl)-1(2H)-pyrimidinyl]-α-méthyl-1,2-benzisothiazole-3-acétonitrile ;
N-(benzylsulfonyl)-5-[3,6-dihydro-3-méthyl-2,6-dioxo-4-(trifluorométhyl)-1(2H)-pyrimidinyl}-N-(éthoxyméthyl)-1,2-benzisothiazole-3-carboxamide ;
5-[3,6-dihydro-3-méthyl-2,6-dioxo-4-(trifluorométhyl)-1(2H)-pyrimidinyl]-N-(diméthylsulfamoyl)-1,2-benzisothiazole-3-carboxamide ;
5-[3,6-dihydro-3-méthyl-2,6-dioxo-4-(trifluorométhyl)-1(2H)-pyrimidinyl]-N-(isopropylsulfamoyl)-1,2-benzisothiazole-3-carboxamide ;
5-[3,6-dihydro-3-méthyl-2,6-dioxo-4-(trifluorométhyl)-1(2H)-pyrimidinyl]-α,α-diméthyl-1,2-benzisothiazole-3-acétate de méthyle ;
3-[3-(hydroxyméthyl)-1,2-benzisothiazol-5-yl]-1-méthyl-6-(trifluorométhyl)-2,4(1H,3H)-pyrimidinedione, 2-furoate (ester) ;
N-[(m-chlorobenzyl)sulfonyl]-5-[3,6-dihydro-3-méthyl-2,6-dioxo-4-(trifluorométhyl)-1(2H)-pyrimidinyl]-1,2-benzisothiazole-3-carboxamide ;
5-[3,6-dihydro-3-méthyl-2,6-dioxo-4-(trifluorométhyl)-1(2H)-pyrimidinyl]-N-(diisopropylsulfamoyl)-1,2-benzisothiazole-3-carboxamide; et
3-[3-(hydroxyméthyl)-1,2-benzisothiazol-5-yl]-1-méthyl-6-(trifluorométhyl)-2,4(1H,3H)-pyrimidinedione, méthylcarbamate (ester).

9. Procédé de lutte contre des espèces végétales indésirables qui consiste à appliquer sur le feuillage desdits végétaux ou sur le sol ou l'eau contenant des semences ou d'autres organes de propagation de ceux-ci, une quantité efficace comme herbicide d'un composé selon l'une quelconque des revendications 1 à 8.

10. Procédé selon la revendication 9 dans lequel le composé est appliqué sur le feuillage des espèces végétales indésirables ou sur le sol ou l'eau contenant des semences ou d'autres organes de propagation de celles-ci en présence de plantes cultivées, de semences de plantes cultivées ou d'autres organes de propagation de plantes cultivées.

11. Procédé selon la revendication 10 dans lequel la plante cultivée est une culture céréalière ou le soja.

12. Procédé selon la revendication 11 dans lequel la culture céréalière est choisie dans le groupe constitué du maïs, du blé et du riz.

13. Procédé selon la revendication 12 dans lequel la culture céréalière est le maïs et le composé est choisi dans le groupe constitué des composés suivants
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'isopropyle ;
{{2-{5-[3,6-dihydro-3-méthyl-4-(trifluorométhyl)-2,6-dioxo-1(2H)-pyrimidinyl]-1,2-benzisothiazol-3-yl}-p-tolyl}oxy}acétate de méthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2-(diméthylamino)-éthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2-propynyle ;
3-[3-(p-éthylphényl)-1,2-benzisothiazol-5-yl]-1-méthyl-6-(trlfluorométhyl)-2,4(1H,3H)-pyrimidinedione ;
{{6-{5-[3,6-dihydro-3-méthyl-4-(trifluorométhyl)-2,6-dioxo-1(2H)-pyrimidinyl]-1,2-benzisothiazol-3-yl}-3,4-xylyl}oxy}acétate de méthyle ;
3-[3-(dibromométhyl)-1,2-benzisothiazol-5-yl]-1-méthyl-6-(trifluorométhyl)-2,4(1H,3H)-pyrimidinedione ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 1-méthyl-2-propynyle ;
{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}acétate de méthyle ;
1-méthyl-3-{3-[(méthylsulfonyl)méthyl]-1,2-benzisothiazol-5-yl}-6-(trifluorométhyl)-2,4(1H,3H)-pyrimidinedione ;
{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}acétate de 2-propynyle ;
3-[3-(o-méthoxyphényl)-1,2-benzisothiazol-5-yl]-1-méthyl-6-(trifluorométhyl)-2,4(1H,3H)-pyrimidinedione ;
N-{{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétyl}méthane sulfonamide ;
{o-{5-[3,6-dihydro-3-méthyl-2,6-dioxo-4-(trifluorométhyl)-1(2H)-pyrimidinyl]-1,2-benzisothiazol-3-yl}phénoxy}acétate de 2-propynyle ;
5-[3,6-dihydro-3-méthyl-2,6-dioxo-4-(trifluorométhyl)-1(2H)-pyrimidinyl]-1,2-benzisothiazole-3-acétonitrile ;
3-{3-[(allyloxy)méthyl]-1,2-benzisothiazol-5-yl}-1-méthyl-6-(trifluorométhyl)-2,4(1H,3H)-pyrimidinedione ;
5-[3,6-dihydro-3-méthyl-2,6-dioxo-4-(trifluorométhyl)-1(2H)-pyrimidinyl]-N-(méthylsulfonyl)-1,2-benzisothiazole-3-carboxamide ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}-N-méthyl-N-(méthylsulfonyl)acétamide ;
3-[3-(hydroxyméthyl)-1,2-benzisothiazol-5-yl]-1-méthyl-6-(trifluorométhyl)-2,4(1H,3H)-pyrimidinedione, méthylcarbamate (ester) ;
N-(benzylsulfonyl)-5-[3,6-dihydro-3-méthyl-2,6-dioxo-4-(trifluorométhyl)-1(2H)-pyrimidinyl]-1,2-benzisothiazole-3-carboxamide ;
5-[3,6-dihydro-3-méthyl-2,6-dioxo-4-(trifluorométhyl)-1(2H)-pyrimidinyl]-N-(isopropylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(éthylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(diisopropylsulfamoyl)-1,2-benzisothiazole-3-carboxamide;
5-[3,6-dihydro-3-méthyl-2,6-dioxo-4-(trifluorométhyl)-1(2H)-pyrimidinyl]-N-(isopropylsulfamoyl)-1,2-benzisothiazole-3-carboxamide ;
5-[3,6-dihydro-3-méthyl-2,6-dioxo-4-(trifluorométhyl)-1(2H)-pyrimidinyl]-N-(diméthylsulfamoyl)-1,2-benzisothiazole-3-carboxamide ;
acide 5-[3,6-dihydro-3-méthyl-2,6-dioxo-4-(trifluorométhyl)-1(2H)-pyrimidinyl]-6-fluoro-1,2-benzisothiazole-3-carboxylique ; et
N-(benzylsulfonyl)-5-[3,6-dihydro-3-méthyl-2,6-dioxo-4-(trifluorométhyl)-1(2H)-pyrimidinyl]-6-fluoro-1,2-benzisothiazole-3-carboxamide.

14. Procédé selon la revendication 12 dans lequel la culture céréalière est le blé et le composé est choisi dans le groupe constitué des composés suivants
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'isopropyle ;
{{2-{5-[3,6-dihydro-3-méthyl-4-(trifluorométhyl)-2,6-dioxo-1(2H)-pyrimidinyl]-1,2-benzisothiazol-3-yl}-p-tolyl}oxy}acétate de méthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2-(diméthylamino)-éthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2-propynyle ;
2-{{6-{5-[3,6-dihydro-3-méthyl-4-(trifluorométhyl)-2,6-dioxo-1(2H)-pyrimidinyl]-1,2-benzisothiazol-3-yl}-m-tolyl}oxy}propionate de méthyle ;
3-(6-fluoro-3-méthyl-1,2-benzisoxazol-5-yl)-1-méthyl-6-(trifluorométhyl)-2,4(1H,3H)-pyrimidinedione ;
3-{3-{6-[(1-benzimidazolylcarbonyl)méthoxy]-m-tolyl}-1,2-benzisothiazol-5-yl}-1-méthyl-6-trifluorométhyl)-2,4(1H,3H)-pyrimidinedione ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'allyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 3-phényl-2-propynyle ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}propionate de 2-chloroéthyle ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate de 2,2,2-trifluoroéthyle ;
{{6-{5-[3,6-dihydro-3-méthyl-2,6-dioxo-4-(trifluorométhyl)-1(2H)-pyrimidinyl]-1,2-benzisothiazol-3-yl]-2,4-xylyl}oxy}acétate de méthyle ;
acide {o-{5-[3,6-dihydro-3-méthyl-2,6-dioxo-4-(trifluorométhyl)-1(2H)-pyrimidinyl]-1,2-benzisothiazol-3-yl}phénoxy}ique ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}-N-méthoxyamide ;
2-{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}-N-méthyl-N-(méthylsulfonyl)-acétamide ;
{{6-{5-[3,6-dihydro-3-méthyl-2,6-dioxo-4-(trifluorométhyl)-1(2H)-pyrimidinyl]-6-fluoro-1,2-benzisothiazol-3-yl}-m-tolyl}oxy}acétate de méthyle ;
3-(3-yl-1,2-benzisothiazol-5-yl)-1-méthyl-6-(trifluorométhyl)-2,4(1H,3H)-pyrimidinedione ;
5-[3,6-dihydro-3-méthyl-2,6-dioxo-4-(trifluorométhyl)-1(2H)-pyrimidinyl]-1,2-benzisothiazole-3-carboxylate de méthyle ;
5-[3,6-dihydro-3-méthyl-2,6-dioxo-4-(trifluorométhyl)-1(2H)-pyrimidinyl]-N-(isopropylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(éthylsulfonyl)-1,2-benzisothiazole-3-carboxamide ;
N-[(m-chlorobenzyl)sulfonyl]-5-[3,6-dihydro-3-méthyl-2,6-dioxo-4-(trifluorométhyl)-1(2H)-pyrimidinyl]-1,2-benzisothiazole-3-carboxamide ;
5-[3,6-dihydro-3-méthyl-2,6-dioxo-4-(trifluorométhyl)-1(2H)-pyrimidinyl]-N-(diisopropylsulfamoyl)-1,2-benzisothiazole-3-carboxamide ;
5-[3,6-dihydro-3-méthyl-2,6-dioxo-4-(trifluorométhyl)-1(2H)-pyrimidinyl]-N-((isopropylsulfamoyl)-1,2-benzisothiazole-3-carboxamide; et
5-[3,6-dihydro-3-méthyl-2,6-dioxo-4-(trifluorométhyl)-1(2H)-pyrimidinyl]-N-(diméthylsulfamoyl)-1,2-benzisothiazole-3-carboxamide.

15. Procédé selon la revendication 12 dans lequel la culture céréalière est le riz et le composé est choisi dans le groupe constitué des composés suivants
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'isopropyle ;
{{2-{5-[3,6-dihydro-3-méthyl-4-(trifluorométhyl)-2,6-dioxo-1(2H)-pyrimidinyl]-1,2-benzisothiazol-3-yl}-p-tolyl}oxy}acétate de méthyle ;
3-{3-(6-méthoxy-m-tolyl)-1,2-benzisothiazol-5-yl]-1-méthyl-6-(trifluorométhyl)-2,4(1H,3H)-pyrimidinedione ;
{{2-{5-[3,6-dihydro-3-méthyl-2,6-dioxo-4-(trifluorométhyl)-1(2H)-pyrimidinyl]-1,2-benzisothiazol-3-yl}-p-tolyl}oxy}acétate d'éthyle;
1-méthyl-3-{3-[6-(2-propynyloxy)-m-tolyl]-1,2-benzisothiazol-5-yl}-6-(trifluorométhyl)-2,4(1H,3H)-pyrimidinedione ;
3-{3-{6-{[(m-cyanophényl)carbamoyl]méthoxy}-m-tolyl}-1,2-benzisothiazol-5-yl}-1-méthyl-6-(trifluorométhyl)-2,4(1H,3H)pyrimidinedione ;
3-[3-(2-méthoxy-p-tolyl)-1,2-benzisothiazol-5-yl]-1-méthyl-6-(trifluorométhyl)-2,4(1H,3H)-pyrimidinedione ;
2-[3-(6-méthoxy-3,4-xylyl)-1,2-benzisothiazol-5-yl]-1-méthyl-6-(trifluorométhyl)-2,4(1H,3H)-pyrimidinedione ;
3-{3-{6-{[(m-cyanophényl)carbamoyl]méthoxy}-m-tolyl}-1,2-benzisothiazol-5-yl}-1-méthyl-6-(trifluorométhyl)-2,4(1H,3H)-pyrimidinedione ;
{o-{5-[3,6-dihydro-3-méthyl-2,6-dioxo-4-(trilluorométhyl)-1(2H)-pyrimidinyl]-1,2-benzisothiazol-3-yl}phénoxy}acétate de 2-propynyle ;
3-{3-[(allyloxy)méthyl]-1,2-benzisothiazol-5-yl}-1-méthyl-6-(trifluorométhyl)-2,4(1H,3H)-pyrimidinedione ;
5-[3,6-dihydro-3-méthyl-2,6-dioxo-4-(trifluorométhyl)-1(2H)-pyrimidinyl]-α-éthyl-1,2-benzisothiazole-3-acétonitrile ;
5-[3,6-dihydro-3-méthyl-2,6-dioxo-4-(trifluorométhyl)-1(2H)-pyrimidinyl]-α,α-diéthyl-1,2-benzisothiazole-3-acétonitrile ;
3-[3-(hydroxyméthyl)-1,2-benzisothiazol-5-yl]-1-méthyl-6-(trifluorométhyl)-2,4(1H,3H)-pyrimidinedione, 5-isoxazolecarboxylate (ester) ;
3-[3-(hydroxyméthyl)-1,2-benzisothiazol-5-yl]-1-méthyl-6-(trifluorométhyl)-2,4(1H,3H)-pyrimidinedione, cyclopropanecarboxylate (ester) ;
5-[3,6-dihydro-3-méthyl-2,6-dioxo-4-(trifluorométhyl)-1(2H)-pyrimidinyl]-1,2-benzisothiazole-3-carboxylate de méthyle ;
5-[3,6-dihydro-3-méthyl-2,6-dioxo-4-(trifluorométhyl)-1(2H)-pyrimidinyl]-N-(isopropylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(éthylsulfonyl)-1,2-benzisothiazole-3-carboxamide ;
5-[3,6-dihydro-3-méthyl-2,6-dioxo-4-(trifluorométhyl)-1(2H)-pyrimidinyl]-α,α-diméthyl-1,2-benzisothiazole-3-acétate de benzyle ;
5-[3,6-dihydro-3-méthyl-2,6-dioxo-4-(trifluorométhyl)-1(2H)-pyrimidinyl]-N-(diisopropylsulfamoyl)-1,2-benzisothiazole-3-carboxamide;
5-[3,6-dihydro-3-méthyl-2,6-dioxo-4-(trifluorométhyl)-1(2H)-pyrimidinyl]-N-(isopropylsulfamoyl)-1,2-benzisothiazole-3-carboxamide ; et
5-[3,6-dihydro-3-méthyl-2,6-dioxo-4-(trifluorométhyl)-1(2H)-pyrimidinyl]-N-(diméthylsulfamoyl)-1,2-benzisothiazole-3-carboxamide.

16. Procédé selon la revendication 11 dans lequel la plante cultivée est le soja et le composé est choisi dans le groupe constitué des composés suivants
2-{{2-{5-{3,6-dihydro-3-méthyl-4-(trifluorométhyl)-2,6-dioxo-1(2H)-pyrimidinyl]-1,2-benzisothiazol-3-yl}-p-tolyl}oxy}propionate de méthyle ;
2-{{6-{5-[3,6-dihydro-3-méthyl-4-(trifluorométhyl)-2,6-dioxo-1(2H)-pyrimidinyl]-1,2-benzisothiazol-3-yl}-3,4-xylyl}oxy}propionate de méthyle ;
1-méthyl-3-(3-méthyl-1,2-benzisothiazol-5-yl)-6-(trifluorométhyl)-2,4(1H,3H)-pyrimidinedione ;
2-{{6-{5-[3,6-dihydro-3-méthyl-4-(trifluorométhyl)-2,6-dioxo-1(2H)-pyrimidinyl]-1,2-benzisothiazol-3-yl}-3,4-xylyl}oxy}propionate de méthyle ;
{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}acétate de 2-propynyle ;
3-[3-(o-méthoxyphényl)-1,2-benzisothiazol-5-yl]-1-méthyl-6-(trifluorométhyl)-2,4(1H,3H)-pyrimidinedione ;
2-{{2-{5-[3,6-dihydro-3-méthyl-2,6-dioxo-4-(trifluorométhyl)-1(2H)-pyrimidinyl]-1,2-benzisoxazol-3-yl}-p-tolyl}oxy}propionate de 2-propynyle ;
{o-{5-[3,6-dihydro-3-méthyl-2,6-dioxo-4-(trifluorométhyl)-1(2H)-pyrimidinyl]-1,2-benzisothiazol-3-yl}phénoxy}acétate de 2-propynyle ;
3-{3-[(allyloxy)méthyl]-1,2-benzisothiazol-5-yl}-1-méthyl-6-(trifluorométhyl)-2,4(1H,3H)-pyrimidinedione ;
5-[3,6-dihydro-3-méthyl-2,6-dioxo-4-(trifluorométhyl)-1(2H)-pyrimidinyl]-N-(méthylsulfonyl)-1,2-benzisothiazole-3-carboxamide ;
{{2-{5-[3,6-dihydro-3-méthyl-2,6-dioxo-4-(trifluorométhyl)-1(2H)-pyrimidinyl]-6-fluoro-1,2-benzisoxazol-3-yl}-p-tolyl}oxy}acétate de méthyle ;
{{6-{5-[3,6-dihydro-3-méthyl-2,6-dioxo-4-(trifluorométhyl)-1(2H)-pyrimidinyl]-6-fluoro-1,2-benzisothiazol-3-yl}-m-tolyl}oxy}acétate de méthyle ;
3-[3-(hydroxyméthyl)-1,2-benzisothiazol-5-yl]-1-méthyl-6-(trifluorométhyl)-2,4(1H,3H)-pyrimidinedione, benzoate (ester) ;
3-[3-(hydroxyméthyl)-1,2-benzisothiazol-5-yl]-1-méthyl-6-(trifluorométhyl)-2,4(1H,3H)-pyrimidinedione, 2-thiophénecarboxylate (ester) ;
3-[3-(hydroxyméthyl)-1,2-benzisothiazol-5-yl]-1-méthyl-6-(trifluorométhyl)-2,4(1H,3H)-pyrimidinedione, méthoxyacétate (ester) ;
3-[3-(hydroxyméthyl)-1,2-benzisothiazol-5-yl]-1-méthyl-6-(trifluorométhyl)-2,4(1H,3H)-pyrimidinedione, cyclopropanecarboxylate (ester) ;
3-[3-(hydroxyméthyl)-1,2-benzisothiazol-5-yl]-1-méthyl-6-(trifluorométhyl)-2,4(1H,3H)-pyrimidinedione, 2-furoate (ester) ;
5-[3,6-dihydro-3-méthyl-2,6-dioxo-4-(trifluorométhyl)-1(2H)-pyrimidinyl]-N-(méthylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(isopropylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(éthylsulfonyl)-1,2-benzisothiazole-3-carboxamide ;
5-[3,6-dihydro-3-méthyl-2,6-dioxo-4-(trifluorométhyl)-1(2H)-pyrimidinyl]-N-(isopropylsulfamoyl)-1,2-benzisothiazole-3-carboxamide; et
5-[3,6-dihydro-3-méthyl-2,6-dioxo-4-(trifluorométhyl)-1(2H)-pyrimidinyl]-N-(diméthylsulfamoyl)-1,2-benzisothiazole-3-carboxamide.

17. Procédé selon l'une quelconque des revendications 9 à 16 dans lequel le composé est appliqué sur le feuillage des espèces végétales indésirables ou sur le sol ou l'eau contenant des semences ou d'autres organes de propagation de celles-ci à raison d'environ 0,001 kg/ha à 1 kg/ha.

18. Composition herbicide qui comprend un support solide ou liquide inerte et une quantité efficace comme herbicide d'un composé selon l'une quelconque des revendications 1 à 8.

19. Composé intermédiaire ayant pour formule développée 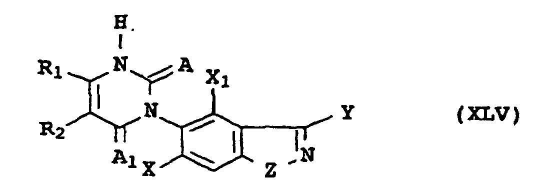 dans laquelle R₁, R₂, X, X₁, Y, A, A₁ et Z sont tels que définis dans l'une quelconque des revendications 1, 2 et 4 et les isomères optiques, diastéréoisomères et/ou tautomères de celui-ci.

20. Procédé de préparation d'un composé de formule I ou II selon la revendication 1 qui comprend l'une des étapes suivantes :
a) réaction d'un composé de formule VIII 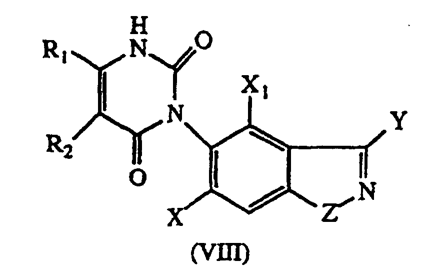 dans laquelle R₁, R₂, X, X₁, Y et Z sont tels que définis dans la revendication 1, avec un composé de formule
**Z**_{**1**}**R** (IX)
dans laquelle R est tel que défini dans la revendication 1 et Z₁ est un chlore, un brome ou un iode pour donner un composé correspondant de formule I ;
b) réaction d'un composé de formule XI 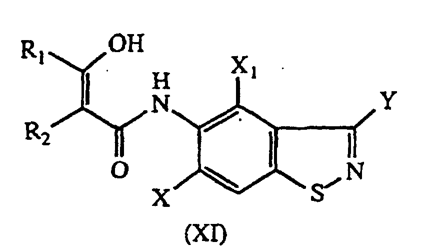 dans laquelle R₁, R₂, X, X₁ et Y sont tels que définis dans la revendication 1, avec
(i) un composé de formule
C(A)Cl₂
dans laquelle A est un oxygène ou un soufre suivi par
(ii) une amine de formule XII
RNH₂ (XII)
dans laquelle R est tel que défini dans la revendication 1, en présence d'une base, par exemple la triéthylamine ou la pyridine, pour donner un composé correspondant de formule I ; dans laquelle A est O ou S ;
c) réaction d'un composé de formule (I) dans laquelle R, R₁, R₂, A, A₁, X, X₁ et Z sont définis dans la revendication 1 et Y est CH₃, avec un agent d'halogénation, par exemple le N-bromosuccinimide, le N-chlorosuccinimide, le N-iodosuccinimide, le brome, le chlore, le chlorure de sulfuryle et le bromure de sulfuryle, pour donner un composé correspondant de formule I dans laquelle Y est un groupe halogénométhyte ;
d) réaction d'un composé de formule I, dans laquelle R, R₁, R₂, A, A₁, X, X₁ et Z sont définis dans la revendication 1 et Y est un groupe bromométhyle, avec un alcool en C₁-C₂₀, pour donner un composé correspondant de formule I dans laquelle Y est un groupe CH₂O(alkyle en C₁-C₂₀) ;
e) réaction d'un composé de formule I dans laquelle R, R₁, R₂, A, A₁, X, X₁ et Z sont définis dans la revendication 1 et Y est un groupe bromométhyle, avec un alcool ou un thiol de formule
**HX**_{**4**}**R**_{**22**} (XXI)
dans laquelle X₄ et R₂₂ sont tels que définis dans la revendication 1 en présence d'une base, par exemple l'hydrure de sodium, pour donner un composé correspondant de formule I dans laquelle Y est CH₂X₄R₂₂ ;
f) réaction d'un composé de formule I dans laquelle R, R₁, R₂, A, A₁, X, X₁ et Z sont définis dans la revendication 1 et Y est un groupe bromométhyle, avec un thioalcoxyde de métal alcalin pour donner un composé correspondant de formule I dans laquelle Y est un groupe CH₂S(alkyle en C₁-C₈);
g) réaction d'un composé de formule I dans laquelle R, R₁, R₂, Y, X, X₁ et Z sont définis dans la revendication 1 et A et A₁ sont O, avec le réactif de Lawesson en présence d'une base, par exemple NaHCO₃, pour donner un composé correspondant de formule I dans laquelle A est O et A₁ est S ;
h) réaction d'un composé de formule I dans laquelle R, R₁, R₂, Y, X, X₁ et Z sont définis dans la revendication 1 et A est S et A₁ est O, avec le réactif de Lawesson en présence d'une base pour donner un composé correspondant de formule I dans laquelle A et A₁ sont S ;
i) hydrolyse d'un composé de formule I dans laquelle au moins un des groupes COOR₁₉, COOR₂₆ ou COOR₃₂ est un ester où R₁₉, R₂₆ et R₃₂ sont définis dans la revendication 1 pour donner un composé de formule I dans laquelle au moins un des R₁₉, R₂₆ ou R₃₂ est un hydrogène ;
j) halogénation d'un composé de formule I dans laquelle au moins un des groupes COOR₁₉, COOR₂₆ et COOR₃₂ est un groupe acide carboxylique, par exemple au moyen de chlorure de thionyle ou de chlorure d'oxalyle, pour donner un chlorure d'acide correspondant ; et réaction dudit chlorure d'acide avec :
(i) un alcool de formule R₁₉OH, R₂₆OH, ou R₃₂OH
ou
(ii) une amine de formule HNR₂₀R₂₁, HNR₃₅R₃₆ ou HNR₄₉R₅₀,
pour donner un composé correspondant de formule I contenant un groupe COOR₁₉, COOR₂₆, COOR₃₂, CONR₂₀R₂₁, CONR₃₅R₃₆ ou CONR₄₉R₅₀ où R₁₉, R₂₀, R₂₁, R₂₆, R₃₂, R₃₅, R₃₆, R₄₉ et R₅₀ sont définis dans la revendication 1 ;
k) réaction d'un composé de formule I dans laquelle au moins un des R₁₉, R₂₆ et R₃₂ est un hydrogène pour donner un composé correspondant de formule I dans laquelle au moins un des R₁₉, R₂₆ et R₃₂ est un métal alcalin, métal alcalino-terreux, manganèse, cuivre, zinc, cobalt, argent, nickel, cation ammonium ou ammonium organique ;
I) oxydation d'un composé de formule I dans laquelle R, R₁, R₂, A, A₁, X, X₁ et Z sont définis dans la revendication 1 et Y est un groupe alkyle, cycloalkyle, alcényle, cycloalcényle, alcynyle ou phényle substitué par un groupe CH₂OH, avec un agent oxydant, par exemple l'oxyde de chrome(VI), pour donner un composé correspondant de formule I dans laquelle Y est un groupe alkyle, cycloalkyle, alcényle, cycloalcényle, alcynyle ou phényle substitué par un groupe CHO ;
m) réaction d'un composé de formule I dans laquelle R, R₁, R₂, A, A₁, X, X₁ et Z sont tels que définis dans la revendication 1 et Y est un groupe méthyle, avec du nitrate d'ammonium cérique pour donner un composé correspondant de formule I dans laquelle Y est CHO ;
n) réaction d'un composé de formule I dans laquelle R, R₁, R₂, A, A₁, X, X₁ et Z sont tels que définis dans la revendication 1 et Y est CH₂Br, avec du cyanure de potassium pour donner un composé correspondant de formule I dans laquelle Y est CH₂CN ;
o) réaction d'un composé de formule I dans laquelle R, R₁, R₂, A, A₁, X, X₁ et Z sont tels que définis dans la revendication 1 et R₁₂ est un groupe bromoalkyle, avec du chlorure de lithium, de l'iodure de sodium, du fluorure de potassium ou du fluorure d'argent(I) pour donner un composé correspondant de formule I dans laquelle R₁₂ est un groupe chloroalkyle, fluoroalkyle ou iodoalkyle ;
p) réaction d'un composé de formule : 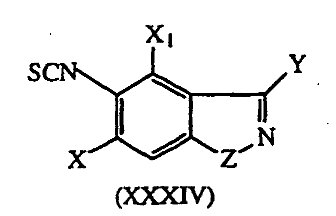 dans laquelle X, X₁, Y et Z sont définis dans la revendication 1 avec un alcène de formule : 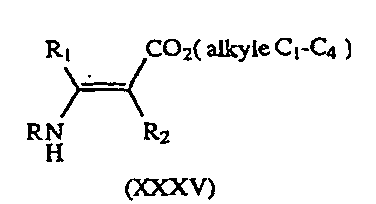 dans laquelle R, R₁, et R₂ sont tels que définis dans la revendication 1 en présence d'une base, par exemple l'hydrure de sodium, pour donner un composé correspondant de formule I dans laquelle R, R₁, R₂, X, X₁, Y et Z sont définis dans la revendication 1 et A est S et A₁ est O ;
q) réaction d'un composé de formule I dans laquelle R, R₁, R₂, A, A₁, X, X₁, et Z sont tels que définis dans la revendication 1 et Y est COOH, avec (i) du chlorure d'oxalyle ou du chlorure de thionyle suivi par (ii) HX₂R₁₂, où X₂ et R₁₂ sont définis dans la revendication 1, en présence d'une base pour donner un composé correspondant de formule I dans laquelle Y est C(O)X₂R₁₂ et X₂, R₁₂ sont tels que définis dans la revendication 1 ;
r) réaction d'un composé de formule I dans laquelle R, R₁, R₂, A, A₁, X, X₁ et Z sont définis dans la revendication 1 et Y est COOH, avec
(i) un agent de couplage, par exemple le 1-(3-diméthylaminopropyl)-3-éthylcarbodiimide, et
(ii) HNR₁₂R₁₃, où R₁₂ et R₁₃ sont définis dans la revendication 1
pour donner un composé correspondant de formule I dans laquelle Y est C(O)NR₁₂R₁₃, et R₁₂ et R₁₃ sont définis dans la revendication 1 ;
s) réaction d'un composé de formule I dans laquelle R, R₁, R₂, A, A₁, X, X₁ et Z sont définis dans la revendication 1 et Y est COOH, avec
(i) du chlorure d'oxalyle ou du chlorure de thionyle et
(ii) HNR₁₃SO₂NR₄₆R₄₈, où R₁₃, R₄₆ et R₄₈ sont définis dans la revendication 1
pour donner un composé correspondant de formule I dans laquelle Y est C(O)NR₁₃SO₂NR₄₆R₄₈ et R₁₃, R₄₆ et R₄₈ sont définis dans la revendication 1;
t) réaction d'un composé de formule I dans laquelle R, R₁, R₂, X, X₁, A, A₁, et Z sont définis dans la revendication 1, et Y est COOH, avec
(i) du chlorure d'oxalyle ou du chlorure de thionyle et
(ii) HN(R₁₂)OR₂₃, où R₁₂ et R₂₃ sont définis dans la revendication 1,
pour donner un composé correspondant de formule I dans laquelle Y est C(O)N(R₁₂)OR₂₃ et R₁₂, R₂₃ sont définis dans la revendication 1 ;
u) réaction d'un composé de formule I dans laquelle R, R₁, R₂, X, X₁, A, A₁, et Z sont définis dans la revendication 1, et Y est CHO avec R₂₃MgBr, où R₂₃ est défini dans la revendication 1 pour donner un composé correspondant de formule I dans laquelle Y est C(OH)R₂₃;
v) réaction d'un composé de formule I dans laquelle R, R₁, R₂, A, A₁, X, X₁, et Z sont définis dans la revendication 1 et Y est C(OH)R₂₃, avec un agent oxydant pour former un composé correspondant de formule I dans laquelle Y est C(O)R₂₃, où R₂₃ est défini dans la revendication 1 ;
w) réaction d'un composé de formule I dans laquelle R, R₁, R₂, A, A₁, X, X₁, et Z sont définis dans la revendication 1 et Y est C(OH)R₂₃, avec R₂₂X où R₂₂ est défini dans la revendication 1 et X est un chlore ou un brome, en présence d'une base pour former un composé correspondant de formule I dans laquelle Y est C(OR₂₂)R₂₃, où R₂₂ et R₂₃ sont définis dans la revendication 1 ;
x) réaction d'un composé de formule I dans laquelle R, R₁, R₂, A, A₁, X, X₁, et Z sont définis dans la revendication 1 et Y est C(OH)R₂₃, où R₂₃ est défini dans la revendication 1, avec XC(O)R₃₃, où R₃₃ est défini dans la revendication 1 et X est un chlore ou un brome, pour donner un composé correspondant de formule I dans laquelle Y est CH(R₂₃)OC(O)R₃₃, où R₂₃ et R₃₃ sont définis dans la revendication 1 ;
ou
y) conversion d'un composé de formule (I) ou (II) ayant un groupe substituant réactif en un composé différent de formule (I) ou (II) au moyen d'un réactif approprié.

21. Procédé de préparation d'un intermédiaire de formule (XLV) selon la revendication 19 qui comprend l'une des étapes suivantes :
a) réaction d'un composé de formule : 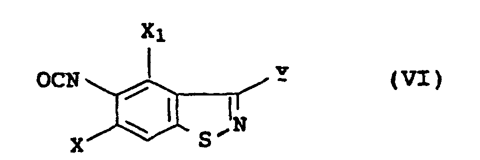 dans laquelle X, X₁ et Y sont définis dans la revendication 1 avec un composé de formule :
R₁C(NH₂)=CR₂-CO₂(alkyle en C₁-C₄) (VII)
dans laquelle R₁ et R₂ sont définis dans la revendication 1
pour produire un composé de formule : 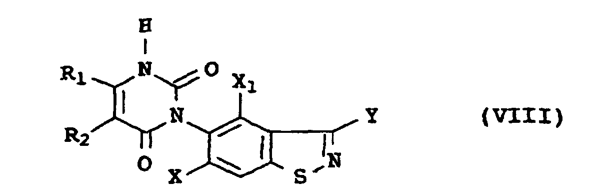 dans laquelle R₁, R₂, X, X₁ et Y sont définis dans la revendication 1 ;
b) réaction d'un composé de formule : 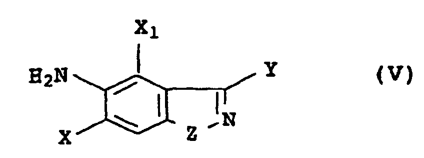 dans laquelle X, X₁, Z et Y sont définis dans la revendication 1
avec un composé de formule : 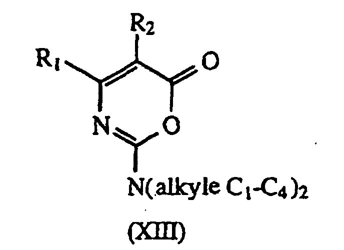 dans laquelle R₁ et R₂ sont définis dans la revendication 1, en présence d'un acide organique, par exemple l'acide acétique, pour former un composé de formule VIII telle que présentée ci-dessus dans laquelle R₁, R₂, X, X₁ et Y sont définis dans la revendication 1 ;
ou
c) réaction d'un composé de formule XXXVIII: 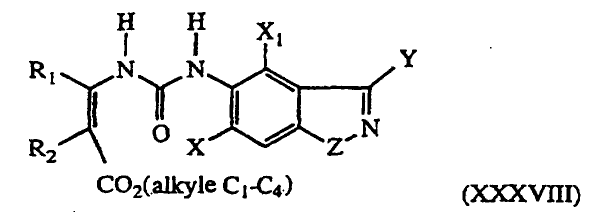 dans laquelle R₁, R₂, X, X₁, Z et Y sont définis dans la revendication 1, avec un acide pour former un composé de formule : 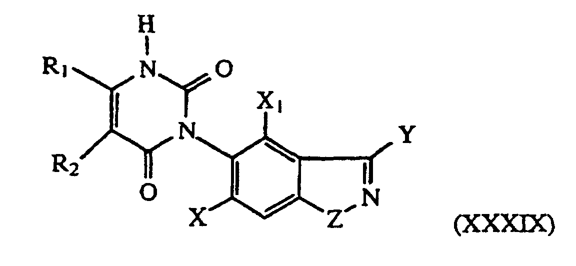 dans laquelle R₁, R₂, X, X₁, Y et Z sont définis dans la revendication 1.
